# EUROPEAN PATENT APPLICATION

(11) **EP 1 637 601 A2**
(43) Date of publication of application: **22.03.2006**
(21) Application number: 05019098.2
(22) Date of filing: 21.02.2002
(51) Int. Cl.: C12N 15/12, C12N 5/10, C07K 14/47, C07K 16/18, G01N 33/50, G01N 33/53, A61K 31/713, A61K 48/00

(54) **Proteins, polynucleotides encoding them and methods of using the same**

(30) Priority: 21.02.2001 US 270523 P; 21.02.2002 US 270220 P; 23.02.2001 US 270797 P; 23.02.2001 US 270810 P; 08.03.2001 US 274295 P; 16.03.2001 US 276677 P; 16.03.2001 US 276400 P; 26.03.2001 US 278796 P; 04.04.2001 US 281521 P; 25.04.2001 US 286548 P; 17.05.2001 US 291765 P; 10.08.2001 US 311595 P; 13.08.2001 US 311980 P; 10.09.2001 US 318526 P; 17.09.2001 US 322712 P; 18.10.2001 US 330307 P
(62) Divisional of application: 02739088.9
(71) Applicant: Curagen Corporation, New Haven, CT 06511 (US)
(72) Inventor: Pena, Carol E. A., New Haven, CT 06511 (US); Shimkets, Richard A., Guildford, CT 06437 (US); Li, Li, Branford, CT 06405 (US); Shenoy, Suresh G., Branford, CT 06405 (US); Kekuda, Ramesh, Danbury, CT 06810 (US); Spytek, Kimberly A., New Haven, CT 06511 (US); Vernet, Corine A. M., Branford, CT 06471 (US); Malyankar, Uriel L., Branford, CT 06405 (US); Guo, Xiaojia, Branford, CT 06405 (US); Gusev, Vladimir Y., Madison, CT 06443 (US); Casman, Stacie J., North Haven, CT 06473 (US); Boldog, Ferenc L., North Haven, CT 06473 (US); Furtak, Katarzyna, Ansonia, CT 06401 (US); Tchernev, Velizar T., Branford, CT 06405 (US); Patturajan, Meera, Branford, CT 06405 (US); Gangolli, Esha A., Madison, CT 06443 (US); Padigaru, Muralidhara, Branford, CT 06405 (US); Liu, Xiaohong, Branford, CT 06405 (US); Baumgartner, Jason C., New Haven, CT 06513 (US); Gerlach, Valerie L., Branford, CT 06405 (US); Spardena, Steven K., Berlin, CT 06037 (DE); Zerhusen, Bryan D., Branford, CT 06405 (US)
(74) Representative: Naylor, Kathryn May

(57) **Abstract**

Disclosed herein are nucleic acid sequences that encode novel polypeptides. Also disclosed are polypeptides encoded by these nucleic acid sequences, and antibodies, which immunospecifically-bind to the polypeptide, as well as derivatives, variants, mutants, or fragments of the aforementioned polypeptide, polynucleotide, or antibody. The invention further discloses therapeutic uses and research methods relating to diagnosis, treatment, and prevention of disorders involving any one of these novel human nucleic acids and proteins.

SEQUENCE LISTING

<110> Curagen Corporation
      Pena, Carol E. A.
      Shimkets, Richard A
      Li, Li
      Shenoy, Suresh G
      Kekuda, Ramesh
      Spytek, Kimberly A
      Vernet, Corine A. M.
      Malyankar, Uriel M
      Guo, Xiaojia
      Gusev, Vladimir Y
      Casman, Stacie J
      Boldog, Ferenc L
      Furtak, Katarzyna
      Tchernev, Velizar T
      Patturajan, Meera
      Gangolli, Esha A
      Padigaru, Muralidhara
      Liu, Xiaohong
      Baumgartner, Jason C.
      Gerlach, Valerie
      Spaderna, Steven K
      Zerhusen, Bryan D

<120> Proteins, Polynucleotides Encoding Them and Methods of
      Using the Same

<130> 21402-275-061

<140> US02/05374
<141> 2002-02-21

<150> 10/080,334
<151> 2002-02-21

<150> 60/270,523
<151> 2001-02-21

<150> 60/322,712
<151> 2001-09-17

<150> 60/311,980
<151> 2001-08-13

<150> 60/330,307
<151> 2001-10-18

<150> 60/278,796
<151> 2001-03-26

<150> 60/281,521
<151> 2001-04-04

<150> 60/276,677
<151> 2001-03-16

<150> 60/311,595
<151> 2001-08-10

<150> 60/270,220
<151> 2001-02-21

<150> 60/274,295
<151> 2001-03-08

<150> 60/318,526
<151> 2001-09-10

<150> 60/286,548
<151> 2001-04-25

<150> 60/291,765
<151> 2001-05-17

<150> 60/270,797
<151> 2001-02-23

<150> 60/276,400
<151> 2001-03-16

<150> 60/270,810
<151> 2001-02-23

<160> 388

<170> PatentIn Ver. 2.1

<210> 1
<211> 872
<212> DNA
<213> Homo sapiens

<400> 1
ctcgtgaagg atggtacgtg atgctcttgt ttcccttgcc gataggaagt tctttgctgg 60
tggagtgtgt agaacaaatg tgcagcttcc tggcaaggta gtggtgatca ctggcgccaa 120
cacgggcatt ggcaaggaga cggccagaga gctcgctagc cgaggagccc gagtctatat 180
tgcctgcaga gatgtactga agggggagtc tgctgccagt gaaatccgag tggatacaaa 240
gaactcccag gtgctggtgc ggaaattgga cctatccgac accaaatcta tccgagcctt 300
tgctgagggc tttctggcag aggaaaagca gctccatatt ctgatcaaca atgcgggagt 360
aatgatgtgt ccatattcca agacagctga tggctttgaa acccacctgg gagtcaacca 420
cctgggccac ttcctcctca cctacctgct cctggagcgg ctaaaggtgt ctgcccctgc 480
acgggtggtt aatgtgtcct cggtggctca ccacattggc aagattccct tccacgacct 540
ccagagcgag aagcgctaca gcaggggttt tgcctattgc cacagcaagc tggccaatgt 600
gctttttact cgtgagctgg ccaagaggct ccaaggcacc ggggtcacca cctacgcagt 660
gcacccaggc gtcgtccgct ctgagctggt ccggcactcc tccctgctct gcctgctctg 720
gcggctcttc tccccctttg tcaagacggc acgggagggg gcgcagacca gcctgcactg 780
cgccctggct gagggcctgg agcccctgag tggcaagtac ttcggtgtct ccaagggccc 840
gaaataacaa aacagctgag cgcctatgga at 872

<210> 2
<211> 278
<212> PRT
<213> Homo sapiens

<400> 2
Met Val Arg Asp Ala Leu Val Ser Leu Ala Asp Arg Lys Phe Phe Ala
1               5                   10                  15

Gly Gly Val Cys Arg Thr Asn Val Gln Leu Pro Gly Lys Val Val Val
            20                  25                  30

Ile Thr Gly Ala Asn Thr Gly Ile Gly Lys Glu Thr Ala Arg Glu Leu
        35                  40                  45

Ala Ser Arg Gly Ala Arg Val Tyr Ile Ala Cys Arg Asp Val Leu Lys
    50                  55                  60

Gly Glu Ser Ala Ala Ser Glu Ile Arg Val Asp Thr Lys Asn Ser Gln
65                  70                  75                  80

Val Leu Val Arg Lys Leu Asp Leu Ser Asp Thr Lys Ser Ile Arg Ala
                85                  90                  95

Phe Ala Glu Gly Phe Leu Ala Glu Glu Lys Gln Leu His Ile Leu Ile
            100                 105                 110

Asn Asn Ala Gly Val Met Met Cys Pro Tyr Ser Lys Thr Ala Asp Gly
        115                 120                 125

Phe Glu Thr His Leu Gly Val Asn His Leu Gly His Phe Leu Leu Thr
    130                 135                 140

Tyr Leu Leu Leu Glu Arg Leu Lys Val Ser Ala Pro Ala Arg Val Val
145                 150                 155                 160

Asn Val Ser Ser Val Ala His His Ile Gly Lys Ile Pro Phe His Asp
                165                 170                 175

Leu Gln Ser Glu Lys Arg Tyr Ser Arg Gly Phe Ala Tyr Cys His Ser
            180                 185                 190

Lys Leu Ala Asn Val Leu Phe Thr Arg Glu Leu Ala Lys Arg Leu Gln
        195                 200                 205

Gly Thr Gly Val Thr Thr Tyr Ala Val His Pro Gly Val Val Arg Ser
    210                 215                 220

Glu Leu Val Arg His Ser Ser Leu Leu Cys Leu Leu Trp Arg Leu Phe
225                 230                 235                 240

Ser Pro Phe Val Lys Thr Ala Arg Glu Gly Ala Gln Thr Ser Leu His
                245                 250                 255

Cys Ala Leu Ala Glu Gly Leu Glu Pro Leu Ser Gly Lys Tyr Phe Gly
            260                 265                 270

Val Ser Lys Gly Pro Lys
        275

<210> 3
<211> 1527
<212> DNA
<213> Homo sapiens

<400> 3
tctcagtact gtgaagaagc tggaaaaagg gatattatgg ggctaaaagc tctctgtttg 60
gggctgcttt gtgttctttt tgtctctcat ttttacacac ccatgccaga caacattgaa 120
gaaagctgga aaataatggc cttggatgcc atcgctaaaa ctttgttctt acaggctatg 180
tgttttgaaa atatgcgtat tatgagatat gaagagttta tatccatgat attcaggctg 240
gattataccc aaccactttc agatgaatac atcacagtga ctgatacaac atttgttgac 300
attccagtac gattgtactt gccaaaaaga aagtcagaaa cccgaaggcg agctgtgata 360
tattttcatg gtggtggttt ttgttttgga agttccaaac agaggctttt tgacttcctg 420
aatagatgga cggcaaacac gcttgatgct gttgttgtag gcgtggacta taggctggct 480
cctcaacacc actttcctgc tcagtttgaa gatggccttg ctgcagtcaa atttttcttt 540
ttggaaaaaa ttcttacaaa atatggagtg gatcccaccc gaatctgcat tgcgggagac 600
agttctgggg gcaatttagc aacagcggtc actcaacagg tgcagaatga tgctgaaata 660
aaacataaaa tcaagatgca agtcttactt taccctggct tacagataac agattcttat 720
ttgccatctc accgagaaaa tgagcatggt atagttttga ccagggatgt agccataaaa 780
ctcgtgagct tatatttcac caaggatgaa gcacttccct gggcaatgag aagaaaccaa 840
cacatgcctc tggagtcaag acatctgttt aagtttgtta actggagtat tcttcttcct 900
gagaagtata gaaaagacta tgtatatact gaaccaattc ttggaggact tagttattca 960
ttgccaggac ttacagacag cagagcatta cccttgttgg ccaatgattc tcagttacag 1020
aatttgccac taacctatat tcttacttgt caacatgatc tcttaagaga tgatggactt 1080
atgtatgtta caagacttcg aaatgttgga gtccaagttg ttcatgaaca tattgaggat 1140
ggaattcatg gagctttatc attcatgact tcaccatttt atttacgtct aggtcttagg 1200
ataagagata tgtatgtaag ttggctggat aagaatttat aaatatgtga tgtgtatgta 1260
tagcccttac atagtggatt gtaatttgtg atattttgtg gttttggagc aaagaacaat 1320
gtcatttgag ttatctaaat ctacatttgc aacatttgta gcagttaatg tgtgtccttg 1380
aagagttatt aaattttctg acttgcagac cctgaatatg taaaatgtat gtaatcctgc 1440
ctattttctc cttacttata atttattata attatgttgg ttctaataag aaccaatgct 1500
tattaaagtt gagaaataag agtggtt 1527

<210> 4
<211> 401
<212> PRT
<213> Homo sapiens

<400> 4
Met Gly Leu Lys Ala Leu Cys Leu Gly Leu Leu Cys Val Leu Phe Val
1               5                   10                  15

Ser His Phe Tyr Thr Pro Met Pro Asp Asn Ile Glu Glu Ser Trp Lys
            20                  25                  30

Ile Met Ala Leu Asp Ala Ile Ala Lys Thr Leu Phe Leu Gln Ala Met
        35                  40                  45

Cys Phe Glu Asn Met Arg Ile Met Arg Tyr Glu Glu Phe Ile Ser Met
    50                  55                  60

Ile Phe Arg Leu Asp Tyr Thr Gln Pro Leu Ser Asp Glu Tyr Ile Thr
65                  70                  75                  80

Val Thr Asp Thr Thr Phe Val Asp Ile Pro Val Arg Leu Tyr Leu Pro
                85                  90                  95

Lys Arg Lys Ser Glu Thr Arg Arg Arg Ala Val Ile Tyr Phe His Gly
            100                 105                 110

Gly Gly Phe Cys Phe Gly Ser Ser Lys Gln Arg Ala Phe Asp Phe Leu
        115                 120                 125

Asn Arg Trp Thr Ala Asn Thr Leu Asp Ala Val Val Val Gly Val Asp
    130                 135                 140

Tyr Arg Leu Ala Pro Gln His His Phe Pro Ala Gln Phe Glu Asp Gly
145                 150                 155                 160

Leu Ala Ala Val Lys Phe Phe Leu Leu Glu Lys Ile Leu Thr Lys Tyr
                165                 170                 175

Gly Val Asp Pro Thr Arg Ile Cys Ile Ala Gly Asp Ser Ser Gly Gly
            180                 185                 190

Asn Leu Ala Thr Ala Val Thr Gln Gln Val Gln Asn Asp Ala Glu Ile
        195                 200                 205

Lys His Lys Ile Lys Met Gln Val Leu Leu Tyr Pro Gly Leu Gln Ile
    210                 215                 220

Thr Asp Ser Tyr Leu Pro Ser His Arg Glu Asn Glu His Gly Ile Val
225                 230                 235                 240

Leu Thr Arg Asp Val Ala Ile Lys Leu Val Ser Leu Tyr Phe Thr Lys
                245                 250                 255

Asp Glu Ala Leu Pro Trp Ala Met Arg Arg Asn Gln His Met Pro Leu
            260                 265                 270

Glu Ser Arg His Leu Phe Lys Phe Val Asn Trp Ser Ile Leu Leu Pro
        275                 280                 285

Glu Lys Tyr Arg Lys Asp Tyr Val Tyr Thr Glu Pro Ile Leu Gly Gly
    290                 295                 300

Leu Ser Tyr Ser Leu Pro Gly Leu Thr Asp Ser Arg Ala Leu Pro Leu
305                 310                 315                 320

Leu Ala Asn Asp Ser Gln Leu Gln Asn Leu Pro Leu Thr Tyr Ile Leu
                325                 330                 335

Thr Cys Gln His Asp Leu Leu Arg Asp Asp Gly Leu Met Tyr Val Thr
            340                 345                 350

Arg Leu Arg Asn Val Gly Val Gln Val Val His Glu His Ile Glu Asp
        355                 360                 365

Gly Ile His Gly Ala Leu Ser Phe Met Thr Ser Pro Phe Tyr Leu Arg
    370                 375                 380

Leu Gly Leu Arg Ile Arg Asp Met Tyr Val Ser Trp Leu Asp Lys Asn
385                 390                 395                 400

Leu

<210> 5
<211> 1324
<212> DNA
<213> Homo sapiens

<400> 5
cagtactgtg aagaagctgg aaaaagggat attatggggc taaaagctct ctgtttgggg 60
ctgctttgtg ttctttttgt ctctcatttt tacacaccca tgccagacaa cattgaagaa 120
agctggaaaa taatggcctt ggatgccatc gctaaaactt gtacatttac ggctatgtgt 180
tttgaaaata tgcgtattat gagatatgaa gagtttatat ccatgatatt caggctggat 240
tatacccaac cactttcaga tgaatacatc acagtgactg atacaacatt tgttgacatt 300
ccagtacgat tgtacttgcc aaaaagaaag tcagaaaccc gaaggcgagc tgtgatatat 360
tttcatggtg gtggtttttg ttttggaagt tccaaacaga gggcttttga cttcctgaat 420
agatggacgg caaacacgct tgatgctgtt gttgtaggcg tggactatag gctggctcct 480
caacaccact ttcctgctca gtttgaagat ggccttgctg cagtcaaatt ttttcttttg 540
gaaaaaattc ttacaaaata tggagtggat cccacccgaa tctgcatttc gggagacagt 600
tctgggggca atttagcaac agcggtcact caacaggtgc agaatgatgc tgaaataaaa 660
cataaaatca agatgcaagt cttactttac cctggcttac agataacaga ttcttatttg 720
ccatctcacc gagaaaatga gcatggtata gttttgacca gggatgtagc cataaaactc 780
gtgagcttat atttcaccaa ggatgaagca cttccctggg caatgagaag aaaccaacac 840
atgcctctgg agtcaagaca tctgtttaag tttgttaact ggagtattct tcttcctgag 900
aagcatagaa aagactatgt atatactgaa ccaattcttg gaggacttag ttattcattg 960
ccaggactta cagacagcag agcattaccc ttgttggcca atgattctca gttacagaat 1020
ttgccactaa cctatattct tacttgtcaa catgatctct taagagatga tggacttatg 1080
tatgttacaa gacttcgaaa tgttggagtc caagttgttc atgaacatat tgaggatgga 1140
attcatggag ctttatcatt catgacttca ccattttatt tacgtctagg tcttaggata 1200
agagatatgt atgtaagttg gctggataag aatttataaa tatgtgatgt gtatgtatag 1260
cctttacata gtggattgta atttgtgata ttttgtggtt ttggagcaaa gaacaatgtc 1320
attt                                                              1324

<210> 6
<211> 401
<212> PRT
<213> Homo sapiens

<400> 6
Met Gly Leu Lys Ala Leu Cys Leu Gly Leu Leu Cys Val Leu Phe Val
1               5                   10                  15

Ser His Phe Tyr Thr Pro Met Pro Asp Asn Ile Glu Glu Ser Trp Lys
            20                  25                  30

Ile Met Ala Leu Asp Ala Ile Ala Lys Thr Cys Thr Phe Thr Ala Met
        35                  40                  45

Cys Phe Glu Asn Met Arg Ile Met Arg Tyr Glu Glu Phe Ile Ser Met
    50                  55                  60

Ile Phe Arg Leu Asp Tyr Thr Gln Pro Leu Ser Asp Glu Tyr Ile Thr
65                  70                  75                  80

Val Thr Asp Thr Thr Phe Val Asp Ile Pro Val Arg Leu Tyr Leu Pro

85                  90                  95

Lys Arg Lys Ser Glu Thr Arg Arg Arg Ala Val Ile Tyr Phe His Gly
            100                 105                 110

Gly Gly Phe Cys Phe Gly Ser Ser Lys Gln Arg Ala Phe Asp Phe Leu
        115                 120                 125

Asn Arg Trp Thr Ala Asn Thr Leu Asp Ala Val Val Val Gly Val Asp
    130                 135                 140

Tyr Arg Leu Ala Pro Gln His His Phe Pro Ala Gln Phe Glu Asp Gly
145                 150                 155                 160

Leu Ala Ala Val Lys Phe Phe Leu Leu Glu Lys Ile Leu Thr Lys Tyr
                165                 170                 175

Gly Val Asp Pro Thr Arg Ile Cys Ile Ser Gly Asp Ser Ser Gly Gly
            180                 185                 190

Asn Leu Ala Thr Ala Val Thr Gln Gln Val Gln Asn Asp Ala Glu Ile
        195                 200                 205

Lys His Lys Ile Lys Met Gln Val Leu Leu Tyr Pro Gly Leu Gln Ile
    210                 215                 220

Thr Asp Ser Tyr Leu Pro Ser His Arg Glu Asn Glu His Gly Ile Val
225                 230                 235                 240

Leu Thr Arg Asp Val Ala Ile Lys Leu Val Ser Leu Tyr Phe Thr Lys
                245                 250                 255

Asp Glu Ala Leu Pro Trp Ala Met Arg Arg Asn Gln His Met Pro Leu
            260                 265                 270

Glu Ser Arg His Leu Phe Lys Phe Val Asn Trp Ser Ile Leu Leu Pro
        275                 280                 285

Glu Lys His Arg Lys Asp Tyr Val Tyr Thr Glu Pro Ile Leu Gly Gly
    290                 295                 300

Leu Ser Tyr Ser Leu Pro Gly Leu Thr Asp Ser Arg Ala Leu Pro Leu
305                 310                 315                 320

Leu Ala Asn Asp Ser Gln Leu Gln Asn Leu Pro Leu Thr Tyr Ile Leu
                325                 330                 335

Thr Cys Gln His Asp Leu Leu Arg Asp Asp Gly Leu Met Tyr Val Thr
            340                 345                 350

Arg Leu Arg Asn Val Gly Val Gln Val Val His Glu His Ile Glu Asp
        355                 360                 365

Gly Ile His Gly Ala Leu Ser Phe Met Thr Ser Pro Phe Tyr Leu Arg
    370                 375                 380

Leu Gly Leu Arg Ile Arg Asp Met Tyr Val Ser Trp Leu Asp Lys Asn
385                 390                 395                 400

Leu


<210> 7
<211> 4321
<212> DNA
<213> Homo sapiens

<400> 7
   ccggctcgaa gcgcaacgag gaagcgtttg cggtgatccc ggcgactgcg ctggctaatg 60
   cggtaccggc tagcgtggct tctgcacccc gcactgccca gcaccttccg ctcagtcctc 120
   ggcgcccgcc tgccgcctcc ggagcgcctg tgtggtttcc aaaaaaagac ttacagcaaa 180
   atgaataatc cagccatcaa gagaatagga aatcacatta ccaagtctcc tgaagacaag 240
   cgagaatatc gagggctaga gctggccaat ggtatcaaag tacttcttat cagtgatccc 300
   accacggata agtcatcagc agcacttgat gtgcacatag gttcattgtc ggatcctcca 360
   aatattgctg gcttaagtca tttttgtgaa catatgcttt ttttgggaac aaagaaatac 420
   cctaaagaaa atgaatacag ccagtttctc agtgagcatg caggaagttc aaatgccttt 480
   actagtggag agcataccaa ttactatttt gatgtttctc atgaacacct agaaggtgcc 540
   ctagacaggt ttgcacagtt ttttctgtgc cccttgttcg atgaaagttg caaagacaga 600
   gaggtgaatg cagttgattc agaacatgag aagaatgtga tgaatgatgc ctggagactc 660
   tttcaattgg aaaaagctac agggaatcct aaacacccct tcagtaaatt tgggacaggt 720
   aacaaatata ctctggagac tagaccaaac caagaaggca ttgatgtaag acaagagcta 780
   ctgaaattcc attctgctta ctattcatcc aacttaatgg ctgtttgtgt tttaggtcga 840
   gaatctttag atgacttgac taatctggtg gtaaagttat tttctgaagt agagaacaaa 900
   aatgttccat tgccagaatt tcctgaacac cctttccaag aagaacatct taaacaactt 960
   tacaaaatag tacccattaa agatattagg aatctctatg tgacatttcc catacctgac 1020
   cttcagaaat actacaaatc aaatcctggt cattatcttg gtcatctcat tgggcatgaa 1080
   ggtcctggaa gtctgttatc agaacttaag tcaaagggct gggttaatac tcttgttggt 1140
   gggcagaagg aaggagcccg aggttttatg ttttttatca ttaatgtgga cttgaccgag 1200
   gaaggattat tacatgttga agatataatt ttgcacatgt ttcaatacat tcagaagtta 1260
   cgtgcagaag gacctcaaga atgggttttc caagagtgca aggacttgaa tgctgttgct 1320
   tttaggttta aagacaaaga gaggccacgg ggctatacat ctaagattgc aggaatattg 1380
   cattattatc ccctagaaga ggtgctcaca gcggaatatt tactggaaga atttagacct 1440
   gacttaatag agatggttct cgataaactc agaccagaaa atgtccgggt tgccatagtt 1500
   tctaaatctt ttgaaggaaa aactgatcgc acagaagagt ggtatggaac ccagtacaaa 1560
   caagaagcta taccggatga agtcatcaag aaatggcaaa atgctgacct gaatgggaaa 1620
   tttaaacttc ctacaaagaa tgaatttatt cctacgaatt ttgagatttt accgttagaa 1680
   aaagaggcga caccataccc tgctcttatt aaggatacag ctatgagcaa actttggttc 1740
   aaacaagatg ataagttttt tttgccgaag gcttgtctca actttgaatt tttcagtcgc 1800
   tacatttatg ctgatcctct ccattgcaac atgacatacc tgtttatcag gttattgaag 1860
   gatgatttaa aagagtatac atatgcagca cgcctctcag gtttgagcta tggcattgca 1920
   tcaggaatga atgcaatact tctttcagtg aaaggttaca atgacaagca gccaatttta 1980
   ctaaagaaga ttattgagaa aatggctacc tttgagattg atgaaaaaag atttgaaatt 2040
   atcaaagaag catatatgcg atctcttaac aatttccggg ctgaacagcc tcaccagcat 2100
   gccatgtact acctccgctt gctgatgact gaagtggcct ggactaaaga tgagttaaaa 2160
   gaagctctgg atgatgtaac ccttcctcgc cttaaggcct tcatacctca gctcctgtca 2220
   cggctgcaca ttgaagccct tctccatgga aacataacaa agcaggctgc attaggaatt 2280
   atgcagatgg ttgaagacac cctcattgaa catgctcata ccaaacctct ccttccaagt 2340
   cagctggttc ggtatagaga agttcagctc cctgacagag gatggtttgt ttatcagcag 2400
   agagatgaag ttcacaataa ctgtggcatc gagatatact accaaacaga catgcaaagc 2460
   acctcagaga atatgtttct ggagctcttc tgtcagatta tctcggaacc ttgcttcaac 2520
   accctgcgca ccaaggagca gttgggctat atcgtcttca gcgggccacg tcgagctaat 2580
   ggcatacagg gcttgagatt catcatccag tcagaaaagc cacctcacta cctagaaagc 2640
   agagtggaag ctttcttaat taccatggaa aagtccatag aggacatgac agaagaggcc 2700
   ttccaaaaac acattcaggc attagcaatt cgtcgactag acaaaccaaa gaagctatct 2760
   gctgagtgtg ctaaatactg gggagaaatc atctcccagc aatataattt tgacagagat 2820
   aacactgagg ttgcatattt aaagacactt accaaggaag atatcatcaa attctacaag 2880
   gaaatgttgg cagtagatgc tccaaggaga cataaggtat ccgtccatgt tcttgccagg 2940
   gaaatggatt cttgtcctgt tgttggagag ttcccatgtc aaaatgacat aaatttgtca 3000
   caagcaccag ccttgccaca acctgaagtg attcagaaca tgaccgaatt caagcgtggt 3060
   ctgccactgt ttccccttgt gaaaccacat attaacttca tggctgcaaa actctgaaga 3120
   ttccccatgc atgggaaagt gcaagtggat gcattcctga gtcttccaga gcctaagaaa 3180
   atcatcttgg ccactttaat agtttctgat tcactattag agaaacaaac aaaaaattgt 3240
   caaatgtcat tatgtagaaa tattataaat ccaaagtaaa ttacaaaatc ttatagatgt 3300
   agaatatttt ttaaatacat gcctcttaaa tattttaaaa tttttctttt gattactgag 3360
   agaaatttcc ccaatataac aatgcttaaa atgaatgata ttcctataga atcttccttc 3420
   cctattctgt aaaatagtca cttgtccgaa gaaagttaaa agttagctct tttctaaaag 3480
   cctcctagct tgacatagaa ggcttcacaa catttagaaa ggtaataact ttttaaaaat 3540
   tgatcctcaa atttgctttc tacttgatgg tttcatgtaa atcagtggaa aacattacat 3600
   ttggcagatg ataaagcaat gtcatctttt attagtgaaa tgctggttat ataaggcatg 3660
   gttttaatct ttttataaaa tttgaacatg ttttttatgc caactcgtaa aatgctagaa 3720
   aaccctactt atttacaatg ctagaaatac agacttacct tacatcaatt ttgtcctaaa 3780
   ccgaatttct caggattact gtggtttctt tcattctgat tgaattatat tgacctactt 3840
   cttcatagtt ggtttgcagt gttccatgag ttttactttt cctcatcaac atattgcttt 3900
   aacacaacat atttatttaa cacgtacaaa tagggtcaac ttcagatcct actgagtgtg 3960
   tgacatgctt ttccaacatc agctttttgt aaccacctgt ataacttttt attacagtga 4020
   aattgcagtc agtatgtgaa ccaaaatatc ttgccccttt atgaatttaa aaggcagcca 4080
   atacaaagcc acctttttgg aaatataaaa agtaaagcct tgcattctta tatagcaggt 4140
   cttcataaaa ctctaaaatc ccttgttgct accagtctaa tcttgcctta aatgttaagt 4200
   tattttttga atatataaat ataaacatat aaacacagat gatgactgga gtagactttt 4260
   aaaaaaatat ttttttcatg agatactatt ttaggtgaaa ttgtactgta gatttacagc 4320
   t 4321

<210> 8
<211> 1019
<212> PRT
<213> Homo sapiens

<400> 8
Met Arg Tyr Arg Leu Ala Trp Leu Leu His Pro Ala Leu Pro Ser Thr
1               5                   10                  15

Phe Arg Ser Val Leu Gly Ala Arg Leu Pro Pro Pro Glu Arg Leu Cys
            20                  25                  30

Gly Phe Gln Lys Lys Thr Tyr Ser Lys Met Asn Asn Pro Ala Ile Lys
        35                  40                  45

Arg Ile Gly Asn His Ile Thr Lys Ser Pro Glu Asp Lys Arg Glu Tyr
    50                  55                  60

Arg Gly Leu Glu Leu Ala Asn Gly Ile Lys Val Leu Leu Ile Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Ala Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Phe Asp Glu
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Gln Glu
225                 230                 235                 240

Leu Leu Lys Phe His Ser Ala Tyr Tyr Ser Ser Asn Leu Met Ala Val
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Lys Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Lys Tyr Tyr Lys Ser Asn Pro Gly His Tyr Leu Gly His
                325                 330                 335

Leu Ile Gly His Glu Gly Pro Gly Ser Leu Leu Ser Glu Leu Lys Ser
            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Lys
385                 390                 395                 400

Leu Arg Ala Glu Gly Pro Gln Glu Trp Val Phe Gln Glu Cys Lys Asp
                405                 410                 415

Leu Asn Ala Val Ala Phe Arg Phe Lys Asp Lys Glu Arg Pro Arg Gly
            420                 425                 430

Tyr Thr Ser Lys Ile Ala Gly Ile Leu His Tyr Tyr Pro Leu Glu Glu
        435                 440                 445

Val Leu Thr Ala Glu Tyr Leu Leu Glu Glu Phe Arg Pro Asp Leu Ile
    450                 455                 460

Glu Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Glu Trp Tyr
                485                 490                 495

Gly Thr Gln Tyr Lys Gln Glu Ala Ile Pro Asp Glu Val Ile Lys Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Pro Leu Glu Lys Glu Ala
    530                 535                 540

Thr Pro Tyr Pro Ala Leu Ile Lys Asp Thr Ala Met Ser Lys Leu Trp
545                 550                 555                 560

Phe Lys Gln Asp Asp Lys Phe Phe Leu Pro Lys Ala Cys Leu Asn Phe
                565                 570                 575

Glu Phe Phe Ser Arg Tyr Ile Tyr Ala Asp Pro Leu His Cys Asn Met
            580                 585                 590

Thr Tyr Leu Phe Ile Arg Leu Leu Lys Asp Asp Leu Lys Glu Tyr Thr
        595                 600                 605

Tyr Ala Ala Arg Leu Ser Gly Leu Ser Tyr Gly Ile Ala Ser Gly Met
    610                 615                 620

Asn Ala Ile Leu Leu Ser Val Lys Gly Tyr Asn Asp Lys Gln Pro Ile
625                 630                 635                 640

Leu Leu Lys Lys Ile Ile Glu Lys Met Ala Thr Phe Glu Ile Asp Glu
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu
705                 710                 715                 720

Ser Arg Leu His Ile Glu Ala Leu Leu His Gly Asn Ile Thr Lys Gln
                725                 730                 735

Ala Ala Leu Gly Ile Met Gln Met Val Glu Asp Thr Leu Ile Glu His
            740                 745                 750

Ala His Thr Lys Pro Leu Leu Pro Ser Gln Leu Val Arg Tyr Arg Glu
        755                 760                 765

Val Gln Leu Pro Asp Arg Gly Trp Phe Val Tyr Gln Gln Arg Asp Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Gly Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ser Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Phe Asp Arg Asp Asn Thr Glu Val Ala Tyr Leu
        915                 920                 925

Lys Thr Leu Thr Lys Glu Asp Ile Ile Lys Phe Tyr Lys Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Cys Gln Asn
                965                 970                 975

Asp Ile Asn Leu Ser Gln Ala Pro Ala Leu Pro Gln Pro Glu Val Ile
            980                 985                 990

Gln Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015


<210> 9
<211> 3153

<212> DNA
<213> Homo sapiens

<400> 9
   aagcgtttgc ggtgatcccg gcgactgcgc tggctaatgc ggtaccggct agcgtggctt 60
   ctgcaccccg cactgcccag caccttccgc tcagtcctcg gcgcccgcct gccgcctccg 120
   gagcgcctgt gtggtttcca aaaaaagact tacagcaaaa tgaataatcc agccatcaag 180
   agaataggaa atcacattac caagcctcct gaagacaagc gagaatatcg agggctagag 240
   ctggccaatg gtatcaaagt acttcttatc agtgatccca ccacggataa gtcatcagca 300
   gcacttgatg tgcacatagg ttcattgtcg gatcctccaa atattgctgg cttaagtcat 360
   ttttgtgaac atatgctttt tttgggaaca aagaaatacc ctaaagaaaa tgaatacagc 420
   cagtttctca gtgagcatgc aggaagttca aatgccttta ctagtggaga gcataccaat 480
   tactattttg atgtttctca tgaacaccta gaaggtgccc tagacaggtt tgcacagttt 540
   tttctgtgcc ccttgttcga tgaaagttgc aaagacagag aggtgaatgc agttgattca 600
   gaacatgaga agaatgtgat gaatgatgcc tggagactcc ttcaattgga aaaagctaca 660
   gggaatccta aacacccctt cagtaaattt gggacaggta acaaatatac tctggagact 720
   agaccaaacc aagaaggcat tgatgtaaga caagagctac tgaaattcca ttctgcttac 780
   tattcatcca acttaatggc tgtttgtgtt ttaggtcgag aatctttaga tgacttgact 840
   aatctggtgg taaagttatt ttctgaagta gagaacaaaa atgttccatt gccagaattt 900
   cctgaacacc ctttccaaga agaacatctt aaacaacttt acaaaatagt acccattaaa 960
   gatattagga atctctatgt gacatttccc atacctgacc ttcagaaata ctacaaatca 1020
   aatcctggtc attatcttgg tcatctcatt gggcatgaag gtcctggaag tctgttatca 1080
   gaacttaagt caaagggctg ggttaatact cttgttggtg ggcagaagga aggagcccga 1140
   ggttttatgt tttttatcat taatgtggac ttgaccgagg aaggattatt acatgttgaa 1200
   gatataattt tgcacatgtt tcaatacatt cagaggttac gtgcagaagg acctcaagaa 1260
   tgggttttcc aagagtgcaa ggacttgaat gctgttactt ttaggtttaa agacaaagag 1320
   aggccacggg gctatacatc taagattgca ggaatattgc attattatcc cctagaagag 1380
   gtgctcacag cggaatattt actggaagaa tttagacctg acttaataga gatggttctc 1440
   gataaactca gaccagaaaa tgtccgggtt gccatagttt ctaaatcttt tgaaggaaaa 1500
   actgatcgca cagaagagtg gtatggaacc cagtacaaac aagaagctat accggatgaa 1560
   gtcatcaaga aatggcaaaa tgctgacctg aatgggaaat ttaaacttcc tacaaagaat 1620
   gaatttattc ctacgaattt tgagatttta ccgttagaaa aagaggcgac accataccct 1680
   gctcttatta aggatacagc tatgagcaaa ctttggttca aacaagatga taagtttttt 1740
   ttgccgaagg cttgtctcaa ctttgaattt ttcagtcgct acatttatgc tgatcctctc 1800
   cattgcaaca tgacatacct gtttatcagg ttattgaagg atgatttaaa agagtataca 1860
   tatgcagcac gcctctcagg tttgagctat ggcattgcat caggaatgaa tgcaatactt 1920
   ctttcagtga aaggttacaa tgacaagcag ccaattttac taaagaagat tattgagaaa 1980
   atggctacct ttgagattga tgaaaaaaga tttgaaatta tcaaagaagc atatatgcga 2040
   tctcttaaca atttccgggc tgaacagcct caccagcatg ccatgtacta cctccgcttg 2100
   ctgatgactg aagtggcctg gactaaagat gagttaaaag aggctctgga tgatgtaacc 2160
   cttcctcgcc ttaaggcctt catacctcag ctcctgtcac ggctgcacat tgaagccctt 2220
   ctccatggaa acataacaaa gcaggctgca ttaggaatta tgcagatggt tgaagacacc 2280
   ctcattgaac atgctcatac caaacctctc cttccaagtc agctggttcg gtatagagaa 2340
   gttcagctcc ctgacagagg atggtttgtt tatcagcaga gaaatgaagt tcacaataac 2400
   tgtggcatcg agatatacta ccaaacagac atgcaaagca cctcagagaa tatgtttctg 2460
   gagctcttct gtcagattat ctcggaacct tgcttcaaca ccctgcgcac caaggagcag 2520
   ttgggctata tcgtcttcag cgggccacgt cgagctaatg gcatacaggg cttgagattc 2580
   atcatccagt cagaaaagcc acctcactac ctagaaagca gagtggaagc tttcttaatt 2640
   accatggaaa agtccataga ggacatgaca gaagaggcct tccaaaaaca cattcaggca 2700
   ttagcaattc gtcgactaga caaaccaaag aagctatctg ctgagtgtgc taaatactgg 2760
   ggagaaatca tctcccagca atataatttt gacagagata acactgaggt agcatattta 2820
   aagacactta ccaaggaaga tatcatcaaa ttctacaagg aaatgttggc agtagatgct 2880
   ccaaggagac ataaggtatc cgtccatgtt cttgccaggg aaatggattc ttgtcctgtt 2940
   gttggagagt tcccatgtca aaatgacata aatttgtcac aagcaccagc cttgccacaa 3000
   cctgaagtga ttcagaacat gaccgaattc aagcgtggtc tgccactgtt tccccttgtg 3060
   aaaccacata ttaacttcat ggctgcaaaa ctctgaagat tccccatgca tgggaaagtg 3120
   caagtggatg cattcctgag tcttccagag cct 3153

<210> 10
<211> 1019
<212> PRT
<213> Homo sapiens

<400> 10
Met Arg Tyr Arg Leu Ala Trp Leu Leu His Pro Ala Leu Pro Ser Thr
1               5                   10                  15

Phe Arg Ser Val Leu Gly Ala Arg Leu Pro Pro Pro Glu Arg Leu Cys

20                  25                  30

Gly Phe Gln Lys Lys Thr Tyr Ser Lys Met Asn Asn Pro Ala Ile Lys
        35                  40                  45

Arg Ile Gly Asn His Ile Thr Lys Pro Pro Glu Asp Lys Arg Glu Tyr
    50                  55                  60

Arg Gly Leu Glu Leu Ala Asn Gly Ile Lys Val Leu Leu Ile Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Ala Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Phe Asp Glu
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Leu Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Gln Glu
225                 230                 235                 240

Leu Leu Lys Phe His Ser Ala Tyr Tyr Ser Ser Asn Leu Met Ala Val
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Lys Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Lys Tyr Tyr Lys Ser Asn Pro Gly His Tyr Leu Gly His
                325                 330                 335

Leu Ile Gly His Glu Gly Pro Gly Ser Leu Leu Ser Glu Leu Lys Ser
            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Arg

385                 390                 395                 400

Leu Arg Ala Glu Gly Pro Gln Glu Trp Val Phe Gln Glu Cys Lys Asp
                405                 410                 415

Leu Asn Ala Val Thr Phe Arg Phe Lys Asp Lys Glu Arg Pro Arg Gly
            420                 425                 430

Tyr Thr Ser Lys Ile Ala Gly Ile Leu His Tyr Tyr Pro Leu Glu Glu
        435                 440                 445

Val Leu Thr Ala Glu Tyr Leu Leu Glu Glu Phe Arg Pro Asp Leu Ile
    450                 455                 460

Glu Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Glu Trp Tyr
                485                 490                 495

Gly Thr Gln Tyr Lys Gln Glu Ala Ile Pro Asp Glu Val Ile Lys Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Pro Leu Glu Lys Glu Ala
    530                 535                 540

Thr Pro Tyr Pro Ala Leu Ile Lys Asp Thr Ala Met Ser Lys Leu Trp
545                 550                 555                 560

Phe Lys Gln Asp Asp Lys Phe Phe Leu Pro Lys Ala Cys Leu Asn Phe
                565                 570                 575

Glu Phe Phe Ser Arg Tyr Ile Tyr Ala Asp Pro Leu His Cys Asn Met
            580                 585                 590

Thr Tyr Leu Phe Ile Arg Leu Leu Lys Asp Asp Leu Lys Glu Tyr Thr
        595                 600                 605

Tyr Ala Ala Arg Leu Ser Gly Leu Ser Tyr Gly Ile Ala Ser Gly Met
    610                 615                 620

Asn Ala Ile Leu Leu Ser Val Lys Gly Tyr Asn Asp Lys Gln Pro Ile
625                 630                 635                 640

Leu Leu Lys Lys Ile Ile Glu Lys Met Ala Thr Phe Glu Ile Asp Glu
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu
705                 710                 715                 720

Ser Arg Leu His Ile Glu Ala Leu Leu His Gly Asn Ile Thr Lys Gln
                725                 730                 735

Ala Ala Leu Gly Ile Met Gln Met Val Glu Asp Thr Leu Ile Glu His
            740                 745                 750

Ala His Thr Lys Pro Leu Leu Pro Ser Gln Leu Val Arg Tyr Arg Glu

755                 760                 765

Val Gln Leu Pro Asp Arg Gly Trp Phe Val Tyr Gln Gln Arg Asn Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Gly Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ser Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Phe Asp Arg Asp Asn Thr Glu Val Ala Tyr Leu
        915                 920                 925

Lys Thr Leu Thr Lys Glu Asp Ile Ile Lys Phe Tyr Lys Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Cys Gln Asn
                965                 970                 975

Asp Ile Asn Leu Ser Gln Ala Pro Ala Leu Pro Gln Pro Glu Val Ile
            980                 985                 990

Gln Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015


<210> 11
<211> 2798
<212> DNA
<213> Homo sapiens

<400> 11
   cgttgctgtc gctctgcacg cacctatgtg gaaactaaag cccagagaga aagtctgact 60
   tgccccacag ccagtgagtg actgcagcag caccagaatc tggtctgttt cctgtttggc 120
   tcttctacca ctacggcttg ggatctcggg catggtggct ttgccaatgg tccttgtttt 180
   gctgctggtc ctgagcagag gtgagagtga attggacgcc aagatcccat ccacagggga 240
   tgccacagaa tggcggaatc ctcacctgtc catgctgggg tcctgccagc cagccccctc 300
   ctgccagaag tgcatcctct cacaccccag ctgtgcatgg tgcaagcaac tgaacttcac 360
   cgcgtcggga gaggcggagg cgcggcgctg cgcccgacga gaggagctgc tggctcgagg 420
   ctgcccgctg gaggagctgg aggagccccg cggccagcag gaggtgctgc aggaccagcc 480
   gctcagccag ggcgcccgcg gagagggtgc cacccagctg gcgccgcagc gggtccgggt 540
   cacgctgcgg cctggggagc cccagcagct ccaggtccgc ttccttcgtg ctgagggata 600
   cccggtggac ctgtactacc ttatggacct gagctactcc atgaaggacg acctggaacg 660
   cgtgcgccag ctcgggcacg ctctgctggt ccggctgcag gaagtcaccc attctgtgcg 720
   cattggtttt ggttcctttg tggacaaaac ggtgctgccc tttgtgagca cagtaccctc 780
   caaactgcgc cacccctgcc ccacccggct ggagcgctgc cagtcaccat tcagctttca 840
   ccatgtgctg tccctgacgg gggacgcaca agccttcgag cgggaggtgg ggcgccagag 900
   tgtgtccggc aatctggact cgcctgaagg tggcttcgat gccattctgc aggctgcact 960
   ctgccaggag cagattggct ggagaaatgt gtcccggctg ctggtgttca cttcagacga 1020
   cacattccat acagctgggg acgggaagtt gggcggcatt ttcatgccca gtgatgggca 1080
   ctgccacttg gacagcaatg gcctctacag tcgcagcaca gagtttgact acccttctgt 1140
   gggtcaggta gcccaggccc tctctgcagc aaatatccag cccatctttg ctgtcaccag 1200
   tgccgcactg cctgtctacc aggagctgag taaactgatt cctaagtctg cagttgggga 1260
   gctgagtgag gactccagca acgtggtaca gctcatcatg gatgcttata atagcctgtc 1320
   ttccaccgtg acccttgaac actcttcact ccctcctggg gtccacattt cttacgaatc 1380
   ccagtgtgag ggtcctgaga agagggaggg taaggctgag gatcgaggac agtgcaacca 1440
   cgtccgaatc aaccagacgg tgactttctg ggtttctctc caagccaccc actgcctccc 1500
   agagccccat ctcctgaggc tccgggccct tggcttctca gaggagctga ttgtggagtt 1560
   gcacacgctg tgtgactgta attgcagtga cacccagccc caggctcccc actgcagtga 1620
   tggccaggga cacctacaat gtggtgtatg cagctgtgcc cctggccgcc taggtcggct 1680
   ctgtgagtgc tctgtggcag agctgtcctc cccagacctg gaatctgggt gccggctccc 1740
   caatggcaca gggcccctgt gcagtggaaa gggtcactgt caatgtggac gctgcagctg 1800
   cagtggacag agctctgggc atctgtgcga gtgtgacgat gccagctgtg agcgacatga 1860
   gggcatcctc tgcggaggct ttggtcgctg ccaatgtgga gtatgtcact gtcatgccaa 1920
   ccgcacgggc agagcatgcg aatgcagtgg ggacatggac agttgcatca gtcccgaggg 1980
   agggctctgc agtgggcatg gacgctgcaa atgcaaccgc tgccagtgct tggacggcta 2040
   ctatggtgct ctatgcgacc aatgcccagg ctgcaagaca ccatgcgaga gacaccggga 2100
   ctgtgcagag tgtggggcct tcaggactgg cccactggcc accaactgca gtacagcttg 2160
   tgcccatacc aatgtgaccc tggccttggc ccctatcttg gatgatggct ggtgcaaaga 2220
   gcggaccctg gacaaccagc tgttcttctt cttggtggag gatgacgcca gaggcacggt 2280
   cgtgctcaga gtgagacccc aagaaaaggg agcagaccac acgcaggcca ttgtgctggg 2340
   ctgcgtaggg ggcatcgtgg cagtggggct ggggctggtc ctggcttacc ggctctcggt 2400
   ggaaatctat gaccgccggg aatacagtcg ctttgagaag gagcagcaac aactcaactg 2460
   gaagcaggac agtaatcctc tctacaaaag tgccatcacg accaccatca atcctcgctt 2520
   tcaagaggca gacagtccca ctctctgaag gagggaggga cacttaccca aggctcttct 2580
   ccttggagga cagtgggaac tggagggtga gaggaagggt gggtctgtaa gaccttggta 2640
   ggggactaat tcactggcga ggtgcggcca ccaccctact tcattttcag agtgacaccc 2700
   aagagggctg cttcccatgc ctgcaacctt gcatccatct gggctacccc acccaagtat 2760
   acaataaagt cttacctcag aaaaaaaaaa aaaaaaaa 2798

<210> 12
<211> 798
<212> PRT
<213> Homo sapiens

<400> 12
Met Val Ala Leu Pro Met Val Leu Val Leu Leu Leu Val Leu Ser Arg
1               5                   10                  15

Gly Glu Ser Glu Leu Asp Ala Lys Ile Pro Ser Thr Gly Asp Ala Thr
            20                  25                  30

Glu Trp Arg Asn Pro His Leu Ser Met Leu Gly Ser Cys Gln Pro Ala
        35                  40                  45

Pro Ser Cys Gln Lys Cys Ile Leu Ser His Pro Ser Cys Ala Trp Cys
    50                  55                  60

Lys Gln Leu Asn Phe Thr Ala Ser Gly Glu Ala Glu Ala Arg Arg Cys
65                  70                  75                  80

Ala Arg Arg Glu Glu Leu Leu Ala Arg Gly Cys Pro Leu Glu Glu Leu
                85                  90                  95

Glu Glu Pro Arg Gly Gln Gln Glu Val Leu Gln Asp Gln Pro Leu Ser
            100                 105                 110

Gln Gly Ala Arg Gly Glu Gly Ala Thr Gln Leu Ala Pro Gln Arg Val
        115                 120                 125

Arg Val Thr Leu Arg Pro Gly Glu Pro Gln Gln Leu Gln Val Arg Phe
    130                 135                 140

Leu Arg Ala Glu Gly Tyr Pro Val Asp Leu Tyr Tyr Leu Met Asp Leu
145                 150                 155                 160

Ser Tyr Ser Met Lys Asp Asp Leu Glu Arg Val Arg Gln Leu Gly His
                165                 170                 175

Ala Leu Leu Val Arg Leu Gln Glu Val Thr His Ser Val Arg Ile Gly
            180                 185                 190

Phe Gly Ser Phe Val Asp Lys Thr Val Leu Pro Phe Val Ser Thr Val
        195                 200                 205

Pro Ser Lys Leu Arg His Pro Cys Pro Thr Arg Leu Glu Arg Cys Gln
    210                 215                 220

Ser Pro Phe Ser Phe His His Val Leu Ser Leu Thr Gly Asp Ala Gln
225                 230                 235                 240

Ala Phe Glu Arg Glu Val Gly Arg Gln Ser Val Ser Gly Asn Leu Asp
                245                 250                 255

Ser Pro Glu Gly Gly Phe Asp Ala Ile Leu Gln Ala Ala Leu Cys Gln
            260                 265                 270

Glu Gln Ile Gly Trp Arg Asn Val Ser Arg Leu Leu Val Phe Thr Ser
        275                 280                 285

Asp Asp Thr Phe His Thr Ala Gly Asp Gly Lys Leu Gly Gly Ile Phe
    290                 295                 300

Met Pro Ser Asp Gly His Cys His Leu Asp Ser Asn Gly Leu Tyr Ser
305                 310                 315                 320

Arg Ser Thr Glu Phe Asp Tyr Pro Ser Val Gly Gln Val Ala Gln Ala
                325                 330                 335

Leu Ser Ala Ala Asn Ile Gln Pro Ile Phe Ala Val Thr Ser Ala Ala
            340                 345                 350

Leu Pro Val Tyr Gln Glu Leu Ser Lys Leu Ile Pro Lys Ser Ala Val
        355                 360                 365

Gly Glu Leu Ser Glu Asp Ser Ser Asn Val Val Gln Leu Ile Met Asp
    370                 375                 380

Ala Tyr Asn Ser Leu Ser Ser Thr Val Thr Leu Glu His Ser Ser Leu
385                 390                 395                 400

Pro Pro Gly Val His Ile Ser Tyr Glu Ser Gln Cys Glu Gly Pro Glu
                405                 410                 415

Lys Arg Glu Gly Lys Ala Glu Asp Arg Gly Gln Cys Asn His Val Arg
            420                 425                 430

Ile Asn Gln Thr Val Thr Phe Trp Val Ser Leu Gln Ala Thr His Cys
        435                 440                 445

Leu Pro Glu Pro His Leu Leu Arg Leu Arg Ala Leu Gly Phe Ser Glu
    450                 455                 460

Glu Leu Ile Val Glu Leu His Thr Leu Cys Asp Cys Asn Cys Ser Asp
465                 470                 475                 480

Thr Gln Pro Gln Ala Pro His Cys Ser Asp Gly Gln Gly His Leu Gln
                485                 490                 495

Cys Gly Val Cys Ser Cys Ala Pro Gly Arg Leu Gly Arg Leu Cys Glu
            500                 505                 510

Cys Ser Val Ala Glu Leu Ser Ser Pro Asp Leu Glu Ser Gly Cys Arg
        515                 520                 525

Ala Pro Asn Gly Thr Gly Pro Leu Cys Ser Gly Lys Gly His Cys Gln
    530                 535                 540

Cys Gly Arg Cys Ser Cys Ser Gly Gln Ser Ser Gly His Leu Cys Glu
545                 550                 555                 560

Cys Asp Asp Ala Ser Cys Glu Arg His Glu Gly Ile Leu Cys Gly Gly
                565                 570                 575

Phe Gly Arg Cys Gln Cys Gly Val Cys His Cys His Ala Asn Arg Thr
            580                 585                 590

Gly Arg Ala Cys Glu Cys Ser Gly Asp Met Asp Ser Cys Ile Ser Pro
        595                 600                 605

Glu Gly Gly Leu Cys Ser Gly His Gly Arg Cys Lys Cys Asn Arg Cys
    610                 615                 620

Gln Cys Leu Asp Gly Tyr Tyr Gly Ala Leu Cys Asp Gln Cys Pro Gly
625                 630                 635                 640

Cys Lys Thr Pro Cys Glu Arg His Arg Asp Cys Ala Glu Cys Gly Ala
                645                 650                 655

Phe Arg Thr Gly Pro Leu Ala Thr Asn Cys Ser Thr Ala Cys Ala His
            660                 665                 670

Thr Asn Val Thr Leu Ala Leu Ala Pro Ile Leu Asp Asp Gly Trp Cys
        675                 680                 685

Lys Glu Arg Thr Leu Asp Asn Gln Leu Phe Phe Phe Leu Val Glu Asp
    690                 695                 700

Asp Ala Arg Gly Thr Val Val Leu Arg Val Arg Pro Gln Glu Lys Gly
705                 710                 715                 720

Ala Asp His Thr Gln Ala Ile Val Leu Gly Cys Val Gly Gly Ile Val
                725                 730                 735

Ala Val Gly Leu Gly Leu Val Leu Ala Tyr Arg Leu Ser Val Glu Ile
            740                 745                 750

Tyr Asp Arg Arg Glu Tyr Ser Arg Phe Glu Lys Glu Gln Gln Gln Leu
        755                 760                 765

Asn Trp Lys Gln Asp Ser Asn Pro Leu Tyr Lys Ser Ala Ile Thr Thr
    770                 775                 780

Thr Ile Asn Pro Arg Phe Gln Glu Ala Asp Ser Pro Thr Leu
785                 790                 795


<210> 13
<211> 2211
<212> DNA
<213> Homo sapiens

<400> 13
gtggctttgc caatggtcct tgttttgctg ctggtcctga gcagaggtga gagtgaattg 60
gacgccaaga tcccatccac aggggatgcc acagaatggc ggaatcctca cctgtccatg 120
ctggggtcct gccagccagc cccctcctgc cagaagtgca tcctctcaca ccccagctgt 180
gcatggtgca agcaactgaa cttcaccgcg tcgggagagg cggaggcgcg gcgctgcgcc 240
cgacgagagg agctgctggc tcgaggctgc ccgctggagg agctggagga gccccgcggc 300
cagcaggagg tgctgcagga ccagccgctc agccagggcg cccgcggaga gggtgccacc 360
cagctggcgc cgcagcgggt ccgggtcacg ctgcggcctg gggagcccca gcagctccag 420

gtccgcttcc ttcgtgctga gggatacccg gtggacctgt actaccttat ggacctgagc 480
tactccatga aggacgacct ggaacgcgtg cgccagctcg ggcacgctct gctggtccgg 540
ctgcaggaag tcacccattc tgtgcgcatt ggttttggtt cctttgtgga caaaacggtg 600
ctgccctttg tgagcacagt accctccaaa ctgcgccacc cctgccccac ccggctggag 660
cgctgccagt caccattcag ctttcaccat gtgctgtccc tgacggggga cgcacaagcc 720
ttcgagcggg aggtggggcg ccagagtgtg tccggcaatc tggactcgcc tgaaggtggc 780
ttcgatgcca ttctgcaggc tgcactctgc caggagcaga ttggctggag aaatgtgtcc 840
cggctgctgg tgttcacttc agacgacaca ttccatacag ctggggacgg gaagttgggc 900
ggcatttcca tgcccagtga tgggcactgc cacttggaca gcaatggcct ctacagtcgc 960
agcacagagt ttgactaccc ttctgtgggt caggtagccc aggccctctc tgcagcaaat 1020
atccagccca tctttgctgt caccagtgcc gcactgcctg tctaccagga gctgagtaaa 1080
ctgattccta agtctgcagt tggggagctg agtgaggact ccagcaacgt ggtacagctc 1140
atcatggatg cttataatag cctgtcttcc accgtgaccc ttgaacactc ttcactccct 1200
cctggggtcc acatttctta cgaatcccag tgtgagggtc ctgagaagag ggagggtaag 1260
gctgaggatc gaggacagtg caaccacgtc cgaatcaacc agacggtgac tttctgggtt 1320
tctctccaag ccacccactg cctcccagag ccccatctcc tgaggctccg ggcccttggc 1380
ttctcagagg agctgattgt ggagttgcac acgctgtgtg actgtaattg cagtgacacc 1440
cagccccagg ctccccactg cagtgatggc cagggacacc tacaatgtgg tgtatgcagc 1500
tgtgcccctg gccgcctagg tcggctctgt gagtgctctg tggcagagct gtcctcccca 1560
gacctggaat ctgggtgccg ggctcccaat ggcacagggc ccctgtgcag tggaaagggt 1620
cactgtcaat gtggacgctg cagctgcagt ggacagagct ctgggcatct gtgcgagtgt 1680
gacgatgcca gctgtgagcg acatgagggc atcctctgcg gagggactgt gcagagtgtg 1740
ggccttcagg gactggccca ctggccacca actgcagtac agcttgtgcc cataccaatg 1800
tgaccctggc cttggcccct atcttggatg atggctggtg caaagagcgg accctggaca 1860
accagctgtt cttcttcttg gtggaggatg acgccagagg cacggtcgtg ctcagagtga 1920
gaccccaaga aaagggagca gaccacacgc aggccattgt gctgggctgc gtagggggca 1980
tcgtggcagt ggggctgggg ctggtcctgg cttaccggct ctcggtggaa atctatgacc 2040
gccgggaata cagtcgcttt gagaaggagc agcaacaact caactggaag caggtgagga 2100
gacttcctgg ttaggcccct ttttagctgt tcccccacca caagaccagc cctgattcct 2160
cccactgggt tcccccagcc cctagcacat gtaaccaacc cctctgctaa c 2211

<210> 14
<211> 596
<212> PRT
<213> Homo sapiens

<400> 14
Met Val Leu Val Leu Leu Leu Val Leu Ser Arg Gly Glu Ser Glu Leu
1               5                   10                  15

Asp Ala Lys Ile Pro Ser Thr Gly Asp Ala Thr Glu Trp Arg Asn Pro
            20                  25                  30

His Leu Ser Met Leu Gly Ser Cys Gln Pro Ala Pro Ser Cys Gln Lys
        35                  40                  45

Cys Ile Leu Ser His Pro Ser Cys Ala Trp Cys Lys Gln Leu Asn Phe
    50                  55                  60

Thr Ala Ser Gly Glu Ala Glu Ala Arg Arg Cys Ala Arg Arg Glu Glu
65                  70                  75                  80

Leu Leu Ala Arg Gly Cys Pro Leu Glu Glu Leu Glu Glu Pro Arg Gly
                85                  90                  95

Gln Gln Glu Val Leu Gln Asp Gln Pro Leu Ser Gln Gly Ala Arg Gly
            100                 105                 110

Glu Gly Ala Thr Gln Leu Ala Pro Gln Arg Val Arg Val Thr Leu Arg
        115                 120                 125

Pro Gly Glu Pro Gln Gln Leu Gln Val Arg Phe Leu Arg Ala Glu Gly
    130                 135                 140

Tyr Pro Val Asp Leu Tyr Tyr Leu Met Asp Leu Ser Tyr Ser Met Lys
145                 150                 155                 160

Asp Asp Leu Glu Arg Val Arg Gln Leu Gly His Ala Leu Leu Val Arg

                165                 170                 175

Leu Gln Glu Val Thr His Ser Val Arg Ile Gly Phe Gly Ser Phe Val
            180                 185                 190

Asp Lys Thr Val Leu Pro Phe Val Ser Thr Val Pro Ser Lys Leu Arg
        195                 200                 205

His Pro Cys Pro Thr Arg Leu Glu Arg Cys Gln Ser Pro Phe Ser Phe
    210                 215                 220

His His Val Leu Ser Leu Thr Gly Asp Ala Gln Ala Phe Glu Arg Glu
225                 230                 235                 240

Val Gly Arg Gln Ser Val Ser Gly Asn Leu Asp Ser Pro Glu Gly Gly
                245                 250                 255

Phe Asp Ala Ile Leu Gln Ala Ala Leu Cys Gln Glu Gln Ile Gly Trp
            260                 265                 270

Arg Asn Val Ser Arg Leu Leu Val Phe Thr Ser Asp Asp Thr Phe His
        275                 280                 285

Thr Ala Gly Asp Gly Lys Leu Gly Gly Ile Phe Met Pro Ser Asp Gly
    290                 295                 300

His Cys His Leu Asp Ser Asn Gly Leu Tyr Ser Arg Ser Thr Glu Phe
305                 310                 315                 320

Asp Tyr Pro Ser Val Gly Gln Val Ala Gln Ala Leu Ser Ala Ala Asn
                325                 330                 335

Ile Gln Pro Ile Phe Ala Val Thr Ser Ala Ala Leu Pro Val Tyr Gln
            340                 345                 350

Glu Leu Ser Lys Leu Ile Pro Lys Ser Ala Val Gly Glu Leu Ser Glu
        355                 360                 365

Asp Ser Ser Asn Val Val Gln Leu Ile Met Asp Ala Tyr Asn Ser Leu
    370                 375                 380

Ser Ser Thr Val Thr Leu Glu His Ser Ser Leu Pro Pro Gly Val His
385                 390                 395                 400

Ile Ser Tyr Glu Ser Gln Cys Glu Gly Pro Glu Lys Arg Glu Gly Lys
                405                 410                 415

Ala Glu Asp Arg Gly Gln Cys Asn His Val Arg Ile Asn Gln Thr Val
            420                 425                 430

Thr Phe Trp Val Ser Leu Gln Ala Thr His Cys Leu Pro Glu Pro His
        435                 440                 445

Leu Leu Arg Leu Arg Ala Leu Gly Phe Ser Glu Glu Leu Ile Val Glu
    450                 455                 460

Leu His Thr Leu Cys Asp Cys Asn Cys Ser Asp Thr Gln Pro Gln Ala
465                 470                 475                 480

Pro His Cys Ser Asp Gly Gln Gly His Leu Gln Cys Gly Val Cys Ser
                485                 490                 495

Cys Ala Pro Gly Arg Leu Gly Arg Leu Cys Glu Cys Ser Val Ala Glu
            500                 505                 510

Leu Ser Ser Pro Asp Leu Glu Ser Gly Cys Arg Ala Pro Asn Gly Thr
        515                 520                 525

Gly Pro Leu Cys Ser Gly Lys Gly His Cys Gln Cys Gly Arg Cys Ser

530                 535                 540

Cys Ser Gly Gln Ser Ser Gly His Leu Cys Glu Cys Asp Asp Ala Ser
545                 550                 555                 560

Cys Glu Arg His Glu Gly Ile Leu Cys Gly Gly Thr Val Gln Ser Val
                565                 570                 575

Gly Pro Ser Gly Leu Ala His Trp Pro Pro Thr Ala Val Gln Leu Val
            580                 585                 590

Pro Ile Pro Met
        595

<210> 15
<211> 1360
<212> DNA
<213> Homo sapiens

<400> 15
gtcgaatatc catgcagagt acctggaata catcagggat ccttgctctc atgaggtcag 60
agatgaggaa ttccatagga tgagtcttct gttcttaaac cgagtgtaag gataaggctg 120
tcggagagga gggagtagga tttgggcatc agagcccccc agctgctgct cgtgggggtg 180
aggacaccag gcccctgact ttgctctggt ctctctccca gctccctgcc caggcccaca 240
gccatggcca tggcccagaa actcagccac ctcctgccga gtctgcggca ggtcatccag 300
gagcctcagc tatctctgca gccagagcct gtcttcacgg tggatcgagc tgaggtgccg 360
ccgctcttct ggaagccgta catctatgcg ggctaccggc cgctgcatca gacctggcgc 420
ttctatttcc gcacgctgtt ccagcagcac aacgaggccg tgaatgtctg gacccacctg 480
ctggcggccc tggcactgct gctgcggctg gccctctttg tggagaccgt ggacttctgg 540
ggagacccac acgccctgcc cctcttcatc attgtccttg cctctttcac ctacctctcc 600
ttcagtgcct tggctcacct cctgcaggcc aagtctgagt tctggcatta cagcttcttc 660
ttcctggact atgtgggggt ggccgtgtac cagtttggca gtgccttggc acacttctac 720
tatgctatcg agcccgcctg gcatgcccag gtgcaggctg tttttctgcc catggctgcc 780
tttctcgcct ggctttcctg cattggctcc tgctataaca agtacatcca gaaaccaggc 840
ctgctgggcc gcacatgcca ggaggtgccc tccgtcctgg cctacgcact ggacattagt 900
cctgtggtgc atcgtatctt cgtgtcctcc gaccccacca cggatgatcc agctcttctc 960
taccacaagt gccaggtggt cttctttctg ctggctgctg ccttcttctc taccttcatg 1020
cccgagcgct ggttccctgg cagctgccat gtcttcgggc agggccacca acttttccac 1080
atcttcttgg tgctgtgcac gctggctcag ctggaggctg tggcactgga ctatgaggcc 1140
cgacggccca tctatgagcc tctgcacacg cactggcctc acaacttttc tggcctcttc 1200
ctgctcacgg tgggcagcag catcctcact gcattcctcc tgagccagct ggtacagcgc 1260
aaacttgatc agaagaccaa gtgaaggggg atggcatctg gtagggaggg aggtatagtt 1320
gggggacagg ggtctgggtt tggctccagg tgggaacaag                       1360

<210> 16
<211> 346
<212> PRT
<213> Homo sapiens

<400> 16
Met Ala Met Ala Gln Lys Leu Ser His Leu Leu Pro Ser Leu Arg Gln
1               5                   10                  15

Val Ile Gln Glu Pro Gln Leu Ser Leu Gln Pro Glu Pro Val Phe Thr
            20                  25                  30

Val Asp Arg Ala Glu Val Pro Pro Leu Phe Trp Lys Pro Tyr Ile Tyr
        35                  40                  45

Ala Gly Tyr Arg Pro Leu His Gln Thr Trp Arg Phe Tyr Phe Arg Thr
    50                  55                  60

Leu Phe Gln Gln His Asn Glu Ala Val Asn Val Trp Thr His Leu Leu
65                  70                  75                  80

Ala Ala Leu Ala Leu Leu Leu Arg Leu Ala Leu Phe Val Glu Thr Val
                85                  90                  95

Asp Phe Trp Gly Asp Pro His Ala Leu Pro Leu Phe Ile Ile Val Leu
            100                 105                 110

Ala Ser Phe Thr Tyr Leu Ser Phe Ser Ala Leu Ala His Leu Leu Gln
        115                 120                 125

Ala Lys Ser Glu Phe Trp His Tyr Ser Phe Phe Phe Leu Asp Tyr Val
    130                 135                 140

Gly Val Ala Val Tyr Gln Phe Gly Ser Ala Leu Ala His Phe Tyr Tyr
145                 150                 155                 160

Ala Ile Glu Pro Ala Trp His Ala Gln Val Gln Ala Val Phe Leu Pro
                165                 170                 175

Met Ala Ala Phe Leu Ala Trp Leu Ser Cys Ile Gly Ser Cys Tyr Asn
            180                 185                 190

Lys Tyr Ile Gln Lys Pro Gly Leu Leu Gly Arg Thr Cys Gln Glu Val
        195                 200                 205

Pro Ser Val Leu Ala Tyr Ala Leu Asp Ile Ser Pro Val Val His Arg
    210                 215                 220

Ile Phe Val Ser Ser Asp Pro Thr Thr Asp Asp Pro Ala Leu Leu Tyr
225                 230                 235                 240

His Lys Cys Gln Val Val Phe Phe Leu Leu Ala Ala Ala Phe Phe Ser
                245                 250                 255

Thr Phe Met Pro Glu Arg Trp Phe Pro Gly Ser Cys His Val Phe Gly
            260                 265                 270

Gln Gly His Gln Leu Phe His Ile Phe Leu Val Leu Cys Thr Leu Ala
        275                 280                 285

Gln Leu Glu Ala Val Ala Leu Asp Tyr Glu Ala Arg Arg Pro Ile Tyr
    290                 295                 300

Glu Pro Leu His Thr His Trp Pro His Asn Phe Ser Gly Leu Phe Leu
305                 310                 315                 320

Leu Thr Val Gly Ser Ser Ile Leu Thr Ala Phe Leu Leu Ser Gln Leu
                325                 330                 335

Val Gln Arg Lys Leu Asp Gln Lys Thr Lys
            340                 345

<210> 17
<211> 3509
<212> DNA
<213> Homo sapiens

<400> 17
ggtccgagcg gggctctgtg acgacagccc caccagctgc ttcggggtgg gcagccttca 60
ggaggaaggg ctggcctggg aggagctggc tgcccctcgg gacgtgttct caggccctgc 120
ccgctgccct gccccatata ccttctcctt cgagatgctg gtgactgggc catgcctgct 180
tgcaggcctg gagagcccct ctcatgctct gcgggcagat gccccccctc atgccagctc 240
tgcagccacc atctgtgtca cactggcaga gggccaccac tgtgaccggg ccttggagat 300
cctgctgcac cccagtgagc cccatcagcc acacctgatg ctggagggcg gcagcctgag 360
ctcagcagaa tatgaggccc gggtgagggc ccgccgagat tttcagaggc tacagcgagg 420
ggacagtgat ggggaccggc aggtgtggtt cctgcagcga cgcttccaca aggacatcct 480
gctgaacccc gtgctggcgc tgagcttctg cccagacctg agctccaagc ccggacacct 540
ggggacagct actcgggagc tactcttcct tttggatagc agcagcgtgg cacacaagga 600
tgccattgtt ttggctgtga agtccctccc accccagacg cttatcaacc tggccgtgtt 660
tgggacgttg gtgcagccac tcttcccaga gagccggcct tgcagtgatg atgctgtgca 720
gctgatctgc gagagcattg agaccctgca ggttccgagt gggcccccag acgtgctggc 780

tgctctggac tgggccgtgg ggcagcccca gcacagggcc taccctcggc agctgttcct 840
gctcactgct gcctcaccca tggccgccac tacccaccga accctggagc tcatgaggtg 900
gcacaggggg acagccaggt gcttctcctt tgggctgggg cccacctgcc accagctgct 960
ccagggttta tctgccctca gcagccgagg tcgcgccact gcactccagc ctgggcgaca 1020
gagcaagcca cagctggtac aggctctgcg gaaggcactg gagcctgctt tgagtgacat 1080
ctctgtggac tggtttgtgc ccgacactgt ggaggcactg ctgacccctc gggagatccc 1140
agcactctac cctggggacc agctgctcgg ttactgctca ctcttcaggg tggatggctt 1200
ccggtcccgc ccaccagggg gccaagagcc tggctggcag agctcgggtg ggtccgtgtt 1260
tccatcccca gaagaggccc cgtctgctgc cagccctggc actgagccca ctggcacctc 1320
agagccactg ggaacaggca ctgtctcagc agaactgtcc agcccatggg ctgccaggga 1380
ctcggagcag agtggtactg atgctctgac agacccagtc acggatcctg gacccaaccc 1440
ctctgacaca gccatatggc gccgcatctt tcagtcctcg tacattcggg agcagtatgt 1500
gctcacccac tgctctgcca gccccgagcc aggcccaggc tccacaggca gcagtgagtc 1560
cccaggctca cagggccctg gctcccccga aggtagtgct cccttggagc cccccttctca 1620
gcagggctgc cgcagtctgg cctggggaga acctgcaggc tcccgctcct gtcccctgcc 1680
tgcacccaca ccagctccat tcaaggtggg ggccttgagt actgaggtgc tgggccgtca 1740
gcacagagcg gctctggctg gccgaagcct ctcatcccct ccaggccggg caaaccaagt 1800
ccccggccga ccccggaaac cctctttggg tgcaatacta gatggcccaa gtcctgagcc 1860
aggccaacag ttgggacaag gcctggatga ctcaggtaac ctgctctccc cagcccctat 1920
ggactgggac atgctgatgg aaccaccctt cttattcacg gctgtgcctc ctagtgggga 1980
gttggcccct ccagcagtgc ctccccaggc tccacgctgc catgtggtga tccgggggcct 2040
gtgtggggag cagcccatgt gctgggaggt gggtgttggg ctggagacac tgtgggacc 2100
tggagatggc tcacagcctc cctcacctcc tgtaagagaa gctgcttggg accaagcact 2160
ccatcggctg acagcagcct ctgtggtccg ggacaatgag cagctggccc tccgaggagg 2220
ggcagagacc acagctgacc ggggccatgc ccggaggtgc tggcttcgag cccttcaaac 2280
aagtaaggtc agctctgccc cctcctgctt cacttgccct gtagctgtgg atgctactac 2340
tagggaggtc ctgcctgggg ccctgcaggt gtgcagctca gagcccgctg agcccccagg 2400
aacccctcct gcctctcaca gccatctaga tgcagctcct ctgcccactg ttgtctactc 2460
taaaggtgcc tgggactcgg accaaaatgg caactccaag cgtgctttgg gggaccctgc 2520
cactcccacg gaaggtcctc gccgcccacc tccccgtcct ccctgtcggc tcagcatggg 2580
ccgccgtcac aaactctgta gccctgaccc gggccaggcc aacaacagtg aaggcagcga 2640
ccatgactac ctgcccttgg tgcggctgca ggaggcacca ggctccttcc gcctggacgc 2700
gcccttctgc gccgctgtgc gcatctcgca ggagcgcctc tgccgtgcct cgccctttgc 2760
cgtgcaccgc gccagcctca gccccacctc ggcctcattg ccctgggcac ttctgggccc 2820
tggtgttggc cagggtgaca gtgccacggc ctcctgcagc ccgtccccca gctcgggctc 2880
tgaggggcca ggccaggtgg acagtgggcg gggctcagac accgaggcct ccgagggggc 2940
ggaagggctg ggcggcaccg acctgcgggg ccggacctgg gccactgccg tagcactcgc 3000
ctggctggag caccgatgcg ccgctgcctt cgacgagtgg gaactgacag cggccaaggc 3060
tgattgctgg ctgcgggccc agcacttgcc tgacggcctt gacctggccg ccctcaaggc 3120
cgcagcccga gggctcttcc tgctactgcg ccactgggac caaaacctgc agctacacct 3180
gctgtgctac agcccagcga acgtgtgaag gctgcccct gctgcttggg ctggcgcccc 3240
acccaacaca ctcaagtcac tgccgcccag ggctggcctc ttggtgctgg gaaagtgtag 3300
gctggttcca gcctgtcccc cactgcttct tactccctcc ctagagccct cttgccccca 3360
caaaaagtgc ctgcctgtgc tctctccctc tcctcccacc ccactcacac tcccctccat 3420
cctttgagct ccctgcaaca cagtggaagg gtagagagcc acagtcccca aatcctatgc 3480
aataaagtgc ctcttaggga aaaaaaaa 3509

<210> 18
<211> 1017
<212> PRT
<213> Homo sapiens

<400> 18
Met Leu Val Thr Gly Pro Cys Leu Leu Ala Gly Leu Glu Ser Pro Ser
1               5                   10                  15

His Ala Leu Arg Ala Asp Ala Pro Pro His Ala Ser Ser Ala Ala Thr
            20                  25                  30

Ile Cys Val Thr Leu Ala Glu Gly His His Cys Asp Arg Ala Leu Glu
        35                  40                  45

Ile Leu Leu His Pro Ser Glu Pro His Gln Pro His Leu Met Leu Glu
    50                  55                  60

Gly Gly Ser Leu Ser Ser Ala Glu Tyr Glu Ala Arg Val Arg Ala Arg
65                  70                  75                  80

Arg Asp Phe Gln Arg Leu Gln Arg Gly Asp Ser Asp Gly Asp Arg Gln
                85                  90                  95

Val Trp Phe Leu Gln Arg Arg Phe His Lys Asp Ile Leu Leu Asn Pro
            100                 105                 110

Val Leu Ala Leu Ser Phe Cys Pro Asp Leu Ser Ser Lys Pro Gly His
        115                 120                 125

Leu Gly Thr Ala Thr Arg Glu Leu Leu Phe Leu Leu Asp Ser Ser Ser
    130                 135                 140

Val Ala His Lys Asp Ala Ile Val Leu Ala Val Lys Ser Leu Pro Pro
145                 150                 155                 160

Gln Thr Leu Ile Asn Leu Ala Val Phe Gly Thr Leu Val Gln Pro Leu
                165                 170                 175

Phe Pro Glu Ser Arg Pro Cys Ser Asp Asp Ala Val Gln Leu Ile Cys
            180                 185                 190

Glu Ser Ile Glu Thr Leu Gln Val Pro Ser Gly Pro Pro Asp Val Leu
        195                 200                 205

Ala Ala Leu Asp Trp Ala Val Gly Gln Pro Gln His Arg Ala Tyr Pro
    210                 215                 220

Arg Gln Leu Phe Leu Leu Thr Ala Ala Ser Pro Met Ala Ala Thr Thr
225                 230                 235                 240

His Arg Thr Leu Glu Leu Met Arg Trp His Arg Gly Thr Ala Arg Cys
                245                 250                 255

Phe Ser Phe Gly Leu Gly Pro Thr Cys His Gln Leu Leu Gln Gly Leu
            260                 265                 270

Ser Ala Leu Ser Ser Arg Gly Arg Ala Thr Ala Leu Gln Pro Gly Arg
        275                 280                 285

Gln Ser Lys Pro Gln Leu Val Gln Ala Leu Arg Lys Ala Leu Glu Pro
    290                 295                 300

Ala Leu Ser Asp Ile Ser Val Asp Trp Phe Val Pro Asp Thr Val Glu
305                 310                 315                 320

Ala Leu Leu Thr Pro Arg Glu Ile Pro Ala Leu Tyr Pro Gly Asp Gln
                325                 330                 335

Leu Leu Gly Tyr Cys Ser Leu Phe Arg Val Asp Gly Phe Arg Ser Arg
            340                 345                 350

Pro Pro Gly Gly Gln Glu Pro Gly Trp Gln Ser Ser Gly Gly Ser Val
        355                 360                 365

Phe Pro Ser Pro Glu Glu Ala Pro Ser Ala Ala Ser Pro Gly Thr Glu
    370                 375                 380

Pro Thr Gly Thr Ser Glu Pro Leu Gly Thr Gly Thr Val Ser Ala Glu
385                 390                 395                 400

Leu Ser Ser Pro Trp Ala Ala Arg Asp Ser Glu Gln Ser Gly Thr Asp
                405                 410                 415

Ala Leu Thr Asp Pro Val Thr Asp Pro Gly Pro Asn Pro Ser Asp Thr
            420                 425                 430

Ala Ile Trp Arg Arg Ile Phe Gln Ser Ser Tyr Ile Arg Glu Gln Tyr
        435                 440                 445

Val Leu Thr His Cys Ser Ala Ser Pro Glu Pro Gly Pro Gly Ser Thr
    450                 455                 460

Gly Ser Ser Glu Ser Pro Gly Ser Gln Gly Pro Gly Ser Pro Glu Gly
465                 470                 475                 480

Ser Ala Pro Leu Glu Pro Pro Ser Gln Gln Gly Cys Arg Ser Leu Ala
                485                 490                 495

Trp Gly Glu Pro Ala Gly Ser Arg Ser Cys Pro Leu Pro Ala Pro Thr
            500                 505                 510

Pro Ala Pro Phe Lys Val Gly Ala Leu Ser Thr Glu Val Leu Gly Arg
        515                 520                 525

Gln His Arg Ala Ala Leu Ala Gly Arg Ser Leu Ser Ser Pro Pro Gly
    530                 535                 540

Arg Ala Asn Gln Val Pro Gly Arg Pro Arg Lys Pro Ser Leu Gly Ala
545                 550                 555                 560

Ile Leu Asp Gly Pro Ser Pro Glu Pro Gly Gln Gln Leu Gly Gln Gly
                565                 570                 575

Leu Asp Asp Ser Gly Asn Leu Leu Ser Pro Ala Pro Met Asp Trp Asp
            580                 585                 590

Met Leu Met Glu Pro Pro Phe Leu Phe Thr Ala Val Pro Pro Ser Gly
        595                 600                 605

Glu Leu Ala Pro Pro Ala Val Pro Pro Gln Ala Pro Arg Cys His Val
    610                 615                 620

Val Ile Arg Gly Leu Cys Gly Glu Gln Pro Met Cys Trp Glu Val Gly
625                 630                 635                 640

Val Gly Leu Glu Thr Leu Trp Gly Pro Gly Asp Gly Ser Gln Pro Pro
                645                 650                 655

Ser Pro Pro Val Arg Glu Ala Ala Trp Asp Gln Ala Leu His Arg Leu
            660                 665                 670

Thr Ala Ala Ser Val Val Arg Asp Asn Glu Gln Leu Ala Leu Arg Gly
        675                 680                 685

Gly Ala Glu Thr Thr Ala Asp Arg Gly His Ala Arg Arg Cys Trp Leu
    690                 695                 700

Arg Ala Leu Gln Thr Ser Lys Val Ser Ser Ala Pro Ser Cys Phe Thr
705                 710                 715                 720

Cys Pro Val Ala Val Asp Ala Thr Thr Arg Glu Val Leu Pro Gly Ala
                725                 730                 735

Leu Gln Val Cys Ser Ser Glu Pro Ala Glu Pro Pro Gly Thr Pro Pro
            740                 745                 750

Ala Ser His Ser His Leu Asp Ala Ala Pro Leu Pro Thr Val Val Tyr
        755                 760                 765

Ser Lys Gly Ala Trp Asp Ser Asp Gln Asn Gly Asn Ser Lys Arg Ala
    770                 775                 780

Leu Gly Asp Pro Ala Thr Pro Thr Glu Gly Pro Arg Arg Pro Pro Pro
785                 790                 795                 800

Arg Pro Pro Cys Arg Leu Ser Met Gly Arg Arg His Lys Leu Cys Ser
                805                 810                 815

Pro Asp Pro Gly Gln Ala Asn Asn Ser Glu Gly Ser Asp His Asp Tyr
            820                 825                 830

Leu Pro Leu Val Arg Leu Gln Glu Ala Pro Gly Ser Phe Arg Leu Asp
        835                 840                 845

Ala Pro Phe Cys Ala Ala Val Arg Ile Ser Gln Glu Arg Leu Cys Arg
    850                 855                 860

Ala Ser Pro Phe Ala Val His Arg Ala Ser Leu Ser Pro Thr Ser Ala
865                 870                 875                 880

Ser Leu Pro Trp Ala Leu Leu Gly Pro Gly Val Gly Gln Gly Asp Ser
                885                 890                 895

Ala Thr Ala Ser Cys Ser Pro Ser Pro Ser Ser Gly Ser Glu Gly Pro
            900                 905                 910

Gly Gln Val Asp Ser Gly Arg Gly Ser Asp Thr Glu Ala Ser Glu Gly
        915                 920                 925

Ala Glu Gly Leu Gly Gly Thr Asp Leu Arg Gly Arg Thr Trp Ala Thr
    930                 935                 940

Ala Val Ala Leu Ala Trp Leu Glu His Arg Cys Ala Ala Ala Phe Asp
945                 950                 955                 960

Glu Trp Glu Leu Thr Ala Ala Lys Ala Asp Cys Trp Leu Arg Ala Gln
                965                 970                 975

His Leu Pro Asp Gly Leu Asp Leu Ala Ala Leu Lys Ala Ala Ala Arg
            980                 985                 990

Gly Leu Phe Leu Leu Leu Arg His Trp Asp Gln Asn Leu Gln Leu His
        995                 1000                1005

Leu Leu Cys Tyr Ser Pro Ala Asn Val
    1010                1015

<210> 19
<211> 1513
<212> DNA
<213> Homo sapiens

<400> 19
tatggagcta aaggctccag ctgcaggagg tcttaatgct ggccctgtcc ccccagctgc 60
catgtccacg cagagacttc ggaatgaaga ctaccacgac tacagctcca cggacgtgag 120
ccctgaggag agcccgtcgg aaggcctcaa caacctctcc tccccgggct cctaccagcg 180
ctttggtcaa agcaatagca caacgtggtt ccagaccttg atccacctgt taaaaggcaa 240
cattggcaca ggactcctgg gactccctct ggcggtgaaa aatgcaggca tcgtgatggg 300
tcccatcagc ctgctgatca taggcatcgt ggccgtgcac tgcatgggta tcctggtgaa 360
atgtgctcac cacttctgcc gcaggctgaa taaatccttt gtggattatg gtgatactgt 420
gatgtatgga ctagaatcca gcccctgctc ctggctccgg aaccacgcac actggggaag 480
acgtgttgtg gacttcttcc tgattgtcac ccagctggga ttctgctgtg tctattttgt 540
gtttctggct gacaacttta aacaggtgat agaagcggcc aatgggacca ccaataactg 600
ccacaacaat gagacggtga ttctgacgcc taccatggac tcgcgactct acatgctctc 660
cttcctgccc ttcctggtgc tgctggtttt catcaggaac ctccgagccc tgtccatctt 720
ctccctgttg gccaacatca ccatgctggt cagcttggtc atgatctacc agttcattgt 780
tttcaggtac atgctttctg tctttcagag gatcccagac cccagccacc tccccttggt 840
ggccccttgg aagacctacc ctctcttctt tggcacagcg atttttcat ttgaaggcat 900
tggaatggta ctgcccctgg aaaacaaaat gaaggatcct cggaagttcc cactcatcct 960
gtacctgggc atggtcatcg tcaccatcct ctacatcagc ctggggtgtc tggggtacct 1020
gcaatttgga gctaatatcc aaggcagcat aaccctcaac ctgcccttgt accagtcagt 1080
taagctgctg tactccatcg ggatcttttt cacctacgca ctccagttct acgtcccggc 1140
tgagatcatc atccccttct ttgtgtcccg agcgcccgag cactgtgagt tagtggtgga 1200
cctgtttgtg cgcacagtgc tggtctgcct gacaatcttg gccatcctca tcccccgcct 1260
ggacctggtc atctccctgg tgggctccgt gagcagcagc gccctggccc tcatcatccc 1320
accgctcctg gaggtcacca ccttctactc agagggcatg agcccctca ccatctttaa 1380

ggacgccctg atcagcatcc tgggcttcgt gggctttgtg gtggggacct atgaggctct 1440
ctatgagctg atccagccaa gcaatgctcc catcttcatc aattccacct gtgccttcat 1500
atagggatct ggg 1513

<210> 20
<211> 500
<212> PRT
<213> Homo sapiens

<400> 20
Met Glu Leu Lys Ala Pro Ala Ala Gly Gly Leu Asn Ala Gly Pro Val
1               5                   10                  15

Pro Pro Ala Ala Met Ser Thr Gln Arg Leu Arg Asn Glu Asp Tyr His
            20                  25                  30

Asp Tyr Ser Ser Thr Asp Val Ser Pro Glu Glu Ser Pro Ser Glu Gly
        35                  40                  45

Leu Asn Asn Leu Ser Ser Pro Gly Ser Tyr Gln Arg Phe Gly Gln Ser
    50                  55                  60

Asn Ser Thr Thr Trp Phe Gln Thr Leu Ile His Leu Leu Lys Gly Asn
65                  70                  75                  80

Ile Gly Thr Gly Leu Leu Gly Leu Pro Leu Ala Val Lys Asn Ala Gly
                85                  90                  95

Ile Val Met Gly Pro Ile Ser Leu Leu Ile Ile Gly Ile Val Ala Val
            100                 105                 110

His Cys Met Gly Ile Leu Val Lys Cys Ala His His Phe Cys Arg Arg
        115                 120                 125

Leu Asn Lys Ser Phe Val Asp Tyr Gly Asp Thr Val Met Tyr Gly Leu
    130                 135                 140

Glu Ser Ser Pro Cys Ser Trp Leu Arg Asn His Ala His Trp Gly Arg
145                 150                 155                 160

Arg Val Val Asp Phe Phe Leu Ile Val Thr Gln Leu Gly Phe Cys Cys
                165                 170                 175

Val Tyr Phe Val Phe Leu Ala Asp Asn Phe Lys Gln Val Ile Glu Ala
            180                 185                 190

Ala Asn Gly Thr Thr Asn Asn Cys His Asn Asn Glu Thr Val Ile Leu
        195                 200                 205

Thr Pro Thr Met Asp Ser Arg Leu Tyr Met Leu Ser Phe Leu Pro Phe
    210                 215                 220

Leu Val Leu Leu Val Phe Ile Arg Asn Leu Arg Ala Leu Ser Ile Phe
225                 230                 235                 240

Ser Leu Leu Ala Asn Ile Thr Met Leu Val Ser Leu Val Met Ile Tyr
                245                 250                 255

Gln Phe Ile Val Phe Arg Tyr Met Leu Ser Val Phe Gln Arg Ile Pro
            260                 265                 270

Asp Pro Ser His Leu Pro Leu Val Ala Pro Trp Lys Thr Tyr Pro Leu
        275                 280                 285

Phe Phe Gly Thr Ala Ile Phe Ser Phe Glu Gly Ile Gly Met Val Leu
    290                 295                 300

Pro Leu Glu Asn Lys Met Lys Asp Pro Arg Lys Phe Pro Leu Ile Leu

305                 310                 315                 320

Tyr Leu Gly Met Val Ile Val Thr Ile Leu Tyr Ile Ser Leu Gly Cys
                325                 330                 335

Leu Gly Tyr Leu Gln Phe Gly Ala Asn Ile Gln Gly Ser Ile Thr Leu
            340                 345                 350

Asn Leu Pro Leu Tyr Gln Ser Val Lys Leu Leu Tyr Ser Ile Gly Ile
        355                 360                 365

Phe Phe Thr Tyr Ala Leu Gln Phe Tyr Val Pro Ala Glu Ile Ile Ile
    370                 375                 380

Pro Phe Phe Val Ser Arg Ala Pro Glu His Cys Glu Leu Val Val Asp
385                 390                 395                 400

Leu Phe Val Arg Thr Val Leu Val Cys Leu Thr Ile Leu Ala Ile Leu
                405                 410                 415

Ile Pro Arg Leu Asp Leu Val Ile Ser Leu Val Gly Ser Val Ser Ser
            420                 425                 430

Ser Ala Leu Ala Leu Ile Ile Pro Pro Leu Leu Glu Val Thr Thr Phe
        435                 440                 445

Tyr Ser Glu Gly Met Ser Pro Leu Thr Ile Phe Lys Asp Ala Leu Ile
    450                 455                 460

Ser Ile Leu Gly Phe Val Gly Phe Val Val Gly Thr Tyr Glu Ala Leu
465                 470                 475                 480

Tyr Glu Leu Ile Gln Pro Ser Asn Ala Pro Ile Phe Ile Asn Ser Thr
                485                 490                 495

Cys Ala Phe Ile
                500


<210> 21
<211> 1501
<212> DNA
<213> Homo sapiens

<400> 21
tatggagcta aaggctccag ctgcaggagg tcttaatgct ggccctgtcc ccccagctgc 60
catgtccacg cagagacttc ggaatgaaga ctaccacgac tacagctcca cggacgtgag 120
ccctgaggag agcccgtcgg aaggcctcaa caacctctcc tccccgggct cctaccagcg 180
ctttggtcaa agcaatagca caacatggtt ccagaccttg atccacctgt taaaaggcaa 240
cattggcaca ggactgctgg gactccctct ggcggtgaaa aatgcaggca tcgtgatggg 300
tcccatcagc ctgctgatca taggcatcgt ggccgtgcac tgcatgggta tcctggtgaa 360
atgtgctcac cacttctgcc gcaggctgaa taaatccttt gtggattatg gtgatactgt 420
gatgtatgga ctagaatcca gcccctgctc ctggctccgg aaccacgcac actggggaag 480
acgtgttgtg gacttcttcc tgattgtcac ccagctggta ttctgctgtg tctattttgt 540
gtttctggct gacaacttta aacaggtgat agaagcggcc aatggaacca ccaataactg 600
ccacaacaat gagacggtga ttctgacgcc taccatggac tcgcgactct acatgctctc 660
cttcctgccc ttcctggtgc tgctggtttt catcaggaac ctccgagccc tgtccatctt 720
ctccctgttg gccaacatca ccatgctggt cagcttggtc atgatctacc agttcattgt 780
tcagaggatc ccagacccca gccacctccc cttggtggcc ccttggaaga cctaccctct 840
cttctttggc acagcgattt tttcatttga aggcattgga atggttctgc ccctggaaaa 900
caaaatgaag gatcctcgga agttcccact catcctgtac ctgggcatgg tcatcgtcac 960
catcctctac atcagcctgg ggtgtctggg gtacctgcaa tttggagcta atatccaagg 1020
cagcataacc ctcaacctgc ccaactgctg gttgtaccag tcagttaagc tgctgtactc 1080
catcgggatc tttttcacct acgcactcca gttctacgtc ccggctgaga tcatcatccc 1140
cttctttgtg tcccgagcgc ccgagcactg tgagttagtg gtggacctgt ttgtgcgcac 1200
agtgctggtc tgcctgacat gcatcttggc catcctcatc ccccgcctgg acctggtcat 1260
ctccctggtg ggctccgtga gcagcagcgc cctggccctc atcatcccac cgctcctgga 1320
ggtcaccacc ttctactcag agggcatgag ccccctcacc atctttaagg acgccctgat 1380
cagcatcctg ggcttcgtgg gctttgtggt ggggacctat gaggctctct atgagctgat 1440

ccagccaagc aatgctccca tcttcatcaa ttccacctgt gccttcatat agggatctgg 1500
g 1501

<210> 22
<211> 496
<212> PRT
<213> Homo sapiens

<400> 22
Met Glu Leu Lys Ala Pro Ala Ala Gly Gly Leu Asn Ala Gly Pro Val
1               5                   10                  15

Pro Pro Ala Ala Met Ser Thr Gln Arg Leu Arg Asn Glu Asp Tyr His
            20                  25                  30

Asp Tyr Ser Ser Thr Asp Val Ser Pro Glu Glu Ser Pro Ser Glu Gly
        35                  40                  45

Leu Asn Asn Leu Ser Ser Pro Gly Ser Tyr Gln Arg Phe Gly Gln Ser
    50                  55                  60

Asn Ser Thr Thr Trp Phe Gln Thr Leu Ile His Leu Leu Lys Gly Asn
65                  70                  75                  80

Ile Gly Thr Gly Leu Leu Gly Leu Pro Leu Ala Val Lys Asn Ala Gly
                85                  90                  95

Ile Val Met Gly Pro Ile Ser Leu Leu Ile Ile Gly Ile Val Ala Val
            100                 105                 110

His Cys Met Gly Ile Leu Val Lys Cys Ala His His Phe Cys Arg Arg
        115                 120                 125

Leu Asn Lys Ser Phe Val Asp Tyr Gly Asp Thr Val Met Tyr Gly Leu
    130                 135                 140

Glu Ser Ser Pro Cys Ser Trp Leu Arg Asn His Ala His Trp Gly Arg
145                 150                 155                 160

Arg Val Val Asp Phe Phe Leu Ile Val Thr Gln Leu Val Phe Cys Cys
                165                 170                 175

Val Tyr Phe Val Phe Leu Ala Asp Asn Phe Lys Gln Val Ile Glu Ala
            180                 185                 190

Ala Asn Gly Thr Thr Asn Asn Cys His Asn Asn Glu Thr Val Ile Leu
        195                 200                 205

Thr Pro Thr Met Asp Ser Arg Leu Tyr Met Leu Ser Phe Leu Pro Phe
    210                 215                 220

Leu Val Leu Leu Val Phe Ile Arg Asn Leu Arg Ala Leu Ser Ile Phe
225                 230                 235                 240

Ser Leu Leu Ala Asn Ile Thr Met Leu Val Ser Leu Val Met Ile Tyr
                245                 250                 255

Gln Phe Ile Val Gln Arg Ile Pro Asp Pro Ser His Leu Pro Leu Val
            260                 265                 270

Ala Pro Trp Lys Thr Tyr Pro Leu Phe Phe Gly Thr Ala Ile Phe Ser
        275                 280                 285

Phe Glu Gly Ile Gly Met Val Leu Pro Leu Glu Asn Lys Met Lys Asp
    290                 295                 300

Pro Arg Lys Phe Pro Leu Ile Leu Tyr Leu Gly Met Val Ile Val Thr
305                 310                 315                 320

Ile Leu Tyr Ile Ser Leu Gly Cys Leu Gly Tyr Leu Gln Phe Gly Ala
                325                 330                 335

Asn Ile Gln Gly Ser Ile Thr Leu Asn Leu Pro Asn Cys Trp Leu Tyr
            340                 345                 350

Gln Ser Val Lys Leu Leu Tyr Ser Ile Gly Ile Phe Phe Thr Tyr Ala
        355                 360                 365

Leu Gln Phe Tyr Val Pro Ala Glu Ile Ile Ile Pro Phe Phe Val Ser
    370                 375                 380

Arg Ala Pro Glu His Cys Glu Leu Val Val Asp Leu Phe Val Arg Thr
385                 390                 395                 400

Val Leu Val Cys Leu Thr Cys Ile Leu Ala Ile Leu Ile Pro Arg Leu
                405                 410                 415

Asp Leu Val Ile Ser Leu Val Gly Ser Val Ser Ser Ser Ala Leu Ala
            420                 425                 430

Leu Ile Ile Pro Pro Leu Leu Glu Val Thr Thr Phe Tyr Ser Glu Gly
        435                 440                 445

Met Ser Pro Leu Thr Ile Phe Lys Asp Ala Leu Ile Ser Ile Leu Gly
    450                 455                 460

Phe Val Gly Phe Val Val Gly Thr Tyr Glu Ala Leu Tyr Glu Leu Ile
465                 470                 475                 480

Gln Pro Ser Asn Ala Pro Ile Phe Ile Asn Ser Thr Cys Ala Phe Ile
                485                 490                 495

<210> 23
<211> 953
<212> DNA
<213> Homo sapiens

<400> 23
tatggagcta aaggctccag ctgcaggagg tcttaatgct ggccctgtcc ccccagctgc 60
catgtccacg cagagacttc ggaatgaaga ctaccacgac tacagctcca cggacgtgag 120
ccctgaggag agcccgtcgg aaggcctcaa caacctctcc tccccgggct cctaccagcg 180
ctttggtcaa agcaatagca caacatggtt ccagaccttg atccacctgt taaaaggcaa 240
cattggcaca ggactgctgg gactccctct ggcggtgaaa aatgcaggca tcgtgatggg 300
tcccatcagc ctgctgatca taggcatcgt ggccgtgcac tgcatgggta tcctggtgaa 360
atgtgctcac cacttctgcc gcaggctgaa taaatccttt gtggattatg gtgatactgt 420
gatgtatgga ctagaatcca gcccctgctc ctggctccgg aaccacgcac actggggaag 480
acgtgttgtg gacttcttcc tgattgtcac ccagctggga ttctgctgtg tctattttgt 540
gtttctggct gacaacttta aacaggtgat agaagcggcc aatgggacca ccaataactg 600
ccacaacaat gagacggtga ttctgacgcc taccatggac tcgcgactct acatgctctc 660
cttcctgccc ttcctggtgc tgctggtttt catcaggaac ctccgagccc tgtccatctt 720
ctccctgttg gccaacatca ccatgctggt cagcttggtc atgatctacc agttcattgt 780
tcagatcctg tgaatggatt tacagcccat gtagcagacc aaggtcttcc acagagagca 840
ggttcctctc tgtcttcagc atgtggagtc tcaaatggaa cagttctggg cagagtgctt 900
tgcacagagg gtgctcccaa taaatgtttt atcactgcaa aaaaaaaaaa aaa       953

<210> 24
<211> 263
<212> PRT
<213> Homo sapiens

<400> 24
Met Glu Leu Lys Ala Pro Ala Ala Gly Gly Leu Asn Ala Gly Pro Val

1                 5                   10                  15

Pro Pro Ala Ala Met Ser Thr Gln Arg Leu Arg Asn Glu Asp Tyr His
                20                  25                  30

Asp Tyr Ser Ser Thr Asp Val Ser Pro Glu Glu Ser Pro Ser Glu Gly
            35                  40                  45

Leu Asn Asn Leu Ser Ser Pro Gly Ser Tyr Gln Arg Phe Gly Gln Ser
    50                  55                  60

Asn Ser Thr Thr Trp Phe Gln Thr Leu Ile His Leu Leu Lys Gly Asn
65                  70                  75                  80

Ile Gly Thr Gly Leu Leu Gly Leu Pro Leu Ala Val Lys Asn Ala Gly
                85                  90                  95

Ile Val Met Gly Pro Ile Ser Leu Leu Ile Ile Gly Ile Val Ala Val
            100                 105                 110

His Cys Met Gly Ile Leu Val Lys Cys Ala His His Phe Cys Arg Arg
        115                 120                 125

Leu Asn Lys Ser Phe Val Asp Tyr Gly Asp Thr Val Met Tyr Gly Leu
    130                 135                 140

Glu Ser Ser Pro Cys Ser Trp Leu Arg Asn His Ala His Trp Gly Arg
145                 150                 155                 160

Arg Val Val Asp Phe Phe Leu Ile Val Thr Gln Leu Gly Phe Cys Cys
                165                 170                 175

Val Tyr Phe Val Phe Leu Ala Asp Asn Phe Lys Gln Val Ile Glu Ala
            180                 185                 190

Ala Asn Gly Thr Thr Asn Asn Cys His Asn Asn Glu Thr Val Ile Leu
        195                 200                 205

Thr Pro Thr Met Asp Ser Arg Leu Tyr Met Leu Ser Phe Leu Pro Phe
    210                 215                 220

Leu Val Leu Leu Val Phe Ile Arg Asn Leu Arg Ala Leu Ser Ile Phe
225                 230                 235                 240

Ser Leu Leu Ala Asn Ile Thr Met Leu Val Ser Leu Val Met Ile Tyr
                245                 250                 255

Gln Phe Ile Val Gln Ile Leu
            260

<210> 25
<211> 468
<212> DNA
<213> Homo sapiens

<400> 25
tgaagtttgt ctgtgcacta gcaccctgga atgagcagtc tccaggccat gaagaccttg 60
tccctggtcc tgctggtggc cctgctgagc atggagagag ctcagggtct gcgctgctac 120
agatgcttgg cggtcttgga aggggcctcc tgcagcgtgg tctcgtgccc cttcctggat 180
ggggtctgtg tctcccagaa agtgagctta agtctgagca agaaaagaag aaaagaaaaa 240
aacaagctct ccctcctctc ctgccagaag gacgtcggat tccccctgct gaaacttaca 300
agtgccgttg tggactccca gatctcttgc tgcaagggag acctctgcaa tgcggtggtc 360
ctggcagcca gcagcccctg ggccctgtgc gtacagctcc tgctcagcct ggggtcagtc 420
ttcctctggg ccctgctgtg agggcccttt cccgccctct cccccgca             468

<210> 26
<211> 136

<212> PRT
<213> Homo sapiens

<400> 26
Met Ser Ser Leu Gln Ala Met Lys Thr Leu Ser Leu Val Leu Leu Val
1               5                   10                  15

Ala Leu Leu Ser Met Glu Arg Ala Gln Gly Leu Arg Cys Tyr Arg Cys
            20                  25                  30

Leu Ala Val Leu Glu Gly Ala Ser Cys Ser Val Val Ser Cys Pro Phe
        35                  40                  45

Leu Asp Gly Val Cys Val Ser Gln Lys Val Ser Leu Ser Leu Ser Lys
    50                  55                  60

Lys Arg Arg Lys Glu Lys Asn Lys Leu Ser Leu Leu Ser Cys Gln Lys
65                  70                  75                  80

Asp Val Gly Phe Pro Leu Leu Lys Leu Thr Ser Ala Val Val Asp Ser
                85                  90                  95

Gln Ile Ser Cys Cys Lys Gly Asp Leu Cys Asn Ala Val Val Leu Ala
            100                 105                 110

Ala Ser Ser Pro Trp Ala Leu Cys Val Gln Leu Leu Leu Ser Leu Gly
        115                 120                 125

Ser Val Phe Leu Trp Ala Leu Leu
    130                 135


<210> 27
<211> 610
<212> DNA
<213> Homo sapiens

<400> 27
cttgtaaggg cgagacagca gagactggca ccagggaggc tcctccatgg gagtcttcca 60
tgattactca cagcgggtgg gcagaggtgt tgctaggaag catgttctgg gggggtcctc 120
tgggtgcaca cgtgcagtag ctgcacctgc ttgctcatac gtcgcatgtg tcattagcat 180
cttaaatctc caccaggggt gtgttttctt gccctctctc ccagctcagg gtctgcgctg 240
ctacagatgc ttggcggtct tggaaggggc ctcctgcagc gtggtctcgt gccccttcct 300
ggatggggtc tgtgtctccc agaaagtgag cgttttggca gtgagtccct ggggtgccag 360
ggcagagggc aggttaagtg ccgttgtgga ctcccagatc tcttgctgca agggagacct 420
ctgcaatgcg gtggtcctgg cagccggcag cccctgggcc ctgtgcgtac agctcctgct 480
cagcctgggg tcagtcttcc tctgggccct gctgtgaggg cctttcccgc cctctccccc 540
gcaggcctac cctctgtccc tgtgcgtcac cagctgcttg gttttgaaga gctgcctcac 600
tgaattgcag                                                        610


<210> 28
<211> 156
<212> PRT
<213> Homo sapiens

<400> 28
Met Gly Val Phe His Asp Tyr Ser Gln Arg Val Gly Arg Gly Val Ala
1               5                   10                  15

Arg Lys His Val Leu Gly Gly Ser Ser Gly Cys Thr Arg Ala Val Ala
            20                  25                  30

Ala Pro Ala Cys Ser Tyr Val Ala Cys Val Ile Ser Ile Leu Asn Leu
        35                  40                  45

His Gln Gly Cys Val Phe Leu Pro Ser Leu Pro Ala Gln Gly Leu Arg
    50                  55                  60

Cys Tyr Arg Cys Leu Ala Val Leu Glu Gly Ala Ser Cys Ser Val Val
65                  70                  75                  80

Ser Cys Pro Phe Leu Asp Gly Val Cys Val Ser Gln Lys Val Ser Val
                85                  90                  95

Leu Ala Val Ser Pro Trp Gly Ala Arg Ala Glu Gly Arg Leu Ser Ala
            100                 105                 110

Val Val Asp Ser Gln Ile Ser Cys Cys Lys Gly Asp Leu Cys Asn Ala
        115                 120                 125

Val Val Leu Ala Ala Gly Ser Pro Trp Ala Leu Cys Val Gln Leu Leu
    130                 135                 140

Leu Ser Leu Gly Ser Val Phe Leu Trp Ala Leu Leu
145                 150                 155

<210> 29
<211> 406
<212> DNA
<213> Homo sapiens

<400> 29
aatgagcagt ctccaggcca tgaagacctt gtccctggtc ctgctggtgg ccctgctgag 60
catggagaga gctcagggtc tgcgctgcta cagatgcttg gcggtcttgg aaggggcctc 120
ctgcagcgtg gtctcgtgcc ccttcctgga tggggtctgt gtctcccaga aagtgagcgt 180
ctttggcagt aaagtgagag gggagaacaa gctctccctc ctctcctgcc agaaggacgt 240
cggattcccc ctgctgaaac ttacaagtgc cgttgtggac tcccagatct cttgctgcaa 300
gggagacctc tgcaatgcgg tggtcctggc agccggcagc ccccgggccc tgtgcgtaca 360
gctcctgctc agcctggggt cagtcttcct ctgggccctg ctgtga                406

<210> 30
<211> 134
<212> PRT
<213> Homo sapiens

<400> 30
Met Ser Ser Leu Gln Ala Met Lys Thr Leu Ser Leu Val Leu Leu Val
1               5                   10                  15

Ala Leu Leu Ser Met Glu Arg Ala Gln Gly Leu Arg Cys Tyr Arg Cys
            20                  25                  30

Leu Ala Val Leu Glu Gly Ala Ser Cys Ser Val Val Ser Cys Pro Phe
        35                  40                  45

Leu Asp Gly Val Cys Val Ser Gln Lys Val Ser Val Phe Gly Ser Lys
    50                  55                  60

Val Arg Gly Glu Asn Lys Leu Ser Leu Leu Ser Cys Gln Lys Asp Val
65                  70                  75                  80

Gly Phe Pro Leu Leu Lys Leu Thr Ser Ala Val Val Asp Ser Gln Ile
                85                  90                  95

Ser Cys Cys Lys Gly Asp Leu Cys Asn Ala Val Val Leu Ala Ala Gly
            100                 105                 110

Ser Pro Arg Ala Leu Cys Val Gln Leu Leu Leu Ser Leu Gly Ser Val
        115                 120                 125

Phe Leu Trp Ala Leu Leu
    130

<210> 31

<211> 611
<212> DNA
<213> Homo sapiens

<400> 31
cttgtaaggg cgagacagca gagactggca ccagggaggc tcctccatgg gagtcttcca 60
tgattactca cagcgggtgg gcagaggtgt tgctaggaag catgttctgg gggggtcctc 120
tgggtgcaca cgtgcagtag ctgcacctgc ttgctcatac gtcgcatgtg tcattagcat 180
cttaaatctc caccaggggt gtgttttctt gccctctctc ccagctcagg gtctgcgctg 240
ctacagatgc ttggcggtct tggaaggggc ctcctgcagc gtggtctcgt gccccttcct 300
ggatggggtc tgtgtctccc agaaagtgag cgtctttggc agtgagtccc tggggtgcca 360
gggcagaggg caggttaagt gccgttgtgg actcccagat ctcttgctgc aagggagacc 420
tctgcaatgc ggtggtcctg gcagccggca gcccctgggc cctgtgcgta cagctcctgc 480
tcagcctggg gtcagtcttc ctctgggccc tgctgtgagg gcctttcccg ccctctcccc 540
cgcaggccta ccctctgtcc ctgtgcgtca ccagctgctt ggttttgaag agctgcctca 600
ctgaattgca g 611

<210> 32
<211> 185
<212> PRT
<213> Homo sapiens

<400> 32
Met Gly Val Phe His Asp Tyr Ser Gln Arg Val Gly Arg Gly Val Ala
1               5                   10                  15

Arg Lys His Val Leu Gly Gly Ser Ser Gly Cys Thr Arg Ala Val Ala
            20                  25                  30

Ala Pro Ala Cys Ser Tyr Val Ala Cys Val Ile Ser Ile Leu Asn Leu
        35                  40                  45

His Gln Gly Cys Val Phe Leu Pro Ser Leu Pro Ala Gln Gly Leu Arg
    50                  55                  60

Cys Tyr Arg Cys Leu Ala Val Leu Glu Gly Ala Ser Cys Ser Val Val
65                  70                  75                  80

Ser Cys Pro Phe Leu Asp Gly Val Cys Val Ser Gln Lys Val Ser Val
                85                  90                  95

Phe Gly Ser Glu Ser Leu Gly Cys Gln Gly Arg Gly Gln Val Lys Cys
            100                 105                 110

Arg Cys Gly Leu Pro Asp Leu Leu Leu Gln Gly Arg Pro Leu Gln Cys
        115                 120                 125

Gly Gly Pro Gly Ser Arg Gln Pro Leu Gly Pro Val Arg Thr Ala Pro
    130                 135                 140

Ala Gln Pro Gly Val Ser Leu Pro Leu Gly Pro Ala Val Arg Ala Phe
145                 150                 155                 160

Pro Ala Leu Ser Pro Ala Gly Leu Pro Ser Val Pro Val Arg His Gln
                165                 170                 175

Leu Leu Gly Phe Glu Glu Leu Pro His
            180                 185

<210> 33
<211> 423
<212> DNA
<213> Homo sapiens

<400> 33
ggatcccacc aggggtgtgt tttcttgccc tctctcccag ctcagggtct gcgctgctac 60
agatgcttgg cggtcttgga aggggcctcc tgcagcgtgg tctcgtgccc cttcctggat 120

ggggtctgtg tctcccagaa agtgagcgtc tttggcagtg agtccctggg gtgccagggc 180
agagggcagg ttaagtgccg ttgtggactc ccagatctct tgctgcaagg gagacctctg 240
caatgcggtg gtcctggcag ccggcagccc ctgggccctg tgcgtacagc tcctgctcag 300
cctggggtca gtcttcctct gggccctgct gtgagggcct ttcccgccct ctcccccgca 360
ggcctaccct ctgtccctgt gcgtcaccag ctgcttggtt ttgaagagct gcctcacctc 420
gag 423

<210> 34
<211> 141
<212> PRT
<213> Homo sapiens

<400> 34
Gly Ser His Gln Gly Cys Val Phe Leu Pro Ser Leu Pro Ala Gln Gly
1               5                   10                  15

Leu Arg Cys Tyr Arg Cys Leu Ala Val Leu Glu Gly Ala Ser Cys Ser
            20                  25                  30

Val Val Ser Cys Pro Phe Leu Asp Gly Val Cys Val Ser Gln Lys Val
        35                  40                  45

Ser Val Phe Gly Ser Glu Ser Leu Gly Cys Gln Gly Arg Gly Gln Val
    50                  55                  60

Lys Cys Arg Cys Gly Leu Pro Asp Leu Leu Leu Gln Gly Arg Pro Leu
65                  70                  75                  80

Gln Cys Gly Gly Pro Gly Ser Arg Gln Pro Leu Gly Pro Val Arg Thr
                85                  90                  95

Ala Pro Ala Gln Pro Gly Val Ser Leu Pro Leu Gly Pro Ala Val Arg
            100                 105                 110

Ala Phe Pro Ala Leu Ser Pro Ala Gly Leu Pro Ser Val Pro Val Arg
        115                 120                 125

His Gln Leu Leu Gly Phe Glu Glu Leu Pro His Leu Glu
    130                 135                 140

<210> 35
<211> 1873
<212> DNA
<213> Homo sapiens

<400> 35
ctgcggccgc cgccagcagt tttcatgttt gggattcagg agaatattcc gcgcgggggg 60
acgaccatga aggaggagcc gctgggcagc ggcatgaacc cggtgcgctc gtggatgcac 120
acggcgggcg tggtggacgc caacacggcc gcccagagcg gcgtggggct ggcgcgggcg 180
cacttcgaga agcagccgcc ttccaacctc cggaaatcca atttcttcca cttcgtgctg 240
gcgctctacg ataggcaggg gcagccggtg gagattgaaa ggaccgcttt tgtggacttt 300
gtggagaaag agaaagagcc aaacaacgag aaaaccaaca acggcatcca ctataaactc 360
cagttattgt acagcaacgg tgtcagaaca gagcaagatc tgtatgttcg cctcatagat 420
tcaatgacca aacaggccat cgtctacgag ggccaggaca agaacccgga gatgtgccgt 480
gtgctgctga cccacgagat catgtgcagc cggtgctgtg acaagaaaag ttgtggcaat 540
agaaacgaaa cgccctcaga ccctgtaatc attgacaggt tctttctaaa gtttttcctc 600
aagtgcaatc agaactgttt gaagaatgca ggcaaccctc gagatatgcg gagattccag 660
gtagttgtat cgacaacagt caacgtggac ggccacgtgc tggccgtgtc agacaacatg 720
tttgtgcaca acaattccaa acacgggagg cgggcccgcc gcctagaccc gtcagaaggt 780
acggcccctt cttatctgga aaatgccact ccgtgcatca aggccatcag tcccagtgaa 840
ggctggacca cggggggtgc caccgtcatc ataattggcg acaacttctt tgacgggctg 900
caagttgtat tcggaactat gttggtgtgg agcgagctga taactcccca tgccatccga 960
gtccagaccc cgccgaggca cattcctggc gtcgtcgaag tgaccctctc ctacaaatcc 1020
aagcagttct gcaaaggtgc tcctgggcgc tttgtctaca ccgcccttaa tgaaccaacc 1080
atagattacg gctttcagag gttgcagaaa gtgatcccaa gacatccggg tgatcccgaa 1140
aggttaccca aggaggtgtt actgaagcgg gcggcggacc tggtggaagc cttatacgga 1200
atgcctcaca acaaccagga gatcatcttg aagcgagcgg cggacatcgc cgaggcgctg 1260

tacagcgttc cccgcaatca caaccagatc cccaccctgg gcaacaaccc tgcacacacg 1320
ggcatgatgg gcgtcaactc cttcagcagc cagctagccg tcaacgtgtc agagacgtca 1380
caagccaacg accaagtcgg ctacagtcgc aatacaagca gcgtgtcccc gcgaggctac 1440
gtccccagca gtactcccca gcagtccaat tacaacacag tcagcactag catgaatgga 1500
tatggaagtg gcgccatggc cagtctaggg gtccctggct cgcctggatt tcttaatggc 1560
tcctccgcta actctcccta cggcgtagtg ccgtccagcc ccaccatggc agcctcttcg 1620
gtcaccctcc cttcaaactg tagcagcaca cacggcattt tctcattctc acctgccaat 1680
gtcatctccg cagtgaaaca gaagagcgcc ttcgcgcccg tggtccggcc ccaagcctct 1740
cctcctcctt cctgcaccag cgccaacggg aatggactgc aagctatgtc tgggctggta 1800
gtcccgccaa tgtgagggac ttctgtttac cttccgcagc acccagcatc aaaggacgga 1860
cttcagggga cac 1873

<210> 36
<211> 596
<212> PRT
<213> Homo sapiens

<400> 36
Met Phe Gly Ile Gln Glu Asn Ile Pro Arg Gly Gly Thr Thr Met Lys
1               5                   10                  15

Glu Glu Pro Leu Gly Ser Gly Met Asn Pro Val Arg Ser Trp Met His
            20                  25                  30

Thr Ala Gly Val Val Asp Ala Asn Thr Ala Ala Gln Ser Gly Val Gly
        35                  40                  45

Leu Ala Arg Ala His Phe Glu Lys Gln Pro Pro Ser Asn Leu Arg Lys
    50                  55                  60

Ser Asn Phe Phe His Phe Val Leu Ala Leu Tyr Asp Arg Gln Gly Gln
65                  70                  75                  80

Pro Val Glu Ile Glu Arg Thr Ala Phe Val Asp Phe Val Glu Lys Glu
                85                  90                  95

Lys Glu Pro Asn Asn Glu Lys Thr Asn Asn Gly Ile His Tyr Lys Leu
            100                 105                 110

Gln Leu Leu Tyr Ser Asn Gly Val Arg Thr Glu Gln Asp Leu Tyr Val
        115                 120                 125

Arg Leu Ile Asp Ser Met Thr Lys Gln Ala Ile Val Tyr Glu Gly Gln
    130                 135                 140

Asp Lys Asn Pro Glu Met Cys Arg Val Leu Leu Thr His Glu Ile Met
145                 150                 155                 160

Cys Ser Arg Cys Cys Asp Lys Lys Ser Cys Gly Asn Arg Asn Glu Thr
                165                 170                 175

Pro Ser Asp Pro Val Ile Ile Asp Arg Phe Phe Leu Lys Phe Phe Leu
            180                 185                 190

Lys Cys Asn Gln Asn Cys Leu Lys Asn Ala Gly Asn Pro Arg Asp Met
        195                 200                 205

Arg Arg Phe Gln Val Val Val Ser Thr Thr Val Asn Val Asp Gly His
    210                 215                 220

Val Leu Ala Val Ser Asp Asn Met Phe Val His Asn Asn Ser Lys His
225                 230                 235                 240

Gly Arg Arg Ala Arg Arg Leu Asp Pro Ser Glu Gly Thr Ala Pro Ser
                245                 250                 255

Tyr Leu Glu Asn Ala Thr Pro Cys Ile Lys Ala Ile Ser Pro Ser Glu
            260                 265                 270

Gly Trp Thr Thr Gly Gly Ala Thr Val Ile Ile Ile Gly Asp Asn Phe
        275                 280                 285

Phe Asp Gly Leu Gln Val Val Phe Gly Thr Met Leu Val Trp Ser Glu
    290                 295                 300

Leu Ile Thr Pro His Ala Ile Arg Val Gln Thr Pro Pro Arg His Ile
305                 310                 315                 320

Pro Gly Val Val Glu Val Thr Leu Ser Tyr Lys Ser Lys Gln Phe Cys
                325                 330                 335

Lys Gly Ala Pro Gly Arg Phe Val Tyr Thr Ala Leu Asn Glu Pro Thr
            340                 345                 350

Ile Asp Tyr Gly Phe Gln Arg Leu Gln Lys Val Ile Pro Arg His Pro
        355                 360                 365

Gly Asp Pro Glu Arg Leu Pro Lys Glu Val Leu Leu Lys Arg Ala Ala
    370                 375                 380

Asp Leu Val Glu Ala Leu Tyr Gly Met Pro His Asn Asn Gln Glu Ile
385                 390                 395                 400

Ile Leu Lys Arg Ala Ala Asp Ile Ala Glu Ala Leu Tyr Ser Val Pro
                405                 410                 415

Arg Asn His Asn Gln Ile Pro Thr Leu Gly Asn Asn Pro Ala His Thr
            420                 425                 430

Gly Met Met Gly Val Asn Ser Phe Ser Ser Gln Leu Ala Val Asn Val
        435                 440                 445

Ser Glu Thr Ser Gln Ala Asn Asp Gln Val Gly Tyr Ser Arg Asn Thr
    450                 455                 460

Ser Ser Val Ser Pro Arg Gly Tyr Val Pro Ser Ser Thr Pro Gln Gln
465                 470                 475                 480

Ser Asn Tyr Asn Thr Val Ser Thr Ser Met Asn Gly Tyr Gly Ser Gly
                485                 490                 495

Ala Met Ala Ser Leu Gly Val Pro Gly Ser Pro Gly Phe Leu Asn Gly
            500                 505                 510

Ser Ser Ala Asn Ser Pro Tyr Gly Val Val Pro Ser Ser Pro Thr Met
        515                 520                 525

Ala Ala Ser Ser Val Thr Leu Pro Ser Asn Cys Ser Ser Thr His Gly
    530                 535                 540

Ile Phe Ser Phe Ser Pro Ala Asn Val Ile Ser Ala Val Lys Gln Lys
545                 550                 555                 560

Ser Ala Phe Ala Pro Val Val Arg Pro Gln Ala Ser Pro Pro Pro Ser
                565                 570                 575

Cys Thr Ser Ala Asn Gly Asn Gly Leu Gln Ala Met Ser Gly Leu Val
            580                 585                 590

Val Pro Pro Met
        595


<210> 37
<211> 1667
<212> DNA
<213> Homo sapiens

<400> 37
tttcatgttt gggattcagg agaatattcc gcgcgggggg acgaccatga aggaggagcc 60
gctgggcagc ggcatgaacc cggtgcgctc gtggatgcac acggcgggcg tggtggatgc 120
caacacggcc gcccagagcg gcgtggggct ggcgcgggcg cacttcgaga agcagccgcc 180
ttccaacctc cggaaatcca atttcttcca cttcgtgctg gcgctctacg ataggcaggg 240
gcagccggtg gagattgaaa ggaccgcttt tgtggacttt gtggagaaag agaaagagcc 300
aaacaacgag aaaaccaaca acggcatcca ctataaactc cagttattgt acagcaacgg 360
agtcagaaca gagcaagatc tgtatgttcg cctcatagat tcaatgacca aacaggccat 420
cgtctacgag ggccaggaca agaacccgga gatgtgccgt gtgctgctga cccacgagat 480
catgtgcagc cggtgctgtg acaagaaaag ttgtggcaat agaaacgaaa cgccctcaga 540
ccctgtaatc attgacagat tctttctaaa gtttttcctc aagtgcaatc agaactgttt 600
gaagaatgca ggcaaccctc gagatatgcg gagattccag gttgttgtat cgacaacagt 660
caacgtggac ggccacgtgc tggccgtgtc agacaacatg tttgtgcaca acaattccaa 720
acacgggagg cgggcccgcc gcctagaccc gtcagaagcc actccgtgca tcaaggccat 780
cagtcccagt gaaggctgga ccacgggggg tgccaccgtc atcataattg gcgacaactt 840
ctttgacggg ctgcaagttg tattcggaac tatgttggtg tggagcgagc tgataactcc 900
ccatgccatc cgagtccaga ccccgccgag gcacattcct ggcgtcgtcg aagtgaccct 960
ctcctacaaa tccaagcagt tctgcaaagg tgctcctggg cgctttgtct acaccgccct 1020
taatgaacca accatagatt acggctttca gaggttgcag aaagtgatcc caagacatcc 1080
gggtgatccc gaaaggttac ccaaggaggt gttactgaag cgggcggcgg acctggtgga 1140
agccttatac ggaatgcctc acaacaacca ggagatcatc ttgaagcgag cggcggacat 1200
cgccgaggcg ctgtacagcg ttccccgcaa tcacaaccag atccccaccc tgggcaacaa 1260
ccctgcacac acgggcatga tgggcgtcaa ctccttcagc agccagctag ccgtcaacgt 1320
gtcagagacg tcacaagcca acgaccaagt cggctacagt cgcaatacaa gcagcgtgtc 1380
cccgcgaggc tacgtcccca gcagtactcc ccagcagtcc aattacaaca cagtcagcac 1440
tagcatgaat ggatatggaa gtggcgccat ggccagtcta ggggtccctg gctcgcctgg 1500
atttcttaat ggctcctccg ctaactctcc ctacggcatg aaacagaaga gcgccttcgc 1560
gcccgtggtc cggccccaag cctctcctcc tccttcctgc accagcgcca acgggaatgg 1620
actgtaagct atgtctgggc tggtagtccc gccaatgtga gggactt 1667

<210> 38
<211> 540
<212> PRT
<213> Homo sapiens

<400> 38
Met Phe Gly Ile Gln Glu Asn Ile Pro Arg Gly Gly Thr Thr Met Lys
1               5                   10                  15

Glu Glu Pro Leu Gly Ser Gly Met Asn Pro Val Arg Ser Trp Met His
            20                  25                  30

Thr Ala Gly Val Val Asp Ala Asn Thr Ala Ala Gln Ser Gly Val Gly
        35                  40                  45

Leu Ala Arg Ala His Phe Glu Lys Gln Pro Pro Ser Asn Leu Arg Lys
    50                  55                  60

Ser Asn Phe Phe His Phe Val Leu Ala Leu Tyr Asp Arg Gln Gly Gln
65                  70                  75                  80

Pro Val Glu Ile Glu Arg Thr Ala Phe Val Asp Phe Val Glu Lys Glu
                85                  90                  95

Lys Glu Pro Asn Asn Glu Lys Thr Asn Asn Gly Ile His Tyr Lys Leu
            100                 105                 110

Gln Leu Leu Tyr Ser Asn Gly Val Arg Thr Glu Gln Asp Leu Tyr Val
        115                 120                 125

Arg Leu Ile Asp Ser Met Thr Lys Gln Ala Ile Val Tyr Glu Gly Gln
    130                 135                 140

Asp Lys Asn Pro Glu Met Cys Arg Val Leu Leu Thr His Glu Ile Met
145                 150                 155                 160

Cys Ser Arg Cys Cys Asp Lys Lys Ser Cys Gly Asn Arg Asn Glu Thr

165                 170                 175

Pro Ser Asp Pro Val Ile Ile Asp Arg Phe Phe Leu Lys Phe Phe Leu
            180                 185                 190

Lys Cys Asn Gln Asn Cys Leu Lys Asn Ala Gly Asn Pro Arg Asp Met
        195                 200                 205

Arg Arg Phe Gln Val Val Val Ser Thr Thr Val Asn Val Asp Gly His
    210                 215                 220

Val Leu Ala Val Ser Asp Asn Met Phe Val His Asn Asn Ser Lys His
225                 230                 235                 240

Gly Arg Arg Ala Arg Arg Leu Asp Pro Ser Glu Ala Thr Pro Cys Ile
                245                 250                 255

Lys Ala Ile Ser Pro Ser Glu Gly Trp Thr Thr Gly Gly Ala Thr Val
            260                 265                 270

Ile Ile Ile Gly Asp Asn Phe Phe Asp Gly Leu Gln Val Val Phe Gly
        275                 280                 285

Thr Met Leu Val Trp Ser Glu Leu Ile Thr Pro His Ala Ile Arg Val
    290                 295                 300

Gln Thr Pro Pro Arg His Ile Pro Gly Val Val Glu Val Thr Leu Ser
305                 310                 315                 320

Tyr Lys Ser Lys Gln Phe Cys Lys Gly Ala Pro Gly Arg Phe Val Tyr
                325                 330                 335

Thr Ala Leu Asn Glu Pro Thr Ile Asp Tyr Gly Phe Gln Arg Leu Gln
            340                 345                 350

Lys Val Ile Pro Arg His Pro Gly Asp Pro Glu Arg Leu Pro Lys Glu
        355                 360                 365

Val Leu Leu Lys Arg Ala Ala Asp Leu Val Glu Ala Leu Tyr Gly Met
    370                 375                 380

Pro His Asn Asn Gln Glu Ile Ile Leu Lys Arg Ala Ala Asp Ile Ala
385                 390                 395                 400

Glu Ala Leu Tyr Ser Val Pro Arg Asn His Asn Gln Ile Pro Thr Leu
                405                 410                 415

Gly Asn Asn Pro Ala His Thr Gly Met Met Gly Val Asn Ser Phe Ser
            420                 425                 430

Ser Gln Leu Ala Val Asn Val Ser Glu Thr Ser Gln Ala Asn Asp Gln
        435                 440                 445

Val Gly Tyr Ser Arg Asn Thr Ser Ser Val Ser Pro Arg Gly Tyr Val
    450                 455                 460

Pro Ser Ser Thr Pro Gln Gln Ser Asn Tyr Asn Thr Val Ser Thr Ser
465                 470                 475                 480

Met Asn Gly Tyr Gly Ser Gly Ala Met Ala Ser Leu Gly Val Pro Gly
                485                 490                 495

Ser Pro Gly Phe Leu Asn Gly Ser Ser Ala Asn Ser Pro Tyr Gly Met
            500                 505                 510

Lys Gln Lys Ser Ala Phe Ala Pro Val Val Arg Pro Gln Ala Ser Pro
        515                 520                 525

Pro Pro Ser Cys Thr Ser Ala Asn Gly Asn Gly Leu

    530                 535                 540

<210> 39
<211> 3892
<212> DNA
<213> Homo sapiens

<400> 39
acgcgagatc cgcgctcgcc gccgcccgcc caggcggcga tgacacggcg cccgcggcgg 60
cccggaggcg ccgggtgggc cgtttgctga ccggatcgcg gctacccgcc agcgtgtccg 120
cggcgccgcc gccagcatgg gctgtgcccc gagcatccac atttccgagc gcctggtggc 180
cgaggacgcg cctagccccg cggcaccgcc gctgtcgtcc ggcgggccgc gcctcccgca 240
gggccagaag acggccgcct tgccccggac ccgcggcgcc ggcctcttgg agtcggaggt 300
tcgcgacggc agcggcaaga aggtagcagt agctgatgtg cagtttggcc ccatgagatt 360
tcatcaagat caacttcagg tacttttagt gtttaccaaa gaagataacc aatgtaatgg 420
attctgcagg gcatgtgaaa aagcagggtt taagtgtaca gttaccaagg aggctcaggc 480
tgtccttgcc tgtttcctgg acaaacatca tgacattatc atcatagacc acagaaatcc 540
tcgacagctg gatgcagagg cactgtgcag gtctatcaga tcatcaaaac tctcagaaaa 600
cacagttatt gttggtgtag tacgcagggt ggatagagaa gagttgtccg taatgccttt 660
catttctgct ggatttacaa ggaggtatgt agaaaacccc aacatcatgg cctgctacaa 720
tgaactgctc cagctggagt ttggagaggt gcgatcacaa ctgaaactca gggcttgtaa 780
ctcagtattc actgcattag aaaacagtga agatgcaatt gaaattacaa gcgaagaccg 840
ttttatacag tatgcaaatc ctgcatttga aacaacaatg ggctatcagt caggtgaatt 900
aatagggaag gagttaggag aagtgcctat aaatgaaaaa aaggctgact tgctcgatac 960
tataaattca tgcatcagga taggcaagga gtggcaagga atttactatg ccaaaaagaa 1020
aaacggagat aatatacaac aaaatgtgaa gataatacct gtcattggac agggaggaaa 1080
aattagacac tatgtgtcca ttatcagagt gtgcaatggc aacaataagg ctgagaaaat 1140
atccgaatgt gttcagtctg acactcgtac agataatcag acaggcaaac ataaagacag 1200
gagaaaaggc tcactagacg tcaaagctgt tgcctcccgt gcaactgaag tttccagcca 1260
gagacgacac tcttccatgg cccggataca ttccatgaca attgaggcgc ccatcaccaa 1320
ggtaatcaat attatcaatg ctgcccagga aagtagtccc atgcctgtga cagaagccct 1380
agaccgtgtg ctggaaattc taagaaccac tgagttatat tcaccacagt ttggtgctaa 1440
agatgatgat ccccatgcca atgaccttgt tggggcttta atgtctgatg gtttgcgaag 1500
actatcaggg aatgaatatg ttctttcaac aaaaaacact caaatggttt caagcaatat 1560
aatcactccc atctcccttg atgatgtccc accacggata gctcgggcca tggaaaatga 1620
ggaatactgg gactttgata tttttgaact ggaggctgcc acccacaata ggcctttgat 1680
ttatcttggt ctcaaaatgt ttgctcgctt tggaatctgt gaattcttac actgctccga 1740
gtcaacgcta agatcatggt tacaaattat cgaagccaat tatcattcct ccaatcccta 1800
ccacaattct acacattctg ctgatgtgct tcatgccact gcctattttc tctccaagga 1860
gaggataaag gaaactttag atccaattga tgaggtcgct gcactcatcg cagccaccat 1920
tcatgatgtg gatcaccctg ggagaaccaa ctccttcctg tgtaatgctg gaagtgagct 1980
ggccattttg tacaatgaca ctgctgtgct ggagagccac catgcggcct tggccttcca 2040
gctgaccact ggagatgata aatgcaatat atttaaaaac atggagagga atgattatcg 2100
gacactgcgc caggggatta tcgacatggt cttagccaca gaaatgacaa agcactttga 2160
gcatgtcaac aaatttgtca acagcatcaa caaacccttg gcaacactag aagaaaatgg 2220
ggaaactgat aaaaaccagg aagtgataaa cactatgctt aggactccag agaaccggac 2280
cctaatcaaa cgaatgctga ttaaatgtgc tgatgtgtcc aatccctgcc gacccctgca 2340
gtactgcatc gagtgggctg cacgcatttc ggaagaatat ttttctcaga ctgatgaaga 2400
gaagcagcag ggcttacctg tggtgatgcc agtgtttgac agaaatacct gcagcatccc 2460
caaatcccaa atctctttca ttgattactt catcacagac atgtttgatg cttgggatgc 2520
ctttgtagac ctgcctgatt taatgcagca tcttgacaac aactttaaat actggaaagg 2580
actggacgaa atgaagctgc ggaacctccg accacctcct gaatagtggg agacaccacc 2640
cagagccctg aagctttgtt ccttcggtca tttggaattc ctgagggcag ccagagctcc 2700
ttggtccttt cagtactagg cagaacagcc cccgatctgc atagcctgtg aaagcccacg 2760
gggacatcag taaccttctg cagccaccat ccaatgccat tactgtcaag tgagacttgg 2820
ccactgtagc ctgggcctgc tgcaggagct cttcagaaag gcacatgagg accacggttt 2880
gcctcagttt ctggtaaaac acaaggtctg gagtgcccct gcaaagggta ttgatggact 2940
tcctgccagt gacagagcat gtctattgca aacaattctc tcagttacgt tcagcactta 3000
agaacggcta atggcaatag gatctttagc aactttttca catcatagaa ggtgcaatcg 3060
ctcacttggg aacactactg agagtgactt ctcttttaaa attgagtagc agatgaaaaa 3120
ttaaaatttg aacttgatta ttaatatcaa ttaaaatgtt ttatttattt tattaaaagc 3180
tcaatatttt ctatgaattc aaaaatactt cagagccaaa gccaacttca aataccgtga 3240
ccaaatttac atgattcata ttcattatgc attacttggt atacagactt attttcataa 3300
tgcaaattaa taaaatgaca cttttactgc actatagaaa tattcatgta tgttaaactt 3360
ttctgattga ggctaactgg aaaaagctgg ggtcgtattc taagtgctaa agaaggctgc 3420
ttctactgta tagaacccag ggctctgaaa cagctctagc cgcctaatgc acttcacagg 3480
taactcccca aggtaaaact agactctctt gttggttcgc aaagaaaagt taggacttaa 3540
cacttttttc taaaatttta taattcaatt tccaaaagtc tactctattt tatactgttt 3600

ctacaaaata ttccttataa aaacaaagaa caaaaattga atatttaatg aattgacatt 3660
ttataaccaa cctgttttta tctacggtgg gaatctttga tgccagaaat ttataaagag 3720
gttctgtatc ttcacacctt gaataagcat aataccataa aaaatgacac ttgacatgtc 3780
aatgtatttg tcatttcatt ttaaactcgt atttgtggtt tttttcccag ataaaaatga 3840
aattaaacca tttcttttta agaaatcaaa aaaaaaaaaa aaaaaaaaaa aa 3892

<210> 40
<211> 829
<212> PRT
<213> Homo sapiens

<400> 40
Met Gly Cys Ala Pro Ser Ile His Ile Ser Glu Arg Leu Val Ala Glu
1               5                   10                  15

Asp Ala Pro Ser Pro Ala Ala Pro Pro Leu Ser Ser Gly Gly Pro Arg
            20                  25                  30

Leu Pro Gln Gly Gln Lys Thr Ala Ala Leu Pro Arg Thr Arg Gly Ala
        35                  40                  45

Gly Leu Leu Glu Ser Glu Val Arg Asp Gly Ser Gly Lys Lys Val Ala
    50                  55                  60

Val Ala Asp Val Gln Phe Gly Pro Met Arg Phe His Gln Asp Gln Leu
65                  70                  75                  80

Gln Val Leu Leu Val Phe Thr Lys Glu Asp Asn Gln Cys Asn Gly Phe
                85                  90                  95

Cys Arg Ala Cys Glu Lys Ala Gly Phe Lys Cys Thr Val Thr Lys Glu
            100                 105                 110

Ala Gln Ala Val Leu Ala Cys Phe Leu Asp Lys His His Asp Ile Ile
        115                 120                 125

Ile Ile Asp His Arg Asn Pro Arg Gln Leu Asp Ala Glu Ala Leu Cys
    130                 135                 140

Arg Ser Ile Arg Ser Ser Lys Leu Ser Glu Asn Thr Val Ile Val Gly
145                 150                 155                 160

Val Val Arg Arg Val Asp Arg Glu Glu Leu Ser Val Met Pro Phe Ile
                165                 170                 175

Ser Ala Gly Phe Thr Arg Arg Tyr Val Glu Asn Pro Asn Ile Met Ala
            180                 185                 190

Cys Tyr Asn Glu Leu Leu Gln Leu Glu Phe Gly Glu Val Arg Ser Gln
        195                 200                 205

Leu Lys Leu Arg Ala Cys Asn Ser Val Phe Thr Ala Leu Glu Asn Ser
    210                 215                 220

Glu Asp Ala Ile Glu Ile Thr Ser Glu Asp Arg Phe Ile Gln Tyr Ala
225                 230                 235                 240

Asn Pro Ala Phe Glu Thr Thr Met Gly Tyr Gln Ser Gly Glu Leu Ile
                245                 250                 255

Gly Lys Glu Leu Gly Glu Val Pro Ile Asn Glu Lys Lys Ala Asp Leu
            260                 265                 270

Leu Asp Thr Ile Asn Ser Cys Ile Arg Ile Gly Lys Glu Trp Gln Gly
        275                 280                 285

Ile Tyr Tyr Ala Lys Lys Lys Asn Gly Asp Asn Ile Gln Gln Asn Val
    290                 295                 300

Lys Ile Ile Pro Val Ile Gly Gln Gly Gly Lys Ile Arg His Tyr Val
305                 310                 315                 320

Ser Ile Ile Arg Val Cys Asn Gly Asn Asn Lys Ala Glu Lys Ile Ser
                325                 330                 335

Glu Cys Val Gln Ser Asp Thr Arg Thr Asp Asn Gln Thr Gly Lys His
            340                 345                 350

Lys Asp Arg Arg Lys Gly Ser Leu Asp Val Lys Ala Val Ala Ser Arg
        355                 360                 365

Ala Thr Glu Val Ser Ser Gln Arg Arg His Ser Ser Met Ala Arg Ile
    370                 375                 380

His Ser Met Thr Ile Glu Ala Pro Ile Thr Lys Val Ile Asn Ile Ile
385                 390                 395                 400

Asn Ala Ala Gln Glu Ser Ser Pro Met Pro Val Thr Glu Ala Leu Asp
                405                 410                 415

Arg Val Leu Glu Ile Leu Arg Thr Thr Glu Leu Tyr Ser Pro Gln Phe
            420                 425                 430

Gly Ala Lys Asp Asp Asp Pro His Ala Asn Asp Leu Val Gly Gly Leu
        435                 440                 445

Met Ser Asp Gly Leu Arg Arg Leu Ser Gly Asn Glu Tyr Val Leu Ser
    450                 455                 460

Thr Lys Asn Thr Gln Met Val Ser Ser Asn Ile Ile Thr Pro Ile Ser
465                 470                 475                 480

Leu Asp Asp Val Pro Pro Arg Ile Ala Arg Ala Met Glu Asn Glu Glu
                485                 490                 495

Tyr Trp Asp Phe Asp Ile Phe Glu Leu Glu Ala Ala Thr His Asn Arg
            500                 505                 510

Pro Leu Ile Tyr Leu Gly Leu Lys Met Phe Ala Arg Phe Gly Ile Cys
        515                 520                 525

Glu Phe Leu His Cys Ser Glu Ser Thr Leu Arg Ser Trp Leu Gln Ile
    530                 535                 540

Ile Glu Ala Asn Tyr His Ser Ser Asn Pro Tyr His Asn Ser Thr His
545                 550                 555                 560

Ser Ala Asp Val Leu His Ala Thr Ala Tyr Phe Leu Ser Lys Glu Arg
                565                 570                 575

Ile Lys Glu Thr Leu Asp Pro Ile Asp Glu Val Ala Ala Leu Ile Ala
            580                 585                 590

Ala Thr Ile His Asp Val Asp His Pro Gly Arg Thr Asn Ser Phe Leu
        595                 600                 605

Cys Asn Ala Gly Ser Glu Leu Ala Ile Leu Tyr Asn Asp Thr Ala Val
    610                 615                 620

Leu Glu Ser His His Ala Ala Leu Ala Phe Gln Leu Thr Thr Gly Asp
625                 630                 635                 640

Asp Lys Cys Asn Ile Phe Lys Asn Met Glu Arg Asn Asp Tyr Arg Thr
                645                 650                 655

Leu Arg Gln Gly Ile Ile Asp Met Val Leu Ala Thr Glu Met Thr Lys
            660                 665                 670

His Phe Glu His Val Asn Lys Phe Val Asn Ser Ile Asn Lys Pro Leu
        675                 680                 685

Ala Thr Leu Glu Glu Asn Gly Glu Thr Asp Lys Asn Gln Glu Val Ile
    690                 695                 700

Asn Thr Met Leu Arg Thr Pro Glu Asn Arg Thr Leu Ile Lys Arg Met
705                 710                 715                 720

Leu Ile Lys Cys Ala Asp Val Ser Asn Pro Cys Arg Pro Leu Gln Tyr
                725                 730                 735

Cys Ile Glu Trp Ala Ala Arg Ile Ser Glu Glu Tyr Phe Ser Gln Thr
            740                 745                 750

Asp Glu Glu Lys Gln Gln Gly Leu Pro Val Val Met Pro Val Phe Asp
        755                 760                 765

Arg Asn Thr Cys Ser Ile Pro Lys Ser Gln Ile Ser Phe Ile Asp Tyr
    770                 775                 780

Phe Ile Thr Asp Met Phe Asp Ala Trp Asp Ala Phe Val Asp Leu Pro
785                 790                 795                 800

Asp Leu Met Gln His Leu Asp Asn Asn Phe Lys Tyr Trp Lys Gly Leu
                805                 810                 815

Asp Glu Met Lys Leu Arg Asn Leu Arg Pro Pro Pro Glu
            820                 825


<210> 41
<211> 6524
<212> DNA
<213> Homo sapiens

<400> 41
agtctccttg ctctgggctg ctccaggcca ggccatgcgg aaagtgtgtt gttatgagga 60
ttcaatgagg agacctgtat gaagtgcctt cccctgtgcc tggctcatag cgaaaacaga 120
agaacagatt gcagcagaag aggcctggaa tgagacggag aaggtgtggc tggtccatag 180
ggacggcttc tcactggcca gtcaactcaa atctgaggag ctcaacttgc ctgaggggaa 240
ggtgcgtgtg aagctggacc acgatggggc catcctggat gtggatgagg atgacgttga 300
gaaggctaat gctccctcct gcgaccgtct ggaggatctg gcctcactgg tgtacctcaa 360
tgagtccagc gtcctgcaca ccttgcgcca gcgctatggc gctagcctgc tgcacacgta 420
tgctggcccc agcctgctgg ttcttggccc ccgtggggcc cctgctgtgt actctgagaa 480
ggtgatgcac atgttcaagg gttgtcggcg ggaggacatg gcaccccaca tctatgcagt 540
ggcccagacc gcatacaggg cgatgctgat gagccgtcag gatcagtcaa tcatcctcct 600
gggcagtagt ggcagtggca agaccaccag ctgccagcat ctggtgcagt acctggccac 660
catcgcgggc atcagcggga acaaggtgtt ttctgtggag aagtggcagg ctctgtacac 720
cctcctggaa gcctttggga acagccccac catcattaat ggcaatgcca cccgcttctc 780
ccagatcctc tccctggact ttgaccaagc tggccaggtg gcctcagcct ccattcagac 840
aatgcttctg gagaagctgc gtgtggctcg gcgcccagcc agtgaagcca cattcaacgt 900
cttctactac ctgctggcct gtggggatgg caccctcagg acagagctcc acctcaacca 960
cttggcagag aacaatgtgt ttgggattgt gccactggcc aagcctgagg aaaagcagaa 1020
ggcagctcag cagtttagta agctgcaggc ggccatgaag gtgctgggca tctcccccga 1080
tgaacagaag gcctgctggt tcattctggc tgccatctac cacctggggg ctgcgggagc 1140
caccaaagaa gctgctgaag ctgggcgcaa gcagtttgcc cgccatgagt gggcccagaa 1200
ggctgcgtac ctactgggct gcagcctgga ggagctgtcc tcagccatct tcaagcacca 1260
gcacaagggt ggcaccctgc agcgctccac ctccttccgc cagggccccg aggagagtgg 1320
cctgggagat gggacaggcc cgaaactgag tgcactggag tgccttgagg gcatggcggc 1380
cggcctctac agcgagctct tcacccttct cgtctccctg gtgaataggg ctctcaagtc 1440
cagccagcac tcactctgct ccatgatgat tgtcgacacc ccgggcttcc agaaccctga 1500
gcagggtggg tcagcccgcg gagcctcctt tgaggagctg tgccacaact acacccaaga 1560
ccggctgcag aggctcttcc acgagcgcac cttcgtgcag gagttggaaa gatacaagga 1620
ggagaacatc gagctggcgt ttgacgactt ggaacccccc acggatgact ctgtggctgc 1680
tgtggaccag gcctcccatc agtccctggt ccgctcgctg gcccgcacag acgaggcgag 1740
gggcctgctc tggctattgg aagaggaggc tctggtgcca ggggccagtg aggacaccct 1800
cctggagcgc cttttctcct attatggccc ccaggaaggt gacaaaaaag gccaaagccc 1860

ccttctgcac agcagcaaac cacaccactt tctcctgggc cacagccatg gcaccaactg 1920
ggtagagtac aatgtgactg gctggctgaa ctacaccaag cagaacccag ccacccagaa 1980
tgtcccccgg ctcctgcagg actcccagaa aaaaatcatc agcaacctgt ttctgggccg 2040
cgcaggcagt gccacggtgc tctctggctc catcgcgggc ctggagggcg gctcgcagct 2100
ggcactgcgc cgggccacca gcatgcggaa aacctttacc acaggcatgg tggctgtcaa 2160
aaagaagtca ctgtgcatcc agatgaagct acaggtggac gccctcatcg acaccatcaa 2220
gaagtcaaag ctgcattttg tgcactgctt cctgcctgta gctgagggct gggctgggga 2280
gccccgttcc gcctcctccc gccgagtcag cagcagcagt gagctggacc tgccctcggg 2340
agaccactgc gaggctgggc tcctgcagct cgacgtgccc ctgctccgca cccagctccg 2400
cggctcccgc ctgctcgatg ccatgcgcat gtaccgccaa ggttaccctg accacatggt 2460
gttttccgag ttccgccgcc gctttgatgt cctggccccg cacctgacca agaaacacgg 2520
gcgtaactac atcgtggtgg atgaaaggcg ggcagtggag gagctgctgg agtgcttgga 2580
tctggagaag agcagctgct gcatgggcct gagccgggtg ttcttccggg cgggcacctt 2640
ggcacggctg gaggagcagc gggatgaaca aaccagcagg aacctaaccc tgttccaagc 2700
agcctgcagg ggctacctgg cccgccagca cttcaagaag agaaagatcc aggacctggc 2760
cattcgctgt gtacagaaga acatcaagaa gaacaaaggg gtgaaggact ggccctggtg 2820
gaagcttttt accacagtga ggcccctcat cgaagtacag ctgtcagagg agcagatccg 2880
gaacaaagac gaggagatcc agcagctgcg gagcaagctc gagaaggcgg agaaggagag 2940
gaacgagctg cggctcaaca gtgaccggct ggagagccgg atctcagagc tgacatcgga 3000
gctgacagat gagcgtaaca caggagagtc cgcctcccag ctgctggacg cggagacagc 3060
agagaggctc cgggctgaga aggagatgaa ggaactgcag acccagtacg atgcactgaa 3120
gaagcagatg gaggttatgg aaatggaggt gatggaggcc cgtctcatcc gggcagcgga 3180
gatcaacggg gaagtggatg atgatgatgc aggtggcgag tggcggctga agtatgagcg 3240
ggctgtgcgg gaggtggact tcaccaagaa acggctccag caggagtttg aggacaagct 3300
ggaggtggag cagcagaaca agaggcagct ggaacggcgg ctcggggacc tgcaggcaga 3360
tagtgaggag agtcagcggg ctctgcagca gctcaagaag aagtgccagc gactgacggc 3420
tgagctgcaa gacaccaagc tgcacctgga gggccagcag gtccgcaacc acgaactgga 3480
gaagaagcag aggaggtttg acagtgagct ctcgcaggca catgaggagg cccagcggga 3540
gaagctgcag cgggagaagc tgcagcggga gaaggacatg ctcctcgctg aggctttcag 3600
cctgaagcag caactagagg aaaaagacat ggacattgca gggttcaccc agaaggttgt 3660
gtctctagag gcagagctcc aggacatttc ttcccaagag tccaaggatg aggcttctct 3720
ggccaaggtc aagaaacagc tccgggacct ggaggccaaa gtcaaggatc aggaagaaga 3780
gctggatgag caggcaggga ccatccagat gctggaacag gccaagctgc gtctggagat 3840
ggagatggag cggatgagac agacccattc taaggagatg gagagtcggg atgaggaggt 3900
ggaggaggcc cggcagtcgt gtcagaagaa gttaaaacag atggaggtgc agctagagga 3960
agagtatgag gacaagcaga aggttctgcg agagaagcgg gagctggagg gcaagctcgc 4020
caccctcagc gaccaggtga accggcggga ctttgagtca gagaagcggc tgcggaagga 4080
cctgaagcgc accaaggccc tgctggcaga tgcccagctc atgctggacc acctgaagaa 4140
cagtgctccc agcaagcgag agattgccca gctcaagaac cagctggagg agtcagagtt 4200
cacctgtgcg gcagccgtga aagcacggaa agcaatggag gtggagatcg aagacctgca 4260
cctgcagatt gatgacatcg ccaaagccaa gacagcgctg gaggagcagc tgagccgcct 4320
tcagcgtgag aagaatgaga tccagaaccg gctggaggaa gatcaggaag acatgaacga 4380
attgatgaag aagcacaagg ctgccgtggc tcaggcttcc cgggacctgg ctcagataaa 4440
tgatctccaa gctcagctag aagaagccaa caaagagaag caggagctgc aggagaagct 4500
acaagccctc cagagccagg tggagttcct ggagcagtcc atggtggaca agtccctggt 4560
gagcaggcag gaagctagga tacgggagat ggagacacgc ctggagtttg aaaggacgca 4620
agtgaaacgg ctggagagcc tggctagccg tctcaaggaa aacatggaga agctgactga 4680
ggagcgggat cagcgcattg cagccgagaa ccgggagaag gaacagaaca agcggctaca 4740
gaggcagctc cgggacacca aggaggagat gggcgagctt gccaggaagg aggccgaggc 4800
gagccgcaag aagcacgaac tggagatgga tctagaaagc ctggaggctg ctaaccagag 4860
cctgcaggct gacctaaagt tggcattcaa gcgcatcggg gacctgcagg ctgccattga 4920
ggatgagatg gagagtgatg agaatgagga cctcatcaac agtttgcagg acatggtgac 4980
aaagtatcag aaaagaaaga ataaacttga gggagactct gatgtggact cggagctgga 5040
ggaccgtgtt gacggggtca agtcctggtt gtcaaaaaac aagggacctt ccaaggcagc 5100
ttctgatgat ggcagcttaa agagttccag ccccaccagc tactggaagt cccttgcccc 5160
tgatcggtca gatgatgagc acgaccctct cgacaacacc tccagaccgc gatactccca 5220
cagttatctg agtgacagcg acacagaggc caagctgacg gagactaacg catagcccag 5280
gggagtggtt ggcagccctc tcaccccagg gcctgtggct gcctgggcac ctctcccagg 5340
aagtggtggg gcaccggtct cccccacccg actgctgatc tgcatgggaa acaccctgac 5400
cttcttctgt caggggcact ttccaggcta tgggtgtctg atgtctccac gtggaagagg 5460
tgggggaaag aggagtttct gaagagaact ttttgctcct ctgtctcaaa atgccagact 5520
cttggcttct accctgtgtc accgtgggca gtggcaggtg gcctggcact gcatggagcc 5580
agcacgttga cctccctctc agctccctgc tcagggacgg tggacaggtt gcctactggg 5640
acactctagg ttgctgggtc catggggagg attgggggag gagaagcagt gccttccctc 5700
tcgtgtgggg tgggggctct ctcttcttgg tgcctgctgt ctttctactt tttaatttaa 5760
atacccaacc tctccatcac agctgcatcc ctgagagtgg gagggggctg tagtggtagc 5820
tggggctccc aagaacgact cgggaatgtc atctccatct tcacccttca gagagcagtc 5880
ctttctctgt gcagctggag acgctggtga ggagagccgg gtccaggttc ttaagaatga 5940
ggtgcggagg ggctctccgg tgctgctggg ctgggttgag caagcctacg cagacaagtg 6000

tgtgtgtgga ccatccgcac ctccagcccc caccccaccc tctttgtctc agcgtgttat 6060
gtgcaatgac ctatttaagg taaacccatt ccaactacag cagttcaggg ctgatccaag 6120
cactgcctcc ctcctgctct gtccaggtgg tctggaccat aaactcaact tgagagggaa 6180
ggcttggggt tgaggacttg tgatcagaaa aactgaagat ggaagttttg gccggtgctc 6240
attagacatg agtcctcact ctgtgtcctg agcccgtgtc attcttccaa cctccctgcc 6300
cccacacact tatcccagac acaacaccat gtggtctgga ggtcccagcc cccaccctaa 6360
aaaggttatc cctgagaact ccaccagact tgggagccca agtgcagtgc ctggtgctgc 6420
tcccatctgc cgcccccctt ctctcctgca attggtttgt actcactggg ctgtgctctc 6480
ccctgtttac ccgatgtatg gaaataaagg cccttttcct cctg 6524

<210> 42
<211> 1596
<212> PRT
<213> Homo sapiens

<400> 42
Met His Met Phe Lys Gly Cys Arg Arg Glu Asp Met Ala Pro His Ile
1               5                   10                  15

Tyr Ala Val Ala Gln Thr Ala Tyr Arg Ala Met Leu Met Ser Arg Gln
            20                  25                  30

Asp Gln Ser Ile Ile Leu Leu Gly Ser Ser Gly Ser Gly Lys Thr Thr
        35                  40                  45

Ser Cys Gln His Leu Val Gln Tyr Leu Ala Thr Ile Ala Gly Ile Ser
    50                  55                  60

Gly Asn Lys Val Phe Ser Val Glu Lys Trp Gln Ala Leu Tyr Thr Leu
65                  70                  75                  80

Leu Glu Ala Phe Gly Asn Ser Pro Thr Ile Ile Asn Gly Asn Ala Thr
                85                  90                  95

Arg Phe Ser Gln Ile Leu Ser Leu Asp Phe Asp Gln Ala Gly Gln Val
            100                 105                 110

Ala Ser Ala Ser Ile Gln Thr Met Leu Leu Glu Lys Leu Arg Val Ala
        115                 120                 125

Arg Arg Pro Ala Ser Glu Ala Thr Phe Asn Val Phe Tyr Tyr Leu Leu
    130                 135                 140

Ala Cys Gly Asp Gly Thr Leu Arg Thr Glu Leu His Leu Asn His Leu
145                 150                 155                 160

Ala Glu Asn Asn Val Phe Gly Ile Val Pro Leu Ala Lys Pro Glu Glu
                165                 170                 175

Lys Gln Lys Ala Ala Gln Gln Phe Ser Lys Leu Gln Ala Ala Met Lys
            180                 185                 190

Val Leu Gly Ile Ser Pro Asp Glu Gln Lys Ala Cys Trp Phe Ile Leu
        195                 200                 205

Ala Ala Ile Tyr His Leu Gly Ala Ala Gly Ala Thr Lys Glu Ala Ala
    210                 215                 220

Glu Ala Gly Arg Lys Gln Phe Ala Arg His Glu Trp Ala Gln Lys Ala
225                 230                 235                 240

Ala Tyr Leu Leu Gly Cys Ser Leu Glu Glu Leu Ser Ser Ala Ile Phe
                245                 250                 255

Lys His Gln His Lys Gly Gly Thr Leu Gln Arg Ser Thr Ser Phe Arg
            260                 265                 270

Gln Gly Pro Glu Glu Ser Gly Leu Gly Asp Gly Thr Gly Pro Lys Leu

275                 280                 285

Ser Ala Leu Glu Cys Leu Glu Gly Met Ala Ala Gly Leu Tyr Ser Glu
    290                 295                 300

Leu Phe Thr Leu Leu Val Ser Leu Val Asn Arg Ala Leu Lys Ser Ser
305                 310                 315                 320

Gln His Ser Leu Cys Ser Met Met Ile Val Asp Thr Pro Gly Phe Gln
                325                 330                 335

Asn Pro Glu Gln Gly Gly Ser Ala Arg Gly Ala Ser Phe Glu Glu Leu
            340                 345                 350

Cys His Asn Tyr Thr Gln Asp Arg Leu Gln Arg Leu Phe His Glu Arg
        355                 360                 365

Thr Phe Val Gln Glu Leu Glu Arg Tyr Lys Glu Glu Asn Ile Glu Leu
    370                 375                 380

Ala Phe Asp Asp Leu Glu Pro Pro Thr Asp Asp Ser Val Ala Ala Val
385                 390                 395                 400

Asp Gln Ala Ser His Gln Ser Leu Val Arg Ser Leu Ala Arg Thr Asp
                405                 410                 415

Glu Ala Arg Gly Leu Leu Trp Leu Leu Glu Glu Glu Ala Leu Val Pro
            420                 425                 430

Gly Ala Ser Glu Asp Thr Leu Leu Glu Arg Leu Phe Ser Tyr Tyr Gly
        435                 440                 445

Pro Gln Glu Gly Asp Lys Lys Gly Gln Ser Pro Leu Leu His Ser Ser
    450                 455                 460

Lys Pro His His Phe Leu Leu Gly His Ser His Gly Thr Asn Trp Val
465                 470                 475                 480

Glu Tyr Asn Val Thr Gly Trp Leu Asn Tyr Thr Lys Gln Asn Pro Ala
                485                 490                 495

Thr Gln Asn Val Pro Arg Leu Leu Gln Asp Ser Gln Lys Lys Ile Ile
            500                 505                 510

Ser Asn Leu Phe Leu Gly Arg Ala Gly Ser Ala Thr Val Leu Ser Gly
        515                 520                 525

Ser Ile Ala Gly Leu Glu Gly Gly Ser Gln Leu Ala Leu Arg Arg Ala
    530                 535                 540

Thr Ser Met Arg Lys Thr Phe Thr Thr Gly Met Val Ala Val Lys Lys
545                 550                 555                 560

Lys Ser Leu Cys Ile Gln Met Lys Leu Gln Val Asp Ala Leu Ile Asp
                565                 570                 575

Thr Ile Lys Lys Ser Lys Leu His Phe Val His Cys Phe Leu Pro Val
            580                 585                 590

Ala Glu Gly Trp Ala Gly Glu Pro Arg Ser Ala Ser Ser Arg Arg Val
        595                 600                 605

Ser Ser Ser Ser Glu Leu Asp Leu Pro Ser Gly Asp His Cys Glu Ala
    610                 615                 620

Gly Leu Leu Gln Leu Asp Val Pro Leu Leu Arg Thr Gln Leu Arg Gly
625                 630                 635                 640

Ser Arg Leu Leu Asp Ala Met Arg Met Tyr Arg Gln Gly Tyr Pro Asp

                645                 650                 655

His Met Val Phe Ser Glu Phe Arg Arg Arg Phe Asp Val Leu Ala Pro
            660                 665                 670

His Leu Thr Lys Lys His Gly Arg Asn Tyr Ile Val Val Asp Glu Arg
        675                 680                 685

Arg Ala Val Glu Glu Leu Leu Glu Cys Leu Asp Leu Glu Lys Ser Ser
    690                 695                 700

Cys Cys Met Gly Leu Ser Arg Val Phe Phe Arg Ala Gly Thr Leu Ala
705                 710                 715                 720

Arg Leu Glu Glu Gln Arg Asp Glu Gln Thr Ser Arg Asn Leu Thr Leu
                725                 730                 735

Phe Gln Ala Ala Cys Arg Gly Tyr Leu Ala Arg Gln His Phe Lys Lys
            740                 745                 750

Arg Lys Ile Gln Asp Leu Ala Ile Arg Cys Val Gln Lys Asn Ile Lys
        755                 760                 765

Lys Asn Lys Gly Val Lys Asp Trp Pro Trp Trp Lys Leu Phe Thr Thr
    770                 775                 780

Val Arg Pro Leu Ile Glu Val Gln Leu Ser Glu Glu Gln Ile Arg Asn
785                 790                 795                 800

Lys Asp Glu Glu Ile Gln Gln Leu Arg Ser Lys Leu Glu Lys Ala Glu
                805                 810                 815

Lys Glu Arg Asn Glu Leu Arg Leu Asn Ser Asp Arg Leu Glu Ser Arg
            820                 825                 830

Ile Ser Glu Leu Thr Ser Glu Leu Thr Asp Glu Arg Asn Thr Gly Glu
        835                 840                 845

Ser Ala Ser Gln Leu Leu Asp Ala Glu Thr Ala Glu Arg Leu Arg Ala
    850                 855                 860

Glu Lys Glu Met Lys Glu Leu Gln Thr Gln Tyr Asp Ala Leu Lys Lys
865                 870                 875                 880

Gln Met Glu Val Met Glu Met Glu Val Met Glu Ala Arg Leu Ile Arg
                885                 890                 895

Ala Ala Glu Ile Asn Gly Glu Val Asp Asp Asp Asp Ala Gly Gly Glu
            900                 905                 910

Trp Arg Leu Lys Tyr Glu Arg Ala Val Arg Glu Val Asp Phe Thr Lys
        915                 920                 925

Lys Arg Leu Gln Gln Glu Phe Glu Asp Lys Leu Glu Val Glu Gln Gln
    930                 935                 940

Asn Lys Arg Gln Leu Glu Arg Arg Leu Gly Asp Leu Gln Ala Asp Ser
945                 950                 955                 960

Glu Glu Ser Gln Arg Ala Leu Gln Gln Leu Lys Lys Lys Cys Gln Arg
                965                 970                 975

Leu Thr Ala Glu Leu Gln Asp Thr Lys Leu His Leu Glu Gly Gln Gln
            980                 985                 990

Val Arg Asn His Glu Leu Glu Lys Lys Gln Arg Arg Phe Asp Ser Glu
        995                 1000                1005

Leu Ser Gln Ala His Glu Glu Ala Gln Arg Glu Lys Leu Gln Arg Glu

1010                1015                1020

Lys Leu Gln Arg Glu Lys Asp Met Leu Leu Ala Glu Ala Phe Ser Leu
1025                1030                1035                1040

Lys Gln Gln Leu Glu Glu Lys Asp Met Asp Ile Ala Gly Phe Thr Gln
                1045                1050                1055

Lys Val Val Ser Leu Glu Ala Glu Leu Gln Asp Ile Ser Ser Gln Glu
            1060                1065                1070

Ser Lys Asp Glu Ala Ser Leu Ala Lys Val Lys Lys Gln Leu Arg Asp
        1075                1080                1085

Leu Glu Ala Lys Val Lys Asp Gln Glu Glu Glu Leu Asp Glu Gln Ala
    1090                1095                1100

Gly Thr Ile Gln Met Leu Glu Gln Ala Lys Leu Arg Leu Glu Met Glu
1105                1110                1115                1120

Met Glu Arg Met Arg Gln Thr His Ser Lys Glu Met Glu Ser Arg Asp
                1125                1130                1135

Glu Glu Val Glu Glu Ala Arg Gln Ser Cys Gln Lys Lys Leu Lys Gln
            1140                1145                1150

Met Glu Val Gln Leu Glu Glu Glu Tyr Glu Asp Lys Gln Lys Val Leu
        1155                1160                1165

Arg Glu Lys Arg Glu Leu Glu Gly Lys Leu Ala Thr Leu Ser Asp Gln
    1170                1175                1180

Val Asn Arg Arg Asp Phe Glu Ser Glu Lys Arg Leu Arg Lys Asp Leu
1185                1190                1195                1200

Lys Arg Thr Lys Ala Leu Leu Ala Asp Ala Gln Leu Met Leu Asp His
                1205                1210                1215

Leu Lys Asn Ser Ala Pro Ser Lys Arg Glu Ile Ala Gln Leu Lys Asn
            1220                1225                1230

Gln Leu Glu Glu Ser Glu Phe Thr Cys Ala Ala Ala Val Lys Ala Arg
        1235                1240                1245

Lys Ala Met Glu Val Glu Ile Glu Asp Leu His Leu Gln Ile Asp Asp
    1250                1255                1260

Ile Ala Lys Ala Lys Thr Ala Leu Glu Glu Gln Leu Ser Arg Leu Gln
1265                1270                1275                1280

Arg Glu Lys Asn Glu Ile Gln Asn Arg Leu Glu Glu Asp Gln Glu Asp
                1285                1290                1295

Met Asn Glu Leu Met Lys Lys His Lys Ala Ala Val Ala Gln Ala Ser
            1300                1305                1310

Arg Asp Leu Ala Gln Ile Asn Asp Leu Gln Ala Gln Leu Glu Glu Ala
        1315                1320                1325

Asn Lys Glu Lys Gln Glu Leu Gln Glu Lys Leu Gln Ala Leu Gln Ser
    1330                1335                1340

Gln Val Glu Phe Leu Glu Gln Ser Met Val Asp Lys Ser Leu Val Ser
1345                1350                1355                1360

Arg Gln Glu Ala Arg Ile Arg Glu Met Glu Thr Arg Leu Glu Phe Glu
                1365                1370                1375

Arg Thr Gln Val Lys Arg Leu Glu Ser Leu Ala Ser Arg Leu Lys Glu

1380                1385                1390

Asn Met Glu Lys Leu Thr Glu Glu Arg Asp Gln Arg Ile Ala Ala Glu
    1395                1400                1405

Asn Arg Glu Lys Glu Gln Asn Lys Arg Leu Gln Arg Gln Leu Arg Asp
1410                1415                1420

Thr Lys Glu Glu Met Gly Glu Leu Ala Arg Lys Glu Ala Glu Ala Ser
1425                1430                1435                1440

Arg Lys Lys His Glu Leu Glu Met Asp Leu Glu Ser Leu Glu Ala Ala
                1445                1450                1455

Asn Gln Ser Leu Gln Ala Asp Leu Lys Leu Ala Phe Lys Arg Ile Gly
            1460                1465                1470

Asp Leu Gln Ala Ala Ile Glu Asp Glu Met Glu Ser Asp Glu Asn Glu
        1475                1480                1485

Asp Leu Ile Asn Ser Leu Gln Asp Met Val Thr Lys Tyr Gln Lys Arg
    1490                1495                1500

Lys Asn Lys Leu Glu Gly Asp Ser Asp Val Asp Ser Glu Leu Glu Asp
1505                1510                1515                1520

Arg Val Asp Gly Val Lys Ser Trp Leu Ser Lys Asn Lys Gly Pro Ser
                1525                1530                1535

Lys Ala Ala Ser Asp Asp Gly Ser Leu Lys Ser Ser Ser Pro Thr Ser
            1540                1545                1550

Tyr Trp Lys Ser Leu Ala Pro Asp Arg Ser Asp Asp Glu His Asp Pro
        1555                1560                1565

Leu Asp Asn Thr Ser Arg Pro Arg Tyr Ser His Ser Tyr Leu Ser Asp
    1570                1575                1580

Ser Asp Thr Glu Ala Lys Leu Thr Glu Thr Asn Ala
1585                1590                1595


<210> 43
<211> 1011
<212> DNA
<213> Homo sapiens

<400> 43
ccccgcctct cccgctatgt accgaccgcg agcccgggcg gctcccgagg gcagggtccg 60
gggctgcgcg gtgcccggca ccgtgctcct gctgctcgcc tacctggctt acctggcgct 120
gggcaccggc gtgttctgga cgctggaggg ccgcgcggcg caggactcca gccgcagctt 180
ccagcgcgac aagtgggagc tgttgcagaa cttcacgtgt ctggaccgcc cggcgctgga 240
ctcgctgatc cgggatgtcg tccaagcata caaaaacgga gccagcctcc tcagcaacac 300
caccagcatg gggcgctggg agctcgtggg ctccttcttc ttttctgtgt ccaccatctt 360
ctttgccctt gtggggatcc cactcaacct cgtggtgctc aaccgactgg ggcatctcat 420
gcagcaggga gtaaaccact gggccagcag gctggggggc acctggcagg atcctgacaa 480
ggcgcggtgg ctggcgggct ctggcgccct cctctcgggc ctcctgctct tcctgctgct 540
gccaccgctg ctcttctccc acatggaggg ctggagctac acagagggct tctacttcgc 600
cttcatcacc ctcagcaccg tgggcttcgg cgactacgtg attggaatga acccctccca 660
gaggtaccca ctgtggtaca agaacatggt gtccctgtgg atcctctttg ggatggcatg 720
gctggccttg atcatcaaac tcatcctctc ccagctggag acgccaggga gggtatgttc 780
ctgctgccac cacagctcta aggaagactt caagtcccaa agctggagac agggacctga 840
ccgggagcca gagtcccact ccccacagca aggatgctat ccagagggac ccatgggaat 900
catacagcat ctggaacctt ctgctcacgc tgcaggctgt ggcaaggaca gctagttata 960
ctccattctt tggtcgtcgt cctcggtagc aagacccctg attttaagct t          1011


<210> 44
<211> 312

<212> PRT
<213> Homo sapiens

<400> 44
Met Tyr Arg Pro Arg Ala Arg Ala Ala Pro Glu Gly Arg Val Arg Gly
1               5                   10                  15

Cys Ala Val Pro Gly Thr Val Leu Leu Leu Leu Ala Tyr Leu Ala Tyr
            20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Asp
65                  70                  75                  80

Val Val Gln Ala Tyr Lys Asn Gly Ala Ser Leu Leu Ser Asn Thr Thr
                85                  90                  95

Ser Met Gly Arg Trp Glu Leu Val Gly Ser Phe Phe Phe Ser Val Ser
            100                 105                 110

Thr Ile Phe Phe Ala Leu Val Gly Ile Pro Leu Asn Leu Val Val Leu
        115                 120                 125

Asn Arg Leu Gly His Leu Met Gln Gln Gly Val Asn His Trp Ala Ser
    130                 135                 140

Arg Leu Gly Gly Thr Trp Gln Asp Pro Asp Lys Ala Arg Trp Leu Ala
145                 150                 155                 160

Gly Ser Gly Ala Leu Leu Ser Gly Leu Leu Leu Phe Leu Leu Leu Pro
                165                 170                 175

Pro Leu Leu Phe Ser His Met Glu Gly Trp Ser Tyr Thr Glu Gly Phe
            180                 185                 190

Tyr Phe Ala Phe Ile Thr Leu Ser Thr Val Gly Phe Gly Asp Tyr Val
        195                 200                 205

Ile Gly Met Asn Pro Ser Gln Arg Tyr Pro Leu Trp Tyr Lys Asn Met
    210                 215                 220

Val Ser Leu Trp Ile Leu Phe Gly Met Ala Trp Leu Ala Leu Ile Ile
225                 230                 235                 240

Lys Leu Ile Leu Ser Gln Leu Glu Thr Pro Gly Arg Val Cys Ser Cys
                245                 250                 255

Cys His His Ser Ser Lys Glu Asp Phe Lys Ser Gln Ser Trp Arg Gln
            260                 265                 270

Gly Pro Asp Arg Glu Pro Glu Ser His Ser Pro Gln Gln Gly Cys Tyr
        275                 280                 285

Pro Glu Gly Pro Met Gly Ile Ile Gln His Leu Glu Pro Ser Ala His
    290                 295                 300

Ala Ala Gly Cys Gly Lys Asp Ser
305                 310


<210> 45
<211> 1083
<212> DNA
<213> Homo sapiens

<400> 45
ccccgcctct cccgctatgt accgaccgcg agcccgggcg gctcccgagg gcaggtccgg 60
gggctgcgcg gtgcccggca ccgtgctcct gctgctcgcc tacctggctt acctggcgct 120
gggcaccggc gtgttctgga cgctggaggg ccgcgcggcg caggactcca gccgcagctt 180
ccagcgcgac aagtgggagc tgttgcagaa cttcacgtgt ctggaccgcc cggcgctgga 240
ctcgctgatc cggcctcttc cccaggatgt cgtccaagca tacaaaaacg gagccagcct 300
cctcagcaac accaccagca tggggcgctg ggagctcgtg ggctccttct tcttttctgt 360
gtccaccatc accaccattg gctatggcaa cctgagcccc aacacgatgg ctgcccgcct 420
cttctgcatc ttctttgccc ttgtggggat cccactcaac ctcgtggtgc tcaaccgact 480
ggggcatctc atgcagcagg gagtaaacca ctgggccagc aggctggggg gcacctggca 540
ggatcctgac aaggcgcggt ggctggcggg ctctggcgcc ctcctctcgg gcctcctgct 600
cttcctgctg ctgccaccgc tgctcttctc ccacatggag ggctggagct acacagaggg 660
cttctacttc gccttcatca ccctcagcac cgtgggcttc ggcgactacg tgattggaat 720
gaacccctcc cagaggtacc cactgtggta caagaacatg gtgtccctgt ggatcctctt 780
tgggatggca tggctggcct tgatcatcaa actcatcctc tcccagctgg agacgccagg 840
gagggtatgt tcctgctgcc accacagctc taaggaagac ttcaagtccc aaagctggag 900
acagggacct gaccgggagc cagagtccca ctccccacag caaggatgct atccagaggg 960
acccatggga atcatacagc atctggaacc ttctgctcac gctgcaggct gtggcaagga 1020
cagctagtta tactccattc tttggtcgtc gtcctcggta gcaagacccc tgattttaag 1080
ctt 1083

<210> 46
<211> 336
<212> PRT
<213> Homo sapiens

<400> 46
Met Tyr Arg Pro Arg Ala Arg Ala Ala Pro Glu Gly Arg Val Arg Gly
1               5                   10                  15

Cys Ala Val Pro Gly Thr Val Leu Leu Leu Leu Ala Tyr Leu Ala Tyr
            20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Pro
65                  70                  75                  80

Leu Pro Gln Asp Val Val Gln Ala Tyr Lys Asn Gly Ala Ser Leu Leu
                85                  90                  95

Ser Asn Thr Thr Ser Met Gly Arg Trp Glu Leu Val Gly Ser Phe Phe
            100                 105                 110

Phe Ser Val Ser Thr Ile Thr Thr Ile Gly Tyr Gly Asn Leu Ser Pro
        115                 120                 125

Asn Thr Met Ala Ala Arg Leu Phe Cys Ile Phe Phe Ala Leu Val Gly
    130                 135                 140

Ile Pro Leu Asn Leu Val Val Leu Asn Arg Leu Gly His Leu Met Gln
145                 150                 155                 160

Gln Gly Val Asn His Trp Ala Ser Arg Leu Gly Gly Thr Trp Gln Asp
                165                 170                 175

Pro Asp Lys Ala Arg Trp Leu Ala Gly Ser Gly Ala Leu Leu Ser Gly
            180                 185                 190

Leu Leu Leu Phe Leu Leu Leu Pro Pro Leu Leu Phe Ser His Met Glu
        195                 200                 205

Gly Trp Ser Tyr Thr Glu Gly Phe Tyr Phe Ala Phe Ile Thr Leu Ser

210                 215                 220

Thr Val Gly Phe Gly Asp Tyr Val Ile Gly Met Asn Pro Ser Gln Arg
225                 230                 235                 240

Tyr Pro Leu Trp Tyr Lys Asn Met Val Ser Leu Trp Ile Leu Phe Gly
                245                 250                 255

Met Ala Trp Leu Ala Leu Ile Ile Lys Leu Ile Leu Ser Gln Leu Glu
            260                 265                 270

Thr Pro Gly Arg Val Cys Ser Cys Cys His His Ser Ser Lys Glu Asp
        275                 280                 285

Phe Lys Ser Gln Ser Trp Arg Gln Gly Pro Asp Arg Glu Pro Glu Ser
    290                 295                 300

His Ser Pro Gln Gln Gly Cys Tyr Pro Glu Gly Pro Met Gly Ile Ile
305                 310                 315                 320

Gln His Leu Glu Pro Ser Ala His Ala Ala Gly Cys Gly Lys Asp Ser
                325                 330                 335

<210> 47
<211> 1256
<212> DNA
<213> Homo sapiens

<400> 47
ccccgcctct cccgctatgt accgaccgcg agcccgggcg gctcccgagg gcagggtccg 60
gggctgcgcg gtgcccggca ccgtgctcct gctgctcgcc tacctggctt acctggcgct 120
gggcaccggc gtgttctgga cgctggaggg ccgcgcggcg caggactcca gccgcagctt 180
ccagcgcgac aagtgggagc tgttgcagaa cttcacgtgt ctggaccgcc cggcgctgga 240
ctcgctgatc cgggatgtcg tccaagcata caaaaacgga gccagcctcc tcagcaacac 300
caccagcatg gggcgctggg agctcgtggg ctccttcttc ttttctgtgt ccaccatctt 360
ctttgccctt gtggggatcc cactcaacct cgtggtgctc aaccgactgg ggcatctcat 420
gcagcaggga gtaaaccact gggccagcag gctggggggc acctggcagg atcctgacaa 480
ggcgcggtgg ctggcgggct ctggcgccct cctctcgggc ctcctgctct tcctgctgct 540
gccaccgctg ctcttctccc acatggaggg ctggagctac acagagggct tctacttcgc 600
cttcatcacc ctcagcaccg tgggcttcgg cgactacgtg attggaatga acccctccca 660
gaggtaccca ctgtggtaca agaacatggt gtccctgtgg atcctctttg ggatggcatg 720
gctggccttg atcatcaaac tcatcctctc ccagctggag acgccaggga gggtatgttc 780
ctgctgccac cacagctcta aggaagactt caagtcccaa agctggagac agggacctga 840
ccgggagcca gagtcccact ccccacagca aggatgctat ccagagggac ccatgggaat 900
catacagcat ctggaacctt ctgctcacgc tgcaggctgt ggcaaggaca gctagttata 960
ctccattctt tggtcgtcgt cctcggtagc aagacccctg attttaagct ttgcacatgt 1020
ccacccaaac taaagactac attttccatc caccctagag gctgggtgca gctatatgat 1080
taattctgcc caatagggta tacagagaca tgtcctgggt gacatgggat gtgactttcg 1140
ggtgtcgggg cagcatgccc ttctccccca cttccttact ttagcgggct gcaatgccgc 1200
cgatatgatg gctgggagct ctggcagcca tacggcacca tgaagtagcg gcaatg     1256

<210> 48
<211> 312
<212> PRT
<213> Homo sapiens

<400> 48
   Met Tyr Arg Pro Arg Ala Arg Ala Ala Pro Glu Gly Arg Val Arg Gly
   1               5                   10                  15
   Cys Ala Val Pro Gly Thr Val Leu Leu Leu Leu Ala Tyr Leu Ala Tyr
               20                  25                  30
   Leu Ala Leu Gly Thr Gly Val Phe Trp Thr Leu Glu Gly Arg Ala Ala
   35 40 45
   Gln Asp Ser Ser Arg Ser Phe Gln Arg Asp Lys Trp Glu Leu Leu Gln
   50 55 60
   Asn Phe Thr Cys Leu Asp Arg Pro Ala Leu Asp Ser Leu Ile Arg Asp
   65 70 75 80
   Val Val Gln Ala Tyr Lys Asn Gly Ala Ser Leu Leu Ser Asn Thr Thr
   85 90 95
   Ser Met Gly Arg Trp Glu Leu Val Gly Ser Phe Phe Phe Ser Val Ser
   100 105 110
   Thr Ile Phe Phe Ala Leu Val Gly Ile Pro Leu Asn Leu Val Val Leu
   115 120 125
   Asn Arg Leu Gly His Leu Met Gln Gln Gly Val Asn His Trp Ala Ser
   130 135 140
   Arg Leu Gly Gly Thr Trp Gln Asp Pro Asp Lys Ala Arg Trp Leu Ala
   145 150 155 160
   Gly Ser Gly Ala Leu Leu Ser Gly Leu Leu Leu Phe Leu Leu Leu Pro
   165 170 175
   Pro Leu Leu Phe Ser His Met Glu Gly Trp Ser Tyr Thr Glu Gly Phe
   180 185 190
   Tyr Phe Ala Phe Ile Thr Leu Ser Thr Val Gly Phe Gly Asp Tyr Val
   195 200 205
   Ile Gly Met Asn Pro Ser Gln Arg Tyr Pro Leu Trp Tyr Lys Asn Met
   210 215 220
   Val Ser Leu Trp Ile Leu Phe Gly Met Ala Trp Leu Ala Leu Ile Ile
   225 230 235 240
   Lys Leu Ile Leu Ser Gln Leu Glu Thr Pro Gly Arg Val Cys Ser Cys
   245 250 255
   Cys His His Ser Ser Lys Glu Asp Phe Lys Ser Gln Ser Trp Arg Gln
   260 265 270
   Gly Pro Asp Arg Glu Pro Glu Ser His Ser Pro Gln Gln Gly Cys Tyr
   275 280 285
   Pro Glu Gly Pro Met Gly Ile Ile Gln His Leu Glu Pro Ser Ala His
   290 295 300
   Ala Ala Gly Cys Gly Lys Asp Ser
   305 310

<210> 49
<211> 1260
<212> DNA
<213> Homo sapiens

<400> 49
ctcaagcagg cgtttgcgag aggagatacg agctggacgc ctggcccttc cctcccaccg 60
ggtcctagtc caccgctccc ggcgccggct ccccgcctct cccgctatgt accgaccgcg 120
agcccgggcg gctcccgagg gcagggtccg gggctgcgcg gtgcccggca ccgtgctcct 180
gctgctcgcc tacctggctt acctggcgct gggcaccggc gtgttctgga cgctggaggg 240
ccgcgcggcg caggactcca gccgcagctt ccagcgcgac aagtgggagc tgttgcagaa 300
cttcacgtgt ctggaccgcc cggcgctgga ctcgctgatc cgggatgtcg tccaagcata 360
caaaaacgga gccagcctcc tcagcaacac caccagcatg gggcgctggg agctcgtggg 420
ctccttcttc ttttctgtgt ccaccatcac caccattggc tatggcaacc tgagccccaa 480
cacgatggct gcccgcctct tctgcatctt ctttgccctt gtggggatcc cactcaacct 540

cgtggtgctc aaccgactgg ggcatctcat gcagcaggga gtaaaccact gggccagcag 600
gctgggggc acctggcagg atcctgacaa ggcgcggtgg ctggcgggct ctggcgccct 660
cctctcgggc ctcctgctct tcctgctgct gccaccgctg ctcttctccc acatggaggg 720
ctggagctac acagagggct tctacttcgc cttcatcacc ctcagcaccg tgggcttcgg 780
cgactacgtg attggaatga acccctccca gaggtaccca ctgtggtaca agaacatggt 840
gtccctgtgg atcctctttg ggatggcatg gctggccttg atcatcaaac tcatcctctc 900
ccagctggag acaaacccat cctctcccag ctggagacaa cagggagggt atgttcctgc 960
tgccaccaca gctctaagga agacttcaag tcccaaagct ggagacaggg acctgaccgg 1020
gagccagagt cccactcccc acagcaagga tgctatccag agggacccat gggaatcata 1080
cagcatctgg aaccttctgc tcacgctgca ggctgtggca aggacagcta gttatactcc 1140
attctttggt cgtcgtcctc ggtagcaaga cccctgattt taagctttgc acatgtccac 1200
ccaaactaaa gactacattt tccatccacc ctagaggctg ggtgcagcta tatgattaat 1260

<210> 50
<211> 352
<212> PRT
<213> Homo sapiens

<400> 50
Met Tyr Arg Pro Arg Ala Arg Ala Ala Pro Glu Gly Arg Val Arg Gly
1               5                   10                  15

Cys Ala Val Pro Gly Thr Val Leu Leu Leu Leu Ala Tyr Leu Ala Tyr
            20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Asp
65                  70                  75                  80

Val Val Gln Ala Tyr Lys Asn Gly Ala Ser Leu Leu Ser Asn Thr Thr
                85                  90                  95

Ser Met Gly Arg Trp Glu Leu Val Gly Ser Phe Phe Phe Ser Val Ser
            100                 105                 110

Thr Ile Thr Thr Ile Gly Tyr Gly Asn Leu Ser Pro Asn Thr Met Ala
        115                 120                 125

Ala Arg Leu Phe Cys Ile Phe Phe Ala Leu Val Gly Ile Pro Leu Asn
    130                 135                 140

Leu Val Val Leu Asn Arg Leu Gly His Leu Met Gln Gln Gly Val Asn
145                 150                 155                 160

His Trp Ala Ser Arg Leu Gly Gly Thr Trp Gln Asp Pro Asp Lys Ala
                165                 170                 175

Arg Trp Leu Ala Gly Ser Gly Ala Leu Leu Ser Gly Leu Leu Leu Phe
            180                 185                 190

Leu Leu Leu Pro Pro Leu Leu Phe Ser His Met Glu Gly Trp Ser Tyr
        195                 200                 205

Thr Glu Gly Phe Tyr Phe Ala Phe Ile Thr Leu Ser Thr Val Gly Phe
    210                 215                 220

Gly Asp Tyr Val Ile Gly Met Asn Pro Ser Gln Arg Tyr Pro Leu Trp
225                 230                 235                 240

Tyr Lys Asn Met Val Ser Leu Trp Ile Leu Phe Gly Met Ala Trp Leu
                245                 250                 255

Ala Leu Ile Ile Lys Leu Ile Leu Ser Gln Leu Glu Thr Asn Pro Ser

            260                 265                 270

Ser Pro Ser Trp Arg Gln Gln Gly Gly Tyr Val Pro Ala Ala Thr Thr
        275                 280                 285

Ala Leu Arg Lys Thr Ser Ser Pro Lys Ala Gly Asp Arg Asp Leu Thr
    290                 295                 300

Gly Ser Gln Ser Pro Thr Pro His Ser Lys Asp Ala Ile Gln Arg Asp
305                 310                 315                 320

Pro Trp Glu Ser Tyr Ser Ile Trp Asn Leu Leu Leu Thr Leu Gln Ala
                325                 330                 335

Val Ala Arg Thr Ala Ser Tyr Thr Pro Phe Phe Gly Arg Arg Pro Arg
            340                 345                 350

<210> 51
<211> 627
<212> DNA
<213> Homo sapiens

<400> 51
tctgctgact ctcaactttt cttggaaaaa atggatcatt cccatcatac ggggatgagc 60
tgtatggact ccaacagtac catgcaacgt cctcaccatc acccaaccac ttcagcctca 120
cactcccgtg gtggaggaga cagcaacatg atgatgatga tgcctatgac cttctacttt 180
ggctttaaga atgtggaact actgttttcc agtttggtga tcaatacagc tggagaaatg 240
gctggagctt ttgtggcagt gcttttgcta caattccatg cctgtcccat agcccaagag 300
agcctgctgt gtaagtcaca agtcagcttt tgctacaatt ccatgcctgt cccaggacca 360
aatggaacca tccttatgga gacacacaaa actgttgggc agcagatgct gagctttcct 420
cacctcctgc aaacagtgct gcacatcatc caggtggtca taagctacct cctcatgctc 480
atcttcatga cctacaatgg gtacctctgc attgcagtaa cagcaggggc cggtacaaga 540
tacttcctct tcagctggaa gaaggcagtg gtagtggaca tcacagagta ttgccattga 600
cgtcaaactc tatggcatgg ccttatc 627

<210> 52
<211> 189
<212> PRT
<213> Homo sapiens

<400> 52
Met Asp His Ser His His Thr Gly Met Ser Cys Met Asp Ser Asn Ser
1               5                   10                  15

Thr Met Gln Arg Pro His His His Pro Thr Thr Ser Ala Ser His Ser
            20                  25                  30

Arg Gly Gly Gly Asp Ser Asn Met Met Met Met Met Pro Met Thr Phe
        35                  40                  45

Tyr Phe Gly Phe Lys Asn Val Glu Leu Leu Phe Ser Ser Leu Val Ile
    50                  55                  60

Asn Thr Ala Gly Glu Met Ala Gly Ala Phe Val Ala Val Leu Leu Leu
65                  70                  75                  80

Gln Phe His Ala Cys Pro Ile Ala Gln Glu Ser Leu Leu Cys Lys Ser
                85                  90                  95

Gln Val Ser Phe Cys Tyr Asn Ser Met Pro Val Pro Gly Pro Asn Gly
            100                 105                 110

Thr Ile Leu Met Glu Thr His Lys Thr Val Gly Gln Gln Met Leu Ser
        115                 120                 125

Phe Pro His Leu Leu Gln Thr Val Leu His Ile Ile Gln Val Val Ile
    130                 135                 140

Ser Tyr Leu Leu Met Leu Ile Phe Met Thr Tyr Asn Gly Tyr Leu Cys
145                 150                 155                 160

Ile Ala Val Thr Ala Gly Ala Gly Thr Arg Tyr Phe Leu Phe Ser Trp
                165                 170                 175

Lys Lys Ala Val Val Val Asp Ile Thr Glu Tyr Cys His
            180                 185


<210> 53
<211> 1478
<212> DNA
<213> Homo sapiens

<400> 53
actccactcc tgcctccacc atgtccatca gggtgaccca gaagtcctac aaggtgtcca 60
cctctggccc ccaggccttt agcagccgct tttacacgaa tggccctggt gcccacatca 120
gctcttcaag cctctcccga gtgagcagca gcagcttccg gggtggcctg ggcagaggct 180
atggtggggc cagcggcata ggaggcatca ccactgtcac gttcaaccag agcctgctga 240
gccctgttaa cctggaggta gatcccaata tccaggccat gcacacccag gagaaggagc 300
agatcaagac cctcaacaac aagtttgcct ccttcatcga caaggtacgg ttcctgcagc 360
agaagaacaa gatgctggag accaagtgga gcctcctgca gcagcagaag atggctcgga 420
tcaacgtatt tgagagctac atgaacaacc ttaggcggca gctggaggct ctgggccagg 480
agaagctgaa gctggaggcg gagcttggca atatgcaggg gctggtggag gacttcaaga 540
acaagtatga ggatgagatc aataagcgta cagaaacgga gaatgaattt gtcctcatca 600
agaaggacat ggatgaagct tacatgaaca aggcagagct ggagtctcgc ctggaagggc 660
tgactgacga gatcaacttc ctcaggcaac tgcatgaaga ggagatccag gagctgcagt 720
cccagatctc gggcacgtct gcggtgctgt ccatggacaa cagcctctcc ctggacatgg 780
acagcatcat cgctgaggtc aaggcacagg aggaggagat cgccaaccgc agctgggctg 840
aggctgagag catgtaccag atcaagtatg cagagctgca gacgctggct ggcaagcacg 900
gggatgacct gcggtgtaca aagactaaga tctccgagat gaaccggaac atcagccggc 960
tccaggctga gattgagggc ctcaaaggcc agagggcttc cctggaggcc cccatcgcag 1020
ataccgagca gcgtggggag ctggccgtta aggatgccag cgccaagcgg tcggagctgg 1080
aggccgccct gcagcgggcc aagcaggaca tggcgcagca gctgcgtgag taccaggagc 1140
tgatgaacgt caaactggcc ctggacatgg agatcgccac ctacaggaag ctgctggagg 1200
gcgaggagag cgcccggctg gagtctggga tgcagaacac gagtatccat aggaagacca 1260
ccagcggcta tgcaggtggt ctgagttccg cctatggggg cctcacaagc cctggcctca 1320
gctatggcct aagctccagc tttggctctg tcgcgggctc cagctcattc agccgcaccg 1380
gctccgccag ggccatggtt gtgcagaaga ttgagacccg cgatgggaag ctggtatccg 1440
agtcctctga cgtcctgccc aagtgaagag ctgcggca                         1478


<210> 54
<211> 481
<212> PRT
<213> Homo sapiens

<400> 54
Met Ser Ile Arg Val Thr Gln Lys Ser Tyr Lys Val Ser Thr Ser Gly
1               5                   10                  15

Pro Gln Ala Phe Ser Ser Arg Phe Tyr Thr Asn Gly Pro Gly Ala His
            20                  25                  30

Ile Ser Ser Ser Ser Leu Ser Arg Val Ser Ser Ser Ser Phe Arg Gly
        35                  40                  45

Gly Leu Gly Arg Gly Tyr Gly Gly Ala Ser Gly Ile Gly Gly Ile Thr
    50                  55                  60

Thr Val Thr Phe Asn Gln Ser Leu Leu Ser Pro Val Asn Leu Glu Val
65                  70                  75                  80

Asp Pro Asn Ile Gln Ala Met His Thr Gln Glu Lys Glu Gln Ile Lys

                85                  90                  95

Thr Leu Asn Asn Lys Phe Ala Ser Phe Ile Asp Lys Val Arg Phe Leu
            100                 105                 110

Gln Gln Lys Asn Lys Met Leu Glu Thr Lys Trp Ser Leu Leu Gln Gln
        115                 120                 125

Gln Lys Met Ala Arg Ile Asn Val Phe Glu Ser Tyr Met Asn Asn Leu
    130                 135                 140

Arg Arg Gln Leu Glu Ala Leu Gly Gln Glu Lys Leu Lys Leu Glu Ala
145                 150                 155                 160

Glu Leu Gly Asn Met Gln Gly Leu Val Glu Asp Phe Lys Asn Lys Tyr
                165                 170                 175

Glu Asp Glu Ile Asn Lys Arg Thr Glu Thr Glu Asn Glu Phe Val Leu
            180                 185                 190

Ile Lys Lys Asp Met Asp Glu Ala Tyr Met Asn Lys Ala Glu Leu Glu
        195                 200                 205

Ser Arg Leu Glu Gly Leu Thr Asp Glu Ile Asn Phe Leu Arg Gln Leu
    210                 215                 220

His Glu Glu Glu Ile Gln Glu Leu Gln Ser Gln Ile Ser Gly Thr Ser
225                 230                 235                 240

Ala Val Leu Ser Met Asp Asn Ser Leu Ser Leu Asp Met Asp Ser Ile
                245                 250                 255

Ile Ala Glu Val Lys Ala Gln Glu Glu Glu Ile Ala Asn Arg Ser Trp
            260                 265                 270

Ala Glu Ala Glu Ser Met Tyr Gln Ile Lys Tyr Ala Glu Leu Gln Thr
        275                 280                 285

Leu Ala Gly Lys His Gly Asp Asp Leu Arg Cys Thr Lys Thr Lys Ile
    290                 295                 300

Ser Glu Met Asn Arg Asn Ile Ser Arg Leu Gln Ala Glu Ile Glu Gly
305                 310                 315                 320

Leu Lys Gly Gln Arg Ala Ser Leu Glu Ala Pro Ile Ala Asp Thr Glu
                325                 330                 335

Gln Arg Gly Glu Leu Ala Val Lys Asp Ala Ser Ala Lys Arg Ser Glu
            340                 345                 350

Leu Glu Ala Ala Leu Gln Arg Ala Lys Gln Asp Met Ala Gln Gln Leu
        355                 360                 365

Arg Glu Tyr Gln Glu Leu Met Asn Val Lys Leu Ala Leu Asp Met Glu
    370                 375                 380

Ile Ala Thr Tyr Arg Lys Leu Leu Glu Gly Glu Glu Ser Ala Arg Leu
385                 390                 395                 400

Glu Ser Gly Met Gln Asn Thr Ser Ile His Arg Lys Thr Thr Ser Gly
                405                 410                 415

Tyr Ala Gly Gly Leu Ser Ser Ala Tyr Gly Gly Leu Thr Ser Pro Gly
            420                 425                 430

Leu Ser Tyr Gly Leu Ser Ser Ser Phe Gly Ser Val Ala Gly Ser Ser
        435                 440                 445

Ser Phe Ser Arg Thr Gly Ser Ala Arg Ala Met Val Val Gln Lys Ile

450                 455                 460

Glu Thr Arg Asp Gly Lys Leu Val Ser Glu Ser Ser Asp Val Leu Pro
465                 470                 475                 480

Lys

<210> 55
<211> 2907
<212> DNA
<213> Homo sapiens

<400> 55
ggccgctgaa tgctagctag aattcagcgg ccgctgaatt ctaggcgctg ccgagggaat 60
gcgcgcagct cacaggccct gggagtgagc tggtgcccgg cgacctggca cccgcgcctg 120
gatatggggc gtctacatcg tcccaggagc agcaccagct acaggaacct gccgcatctg 180
tttctgtttt tcctcttcgt gggacccttc agctgcctcg ggagttacag ccgggccacc 240
gagcttctgt acagcctaaa cgagggacta cccgcggggg tgctcatcgg cagcctggcc 300
gaggacctgc ggctgctgcc caggtctgca gggaggccgg acccgcagtc gcagctgcca 360
gagcgcaccg gtgctgagtg gaacccccct ctctccttca gcctggcctc ccggggactg 420
agtggccagt acgtgaccct agacaaccgc tctggggagc tgcacacttc agctcaggag 480
atcgacaggg aggccctgtg tgttgaaggg ggtggaggga ctgcgtggag cggcagcgtt 540
tccatctcct cctctccttc tgactcttgt cttttgctgc tggatgtgct tgtcctgcct 600
caggaatact tcaggtttgt gaaggtgaag atcgccatca gagacatcaa tgacaacgcc 660
ccgcagttcc ctgtttccca gatctcggtg tgggtcccgg aaaatgcacc tgtaaacacc 720
cgactggcca tagagcatcc tgctgtggac ccagatgtag gcattaatgg ggtacagacc 780
tatcgcttac tggactacca tggtatgttc accctggacg tggaggagaa tgagaatggg 840
gagcgcaccc cctacctaat tgtcatgggt gctttggaca gggaaaccca ggaccagtat 900
gtgagcatca tcatagctga ggatggtggg tctccaccac ttttgggcag tgccactctc 960
accattggca tcagtgacat taatgacaat tgccctctct tcacagactc acaaatcaat 1020
gtcactgtgt atgggaatgc tacagtgggc accccaattg cagctgtcca ggctgtggat 1080
aaagacttgg ggaccaatgc tcaaattact tattcttaca gtcagaaagt tccacaagca 1140
tctaaggatt tatttcacct ggatgaaaac actggagtca ttaaactttt cagtaagatt 1200
ggaggaagtg ttctggagtc ccacaagctc accatccttg ctaatggacc aggctgcatc 1260
cctgctgtaa tcactgctct tgtgtccatt attaaagtta ttttcagacc ccctgaaatt 1320
gtccctcgtt acatagcaaa cgagatagat ggtgttgttt atctgaaaga actggaaccc 1380
gttaacactc ccattgcgtt tttcaccata agagatccag aaggtaaata caaggttaac 1440
tgctacctgg atggtgaagg gccgtttagg ttatcacctt acaaaccata caataatgaa 1500
tatttactag agaccacaaa acctatggac tatgagctac agcagttcta tgaagtagct 1560
gtggtggctt ggaactctga gggatttcat gtcaaaaggg tcattaaagt gcaactttta 1620
gatgacaatg ataatgctcc aattttcctt caacccttaa tagaactaac catcgaagag 1680
aacaactcac ccaatgcctt tttgactaag ctgtatgcta cagatgccga cagcgaggag 1740
agaggccaag tttcatattt tctgggacct gatgctccat catatttttc cttagacagt 1800
gtcacaggaa ttctgacagt ttctactcag ctggaccgag aagagaaaga aaagtacaga 1860
tacactgtca gagctgttga ctgtgggaag ccacccagag aatcagtagc cactgtggcc 1920
ctcacagtgt tggataaaaa tgacaacagt cctcggttta tcaacaagga cttcagcttt 1980
tttgtgcctg aaaactttcc aggctatggt gagattggag taattagtgt aacagatgct 2040
gacgctggac gaaatggatg ggtcgccctc tctgtggtga accagagtga tatttttgtc 2100
atagatacag gaaagggtat gctgagggct aaagtctctt tggacagaga gcagcaaagc 2160
tcctatactt tgtgggttga agctgttgat gggggtgagc ctgccctctc ctctacagca 2220
aaaatcacaa ttctccttct agatatcaat gacaaccctc ctcttgtttt gtttcctcag 2280
tctaatatgt cttatctgtt agtactgcct tctactctgc caggctcccc ggttacagaa 2340
gtctatgctg tcgacaaaga cacaggcatg aatgctgtca tagcttacag catcataggg 2400
agaagaggtc ctaggcctga gtccttcagg attgacccta aaactggcaa cattactttg 2460
gaagaggcat tgctgcagac agattatggg ctccatcgct tactggtgaa agtgagtgat 2520
catggttatc ccgagcctct ccactccaca gtcatggtga acctatttgt caatgacact 2580
gtcagtaatg agagttacat tgagagtctt ttaagaaaag aaccagagat taatatagag 2640
gagaaagaac cacaaatctc aatagaaccg actcatagga aggtagaatc tgtgtcttgt 2700
atgcccacct tagtagctct gtctgtaata agcttgggtt ccatcacact ggtcacaggg 2760
atgggcatat acatctgttt aaggaaaggg gaaaagcatc ccagggaaga tgaaaatttg 2820
gaagtacaga ttccactgaa aggaaaaatt gacttgcata tgcgagagag aaagccaatg 2880
gatatttcta atatttgata tttcatg 2907

<210> 56
<211> 962
<212> PRT

<213> Homo sapiens

<400> 56
Met Leu Ala Arg Ile Gln Arg Pro Leu Asn Ser Arg Arg Cys Arg Gly
1               5                   10                  15

Asn Ala Arg Ser Ser Gln Ala Leu Gly Val Ser Trp Cys Pro Ala Thr
            20                  25                  30

Trp His Pro Arg Leu Asp Met Gly Arg Leu His Arg Pro Arg Ser Ser
        35                  40                  45

Thr Ser Tyr Arg Asn Leu Pro His Leu Phe Leu Phe Phe Leu Phe Val
    50                  55                  60

Gly Pro Phe Ser Cys Leu Gly Ser Tyr Ser Arg Ala Thr Glu Leu Leu
65                  70                  75                  80

Tyr Ser Leu Asn Glu Gly Leu Pro Ala Gly Val Leu Ile Gly Ser Leu
                85                  90                  95

Ala Glu Asp Leu Arg Leu Leu Pro Arg Ser Ala Gly Arg Pro Asp Pro
            100                 105                 110

Gln Ser Gln Leu Pro Glu Arg Thr Gly Ala Glu Trp Asn Pro Pro Leu
        115                 120                 125

Ser Phe Ser Leu Ala Ser Arg Gly Leu Ser Gly Gln Tyr Val Thr Leu
    130                 135                 140

Asp Asn Arg Ser Gly Glu Leu His Thr Ser Ala Gln Glu Ile Asp Arg
145                 150                 155                 160

Glu Ala Leu Cys Val Glu Gly Gly Gly Gly Thr Ala Trp Ser Gly Ser
                165                 170                 175

Val Ser Ile Ser Ser Ser Pro Ser Asp Ser Cys Leu Leu Leu Leu Asp
            180                 185                 190

Val Leu Val Leu Pro Gln Glu Tyr Phe Arg Phe Val Lys Val Lys Ile
        195                 200                 205

Ala Ile Arg Asp Ile Asn Asp Asn Ala Pro Gln Phe Pro Val Ser Gln
    210                 215                 220

Ile Ser Val Trp Val Pro Glu Asn Ala Pro Val Asn Thr Arg Leu Ala
225                 230                 235                 240

Ile Glu His Pro Ala Val Asp Pro Asp Val Gly Ile Asn Gly Val Gln
                245                 250                 255

Thr Tyr Arg Leu Leu Asp Tyr His Gly Met Phe Thr Leu Asp Val Glu
            260                 265                 270

Glu Asn Glu Asn Gly Glu Arg Thr Pro Tyr Leu Ile Val Met Gly Ala
        275                 280                 285

Leu Asp Arg Glu Thr Gln Asp Gln Tyr Val Ser Ile Ile Ile Ala Glu
    290                 295                 300

Asp Gly Gly Ser Pro Pro Leu Leu Gly Ser Ala Thr Leu Thr Ile Gly
305                 310                 315                 320

Ile Ser Asp Ile Asn Asp Asn Cys Pro Leu Phe Thr Asp Ser Gln Ile
                325                 330                 335

Asn Val Thr Val Tyr Gly Asn Ala Thr Val Gly Thr Pro Ile Ala Ala
            340                 345                 350

Val Gln Ala Val Asp Lys Asp Leu Gly Thr Asn Ala Gln Ile Thr Tyr
        355                 360                 365

Ser Tyr Ser Gln Lys Val Pro Gln Ala Ser Lys Asp Leu Phe His Leu
    370                 375                 380

Asp Glu Asn Thr Gly Val Ile Lys Leu Phe Ser Lys Ile Gly Gly Ser
385                 390                 395                 400

Val Leu Glu Ser His Lys Leu Thr Ile Leu Ala Asn Gly Pro Gly Cys
                405                 410                 415

Ile Pro Ala Val Ile Thr Ala Leu Val Ser Ile Ile Lys Val Ile Phe
            420                 425                 430

Arg Pro Pro Glu Ile Val Pro Arg Tyr Ile Ala Asn Glu Ile Asp Gly
        435                 440                 445

Val Val Tyr Leu Lys Glu Leu Glu Pro Val Asn Thr Pro Ile Ala Phe
    450                 455                 460

Phe Thr Ile Arg Asp Pro Glu Gly Lys Tyr Lys Val Asn Cys Tyr Leu
465                 470                 475                 480

Asp Gly Glu Gly Pro Phe Arg Leu Ser Pro Tyr Lys Pro Tyr Asn Asn
                485                 490                 495

Glu Tyr Leu Leu Glu Thr Thr Lys Pro Met Asp Tyr Glu Leu Gln Gln
            500                 505                 510

Phe Tyr Glu Val Ala Val Val Ala Trp Asn Ser Glu Gly Phe His Val
        515                 520                 525

Lys Arg Val Ile Lys Val Gln Leu Leu Asp Asp Asn Asp Asn Ala Pro
    530                 535                 540

Ile Phe Leu Gln Pro Leu Ile Glu Leu Thr Ile Glu Glu Asn Asn Ser
545                 550                 555                 560

Pro Asn Ala Phe Leu Thr Lys Leu Tyr Ala Thr Asp Ala Asp Ser Glu
                565                 570                 575

Glu Arg Gly Gln Val Ser Tyr Phe Leu Gly Pro Asp Ala Pro Ser Tyr
            580                 585                 590

Phe Ser Leu Asp Ser Val Thr Gly Ile Leu Thr Val Ser Thr Gln Leu
        595                 600                 605

Asp Arg Glu Glu Lys Glu Lys Tyr Arg Tyr Thr Val Arg Ala Val Asp
    610                 615                 620

Cys Gly Lys Pro Pro Arg Glu Ser Val Ala Thr Val Ala Leu Thr Val
625                 630                 635                 640

Leu Asp Lys Asn Asp Asn Ser Pro Arg Phe Ile Asn Lys Asp Phe Ser
                645                 650                 655

Phe Phe Val Pro Glu Asn Phe Pro Gly Tyr Gly Glu Ile Gly Val Ile
            660                 665                 670

Ser Val Thr Asp Ala Asp Ala Gly Arg Asn Gly Trp Val Ala Leu Ser
        675                 680                 685

Val Val Asn Gln Ser Asp Ile Phe Val Ile Asp Thr Gly Lys Gly Met
    690                 695                 700

Leu Arg Ala Lys Val Ser Leu Asp Arg Glu Gln Gln Ser Ser Tyr Thr
705                 710                 715                 720

Leu Trp Val Glu Ala Val Asp Gly Gly Glu Pro Ala Leu Ser Ser Thr
                725                 730                 735

Ala Lys Ile Thr Ile Leu Leu Leu Asp Ile Asn Asp Asn Pro Pro Leu
            740                 745                 750

Val Leu Phe Pro Gln Ser Asn Met Ser Tyr Leu Leu Val Leu Pro Ser
        755                 760                 765

Thr Leu Pro Gly Ser Pro Val Thr Glu Val Tyr Ala Val Asp Lys Asp
    770                 775                 780

Thr Gly Met Asn Ala Val Ile Ala Tyr Ser Ile Ile Gly Arg Arg Gly
785                 790                 795                 800

Pro Arg Pro Glu Ser Phe Arg Ile Asp Pro Lys Thr Gly Asn Ile Thr
                805                 810                 815

Leu Glu Glu Ala Leu Leu Gln Thr Asp Tyr Gly Leu His Arg Leu Leu
            820                 825                 830

Val Lys Val Ser Asp His Gly Tyr Pro Glu Pro Leu His Ser Thr Val
        835                 840                 845

Met Val Asn Leu Phe Val Asn Asp Thr Val Ser Asn Glu Ser Tyr Ile
    850                 855                 860

Glu Ser Leu Leu Arg Lys Glu Pro Glu Ile Asn Ile Glu Glu Lys Glu
865                 870                 875                 880

Pro Gln Ile Ser Ile Glu Pro Thr His Arg Lys Val Glu Ser Val Ser
                885                 890                 895

Cys Met Pro Thr Leu Val Ala Leu Ser Val Ile Ser Leu Gly Ser Ile
            900                 905                 910

Thr Leu Val Thr Gly Met Gly Ile Tyr Ile Cys Leu Arg Lys Gly Glu
        915                 920                 925

Lys His Pro Arg Glu Asp Glu Asn Leu Glu Val Gln Ile Pro Leu Lys
    930                 935                 940

Gly Lys Ile Asp Leu His Met Arg Glu Arg Lys Pro Met Asp Ile Ser
945                 950                 955                 960

Asn Ile

<210> 57
<211> 2801
<212> DNA
<213> Homo sapiens

<400> 57
accggatgca cccggagatc tcctactcaa ttcctgagga aagagagaaa ggctctttcg 60
tgggcaacat ctccaaggac ttgggtctgg cgcccccgga gctggcggag cgcggagtcc 120
gcatagtctc cagaggtagg acgcagcttt tctctctgaa cccgcgcagc ggcagcttgg 180
tcaccgcggg caggatagac cgggaggagc tctgcgctca gagcgcgcgg tgcgtggtga 240
gttttaatat ccttgtggaa gacagggtga aactttttgg gatagaaata gaagtaactg 300
atatcaatga caatgctcca aaattccaag cagaaaatct agacgtaaaa attaatgaaa 360
atgtcgctgc gggaatgcgt tttcctctcc cggaagctat tgatccggat ataggtgtaa 420
actccctgca gggttaccag ctcaactcaa acggttactt ttccctggac gtgcaaagtg 480
gggccgatgg gattaagtac ccagagctgg tgctggaacg cgctctagat cgcgaggaag 540
aggcggttca ccacctggtc cttactgcca tggatggcgg cgaccctctc cgctcaagcg 600
tcgcccaaat tctggtaaca gttctagatg tgaatgacaa cactccaatg tttactcagc 660
ctgtctaccg tgtaagtgtt cctgaaaacc tgccagctcc cggaactcgg gtgctgatgg 720
ttaatgcaac ggatccagat gaaggagtca atgcggaagt aatgtattca tttcggaaag 780
tcagagacga aagagcacag ctattacagt tgttttatct gagtgcggag ataacgataa 840

tgaggggtct ggaggatgtg gactatggat actatgacat agatgacata gacgatgaag 900
gccatggtgt ccgtgctaga agagcggtac gcaaggtagt ggtggaagtt ttggatgaaa 960
atgacaacgc cccagaaatc acagtcacct ccgtcaccac cgcagtcccc gaagctgctt 1020
ctggaactgc cattatttc ctcaatgata gtgaccgaga ggacgggggg aacagtccat 1080
ttatcagttc tgtcaatccg ggtctttcat tcaaaaaatt agataaaaaa gatgattatt 1140
tcatttttaa aacgactcaa gacatagacc gaaaaactgt gtccgaatac aacatcaccg 1200
caatagcccc agaaacccga gctccttccc cttcaactca tattacactc cttgtgctag 1260
tgatcgacat caatgaaaac cctccccctt attctcaatc ctcctactac gtttacgtaa 1320
acgaaaacaa cggcgccggc acttcaatta tgaccgttaa tgactctgac cccgatgaca 1380
attctagtgt tatttactcc ttggcagagg ctacccaagg agctcctccc tcctccacct 1440
atgcctctat cacatcaaac actggtgtgc ttgatgtgtt gtccttcttc tactatgagt 1500
attttgattt tctgcaaatg cagatgacgg ctaatgacag tggcagccca ccacttagca 1560
gtaatgcgtc aatgagattg tttgtgttgg acaaggatga caatgcccaa gacctccagt 1620
accctgcccc ccccactggt ggtactgctg ttgaggtgct gccccgctct gcagcgcctg 1680
gctacctggt gaccaaggtg gtggcagtgg acggagactc aggccagaat gcttggctct 1740
cctaccgcct attcgaggcc agtgagccgg ggctcttctc ggtggggctg cacacaggtg 1800
gaaaagtgcg caccgctcgg gccctgctag atagagatgc gctcaaacag agccttgtgg 1860
tggctgtaca ggaccatggc cagccccctc tctccgccgc caccctcact gtggcggttg 1920
ccgacgactc cctccaagac ctcgcggatt tcttcggcag cctcacgcct tcagaacacc 1980
aagacgactc cggcctcaca ctccttcttg tggtagtagt ggctgcagtc tgcttcgtct 2040
tcccggtctt cgtcgtcgtg ctgctagtac tcaagctgag gcgctggcac aagtcccgcc 2100
tgcttcacgc tgaaggcagc aggttggcag ggtggctgc ctcccacttt gggggcgtgg 2160
agggggttgg ggttttcctg ccgaactatt cccacgaggt ctccctcacc gcggactcgc 2220
ggaagagccc cctgatcccc acccaacccc gcgctgaaat tcccctcagc aaccgggaga 2280
gtggagagag aagccgccgt ctggtgatac ttaaggatgt gcttgaaaca gagggcgacc 2340
ctagtggaca gcaagccccg cccaacacgg actggcgttt ctctcaggcc cagagacccg 2400
gcaccagcgg ctcccaaaat ggcgatgaca ccggcacctg gcccaacaac cagtttgaca 2460
cagagatgct gcaagccatg atcttggcgt ccgccagtga agctgctgat gggagctcca 2520
ccctgggagg gggtgccggc accatgggat tgagcgcccg ctacggaccc cagttcaccc 2580
tgcagcacgt gcccgactac cgccagaatg tctacatccc aggcagcaat gccacactga 2640
ccaacgcagc tggcaagcgg gatggcaagg ccccagcagg tggcaatggc aacaagaaga 2700
agtcgggcaa gaaggagaag aagtaacatg gaggccaggc caagagccac agggcggcct 2760
ctccccaacc agcccagctt ctccttacct gcacccaggc c 2801

<210> 58
<211> 906
<212> PRT
<213> Homo sapiens

<400> 58
Met His Pro Glu Ile Ser Tyr Ser Ile Pro Glu Glu Arg Glu Lys Gly
1               5                   10                  15

Ser Phe Val Gly Asn Ile Ser Lys Asp Leu Gly Leu Ala Pro Arg Glu
            20                  25                  30

Leu Ala Glu Arg Gly Val Arg Ile Val Ser Arg Gly Arg Thr Gln Leu
        35                  40                  45

Phe Ser Leu Asn Pro Arg Ser Gly Ser Leu Val Thr Ala Gly Arg Ile
    50                  55                  60

Asp Arg Glu Glu Leu Cys Ala Gln Ser Ala Arg Cys Val Val Ser Phe
65                  70                  75                  80

Asn Ile Leu Val Glu Asp Arg Val Lys Leu Phe Gly Ile Glu Ile Glu
                85                  90                  95

Val Thr Asp Ile Asn Asp Asn Ala Pro Lys Phe Gln Ala Glu Asn Leu
            100                 105                 110

Asp Val Lys Ile Asn Glu Asn Val Ala Ala Gly Met Arg Phe Pro Leu
        115                 120                 125

Pro Glu Ala Ile Asp Pro Asp Ile Gly Val Asn Ser Leu Gln Gly Tyr
    130                 135                 140

Gln Leu Asn Ser Asn Gly Tyr Phe Ser Leu Asp Val Gln Ser Gly Ala

145                 150                 155                 160

Asp Gly Ile Lys Tyr Pro Glu Leu Val Leu Glu Arg Ala Leu Asp Arg
                165                 170                 175

Glu Glu Glu Ala Val His His Leu Val Leu Thr Ala Met Asp Gly Gly
            180                 185                 190

Asp Pro Leu Arg Ser Ser Val Ala Gln Ile Leu Val Thr Val Leu Asp
        195                 200                 205

Val Asn Asp Asn Thr Pro Met Phe Thr Gln Pro Val Tyr Arg Val Ser
    210                 215                 220

Val Pro Glu Asn Leu Pro Ala Pro Gly Thr Arg Val Leu Met Val Asn
225                 230                 235                 240

Ala Thr Asp Pro Asp Glu Gly Val Asn Ala Glu Val Met Tyr Ser Phe
                245                 250                 255

Arg Lys Val Arg Asp Glu Arg Ala Gln Leu Leu Gln Leu Phe Tyr Leu
            260                 265                 270

Ser Ala Glu Ile Thr Ile Met Arg Gly Leu Glu Asp Val Asp Tyr Gly
        275                 280                 285

Tyr Tyr Asp Ile Asp Asp Ile Asp Asp Glu Gly His Gly Val Arg Ala
    290                 295                 300

Arg Arg Ala Val Arg Lys Val Val Val Glu Val Leu Asp Glu Asn Asp
305                 310                 315                 320

Asn Ala Pro Glu Ile Thr Val Thr Ser Val Thr Thr Ala Val Pro Glu
                325                 330                 335

Ala Ala Ser Gly Thr Ala Ile Ile Phe Leu Asn Asp Ser Asp Arg Glu
            340                 345                 350

Asp Gly Gly Asn Ser Pro Phe Ile Ser Ser Val Asn Pro Gly Leu Ser
        355                 360                 365

Phe Lys Lys Leu Asp Lys Lys Asp Asp Tyr Phe Ile Phe Lys Thr Thr
    370                 375                 380

Gln Asp Ile Asp Arg Lys Thr Val Ser Glu Tyr Asn Ile Thr Ala Ile
385                 390                 395                 400

Ala Pro Glu Thr Arg Ala Pro Ser Pro Ser Thr His Ile Thr Leu Leu
                405                 410                 415

Val Leu Val Ile Asp Ile Asn Glu Asn Pro Pro Pro Tyr Ser Gln Ser
            420                 425                 430

Ser Tyr Tyr Val Tyr Val Asn Glu Asn Asn Gly Ala Gly Thr Ser Ile
        435                 440                 445

Met Thr Val Asn Asp Ser Asp Pro Asp Asp Asn Ser Ser Val Ile Tyr
    450                 455                 460

Ser Leu Ala Glu Ala Thr Gln Gly Ala Pro Pro Ser Ser Thr Tyr Ala
465                 470                 475                 480

Ser Ile Thr Ser Asn Thr Gly Val Leu Asp Val Leu Ser Phe Phe Tyr
                485                 490                 495

Tyr Glu Tyr Phe Asp Phe Leu Gln Met Gln Met Thr Ala Asn Asp Ser
            500                 505                 510

Gly Ser Pro Pro Leu Ser Ser Asn Ala Ser Met Arg Leu Phe Val Leu

515                 520                 525

Asp Lys Asp Asp Asn Ala Gln Asp Leu Gln Tyr Pro Ala Pro Pro Thr
    530                 535                 540

Gly Gly Thr Ala Val Glu Val Leu Pro Arg Ser Ala Ala Pro Gly Tyr
545                 550                 555                 560

Leu Val Thr Lys Val Val Ala Val Asp Gly Asp Ser Gly Gln Asn Ala
                565                 570                 575

Trp Leu Ser Tyr Arg Leu Phe Glu Ala Ser Glu Pro Gly Leu Phe Ser
            580                 585                 590

Val Gly Leu His Thr Gly Gly Lys Val Arg Thr Ala Arg Ala Leu Leu
        595                 600                 605

Asp Arg Asp Ala Leu Lys Gln Ser Leu Val Val Ala Val Gln Asp His
    610                 615                 620

Gly Gln Pro Pro Leu Ser Ala Ala Thr Leu Thr Val Ala Val Ala Asp
625                 630                 635                 640

Asp Ser Leu Gln Asp Leu Ala Asp Phe Phe Gly Ser Leu Thr Pro Ser
                645                 650                 655

Glu His Gln Asp Asp Ser Gly Leu Thr Leu Leu Leu Val Val Val Val
            660                 665                 670

Ala Ala Val Cys Phe Val Phe Pro Val Phe Val Val Val Leu Leu Val
        675                 680                 685

Leu Lys Leu Arg Arg Trp His Lys Ser Arg Leu Leu His Ala Glu Gly
    690                 695                 700

Ser Arg Leu Ala Gly Val Ala Ala Ser His Phe Gly Gly Val Glu Gly
705                 710                 715                 720

Val Gly Val Phe Leu Pro Asn Tyr Ser His Glu Val Ser Leu Thr Ala
                725                 730                 735

Asp Ser Arg Lys Ser Pro Leu Ile Pro Thr Gln Pro Arg Ala Glu Ile
            740                 745                 750

Pro Leu Ser Asn Arg Glu Ser Gly Glu Arg Ser Arg Arg Leu Val Ile
        755                 760                 765

Leu Lys Asp Val Leu Glu Thr Glu Gly Asp Pro Ser Gly Gln Gln Ala
    770                 775                 780

Pro Pro Asn Thr Asp Trp Arg Phe Ser Gln Ala Gln Arg Pro Gly Thr
785                 790                 795                 800

Ser Gly Ser Gln Asn Gly Asp Asp Thr Gly Thr Trp Pro Asn Asn Gln
                805                 810                 815

Phe Asp Thr Glu Met Leu Gln Ala Met Ile Leu Ala Ser Ala Ser Glu
            820                 825                 830

Ala Ala Asp Gly Ser Ser Thr Leu Gly Gly Gly Ala Gly Thr Met Gly
        835                 840                 845

Leu Ser Ala Arg Tyr Gly Pro Gln Phe Thr Leu Gln His Val Pro Asp
    850                 855                 860

Tyr Arg Gln Asn Val Tyr Ile Pro Gly Ser Asn Ala Thr Leu Thr Asn
865                 870                 875                 880

Ala Ala Gly Lys Arg Asp Gly Lys Ala Pro Ala Gly Gly Asn Gly Asn

                885                 890                 895

Lys Lys Lys Ser Gly Lys Lys Glu Lys Lys
            900                 905

<210> 59
<211> 2836
<212> DNA
<213> Homo sapiens

<400> 59
aacccgagcg aacgatggga gggagctgcg cgcagaggcg ccgggccggc ccgcggcagg 60
tactatttcc tttgctgctg cctttgttct accccacgct gtgtgagccg atccgctact 120
cgattccgga ggagctggcc aagggctcgg tggtggggaa cctcgctaag gatctagggc 180
ttagtgtcct ggatgtgtcg gctcgcgagc tgcgagtgag cgcggagaag ctgcacttca 240
gcgtagacgc gcagagcggg gacttacttg tgaaggaccg aatagaccgt gagcaaatat 300
gcaaagagag aagaagatgt gagttgcaat tggaagctgt ggtggaaaat cctttaaata 360
tttttcatgt cattgtggtg attgaggatg ttaatgacca cgcccctcaa ttccggaaag 420
atgaaataaa cttagaaatc agtgaatccg tcagcctggg gatgggaaca attcttgagt 480
ctgcagaaga tcctgatatt agtatgaatt cgctgagcaa ataccaacta agtcctaacg 540
agtatttctc attggtggag aaagacaatc ctgatggtgg caaatatcca gaattagtat 600
tgcagaagac tctggaccga gaaacgcaga gcgctcacca cttgatattg accgccttgg 660
acggagggga cccaccaaga agtgccaccg ctcacataga aatttctgtc aaggatacca 720
atgataaccc cccggttttc agcagagacg aatatagaat tagtcttagt gaaaatctgc 780
cccctgggtc ccctgtgttg caagtgacag ccactgacca ggatgagggg gtcaatgctg 840
agataaacta ctacttccga agcactgccc agagcacaaa acatatgttc tcattggatg 900
agaaaacagg tatgattaag aataaccagt catttgattt tgaagatgta gaaaggtaca 960
ccatggaagt ggaagcgaag gacggaggtg gtctctctac ccagtgtaaa gtaatcatag 1020
aaatccttga tgaaaacgac aacagcccag aaataatcat cacttctctc tctgatcaga 1080
ttttggagaa ttcacctcca ggaatggttg ttgccctctt caaaacacgg gatctggatt 1140
tcggaggaaa tggagaagtc aggtgtaata tagaaacaga cattccattc aagatttatt 1200
cttcttccaa taactactac aaactggtga cagatggagc cctggaccga gagcagacac 1260
cagaatacaa tgtcaccatc gtagccactg acaggggcaa gccgcctctt tcttccagta 1320
gaagcatcac cttgtatgtc gctgacatca acgacaacgc cccagttttc gaccagacgt 1380
cctacgtggt ccacgtggcc gagaacaacc cgccaggagc ctccattgcg caagtgagcg 1440
cctctgaccc ggatttgggg ctcaatggcc acatctccta ctctctcatt gccagcgacc 1500
tggagtcacg aacgctgtcg tcctacgtgt ccgtgagcgc gcagagcggg gtggtgttcg 1560
cgcagcgcgc cttcgaccac gagcagctgc gcgccttcgc gctcacgctg caggcccgcg 1620
accagggctc gcccgcgctc agcgcgaacg tgagcctgcg cgtgttagtg gacgaccgca 1680
acgacaatgc gccacgggtg ctgtacccag ctctgggtcc tgacggctcc gcgttcttcg 1740
atatggtacc tcgctctgca gagcccggct acctagtgac taaggtggta gcggtggacg 1800
ccgactcggg acacaacgcc tggctgtcct accacgtgct gcaggccagt gagcccgggc 1860
tcttcagcct ggggctgcga acaggcgagg tgcgcatggt gcgtgctttg ggtgacaagg 1920
actcggtccg ccagcgcctg ctagtcgcta taagagatgg aggacagcca cccctttcag 1980
ccactgccac gctgcacctg gtgttcgcag atagcttgca agaggtactg ccggatttca 2040
gcgaccatcc cacaccctct gactcccagg ctgagatgca gttttacctg gtggtggcct 2100
tggccttgat ttctgtgctc tttctcctcg cggtgattct agctattgct ctacgcctgc 2160
gacagtcttt cagccctact gcaggagact gctttgagtc agttctctgc tccaagtccg 2220
gacctgtggg tccccccaac tacagtgagg gaacgttgcc ctatgcctat aatttttgtg 2280
tgcctgggga tcaaatgaat ccagaattta atttttcac atctgttgat cattgtccag 2340
ccacacaaga taacctcaac aaagatagca tgctactggc tagcatttta actcccagcg 2400
ttgaagcaga taagaagatt cttaaacagc aagccccgcc caacacggac tggcgtttct 2460
ctcaggccca gagacccggc accagcggct cccaaaatgg cgatgacacc ggcacctggc 2520
ccaacaacca gtttgacaca gagatgctgc aagccatgat cttggcgtcc gccagtgaag 2580
ctgctgatgg gagctccacc ctgggagggg gtgccggcac catgggattg agcgcccgct 2640
acggacccca gttcaccctg cagcacgtgc ccgactaccg ccagaatgtc tacatcccag 2700
gcagcaatgc cacactgacc aacgcagctg gcaagcggga tggcaaggcc ccagcaggtg 2760
gcaatggcaa caagaagaag tcgggcaaga aggagaagaa gtaacatgga ggccaggcca 2820
agagccacag ggcggc 2836

<210> 60
<211> 929
<212> PRT
<213> Homo sapiens

<400> 60
Met Gly Gly Ser Cys Ala Gln Arg Arg Arg Ala Gly Pro Arg Gln Val

1                   5                   10                  15

Leu Phe Pro Leu Leu Leu Pro Leu Phe Tyr Pro Thr Leu Cys Glu Pro
            20                  25                  30

Ile Arg Tyr Ser Ile Pro Glu Glu Leu Ala Lys Gly Ser Val Val Gly
        35                  40                  45

Asn Leu Ala Lys Asp Leu Gly Leu Ser Val Leu Asp Val Ser Ala Arg
    50                  55                  60

Glu Leu Arg Val Ser Ala Glu Lys Leu His Phe Ser Val Asp Ala Gln
65                  70                  75                  80

Ser Gly Asp Leu Leu Val Lys Asp Arg Ile Asp Arg Glu Gln Ile Cys
                85                  90                  95

Lys Glu Arg Arg Arg Cys Glu Leu Gln Leu Glu Ala Val Val Glu Asn
            100                 105                 110

Pro Leu Asn Ile Phe His Val Ile Val Val Ile Glu Asp Val Asn Asp
        115                 120                 125

His Ala Pro Gln Phe Arg Lys Asp Glu Ile Asn Leu Glu Ile Ser Glu
    130                 135                 140

Ser Val Ser Leu Gly Met Gly Thr Ile Leu Glu Ser Ala Glu Asp Pro
145                 150                 155                 160

Asp Ile Ser Met Asn Ser Leu Ser Lys Tyr Gln Leu Ser Pro Asn Glu
                165                 170                 175

Tyr Phe Ser Leu Val Glu Lys Asp Asn Pro Asp Gly Gly Lys Tyr Pro
            180                 185                 190

Glu Leu Val Leu Gln Lys Thr Leu Asp Arg Glu Thr Gln Ser Ala His
        195                 200                 205

His Leu Ile Leu Thr Ala Leu Asp Gly Gly Asp Pro Pro Arg Ser Ala
    210                 215                 220

Thr Ala His Ile Glu Ile Ser Val Lys Asp Thr Asn Asp Asn Pro Pro
225                 230                 235                 240

Val Phe Ser Arg Asp Glu Tyr Arg Ile Ser Leu Ser Glu Asn Leu Pro
                245                 250                 255

Pro Gly Ser Pro Val Leu Gln Val Thr Ala Thr Asp Gln Asp Glu Gly
            260                 265                 270

Val Asn Ala Glu Ile Asn Tyr Tyr Phe Arg Ser Thr Ala Gln Ser Thr
        275                 280                 285

Lys His Met Phe Ser Leu Asp Glu Lys Thr Gly Met Ile Lys Asn Asn
    290                 295                 300

Gln Ser Phe Asp Phe Glu Asp Val Glu Arg Tyr Thr Met Glu Val Glu
305                 310                 315                 320

Ala Lys Asp Gly Gly Gly Leu Ser Thr Gln Cys Lys Val Ile Ile Glu
                325                 330                 335

Ile Leu Asp Glu Asn Asp Asn Ser Pro Glu Ile Ile Ile Thr Ser Leu
            340                 345                 350

Ser Asp Gln Ile Leu Glu Asn Ser Pro Pro Gly Met Val Val Ala Leu
        355                 360                 365

Phe Lys Thr Arg Asp Leu Asp Phe Gly Gly Asn Gly Glu Val Arg Cys

370                 375                 380

Asn Ile Glu Thr Asp Ile Pro Phe Lys Ile Tyr Ser Ser Ser Asn Asn
385                 390                 395                 400

Tyr Tyr Lys Leu Val Thr Asp Gly Ala Leu Asp Arg Glu Gln Thr Pro
                405                 410                 415

Glu Tyr Asn Val Thr Ile Val Ala Thr Asp Arg Gly Lys Pro Pro Leu
            420                 425                 430

Ser Ser Ser Arg Ser Ile Thr Leu Tyr Val Ala Asp Ile Asn Asp Asn
        435                 440                 445

Ala Pro Val Phe Asp Gln Thr Ser Tyr Val Val His Val Ala Glu Asn
    450                 455                 460

Asn Pro Pro Gly Ala Ser Ile Ala Gln Val Ser Ala Ser Asp Pro Asp
465                 470                 475                 480

Leu Gly Leu Asn Gly His Ile Ser Tyr Ser Leu Ile Ala Ser Asp Leu
                485                 490                 495

Glu Ser Arg Thr Leu Ser Ser Tyr Val Ser Val Ser Ala Gln Ser Gly
            500                 505                 510

Val Val Phe Ala Gln Arg Ala Phe Asp His Glu Gln Leu Arg Ala Phe
        515                 520                 525

Ala Leu Thr Leu Gln Ala Arg Asp Gln Gly Ser Pro Ala Leu Ser Ala
    530                 535                 540

Asn Val Ser Leu Arg Val Leu Val Asp Asp Arg Asn Asp Asn Ala Pro
545                 550                 555                 560

Arg Val Leu Tyr Pro Ala Leu Gly Pro Asp Gly Ser Ala Phe Phe Asp
                565                 570                 575

Met Val Pro Arg Ser Ala Glu Pro Gly Tyr Leu Val Thr Lys Val Val
            580                 585                 590

Ala Val Asp Ala Asp Ser Gly His Asn Ala Trp Leu Ser Tyr His Val
        595                 600                 605

Leu Gln Ala Ser Glu Pro Gly Leu Phe Ser Leu Gly Leu Arg Thr Gly
    610                 615                 620

Glu Val Arg Met Val Arg Ala Leu Gly Asp Lys Asp Ser Val Arg Gln
625                 630                 635                 640

Arg Leu Leu Val Ala Ile Arg Asp Gly Gly Gln Pro Pro Leu Ser Ala
                645                 650                 655

Thr Ala Thr Leu His Leu Val Phe Ala Asp Ser Leu Gln Glu Val Leu
            660                 665                 670

Pro Asp Phe Ser Asp His Pro Thr Pro Ser Asp Ser Gln Ala Glu Met
        675                 680                 685

Gln Phe Tyr Leu Val Val Ala Leu Ala Leu Ile Ser Val Leu Phe Leu
    690                 695                 700

Leu Ala Val Ile Leu Ala Ile Ala Leu Arg Leu Arg Gln Ser Phe Ser
705                 710                 715                 720

Pro Thr Ala Gly Asp Cys Phe Glu Ser Val Leu Cys Ser Lys Ser Gly
                725                 730                 735

Pro Val Gly Pro Pro Asn Tyr Ser Glu Gly Thr Leu Pro Tyr Ala Tyr

740                 745                 750

Asn Phe Cys Val Pro Gly Asp Gln Met Asn Pro Glu Phe Asn Phe Phe
        755                 760                 765

Thr Ser Val Asp His Cys Pro Ala Thr Gln Asp Asn Leu Asn Lys Asp
    770                 775                 780

Ser Met Leu Leu Ala Ser Ile Leu Thr Pro Ser Val Glu Ala Asp Lys
785                 790                 795                 800

Lys Ile Leu Lys Gln Gln Ala Pro Pro Asn Thr Asp Trp Arg Phe Ser
                805                 810                 815

Gln Ala Gln Arg Pro Gly Thr Ser Gly Ser Gln Asn Gly Asp Asp Thr
            820                 825                 830

Gly Thr Trp Pro Asn Asn Gln Phe Asp Thr Glu Met Leu Gln Ala Met
        835                 840                 845

Ile Leu Ala Ser Ala Ser Glu Ala Ala Asp Gly Ser Ser Thr Leu Gly
    850                 855                 860

Gly Gly Ala Gly Thr Met Gly Leu Ser Ala Arg Tyr Gly Pro Gln Phe
865                 870                 875                 880

Thr Leu Gln His Val Pro Asp Tyr Arg Gln Asn Val Tyr Ile Pro Gly
                885                 890                 895

Ser Asn Ala Thr Leu Thr Asn Ala Ala Gly Lys Arg Asp Gly Lys Ala
            900                 905                 910

Pro Ala Gly Gly Asn Gly Asn Lys Lys Lys Ser Gly Lys Lys Glu Lys
        915                 920                 925

Lys

<210> 61
<211> 2436
<212> DNA
<213> Homo sapiens

<400> 61
atggctgtca gagagttgtg cttcccaaga caaaggcaag tcctgtttct tttttctttt 60
tggggagtgt ccttggcagg ttctgggttt ggacgttatt cggtgactga ggaaacagag 120
aaaggatcct ttgtggtcaa tctggcaaag gatctgggac tagcagaggg ggagctggct 180
gcaaggggaa ccagggtggt ttccgatgat aacaaacaat acctgctcct ggattcacat 240
accgggaatt tgctcacaaa tgagaaactg gaccgagaga agctgtgtgg ccctaaagag 300
ccctgtatgc tgtatttcca aattttaatg gatgatccct ttcagattta ccgggctgag 360
ctgagagtca gggatataaa tgatcacgcg ccagtatttc aggacaaaga aacagtctta 420
aaaatatcag aaaatacagc tgaagggaca gcatttagac tagaaagagc acaggatcca 480
gatggaggac ttaacggtat ccaaaactac acgatcagcc ccaactcttt tttccatatt 540
aacattagtg gcggtgatga aggcatgata tatccagagc tagtgttgga caaagcactg 600
gatcgggagg agcagggaga gctcagctta accctcacag cgctggatgg tgggtctcca 660
tccaggtctg ggacctctac tgtacgcatc gttgtcttgg acgtcaatga caatgcccca 720
cagtttgccc aggctctgta tgagacccag gctccagaaa acagccccat tgggttcctt 780
attgttaagg tatgggcaga agatgtagac tctggagtca acgcggaagt atcctattca 840
ttttttgatg cctcagaaaa tattcgaaca acctttcaaa tcaatccttt ttctggggaa 900
atctttctca gagaattgct tgattatgag ttagtaaatt cttacaaaat aaatatacag 960
gcaatggacg gtggaggcct ttctgcaaga tgtagggttt tagtggaagt attggacacc 1020
aatgacaatc cccctgaact gatcgtatca tcattttcca actctgttgc tgagaattct 1080
cctgagacgc cgctggctgt ttttaagatt aatgacagag actctggaga aaatggaagg 1140
atggtgtgct acattcaaga tgatctgcca ttcctactaa aaccttctgt tgagaatttt 1200
tacatcctaa tgactgaagg cgcgctggac agagaagcaa gagctgaata taatatcacc 1260
ctcaccgtca cagatatggg gactccaagg ctgaaaacgg agcacaacat aacagtgcag 1320
atatcagatg tcaatgataa cgcccccact ttcacccaaa cctcctacgc cctgttcgtc 1380
cgcgagaaca acagccccgc cctgcacatc ggcagcgtca gcgccacaga cagagactca 1440

ggcaccaacg cccaggtcac ctactcgctg ctgccgcccc aggacccgca cctgcccctc 1500
gcctccctgg tctccatcaa cgcagacaac ggccacctgt tcgccctcag gtcgctggac 1560
tacgaggccc tgcaggcttt cgagttccgc gtgggcgcca cagaccgcgg ctcccccgcg 1620
ctgagcagag aggcgctggt gcgcgtgctg gtgctggacg ccaacgacaa ctcgcccttc 1680
gtgctgtacc cgctgcagaa cggctccgcg ccctgcactg agctggtgcc ccgggcggcc 1740
gagccgggct acctggtgac caaggtggtg gcggtggacg gcgactcggg ccagaacgcc 1800
tggctgtcgt accagctgct caaggccacg gagcccgggc tgttcggtgt gtgggcgcac 1860
aatggggagg tgcgcaccgc caggctgctg agcgagcgcg acgcagccaa gcacaggctc 1920
gtggtgcttg tcaaggacaa tggcgagcct cctcgctcgg ccaccgccac gctgcacttg 1980
ctcctggtgg acggcttctc ccagccctac ctgcctctcc cggaggcggc cccggcccag 2040
gcccaggccg aggccgactt gctcaccgtc tacctggtgg tggcgttggc ctcggtgtct 2100
tcgctcttcc tcctctcggt gctcctgttc gtggcggtgc ggctgtgcag gaggagcagg 2160
gcggcctcgg tgggtcgctg ctcggtgccc gagggtcctt ttccagggca tctggtggac 2220
gtgagcggca ccgggaccct gttccagagc taccagtacg aggtgtgtct gactggaggt 2280
tcagagaccg gcgagttcaa gttcttgaag ccgattaccc cccacctccc gccccatagg 2340
ggtgggaaag aaatagagga aaattctact ctccccaata gctttggatt taattattga 2400
aaggaaccca cttaataaag acatttactt ctttaa 2436

<210> 62
<211> 799
<212> PRT
<213> Homo sapiens

<400> 62
Met Ala Val Arg Glu Leu Cys Phe Pro Arg Gln Arg Gln Val Leu Phe
1               5                   10                  15

Leu Phe Leu Phe Trp Gly Val Ser Leu Ala Gly Ser Gly Phe Gly Arg
            20                  25                  30

Tyr Ser Val Thr Glu Glu Thr Glu Lys Gly Ser Phe Val Val Asn Leu
        35                  40                  45

Ala Lys Asp Leu Gly Leu Ala Glu Gly Glu Leu Ala Ala Arg Gly Thr
    50                  55                  60

Arg Val Val Ser Asp Asp Asn Lys Gln Tyr Leu Leu Leu Asp Ser His
65                  70                  75                  80

Thr Gly Asn Leu Leu Thr Asn Glu Lys Leu Asp Arg Glu Lys Leu Cys
                85                  90                  95

Gly Pro Lys Glu Pro Cys Met Leu Tyr Phe Gln Ile Leu Met Asp Asp
            100                 105                 110

Pro Phe Gln Ile Tyr Arg Ala Glu Leu Arg Val Arg Asp Ile Asn Asp
        115                 120                 125

His Ala Pro Val Phe Gln Asp Lys Glu Thr Val Leu Lys Ile Ser Glu
    130                 135                 140

Asn Thr Ala Glu Gly Thr Ala Phe Arg Leu Glu Arg Ala Gln Asp Pro
145                 150                 155                 160

Asp Gly Gly Leu Asn Gly Ile Gln Asn Tyr Thr Ile Ser Pro Asn Ser
                165                 170                 175

Phe Phe His Ile Asn Ile Ser Gly Gly Asp Glu Gly Met Ile Tyr Pro
            180                 185                 190

Glu Leu Val Leu Asp Lys Ala Leu Asp Arg Glu Glu Gln Gly Glu Leu
        195                 200                 205

Ser Leu Thr Leu Thr Ala Leu Asp Gly Gly Ser Pro Ser Arg Ser Gly
    210                 215                 220

Thr Ser Thr Val Arg Ile Val Val Leu Asp Val Asn Asp Asn Ala Pro
225                 230                 235                 240

Gln Phe Ala Gln Ala Leu Tyr Glu Thr Gln Ala Pro Glu Asn Ser Pro
                245                 250                 255

Ile Gly Phe Leu Ile Val Lys Val Trp Ala Glu Asp Val Asp Ser Gly
            260                 265                 270

Val Asn Ala Glu Val Ser Tyr Ser Phe Phe Asp Ala Ser Glu Asn Ile
        275                 280                 285

Arg Thr Thr Phe Gln Ile Asn Pro Phe Ser Gly Glu Ile Phe Leu Arg
    290                 295                 300

Glu Leu Leu Asp Tyr Glu Leu Val Asn Ser Tyr Lys Ile Asn Ile Gln
305                 310                 315                 320

Ala Met Asp Gly Gly Gly Leu Ser Ala Arg Cys Arg Val Leu Val Glu
                325                 330                 335

Val Leu Asp Thr Asn Asp Asn Pro Pro Glu Leu Ile Val Ser Ser Phe
            340                 345                 350

Ser Asn Ser Val Ala Glu Asn Ser Pro Glu Thr Pro Leu Ala Val Phe
        355                 360                 365

Lys Ile Asn Asp Arg Asp Ser Gly Glu Asn Gly Arg Met Val Cys Tyr
    370                 375                 380

Ile Gln Asp Asp Leu Pro Phe Leu Leu Lys Pro Ser Val Glu Asn Phe
385                 390                 395                 400

Tyr Ile Leu Met Thr Glu Gly Ala Leu Asp Arg Glu Ala Arg Ala Glu
                405                 410                 415

Tyr Asn Ile Thr Leu Thr Val Thr Asp Met Gly Thr Pro Arg Leu Lys
            420                 425                 430

Thr Glu His Asn Ile Thr Val Gln Ile Ser Asp Val Asn Asp Asn Ala
        435                 440                 445

Pro Thr Phe Thr Gln Thr Ser Tyr Ala Leu Phe Val Arg Glu Asn Asn
    450                 455                 460

Ser Pro Ala Leu His Ile Gly Ser Val Ser Ala Thr Asp Arg Asp Ser
465                 470                 475                 480

Gly Thr Asn Ala Gln Val Thr Tyr Ser Leu Leu Pro Pro Gln Asp Pro
                485                 490                 495

His Leu Pro Leu Ala Ser Leu Val Ser Ile Asn Ala Asp Asn Gly His
            500                 505                 510

Leu Phe Ala Leu Arg Ser Leu Asp Tyr Glu Ala Leu Gln Ala Phe Glu
        515                 520                 525

Phe Arg Val Gly Ala Thr Asp Arg Gly Ser Pro Ala Leu Ser Arg Glu
    530                 535                 540

Ala Leu Val Arg Val Leu Val Leu Asp Ala Asn Asp Asn Ser Pro Phe
545                 550                 555                 560

Val Leu Tyr Pro Leu Gln Asn Gly Ser Ala Pro Cys Thr Glu Leu Val
                565                 570                 575

Pro Arg Ala Ala Glu Pro Gly Tyr Leu Val Thr Lys Val Val Ala Val
            580                 585                 590

Asp Gly Asp Ser Gly Gln Asn Ala Trp Leu Ser Tyr Gln Leu Leu Lys
        595                 600                 605

Ala Thr Glu Pro Gly Leu Phe Gly Val Trp Ala His Asn Gly Glu Val
    610                 615                 620

Arg Thr Ala Arg Leu Leu Ser Glu Arg Asp Ala Ala Lys His Arg Leu
625                 630                 635                 640

Val Val Leu Val Lys Asp Asn Gly Glu Pro Pro Arg Ser Ala Thr Ala
                645                 650                 655

Thr Leu His Leu Leu Leu Val Asp Gly Phe Ser Gln Pro Tyr Leu Pro
            660                 665                 670

Leu Pro Glu Ala Ala Pro Ala Gln Ala Gln Ala Glu Ala Asp Leu Leu
        675                 680                 685

Thr Val Tyr Leu Val Val Ala Leu Ala Ser Val Ser Ser Leu Phe Leu
    690                 695                 700

Leu Ser Val Leu Leu Phe Val Ala Val Arg Leu Cys Arg Arg Ser Arg
705                 710                 715                 720

Ala Ala Ser Val Gly Arg Cys Ser Val Pro Glu Gly Pro Phe Pro Gly
                725                 730                 735

His Leu Val Asp Val Ser Gly Thr Gly Thr Leu Phe Gln Ser Tyr Gln
            740                 745                 750

Tyr Glu Val Cys Leu Thr Gly Gly Ser Glu Thr Gly Glu Phe Lys Phe
        755                 760                 765

Leu Lys Pro Ile Thr Pro His Leu Pro Pro His Arg Gly Gly Lys Glu
    770                 775                 780

Ile Glu Glu Asn Ser Thr Leu Pro Asn Ser Phe Gly Phe Asn Tyr
785                 790                 795

<210> 63
<211> 1303
<212> DNA
<213> Homo sapiens

<400> 63
atatccaatg ggctgattta tctgacggtc atggccatgg atgctggcaa cccccctctc 60
aacagcaccg tccctgtcac catcgaggtg tttgatgaga atgacaaccc tcccaccttc 120
agcaagcccg cctacttcgt ctccgtggtg gagaacatca tggcaggagc cacggtgctg 180
ttcctgaatg ccacagacct ggaccgctcc cgggagtacg gccaggagtc catcatctac 240
tccttggaag gctccaccca gtttcggatc aatgcccgct caggggaaat caccaccacg 300
tctctgcttg accgagagac caagtctgaa tacatcctca tcgttcgcgc agtggacggg 360
ggtgtgggcc acaaccagaa aactggcatc gccaccgtaa acatcaccct cctggacatc 420
aacgacaacc accccacgtg gaaggacgca ccctactaca tcaacctggt ggagatgacc 480
cctccagact ctgacgtgac cacggtggtg gctgttgacc cagacctggg ggagaatggc 540
accctggtgt acagcatcca gccacccaac aagttctaca gcctcaacag caccacgggc 600
aagatccgca ccacccacgc catgctggac cgggagaacc ccgaccccca tgaggccgag 660
ctgatgcgca aaatcgtcgt ctctgttact gactgtggca ggccccctct gaaagccacc 720
agcagtgcca cagtgtttgt gaacctcttg gatctcaatg acaatgaccc cacctttcag 780
aacctgcctt ttgtggccga ggtgcttgaa ggcatcccgg cgggggtctc catctaccaa 840
gtggtggcca tcgacctcga tgagggcctg aacggcctgg tgtcctaccg catgccggtg 900
ggcatgcccc gcatggactt cctcatcaac agcagcagcg gcgtggtggt caccaccacc 960
gagctggacc gcgagcgcat cgcggagtac cagctgcggg tggtggccag tgatgcaggc 1020
acgcccacca agagctccac cagcacgctc accatccatg tgctggatgt gaacgacgag 1080
acgcccacct tcttcccggc cgtgtacaat gtgtctgtgt ccgaggacgt gccacgcgag 1140
ttccgggtgg tctggctgaa ctgcacggac aacgacgtgg gcctcaatgc agagctcagc 1200
tacttcatca caggtgctgc cccggcctcc gcccacctgt gcaggcctcc tggggccctg 1260
cctccacccc tcccagatgg acagccagac taggtggggg cag 1303

<210> 64

<211> 420
<212> PRT
<213> Homo sapiens

<400> 64
Met Ala Met Asp Ala Gly Asn Pro Pro Leu Asn Ser Thr Val Pro Val
1               5                   10                  15

Thr Ile Glu Val Phe Asp Glu Asn Asp Asn Pro Pro Thr Phe Ser Lys
            20                  25                  30

Pro Ala Tyr Phe Val Ser Val Val Glu Asn Ile Met Ala Gly Ala Thr
        35                  40                  45

Val Leu Phe Leu Asn Ala Thr Asp Leu Asp Arg Ser Arg Glu Tyr Gly
    50                  55                  60

Gln Glu Ser Ile Ile Tyr Ser Leu Glu Gly Ser Thr Gln Phe Arg Ile
65                  70                  75                  80

Asn Ala Arg Ser Gly Glu Ile Thr Thr Thr Ser Leu Leu Asp Arg Glu
                85                  90                  95

Thr Lys Ser Glu Tyr Ile Leu Ile Val Arg Ala Val Asp Gly Gly Val
            100                 105                 110

Gly His Asn Gln Lys Thr Gly Ile Ala Thr Val Asn Ile Thr Leu Leu
        115                 120                 125

Asp Ile Asn Asp Asn His Pro Thr Trp Lys Asp Ala Pro Tyr Tyr Ile
    130                 135                 140

Asn Leu Val Glu Met Thr Pro Pro Asp Ser Asp Val Thr Thr Val Val
145                 150                 155                 160

Ala Val Asp Pro Asp Leu Gly Glu Asn Gly Thr Leu Val Tyr Ser Ile
                165                 170                 175

Gln Pro Pro Asn Lys Phe Tyr Ser Leu Asn Ser Thr Thr Gly Lys Ile
            180                 185                 190

Arg Thr Thr His Ala Met Leu Asp Arg Glu Asn Pro Asp Pro His Glu
        195                 200                 205

Ala Glu Leu Met Arg Lys Ile Val Val Ser Val Thr Asp Cys Gly Arg
    210                 215                 220

Pro Pro Leu Lys Ala Thr Ser Ser Ala Thr Val Phe Val Asn Leu Leu
225                 230                 235                 240

Asp Leu Asn Asp Asn Asp Pro Thr Phe Gln Asn Leu Pro Phe Val Ala
                245                 250                 255

Glu Val Leu Glu Gly Ile Pro Ala Gly Val Ser Ile Tyr Gln Val Val
            260                 265                 270

Ala Ile Asp Leu Asp Glu Gly Leu Asn Gly Leu Val Ser Tyr Arg Met
        275                 280                 285

Pro Val Gly Met Pro Arg Met Asp Phe Leu Ile Asn Ser Ser Ser Gly
    290                 295                 300

Val Val Val Thr Thr Thr Glu Leu Asp Arg Glu Arg Ile Ala Glu Tyr
305                 310                 315                 320

Gln Leu Arg Val Val Ala Ser Asp Ala Gly Thr Pro Thr Lys Ser Ser
                325                 330                 335

Thr Ser Thr Leu Thr Ile His Val Leu Asp Val Asn Asp Glu Thr Pro

340                    345                    350

Thr Phe Phe Pro Ala Val Tyr Asn Val Ser Val Ser Glu Asp Val Pro
        355                    360                    365

Arg Glu Phe Arg Val Val Trp Leu Asn Cys Thr Asp Asn Asp Val Gly
    370                    375                    380

Leu Asn Ala Glu Leu Ser Tyr Phe Ile Thr Gly Ala Ala Pro Ala Ser
385                    390                    395                    400

Ala His Leu Cys Arg Pro Pro Gly Ala Leu Pro Pro Pro Leu Pro Asp
                405                    410                    415

Gly Gln Pro Asp
            420

<210> 65
<211> 1278
<212> DNA
<213> Homo sapiens

<400> 65
tggtcatggc catggatgct ggcaaccccc ctctcaacag caccgtccct gtcaccatcg 60
aggtgtttga tgagaatgac aaccctccca ccttcagcaa gcccgcctac ttcgtctccg 120
tggtggagaa catcatggca ggagccacgg tgctgttcct gaatgccaca gacctggacc 180
gctcccggga gtacggccag gagtccatca tctactcctt ggaaggctcc acccagtttc 240
ggatcaatgc ccgctcaggg gaaatcacca ccacgtctct gcttgaccga gagaccaagt 300
ctgaatacat cctcatcgtt cgcgcagtgg acgggggtgt gggccacaac cagaaaactg 360
gcatcgccac cgtaaacatc accctcctgg acatcaatga caaccacccc acgtggaagg 420
acgcacccta ctacatcaac ctggtggaga tgacccctcc agactctgat gtgaccacgg 480
tggtggctgt tgacccagac ctgggggaga atggcaccct ggtgtacagc atccagccac 540
ccaacaagtt ctacagcctc aacagcacca cgggcaagat ccgcaccacc cacgccatgc 600
tggaccggga gaaccccgac ccccatgagg ccgagctgat gcgcaaaatc gtcgtctctg 660
ttactgactg tggcaggccc cctctgaaag ccaccagcag tgccacagtg tttgtgaacc 720
tcttggatct caatgacaat gaccccacct ttcagaacct gccttttgtg gccgaggtgc 780
ttgaaggcat cccgacgggg gtctccatct accaagtggt ggccatcgac ctcgatgagg 840
gcctgaacgg cctggtgtcc taccgcatgc cggtgggcat gccccgcatg gacttcctca 900
tcaacagcag cagcggcgtg gtggtcacca ccaccgagct ggaccgcgag cgcatcgcgg 960
agtaccagct gcgggtggtg gccagtgatg caggcacgcc caccaagagc tccaccagca 1020
cgctcaccat ccatgtgctg gatgtgaacg acgagacgcc caccttcttc ccggccgtgt 1080
acaatgtgtc tgtgtccgag gacgtgccac gcgagttccg ggtggtctgg ctgaactgca 1140
cggacaacga cgtgggcctc aatgcagagc tcagctactt catcacaggt gctgccccgg 1200
cctccgccca cctgtgcagg cctcctgggg ccctgcctcc acccctccca gatggacagc 1260
cagactaggt gggggcag                                                1278

<210> 66
<211> 420
<212> PRT
<213> Homo sapiens

<400> 66
Met Ala Met Asp Ala Gly Asn Pro Pro Leu Asn Ser Thr Val Pro Val
1               5                   10                  15

Thr Ile Glu Val Phe Asp Glu Asn Asp Asn Pro Pro Thr Phe Ser Lys
            20                  25                  30

Pro Ala Tyr Phe Val Ser Val Val Glu Asn Ile Met Ala Gly Ala Thr
        35                  40                  45

Val Leu Phe Leu Asn Ala Thr Asp Leu Asp Arg Ser Arg Glu Tyr Gly
    50                  55                  60

Gln Glu Ser Ile Ile Tyr Ser Leu Glu Gly Ser Thr Gln Phe Arg Ile
65                  70                  75                  80

Asn Ala Arg Ser Gly Glu Ile Thr Thr Thr Ser Leu Leu Asp Arg Glu
                85                  90                  95

Thr Lys Ser Glu Tyr Ile Leu Ile Val Arg Ala Val Asp Gly Gly Val
            100                 105                 110

Gly His Asn Gln Lys Thr Gly Ile Ala Thr Val Asn Ile Thr Leu Leu
        115                 120                 125

Asp Ile Asn Asp Asn His Pro Thr Trp Lys Asp Ala Pro Tyr Tyr Ile
    130                 135                 140

Asn Leu Val Glu Met Thr Pro Pro Asp Ser Asp Val Thr Thr Val Val
145                 150                 155                 160

Ala Val Asp Pro Asp Leu Gly Glu Asn Gly Thr Leu Val Tyr Ser Ile
                165                 170                 175

Gln Pro Pro Asn Lys Phe Tyr Ser Leu Asn Ser Thr Thr Gly Lys Ile
            180                 185                 190

Arg Thr Thr His Ala Met Leu Asp Arg Glu Asn Pro Asp Pro His Glu
        195                 200                 205

Ala Glu Leu Met Arg Lys Ile Val Val Ser Val Thr Asp Cys Gly Arg
    210                 215                 220

Pro Pro Leu Lys Ala Thr Ser Ser Ala Thr Val Phe Val Asn Leu Leu
225                 230                 235                 240

Asp Leu Asn Asp Asn Asp Pro Thr Phe Gln Asn Leu Pro Phe Val Ala
                245                 250                 255

Glu Val Leu Glu Gly Ile Pro Thr Gly Val Ser Ile Tyr Gln Val Val
            260                 265                 270

Ala Ile Asp Leu Asp Glu Gly Leu Asn Gly Leu Val Ser Tyr Arg Met
        275                 280                 285

Pro Val Gly Met Pro Arg Met Asp Phe Leu Ile Asn Ser Ser Ser Gly
    290                 295                 300

Val Val Val Thr Thr Thr Glu Leu Asp Arg Glu Arg Ile Ala Glu Tyr
305                 310                 315                 320

Gln Leu Arg Val Val Ala Ser Asp Ala Gly Thr Pro Thr Lys Ser Ser
                325                 330                 335

Thr Ser Thr Leu Thr Ile His Val Leu Asp Val Asn Asp Glu Thr Pro
            340                 345                 350

Thr Phe Phe Pro Ala Val Tyr Asn Val Ser Val Ser Glu Asp Val Pro
        355                 360                 365

Arg Glu Phe Arg Val Val Trp Leu Asn Cys Thr Asp Asn Asp Val Gly
    370                 375                 380

Leu Asn Ala Glu Leu Ser Tyr Phe Ile Thr Gly Ala Ala Pro Ala Ser
385                 390                 395                 400

Ala His Leu Cys Arg Pro Pro Gly Ala Leu Pro Pro Pro Leu Pro Asp
                405                 410                 415

Gly Gln Pro Asp
            420

<210> 67
<211> 1614

<212> DNA
<213> Homo sapiens

<400> 67
caagcaggat acgtttttct gttgggcatt gactagattg tttgcaaaag tttcgcatca 60
aaaacaaaca acaacaacaa aaaaccaaac aactctcctt gatctatact ttgagaattg 120
ttgatttctt tttttttatt ctgactttta aaaacaactt ttttttccac ttttttaaaa 180
aatgcactac tgtgtgctga gcgcttttct gatcctgcat ctggtcacgg tcgcgctcag 240
cctgtctacc tgcagcacac tcgatatgga ccagttcatg cgcaagagga tcgaggcgat 300
ccgcgggcag atcctgagca agctgaagct caccagtccc ccagaagact atcctgagcc 360
cgaggaagtc cccccggagg tgatttccat ctacaacagc accagggact tgctccagga 420
gaaggcgagc cggagggcgg ccgcctgcga gcgcgagagg agcgacgaag agtactactt 480
cagaattgtt cgatttgacg tctcagcaat ggagaagaat gcttccaatt tggtgaaagc 540
agagttcaga gtctttcgtt tgcagaaccc aaaagccaga gtgcctgaac aacggattga 600
gctatatcag attctcaagt ccaaagattt aacatctcca acccagcgct acatcgacag 660
caaagttgtg aaaacaagag cagaaggcga atggctctcc ttcgatgtaa ctgatgctgt 720
tcatgaatgg cttcaccata aagacaggaa cctgggattt aaaataagct tacactgtcc 780
ctgctgcact tttgtaccat ctaataatta catcatccca aataaaagtg aagaactaga 840
agcaagattt gcaggtattg atggcacctc cacatatacc agtggtgatc agaaaactat 900
aaagtccact aggaaaaaaa acagtgggaa gaccccacat ctcctgctaa tgttattgcc 960
ctcctacaga cttgagtcac aacagaccaa ccggcggaag aagcgtgctt tggatgcggc 1020
ctattgcttt agaaatgtgc aggataattg ctgcctacgt ccactttaca ttgatttcaa 1080
gagggatcta gggtggaaat ggatacacga acccaaaggg tacaatgcca acttctgtgc 1140
tggagcatgc ccgtatttat ggagttcaga cactcagcac agcagggtcc tgagcttata 1200
taataccata aatccagaag catctgcttc tccttgctgc gtgtcccaag atttagaacc 1260
tctaaccatt ctctactaca ttggcaaaac acccaagatt gaacagcttt ctaatatgat 1320
tgtaaagtct tgcaaatgca gctaaaattc ttggaaaagt ggcaagacca aaatgacaat 1380
gatgatgata atgatgatga cgacgacaac gatgatgctt gtaacaagaa aacataagag 1440
agccttggtt catcagtgtt aaaaaatttt tgaaaaggcg gtactagttc agacactttg 1500
gaagtttgtg ttctgtttgt taaaactggc atctgacaca aaaaaagttg aaggccttat 1560
tctacatttc acctactttg taagtgagag agacaagaag caaatttttt taaa 1614

<210> 68
<211> 387
<212> PRT
<213> Homo sapiens

<400> 68
Met His Tyr Cys Val Leu Ser Ala Phe Leu Ile Leu His Leu Val Thr
1               5                   10                  15

Val Ala Leu Ser Leu Ser Thr Cys Ser Thr Leu Asp Met Asp Gln Phe
            20                  25                  30

Met Arg Lys Arg Ile Glu Ala Ile Arg Gly Gln Ile Leu Ser Lys Leu
        35                  40                  45

Lys Leu Thr Ser Pro Pro Glu Asp Tyr Pro Glu Pro Glu Glu Val Pro
    50                  55                  60

Pro Glu Val Ile Ser Ile Tyr Asn Ser Thr Arg Asp Leu Leu Gln Glu
65                  70                  75                  80

Lys Ala Ser Arg Arg Ala Ala Ala Cys Glu Arg Glu Arg Ser Asp Glu
                85                  90                  95

Glu Tyr Tyr Phe Arg Ile Val Arg Phe Asp Val Ser Ala Met Glu Lys
            100                 105                 110

Asn Ala Ser Asn Leu Val Lys Ala Glu Phe Arg Val Phe Arg Leu Gln
        115                 120                 125

Asn Pro Lys Ala Arg Val Pro Glu Gln Arg Ile Glu Leu Tyr Gln Ile
    130                 135                 140

Leu Lys Ser Lys Asp Leu Thr Ser Pro Thr Gln Arg Tyr Ile Asp Ser
145                 150                 155                 160

Lys Val Val Lys Thr Arg Ala Glu Gly Glu Trp Leu Ser Phe Asp Val
                165                 170                 175

Thr Asp Ala Val His Glu Trp Leu His His Lys Asp Arg Asn Leu Gly
            180                 185                 190

Phe Lys Ile Ser Leu His Cys Pro Cys Cys Thr Phe Val Pro Ser Asn
        195                 200                 205

Asn Tyr Ile Ile Pro Asn Lys Ser Glu Glu Leu Glu Ala Arg Phe Ala
    210                 215                 220

Gly Ile Asp Gly Thr Ser Thr Tyr Thr Ser Gly Asp Gln Lys Thr Ile
225                 230                 235                 240

Lys Ser Thr Arg Lys Lys Asn Ser Gly Lys Thr Pro His Leu Leu Leu
                245                 250                 255

Met Leu Leu Pro Ser Tyr Arg Leu Glu Ser Gln Gln Thr Asn Arg Arg
            260                 265                 270

Lys Lys Arg Ala Leu Asp Ala Ala Tyr Cys Phe Arg Asn Val Gln Asp
        275                 280                 285

Asn Cys Cys Leu Arg Pro Leu Tyr Ile Asp Phe Lys Arg Asp Leu Gly
    290                 295                 300

Trp Lys Trp Ile His Glu Pro Lys Gly Tyr Asn Ala Asn Phe Cys Ala
305                 310                 315                 320

Gly Ala Cys Pro Tyr Leu Trp Ser Ser Asp Thr Gln His Ser Arg Val
                325                 330                 335

Leu Ser Leu Tyr Asn Thr Ile Asn Pro Glu Ala Ser Ala Ser Pro Cys
            340                 345                 350

Cys Val Ser Gln Asp Leu Glu Pro Leu Thr Ile Leu Tyr Tyr Ile Gly
        355                 360                 365

Lys Thr Pro Lys Ile Glu Gln Leu Ser Asn Met Ile Val Lys Ser Cys
    370                 375                 380

Lys Cys Ser
385


<210> 69
<211> 2939
<212> DNA
<213> Homo sapiens

<220>
<221> misc_feature
<222> (2759)
<223> Wherein n is a or c or t or g

<400> 69
   gaatggaggg aaggcctaga tggatgagtg gaaaggaaga tggggggagg gaaaggtgga 60
   tgaagagtca ggaggctggg cggcttcccc gactctaatt ctttgggtct cttctacccc 120
   agggtccccc cagctgcgcc tggtggctgg gcccagcaag tgctcaggtc gactggaggt 180
   gtggcatgac cagcgctggg ggaccgtgtg tgacgatagc tgggacatgc gggattcagc 240
   tgtggtctgc cgggagctgg gctgtggtgg acctcagcag ccagaccctg ctgccggccg 300
   cctggaggtc tggcacgggg gtcgctgggg gtcggtgtgt gacgacgcct gggacctgcg 360
   agacgccgct gtggcctgcc gagagctggg ctgcggaggg gcgctggccg cccccggggg 420
   cgccttcttt ggggaggggt ctggacccat catcctggac gaccttcggt gtcggggaaa 480
   cgagacggcc ttacgattct gcccagctcg gccctggggc cagcatgact gtcaccaccg 540
   cgaggacgcc ggggccgtgt gtgacggcat gcccctgggc tacgtccctc ccacggcccc 600
   cacggacagc aacaactcca cgcccaggga ggctgcctcc aggcccccgt ccaccatgac 660
   gagccaggct ccagggacgg caggcgtttc acctcctcca gcctccccta ctgtcctttg 720
   ggagcctgga ccggaagccg ggtcccccca gctgcgcctg gtggctgggc ccagcaagtg 780
   ctcaggtcga ctggaggtgt ggcatgacca gcgctggggg accgtgtgtg acgatagctg 840
   ggacatgcgg gattcagctg tggtctgccg ggagctgggc tgtggtggac ctcagcagcc 900
   agaccctgct gctggccgct ttggctgggg tgcggccccc atctggctag atgatgtgag 960
   ctgtgtgggg accgaggctt cactgtccga ctgccctgct gctccctggg gaaagcacaa 1020
   ctgcgctcac aatgaggatg ttggggtcac ctgcactggg cccccaggcc tggactccat 1080
   ctcagacccc ttcagctgga gctggattcc tggactgggg agagatcggg atgcctggct 1140
   cccgggagag ctggccacca agccctctgc aagtgtgact gccagtgttc tggagaaaac 1200
   aaccacgaag gccccaggga aaatgcctaa gagtactaag aagtgggtga caaaaaatgc 1260
   aaagagacca accactcaac ccccagtgat gccaaccacg aaacactcca gggcccaaag 1320
   ccccccagac ctaacttcac agaccactgc agcactgacc actgaggcct cccgaagacc 1380
   tacctctgag tttaccagaa ggccgaccac ggaggccccc cagagatgga cctctcacac 1440
   cactgccacg ctgacccctc aggccccccg agaacggacc actaagacca tggcaatgct 1500
   gaccactcaa ggcccccaag aaatgacctc tgagtccact atcaagagta tccctcaggc 1560
   ctccctggag ccatctgctg agatcccaga agggtctcca gagtcaccca aagacccggc 1620
   cccctctccc agtgttagca ccactgggga atcaggcctg ttccgggttc gtctggccga 1680
   tgggcccaac cgctgtgctg gccggctgga agtgtggcat gccggacgct ggggaacagt 1740
   gtgtgatgac aactggacct tgcggacgcc cactgtggcc tgctgggaac tgggctgtgg 1800
   aaaggtccgg cctcgagtag gcaaaaccca ttacggtcct gggactgggc ccatctggct 1860
   ggatgacatg ggctgtaagg gaagcgaggc ctcactgagc gactgcccct cgggggcttg 1920
   ggggaagcac aactgtgacc acgaggaaga cgtggggctc acctgcactg gctacacaga 1980
   ctatgacgat tatcccccct ggacctggga ccccacctca agagaggacc tggccaaggg 2040
   gactaccaca gcgggggtac ctggacacac tctcccctgg aggaccaccc ggcgcccggg 2100
   tagctcctcc ccagcaataa ggcgcctgcc ggacacaggc agcaaagatg gttacaagct 2160
   tccctggacg tgggacacac catcaggaag gggcctggct gaggggaccc ctaccgcagg 2220
   caaactagga ccaactcttg gggctggcac caccaggagc ccaggcagtc ctccaactcc 2280
   gagagtccat ggagacacag gttccccgag gaaaccgtgg cccgagcgcc ggccaccgcg 2340
   gcccgctgcg accaggacag cgcccccaac cccgtcccca ggtccctccg cctctccggg 2400
   acccccaggc ccagcgctga cctctgactc cagtcgagag ctcactcccc actcagcctt 2460
   gacgtccgag gcgacctctg acgctccgga cacttcacca cccaccccag acccggcctc 2520
   ccggacgaac cccgacctca tcttgacaag ccctgacttt gctttgtcca cccctgactc 2580
   cagtgtggtt cccgcgttga ccccggagcc ctcacccacg cccttaccca ccttgcccaa 2640
   agagctgacc tctgaccctt ctacaccgtc ggaggtgacc agcctttccc ctacctcaga 2700
   gcaggtccca gaatctgaca caaccccaga tttggacaca actccatact ccagtacang 2760
   actcctgacc ccaccacgac cccttacccc accactactc ctgatcccac cacgacccct 2820
   caccccacaa ctcctgaccc ttcctcaacc cctgtcatca ctactgtgtc ccttccaacc 2880
   tccttgggga cagaactctc ctctcccact ctagcaccaa cagtcaagcc cagtctgca 2939

<210> 70
<211> 964
<212> PRT
<213> Homo sapiens

<220>
<221> VARIANT
<222> (914)
<223> Wherein Xaa is any amino acid as defined in the
      specification.

<400> 70
Met Asp Glu Trp Lys Gly Arg Trp Gly Glu Gly Lys Val Asp Glu Glu
1               5                   10                  15

Ser Gly Gly Trp Ala Ala Ser Pro Thr Leu Ile Leu Trp Val Ser Ser
            20                  25                  30

Thr Pro Gly Ser Pro Gln Leu Arg Leu Val Ala Gly Pro Ser Lys Cys
        35                  40                  45

Ser Gly Arg Leu Glu Val Trp His Asp Gln Arg Trp Gly Thr Val Cys
    50                  55                  60

Asp Asp Ser Trp Asp Met Arg Asp Ser Ala Val Val Cys Arg Glu Leu
65                  70                  75                  80

Gly Cys Gly Gly Pro Gln Gln Pro Asp Pro Ala Ala Gly Arg Leu Glu
                85                  90                  95

Val Trp His Gly Gly Arg Trp Gly Ser Val Cys Asp Asp Ala Trp Asp
            100                 105                 110

Leu Arg Asp Ala Ala Val Ala Cys Arg Glu Leu Gly Cys Gly Gly Ala
        115                 120                 125

Leu Ala Ala Pro Gly Gly Ala Phe Phe Gly Glu Gly Ser Gly Pro Ile
    130                 135                 140

Ile Leu Asp Asp Leu Arg Cys Arg Gly Asn Glu Thr Ala Leu Arg Phe
145                 150                 155                 160

Cys Pro Ala Arg Pro Trp Gly Gln His Asp Cys His His Arg Glu Asp
                165                 170                 175

Ala Gly Ala Val Cys Asp Gly Met Pro Leu Gly Tyr Val Pro Pro Thr
            180                 185                 190

Ala Pro Thr Asp Ser Asn Asn Ser Thr Pro Arg Glu Ala Ala Ser Arg
        195                 200                 205

Pro Pro Ser Thr Met Thr Ser Gln Ala Pro Gly Thr Ala Gly Val Ser
    210                 215                 220

Pro Pro Pro Ala Ser Pro Thr Val Leu Trp Glu Pro Gly Pro Glu Ala
225                 230                 235                 240

Gly Ser Pro Gln Leu Arg Leu Val Ala Gly Pro Ser Lys Cys Ser Gly
                245                 250                 255

Arg Leu Glu Val Trp His Asp Gln Arg Trp Gly Thr Val Cys Asp Asp
            260                 265                 270

Ser Trp Asp Met Arg Asp Ser Ala Val Val Cys Arg Glu Leu Gly Cys
        275                 280                 285

Gly Gly Pro Gln Gln Pro Asp Pro Ala Ala Gly Arg Phe Gly Trp Gly
    290                 295                 300

Ala Gly Pro Ile Trp Leu Asp Asp Val Ser Cys Val Gly Thr Glu Ala
305                 310                 315                 320

Ser Leu Ser Asp Cys Pro Ala Ala Pro Trp Gly Lys His Asn Cys Ala
                325                 330                 335

His Asn Glu Asp Val Gly Val Thr Cys Thr Gly Pro Pro Gly Leu Asp
            340                 345                 350

Ser Ile Ser Asp Pro Phe Ser Trp Ser Trp Ile Pro Gly Leu Gly Arg
        355                 360                 365

Asp Arg Asp Ala Trp Leu Pro Gly Glu Leu Ala Thr Lys Pro Ser Ala
    370                 375                 380

Ser Val Thr Ala Ser Val Leu Glu Lys Thr Thr Thr Lys Ala Pro Gly
385                 390                 395                 400

Lys Met Pro Lys Ser Thr Lys Lys Trp Val Thr Lys Asn Ala Lys Arg
                405                 410                 415

Pro Thr Thr Gln Pro Pro Val Met Pro Thr Thr Lys His Ser Arg Ala
            420                 425                 430

Gln Ser Pro Pro Asp Leu Thr Ser Gln Thr Thr Ala Ala Leu Thr Thr
        435                 440                 445

Glu Ala Ser Arg Arg Pro Thr Ser Glu Phe Thr Arg Arg Pro Thr Thr
    450                 455                 460

Glu Ala Pro Gln Arg Trp Thr Ser His Thr Thr Ala Thr Leu Thr Pro
465                 470                 475                 480

Gln Ala Pro Arg Glu Arg Thr Thr Lys Thr Met Ala Met Leu Thr Thr
                485                 490                 495

Gln Gly Pro Gln Glu Met Thr Ser Glu Ser Thr Ile Lys Ser Ile Pro
            500                 505                 510

Gln Ala Ser Leu Glu Pro Ser Ala Glu Ile Pro Glu Gly Ser Pro Glu
        515                 520                 525

Ser Pro Lys Asp Pro Ala Pro Ser Pro Ser Val Ser Thr Thr Gly Glu
    530                 535                 540

Ser Gly Leu Phe Arg Val Arg Leu Ala Asp Gly Pro Asn Arg Cys Ala
545                 550                 555                 560

Gly Arg Leu Glu Val Trp His Ala Gly Arg Trp Gly Thr Val Cys Asp
                565                 570                 575

Asp Asn Trp Asp Leu Arg Asp Ala Thr Val Ala Cys Trp Glu Leu Gly
            580                 585                 590

Cys Gly Lys Val Arg Pro Arg Val Gly Lys Thr His Tyr Gly Pro Gly
        595                 600                 605

Thr Gly Pro Ile Trp Leu Asp Asp Met Gly Cys Lys Gly Ser Glu Ala
    610                 615                 620

Ser Leu Ser Asp Cys Pro Ser Gly Ala Trp Gly Lys His Asn Cys Asp
625                 630                 635                 640

His Glu Glu Asp Val Gly Leu Thr Cys Thr Gly Tyr Thr Asp Tyr Asp
                645                 650                 655

Asp Tyr Pro Pro Trp Thr Trp Asp Pro Thr Ser Arg Glu Asp Leu Ala
            660                 665                 670

Lys Gly Thr Thr Thr Ala Gly Val Pro Gly His Thr Leu Pro Trp Arg
        675                 680                 685

Thr Thr Arg Arg Pro Gly Ser Ser Ser Pro Ala Ile Arg Arg Leu Pro
    690                 695                 700

Asp Thr Gly Ser Lys Asp Gly Tyr Lys Leu Pro Trp Thr Trp Asp Thr
705                 710                 715                 720

Pro Ser Gly Arg Gly Leu Ala Glu Gly Thr Pro Thr Ala Gly Lys Leu
                725                 730                 735

Gly Pro Thr Leu Gly Ala Gly Thr Thr Arg Ser Pro Gly Ser Pro Pro
            740                 745                 750

Thr Pro Arg Val His Gly Asp Thr Gly Ser Pro Arg Lys Pro Trp Pro
        755                 760                 765

Glu Arg Arg Pro Pro Arg Pro Ala Ala Thr Arg Thr Ala Pro Pro Thr
    770                 775                 780

Pro Ser Pro Gly Pro Ser Ala Ser Pro Gly Pro Pro Gly Pro Ala Leu
785                 790                 795                 800

Thr Ser Asp Ser Ser Arg Glu Leu Thr Pro His Ser Ala Leu Thr Ser
                805                 810                 815

Glu Ala Thr Ser Asp Ala Pro Asp Thr Ser Pro Pro Thr Pro Asp Pro
            820                 825                 830

Ala Ser Arg Thr Asn Pro Asp Leu Ile Leu Thr Ser Pro Asp Phe Ala
        835                 840                 845

Leu Ser Thr Pro Asp Ser Ser Val Val Pro Ala Leu Thr Pro Glu Pro
    850                 855                 860

Ser Pro Thr Pro Leu Pro Thr Leu Pro Lys Glu Leu Thr Ser Asp Pro
865                 870                 875                 880

Ser Thr Pro Ser Glu Val Thr Ser Leu Ser Pro Thr Ser Glu Gln Val
                885                 890                 895

Pro Glu Ser Asp Thr Thr Pro Asp Leu Asp Thr Thr Pro Tyr Ser Ser
            900                 905                 910

Thr Xaa Leu Leu Thr Pro Pro Arg Pro Leu Thr Pro Pro Leu Leu Leu
        915                 920                 925

Ile Pro Pro Arg Pro Leu Thr Pro Gln Leu Leu Thr Leu Pro Gln Pro
    930                 935                 940

Leu Ser Ser Leu Leu Cys Pro Phe Gln Pro Pro Trp Gly Gln Asn Ser
945                 950                 955                 960

Pro Leu Pro Leu

<210> 71
<211> 400
<212> DNA
<213> Homo sapiens

<400> 71
tatggtaagg gtggaggagg ctttctgtgc tacctggaag ctgaccaaca gtcagaactt 60
tgatgagtac atgaaggctc taggcgtggg ctttgccact aggcaggtgg gaaatgtgac 120
caaaccaacg gtaattatca gtcaagaagg agacaaagtg gtcatcagga ctctcagcac 180
attcaagaac acggagatta gtttccagct gggagaagag tttgatgaaa ccactgcaga 240
tgatagaaac tgtaagtctg ttgttagcct ggatggagac aaacttgttc acatacagaa 300
atgggatggc aaagaaacaa attttgtaag agaaattaag gatggcaaaa tggttatgac 360
ccttactttt ggtgatgatg tggttgccgt tcaccactag                       400

<210> 72
<211> 132
<212> PRT
<213> Homo sapiens

<400> 72
Met Val Arg Val Glu Glu Ala Phe Cys Ala Thr Trp Lys Leu Thr Asn
  1               5                  10                  15

Ser Gln Asn Phe Asp Glu Tyr Met Lys Ala Leu Gly Val Gly Phe Ala
             20                  25                  30

Thr Arg Gln Val Gly Asn Val Thr Lys Pro Thr Val Ile Ile Ser Gln
         35                  40                  45

Glu Gly Asp Lys Val Val Ile Arg Thr Leu Ser Thr Phe Lys Asn Thr
     50                  55                  60

Glu Ile Ser Phe Gln Leu Gly Glu Glu Phe Asp Glu Thr Thr Ala Asp
 65                  70                  75                  80

Asp Arg Asn Cys Lys Ser Val Val Ser Leu Asp Gly Asp Lys Leu Val
                 85                  90                  95

His Ile Gln Lys Trp Asp Gly Lys Glu Thr Asn Phe Val Arg Glu Ile
            100                 105                 110

Lys Asp Gly Lys Met Val Met Thr Leu Thr Phe Gly Asp Asp Val Val
        115                 120                 125

Ala Val His His
    130

<210> 73
<211> 417
<212> DNA
<213> Homo sapiens

<400> 73
tataatggta agggtggagg aggctttctg tgctacctgg aggctgacca acagtcagaa 60
ctttgatgag tacatgaagg ctctaggcgt gggctttgcc actaggcagg tgggaaatgt 120
gaccaaacca acggtaatta tcagtcaaga aggagacaaa gtggtcatca ggactctcag 180
cacattcaag aacacggaga ttagtttcca gctgggagaa gagtttgatg aaaccactgc 240
agatgataga aactgtaagt ctgttgttag cctggatgga gacaaacttg ttcacataca 300
gaaatgggat ggcaaagaaa caaattttgt aagagaaatt aaggatggca aaatggttat 360
gacccttact tttggtgatg tggttgccgt tcaccactat aagaaggcat aaaaata    417

<210> 74
<211> 135
<212> PRT
<213> Homo sapiens

<400> 74
Met Val Arg Val Glu Glu Ala Phe Cys Ala Thr Trp Arg Leu Thr Asn
1               5                   10                  15

Ser Gln Asn Phe Asp Glu Tyr Met Lys Ala Leu Gly Val Gly Phe Ala
            20                  25                  30

Thr Arg Gln Val Gly Asn Val Thr Lys Pro Thr Val Ile Ile Ser Gln
        35                  40                  45

Glu Gly Asp Lys Val Val Ile Arg Thr Leu Ser Thr Phe Lys Asn Thr
    50                  55                  60

Glu Ile Ser Phe Gln Leu Gly Glu Glu Phe Asp Glu Thr Thr Ala Asp
65                  70                  75                  80

Asp Arg Asn Cys Lys Ser Val Val Ser Leu Asp Gly Asp Lys Leu Val
                85                  90                  95

His Ile Gln Lys Trp Asp Gly Lys Glu Thr Asn Phe Val Arg Glu Ile
            100                 105                 110

Lys Asp Gly Lys Met Val Met Thr Leu Thr Phe Gly Asp Val Val Ala
        115                 120                 125

Val His His Tyr Lys Lys Ala
    130                 135

<210> 75
<211> 1931
<212> DNA
<213> Homo sapiens

<400> 75
agcatgcgtt cagtatgtga tgatggggtt caaagccaag ccagcattgg cactggagtt 60
gatgttggct taaaacttgc cagtgtcagg tagaggaccc tgactaacgt gtgctctctc 120
cccttgcaca ggctatgcca ctgaagcatt atctcctttt gctggtgggc tgccaagcct 180
ggggtgcagg gttggcctac catggctgcc ctagcgagtg tacctgctcc agggcctccc 240
aggtggagtg caccggggca cgcattgtgg cggtgcccac ccctctgccc tggaacgcca 300
tgagcctgca gatcctcaac acgcacatca ctgaactcaa tgagtccccg ttcctcaata 360

tttcagccct catcgccctg aggattgaga agaatgagct gtcgcgcatc acgcctgggg 420
ccttccgaaa cctgggctcg ctgcgctatc tcagcctcgc caacaacaag ctgcaggttc 480
tgcccatcgg cctcttccag ggcctggaca gccttgagtc tctccttctg tccagtaacc 540
agctgttgca gatccagccg gcccacttct cccagtgcag caacctcaag gagctgcagt 600
tgcacggcaa ccacctggaa tacatccctg acggagcctt cgaccacctg gtaggactca 660
cgaagctcaa tctgggcaag aatagcctca cccacatctc acccagggtc ttccagcacc 720
tgggcaatct ccaggtcctc cggctgtatg agaacaggct cacggatatc cccatgggca 780
cttttgatgg gcttgttaac ctgcaggaac tggctctaca gcagaaccag attggactgc 840
tctcccctgg tctcttccac aacaaccaca acctccagag actctacctg tccaacaacc 900
acatctccca gctgccaccc agcatcttca tgcagctgcc ccagctcaac cgtcttactc 960
tctttgggaa ttccctgaag gagctctctc tggggatctt cgggcccatg cccaacctgc 1020
gggagctttg gctctatgac aaccacatct cttctctacc cgacaatgtc ttcagcaacc 1080
tccgccagtt gcaggtcctg attcttagcc gcaatcagat cagcttcatc tccccgggtg 1140
ccttcaacgg gctaacggag cttcgggagc tgtccctcca caccaacgca ctgcaggacc 1200
tggacgggaa tgtcttccgc atgttggcca acctgcagaa catctccctg cagaacaatc 1260
gcctcagaca gctcccaggg aatatcttcg ccaacgtcaa tggcctcatg gccatccagc 1320
tgcagaacaa ccagctggag aacttgcccc tcggcatctt cgatcacctg gggaaactgt 1380
gtgagctgcg gctgtatgac aatccctgga ggtgtgactc agacatcctt ccgctccgca 1440
actggctcct gctcaaccag cctaggttag ggacggacac tgtacctgtg tgtttcagcc 1500
cagccaatgt ccgaggccag tccctcatta tcatcaatgt caacgttgct gttccaagcg 1560
tccatgtacc tgaggtgcct agttacccag aaacaccatg gtacccagac acacccagtt 1620
accctgacac cacatccgtc tcttctacca ctgagctaac cagccctgtg gaagactaca 1680
ctgatctgac taccattcag gtcactgatg accgcagcgt ttggggcatg acccatgccc 1740
atagcgggct ggccattgcc gccattgtaa ttggcattgt cgccctggcc tgctccctgg 1800
ctgcctgcgt cggctgttgc tgctgcaaga agaggagcca agctgtcctg atgcagatga 1860
aggcacccaa tgagtgttaa agaggcaggc tggagcaggg ctggggaatg atgggactgg 1920
aggacctggg a 1931

<210> 76
<211> 581
<212> PRT
<213> Homo sapiens

<400> 76
Met Pro Leu Lys His Tyr Leu Leu Leu Leu Val Gly Cys Gln Ala Trp
1               5                   10                  15

Gly Ala Gly Leu Ala Tyr His Gly Cys Pro Ser Glu Cys Thr Cys Ser
            20                  25                  30

Arg Ala Ser Gln Val Glu Cys Thr Gly Ala Arg Ile Val Ala Val Pro
        35                  40                  45

Thr Pro Leu Pro Trp Asn Ala Met Ser Leu Gln Ile Leu Asn Thr His
    50                  55                  60

Ile Thr Glu Leu Asn Glu Ser Pro Phe Leu Asn Ile Ser Ala Leu Ile
65                  70                  75                  80

Ala Leu Arg Ile Glu Lys Asn Glu Leu Ser Arg Ile Thr Pro Gly Ala
                85                  90                  95

Phe Arg Asn Leu Gly Ser Leu Arg Tyr Leu Ser Leu Ala Asn Asn Lys
            100                 105                 110

Leu Gln Val Leu Pro Ile Gly Leu Phe Gln Gly Leu Asp Ser Leu Glu
        115                 120                 125

Ser Leu Leu Leu Ser Ser Asn Gln Leu Leu Gln Ile Gln Pro Ala His
    130                 135                 140

Phe Ser Gln Cys Ser Asn Leu Lys Glu Leu Gln Leu His Gly Asn His
145                 150                 155                 160

Leu Glu Tyr Ile Pro Asp Gly Ala Phe Asp His Leu Val Gly Leu Thr
                165                 170                 175

Lys Leu Asn Leu Gly Lys Asn Ser Leu Thr His Ile Ser Pro Arg Val

            180                 185                 190

Phe Gln His Leu Gly Asn Leu Gln Val Leu Arg Leu Tyr Glu Asn Arg
        195                 200                 205

Leu Thr Asp Ile Pro Met Gly Thr Phe Asp Gly Leu Val Asn Leu Gln
    210                 215                 220

Glu Leu Ala Leu Gln Gln Asn Gln Ile Gly Leu Leu Ser Pro Gly Leu
225                 230                 235                 240

Phe His Asn Asn His Asn Leu Gln Arg Leu Tyr Leu Ser Asn Asn His
                245                 250                 255

Ile Ser Gln Leu Pro Pro Ser Ile Phe Met Gln Leu Pro Gln Leu Asn
            260                 265                 270

Arg Leu Thr Leu Phe Gly Asn Ser Leu Lys Glu Leu Ser Leu Gly Ile
        275                 280                 285

Phe Gly Pro Met Pro Asn Leu Arg Glu Leu Trp Leu Tyr Asp Asn His
    290                 295                 300

Ile Ser Ser Leu Pro Asp Asn Val Phe Ser Asn Leu Arg Gln Leu Gln
305                 310                 315                 320

Val Leu Ile Leu Ser Arg Asn Gln Ile Ser Phe Ile Ser Pro Gly Ala
                325                 330                 335

Phe Asn Gly Leu Thr Glu Leu Arg Glu Leu Ser Leu His Thr Asn Ala
            340                 345                 350

Leu Gln Asp Leu Asp Gly Asn Val Phe Arg Met Leu Ala Asn Leu Gln
        355                 360                 365

Asn Ile Ser Leu Gln Asn Asn Arg Leu Arg Gln Leu Pro Gly Asn Ile
    370                 375                 380

Phe Ala Asn Val Asn Gly Leu Met Ala Ile Gln Leu Gln Asn Asn Gln
385                 390                 395                 400

Leu Glu Asn Leu Pro Leu Gly Ile Phe Asp His Leu Gly Lys Leu Cys
                405                 410                 415

Glu Leu Arg Leu Tyr Asp Asn Pro Trp Arg Cys Asp Ser Asp Ile Leu
            420                 425                 430

Pro Leu Arg Asn Trp Leu Leu Leu Asn Gln Pro Arg Leu Gly Thr Asp
        435                 440                 445

Thr Val Pro Val Cys Phe Ser Pro Ala Asn Val Arg Gly Gln Ser Leu
    450                 455                 460

Ile Ile Ile Asn Val Asn Val Ala Val Pro Ser Val His Val Pro Glu
465                 470                 475                 480

Val Pro Ser Tyr Pro Glu Thr Pro Trp Tyr Pro Asp Thr Pro Ser Tyr
                485                 490                 495

Pro Asp Thr Thr Ser Val Ser Ser Thr Thr Glu Leu Thr Ser Pro Val
            500                 505                 510

Glu Asp Tyr Thr Asp Leu Thr Thr Ile Gln Val Thr Asp Asp Arg Ser
        515                 520                 525

Val Trp Gly Met Thr His Ala His Ser Gly Leu Ala Ile Ala Ala Ile
    530                 535                 540

Val Ile Gly Ile Val Ala Leu Ala Cys Ser Leu Ala Ala Cys Val Gly

545                 550                 555                 560

Cys Cys Cys Cys Lys Lys Arg Ser Gln Ala Val Leu Met Gln Met Lys
                565                 570                 575

Ala Pro Asn Glu Cys
            580


<210> 77
<211> 2189
<212> DNA
<213> Homo sapiens

<400> 77
   ccggagacga tgcgcccccc ttcaagcctt ctcattattg ctgttctctg tgcactggcc 60
   aggcaagcgg gctggcaacc acgtttcgtt aaccaccttt attcttgccg agggcagagc 120
   ctcgcttcgg tgcccagcag cctcccgccc cacgcccgga tgctcaccct ggatgccaac 180
   cctctcaaga ccctgtggaa tcactccctc cagccttacc ctctcctgga gagcctcagc 240
   ctgcacagct gccacctgga gcgcatcagc cgcggcgcct tccaggagca aggtcacctg 300
   cgcagcctgg tcctggggga caactgcctc tcagagaact acgaagagac ggcagccgcc 360
   ctccacgccc tgccgggcct gcggaggctg gacttgtcag gaaacgccct gacggaggac 420
   atggcagccc tcatgctcca gaacctctcc tcgctgcggt ccgtgtccct ggcggggaac 480
   accatcatgc ggctggacga ctccgtcttc gagggcctgg agcgtctccg ggagctggat 540
   ctgcagagga actacatctt cgagatcgag ggcggcgctt tcgacggcct ggctgagctg 600
   aggcacctca acctggcctt caacaacctc ccctgcatcg tggacttcgg gctcacgcgg 660
   ctgcgggtcc tcaacgtcag ctacaacgtc ctggagtggt tcctcgcgac cgggggagag 720
   gctgccttcg agctggagac gctggacctg tctcacaacc agctgctgtt cttcccgctg 780
   ctgccccagt acagcaagtt gcggaccctc ctgctgcgcg acaacaacat gggcttctac 840
   cgggacctgt acaacacctc gtcgccgagg gagatggtgg cccagttcct cctcgtggac 900
   ggcaacgtga ccaacatcac caccgtcagc ctctgggaag aattctcctc cagcgacctc 960
   gcagatctcc gcttcctgga catgagccag aaccagttcc agtacctgcc agacggcttc 1020
   ctgaggaaaa tgccttccct ctcccacctg aacctccacc agaattgcct gatgacgctt 1080
   cacattcggg agcacgagcc ccccggagcg ctcaccgagc tggacctgag ccacaaccag 1140
   ctgtcggagc tgcacctggc tccggggctg gccagctgcc tgggcagcct gcgcttgttc 1200
   aacctgagct ccaaccagct cctgggcgtc ccccctggcc tcttcgccaa tgctaggaac 1260
   atcactacac ttgacatgag ccacaatcag atctcacttt gtcccctgcc agctgcctcg 1320
   gaccgggtgg gcccccctag ctgtgtggat ttcaggaata tggcatcttt aaggagcctg 1380
   tctctggagg gctgtggcct gggggcattg ccagactgcc cattccaagg gacctccctg 1440
   acctacttag acctctcaag caactggggg gttctgaatg ggagcctcgc cccactccag 1500
   gatgttgccc ccatgttaca ggtcctgtct ctcaggaaca tgggcctcca ctccagcttt 1560
   atggcgttgg acttctctgg gtttgggaat ctcagggact tagatctgtc ggggaattgc 1620
   ttgaccacct tcccaaggtt tgggggcagc ctggccctgg agaccctgga tctccgtaga 1680
   aactcgctca cagcccttcc ccagaaggct gtgtctgagc agctctcgag aggtctgcgg 1740
   accatctacc tcagtcagaa tccatatgac tgctgtgggg tggatggctg gggggccctg 1800
   cagcatgggc agacggtggc cgactgggcc atggtcacct gcaacctctc ctccaagatc 1860
   atccgcgtga cggagctgcc cggaggtgtg cctcgggact gcaagtggga gcggctggac 1920
   ctgggcctgc tctacctcgt gctcatcctc cccagctgcc tcaccctgct ggtggcctgc 1980
   actgtcatcg tcctcacttt taagaagcct ctgcttcagg tcatcaagag ccgctgccac 2040
   tggtcctccg tttactgacc tggctgtgtg ccaagactcg aaattcggtc cgcacacaac 2100
   aggacacttt ctctgccagc tttcaagatg tgatgcagag gccaagtctg acgaattgaa 2160
   gtttcaatta aaatttaata tgtttccat                                   2189


<210> 78
<211> 682
<212> PRT
<213> Homo sapiens

<400> 78
Met Arg Pro Pro Ser Ser Leu Leu Ile Ile Ala Val Leu Cys Ala Leu
1               5                   10                  15

Ala Arg Gln Ala Gly Trp Gln Pro Arg Phe Val Asn His Leu Tyr Ser
            20                  25                  30

Cys Arg Gly Gln Ser Leu Ala Ser Val Pro Ser Ser Leu Pro Pro His
        35                  40                  45

Ala Arg Met Leu Thr Leu Asp Ala Asn Pro Leu Lys Thr Leu Trp Asn
    50                  55                  60

His Ser Leu Gln Pro Tyr Pro Leu Leu Glu Ser Leu Ser Leu His Ser
65                  70                  75                  80

Cys His Leu Glu Arg Ile Ser Arg Gly Ala Phe Gln Glu Gln Gly His
                85                  90                  95

Leu Arg Ser Leu Val Leu Gly Asp Asn Cys Leu Ser Glu Asn Tyr Glu
            100                 105                 110

Glu Thr Ala Ala Ala Leu His Ala Leu Pro Gly Leu Arg Arg Leu Asp
        115                 120                 125

Leu Ser Gly Asn Ala Leu Thr Glu Asp Met Ala Ala Leu Met Leu Gln
    130                 135                 140

Asn Leu Ser Ser Leu Arg Ser Val Ser Leu Ala Gly Asn Thr Ile Met
145                 150                 155                 160

Arg Leu Asp Asp Ser Val Phe Glu Gly Leu Glu Arg Leu Arg Glu Leu
                165                 170                 175

Asp Leu Gln Arg Asn Tyr Ile Phe Glu Ile Glu Gly Gly Ala Phe Asp
            180                 185                 190

Gly Leu Ala Glu Leu Arg His Leu Asn Leu Ala Phe Asn Asn Leu Pro
        195                 200                 205

Cys Ile Val Asp Phe Gly Leu Thr Arg Leu Arg Val Leu Asn Val Ser
    210                 215                 220

Tyr Asn Val Leu Glu Trp Phe Leu Ala Thr Gly Gly Glu Ala Ala Phe
225                 230                 235                 240

Glu Leu Glu Thr Leu Asp Leu Ser His Asn Gln Leu Leu Phe Phe Pro
                245                 250                 255

Leu Leu Pro Gln Tyr Ser Lys Leu Arg Thr Leu Leu Leu Arg Asp Asn
            260                 265                 270

Asn Met Gly Phe Tyr Arg Asp Leu Tyr Asn Thr Ser Ser Pro Arg Glu
        275                 280                 285

Met Val Ala Gln Phe Leu Leu Val Asp Gly Asn Val Thr Asn Ile Thr
    290                 295                 300

Thr Val Ser Leu Trp Glu Glu Phe Ser Ser Ser Asp Leu Ala Asp Leu
305                 310                 315                 320

Arg Phe Leu Asp Met Ser Gln Asn Gln Phe Gln Tyr Leu Pro Asp Gly
                325                 330                 335

Phe Leu Arg Lys Met Pro Ser Leu Ser His Leu Asn Leu His Gln Asn
            340                 345                 350

Cys Leu Met Thr Leu His Ile Arg Glu His Glu Pro Pro Gly Ala Leu
        355                 360                 365

Thr Glu Leu Asp Leu Ser His Asn Gln Leu Ser Glu Leu His Leu Ala
    370                 375                 380

Pro Gly Leu Ala Ser Cys Leu Gly Ser Leu Arg Leu Phe Asn Leu Ser
385                 390                 395                 400

Ser Asn Gln Leu Leu Gly Val Pro Pro Gly Leu Phe Ala Asn Ala Arg
                405                 410                 415

Asn Ile Thr Thr Leu Asp Met Ser His Asn Gln Ile Ser Leu Cys Pro
            420                 425                 430

Leu Pro Ala Ala Ser Asp Arg Val Gly Pro Pro Ser Cys Val Asp Phe
        435                 440                 445

Arg Asn Met Ala Ser Leu Arg Ser Leu Ser Leu Glu Gly Cys Gly Leu
    450                 455                 460

Gly Ala Leu Pro Asp Cys Pro Phe Gln Gly Thr Ser Leu Thr Tyr Leu
465                 470                 475                 480

Asp Leu Ser Ser Asn Trp Gly Val Leu Asn Gly Ser Leu Ala Pro Leu
                485                 490                 495

Gln Asp Val Ala Pro Met Leu Gln Val Leu Ser Leu Arg Asn Met Gly
            500                 505                 510

Leu His Ser Ser Phe Met Ala Leu Asp Phe Ser Gly Phe Gly Asn Leu
        515                 520                 525

Arg Asp Leu Asp Leu Ser Gly Asn Cys Leu Thr Thr Phe Pro Arg Phe
    530                 535                 540

Gly Gly Ser Leu Ala Leu Glu Thr Leu Asp Leu Arg Arg Asn Ser Leu
545                 550                 555                 560

Thr Ala Leu Pro Gln Lys Ala Val Ser Glu Gln Leu Ser Arg Gly Leu
                565                 570                 575

Arg Thr Ile Tyr Leu Ser Gln Asn Pro Tyr Asp Cys Cys Gly Val Asp
            580                 585                 590

Gly Trp Gly Ala Leu Gln His Gly Gln Thr Val Ala Asp Trp Ala Met
        595                 600                 605

Val Thr Cys Asn Leu Ser Ser Lys Ile Ile Arg Val Thr Glu Leu Pro
    610                 615                 620

Gly Gly Val Pro Arg Asp Cys Lys Trp Glu Arg Leu Asp Leu Gly Leu
625                 630                 635                 640

Leu Tyr Leu Val Leu Ile Leu Pro Ser Cys Leu Thr Leu Leu Val Ala
                645                 650                 655

Cys Thr Val Ile Val Leu Thr Phe Lys Lys Pro Leu Leu Gln Val Ile
            660                 665                 670

Lys Ser Arg Cys His Trp Ser Ser Val Tyr
        675                 680


<210> 79
<211> 2484
<212> DNA
<213> Homo sapiens

<400> 79
cggccgccgc agccatgacc gtggagttcg agaagtgcgt caagaactcc ccgcgcttca 60
gggcgaccat tgacgaggtg gagacggacg tggtggagat tgaggccaaa ctggacaagc 120
tggtgaagct gtgcagtggc atggtggaag ccggtaaggc ctacgtcagc accagcaggc 180
ttttcgtgag cggcgtccgc gacctgtccc agcagtgcca gggcgacacc gtcatctcgg 240
aatgtctgca gaggttcgct gacagcctac aggaggtggt gaactaccac atgatcctgt 300
ttgaccaggc ccagaggtcc gtgcggcagc agctccagag ctttgtcaaa gaggatgtgc 360
ggaagttcaa ggagacaaag aagcagtttg acaaggtgcg ggaggacctg gagctgtccc 420
tggtgaggaa cgcccaggcc ccgaggcacc ggccccacga ggtggaggaa gccaccgggg 480
ccctcaccct caccaggaag tgcttccgcc acctggcact ggactatgtg ctccagatca 540
atgttctgca ggccaagaag aagtttgaga tcctggactc tatgctgtcc ttcatgcacg 600
cccagtccag cttcttccag cagggctaca gcctcctgca ccagctggac ccctacatga 660

agaagctggc agccgagctg gaccagctgg tgatcgactc tgcggtggaa aagcgtgaga 720
tggagcgaaa gcacgccgcc atccagcagc ggacccttag ggacttctcc tacgatgagt 780
ccaaagtgga gtttgacgtg gacgcgccca gtggggtggt gatggagggc tacctcttca 840
agagggccag caacgctttc aagacatgga accggcgctg gttctccatt cagaacagcc 900
agctggtcta ccagaagaag ctcaaggatg ccctcaccgt ggtggtggat gacctccgcc 960
tgtgctctgt gaagccgtgt gaggacatcg agcggaggtt ctgcttcgag gtgctgtcac 1020
ccaccaagag ctgcatgctg caggctgact ccgagaagct gcggcaagcc tgggtccagg 1080
ctgtgcaggc cagcatcgcc tccgcctacc gcgagagccc tgacagttgc tatagcgaga 1140
ggctggaccg cacagcatcc ccgtccacga gcagcatcga ctccgccacc gacactcggg 1200
agcgtggcgt gaagggcgag agtgtgctgc agcgtgtgca gagtgtggcc ggcaacagcc 1260
agtgcggcga ctgcggccag ccggaccccc gctgggccag catcaacctg ggcgtgctgc 1320
tctgcattga gtgctccggc atccacagga gcctgggtgt ccactgctcc aaggtgcggt 1380
ccctgacgct ggactcgtgg gagcctgagc tgctaaagct gatgtgtgag cttggaaaca 1440
gcgctgtgaa tcagatctat gaggcccagt gtgagggtgc aggcagcagg aaacccacag 1500
ccagcagctc ccggcaggac aaggaggcct ggatcaagga caaatacgtg gaaaagaagt 1560
ttctgcggaa ggcgcccatg gcaccagccc tggaggcccc aagacgctgg agggtgcaga 1620
agtgcctgcg gccccacagc tctccccgcg ctcccactgc ccgccgcaag gtccggcttg 1680
agcccgttct gccctgtgtg gccgctctgt cctcagaggg tgcagagtcg gaggagtcca 1740
gcggtgaggc agacggggac actgaggccg aggcctgggg cctggcggac gtgcgcgagc 1800
tgcacccggg gctcttggcg caccgcgcag cgcgtgcccg cgaccttcct gcgctggcgg 1860
cggcgctggc ccacggggcc gaggtcaact gggcggacgc ggaggatgag ggcaagacgc 1920
cgctggtgca ggccgtgcta gggggctcct tgatcgtctg tgagttcctg ctgcaaaacg 1980
gagcggacgt gaaccaaaga gacagccggg gccgggcgcc cctgcaccac gccacgctgc 2040
tgggccgcac cggccaggtt tgcctgttcc tgaagcgggg cgcggaccag cacgccctgg 2100
accaagagca gcgggacccg ttggccatcg cagtgcaggc ggccaacgct gacatcgtga 2160
cactgctccg tctggcgcgc atggcggagg aaatgcgcga ggccgaggct gcccctggtc 2220
ccccgggcgc cctggcgggc agccccacgg agctccagtt ccgcaggtgt atccaggagt 2280
tcatcagcct ccacctggaa gagagctagg gccgggcagg ccgggcagct gccaccccgc 2340
ccggcccgac gccccgcatg ccccgaagtc cctggcgccc acccggccgc ggccctgcgt 2400
gtgacccgcg ggtcgatacc tggcagcccc agtgctgggg cgccgcggcc ctgctcgccc 2460
aggaggagag cgagggcccc acac 2484

<210> 80
<211> 764
<212> PRT
<213> Homo sapiens

<400> 80
Met Thr Val Glu Phe Glu Lys Cys Val Lys Asn Ser Pro Arg Phe Arg
1               5                   10                  15

Ala Thr Ile Asp Glu Val Glu Thr Asp Val Val Glu Ile Glu Ala Lys
            20                  25                  30

Leu Asp Lys Leu Val Lys Leu Cys Ser Gly Met Val Glu Ala Gly Lys
        35                  40                  45

Ala Tyr Val Ser Thr Ser Arg Leu Phe Val Ser Gly Val Arg Asp Leu
    50                  55                  60

Ser Gln Gln Cys Gln Gly Asp Thr Val Ile Ser Glu Cys Leu Gln Arg
65                  70                  75                  80

Phe Ala Asp Ser Leu Gln Glu Val Val Asn Tyr His Met Ile Leu Phe
                85                  90                  95

Asp Gln Ala Gln Arg Ser Val Arg Gln Gln Leu Gln Ser Phe Val Lys
            100                 105                 110

Glu Asp Val Arg Lys Phe Lys Glu Thr Lys Lys Gln Phe Asp Lys Val
        115                 120                 125

Arg Glu Asp Leu Glu Leu Ser Leu Val Arg Asn Ala Gln Ala Pro Arg
    130                 135                 140

His Arg Pro His Glu Val Glu Glu Ala Thr Gly Ala Leu Thr Leu Thr
145                 150                 155                 160

Arg Lys Cys Phe Arg His Leu Ala Leu Asp Tyr Val Leu Gln Ile Asn
                165                 170                 175

Val Leu Gln Ala Lys Lys Lys Phe Glu Ile Leu Asp Ser Met Leu Ser
            180                 185                 190

Phe Met His Ala Gln Ser Ser Phe Phe Gln Gln Gly Tyr Ser Leu Leu
        195                 200                 205

His Gln Leu Asp Pro Tyr Met Lys Lys Leu Ala Ala Glu Leu Asp Gln
    210                 215                 220

Leu Val Ile Asp Ser Ala Val Glu Lys Arg Glu Met Glu Arg Lys His
225                 230                 235                 240

Ala Ala Ile Gln Gln Arg Thr Leu Arg Asp Phe Ser Tyr Asp Glu Ser
                245                 250                 255

Lys Val Glu Phe Asp Val Asp Ala Pro Ser Gly Val Val Met Glu Gly
            260                 265                 270

Tyr Leu Phe Lys Arg Ala Ser Asn Ala Phe Lys Thr Trp Asn Arg Arg
        275                 280                 285

Trp Phe Ser Ile Gln Asn Ser Gln Leu Val Tyr Gln Lys Lys Leu Lys
    290                 295                 300

Asp Ala Leu Thr Val Val Val Asp Asp Leu Arg Leu Cys Ser Val Lys
305                 310                 315                 320

Pro Cys Glu Asp Ile Glu Arg Arg Phe Cys Phe Glu Val Leu Ser Pro
                325                 330                 335

Thr Lys Ser Cys Met Leu Gln Ala Asp Ser Glu Lys Leu Arg Gln Ala
            340                 345                 350

Trp Val Gln Ala Val Gln Ala Ser Ile Ala Ser Ala Tyr Arg Glu Ser
        355                 360                 365

Pro Asp Ser Cys Tyr Ser Glu Arg Leu Asp Arg Thr Ala Ser Pro Ser
    370                 375                 380

Thr Ser Ser Ile Asp Ser Ala Thr Asp Thr Arg Glu Arg Gly Val Lys
385                 390                 395                 400

Gly Glu Ser Val Leu Gln Arg Val Gln Ser Val Ala Gly Asn Ser Gln
                405                 410                 415

Cys Gly Asp Cys Gly Gln Pro Asp Pro Arg Trp Ala Ser Ile Asn Leu
            420                 425                 430

Gly Val Leu Leu Cys Ile Glu Cys Ser Gly Ile His Arg Ser Leu Gly
        435                 440                 445

Val His Cys Ser Lys Val Arg Ser Leu Thr Leu Asp Ser Trp Glu Pro
    450                 455                 460

Glu Leu Leu Lys Leu Met Cys Glu Leu Gly Asn Ser Ala Val Asn Gln
465                 470                 475                 480

Ile Tyr Glu Ala Gln Cys Glu Gly Ala Gly Ser Arg Lys Pro Thr Ala
                485                 490                 495

Ser Ser Ser Arg Gln Asp Lys Glu Ala Trp Ile Lys Asp Lys Tyr Val
            500                 505                 510

Glu Lys Lys Phe Leu Arg Lys Ala Pro Met Ala Pro Ala Leu Glu Ala
        515                 520                 525

Pro Arg Arg Trp Arg Val Gln Lys Cys Leu Arg Pro His Ser Ser Pro
    530                 535                 540

Arg Ala Pro Thr Ala Arg Arg Lys Val Arg Leu Glu Pro Val Leu Pro
545                 550                 555                 560

Cys Val Ala Ala Leu Ser Ser Glu Gly Ala Glu Ser Glu Glu Ser Ser
                565                 570                 575

Gly Glu Ala Asp Gly Asp Thr Glu Ala Glu Ala Trp Gly Leu Ala Asp
            580                 585                 590

Val Arg Glu Leu His Pro Gly Leu Leu Ala His Arg Ala Ala Arg Ala
        595                 600                 605

Arg Asp Leu Pro Ala Leu Ala Ala Ala Leu Ala His Gly Ala Glu Val
    610                 615                 620

Asn Trp Ala Asp Ala Glu Asp Glu Gly Lys Thr Pro Leu Val Gln Ala
625                 630                 635                 640

Val Leu Gly Gly Ser Leu Ile Val Cys Glu Phe Leu Leu Gln Asn Gly
                645                 650                 655

Ala Asp Val Asn Gln Arg Asp Ser Arg Gly Arg Ala Pro Leu His His
            660                 665                 670

Ala Thr Leu Leu Gly Arg Thr Gly Gln Val Cys Leu Phe Leu Lys Arg
        675                 680                 685

Gly Ala Asp Gln His Ala Leu Asp Gln Glu Gln Arg Asp Pro Leu Ala
    690                 695                 700

Ile Ala Val Gln Ala Ala Asn Ala Asp Ile Val Thr Leu Leu Arg Leu
705                 710                 715                 720

Ala Arg Met Ala Glu Glu Met Arg Glu Ala Glu Ala Ala Pro Gly Pro
                725                 730                 735

Pro Gly Ala Leu Ala Gly Ser Pro Thr Glu Leu Gln Phe Arg Arg Cys
            740                 745                 750

Ile Gln Glu Phe Ile Ser Leu His Leu Glu Glu Ser
        755                 760


<210> 81
<211> 1930
<212> DNA
<213> Homo sapiens

<400> 81
agtcgggacc gccagtcggg gcgccgggac catggcgctg cgcgcccggg cgctgtacga 60
cttcaggtcg gagaacccag gagagatctc gctgcgagag cacgaggtgc tgagcctgtg 120
cagcgagcag gacatcgagg gctggctcga gggggtcaac agccgcggcg accgcggcct 180
cttcccggcc tcctatgtgc aggtgatccg cgcccccgag cctggcccgg cgggagacgg 240
cggcccgggc gccccggccc gctacgccaa tgtgcccccc gggggcttcg agcccctgcc 300
cgtcgcgccc cccgcctcct tcaagccgcc gcctgacgcc ttccaggcgc tgctgcagcc 360
acagcaggcg ccgcctccga gcaccttcca gccgcccggc gcgggcttcc cgtacggcgg 420
gggcgccctg cagccgtcgc ctcagcagct ctacggcggc taccaggcca gccaaggcag 480
cgatgatgac tgggacgacg agtgggacga cagctccacg gtggcggacg agccgggcgc 540
tctgggcagc ggagcatacc cggacctcga cggctcgtct tcggcgggtg tgggcgcagc 600
cggccgctac cgcctgtcca cgcgctccga cctgtccctg ggctcccgcg gcggctcggt 660
ccccccgcag caccacccgt cggggcccaa gagctcggcc accgtgagcc gcaacctcaa 720
tcgcttctcc accttcgtca agtccggcgg ggaggccttc gtgctggggg aggcgtcagg 780
cttcgtgaag gacggggaca agctgtgcgt ggtgctgggg ccctatggcc ccgagtggca 840
ggagaacccc tacccgttcc agtgcaccat cgacgacccc accaagcaga ccaagttcaa 900
gggcatgaag agctacatct cctacaagct ggtgcccacg cacacgcagg tgccggtgca 960
tcggcgctac aagcacttcg actggctgta cgcgcgcctg gcggagaagt tcccggtcat 1020

ctccgtgccc cacctgcccg agaagcaggc caccggccgc ttcgaggagg acttcatctc 1080
taagcgcagg aagggcctga tctggtggat gaaccacatg gccagccacc cagtgctggc 1140
gcagtgcgac gtcttccagc acttcctgac gtgccccagc agcaccgacg agaaagcctg 1200
gaagcagggc aagaggaagg ccgagaagga cgagatggtg ggcgccaact tcttcctgac 1260
ccttagcacg ccccccgccg ctgcccttga cctgcaggag gtggagagca agatagacgg 1320
cttcaagtgc ttcaccaaga agatggacga cagcgcgctg cagctcaacc acacggccaa 1380
cgagttcgcg cgcaagcagg tgaccggctt caaaaaggag tatcagaagg tgggccagtc 1440
cttccgcggc ctcagccagg cctttgagct ggaccagcag gccttctcgg tgggcctgaa 1500
ccaggctatc gccttcaccg gagatgccta tgacgccatt ggcgagctct tcgcggagca 1560
gcccaggcag gacctggatc ccgtcatgga cctattagcg ctgtatcagg ggcatctggc 1620
taacttcccg gacatcatcc acgttcagaa aggagctctt accaaagtca aggagagtag 1680
gcgacacgtg gaggaaggga agatggaggt gcagaaggct gacggcattc aggatcgctg 1740
taacactatt tcttttgcca ctttggctga aattcaccac ttccatcaaa ttcgagtgag 1800
agactttaaa tcacagatgc agcatttctt acaacaacaa ataatatttt tccaaaaagt 1860
tacccagaag ttggaagaag ctcttcacaa atatgatagt gtttaatgac tggacgttgg 1920
attatggact 1930

<210> 82
<211> 624
<212> PRT
<213> Homo sapiens

<400> 82
Met Ala Leu Arg Ala Arg Ala Leu Tyr Asp Phe Arg Ser Glu Asn Pro
1               5                   10                  15

Gly Glu Ile Ser Leu Arg Glu His Glu Val Leu Ser Leu Cys Ser Glu
            20                  25                  30

Gln Asp Ile Glu Gly Trp Leu Glu Gly Val Asn Ser Arg Gly Asp Arg
        35                  40                  45

Gly Leu Phe Pro Ala Ser Tyr Val Gln Val Ile Arg Ala Pro Glu Pro
    50                  55                  60

Gly Pro Ala Gly Asp Gly Gly Pro Gly Ala Pro Ala Arg Tyr Ala Asn
65                  70                  75                  80

Val Pro Pro Gly Gly Phe Glu Pro Leu Pro Val Ala Pro Pro Ala Ser
                85                  90                  95

Phe Lys Pro Pro Pro Asp Ala Phe Gln Ala Leu Leu Gln Pro Gln Gln
            100                 105                 110

Ala Pro Pro Pro Ser Thr Phe Gln Pro Pro Gly Ala Gly Phe Pro Tyr
        115                 120                 125

Gly Gly Gly Ala Leu Gln Pro Ser Pro Gln Gln Leu Tyr Gly Gly Tyr
    130                 135                 140

Gln Ala Ser Gln Gly Ser Asp Asp Asp Trp Asp Asp Glu Trp Asp Asp
145                 150                 155                 160

Ser Ser Thr Val Ala Asp Glu Pro Gly Ala Leu Gly Ser Gly Ala Tyr
                165                 170                 175

Pro Asp Leu Asp Gly Ser Ser Ser Ala Gly Val Gly Ala Ala Gly Arg
            180                 185                 190

Tyr Arg Leu Ser Thr Arg Ser Asp Leu Ser Leu Gly Ser Arg Gly Gly
        195                 200                 205

Ser Val Pro Pro Gln His His Pro Ser Gly Pro Lys Ser Ser Ala Thr
    210                 215                 220

Val Ser Arg Asn Leu Asn Arg Phe Ser Thr Phe Val Lys Ser Gly Gly
225                 230                 235                 240

Glu Ala Phe Val Leu Gly Glu Ala Ser Gly Phe Val Lys Asp Gly Asp
                245                 250                 255

Lys Leu Cys Val Val Leu Gly Pro Tyr Gly Pro Glu Trp Gln Glu Asn
            260                 265                 270

Pro Tyr Pro Phe Gln Cys Thr Ile Asp Asp Pro Thr Lys Gln Thr Lys
        275                 280                 285

Phe Lys Gly Met Lys Ser Tyr Ile Ser Tyr Lys Leu Val Pro Thr His
    290                 295                 300

Thr Gln Val Pro Val His Arg Arg Tyr Lys His Phe Asp Trp Leu Tyr
305                 310                 315                 320

Ala Arg Leu Ala Glu Lys Phe Pro Val Ile Ser Val Pro His Leu Pro
                325                 330                 335

Glu Lys Gln Ala Thr Gly Arg Phe Glu Glu Asp Phe Ile Ser Lys Arg
            340                 345                 350

Arg Lys Gly Leu Ile Trp Trp Met Asn His Met Ala Ser His Pro Val
        355                 360                 365

Leu Ala Gln Cys Asp Val Phe Gln His Phe Leu Thr Cys Pro Ser Ser
    370                 375                 380

Thr Asp Glu Lys Ala Trp Lys Gln Gly Lys Arg Lys Ala Glu Lys Asp
385                 390                 395                 400

Glu Met Val Gly Ala Asn Phe Phe Leu Thr Leu Ser Thr Pro Pro Ala
                405                 410                 415

Ala Ala Leu Asp Leu Gln Glu Val Glu Ser Lys Ile Asp Gly Phe Lys
            420                 425                 430

Cys Phe Thr Lys Lys Met Asp Asp Ser Ala Leu Gln Leu Asn His Thr
        435                 440                 445

Ala Asn Glu Phe Ala Arg Lys Gln Val Thr Gly Phe Lys Lys Glu Tyr
    450                 455                 460

Gln Lys Val Gly Gln Ser Phe Arg Gly Leu Ser Gln Ala Phe Glu Leu
465                 470                 475                 480

Asp Gln Gln Ala Phe Ser Val Gly Leu Asn Gln Ala Ile Ala Phe Thr
                485                 490                 495

Gly Asp Ala Tyr Asp Ala Ile Gly Glu Leu Phe Ala Glu Gln Pro Arg
            500                 505                 510

Gln Asp Leu Asp Pro Val Met Asp Leu Leu Ala Leu Tyr Gln Gly His
        515                 520                 525

Leu Ala Asn Phe Pro Asp Ile Ile His Val Gln Lys Gly Ala Leu Thr
    530                 535                 540

Lys Val Lys Glu Ser Arg Arg His Val Glu Glu Gly Lys Met Glu Val
545                 550                 555                 560

Gln Lys Ala Asp Gly Ile Gln Asp Arg Cys Asn Thr Ile Ser Phe Ala
                565                 570                 575

Thr Leu Ala Glu Ile His His Phe His Gln Ile Arg Val Arg Asp Phe
            580                 585                 590

Lys Ser Gln Met Gln His Phe Leu Gln Gln Gln Ile Ile Phe Phe Gln
        595                 600                 605

Lys Val Thr Gln Lys Leu Glu Glu Ala Leu His Lys Tyr Asp Ser Val
    610                 615                 620

<210> 83
<211> 1514
<212> DNA
<213> Homo sapiens

<400> 83
agtagtttta aacatatttt aaagaataat aaatgtttta aagtcagtgc tgaaaatttt 60
taaaaagtca ttatatttta tgtttaggtc tctgaaagaa gatgaatttg gctgagattt 120
gtgataatgc aaagaaagga agagaatatg cccttcttgg aaattacgac tcatcaatgg 180
tatattacca gggggtgatg cagcagattc agagacattg ccagtcagtc agagatccag 240
ctatcaaagg caaatggcaa caggtaagat ggggttatga taaggatctg gtggaagccc 300
ttgaaagaga cattgtatcc aggaatccta gcattcattg ggatgacata gcagatctgg 360
aagaagctaa gaagttgcta agggaagctg ttgttcttcc aatgtggatg cctgactttt 420
tcaaagggat tagaaggcca tggaagggtg tactgatggt tggaccccca ggcactggta 480
aaactatgct agctaaagct gttgccactg aatgtggtac aacattcttc aacgtttcgt 540
cttctacact gacatctaaa tacagaggtg aatctgagaa gttagttcgt ctgttgtttg 600
agatggctag attttatgcc cctaccacga tcttcattga tgagatagat tctatctgca 660
gtcgaagagg aacctctgat gaacatgagg caagtcgcag ggtcaagtct gaactgctca 720
ttcagatgga tggtgttgga ggagctttag aaaatgatga tccttccaaa atggttatgg 780
tattggctgc tactaatttc ccgtgggaca ttgatgaagc tttgcgaaga aggttagaaa 840
aaaggatata tatacctctc ccaacagcaa aaggaagagc tgagcttctg aagatcaacc 900
ttcgtgaggt cgaattagat cctgatattc aactggaaga tatagccgag aagattgagg 960
gctattctgg tgctgacatc actaatgttt gcagggatgc ctctttaatg gcaatgagac 1020
ggcgtatcaa tggcttaagt ccagaagaaa tccgtgcact ttctaaagag gaacttcaga 1080
tgcctgttac caaaggagac tttgaattgg ccctaaaaaa aattgctaag tctgtctctg 1140
ctgcagactt ggagaagtat gaaaaatgga tggttgaatt tggatctgct tgaatttctg 1200
tcagctcttt aatttctggt atttttgttg ataaaatacg aagaaattcc tgcaattttt 1260
aaaaaacaag tttggaactt ttttcagtgg agtggttttc gcaaaaaaaa aaaaaaaatc 1320
taaaactgcg aagaatacta aatgtagttg agaaataatt gatggcgaga gtttgctagt 1380
ctccctcccc ggctttgtgc tggtattcca cgtattcctg cattaatatt gcacacccaa 1440
accagtctat cagggaggct gaagcagggc gcagtgtgat atttaggaat cagaagatta 1500
gaaatcccct attc 1514

<210> 84
<211> 363
<212> PRT
<213> Homo sapiens

<400> 84
Met Asn Leu Ala Glu Ile Cys Asp Asn Ala Lys Lys Gly Arg Glu Tyr
1               5                   10                  15

Ala Leu Leu Gly Asn Tyr Asp Ser Ser Met Val Tyr Tyr Gln Gly Val
            20                  25                  30

Met Gln Gln Ile Gln Arg His Cys Gln Ser Val Arg Asp Pro Ala Ile
        35                  40                  45

Lys Gly Lys Trp Gln Gln Val Arg Trp Gly Tyr Asp Lys Asp Leu Val
    50                  55                  60

Glu Ala Leu Glu Arg Asp Ile Val Ser Arg Asn Pro Ser Ile His Trp
65                  70                  75                  80

Asp Asp Ile Ala Asp Leu Glu Glu Ala Lys Lys Leu Leu Arg Glu Ala
                85                  90                  95

Val Val Leu Pro Met Trp Met Pro Asp Phe Phe Lys Gly Ile Arg Arg
            100                 105                 110

Pro Trp Lys Gly Val Leu Met Val Gly Pro Pro Gly Thr Gly Lys Thr

115                 120                 125

Met Leu Ala Lys Ala Val Ala Thr Glu Cys Gly Thr Thr Phe Phe Asn
    130                 135                 140

Val Ser Ser Ser Thr Leu Thr Ser Lys Tyr Arg Gly Glu Ser Glu Lys
145                 150                 155                 160

Leu Val Arg Leu Leu Phe Glu Met Ala Arg Phe Tyr Ala Pro Thr Thr
                165                 170                 175

Ile Phe Ile Asp Glu Ile Asp Ser Ile Cys Ser Arg Arg Gly Thr Ser
            180                 185                 190

Asp Glu His Glu Ala Ser Arg Arg Val Lys Ser Glu Leu Leu Ile Gln
        195                 200                 205

Met Asp Gly Val Gly Gly Ala Leu Glu Asn Asp Asp Pro Ser Lys Met
    210                 215                 220

Val Met Val Leu Ala Ala Thr Asn Phe Pro Trp Asp Ile Asp Glu Ala
225                 230                 235                 240

Leu Arg Arg Arg Leu Glu Lys Arg Ile Tyr Ile Pro Leu Pro Thr Ala
                245                 250                 255

Lys Gly Arg Ala Glu Leu Leu Lys Ile Asn Leu Arg Glu Val Glu Leu
            260                 265                 270

Asp Pro Asp Ile Gln Leu Glu Asp Ile Ala Glu Lys Ile Glu Gly Tyr
        275                 280                 285

Ser Gly Ala Asp Ile Thr Asn Val Cys Arg Asp Ala Ser Leu Met Ala
    290                 295                 300

Met Arg Arg Arg Ile Asn Gly Leu Ser Pro Glu Glu Ile Arg Ala Leu
305                 310                 315                 320

Ser Lys Glu Glu Leu Gln Met Pro Val Thr Lys Gly Asp Phe Glu Leu
                325                 330                 335

Ala Leu Lys Lys Ile Ala Lys Ser Val Ser Ala Ala Asp Leu Glu Lys
            340                 345                 350

Tyr Glu Lys Trp Met Val Glu Phe Gly Ser Ala
        355                 360

<210> 85
<211> 2442
<212> DNA
<213> Homo sapiens

<400> 85
tgcagttgct tcctttcctt gaaggtagct gtatcttatt ttctttaaaa agctttttct 60
tccaaagcca cttgccatgc cgaccgtcat tagcgcatct gtggctccaa ggacagcggc 120
tgagccccgg tccccagggc cagttcctca cccggcccag agcaaggcca ctgaggctgg 180
gggtggaaac ccaagtggca tctattcagc catcatcagc cgcaattttc ctattatcgg 240
agtgaaagag aagacattcg agcaacttca caagaaatgt ctagaaaaga aagttcttta 300
tgtggaccct gagttcccac cggatgagac ctctctcttt tatagccaga agttccccat 360
ccagttcgtc tggaagagac ctccggaaat ttgcgagaat ccccgattta tcattgatgg 420
agccaacaga actgacatct gtcaaggaga gctaggggac tgctggtttc tcgcagccat 480
tgcctgcctg accctgaacc agcaccttct tttccgagtc ataccccatg atcaaagttt 540
catcgaaaac tacgcaggga tcttccactt ccagttctgg cgctatggag agtgggtgga 600
cgtggttata gatgactgcc tgccaacgta caacaatcaa ctggttttca ccaagtccaa 660
ccaccgcaat gagttctgga gtgctctgct ggagaaggct tatgctaagc tccatggttc 720
ctacgaagct ctgaaaggtg ggaacaccac agaggccatg gaggacttca caggaggggt 780
ggcagagttt tttgagatca gggatgctcc tagtgacatg tacaagatca tgaagaaagc 840
catcgagaga ggctccctca tgggctgctc cattgataca atcattccgg ttcagtatga 900

gacaagaatg gcctgcgggc tggtcagagg tcacgcctac tctgtcacgg ggctggatga 960
ggtcccgttc aaaggtgaga aagtgaagct ggtgcggctg cggaatccgt ggggccaggt 1020
ggagtggaac ggttcttgga gtgatagatg gaaggactgg agctttgtgg acaaagatga 1080
gaaggcccgt ctgcagcacc aggtcactga ggatggagag ttctggatgt cctatgagga 1140
tttcatctac catttcacaa agttggagat ctgcaacctc acggccgatg ctctgcagtc 1200
tgacaagctt cagacctgga cagtgtctgt gaacgagggc cgctgggtac ggggttgctc 1260
tgccggaggc tgccgcaact tcccagatac tttctggacc aaccctcagt accgtctgaa 1320
gctcctggag gaggacgatg accctgatga ctcggaggtg atttgcagct tcctggtggc 1380
cctgatgcag aagaaccggc ggaaggaccg gaagctaggg gccagtctct tcaccattgg 1440
cttcgccatc tacgaggttc ccaaagagat gcacggaaac aagcagcacc tgcagaagga 1500
cttcttcctg tacaacgcct ccaaggccag gagcaaaacc tacatcaaca tgcgggaggt 1560
gtcccagcgc ttccgcctgc ctcccagcga gtacgtcatc gtgccctcca cctacgagcc 1620
ccaccaggag ggggaattca tcctccgggt cttctctgaa aagaggaacc tctctgagga 1680
agttgaaaat accatctccg tggatcggcc agtgaaaaag aaaaaaacca agcccatcat 1740
cttcgtttcg gacagagcaa acagcaacaa ggagctgggt gtggaccagg agtcagagga 1800
gggcaaaggc aaaacaagcc ctgataagca aaagcagtcc ccacagccac agcctggcag 1860
ctctgatcag gaaagtgagg aacagcaaca attccggaac attttcaagc agatagcagg 1920
agatgacatg gagatctgtg cagatgagct caagaaggtc cttaacacag tcgtgaacaa 1980
acacaaggac ctgaagacac acgggttcac actggagtcc tgccgtagca tgattgcgct 2040
catggataca gatggctctg gaaagctcaa cctgcaggag ttccaccacc tctggaacaa 2100
gattaaggcc tggcagaaaa ttttcaaaca ctatgacaca gaccagtccg gcaccatcaa 2160
cagctacgag atgcgaaatg cagtcaacga cgcaggattc cacctcaaca accagctcta 2220
tgacatcatt accatgcggt acgcagacaa acacatgaac atcgactttg acagtttcat 2280
ctgctgcttc gttaggctgg agggcatgtt cagagctttt catgcatttg acaaggatgg 2340
agatggtatc atcaagctca acgttctgga gtggctgcag ctcaccatgt atgcctgaac 2400
caggctggcc tcatccaaag ccatgcagga tcactcagga tt 2442

<210> 86
<211> 773
<212> PRT
<213> Homo sapiens

<400> 86
Met Pro Thr Val Ile Ser Ala Ser Val Ala Pro Arg Thr Ala Ala Glu
1               5                   10                  15

Pro Arg Ser Pro Gly Pro Val Pro His Pro Ala Gln Ser Lys Ala Thr
            20                  25                  30

Glu Ala Gly Gly Gly Asn Pro Ser Gly Ile Tyr Ser Ala Ile Ile Ser
        35                  40                  45

Arg Asn Phe Pro Ile Ile Gly Val Lys Glu Lys Thr Phe Glu Gln Leu
    50                  55                  60

His Lys Lys Cys Leu Glu Lys Lys Val Leu Tyr Val Asp Pro Glu Phe
65                  70                  75                  80

Pro Pro Asp Glu Thr Ser Leu Phe Tyr Ser Gln Lys Phe Pro Ile Gln
                85                  90                  95

Phe Val Trp Lys Arg Pro Pro Glu Ile Cys Glu Asn Pro Arg Phe Ile
            100                 105                 110

Ile Asp Gly Ala Asn Arg Thr Asp Ile Cys Gln Gly Glu Leu Gly Asp
        115                 120                 125

Cys Trp Phe Leu Ala Ala Ile Ala Cys Leu Thr Leu Asn Gln His Leu
    130                 135                 140

Leu Phe Arg Val Ile Pro His Asp Gln Ser Phe Ile Glu Asn Tyr Ala
145                 150                 155                 160

Gly Ile Phe His Phe Gln Phe Trp Arg Tyr Gly Glu Trp Val Asp Val
                165                 170                 175

Val Ile Asp Asp Cys Leu Pro Thr Tyr Asn Asn Gln Leu Val Phe Thr
            180                 185                 190

Lys Ser Asn His Arg Asn Glu Phe Trp Ser Ala Leu Leu Glu Lys Ala
        195                 200                 205

Tyr Ala Lys Leu His Gly Ser Tyr Glu Ala Leu Lys Gly Gly Asn Thr
    210                 215                 220

Thr Glu Ala Met Glu Asp Phe Thr Gly Gly Val Ala Glu Phe Phe Glu
225                 230                 235                 240

Ile Arg Asp Ala Pro Ser Asp Met Tyr Lys Ile Met Lys Lys Ala Ile
                245                 250                 255

Glu Arg Gly Ser Leu Met Gly Cys Ser Ile Asp Thr Ile Ile Pro Val
            260                 265                 270

Gln Tyr Glu Thr Arg Met Ala Cys Gly Leu Val Arg Gly His Ala Tyr
        275                 280                 285

Ser Val Thr Gly Leu Asp Glu Val Pro Phe Lys Gly Glu Lys Val Lys
    290                 295                 300

Leu Val Arg Leu Arg Asn Pro Trp Gly Gln Val Glu Trp Asn Gly Ser
305                 310                 315                 320

Trp Ser Asp Arg Trp Lys Asp Trp Ser Phe Val Asp Lys Asp Glu Lys
                325                 330                 335

Ala Arg Leu Gln His Gln Val Thr Glu Asp Gly Glu Phe Trp Met Ser
            340                 345                 350

Tyr Glu Asp Phe Ile Tyr His Phe Thr Lys Leu Glu Ile Cys Asn Leu
        355                 360                 365

Thr Ala Asp Ala Leu Gln Ser Asp Lys Leu Gln Thr Trp Thr Val Ser
    370                 375                 380

Val Asn Glu Gly Arg Trp Val Arg Gly Cys Ser Ala Gly Gly Cys Arg
385                 390                 395                 400

Asn Phe Pro Asp Thr Phe Trp Thr Asn Pro Gln Tyr Arg Leu Lys Leu
                405                 410                 415

Leu Glu Glu Asp Asp Asp Pro Asp Asp Ser Glu Val Ile Cys Ser Phe
            420                 425                 430

Leu Val Ala Leu Met Gln Lys Asn Arg Arg Lys Asp Arg Lys Leu Gly
        435                 440                 445

Ala Ser Leu Phe Thr Ile Gly Phe Ala Ile Tyr Glu Val Pro Lys Glu
    450                 455                 460

Met His Gly Asn Lys Gln His Leu Gln Lys Asp Phe Phe Leu Tyr Asn
465                 470                 475                 480

Ala Ser Lys Ala Arg Ser Lys Thr Tyr Ile Asn Met Arg Glu Val Ser
                485                 490                 495

Gln Arg Phe Arg Leu Pro Pro Ser Glu Tyr Val Ile Val Pro Ser Thr
            500                 505                 510

Tyr Glu Pro His Gln Glu Gly Glu Phe Ile Leu Arg Val Phe Ser Glu
        515                 520                 525

Lys Arg Asn Leu Ser Glu Glu Val Glu Asn Thr Ile Ser Val Asp Arg
    530                 535                 540

Pro Val Lys Lys Lys Lys Thr Lys Pro Ile Ile Phe Val Ser Asp Arg
545                 550                 555                 560

Ala Asn Ser Asn Lys Glu Leu Gly Val Asp Gln Glu Ser Glu Glu Gly
                565                 570                 575

Lys Gly Lys Thr Ser Pro Asp Lys Gln Lys Gln Ser Pro Gln Pro Gln
            580                 585                 590

Pro Gly Ser Ser Asp Gln Glu Ser Glu Glu Gln Gln Gln Phe Arg Asn
        595                 600                 605

Ile Phe Lys Gln Ile Ala Gly Asp Asp Met Glu Ile Cys Ala Asp Glu
    610                 615                 620

Leu Lys Lys Val Leu Asn Thr Val Val Asn Lys His Lys Asp Leu Lys
625                 630                 635                 640

Thr His Gly Phe Thr Leu Glu Ser Cys Arg Ser Met Ile Ala Leu Met
                645                 650                 655

Asp Thr Asp Gly Ser Gly Lys Leu Asn Leu Gln Glu Phe His His Leu
            660                 665                 670

Trp Asn Lys Ile Lys Ala Trp Gln Lys Ile Phe Lys His Tyr Asp Thr
        675                 680                 685

Asp Gln Ser Gly Thr Ile Asn Ser Tyr Glu Met Arg Asn Ala Val Asn
    690                 695                 700

Asp Ala Gly Phe His Leu Asn Asn Gln Leu Tyr Asp Ile Ile Thr Met
705                 710                 715                 720

Arg Tyr Ala Asp Lys His Met Asn Ile Asp Phe Asp Ser Phe Ile Cys
                725                 730                 735

Cys Phe Val Arg Leu Glu Gly Met Phe Arg Ala Phe His Ala Phe Asp
            740                 745                 750

Lys Asp Gly Asp Gly Ile Ile Lys Leu Asn Val Leu Glu Trp Leu Gln
        755                 760                 765

Leu Thr Met Tyr Ala
    770

<210> 87
<211> 1327
<212> DNA
<213> Homo sapiens

<400> 87
gcttcaccac tccctccacc ttctccacca actaccagtc cctgggctct gtccagccgc 60
ccagctatgg cacctggccg gtcagcagcg cagccagcat ctatgcaggc actggggggc 120
ttgggatccc agatctccat gtcctgttct accagtttct ggggcggctt ggggtctggg 180
ggcctggcca cagagatggc tgggggtctg gcagaaatgg ggggcatcca gaatgagaag 240
gagaccatgc aaagcctgaa cgaccacctg gactacctgg acagagtgag gaacctggag 300
accgagaact ggaggctgga gagcaaaatc caggagtatc tggagaagag accccatgtc 360
agagactggg gccattactt caagaccatc aaggaactga gggctcagat cttcgcaaat 420
actgtggaca atgtccacat cattctgcag atcgacaatg cccgtcttgc tgctgatgac 480
ttcagagtca agtatgagac aagagctggc catgcgccag tctgtggaga gaacatccat 540
gggctctgca aggtcattga tgacaccaat gtcactctgc tgcagctgga gacagagatg 600
ggcgctctca aggaggagct gctcctcatg aagaagaacc atgaagagga agtaaaaggc 660
ttgcaagtcc agattgccaa ctctgggttg gccgtggagg tagatgcccc caaatctcaa 720
gtcctcgcca aggtcatggc agacatcagg gcccaagatg agctgtctca gaagaactca 780
gagaagctag gcaagtactg gtctcagcag actgaggaga gcaccacagt ggtcaccaca 840
cactctgcca aggtcagagc tgctgagatg acaacggagc tgagacgtac agtccagtgc 900
ttggagattg acctggactc aatgagaaat ctgaagacca gcttgaacag cctgagggag 960
gtggaggccc gctacgccct gcagatggag cagctcaaca gaatcctgct gtacttggag 1020
tcaaagctgg cacagaactg ggcagagggc cagcgcaagg tccaggagta caaggacttg 1080
ctgaacatca gggtcaagct ggaggctgag atcgccacct accgccgcct gctggaagac 1140

agcgagggcc tcaatcttgg tgatgccctg gacagcagca actccatgca aaccatccaa 1200
aagaccacca cccgccagat agtggatagc aaagtggtgt ctgagatcag tgacaccaaa 1260
gttctgagac attaagccag cagaagcagg gtaccctgtg gggagtaaga ggccaataaa 1320
aagttca 1327

<210> 88
<211> 402
<212> PRT
<213> Homo sapiens

<400> 88
Met Ala Pro Gly Arg Ser Ala Ala Gln Pro Ala Ser Met Gln Ala Leu
1               5                   10                  15

Gly Gly Leu Gly Ser Gln Ile Ser Met Ser Cys Ser Thr Ser Phe Trp
            20                  25                  30

Gly Gly Leu Gly Ser Gly Gly Leu Ala Thr Glu Met Ala Gly Gly Leu
        35                  40                  45

Ala Glu Met Gly Gly Ile Gln Asn Glu Lys Glu Thr Met Gln Ser Leu
    50                  55                  60

Asn Asp His Leu Asp Tyr Leu Asp Arg Val Arg Asn Leu Glu Thr Glu
65                  70                  75                  80

Asn Trp Arg Leu Glu Ser Lys Ile Gln Glu Tyr Leu Glu Lys Arg Pro
                85                  90                  95

His Val Arg Asp Trp Gly His Tyr Phe Lys Thr Ile Lys Glu Leu Arg
            100                 105                 110

Ala Gln Ile Phe Ala Asn Thr Val Asp Asn Val His Ile Ile Leu Gln
        115                 120                 125

Ile Asp Asn Ala Arg Leu Ala Ala Asp Asp Phe Arg Val Lys Tyr Glu
    130                 135                 140

Thr Arg Ala Gly His Ala Pro Val Cys Gly Glu Asn Ile His Gly Leu
145                 150                 155                 160

Cys Lys Val Ile Asp Asp Thr Asn Val Thr Leu Leu Gln Leu Glu Thr
                165                 170                 175

Glu Met Gly Ala Leu Lys Glu Glu Leu Leu Leu Met Lys Lys Asn His
            180                 185                 190

Glu Glu Glu Val Lys Gly Leu Gln Val Gln Ile Ala Asn Ser Gly Leu
        195                 200                 205

Ala Val Glu Val Asp Ala Pro Lys Ser Gln Val Leu Ala Lys Val Met
    210                 215                 220

Ala Asp Ile Arg Ala Gln Asp Glu Leu Ser Gln Lys Asn Ser Glu Lys
225                 230                 235                 240

Leu Gly Lys Tyr Trp Ser Gln Gln Thr Glu Glu Ser Thr Thr Val Val
                245                 250                 255

Thr Thr His Ser Ala Lys Val Arg Ala Ala Glu Met Thr Thr Glu Leu
            260                 265                 270

Arg Arg Thr Val Gln Cys Leu Glu Ile Asp Leu Asp Ser Met Arg Asn
        275                 280                 285

Leu Lys Thr Ser Leu Asn Ser Leu Arg Glu Val Glu Ala Arg Tyr Ala
    290                 295                 300

Leu Gln Met Glu Gln Leu Asn Arg Ile Leu Leu Tyr Leu Glu Ser Lys
305                 310                 315                 320

Leu Ala Gln Asn Trp Ala Glu Gly Gln Arg Lys Val Gln Glu Tyr Lys
                325                 330                 335

Asp Leu Leu Asn Ile Arg Val Lys Leu Glu Ala Glu Ile Ala Thr Tyr
            340                 345                 350

Arg Arg Leu Leu Glu Asp Ser Glu Gly Leu Asn Leu Gly Asp Ala Leu
        355                 360                 365

Asp Ser Ser Asn Ser Met Gln Thr Ile Gln Lys Thr Thr Thr Arg Gln
    370                 375                 380

Ile Val Asp Ser Lys Val Val Ser Glu Ile Ser Asp Thr Lys Val Leu
385                 390                 395                 400

Arg His

<210> 89
<211> 12992
<212> DNA
<213> Homo sapiens

<400> 89
cgcgcgcggg cgggcctggg gacggcgggg ccatgcgcgc gctgccctaa cgatgccgcc 60
cgccgcgccc gcccgcctgg cgctggccct gggcctgggc ctgtggctcg gggcgctggc 120
gggggccccc gggcgcggct gcgggccctg cgagcccccc tgcctctgcg gcccagcgcc 180
cggcgccgcc tgccgcgtca actgctcggg ccgcgggctg cggacgctcg gtcccgcgct 240
gcgcatcccc gcggacgcca cagcgctaga cgtctcccac aacctgctcc gggcgctgga 300
cgttgggctc ctggcgaacc tctcggcgct ggcagagctg gatataagca acaacaagat 360
ttctacgtta gaagaaggaa tatttgctaa tttatttaat ttaagtgaaa taaacctgag 420
tgggaacccg tttgagtgtg actgtggcct ggcgtggctg ccgcgatggg cggaggagca 480
gcaggtgcgg gtggtgcagc ccgaggcagc cacgtgtgct gggcctggct ccctggctgg 540
ccagcctctg cttggcatcc ccttgctgga cagtggctgt ggtgaggagt atgtcgcctg 600
cctccctgac aacagctcag gcaccgtggc agcagtgtcc ttttcagctg cccacgaagg 660
cctgcttcag ccagaggcct gcagcgcctt ctgcttctcc accggccagg gcctcgcagc 720
cctctcggag cagggctggt gcctgtgtgg ggcggcccag ccctccagtg cctcctttgc 780
ctgcctgtcc ctctgctccg gcccccgcc acctcctgcc cccacctgta ggggccccac 840
cctcctccag cacgtcttcc ctgcctcccc aggggccacc ctggtggggc cccacggacc 900
tctggcctct ggccagctag cagccttcca catcgctgcc ccgctccctg tcactgccac 960
acgctgggac ttcggagacg gctccgccga ggtggatgcc gctgggccgg ctgcctcgca 1020
tcgctatgtg ctgcctgggc gctatcacgt gacggccgtg ctggccctgg gggccggctc 1080
agccctgctg gggacagacg tgcaggtgga agcggcacct gccgccctgg agctcgtgtg 1140
cccgtcctcg gtgcagagtg acgagagcct tgacctcagc atccagaacc gcggtggttc 1200
aggcctggag gccgcctaca gcatcgtggc cctgggcgag gagccggccc gagcggtgca 1260
cccgctctgc ccctcggaca cggagatctt ccctggcaac gggcactgct accgcctggt 1320
ggtggagaag gcggcctggc tgcaggcgca ggagcagtgt caggcctggg ccggggccgc 1380
cctggcaatg gtggacagtc ccgccgtgca gcgcttcctg gtctcccggg tcaccaggag 1440
cctagacgtg tggatcggct tctcgactgt gcagggggtg gaggtgggcc cagcgccgca 1500
gggcgaggcc ttcagcctgg agagctgcca gaactggctg cccggggagc cacacccagc 1560
cacagccgag cactgcgtcc ggctcgggcc caccgggtgg tgtaacaccg acctgtgctc 1620
agcgccgcac agctacgtct gcgagctgca gcccggaggc ccagtgcagg atgccgagaa 1680
cctcctcgtg ggagcgccca gtggggacct gcaggaccc ctgacgcctc tggcacagca 1740
ggacggcctc tcagccccgc acgagcccgt ggaggtcatg gtattcccgg gcctgcgtct 1800
gagccgtgaa gccttcctca ccacggccga atttgggacc caggagctcc ggcggcccgc 1860
ccagctgcgg ctgcaggtgt accggctcct cagcacagca gggaccccgg agaacggcag 1920
cgagcctgag agcaggtccc cggacaacag gacccagctg gcccccgcgt gcatgccagg 1980
gggacgctgg tgccctggag ccaacatctg cttgccgctg gacgcctctt gccaccccca 2040
ggcctgcgcc aatggctgca cgtcagggcc agggctaccc ggggccccct atgcgctatg 2100
gagagagttc ctcttctccg ttgccgcggg gcccccgcg cagtactcgg tcaccctcca 2160
cggccaggat gtcctcatgc tccctggtga cctcgttggc ttgcagcacg acgctggccc 2220
tggcgccctc ctgcactgct cgccggctcc cggccaccct ggtccccagg ccccgtacct 2280
ctccgccaac gcctcgtcat ggctgcccca cttgccagcc cagctggagg gcacttgggc 2340
ctgccctgcc tgtgccctgc ggctgcttgc agccacggaa cagctcaccg tgctgctggg 2400
cttgaggccc aaccctggac tgcggatgcc tgggcgctat gaggtccggg cagaggtggg 2460

caatggcgtg tccaggcaca acctctcctg cagctttgac gtggtctccc cagtggctgg 2520
gctgcgggtc atctaccctg cccccgcga cggccgcctc tacgtgccca ccaacggctc 2580
agccttggtg ctccaggtgg actctggtgc caacgccacg gccacggctc gctggcctgg 2640
gggcagtgtc agcgcccgct ttgagaatgt ctgccctgcc ctggtggcca ccttcgtgcc 2700
cggctgcccc tgggagacca acgataccct gttctcagtg gtagcactgc cgtggctcag 2760
tgagggggag cacgtggtgg acgtggtggt ggaaaacagc gccagccggg ccaacctcag 2820
cctgcgggtg acggcggagg agcccatctg tggcctccgc gccacgccca gccccgaggc 2880
ccgtgtactg cagggagtcc tagtgaggta cagccccgtg gtggaggccg gctcggacat 2940
ggtcttccgg tggaccatca acgacaagca gtccctgacc ttccagaacg tggtcttcaa 3000
tgtcatttat cagagcgcgg cggtcttcaa gctctcactg acggcctcca accacgtgag 3060
caacgtcacc gtgaactaca acgtaaccgt ggagcggatg aacaggatgc agggtctgca 3120
ggtctccaca gtgccggccg tgctgtcccc caatgccacg ctagcactga cggcgggcgt 3180
gctggtggac tcggccgtgg aggtggcctt cctgtggaac tttggggatg gggagcaggc 3240
cctccaccag ttccagcctc cgtacaacga gtccttcccg gttccagacc cctcggtggc 3300
ccaggtgctg gtggagcaca atgtcatgca cacctacgct gccccaggtg agtacctcct 3360
gaccgtgctg gcatctaatg ccttcgagaa cctgacgcag caggtgcctg tgagcgtgcg 3420
cgcctccctg ccctccgtgg ctgtgggtgt gagtgacggc gtcctggtgg ccggccggcc 3480
cgtcaccttc tacccgcacc cgctgccctc gcctgggggt gttctttaca cgtggacttt 3540
cggggacggc tcccctgtcc tgacccagag ccagccggct gccaaccaca cctatgcctc 3600
gaggggcacc taccacgtgc gcctggaggt caacaacacg gtgagcggtg cggcggccca 3660
ggcggatgtg cgcgtctttg aggagctccg cggactcagc gtggacatga gcctggccgt 3720
ggagcagggc gcccccgtgg tggtcagcgc cgcggtgcag acgggcgaca acatcacgtg 3780
gaccttcgac atgggggacg gcaccgtgct gtcgggcccg gaggcaacag tggagcatgt 3840
gtacctgcgg gcacagaact gcacagtgac cgtgggtgcg gccagccccg ccggccacct 3900
ggcccggagc ctgcacgtgc tggtcttcgt cctggaggtg ctgcgcgttg aacccgccgc 3960
ctgcatcccc acgcagcctg acgcgcggct cacggcctac gtcaccggga acccggccca 4020
ctacctcttc gactggacct tcggggatgg ctcctccaac acgaccgtgc gggggtgccc 4080
gacggtgaca cacaacttca cgcggagcgg cacgttcccc ctggcgctgg tgctgtccag 4140
ccgcgtgaac agggcgcatt acttcaccag catctgcgtg gagccagagg tgggcaacgt 4200
caccctgcag ccagagaggc agtttgtgca gctcggggac gaggcctggc tggtggcatg 4260
tgcctggccc ccgttcccct accgctacac ctgggacttt ggcaccgagg aagccgcccc 4320
cacccgtgcc aggggccctg aggtgacgtt catctaccga gacccaggct cctatcttgt 4380
gacagtcacc gcgtccaaca acatctctgc tgccaatgac tcagccctgg tggaggtgca 4440
ggagcccgtg ctggtcacca gcatcaaggt caatggctcc cttgggctgg agctgcagca 4500
gccgtacctg ttctctgctg tgggccgtgg gcgccccgcc agctacctgt gggatctggg 4560
ggacggtggg tggctcgagg gtccggaggt cacccacgct tacaacagca caggtgactt 4620
caccgttagg gtggccggct ggaatgaggt gagccgcagc gaggcctggc tcaatgtgac 4680
ggtgaagcgg cgcgtgcggg ggctcgtcgt caatgcaagc cgcacggtgg tgcccctgaa 4740
tgggagcgtg agcttcagca cgtcgctgga ggccggcagt gatgtgcgct attcctgggt 4800
gctctgtgac cgctgcacgc ccatccctgg gggtcctacc atctcttaca ccttccgctc 4860
cgtgggcacc ttcaatatca tcgtcacggc tgagaacgag gtgggctccg cccaggacag 4920
catcttcgtc tatgtcctgc agctcataga ggggctgcag gtggtgggcg gtggccgcta 4980
cttccccacc aaccacacgg tacagctgca ggccgtggtt agggatggca ccaacgtctc 5040
ctacagctgg actgcctgga gggacagggg cccggccctg gccggcagcg gcaaaggctt 5100
ctcgctcacc gtgctcgagg ccggcaccta ccatgtgcag ctgcgggcca ccaacatgct 5160
gggcagcgcc tgggccgact gcaccatgga cttcgtggag cctgtggggt ggctgatggt 5220
gaccgcctcc ccgaacccag ctgccgtcaa cacaagcgtc accctcagtg ccgagctggc 5280
tggtggcagt ggtgtcgtat acacttggtc cttggaggag gggctgagct gggagacctc 5340
cgagccattt accacccata gcttccccac acccggcctg cacttggtca ccatgacggc 5400
agggaacccg ctgggctcag ccaacgccac cgtggaagtg gatgtgcagg tgcctgtgag 5460
tggcctcagc atcagggcca gcgagcccgg aggcagcttc gtggcggccg ggtcctctgt 5520
gccctttgg gggcagctgg ccacgggcac caatgtgagc tggtgctggg ctgtgcccgg 5580
cggcagcagc aagcgtggcc ctcatgtcac catggtcttc ccggatgctg gcaccttctc 5640
catccggctc aatgcctcca acgcagtcag ctgggtctca gccacgtaca acctcacggc 5700
ggaggagccc atcgtgggcc tggtgctgtg ggccagcagc aaggtggtgg cgcccgggca 5760
gctggtccat tttcagatcc tgctggctgc cggctcagct gtcaccttcc gcctgcaggt 5820
cggcggggcc aaccccgagg tgctccccgg gccccgtttc tcccacagct tcccccgcgt 5880
cggagaccac gtggtgagcg tgcggggcaa aaaccacgtg agctgggccc aggcgcaggt 5940
gcgcatcgtg gtgctggagg ccgtgagtgg gctgcagatg cccaactgct gcgagcctgg 6000
catcgccacg ggcactgaga ggaacttcac agcccgcgtg cagcgcggct ctcgggtcgc 6060
ctacgcctgg tacttctcgc tgcagaaggt ccagggcgac tcgctggtca tcctgtcggg 6120
ccgcgacgtc acctacacgc ccgtggccgc ggggctgttg gagatccagg tgcgcgcctt 6180
caacgccctg ggcagtgaga accgcacgct ggtgctggag gttcaggacg ccgtccagta 6240
tgtggccctg cagagcggcc cctgcttcac caaccgctcg gcgcagtttg aggccgccac 6300
cagccccagc ccccggcgtg tggcctacca ctgggacttt ggggatgggt cgccagggca 6360
ggacacagat gagcccaggg ccgagcactc ctacctgagg cctggggact accgcgtgca 6420
ggtgaacgcc tccaacctgg tgagcttctt cgtggcgcag gccacggtga ccgtccaggt 6480
gctggcctgc cgggagccgg aggtggacgt ggtcctgccc ctgcaggtgc tgatgcggcg 6540
atcacagcgc aactacttgg aggcccacgt tgacctgcgc gactgcgtca cctaccagac 6600

tgagtaccgc tgggaggtgt atcgcaccgc cagctgccag cggccggggc gcccagcgcg 6660
tgtggccctg cccggcgtgg acgtgagccg gcctcggctg gtgctgccgc ggctggcgct 6720
gcctgtgggg cactactgct ttgtgtttgt cgtgtcattt ggggacacgc cactgacaca 6780
gagcatccag gccaatgtga cggtggcccc cgagcgcctg gtgcccatca ttgagggtgg 6840
ctcataccgc gtgtggtcag acacacggga cctggtgctg gatgggagcg agtcctacga 6900
ccccaacctg gaggacggcg accagacgcc gctcagtttc cactgggcct gtgtggcttc 6960
gacacagagg gaggctggcg ggtgtgcgct gaactttggg ccccgcggga gcagcacggt 7020
caccattcca cgggagcggc tggcggctgg cgtggagtac accttcagcc tgaccgtgtg 7080
gaaggccggc cgcaaggagg aggccaccaa ccagacggtg ctgatccgga gtggccgggt 7140
gcccattgtg tccttggagt gtgtgtcctg caaggcacag gccgtgtacg aagtgagccg 7200
cagctcctac gtgtacttgg agggccgctg cctcaattgc agcagcggct ccaagcgagg 7260
gcggtgggct gcacgtacgt tcagcaacaa gacgctggtg ctggatgaga ccaccacatc 7320
cacgggcagt gcaggcatgc gactggtgct gcggcggggc gtgctgcggg acggcgaggg 7380
atacaccttc acgctcacgg tgctgggccg ctctggcgag gaggagggct gcgcctccat 7440
ccgcctgtcc cccaaccgcc cgccgctggg gggctcttgc cgcctcttcc cactgggcgc 7500
tgtgcacgcc ctcaccacca aggtgcactt cgaatgcacg ggctggcatg acgcggagga 7560
tgctggcgcc ccgctggtgt acgccctgct gctgcggcgc tgtcgccagg gccactgcga 7620
ggagttctgt gtctacaagg gcagcctctc cagctacgga gccgtgctgc ccccgggttt 7680
caggccacac ttcgaggtgg gcctggccgt ggtggtgcag gaccagctgg gagccgctgt 7740
ggtcgccctc aacaggtctt tggccatcac cctcccagag cccaacggca gcgcaacggg 7800
gctcacagtc tggctgcacg ggctcaccgc tagtgtgctc ccagggctgc tgcggcaggc 7860
cgatccccag cacgtcatcg agtactcgtt ggccctggtc accgtgctga acgagtacga 7920
gcgggccctg gacgtggcgg cagagcccaa gcacgagcgg cagcaccgag cccagatacg 7980
caagaacatc acggagactc tggtgtccct gagggtccac actgtggatg acatccagca 8040
gatcgctgct gcgctggccc agtgcatggg gcccagcagg gagctcgtat gccgctcgtg 8100
cctgaagcag acgctgcaca agctggaggc catgatgctc atcctgcagg cagagaccac 8160
cgcgggcacc gtgacgccca ccgccatcgg agacagcatc ctcaacatca caggagacct 8220
catccacctg gccagctcgg acgtgcgggc accacagccc tcagagctgg gagccgagtc 8280
accatctcgg atggtggcgt cccaggccta caacctgacc tctgccctca tgcgcatcct 8340
catgcgctcc cgcgtgctca acgaggagcc cctgacgctg gcgggcgagg agatcgtggc 8400
ccagggcaag cgctcggacc cgcggagcct gctgtgctat ggcggcgccc cagggcctgg 8460
ctgccacttc tccatccccg aggctttcag cggggccctg gccaacctca gtgacgtggt 8520
gcagctcatc tttctggtgg actccaatcc ctttcccttt ggctatatca gcaactacac 8580
cgtctccacc aaggtggcct cgatggcatt ccagacacag gccggcgccc agatccccat 8640
cgagcggctg gcctcagagc gcgccatcac cgtgaaggtg cccaacaact cggactgggc 8700
tgcccggggc caccgcagct ccgccaactc cgttgtggtc cagccccagg cctccgtcgg 8760
tgctgtggtc accctggaca gcagcaaccc tgcggccgtg ctgcatctgc agctcaacta 8820
tacgctgctg gacggccact acctgtctga ggaacctgag ccctacctgg cagtctacct 8880
acactcggag ccccggccca atgagcacaa ctgctcggct agcaggagga tccgcccaga 8940
gtcactccag ggtgctgacc accggcccta caccttcttc atttccccgg ggagcagaga 9000
cccagcgggg agttaccatc tgaacctctc cagccacttc cgctggtcgg cgctgcaggt 9060
gtccgtgggc ctgtacacgt ccctgtgcca gtacttcagc gaggaggaca tggtgtggcg 9120
gacagagggg ctgctgcccc tggaggagac ctcgccccgc caggccgtct gcctcacccg 9180
ccacctcacc gccttcggcg ccagcctctt cgtgccccca agccatgtcc gctttgtgtt 9240
tcctgagccg acagcggatg taaactacat cgtcatgctg acatgtgctg tgtgcctggt 9300
gacctacatg gtcatggccg ccatcctgca caagctggac cagttggatg ccagccgggg 9360
ccgcgccatc cctttctgtg ggcagcgggg ccgcttcaag tacgagatcc tcgtcaagac 9420
aggctggggc cggggctcag gtaccacggc ccacgtgggc atcatgctgt atggggtgga 9480
cagccggagc ggccaccggc acctggacgg cgacagagcc ttccaccgca acagcctgga 9540
catcttccgg atcgccaccc cgcacagcct gggtagcgtg tggaagatcc gagtgtggca 9600
cgacaacaaa gggctcagcc ctgcctggtt cctgcagcac gtcatcgtca gggacctgca 9660
gacggcacgc agcgccttct tcctggtcaa tgactggctt tcggtggaga cggaggccaa 9720
cgggggcctg gtggagaagg aggtgctggc cgcgagcgac gcagcccttt tgcgcttccg 9780
gcgcctgctg gtggctgagc tgcagcgtgg cttctttgac aagcacatct ggctctccat 9840
atgggaccgg ccgcctcgta gccgtttcac tcgcatccag agggccacct gctgcgttct 9900
cctcatctgc ctcttcctgg gcgccaacgc cgtgtggtac ggggctgttg gcgactctgc 9960
ctacagcacg gggcatgtgt ccaggctgag cccgctgagc gtcgacacag tcgctgttgg 10020
cctggtgtcc agcgtggttg tctatcccgt ctacctggcc atcctttttc tcttccggat 10080
gtcccggagc aaggtggctg ggagcccgag ccccacacct gccgggcagc aggtgctgga 10140
catcgacagc tgcctggact cgtccgtgct ggacagctcc ttcctcacgt tctcaggcct 10200
ccacgctgag gcctttgttg gacagatgaa gagtgacttg tttctggatg attctaagag 10260
tctggtgtgc tggccctccg gcgagggaac gctcagttgg ccggacctgc tcagtgaccc 10320
gtccattgtg ggtagcaatc tgcggcagct ggcacggggc caggcgggcc atgggctggg 10380
cccagaggag gacggcttct ccctggccag cccctactcg cctgccaaat ccttctcagc 10440
atcagatgaa gacctgatcc agcaggtcct tgccgagggg gtcagcagcc cagcccctac 10500
ccaagacacc cacatggaaa cggacctgct cagcagcctg tccagcactc ctggggagaa 10560
gacagagacg ctggcgctgc agaggctggg ggagctgggg ccacccagcc caggcctgaa 10620
ctgggaacag ccccaggcag cgaggctgtc caggacagga ctggtggagg gtctgcggaa 10680
gcgcctgctg ccggcctggt gtgcctccct ggcccacggg ctcagcctgc tcctggtggc 10740

tgtggctgtg gctgtctcag ggtgggtggg tgcgagcttc cccccgggcg tgagtgttgc 10800
gtggctcctg tccagcagcg ccagcttcct ggcctcattc ctcggctggg agccactgaa 10860
ggtcttgctg gaagccctgt acttctcact ggtggccaag cggctgcacc cggatgaaga 10920
tgacaccctg gtagagagcc cggctgtgac gcctgtgagc gcacgtgtgc cccgcgtacg 10980
gccaccccac ggctttgcac tcttcctggc caaggaagaa gcccgcaagg tcaagaggct 11040
acatggcatg ctgcggagcc tcctggtgta catgcttttt ctgctggtga ccctgctggc 11100
cagctatggg gatgcctcat gccatgggca cgcctaccgt ctgcaaagcg ccatcaagca 11160
ggagctgcac agccgggcct tcctggccat cacgcggtct gaggagctct ggccatggat 11220
ggcccacgtg ctgctgccct acgtccacgg gaaccagtcc agcccagagc tggggccccc 11280
acggctgcgg caggtgcggc tgcaggaagc actctaccca gaccctcccg gccccagggt 11340
ccacacgtgc tcggccgcag gaggcttcag caccagcgat tacgacgttg gctgggagag 11400
tcctcacaat ggctcgggga cgtgggccta ttcagcgccg gatctgctgg gggcatggtc 11460
ctggggctcc tgtgccgtgt atgacagcgg gggctacgtg caggagctgg gcctgagcct 11520
ggaggagagc cgcgaccggc tgcgcttcct gcagctgcac aactggctgg acaacaggag 11580
ccgcgctgtg ttcctggagc tcacgcgcta cagcccggcc gtggggctgc acgccgccgt 11640
cacgctgcgc ctcgagttcc cggcggccgg ccgcgccctg gccgccctca gcgtccgccc 11700
ctttgcgctg cgccgcctca gcgcgggcct ctcgctgcct ctgctcacct cggtgtgcct 11760
gctgctgttc gccgtgcact tcgccgtggc cgaggcccgt acttggcaca gggaagggcg 11820
ctggcgcgtg ctgcggctcg gagcctgggc gcggtggctg ctggtggcgc tgacggcggc 11880
cacggcactg gtacgcctcg cccagctggg tgccgctgac cgccagtgga cccgtttcgt 11940
gcgcggccgc ccgcgccgct tcactagctt cgaccaggtg gcgcagctga gctccgcagc 12000
ccgtggcctg gcggcctcgc tgctcttcct gcttttggtc aaggctgccc agcagctacg 12060
cttcgtgcgc cagtggtccg tctttggcaa gacattatgc cgagctctgc cagagctcct 12120
gggggtcacc ttgggcctgg tggtgctcgg ggtagcctac gcccagctgg ccatcctgct 12180
cgtgtcttcc tgtgtggact ccctctggag cgtggcccag gccctgttgg tgctgtgccc 12240
tgggactggg ctctctaccc tgtgtcctgc cgagtcctgg cacctgtcac ccctgctgtg 12300
tgtggggctc tgggcactgc ggctgtgggg cgccctacgg ctgggggctg ttattctccg 12360
ctggcgctac cacgccttgc gtggagagct gtaccggccg gcctgggagc cccaggacta 12420
cgagatggtg gagttgttcc tgcgcaggct gcgcctctgg atgggcctca gcaaggtcaa 12480
ggagttccgc cacaaagtcc gctttgaagg gatggagccg ctgccctctc gctcctccag 12540
gggctccaag gtatccccgg atgtgccccc acccagcgct ggctccgatg cctcgcaccc 12600
ctccacctcc tccagccagc tggatgggct gagcgtgagc ctgggccggc tggggacaag 12660
gtgtgagcct gagccctccc gcctccaagc cgtgttcgag gccctgctca cccagtttga 12720
ccgactcaac caggccacag aggacgtcta ccagctggag cagcagctgc acagcctgca 12780
aggccgcagg agcagccggg cgcccgccgg atcttcccgt ggcccatccc cgggcctgcg 12840
gccagcactg cccagccgcc ttgcccgggc cagtcggggt gtggacctgg ccactggccc 12900
cagcaggaca ccccttcggg ccaagaacaa ggtccacccc agcagcactt agtcctcctt 12960
cctggcgggg gtgggccgtg gagtcggagt gg 12992

<210> 90
<211> 4299
<212> PRT
<213> Homo sapiens

<400> 90
Met Pro Pro Ala Ala Pro Ala Arg Leu Ala Leu Ala Leu Gly Leu Gly
1               5                   10                  15

Leu Trp Leu Gly Ala Leu Ala Gly Gly Pro Gly Arg Gly Cys Gly Pro
            20                  25                  30

Cys Glu Pro Pro Cys Leu Cys Gly Pro Ala Pro Gly Ala Ala Cys Arg
        35                  40                  45

Val Asn Cys Ser Gly Arg Gly Leu Arg Thr Leu Gly Pro Ala Leu Arg
    50                  55                  60

Ile Pro Ala Asp Ala Thr Ala Leu Asp Val Ser His Asn Leu Leu Arg
65                  70                  75                  80

Ala Leu Asp Val Gly Leu Leu Ala Asn Leu Ser Ala Leu Ala Glu Leu
                85                  90                  95

Asp Ile Ser Asn Asn Lys Ile Ser Thr Leu Glu Glu Gly Ile Phe Ala
            100                 105                 110

Asn Leu Phe Asn Leu Ser Glu Ile Asn Leu Ser Gly Asn Pro Phe Glu
        115                 120                 125

Cys Asp Cys Gly Leu Ala Trp Leu Pro Arg Trp Ala Glu Glu Gln Gln
    130                 135                 140

Val Arg Val Val Gln Pro Glu Ala Ala Thr Cys Ala Gly Pro Gly Ser
145                 150                 155                 160

Leu Ala Gly Gln Pro Leu Leu Gly Ile Pro Leu Leu Asp Ser Gly Cys
                165                 170                 175

Gly Glu Glu Tyr Val Ala Cys Leu Pro Asp Asn Ser Ser Gly Thr Val
            180                 185                 190

Ala Ala Val Ser Phe Ser Ala Ala His Glu Gly Leu Leu Gln Pro Glu
        195                 200                 205

Ala Cys Ser Ala Phe Cys Phe Ser Thr Gly Gln Gly Leu Ala Ala Leu
    210                 215                 220

Ser Glu Gln Gly Trp Cys Leu Cys Gly Ala Ala Gln Pro Ser Ser Ala
225                 230                 235                 240

Ser Phe Ala Cys Leu Ser Leu Cys Ser Gly Pro Pro Pro Pro Pro Ala
                245                 250                 255

Pro Thr Cys Arg Gly Pro Thr Leu Leu Gln His Val Phe Pro Ala Ser
            260                 265                 270

Pro Gly Ala Thr Leu Val Gly Pro His Gly Pro Leu Ala Ser Gly Gln
        275                 280                 285

Leu Ala Ala Phe His Ile Ala Ala Pro Leu Pro Val Thr Ala Thr Arg
    290                 295                 300

Trp Asp Phe Gly Asp Gly Ser Ala Glu Val Asp Ala Ala Gly Pro Ala
305                 310                 315                 320

Ala Ser His Arg Tyr Val Leu Pro Gly Arg Tyr His Val Thr Ala Val
                325                 330                 335

Leu Ala Leu Gly Ala Gly Ser Ala Leu Leu Gly Thr Asp Val Gln Val
            340                 345                 350

Glu Ala Ala Pro Ala Ala Leu Glu Leu Val Cys Pro Ser Ser Val Gln
        355                 360                 365

Ser Asp Glu Ser Leu Asp Leu Ser Ile Gln Asn Arg Gly Gly Ser Gly
    370                 375                 380

Leu Glu Ala Ala Tyr Ser Ile Val Ala Leu Gly Glu Glu Pro Ala Arg
385                 390                 395                 400

Ala Val His Pro Leu Cys Pro Ser Asp Thr Glu Ile Phe Pro Gly Asn
                405                 410                 415

Gly His Cys Tyr Arg Leu Val Val Glu Lys Ala Ala Trp Leu Gln Ala
            420                 425                 430

Gln Glu Gln Cys Gln Ala Trp Ala Gly Ala Ala Leu Ala Met Val Asp
        435                 440                 445

Ser Pro Ala Val Gln Arg Phe Leu Val Ser Arg Val Thr Arg Ser Leu
    450                 455                 460

Asp Val Trp Ile Gly Phe Ser Thr Val Gln Gly Val Glu Val Gly Pro
465                 470                 475                 480

Ala Pro Gln Gly Glu Ala Phe Ser Leu Glu Ser Cys Gln Asn Trp Leu
                485                 490                 495

Pro Gly Glu Pro His Pro Ala Thr Ala Glu His Cys Val Arg Leu Gly
            500                 505                 510

Pro Thr Gly Trp Cys Asn Thr Asp Leu Cys Ser Ala Pro His Ser Tyr
        515                 520                 525

Val Cys Glu Leu Gln Pro Gly Gly Pro Val Gln Asp Ala Glu Asn Leu
    530                 535                 540

Leu Val Gly Ala Pro Ser Gly Asp Leu Gln Gly Pro Leu Thr Pro Leu
545                 550                 555                 560

Ala Gln Gln Asp Gly Leu Ser Ala Pro His Glu Pro Val Glu Val Met
                565                 570                 575

Val Phe Pro Gly Leu Arg Leu Ser Arg Glu Ala Phe Leu Thr Thr Ala
            580                 585                 590

Glu Phe Gly Thr Gln Glu Leu Arg Arg Pro Ala Gln Leu Arg Leu Gln
        595                 600                 605

Val Tyr Arg Leu Leu Ser Thr Ala Gly Thr Pro Glu Asn Gly Ser Glu
    610                 615                 620

Pro Glu Ser Arg Ser Pro Asp Asn Arg Thr Gln Leu Ala Pro Ala Cys
625                 630                 635                 640

Met Pro Gly Gly Arg Trp Cys Pro Gly Ala Asn Ile Cys Leu Pro Leu
                645                 650                 655

Asp Ala Ser Cys His Pro Gln Ala Cys Ala Asn Gly Cys Thr Ser Gly
            660                 665                 670

Pro Gly Leu Pro Gly Ala Pro Tyr Ala Leu Trp Arg Glu Phe Leu Phe
        675                 680                 685

Ser Val Ala Ala Gly Pro Pro Ala Gln Tyr Ser Val Thr Leu His Gly
    690                 695                 700

Gln Asp Val Leu Met Leu Pro Gly Asp Leu Val Gly Leu Gln His Asp
705                 710                 715                 720

Ala Gly Pro Gly Ala Leu Leu His Cys Ser Pro Ala Pro Gly His Pro
                725                 730                 735

Gly Pro Gln Ala Pro Tyr Leu Ser Ala Asn Ala Ser Ser Trp Leu Pro
            740                 745                 750

His Leu Pro Ala Gln Leu Glu Gly Thr Trp Ala Cys Pro Ala Cys Ala
        755                 760                 765

Leu Arg Leu Leu Ala Ala Thr Glu Gln Leu Thr Val Leu Leu Gly Leu
    770                 775                 780

Arg Pro Asn Pro Gly Leu Arg Met Pro Gly Arg Tyr Glu Val Arg Ala
785                 790                 795                 800

Glu Val Gly Asn Gly Val Ser Arg His Asn Leu Ser Cys Ser Phe Asp
                805                 810                 815

Val Val Ser Pro Val Ala Gly Leu Arg Val Ile Tyr Pro Ala Pro Arg
            820                 825                 830

Asp Gly Arg Leu Tyr Val Pro Thr Asn Gly Ser Ala Leu Val Leu Gln
        835                 840                 845

Val Asp Ser Gly Ala Asn Ala Thr Ala Thr Ala Arg Trp Pro Gly Gly
    850                 855                 860

Ser Val Ser Ala Arg Phe Glu Asn Val Cys Pro Ala Leu Val Ala Thr
865                 870                 875                 880

Phe Val Pro Gly Cys Pro Trp Glu Thr Asn Asp Thr Leu Phe Ser Val
                885                 890                 895

Val Ala Leu Pro Trp Leu Ser Glu Gly Glu His Val Val Asp Val Val
            900                 905                 910

Val Glu Asn Ser Ala Ser Arg Ala Asn Leu Ser Leu Arg Val Thr Ala
        915                 920                 925

Glu Glu Pro Ile Cys Gly Leu Arg Ala Thr Pro Ser Pro Glu Ala Arg
    930                 935                 940

Val Leu Gln Gly Val Leu Val Arg Tyr Ser Pro Val Val Glu Ala Gly
945                 950                 955                 960

Ser Asp Met Val Phe Arg Trp Thr Ile Asn Asp Lys Gln Ser Leu Thr
                965                 970                 975

Phe Gln Asn Val Val Phe Asn Val Ile Tyr Gln Ser Ala Ala Val Phe
            980                 985                 990

Lys Leu Ser Leu Thr Ala Ser Asn His Val Ser Asn Val Thr Val Asn
        995                 1000                1005

Tyr Asn Val Thr Val Glu Arg Met Asn Arg Met Gln Gly Leu Gln Val
    1010                1015                1020

Ser Thr Val Pro Ala Val Leu Ser Pro Asn Ala Thr Leu Ala Leu Thr
1025                1030                1035                1040

Ala Gly Val Leu Val Asp Ser Ala Val Glu Val Ala Phe Leu Trp Asn
                1045                1050                1055

Phe Gly Asp Gly Glu Gln Ala Leu His Gln Phe Gln Pro Pro Tyr Asn
            1060                1065                1070

Glu Ser Phe Pro Val Pro Asp Pro Ser Val Ala Gln Val Leu Val Glu
        1075                1080                1085

His Asn Val Met His Thr Tyr Ala Ala Pro Gly Glu Tyr Leu Leu Thr
    1090                1095                1100

Val Leu Ala Ser Asn Ala Phe Glu Asn Leu Thr Gln Gln Val Pro Val
1105                1110                1115                1120

Ser Val Arg Ala Ser Leu Pro Ser Val Ala Val Gly Val Ser Asp Gly
                1125                1130                1135

Val Leu Val Ala Gly Arg Pro Val Thr Phe Tyr Pro His Pro Leu Pro
            1140                1145                1150

Ser Pro Gly Gly Val Leu Tyr Thr Trp Asp Phe Gly Asp Gly Ser Pro
        1155                1160                1165

Val Leu Thr Gln Ser Gln Pro Ala Ala Asn His Thr Tyr Ala Ser Arg
    1170                1175                1180

Gly Thr Tyr His Val Arg Leu Glu Val Asn Asn Thr Val Ser Gly Ala
1185                1190                1195                1200

Ala Ala Gln Ala Asp Val Arg Val Phe Glu Glu Leu Arg Gly Leu Ser
                1205                1210                1215

Val Asp Met Ser Leu Ala Val Glu Gln Gly Ala Pro Val Val Val Ser
            1220                1225                1230

Ala Ala Val Gln Thr Gly Asp Asn Ile Thr Trp Thr Phe Asp Met Gly
        1235                1240                1245

Asp Gly Thr Val Leu Ser Gly Pro Glu Ala Thr Val Glu His Val Tyr
    1250                1255                1260

Leu Arg Ala Gln Asn Cys Thr Val Thr Val Gly Ala Ala Ser Pro Ala
1265                1270                1275                1280

Gly His Leu Ala Arg Ser Leu His Val Leu Val Phe Val Leu Glu Val
                1285                1290                1295

Leu Arg Val Glu Pro Ala Ala Cys Ile Pro Thr Gln Pro Asp Ala Arg
            1300                1305                1310

Leu Thr Ala Tyr Val Thr Gly Asn Pro Ala His Tyr Leu Phe Asp Trp
        1315                1320                1325

Thr Phe Gly Asp Gly Ser Ser Asn Thr Thr Val Arg Gly Cys Pro Thr
    1330                1335                1340

Val Thr His Asn Phe Thr Arg Ser Gly Thr Phe Pro Leu Ala Leu Val
1345                1350                1355                1360

Leu Ser Ser Arg Val Asn Arg Ala His Tyr Phe Thr Ser Ile Cys Val
                1365                1370                1375

Glu Pro Glu Val Gly Asn Val Thr Leu Gln Pro Glu Arg Gln Phe Val
            1380                1385                1390

Gln Leu Gly Asp Glu Ala Trp Leu Val Ala Cys Ala Trp Pro Pro Phe
        1395                1400                1405

Pro Tyr Arg Tyr Thr Trp Asp Phe Gly Thr Glu Glu Ala Ala Pro Thr
    1410                1415                1420

Arg Ala Arg Gly Pro Glu Val Thr Phe Ile Tyr Arg Asp Pro Gly Ser
1425                1430                1435                1440

Tyr Leu Val Thr Val Thr Ala Ser Asn Asn Ile Ser Ala Ala Asn Asp
                1445                1450                1455

Ser Ala Leu Val Glu Val Gln Glu Pro Val Leu Val Thr Ser Ile Lys
            1460                1465                1470

Val Asn Gly Ser Leu Gly Leu Glu Leu Gln Gln Pro Tyr Leu Phe Ser
        1475                1480                1485

Ala Val Gly Arg Gly Arg Pro Ala Ser Tyr Leu Trp Asp Leu Gly Asp
    1490                1495                1500

Gly Gly Trp Leu Glu Gly Pro Glu Val Thr His Ala Tyr Asn Ser Thr
1505                1510                1515                1520

Gly Asp Phe Thr Val Arg Val Ala Gly Trp Asn Glu Val Ser Arg Ser
                1525                1530                1535

Glu Ala Trp Leu Asn Val Thr Val Lys Arg Arg Val Arg Gly Leu Val
            1540                1545                1550

Val Asn Ala Ser Arg Thr Val Val Pro Leu Asn Gly Ser Val Ser Phe
        1555                1560                1565

Ser Thr Ser Leu Glu Ala Gly Ser Asp Val Arg Tyr Ser Trp Val Leu
    1570                1575                1580

Cys Asp Arg Cys Thr Pro Ile Pro Gly Gly Pro Thr Ile Ser Tyr Thr
1585                1590                1595                1600

Phe Arg Ser Val Gly Thr Phe Asn Ile Ile Val Thr Ala Glu Asn Glu
                1605                1610                1615

Val Gly Ser Ala Gln Asp Ser Ile Phe Val Tyr Val Leu Gln Leu Ile
            1620                1625                1630

Glu Gly Leu Gln Val Val Gly Gly Gly Arg Tyr Phe Pro Thr Asn His
        1635                1640                1645

Thr Val Gln Leu Gln Ala Val Val Arg Asp Gly Thr Asn Val Ser Tyr
    1650                1655                1660

Ser Trp Thr Ala Trp Arg Asp Arg Gly Pro Ala Leu Ala Gly Ser Gly
1665                1670                1675                1680

Lys Gly Phe Ser Leu Thr Val Leu Glu Ala Gly Thr Tyr His Val Gln
                1685                1690                1695

Leu Arg Ala Thr Asn Met Leu Gly Ser Ala Trp Ala Asp Cys Thr Met
            1700                1705                1710

Asp Phe Val Glu Pro Val Gly Trp Leu Met Val Thr Ala Ser Pro Asn
        1715                1720                1725

Pro Ala Ala Val Asn Thr Ser Val Thr Leu Ser Ala Glu Leu Ala Gly
    1730                1735                1740

Gly Ser Gly Val Val Tyr Thr Trp Ser Leu Glu Glu Gly Leu Ser Trp
1745                1750                1755                1760

Glu Thr Ser Glu Pro Phe Thr Thr His Ser Phe Pro Thr Pro Gly Leu
                1765                1770                1775

His Leu Val Thr Met Thr Ala Gly Asn Pro Leu Gly Ser Ala Asn Ala
            1780                1785                1790

Thr Val Glu Val Asp Val Gln Val Pro Val Ser Gly Leu Ser Ile Arg
        1795                1800                1805

Ala Ser Glu Pro Gly Gly Ser Phe Val Ala Ala Gly Ser Ser Val Pro
    1810                1815                1820

Phe Trp Gly Gln Leu Ala Thr Gly Thr Asn Val Ser Trp Cys Trp Ala
1825                1830                1835                1840

Val Pro Gly Gly Ser Ser Lys Arg Gly Pro His Val Thr Met Val Phe
                1845                1850                1855

Pro Asp Ala Gly Thr Phe Ser Ile Arg Leu Asn Ala Ser Asn Ala Val
            1860                1865                1870

Ser Trp Val Ser Ala Thr Tyr Asn Leu Thr Ala Glu Glu Pro Ile Val
        1875                1880                1885

Gly Leu Val Leu Trp Ala Ser Ser Lys Val Val Ala Pro Gly Gln Leu
    1890                1895                1900

Val His Phe Gln Ile Leu Leu Ala Ala Gly Ser Ala Val Thr Phe Arg
1905                1910                1915                1920

Leu Gln Val Gly Gly Ala Asn Pro Glu Val Leu Pro Gly Pro Arg Phe
                1925                1930                1935

Ser His Ser Phe Pro Arg Val Gly Asp His Val Val Ser Val Arg Gly
            1940                1945                1950

Lys Asn His Val Ser Trp Ala Gln Ala Gln Val Arg Ile Val Val Leu
        1955                1960                1965

Glu Ala Val Ser Gly Leu Gln Met Pro Asn Cys Cys Glu Pro Gly Ile
    1970                1975                1980

Ala Thr Gly Thr Glu Arg Asn Phe Thr Ala Arg Val Gln Arg Gly Ser
1985                1990                1995                2000

Arg Val Ala Tyr Ala Trp Tyr Phe Ser Leu Gln Lys Val Gln Gly Asp
                2005                2010                2015

Ser Leu Val Ile Leu Ser Gly Arg Asp Val Thr Tyr Thr Pro Val Ala
            2020                2025                2030

Ala Gly Leu Leu Glu Ile Gln Val Arg Ala Phe Asn Ala Leu Gly Ser
        2035                2040                2045

Glu Asn Arg Thr Leu Val Leu Glu Val Gln Asp Ala Val Gln Tyr Val
    2050                2055                2060

Ala Leu Gln Ser Gly Pro Cys Phe Thr Asn Arg Ser Ala Gln Phe Glu
2065                2070                2075                2080

Ala Ala Thr Ser Pro Ser Pro Arg Arg Val Ala Tyr His Trp Asp Phe
                2085                2090                2095

Gly Asp Gly Ser Pro Gly Gln Asp Thr Asp Glu Pro Arg Ala Glu His
            2100                2105                2110

Ser Tyr Leu Arg Pro Gly Asp Tyr Arg Val Gln Val Asn Ala Ser Asn
        2115                2120                2125

Leu Val Ser Phe Phe Val Ala Gln Ala Thr Val Thr Val Gln Val Leu
    2130                2135                2140

Ala Cys Arg Glu Pro Glu Val Asp Val Val Leu Pro Leu Gln Val Leu
2145                2150                2155                2160

Met Arg Arg Ser Gln Arg Asn Tyr Leu Glu Ala His Val Asp Leu Arg
                2165                2170                2175

Asp Cys Val Thr Tyr Gln Thr Glu Tyr Arg Trp Glu Val Tyr Arg Thr
            2180                2185                2190

Ala Ser Cys Gln Arg Pro Gly Arg Pro Ala Arg Val Ala Leu Pro Gly
        2195                2200                2205

Val Asp Val Ser Arg Pro Arg Leu Val Leu Pro Arg Leu Ala Leu Pro
    2210                2215                2220

Val Gly His Tyr Cys Phe Val Phe Val Val Ser Phe Gly Asp Thr Pro
2225                2230                2235                2240

Leu Thr Gln Ser Ile Gln Ala Asn Val Thr Val Ala Pro Glu Arg Leu
                2245                2250                2255

Val Pro Ile Ile Glu Gly Gly Ser Tyr Arg Val Trp Ser Asp Thr Arg
            2260                2265                2270

Asp Leu Val Leu Asp Gly Ser Glu Ser Tyr Asp Pro Asn Leu Glu Asp
        2275                2280                2285

Gly Asp Gln Thr Pro Leu Ser Phe His Trp Ala Cys Val Ala Ser Thr
    2290                2295                2300

Gln Arg Glu Ala Gly Gly Cys Ala Leu Asn Phe Gly Pro Arg Gly Ser
2305                2310                2315                2320

Ser Thr Val Thr Ile Pro Arg Glu Arg Leu Ala Ala Gly Val Glu Tyr
                2325                2330                2335

Thr Phe Ser Leu Thr Val Trp Lys Ala Gly Arg Lys Glu Glu Ala Thr
            2340                2345                2350

Asn Gln Thr Val Leu Ile Arg Ser Gly Arg Val Pro Ile Val Ser Leu
        2355                2360                2365

Glu Cys Val Ser Cys Lys Ala Gln Ala Val Tyr Glu Val Ser Arg Ser
    2370                2375                2380

Ser Tyr Val Tyr Leu Glu Gly Arg Cys Leu Asn Cys Ser Ser Gly Ser
2385                2390                2395                2400

Lys Arg Gly Arg Trp Ala Ala Arg Thr Phe Ser Asn Lys Thr Leu Val
                2405                2410                2415

Leu Asp Glu Thr Thr Thr Ser Thr Gly Ser Ala Gly Met Arg Leu Val
            2420                2425                2430

Leu Arg Arg Gly Val Leu Arg Asp Gly Glu Gly Tyr Thr Phe Thr Leu
        2435                2440                2445

Thr Val Leu Gly Arg Ser Gly Glu Glu Glu Gly Cys Ala Ser Ile Arg
    2450                2455                2460

Leu Ser Pro Asn Arg Pro Pro Leu Gly Gly Ser Cys Arg Leu Phe Pro
2465                2470                2475                2480

Leu Gly Ala Val His Ala Leu Thr Thr Lys Val His Phe Glu Cys Thr
                2485                2490                2495

Gly Trp His Asp Ala Glu Asp Ala Gly Ala Pro Leu Val Tyr Ala Leu
            2500                2505                2510

Leu Leu Arg Arg Cys Arg Gln Gly His Cys Glu Glu Phe Cys Val Tyr
        2515                2520                2525

Lys Gly Ser Leu Ser Ser Tyr Gly Ala Val Leu Pro Pro Gly Phe Arg
    2530                2535                2540

Pro His Phe Glu Val Gly Leu Ala Val Val Val Gln Asp Gln Leu Gly
2545                2550                2555                2560

Ala Ala Val Val Ala Leu Asn Arg Ser Leu Ala Ile Thr Leu Pro Glu
                2565                2570                2575

Pro Asn Gly Ser Ala Thr Gly Leu Thr Val Trp Leu His Gly Leu Thr
            2580                2585                2590

Ala Ser Val Leu Pro Gly Leu Leu Arg Gln Ala Asp Pro Gln His Val
        2595                2600                2605

Ile Glu Tyr Ser Leu Ala Leu Val Thr Val Leu Asn Glu Tyr Glu Arg
    2610                2615                2620

Ala Leu Asp Val Ala Ala Glu Pro Lys His Glu Arg Gln His Arg Ala
2625                2630                2635                2640

Gln Ile Arg Lys Asn Ile Thr Glu Thr Leu Val Ser Leu Arg Val His
                2645                2650                2655

Thr Val Asp Asp Ile Gln Gln Ile Ala Ala Ala Leu Ala Gln Cys Met
            2660                2665                2670

Gly Pro Ser Arg Glu Leu Val Cys Arg Ser Cys Leu Lys Gln Thr Leu
        2675                2680                2685

His Lys Leu Glu Ala Met Met Leu Ile Leu Gln Ala Glu Thr Thr Ala
    2690                2695                2700

Gly Thr Val Thr Pro Thr Ala Ile Gly Asp Ser Ile Leu Asn Ile Thr
2705                2710                2715                2720

Gly Asp Leu Ile His Leu Ala Ser Ser Asp Val Arg Ala Pro Gln Pro
                2725                2730                2735

Ser Glu Leu Gly Ala Glu Ser Pro Ser Arg Met Val Ala Ser Gln Ala
            2740                2745                2750

Tyr Asn Leu Thr Ser Ala Leu Met Arg Ile Leu Met Arg Ser Arg Val
        2755                2760                2765

Leu Asn Glu Glu Pro Leu Thr Leu Ala Gly Glu Glu Ile Val Ala Gln
    2770                2775                2780

Gly Lys Arg Ser Asp Pro Arg Ser Leu Leu Cys Tyr Gly Gly Ala Pro
2785                2790                2795                2800

Gly Pro Gly Cys His Phe Ser Ile Pro Glu Ala Phe Ser Gly Ala Leu
                2805                2810                2815

Ala Asn Leu Ser Asp Val Val Gln Leu Ile Phe Leu Val Asp Ser Asn
            2820                2825                2830

Pro Phe Pro Phe Gly Tyr Ile Ser Asn Tyr Thr Val Ser Thr Lys Val
        2835                2840                2845

Ala Ser Met Ala Phe Gln Thr Gln Ala Gly Ala Gln Ile Pro Ile Glu
    2850                2855                2860

Arg Leu Ala Ser Glu Arg Ala Ile Thr Val Lys Val Pro Asn Asn Ser
2865                2870                2875                2880

Asp Trp Ala Ala Arg Gly His Arg Ser Ser Ala Asn Ser Val Val Val
                2885                2890                2895

Gln Pro Gln Ala Ser Val Gly Ala Val Val Thr Leu Asp Ser Ser Asn
            2900                2905                2910

Pro Ala Ala Val Leu His Leu Gln Leu Asn Tyr Thr Leu Leu Asp Gly
        2915                2920                2925

His Tyr Leu Ser Glu Glu Pro Glu Pro Tyr Leu Ala Val Tyr Leu His
    2930                2935                2940

Ser Glu Pro Arg Pro Asn Glu His Asn Cys Ser Ala Ser Arg Arg Ile
2945                2950                2955                2960

Arg Pro Glu Ser Leu Gln Gly Ala Asp His Arg Pro Tyr Thr Phe Phe
                2965                2970                2975

Ile Ser Pro Gly Ser Arg Asp Pro Ala Gly Ser Tyr His Leu Asn Leu
            2980                2985                2990

Ser Ser His Phe Arg Trp Ser Ala Leu Gln Val Ser Val Gly Leu Tyr
        2995                3000                3005

Thr Ser Leu Cys Gln Tyr Phe Ser Glu Glu Asp Met Val Trp Arg Thr
    3010                3015                3020

Glu Gly Leu Leu Pro Leu Glu Glu Thr Ser Pro Arg Gln Ala Val Cys
3025                3030                3035                3040

Leu Thr Arg His Leu Thr Ala Phe Gly Ala Ser Leu Phe Val Pro Pro
                3045                3050                3055

Ser His Val Arg Phe Val Phe Pro Glu Pro Thr Ala Asp Val Asn Tyr
            3060                3065                3070

Ile Val Met Leu Thr Cys Ala Val Cys Leu Val Thr Tyr Met Val Met
        3075                3080                3085

Ala Ala Ile Leu His Lys Leu Asp Gln Leu Asp Ala Ser Arg Gly Arg
    3090                3095                3100

Ala Ile Pro Phe Cys Gly Gln Arg Gly Arg Phe Lys Tyr Glu Ile Leu
3105                3110                3115                3120

Val Lys Thr Gly Trp Gly Arg Gly Ser Gly Thr Thr Ala His Val Gly
                3125                3130                3135

Ile Met Leu Tyr Gly Val Asp Ser Arg Ser Gly His Arg His Leu Asp
            3140                3145                3150

Gly Asp Arg Ala Phe His Arg Asn Ser Leu Asp Ile Phe Arg Ile Ala
        3155                3160                3165

Thr Pro His Ser Leu Gly Ser Val Trp Lys Ile Arg Val Trp His Asp
    3170                3175                3180

Asn Lys Gly Leu Ser Pro Ala Trp Phe Leu Gln His Val Ile Val Arg
3185                3190                3195                3200

Asp Leu Gln Thr Ala Arg Ser Ala Phe Phe Leu Val Asn Asp Trp Leu
                3205                3210                3215

Ser Val Glu Thr Glu Ala Asn Gly Gly Leu Val Glu Lys Glu Val Leu
            3220                3225                3230

Ala Ala Ser Asp Ala Ala Leu Leu Arg Phe Arg Arg Leu Leu Val Ala
        3235                3240                3245

Glu Leu Gln Arg Gly Phe Phe Asp Lys His Ile Trp Leu Ser Ile Trp
    3250                3255                3260

Asp Arg Pro Pro Arg Ser Arg Phe Thr Arg Ile Gln Arg Ala Thr Cys
3265                3270                3275                3280

Cys Val Leu Leu Ile Cys Leu Phe Leu Gly Ala Asn Ala Val Trp Tyr
                3285                3290                3295

Gly Ala Val Gly Asp Ser Ala Tyr Ser Thr Gly His Val Ser Arg Leu
            3300                3305                3310

Ser Pro Leu Ser Val Asp Thr Val Ala Val Gly Leu Val Ser Ser Val
        3315                3320                3325

Val Val Tyr Pro Val Tyr Leu Ala Ile Leu Phe Leu Phe Arg Met Ser
    3330                3335                3340

Arg Ser Lys Val Ala Gly Ser Pro Ser Pro Thr Pro Ala Gly Gln Gln
3345                3350                3355                3360

Val Leu Asp Ile Asp Ser Cys Leu Asp Ser Ser Val Leu Asp Ser Ser
                3365                3370                3375

Phe Leu Thr Phe Ser Gly Leu His Ala Glu Ala Phe Val Gly Gln Met
            3380                3385                3390

Lys Ser Asp Leu Phe Leu Asp Asp Ser Lys Ser Leu Val Cys Trp Pro
        3395                3400                3405

Ser Gly Glu Gly Thr Leu Ser Trp Pro Asp Leu Leu Ser Asp Pro Ser
    3410                3415                3420

Ile Val Gly Ser Asn Leu Arg Gln Leu Ala Arg Gly Gln Ala Gly His
3425                3430                3435                3440

Gly Leu Gly Pro Glu Glu Asp Gly Phe Ser Leu Ala Ser Pro Tyr Ser
                3445                3450                3455

Pro Ala Lys Ser Phe Ser Ala Ser Asp Glu Asp Leu Ile Gln Gln Val
            3460                3465                3470

Leu Ala Glu Gly Val Ser Ser Pro Ala Pro Thr Gln Asp Thr His Met
        3475                3480                3485

Glu Thr Asp Leu Leu Ser Ser Leu Ser Ser Thr Pro Gly Glu Lys Thr
    3490                3495                3500

Glu Thr Leu Ala Leu Gln Arg Leu Gly Glu Leu Gly Pro Pro Ser Pro
3505                3510                3515                3520

Gly Leu Asn Trp Glu Gln Pro Gln Ala Ala Arg Leu Ser Arg Thr Gly
                3525                3530                3535

Leu Val Glu Gly Leu Arg Lys Arg Leu Leu Pro Ala Trp Cys Ala Ser
            3540                3545                3550

Leu Ala His Gly Leu Ser Leu Leu Leu Val Ala Val Ala Val Ala Val
        3555                3560                3565

Ser Gly Trp Val Gly Ala Ser Phe Pro Pro Gly Val Ser Val Ala Trp
    3570                3575                3580

Leu Leu Ser Ser Ser Ala Ser Phe Leu Ala Ser Phe Leu Gly Trp Glu
3585                3590                3595                3600

Pro Leu Lys Val Leu Leu Glu Ala Leu Tyr Phe Ser Leu Val Ala Lys
                3605                3610                3615

Arg Leu His Pro Asp Glu Asp Asp Thr Leu Val Glu Ser Pro Ala Val
            3620                3625                3630

Thr Pro Val Ser Ala Arg Val Pro Arg Val Arg Pro Pro His Gly Phe
        3635                3640                3645

Ala Leu Phe Leu Ala Lys Glu Glu Ala Arg Lys Val Lys Arg Leu His
    3650                3655                3660

Gly Met Leu Arg Ser Leu Leu Val Tyr Met Leu Phe Leu Leu Val Thr
3665                3670                3675                3680

Leu Leu Ala Ser Tyr Gly Asp Ala Ser Cys His Gly His Ala Tyr Arg
                3685                3690                3695

Leu Gln Ser Ala Ile Lys Gln Glu Leu His Ser Arg Ala Phe Leu Ala
            3700                3705                3710

Ile Thr Arg Ser Glu Glu Leu Trp Pro Trp Met Ala His Val Leu Leu
        3715                3720                3725

Pro Tyr Val His Gly Asn Gln Ser Ser Pro Glu Leu Gly Pro Pro Arg
    3730                3735                3740

Leu Arg Gln Val Arg Leu Gln Glu Ala Leu Tyr Pro Asp Pro Pro Gly
3745                3750                3755                3760

Pro Arg Val His Thr Cys Ser Ala Ala Gly Gly Phe Ser Thr Ser Asp
                3765                3770                3775

Tyr Asp Val Gly Trp Glu Ser Pro His Asn Gly Ser Gly Thr Trp Ala
            3780                3785                3790

Tyr Ser Ala Pro Asp Leu Leu Gly Ala Trp Ser Trp Gly Ser Cys Ala
        3795                3800                3805

Val Tyr Asp Ser Gly Gly Tyr Val Gln Glu Leu Gly Leu Ser Leu Glu
    3810                3815                3820

Glu Ser Arg Asp Arg Leu Arg Phe Leu Gln Leu His Asn Trp Leu Asp
3825                3830                3835                3840

Asn Arg Ser Arg Ala Val Phe Leu Glu Leu Thr Arg Tyr Ser Pro Ala
                3845                3850                3855

Val Gly Leu His Ala Ala Val Thr Leu Arg Leu Glu Phe Pro Ala Ala
            3860                3865                3870

Gly Arg Ala Leu Ala Ala Leu Ser Val Arg Pro Phe Ala Leu Arg Arg
        3875                3880                3885

Leu Ser Ala Gly Leu Ser Leu Pro Leu Leu Thr Ser Val Cys Leu Leu
    3890                3895                3900

Leu Phe Ala Val His Phe Ala Val Ala Glu Ala Arg Thr Trp His Arg
3905                3910                3915                3920

Glu Gly Arg Trp Arg Val Leu Arg Leu Gly Ala Trp Ala Arg Trp Leu
                3925                3930                3935

Leu Val Ala Leu Thr Ala Ala Thr Ala Leu Val Arg Leu Ala Gln Leu
            3940                3945                3950

Gly Ala Ala Asp Arg Gln Trp Thr Arg Phe Val Arg Gly Arg Pro Arg
        3955                3960                3965

Arg Phe Thr Ser Phe Asp Gln Val Ala Gln Leu Ser Ser Ala Ala Arg
    3970                3975                3980

Gly Leu Ala Ala Ser Leu Leu Phe Leu Leu Leu Val Lys Ala Ala Gln
3985                3990                3995                4000

Gln Leu Arg Phe Val Arg Gln Trp Ser Val Phe Gly Lys Thr Leu Cys
                4005                4010                4015

Arg Ala Leu Pro Glu Leu Leu Gly Val Thr Leu Gly Leu Val Val Leu
            4020                4025                4030

Gly Val Ala Tyr Ala Gln Leu Ala Ile Leu Leu Val Ser Ser Cys Val
        4035                4040                4045

Asp Ser Leu Trp Ser Val Ala Gln Ala Leu Leu Val Leu Cys Pro Gly
    4050                4055                4060

Thr Gly Leu Ser Thr Leu Cys Pro Ala Glu Ser Trp His Leu Ser Pro
4065                4070                4075                4080

Leu Leu Cys Val Gly Leu Trp Ala Leu Arg Leu Trp Gly Ala Leu Arg
                4085                4090                4095

Leu Gly Ala Val Ile Leu Arg Trp Arg Tyr His Ala Leu Arg Gly Glu
            4100                4105                4110

Leu Tyr Arg Pro Ala Trp Glu Pro Gln Asp Tyr Glu Met Val Glu Leu
        4115                4120                4125

Phe Leu Arg Arg Leu Arg Leu Trp Met Gly Leu Ser Lys Val Lys Glu
    4130                4135                4140

Phe Arg His Lys Val Arg Phe Glu Gly Met Glu Pro Leu Pro Ser Arg
4145                4150                4155                4160

Ser Ser Arg Gly Ser Lys Val Ser Pro Asp Val Pro Pro Pro Ser Ala
                4165                4170                4175

Gly Ser Asp Ala Ser His Pro Ser Thr Ser Ser Ser Gln Leu Asp Gly
            4180                4185                4190

Leu Ser Val Ser Leu Gly Arg Leu Gly Thr Arg Cys Glu Pro Glu Pro
        4195                4200                4205

Ser Arg Leu Gln Ala Val Phe Glu Ala Leu Leu Thr Gln Phe Asp Arg
    4210                4215                4220

Leu Asn Gln Ala Thr Glu Asp Val Tyr Gln Leu Glu Gln Gln Leu His
4225                4230                4235                4240

Ser Leu Gln Gly Arg Arg Ser Ser Arg Ala Pro Ala Gly Ser Ser Arg
                4245                4250                4255

Gly Pro Ser Pro Gly Leu Arg Pro Ala Leu Pro Ser Arg Leu Ala Arg
            4260                4265                4270

Ala Ser Arg Gly Val Asp Leu Ala Thr Gly Pro Ser Arg Thr Pro Leu
        4275                4280                4285

Arg Ala Lys Asn Lys Val His Pro Ser Ser Thr
    4290                4295


<210> 91
<211> 1222
<212> DNA
<213> Homo sapiens

<400> 91
attgcgggcg gcggcgttcg gagtcgccgg gagctgccag gctgtccgcg ccgccgctgc 60
ggggccatga tccggaacgg gcgcggggct gcaggcggcg cagagcagcc gggcccgggg 120
ggcaggcgcg ccgtgagggt gtggtgcgat ggctgctatg acatggtgca ttacggccac 180
tccaaccagc tgcgccaggc acgggccatg ggtgactacc tcatcgtagg cgtgcacacc 240
gatgaggaga tcgccaagca caaggggccc ccggtgttca ctcaggagga gagatacaag 300
atggtgcagg ccatcaaatg ggtggacgag gtggtgccag cggctcccta cgtcactaca 360
ctagagaccc tggacaaata caactgtgac ttctgtgttc acggcaatga catcaccctg 420
actgtagatg gccgggacac ctatgaggaa gtaaagcagg ctgggaggta cagagaatgc 480
aagcgcacgc aaggggtgtc caccacagac ctcgtgggcc gcatgctgct ggtaaccaaa 540
gcccatcaca gcagccagga gatgtcctct gagtaccggg agtatgcaga cagttttggc 600
aagtgccctg gtgggcggaa cccctggacc ggggtatccc agttcctgca gacatctcag 660
aagatcatcc agtttgcttc tgggaaggag ccccagccag gggagacagt catctatgtg 720
gctggtgcct tcgacctgtt ccacatcggg catgtggact tcctggagaa ggtgcacagg 780
ctggcagaga ggccctacat catcgcgggc ttacactttg accagtacgt gtcagaagtg 840
gtgattggag ccccgtacgc ggtcacagca gagctcctaa gtcacttcaa ggtggacctg 900
gtgtgtcacg gcaagacagg aattatccct gacagggatg gctccgaccc ataccaggag 960
cccaagagaa ggggcatctt ccgtcagatt gacagtggca gcaacctcac cacagacctc 1020
atcgtccagc ggatcatcac caacaggttg gagtatgagg cgcgaaacca gaagaaggaa 1080
gccaaggagc tggccttcct ggaggctgcc aggcagcagg cggcacagcc cctgggggag 1140
cgcgatggtg acttctaacc tggcagaggc cctggccggc cctccccctg ctctgcttct 1200
gcgccttctg cgtttggaca ta 1222


<210> 92
<211> 363
<212> PRT
<213> Homo sapiens

<400> 92
Met Ile Arg Asn Gly Arg Gly Ala Ala Gly Gly Ala Glu Gln Pro Gly
1               5                   10                  15

Pro Gly Gly Arg Arg Ala Val Arg Val Trp Cys Asp Gly Cys Tyr Asp
            20                  25                  30

Met Val His Tyr Gly His Ser Asn Gln Leu Arg Gln Ala Arg Ala Met
        35                  40                  45

Gly Asp Tyr Leu Ile Val Gly Val His Thr Asp Glu Glu Ile Ala Lys
    50                  55                  60

His Lys Gly Pro Pro Val Phe Thr Gln Glu Glu Arg Tyr Lys Met Val
65                  70                  75                  80

Gln Ala Ile Lys Trp Val Asp Glu Val Val Pro Ala Ala Pro Tyr Val
                85                  90                  95

Thr Thr Leu Glu Thr Leu Asp Lys Tyr Asn Cys Asp Phe Cys Val His
            100                 105                 110

Gly Asn Asp Ile Thr Leu Thr Val Asp Gly Arg Asp Thr Tyr Glu Glu
        115                 120                 125

Val Lys Gln Ala Gly Arg Tyr Arg Glu Cys Lys Arg Thr Gln Gly Val
    130                 135                 140

Ser Thr Thr Asp Leu Val Gly Arg Met Leu Leu Val Thr Lys Ala His
145                 150                 155                 160

His Ser Ser Gln Glu Met Ser Ser Glu Tyr Arg Glu Tyr Ala Asp Ser
                165                 170                 175

Phe Gly Lys Cys Pro Gly Gly Arg Asn Pro Trp Thr Gly Val Ser Gln
            180                 185                 190

Phe Leu Gln Thr Ser Gln Lys Ile Ile Gln Phe Ala Ser Gly Lys Glu
        195                 200                 205

Pro Gln Pro Gly Glu Thr Val Ile Tyr Val Ala Gly Ala Phe Asp Leu
    210                 215                 220

Phe His Ile Gly His Val Asp Phe Leu Glu Lys Val His Arg Leu Ala
225                 230                 235                 240

Glu Arg Pro Tyr Ile Ile Ala Gly Leu His Phe Asp Gln Tyr Val Ser
                245                 250                 255

Glu Val Val Ile Gly Ala Pro Tyr Ala Val Thr Ala Glu Leu Leu Ser
            260                 265                 270

His Phe Lys Val Asp Leu Val Cys His Gly Lys Thr Gly Ile Ile Pro
        275                 280                 285

Asp Arg Asp Gly Ser Asp Pro Tyr Gln Glu Pro Lys Arg Arg Gly Ile
    290                 295                 300

Phe Arg Gln Ile Asp Ser Gly Ser Asn Leu Thr Thr Asp Leu Ile Val
305                 310                 315                 320

Gln Arg Ile Ile Thr Asn Arg Leu Glu Tyr Glu Ala Arg Asn Gln Lys
                325                 330                 335

Lys Glu Ala Lys Glu Leu Ala Phe Leu Glu Ala Ala Arg Gln Gln Ala
            340                 345                 350

Ala Gln Pro Leu Gly Glu Arg Asp Gly Asp Phe
        355                 360

<210> 93
<211> 893
<212> DNA
<213> Homo sapiens

<400> 93
gtaccttaaa gacaacaaac aagcaaacac aacttataat taaaaaacat gcaaagggct 60

caccttccac ttccttctgg tcctgctcct cttcttcctc ctctcctgcc tcctcttctc 120
cctgttccat cagaccttct ggggcccctt tcaataagca gctgctggcc ggccagccct 180
tgggggcagg gctggaaccg gggcagggga ggctgcgggg ccactcgctg gagaggcaaa 240
caggaaggac tgccccctga gcgccaggct tcgggcccgg gaatcgccgc cgccgccgcc 300
gcagagctgc agctcggggc cgagggtaag gaggcgagcc gggagcggga ggcccgggag 360
agctccgcgg gtccccgcgc ccagtcccca gccgcgcccc gaccccgccg ccccgggcct 420
aacgcggccg gcgaggccta cgcggcggcc gccgtcaccg tgctggagcc gccggcctcc 480
gaccccgagc tgcagcccgc cgagcgcccg ctgccatcgc cggggtccgg ggagggcgcc 540
ccggtcttcc tcacggggcc tcgatcccag tgggtgctgc ggggggcgga ggtggtgctg 600
acgtgccggg cggggggcct ccccgagccc acactgtact gggagaagga cgggatggcc 660
ctggacgaag tgtgggacag cagccacttc gcgctccagc cgggccgcgc cgaggacggc 720
cccggcgcga gcctggcact gcgcatcctg gcggctcggc tgccggattc cggcgtctac 780
gtgtgccacg cccgcaacgc gcacggccac gcgcaggcgg gggcgctgct ccaggtgctg 840
acccccacct tcctgccgcc aagacagccc taaccaaggc ccagaaaggg tag 893

<210> 94
<211> 274
<212> PRT
<213> Homo sapiens

<400> 94
Met Gln Arg Ala His Leu Pro Leu Pro Ser Gly Pro Ala Pro Leu Leu
1               5                   10                  15

Pro Pro Leu Leu Pro Pro Leu Leu Pro Val Pro Ser Asp Leu Leu Gly
            20                  25                  30

Pro Leu Ser Ile Ser Ser Cys Trp Pro Ala Ser Pro Trp Gly Gln Gly
        35                  40                  45

Trp Asn Arg Gly Arg Gly Gly Cys Gly Ala Thr Arg Trp Arg Gly Lys
    50                  55                  60

Gln Glu Gly Leu Pro Pro Glu Arg Gln Ala Ser Gly Pro Gly Ile Ala
65                  70                  75                  80

Ala Ala Ala Ala Ala Glu Leu Gln Leu Gly Ala Glu Gly Lys Glu Ala
                85                  90                  95

Ser Arg Glu Arg Glu Ala Arg Glu Ser Ser Ala Gly Pro Arg Ala Gln
            100                 105                 110

Ser Pro Ala Ala Pro Arg Pro Arg Arg Pro Gly Pro Asn Ala Ala Gly
        115                 120                 125

Glu Ala Tyr Ala Ala Ala Ala Val Thr Val Leu Glu Pro Pro Ala Ser
    130                 135                 140

Asp Pro Glu Leu Gln Pro Ala Glu Arg Pro Leu Pro Ser Pro Gly Ser
145                 150                 155                 160

Gly Glu Gly Ala Pro Val Phe Leu Thr Gly Pro Arg Ser Gln Trp Val
                165                 170                 175

Leu Arg Gly Ala Glu Val Val Leu Thr Cys Arg Ala Gly Gly Leu Pro
            180                 185                 190

Glu Pro Thr Leu Tyr Trp Glu Lys Asp Gly Met Ala Leu Asp Glu Val
        195                 200                 205

Trp Asp Ser Ser His Phe Ala Leu Gln Pro Gly Arg Ala Glu Asp Gly
    210                 215                 220

Pro Gly Ala Ser Leu Ala Leu Arg Ile Leu Ala Ala Arg Leu Pro Asp
225                 230                 235                 240

Ser Gly Val Tyr Val Cys His Ala Arg Asn Ala His Gly His Ala Gln
                245                 250                 255

Ala Gly Ala Leu Leu Gln Val Leu Thr Pro Thr Phe Leu Pro Pro Arg
            260                 265                 270

Gln Pro

<210> 95
<211> 316
<212> PRT
<213> Mus musculus

<400> 95
Met Leu Phe Ile Leu Val Leu Leu Thr Ser Phe Leu Ser Ile Leu Tyr
  1               5                  10                  15

Leu Thr Ala Pro Ser Ile Arg Lys Phe Phe Ala Gly Gly Val Cys Thr
            20                  25                  30

Thr Asn Val Gln Ile Pro Gly Lys Val Val Val Ile Thr Gly Ala Asn
        35                  40                  45

Thr Gly Ile Gly Lys Glu Thr Ala Arg Glu Leu Ala Arg Arg Gly Ala
    50                  55                  60

Arg Val Tyr Ile Ala Cys Arg Asp Val Leu Lys Gly Glu Ser Ala Ala
 65                  70                  75                  80

Ser Glu Ile Arg Ala Asp Thr Lys Asn Ser Gln Val Leu Val Arg Lys
                 85                  90                  95

Leu Asp Leu Ser Asp Thr Lys Ser Ile Arg Ala Phe Ala Glu Arg Phe
            100                 105                 110

Leu Ala Glu Glu Lys Lys Leu Asp Ile Leu Ile Asn Asn Ala Gly Val
        115                 120                 125

Met Met Cys Pro Tyr Ser Lys Thr Thr Asp Gly Phe Glu Thr His Phe
    130                 135                 140

Gly Val Asn His Leu Gly His Phe Leu Leu Thr Tyr Leu Leu Leu Glu
145                 150                 155                 160

Arg Leu Lys Glu Ser Ala Pro Ala Arg Val Val Asn Leu Ser Ser Ile
                165                 170                 175

Ala His Leu Ile Gly Lys Ile Arg Phe His Asp Leu Gln Gly Gln Lys
            180                 185                 190

Arg Tyr Cys Ser Ala Phe Ala Tyr Gly His Ser Lys Leu Ala Asn Leu
        195                 200                 205

Leu Phe Thr Arg Glu Leu Ala Lys Arg Leu Gln Gly Thr Gly Val Thr
    210                 215                 220

Ala Tyr Ala Val His Pro Gly Val Val Leu Ser Glu Ile Thr Arg Asn
225                 230                 235                 240

Ser Tyr Leu Leu Cys Leu Leu Trp Arg Leu Phe Ser Pro Phe Phe Lys
                245                 250                 255

Ser Thr Ser Gln Gly Ala Gln Thr Ser Leu His Cys Ala Leu Ala Glu
            260                 265                 270

Asp Leu Glu Pro Leu Ser Gly Lys Tyr Phe Ser Asp Cys Lys Arg Met
        275                 280                 285

Trp Val Ser Ser Arg Ala Arg Asn Lys Lys Thr Ala Glu Arg Leu Trp

290                 295                 300

Asn Val Ser Cys Glu Leu Leu Gly Ile Gln Trp Glu
305                 310                 315

<210> 96
<211> 304
<212> PRT
<213> Mus musculus

<400> 96
Met Leu Phe Ile Leu Val Leu Leu Thr Ser Phe Leu Ser Ile Leu Tyr
1               5                   10                  15

Leu Thr Ala Pro Ser Ile Arg Lys Phe Phe Ala Gly Gly Val Cys Thr
            20                  25                  30

Thr Asn Val Gln Ile Pro Gly Lys Val Val Val Ile Thr Gly Ala Asn
        35                  40                  45

Thr Gly Ile Gly Lys Glu Thr Ala Arg Glu Leu Ala Arg Arg Gly Ala
    50                  55                  60

Arg Val Tyr Ile Ala Cys Arg Asp Val Leu Lys Gly Glu Ser Ala Ala
65                  70                  75                  80

Ser Glu Ile Arg Ala Asp Thr Lys Asn Ser Gln Val Leu Val Arg Lys
                85                  90                  95

Leu Asp Leu Ser Asp Thr Lys Ser Ile Arg Ala Phe Ala Glu Arg Phe
            100                 105                 110

Leu Ala Gly Val Met Met Cys Pro Tyr Ser Lys Thr Thr Asp Gly Phe
        115                 120                 125

Glu Thr His Phe Gly Val Asn His Leu Gly His Phe Leu Leu Thr Tyr
    130                 135                 140

Leu Leu Leu Glu Arg Leu Lys Glu Ser Ala Pro Ala Arg Val Val Asn
145                 150                 155                 160

Leu Ser Ser Ile Ala His Leu Ile Gly Lys Ile Arg Phe His Asp Leu
                165                 170                 175

Gln Gly Gln Lys Arg Tyr Cys Ser Ala Phe Ala Tyr Gly His Ser Lys
            180                 185                 190

Leu Ala Asn Leu Leu Phe Thr Arg Glu Leu Ala Lys Arg Leu Gln Gly
        195                 200                 205

Thr Gly Val Thr Ala Tyr Ala Val His Pro Gly Val Val Leu Ser Glu
    210                 215                 220

Ile Thr Arg Asn Ser Tyr Leu Leu Cys Leu Leu Trp Arg Leu Phe Ser
225                 230                 235                 240

Pro Phe Phe Lys Ser Thr Ser Gln Gly Ala Gln Thr Ser Leu His Cys
                245                 250                 255

Ala Leu Ala Glu Asp Leu Glu Pro Leu Ser Gly Lys Tyr Phe Ser Asp
            260                 265                 270

Cys Lys Arg Met Trp Val Ser Ser Arg Ala Arg Asn Lys Lys Thr Ala
        275                 280                 285

Glu Arg Leu Trp Asn Val Ser Cys Glu Leu Leu Gly Ile Gln Trp Glu
    290                 295                 300

<210> 97
<211> 318
<212> PRT
<213> Homo sapiens

<400> 97
Met Val Glu Leu Met Phe Pro Leu Leu Leu Leu Leu Leu Pro Phe Leu
1               5                   10                  15

Leu Tyr Met Ala Ala Pro Gln Ile Arg Lys Met Leu Ser Ser Gly Val
            20                  25                  30

Cys Thr Ser Thr Val Gln Leu Pro Gly Lys Val Val Val Val Thr Gly
        35                  40                  45

Ala Asn Thr Gly Ile Gly Lys Glu Thr Ala Lys Glu Leu Ala Gln Arg
    50                  55                  60

Gly Ala Arg Val Tyr Leu Ala Cys Arg Asp Val Glu Lys Gly Glu Leu
65                  70                  75                  80

Val Ala Lys Glu Ile Gln Thr Thr Thr Gly Asn Gln Gln Val Leu Val
                85                  90                  95

Arg Lys Leu Asp Leu Ser Asp Thr Lys Ser Ile Arg Ala Phe Ala Lys
            100                 105                 110

Gly Phe Leu Ala Glu Glu Lys His Leu His Val Leu Ile Asn Asn Ala
        115                 120                 125

Gly Val Met Met Cys Pro Tyr Ser Lys Thr Ala Asp Gly Phe Glu Met
    130                 135                 140

His Ile Gly Val Asn His Leu Gly His Phe Leu Leu Thr His Leu Leu
145                 150                 155                 160

Leu Glu Lys Leu Lys Glu Ser Ala Pro Ser Arg Ile Val Asn Val Ser
                165                 170                 175

Ser Leu Ala His His Leu Gly Arg Ile His Phe His Asn Leu Gln Gly
            180                 185                 190

Glu Lys Phe Tyr Asn Ala Gly Leu Ala Tyr Cys His Ser Lys Leu Ala
        195                 200                 205

Asn Ile Leu Phe Thr Gln Glu Leu Ala Arg Arg Leu Lys Gly Ser Gly
    210                 215                 220

Val Thr Thr Tyr Ser Val His Pro Gly Thr Val Gln Ser Glu Leu Val
225                 230                 235                 240

Arg His Ser Ser Phe Met Arg Trp Met Trp Trp Leu Phe Ser Phe Phe
                245                 250                 255

Ile Lys Thr Pro Gln Gln Gly Ala Gln Thr Ser Leu His Cys Ala Leu
            260                 265                 270

Thr Glu Gly Leu Glu Ile Leu Ser Gly Asn His Phe Ser Asp Cys His
        275                 280                 285

Val Ala Trp Val Ser Val Gln Ala Arg Asn Glu Thr Ile Ala Arg Arg
    290                 295                 300

Leu Trp Asp Val Ser Cys Asp Leu Leu Gly Leu Pro Ile Asp
305                 310                 315

<210> 98
<211> 318
<212> PRT
<213> Homo sapiens

<400> 98
Met Val Glu Leu Met Phe Pro Leu Leu Leu Leu Leu Leu Pro Phe Leu
1               5                   10                  15

Leu Tyr Met Ala Ala Pro Gln Ile Arg Lys Met Leu Ser Ser Gly Val
            20                  25                  30

Cys Thr Ser Thr Val Gln Leu Pro Gly Lys Val Val Val Val Thr Gly
        35                  40                  45

Ala Asn Thr Gly Ile Gly Lys Glu Thr Ala Lys Glu Leu Ala Gln Arg
    50                  55                  60

Gly Ala Arg Val Tyr Leu Ala Cys Arg Asp Val Glu Lys Gly Glu Leu
65                  70                  75                  80

Val Ala Lys Glu Ile Gln Thr Thr Thr Gly Asn Gln Gln Val Leu Val
                85                  90                  95

Arg Lys Leu Asp Leu Ser Asp Thr Lys Ser Ile Arg Ala Phe Ala Lys
            100                 105                 110

Gly Phe Leu Ala Glu Glu Lys His Leu His Val Leu Ile Asn Asn Ala
        115                 120                 125

Gly Val Met Met Cys Pro Tyr Ser Lys Thr Ala Asp Gly Phe Glu Met
    130                 135                 140

His Ile Gly Val Asn His Leu Gly His Phe Leu Leu Thr His Leu Leu
145                 150                 155                 160

Leu Glu Lys Leu Lys Glu Ser Ala Pro Ser Arg Ile Val Asn Val Ser
                165                 170                 175

Ser Leu Ala His His Leu Gly Arg Ile His Phe His Asn Leu Gln Gly
            180                 185                 190

Glu Lys Phe Tyr Asn Ala Gly Leu Ala Tyr Cys His Ser Lys Leu Ala
        195                 200                 205

Asn Ile Leu Phe Thr Gln Glu Leu Ala Arg Arg Leu Lys Gly Ser Gly
    210                 215                 220

Val Thr Thr Tyr Ser Val His Pro Gly Thr Val Gln Ser Glu Leu Val
225                 230                 235                 240

Arg His Ser Ser Phe Met Arg Trp Met Trp Trp Leu Phe Ser Phe Phe
                245                 250                 255

Ile Lys Thr Pro Gln Gln Gly Ala Gln Thr Ser Leu His Cys Ala Leu
            260                 265                 270

Thr Glu Gly Leu Glu Ile Leu Ser Gly Asn His Phe Ser Asp Cys His
        275                 280                 285

Val Ala Trp Val Ser Ala Gln Ala Arg Asn Glu Thr Ile Ala Arg Arg
    290                 295                 300

Leu Trp Asp Val Ser Cys Asp Leu Leu Gly Leu Pro Ile Asp
305                 310                 315

<210> 99
<211> 316
<212> PRT
<213> Mus musculus

<400> 99
Met Phe Gly Phe Leu Leu Leu Leu Ser Leu Pro Phe Ile Leu Tyr Leu
1               5                   10                  15

Val Thr Pro Lys Ile Arg Lys Met Leu Ser Ser Gly Val Cys Thr Ser
            20                  25                  30

Asn Val Gln Leu Pro Gly Lys Val Ala Ile Val Thr Gly Ala Asn Thr
        35                  40                  45

Gly Ile Gly Lys Glu Thr Ala Lys Asp Leu Ala Gln Arg Gly Ala Arg
    50                  55                  60

Val Tyr Leu Ala Cys Arg Asp Val Asp Lys Gly Glu Leu Ala Ala Arg
65                  70                  75                  80

Glu Ile Gln Ala Val Thr Gly Asn Ser Gln Val Phe Val Arg Lys Leu
                85                  90                  95

Asp Leu Ala Asp Thr Lys Ser Ile Arg Ala Phe Ala Lys Asp Phe Leu
            100                 105                 110

Ala Glu Glu Lys His Leu His Leu Leu Ile Asn Asn Ala Gly Val Met
        115                 120                 125

Met Cys Pro Tyr Ser Lys Thr Ala Asp Gly Phe Glu Met His Ile Gly
    130                 135                 140

Val Asn His Leu Gly His Phe Leu Leu Thr His Leu Leu Leu Glu Lys
145                 150                 155                 160

Leu Lys Glu Ser Ala Pro Ser Arg Ile Val Asn Leu Ser Ser Leu Gly
                165                 170                 175

His His Leu Gly Arg Ile His Phe His Asn Leu Gln Gly Glu Lys Phe
            180                 185                 190

Tyr Ser Ala Gly Leu Ala Tyr Cys His Ser Lys Leu Ala Asn Ile Leu
        195                 200                 205

Phe Thr Lys Glu Leu Ala Lys Arg Leu Lys Gly Ser Gly Val Thr Thr
    210                 215                 220

Tyr Ser Val His Pro Gly Thr Val His Ser Glu Leu Thr Arg Tyr Ser
225                 230                 235                 240

Ser Ile Met Arg Trp Leu Trp Gln Leu Phe Phe Val Phe Ile Lys Thr
                245                 250                 255

Pro Gln Glu Gly Ala Gln Thr Ser Leu Tyr Cys Ala Leu Thr Glu Gly
            260                 265                 270

Leu Glu Ser Leu Ser Gly Ser His Phe Ser Asp Cys Gln Leu Ala Trp
        275                 280                 285

Val Ser Tyr Gln Gly Arg Asn Glu Ile Ile Ala Arg Arg Leu Trp Asp
    290                 295                 300

Val Ser Cys Asp Leu Leu Gly Leu Pro Val Asp Trp
305                 310                 315

<210> 100
<211> 249

<212> PRT
<213> Homo sapiens

<400> 100
Thr Gly Lys Val Ala Leu Val Thr Gly Ala Ser Ser Gly Ile Gly Leu
1               5                   10                  15

Ala Ile Ala Lys Arg Leu Ala Glu Glu Gly Ala Lys Val Val Val Val
            20                  25                  30

Asp Arg Arg Glu Glu Lys Ala Glu Ala Ala Ala Glu Leu Lys Ala Glu
        35                  40                  45

Leu Gly Asp Arg Ala Leu Phe Ile Gln Leu Asp Val Thr Asp Glu Glu
    50                  55                  60

Ser Ile Lys Ala Ala Val Ala Gln Ala Val Glu Glu Leu Gly Arg Leu
65                  70                  75                  80

Asp Val Leu Val Asn Asn Ala Gly Ile Leu Gly Pro Gly Glu Pro Phe
                85                  90                  95

Glu Leu Ser Glu Asp Asp Trp Glu Arg Val Ile Asp Val Asn Leu Thr
            100                 105                 110

Gly Val Phe Leu Leu Thr Gln Ala Val Leu Pro His Met Leu Lys Arg
        115                 120                 125

Ser Gly Gly Arg Ile Val Asn Ile Ser Ser Val Ala Gly Leu Val Pro
    130                 135                 140

Ser Pro Gly Leu Ser Ala Tyr Ser Ala Ser Lys Ala Ala Val Val Gly
145                 150                 155                 160

Phe Thr Arg Ser Leu Ala Leu Glu Leu Ala Pro His Gly Ile Arg Val
                165                 170                 175

Asn Ala Ile Ala Pro Gly Gly Val Asp Thr Asp Met Thr Lys Ala Leu
            180                 185                 190

Arg Ser Ile Ala Pro Ala Asp Pro Glu Leu Val Glu Arg Ile Thr Ser
        195                 200                 205

Ala Leu Val Pro Leu Gly Arg Tyr Gly Thr Pro Glu Glu Val Ala Asn
    210                 215                 220

Ala Val Leu Phe Leu Ala Ser Asp Gly Ala Ser Tyr Ser Val Thr Gly
225                 230                 235                 240

Gln Thr Leu Asn Val Asp Gly Gly Leu
                245


<210> 101
<211> 399
<212> PRT
<213> Homo sapiens

<400> 101
Met Gly Arg Lys Ser Leu Tyr Leu Leu Ile Val Gly Ile Leu Ile Ala
1               5                   10                  15

Tyr Tyr Ile Tyr Thr Pro Leu Pro Asp Asn Val Glu Glu Pro Trp Arg
            20                  25                  30

Met Met Trp Ile Asn Ala His Leu Lys Thr Ile Gln Asn Leu Ala Thr
        35                  40                  45

Phe Val Glu Leu Leu Gly Leu His His Phe Met Asp Ser Phe Lys Val

50                  55                  60

Val Gly Ser Phe Asp Glu Val Pro Pro Thr Ser Asp Glu Asn Val Thr
65                  70                  75                  80

Val Thr Glu Thr Lys Phe Asn Asn Ile Leu Val Arg Val Tyr Val Pro
                85                  90                  95

Lys Arg Lys Ser Glu Ala Leu Arg Arg Gly Leu Phe Tyr Ile His Gly
            100                 105                 110

Gly Gly Trp Cys Val Gly Ser Ala Ala Leu Ser Gly Tyr Asp Leu Leu
        115                 120                 125

Ser Arg Trp Thr Ala Asp Arg Leu Asp Ala Val Val Val Ser Thr Asn
    130                 135                 140

Tyr Arg Leu Ala Pro Lys Tyr His Phe Pro Ile Gln Phe Glu Asp Val
145                 150                 155                 160

Tyr Asn Ala Leu Arg Trp Phe Leu Arg Lys Lys Val Leu Ala Lys Tyr
                165                 170                 175

Gly Val Asn Pro Glu Arg Ile Gly Ile Ser Gly Asp Ser Ala Gly Gly
            180                 185                 190

Asn Leu Ala Ala Ala Val Thr Gln Gln Leu Leu Asp Asp Pro Asp Val
        195                 200                 205

Lys Ile Lys Leu Lys Ile Gln Ser Leu Ile Tyr Pro Ala Leu Gln Pro
    210                 215                 220

Leu Asp Val Asp Leu Pro Ser Tyr Gln Glu Asn Ser Asn Phe Leu Phe
225                 230                 235                 240

Leu Ser Lys Ser Leu Met Val Arg Phe Trp Ser Glu Tyr Phe Thr Thr
                245                 250                 255

Asp Arg Ser Leu Glu Lys Ala Met Leu Ser Arg Gln His Val Pro Val
            260                 265                 270

Glu Ser Ser His Leu Phe Lys Phe Val Asn Trp Ser Ser Leu Leu Pro
        275                 280                 285

Glu Arg Phe Ile Lys Gly His Val Tyr Asn Asn Pro Asn Tyr Gly Ser
    290                 295                 300

Ser Glu Leu Ala Lys Lys Tyr Pro Gly Phe Leu Asp Val Arg Ala Ala
305                 310                 315                 320

Pro Leu Leu Ala Asp Asp Asn Lys Leu Arg Gly Leu Pro Leu Thr Tyr
                325                 330                 335

Val Ile Thr Cys Gln Tyr Asp Leu Leu Arg Asp Asp Gly Leu Met Tyr
            340                 345                 350

Val Thr Arg Leu Arg Asn Thr Gly Val Gln Val Thr His Asn His Val
        355                 360                 365

Glu Asp Gly Phe His Gly Ala Phe Ser Phe Leu Gly Leu Lys Ile Ser
    370                 375                 380

His Arg Leu Ile Asn Gln Tyr Ile Glu Trp Leu Lys Glu Asn Leu
385                 390                 395

<210> 102
<211> 399
<212> PRT

<213> Homo sapiens

<400> 102
Met Gly Arg Lys Ser Leu Tyr Leu Leu Ile Val Gly Ile Leu Ile Ala
1               5                   10                  15

Tyr Tyr Ile Tyr Thr Pro Leu Pro Asp Asn Val Glu Glu Pro Trp Arg
            20                  25                  30

Met Met Trp Ile Asn Ala His Leu Lys Thr Ile Gln Asn Leu Ala Thr
        35                  40                  45

Phe Val Glu Leu His Gly Ser Ser Ile Phe Met Asp Ser Phe Lys Val
    50                  55                  60

Val Gly Ser Phe Asp Glu Val Pro Pro Thr Ser Asp Glu Asn Val Thr
65                  70                  75                  80

Val Thr Glu Thr Lys Phe Asn Asn Ile Leu Val Arg Val Tyr Val Pro
                85                  90                  95

Lys Arg Lys Ser Glu Ala Leu Arg Arg Gly Leu Phe Tyr Ile His Gly
            100                 105                 110

Gly Gly Trp Cys Val Gly Ser Ala Ala Leu Ser Gly Tyr Asp Leu Leu
        115                 120                 125

Ser Arg Trp Thr Ala Asp Arg Leu Asp Ala Val Val Val Ser Thr Asn
    130                 135                 140

Tyr Arg Leu Ala Pro Lys Tyr His Phe Pro Ile Gln Phe Glu Asp Val
145                 150                 155                 160

Tyr Asn Ala Leu Arg Trp Phe Leu Arg Lys Lys Val Leu Ala Lys Tyr
                165                 170                 175

Gly Val Asn Pro Glu Arg Ile Gly Ile Ser Gly Asp Ser Ala Gly Gly
            180                 185                 190

Asn Leu Ala Ala Ala Val Thr Gln Gln Leu Leu Asp Asp Pro Asp Val
        195                 200                 205

Lys Ile Lys Leu Lys Ile Gln Ser Leu Ile Tyr Pro Ala Leu Gln Pro
    210                 215                 220

Leu Asp Val Asp Leu Pro Ser Tyr Gln Glu Asn Ser Asn Phe Leu Phe
225                 230                 235                 240

Leu Ser Lys Ser Leu Met Val Arg Phe Trp Ser Glu Tyr Phe Thr Thr
                245                 250                 255

Asp Arg Ser Leu Glu Lys Ala Met Leu Ser Arg Gln His Val Pro Val
            260                 265                 270

Glu Ser Ser His Leu Phe Lys Phe Ile Asn Trp Ser Ser Leu Leu Pro
        275                 280                 285

Glu Arg Phe Ile Lys Gly His Val Tyr Asn Asn Pro Asn Tyr Gly Ser
    290                 295                 300

Ser Glu Leu Ala Lys Lys Tyr Pro Gly Phe Leu Asp Val Arg Ala Ala
305                 310                 315                 320

Pro Leu Leu Ala Asp Asp Asn Lys Leu Arg Gly Leu Pro Leu Thr Tyr
                325                 330                 335

Val Ile Thr Cys Gln Tyr Asp Leu Leu Arg Asp Asp Gly Leu Met Tyr
            340                 345                 350

Val Thr Arg Leu Arg Asn Thr Gly Val Gln Val Thr His Asn His Val
        355                 360                 365

Glu Asp Gly Phe His Gly Ala Phe Ser Phe Leu Gly Leu Lys Ile Ser
    370                 375                 380

His Arg Leu Ile Asn Gln Tyr Ile Glu Trp Leu Lys Glu Asn Leu
385                 390                 395

<210> 103
<211> 398
<212> PRT
<213> Orycctolagus cuniculus

<400> 103
Gly Val Lys Thr Val Leu Leu Leu Ile Val Gly Val Leu Gly Ala Tyr
1               5                   10                  15

Tyr Val Tyr Thr Pro Leu Pro Asp Asn Ile Glu Glu Pro Trp Arg Leu
            20                  25                  30

Leu Trp Val Asn Ala His Met Lys Thr Leu Thr Asn Leu Ala Leu Phe
        35                  40                  45

Ala Glu Tyr Leu Gly Ser Asn Ile Phe Met Asn Thr Val Lys Phe Leu
    50                  55                  60

Thr Ser Phe Gln Glu Val Pro Pro Thr Ser Asp Glu Asn Val Thr Val
65                  70                  75                  80

Thr Glu Thr Thr Phe Asn Asn Val Pro Val Arg Val Tyr Val Pro Lys
                85                  90                  95

Arg Lys Ser Lys Thr Leu Arg Arg Gly Leu Phe Tyr Ile His Gly Gly
            100                 105                 110

Gly Trp Cys Val Gly Ser Ala Ala Leu Ser Gly Tyr Asp Leu Leu Ser
        115                 120                 125

Arg Arg Thr Ala Asp Arg Leu Asp Val Val Val Val Ser Thr Asn Tyr
    130                 135                 140

Arg Leu Ala Pro Glu Tyr His Phe Pro Ile Gln Phe Glu Asp Val Tyr
145                 150                 155                 160

Asp Ala Leu Lys Trp Phe Leu Arg Gln Asp Val Leu Glu Lys Tyr Gly
                165                 170                 175

Val Asp Pro Glu Arg Val Gly Val Ser Gly Asp Ser Ala Gly Gly Asn
            180                 185                 190

Leu Ala Ala Ala Val Ala Gln Gln Leu Ile Lys Asp Pro Asp Val Lys
        195                 200                 205

Ile Lys Leu Lys Thr Gln Ser Leu Ile Tyr Pro Ala Leu Gln Thr Leu
    210                 215                 220

Asp Met Asp Leu Pro Ser Tyr Arg Glu Asn Ala Gln Phe Pro Ile Leu
225                 230                 235                 240

Ser Lys Ser Phe Met Val Arg Leu Trp Ser Glu Tyr Phe Thr Ser Asp
                245                 250                 255

Arg Ser Leu Glu Lys Ala Met Leu Leu Asn Gln His Val Pro Val Glu
            260                 265                 270

Ser Ser His Leu Phe Lys Phe Thr Asn Trp Ser Ser Leu Leu Pro Glu
        275                 280                 285

Lys Phe Lys Lys Gly His Val Tyr Asn Thr Pro Thr Tyr Gly Ser Ser
    290                 295                 300

Glu Leu Ala Arg Lys Tyr Pro Gly Phe Leu Asp Val Arg Ala Ala Pro
305                 310                 315                 320

Leu Leu Ala Asp Asp Ala Gln Leu Arg Gly Phe Pro Leu Thr Tyr Val
                325                 330                 335

Ile Thr Cys Gln Tyr Asp Val Leu Arg Asp Asp Gly Val Met Tyr Val
            340                 345                 350

Thr Arg Leu Arg Asn Ala Gly Val Gln Val Thr His Asn His Ile Glu
        355                 360                 365

Asp Gly Phe His Gly Ala Leu Ser Tyr Asn Gly Phe Lys Thr Gly Tyr
    370                 375                 380

Arg Val Glu Lys Gln Tyr Phe Glu Trp Leu Arg Glu Asn Val
385                 390                 395

<210> 104
<211> 398
<212> PRT
<213> Mus musculus

<400> 104
Met Gly Lys Thr Ile Ser Leu Leu Ile Ser Val Val Leu Val Ala Tyr
1               5                   10                  15

Tyr Leu Tyr Ile Pro Leu Pro Asp Ala Ile Glu Glu Pro Trp Lys Val
            20                  25                  30

Val Trp Glu Thr Ala Phe Val Lys Ile Gly Thr Asp Leu Ala Ser Phe
        35                  40                  45

Gly Glu Leu Leu Gly Ile Ser His Phe Met Glu Thr Ile Gln Leu Leu
    50                  55                  60

Met Ser Phe Gln Glu Val Pro Pro Thr Ser Asp Glu His Val Thr Val
65                  70                  75                  80

Met Glu Thr Ala Phe Asp Ser Val Pro Val Arg Ile Tyr Ile Pro Lys
                85                  90                  95

Arg Lys Ser Met Thr Leu Arg Arg Gly Leu Phe Tyr Ile His Gly Gly
            100                 105                 110

Gly Trp Cys Leu Gly Ser Ala Ala His Phe Ser Tyr Asp Thr Leu Ser
        115                 120                 125

Arg Trp Thr Ala His Lys Leu Asp Ala Val Val Val Ser Thr Asp Tyr
    130                 135                 140

Gly Leu Ala Pro Lys His His Phe Pro Arg Gln Phe Glu Asp Val Tyr
145                 150                 155                 160

Arg Ser Leu Arg Trp Phe Leu Gln Glu Asp Val Leu Glu Lys Tyr Gly
                165                 170                 175

Val Asp Pro Arg Arg Val Gly Val Ser Gly Asp Ser Ala Gly Gly Asn
            180                 185                 190

Leu Ala Ala Ala Val Thr Gln Gln Leu Ile Gln Asp Pro Asp Val Lys
        195                 200                 205

Ile Lys Leu Lys Val Gln Ala Leu Ile Tyr Pro Ala Leu Gln Ala Leu

210                 215                 220

Asp Thr Asn Val Pro Ser Gln Gln Glu Gly Ser His Phe Pro Val Leu
225                 230                 235                 240

Thr Arg Ser Leu Met Val Arg Phe Trp Ser Glu Tyr Phe Thr Thr Asp
                245                 250                 255

Arg Gly Leu Glu Lys Ala Met Leu Leu Asn Gln His Val Pro Met Glu
            260                 265                 270

Ser Ser His Leu Leu Gln Phe Val Asn Trp Ser Ser Leu Leu Pro Glu
        275                 280                 285

Arg Tyr Lys Lys Ser Pro Val Tyr Lys Asn Pro Thr Pro Gly Ser Ser
    290                 295                 300

Glu Leu Ala Gln Lys Tyr Pro Gly Phe Ile Asp Val Lys Ala Cys Pro
305                 310                 315                 320

Leu Leu Ala Asn Asp Asn Ile Leu His His Leu Pro Lys Thr Tyr Ile
                325                 330                 335

Ile Thr Cys Gln Tyr Asp Val Leu Arg Asp Asp Gly Leu Met Tyr Val
            340                 345                 350

Lys Arg Leu Gln Asn Val Gly Val His Val Thr His His His Val Glu
        355                 360                 365

Asp Gly Phe His Gly Thr Phe Ser Phe Pro Gly Leu Lys Leu Ser Glu
    370                 375                 380

Arg Met Lys Asn Gln Tyr Leu Ser Trp Leu Ile Lys Asn Leu
385                 390                 395

<210> 105
<211> 398
<212> PRT
<213> Mus musculus

<400> 105
Met Gly Lys Thr Ile Ser Leu Leu Ile Ser Val Val Leu Val Ala Tyr
1               5                   10                  15

Tyr Leu Tyr Ile Pro Leu Pro Asp Ala Ile Glu Glu Pro Trp Lys Val
            20                  25                  30

Val Trp Glu Thr Ala Phe Val Lys Ile Gly Thr Asp Leu Ala Ser Phe
        35                  40                  45

Gly Glu Leu Leu Gly Ile Ser His Phe Met Glu Thr Ile Gln Leu Leu
    50                  55                  60

Met Ser Phe Gln Glu Val Pro Pro Thr Ser Asp Glu His Val Thr Val
65                  70                  75                  80

Met Glu Thr Ala Phe Asp Ser Val Pro Val Arg Ile Tyr Ile Pro Lys
                85                  90                  95

Arg Lys Ser Met Ala Leu Arg Arg Gly Leu Phe Tyr Ile His Gly Gly
            100                 105                 110

Gly Trp Cys Leu Gly Ser Ala Ala His Phe Ser Tyr Asp Thr Leu Ser
        115                 120                 125

Arg Trp Thr Ala His Lys Leu Asp Ala Val Val Val Ser Thr Asp Tyr
    130                 135                 140

Gly Leu Ala Pro Lys His His Phe Pro Arg Gln Phe Glu Asp Val Tyr
145                 150                 155                 160

Arg Ser Leu Arg Trp Phe Leu Gln Glu Asp Val Leu Glu Lys Tyr Gly
                165                 170                 175

Val Asp Pro Arg Arg Val Gly Val Ser Gly Asp Ser Ala Gly Gly Asn
            180                 185                 190

Leu Ala Ala Ala Val Thr Gln Gln Leu Ile Gln Asp Pro Asp Val Lys
        195                 200                 205

Ile Lys Leu Lys Val Gln Ala Leu Ile Tyr Pro Ala Leu Gln Ala Leu
    210                 215                 220

Asp Thr Asn Val Pro Ser Gln Gln Glu Gly Ser His Phe Pro Val Leu
225                 230                 235                 240

Thr Arg Ser Leu Met Val Arg Phe Trp Ser Glu Tyr Phe Thr Thr Asp
                245                 250                 255

Arg Gly Leu Glu Lys Ala Met Leu Leu Asn Gln His Val Pro Met Glu
            260                 265                 270

Ser Ser His Leu Leu Gln Phe Val Asn Trp Ser Ser Leu Leu Pro Glu
        275                 280                 285

Arg Tyr Lys Lys Ser Pro Val Tyr Lys Asn Pro Thr Pro Gly Ser Ser
    290                 295                 300

Glu Leu Ala Gln Lys Tyr Pro Gly Phe Ile Asp Val Lys Ala Cys Pro
305                 310                 315                 320

Leu Leu Ala Asn Asp Asn Ile Leu His His Leu Pro Lys Thr Tyr Ile
                325                 330                 335

Ile Thr Cys Gln Tyr Asp Val Leu Arg Asp Asp Gly Leu Met Tyr Val
            340                 345                 350

Lys Arg Leu Gln Asn Val Gly Val His Val Thr His His His Val Glu
        355                 360                 365

Asp Gly Phe His Gly Thr Phe Ser Phe Pro Gly Leu Lys Leu Ser Glu
    370                 375                 380

Arg Met Lys Asn Gln Tyr Leu Ser Trp Leu Ile Lys Asn Leu
385                 390                 395

<210> 106
<211> 1019
<212> PRT
<213> Homo sapiens

<400> 106
Met Arg Tyr Arg Leu Ala Trp Leu Leu His Pro Ala Leu Pro Ser Thr
1               5                   10                  15

Phe Arg Ser Val Leu Gly Ala Arg Leu Pro Pro Pro Glu Arg Leu Cys
            20                  25                  30

Gly Phe Gln Lys Lys Thr Tyr Ser Lys Met Asn Asn Pro Ala Ile Lys
        35                  40                  45

Arg Ile Gly Asn His Ile Thr Lys Ser Pro Glu Asp Lys Arg Glu Tyr
    50                  55                  60

Arg Gly Leu Glu Leu Ala Asn Gly Ile Lys Val Leu Leu Met Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Ala Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Phe Asp Glu
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Gln Glu
225                 230                 235                 240

Leu Leu Lys Phe His Ser Ala Tyr Tyr Ser Ser Asn Leu Met Ala Val
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Lys Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Lys Tyr Tyr Lys Ser Asn Pro Gly His Tyr Leu Gly His
                325                 330                 335

Leu Ile Gly His Glu Gly Pro Gly Ser Leu Leu Ser Glu Leu Lys Ser
            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Lys
385                 390                 395                 400

Leu Arg Ala Glu Gly Pro Gln Glu Trp Val Phe Gln Glu Cys Lys Asp
                405                 410                 415

Leu Asn Ala Val Ala Phe Arg Phe Lys Asp Lys Glu Arg Pro Arg Gly
            420                 425                 430

Tyr Thr Ser Lys Ile Ala Gly Ile Leu His Tyr Tyr Pro Leu Glu Glu
        435                 440                 445

Val Leu Thr Ala Glu Tyr Leu Leu Glu Glu Phe Arg Pro Asp Leu Ile
    450                 455                 460

Glu Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Glu Trp Tyr
                485                 490                 495

Gly Thr Gln Tyr Lys Gln Glu Ala Ile Pro Asp Glu Val Ile Lys Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Pro Leu Glu Lys Glu Ala
    530                 535                 540

Thr Pro Tyr Pro Ala Leu Ile Lys Asp Thr Val Met Ser Lys Leu Trp
545                 550                 555                 560

Phe Lys Gln Asp Asp Lys Lys Lys Lys Pro Lys Ala Cys Leu Asn Phe
                565                 570                 575

Glu Phe Phe Ser Pro Phe Ala Tyr Val Asp Pro Leu His Cys Asn Met
            580                 585                 590

Ala Tyr Leu Tyr Leu Glu Leu Leu Lys Asp Ser Leu Asn Glu Tyr Ala
        595                 600                 605

Tyr Ala Ala Glu Leu Ala Gly Leu Ser Tyr Asp Leu Gln Asn Thr Ile
    610                 615                 620

Tyr Gly Met Tyr Leu Ser Val Lys Gly Tyr Asn Asp Lys Gln Pro Ile
625                 630                 635                 640

Leu Leu Lys Lys Ile Ile Glu Lys Met Ala Thr Phe Glu Ile Asp Glu
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu
705                 710                 715                 720

Ser Arg Leu His Ile Glu Ala Leu Leu His Gly Asn Ile Thr Lys Gln
                725                 730                 735

Ala Ala Leu Gly Ile Met Gln Met Val Glu Asp Thr Leu Ile Glu His
            740                 745                 750

Ala His Thr Lys Pro Leu Leu Pro Ser Gln Leu Val Arg Tyr Arg Glu
        755                 760                 765

Val Gln Leu Pro Asp Arg Gly Trp Phe Val Tyr Gln Gln Arg Asn Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Ser Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ser Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Phe Asp Arg Asp Asn Thr Glu Val Ala Tyr Leu
        915                 920                 925

Lys Thr Leu Thr Lys Glu Asp Ile Ile Lys Phe Tyr Lys Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Cys Gln Asn
                965                 970                 975

Asp Ile Asn Leu Ser Gln Ala Pro Ala Leu Pro Gln Pro Glu Val Ile
            980                 985                 990

Gln Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015

<210> 107
<211> 1019
<212> PRT
<213> Rattus norvegicus

<400> 107
Met Arg Asn Gly Leu Val Trp Leu Leu His Pro Ala Leu Pro Ser Thr
1               5                   10                  15

Leu His Ser Ile Leu Gly Ala Arg Pro Pro Pro Val Lys Arg Leu Cys
            20                  25                  30

Gly Phe Pro Lys Gln Ile Tyr Ser Thr Met Asn Asn Pro Ala Ile Gln
        35                  40                  45

Arg Ile Glu Asp His Ile Val Lys Ser Pro Glu Asp Lys Arg Glu Tyr
    50                  55                  60

Arg Gly Leu Glu Leu Ala Asn Gly Ile Lys Val Leu Leu Ile Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Pro Gly Leu Ser His Phe Cys Glu His
            100                 105                 110

Met Leu Phe Leu Gly Thr Lys Lys Tyr Pro Lys Glu Asn Glu Tyr Ser

115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Phe Asp Ala
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Glu Glu
225                 230                 235                 240

Leu Leu Lys Phe His Ser Thr Tyr Tyr Ser Ser Asn Leu Met Ala Ile
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Lys Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Gln Tyr Tyr Lys Ser Asn Pro Gly His Tyr Leu Gly His
                325                 330                 335

Leu Ile Gly His Glu Gly Pro Gly Ser Leu Leu Ser Glu Leu Lys Ser
            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Lys
385                 390                 395                 400

Leu Arg Ala Glu Gly Pro Gln Glu Trp Val Phe Gln Glu Cys Lys Asp
                405                 410                 415

Leu Asn Ala Val Ala Phe Arg Phe Lys Asp Lys Glu Arg Pro Arg Gly
            420                 425                 430

Tyr Thr Ser Lys Ile Ala Gly Lys Leu His Tyr Tyr Pro Leu Asn Gly
        435                 440                 445

Val Leu Thr Ala Glu Tyr Leu Leu Glu Glu Phe Arg Pro Asp Leu Ile
    450                 455                 460

Asp Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Gln Trp Tyr

                485                 490                 495

Gly Thr Gln Tyr Lys Gln Glu Ala Ile Pro Glu Asp Val Ile Gln Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Ala Leu Glu Lys Asp Ala
    530                 535                 540

Thr Pro Tyr Pro Ala Leu Ile Lys Asp Thr Ala Met Ser Lys Leu Trp
545                 550                 555                 560

Phe Lys Gln Asp Asp Lys Phe Phe Leu Pro Lys Ala Cys Leu Asn Phe
                565                 570                 575

Glu Phe Phe Ser Pro Phe Ala Tyr Val Asp Pro Leu His Cys Asn Met
            580                 585                 590

Ala Tyr Leu Tyr Leu Glu Leu Leu Lys Asp Ser Leu Asn Glu Tyr Ala
        595                 600                 605

Tyr Ala Ala Glu Leu Ala Gly Leu Ser Tyr Asp Leu Gln Asn Thr Ile
    610                 615                 620

Tyr Gly Met Tyr Leu Ser Val Lys Gly Tyr Asn Asp Lys Gln Pro Ile
625                 630                 635                 640

Leu Leu Lys Lys Ile Thr Glu Lys Met Ala Thr Phe Glu Ile Asp Lys
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu
705                 710                 715                 720

Ser Arg Leu His Ile Glu Ala Leu Leu His Gly Asn Ile Thr Lys Gln
                725                 730                 735

Ala Ala Leu Gly Val Met Gln Met Val Glu Asp Thr Leu Ile Glu His
            740                 745                 750

Ala His Thr Lys Pro Leu Leu Pro Ser Gln Leu Val Arg Tyr Arg Glu
        755                 760                 765

Val Gln Leu Pro Asp Arg Gly Trp Phe Val Tyr Gln Arg Arg Asn Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Gly Leu Arg Phe
        835                 840                 845

Ile Ile Gln Ser Glu Lys Pro Pro His Tyr Leu Glu Ser Arg Val Glu

850                 855                 860

Ala Phe Leu Ile Thr Met Glu Lys Ala Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Tyr Asp Arg Asp Asn Ile Glu Val Ala Tyr Leu
        915                 920                 925

Lys Thr Leu Ser Lys Asp Asp Ile Ile Lys Phe Tyr Lys Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Ser Gln Asn
                965                 970                 975

Asp Ile Asn Leu Ser Glu Ala Pro Pro Leu Pro Gln Pro Glu Val Ile
            980                 985                 990

His Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015

<210> 108
<211> 1019
<212> PRT
<213> Mus musculus

<400> 108
Met Arg Asn Gly Leu Val Trp Leu Leu His Pro Ala Leu Pro Gly Thr
1               5                   10                  15

Leu Arg Ser Ile Leu Gly Ala Arg Pro Pro Pro Ala Lys Arg Leu Cys
            20                  25                  30

Gly Phe Pro Lys Gln Thr Tyr Ser Thr Met Ser Asn Pro Ala Ile Gln
        35                  40                  45

Arg Ile Glu Asp Gln Ile Val Lys Ser Pro Glu Asp Lys Arg Glu Tyr
    50                  55                  60

Arg Gly Leu Glu Leu Ala Asn Gly Ile Lys Val Leu Leu Ile Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Pro Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Leu Asp Ala
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Glu Glu
225                 230                 235                 240

Leu Leu Lys Phe His Ser Thr Tyr Tyr Ser Ser Asn Leu Met Ala Ile
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Arg Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Gln Tyr Tyr Lys Ser Asn Pro Gly Tyr Tyr Leu Gly His
                325                 330                 335

Leu Ile Gly His Glu Gly Pro Gly Ser Leu Leu Ser Glu Leu Lys Ser
            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Lys
385                 390                 395                 400

Leu Arg Ala Glu Gly Pro Gln Glu Trp Val Phe Gln Glu Cys Lys Asp
                405                 410                 415

Leu Asn Ala Val Ala Phe Arg Phe Lys Asp Lys Glu Arg Pro Arg Gly
            420                 425                 430

Tyr Thr Ser Lys Ile Ala Gly Lys Leu His Tyr Tyr Pro Leu Asn Gly
        435                 440                 445

Val Leu Thr Ala Glu Tyr Leu Leu Glu Glu Phe Arg Pro Asp Leu Ile
    450                 455                 460

Asp Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Gln Trp Tyr
                485                 490                 495

Gly Thr Gln Tyr Lys Gln Glu Ala Ile Pro Glu Asp Val Ile Gln Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Ser Leu Glu Lys Asp Ala
    530                 535                 540

Thr Pro Tyr Pro Ala Leu Ile Lys Asp Thr Ala Met Ser Lys Leu Trp
545                 550                 555                 560

Phe Lys Gln Asp Asp Lys Phe Phe Leu Pro Lys Ala Cys Leu Asn Phe
                565                 570                 575

Glu Phe Phe Ser Pro Phe Ala Tyr Val Asp Pro Leu His Cys Asn Met
            580                 585                 590

Ala Tyr Leu Tyr Leu Glu Leu Leu Lys Asp Ser Leu Asn Glu Tyr Ala
        595                 600                 605

Tyr Ala Ala Glu Leu Ala Gly Leu Ser Tyr Asp Leu Gln Asn Thr Ile
    610                 615                 620

Tyr Gly Met Tyr Leu Ser Val Lys Arg Tyr Asn Asp Lys Gln Pro Ile
625                 630                 635                 640

Leu Leu Lys Lys Ile Thr Glu Lys Met Ala Thr Phe Glu Ile Asp Lys
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu
705                 710                 715                 720

Ser Arg Leu His Ile Glu Ala Leu Leu His Gly Asn Ile Thr Lys Gln
                725                 730                 735

Ala Ala Leu Gly Val Met Gln Met Val Glu Asp Thr Leu Ile Glu His
            740                 745                 750

Ala His Thr Lys Pro Leu Leu Pro Ser Gln Leu Val Arg Tyr Arg Glu
        755                 760                 765

Val Gln Leu Pro Asp Arg Gly Trp Phe Val Tyr Gln Gln Arg Asn Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Gly Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ala Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Tyr Asp Arg Asp Asn Ile Glu Val Ala Tyr Leu
        915                 920                 925

Lys Thr Leu Thr Lys Asp Asp Ile Ile Arg Phe Tyr Gln Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Ser Gln Asn
                965                 970                 975

Asp Ile Asn Leu Ser Glu Ala Pro Pro Leu Pro Gln Pro Glu Val Ile
            980                 985                 990

His Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015


<210> 109
<211> 554
<212> PRT
<213> Homo sapiens

<400> 109
Met Val Leu Asp Lys Leu Arg Pro Glu Asn Val Arg Val Ala Ile Val
1               5                   10                  15

Ser Lys Ser Phe Glu Gly Lys Thr Asp Arg Thr Glu Glu Trp Tyr Gly
            20                  25                  30

Thr Gln Tyr Lys Gln Glu Ala Ile Pro Asp Glu Val Ile Lys Lys Trp
        35                  40                  45

Gln Asn Ala Asp Leu Asn Gly Lys Phe Lys Leu Pro Thr Lys Asn Glu
    50                  55                  60

Phe Ile Pro Thr Asn Phe Glu Ile Leu Pro Leu Glu Lys Glu Ala Thr
65                  70                  75                  80

Pro Tyr Pro Ala Leu Ile Lys Asp Thr Ala Met Ser Lys Leu Trp Phe
                85                  90                  95

Lys Gln Asp Asp Lys Phe Phe Leu Pro Lys Ala Cys Leu Asn Phe Glu
            100                 105                 110

Phe Phe Ser Pro Phe Ala Tyr Val Asp Pro Leu His Cys Asn Met Ala
        115                 120                 125

Tyr Leu Tyr Leu Glu Leu Leu Lys Asp Ser Leu Asn Glu Tyr Ala Tyr
    130                 135                 140

Ala Ala Glu Leu Ala Gly Leu Ser Tyr Asp Leu Gln Asn Thr Ile Tyr
145                 150                 155                 160

Gly Met Tyr Leu Ser Val Lys Gly Tyr Asn Asp Lys Gln Pro Ile Leu
                165                 170                 175

Leu Lys Lys Ile Ile Glu Lys Met Ala Thr Phe Glu Ile Asp Glu Lys
            180                 185                 190

Arg Phe Glu Ile Ile Lys Glu Ala Tyr Met Arg Ser Leu Asn Asn Phe
        195                 200                 205

Arg Ala Glu Gln Pro His Gln His Ala Met Tyr Tyr Leu Arg Leu Leu
    210                 215                 220

Met Thr Glu Val Ala Trp Thr Lys Asp Glu Leu Lys Glu Ala Leu Asp
225                 230                 235                 240

Asp Val Thr Leu Pro Arg Leu Lys Ala Phe Ile Pro Gln Leu Leu Ser
                245                 250                 255

Arg Leu His Ile Glu Ala Leu Leu His Gly Asn Ile Thr Lys Gln Ala
            260                 265                 270

Ala Leu Gly Ile Met Gln Met Val Glu Asp Thr Leu Ile Glu His Ala
        275                 280                 285

His Thr Lys Pro Leu Leu Pro Ser Gln Leu Val Arg Tyr Arg Glu Val
    290                 295                 300

Gln Leu Pro Asp Arg Gly Trp Phe Val Tyr Gln Gln Arg Asn Glu Val
305                 310                 315                 320

His Asn Asn Cys Gly Ile Glu Ile Tyr Tyr Gln Thr Asp Met Gln Ser
                325                 330                 335

Thr Ser Glu Asn Met Phe Leu Glu Leu Phe Cys Gln Ile Ile Ser Glu
            340                 345                 350

Pro Cys Phe Asn Thr Leu Arg Thr Lys Glu Gln Leu Gly Tyr Ile Val
        355                 360                 365

Phe Ser Gly Pro Arg Arg Ala Asn Gly Ile Gln Gly Leu Arg Phe Ile
    370                 375                 380

Ile Gln Ser Glu Lys Pro Pro His Tyr Leu Glu Ser Arg Val Glu Ala
385                 390                 395                 400

Phe Leu Ile Thr Met Glu Lys Ser Ile Glu Asp Met Thr Glu Glu Ala
                405                 410                 415

Phe Gln Lys His Ile Gln Ala Leu Ala Ile Arg Arg Leu Asp Lys Pro
            420                 425                 430

Lys Lys Leu Ser Ala Glu Cys Ala Lys Tyr Trp Gly Glu Ile Ile Ser
        435                 440                 445

Gln Gln Tyr Asn Phe Asp Arg Asp Asn Thr Glu Val Ala Tyr Leu Lys
    450                 455                 460

Thr Leu Thr Lys Glu Asp Ile Ile Lys Phe Tyr Lys Glu Met Leu Ala
465                 470                 475                 480

Val Asp Ala Pro Arg Arg His Lys Val Ser Val His Val Leu Ala Arg
                485                 490                 495

Glu Met Asp Ser Cys Pro Val Val Gly Glu Phe Pro Cys Gln Asn Asp
            500                 505                 510

Ile Asn Leu Ser Gln Ala Pro Ala Leu Pro Gln Pro Glu Val Ile Gln
        515                 520                 525

Asn Met Thr Glu Phe Lys Arg Gly Leu Pro Leu Phe Pro Leu Val Lys
    530                 535                 540

Pro His Ile Asn Phe Met Ala Ala Lys Leu
545                 550

<210> 110

<211> 990
<212> PRT
<213> Drosophila melanogaster

<400> 110
Met Thr Ile Ala Glu Ser Ser Gln Lys Ser Ala Thr Arg Lys Pro Asp
1               5                   10                  15

Ser Met Glu Pro Ile Leu Arg Leu Asn Asn Ile Glu Lys Ser Leu Gln
            20                  25                  30

Asp Thr Arg Asp Tyr Arg Gly Leu Gln Leu Glu Asn Gly Leu Lys Val
        35                  40                  45

Leu Leu Ile Ser Asp Pro Asn Thr Asp Val Ser Ala Ala Ala Leu Ser
    50                  55                  60

Val Gln Val Gly His Met Ser Asp Pro Thr Asn Leu Pro Gly Leu Ala
65                  70                  75                  80

His Phe Cys Glu His Met Leu Phe Leu Gly Thr Glu Lys Tyr Pro His
                85                  90                  95

Glu Asn Gly Tyr Thr Thr Tyr Leu Ser Gln Ser Gly Gly Ser Ser Asn
            100                 105                 110

Ala Ala Thr Tyr Pro Leu Met Thr Lys Tyr His Phe His Val Ala Pro
        115                 120                 125

Asp Lys Leu Asp Gly Ala Leu Asp Arg Phe Ala Gln Phe Phe Ile Ala
    130                 135                 140

Pro Leu Phe Thr Pro Ser Ala Thr Glu Arg Glu Ile Asn Ala Val Asn
145                 150                 155                 160

Ser Glu His Glu Lys Asn Leu Pro Ser Asp Leu Trp Arg Ile Lys Gln
                165                 170                 175

Val Asn Arg His Leu Ala Lys Pro Asp His Ala Tyr Ser Lys Phe Gly
            180                 185                 190

Ser Gly Asn Lys Thr Thr Leu Ser Glu Ile Pro Lys Ser Lys Asn Ile
        195                 200                 205

Asp Val Arg Asp Glu Leu Leu Lys Phe His Lys Gln Trp Tyr Ser Ala
    210                 215                 220

Asn Ile Met Cys Leu Ala Val Ile Gly Lys Glu Ser Leu Asp Glu Leu
225                 230                 235                 240

Glu Gly Met Val Leu Glu Lys Phe Ser Glu Ile Glu Asn Lys Asn Val
                245                 250                 255

Lys Val Pro Gly Trp Pro Arg His Pro Tyr Ala Glu Glu Arg Tyr Gly
            260                 265                 270

Gln Lys Val Lys Ile Val Pro Ile Lys Asp Ile Arg Ser Leu Thr Ile
        275                 280                 285

Ser Phe Thr Thr Asp Asp Leu Thr Gln Phe Tyr Lys Ser Gly Pro Asp
    290                 295                 300

Asn Tyr Leu Thr His Leu Ile Gly His Glu Gly Lys Gly Ser Ile Leu
305                 310                 315                 320

Ser Glu Leu Arg Arg Leu Gly Trp Cys Asn Asp Leu Met Ala Gly His
                325                 330                 335

Gln Asn Thr Gln Asn Gly Phe Gly Phe Phe Asp Ile Val Val Asp Leu

340                 345                 350

Thr Gln Glu Gly Leu Glu His Val Asp Asp Ile Val Lys Ile Val Phe
        355                 360                 365

Gln Tyr Leu Glu Met Leu Arg Lys Glu Gly Pro Lys Lys Trp Ile Phe
    370                 375                 380

Asp Glu Cys Val Lys Leu Asn Glu Met Arg Phe Arg Phe Lys Glu Lys
385                 390                 395                 400

Glu Gln Pro Glu Asn Leu Val Thr His Ala Val Ser Ser Met Gln Ile
                405                 410                 415

Phe Pro Leu Glu Glu Val Leu Ile Ala Pro Tyr Leu Ser Asn Glu Trp
            420                 425                 430

Arg Pro Asp Leu Ile Lys Gly Leu Leu Asp Glu Leu Val Pro Ser Lys
        435                 440                 445

Ser Arg Ile Val Ile Val Ser Gln Ser Phe Glu Pro Asp Cys Asp Leu
    450                 455                 460

Ala Glu Pro Tyr Tyr Lys Thr Lys Tyr Gly Ile Thr Arg Val Ala Lys
465                 470                 475                 480

Asp Thr Val Gln Ser Trp Glu Asn Cys Glu Leu Asn Glu Asn Leu Lys
                485                 490                 495

Leu Ala Leu Pro Asn Ser Phe Ile Pro Thr Asn Phe Asp Ile Ser Asp
            500                 505                 510

Val Pro Ala Asp Ala Pro Lys His Pro Thr Ile Ile Leu Asp Thr Pro
        515                 520                 525

Ile Leu Arg Val Trp His Lys Gln Asp Asn Gln Phe Asn Lys Pro Lys
    530                 535                 540

Ala Cys Met Thr Phe Asp Met Ser Asn Pro Ile Ala Tyr Leu Asp Pro
545                 550                 555                 560

Leu Asn Cys Asn Leu Asn His Met Met Val Met Leu Leu Lys Asp Gln
                565                 570                 575

Leu Asn Glu Tyr Leu Tyr Asp Ala Glu Leu Ala Ser Leu Lys Leu Ser
            580                 585                 590

Val Met Gly Lys Ser Cys Gly Ile Asp Phe Thr Ile Arg Gly Phe Ser
        595                 600                 605

Asp Lys Gln Val Val Leu Leu Glu Lys Leu Leu Asp His Leu Phe Asp
    610                 615                 620

Phe Ser Ile Asp Glu Lys Arg Phe Asp Ile Leu Lys Glu Glu Tyr Val
625                 630                 635                 640

Arg Ser Leu Lys Asn Phe Lys Ala Glu Gln Pro Tyr Gln His Ser Ile
                645                 650                 655

Tyr Tyr Leu Ala Leu Leu Leu Thr Glu Asn Ala Trp Ala Asn Met Glu
            660                 665                 670

Leu Leu Asp Ala Met Glu Leu Val Thr Tyr Asp Arg Val Leu Asn Phe
        675                 680                 685

Ala Lys Glu Phe Phe Gln Arg Leu His Thr Glu Cys Phe Ile Phe Gly
    690                 695                 700

Asn Val Thr Lys Gln Gln Ala Thr Asp Ile Ala Gly Arg Val Asn Thr

705                 710                 715                 720

Arg Leu Glu Ala Thr Asn Ala Ser Lys Leu Pro Ile Leu Ala Arg Gln
                725                 730                 735

Met Leu Lys Lys Arg Glu Tyr Lys Leu Leu Ala Gly Asp Ser Tyr Leu
            740                 745                 750

Phe Glu Lys Glu Asn Glu Phe His Lys Ser Ser Cys Ala Gln Leu Tyr
        755                 760                 765

Leu Gln Cys Gly Ala Gln Thr Asp His Thr Asn Ile Met Val Asn Leu
    770                 775                 780

Val Ser Gln Val Leu Ser Glu Pro Cys Tyr Asp Cys Leu Arg Thr Lys
785                 790                 795                 800

Glu Gln Leu Gly Tyr Ile Val Phe Ser Gly Val Arg Lys Val Asn Gly
                805                 810                 815

Ala Asn Gly Ile Arg Ile Ile Val Gln Ser Ala Lys His Pro Ser Tyr
            820                 825                 830

Val Glu Asp Arg Ile Glu Asn Phe Leu Gln Thr Tyr Leu Gln Val Ile
        835                 840                 845

Glu Asp Met Pro Leu Asp Glu Phe Glu Arg His Lys Glu Ala Leu Ala
    850                 855                 860

Val Lys Lys Leu Glu Lys Pro Lys Thr Ile Phe Gln Gln Phe Ser Gln
865                 870                 875                 880

Phe Tyr Gly Glu Ile Ala Met Gln Thr Tyr His Phe Glu Arg Glu Glu
                885                 890                 895

Ala Glu Val Ala Ile Leu Arg Lys Ile Ser Lys Ala Asp Phe Val Asp
            900                 905                 910

Tyr Phe Lys Lys Phe Ile Ala Lys Asp Gly Glu Glu Arg Arg Val Leu
        915                 920                 925

Ser Val His Ile Val Ser Gln Gln Thr Asp Glu Asn Ala Thr Ser Glu
    930                 935                 940

Ala Glu Pro Val Glu Ile Thr Asn Met Glu Arg His Lys Pro Ile Ser
945                 950                 955                 960

Asp Ile Val Thr Phe Lys Ser Cys Lys Glu Leu Tyr Pro Ile Ala Leu
                965                 970                 975

Pro Phe Leu Asp Ile Lys Ala Lys Gly Ala Arg Ser Lys Leu
            980                 985                 990


<210> 111
<211> 149
<212> PRT
<213> Homo sapiens

<400> 111
Arg Val Ala Leu Glu His Asp Pro Pro Ala Asp Thr Ser Ala Val Gly
1               5                   10                  15

Leu Trp Val Asp Ala Gly Ser Arg Tyr Glu Pro Asp Asp Asn Asn Gly
            20                  25                  30

Leu Ala His Phe Leu Glu His Met Ala Phe Lys Gly Thr Lys Lys Tyr
        35                  40                  45

Pro Ser Asn Glu Leu Glu Glu Glu Leu Glu Lys Leu Gly Gly Ser Leu
    50                  55                  60

Asn Ala Tyr Thr Ser Arg Glu His Thr Ala Tyr Tyr Val Glu Val Leu
65                  70                  75                  80

Asn Asp Asp Leu Pro Lys Ala Val Asp Arg Leu Ala Asp Phe Phe Leu
                85                  90                  95

Asn Pro Leu Phe Ser Pro Ser Glu Val Glu Arg Glu Arg Leu Val Val
            100                 105                 110

Leu Tyr Glu Val Glu Ala Val Asp Ala Glu Pro Gln Ala Val Leu Leu
        115                 120                 125

Asp Asn Leu His Ala Ala Ala Tyr Arg Gly Thr Pro Leu Gly Arg Ser
    130                 135                 140

Leu Leu Gly Pro Gly
145

<210> 112
<211> 798
<212> PRT
<213> Homo sapiens

<400> 112
Met Val Ala Leu Pro Met Val Leu Val Leu Leu Leu Val Leu Ser Arg
1               5                   10                  15

Gly Glu Ser Glu Leu Asp Ala Lys Ile Pro Ser Thr Gly Asp Ala Thr
            20                  25                  30

Glu Trp Arg Asn Pro His Leu Ser Met Leu Gly Ser Cys Gln Pro Ala
        35                  40                  45

Pro Ser Cys Gln Lys Cys Ile Leu Ser His Pro Ser Cys Ala Trp Cys
    50                  55                  60

Lys Gln Leu Asn Phe Thr Ala Ser Gly Glu Ala Glu Ala Arg Arg Cys
65                  70                  75                  80

Ala Arg Arg Glu Glu Leu Leu Ala Arg Gly Cys Pro Leu Glu Glu Leu
                85                  90                  95

Glu Glu Pro Arg Gly Gln Gln Glu Val Leu Gln Asp Gln Pro Leu Ser
            100                 105                 110

Gln Gly Ala Arg Gly Glu Gly Ala Thr Gln Leu Ala Pro Gln Arg Val
        115                 120                 125

Arg Val Thr Leu Arg Pro Gly Glu Pro Gln Gln Leu Gln Val Arg Phe
    130                 135                 140

Leu Arg Ala Glu Gly Tyr Pro Val Asp Leu Tyr Tyr Leu Met Asp Leu
145                 150                 155                 160

Ser Tyr Ser Met Lys Asp Asp Leu Glu Arg Val Arg Gln Leu Gly His
                165                 170                 175

Ala Leu Leu Val Arg Leu Gln Glu Val Thr His Ser Val Arg Ile Gly
            180                 185                 190

Phe Gly Ser Phe Val Asp Lys Thr Val Leu Pro Phe Val Ser Thr Val
        195                 200                 205

Pro Ser Lys Leu Arg His Pro Cys Pro Thr Arg Leu Glu Arg Cys Gln
    210                 215                 220

Ser Pro Phe Ser Phe His His Val Leu Ser Leu Thr Gly Asp Ala Gln
225                 230                 235                 240

Ala Phe Glu Arg Glu Val Gly Arg Gln Ser Val Ser Gly Asn Leu Asp
                245                 250                 255

Ser Pro Glu Gly Gly Phe Asp Ala Ile Leu Gln Ala Ala Leu Cys Gln
            260                 265                 270

Glu Gln Ile Gly Trp Arg Asn Val Ser Arg Leu Leu Val Phe Thr Ser
        275                 280                 285

Asp Asp Thr Phe His Thr Ala Gly Asp Gly Lys Leu Gly Gly Ile Phe
    290                 295                 300

Met Pro Ser Asp Gly His Cys His Leu Asp Ser Asn Gly Leu Tyr Ser
305                 310                 315                 320

Arg Ser Thr Glu Phe Asp Tyr Pro Ser Val Gly Gln Val Ala Gln Ala
                325                 330                 335

Leu Ser Ala Ala Asn Ile Gln Pro Ile Phe Ala Val Thr Ser Ala Ala
            340                 345                 350

Leu Pro Val Tyr Gln Glu Leu Ser Lys Leu Ile Pro Lys Ser Ala Val
        355                 360                 365

Gly Glu Leu Ser Glu Asp Ser Ser Asn Val Val Gln Leu Ile Met Asp
    370                 375                 380

Ala Tyr Asn Ser Leu Ser Ser Thr Val Thr Leu Glu His Ser Ser Leu
385                 390                 395                 400

Pro Pro Gly Val His Ile Ser Tyr Glu Ser Gln Cys Glu Gly Pro Glu
                405                 410                 415

Lys Arg Glu Gly Lys Ala Glu Asp Arg Gly Gln Cys Asn His Val Arg
            420                 425                 430

Ile Asn Gln Thr Val Thr Phe Trp Val Ser Leu Gln Ala Thr His Cys
        435                 440                 445

Leu Pro Glu Pro His Leu Leu Arg Leu Arg Ala Leu Gly Phe Ser Glu
    450                 455                 460

Glu Leu Ile Val Glu Leu His Thr Leu Cys Asp Cys Asn Cys Ser Asp
465                 470                 475                 480

Thr Gln Pro Gln Ala Pro His Cys Ser Asp Gly Gln Gly His Leu Gln
                485                 490                 495

Cys Gly Val Cys Ser Cys Ala Pro Gly Arg Leu Gly Arg Leu Cys Glu
            500                 505                 510

Cys Ser Val Ala Glu Leu Ser Ser Pro Asp Leu Glu Ser Gly Cys Arg
        515                 520                 525

Ala Pro Asn Gly Thr Gly Pro Leu Cys Ser Gly Lys Gly His Cys Gln
    530                 535                 540

Cys Gly Arg Cys Ser Cys Ser Gly Gln Ser Ser Gly His Leu Cys Glu
545                 550                 555                 560

Cys Asp Asp Ala Ser Cys Glu Arg His Glu Gly Ile Leu Cys Gly Gly
                565                 570                 575

Phe Gly Arg Cys Gln Cys Gly Val Cys His Cys His Ala Asn Arg Thr
            580                 585                 590

Gly Arg Ala Cys Glu Cys Ser Gly Asp Met Asp Ser Cys Ile Ser Pro
        595                 600                 605

Glu Gly Gly Leu Cys Ser Gly His Gly Arg Cys Lys Cys Asn Arg Cys
    610                 615                 620

Gln Cys Leu Asp Gly Tyr Tyr Gly Ala Leu Cys Asp Gln Cys Pro Gly
625                 630                 635                 640

Cys Lys Thr Pro Cys Glu Arg His Arg Asp Cys Ala Glu Cys Gly Ala
                645                 650                 655

Phe Arg Thr Gly Pro Leu Ala Thr Asn Cys Ser Thr Ala Cys Ala His
            660                 665                 670

Thr Asn Val Thr Leu Ala Leu Ala Pro Ile Leu Asp Asp Gly Trp Cys
        675                 680                 685

Lys Glu Arg Thr Leu Asp Asn Gln Leu Phe Phe Phe Leu Val Glu Asp
    690                 695                 700

Asp Ala Arg Gly Thr Val Val Leu Arg Val Arg Pro Gln Glu Lys Gly
705                 710                 715                 720

Ala Asp His Thr Gln Ala Ile Val Leu Gly Cys Val Gly Gly Ile Val
                725                 730                 735

Ala Val Gly Leu Gly Leu Val Leu Ala Tyr Arg Leu Ser Val Glu Ile
            740                 745                 750

Tyr Asp Arg Arg Glu Tyr Ser Arg Phe Glu Lys Glu Gln Gln Gln Leu
        755                 760                 765

Asn Trp Lys Gln Asp Ser Asn Pro Leu Tyr Lys Ser Ala Ile Thr Thr
    770                 775                 780

Thr Ile Asn Pro Arg Phe Gln Glu Ala Asp Ser Pro Thr Leu
785                 790                 795


<210> 113
<211> 806
<212> PRT
<213> Mus musculus

<400> 113
Met Val Asp Ser Ser Thr Val Leu Ile Phe Leu Leu Val Leu Gly Gly
  1               5                  10                  15

Gly Gln Ser Glu Leu Asp Thr Lys Ile Thr Ser Ser Gly Glu Ala Ala
             20                  25                  30

Glu Trp Glu Asp Pro Asp Leu Ser Leu Gln Gly Ser Cys Gln Pro Val
         35                  40                  45

Pro Ser Cys Gln Lys Cys Ile Leu Ser His Pro Ser Cys Ala Trp Cys
     50                  55                  60

Lys Gln Leu Asn Phe Thr Ala Ser Gly Glu Ala Glu Ala Arg Arg Cys
 65                  70                  75                  80

Ala Arg Arg Glu Glu Leu Leu Ala Arg Gly Cys Pro Ala Gln Glu Leu
                 85                  90                  95

Glu Glu Pro Arg Gly Arg Gln Glu Val Leu Gln Asp Lys Pro Leu Ser
            100                 105                 110

Gln Gly Asp Arg Gly Glu Gly Ala Thr Gln Leu Ala Pro Gln Arg Ile

        115                 120                 125

Arg Val Thr Leu Arg Pro Gly Glu Pro Gln Lys Phe Arg Val Arg Phe
    130                 135                 140

Leu Arg Ala Ala Gly Tyr Pro Val Asp Leu Tyr Tyr Leu Met Asp Leu
145                 150                 155                 160

Ser Tyr Ser Met Lys Asp Asp Leu Glu Arg Val Arg Gln Leu Gly His
                165                 170                 175

Ala Leu Leu Val Arg Leu Gln Glu Val Thr His Ser Val Arg Ile Gly
            180                 185                 190

Phe Gly Ser Phe Val Asp Lys Thr Val Leu Pro Phe Val Ser Thr Val
        195                 200                 205

Pro Ser Lys Leu His His Pro Cys Pro Ser Arg Leu Glu Arg Cys Gln
    210                 215                 220

Pro Pro Phe Ser Phe His His Val Leu Ser Leu Thr Gly Asp Ala Gln
225                 230                 235                 240

Ala Phe Glu Arg Glu Val Gly Arg Gln Asn Val Ser Gly Asn Leu Asp
                245                 250                 255

Ser Pro Glu Gly Gly Phe Asp Ala Ile Leu Gln Ala Ala Leu Cys Gln
            260                 265                 270

Glu Gln Ile Gly Trp Arg Asn Val Ser Arg Leu Leu Val Phe Thr Ser
        275                 280                 285

Asp Asp Thr Phe His Thr Ala Gly Asp Gly Lys Leu Gly Gly Ile Phe
    290                 295                 300

Met Pro Ser Asp Gly Arg Cys His Leu Asp Ser Asn Gly Val Tyr Thr
305                 310                 315                 320

Asn Ser Ala Glu Phe Asp Tyr Pro Ser Val Gly Gln Val Ala Gln Ala
                325                 330                 335

Leu Thr Ala Ala Asn Ile Gln Pro Ile Phe Ala Val Thr Gly Ala Thr
            340                 345                 350

Leu Pro Val Tyr Gln Glu Leu Arg Gln Leu Ile Pro Lys Ser Ala Val
        355                 360                 365

Gly Glu Leu Ser Glu Asp Ser Ser Asn Val Val Gln Leu Ile Met Asp
    370                 375                 380

Ala Tyr Asp Ser Leu Ser Ser Thr Val Thr Leu Glu His Ser Pro Leu
385                 390                 395                 400

Pro Pro Gly Val Ser Ile Ser Phe Glu Ser His Cys Lys Gly Pro Glu
                405                 410                 415

Lys Thr Glu Gly Glu Ala Gly Asp Arg Gly Gln Cys Asn Asp Val Arg
            420                 425                 430

Val Asn Gln Thr Val Asp Phe Trp Val Thr Leu Gln Ala Thr His Cys
        435                 440                 445

Leu Pro Glu Ala His Val Leu Arg Leu Trp Ala Leu Gly Phe Ser Glu
    450                 455                 460

Glu Leu Thr Val Glu Leu His Thr Val Cys Asp Cys Asn Cys Gly Asp
465                 470                 475                 480

Ala Gln Pro His Ala Pro Tyr Cys Ser Asp Gly Gln Gly Asp Leu Gln

                485                 490                 495

Cys Gly Ile Cys Ser Cys Ala Pro Gly Arg Leu Gly Gln Leu Cys Glu
            500                 505                 510

Cys Ser Glu Ala Asp Leu Ser Ser Pro Asp Leu Glu Ser Gly Cys Arg
        515                 520                 525

Ala Pro Asn Gly Thr Gly Pro Leu Cys Ser Gly Lys Gly Arg Cys Gln
    530                 535                 540

Cys Gly Arg Cys Ser Cys Ser Gly Gln Ser Ser Gly Arg Leu Cys Glu
545                 550                 555                 560

Cys Asp Asp Ala Ser Cys Glu Arg His Glu Gly Ile Leu Cys Gly Gly
                565                 570                 575

Phe Gly His Cys Gln Cys Gly Val Cys His Cys His Ala Asn His Thr
            580                 585                 590

Gly Arg Ala Cys Glu Cys Ser Lys Ser Val Asp Ser Cys Val Ser Pro
        595                 600                 605

Glu Gly Gly Leu Cys Ser Gly His Gly Tyr Cys Lys Cys Asn Arg Cys
    610                 615                 620

Gln Cys Leu Asp Gly Tyr Tyr Gly Ala Leu Cys Asp Gln Cys Leu Gly
625                 630                 635                 640

Cys Lys Ser Pro Cys Glu Gln Tyr Arg Asp Cys Ala Glu Cys Gly Ala
                645                 650                 655

Phe Gly Thr Gly Pro Leu Ala Ala Asn Cys Ser Val Val Cys Ala Asp
            660                 665                 670

Val Asn Val Thr Leu Thr Leu Ala Pro Asn Leu Asp Asp Gly Trp Cys
        675                 680                 685

Lys Glu Arg Thr Ile Asp Asn Gln Leu Phe Phe Phe Leu Val Glu His
    690                 695                 700

Ala Ala Ser Gly Ile Val Leu Arg Val Arg Pro Gln Glu Lys Gly Val
705                 710                 715                 720

Asp His Thr Arg Ala Ile Ile Leu Gly Cys Thr Gly Gly Ile Val Ala
                725                 730                 735

Val Gly Leu Gly Leu Val Leu Ala Tyr Arg Leu Ser Val Glu Ile Tyr
            740                 745                 750

Asp Arg Arg Glu Tyr Arg Arg Phe Glu Lys Glu Gln Gln Gln Leu Asn
        755                 760                 765

Trp Lys Gln Asp Asn Asn Pro Leu Tyr Lys Ser Ala Ile Thr Thr Thr
    770                 775                 780

Val Asn Pro Arg Phe Gln Gly Thr Asn Gly Arg Ser Pro Ser Leu Ser
785                 790                 795                 800

Leu Thr Arg Glu Ala Asp
                805

<210> 114
<211> 806
<212> PRT
<213> Mus musculus

<400> 114

Met Val Asp Ser Ser Thr Val Leu Ile Phe Leu Leu Val Leu Gly Gly
1               5                   10                  15

Gly Gln Ser Glu Leu Asp Thr Lys Ile Thr Ser Ser Gly Glu Ala Ala
            20                  25                  30

Glu Trp Glu Asp Pro Asp Leu Ser Leu Gln Gly Ser Cys Gln Pro Val
        35                  40                  45

Pro Ser Cys Gln Lys Cys Ile Leu Ser His Pro Ser Cys Ala Trp Cys
    50                  55                  60

Lys Gln Leu Asn Phe Thr Ala Ser Gly Glu Ala Glu Ala Arg Arg Cys
65                  70                  75                  80

Gly Arg Arg Glu Glu Leu Leu Ala Arg Gly Cys Pro Ala Gln Glu Leu
                85                  90                  95

Glu Glu Pro Arg Gly Arg Gln Glu Val Leu Gln Asp Lys Pro Leu Ser
            100                 105                 110

Gln Gly Asp Arg Gly Glu Gly Ala Thr Gln Leu Ala Pro Gln Arg Ile
        115                 120                 125

Arg Val Thr Leu Arg Pro Gly Glu Pro Gln Lys Phe Arg Val Arg Phe
    130                 135                 140

Leu Arg Ala Ala Gly Tyr Pro Val Asp Leu Tyr Tyr Leu Met Asp Leu
145                 150                 155                 160

Ser Tyr Ser Met Lys Asp Asp Leu Glu Arg Val Arg Gln Leu Gly His
                165                 170                 175

Ala Leu Leu Val Arg Leu Gln Glu Val Thr His Ser Val Arg Ile Gly
            180                 185                 190

Phe Gly Ser Phe Val Asp Lys Thr Val Leu Pro Phe Val Ser Thr Val
        195                 200                 205

Pro Ser Lys Leu His His Pro Cys Pro Ser Arg Leu Glu Arg Cys Gln
    210                 215                 220

Pro Pro Phe Ser Phe His His Val Leu Ser Leu Thr Gly Asp Ala Gln
225                 230                 235                 240

Ala Phe Glu Arg Glu Val Gly Arg Gln Asn Val Ser Gly Asn Leu Asp
                245                 250                 255

Ser Pro Glu Gly Gly Phe Asp Ala Ile Leu Gln Ala Ala Leu Cys Gln
            260                 265                 270

Glu Gln Ile Gly Trp Arg Asn Val Ser Arg Leu Leu Val Phe Thr Ser
        275                 280                 285

Asp Asp Thr Phe His Thr Ala Gly Asp Gly Lys Leu Gly Gly Ile Phe
    290                 295                 300

Met Pro Ser Asp Gly Arg Cys His Leu Asp Ser Asn Gly Val Tyr Thr
305                 310                 315                 320

Asn Ser Ala Glu Phe Asp Tyr Pro Ser Val Gly Gln Val Ala Gln Ala
                325                 330                 335

Leu Thr Ala Ala Asn Ile Gln Pro Ile Phe Ala Val Thr Gly Ala Thr
            340                 345                 350

Leu Pro Val Tyr Gln Glu Leu Arg Gln Leu Ile Pro Lys Ser Ala Val
        355                 360                 365

Gly Glu Leu Ser Glu Asp Ser Ser Asn Val Val Gln Leu Ile Met Asp
    370                 375                 380

Ala Tyr Asp Ser Leu Ser Ser Thr Val Thr Leu Glu His Ser Pro Leu
385                 390                 395                 400

Pro Pro Gly Val Ser Ile Ser Phe Glu Ser His Cys Lys Gly Pro Glu
                405                 410                 415

Lys Thr Glu Gly Glu Ala Gly Asp Arg Gly Gln Cys Asn Asp Val Arg
            420                 425                 430

Val Asn Gln Thr Val Asp Phe Trp Val Thr Leu Gln Ala Thr His Cys
        435                 440                 445

Leu Pro Glu Ala His Val Leu Arg Leu Trp Ala Leu Gly Phe Ser Glu
    450                 455                 460

Glu Leu Thr Val Glu Leu His Thr Val Cys Asp Cys Asn Cys Gly Asp
465                 470                 475                 480

Ala Gln Pro His Ala Pro Tyr Cys Ser Asp Gly Gln Gly Asp Leu Gln
                485                 490                 495

Cys Gly Ile Cys Ser Cys Ala Pro Gly Arg Leu Gly Gln Leu Cys Glu
            500                 505                 510

Cys Ser Glu Ala Asp Leu Ser Ser Pro Asp Leu Glu Ser Gly Cys Arg
        515                 520                 525

Ala Pro Asn Gly Thr Gly Pro Leu Cys Cys Gly Lys Gly Arg Cys Gln
    530                 535                 540

Cys Gly Arg Cys Ser Cys Ser Gly Gln Ser Ser Gly Arg Leu Cys Glu
545                 550                 555                 560

Cys Asp Asp Ala Ser Cys Glu Arg His Glu Gly Ile Leu Cys Gly Gly
                565                 570                 575

Phe Gly His Cys Gln Cys Gly Val Cys His Cys His Ala Asn His Thr
            580                 585                 590

Gly Arg Ala Cys Glu Cys Ser Lys Ser Val Asp Ser Cys Val Ser Pro
        595                 600                 605

Glu Gly Gly Leu Cys Ser Gly His Gly Tyr Cys Lys Cys Asn Arg Cys
    610                 615                 620

Gln Cys Leu Asp Gly Tyr Tyr Gly Ala Leu Cys Asp Gln Cys Leu Gly
625                 630                 635                 640

Cys Lys Ser Pro Cys Glu Gln Tyr Arg Asp Cys Ala Glu Cys Gly Ala
                645                 650                 655

Phe Gly Thr Gly Pro Leu Ala Ala Asn Cys Ser Val Val Cys Ala Asp
            660                 665                 670

Val Asn Val Thr Leu Thr Leu Ala Pro Asn Leu Asp Asp Gly Trp Cys
        675                 680                 685

Lys Glu Arg Thr Ile Asp Asn Gln Leu Phe Phe Phe Leu Val Glu His
    690                 695                 700

Ala Ala Ser Gly Ile Val Leu Arg Val Arg Pro Gln Glu Lys Gly Val
705                 710                 715                 720

Asp His Thr Arg Ala Ile Ile Leu Gly Cys Thr Gly Gly Ile Val Ala
                725                 730                 735

Val Gly Leu Gly Leu Val Leu Ala Tyr Arg Leu Ser Val Glu Ile Tyr
            740                 745                 750

Asp Arg Arg Glu Tyr Arg Arg Phe Glu Lys Glu Gln Gln Gln Leu Asn
        755                 760                 765

Trp Lys Gln Asp Asn Asn Pro Leu Tyr Lys Ser Ala Ile Thr Thr Thr
    770                 775                 780

Val Asn Pro Arg Phe Gln Gly Thr Asn Gly Arg Ser Pro Ser Leu Ser
785                 790                 795                 800

Leu Thr Arg Glu Ala Asp
                805

<210> 115
<211> 805
<212> PRT
<213> Mus musculus

<400> 115
Met Val Asp Ser Ser Thr Val Leu Ile Phe Leu Leu Val Leu Gly Gly
  1               5                  10                  15

Gly Gln Ser Glu Leu Asp Thr Lys Ile Thr Ser Ser Gly Glu Ala Ala
            20                  25                  30

Glu Trp Glu Asp Pro Asp Leu Ser Leu Gln Gly Ser Cys Gln Pro Val
        35                  40                  45

Pro Ser Cys Gln Lys Cys Ile Leu Ser His Pro Ser Cys Ala Trp Cys
    50                  55                  60

Lys Gln Leu Asn Phe Thr Ala Ser Gly Glu Ala Glu Ala Arg Arg Cys
65                  70                  75                  80

Ala Arg Arg Glu Glu Leu Leu Ala Arg Gly Cys Pro Ala Gln Glu Leu
                85                  90                  95

Glu Glu Pro Arg Gly Arg Gln Glu Val Leu Gln Asp Lys Pro Leu Ser
            100                 105                 110

Gln Gly Asp Arg Gly Glu Gly Ala Thr Gln Leu Pro Gln Arg Ile Arg
        115                 120                 125

Val Thr Leu Arg Pro Gly Glu Pro Gln Lys Phe Arg Val Arg Phe Leu
    130                 135                 140

Arg Ala Ala Gly Tyr Pro Val Asp Leu Tyr Tyr Leu Met Asp Leu Ser
145                 150                 155                 160

Tyr Ser Met Lys Asp Asp Leu Glu Arg Val Arg Gln Leu Gly His Ala
                165                 170                 175

Leu Leu Val Arg Leu Gln Glu Val Thr His Ser Val Arg Ile Gly Phe
            180                 185                 190

Gly Ser Phe Val Asp Lys Thr Val Leu Pro Phe Val Ser Thr Val Pro
        195                 200                 205

Ser Lys Leu His His Pro Cys Pro Ser Arg Leu Glu Arg Cys Gln Pro
    210                 215                 220

Pro Phe Ser Phe His His Val Leu Ser Leu Thr Gly Asp Ala Gln Ala
225                 230                 235                 240

Phe Glu Arg Glu Val Gly Arg Gln Asn Val Ser Gly Asn Leu Asp Ser
                245                 250                 255

Pro Glu Gly Gly Phe Asp Ala Ile Leu Gln Ala Ala Leu Cys Gln Glu
            260                 265                 270

Gln Ile Gly Trp Arg Asn Val Ser Arg Leu Leu Val Phe Thr Ser Asp
        275                 280                 285

Asp Thr Phe His Thr Ala Gly Asp Gly Lys Leu Gly Gly Ile Phe Met
    290                 295                 300

Pro Ser Asp Gly Arg Cys His Leu Asp Ser Asn Gly Val Tyr Thr Asn
305                 310                 315                 320

Ser Ala Glu Phe Asp Tyr Pro Ser Val Gly Gln Val Ala Gln Ala Leu
                325                 330                 335

Thr Ala Ala Asn Ile Gln Pro Ile Phe Ala Val Thr Gly Ala Thr Leu
            340                 345                 350

Pro Val Tyr Gln Glu Leu Arg Gln Leu Ile Pro Lys Ser Ala Val Gly
        355                 360                 365

Glu Leu Ser Glu Asp Ser Ser Asn Val Val Gln Leu Ile Met Asp Ala
    370                 375                 380

Tyr Asp Ser Leu Ser Ser Thr Val Thr Leu Glu His Ser Pro Leu Pro
385                 390                 395                 400

Pro Gly Val Ser Ile Ser Phe Glu Ser His Cys Lys Gly Pro Glu Lys
                405                 410                 415

Thr Glu Gly Glu Ala Gly Asp Arg Gly Gln Cys Asn Asp Val Arg Val
            420                 425                 430

Asn Gln Thr Val Asp Phe Trp Val Thr Leu Gln Ala Thr His Cys Leu
        435                 440                 445

Pro Glu Ala His Val Leu Arg Leu Trp Ala Leu Gly Phe Ser Glu Glu
    450                 455                 460

Leu Thr Val Glu Leu His Thr Val Cys Asp Cys Asn Cys Gly Asp Ala
465                 470                 475                 480

Gln Pro His Ala Pro Tyr Cys Ser Asp Gly Gln Gly Asp Leu Gln Cys
                485                 490                 495

Gly Ile Cys Ser Cys Ala Pro Gly Arg Leu Gly Gln Leu Cys Glu Cys
            500                 505                 510

Ser Glu Ala Asp Leu Ser Ser Pro Asp Leu Glu Ser Gly Cys Arg Ala
        515                 520                 525

Pro Asn Gly Thr Gly Pro Leu Cys Ser Gly Lys Gly Arg Cys Gln Cys
    530                 535                 540

Gly Arg Cys Ser Cys Ser Gly Gln Ser Ser Gly His Leu Cys Glu Cys
545                 550                 555                 560

Asp Asp Ala Ser Cys Glu Arg His Glu Gly Ile Leu Cys Gly Gly Phe
                565                 570                 575

Gly His Cys Gln Cys Gly Val Cys His Cys His Ala Asn His Thr Gly
            580                 585                 590

Arg Ala Cys Glu Cys Ser Lys Ser Val Asp Ser Cys Val Ser Pro Glu
        595                 600                 605

Gly Gly Leu Cys Ser Gly His Gly Tyr Cys Lys Cys Asn Arg Cys Gln
    610                 615                 620

Cys Leu Asp Gly Tyr Tyr Gly Ala Leu Cys Asp Gln Cys Leu Gly Cys
625                 630                 635                 640

Lys Ser Pro Cys Glu Gln Tyr Arg Asp Cys Ala Glu Cys Gly Ala Phe
                645                 650                 655

Gly Thr Gly Pro Leu Ala Ala Asn Cys Ser Val Val Cys Ala Asp Val
            660                 665                 670

Asn Val Thr Leu Thr Leu Ala Pro Asn Leu Asp Asp Gly Trp Cys Lys
        675                 680                 685

Glu Arg Thr Ile Asp Asn Gln Leu Phe Phe Phe Leu Val Glu His Ala
    690                 695                 700

Ala Ser Gly Ile Val Leu Arg Val Arg Pro Gln Glu Lys Gly Val Asp
705                 710                 715                 720

His Thr Arg Ala Ile Ile Leu Gly Cys Thr Gly Gly Ile Val Ala Val
                725                 730                 735

Gly Leu Gly Leu Val Leu Ala Tyr Arg Leu Ser Val Glu Ile Tyr Asp
            740                 745                 750

Arg Arg Glu Tyr Arg Arg Phe Glu Lys Glu Gln Gln Gln Leu Asn Trp
        755                 760                 765

Lys Gln Asp Asn Asn Pro Leu Tyr Lys Ser Ala Ile Thr Thr Thr Val
    770                 775                 780

Asn Pro Arg Phe Gln Gly Thr Asn Gly Arg Ser Pro Ser Leu Ser Leu
785                 790                 795                 800

Thr Arg Glu Ala Asp
                805

<210> 116
<211> 640
<212> PRT
<213> Rattus norvegicus

<400> 116
Leu Glu Arg Val Arg Gln Leu Gly Gln Ala Leu Leu Leu Arg Leu Arg
  1               5                  10                  15

Glu Val Thr His Ser Val Arg Ile Gly Phe Gly Ser Phe Val Asp Lys
             20                  25                  30

Thr Val Leu Pro Phe Val Ser Thr Val Pro Ser Lys Leu Asn His Pro
         35                  40                  45

Cys Pro Ser Arg Leu Glu Arg Cys Gln Ala Pro Phe Ser Tyr His His
     50                  55                  60

Val Leu Pro Leu Thr Arg Asp Ala Gln Ala Phe Glu Arg Glu Val Gly
 65                  70                  75                  80

Arg Gln Asn Val Ser Gly Asn Leu Asp Ser Pro Glu Gly Gly Phe Asp
                 85                  90                  95

Ala Ile Leu Gln Ala Ala Leu Cys Gln Glu Gln Ile Gly Trp Arg Asn
            100                 105                 110

Val Ser Arg Leu Leu Val Phe Thr Ser Asp Asp Thr Phe His Thr Ala
        115                 120                 125

Gly Asp Gly Lys Leu Gly Gly Ile Phe Met Pro Ser Asp Gly Arg Cys

130                 135                 140

His Leu Asp Ser Asn Gly Val Tyr Thr Asn Ser Ala Glu Phe Asp Tyr
145                 150                 155                 160

Pro Ser Val Gly Gln Val Ala Gln Ala Leu Thr Ala Ala Asn Ile Gln
                165                 170                 175

Pro Ile Phe Ala Val Thr Gly Ala Thr Leu Pro Val Tyr Gln Glu Leu
            180                 185                 190

Ser Gln Leu Ile Pro Lys Ser Ala Val Gly Glu Leu Ser Glu Asp Ser
        195                 200                 205

Ser Asn Val Val Gln Leu Ile Met Asp Ala Tyr Asp Asn Leu Ser Ser
    210                 215                 220

Thr Val Thr Leu Glu His Ser Ser Leu Pro Pro Gly Val Ser Ile Ser
225                 230                 235                 240

Phe Glu Ser His Cys Arg Ser Pro Glu Lys Ser Glu Gly Glu Ala Gly
                245                 250                 255

Asp Arg Arg Gly Gln Cys Asn His Val Arg Val Asn Arg Met Val Asp
            260                 265                 270

Phe Trp Val Thr Leu Gln Ala Ser His Cys Leu Pro Glu Ala His Val
        275                 280                 285

Leu Arg Leu Trp Ala Leu Gly Phe Ser Glu Glu Leu Thr Val Glu Leu
    290                 295                 300

His Thr Leu Cys Asp Cys Asn Cys Ser Asp Ala Gln Pro Arg Ala Pro
305                 310                 315                 320

Tyr Cys Ser Asp Gly Gln Gly Asp Leu Gln Cys Gly Ile Cys Ser Cys
                325                 330                 335

Ala Pro Gly Arg Leu Gly Gln Leu Cys Glu Cys Ser Glu Ala Asp Leu
            340                 345                 350

Ser Ser Pro Asp Leu Glu Ser Gly Cys Arg Ala Pro Asn Gly Thr Gly
        355                 360                 365

Pro Leu Cys Ser Gly Lys Gly Arg Cys Gln Cys Gly His Cys Ser Cys
    370                 375                 380

Ser Gly Gln Ser Ser Gly Arg Leu Cys Glu Cys Asp Asp Ala Ser Cys
385                 390                 395                 400

Glu Arg His Gly Gly Ile Phe Cys Gly Gly Phe Gly His Cys Gln Cys
                405                 410                 415

Gly Val Cys His Cys His Ala Asn Arg Thr Gly Arg Ala Cys Glu Cys
            420                 425                 430

Ser Glu Ser Val Asp Ser Cys Val Ser Pro Glu Gly Gly Leu Cys Ser
        435                 440                 445

Gly His Gly Asp Cys Lys Cys Asn Arg Cys Gln Cys Leu Asp Gly Tyr
    450                 455                 460

Tyr Gly Ala Leu Cys Asp Gln Cys Leu Gly Cys Lys Ser Pro Cys Glu
465                 470                 475                 480

Gln Tyr Arg Asp Cys Ala Glu Cys Gly Ala Phe Gly Thr Gly Pro Leu
                485                 490                 495

Ala Ala Asn Cys Ser Ala Ala Cys Ala Asp Val Asn Val Thr Leu Ala

500                 505                 510

Leu Ala Pro Asn Leu Asp Asp Gly Trp Cys Lys Glu Arg Thr Leu Asp
        515                 520                 525

Asn Gln Leu Phe Phe Phe Leu Val Glu Asn Ser Ala Arg Gly Val Ile
    530                 535                 540

Leu Arg Val Arg Pro Gln Glu Lys Gly Ala Asp His Thr Arg Ala Ile
545                 550                 555                 560

Ile Leu Gly Cys Val Gly Gly Ile Val Ala Val Gly Leu Gly Leu Val
                565                 570                 575

Leu Ala Tyr Arg Leu Ser Val Glu Val Tyr Asp Arg Leu Glu Tyr Ser
            580                 585                 590

Arg Phe Glu Lys Glu Arg Gln Gln Leu Asn Trp Lys Gln Asp Ser Asn
        595                 600                 605

Pro Leu Tyr Lys Ser Ala Val Thr Thr Thr Val Asn Pro Arg Phe Gln
    610                 615                 620

Gly Gly Asn Lys Gln Ser Leu Ser Leu Pro Leu Thr Gln Glu Ala Asp
625                 630                 635                 640

<210> 117
<211> 428
<212> PRT
<213> Homo sapiens

<400> 117
Ser Cys Glu Glu Cys Leu Leu Ser Gly Pro Gly Cys Ala Trp Cys Thr
  1               5                  10                  15

Lys Glu Asp Phe Thr Ser Pro Gly Glu Pro Asp Ser Glu Arg Cys Asp
             20                  25                  30

Thr Arg Ala Asn Leu Leu Ser Lys Gly Cys Pro Leu Asp Ser Ile Glu
         35                  40                  45

Asn Pro Arg Ser Ser Ala Glu Ile Leu Glu Asp Gln Pro Leu Ser Asn
     50                  55                  60

Lys Gly Ser Arg Asp Ser Ser Thr Gln Leu Ser Pro Gln Glu Val Arg
 65                  70                  75                  80

Leu Arg Leu Arg Pro Gly Glu Glu Gln Thr Phe Gln Leu Thr Val Arg
                 85                  90                  95

Arg Ala Glu Asp Tyr Pro Val Asp Leu Tyr Tyr Leu Met Asp Leu Ser
            100                 105                 110

Tyr Ser Met Lys Asp Asp Leu Glu Asn Val Lys Ser Leu Gly Thr Asp
        115                 120                 125

Leu Ala Arg Glu Met Ser Lys Leu Thr Ser Asp Phe Arg Ile Gly Phe
    130                 135                 140

Gly Ser Phe Val Asp Lys Thr Val Ser Pro Phe Val Ser Thr His Pro
145                 150                 155                 160

Glu Lys Leu Arg Asn Pro Cys Pro Asn Lys Glu Lys Asn Cys Gln Pro
                165                 170                 175

Pro Phe Gly Phe Lys His Val Leu Ser Leu Thr Asp Asp Val Asp Glu
            180                 185                 190

Phe Asn Glu Glu Val Gly Lys Gln Arg Ile Ser Gly Asn Leu Asp Ala
        195                 200                 205

Pro Glu Gly Gly Phe Asp Ala Ile Met Gln Ala Ala Val Cys Lys Glu
    210                 215                 220

Lys Ile Gly Trp Arg Asn Asp Val Thr Arg Leu Leu Val Phe Ser Thr
225                 230                 235                 240

Asp Ala Gly Phe His Phe Ala Gly Asp Gly Lys Leu Gly Gly Ile Val
                245                 250                 255

Gln Pro Asn Asp Gly Arg Cys His Leu Asp Ser Asn Asn Met Tyr Thr
            260                 265                 270

Met Ser Thr Thr Met Asp Tyr Pro Ser Val Gly Gln Leu Val Glu Lys
        275                 280                 285

Leu Ser Glu Asn Asn Ile Gln Pro Ile Phe Ala Val Thr Glu Lys Gln
    290                 295                 300

Val His Leu Tyr Glu Lys Leu Thr Glu Leu Ile Pro Gly Ser Ala Val
305                 310                 315                 320

Gly Glu Leu Ser Glu Asp Ser Ser Asn Val Val Gln Leu Ile Ile Asp
                325                 330                 335

Ala Tyr Asn Lys Leu Arg Ser Glu Val Glu Leu Glu Val Ser Asp Leu
            340                 345                 350

Pro Glu Gly Leu Asn Leu Ser Tyr Thr Ser Phe Cys Lys Asn Gly Val
        355                 360                 365

Ser Ser Pro Gly Gln Arg Arg Gly Lys Cys Ser Gly Val Gln Ile Gly
    370                 375                 380

Asp Thr Val Ser Phe Glu Val Ser Val Thr Ala Arg Glu Cys Pro Pro
385                 390                 395                 400

Glu Gly Gln Lys His Ser Ile Ile Ile Lys Pro Leu Gly Phe Ser Asp
                405                 410                 415

Thr Leu Glu Val His Val His Pro Asn Cys Asp Cys
            420                 425


<210> 118
<211> 423
<212> PRT
<213> Homo sapiens

<400> 118
Ser Cys Gly Glu Cys Ile Gln Ser His Pro Asn Cys Ala Trp Cys Thr
1               5                   10                  15

Asp Glu Asn Phe Thr Ser Gly Gly Ser Ser Ala Arg Cys Asp Ser Arg
            20                  25                  30

Ala Asn Leu Leu Ala Lys Gly Cys Ser Pro Glu Ser Ile Glu Asp Pro
        35                  40                  45

Lys Ser Glu Ile Glu Val Leu Glu Asp Gln Pro Leu Ser Asp Ser Glu
    50                  55                  60

Ser Ser Gly Gln Ala Val Gln Val Ser Pro Gln Arg Val Arg Leu Lys
65                  70                  75                  80

Leu Arg Pro Gly Glu Pro Gln Asn Phe Thr Leu Thr Val Arg Gln Ala
                85                  90                  95

Glu Asp Tyr Pro Val Asp Leu Tyr Tyr Leu Met Asp Leu Ser Tyr Ser
            100                 105                 110

Met Lys Asp Asp Leu Asp Asn Leu Lys Ser Leu Gly Asp Asp Leu Ala
        115                 120                 125

Arg Glu Met Lys Gly Leu Thr Ser Asn Phe Arg Leu Gly Phe Gly Ser
    130                 135                 140

Phe Val Asp Lys Thr Val Ser Pro Phe Val Ser Thr Arg Pro Glu Lys
145                 150                 155                 160

Leu Glu Asn Pro Cys Pro Asn Tyr Asn Leu Thr Cys Glu Pro Pro Tyr
                165                 170                 175

Gly Phe Lys His Val Leu Ser Leu Thr Asp Asp Thr Asp Glu Phe Asn
            180                 185                 190

Glu Glu Val Lys Lys Gln Arg Ile Ser Gly Asn Leu Asp Ala Pro Glu
        195                 200                 205

Gly Gly Phe Asp Ala Ile Met Gln Ala Ala Val Cys Thr Glu Gln Ile
    210                 215                 220

Gly Trp Arg Glu Asp Ala Arg Arg Leu Leu Val Phe Ser Thr Asp Ala
225                 230                 235                 240

Gly Phe His Phe Ala Gly Asp Gly Lys Leu Ala Gly Ile Val Gln Pro
                245                 250                 255

Asn Asp Gly Gln Cys His Leu Asp Asn Asn Gly Glu Tyr Thr Met Ser
            260                 265                 270

Thr Thr Gln Asp Tyr Pro Ser Ile Gly Gln Leu Asn Gln Lys Leu Ala
        275                 280                 285

Glu Asn Asn Ile Asn Pro Ile Phe Ala Val Thr Lys Lys Gln Val Ser
    290                 295                 300

Leu Tyr Lys Glu Leu Ser Ala Leu Ile Pro Gly Ser Ser Val Gly Val
305                 310                 315                 320

Leu Ser Glu Asp Ser Ser Asn Val Val Glu Leu Ile Lys Asp Ala Tyr
                325                 330                 335

Asn Lys Ile Ser Ser Arg Val Glu Leu Glu Asp Asn Ser Leu Pro Glu
            340                 345                 350

Gly Val Ser Val Thr Tyr Thr Ser Ser Cys Pro Gly Gly Val Val Gly
        355                 360                 365

Pro Gly Thr Arg Lys Cys Glu Gly Val Lys Ile Gly Asp Thr Val Ser
    370                 375                 380

Phe Glu Val Thr Val Thr Ala Thr Lys Cys Pro Pro Glu Lys Glu His
385                 390                 395                 400

Ser Ile Arg Ile Arg Pro Val Gly Phe Ser Glu Thr Leu Glu Val Glu
                405                 410                 415

Leu Thr Phe Leu Cys Asp Cys
            420

<210> 119

<211> 346
<212> PRT
<213> Homo sapiens

<400> 119
Met Ala Met Ala Gln Lys Leu Ser His Leu Leu Pro Ser Leu Arg Gln
1               5                   10                  15

Val Ile Gln Glu Pro Gln Leu Ser Leu Gln Pro Glu Pro Val Phe Thr
            20                  25                  30

Val Asp Arg Ala Glu Val Pro Pro Leu Phe Trp Lys Pro Tyr Ile Tyr
        35                  40                  45

Ala Gly Tyr Arg Pro Leu His Gln Thr Trp Arg Phe Tyr Phe Arg Thr
    50                  55                  60

Leu Phe Gln Gln His Asn Glu Ala Val Asn Val Trp Thr His Leu Leu
65                  70                  75                  80

Ala Ala Leu Val Leu Leu Leu Arg Leu Ala Leu Phe Val Glu Thr Val
                85                  90                  95

Asp Phe Trp Gly Asp Pro His Ala Leu Pro Leu Phe Ile Ile Val Leu
            100                 105                 110

Ala Ser Phe Thr Tyr Leu Ser Phe Ser Ala Leu Ala His Leu Leu Gln
        115                 120                 125

Ala Lys Ser Glu Phe Trp His Tyr Ser Phe Phe Phe Leu Asp Tyr Val
    130                 135                 140

Gly Val Ala Val Tyr Gln Phe Gly Ser Ala Leu Ala His Phe Tyr Tyr
145                 150                 155                 160

Ala Ile Glu Pro Ala Trp His Ala Gln Val Gln Ala Val Phe Leu Pro
                165                 170                 175

Met Ala Ala Phe Leu Ala Trp Leu Ser Cys Ile Gly Ser Cys Tyr Asn
            180                 185                 190

Lys Tyr Ile Gln Lys Pro Gly Leu Leu Gly Arg Thr Cys Gln Glu Val
        195                 200                 205

Pro Ser Val Leu Ala Tyr Ala Leu Asp Ile Ser Pro Val Val His Arg
    210                 215                 220

Ile Phe Val Ser Ser Asp Pro Thr Thr Asp Asp Pro Ala Leu Leu Tyr
225                 230                 235                 240

His Lys Cys Gln Val Val Phe Phe Leu Leu Ala Ala Ala Phe Phe Ser
                245                 250                 255

Thr Phe Met Pro Glu Arg Trp Phe Pro Gly Ser Cys His Val Phe Gly
            260                 265                 270

Gln Gly His Gln Leu Phe His Ile Phe Leu Val Leu Cys Thr Leu Ala
        275                 280                 285

Gln Leu Glu Ala Val Ala Leu Asp Tyr Glu Ala Arg Arg Pro Ile Tyr
    290                 295                 300

Glu Pro Leu His Thr His Trp Pro His Asn Phe Ser Gly Leu Phe Leu
305                 310                 315                 320

Leu Thr Val Gly Ser Ser Ile Leu Thr Ala Phe Leu Leu Ser Gln Leu
                325                 330                 335

Val Gln Arg Lys Leu Asp Gln Lys Thr Lys

340                 345

<210> 120
<211> 345
<212> PRT
<213> Mus musculus

<400> 120
Met Ala Met Ala Val Ala Gln Lys Phe Asn His Leu Leu Ser Ser Leu
1               5                   10                  15

Trp His Val Gly Gln Lys Pro Pro Gln Pro Glu Pro Val Phe Thr Val
            20                  25                  30

Asp Arg Ala Gln Val Pro Pro Leu Phe Trp Lys Pro Tyr Ile Tyr Ala
        35                  40                  45

Gly Tyr Arg Pro Leu His Gln Asn Trp Cys Phe Tyr Phe Arg Thr Leu
    50                  55                  60

Phe Gln Arg His Asn Glu Ala Val Asn Val Trp Thr His Leu Leu Ala
65                  70                  75                  80

Ala Leu Ala Leu Leu Leu Arg Leu Ile Gly Leu Ala Ala Ser Val Asp
                85                  90                  95

Phe Arg Glu Asp Pro His Ala Leu Pro Leu Phe Phe Ile Val Leu Ala
            100                 105                 110

Ser Phe Thr Tyr Leu Ser Phe Ser Ala Val Ala His Leu Leu Gln Ala
        115                 120                 125

Lys Ser Glu Phe Trp His Tyr Ser Phe Phe Phe Leu Asp Tyr Val Gly
    130                 135                 140

Val Ala Val Tyr Gln Phe Gly Ser Ala Leu Ala His Phe Tyr Tyr Ala
145                 150                 155                 160

Ile Glu Pro Ser Trp His Asp Lys Val Gln Ala Ile Phe Leu Pro Thr
                165                 170                 175

Ala Ala Phe Leu Ala Trp Leu Ser Cys Ala Gly Ser Cys Tyr Asn Lys
            180                 185                 190

Tyr Ser Gln Lys Pro Gly Leu Leu Gly Arg Ile Phe Gln Glu Ala Pro
        195                 200                 205

Ser Ala Leu Ala Tyr Val Leu Asp Ile Ser Pro Val Leu His Arg Ile
    210                 215                 220

Ile Val Ser Pro Leu Pro Ala Glu Glu Asp Pro Ala Leu Leu Tyr His
225                 230                 235                 240

Lys Cys Gln Val Val Phe Phe Leu Leu Ala Ala Ala Phe Phe Ser Thr
                245                 250                 255

Val Met Pro Glu Ser Trp Phe Pro Gly Ser Cys His Ile Phe Gly Gln
            260                 265                 270

Gly His Gln Val Phe His Val Phe Leu Val Leu Cys Thr Leu Ala Gln
        275                 280                 285

Leu Glu Ala Val Thr Leu Asp Tyr Gln Ala Arg Arg Gly Ile Tyr Glu
    290                 295                 300

Pro Leu His Ala Arg Trp Pro His Asn Phe Ser Gly Leu Phe Leu Leu
305                 310                 315                 320

Thr Val Ala Ser Ser Ser Leu Thr Ala Leu Leu Leu Ser Gln Leu Val
                325                 330                 335

Arg Arg Lys Leu His Gln Lys Thr Lys
            340                 345


<210> 121
<211> 354
<212> PRT
<213> Mus musculus

<400> 121
Met Thr Thr Ala Ile Leu Glu Arg Leu Ser Thr Leu Ser Met Ser Gly
1               5                   10                  15

Gln Gln Leu Arg Arg Leu Pro Lys Ile Leu Glu Glu Gly Leu Pro Lys
            20                  25                  30

Met Pro Cys Thr Val Pro Glu Thr Asp Val Pro Gln Leu Phe Arg Glu
        35                  40                  45

Pro Tyr Ile His Ala Gly Tyr Arg Pro Thr Gly His Glu Trp Arg Tyr
    50                  55                  60

Tyr Phe Phe Ser Leu Phe Gln Lys His Asn Glu Val Val Asn Val Trp
65                  70                  75                  80

Thr His Leu Leu Ala Ala Leu Ala Val Leu Leu Arg Phe Trp Ala Phe
                85                  90                  95

Val Glu Ala Gly Ala Leu Gln Trp Ala Ser Pro His Thr Leu Pro Leu
            100                 105                 110

Leu Leu Phe Ile Leu Ser Ser Ile Thr Tyr Leu Thr Cys Ser Leu Leu
        115                 120                 125

Ala His Leu Leu Gln Ser Lys Ser Glu Leu Ser His Tyr Thr Phe Tyr
    130                 135                 140

Phe Val Asp Tyr Val Gly Val Ser Val Tyr Gln Tyr Gly Ser Ala Leu
145                 150                 155                 160

Ala His Phe Phe Tyr Ser Ser Asp Gln Ala Trp Tyr Glu Leu Phe Trp
                165                 170                 175

Ile Phe Phe Leu Pro Ala Ala Ala Phe Cys Gly Trp Leu Ser Cys Ala
            180                 185                 190

Gly Cys Cys Tyr Ala Lys Tyr Arg Tyr Arg Arg Pro Tyr Pro Val Met
        195                 200                 205

Arg Lys Ile Cys Gln Val Val Pro Ala Gly Leu Ala Phe Val Leu Asp
    210                 215                 220

Ile Ser Pro Val Ala His Arg Val Ala Leu Cys His Leu Ala Gly Cys
225                 230                 235                 240

Gln Glu Gln Ala Ala Trp Tyr His Thr Leu Gln Ile Leu Phe Phe Leu
                245                 250                 255

Val Ser Ala Tyr Phe Phe Ser Cys Pro Val Pro Glu Lys Tyr Phe Pro
            260                 265                 270

Gly Ser Cys Asp Ile Val Gly His Gly His Gln Ile Phe His Ala Phe
        275                 280                 285

Leu Ser Val Cys Thr Leu Ser Gln Leu Glu Ala Ile Leu Leu Asp Tyr
    290                 295                 300

Gln Gly Arg His Glu Ile Phe Leu Gln Arg His Gly Pro Leu Ser Val
305                 310                 315                 320

Tyr Ser Ala Cys Leu Ser Phe Phe Val Leu Ala Ala Cys Ser Ala Ala
                325                 330                 335

Thr Ala Thr Leu Leu Arg His Lys Val Lys Asp Arg Leu Ile Lys Lys
            340                 345                 350

Asp Ser

<210> 122
<211> 359
<212> PRT
<213> Homo sapiens

<400> 122
Met Arg Ala Ala Ala Met Thr Thr Ala Ile Leu Glu Arg Leu Ser Thr
  1               5                  10                  15

Leu Ser Val Ser Gly Gln Gln Leu Arg Arg Leu Pro Lys Ile Leu Glu
             20                  25                  30

Asp Gly Leu Pro Lys Met Pro Cys Thr Val Pro Glu Thr Asp Val Pro
         35                  40                  45

Gln Leu Phe Arg Glu Pro Tyr Ile Arg Thr Gly Tyr Arg Pro Thr Gly
     50                  55                  60

His Glu Trp Arg Tyr Tyr Phe Phe Ser Leu Phe Gln Lys His Asn Glu
 65                  70                  75                  80

Val Val Asn Val Trp Thr His Leu Leu Ala Ala Leu Ala Val Leu Leu
                 85                  90                  95

Arg Phe Trp Ala Phe Ala Glu Ala Glu Ala Leu Pro Trp Ala Ser Thr
            100                 105                 110

His Ser Leu Pro Leu Leu Leu Phe Ile Leu Ser Ser Ile Thr Tyr Leu
        115                 120                 125

Thr Cys Ser Leu Leu Ala His Leu Leu Gln Ser Lys Ser Glu Leu Ser
    130                 135                 140

His Tyr Thr Phe Tyr Phe Val Asp Tyr Val Gly Val Ser Val Tyr Gln
145                 150                 155                 160

Tyr Gly Ser Ala Leu Ala His Phe Phe Tyr Ser Ser Asp Gln Ala Trp
                165                 170                 175

Tyr Asp Arg Phe Trp Leu Phe Phe Leu Pro Ala Ala Ala Phe Cys Gly
            180                 185                 190

Trp Leu Ser Cys Ala Gly Cys Cys Tyr Ala Lys Tyr Arg Tyr Arg Arg
        195                 200                 205

Pro Tyr Pro Val Met Arg Lys Ile Cys Gln Val Val Pro Ala Gly Leu
    210                 215                 220

Ala Phe Ile Leu Asp Ile Ser Pro Val Ala His Arg Val Ala Leu Cys
225                 230                 235                 240

His Leu Ala Gly Cys Gln Glu Gln Ala Ala Trp Tyr His Thr Leu Gln
                245                 250                 255

Ile Leu Phe Phe Leu Val Ser Ala Tyr Phe Phe Ser Cys Pro Val Pro

            260                 265                 270

Glu Lys Tyr Phe Pro Gly Ser Cys Asp Ile Val Gly His Gly His Gln
        275                 280                 285

Ile Phe His Ala Phe Leu Ser Ile Cys Thr Leu Ser Gln Leu Glu Ala
    290                 295                 300

Ile Leu Leu Asp Tyr Gln Gly Arg Gln Glu Ile Phe Leu Gln Arg His
305                 310                 315                 320

Gly Pro Leu Ser Val His Met Ala Cys Leu Ser Phe Phe Phe Leu Ala
                325                 330                 335

Ala Cys Ser Ala Ala Thr Ala Ala Leu Leu Arg His Lys Val Lys Ala
            340                 345                 350

Arg Leu Thr Lys Lys Asp Ser
        355

<210> 123
<211> 354
<212> PRT
<213> Homo sapiens

<400> 123
Met Thr Thr Ala Ile Leu Glu Arg Leu Ser Thr Leu Ser Val Ser Gly
1               5                   10                  15

Gln Gln Leu Arg Arg Leu Pro Lys Ile Leu Glu Asp Gly Leu Pro Lys
            20                  25                  30

Met Pro Cys Thr Val Pro Glu Thr Asp Val Pro Gln Leu Phe Arg Glu
        35                  40                  45

Pro Tyr Ile Arg Thr Gly Tyr Arg Pro Thr Gly His Glu Trp Arg Tyr
    50                  55                  60

Tyr Phe Phe Ser Leu Phe Gln Lys His Asn Glu Val Val Asn Val Trp
65                  70                  75                  80

Thr His Leu Leu Ala Ala Leu Ala Val Leu Leu Arg Phe Trp Ala Phe
                85                  90                  95

Ala Glu Ala Glu Ala Leu Pro Trp Ala Ser Thr His Ser Leu Pro Leu
            100                 105                 110

Leu Leu Phe Ile Leu Ser Ser Ile Thr Tyr Leu Thr Cys Ser Leu Leu
        115                 120                 125

Ala His Leu Leu Gln Ser Lys Ser Glu Leu Ser His Tyr Thr Phe Tyr
    130                 135                 140

Phe Val Asp Tyr Val Gly Val Ser Val Tyr Gln Tyr Gly Ser Ala Leu
145                 150                 155                 160

Ala His Phe Phe Tyr Ser Ser Asp Gln Ala Trp Tyr Asp Arg Phe Trp
                165                 170                 175

Leu Phe Phe Leu Pro Ala Ala Ala Phe Cys Gly Trp Leu Ser Cys Ala
            180                 185                 190

Gly Cys Cys Tyr Ala Lys Tyr Arg Tyr Arg Arg Pro Tyr Pro Val Met
        195                 200                 205

Arg Lys Ile Cys Gln Val Val Pro Ala Gly Leu Ala Phe Ile Leu Asp
    210                 215                 220

Ile Ser Pro Val Ala His Arg Val Ala Leu Cys His Leu Ala Gly Cys
225                 230                 235                 240

Gln Glu Gln Ala Ala Trp Tyr His Thr Leu Gln Ile Leu Phe Phe Leu
                245                 250                 255

Val Ser Ala Tyr Phe Phe Ser Cys Pro Val Pro Glu Lys Tyr Phe Pro
            260                 265                 270

Gly Ser Cys Asp Ile Val Gly His Gly His Gln Ile Phe His Ala Phe
        275                 280                 285

Leu Ser Ile Cys Thr Leu Ser Gln Leu Glu Ala Ile Leu Leu Asp Tyr
    290                 295                 300

Gln Gly Arg Gln Glu Ile Phe Leu Gln Arg His Gly Pro Leu Ser Val
305                 310                 315                 320

His Met Ala Cys Leu Ser Phe Phe Phe Leu Ala Ala Cys Ser Ala Ala
                325                 330                 335

Thr Ala Ala Leu Leu Arg His Lys Val Lys Ala Arg Leu Thr Lys Lys
            340                 345                 350

Asp Ser

<210> 124
<211> 238
<212> PRT
<213> Homo sapiens

<400> 124
Ser Ser Phe Lys Lys Cys Phe Lys Ser Ile Phe Ser Trp His Asn Glu
1               5                   10                  15

Thr Ser Asn Ile Trp Thr His Leu Leu Gly Phe Ile Leu Phe Phe Phe
            20                  25                  30

Leu Leu Ile Leu Phe Phe Leu Phe Leu Leu Pro Pro Ile Leu Ala Thr
        35                  40                  45

Trp Gln Asp Arg Val Val Phe Gly Phe Phe Leu Ala Gly Ala Phe Leu
    50                  55                  60

Cys Leu Leu Leu Ser Ser Ile Tyr His Thr Phe Ser Cys His Ser Glu
65                  70                  75                  80

Lys His Ser Glu Phe Phe Leu Lys Leu Asp Tyr Leu Gly Ile Ser Leu
                85                  90                  95

Leu Ile Val Ala Ser Phe Ile Pro Ile Ile Tyr Tyr Ala Phe Tyr Cys
            100                 105                 110

His Pro Phe Phe Arg Thr Leu Tyr Ile Ser Ile Ile Leu Val Leu Gly
        115                 120                 125

Leu Ile Ala Ile Tyr Val Ser Leu Ser Asp Lys Phe Val Ser Pro Lys
    130                 135                 140

Phe Arg Pro Leu Arg Ala Gly Phe Phe Val Leu Leu Gly Cys Ser Gly
145                 150                 155                 160

Val Ile Pro Leu Leu His Ala Leu Ile Leu Phe Gly Gly His Glu Asn
                165                 170                 175

Leu Val Arg Ile Ala Leu Pro Trp Val Leu Leu Met Ala Val Leu Tyr
            180                 185                 190

Ile Val Gly Ala Val Phe Tyr Gly Thr Arg Ile Pro Glu Arg Phe Phe
        195                 200                 205

Pro Gly Lys Phe Asp Ile Trp Gly His Ser His Gln Leu Phe His Leu
    210                 215                 220

Leu Val Val Leu Ala Ala Phe Tyr His Tyr Arg Ala Gly Leu
225                 230                 235


<210> 125
<211> 1001
<212> PRT
<213> Homo sapiens

<400> 125
Lys Ala Gly Pro Ala Gly Ser Ser Trp Thr Val Lys Leu Gly Thr Trp
1               5                   10                  15

Gly Pro Glu Ala Gly Pro Ser Leu Thr Glu Leu Cys Pro Leu Leu Ser
            20                  25                  30

Glu Pro His Gln Pro His Leu Met Leu Glu Gly Gly Ser Leu Ser Ser
        35                  40                  45

Ala Glu Tyr Glu Ala Arg Val Arg Ala Arg Arg Asp Phe Gln Arg Leu
    50                  55                  60

Gln Arg Arg Asp Ser Asp Gly Asp Arg Gln Val Trp Phe Leu Gln Arg
65                  70                  75                  80

Arg Phe His Lys Asp Ile Leu Leu Asn Pro Val Leu Ala Leu Ser Phe
                85                  90                  95

Cys Pro Asp Leu Ser Ser Lys Pro Gly His Leu Gly Thr Ala Thr Arg
            100                 105                 110

Glu Leu Leu Phe Leu Leu Asp Ser Ser Ser Val Ala His Lys Asp Ala
        115                 120                 125

Ile Val Leu Ala Val Lys Ser Leu Pro Pro Gln Thr Leu Ile Asn Leu
    130                 135                 140

Ala Val Phe Gly Thr Leu Val Gln Pro Leu Phe Pro Glu Ser Arg Pro
145                 150                 155                 160

Cys Ser Asp Asp Ala Val Gln Leu Ile Cys Glu Ser Ile Glu Thr Leu
                165                 170                 175

Gln Val Pro Ser Gly Pro Pro Asp Val Leu Ala Ala Leu Asp Trp Ala
            180                 185                 190

Val Gly Gln Pro Gln His Arg Ala Tyr Pro Arg Gln Leu Phe Leu Leu
        195                 200                 205

Thr Ala Ala Ser Pro Met Ala Ala Thr Thr His Arg Thr Leu Glu Leu
    210                 215                 220

Met Arg Trp His Arg Gly Thr Ala Arg Cys Phe Ser Phe Gly Leu Gly
225                 230                 235                 240

Pro Thr Cys His Gln Leu Leu Gln Gly Leu Ser Ala Leu Ser Arg Gly
                245                 250                 255

Gln Ala Tyr Phe Leu Arg Pro Gly Gln Arg Leu Gln Pro Met Leu Val
            260                 265                 270

Gln Ala Leu Arg Lys Ala Leu Glu Pro Ala Leu Ser Asp Ile Ser Val

        275                 280                 285

Asp Trp Phe Val Pro Asp Thr Val Glu Ala Leu Leu Thr Pro Arg Glu
    290                 295                 300

Ile Pro Ala Leu Tyr Pro Gly Asp Gln Leu Leu Gly Tyr Cys Ser Leu
305                 310                 315                 320

Phe Arg Val Asp Gly Phe Arg Ser Arg Pro Pro Gly Gly Gln Glu Pro
                325                 330                 335

Gly Trp Gln Ser Ser Gly Gly Ser Val Phe Pro Ser Pro Glu Glu Ala
            340                 345                 350

Pro Ser Ala Ala Ser Pro Gly Thr Glu Pro Thr Gly Thr Ser Glu Pro
        355                 360                 365

Leu Gly Thr Gly Thr Val Ser Ala Glu Leu Ser Ser Pro Trp Ala Ala
    370                 375                 380

Arg Asp Ser Glu Gln Ser Thr Asp Ala Leu Thr Asp Pro Val Thr Asp
385                 390                 395                 400

Pro Gly Pro Asn Pro Ser Asp Thr Ala Ile Trp Arg Arg Ile Phe Gln
                405                 410                 415

Ser Ser Tyr Ile Arg Glu Gln Tyr Val Leu Thr His Cys Ser Ala Ser
            420                 425                 430

Pro Glu Pro Gly Pro Gly Ser Thr Gly Ser Ser Glu Ser Pro Gly Ser
        435                 440                 445

Gln Gly Pro Gly Ser Pro Glu Gly Ser Ala Pro Leu Glu Pro Pro Ser
    450                 455                 460

Gln Gln Gly Cys Arg Ser Leu Ala Trp Gly Glu Pro Ala Gly Ser Arg
465                 470                 475                 480

Ser Cys Pro Leu Pro Ala Pro Thr Pro Ala Pro Phe Lys Val Gly Ala
                485                 490                 495

Leu Ser Thr Glu Val Leu Gly Arg Gln His Arg Ala Ala Leu Ala Gly
            500                 505                 510

Arg Ser Leu Ser Ser Pro Pro Gly Arg Ala Asn Gln Val Pro Gly Arg
        515                 520                 525

Pro Arg Lys Pro Ser Leu Gly Ala Ile Leu Asp Gly Pro Ser Pro Glu
    530                 535                 540

Pro Gly Gln Gln Leu Gly Gln Gly Leu Asp Asp Ser Gly Asn Leu Leu
545                 550                 555                 560

Ser Pro Ala Pro Met Asp Trp Asp Met Leu Met Glu Pro Pro Phe Leu
                565                 570                 575

Phe Thr Ala Val Pro Pro Ser Gly Glu Leu Ala Pro Pro Ala Val Pro
            580                 585                 590

Pro Gln Ala Pro Arg Cys His Val Val Ile Arg Gly Leu Cys Gly Glu
        595                 600                 605

Gln Pro Met Cys Trp Glu Val Gly Val Gly Leu Glu Thr Leu Trp Gly
    610                 615                 620

Pro Gly Asp Gly Ser Gln Pro Pro Ser Pro Pro Val Arg Glu Ala Ala
625                 630                 635                 640

Trp Asp Gln Ala Leu His Arg Leu Thr Ala Ala Ser Val Val Arg Asp

                645                 650                 655

Asn Glu Gln Leu Ala Leu Arg Gly Gly Ala Glu Thr Thr Ala Asp Arg
            660                 665                 670

Gly His Ala Arg Arg Cys Trp Leu Arg Ala Leu Gln Thr Ser Lys Val
        675                 680                 685

Ser Ser Ala Pro Ser Cys Phe Thr Cys Pro Val Ala Val Asp Ala Thr
    690                 695                 700

Thr Arg Glu Val Leu Pro Gly Ala Leu Gln Val Cys Ser Ser Glu Pro
705                 710                 715                 720

Ala Glu Pro Pro Gly Thr Pro Pro Ala Ser His Ser His Leu Asp Ala
                725                 730                 735

Ala Pro Leu Pro Thr Val Val Tyr Ser Lys Gly Leu Gln Arg Gly Ser
            740                 745                 750

Pro Ala Gly Ala Trp Asp Ser Asp Gln Asn Gly Asn Ser Lys Arg Ala
        755                 760                 765

Leu Gly Asp Pro Ala Thr Pro Thr Glu Gly Pro Arg Arg Pro Pro Pro
    770                 775                 780

Arg Pro Pro Cys Arg Leu Ser Met Gly Arg Arg His Lys Leu Cys Ser
785                 790                 795                 800

Pro Asp Pro Gly Gln Ala Asn Asn Ser Glu Gly Ser Asp His Asp Tyr
                805                 810                 815

Leu Pro Leu Val Arg Leu Gln Glu Ala Pro Gly Ser Phe Arg Leu Asp
            820                 825                 830

Ala Pro Phe Cys Ala Ala Val Arg Ile Ser Gln Glu Arg Leu Cys Arg
        835                 840                 845

Ala Ser Pro Phe Ala Val His Arg Ala Ser Leu Ser Pro Thr Ser Ala
    850                 855                 860

Ser Leu Pro Trp Ala Leu Leu Gly Pro Gly Val Gly Gln Gly Asp Ser
865                 870                 875                 880

Ala Thr Ala Ser Cys Ser Pro Ser Pro Ser Ser Gly Ser Glu Gly Pro
                885                 890                 895

Gly Gln Val Asp Ser Gly Arg Gly Ser Asp Thr Glu Ala Ser Glu Gly
            900                 905                 910

Ala Glu Gly Leu Gly Gly Thr Asp Leu Arg Gly Arg Thr Trp Ala Thr
        915                 920                 925

Ala Val Ala Leu Ala Trp Leu Glu His Arg Cys Ala Ala Ala Phe Asp
    930                 935                 940

Glu Trp Glu Leu Thr Ala Ala Lys Ala Asp Cys Trp Leu Arg Ala Gln
945                 950                 955                 960

His Leu Pro Asp Gly Leu Asp Leu Ala Ala Leu Lys Ala Ala Ala Arg
                965                 970                 975

Gly Leu Phe Leu Leu Leu Arg His Trp Asp Gln Asn Leu Gln Leu His
            980                 985                 990

Leu Leu Cys Tyr Ser Pro Ala Asn Val
        995                 1000

<210> 126
<211> 803
<212> PRT
<213> Macaca fascicularis

<400> 126
Met Gly Gln Pro Gln His Arg Ala Tyr Pro Arg Gln Leu Phe Leu Leu
1               5                   10                  15

Thr Ala Ala Ser Pro Met Ala Ala Thr Thr His Arg Thr Leu Glu Leu
            20                  25                  30

Met Arg Trp His Arg Gly Thr Ala Arg Cys Phe Ser Phe Gly Leu Gly
        35                  40                  45

Pro Thr Cys His Gln Leu Leu Gln Gly Leu Ser Ala Leu Ser Arg Gly
    50                  55                  60

Gln Ala Tyr Phe Leu Arg Pro Gly Gln Arg Leu Gln Pro Met Leu Val
65                  70                  75                  80

Gln Ala Leu Arg Lys Ala Leu Glu Pro Ala Leu Ser Asp Ile Ser Val
                85                  90                  95

Asp Trp Phe Val Pro Asp Thr Val Glu Ala Leu Leu Thr Pro Arg Glu
            100                 105                 110

Ile Pro Ala Leu Tyr Pro Gly Asp Gln Leu Leu Gly Tyr Cys Ser Leu
        115                 120                 125

Phe Arg Val Asp Gly Phe Arg Pro Arg Pro Pro Gly Gly Gln Glu Pro
    130                 135                 140

Gly Trp Gln Ser Leu Gly Gly Ser Val Phe Pro Ser Pro Glu Glu Ala
145                 150                 155                 160

Pro Ser Ala Val Ser Pro Gly Thr Glu Pro Thr Gly Thr Ser Glu Pro
                165                 170                 175

Leu Gly Thr Gly Thr Val Ser Ala Glu Leu Ser Ser Pro Trp Ala Ala
            180                 185                 190

Gly Asp Leu Glu Arg Thr Gly Thr Asp Ala Leu Thr Asp Pro Val Thr
        195                 200                 205

Asp Pro Gly Pro Asn Pro Ser Ser Asp Thr Ala Ile Trp Arg Arg Ile
    210                 215                 220

Phe Gln Ser Ser Tyr Ile Arg Glu Gln Tyr Val Leu Thr His Cys Ser
225                 230                 235                 240

Ala Ser Pro Glu Pro Gly Pro Cys Ser Thr Gly Ser Ser Glu Ser Pro
                245                 250                 255

Gly Ser Gln Gly Pro Gly Ser Pro Glu Gly Ser Ala Pro Leu Glu Pro
            260                 265                 270

Pro Ser Gln Gln Gly Cys Arg Ser Leu Ala Trp Gly Glu Pro Ala Gly
        275                 280                 285

Ser Arg Ser Cys Pro Leu Pro Ala Pro Thr Pro Ala Pro Phe Lys Val
    290                 295                 300

Gly Ala Leu Ser Thr Glu Val Leu Gly Arg Gln His Arg Ala Ala Leu
305                 310                 315                 320

Ala Gly Arg Ser Leu Ser Ser Pro Pro Gly Arg Ala Asn Gln Val Pro
                325                 330                 335

Gly Arg Pro Arg Lys Pro Ser Leu Gly Ala Ile Leu Asp Gly Pro Ser
            340                 345                 350

Pro Glu Pro Gly Gln Gln Leu Gly Gln Gly Leu Asp Asp Ser Gly Ser
        355                 360                 365

Leu Leu Ser Pro Ala Pro Met Asp Trp Asp Met Leu Met Glu Pro Pro
    370                 375                 380

Phe Leu Phe Thr Ala Val Pro Pro Ser Gly Glu Pro Ala Pro Pro Ala
385                 390                 395                 400

Val Pro Pro Gln Ala Pro Arg Cys His Val Val Ile Arg Gly Leu Cys
                405                 410                 415

Gly Glu Gln Pro Met Cys Trp Glu Val Gly Val Gly Leu Glu Thr Leu
            420                 425                 430

Trp Gly Pro Gly Asp Gly Ser Gln Pro Pro Ser Pro Pro Val Arg Glu
        435                 440                 445

Ala Ala Trp Asp Gln Ala Leu His Arg Leu Thr Ala Ala Ser Val Val
    450                 455                 460

Arg Asp Asn Glu Gln Leu Ala Leu Arg Gly Gly Ala Glu Thr Thr Ala
465                 470                 475                 480

Asp Arg Gly His Ala Arg Arg Cys Trp Leu Arg Ala Leu Gln Thr Ser
                485                 490                 495

Lys Val Ser Ser Ala Pro Ser Cys Phe Thr Cys Pro Val Ala Val Asp
            500                 505                 510

Ala Thr Thr Arg Glu Val Leu Pro Gly Val Leu Gln Val Cys Ser Ser
        515                 520                 525

Glu Pro Ala Glu Pro Pro Gly Thr Pro Pro Ala Ala His Ser Arg Leu
    530                 535                 540

Asp Ala Ala Pro Leu Pro Thr Val Val Tyr Ser Lys Gly Ala Trp Asp
545                 550                 555                 560

Ser Asp Arg Asn Gly Asn Ser Lys Cys Ala Leu Gly Asp Ala Ala Thr
                565                 570                 575

Pro Met Glu Gly Pro Arg Cys Pro Pro Pro Arg Ser Pro Ser Arg Leu
            580                 585                 590

Ser Leu Gly Arg Arg His Lys Leu Cys Arg Pro Asp Leu Gly Gln Ala
        595                 600                 605

Asn Asn Ser Glu Gly Ile Asp His Asp Tyr Leu Pro Leu Val Arg Leu
    610                 615                 620

Gln Glu Ala Pro Gly Ser Phe Arg Leu Asp Ala Pro Phe Cys Ala Ala
625                 630                 635                 640

Val Arg Ile Ser Gln Glu Arg Leu Cys Arg Ala Ser Pro Phe Ala Val
                645                 650                 655

His Arg Ala Ser Leu Ser Pro Thr Ser Ala Ser Leu Pro Trp Ala Leu
            660                 665                 670

Leu Gly Pro Gly Val Gly Gln Gly Asp Ser Ala Thr Ala Ser Cys Ser
        675                 680                 685

Pro Ser Pro Ser Ser Gly Ser Glu Gly Pro Gly Gln Val Asp Ser Gly
    690                 695                 700

Arg Gly Ser Asp Thr Glu Ala Ser Glu Gly Ala Glu Gly Leu Gly Gly
705                 710                 715                 720

Thr Asp Leu Arg Gly Arg Thr Trp Ala Thr Ala Val Ala Leu Ala Trp
                725                 730                 735

Leu Glu His Arg Cys Ala Ala Ala Phe Gly Glu Trp Glu Leu Thr Ala
            740                 745                 750

Ala Lys Ala Asp Cys Trp Leu Arg Ala Gln His Leu Pro Asp Gly Leu
        755                 760                 765

Asp Leu Ala Ala Leu Lys Ala Ala Ala Arg Gly Leu Phe Leu Leu Leu
    770                 775                 780

Arg His Trp Asp Gln Asn Leu Gln Leu His Leu Leu Cys Tyr Ser Pro
785                 790                 795                 800

Ala Asn Val

<210> 127
<211> 620
<212> PRT
<213> Homo sapiens

<400> 127
Met Gly Cys Gln Gly Leu Gly Ala Asp Thr Asp Ala Leu Thr Asp Pro
  1               5                  10                  15

Val Thr Asp Pro Gly Pro Asn Pro Ser Asp Thr Ala Ile Trp Arg Arg
             20                  25                  30

Ile Phe Gln Ser Ser Tyr Ile Arg Glu Gln Tyr Val Leu Thr His Cys
         35                  40                  45

Ser Ala Ser Pro Glu Pro Gly Pro Gly Ser Thr Gly Ser Ser Glu Ser
     50                  55                  60

Pro Gly Ser Gln Gly Pro Gly Ser Pro Glu Gly Ser Ala Pro Leu Glu
 65                  70                  75                  80

Pro Pro Ser Gln Gln Gly Cys Arg Ser Leu Ala Trp Gly Glu Pro Ala
                 85                  90                  95

Gly Ser Arg Ser Cys Pro Leu Pro Ala Pro Thr Pro Ala Pro Phe Lys
            100                 105                 110

Val Gly Ala Leu Ser Thr Glu Val Leu Gly Arg Gln His Arg Ala Ala
        115                 120                 125

Leu Ala Gly Arg Ser Leu Ser Ser Pro Pro Gly Arg Ala Asn Gln Val
    130                 135                 140

Pro Gly Arg Pro Arg Lys Pro Ser Leu Gly Ala Ile Leu Asp Gly Pro
145                 150                 155                 160

Ser Pro Glu Pro Gly Gln Gln Leu Gly Gln Gly Leu Asp Asp Ser Gly
                165                 170                 175

Asn Leu Leu Ser Pro Ala Pro Met Asp Trp Asp Met Leu Met Glu Pro
            180                 185                 190

Pro Phe Leu Phe Thr Ala Val Pro Pro Ser Gly Glu Leu Ala Pro Pro
        195                 200                 205

Ala Val Pro Pro Gln Ala Pro Arg Cys His Val Val Ile Arg Gly Leu
    210                 215                 220

Cys Gly Glu Gln Pro Met Cys Trp Glu Val Gly Val Gly Leu Glu Thr
225                 230                 235                 240

Leu Trp Gly Pro Gly Asp Gly Ser Gln Pro Pro Ser Pro Pro Val Arg
                245                 250                 255

Glu Ala Ala Trp Asp Gln Ala Leu His Arg Leu Thr Ala Ala Ser Val
            260                 265                 270

Val Arg Asp Asn Glu Gln Leu Ala Leu Arg Gly Gly Ala Glu Thr Thr
        275                 280                 285

Ala Asp Arg Gly His Ala Arg Arg Cys Trp Leu Arg Ala Leu Gln Thr
    290                 295                 300

Ser Lys Val Ser Ser Ala Pro Ser Cys Phe Thr Cys Pro Val Ala Val
305                 310                 315                 320

Asp Ala Thr Thr Arg Glu Val Leu Pro Gly Ala Leu Gln Val Cys Ser
                325                 330                 335

Ser Glu Pro Ala Glu Pro Pro Gly Thr Pro Pro Ala Ser His Ser His
            340                 345                 350

Leu Asp Ala Ala Pro Leu Pro Thr Val Val Tyr Ser Lys Gly Leu Gln
        355                 360                 365

Arg Gly Ser Pro Ala Gly Ala Trp Asp Ser Asp Gln Asn Gly Asn Ser
    370                 375                 380

Lys Arg Ala Leu Gly Asp Pro Ala Thr Pro Thr Glu Gly Pro Arg Arg
385                 390                 395                 400

Pro Pro Pro Arg Pro Pro Cys Arg Leu Ser Met Gly Arg Arg His Lys
                405                 410                 415

Leu Cys Ser Pro Asp Pro Gly Gln Ala Asn Asn Ser Glu Gly Ser Asp
            420                 425                 430

His Asp Tyr Leu Pro Leu Val Arg Leu Gln Glu Ala Pro Gly Ser Phe
        435                 440                 445

Arg Leu Asp Ala Pro Phe Cys Ala Ala Val Arg Ile Ser Gln Glu Arg
    450                 455                 460

Leu Cys Arg Ala Ser Pro Phe Ala Val His Arg Ala Ser Leu Ser Pro
465                 470                 475                 480

Thr Ser Ala Ser Leu Pro Trp Ala Leu Leu Gly Pro Gly Val Gly Gln
                485                 490                 495

Gly Asp Ser Ala Thr Ala Ser Cys Ser Pro Ser Pro Ser Ser Gly Ser
            500                 505                 510

Glu Gly Pro Gly Gln Val Asp Ser Gly Arg Gly Ser Asp Thr Glu Ala
        515                 520                 525

Ser Glu Gly Ala Glu Gly Leu Gly Gly Thr Asp Leu Arg Gly Arg Thr
    530                 535                 540

Trp Ala Thr Ala Val Ala Leu Ala Trp Leu Glu His Arg Cys Ala Ala
545                 550                 555                 560

Ala Phe Asp Glu Trp Glu Leu Thr Ala Ala Lys Ala Asp Cys Trp Leu
                565                 570                 575

Arg Ala Gln His Leu Pro Asp Gly Leu Asp Leu Ala Ala Leu Lys Ala
            580                 585                 590

Ala Ala Arg Gly Leu Phe Leu Leu Leu Arg His Trp Asp Gln Asn Leu
        595                 600                 605

Gln Leu His Leu Leu Cys Tyr Ser Pro Ala Asn Val
    610                 615                 620

<210> 128
<211> 260
<212> PRT
<213> Macaca fascicularis

<400> 128
Met Ile Pro Thr Ala Gly Leu Gln Gly Gly Ser Pro Ala Gly Ala Trp
1               5                   10                  15

Asp Ser Asp Arg Asn Gly Asn Ser Lys Cys Ala Leu Gly Asp Ala Ala
            20                  25                  30

Thr Pro Met Glu Gly Pro Arg Cys Pro Pro Pro Arg Ser Pro Ser Arg
        35                  40                  45

Leu Ser Leu Gly Arg Arg His Lys Leu Cys Arg Pro Asp Leu Gly Gln
    50                  55                  60

Ala Asn Asn Ser Glu Gly Ile Asp His Asp Tyr Leu Pro Leu Val Arg
65                  70                  75                  80

Leu Gln Glu Ala Pro Gly Ser Phe Arg Leu Asp Ala Pro Phe Cys Ala
                85                  90                  95

Ala Val Arg Ile Ser Gln Glu Arg Leu Cys Arg Ala Ser Pro Phe Ala
            100                 105                 110

Val His Arg Ala Ser Leu Ser Pro Thr Ser Ala Ser Leu Pro Trp Ala
        115                 120                 125

Leu Leu Gly Pro Gly Val Gly Gln Gly Asp Ser Ala Thr Ala Phe Cys
    130                 135                 140

Ser Pro Ser Pro Ser Ser Gly Ser Glu Gly Pro Gly Gln Val Asp Ser
145                 150                 155                 160

Gly Arg Gly Ser Asp Thr Glu Ala Ser Glu Gly Ala Glu Gly Leu Gly
                165                 170                 175

Gly Thr Asp Leu Arg Gly Arg Thr Trp Ala Thr Ala Val Ala Leu Ala
            180                 185                 190

Trp Leu Glu His Arg Cys Ala Ala Ala Phe Gly Glu Trp Glu Leu Thr
        195                 200                 205

Ala Ala Lys Ala Asp Cys Trp Leu Arg Ala Gln His Leu Pro Asp Gly
    210                 215                 220

Leu Asp Leu Ala Ala Leu Lys Ala Ala Ala Arg Gly Leu Phe Leu Leu
225                 230                 235                 240

Leu Arg His Trp Asp Gln Asn Leu Gln Leu His Leu Leu Cys Tyr Ser
                245                 250                 255

Pro Ala Asn Val
            260

<210> 129
<211> 1209
<212> PRT

<213> Mus musculus

<400> 129
Met Pro Gly Leu Leu Asn Cys Leu Thr Gly Ala Ala Leu Pro Leu Met
1               5                   10                  15

Glu Ser Asp Val Thr Ser Tyr Val Ser Gly Tyr Ala Leu Gly Leu Thr
            20                  25                  30

Ala Ser Leu Thr Tyr Gly Asn Leu Glu Ala Gln Pro Phe Gln Gly Leu
        35                  40                  45

Phe Val Tyr Pro Ile Asp Glu Tyr Ser Thr Val Val Gly Phe Glu Ala
    50                  55                  60

Val Ile Ala Asp Arg Val Val Thr Ile Gln Leu Arg Asp Lys Ala Lys
65                  70                  75                  80

Leu Asp Arg Ser His Leu Asp Ile Gln Pro Ala Thr Val Thr Gly Asn
                85                  90                  95

Phe Pro Glu Glu Glu Ser Pro Ile Ala Pro Gly Lys Val Thr Leu Asp
            100                 105                 110

Glu Asp Leu Glu Arg Val Leu Phe Val Val Asn Leu Gly Thr Ile Ala
        115                 120                 125

Pro Met Glu Asn Val Thr Val Phe Ile Ser Thr Ser Ser Glu Leu Pro
    130                 135                 140

Thr Leu Pro Ser Gly Ala Val Arg Val Leu Leu Pro Ala Ile Cys Ala
145                 150                 155                 160

Pro Thr Val Pro Pro Ser Cys Thr His Arg Phe Gly Ser Ser Ser Pro
                165                 170                 175

Gln Pro Gln Gly Lys Asp Pro His Cys Phe Gly Thr Gln Thr Lys Asp
            180                 185                 190

Ser Tyr Asn Arg Leu Cys Leu Ala Thr Leu Leu Asp Thr Lys Val Thr
        195                 200                 205

Asn Pro Met Glu Tyr Glu Phe Lys Phe Gln Leu Glu Ile Arg Gly Pro
    210                 215                 220

Cys Leu Leu Ala Gly Val Glu Ser Pro Thr His Glu Ile Arg Ala Asp
225                 230                 235                 240

Ala Ala Pro Ser Ala His Ser Ala Lys Ser Ile Ile Ile Thr Leu Ala
                245                 250                 255

Lys Lys His Thr Phe Asp Arg Pro Val Glu Ile Leu Leu His Pro Ser
            260                 265                 270

Glu Pro His Met Pro His Val Leu Val Glu Lys Gly Asp Met Thr Leu
        275                 280                 285

Gly Glu Tyr Asp Gln His Leu Lys Gly Lys Ala Asp Phe Ile Arg Gly
    290                 295                 300

Thr Lys Lys Asp Asn Ser Ala Glu Arg Lys Thr Glu Val Ile Arg Lys
305                 310                 315                 320

Arg Leu His Lys Asp Ile Pro His His Ser Val Ile Met Leu Asn Phe
                325                 330                 335

Cys Pro Asp Leu Gln Ser Val Gln Pro Asn Pro Arg Lys Ala His Gly
            340                 345                 350

Glu Phe Ile Phe Leu Ile Asp Arg Ser Asn Ser Met Ser Lys Thr Asn
        355                 360                 365

Ile Gln Cys Ile Lys Glu Ala Met Leu Val Ala Leu Lys Ser Leu Met
    370                 375                 380

Pro Ala Cys Phe Phe Asn Ile Ile Gly Phe Gly Ser Thr Phe Lys Ala
385                 390                 395                 400

Val Phe Ala Ser Ser Arg Ile Tyr Asn Glu Glu Asn Leu Thr Met Ala
                405                 410                 415

Cys Asp Cys Ile Gln Arg Met Gln Ala Asp Met Gly Gly Thr Asn Met
            420                 425                 430

Leu Ser Pro Leu Lys Trp Val Leu Arg Gln Pro Leu Arg Arg Gly His
        435                 440                 445

Pro Arg Leu Leu Phe Leu Ile Thr Asp Gly Ser Val Asn Asn Thr Gly
    450                 455                 460

Lys Val Leu Glu Leu Val Arg Asn His Ala Ser Ser Thr Arg Cys Tyr
465                 470                 475                 480

Ser Phe Gly Ile Gly Pro Thr Val Cys Tyr Arg Leu Val Lys Gly Leu
                485                 490                 495

Ala Ser Val Ser Lys Gly Ser Ala Glu Phe Leu Met Glu Gly Glu Arg
            500                 505                 510

Leu Gln Pro Lys Met Val Lys Ser Leu Lys Lys Ala Met Ala Pro Val
        515                 520                 525

Leu Ser Asp Val Thr Val Glu Trp Val Leu Pro Glu Thr Thr Glu Ala
    530                 535                 540

Leu Ile Ser Pro Val Ser Thr Ser Ser Leu Phe Pro Gly Glu Arg Leu
545                 550                 555                 560

Met Gly Tyr Gly Ile Val Cys Asp Ala Ser Leu Tyr Ile Ser Asn Ser
                565                 570                 575

Arg Ser Asp Lys Arg Arg Lys Tyr Gly Met Leu His Thr Gln Glu Ser
            580                 585                 590

Ser Ser Ser Val Phe Tyr Pro Ser Gln Asp Glu Gly Leu Ser Pro Gly
        595                 600                 605

Ser Gly Asn Cys Ala Lys Asn Val Asn Gln Gly Gln Thr Lys Asp Ala
    610                 615                 620

His Pro Cys Asn Gly Asp Ser Pro Thr His His Gly Leu Asp Val Ser
625                 630                 635                 640

Arg Arg Arg Arg Ala Tyr Ser Thr Asn Gln Ile Ser Ser His Lys Thr
                645                 650                 655

Cys Pro Arg Ala Thr Thr Ala Ser Asp Pro Thr Gly Thr Ala Arg Arg
            660                 665                 670

Tyr Pro Leu Arg Lys Ala Lys Val Gln Asp Leu Ala Ser Glu Ser Asp
        675                 680                 685

Trp Glu Ser Gln Lys Trp Gln Thr Asp Leu Gln Thr Leu Leu Asn Glu
    690                 695                 700

Gly His Asn Leu Ser Gln Gly Pro Lys Leu His Gly Pro Gly Ala Arg
705                 710                 715                 720

Arg Pro Ser Leu Leu Pro Gln Gly Cys Gln Leu Met Arg Phe Phe Asp
                725                 730                 735

Gln Lys Pro Gln Ala Trp Gly Pro Val Arg Glu Leu Asp Cys Gly Ala
            740                 745                 750

Ser Arg Thr Ser Ala Pro Asn Ser Gln Ser Ser Glu Asp Leu Ala Ile
        755                 760                 765

Glu Pro Ala His Cys Pro Ser Thr Phe Glu Arg Glu Thr Ser Leu Asp
    770                 775                 780

Leu Glu Pro Met Ala Glu Ser Glu Glu Gln Ala Asn Pro Cys Arg Thr
785                 790                 795                 800

Ala Thr Pro Ser Pro Val Val Gly Lys Ala Leu Val Lys Gly Leu Cys
                805                 810                 815

Ala Asn Gln Arg Met Gln Trp Glu Val Ser Phe Glu Leu Glu Pro Pro
            820                 825                 830

Ala Leu Lys Arg Gly Asp Thr Gln Asn Ala Asp Met Trp Ser Glu Thr
        835                 840                 845

Phe His His Leu Ala Ala Arg Ala Ile Ile Arg Glu Phe Glu His Leu
    850                 855                 860

Ala Glu Arg Glu Asp Glu Ile Glu Leu Gly Ser Asn Arg Arg Tyr Gln
865                 870                 875                 880

Val Asn Ala Val His Thr Ser Lys Ala Cys Ser Val Ile Ser Lys Tyr
                885                 890                 895

Thr Ala Phe Val Pro Val Asp Ile Asn Lys Arg Gln Tyr Leu Pro Thr
            900                 905                 910

Val Val Lys Tyr Pro Asn Ser Gly Ala Met Leu Ser Phe Arg Asn Leu
        915                 920                 925

Thr Arg Gln Trp Gly Gly Ser Ser Ala Gly Leu Gly Arg Pro Gln Ser
    930                 935                 940

Met Leu Arg Glu His Ser Ser Ala Ala Gly Asp Ser Lys Phe Gln Thr
945                 950                 955                 960

Leu Ala Leu Gln Asp Ser Pro Thr Ser Thr Phe Asn Lys Thr Pro Ser
                965                 970                 975

Pro Gly His Glu Lys Gln Thr Thr Ala Glu Gly Pro Pro Gln Asn Leu
            980                 985                 990

Ser Ala Ser Ala Pro Ser Ser Met Lys Ala Thr Glu Thr Leu Phe Gly
        995                 1000                1005

Ser Lys Leu Asn Leu Asn Lys Ser Arg Leu Leu Thr Arg Ala Thr Lys
    1010                1015                1020

Gly Phe Leu Ser Lys Ser Leu Pro Lys Ala Ser Glu Ala Thr Pro Gly
1025                1030                1035                1040

Ser Gln Ser Ser Asp Tyr Ile Pro Leu Val Ser Leu Gln Leu Ala Ser
                1045                1050                1055

Gly Ala Phe Leu Leu Asn Glu Ala Phe Cys Thr Thr Ile Gln Ile Pro
            1060                1065                1070

Met Glu Lys Leu Lys Trp Thr Ser Pro Phe Ser Cys Leu Arg Met Ser
        1075                1080                1085

Leu Val Thr Arg Arg Gln Asp Leu Lys Thr Gln Ser Pro Gln Asp Cys
    1090                1095                1100

Thr Ser Leu Ser Ser Ser Pro Pro Ser Cys Asp Gly Ile Ser Leu Lys
1105                1110                1115                1120

Ser Glu Glu Ser Ser Asp Gln Glu Ser Asn Ala Met Leu Glu His Met
                1125                1130                1135

Gly Lys Leu Trp Ala Thr Val Val Ala Leu Ala Trp Leu Glu His Ser
            1140                1145                1150

Ser Ala Asn Tyr Ile Ile Glu Trp Glu Leu Val Ala Ala Lys Ala Ser
        1155                1160                1165

Ser Trp Val Glu Lys Gln Lys Val Pro Glu Gly Arg Thr Leu Ser Thr
    1170                1175                1180

Leu Lys Asn Thr Ala Arg Gln Leu Phe Val Leu Leu Arg His Trp Asp
1185                1190                1195                1200

Glu Lys Leu Glu Phe Asn Met Leu Cys
                1205

<210> 130
<211> 180
<212> PRT
<213> Homo sapiens

<400> 130
Pro Leu Asp Val Val Phe Leu Leu Asp Gly Ser Gly Ser Met Gly Gly
1               5                   10                  15

Asn Arg Phe Glu Leu Ala Lys Glu Phe Val Leu Lys Leu Val Glu Gln
            20                  25                  30

Leu Asp Ile Gly Pro Asp Gly Asp Arg Val Gly Leu Val Thr Phe Ser
        35                  40                  45

Ser Asp Ala Arg Val Leu Phe Pro Leu Asn Asp Ser Gln Ser Lys Asp
    50                  55                  60

Ala Leu Leu Glu Ala Leu Ala Ser Leu Ser Tyr Ser Leu Gly Gly Gly
65                  70                  75                  80

Thr Asn Leu Gly Ala Ala Leu Glu Tyr Ala Leu Glu Asn Leu Phe Ser
                85                  90                  95

Glu Ser Ala Gly Ser Arg Arg Gly Ala Pro Lys Val Leu Ile Leu Ile
            100                 105                 110

Thr Asp Gly Glu Ser Asn Asp Gly Gly Glu Asp Ile Leu Lys Ala Ala
        115                 120                 125

Lys Glu Leu Lys Arg Ser Gly Val Lys Val Phe Val Val Gly Val Gly
    130                 135                 140

Asn Asp Val Asp Glu Glu Glu Leu Lys Lys Leu Ala Ser Ala Pro Gly
145                 150                 155                 160

Gly Val Phe Val Val Glu Asp Leu Pro Ser Leu Leu Asp Leu Leu Ile
                165                 170                 175

Asp Leu Leu Leu
            180

<210> 131

<211> 1019
<212> PRT
<213> Homo sapiens

<400> 131
Met Arg Tyr Arg Leu Ala Trp Leu Leu His Pro Ala Leu Pro Ser Thr
1               5                   10                  15

Phe Arg Ser Val Leu Gly Ala Arg Leu Pro Pro Pro Glu Arg Leu Cys
            20                  25                  30

Gly Phe Gln Lys Lys Thr Tyr Ser Lys Met Asn Asn Pro Ala Ile Lys
        35                  40                  45

Arg Ile Gly Asn His Ile Thr Lys Ser Pro Glu Asp Lys Arg Glu Tyr
    50                  55                  60

Arg Gly Leu Glu Leu Ala Asn Gly Ile Lys Val Leu Leu Met Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Ala Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Phe Asp Glu
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Gln Glu
225                 230                 235                 240

Leu Leu Lys Phe His Ser Ala Tyr Tyr Ser Ser Asn Leu Met Ala Val
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Lys Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Lys Tyr Tyr Lys Ser Asn Pro Gly His Tyr Leu Gly His
                325                 330                 335

Leu Ile Gly His Glu Gly Pro Gly Ser Leu Leu Ser Glu Leu Lys Ser

            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Lys
385                 390                 395                 400

Leu Arg Ala Glu Gly Pro Gln Glu Trp Val Phe Gln Glu Cys Lys Asp
                405                 410                 415

Leu Asn Ala Val Ala Phe Arg Phe Lys Asp Lys Glu Arg Pro Arg Gly
            420                 425                 430

Tyr Thr Ser Lys Ile Ala Gly Ile Leu His Tyr Tyr Pro Leu Glu Glu
        435                 440                 445

Val Leu Thr Ala Glu Tyr Leu Leu Glu Glu Phe Arg Pro Asp Leu Ile
    450                 455                 460

Glu Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Glu Trp Tyr
                485                 490                 495

Gly Thr Gln Tyr Lys Gln Glu Ala Ile Pro Asp Glu Val Ile Lys Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Pro Leu Glu Lys Glu Ala
    530                 535                 540

Thr Pro Tyr Pro Ala Leu Ile Lys Asp Thr Val Met Ser Lys Leu Trp
545                 550                 555                 560

Phe Lys Gln Asp Asp Lys Lys Lys Lys Pro Lys Ala Cys Leu Asn Phe
                565                 570                 575

Glu Phe Phe Ser Pro Phe Ala Tyr Val Asp Pro Leu His Cys Asn Met
            580                 585                 590

Ala Tyr Leu Tyr Leu Glu Leu Leu Lys Asp Ser Leu Asn Glu Tyr Ala
        595                 600                 605

Tyr Ala Ala Glu Leu Ala Gly Leu Ser Tyr Asp Leu Gln Asn Thr Ile
    610                 615                 620

Tyr Gly Met Tyr Leu Ser Val Lys Gly Tyr Asn Asp Lys Gln Pro Ile
625                 630                 635                 640

Leu Leu Lys Lys Ile Ile Glu Lys Met Ala Thr Phe Glu Ile Asp Glu
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu

705                 710                 715                 720

Ser Arg Leu His Ile Glu Ala Leu Leu His Gly Asn Ile Thr Lys Gln
                725                 730                 735

Ala Ala Leu Gly Ile Met Gln Met Val Glu Asp Thr Leu Ile Glu His
            740                 745                 750

Ala His Thr Lys Pro Leu Leu Pro Ser Gln Leu Val Arg Tyr Arg Glu
        755                 760                 765

Val Gln Leu Pro Asp Arg Gly Trp Phe Val Tyr Gln Gln Arg Asn Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Ser Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ser Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Phe Asp Arg Asp Asn Thr Glu Val Ala Tyr Leu
        915                 920                 925

Lys Thr Leu Thr Lys Glu Asp Ile Ile Lys Phe Tyr Lys Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Cys Gln Asn
                965                 970                 975

Asp Ile Asn Leu Ser Gln Ala Pro Ala Leu Pro Gln Pro Glu Val Ile
            980                 985                 990

Gln Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015


<210> 132
<211> 1019
<212> PRT
<213> Rattus norvegicus

<400> 132
Met Arg Asn Gly Leu Val Trp Leu Leu His Pro Ala Leu Pro Ser Thr
1               5                   10                  15

Leu His Ser Ile Leu Gly Ala Arg Pro Pro Pro Val Lys Arg Leu Cys
            20                  25                  30

Gly Phe Pro Lys Gln Ile Tyr Ser Thr Met Asn Asn Pro Ala Ile Gln
        35                  40                  45

Arg Ile Glu Asp His Ile Val Lys Ser Pro Glu Asp Lys Arg Glu Tyr
    50                  55                  60

Arg Gly Leu Glu Leu Ala Asn Gly Ile Lys Val Leu Leu Ile Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Pro Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Phe Asp Ala
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Glu Glu
225                 230                 235                 240

Leu Leu Lys Phe His Ser Thr Tyr Tyr Ser Ser Asn Leu Met Ala Ile
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Lys Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Gln Tyr Tyr Lys Ser Asn Pro Gly His Tyr Leu Gly His
                325                 330                 335

Leu Ile Gly His Glu Gly Pro Gly Ser Leu Leu Ser Glu Leu Lys Ser
            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Lys
385                 390                 395                 400

Leu Arg Ala Glu Gly Pro Gln Glu Trp Val Phe Gln Glu Cys Lys Asp
                405                 410                 415

Leu Asn Ala Val Ala Phe Arg Phe Lys Asp Lys Glu Arg Pro Arg Gly
            420                 425                 430

Tyr Thr Ser Lys Ile Ala Gly Lys Leu His Tyr Tyr Pro Leu Asn Gly
        435                 440                 445

Val Leu Thr Ala Glu Tyr Leu Leu Glu Glu Phe Arg Pro Asp Leu Ile
    450                 455                 460

Asp Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Gln Trp Tyr
                485                 490                 495

Gly Thr Gln Tyr Lys Gln Glu Ala Ile Pro Glu Asp Val Ile Gln Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Ala Leu Glu Lys Asp Ala
    530                 535                 540

Thr Pro Tyr Pro Ala Leu Ile Lys Asp Thr Ala Met Ser Lys Leu Trp
545                 550                 555                 560

Phe Lys Gln Asp Asp Lys Phe Phe Leu Pro Lys Ala Cys Leu Asn Phe
                565                 570                 575

Glu Phe Phe Ser Pro Phe Ala Tyr Val Asp Pro Leu His Cys Asn Met
            580                 585                 590

Ala Tyr Leu Tyr Leu Glu Leu Leu Lys Asp Ser Leu Asn Glu Tyr Ala
        595                 600                 605

Tyr Ala Ala Glu Leu Ala Gly Leu Ser Tyr Asp Leu Gln Asn Thr Ile
    610                 615                 620

Tyr Gly Met Tyr Leu Ser Val Lys Gly Tyr Asn Asp Lys Gln Pro Ile
625                 630                 635                 640

Leu Leu Lys Lys Ile Thr Glu Lys Met Ala Thr Phe Glu Ile Asp Lys
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu
705                 710                 715                 720

Ser Arg Leu His Ile Glu Ala Leu Leu His Gly Asn Ile Thr Lys Gln
                725                 730                 735

Ala Ala Leu Gly Val Met Gln Met Val Glu Asp Thr Leu Ile Glu His
            740                 745                 750

Ala His Thr Lys Pro Leu Leu Pro Ser Gln Leu Val Arg Tyr Arg Glu
        755                 760                 765

Val Gln Leu Pro Asp Arg Gly Trp Phe Val Tyr Gln Arg Arg Asn Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Gly Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ala Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Tyr Asp Arg Asp Asn Ile Glu Val Ala Tyr Leu
        915                 920                 925

Lys Thr Leu Ser Lys Asp Asp Ile Ile Lys Phe Tyr Lys Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Ser Gln Asn
                965                 970                 975

Asp Ile Asn Leu Ser Glu Ala Pro Pro Leu Pro Gln Pro Glu Val Ile
            980                 985                 990

His Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015


<210> 133
<211> 1019
<212> PRT
<213> Mus musculus

<400> 133
Met Arg Asn Gly Leu Val Trp Leu Leu His Pro Ala Leu Pro Gly Thr
1               5                   10                  15

Leu Arg Ser Ile Leu Gly Ala Arg Pro Pro Pro Ala Lys Arg Leu Cys
            20                  25                  30

Gly Phe Pro Lys Gln Thr Tyr Ser Thr Met Ser Asn Pro Ala Ile Gln
        35                  40                  45

Arg Ile Glu Asp Gln Ile Val Lys Ser Pro Glu Asp Lys Arg Glu Tyr
    50                  55                  60

Arg Gly Leu Glu Leu Ala Asn Gly Ile Lys Val Leu Leu Ile Ser Asp
65                  70                  75                  80

Pro Thr Thr Asp Lys Ser Ser Ala Ala Leu Asp Val His Ile Gly Ser
                85                  90                  95

Leu Ser Asp Pro Pro Asn Ile Pro Gly Leu Ser His Phe Cys Glu His
            100                 105                 110

Met Leu Phe Leu Gly Thr Lys Lys Tyr Pro Lys Glu Asn Glu Tyr Ser
        115                 120                 125

Gln Phe Leu Ser Glu His Ala Gly Ser Ser Asn Ala Phe Thr Ser Gly
    130                 135                 140

Glu His Thr Asn Tyr Tyr Phe Asp Val Ser His Glu His Leu Glu Gly
145                 150                 155                 160

Ala Leu Asp Arg Phe Ala Gln Phe Phe Leu Cys Pro Leu Leu Asp Ala
                165                 170                 175

Ser Cys Lys Asp Arg Glu Val Asn Ala Val Asp Ser Glu His Glu Lys
            180                 185                 190

Asn Val Met Asn Asp Ala Trp Arg Leu Phe Gln Leu Glu Lys Ala Thr
        195                 200                 205

Gly Asn Pro Lys His Pro Phe Ser Lys Phe Gly Thr Gly Asn Lys Tyr
    210                 215                 220

Thr Leu Glu Thr Arg Pro Asn Gln Glu Gly Ile Asp Val Arg Glu Glu
225                 230                 235                 240

Leu Leu Lys Phe His Ser Thr Tyr Tyr Ser Ser Asn Leu Met Ala Ile
                245                 250                 255

Cys Val Leu Gly Arg Glu Ser Leu Asp Asp Leu Thr Asn Leu Val Val
            260                 265                 270

Lys Leu Phe Ser Glu Val Glu Asn Lys Asn Val Pro Leu Pro Glu Phe
        275                 280                 285

Pro Glu His Pro Phe Gln Glu Glu His Leu Arg Gln Leu Tyr Lys Ile
    290                 295                 300

Val Pro Ile Lys Asp Ile Arg Asn Leu Tyr Val Thr Phe Pro Ile Pro
305                 310                 315                 320

Asp Leu Gln Gln Tyr Tyr Lys Ser Asn Pro Gly Tyr Tyr Leu Gly His
                325                 330                 335

Leu Ile Gly His Glu Gly Pro Gly Ser Leu Leu Ser Glu Leu Lys Ser
            340                 345                 350

Lys Gly Trp Val Asn Thr Leu Val Gly Gly Gln Lys Glu Gly Ala Arg
        355                 360                 365

Gly Phe Met Phe Phe Ile Ile Asn Val Asp Leu Thr Glu Glu Gly Leu
    370                 375                 380

Leu His Val Glu Asp Ile Ile Leu His Met Phe Gln Tyr Ile Gln Lys
385                 390                 395                 400

Leu Arg Ala Glu Gly Pro Gln Glu Trp Val Phe Gln Glu Cys Lys Asp
                405                 410                 415

Leu Asn Ala Val Ala Phe Arg Phe Lys Asp Lys Glu Arg Pro Arg Gly
            420                 425                 430

Tyr Thr Ser Lys Ile Ala Gly Lys Leu His Tyr Tyr Pro Leu Asn Gly
        435                 440                 445

Val Leu Thr Ala Glu Tyr Leu Leu Glu Glu Phe Arg Pro Asp Leu Ile
    450                 455                 460

Asp Met Val Leu Asp Lys Leu Arg Pro Glu Asn Val Arg Val Ala Ile
465                 470                 475                 480

Val Ser Lys Ser Phe Glu Gly Lys Thr Asp Arg Thr Glu Gln Trp Tyr
                485                 490                 495

Gly Thr Gln Tyr Lys Gln Glu Ala Ile Pro Glu Asp Val Ile Gln Lys
            500                 505                 510

Trp Gln Asn Ala Asp Leu Asn Gly Lys Phe Lys Leu Pro Thr Lys Asn
        515                 520                 525

Glu Phe Ile Pro Thr Asn Phe Glu Ile Leu Ser Leu Glu Lys Asp Ala
    530                 535                 540

Thr Pro Tyr Pro Ala Leu Ile Lys Asp Thr Ala Met Ser Lys Leu Trp
545                 550                 555                 560

Phe Lys Gln Asp Asp Lys Phe Phe Leu Pro Lys Ala Cys Leu Asn Phe
                565                 570                 575

Glu Phe Phe Ser Pro Phe Ala Tyr Val Asp Pro Leu His Cys Asn Met
            580                 585                 590

Ala Tyr Leu Tyr Leu Glu Leu Leu Lys Asp Ser Leu Asn Glu Tyr Ala
        595                 600                 605

Tyr Ala Ala Glu Leu Ala Gly Leu Ser Tyr Asp Leu Gln Asn Thr Ile
    610                 615                 620

Tyr Gly Met Tyr Leu Ser Val Lys Arg Tyr Asn Asp Lys Gln Pro Ile
625                 630                 635                 640

Leu Leu Lys Lys Ile Thr Glu Lys Met Ala Thr Phe Glu Ile Asp Lys
                645                 650                 655

Lys Arg Phe Glu Ile Ile Lys Glu Ala Tyr Met Arg Ser Leu Asn Asn
            660                 665                 670

Phe Arg Ala Glu Gln Pro His Gln His Ala Met Tyr Tyr Leu Arg Leu
        675                 680                 685

Leu Met Thr Glu Val Ala Trp Thr Lys Asp Glu Leu Lys Glu Ala Leu
    690                 695                 700

Asp Asp Val Thr Leu Pro Arg Leu Lys Ala Phe Ile Pro Gln Leu Leu
705                 710                 715                 720

Ser Arg Leu His Ile Glu Ala Leu Leu His Gly Asn Ile Thr Lys Gln
                725                 730                 735

Ala Ala Leu Gly Val Met Gln Met Val Glu Asp Thr Leu Ile Glu His
            740                 745                 750

Ala His Thr Lys Pro Leu Leu Pro Ser Gln Leu Val Arg Tyr Arg Glu
        755                 760                 765

Val Gln Leu Pro Asp Arg Gly Trp Phe Val Tyr Gln Gln Arg Asn Glu
    770                 775                 780

Val His Asn Asn Cys Gly Ile Glu Ile Tyr Tyr Gln Thr Asp Met Gln
785                 790                 795                 800

Ser Thr Ser Glu Asn Met Phe Leu Glu Leu Phe Cys Gln Ile Ile Ser
                805                 810                 815

Glu Pro Cys Phe Asn Thr Leu Arg Thr Lys Glu Gln Leu Gly Tyr Ile
            820                 825                 830

Val Phe Ser Gly Pro Arg Arg Ala Asn Gly Ile Gln Gly Leu Arg Phe
        835                 840                 845

Ile Ile Gln Ser Glu Lys Pro Pro His Tyr Leu Glu Ser Arg Val Glu
    850                 855                 860

Ala Phe Leu Ile Thr Met Glu Lys Ala Ile Glu Asp Met Thr Glu Glu
865                 870                 875                 880

Ala Phe Gln Lys His Ile Gln Ala Leu Ala Ile Arg Arg Leu Asp Lys
                885                 890                 895

Pro Lys Lys Leu Ser Ala Glu Cys Ala Lys Tyr Trp Gly Glu Ile Ile
            900                 905                 910

Ser Gln Gln Tyr Asn Tyr Asp Arg Asp Asn Ile Glu Val Ala Tyr Leu
        915                 920                 925

Lys Thr Leu Thr Lys Asp Asp Ile Ile Arg Phe Tyr Gln Glu Met Leu
    930                 935                 940

Ala Val Asp Ala Pro Arg Arg His Lys Val Ser Val His Val Leu Ala
945                 950                 955                 960

Arg Glu Met Asp Ser Cys Pro Val Val Gly Glu Phe Pro Ser Gln Asn
                965                 970                 975

Asp Ile Asn Leu Ser Glu Ala Pro Pro Leu Pro Gln Pro Glu Val Ile
            980                 985                 990

His Asn Met Thr Glu Phe Lys Arg Gly Leu Pro Leu Phe Pro Leu Val
        995                 1000                1005

Lys Pro His Ile Asn Phe Met Ala Ala Lys Leu
    1010                1015


<210> 134
<211> 554
<212> PRT
<213> Homo sapiens

<400> 134
Met Val Leu Asp Lys Leu Arg Pro Glu Asn Val Arg Val Ala Ile Val
1               5                   10                  15

Ser Lys Ser Phe Glu Gly Lys Thr Asp Arg Thr Glu Glu Trp Tyr Gly
            20                  25                  30

Thr Gln Tyr Lys Gln Glu Ala Ile Pro Asp Glu Val Ile Lys Lys Trp
        35                  40                  45

Gln Asn Ala Asp Leu Asn Gly Lys Phe Lys Leu Pro Thr Lys Asn Glu
    50                  55                  60

Phe Ile Pro Thr Asn Phe Glu Ile Leu Pro Leu Glu Lys Glu Ala Thr
65                  70                  75                  80

Pro Tyr Pro Ala Leu Ile Lys Asp Thr Ala Met Ser Lys Leu Trp Phe
                85                  90                  95

Lys Gln Asp Asp Lys Phe Phe Leu Pro Lys Ala Cys Leu Asn Phe Glu

            100                 105                 110

Phe Phe Ser Pro Phe Ala Tyr Val Asp Pro Leu His Cys Asn Met Ala
        115                 120                 125

Tyr Leu Tyr Leu Glu Leu Leu Lys Asp Ser Leu Asn Glu Tyr Ala Tyr
    130                 135                 140

Ala Ala Glu Leu Ala Gly Leu Ser Tyr Asp Leu Gln Asn Thr Ile Tyr
145                 150                 155                 160

Gly Met Tyr Leu Ser Val Lys Gly Tyr Asn Asp Lys Gln Pro Ile Leu
                165                 170                 175

Leu Lys Lys Ile Ile Glu Lys Met Ala Thr Phe Glu Ile Asp Glu Lys
            180                 185                 190

Arg Phe Glu Ile Ile Lys Glu Ala Tyr Met Arg Ser Leu Asn Asn Phe
        195                 200                 205

Arg Ala Glu Gln Pro His Gln His Ala Met Tyr Tyr Leu Arg Leu Leu
    210                 215                 220

Met Thr Glu Val Ala Trp Thr Lys Asp Glu Leu Lys Glu Ala Leu Asp
225                 230                 235                 240

Asp Val Thr Leu Pro Arg Leu Lys Ala Phe Ile Pro Gln Leu Leu Ser
                245                 250                 255

Arg Leu His Ile Glu Ala Leu Leu His Gly Asn Ile Thr Lys Gln Ala
            260                 265                 270

Ala Leu Gly Ile Met Gln Met Val Glu Asp Thr Leu Ile Glu His Ala
        275                 280                 285

His Thr Lys Pro Leu Leu Pro Ser Gln Leu Val Arg Tyr Arg Glu Val
    290                 295                 300

Gln Leu Pro Asp Arg Gly Trp Phe Val Tyr Gln Gln Arg Asn Glu Val
305                 310                 315                 320

His Asn Asn Cys Gly Ile Glu Ile Tyr Tyr Gln Thr Asp Met Gln Ser
                325                 330                 335

Thr Ser Glu Asn Met Phe Leu Glu Leu Phe Cys Gln Ile Ile Ser Glu
            340                 345                 350

Pro Cys Phe Asn Thr Leu Arg Thr Lys Glu Gln Leu Gly Tyr Ile Val
        355                 360                 365

Phe Ser Gly Pro Arg Arg Ala Asn Gly Ile Gln Gly Leu Arg Phe Ile
    370                 375                 380

Ile Gln Ser Glu Lys Pro Pro His Tyr Leu Glu Ser Arg Val Glu Ala
385                 390                 395                 400

Phe Leu Ile Thr Met Glu Lys Ser Ile Glu Asp Met Thr Glu Glu Ala
                405                 410                 415

Phe Gln Lys His Ile Gln Ala Leu Ala Ile Arg Arg Leu Asp Lys Pro
            420                 425                 430

Lys Lys Leu Ser Ala Glu Cys Ala Lys Tyr Trp Gly Glu Ile Ile Ser
        435                 440                 445

Gln Gln Tyr Asn Phe Asp Arg Asp Asn Thr Glu Val Ala Tyr Leu Lys
    450                 455                 460

Thr Leu Thr Lys Glu Asp Ile Ile Lys Phe Tyr Lys Glu Met Leu Ala

465                 470                 475                 480

Val Asp Ala Pro Arg Arg His Lys Val Ser Val His Val Leu Ala Arg
                485                 490                 495

Glu Met Asp Ser Cys Pro Val Val Gly Glu Phe Pro Cys Gln Asn Asp
            500                 505                 510

Ile Asn Leu Ser Gln Ala Pro Ala Leu Pro Gln Pro Glu Val Ile Gln
        515                 520                 525

Asn Met Thr Glu Phe Lys Arg Gly Leu Pro Leu Phe Pro Leu Val Lys
    530                 535                 540

Pro His Ile Asn Phe Met Ala Ala Lys Leu
545                 550

<210> 135
<211> 990
<212> PRT
<213> Drosophila melanogaster

<400> 135
Met Thr Ile Ala Glu Ser Ser Gln Lys Ser Ala Thr Arg Lys Pro Asp
  1               5                  10                  15

Ser Met Glu Pro Ile Leu Arg Leu Asn Asn Ile Glu Lys Ser Leu Gln
             20                  25                  30

Asp Thr Arg Asp Tyr Arg Gly Leu Gln Leu Glu Asn Gly Leu Lys Val
         35                  40                  45

Leu Leu Ile Ser Asp Pro Asn Thr Asp Val Ser Ala Ala Ala Leu Ser
     50                  55                  60

Val Gln Val Gly His Met Ser Asp Pro Thr Asn Leu Pro Gly Leu Ala
 65                  70                  75                  80

His Phe Cys Glu His Met Leu Phe Leu Gly Thr Glu Lys Tyr Pro His
                 85                  90                  95

Glu Asn Gly Tyr Thr Thr Tyr Leu Ser Gln Ser Gly Gly Ser Ser Asn
            100                 105                 110

Ala Ala Thr Tyr Pro Leu Met Thr Lys Tyr His Phe His Val Ala Pro
        115                 120                 125

Asp Lys Leu Asp Gly Ala Leu Asp Arg Phe Ala Gln Phe Phe Ile Ala
    130                 135                 140

Pro Leu Phe Thr Pro Ser Ala Thr Glu Arg Glu Ile Asn Ala Val Asn
145                 150                 155                 160

Ser Glu His Glu Lys Asn Leu Pro Ser Asp Leu Trp Arg Ile Lys Gln
                165                 170                 175

Val Asn Arg His Leu Ala Lys Pro Asp His Ala Tyr Ser Lys Phe Gly
            180                 185                 190

Ser Gly Asn Lys Thr Thr Leu Ser Glu Ile Pro Lys Ser Lys Asn Ile
        195                 200                 205

Asp Val Arg Asp Glu Leu Leu Lys Phe His Lys Gln Trp Tyr Ser Ala
    210                 215                 220

Asn Ile Met Cys Leu Ala Val Ile Gly Lys Glu Ser Leu Asp Glu Leu
225                 230                 235                 240

Glu Gly Met Val Leu Glu Lys Phe Ser Glu Ile Glu Asn Lys Asn Val
                245                 250                 255

Lys Val Pro Gly Trp Pro Arg His Pro Tyr Ala Glu Glu Arg Tyr Gly
            260                 265                 270

Gln Lys Val Lys Ile Val Pro Ile Lys Asp Ile Arg Ser Leu Thr Ile
        275                 280                 285

Ser Phe Thr Thr Asp Asp Leu Thr Gln Phe Tyr Lys Ser Gly Pro Asp
    290                 295                 300

Asn Tyr Leu Thr His Leu Ile Gly His Glu Gly Lys Gly Ser Ile Leu
305                 310                 315                 320

Ser Glu Leu Arg Arg Leu Gly Trp Cys Asn Asp Leu Met Ala Gly His
                325                 330                 335

Gln Asn Thr Gln Asn Gly Phe Gly Phe Phe Asp Ile Val Val Asp Leu
            340                 345                 350

Thr Gln Glu Gly Leu Glu His Val Asp Asp Ile Val Lys Ile Val Phe
        355                 360                 365

Gln Tyr Leu Glu Met Leu Arg Lys Glu Gly Pro Lys Lys Trp Ile Phe
    370                 375                 380

Asp Glu Cys Val Lys Leu Asn Glu Met Arg Phe Arg Phe Lys Glu Lys
385                 390                 395                 400

Glu Gln Pro Glu Asn Leu Val Thr His Ala Val Ser Ser Met Gln Ile
                405                 410                 415

Phe Pro Leu Glu Glu Val Leu Ile Ala Pro Tyr Leu Ser Asn Glu Trp
            420                 425                 430

Arg Pro Asp Leu Ile Lys Gly Leu Leu Asp Glu Leu Val Pro Ser Lys
        435                 440                 445

Ser Arg Ile Val Ile Val Ser Gln Ser Phe Glu Pro Asp Cys Asp Leu
    450                 455                 460

Ala Glu Pro Tyr Tyr Lys Thr Lys Tyr Gly Ile Thr Arg Val Ala Lys
465                 470                 475                 480

Asp Thr Val Gln Ser Trp Glu Asn Cys Glu Leu Asn Glu Asn Leu Lys
                485                 490                 495

Leu Ala Leu Pro Asn Ser Phe Ile Pro Thr Asn Phe Asp Ile Ser Asp
            500                 505                 510

Val Pro Ala Asp Ala Pro Lys His Pro Thr Ile Ile Leu Asp Thr Pro
        515                 520                 525

Ile Leu Arg Val Trp His Lys Gln Asp Asn Gln Phe Asn Lys Pro Lys
    530                 535                 540

Ala Cys Met Thr Phe Asp Met Ser Asn Pro Ile Ala Tyr Leu Asp Pro
545                 550                 555                 560

Leu Asn Cys Asn Leu Asn His Met Met Val Met Leu Leu Lys Asp Gln
                565                 570                 575

Leu Asn Glu Tyr Leu Tyr Asp Ala Glu Leu Ala Ser Leu Lys Leu Ser
            580                 585                 590

Val Met Gly Lys Ser Cys Gly Ile Asp Phe Thr Ile Arg Gly Phe Ser
        595                 600                 605

Asp Lys Gln Val Val Leu Leu Glu Lys Leu Leu Asp His Leu Phe Asp
610                 615                 620

Phe Ser Ile Asp Glu Lys Arg Phe Asp Ile Leu Lys Glu Glu Tyr Val
625                 630                 635                 640

Arg Ser Leu Lys Asn Phe Lys Ala Glu Gln Pro Tyr Gln His Ser Ile
                645                 650                 655

Tyr Tyr Leu Ala Leu Leu Leu Thr Glu Asn Ala Trp Ala Asn Met Glu
            660                 665                 670

Leu Leu Asp Ala Met Glu Leu Val Thr Tyr Asp Arg Val Leu Asn Phe
        675                 680                 685

Ala Lys Glu Phe Phe Gln Arg Leu His Thr Glu Cys Phe Ile Phe Gly
    690                 695                 700

Asn Val Thr Lys Gln Gln Ala Thr Asp Ile Ala Gly Arg Val Asn Thr
705                 710                 715                 720

Arg Leu Glu Ala Thr Asn Ala Ser Lys Leu Pro Ile Leu Ala Arg Gln
                725                 730                 735

Met Leu Lys Lys Arg Glu Tyr Lys Leu Leu Ala Gly Asp Ser Tyr Leu
            740                 745                 750

Phe Glu Lys Glu Asn Glu Phe His Lys Ser Ser Cys Ala Gln Leu Tyr
        755                 760                 765

Leu Gln Cys Gly Ala Gln Thr Asp His Thr Asn Ile Met Val Asn Leu
    770                 775                 780

Val Ser Gln Val Leu Ser Glu Pro Cys Tyr Asp Cys Leu Arg Thr Lys
785                 790                 795                 800

Glu Gln Leu Gly Tyr Ile Val Phe Ser Gly Val Arg Lys Val Asn Gly
                805                 810                 815

Ala Asn Gly Ile Arg Ile Ile Val Gln Ser Ala Lys His Pro Ser Tyr
            820                 825                 830

Val Glu Asp Arg Ile Glu Asn Phe Leu Gln Thr Tyr Leu Gln Val Ile
        835                 840                 845

Glu Asp Met Pro Leu Asp Glu Phe Glu Arg His Lys Glu Ala Leu Ala
    850                 855                 860

Val Lys Lys Leu Glu Lys Pro Lys Thr Ile Phe Gln Gln Phe Ser Gln
865                 870                 875                 880

Phe Tyr Gly Glu Ile Ala Met Gln Thr Tyr His Phe Glu Arg Glu Glu
                885                 890                 895

Ala Glu Val Ala Ile Leu Arg Lys Ile Ser Lys Ala Asp Phe Val Asp
            900                 905                 910

Tyr Phe Lys Lys Phe Ile Ala Lys Asp Gly Glu Glu Arg Arg Val Leu
        915                 920                 925

Ser Val His Ile Val Ser Gln Gln Thr Asp Glu Asn Ala Thr Ser Glu
    930                 935                 940

Ala Glu Pro Val Glu Ile Thr Asn Met Glu Arg His Lys Pro Ile Ser
945                 950                 955                 960

Asp Ile Val Thr Phe Lys Ser Cys Lys Glu Leu Tyr Pro Ile Ala Leu
                965                 970                 975

Pro Phe Leu Asp Ile Lys Ala Lys Gly Ala Arg Ser Lys Leu
            980                 985                 990

<210> 136
<211> 370
<212> PRT
<213> Homo sapiens

<400> 136
Ala Phe Lys Gln Leu Gly Trp Ile Pro Gly Leu Val Leu Leu Leu Leu
1               5                   10                  15

Ala Gly Phe Ile Thr Leu Tyr Thr Gly Leu Leu Leu Ser Glu Cys Tyr
            20                  25                  30

Glu Tyr Val Pro Gly Lys Arg Asn Asp Ser Tyr Leu Asp Leu Gly Arg
        35                  40                  45

Ser Ala Tyr Gly Gly Lys Gly Leu Leu Leu Thr Ser Phe Val Gly Gln
    50                  55                  60

Tyr Val Asn Leu Phe Gly Val Asn Ile Gly Tyr Leu Ile Leu Ala Gly
65                  70                  75                  80

Asp Leu Leu Pro Lys Ile Ile Ser Ser Phe Cys Gly Asp Asn Cys Asp
                85                  90                  95

His Leu Asp Gly Asn Ser Trp Ile Ile Ile Phe Ala Ala Ile Ile Ile
            100                 105                 110

Thr Leu Ser Phe Ile Pro Asn Phe Asn Leu Leu Ser Ile Ser Ser Leu
        115                 120                 125

Ser Ala Phe Ser Ser Leu Ala Tyr Leu Ser Ile Ile Ser Phe Leu Ile
    130                 135                 140

Ile Val Ala Val Ile Ala Gly Ile Phe Val Leu Leu Gly Ala Val Tyr
145                 150                 155                 160

Gly Ile Leu Trp Ser Pro Ser Phe Thr Lys Leu Thr Gly Leu Phe Leu
                165                 170                 175

Ala Ile Gly Ile Ile Val Phe Ala Phe Glu Gly His Ala Val Leu Leu
            180                 185                 190

Pro Ile Gln Asn Thr Met Lys Ser Pro Ser Ala Lys Lys Phe Lys Lys
        195                 200                 205

Val Leu Asn Val Ala Ile Ile Ile Val Thr Val Leu Tyr Ile Leu Val
    210                 215                 220

Gly Phe Phe Gly Tyr Leu Thr Phe Gly Asn Asn Val Lys Gly Asn Ile
225                 230                 235                 240

Leu Leu Asn Leu Pro Asn Asn Pro Phe Trp Leu Ile Val Asn Leu Asn
                245                 250                 255

Leu Val Val Ala Ile Leu Leu Thr Phe Pro Leu Gln Ala Phe Pro Ile
            260                 265                 270

Val Arg Ile Ile Glu Asn Leu Leu Thr Lys Lys Asn Asn Phe Ala Pro
        275                 280                 285

Asn Lys Ser Lys Leu Leu Arg Val Val Ile Arg Ser Gly Leu Val Val
    290                 295                 300

Phe Thr Leu Leu Ile Ala Ile Leu Val Pro Phe Phe Gly Asp Phe Leu
305                 310                 315                 320

Ser Leu Val Gly Ala Thr Ser Gly Ala Pro Leu Thr Phe Ile Leu Pro
                325                 330                 335

Pro Phe Glu Met Tyr Ile Leu Leu Lys Lys Glu Lys Lys Trp Gly Lys
            340                 345                 350

Arg Trp Pro Lys Leu Ile Asp Leu Ile Ile Ile Val Val Gly Leu Leu
        355                 360                 365

Leu Ser
    370


<210> 137
<211> 434
<212> PRT
<213> Homo sapiens

<400> 137
Met Ser Thr Gln Arg Leu Arg Asn Glu Asp Tyr His Asp Tyr Ser Ser
  1               5                  10                  15

Thr Asp Val Ser Pro Glu Glu Ser Pro Ser Glu Gly Leu Asn Asn Leu
            20                  25                  30

Ser Ser Pro Gly Ser Tyr Gln Arg Phe Gly Gln Ser Asn Ser Thr Thr
        35                  40                  45

Trp Phe Gln Thr Leu Ile His Leu Leu Lys Gly Asn Ile Gly Thr Gly
    50                  55                  60

Leu Leu Gly Leu Pro Leu Ala Ala Lys Asn Ala Gly Ile Val Met Gly
 65                  70                  75                  80

Pro Ile Ser Leu Leu Ile Ile Gly Ile Val Ala Val His Cys Met Gly
                85                  90                  95

Ile Leu Val Lys Cys Ala His His Phe Cys Arg Arg Leu Asn Lys Ser
            100                 105                 110

Phe Val Asp Tyr Gly Asp Thr Val Met Tyr Gly Leu Glu Ser Ser Pro
        115                 120                 125

Cys Ser Trp Leu Arg Asn His Ala His Trp Gly Arg Arg Val Val Asp
    130                 135                 140

Phe Phe Leu Ile Val Thr Gln Leu Gly Phe Cys Cys Val Tyr Phe Val
145                 150                 155                 160

Phe Leu Ala Asp Asn Phe Lys Gln Val Ile Glu Ala Ala Asn Gly Thr
                165                 170                 175

Thr Asn Asn Cys His Asn Asn Glu Thr Val Ile Leu Thr Pro Thr Met
            180                 185                 190

Asp Ser Arg Leu Tyr Met Leu Ser Phe Leu Pro Phe Leu Val Leu Leu
        195                 200                 205

Val Phe Ile Arg Asn Leu Arg Ala Leu Ser Ile Phe Ser Leu Leu Ala
    210                 215                 220

Asn Ile Thr Met Leu Val Ser Leu Val Met Ile Tyr Gln Phe Ile Val
225                 230                 235                 240

Gln Arg Ile Pro Asp Pro Ser His Leu Pro Leu Val Ala Pro Trp Lys
                245                 250                 255

Thr Tyr Pro Leu Phe Phe Gly Thr Ala Ile Phe Ser Phe Glu Gly Ile

260                 265                 270

Gly Met Val Leu Pro Leu Glu Asn Lys Met Lys Asp Pro Arg Lys Phe
        275                 280                 285

Pro Leu Ile Leu Tyr Leu Gly Met Val Ile Val Thr Ile Leu Tyr Ile
    290                 295                 300

Ser Leu Gly Cys Leu Gly Tyr Leu Gln Phe Gly Ala Asn Ile Gln Gly
305                 310                 315                 320

Ser Ile Thr Leu Asn Leu Pro Asn Cys Trp Leu Tyr Gln Ser Val Lys
                325                 330                 335

Leu Leu Tyr Ser Ile Gly Ile Phe Phe Thr Tyr Ala Leu Gln Phe Tyr
            340                 345                 350

Val Pro Ala Glu Ile Ile Ser Leu Val Gly Ser Val Ser Ser Ser Ala
        355                 360                 365

Leu Ala Leu Ile Ile Pro Pro Leu Leu Glu Val Thr Thr Phe Tyr Ser
    370                 375                 380

Glu Gly Met Ser Pro Leu Thr Ile Phe Lys Asp Ala Leu Ile Ser Ile
385                 390                 395                 400

Leu Gly Phe Val Gly Phe Val Val Gly Thr Tyr Glu Ala Leu Tyr Glu
                405                 410                 415

Leu Ile Arg Pro Ser Asn Ala Pro Ile Phe Ile Asn Ser Thr Cys Ala
            420                 425                 430

Phe Ile

<210> 138
<211> 475
<212> PRT
<213> Rattus norvegicus

<400> 138
Met Ser Thr Gln Arg Leu Arg Asn Glu Asp Tyr His Asp Tyr Ser Ser
1               5                   10                  15

Thr Asp Val Ser Pro Glu Glu Ser Pro Ser Glu Gly Leu Gly Ser Phe
            20                  25                  30

Ser Pro Gly Ser Tyr Gln Arg Leu Gly Glu Asn Ser Ser Met Thr Trp
        35                  40                  45

Phe Gln Thr Leu Ile His Leu Leu Lys Gly Asn Ile Gly Thr Gly Leu
    50                  55                  60

Leu Gly Leu Pro Leu Ala Val Lys Asn Ala Gly Leu Leu Leu Gly Pro
65                  70                  75                  80

Leu Ser Leu Leu Val Ile Gly Ile Val Ala Val His Cys Met Gly Ile
                85                  90                  95

Leu Val Lys Cys Ala His His Leu Cys Arg Arg Leu Asn Lys Pro Phe
            100                 105                 110

Leu Asp Tyr Gly Asp Thr Val Met Tyr Gly Leu Glu Cys Ser Pro Ser
        115                 120                 125

Thr Trp Ile Arg Asn His Ser His Trp Gly Arg Arg Ile Val Asp Phe
    130                 135                 140

Phe Leu Val Val Thr Gln Leu Gly Phe Cys Cys Val Tyr Phe Val Phe
145                 150                 155                 160

Leu Ala Asp Asn Phe Lys Gln Val Ile Glu Ala Ala Asn Gly Thr Thr
                165                 170                 175

Thr Asn Cys Asn Asn Asn Glu Thr Val Ile Leu Thr Pro Thr Met Asp
            180                 185                 190

Ser Arg Leu Tyr Met Leu Thr Phe Leu Pro Phe Leu Val Leu Leu Ser
        195                 200                 205

Phe Ile Arg Asn Leu Arg Ile Leu Ser Ile Phe Ser Leu Leu Ala Asn
    210                 215                 220

Ile Ser Met Phe Val Ser Leu Ile Met Ile Tyr Gln Phe Ile Val Gln
225                 230                 235                 240

Arg Ile Pro Asp Pro Ser His Leu Pro Leu Val Ala Pro Trp Lys Thr
                245                 250                 255

Tyr Pro Leu Phe Phe Gly Thr Ala Ile Phe Ala Phe Glu Gly Ile Gly
            260                 265                 270

Val Val Leu Pro Leu Glu Asn Lys Met Lys Asp Ser Gln Lys Phe Pro
        275                 280                 285

Leu Ile Leu Tyr Leu Gly Met Ala Ile Ile Thr Val Leu Tyr Ile Ser
    290                 295                 300

Leu Gly Ser Leu Gly Tyr Leu Gln Phe Gly Ala Asp Ile Lys Gly Ser
305                 310                 315                 320

Ile Thr Leu Asn Leu Pro Asn Cys Trp Leu Tyr Gln Ser Val Lys Leu
                325                 330                 335

Leu Tyr Ser Ile Gly Ile Phe Phe Thr Tyr Ala Leu Gln Phe Tyr Val
            340                 345                 350

Ala Ala Glu Ile Ile Ile Pro Ala Ile Val Ser Arg Val Pro Glu Arg
        355                 360                 365

Phe Glu Leu Val Val Asp Leu Ser Ala Arg Thr Ala Met Val Cys Val
    370                 375                 380

Thr Cys Val Leu Ala Val Leu Ile Pro Arg Leu Asp Leu Val Ile Ser
385                 390                 395                 400

Leu Val Gly Ser Val Ser Ser Ser Ala Leu Ala Leu Ile Ile Pro Pro
                405                 410                 415

Leu Leu Glu Val Thr Thr Tyr Tyr Gly Glu Gly Ile Ser Pro Leu Thr
            420                 425                 430

Ile Thr Lys Asp Ala Leu Ile Ser Ile Leu Gly Phe Val Gly Phe Val
        435                 440                 445

Val Gly Thr Tyr Glu Ser Leu Trp Glu Leu Ile Gln Pro Ser His Ser
    450                 455                 460

Asp Ser Ser Thr Asn Ser Thr Ser Ala Phe Ile
465                 470                 475


<210> 139
<211> 504
<212> PRT
<213> Homo sapiens

<400> 139
Met Glu Ala Ala Ala Thr Pro Ala Ala Ala Gly Ala Ala Arg Arg Glu
1               5                   10                  15

Glu Leu Asp Met Asp Val Met Arg Pro Leu Ile Asn Glu Gln Asn Phe
            20                  25                  30

Asp Gly Thr Ser Asp Glu Glu His Glu Gln Glu Leu Leu Pro Val Gln
        35                  40                  45

Lys His Tyr Gln Leu Asp Asp Gln Glu Gly Ile Ser Phe Val Gln Thr
    50                  55                  60

Leu Met His Leu Leu Lys Gly Asn Ile Gly Thr Gly Leu Leu Gly Leu
65                  70                  75                  80

Pro Leu Ala Ile Lys Asn Ala Gly Ile Val Leu Gly Pro Ile Ser Leu
                85                  90                  95

Val Phe Ile Gly Ile Ile Ser Val His Cys Met His Ile Leu Val Arg
            100                 105                 110

Cys Ser His Phe Leu Cys Leu Arg Phe Lys Lys Ser Thr Leu Gly Tyr
        115                 120                 125

Ser Asp Thr Val Ser Phe Ala Met Glu Val Ser Pro Trp Ser Cys Leu
    130                 135                 140

Gln Lys Gln Ala Ala Trp Gly Arg Ser Val Val Asp Phe Phe Leu Val
145                 150                 155                 160

Ile Thr Gln Leu Gly Phe Cys Ser Val Tyr Ile Val Phe Leu Ala Glu
                165                 170                 175

Asn Val Lys Gln Val His Glu Gly Phe Leu Glu Ser Lys Val Phe Ile
            180                 185                 190

Ser Asn Ser Thr Asn Ser Ser Asn Pro Cys Glu Arg Arg Ser Val Asp
        195                 200                 205

Leu Arg Ile Tyr Met Leu Cys Phe Leu Pro Phe Ile Ile Leu Leu Val
    210                 215                 220

Phe Ile Arg Glu Leu Lys Asn Leu Phe Val Leu Ser Phe Leu Ala Asn
225                 230                 235                 240

Val Ser Met Ala Val Ser Leu Val Ile Ile Tyr Gln Tyr Val Val Arg
                245                 250                 255

Asn Met Pro Asp Pro His Asn Leu Pro Ile Val Ala Gly Trp Lys Lys
            260                 265                 270

Tyr Pro Leu Phe Phe Gly Thr Ala Val Phe Ala Phe Glu Gly Ile Gly
        275                 280                 285

Val Val Leu Pro Leu Glu Asn Gln Met Lys Glu Ser Lys Arg Phe Pro
    290                 295                 300

Gln Ala Leu Asn Ile Gly Met Gly Ile Val Thr Thr Leu Tyr Val Thr
305                 310                 315                 320

Leu Ala Thr Leu Gly Tyr Met Cys Phe His Asp Glu Ile Lys Gly Ser
                325                 330                 335

Ile Thr Leu Asn Leu Pro Gln Asp Val Trp Leu Tyr Gln Ser Val Lys
            340                 345                 350

Ile Leu Tyr Ser Phe Gly Ile Phe Val Thr Tyr Ser Ile Gln Phe Tyr
        355                 360                 365

Val Pro Ala Glu Ile Ile Ile Pro Gly Ile Thr Ser Lys Phe His Thr
    370                 375                 380

Lys Trp Lys Gln Ile Cys Glu Phe Gly Ile Arg Ser Phe Leu Val Ser
385                 390                 395                 400

Ile Thr Cys Ala Gly Ala Ile Leu Ile Pro Arg Leu Asp Ile Val Ile
                405                 410                 415

Ser Phe Val Gly Ala Val Ser Ser Ser Thr Leu Ala Leu Ile Leu Pro
            420                 425                 430

Pro Leu Val Glu Ile Leu Thr Phe Ser Lys Glu His Tyr Asn Ile Trp
        435                 440                 445

Met Val Leu Lys Asn Ile Ser Ile Ala Phe Thr Gly Val Val Gly Phe
    450                 455                 460

Leu Leu Gly Thr Tyr Ile Thr Val Glu Glu Ile Ile Tyr Pro Thr Pro
465                 470                 475                 480

Lys Val Val Ala Gly Thr Pro Gln Ser Pro Phe Leu Asn Leu Asn Ser
                485                 490                 495

Thr Cys Leu Thr Ser Gly Leu Lys
            500

<210> 140
<211> 165
<212> PRT
<213> Homo sapiens

<400> 140
Met Gly Pro Ile Ser Leu Leu Ile Ile Gly Ile Val Ala Val His Cys
  1               5                  10                  15

Met Gly Ile Leu Val Lys Cys Ala His His Phe Cys Arg Arg Leu Asn
             20                  25                  30

Lys Ser Phe Val Asp Tyr Gly Asp Thr Val Met Tyr Gly Leu Glu Ser
         35                  40                  45

Ser Pro Cys Ser Trp Leu Arg Asn His Ala His Trp Gly Arg Arg Val
     50                  55                  60

Val Asp Phe Phe Leu Ile Val Thr Gln Leu Gly Phe Cys Cys Val Tyr
 65                  70                  75                  80

Phe Val Phe Leu Ala Asp Asn Phe Lys Gln Val Ile Glu Ala Ala Asn
                 85                  90                  95

Gly Thr Thr Asn Asn Cys His Asn Asn Glu Thr Val Ile Leu Thr Pro
            100                 105                 110

Thr Met Asp Ser Arg Leu Tyr Met Leu Ser Phe Leu Pro Phe Leu Val
        115                 120                 125

Leu Leu Val Phe Ile Arg Asn Leu Arg Ala Leu Ser Ile Phe Ser Leu
    130                 135                 140

Leu Ala Asn Ile Thr Met Leu Val Ser Leu Val Met Ile Tyr Gln Phe
145                 150                 155                 160

Ile Val Gln Ile Leu
                165

<210> 141
<211> 504
<212> PRT
<213> Drosophila melanogaster

<400> 141
Met Glu Asp Leu Thr Pro Leu Thr Asn Leu Gln Gln Ile Pro Glu Gly
1               5                   10                  15

Ala Pro Arg Lys Lys Lys Met Thr Glu Arg Gln Pro Leu Leu Leu Gln
            20                  25                  30

Ser Asp Ala Ser Asp Tyr Glu Gly Ser Arg Gly Ser Ala Ala Arg Pro
        35                  40                  45

Val Arg Ser Ser Pro Pro Asp Asn Thr Leu Val Asn Val His Ser Glu
    50                  55                  60

Asp Ser Leu Ala Ala Ser Gly Ser Gly Asp Asp Glu Ile Gly Ser Thr
65                  70                  75                  80

Asp Lys Ser Tyr Asn Pro Thr His His Arg Asp Leu Glu His Pro Thr
                85                  90                  95

Ser Asn Phe Asp Thr Leu Val His Leu Leu Lys Gly Asn Ile Gly Thr
            100                 105                 110

Gly Ile Leu Ala Met Pro Asp Ala Phe Lys Asn Ala Gly Leu Tyr Val
        115                 120                 125

Gly Leu Phe Gly Thr Met Ile Met Gly Ala Ile Cys Thr His Cys Met
    130                 135                 140

His Met Leu Val Asn Cys Ser His Glu Leu Cys Arg Arg Phe Gln Gln
145                 150                 155                 160

Pro Ser Leu Asp Phe Ser Glu Val Ala Tyr Cys Ser Phe Glu Ser Gly
                165                 170                 175

Pro Leu Gly Leu Arg Arg Tyr Ser Met Leu Ala Arg Arg Ile Val Thr
            180                 185                 190

Thr Phe Leu Phe Ile Thr Gln Ile Gly Phe Cys Cys Val Tyr Phe Leu
        195                 200                 205

Phe Val Ala Leu Asn Ile Lys Asp Val Met Asp His Tyr Tyr Lys Met
    210                 215                 220

Pro Val Gln Ile Tyr Leu Leu Ile Met Leu Gly Pro Met Ile Leu Leu
225                 230                 235                 240

Asn Leu Val Arg Asn Leu Lys Tyr Leu Thr Pro Val Ser Leu Val Ala
                245                 250                 255

Ala Leu Leu Thr Val Ala Gly Leu Ala Ile Thr Phe Ser Tyr Met Leu
            260                 265                 270

Val Asp Leu Pro Asp Val His Thr Val Lys Pro Val Ala Thr Trp Ala
        275                 280                 285

Thr Leu Pro Leu Tyr Phe Gly Thr Ala Ile Tyr Ala Phe Glu Gly Ile
    290                 295                 300

Gly Val Val Leu Pro Leu Glu Asn Asn Met Arg Thr Pro Glu Asp Phe
305                 310                 315                 320

Gly Gly Thr Thr Gly Val Leu Asn Thr Gly Met Val Ile Val Ala Cys
                325                 330                 335

Leu Tyr Thr Ala Val Gly Phe Phe Gly Tyr Leu Lys Tyr Gly Glu His
            340                 345                 350

Val Glu Gly Ser Ile Thr Leu Asn Leu Pro Gln Gly Asp Thr Leu Ser
        355                 360                 365

Gln Leu Val Arg Ile Ser Met Ala Val Ala Ile Phe Leu Ser Tyr Thr
    370                 375                 380

Leu Gln Phe Tyr Val Pro Val Asn Ile Val Glu Pro Phe Val Arg Ser
385                 390                 395                 400

His Phe Asp Thr Thr Arg Ala Lys Asp Leu Ser Ala Thr Val Leu Arg
                405                 410                 415

Val Val Leu Val Thr Phe Thr Phe Leu Leu Ala Thr Cys Ile Pro Asn
            420                 425                 430

Leu Gly Ser Ile Ile Ser Leu Val Gly Ala Val Ser Ser Ser Ala Leu
        435                 440                 445

Ala Leu Ile Ala Pro Pro Ile Ile Glu Val Ile Thr Phe Tyr Asn Val
    450                 455                 460

Gly Tyr Gly Arg Phe Asn Trp Met Leu Trp Lys Asp Val Leu Ile Leu
465                 470                 475                 480

Ile Phe Gly Leu Cys Gly Phe Val Phe Gly Thr Trp Ala Ser Leu Ala
                485                 490                 495

Gln Ile Leu Asn Asp Arg Thr His
            500

<210> 142
<211> 370
<212> PRT
<213> Homo sapiens

<400> 142
Ala Phe Lys Gln Leu Gly Trp Ile Pro Gly Leu Val Leu Leu Leu Leu
  1               5                  10                  15

Ala Gly Phe Ile Thr Leu Tyr Thr Gly Leu Leu Leu Ser Glu Cys Tyr
             20                  25                  30

Glu Tyr Val Pro Gly Lys Arg Asn Asp Ser Tyr Leu Asp Leu Gly Arg
         35                  40                  45

Ser Ala Tyr Gly Gly Lys Gly Leu Leu Leu Thr Ser Phe Val Gly Gln
     50                  55                  60

Tyr Val Asn Leu Phe Gly Val Asn Ile Gly Tyr Leu Ile Leu Ala Gly
 65                  70                  75                  80

Asp Leu Leu Pro Lys Ile Ile Ser Ser Phe Cys Gly Asp Asn Cys Asp
                 85                  90                  95

His Leu Asp Gly Asn Ser Trp Ile Ile Ile Phe Ala Ala Ile Ile Ile
            100                 105                 110

Thr Leu Ser Phe Ile Pro Asn Phe Asn Leu Leu Ser Ile Ser Ser Leu
        115                 120                 125

Ser Ala Phe Ser Ser Leu Ala Tyr Leu Ser Ile Ile Ser Phe Leu Ile
    130                 135                 140

Ile Val Ala Val Ile Ala Gly Ile Phe Val Leu Leu Gly Ala Val Tyr
145                 150                 155                 160

Gly Ile Leu Trp Ser Pro Ser Phe Thr Lys Leu Thr Gly Leu Phe Leu
                165                 170                 175

Ala Ile Gly Ile Ile Val Phe Ala Phe Glu Gly His Ala Val Leu Leu
            180                 185                 190

Pro Ile Gln Asn Thr Met Lys Ser Pro Ser Ala Lys Lys Phe Lys Lys
        195                 200                 205

Val Leu Asn Val Ala Ile Ile Ile Val Thr Val Leu Tyr Ile Leu Val
    210                 215                 220

Gly Phe Phe Gly Tyr Leu Thr Phe Gly Asn Asn Val Lys Gly Asn Ile
225                 230                 235                 240

Leu Leu Asn Leu Pro Asn Asn Pro Phe Trp Leu Ile Val Asn Leu Asn
                245                 250                 255

Leu Val Val Ala Ile Leu Leu Thr Phe Pro Leu Gln Ala Phe Pro Ile
            260                 265                 270

Val Arg Ile Ile Glu Asn Leu Leu Thr Lys Lys Asn Asn Phe Ala Pro
        275                 280                 285

Asn Lys Ser Lys Leu Leu Arg Val Val Ile Arg Ser Gly Leu Val Val
    290                 295                 300

Phe Thr Leu Leu Ile Ala Ile Leu Val Pro Phe Phe Gly Asp Phe Leu
305                 310                 315                 320

Ser Leu Val Gly Ala Thr Ser Gly Ala Pro Leu Thr Phe Ile Leu Pro
                325                 330                 335

Pro Phe Glu Met Tyr Ile Leu Leu Lys Lys Glu Lys Lys Trp Gly Lys
            340                 345                 350

Arg Trp Pro Lys Leu Ile Asp Leu Ile Ile Ile Val Val Gly Leu Leu
        355                 360                 365

Leu Ser
    370


<210> 143
<211> 596
<212> PRT
<213> Homo sapiens

<400> 143
Met Phe Gly Ile Gln Glu Asn Ile Pro Arg Gly Gly Thr Thr Met Lys
  1               5                  10                  15

Glu Glu Pro Leu Gly Ser Gly Met Asn Pro Val Arg Ser Trp Met His
             20                  25                  30

Thr Ala Gly Val Val Asp Ala Asn Thr Ala Ala Gln Ser Gly Val Gly
         35                  40                  45

Leu Ala Arg Ala His Phe Glu Lys Gln Pro Pro Ser Asn Leu Arg Lys
     50                  55                  60

Ser Asn Phe Phe His Phe Val Leu Ala Leu Tyr Asp Arg Gln Gly Gln
 65                  70                  75                  80

Pro Val Glu Ile Glu Arg Thr Ala Phe Val Asp Phe Val Glu Lys Glu
                 85                  90                  95

Lys Glu Pro Asn Asn Glu Lys Thr Asn Asn Gly Ile His Tyr Lys Leu

            100                 105                 110

Gln Leu Leu Tyr Ser Asn Gly Val Arg Thr Glu Gln Asp Leu Tyr Val
        115                 120                 125

Arg Leu Ile Asp Ser Met Thr Lys Gln Ala Ile Val Tyr Glu Gly Gln
    130                 135                 140

Asp Lys Asn Pro Glu Met Cys Arg Val Leu Leu Thr His Glu Ile Met
145                 150                 155                 160

Cys Ser Arg Cys Cys Asp Lys Lys Ser Cys Gly Asn Arg Asn Glu Thr
                165                 170                 175

Pro Ser Asp Pro Val Ile Ile Asp Arg Phe Phe Leu Lys Phe Phe Leu
            180                 185                 190

Lys Cys Asn Gln Asn Cys Leu Lys Asn Ala Gly Asn Pro Arg Asp Met
        195                 200                 205

Arg Arg Phe Gln Val Val Val Ser Thr Thr Val Asn Val Asp Gly His
    210                 215                 220

Val Leu Ala Val Ser Asp Asn Met Phe Val His Asn Asn Ser Lys His
225                 230                 235                 240

Gly Arg Arg Ala Arg Arg Leu Asp Pro Ser Glu Gly Thr Ala Pro Ser
                245                 250                 255

Tyr Leu Glu Asn Ala Thr Pro Cys Ile Lys Ala Ile Ser Pro Ser Glu
            260                 265                 270

Gly Trp Thr Thr Gly Gly Ala Thr Val Ile Ile Ile Gly Asp Asn Phe
        275                 280                 285

Phe Asp Gly Leu Gln Val Val Phe Gly Thr Met Leu Val Trp Ser Glu
    290                 295                 300

Leu Ile Thr Pro His Ala Ile Arg Val Gln Thr Pro Pro Arg His Ile
305                 310                 315                 320

Pro Gly Val Val Glu Val Thr Leu Ser Tyr Lys Ser Lys Gln Phe Cys
                325                 330                 335

Lys Gly Ala Pro Gly Arg Phe Val Tyr Thr Ala Leu Asn Glu Pro Thr
            340                 345                 350

Ile Asp Tyr Gly Phe Gln Arg Leu Gln Lys Val Ile Pro Arg His Pro
        355                 360                 365

Gly Asp Pro Glu Arg Leu Pro Lys Glu Val Leu Leu Lys Arg Ala Ala
    370                 375                 380

Asp Leu Val Glu Ala Leu Tyr Gly Met Pro His Asn Asn Gln Glu Ile
385                 390                 395                 400

Ile Leu Lys Arg Ala Ala Asp Ile Ala Glu Ala Leu Tyr Ser Val Pro
                405                 410                 415

Arg Asn His Asn Gln Ile Pro Thr Leu Gly Asn Asn Pro Ala His Thr
            420                 425                 430

Gly Met Met Gly Val Asn Ser Phe Ser Ser Gln Leu Ala Val Asn Val
        435                 440                 445

Ser Glu Thr Ser Gln Ala Asn Asp Gln Val Gly Tyr Ser Arg Asn Thr
    450                 455                 460

Ser Ser Val Ser Pro Arg Gly Tyr Val Pro Ser Ser Thr Pro Gln Gln

465                 470                 475                 480

Ser Asn Tyr Asn Thr Val Ser Thr Ser Met Asn Gly Tyr Gly Ser Gly
                485                 490                 495

Ala Met Ala Ser Leu Gly Val Pro Gly Ser Pro Gly Phe Leu Asn Gly
            500                 505                 510

Ser Ser Ala Asn Ser Pro Tyr Gly Ile Val Pro Ser Ser Pro Thr Met
        515                 520                 525

Ala Ala Ser Ser Val Thr Leu Pro Ser Asn Cys Ser Ser Thr His Gly
    530                 535                 540

Ile Phe Ser Phe Ser Pro Ala Asn Val Ile Ser Ala Val Lys Gln Lys
545                 550                 555                 560

Ser Ala Phe Ala Pro Val Val Arg Pro Gln Ala Ser Pro Pro Pro Ser
                565                 570                 575

Cys Thr Ser Ala Asn Gly Asn Gly Leu Gln Ala Met Ser Gly Leu Val
            580                 585                 590

Val Pro Pro Met
        595

<210> 144
<211> 596
<212> PRT
<213> Mus musculus

<400> 144
Met Phe Gly Ile Gln Glu Asn Ile Pro Arg Gly Gly Thr Thr Met Lys
1               5                   10                  15

Glu Glu Pro Leu Gly Ser Gly Met Asn Pro Val Arg Ser Trp Met His
            20                  25                  30

Thr Ala Gly Val Val Asp Ala Asn Thr Ala Ala Gln Ser Gly Val Gly
        35                  40                  45

Leu Ala Arg Ala His Phe Glu Lys Gln Pro Pro Ser Asn Leu Arg Lys
    50                  55                  60

Ser Asn Phe Phe His Phe Val Leu Ala Leu Tyr Asp Arg Gln Gly Gln
65                  70                  75                  80

Pro Val Glu Ile Glu Arg Thr Ala Phe Val Asp Phe Val Glu Lys Glu
                85                  90                  95

Lys Glu Pro Asn Asn Glu Lys Thr Asn Asn Gly Ile His Tyr Lys Leu
            100                 105                 110

Gln Leu Leu Tyr Ser Asn Gly Val Arg Thr Glu Gln Asp Leu Tyr Val
        115                 120                 125

Arg Leu Ile Asp Ser Met Thr Lys Gln Ala Ile Val Tyr Glu Gly Gln
    130                 135                 140

Asp Lys Asn Pro Glu Met Cys Arg Val Leu Leu Thr His Glu Ile Met
145                 150                 155                 160

Cys Ser Arg Cys Cys Asp Lys Lys Ser Cys Gly Asn Arg Asn Glu Thr
                165                 170                 175

Pro Ser Asp Pro Val Ile Ile Asp Arg Phe Phe Leu Lys Phe Phe Leu
            180                 185                 190

Lys Cys Asn Gln Asn Cys Leu Lys Asn Ala Gly Asn Pro Arg Asp Met
        195                 200                 205

Arg Arg Phe Gln Val Val Val Ser Thr Thr Val Asn Val Asp Gly His
    210                 215                 220

Val Leu Ala Val Ser Asp Asn Met Phe Val His Asn Asn Ser Lys His
225                 230                 235                 240

Gly Arg Arg Ala Arg Arg Leu Asp Pro Ser Glu Gly Thr Ala Pro Ser
                245                 250                 255

Tyr Leu Glu Asn Ala Thr Pro Cys Ile Lys Ala Ile Ser Pro Ser Glu
            260                 265                 270

Gly Trp Thr Thr Gly Gly Ala Thr Val Ile Ile Ile Gly Asp Asn Phe
        275                 280                 285

Phe Asp Gly Leu Gln Val Val Phe Gly Thr Met Leu Val Trp Ser Glu
    290                 295                 300

Leu Ile Thr Pro His Ala Ile Arg Val Gln Thr Pro Pro Arg His Ile
305                 310                 315                 320

Pro Gly Val Val Glu Val Thr Leu Ser Tyr Lys Ser Lys Gln Phe Cys
                325                 330                 335

Lys Gly Ala Pro Gly Arg Phe Val Tyr Thr Ala Leu Asn Glu Pro Thr
            340                 345                 350

Ile Asp Tyr Gly Phe Gln Arg Leu Gln Lys Val Ile Pro Arg His Pro
        355                 360                 365

Gly Asp Pro Glu Arg Leu Pro Lys Glu Val Leu Leu Lys Arg Ala Ala
    370                 375                 380

Asp Leu Val Glu Ala Leu Tyr Gly Met Pro His Asn Asn Gln Glu Ile
385                 390                 395                 400

Ile Leu Lys Arg Ala Ala Asp Ile Ala Glu Ala Leu Tyr Ser Val Pro
                405                 410                 415

Arg Asn His Asn Gln Ile Pro Thr Leu Gly Asn Thr Pro Ala His Thr
            420                 425                 430

Gly Met Met Gly Val Asn Ser Phe Ser Ser Gln Leu Ala Val Asn Val
        435                 440                 445

Ser Glu Thr Ser Gln Ala Asn Asp Gln Val Gly Tyr Ser Arg Asn Thr
    450                 455                 460

Ser Ser Val Ser Pro Arg Gly Tyr Val Pro Ser Ser Thr Pro Gln Gln
465                 470                 475                 480

Ser Asn Tyr Asn Thr Val Ser Thr Ser Met Asn Gly Tyr Gly Ser Gly
                485                 490                 495

Ala Met Ala Asn Leu Gly Val Pro Gly Ser Pro Gly Phe Leu Asn Gly
            500                 505                 510

Ser Ser Ala Asn Ser Pro Tyr Gly Ile Val Pro Ser Ser Pro Thr Met
        515                 520                 525

Ala Ala Ser Ser Val Thr Leu Pro Ser Asn Cys Ser Ser Thr His Gly
    530                 535                 540

Ile Phe Ser Phe Ser Pro Ala Asn Val Ile Ser Ala Val Lys Gln Lys
545                 550                 555                 560

Ser Ala Phe Ala Pro Val Val Arg Pro Gln Ala Ser Pro Pro Pro Ser
                565                 570                 575

Cys Thr Ser Ala Asn Gly Asn Gly Leu Gln Ala Met Ser Gly Leu Val
            580                 585                 590

Val Pro Pro Met
        595

<210> 145
<211> 582
<212> PRT
<213> Homo sapiens

<400> 145
Met Lys Glu Glu Pro Leu Gly Ser Gly Met Asn Pro Val Arg Ser Trp
1               5                   10                  15

Met His Thr Ala Gly Val Val Asp Ala Asn Thr Ala Ala Gln Ser Gly
            20                  25                  30

Val Gly Leu Ala Arg Ala His Phe Glu Lys Gln Pro Pro Ser Asn Leu
        35                  40                  45

Arg Lys Ser Asn Phe Phe His Phe Val Leu Ala Leu Tyr Asp Arg Gln
    50                  55                  60

Gly Gln Pro Val Glu Ile Glu Arg Thr Ala Phe Val Asp Phe Val Glu
65                  70                  75                  80

Lys Glu Lys Glu Pro Asn Asn Glu Lys Thr Asn Asn Gly Ile His Tyr
                85                  90                  95

Lys Leu Gln Leu Leu Tyr Ser Asn Gly Val Arg Thr Glu Gln Asp Leu
            100                 105                 110

Tyr Val Arg Leu Ile Asp Ser Met Thr Lys Gln Ala Ile Val Tyr Glu
        115                 120                 125

Gly Gln Asp Lys Asn Pro Glu Met Cys Arg Val Leu Leu Thr His Glu
    130                 135                 140

Ile Met Cys Ser Arg Cys Cys Asp Lys Lys Ser Cys Gly Asn Arg Asn
145                 150                 155                 160

Glu Thr Pro Ser Asp Pro Val Ile Ile Asp Arg Phe Phe Leu Lys Phe
                165                 170                 175

Phe Leu Lys Cys Asn Gln Asn Cys Leu Lys Asn Ala Gly Asn Pro Arg
            180                 185                 190

Asp Met Arg Arg Phe Gln Val Val Val Ser Thr Thr Val Asn Val Asp
        195                 200                 205

Gly His Val Leu Ala Val Ser Asp Asn Met Phe Val His Asn Asn Ser
    210                 215                 220

Lys His Gly Arg Arg Ala Arg Arg Leu Asp Pro Ser Glu Gly Thr Ala
225                 230                 235                 240

Pro Ser Tyr Leu Glu Asn Ala Thr Pro Cys Ile Lys Ala Ile Ser Pro
                245                 250                 255

Ser Glu Gly Trp Thr Thr Gly Gly Ala Thr Val Ile Ile Ile Gly Asp
            260                 265                 270

Asn Phe Phe Asp Gly Leu Gln Val Val Phe Gly Thr Met Leu Val Trp
        275                 280                 285

Ser Glu Leu Ile Thr Pro His Ala Ile Arg Val Gln Thr Pro Pro Arg
        290                 295                 300

His Ile Pro Gly Val Val Glu Val Thr Leu Ser Tyr Lys Ser Lys Gln
305                 310                 315                 320

Phe Cys Lys Gly Ala Pro Gly Arg Phe Val Tyr Thr Ala Leu Asn Glu
                325                 330                 335

Pro Thr Ile Asp Tyr Gly Phe Gln Arg Leu Gln Lys Val Ile Pro Arg
            340                 345                 350

His Pro Gly Asp Pro Glu Arg Leu Pro Lys Glu Val Leu Leu Lys Arg
        355                 360                 365

Ala Ala Asp Leu Val Glu Ala Leu Tyr Gly Met Pro His Asn Asn Gln
    370                 375                 380

Glu Ile Ile Leu Lys Arg Ala Ala Asp Ile Ala Glu Ala Leu Tyr Ser
385                 390                 395                 400

Val Pro Arg Asn His Asn Gln Ile Pro Thr Leu Gly Asn Asn Pro Ala
                405                 410                 415

His Thr Gly Met Met Gly Val Asn Ser Phe Ser Ser Gln Leu Ala Val
            420                 425                 430

Asn Val Ser Glu Thr Ser Gln Ala Asn Asp Gln Val Gly Tyr Ser Arg
        435                 440                 445

Asn Thr Ser Ser Val Ser Pro Arg Gly Tyr Val Pro Ser Ser Thr Pro
    450                 455                 460

Gln Gln Ser Asn Tyr Asn Thr Val Ser Thr Ser Met Asn Gly Tyr Gly
465                 470                 475                 480

Ser Gly Ala Met Ala Ser Leu Gly Val Pro Gly Ser Pro Gly Phe Leu
                485                 490                 495

Asn Gly Ser Ser Ala Asn Ser Pro Tyr Gly Ile Val Pro Ser Ser Pro
            500                 505                 510

Thr Met Ala Ala Ser Ser Val Thr Leu Pro Ser Asn Cys Ser Ser Thr
        515                 520                 525

His Gly Ile Phe Ser Phe Ser Pro Ala Asn Val Ile Ser Ala Val Lys
    530                 535                 540

Gln Lys Ser Ala Phe Ala Pro Val Val Arg Pro Gln Ala Ser Pro Pro
545                 550                 555                 560

Pro Ser Cys Thr Ser Ala Asn Gly Asn Gly Leu Gln Ala Met Ser Gly
                565                 570                 575

Leu Val Val Pro Pro Met
            580

<210> 146
<211> 598
<212> PRT
<213> Xenopus laevis

<400> 146
Met Phe Gly Ile Gln Glu Asn Ile Pro Arg Gly Gly Thr Thr Met Lys
  1               5                  10                  15

Glu Glu Pro Leu Gly Gly Gly Met Asn Pro Val Arg Ser Trp Met His

20                  25                  30

Thr Ala Gly Val Val Asp Ala Asn Thr Ala Ala Gln Ser Gly Val Gly
        35                  40                  45

Leu Ala Arg Ala His Phe Glu Lys Gln Pro Pro Ser Asn Leu Arg Lys
    50                  55                  60

Ser Asn Phe Phe His Phe Val Leu Ala Met Tyr Asp Arg Gln Gly Gln
65                  70                  75                  80

Pro Val Glu Ile Glu Arg Thr Thr Phe Val Asp Phe Val Glu Lys Asp
                85                  90                  95

Lys Glu Pro Asn Ser Glu Lys Thr Asn Asn Gly Ile His Tyr Lys Leu
            100                 105                 110

Gln Leu Leu Tyr Ser Asn Gly Val Arg Thr Glu Gln Asp Leu Tyr Val
        115                 120                 125

Arg Leu Ile Asp Ser Met Thr Lys Gln Ala Ile Ile Tyr Glu Gly Gln
    130                 135                 140

Asp Lys Asn Pro Glu Met Cys Arg Val Leu Leu Thr His Glu Ile Met
145                 150                 155                 160

Cys Ser Arg Cys Cys Asp Lys Lys Ser Cys Gly Asn Arg Asn Glu Thr
                165                 170                 175

Pro Ser Asp Pro Val Ile Ile Asp Arg Phe Phe Leu Lys Phe Phe Leu
            180                 185                 190

Lys Cys Asn Gln Asn Cys Leu Lys Asn Ala Gly Asn Pro Arg Asp Met
        195                 200                 205

Arg Arg Phe Gln Val Val Val Ser Thr Thr Val Asn Val Asp Gly His
    210                 215                 220

Val Leu Ala Val Ser Asp Asn Met Phe Val His Asn Asn Ser Lys His
225                 230                 235                 240

Gly Arg Arg Ala Arg Arg Leu Asp Pro Ser Glu Gly Thr Ala Pro Ser
                245                 250                 255

Tyr Leu Glu Asn Val Ala Thr Pro Cys Ile Lys Ala Ile Ser Pro Ser
            260                 265                 270

Glu Gly Trp Thr Thr Gly Gly Ala Thr Val Ile Ile Ile Gly Asp Asn
        275                 280                 285

Phe Phe Asp Gly Leu Gln Val Val Phe Gly Thr Met Leu Val Trp Ser
    290                 295                 300

Glu Leu Ile Thr Pro His Ala Ile Arg Val Gln Thr Pro Pro Arg His
305                 310                 315                 320

Ile Pro Gly Val Val Glu Val Thr Leu Ser Tyr Lys Ser Lys Gln Phe
                325                 330                 335

Cys Lys Gly Ala Pro Gly Arg Phe Val Tyr Thr Ala Leu Asn Glu Pro
            340                 345                 350

Thr Ile Asp Tyr Gly Phe Gln Arg Leu Gln Lys Val Ile Pro Arg His
        355                 360                 365

Pro Gly Asp Pro Glu Arg Leu Pro Lys Glu Val Leu Leu Lys Arg Ala
    370                 375                 380

Ala Asp Leu Val Glu Ala Leu Tyr Gly Met Pro His Asn Asn Gln Glu

385                 390                 395                 400

Ile Ile Leu Lys Arg Ala Ala Asp Ile Ala Glu Ala Leu Tyr Ser Val
                405                 410                 415

Pro Arg Asn His Asn Gln Ile Pro Ser Leu Ala Asn Thr Pro Ser His
            420                 425                 430

Ser Gly Met Met Gly Val Asn Ser Phe Ser Ser Gln Leu Ala Val Asn
        435                 440                 445

Val Ser Glu Thr Ser Gln Ala Asn Asp Gln Val Gly Tyr Ser Arg Asn
    450                 455                 460

Thr Ser Ser Val Ser Pro Arg Gly Tyr Val Pro Ser Ser Thr Pro Gln
465                 470                 475                 480

Gln Ser Asn Tyr Asn Thr Val Ser Asn Ser Met Asn Gly Tyr Gly Asn
                485                 490                 495

Ala Gly Met Pro Asn Leu Gly Val Pro Gly Ser Pro Gly Phe Leu Asn
            500                 505                 510

Gly Ser Ser Ala Asn Ser Pro Tyr Gly Ile Val Pro Ser Ser Pro Thr
        515                 520                 525

Met Ala Ala Ser Ser Val Thr Leu Pro Ser Asn Cys Ser Ser Thr His
    530                 535                 540

Gly Ile Phe Ser Phe Ser Pro Ala Asn Val Ile Ser Ala Val Lys Gln
545                 550                 555                 560

Lys Ser Ala Phe Ala Pro Val Val Arg Pro Gln Ala Ser Pro Pro Pro
                565                 570                 575

Ser Cys Thr Ser Ala Asn Gly Asn Gly Leu Gln Asp Met Tyr Phe Ser
            580                 585                 590

Pro Thr Phe Ser Lys Ser
        595


<210> 147
<211> 551
<212> PRT
<213> Mus musculus

<400> 147
Met Phe Gly Ile Gln Glu Asn Ile Pro Arg Gly Gly Thr Thr Met Lys
1               5                   10                  15

Glu Glu Pro Leu Gly Ser Gly Met Asn Pro Val Arg Ser Trp Met His
            20                  25                  30

Thr Ala Gly Val Val Asp Ala Asn Thr Ala Ala Gln Ser Gly Val Gly
        35                  40                  45

Leu Ala Arg Ala His Phe Glu Lys Gln Pro Pro Ser Asn Leu Arg Lys
    50                  55                  60

Ser Asn Phe Phe His Phe Val Leu Ala Leu Tyr Asp Arg Gln Gly Gln
65                  70                  75                  80

Pro Val Glu Ile Glu Arg Thr Ala Phe Val Asp Phe Val Glu Lys Glu
                85                  90                  95

Lys Glu Pro Asn Asn Glu Lys Thr Asn Asn Gly Ile His Tyr Lys Leu
            100                 105                 110

Gln Leu Leu Tyr Ser Asn Gly Val Arg Thr Glu Gln Asp Leu Tyr Val
        115                 120                 125

Arg Leu Ile Asp Ser Met Thr Lys Gln Ala Ile Val Tyr Glu Gly Gln
    130                 135                 140

Asp Lys Asn Pro Glu Met Cys Arg Val Leu Leu Thr His Glu Ile Met
145                 150                 155                 160

Cys Ser Arg Cys Cys Asp Lys Lys Ser Cys Gly Asn Arg Asn Glu Thr
                165                 170                 175

Pro Ser Asp Pro Val Ile Ile Asp Arg Phe Phe Leu Lys Phe Phe Leu
            180                 185                 190

Lys Cys Asn Gln Asn Cys Leu Lys Asn Ala Gly Asn Pro Arg Asp Met
        195                 200                 205

Arg Arg Phe Gln Val Val Val Ser Thr Thr Val Asn Val Asp Gly His
    210                 215                 220

Val Leu Ala Val Ser Asp Asn Met Phe Val His Asn Asn Ser Lys His
225                 230                 235                 240

Gly Arg Arg Ala Arg Arg Leu Asp Pro Ser Glu Ala Thr Pro Cys Ile
                245                 250                 255

Lys Ala Ile Ser Pro Ser Glu Gly Trp Thr Thr Gly Gly Ala Thr Val
            260                 265                 270

Ile Ile Ile Gly Asp Asn Phe Phe Asp Gly Leu Gln Val Val Phe Gly
        275                 280                 285

Thr Met Leu Val Trp Ser Glu Leu Ile Thr Pro His Ala Ile Arg Val
    290                 295                 300

Gln Thr Pro Pro Arg His Ile Pro Gly Val Val Glu Val Thr Leu Ser
305                 310                 315                 320

Tyr Lys Ser Lys Gln Phe Cys Lys Gly Ala Pro Gly Arg Phe Val Tyr
                325                 330                 335

Thr Ala Leu Asn Glu Pro Thr Ile Asp Tyr Gly Phe Gln Arg Leu Gln
            340                 345                 350

Lys Val Ile Pro Arg His Pro Gly Asp Pro Glu Arg Leu Pro Lys Glu
        355                 360                 365

Val Leu Leu Lys Arg Ala Ala Asp Leu Val Glu Ala Leu Tyr Gly Met
    370                 375                 380

Pro His Asn Asn Gln Glu Ile Ile Leu Lys Arg Ala Ala Asp Ile Ala
385                 390                 395                 400

Glu Ala Leu Tyr Ser Val Pro Arg Asn His Asn Gln Ile Pro Thr Leu
                405                 410                 415

Gly Asn Thr Pro Ala His Thr Gly Met Met Gly Val Asn Ser Phe Ser
            420                 425                 430

Ser Gln Leu Ala Val Asn Val Ser Glu Thr Ser Gln Ala Asn Asp Gln
        435                 440                 445

Val Gly Tyr Ser Arg Asn Thr Ser Ser Val Ser Pro Arg Gly Tyr Val
    450                 455                 460

Pro Ser Ser Thr Pro Gln Gln Ser Asn Tyr Asn Thr Val Ser Thr Ser
465                 470                 475                 480

Met Asn Gly Tyr Gly Ser Gly Ala Met Ala Asn Leu Gly Val Pro Gly
                485                 490                 495

Ser Pro Gly Phe Leu Asn Gly Ser Ser Ala Asn Ser Pro Tyr Gly Met
            500                 505                 510

Lys Gln Lys Ser Ala Phe Ala Pro Val Val Arg Pro Gln Ala Ser Pro
        515                 520                 525

Pro Pro Ser Cys Thr Ser Ala Asn Gly Asn Gly Leu Gln Ala Met Ser
    530                 535                 540

Gly Leu Val Val Pro Pro Met
545                 550


<210> 148
<211> 85
<212> PRT
<213> Homo sapiens

<400> 148
Pro Val Ile Thr Ser Ile Ser Pro Ser Ser Gly Pro Leu Ser Gly Gly
  1               5                  10                  15

Thr Glu Ile Thr Ile Thr Gly Ser Asn Leu Gly Ser Gly Glu Asp Ile
             20                  25                  30

Lys Val Thr Phe Gly Gly Thr Glu Cys Asp Val Val Ser Gln Glu Ala
         35                  40                  45

Ser Gln Ile Val Cys Lys Thr Pro Pro Tyr Ala Asn Gly Gly Pro Gln
     50                  55                  60

Pro Val Thr Val Ser Leu Asp Gly Gly Gly Leu Ser Ser Ser Pro Val
 65                  70                  75                  80

Thr Phe Thr Tyr Val
                 85


<210> 149
<211> 93
<212> PRT
<213> Homo sapiens

<400> 149
Asp Pro Val Ile Thr Arg Ile Ser Pro Asn Ser Gly Pro Leu Ser Gly
  1               5                  10                  15

Gly Thr Arg Ile Thr Leu Cys Gly Lys Asn Leu Asp Ser Ile Ser Val
             20                  25                  30

Val Phe Val Glu Val Gly Val Gly Glu Val Pro Cys Thr Phe Leu Pro
         35                  40                  45

Ser Asp Val Ser Gln Thr Ala Ile Val Cys Lys Thr Pro Pro Tyr His
     50                  55                  60

Asn Ile Pro Gly Ser Val Pro Val Arg Val Glu Val Gly Leu Arg Asn
 65                  70                  75                  80

Gly Gly Val Pro Gly Glu Pro Ser Pro Phe Thr Tyr Val
                 85                  90


<210> 150
<211> 53
<212> PRT

<213> Homo sapiens

<400> 150
Asn Ala Arg Glu Arg Arg Arg Arg Arg Lys Ile Asn Glu Ala Phe Asp
1               5                   10                  15

Glu Leu Arg Ser Leu Leu Pro Pro Leu Pro Asn Asn Lys Lys Leu Ser
            20                  25                  30

Lys Ala Ser Ile Leu Arg Leu Ala Ile Asp Tyr Ile Lys Ser Leu Gln
        35                  40                  45

Glu Gln Leu Gln Lys
    50


<210> 151
<211> 829
<212> PRT
<213> Homo sapiens

<400> 151


<210> 152
<211> 829
<212> PRT
<213> Homo sapiens

<400> 152
Met Gly Cys Ala Pro Ser Ile His Ile Ser Glu Arg Leu Val Ala Glu
1               5                   10                  15

Asp Ala Pro Ser Pro Ala Ala Pro Pro Leu Ser Ser Gly Gly Pro Arg
            20                  25                  30

Leu Pro Gln Gly Gln Lys Thr Ala Ala Leu Pro Arg Thr Arg Gly Ala
        35                  40                  45

Gly Leu Leu Glu Ser Glu Leu Arg Asp Gly Ser Gly Lys Lys Val Ala
    50                  55                  60

Val Ala Asp Val Gln Phe Gly Pro Met Arg Phe His Gln Asp Gln Leu
65                  70                  75                  80

Gln Val Leu Leu Val Phe Thr Lys Glu Asp Asn Gln Cys Asn Gly Phe
                85                  90                  95

Cys Arg Ala Cys Glu Lys Ala Gly Phe Lys Cys Thr Val Thr Lys Glu
            100                 105                 110

Ala Gln Ala Val Leu Ala Cys Phe Leu Asp Lys His His Asp Ile Ile

115                 120                 125

Ile Ile Asp His Arg Asn Pro Arg Gln Leu Asp Ala Glu Ala Leu Cys
    130                 135                 140

Arg Ser Ile Arg Ser Ser Lys Leu Ser Glu Asn Thr Val Ile Val Gly
145                 150                 155                 160

Val Val Arg Arg Val Asp Arg Glu Glu Leu Ser Val Met Pro Phe Ile
                165                 170                 175

Ser Ala Gly Phe Thr Arg Arg Tyr Val Glu Asn Pro Asn Ile Met Ala
            180                 185                 190

Cys Tyr Asn Glu Leu Leu Gln Leu Glu Phe Gly Glu Val Arg Ser Gln
        195                 200                 205

Leu Lys Leu Arg Ala Cys Asn Ser Val Phe Thr Ala Leu Glu Asn Ser
    210                 215                 220

Glu Asp Ala Ile Glu Ile Thr Ser Glu Asp Arg Phe Ile Gln Tyr Ala
225                 230                 235                 240

Asn Pro Ala Phe Glu Thr Thr Met Gly Tyr Gln Ser Gly Glu Leu Ile
                245                 250                 255

Gly Lys Glu Leu Gly Glu Val Pro Ile Asn Glu Lys Lys Ala Asp Leu
            260                 265                 270

Leu Asp Thr Ile Asn Ser Cys Ile Arg Ile Gly Lys Glu Trp Gln Gly
        275                 280                 285

Ile Tyr Tyr Ala Lys Lys Lys Asn Gly Asp Asn Ile Gln Gln Asn Val
    290                 295                 300

Lys Ile Ile Pro Val Ile Gly Gln Gly Gly Lys Ile Arg His Tyr Val
305                 310                 315                 320

Ser Ile Ile Arg Val Cys Asn Gly Asn Asn Lys Ala Glu Lys Ile Ser
                325                 330                 335

Glu Cys Val Gln Ser Asp Thr His Thr Asp Asn Gln Thr Gly Lys His
            340                 345                 350

Lys Asp Arg Arg Lys Gly Ser Leu Asp Val Lys Ala Val Ala Ser Arg
        355                 360                 365

Ala Thr Glu Val Ser Ser Gln Arg Arg His Ser Ser Met Ala Arg Ile
    370                 375                 380

His Ser Met Thr Ile Glu Ala Pro Ile Thr Lys Val Ile Asn Ile Ile
385                 390                 395                 400

Asn Ala Ala Gln Glu Ser Ser Pro Met Pro Val Thr Glu Ala Leu Asp
                405                 410                 415

Arg Val Leu Glu Ile Leu Arg Thr Thr Glu Leu Tyr Ser Pro Gln Phe
            420                 425                 430

Gly Ala Lys Asp Asp Asp Pro His Ala Asn Asp Leu Val Gly Gly Leu
        435                 440                 445

Met Ser Asp Gly Leu Arg Arg Leu Ser Gly Asn Glu Tyr Val Leu Ser
    450                 455                 460

Thr Lys Asn Thr Gln Met Val Ser Ser Asn Ile Ile Thr Pro Ile Ser
465                 470                 475                 480

Leu Asp Asp Val Pro Pro Arg Ile Ala Arg Ala Met Glu Asn Glu Glu

                485                 490                 495

Tyr Trp Asp Phe Asp Ile Phe Glu Leu Glu Ala Ala Thr His Asn Arg
            500                 505                 510

Pro Leu Ile Tyr Leu Gly Leu Lys Met Phe Ala Arg Phe Gly Ile Cys
        515                 520                 525

Glu Phe Leu His Cys Ser Glu Ser Thr Leu Arg Ser Trp Leu Gln Ile
    530                 535                 540

Ile Glu Ala Asn Tyr His Ser Ser Asn Pro Tyr His Asn Ser Thr His
545                 550                 555                 560

Ser Ala Asp Val Leu His Ala Thr Ala Tyr Phe Leu Ser Lys Glu Arg
                565                 570                 575

Ile Lys Glu Thr Leu Asp Pro Ile Asp Glu Val Ala Ala Leu Ile Ala
            580                 585                 590

Ala Thr Ile His Asp Val Asp His Pro Gly Arg Thr Asn Ser Phe Leu
        595                 600                 605

Cys Asn Ala Gly Ser Glu Leu Ala Ile Leu Tyr Asn Asp Thr Ala Val
    610                 615                 620

Leu Glu Ser His His Ala Ala Leu Ala Phe Gln Leu Thr Thr Gly Asp
625                 630                 635                 640

Asp Lys Cys Asn Ile Phe Lys Asn Met Glu Arg Asn Asp Tyr Arg Thr
                645                 650                 655

Leu Arg Gln Gly Ile Ile Asp Met Val Leu Ala Thr Glu Met Thr Lys
            660                 665                 670

His Phe Glu His Val Asn Lys Phe Val Asn Ser Ile Asn Lys Pro Leu
        675                 680                 685

Ala Thr Leu Glu Glu Asn Gly Glu Thr Asp Lys Asn Gln Glu Val Ile
    690                 695                 700

Asn Thr Met Leu Arg Thr Pro Glu Asn Arg Thr Leu Ile Lys Arg Met
705                 710                 715                 720

Leu Ile Lys Cys Ala Asp Val Ser Asn Pro Cys Arg Pro Leu Gln Tyr
                725                 730                 735

Cys Ile Glu Trp Ala Ala Arg Ile Ser Glu Glu Tyr Phe Ser Gln Thr
            740                 745                 750

Asp Glu Glu Lys Gln Gln Gly Leu Pro Val Val Met Pro Val Phe Asp
        755                 760                 765

Arg Asn Thr Cys Ser Ile Pro Lys Ser Gln Ile Ser Phe Ile Asp Tyr
    770                 775                 780

Phe Ile Thr Asp Met Phe Asp Ala Trp Asp Ala Phe Val Asp Leu Pro
785                 790                 795                 800

Asp Leu Met Gln His Leu Asp Asn Asn Phe Lys Tyr Trp Lys Gly Leu
                805                 810                 815

Asp Glu Met Lys Leu Arg Asn Leu Arg Pro Pro Pro Glu
            820                 825

<210> 153
<211> 757
<212> PRT

<213> Homo sapiens

<400> 153
Met Arg Phe His Gln Asp Gln Leu Gln Val Leu Leu Val Phe Thr Lys
1               5                   10                  15

Glu Asp Asn Gln Cys Asn Gly Phe Cys Arg Ala Cys Glu Lys Ala Gly
            20                  25                  30

Phe Lys Cys Thr Val Thr Lys Glu Ala Gln Ala Val Leu Ala Cys Phe
        35                  40                  45

Leu Asp Lys His His Asp Ile Ile Ile Ile Asp His Arg Asn Pro Arg
    50                  55                  60

Gln Leu Asp Ala Glu Ala Leu Cys Arg Ser Ile Arg Ser Ser Lys Leu
65                  70                  75                  80

Ser Glu Asn Thr Val Ile Val Gly Val Val Arg Arg Val Asp Arg Glu
                85                  90                  95

Glu Leu Ser Val Met Pro Phe Ile Ser Ala Gly Phe Thr Arg Arg Tyr
            100                 105                 110

Val Glu Asn Pro Asn Ile Met Ala Cys Tyr Asn Glu Leu Leu Gln Leu
        115                 120                 125

Glu Phe Gly Glu Val Arg Ser Gln Leu Lys Leu Arg Ala Cys Asn Ser
    130                 135                 140

Val Phe Thr Ala Leu Glu Asn Ser Glu Asp Ala Ile Glu Ile Thr Ser
145                 150                 155                 160

Glu Asp Arg Phe Ile Gln Tyr Ala Asn Pro Ala Phe Glu Thr Thr Met
                165                 170                 175

Gly Tyr Gln Ser Gly Glu Leu Ile Gly Lys Glu Leu Gly Glu Val Pro
            180                 185                 190

Ile Asn Glu Lys Lys Ala Asp Leu Leu Asp Thr Ile Asn Ser Cys Ile
        195                 200                 205

Arg Ile Gly Lys Glu Trp Gln Gly Ile Tyr Tyr Ala Lys Lys Lys Asn
    210                 215                 220

Gly Asp Asn Ile Gln Gln Asn Val Lys Ile Ile Pro Val Ile Gly Gln
225                 230                 235                 240

Gly Gly Lys Ile Arg His Tyr Val Ser Ile Ile Arg Val Cys Asn Gly
                245                 250                 255

Asn Asn Lys Ala Glu Lys Ile Ser Glu Cys Val Gln Ser Asp Thr His
            260                 265                 270

Thr Asp Asn Gln Thr Gly Lys His Lys Asp Arg Arg Lys Gly Ser Leu
        275                 280                 285

Asp Val Lys Ala Val Ala Ser Arg Ala Thr Glu Val Ser Ser Gln Arg
    290                 295                 300

Arg His Ser Ser Met Ala Arg Ile His Ser Met Thr Ile Glu Ala Pro
305                 310                 315                 320

Ile Thr Lys Val Ile Asn Ile Ile Asn Ala Ala Gln Glu Ser Ser Pro
                325                 330                 335

Met Pro Val Thr Glu Ala Leu Asp Arg Val Leu Glu Ile Leu Arg Thr
            340                 345                 350

Thr Glu Leu Tyr Ser Pro Gln Phe Gly Ala Lys Asp Asp Asp Pro His
        355                 360                 365

Ala Asn Asp Leu Val Gly Gly Leu Met Ser Asp Gly Leu Arg Arg Leu
    370                 375                 380

Ser Gly Asn Glu Tyr Val Leu Ser Thr Lys Asn Thr Gln Met Val Ser
385                 390                 395                 400

Ser Asn Ile Ile Thr Pro Ile Ser Leu Asp Asp Val Pro Pro Arg Ile
                405                 410                 415

Ala Arg Ala Met Glu Asn Glu Glu Tyr Trp Asp Phe Asp Ile Phe Glu
            420                 425                 430

Leu Glu Ala Ala Thr His Asn Arg Pro Leu Ile Tyr Leu Gly Leu Lys
        435                 440                 445

Met Phe Ala Arg Phe Gly Ile Cys Glu Phe Leu His Cys Ser Glu Ser
    450                 455                 460

Thr Leu Arg Ser Trp Leu Gln Ile Ile Glu Ala Asn Tyr His Ser Ser
465                 470                 475                 480

Asn Pro Tyr His Asn Ser Thr His Ser Ala Asp Val Leu His Ala Thr
                485                 490                 495

Ala Tyr Phe Leu Ser Lys Glu Arg Ile Lys Glu Thr Leu Asp Pro Ile
            500                 505                 510

Asp Glu Val Ala Ala Leu Ile Ala Ala Thr Ile His Asp Val Asp His
        515                 520                 525

Pro Gly Arg Thr Asn Ser Phe Leu Cys Asn Ala Gly Ser Glu Leu Ala
    530                 535                 540

Ile Leu Tyr Asn Asp Thr Ala Val Leu Glu Ser His His Ala Ala Leu
545                 550                 555                 560

Ala Phe Gln Leu Thr Thr Gly Asp Asp Lys Cys Asn Ile Phe Lys Asn
                565                 570                 575

Met Glu Arg Asn Asp Tyr Arg Thr Leu Arg Gln Gly Ile Ile Asp Met
            580                 585                 590

Val Leu Ala Thr Glu Met Thr Lys His Phe Glu His Val Asn Lys Phe
        595                 600                 605

Val Asn Ser Ile Asn Lys Pro Leu Ala Thr Leu Glu Glu Asn Gly Glu
    610                 615                 620

Thr Asp Lys Asn Gln Glu Val Ile Asn Thr Met Leu Arg Thr Pro Glu
625                 630                 635                 640

Asn Arg Thr Leu Ile Lys Arg Met Leu Ile Lys Cys Ala Asp Val Ser
                645                 650                 655

Asn Pro Cys Arg Pro Leu Gln Tyr Cys Ile Glu Trp Ala Ala Arg Ile
            660                 665                 670

Ser Glu Glu Tyr Phe Ser Gln Thr Asp Glu Glu Lys Gln Gln Gly Leu
        675                 680                 685

Pro Val Val Met Pro Val Phe Asp Arg Asn Thr Cys Ser Ile Pro Lys
    690                 695                 700

Ser Gln Ile Ser Phe Ile Asp Tyr Phe Ile Thr Asp Met Phe Asp Ala
705                 710                 715                 720

Trp Asp Ala Phe Val Asp Leu Pro Asp Leu Met Gln His Leu Asp Asn
                725                 730                 735

Asn Phe Lys Tyr Trp Lys Gly Leu Asp Glu Met Lys Leu Arg Asn Leu
            740                 745                 750

Arg Pro Pro Pro Glu
        755


<210> 154
<211> 783
<212> PRT
<213> Homo sapiens

<400> 154
Met Gly Cys Ala Pro Ser Ile His Ile Ser Glu Arg Leu Val Ala Glu
  1               5                  10                  15

Asp Ala Pro Ser Pro Ala Ala Pro Pro Leu Ser Ser Gly Gly Pro Arg
            20                  25                  30

Leu Pro Gln Gly Gln Lys Thr Ala Ala Leu Pro Arg Thr Arg Gly Ala
        35                  40                  45

Gly Leu Leu Glu Ser Glu Leu Arg Asp Gly Ser Gly Lys Lys Val Ala
    50                  55                  60

Val Ala Asp Val Gln Phe Gly Pro Met Arg Phe His Gln Asp Gln Leu
 65                  70                  75                  80

Gln Val Leu Leu Val Phe Thr Lys Glu Asp Asn Gln Cys Asn Gly Phe
                85                  90                  95

Cys Arg Ala Cys Glu Lys Ala Gly Phe Lys Cys Thr Val Thr Lys Glu
            100                 105                 110

Ala Gln Ala Val Leu Ala Cys Phe Leu Asp Lys His His Asp Ile Ile
        115                 120                 125

Ile Ile Asp His Arg Asn Pro Arg Gln Leu Asp Ala Glu Ala Leu Cys
    130                 135                 140

Arg Ser Ile Arg Ser Ser Lys Leu Ser Glu Asn Thr Val Ile Val Gly
145                 150                 155                 160

Val Val Arg Arg Val Asp Arg Glu Glu Leu Ser Val Met Pro Phe Ile
                165                 170                 175

Ser Ala Gly Phe Thr Arg Arg Tyr Val Glu Asn Pro Asn Ile Met Ala
            180                 185                 190

Cys Tyr Asn Glu Leu Leu Gln Leu Glu Phe Gly Glu Val Arg Ser Gln
        195                 200                 205

Leu Lys Leu Arg Ala Cys Asn Ser Val Phe Thr Ala Leu Glu Asn Ser
    210                 215                 220

Glu Asp Ala Ile Glu Ile Thr Ser Glu Asp Arg Phe Ile Gln Glu Trp
225                 230                 235                 240

Gln Gly Ile Tyr Tyr Ala Lys Lys Lys Asn Gly Asp Asn Ile Gln Gln
                245                 250                 255

Asn Val Lys Ile Ile Pro Val Ile Gly Gln Gly Gly Lys Ile Arg His
            260                 265                 270

Tyr Val Ser Ile Ile Arg Val Cys Asn Gly Asn Asn Lys Ala Glu Lys
        275                 280                 285

Ile Ser Glu Cys Val Gln Ser Asp Thr His Thr Asp Asn Gln Thr Gly
    290                 295                 300

Lys His Lys Asp Arg Arg Lys Gly Ser Leu Asp Val Lys Ala Val Ala
305                 310                 315                 320

Ser Arg Ala Thr Glu Val Ser Ser Gln Arg Arg His Ser Ser Met Ala
                325                 330                 335

Arg Ile His Ser Met Thr Ile Glu Ala Pro Ile Thr Lys Val Ile Asn
            340                 345                 350

Ile Ile Asn Ala Ala Gln Glu Ser Ser Pro Met Pro Val Thr Glu Ala
        355                 360                 365

Leu Asp Arg Val Leu Glu Ile Leu Arg Thr Thr Glu Leu Tyr Ser Pro
    370                 375                 380

Gln Phe Gly Ala Lys Asp Asp Asp Pro His Ala Asn Asp Leu Val Gly
385                 390                 395                 400

Gly Leu Met Ser Asp Gly Leu Arg Arg Leu Ser Gly Asn Glu Tyr Val
                405                 410                 415

Leu Ser Thr Lys Asn Thr Gln Met Val Ser Ser Asn Ile Ile Thr Pro
            420                 425                 430

Ile Ser Leu Asp Asp Val Pro Pro Arg Ile Ala Arg Ala Met Glu Asn
        435                 440                 445

Glu Glu Tyr Trp Asp Phe Asp Ile Phe Glu Leu Glu Ala Ala Thr His
    450                 455                 460

Asn Arg Pro Leu Ile Tyr Leu Gly Leu Lys Met Phe Ala Arg Phe Gly
465                 470                 475                 480

Ile Cys Glu Phe Leu His Cys Ser Glu Ser Thr Leu Arg Ser Trp Leu
                485                 490                 495

Gln Ile Ile Glu Ala Asn Tyr His Ser Ser Asn Pro Tyr His Asn Ser
            500                 505                 510

Thr His Ser Ala Asp Val Leu His Ala Thr Ala Tyr Phe Leu Ser Lys
        515                 520                 525

Glu Arg Ile Lys Glu Thr Leu Asp Pro Ile Asp Glu Val Ala Ala Leu
    530                 535                 540

Ile Ala Ala Thr Ile His Asp Val Asp His Pro Gly Arg Thr Asn Ser
545                 550                 555                 560

Phe Leu Cys Asn Ala Gly Ser Glu Leu Ala Ile Leu Tyr Asn Asp Thr
                565                 570                 575

Ala Val Leu Glu Ser His His Ala Ala Leu Ala Phe Gln Leu Thr Thr
            580                 585                 590

Gly Asp Asp Lys Cys Asn Ile Phe Lys Asn Met Glu Arg Asn Asp Tyr
        595                 600                 605

Arg Thr Leu Arg Gln Gly Ile Ile Asp Met Val Leu Ala Thr Glu Met
    610                 615                 620

Thr Lys His Phe Glu His Val Asn Lys Phe Val Asn Ser Ile Asn Lys
625                 630                 635                 640

Pro Leu Ala Thr Leu Glu Glu Asn Gly Glu Thr Asp Lys Asn Gln Glu
                645                 650                 655

Val Ile Asn Thr Met Leu Arg Thr Pro Glu Asn Arg Thr Leu Ile Lys
            660                 665                 670

Arg Met Leu Ile Lys Cys Ala Asp Val Ser Asn Pro Cys Arg Pro Leu
        675                 680                 685

Gln Tyr Cys Ile Glu Trp Ala Ala Arg Ile Ser Glu Glu Tyr Phe Ser
    690                 695                 700

Gln Thr Asp Glu Glu Lys Gln Gln Gly Leu Pro Val Val Met Pro Val
705                 710                 715                 720

Phe Asp Arg Asn Thr Cys Ser Ile Pro Lys Ser Gln Ile Ser Phe Ile
                725                 730                 735

Asp Tyr Phe Ile Thr Asp Met Phe Asp Ala Trp Asp Ala Phe Val Asp
            740                 745                 750

Leu Pro Asp Leu Met Gln His Leu Asp Asn Asn Phe Lys Tyr Trp Lys
        755                 760                 765

Gly Leu Asp Glu Met Lys Leu Arg Asn Leu Arg Pro Pro Pro Glu
    770                 775                 780


<210> 155
<211> 713
<212> PRT
<213> Homo sapiens

<400> 155
Leu Ala Cys Phe Leu Asp Lys His His Asp Ile Ile Ile Ile Asp His
1               5                   10                  15

Arg Asn Pro Arg Gln Leu Asp Ala Glu Ala Leu Cys Arg Ser Ile Arg
            20                  25                  30

Ser Ser Lys Leu Ser Glu Asn Thr Val Ile Val Gly Val Val Arg Arg
        35                  40                  45

Val Asp Arg Glu Glu Leu Ser Val Met Pro Phe Ile Ser Ala Gly Phe
    50                  55                  60

Thr Arg Arg Tyr Val Glu Asn Pro Asn Ile Met Ala Cys Tyr Asn Glu
65                  70                  75                  80

Leu Leu Gln Leu Glu Phe Gly Glu Val Arg Ser Gln Leu Lys Leu Arg
                85                  90                  95

Ala Cys Asn Ser Val Phe Thr Ala Leu Glu Asn Ser Glu Asp Ala Ile
            100                 105                 110

Glu Ile Thr Ser Glu Asp Arg Phe Ile Gln Tyr Ala Asn Pro Ala Phe
        115                 120                 125

Glu Thr Thr Met Gly Tyr Gln Ser Gly Glu Leu Ile Gly Lys Glu Leu
    130                 135                 140

Gly Glu Val Pro Ile Asn Glu Lys Lys Ala Asp Leu Leu Asp Thr Ile
145                 150                 155                 160

Asn Ser Cys Ile Arg Ile Gly Lys Glu Trp Gln Gly Ile Tyr Tyr Ala
                165                 170                 175

Lys Lys Lys Asn Gly Asp Asn Ile Gln Gln Asn Val Lys Ile Ile Pro
            180                 185                 190

Val Ile Gly Gln Gly Gly Lys Ile Arg His Tyr Val Ser Ile Ile Arg

195                 200                 205

Val Cys Asn Gly Asn Asn Lys Ala Glu Lys Ile Ser Glu Cys Val Gln
    210                 215                 220

Ser Asp Thr Arg Thr Asp Asn Gln Thr Gly Lys His Lys Asp Arg Arg
225                 230                 235                 240

Lys Gly Ser Leu Asp Val Lys Ala Val Ala Ser Arg Ala Thr Glu Val
                245                 250                 255

Ser Ser Gln Arg Arg His Ser Ser Met Ala Arg Ile His Ser Met Thr
            260                 265                 270

Ile Glu Ala Pro Ile Thr Lys Val Ile Asn Val Ile Asn Ala Ala Gln
        275                 280                 285

Glu Ser Ser Pro Met Pro Val Thr Glu Ala Leu Asp Arg Val Leu Glu
    290                 295                 300

Ile Leu Arg Thr Thr Glu Leu Tyr Ser Pro Gln Phe Gly Ala Lys Asp
305                 310                 315                 320

Asp Asp Pro His Ala Asn Asp Leu Val Gly Gly Leu Met Ser Asp Gly
                325                 330                 335

Leu Arg Arg Leu Ser Gly Asn Glu Tyr Val Leu Ser Thr Lys Asn Thr
            340                 345                 350

Gln Met Val Ser Ser Asn Ile Ile Thr Pro Ile Ser Leu Asp Asp Val
        355                 360                 365

Pro Pro Arg Ile Ala Arg Ala Met Glu Asn Glu Glu Tyr Trp Asp Phe
    370                 375                 380

Asp Ile Phe Glu Leu Glu Ala Ala Thr His Asn Arg Pro Leu Ile Tyr
385                 390                 395                 400

Leu Gly Leu Lys Met Phe Ala Arg Phe Gly Ile Cys Glu Phe Leu His
                405                 410                 415

Cys Ser Glu Ser Thr Leu Arg Ser Trp Leu Gln Ile Ile Glu Ala Asn
            420                 425                 430

Tyr His Ser Ser Asn Pro Tyr His Asn Ser Thr His Ser Ala Asp Val
        435                 440                 445

Leu His Ala Thr Ala Tyr Phe Leu Ser Lys Glu Arg Ile Lys Glu Thr
    450                 455                 460

Leu Asp Pro Ile Asp Glu Val Ala Ala Leu Ile Ala Ala Thr Ile His
465                 470                 475                 480

Asp Val Asp His Pro Gly Arg Thr Asn Ser Phe Leu Cys Asn Ala Gly
                485                 490                 495

Ser Glu Leu Ala Ile Leu Tyr Asn Asp Thr Ala Val Leu Glu Ser His
            500                 505                 510

His Ala Ala Leu Ala Phe Gln Leu Thr Thr Gly Asp Asp Lys Cys Asn
        515                 520                 525

Ile Phe Lys Asn Met Glu Arg Asn Asp Tyr Arg Thr Leu Arg Gln Gly
    530                 535                 540

Ile Ile Asp Met Val Leu Ala Thr Glu Met Thr Lys His Phe Glu His
545                 550                 555                 560

Val Asn Lys Phe Val Asn Ser Ile Asn Lys Pro Leu Ala Thr Leu Glu

                        565                     570                     575

Glu Asn Gly Glu Thr Asp Lys Asn Gln Glu Val Ile Asn Thr Met Leu
                580                 585                 590

Arg Thr Pro Glu Asn Arg Thr Leu Ile Lys Arg Met Leu Ile Lys Cys
            595                 600                 605

Ala Asp Val Ser Asn Pro Cys Arg Pro Leu Gln Tyr Cys Ile Glu Trp
    610                 615                 620

Ala Ala Arg Ile Ser Glu Glu Tyr Phe Ser Gln Thr Asp Glu Glu Lys
625                 630                 635                 640

Gln Gln Gly Leu Pro Val Val Met Pro Val Phe Asp Arg Asn Thr Cys
                645                 650                 655

Ser Ile Pro Lys Ser Gln Ile Ser Phe Ile Asp Tyr Phe Ile Thr Asp
                660                 665                 670

Met Phe Asp Ala Trp Asp Ala Phe Val Asp Leu Pro Asp Leu Met Gln
            675                 680                 685

His Leu Asp Asn Asn Phe Lys Tyr Trp Lys Gly Leu Asp Glu Met Lys
    690                 695                 700

Leu Arg Asn Leu Arg Pro Pro Pro Glu
705                 710


<210> 156
<211> 240
<212> PRT
<213> Homo sapiens

<400> 156
Tyr His Asn Trp Arg His Ala Phe Asp Val Thr Gln Thr Thr His Leu
  1               5                  10                  15

Leu Leu Leu Thr Leu Ala Leu Asp Arg Tyr Leu Thr Asp Leu Glu Ile
             20                  25                  30

Leu Ala Leu Val Phe Ala Ala Ala Cys His Asp Val Asp His Arg Gly
         35                  40                  45

Thr Asn Asn Ser Phe Gln Ile Gln Ser Lys Ser Glu Leu Ala Ile Leu
     50                  55                  60

Tyr Asn Asp Glu Ser Val Leu Glu Asn His His Leu Ala Gln Gly Ile
 65                  70                  75                  80

Lys Leu Leu Gln Asp Glu Glu Cys Asn Ile Phe Ile Asn Leu Ser Lys
                 85                  90                  95

Lys Asp Phe Gln Thr Leu Arg Asp Leu Val Ile Glu Met Ile Leu Ala
            100                 105                 110

Thr Asp Met Ser Leu His Phe Gln Lys Glu Lys Arg Leu Lys Thr Met
        115                 120                 125

Val Glu Gln Lys Lys Thr Tyr Met Leu Asp Asn Gln Thr His Lys Ile
    130                 135                 140

Leu Leu Met Ser Leu Ile Met Thr Ala Ala Asp Leu Ser Asn Pro Thr
145                 150                 155                 160

Lys Ser Trp Ser Val His Arg Arg Trp Ala Glu Leu Ile Met Glu Glu
                165                 170                 175

Phe Phe Asp Gln Gly Asp Leu Glu Arg Glu Leu Gly Leu Pro Pro Ser
            180                 185                 190

Pro Met Cys Asp Arg Thr Ser Ala Tyr Val Pro Lys Ser Gln Ile Gly
        195                 200                 205

Phe Ile Asp Phe Ile Val Glu Pro Ile Phe Lys Leu Leu Ala Asp Val
    210                 215                 220

Thr Glu Lys Asp Ile Ile Pro Leu Leu Asp Arg Ile Glu Asp Asn Arg
225                 230                 235                 240

<210> 157
<211> 65
<212> PRT
<213> Homo sapiens

<400> 157
Glu Asp Tyr Arg Ala Ile Leu Glu Ser Leu Pro Asp Pro Ile Phe Val
1               5                   10                  15

Val Asp Glu Asp Gly Arg Ile Leu Tyr Trp Asn Ala Ala Ala Glu Glu
            20                  25                  30

Leu Thr Gly Leu Ser Arg Glu Glu Val Ile Gly Lys Ser Leu Leu Asp
        35                  40                  45

Leu Val His Glu Glu Asp Leu Ala Arg Val Arg Glu Ile Leu Gln Thr
    50                  55                  60

Ala
65

<210> 158
<211> 67
<212> PRT
<213> Homo sapiens

<400> 158
Glu Arg Leu Arg Ala Ile Leu Glu Ser Leu Pro Asp Gly Val Phe Val
1               5                   10                  15

Leu Asp Leu Asp Gly Arg Ile Leu Tyr Ala Asn Pro Ala Ala Glu Glu
            20                  25                  30

Leu Leu Gly Tyr Ser Pro Glu Glu Leu Ile Gly Lys Ser Leu Leu Glu
        35                  40                  45

Leu Ile His Pro Glu Asp Arg Glu Glu Leu Gln Glu Arg Leu Gln Arg
    50                  55                  60

Leu Leu Ser
65

<210> 159
<211> 1581
<212> PRT
<213> Homo sapiens

<400> 159


<210> 160
<211> 2035
<212> PRT
<213> Mus musculus

<400> 160
Met Phe Asn Leu Met Lys Lys Asp Lys Asp Lys Asp Gly Gly Arg Lys
  1               5                  10                  15

Glu Lys Lys Glu Lys Lys Glu Lys Lys Glu Arg Met Ser Ala Ala Glu
             20                  25                  30

Leu Arg Ser Leu Glu Glu Met Ser Met Arg Arg Gly Phe Phe Asn Leu
         35                  40                  45

Asn Arg Ser Ser Lys Arg Glu Ser Lys Thr Arg Leu Glu Ile Ser Asn
     50                  55                  60

Pro Ile Pro Ile Lys Val Ala Ser Gly Ser Asp Leu His Leu Thr Asp
 65                  70                  75                  80

Ile Asp Ser Asp Ser Asn Arg Gly Ser Ile Ile Leu Asp Ser Gly His
                 85                  90                  95

Leu Ser Thr Ala Ser Ser Ser Asp Asp Leu Lys Gly Glu Glu Gly Ser
            100                 105                 110

Phe Arg Gly Ser Val Leu Gln Arg Ala Ala Lys Phe Gly Ser Leu Ala
        115                 120                 125

Lys Gln Asn Ser Gln Met Ile Val Lys Arg Phe Ser Phe Ser Gln Arg
    130                 135                 140

Ser Arg Asp Glu Ser Ala Ser Glu Thr Ser Thr Pro Ser Glu His Ser
145                 150                 155                 160

Ala Ala Pro Ser Pro Gln Val Glu Val Arg Thr Leu Glu Gly Gln Leu
                165                 170                 175

Met Gln His Pro Gly Leu Gly Ile Pro Arg Pro Gly Pro Arg Ser Arg
            180                 185                 190

Val Pro Glu Leu Val Thr Lys Arg Phe Pro Ala Asp Leu Arg Leu Pro
        195                 200                 205

Ala Leu Val Pro Pro Pro Pro Pro Ala Leu Arg Glu Leu Glu Leu Gln
    210                 215                 220

Arg Arg Pro Thr Gly Asp Phe Gly Phe Ser Leu Arg Arg Thr Thr Met
225                 230                 235                 240

Leu Asp Arg Ala Pro Glu Gly Gln Ala Tyr Arg Arg Val Val His Phe
                245                 250                 255

Ala Glu Pro Gly Ala Gly Thr Lys Asp Leu Ala Leu Gly Leu Val Pro
            260                 265                 270

Gly Asp Arg Leu Val Glu Ile Asn Gly Gln Asn Val Glu Asn Lys Ser
        275                 280                 285

Arg Asp Glu Ile Val Glu Met Ile Arg Gln Ser Gly Asp Ser Val Arg
    290                 295                 300

Leu Lys Val Gln Pro Ile Pro Glu Leu Ser Glu Leu Ser Arg Ser Trp
305                 310                 315                 320

Leu Arg Thr Gly Glu Gly His Arg Arg Glu Pro Ala Asp Ala Lys Thr
                325                 330                 335

Glu Glu Gln Ile Ala Ala Glu Glu Ala Trp Tyr Glu Thr Glu Lys Val
            340                 345                 350

Trp Leu Val His Arg Asp Gly Phe Ser Leu Ala Ser Gln Leu Lys Ser
        355                 360                 365

Glu Glu Leu Ser Leu Pro Glu Gly Lys Ala Arg Val Lys Leu Asp His
    370                 375                 380

Asp Gly Ala Ile Leu Asp Val Asp Glu Asp Asp Ile Glu Lys Ala Asn
385                 390                 395                 400

Ala Pro Ser Cys Asp Arg Leu Glu Asp Leu Ala Ser Leu Val Tyr Leu
                405                 410                 415

Asn Glu Ser Ser Val Leu His Thr Leu Arg Gln Arg Tyr Gly Ala Ser
            420                 425                 430

Leu Leu His Thr Tyr Ala Gly Pro Ser Leu Leu Val Leu Ser Thr Arg
        435                 440                 445

Gly Ala Pro Ala Val Tyr Ser Glu Lys Val Met His Met Phe Lys Gly
    450                 455                 460

Cys Arg Arg Glu Asp Met Ala Pro His Ile Tyr Ala Val Ala Gln Thr
465                 470                 475                 480

Ala Tyr Arg Ala Met Leu Met Ser Arg Gln Asp Gln Ser Ile Val Leu
                485                 490                 495

Leu Gly Ser Ser Gly Ser Gly Lys Thr Thr Ser Phe Gln His Leu Val
            500                 505                 510

Gln Tyr Leu Ala Thr Ile Ala Gly Thr Ser Gly Thr Lys Val Phe Ser
        515                 520                 525

Val Glu Lys Trp Gln Ala Leu Ser Thr Leu Leu Glu Ala Phe Gly Asn
    530                 535                 540

Ser Pro Thr Ile Met Asn Gly Ser Ala Thr Arg Phe Ser Gln Ile Leu
545                 550                 555                 560

Ser Leu Asp Phe Asp Gln Ala Gly Gln Val Ala Ser Ala Ser Ile Gln
                565                 570                 575

Thr Met Leu Leu Glu Lys Leu Arg Val Ala Arg Arg Pro Ala Ser Glu
            580                 585                 590

Ala Thr Phe Asn Val Phe Tyr Tyr Leu Leu Ala Cys Gly Asp Ala Thr
        595                 600                 605

Leu Arg Thr Glu Leu His Leu Asn His Leu Ala Glu Asn Asn Val Phe
    610                 615                 620

Gly Ile Val Pro Leu Ser Lys Pro Glu Glu Lys Gln Lys Ala Ala Gln
625                 630                 635                 640

Gln Phe Ser Lys Leu Gln Ala Ala Met Lys Val Leu Ala Ile Ser Pro
                645                 650                 655

Glu Glu Gln Lys Thr Cys Trp Leu Ile Leu Ala Ser Ile Tyr His Leu
            660                 665                 670

Gly Ala Ala Gly Ala Thr Lys Glu Ala Ala Glu Ala Gly Arg Lys Gln
        675                 680                 685

Phe Ala Arg His Glu Trp Ala Gln Lys Ala Ala Tyr Leu Leu Gly Cys
    690                 695                 700

Ser Leu Glu Glu Leu Ser Ser Ala Ile Phe Lys His Gln Leu Lys Gly
705                 710                 715                 720

Gly Thr Leu Gln Arg Ser Thr Ser Phe Arg Gln Gly Pro Glu Glu Ser
                725                 730                 735

Gly Leu Gly Glu Gly Thr Lys Leu Ser Ala Leu Glu Cys Leu Glu Gly
            740                 745                 750

Met Ala Ser Gly Leu Tyr Ser Glu Leu Phe Thr Leu Leu Ile Ser Leu
        755                 760                 765

Val Asn Arg Ala Leu Lys Ser Ser Gln His Ser Leu Cys Ser Met Met
    770                 775                 780

Ile Val Asp Thr Pro Gly Phe Gln Asn Pro Glu Trp Gly Gly Ser Ala
785                 790                 795                 800

Arg Gly Ala Ser Phe Glu Glu Leu Cys His Asn Tyr Ala Gln Asp Arg
                805                 810                 815

Leu Gln Arg Leu Phe His Glu Arg Thr Phe Leu Gln Glu Leu Glu Arg
            820                 825                 830

Tyr Lys Glu Asp Asn Ile Glu Leu Ala Phe Asp Asp Leu Glu Pro Val
        835                 840                 845

Ala Asp Asp Ser Val Ala Ala Val Asp Gln Ala Ser His Leu Val Arg
    850                 855                 860

Ser Leu Ala His Ala Asp Glu Ala Arg Gly Leu Leu Trp Leu Leu Glu
865                 870                 875                 880

Glu Glu Ala Leu Val Pro Gly Ala Thr Glu Asp Ala Leu Leu Asp Arg
                885                 890                 895

Leu Phe Ser Tyr Tyr Gly Pro Gln Glu Gly Asp Lys Lys Gly Gln Ser
            900                 905                 910

Pro Leu Leu Arg Ser Ser Lys Pro Arg His Phe Leu Leu Gly His Ser
        915                 920                 925

His Gly Thr Asn Trp Val Glu Tyr Asn Val Ala Gly Trp Leu Asn Tyr
    930                 935                 940

Thr Lys Gln Asn Pro Ala Thr Gln Asn Ala Pro Arg Leu Leu Gln Asp
945                 950                 955                 960

Ser Gln Lys Lys Ile Ile Ser Asn Leu Phe Leu Gly Arg Ala Gly Ser
                965                 970                 975

Ala Thr Val Leu Ser Gly Ser Ile Ala Gly Leu Glu Gly Gly Ser Gln
            980                 985                 990

Leu Ala Leu Arg Arg Ala Thr Ser Met Arg Lys Thr Phe Thr Thr Gly
        995                 1000                1005

Met Ala Ala Val Lys Lys Lys Ser Leu Cys Ile Gln Ile Lys Leu Gln
    1010                1015                1020

Val Asp Ala Leu Ile Asp Thr Ile Lys Arg Ser Lys Met His Phe Val
1025                1030                1035                1040

His Cys Phe Leu Pro Val Ala Glu Gly Trp Pro Gly Glu Pro Arg Ser
                1045                1050                1055

Ala Ser Ser Arg Arg Val Ser Ser Ser Ser Glu Leu Asp Leu Pro Pro
            1060                1065                1070

Gly Asp Pro Cys Glu Ala Gly Leu Leu Gln Leu Asp Val Ser Leu Leu
        1075                1080                1085

Arg Ala Gln Leu Arg Gly Ser Arg Leu Leu Asp Ala Met Arg Met Tyr
    1090                1095                1100

Arg Gln Gly Tyr Pro Asp His Met Val Phe Ser Glu Phe Arg Arg Arg
1105                1110                1115                1120

Phe Asp Val Leu Ala Pro His Leu Thr Lys Lys His Gly Arg Asn Tyr
                1125                1130                1135

Ile Val Val Asp Glu Lys Arg Ala Val Glu Glu Leu Leu Glu Ser Leu
            1140                1145                1150

Asp Leu Glu Lys Ser Ser Cys Cys Leu Gly Leu Ser Arg Val Phe Phe
        1155                1160                1165

Arg Ala Gly Thr Leu Ala Arg Leu Glu Glu Gln Arg Asp Glu Gln Thr
    1170                1175                1180

Ser Arg His Leu Thr Leu Phe Gln Ala Ala Cys Arg Gly Tyr Leu Ala
1185                1190                1195                1200

Arg Gln His Phe Lys Lys Arg Lys Ile Gln Asp Leu Ala Ile Arg Cys
                1205                1210                1215

Val Gln Lys Asn Ile Lys Lys Asn Lys Gly Val Lys Asp Trp Pro Trp
            1220                1225                1230

Trp Lys Leu Phe Thr Thr Val Arg Pro Leu Ile Gln Val Gln Leu Ser
        1235                1240                1245

Glu Glu Gln Ile Arg Asn Lys Asp Glu Glu Ile Gln Gln Leu Arg Ser
    1250                1255                1260

Lys Leu Glu Lys Val Glu Lys Glu Arg Asn Glu Leu Arg Leu Ser Ser
1265                1270                1275                1280

Asp Arg Leu Glu Thr Arg Ile Ser Glu Leu Thr Ser Glu Leu Thr Asp
                1285                1290                1295

Glu Arg Asn Thr Gly Glu Ser Ala Ser Gln Leu Leu Asp Ala Glu Thr
            1300                1305                1310

Ala Glu Arg Leu Arg Thr Glu Lys Glu Met Lys Glu Leu Gln Thr Gln
        1315                1320                1325

Tyr Asp Ala Leu Lys Lys Gln Met Glu Val Met Glu Met Glu Val Met
    1330                1335                1340

Glu Ala Arg Leu Ile Arg Ala Ala Glu Ile Asn Gly Glu Val Asp Asp
1345                1350                1355                1360

Asp Asp Ala Gly Gly Glu Trp Arg Leu Lys Tyr Glu Arg Ala Val Arg
                1365                1370                1375

Glu Val Asp Phe Thr Lys Lys Arg Leu Gln Gln Glu Leu Glu Asp Lys
            1380                1385                1390

Met Glu Val Glu Gln Gln Ser Arg Arg Gln Leu Glu Arg Arg Leu Gly
        1395                1400                1405

Asp Leu Gln Ala Asp Ser Asp Glu Ser Gln Arg Ala Leu Gln Gln Leu
    1410                1415                1420

Lys Lys Lys Cys Gln Arg Leu Thr Ala Glu Leu Gln Asp Thr Lys Leu
1425                1430                1435                1440

His Leu Glu Gly Gln Gln Val Arg Asn His Glu Leu Glu Lys Lys Gln
                1445                1450                1455

Arg Arg Phe Asp Ser Glu Leu Ser Gln Ala His Glu Glu Thr Gln Arg
            1460                1465                1470

Glu Lys Leu Gln Arg Glu Lys Leu Gln Arg Glu Lys Asp Met Leu Leu
        1475                1480                1485

Ala Glu Ala Phe Ser Leu Lys Gln Gln Met Glu Glu Lys Asp Leu Asp
    1490                1495                1500

Ile Ala Gly Phe Thr Gln Lys Val Val Ser Leu Glu Ala Glu Leu Gln
1505                1510                1515                1520

Asp Ile Ser Ser Gln Glu Ser Lys Asp Glu Ala Ser Leu Ala Lys Val
                1525                1530                1535

Lys Lys Gln Leu Arg Asp Leu Glu Ala Lys Val Lys Asp Gln Glu Glu
            1540                1545                1550

Glu Leu Asp Glu Gln Ala Gly Ser Ile Gln Met Leu Glu Gln Ala Lys
        1555                1560                1565

Leu Arg Leu Glu Met Glu Met Glu Arg Met Arg Gln Thr His Ser Lys
    1570                1575                1580

Glu Met Glu Ser Arg Asp Glu Glu Val Glu Glu Ala Arg Gln Ser Cys
1585                1590                1595                1600

Gln Lys Lys Leu Lys Gln Met Glu Val Gln Leu Glu Glu Glu Tyr Glu
                1605                1610                1615

Asp Lys Gln Lys Ala Leu Arg Glu Lys Arg Glu Leu Glu Ser Lys Leu
            1620                1625                1630

Ser Thr Leu Ser Asp Gln Val Asn Gln Arg Asp Phe Glu Ser Glu Lys
        1635                1640                1645

Arg Leu Arg Lys Asp Leu Lys Arg Thr Lys Ala Leu Leu Ala Asp Ala
    1650                1655                1660

Gln Ile Met Leu Asp His Leu Lys Asn Asn Ala Pro Ser Lys Arg Glu
1665                1670                1675                1680

Ile Ala Gln Leu Lys Asn Gln Leu Glu Glu Ser Glu Phe Thr Cys Ala
                1685                1690                1695

Ala Ala Val Lys Ala Arg Lys Ala Met Glu Val Glu Met Glu Asp Leu
            1700                1705                1710

His Leu Gln Ile Asp Asp Ile Ala Lys Ala Lys Thr Ala Leu Glu Glu
        1715                1720                1725

Gln Leu Ser Arg Leu Gln Arg Glu Lys Asn Glu Ile Gln Asn Arg Leu
    1730                1735                1740

Glu Glu Asp Gln Glu Asp Met Asn Glu Leu Met Lys Lys His Lys Ala
1745                1750                1755                1760

Ala Val Ala Gln Ala Ser Arg Asp Met Ala Gln Met Asn Asp Leu Gln
                1765                1770                1775

Ala Gln Ile Glu Glu Ser Asn Lys Glu Lys Gln Glu Leu Gln Glu Lys
            1780                1785                1790

Leu Gln Ala Leu Gln Ser Gln Val Glu Phe Leu Glu Gln Ser Met Val
        1795                1800                1805

Asp Lys Ser Leu Val Ser Arg Gln Glu Ala Lys Ile Arg Glu Leu Glu
    1810                1815                1820

Thr Arg Leu Glu Phe Glu Lys Thr Gln Val Lys Arg Leu Glu Asn Leu
1825                1830                1835                1840

Ala Ser Arg Leu Lys Glu Thr Met Glu Lys Leu Thr Glu Glu Arg Asp
                1845                1850                1855

Gln Arg Ala Ala Ala Glu Asn Arg Glu Lys Glu Gln Asn Lys Arg Leu
            1860                1865                1870

Gln Arg Gln Leu Arg Asp Thr Lys Glu Glu Met Ser Glu Leu Ala Arg
        1875                1880                1885

Lys Glu Ala Glu Ala Ser Arg Lys Lys His Glu Leu Glu Met Asp Leu
    1890                1895                1900

Glu Ser Leu Glu Ala Ala Asn Gln Ser Leu Gln Ala Asp Leu Lys Leu
1905                1910                1915                1920

Ala Phe Lys Arg Ile Gly Asp Leu Gln Ala Ala Ile Glu Asp Glu Met
                1925                1930                1935

Glu Ser Asp Glu Asn Glu Asp Leu Ile Asn Ser Glu Gly Asp Ser Asp
            1940                1945                1950

Val Asp Ser Glu Leu Glu Asp Arg Val Asp Gly Val Lys Ser Trp Leu
        1955                1960                1965

Ser Lys Asn Lys Gly Pro Ser Lys Ala Pro Ser Asp Asp Gly Ser Leu
    1970                1975                1980

Lys Ser Ser Ser Pro Thr Ser His Trp Lys Pro Leu Ala Pro Asp Pro
1985                1990                1995                2000

Ser Asp Asp Glu His Asp Pro Val Asp Ser Ile Ser Arg Pro Arg Phe
                2005                2010                2015

Ser His Ser Tyr Leu Ser Asp Ser Asp Thr Glu Ala Lys Leu Thr Glu
            2020                2025                2030

Thr Ser Ala
        2035


<210> 161
<211> 1355
<212> PRT
<213> Homo sapiens

<400> 161
Met Lys Val Leu Gly Ile Ser Pro Asp Glu Gln Lys Ala Cys Trp Phe
1               5                   10                  15

Ile Leu Ala Ala Ile Tyr His Leu Gly Ala Ala Gly Ala Thr Lys Glu
            20                  25                  30

Ala Ala Glu Ala Gly Arg Lys Gln Phe Ala Arg His Glu Trp Ala Gln
        35                  40                  45

Lys Ala Ala Tyr Leu Leu Gly Cys Ser Leu Glu Glu Leu Ser Ser Ala
    50                  55                  60

Ile Phe Lys His Gln His Lys Gly Gly Thr Leu Gln Arg Ser Thr Ser
65                  70                  75                  80

Phe Arg Gln Gly Pro Glu Glu Ser Gly Leu Gly Asp Gly Thr Gly Pro
                85                  90                  95

Lys Leu Ser Ala Leu Glu Cys Leu Glu Gly Met Ala Ala Gly Leu Tyr
            100                 105                 110

Ser Glu Leu Phe Thr Leu Leu Val Ser Leu Val Asn Arg Ala Leu Lys
        115                 120                 125

Ser Ser Gln His Ser Leu Cys Ser Met Met Ile Val Asp Thr Pro Gly
    130                 135                 140

Phe Gln Asn Pro Glu Gln Gly Gly Ser Ala Arg Gly Ala Ser Phe Glu
145                 150                 155                 160

Glu Leu Cys His Asn Tyr Thr Gln Asp Arg Leu Gln Arg Leu Phe His
                165                 170                 175

Glu Arg Thr Phe Val Gln Glu Leu Glu Arg Tyr Lys Glu Glu Asn Ile
            180                 185                 190

Glu Leu Ala Phe Asp Asp Leu Glu Pro Pro Thr Asp Asp Ser Val Ala
        195                 200                 205

Ala Val Asp Gln Ala Ser His Gln Ser Leu Val Arg Ser Leu Ala Arg
    210                 215                 220

Thr Asp Glu Ala Arg Gly Leu Leu Trp Leu Leu Glu Glu Glu Ala Leu
225                 230                 235                 240

Val Pro Gly Ala Ser Glu Asp Thr Leu Leu Glu Arg Leu Phe Ser Tyr
                245                 250                 255

Tyr Gly Pro Gln Glu Gly Asp Lys Lys Gly Gln Ser Pro Leu Leu His
            260                 265                 270

Ser Ser Lys Pro His His Phe Leu Leu Gly His Ser His Gly Thr Asn
        275                 280                 285

Trp Val Glu Tyr Asn Val Thr Gly Trp Leu Asn Tyr Thr Lys Gln Asn
    290                 295                 300

Pro Ala Thr Gln Asn Ala Pro Arg Leu Leu Gln Asp Ser Gln Lys Lys
305                 310                 315                 320

Ile Ile Ser Asn Leu Phe Leu Gly Arg Ala Gly Ser Ala Thr Val Leu
                325                 330                 335

Ser Gly Ser Ile Ala Gly Leu Glu Gly Gly Ser Gln Leu Ala Leu Arg
            340                 345                 350

Arg Ala Thr Ser Met Arg Lys Thr Phe Thr Thr Gly Met Ala Ala Val

        355                 360                 365

Lys Lys Lys Ser Leu Cys Ile Gln Met Lys Leu Gln Val Asp Ala Leu
    370                 375                 380

Ile Asp Thr Ile Lys Lys Ser Lys Leu His Phe Val His Cys Phe Leu
385                 390                 395                 400

Pro Val Ala Glu Gly Trp Ala Gly Glu Pro Arg Ser Ala Ser Ser Arg
                405                 410                 415

Arg Val Ser Ser Ser Ser Glu Leu Asp Leu Pro Ser Gly Asp His Cys
            420                 425                 430

Glu Ala Gly Leu Leu Gln Leu Asp Val Pro Leu Leu Arg Thr Gln Leu
        435                 440                 445

Arg Gly Ser Arg Leu Leu Asp Ala Met Arg Met Tyr Arg Gln Gly Tyr
    450                 455                 460

Pro Asp His Met Val Phe Ser Glu Phe Arg Arg Arg Phe Asp Val Leu
465                 470                 475                 480

Ala Pro His Leu Thr Lys Lys His Gly Arg Asn Tyr Ile Val Val Asp
                485                 490                 495

Glu Arg Arg Ala Val Glu Glu Leu Leu Glu Cys Leu Asp Leu Glu Lys
            500                 505                 510

Ser Ser Cys Cys Met Gly Leu Ser Arg Val Phe Phe Arg Ala Gly Thr
        515                 520                 525

Leu Ala Arg Leu Glu Glu Gln Arg Asp Glu Gln Thr Ser Arg Asn Leu
    530                 535                 540

Thr Leu Phe Gln Ala Ala Cys Arg Gly Tyr Leu Ala Arg Gln His Phe
545                 550                 555                 560

Lys Lys Arg Lys Ile Gln Asp Leu Ala Ile Arg Cys Val Gln Lys Asn
                565                 570                 575

Ile Lys Lys Asn Lys Gly Val Lys Asp Trp Pro Trp Trp Lys Leu Phe
            580                 585                 590

Thr Thr Val Arg Pro Leu Ile Glu Val Gln Leu Ser Glu Glu Gln Ile
        595                 600                 605

Arg Asn Lys Asp Glu Glu Ile Gln Gln Leu Arg Ser Lys Leu Glu Lys
    610                 615                 620

Ala Glu Lys Glu Arg Asn Glu Leu Arg Leu Asn Ser Asp Arg Leu Glu
625                 630                 635                 640

Ser Arg Ile Ser Glu Leu Thr Ser Glu Leu Thr Asp Glu Arg Asn Thr
                645                 650                 655

Gly Glu Ser Ala Ser Gln Leu Leu Asp Ala Glu Thr Ala Glu Arg Leu
            660                 665                 670

Arg Ala Glu Lys Glu Met Lys Glu Leu Gln Thr Gln Tyr Asp Ala Leu
        675                 680                 685

Lys Lys Gln Met Glu Val Met Glu Met Glu Val Met Glu Ala Arg Leu
    690                 695                 700

Ile Arg Ala Ala Glu Ile Asn Gly Glu Val Asp Asp Asp Asp Ala Gly
705                 710                 715                 720

Gly Glu Trp Arg Leu Lys Tyr Glu Arg Ala Val Arg Glu Val Asp Phe

                725                 730                 735

Thr Lys Lys Arg Leu Gln Gln Glu Phe Glu Asp Lys Leu Glu Val Glu
            740                 745                 750

Gln Gln Asn Lys Arg Gln Leu Glu Arg Arg Leu Gly Asp Leu Gln Ala
        755                 760                 765

Asp Ser Glu Glu Ser Gln Arg Ala Leu Gln Gln Leu Lys Lys Lys Cys
    770                 775                 780

Gln Arg Leu Thr Ala Glu Leu Gln Asp Thr Lys Leu His Leu Glu Gly
785                 790                 795                 800

Gln Gln Val Arg Asn His Glu Leu Glu Lys Lys Gln Arg Arg Phe Asp
                805                 810                 815

Ser Glu Leu Ser Gln Ala His Glu Glu Ala Gln Arg Glu Lys Leu Gln
            820                 825                 830

Arg Glu Lys Leu Gln Arg Glu Lys Asp Met Leu Leu Ala Glu Ala Phe
        835                 840                 845

Ser Leu Lys Gln Gln Leu Glu Glu Lys Asp Met Asp Ile Ala Gly Phe
    850                 855                 860

Thr Gln Lys Val Val Ser Leu Glu Ala Glu Leu Gln Asp Ile Ser Ser
865                 870                 875                 880

Gln Glu Ser Lys Asp Glu Ala Ser Leu Ala Lys Val Lys Lys Gln Leu
                885                 890                 895

Arg Asp Leu Glu Ala Lys Val Lys Asp Gln Glu Glu Glu Leu Asp Glu
            900                 905                 910

Gln Ala Gly Thr Ile Gln Met Leu Glu Gln Ala Lys Leu Arg Leu Glu
        915                 920                 925

Met Glu Met Glu Arg Met Arg Gln Thr His Ser Lys Glu Met Glu Ser
    930                 935                 940

Arg Asp Glu Glu Val Glu Glu Ala Arg Gln Ser Cys Gln Lys Lys Leu
945                 950                 955                 960

Lys Gln Met Glu Val Gln Leu Glu Glu Glu Tyr Glu Asp Lys Gln Lys
                965                 970                 975

Val Leu Arg Glu Lys Arg Glu Leu Glu Gly Lys Leu Ala Thr Leu Ser
            980                 985                 990

Asp Gln Val Asn Arg Arg Asp Phe Glu Ser Glu Lys Arg Leu Arg Lys
        995                 1000                1005

Asp Leu Lys Arg Thr Lys Ala Leu Leu Ala Asp Ala Gln Leu Met Leu
    1010                1015                1020

Asp His Leu Lys Asn Ser Ala Pro Ser Lys Arg Glu Ile Ala Gln Leu
1025                1030                1035                1040

Lys Asn Gln Leu Glu Glu Ser Glu Phe Thr Cys Ala Ala Ala Val Lys
                1045                1050                1055

Ala Arg Lys Ala Met Glu Val Glu Ile Glu Asp Leu His Leu Gln Ile
            1060                1065                1070

Asp Asp Ile Ala Lys Ala Lys Thr Ala Leu Glu Glu Gln Leu Ser Arg
        1075                1080                1085

Leu Gln Arg Glu Lys Asn Glu Ile Gln Asn Arg Leu Glu Glu Asp Gln

1090                1095                1100

Glu Asp Met Asn Glu Leu Met Lys Lys His Lys Ala Ala Val Ala Gln
1105                1110                1115                1120

Ala Ser Arg Asp Leu Ala Gln Ile Asn Asp Leu Gln Ala Gln Leu Glu
                1125                1130                1135

Glu Ala Asn Lys Glu Lys Gln Glu Leu Gln Glu Lys Leu Gln Ala Leu
            1140                1145                1150

Gln Ser Gln Val Glu Phe Leu Glu Gln Ser Met Val Asp Lys Ser Leu
        1155                1160                1165

Val Ser Arg Gln Glu Ala Lys Ile Arg Glu Leu Glu Thr Arg Leu Glu
    1170                1175                1180

Phe Glu Arg Thr Gln Val Lys Arg Leu Glu Ser Leu Ala Ser Arg Leu
1185                1190                1195                1200

Lys Glu Asn Met Glu Lys Leu Thr Glu Glu Arg Asp Gln Arg Ile Ala
                1205                1210                1215

Ala Glu Asn Arg Glu Lys Glu Gln Asn Lys Arg Leu Gln Arg Gln Leu
            1220                1225                1230

Arg Asp Thr Lys Glu Glu Met Gly Glu Leu Ala Arg Lys Glu Ala Glu
        1235                1240                1245

Ala Ser Arg Lys Lys His Glu Leu Glu Met Asp Leu Glu Ser Leu Glu
    1250                1255                1260

Ala Ala Asn Gln Ser Leu Gln Ala Asp Leu Lys Leu Ala Phe Lys Arg
1265                1270                1275                1280

Ile Gly Asp Leu Gln Ala Ala Ile Glu Asp Glu Met Glu Ser Asp Glu
                1285                1290                1295

Asn Glu Asp Leu Ile Asn Ser Leu Gln Asp Met Val Thr Lys Tyr Gln
            1300                1305                1310

Lys Arg Lys Asn Lys Leu Val Arg Glu Thr Leu Met Trp Thr Arg Ser
        1315                1320                1325

Trp Arg Thr Val Leu Thr Gly Ser Ser Pro Gly Cys Gln Lys Thr Arg
    1330                1335                1340

Asp Leu Pro Arg Gln Leu Leu Met Met Ala Ala
1345                1350                1355


<210> 162
<211> 2566
<212> PRT
<213> Homo sapiens

<400> 162
Met Ala Ile Ser Ser Arg Leu Ala Leu Trp Glu Gln Lys Ile Arg Glu
1               5                   10                  15

Glu Asp Lys Ser Pro Pro Pro Ser Ser Pro Pro Pro Leu Phe Ser Val
            20                  25                  30

Ile Pro Gly Gly Phe Ile Lys Gln Leu Val Arg Gly Thr Glu Lys Glu
        35                  40                  45

Ala Lys Glu Ala Arg Gln Arg Lys Gln Leu Ala Val Ala Ser Pro Glu
    50                  55                  60

Arg Glu Ile Pro Glu Ile Ser Ile Ser Gln Pro Asn Ser Lys Ser Ser
65                  70                  75                  80

Ser Gly Thr Arg Ser Gly Ser Gln Gln Ile Ser Gln Asp Asp Gln Ser
                85                  90                  95

Ser Ser Pro Gly Ser Ser Asp Ile Leu Gly Lys Glu Ser Glu Gly Ser
            100                 105                 110

Arg Ser Pro Asp Pro Glu Gln Met Thr Ser Ile Asn Gly Glu Lys Ala
        115                 120                 125

Gln Glu Leu Gly Ser Ser Ala Thr Pro Thr Lys Lys Thr Val Pro Phe
    130                 135                 140

Lys Arg Gly Val Arg Arg Gly Asp Val Leu Leu Met Val Ala Lys Leu
145                 150                 155                 160

Asp Pro Asp Ser Ala Lys Pro Glu Lys Thr His Pro His Asp Ala Pro
                165                 170                 175

Pro Cys Lys Thr Ser Pro Pro Ala Thr Asp Thr Gly Lys Glu Lys Lys
            180                 185                 190

Gly Glu Thr Ser Arg Thr Pro Cys Gly Ser Gln Ala Ser Thr Glu Ile
        195                 200                 205

Leu Ala Pro Lys Ala Glu Lys Thr Arg Thr Gly Gly Leu Gly Asp Pro
    210                 215                 220

Gly Gln Gly Thr Val Ala Leu Lys Lys Gly Glu Glu Gly Gln Ser Ile
225                 230                 235                 240

Val Gly Lys Gly Leu Gly Thr Pro Lys Thr Thr Glu Leu Lys Glu Ala
                245                 250                 255

Glu Pro Gln Gly Lys Asp Arg Gln Gly Thr Arg Pro Gln Ala Gln Gly
            260                 265                 270

Pro Gly Glu Gly Val Arg Pro Gly Lys Ala Glu Lys Glu Gly Ala Glu
        275                 280                 285

Pro Thr Asn Thr Val Glu Lys Gly Asn Val Ser Lys Asp Val Gly Ser
    290                 295                 300

Glu Gly Lys His Val Arg Pro Gln Ile Pro Gly Arg Lys Trp Gly Gly
305                 310                 315                 320

Phe Leu Gly Arg Arg Ser Lys Trp Asp Gly Pro Gln Asn Lys Lys Asp
                325                 330                 335

Lys Glu Gly Val Leu Leu Ser Lys Ala Glu Lys Thr Gly Glu Pro Gln
            340                 345                 350

Thr Gln Met Glu Lys Thr Ser Gln Val Gln Gly Glu Leu Gly Asp Asp
        355                 360                 365

Leu Arg Met Gly Glu Lys Ala Gly Glu Leu Arg Ser Thr Thr Gly Lys
    370                 375                 380

Ala Gly Glu Ser Trp Asp Lys Lys Glu Lys Met Gly Gln Pro Gln Gly
385                 390                 395                 400

Lys Ser Gly Asn Ala Gly Glu Ala Arg Ser Gln Thr Glu Lys Gly Cys
                405                 410                 415

Glu Ala Pro Lys Glu Val Ser Thr Met Val Glu Ser Pro Ala Ala Pro
            420                 425                 430

Gly Lys Gly Gly Trp Pro Gly Ser Arg Gly Gln Glu Ala Glu Glu Pro
        435                 440                 445

Cys Ser Arg Ala Gly Asp Gly Ala Gly Ala Leu Glu Thr Glu Leu Glu
    450                 455                 460

Gly Pro Ser Gln Pro Ala Leu Glu Lys Asp Ala Glu Arg Pro Arg Ile
465                 470                 475                 480

Arg Lys Glu Asn Gln Asp Gly Pro Ala Pro Gln Glu Glu Gly Lys Gly
                485                 490                 495

Gly Gln Ser Arg Asp Ser Asp Gln Ala Pro Glu Asp Arg Trp Tyr Glu
            500                 505                 510

Ala Glu Lys Val Trp Leu Ala Gln Lys Asp Gly Phe Thr Leu Ala Thr
        515                 520                 525

Val Leu Lys Pro Asp Glu Gly Thr Ala Asp Leu Pro Ala Gly Arg Val
    530                 535                 540

Arg Leu Trp Ile Asp Ala Asp Lys Thr Ile Thr Glu Val Asp Glu Glu
545                 550                 555                 560

His Val His Arg Ala Asn Pro Pro Glu Leu Asp Gln Val Glu Asp Leu
                565                 570                 575

Ala Ser Leu Ile Ser Val Asn Glu Ser Ser Val Leu Asn Thr Leu Leu
            580                 585                 590

Gln Arg Tyr Lys Ala Gln Leu Leu His Thr Cys Thr Gly Pro Asp Leu
        595                 600                 605

Ile Val Leu Gln Pro Arg Gly Pro Ser Val Pro Ser Ala Gly Lys Val
    610                 615                 620

Pro Lys Gly Arg Arg Asp Gly Leu Pro Ala His Ile Gly Ser Met Ala
625                 630                 635                 640

Gln Arg Ala Tyr Trp Ala Leu Leu Asn Gln Arg Arg Asp Gln Ser Ile
                645                 650                 655

Val Ala Leu Gly Trp Ser Gly Ala Gly Lys Thr Thr Cys Cys Glu Gln
            660                 665                 670

Val Leu Glu His Leu Val Gly Met Ala Gly Ser Val Asp Gly Arg Val
        675                 680                 685

Ser Val Glu Lys Ile Arg Ala Thr Phe Thr Val Leu Arg Ala Phe Gly
    690                 695                 700

Ser Val Ser Met Ala His Ser Arg Ser Ala Thr Arg Phe Ser Met Val
705                 710                 715                 720

Met Ser Leu Asp Phe Asn Ala Thr Gly Arg Ile Thr Ala Ala Gln Leu
                725                 730                 735

Gln Thr Met Leu Leu Glu Lys Ser Arg Val Ala Arg Gln Pro Glu Gly
            740                 745                 750

Glu Ser Asn Phe Leu Val Phe Ser Gln Met Leu Ala Gly Leu Asp Leu
        755                 760                 765

Asp Leu Arg Thr Glu Leu Asn Leu His Gln Met Ala Asp Ser Ser Ser
    770                 775                 780

Phe Gly Met Gly Val Trp Ser Lys Pro Glu Asp Lys Gln Lys Ala Ala
785                 790                 795                 800

Ala Ala Phe Ala Gln Leu Gln Gly Ala Met Glu Met Leu Gly Ile Ser
                805                 810                 815

Glu Ser Glu Gln Arg Ala Val Trp Arg Val Leu Ala Ala Ile Tyr His
            820                 825                 830

Leu Gly Ala Ala Gly Ala Cys Lys Val Gly Arg Lys Gln Phe Met Arg
        835                 840                 845

Phe Glu Trp Ala Asn Tyr Ala Ala Glu Ala Leu Gly Cys Glu Tyr Glu
    850                 855                 860

Glu Leu Asn Thr Ala Thr Phe Lys His His Leu Arg Gln Ile Ile Gln
865                 870                 875                 880

Gln Met Thr Phe Gly Pro Ser Arg Trp Gly Leu Glu Asp Glu Glu Thr
                885                 890                 895

Ser Ser Gly Leu Lys Met Thr Gly Val Asp Cys Val Glu Gly Met Ala
            900                 905                 910

Ser Gly Leu Tyr Gln Glu Leu Phe Ala Ala Val Val Leu Leu Ile Asn
        915                 920                 925

Arg Ser Phe Ser Ser His His Leu Ser Met Ala Ser Ile Met Val Val
    930                 935                 940

Asp Ser Pro Gly Phe Gln Asn Pro Arg His Gln Gly Lys Asp Arg Ala
945                 950                 955                 960

Ala Thr Phe Glu Glu Leu Cys His Asn Tyr Ala His Glu Arg Leu Gln
                965                 970                 975

Leu Leu Phe Tyr Gln Arg Thr Phe Val Ser Thr Leu Gln Arg Tyr Gln
            980                 985                 990

Glu Glu Gly Val Pro Val Gln Phe Asp Leu Pro Asp Pro Ser Pro Gly
        995                 1000                1005

Thr Thr Val Ala Val Val Asp Gln Asn Pro Ser Gln Val Arg Leu Pro
    1010                1015                1020

Ala Gly Gly Gly Ala Gln Asp Ala Arg Gly Leu Phe Trp Val Leu Asp
1025                1030                1035                1040

Glu Glu Val His Val Glu Gly Ser Ser Asp Ser Val Val Leu Glu Arg
                1045                1050                1055

Leu Cys Ala Ala Phe Glu Lys Lys Gly Ala Gly Thr Glu Gly Ser Ser
            1060                1065                1070

Ala Leu Arg Thr Cys Glu Gln Pro Leu Gln Cys Glu Ile Phe His Gln
        1075                1080                1085

Leu Gly Trp Asp Pro Val Arg Tyr Asp Leu Thr Gly Trp Leu His Arg
    1090                1095                1100

Ala Lys Pro Asn Leu Ser Ala Leu Asp Ala Pro Gln Val Leu His Gln
1105                1110                1115                1120

Ser Lys Arg Glu Glu Leu Arg Ser Leu Phe Gln Ala Arg Ala Lys Leu
                1125                1130                1135

Pro Pro Val Cys Arg Ala Val Ala Gly Leu Glu Gly Thr Ser Gln Gln
            1140                1145                1150

Ala Leu Gln Arg Ser Arg Met Val Arg Arg Thr Phe Ala Ser Ser Leu
        1155                1160                1165

Ala Ala Val Arg Arg Lys Ala Pro Cys Ser Gln Ile Lys Leu Gln Met
    1170                1175                1180

Asp Ala Leu Thr Ser Met Ile Lys Arg Ser Arg Leu His Phe Ile His
1185                1190                1195                1200

Cys Leu Val Pro Asn Pro Val Val Glu Ser Arg Ser Gly Gln Glu Ser
                1205                1210                1215

Pro Pro Pro Pro Gln Pro Gly Arg Asp Lys Pro Gly Ala Gly Gly Pro
            1220                1225                1230

Leu Ala Leu Asp Ile Pro Ala Leu Arg Val Gln Leu Ala Gly Phe His
        1235                1240                1245

Ile Leu Glu Ala Leu Arg Leu His Arg Thr Gly Tyr Ala Asp His Met
    1250                1255                1260

Gly Leu Thr Arg Phe Arg Arg Gln Phe Gln Val Leu Asp Ala Pro Leu
1265                1270                1275                1280

Leu Lys Lys Leu Met Ser Thr Ser Glu Gly Ile Asp Glu Arg Lys Ala
                1285                1290                1295

Val Glu Glu Leu Leu Glu Thr Leu Asp Leu Glu Lys Lys Ala Val Ala
            1300                1305                1310

Val Gly His Ser Gln Val Phe Leu Lys Ala Gly Val Ile Ser Arg Leu
        1315                1320                1325

Glu Lys Gln Arg Glu Lys Leu Val Ser Gln Ser Ile Val Leu Phe Gln
    1330                1335                1340

Ala Ala Cys Lys Gly Phe Leu Ser Arg Gln Glu Phe Lys Lys Leu Lys
1345                1350                1355                1360

Ile Arg Arg Leu Ala Ala Gln Cys Ile Gln Lys Asn Val Ala Val Phe
                1365                1370                1375

Leu Ala Val Lys Asp Trp Pro Trp Trp Gln Leu Leu Gly Ser Leu Gln
            1380                1385                1390

Pro Leu Leu Ser Ala Thr Ile Gly Thr Glu Gln Leu Arg Ala Lys Glu
        1395                1400                1405

Glu Glu Leu Thr Thr Leu Arg Arg Lys Leu Glu Lys Ser Glu Lys Leu
    1410                1415                1420

Arg Asn Glu Leu Arg Gln Asn Thr Asp Leu Leu Glu Ser Lys Ile Ala
1425                1430                1435                1440

Asp Leu Thr Ser Asp Leu Ala Asp Glu Arg Phe Lys Gly Asp Val Ala
                1445                1450                1455

Cys Gln Val Leu Glu Ser Glu Arg Ala Glu Arg Leu Gln Ala Phe Arg
            1460                1465                1470

Glu Val Gln Glu Leu Lys Ser Lys His Glu Gln Val Gln Lys Lys Leu
        1475                1480                1485

Gly Asp Val Asn Lys Gln Leu Glu Glu Ala Gln Gln Lys Ile Gln Leu
    1490                1495                1500

Asn Asp Leu Glu Arg Asn Pro Thr Gly Gly Asp Glu Trp Gln Met Arg
1505                1510                1515                1520

Phe Asp Cys Ala Gln Met Glu Asn Glu Phe Leu Arg Lys Arg Leu Gln
                1525                1530                1535

Gln Cys Glu Glu Arg Leu Asp Ser Glu Leu Thr Ala Arg Lys Glu Leu
            1540                1545                1550

Glu Gln Lys Leu Gly Glu Leu Gln Ser Ala Tyr Asp Gly Ala Lys Lys
        1555                1560                1565

Met Ala His Gln Leu Lys Arg Lys Cys His His Leu Thr Cys Asp Leu
    1570                1575                1580

Glu Asp Thr Cys Val Leu Leu Glu Asn Gln Gln Ser Arg Asn His Glu
1585                1590                1595                1600

Leu Glu Lys Lys Gln Lys Lys Phe Asp Leu Gln Leu Ala Gln Ala Leu
                1605                1610                1615

Gly Glu Ser Val Phe Glu Lys Gly Leu Arg Glu Lys Val Thr Gln Glu
            1620                1625                1630

Asn Thr Ser Val Arg Trp Glu Leu Gly Gln Leu Gln Gln Gln Leu Lys
        1635                1640                1645

Gln Lys Glu Gln Glu Ala Ser Gln Leu Lys Gln Gln Val Glu Met Leu
    1650                1655                1660

Gln Asp His Lys Arg Glu Leu Leu Gly Ser Pro Ser Leu Gly Glu Asn
1665                1670                1675                1680

Cys Val Ala Gly Leu Lys Glu Arg Leu Trp Lys Leu Glu Ser Ser Ala
                1685                1690                1695

Leu Glu Gln Gln Lys Ile Gln Ser Gln Gln Glu Asn Thr Ile Lys Gln
            1700                1705                1710

Leu Glu Gln Leu Arg Gln Arg Phe Glu Leu Glu Ile Glu Arg Met Lys
        1715                1720                1725

Gln Met His Gln Lys Asp Arg Glu Asp Gln Glu Glu Glu Leu Glu Asp
    1730                1735                1740

Val Arg Gln Ser Cys Gln Lys Arg Leu His Gln Leu Glu Met Gln Leu
1745                1750                1755                1760

Glu Gln Glu Tyr Glu Glu Lys Gln Met Val Leu His Glu Lys Gln Asp
                1765                1770                1775

Leu Glu Gly Leu Ile Gly Thr Leu Cys Asp Gln Ile Gly His Arg Asp
            1780                1785                1790

Phe Asp Val Glu Lys Arg Leu Arg Arg Asp Leu Arg Arg Thr His Ala
        1795                1800                1805

Leu Leu Ser Asp Val Gln Leu Leu Leu Gly Thr Met Glu Asp Gly Lys
    1810                1815                1820

Thr Ser Val Ser Lys Glu Glu Leu Glu Lys Val His Ser Gln Leu Glu
1825                1830                1835                1840

Gln Ser Glu Ala Lys Cys Glu Glu Ala Leu Lys Thr Gln Lys Val Leu
                1845                1850                1855

Thr Ala Asp Leu Glu Ser Met His Ser Glu Leu Glu Asn Met Thr Arg
            1860                1865                1870

Asn Lys Ser Leu Val Asp Glu Gln Leu Tyr Arg Leu Gln Phe Glu Lys
        1875                1880                1885

Ala Asp Leu Leu Lys Arg Ile Asp Glu Asp Gln Asp Asp Leu Asn Glu
    1890                1895                1900

Leu Met Gln Lys His Lys Asp Leu Ile Ala Gln Ser Ala Ala Asp Ile
1905                1910                1915                1920

Gly Gln Ile Gln Glu Leu Gln Leu Gln Leu Glu Glu Ala Lys Lys Glu
                1925                1930                1935

Lys His Lys Leu Gln Glu Gln Leu Gln Val Ala Gln Met Arg Ile Glu
            1940                1945                1950

Tyr Leu Glu Gln Ser Thr Val Asp Arg Ala Ile Val Ser Arg Gln Glu
        1955                1960                1965

Ala Val Ile Cys Asp Leu Glu Asn Lys Thr Glu Phe Gln Lys Val Gln
    1970                1975                1980

Ile Lys Arg Phe Glu Val Leu Val Ile Arg Leu Arg Asp Ser Leu Ile
1985                1990                1995                2000

Lys Met Gly Glu Glu Leu Ser Gln Ala Ala Thr Ser Glu Ser Gln Gln
                2005                2010                2015

Arg Glu Ser Ser Gln Tyr Tyr Gln Arg Arg Leu Glu Glu Leu Lys Ala
            2020                2025                2030

Asp Met Glu Glu Leu Val Gln Arg Glu Ala Glu Ala Ser Arg Arg Cys
        2035                2040                2045

Met Glu Leu Glu Lys Tyr Val Glu Glu Leu Ala Ala Val Arg Gln Thr
    2050                2055                2060

Leu Gln Thr Asp Leu Glu Thr Ser Ile Arg Arg Ile Ala Asp Leu Gln
2065                2070                2075                2080

Ala Ala Leu Glu Glu Val Ala Ser Ser Asp Ser Asp Thr Glu Ser Val
                2085                2090                2095

Gln Thr Ala Val Asp Cys Gly Ser Ser Gly Arg Lys Glu Met Asp Asn
            2100                2105                2110

Val Ser Ile Leu Ser Ser Gln Pro Glu Gly Ser Leu Gln Ser Trp Leu
        2115                2120                2125

Ser Cys Thr Leu Ser Leu Ala Thr Asp Thr Met Arg Thr Pro Ser Arg
    2130                2135                2140

Gln Ser Ala Thr Ser Ser Arg Ile Leu Ser Pro Arg Ile Asn Glu Glu
2145                2150                2155                2160

Ala Gly Asp Thr Glu Arg Thr Gln Ser Ala Leu Ala Leu Ser Arg Ala
                2165                2170                2175

Arg Ser Thr Asn Val His Ser Lys Thr Ser Gly Asp Lys Pro Val Ser
            2180                2185                2190

Pro His Phe Val Arg Arg Gln Lys Tyr Cys His Phe Gly Asp Gly Glu
        2195                2200                2205

Val Leu Ala Val Gln Arg Lys Ser Thr Glu Arg Leu Glu Pro Ala Ser
    2210                2215                2220

Ser Pro Leu Ala Ser Arg Ser Thr Asn Thr Ser Pro Leu Ser Arg Glu
2225                2230                2235                2240

Lys Leu Pro Ser Pro Ser Ala Ala Leu Ser Glu Phe Val Glu Gly Leu
                2245                2250                2255

Arg Arg Lys Arg Ala Gln Arg Gly Gln Gly Ser Thr Leu Gly Leu Glu
            2260                2265                2270

Asp Trp Pro Thr Leu Pro Ile Tyr Gln Thr Thr Gly Ala Ser Thr Leu
        2275                2280                2285

Arg Arg Gly Arg Ala Gly Ser Asp Glu Gly Asn Leu Ser Leu Arg Val
    2290                2295                2300

Gly Ala Lys Ser Pro Leu Glu Ile Glu Gly Ala Ala Gly Gly Leu Leu
2305                2310                2315                2320

Arg Ser Thr Ser Leu Lys Cys Ile Ser Ser Asp Gly Val Gly Gly Thr
                2325                2330                2335

Thr Leu Leu Pro Glu Lys Ser Lys Thr Gln Phe Ser Ser Cys Glu Ser
            2340                2345                2350

Leu Leu Glu Ser Arg Pro Ser Met Gly Arg Lys Leu Ser Ser Pro Thr
        2355                2360                2365

Thr Pro Arg Asp Met Leu Leu Ser Pro Thr Leu Arg Pro Arg Arg Arg
    2370                2375                2380

Cys Leu Glu Ser Ser Val Asp Asp Ala Gly Cys Pro Asp Leu Gly Lys
2385                2390                2395                2400

Glu Pro Leu Val Phe Gln Asn Arg Gln Phe Ala His Leu Met Glu Glu
                2405                2410                2415

Pro Leu Gly Ser Asp Pro Phe Ser Trp Lys Leu Pro Ser Leu Asp Tyr
            2420                2425                2430

Glu Arg Lys Thr Lys Val Asp Phe Asp Asp Phe Leu Pro Ala Ile Arg
        2435                2440                2445

Lys Pro Gln Thr Pro Thr Ser Leu Ala Gly Ser Ala Lys Gly Gly Gln
    2450                2455                2460

Asp Gly Ser Gln Arg Ser Ser Ile His Phe Glu Thr Glu Glu Ala Asn
2465                2470                2475                2480

Arg Ser Phe Leu Ser Gly Ile Lys Thr Ile Leu Lys Lys Ser Pro Glu
                2485                2490                2495

Pro Lys Glu Asp Pro Ala His Leu Ser Asp Ser Ser Ser Ser Ser Gly
            2500                2505                2510

Ser Ile Val Ser Phe Lys Ser Ala Asp Ser Ile Lys Ser Arg Pro Gly
        2515                2520                2525

Ile Pro Arg Leu Ala Gly Asp Gly Gly Glu Arg Thr Ser Pro Glu Arg
    2530                2535                2540

Arg Glu Pro Gly Thr Gly Arg Lys Asp Asp Asp Val Ala Ser Ile Met
2545                2550                2555                2560

Lys Lys Tyr Leu Gln Lys
                2565


<210> 163
<211> 2566
<212> PRT
<213> Homo sapiens

<220>
<221> VARIANT
<222> (3)
<223> Wherein Xaa is any amino acid as defined in the
      specification.

<220>
<221> VARIANT
<222> (925)
<223> Wherein Xaa is any amino acid as defined in the
      specification.

<400> 163
Met Ala Xaa Ser Ser Arg Leu Ala Leu Trp Glu Gln Lys Ile Arg Glu
1               5                   10                  15

Glu Asp Lys Ser Pro Pro Pro Ser Ser Pro Pro Pro Leu Phe Ser Val
            20                  25                  30

Ile Pro Gly Gly Phe Ile Lys Gln Leu Val Arg Gly Thr Glu Lys Glu
        35                  40                  45

Ala Lys Glu Ala Arg Gln Arg Lys Gln Leu Ala Val Ala Ser Pro Glu
    50                  55                  60

Arg Glu Ile Pro Glu Ile Ser Ile Ser Gln Pro Asn Ser Lys Ser Ser
65                  70                  75                  80

Ser Gly Thr Arg Ser Gly Ser Gln Gln Ile Ser Gln Asp Asp Gln Ser
                85                  90                  95

Ser Ser Pro Gly Ser Ser Asp Ile Leu Gly Lys Glu Ser Glu Gly Ser
            100                 105                 110

Arg Ser Pro Asp Pro Glu Gln Met Thr Ser Ile Asn Gly Glu Lys Ala
        115                 120                 125

Gln Glu Leu Gly Ser Ser Ala Thr Pro Thr Lys Lys Thr Val Pro Phe
    130                 135                 140

Lys Arg Gly Val Arg Arg Gly Asp Val Leu Leu Met Val Ala Lys Leu
145                 150                 155                 160

Asp Pro Asp Ser Ala Lys Pro Glu Lys Thr His Pro His Asp Ala Pro
                165                 170                 175

Pro Cys Lys Thr Ser Pro Pro Ala Thr Asp Thr Gly Lys Glu Lys Lys
            180                 185                 190

Gly Glu Thr Ser Arg Thr Pro Cys Gly Ser Gln Ala Ser Thr Glu Ile
        195                 200                 205

Leu Ala Pro Lys Ala Glu Lys Thr Arg Thr Gly Gly Leu Gly Asp Pro
    210                 215                 220

Gly Gln Gly Thr Val Ala Leu Lys Lys Gly Glu Glu Gly Gln Ser Ile
225                 230                 235                 240

Val Gly Lys Gly Leu Gly Thr Pro Lys Thr Thr Glu Leu Lys Glu Ala
                245                 250                 255

Glu Pro Gln Gly Lys Asp Arg Gln Gly Thr Arg Pro Gln Ala Gln Gly
            260                 265                 270

Pro Gly Glu Gly Val Arg Pro Gly Lys Ala Glu Lys Glu Gly Ala Glu
        275                 280                 285

Pro Thr Asn Thr Val Glu Lys Gly Asn Val Ser Lys Asp Val Gly Ser
    290                 295                 300

Glu Gly Lys His Val Arg Pro Gln Ile Pro Gly Arg Lys Trp Gly Gly
305                 310                 315                 320

Phe Leu Gly Arg Arg Ser Lys Trp Asp Gly Pro Gln Asn Lys Lys Asp
                325                 330                 335

Lys Glu Gly Val Leu Leu Ser Lys Ala Glu Lys Thr Gly Glu Pro Gln
            340                 345                 350

Thr Gln Met Glu Lys Thr Ser Gln Val Gln Gly Glu Leu Gly Asp Asp
        355                 360                 365

Leu Arg Met Gly Glu Lys Ala Gly Glu Leu Arg Ser Thr Thr Gly Lys
    370                 375                 380

Ala Gly Glu Ser Trp Asp Lys Lys Glu Lys Met Gly Gln Pro Gln Gly
385                 390                 395                 400

Lys Ser Gly Asn Ala Gly Glu Ala Arg Ser Gln Thr Glu Lys Gly Cys
                405                 410                 415

Glu Ala Pro Lys Glu Val Ser Thr Met Val Glu Ser Pro Ala Ala Pro
            420                 425                 430

Gly Lys Gly Gly Trp Pro Gly Ser Arg Gly Gln Glu Ala Glu Glu Pro
        435                 440                 445

Cys Ser Arg Ala Gly Asp Gly Ala Gly Ala Leu Glu Thr Glu Leu Glu
    450                 455                 460

Gly Pro Ser Gln Pro Ala Leu Glu Lys Asp Ala Glu Arg Pro Arg Ile
465                 470                 475                 480

Arg Lys Glu Asn Gln Asp Gly Pro Ala Pro Gln Glu Glu Gly Lys Gly
                485                 490                 495

Gly Gln Ser Arg Asp Ser Asp Gln Ala Pro Glu Asp Arg Trp Tyr Glu
            500                 505                 510

Ala Glu Lys Val Trp Leu Ala Gln Lys Asp Gly Phe Thr Leu Ala Thr
        515                 520                 525

Val Leu Lys Pro Asp Glu Gly Thr Ala Asp Leu Pro Ala Gly Arg Val
    530                 535                 540

Arg Leu Trp Ile Asp Ala Asp Lys Thr Ile Thr Glu Val Asp Glu Glu
545                 550                 555                 560

His Val His Arg Ala Asn Pro Pro Glu Leu Asp Gln Val Glu Asp Leu
                565                 570                 575

Ala Ser Leu Ile Ser Val Asn Glu Ser Ser Val Leu Asn Thr Leu Leu
            580                 585                 590

Gln Arg Tyr Lys Ala Gln Leu Leu His Thr Cys Thr Gly Pro Asp Leu
        595                 600                 605

Ile Val Leu Gln Pro Arg Gly Pro Ser Val Pro Ser Ala Gly Lys Val
    610                 615                 620

Pro Lys Gly Arg Arg Asp Gly Leu Pro Ala His Ile Gly Ser Met Ala
625                 630                 635                 640

Gln Arg Ala Tyr Trp Ala Leu Leu Asn Gln Arg Arg Asp Gln Ser Ile
                645                 650                 655

Val Ala Leu Gly Trp Ser Gly Ala Gly Lys Thr Thr Cys Cys Glu Gln
            660                 665                 670

Val Leu Glu His Leu Val Gly Met Ala Gly Ser Val Asp Gly Arg Val
        675                 680                 685

Ser Val Glu Lys Ile Arg Ala Thr Phe Thr Val Leu Arg Ala Phe Gly
    690                 695                 700

Ser Val Ser Met Ala His Ser Arg Ser Ala Thr Arg Phe Ser Met Val
705                 710                 715                 720

Met Ser Leu Asp Phe Asn Ala Thr Gly Arg Ile Thr Ala Ala Gln Leu
                725                 730                 735

Gln Thr Met Leu Leu Glu Lys Ser Arg Val Ala Arg Gln Pro Glu Gly
            740                 745                 750

Glu Ser Asn Phe Leu Val Phe Ser Gln Met Leu Ala Gly Leu Asp Leu
        755                 760                 765

Asp Leu Arg Thr Glu Leu Asn Leu His Gln Met Ala Asp Ser Ser Ser
    770                 775                 780

Phe Gly Met Gly Val Trp Ser Lys Pro Glu Asp Lys Gln Lys Ala Ala
785                 790                 795                 800

Ala Ala Phe Ala Gln Leu Gln Gly Ala Met Glu Met Leu Gly Ile Ser
                805                 810                 815

Glu Ser Glu Gln Arg Ala Val Trp Arg Val Leu Ala Ala Ile Tyr His
            820                 825                 830

Leu Gly Ala Ala Gly Ala Cys Lys Val Gly Arg Lys Gln Phe Met Arg
        835                 840                 845

Phe Glu Trp Ala Asn Tyr Ala Ala Glu Ala Leu Gly Cys Glu Tyr Glu
    850                 855                 860

Glu Leu Asn Thr Ala Thr Phe Lys His His Leu Arg Gln Ile Ile Gln
865                 870                 875                 880

Gln Met Thr Phe Gly Pro Ser Arg Trp Gly Leu Glu Asp Glu Glu Thr
                885                 890                 895

Ser Ser Gly Leu Lys Met Thr Gly Val Asp Cys Val Glu Gly Met Ala
            900                 905                 910

Ser Gly Leu Tyr Gln Glu Leu Phe Ala Ala Val Val Xaa Leu Ile Asn
        915                 920                 925

Arg Ser Phe Ser Ser His His Leu Ser Met Ala Ser Ile Met Val Val
    930                 935                 940

Asp Ser Pro Gly Phe Gln Asn Pro Arg His Gln Gly Lys Asp Arg Ala
945                 950                 955                 960

Ala Thr Phe Glu Glu Leu Cys His Asn Tyr Ala His Glu Arg Leu Gln
                965                 970                 975

Leu Leu Phe Tyr Gln Arg Thr Phe Val Ser Thr Leu Gln Arg Tyr Gln
            980                 985                 990

Glu Glu Gly Val Pro Val Gln Phe Asp Leu Pro Asp Pro Ser Pro Gly
        995                 1000                1005

Thr Thr Val Ala Val Val Asp Gln Asn Pro Ser Gln Val Arg Leu Pro
    1010                1015                1020

Ala Gly Gly Gly Ala Gln Asp Ala Arg Gly Leu Phe Trp Val Leu Asp
1025                1030                1035                1040

Glu Glu Val His Val Glu Gly Ser Ser Asp Ser Val Val Leu Glu Arg
                1045                1050                1055

Leu Cys Ala Ala Phe Glu Lys Lys Gly Ala Gly Thr Glu Gly Ser Ser
            1060                1065                1070

Ala Leu Arg Thr Cys Glu Gln Pro Leu Gln Cys Glu Ile Phe His Gln
        1075                1080                1085

Leu Gly Trp Asp Pro Val Arg Tyr Asp Leu Thr Gly Trp Leu His Arg
    1090                1095                1100

Ala Lys Pro Asn Leu Ser Ala Leu Asp Ala Pro Gln Val Leu His Gln
1105                1110                1115                1120

Ser Lys Arg Glu Glu Leu Arg Ser Leu Phe Gln Ala Arg Ala Lys Leu
                1125                1130                1135

Pro Pro Val Cys Arg Ala Val Ala Gly Leu Glu Gly Thr Ser Gln Gln
            1140                1145                1150

Ala Leu Gln Arg Ser Arg Met Val Arg Arg Thr Phe Ala Ser Ser Leu
        1155                1160                1165

Ala Ala Val Arg Arg Lys Ala Pro Cys Ser Gln Ile Lys Leu Gln Met
    1170                1175                1180

Asp Ala Leu Thr Ser Met Ile Lys Arg Ser Arg Leu His Phe Ile His
1185                1190                1195                1200

Cys Leu Val Pro Asn Pro Val Val Glu Ser Arg Ser Gly Gln Glu Ser
                1205                1210                1215

Pro Pro Pro Pro Gln Pro Gly Arg Asp Lys Pro Gly Ala Gly Gly Pro
            1220                1225                1230

Leu Ala Leu Asp Ile Pro Ala Leu Arg Val Gln Leu Ala Gly Phe His
        1235                1240                1245

Ile Leu Glu Ala Leu Arg Leu His Arg Thr Gly Tyr Ala Asp His Met
    1250                1255                1260

Gly Leu Thr Arg Phe Arg Arg Gln Phe Gln Val Leu Asp Ala Pro Leu
1265                1270                1275                1280

Leu Lys Lys Leu Met Ser Thr Ser Glu Gly Ile Asp Glu Arg Lys Ala
                1285                1290                1295

Val Glu Glu Leu Leu Glu Thr Leu Asp Leu Glu Lys Lys Ala Val Ala
            1300                1305                1310

Val Gly His Ser Gln Val Phe Leu Lys Ala Gly Val Ile Ser Arg Leu
        1315                1320                1325

Glu Lys Gln Arg Glu Lys Leu Val Ser Gln Ser Ile Val Leu Phe Gln
    1330                1335                1340

Ala Ala Cys Lys Gly Phe Leu Ser Arg Gln Glu Phe Lys Lys Leu Lys
1345                1350                1355                1360

Ile Arg Arg Leu Ala Ala Gln Cys Ile Gln Lys Asn Val Ala Val Phe
                1365                1370                1375

Leu Ala Val Lys Asp Trp Pro Trp Trp Gln Leu Leu Gly Ser Leu Gln
            1380                1385                1390

Pro Leu Leu Ser Ala Thr Ile Gly Thr Glu Gln Leu Arg Ala Lys Glu
        1395                1400                1405

Glu Glu Leu Thr Thr Leu Arg Arg Lys Leu Glu Lys Ser Glu Lys Leu
    1410                1415                1420

Arg Asn Glu Leu Arg Gln Asn Thr Asp Leu Leu Glu Ser Lys Ile Ala
1425                1430                1435                1440

Asp Leu Thr Ser Asp Leu Ala Asp Glu Arg Phe Lys Gly Asp Val Ala
            1445                1450                1455

Cys Gln Val Leu Glu Ser Glu Arg Ala Glu Arg Leu Gln Ala Phe Arg
        1460                1465                1470

Glu Val Gln Glu Leu Lys Ser Lys His Glu Gln Val Gln Lys Lys Leu
    1475                1480                1485

Gly Asp Val Asn Lys Gln Leu Glu Glu Ala Gln Gln Lys Ile Gln Leu
    1490                1495                1500

Asn Asp Leu Glu Arg Asn Pro Thr Gly Gly Asp Glu Trp Gln Met Arg
1505                1510                1515                1520

Phe Asp Cys Ala Gln Met Glu Asn Glu Phe Leu Arg Lys Arg Leu Gln
            1525                1530                1535

Gln Cys Glu Glu Arg Leu Asp Ser Glu Leu Thr Ala Arg Lys Glu Leu
        1540                1545                1550

Glu Gln Lys Leu Gly Glu Leu Gln Ser Ala Tyr Asp Gly Ala Lys Lys
    1555                1560                1565

Met Ala His Gln Leu Lys Arg Lys Cys His His Leu Thr Cys Asp Leu
    1570                1575                1580

Glu Asp Thr Cys Val Leu Leu Glu Asn Gln Gln Ser Arg Asn His Glu
1585                1590                1595                1600

Leu Glu Lys Lys Gln Lys Lys Phe Asp Leu Gln Leu Ala Gln Ala Leu
            1605                1610                1615

Gly Glu Ser Val Phe Glu Lys Gly Leu Arg Glu Lys Val Thr Gln Glu
        1620                1625                1630

Asn Thr Ser Val Arg Trp Glu Leu Gly Gln Leu Gln Gln Gln Leu Lys
    1635                1640                1645

Gln Lys Glu Gln Glu Ala Ser Gln Leu Lys Gln Gln Val Glu Met Leu
    1650                1655                1660

Gln Asp His Lys Arg Glu Leu Leu Gly Ser Pro Ser Leu Gly Glu Asn
1665                1670                1675                1680

Cys Val Ala Gly Leu Lys Glu Arg Leu Trp Lys Leu Glu Ser Ser Ala
            1685                1690                1695

Leu Glu Gln Gln Lys Ile Gln Ser Gln Gln Glu Asn Thr Ile Lys Gln
        1700                1705                1710

Leu Glu Gln Leu Arg Gln Arg Phe Glu Leu Glu Ile Glu Arg Met Lys
    1715                1720                1725

Gln Met His Gln Lys Asp Arg Glu Asp Gln Glu Glu Glu Leu Glu Asp
    1730                1735                1740

Val Arg Gln Ser Cys Gln Lys Arg Leu His Gln Leu Glu Met Gln Leu
1745                1750                1755                1760

Glu Gln Glu Tyr Glu Glu Lys Gln Met Val Leu His Glu Lys Gln Asp
            1765                1770                1775

Leu Glu Gly Leu Ile Gly Thr Leu Cys Asp Gln Ile Gly His Arg Asp
        1780                1785                1790

Phe Asp Val Glu Lys Arg Leu Arg Arg Asp Leu Arg Arg Thr His Ala
    1795                1800                1805

Leu Leu Ser Asp Val Gln Leu Leu Leu Gly Thr Met Glu Asp Gly Lys
    1810                1815                1820

Thr Ser Val Ser Lys Glu Glu Leu Glu Lys Val His Ser Gln Leu Glu
1825                1830                1835                1840

Gln Ser Glu Ala Lys Cys Glu Glu Ala Leu Lys Thr Gln Lys Val Leu
                1845                1850                1855

Thr Ala Asp Leu Glu Ser Met His Ser Glu Leu Glu Asn Met Thr Arg
            1860                1865                1870

Asn Lys Ser Leu Val Asp Glu Gln Leu Tyr Arg Leu Gln Phe Glu Lys
        1875                1880                1885

Ala Asp Leu Leu Lys Arg Ile Asp Glu Asp Gln Asp Asp Leu Asn Glu
    1890                1895                1900

Leu Met Gln Lys His Lys Asp Leu Ile Ala Gln Ser Ala Ala Asp Ile
1905                1910                1915                1920

Gly Gln Ile Gln Glu Leu Gln Leu Gln Leu Glu Glu Ala Lys Lys Glu
                1925                1930                1935

Lys His Lys Leu Gln Glu Gln Leu Gln Val Ala Gln Met Arg Ile Glu
            1940                1945                1950

Tyr Leu Glu Gln Ser Thr Val Asp Arg Ala Ile Val Ser Arg Gln Glu
        1955                1960                1965

Ala Val Ile Cys Asp Leu Glu Asn Lys Thr Glu Phe Gln Lys Val Gln
    1970                1975                1980

Ile Lys Arg Phe Glu Val Leu Val Ile Arg Leu Arg Asp Ser Leu Ile
1985                1990                1995                2000

Lys Met Gly Glu Glu Leu Ser Gln Ala Ala Thr Ser Glu Ser Gln Gln
                2005                2010                2015

Arg Glu Ser Ser Gln Tyr Tyr Gln Arg Arg Leu Glu Glu Leu Lys Ala
            2020                2025                2030

Asp Met Glu Glu Leu Val Gln Arg Glu Ala Glu Ala Ser Arg Arg Cys
        2035                2040                2045

Met Glu Leu Glu Lys Tyr Val Glu Glu Leu Ala Ala Val Arg Gln Thr
    2050                2055                2060

Leu Gln Thr Asp Leu Glu Thr Ser Ile Arg Arg Ile Ala Asp Leu Gln
2065                2070                2075                2080

Ala Ala Leu Glu Glu Val Ala Ser Ser Asp Ser Asp Thr Glu Ser Val
                2085                2090                2095

Gln Thr Ala Val Asp Cys Gly Ser Ser Gly Arg Lys Glu Met Asp Asn
            2100                2105                2110

Val Ser Ile Leu Ser Ser Gln Pro Glu Gly Ser Leu Gln Ser Trp Leu
        2115                2120                2125

Ser Cys Thr Leu Ser Leu Ala Thr Asp Thr Met Arg Thr Pro Ser Arg
    2130                2135                2140

Gln Ser Ala Thr Ser Ser Arg Ile Leu Ser Pro Arg Ile Asn Glu Glu
2145                2150                2155                2160

Ala Gly Asp Thr Glu Arg Thr Gln Ser Ala Leu Ala Leu Ser Arg Ala
                2165                2170                2175

Arg Ser Thr Asn Val His Ser Lys Thr Ser Gly Asp Lys Pro Val Ser
            2180                2185                2190

Pro His Phe Val Arg Arg Gln Lys Tyr Cys His Phe Gly Asp Gly Glu
        2195                2200                2205

Val Leu Ala Val Gln Arg Lys Ser Thr Glu Arg Leu Glu Pro Ala Ser
    2210                2215                2220

Ser Pro Leu Ala Ser Arg Ser Thr Asn Thr Ser Pro Leu Ser Arg Glu
2225                2230                2235                2240

Lys Leu Pro Ser Pro Ser Ala Ala Leu Ser Glu Phe Val Glu Gly Leu
                2245                2250                2255

Arg Arg Lys Arg Ala Gln Arg Gly Gln Gly Ser Thr Leu Gly Leu Glu
            2260                2265                2270

Asp Trp Pro Thr Leu Pro Ile Tyr Gln Thr Thr Gly Ala Ser Thr Leu
        2275                2280                2285

Arg Arg Gly Arg Ala Gly Ser Asp Glu Gly Asn Leu Ser Leu Arg Val
    2290                2295                2300

Gly Ala Lys Ser Pro Leu Glu Ile Glu Gly Ala Ala Gly Gly Leu Leu
2305                2310                2315                2320

Arg Ser Thr Ser Leu Lys Cys Ile Ser Ser Asp Gly Val Gly Gly Thr
                2325                2330                2335

Thr Leu Leu Pro Glu Lys Ser Lys Thr Gln Phe Ser Ser Cys Glu Ser
            2340                2345                2350

Leu Leu Glu Ser Arg Pro Ser Met Gly Arg Lys Leu Ser Ser Pro Thr
        2355                2360                2365

Thr Pro Arg Asp Met Leu Leu Ser Pro Thr Leu Arg Pro Arg Arg Arg
    2370                2375                2380

Cys Leu Glu Ser Ser Val Asp Asp Ala Gly Cys Pro Asp Leu Gly Lys
2385                2390                2395                2400

Glu Pro Leu Val Phe Gln Asn Arg Gln Phe Ala His Leu Met Glu Glu
                2405                2410                2415

Pro Leu Gly Ser Asp Pro Phe Ser Trp Lys Leu Pro Ser Leu Asp Tyr
            2420                2425                2430

Glu Arg Lys Thr Lys Val Asp Phe Asp Asp Phe Leu Pro Ala Ile Arg
        2435                2440                2445

Lys Pro Gln Thr Pro Thr Ser Leu Ala Gly Ser Ala Lys Gly Gly Gln
    2450                2455                2460

Asp Gly Ser Gln Arg Ser Ser Ile His Phe Glu Thr Glu Glu Ala Asn
2465                2470                2475                2480

Arg Ser Phe Leu Ser Gly Ile Lys Thr Ile Leu Lys Lys Ser Pro Glu
                2485                2490                2495

Pro Lys Glu Asp Pro Ala His Leu Ser Asp Ser Ser Ser Ser Ser Gly
            2500                2505                2510

Ser Ile Val Ser Phe Lys Ser Ala Asp Ser Ile Lys Ser Arg Pro Gly
        2515                2520                2525

Ile Pro Arg Leu Ala Gly Asp Gly Gly Glu Arg Thr Ser Pro Glu Arg
    2530                2535                2540

Arg Glu Pro Gly Thr Gly Arg Lys Asp Asp Asp Val Ala Ser Ile Met
2545                2550                2555                2560

Lys Lys Tyr Leu Gln Lys
                2565


<210> 164
<211> 688
<212> PRT
<213> Homo sapiens

<400> 164
Asn Pro Pro Lys Phe Glu Gly Val Glu Asp Leu Val Leu Leu Thr Tyr
1               5                   10                  15

Leu Asn Glu Pro Ala Val Leu His Asn Leu Lys Lys Arg Tyr Leu Lys
            20                  25                  30

Asp Leu Ile Tyr Thr Tyr Ile Gly Leu Val Leu Val Ser Val Asn Pro
        35                  40                  45

Tyr Lys Gln Leu Pro Ile Tyr Thr Pro Glu Val Ile Lys Lys Tyr Arg
    50                  55                  60

Gly Lys Ser Arg Gly Glu Leu Pro Pro His Val Phe Ala Ile Ala Asp
65                  70                  75                  80

Asn Ala Tyr Arg Asn Met Leu Asn Asp Lys Glu Asn Gln Ser Ile Ile
                85                  90                  95

Ile Ser Gly Glu Ser Gly Ala Gly Lys Thr Glu Asn Thr Lys Lys Ile
            100                 105                 110

Met Gln Tyr Leu Ala Ala Val Ser Gly Ser Ser Gly Ser Val Gly Ser
        115                 120                 125

Val Glu Asp Gln Ile Leu Glu Ser Asn Pro Ile Leu Glu Ala Phe Gly
    130                 135                 140

Asn Ala Lys Thr Leu Arg Asn Asn Asn Ser Ser Arg Phe Gly Lys Phe
145                 150                 155                 160

Ile Glu Ile His Phe Asp Ala Lys Gly Lys Ile Val Gly Ala Lys Ile
                165                 170                 175

Glu Thr Tyr Leu Leu Glu Lys Ser Arg Val Val Ser Gln Ala Lys Gly
            180                 185                 190

Glu Arg Asn Tyr His Ile Phe Tyr Gln Leu Leu Ala Gly Ala Ser Glu
        195                 200                 205

Glu Leu Lys Lys Lys Leu Gly Leu Lys Lys Ser Pro Glu Asp Tyr Arg
    210                 215                 220

Tyr Leu Asn Gln Gly Gly Cys Leu Thr Val Asp Gly Ile Asp Asp Ala
225                 230                 235                 240

Glu Glu Phe Lys Glu Thr Leu Asn Ala Met Arg Val Leu Gly Phe Ser
                245                 250                 255

Glu Glu Glu Gln Glu Ser Ile Phe Lys Ile Leu Ala Ala Ile Leu His
            260                 265                 270

Leu Gly Asn Ile Glu Phe Glu Glu Gly Arg Asn Asp Asn Ala Ala Glu
        275                 280                 285

Thr Val Lys Asp Lys Glu Glu Leu Asp Asn Ala Ala Glu Leu Leu Gly

290                 295                 300

Val Asp Pro Glu Glu Leu Glu Lys Ala Leu Thr Lys Arg Lys Ile Lys
305                 310                 315                 320

Thr Gly Gly Glu Val Ile Thr Val Pro Leu Thr Val Glu Gln Ala Leu
                325                 330                 335

Asp Ala Arg Asp Ala Leu Ala Lys Ala Ile Tyr Ser Arg Leu Phe Asp
            340                 345                 350

Trp Leu Val Lys Arg Ile Asn Gln Ser Leu Ser Phe Lys Arg Asp Gly
        355                 360                 365

Ser Thr Asn Phe Ile Gly Val Leu Asp Ile Tyr Gly Phe Glu Ile Phe
    370                 375                 380

Glu Lys Asn Ser Phe Glu Gln Leu Cys Ile Asn Tyr Ala Asn Glu Lys
385                 390                 395                 400

Leu Gln Gln Phe Phe Asn Gln His Val Phe Lys Leu Glu Gln Glu Glu
                405                 410                 415

Tyr Glu Arg Glu Gly Ile Glu Trp Thr Phe Ile Asp Phe Phe Asp Asn
            420                 425                 430

Gln Asp Cys Ile Asp Leu Ile Glu Lys Lys Pro Leu Gly Ile Leu Ser
        435                 440                 445

Leu Leu Asp Glu Glu Cys Arg Phe Pro Lys Gly Thr Asp Gln Thr Phe
    450                 455                 460

Leu Glu Lys Leu Asn Lys Gln His Leu Lys Lys His Pro His Phe Ser
465                 470                 475                 480

Lys Pro Lys Lys Asn Gly Arg Thr Glu Phe Ile Ile Lys His Tyr Ala
                485                 490                 495

Gly Asp Val Thr Tyr Asp Val Thr Gly Phe Leu Glu Lys Asn Lys Asp
            500                 505                 510

Thr Leu Ser Asp Asp Leu Ile Glu Leu Leu Gln Ser Ser Lys Asn Pro
        515                 520                 525

Leu Ile Ala Leu Leu Phe Pro Glu Glu Ala Gly Gln Thr Ser Ser Ala
    530                 535                 540

Pro Lys Glu Lys Ser Ala Lys Lys Lys Phe Gln Thr Val Gly Ser Gln
545                 550                 555                 560

Phe Lys Glu Ser Leu Asn Glu Leu Met Asp Thr Leu Asn Ser Thr Asn
                565                 570                 575

Pro His Phe Ile Arg Cys Ile Lys Pro Asn Glu Glu Lys Lys Pro Gly
            580                 585                 590

Asp Phe Asp Ser Ser Leu Val Leu His Gln Leu Arg Tyr Leu Gly Val
        595                 600                 605

Leu Glu Thr Ile Arg Ile Arg Arg Ala Gly Phe Pro Tyr Arg Leu Pro
    610                 615                 620

Phe Asp Glu Phe Leu Gln Arg Tyr Arg Val Leu Leu Pro Asp Thr Trp
625                 630                 635                 640

Pro Pro Trp Gly Gly Asp Ala Lys Glu Ala Cys Glu Leu Leu Leu Gln
                645                 650                 655

Ser Leu Gly Leu Asp Glu Asp Glu Tyr Gln Ile Gly Lys Thr Lys Val

660                 665                 670

Phe Leu Arg Pro Gly Gln Leu Ala Glu Leu Glu Glu Leu Arg Glu Glu
        675                 680                 685

<210> 165
<211> 670
<212> PRT
<213> Homo sapiens

<400> 165
Val Glu Asp Met Ala Glu Leu Thr Tyr Leu Asn Glu Pro Ser Val Leu
1               5                   10                  15

His Asn Leu Lys Lys Arg Tyr Lys Ala Asp Leu Ile Tyr Thr Tyr Ser
            20                  25                  30

Gly Leu Val Leu Val Ser Val Asn Pro Tyr Lys Arg Leu Pro Ile Tyr
        35                  40                  45

Thr Glu Glu Phe Ile Lys Lys Tyr Arg Gly Lys Arg Arg Tyr Glu Leu
    50                  55                  60

Pro Pro His Ile Phe Ala Ile Ala Asp Glu Ala Tyr Arg Ser Met Leu
65                  70                  75                  80

Ser Asp Lys Glu Asn Gln Ser Ile Leu Ile Ser Gly Glu Ser Gly Ala
                85                  90                  95

Gly Lys Thr Glu Asn Thr Lys Lys Val Met Gln Tyr Leu Ala Ala Val
            100                 105                 110

Ser Gly Gly Asn Gly Gly Lys Val Gly Arg Val Glu Asp Gln Ile Leu
        115                 120                 125

Gln Ser Asn Pro Ile Leu Glu Ala Phe Gly Asn Ala Lys Thr Thr Arg
    130                 135                 140

Asn Asn Asn Ser Ser Arg Phe Gly Lys Tyr Ile Glu Ile Gln Phe Asp
145                 150                 155                 160

Lys Thr Gly Lys Ile Val Gly Ala Lys Ile Glu Asn Tyr Leu Leu Glu
                165                 170                 175

Lys Ser Arg Val Val Tyr Gln Thr Pro Gly Glu Arg Asn Phe His Ile
            180                 185                 190

Phe Tyr Gln Leu Leu Ala Gly Ala Ser Gln Gln Leu Lys Lys Glu Leu
        195                 200                 205

Asn Leu Thr Asp Pro Asp Asp Tyr His Tyr Leu Asn Gln Gly Gly Cys
    210                 215                 220

Tyr Thr Val Asp Gly Ile Asp Asp Ser Glu Glu Phe Lys Glu Thr Asp
225                 230                 235                 240

Lys Ala Met Asp Ile Leu Gly Phe Ser Asp Glu Glu Gln Leu Ser Ile
                245                 250                 255

Phe Arg Ile Val Ala Ala Ile Leu His Leu Gly Asn Ile Lys Phe Lys
            260                 265                 270

Gln Arg Arg Lys Glu Glu Ala Ala Glu Pro Asp Asp Thr Lys Ala Leu
        275                 280                 285

Gln Ile Ala Ala Glu Leu Leu Gly Val Asp Ala Lys Glu Leu Glu Lys
    290                 295                 300

Ala Leu Leu Ser Arg Arg Ile Lys Thr Gly Gly Glu Gly Val Thr Val
305                 310                 315                 320

Pro Gln Asn Val Glu Gln Ala Asn Tyr Ala Arg Asp Ala Leu Ala Lys
                325                 330                 335

Ala Leu Tyr Ser Arg Leu Phe Asp Trp Ile Val Asn Arg Ile Asn Lys
            340                 345                 350

Ser Leu Asp Phe Lys Ala Lys Glu Gly Ala Asn Phe Ile Gly Val Leu
        355                 360                 365

Asp Ile Tyr Gly Phe Glu Ile Phe Glu Lys Asn Ser Phe Glu Gln Leu
    370                 375                 380

Cys Ile Asn Tyr Thr Asn Glu Lys Leu Gln Gln Phe Phe Asn His His
385                 390                 395                 400

Met Phe Lys Leu Glu Gln Glu Glu Tyr Lys Arg Glu Gly Ile Glu Trp
                405                 410                 415

Thr Phe Ile Asp Phe Gly Asp Asn Gln Pro Cys Ile Asp Leu Ile Glu
            420                 425                 430

Lys Lys Pro Pro Gly Ile Leu Ser Leu Leu Asp Glu Glu Cys Arg Phe
        435                 440                 445

Pro Lys Ala Thr Asp Gln Thr Phe Leu Asp Lys Leu Tyr Ser Glu Phe
    450                 455                 460

Ser Asn His Pro His Phe Lys Lys Pro Arg Phe Arg Gln Lys Lys Ser
465                 470                 475                 480

Phe Ile Ile Lys His Tyr Ala Gly Asp Val Glu Tyr Asn Val Glu Gly
                485                 490                 495

Phe Leu Glu Lys Asn Lys Asp Pro Leu Phe Asp Asp Leu Ile Glu Leu
            500                 505                 510

Leu Lys Ser Ser Ser Asn Pro Leu Leu Ala Glu Leu Phe Pro Asp Tyr
        515                 520                 525

Glu Glu Ala Asp Pro Ser Ser Leu Ser Lys Lys Arg Lys Ile Thr Lys
    530                 535                 540

Lys Ser Asn Phe Ile Thr Val Gly Ala Gln Phe Lys Glu Ser Leu Asn
545                 550                 555                 560

Thr Leu Met Lys Thr Leu Ser Ser Thr Asn Pro His Phe Val Arg Cys
                565                 570                 575

Ile Lys Pro Asn Glu Glu Lys Lys Pro Gly Val Phe Asp Ala Ser Leu
            580                 585                 590

Val Leu His Gln Leu Arg Cys Leu Gly Val Leu Glu Gly Ile Arg Ile
        595                 600                 605

Arg Arg Ala Gly Phe Pro Ser Arg Ile Thr Phe Asp Glu Phe Leu Gln
    610                 615                 620

Arg Tyr Arg Ile Leu Ala Pro Lys Thr Trp Pro Lys Trp Ser Gly Asp
625                 630                 635                 640

Ala Lys Lys Gly Ala Cys Glu Leu Leu Leu Gln Ala Leu Asn Leu Asp
                645                 650                 655

Lys Glu Glu Tyr Gln Phe Gly Lys Thr Lys Ile Phe Phe Arg
            660                 665                 670

<210> 166
<211> 860
<212> PRT
<213> Homo sapiens

<400> 166
Glu Leu Glu Arg Gln Lys Arg Glu Leu Glu Asn Gln Leu Tyr Arg Lys
1               5                   10                  15

Glu Ser Glu Leu Ser Gln Leu Ser Ser Lys Leu Glu Asp Glu Gln Ala
            20                  25                  30

Leu Val Ala Gln Leu Gln Lys Lys Ile Lys Glu Leu Glu Ala Arg Ile
        35                  40                  45

Arg Glu Leu Glu Glu Glu Leu Glu Ala Glu Arg Ala Ala Arg Ala Lys
    50                  55                  60

Ala Glu Lys Ala Arg Ala Asp Leu Ser Arg Glu Leu Glu Glu Leu Ser
65                  70                  75                  80

Glu Arg Leu Glu Glu Ala Gly Gly Ala Thr Ala Ala Gln Ile Glu Leu
                85                  90                  95

Asn Lys Lys Arg Glu Ala Glu Leu Ala Lys Leu Arg Lys Asp Leu Glu
            100                 105                 110

Glu Ala Asn Leu Gln His Glu Glu Ala Leu Ala Thr Leu Arg Lys Lys
        115                 120                 125

His Gln Asp Ala Ile Asn Glu Leu Ser Glu Gln Ile Glu Gln Leu Gln
    130                 135                 140

Lys Gln Lys Ala Lys Ala Glu Lys Glu Lys Ser Gln Leu Gln Ala Glu
145                 150                 155                 160

Val Asp Asp Leu Leu Ala Gln Leu Asp Ser Ile Thr Lys Ala Lys Leu
                165                 170                 175

Asn Ala Glu Lys Lys Ala Lys Gln Leu Glu Ser Gln Leu Ser Glu Leu
            180                 185                 190

Gln Val Lys Leu Asp Glu Leu Gln Arg Gln Leu Asn Asp Leu Thr Ser
        195                 200                 205

Gln Lys Ser Arg Leu Gln Ser Glu Asn Ser Asp Leu Thr Arg Gln Leu
    210                 215                 220

Glu Glu Ala Glu Ala Gln Val Ser Asn Leu Ser Lys Leu Lys Ser Gln
225                 230                 235                 240

Leu Glu Ser Gln Leu Glu Glu Ala Lys Arg Ser Leu Glu Glu Glu Ser
                245                 250                 255

Arg Glu Arg Ala Asn Leu Gln Ala Gln Leu Arg Gln Leu Glu His Asp
            260                 265                 270

Leu Asp Ser Leu Arg Glu Gln Leu Glu Glu Glu Ser Glu Ala Lys Ala
        275                 280                 285

Glu Leu Glu Arg Gln Leu Ser Lys Ala Asn Ala Glu Ile Gln Gln Trp
    290                 295                 300

Arg Ser Lys Phe Glu Ser Glu Gly Ala Leu Arg Ala Glu Glu Leu Glu
305                 310                 315                 320

Glu Leu Lys Lys Lys Leu Asn Gln Lys Ile Ser Glu Leu Glu Glu Ala
                325                 330                 335

Ala Glu Ala Ala Asn Ala Lys Cys Asp Ser Leu Glu Lys Thr Lys Ser
            340                 345                 350

Arg Leu Gln Ser Glu Leu Glu Asp Leu Gln Ile Glu Leu Glu Arg Ala
        355                 360                 365

Asn Ala Ala Ala Ser Glu Leu Glu Lys Lys Gln Lys Asn Phe Asp Lys
    370                 375                 380

Ile Leu Ala Glu Trp Lys Arg Lys Val Asp Glu Leu Gln Ala Glu Leu
385                 390                 395                 400

Asp Thr Ala Gln Arg Glu Ala Arg Asn Leu Ser Thr Glu Leu Phe Arg
                405                 410                 415

Leu Lys Asn Glu Leu Glu Glu Leu Lys Asp Gln Val Glu Ala Leu Arg
            420                 425                 430

Arg Glu Asn Lys Asn Leu Gln Asp Glu Ile His Asp Leu Thr Asp Gln
        435                 440                 445

Leu Gly Glu Gly Gly Arg Asn Val His Glu Leu Glu Lys Ala Arg Arg
    450                 455                 460

Arg Leu Glu Ala Glu Lys Asp Glu Leu Gln Ala Ala Leu Glu Glu Ala
465                 470                 475                 480

Glu Ala Ala Leu Glu Leu Glu Glu Ser Lys Val Leu Arg Ala Gln Val
                485                 490                 495

Glu Leu Ser Gln Ile Arg Ser Glu Ile Glu Arg Arg Leu Ala Glu Lys
            500                 505                 510

Glu Glu Glu Phe Glu Asn Thr Arg Lys Asn His Gln Arg Ala Ile Glu
        515                 520                 525

Ser Leu Gln Ala Thr Leu Glu Ala Glu Thr Lys Gly Lys Ala Glu Ala
    530                 535                 540

Ser Arg Leu Lys Lys Lys Leu Glu Gly Asp Ile Asn Glu Leu Glu Ile
545                 550                 555                 560

Ala Leu Asp His Ala Asn Lys Ala Asn Ala Glu Ala Gln Lys Asn Val
                565                 570                 575

Lys Lys Tyr Gln Gln Gln Val Lys Glu Leu Gln Thr Gln Val Glu Glu
            580                 585                 590

Glu Gln Arg Ala Arg Glu Asp Ala Arg Glu Gln Leu Ala Val Ala Glu
        595                 600                 605

Arg Arg Ala Thr Ala Leu Glu Ala Glu Leu Glu Glu Leu Arg Ser Ala
    610                 615                 620

Leu Glu Gln Ala Glu Arg Ala Arg Lys Gln Ala Glu Thr Glu Leu Ala
625                 630                 635                 640

Glu Ala Ser Glu Arg Val Asn Glu Leu Thr Ala Gln Asn Ser Ser Leu
                645                 650                 655

Ile Ala Gln Lys Arg Lys Leu Glu Gly Glu Leu Ala Ala Leu Gln Ser
            660                 665                 670

Asp Leu Asp Glu Ala Val Asn Glu Leu Lys Ala Ala Glu Glu Arg Ala
        675                 680                 685

Lys Lys Ala Gln Ala Asp Ala Ala Arg Leu Ala Glu Glu Leu Arg Gln
    690                 695                 700

Glu Gln Glu His Ser Gln His Leu Glu Arg Leu Arg Lys Gln Leu Glu
705                 710                 715                 720

Ser Gln Val Lys Glu Leu Gln Val Arg Leu Asp Glu Ala Glu Ala Ala
                725                 730                 735

Ala Leu Lys Gly Gly Lys Lys Met Ile Gln Lys Leu Glu Ala Arg Val
            740                 745                 750

Arg Glu Leu Glu Ala Glu Leu Asp Gly Glu Gln Arg Arg His Ala Glu
        755                 760                 765

Thr Gln Lys Asn Leu Arg Lys Met Glu Arg Arg Val Lys Glu Leu Gln
    770                 775                 780

Phe Gln Val Glu Glu Asp Lys Lys Asn Leu Glu Arg Leu Gln Asp Leu
785                 790                 795                 800

Val Asp Lys Leu Gln Ala Lys Ile Lys Thr Tyr Lys Arg Gln Leu Glu
                805                 810                 815

Glu Ala Glu Glu Val Ala Gln Ile Asn Leu Ser Lys Tyr Arg Lys Ala
            820                 825                 830

Gln Arg Glu Leu Glu Asp Ala Glu Glu Arg Ala Asp Thr Ala Glu Arg
        835                 840                 845

Ser Leu Asn Lys Leu Arg Ala Lys Ser Arg Arg Thr
    850                 855                 860

<210> 167
<211> 312
<212> PRT
<213> Homo sapiens

<400> 167
Asn Glu Lys Glu Gln Met Gln Asn Leu Asn Asp Arg Leu Ala Ser Tyr
1               5                   10                  15

Ile Asp Lys Val Arg Phe Leu Glu Gln Gln Asn Lys Glu Leu Glu Val
            20                  25                  30

Lys Ile Glu Glu Leu Arg Gln Lys Gln Ala Pro Ser Val Ser Arg Leu
        35                  40                  45

Tyr Ser Leu Tyr Glu Thr Glu Ile Glu Glu Leu Arg Arg Gln Ile Asp
    50                  55                  60

Gln Leu Thr Asn Glu Arg Ala Arg Leu Gln Leu Glu Ile Asp Asn Leu
65                  70                  75                  80

Arg Glu Ala Ala Glu Asp Phe Arg Lys Lys Tyr Glu Asp Glu Ile Asn
                85                  90                  95

Leu Arg Gln Glu Ala Glu Asn Asp Leu Val Gly Leu Arg Lys Asp Leu
            100                 105                 110

Asp Glu Ala Thr Leu Ala Arg Val Asp Leu Glu Asn Lys Val Glu Ser
        115                 120                 125

Leu Gln Glu Glu Leu Glu Phe Leu Lys Lys Asn His Glu Glu Glu Val
    130                 135                 140

Lys Glu Leu Gln Ala Gln Ile Gln Asp Thr Val Asn Val Glu Met Asp

145                 150                 155                 160

Ala Ala Arg Lys Leu Asp Leu Thr Lys Ala Leu Arg Glu Ile Arg Ala
                165                 170                 175

Gln Tyr Glu Glu Ile Ala Lys Lys Asn Arg Gln Glu Ala Glu Glu Trp
            180                 185                 190

Tyr Lys Ser Lys Leu Glu Glu Leu Gln Thr Ala Ala Ala Arg Asn Gly
        195                 200                 205

Glu Ala Leu Arg Ser Ala Lys Glu Glu Ile Thr Glu Leu Arg Arg Gln
    210                 215                 220

Ile Gln Ser Leu Glu Ile Glu Leu Gln Ser Leu Lys Ala Gln Asn Ala
225                 230                 235                 240

Ser Leu Glu Arg Gln Leu Ala Glu Leu Glu Glu Arg Tyr Glu Leu Glu
                245                 250                 255

Leu Arg Gln Tyr Gln Ala Leu Ile Ser Gln Leu Glu Glu Glu Leu Gln
            260                 265                 270

Gln Leu Arg Glu Glu Met Ala Arg Gln Leu Arg Glu Tyr Gln Glu Leu
        275                 280                 285

Leu Asp Val Lys Leu Ala Leu Asp Ile Glu Ile Ala Thr Tyr Arg Lys
    290                 295                 300

Leu Leu Glu Gly Glu Glu Ser Arg
305                 310


<210> 168
<211> 332
<212> PRT
<213> Homo sapiens

<400> 168
Met Tyr Arg Pro Arg Ala Arg Ala Ala Pro Glu Gly Arg Val Arg Gly
1               5                   10                  15

Cys Ala Val Pro Ser Thr Val Leu Leu Leu Leu Ala Tyr Leu Ala Tyr
            20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Asp
65                  70                  75                  80

Val Val Gln Ala Tyr Lys Asn Gly Ala Ser Leu Leu Ser Asn Thr Thr
                85                  90                  95

Ser Met Gly Arg Trp Glu Leu Val Gly Ser Phe Phe Phe Ser Val Ser
            100                 105                 110

Thr Ile Thr Thr Ile Gly Tyr Gly Asn Leu Ser Pro Asn Thr Met Ala
        115                 120                 125

Ala Arg Leu Phe Cys Ile Phe Phe Ala Leu Val Gly Ile Pro Leu Asn
    130                 135                 140

Leu Val Val Leu Asn Arg Leu Gly His Leu Met Gln Gln Gly Val Asn
145                 150                 155                 160

His Trp Ala Ser Arg Leu Gly Gly Thr Trp Gln Asp Pro Asp Lys Ala
                165                 170                 175

Arg Trp Leu Ala Gly Ser Gly Ala Leu Leu Ser Gly Leu Leu Leu Phe
            180                 185                 190

Leu Leu Leu Pro Pro Leu Leu Phe Ser His Met Glu Gly Trp Ser Tyr
        195                 200                 205

Thr Glu Gly Phe Tyr Phe Ala Phe Ile Thr Leu Ser Thr Val Gly Phe
    210                 215                 220

Gly Asp Tyr Val Ile Gly Met Asn Pro Ser Gln Arg Tyr Pro Leu Trp
225                 230                 235                 240

Tyr Lys Asn Met Val Ser Leu Trp Ile Leu Phe Gly Met Ala Trp Leu
                245                 250                 255

Ala Leu Ile Ile Lys Leu Ile Leu Ser Gln Leu Glu Thr Pro Gly Arg
            260                 265                 270

Val Cys Ser Cys Cys His His Ser Ser Lys Glu Asp Phe Lys Ser Gln
        275                 280                 285

Ser Trp Arg Gln Gly Pro Asp Arg Glu Pro Glu Ser His Ser Pro Gln
    290                 295                 300

Gln Gly Cys Tyr Pro Glu Gly Pro Met Gly Ile Ile Gln His Leu Glu
305                 310                 315                 320

Pro Ser Ala His Ala Ala Gly Cys Gly Lys Asp Ser
                325                 330

<210> 169
<211> 343
<212> PRT
<213> Homo sapiens

<400> 169


<210> 170
<211> 229
<212> PRT
<213> Homo sapiens

<400> 170
Met Tyr Arg Pro Arg Ala Arg Ala Ala Pro Glu Gly Arg Val Arg Gly
  1               5                  10                  15

Cys Ala Val Pro Ser Thr Val Leu Leu Leu Leu Ala Tyr Leu Ala Tyr
             20                  25                  30

Leu Ala Leu Gly Thr Gly Val Phe Trp Thr Leu Glu Gly Arg Ala Ala
         35                  40                  45

Gln Asp Ser Ser Arg Ser Phe Gln Arg Asp Lys Trp Glu Leu Leu Gln
     50                  55                  60

Asn Phe Thr Cys Leu Asp Arg Pro Ala Leu Asp Ser Leu Ile Arg Asp
 65                  70                  75                  80

Val Val Gln Ala Tyr Lys Asn Gly Ala Ser Leu Leu Ser Asn Thr Thr
                 85                  90                  95

Ser Met Gly Arg Trp Glu Leu Val Gly Ser Phe Phe Phe Ser Val Ser
            100                 105                 110

Thr Ile Thr Thr Ile Gly Tyr Gly Asn Leu Ser Pro Asn Thr Met Ala
        115                 120                 125

Ala Arg Leu Phe Cys Ile Phe Phe Ala Leu Val Gly Ile Pro Leu Asn

130                 135                 140

Leu Val Val Leu Asn Arg Leu Gly His Leu Met Gln Gln Gly Val Asn
145                 150                 155                 160

His Trp Ala Ser Arg Leu Gly Gly Thr Trp Gln Asp Pro Asp Lys Ala
                165                 170                 175

Arg Trp Leu Ala Gly Ser Gly Ala Leu Leu Ser Gly Leu Leu Leu Phe
            180                 185                 190

Leu Leu Leu Pro Pro Leu Leu Phe Ser His Met Glu Gly Trp Ser Tyr
        195                 200                 205

Thr Glu Gly Phe Tyr Phe Ala Phe Ile Thr Leu Ser Thr Val Gly Phe
    210                 215                 220

Gly Asp Tyr Val Ile
225


<210> 171
<211> 229
<212> PRT
<213> Homo sapiens

<400> 171
Met Tyr Arg Pro Arg Ala Arg Ala Ala Pro Glu Gly Arg Val Arg Gly
1               5                   10                  15

Cys Ala Val Pro Ser Thr Val Leu Leu Leu Leu Ala Tyr Leu Ala Tyr
            20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Asp
65                  70                  75                  80

Val Val Gln Ala Tyr Lys Asn Gly Ala Ser Leu Leu Ser Asn Thr Thr
                85                  90                  95

Ser Met Gly Arg Trp Glu Leu Val Gly Ser Phe Phe Phe Ser Val Ser
            100                 105                 110

Thr Ile Thr Thr Ile Gly Tyr Gly Asn Leu Ser Pro Asn Thr Met Ala
        115                 120                 125

Ala Arg Leu Phe Cys Ile Phe Phe Ala Leu Val Gly Ile Pro Leu Asn
    130                 135                 140

Leu Val Val Leu Asn Arg Leu Gly His Leu Met Gln Gln Gly Val Asn
145                 150                 155                 160

His Trp Ala Ser Arg Leu Gly Gly Thr Trp Gln Asp Pro Asp Lys Ala
                165                 170                 175

Arg Trp Leu Ala Gly Ser Gly Ala Leu Leu Ser Gly Leu Leu Leu Phe
            180                 185                 190

Leu Leu Leu Pro Pro Leu Leu Phe Ser His Met Glu Gly Trp Ser Tyr
        195                 200                 205

Thr Glu Gly Phe Tyr Phe Ala Phe Ile Thr Leu Ser Thr Val Gly Phe
    210                 215                 220

Gly Asp Tyr Val Ile
225

<210> 172
<211> 309
<212> PRT
<213> Homo sapiens

<400> 172
Met Pro Ser Ala Gly Leu Cys Ser Cys Trp Gly Gly Arg Val Leu Pro
1               5                   10                  15

Leu Leu Leu Ala Tyr Val Cys Tyr Leu Leu Leu Gly Ala Thr Ile Phe
            20                  25                  30

Gln Leu Leu Glu Arg Gln Ala Glu Ala Gln Ser Arg Asp Gln Phe Gln
        35                  40                  45

Leu Glu Lys Leu Arg Phe Leu Glu Asn Tyr Thr Cys Leu Asp Gln Trp
    50                  55                  60

Ala Met Glu Gln Phe Val Gln Val Ile Met Glu Ala Trp Val Lys Gly
65                  70                  75                  80

Val Asn Pro Lys Gly Asn Ser Thr Asn Pro Ser Asn Trp Asp Phe Gly
                85                  90                  95

Ser Ser Phe Phe Phe Ala Gly Thr Val Val Thr Thr Ile Gly Tyr Gly
            100                 105                 110

Asn Leu Ala Pro Ser Thr Glu Ala Gly Gln Val Phe Cys Val Phe Tyr
        115                 120                 125

Ala Leu Leu Gly Ile Pro Leu Asn Val Ile Phe Leu Asn His Leu Gly
    130                 135                 140

Thr Gly Leu Arg Ala His Leu Ala Ala Ile Glu Arg Trp Glu Asp Arg
145                 150                 155                 160

Pro Arg Arg Ser Gln Val Leu Gln Val Leu Gly Leu Ala Leu Phe Leu
                165                 170                 175

Thr Leu Gly Thr Leu Val Ile Leu Ile Phe Pro Pro Met Val Phe Ser
            180                 185                 190

His Val Glu Gly Trp Ser Phe Ser Glu Gly Phe Tyr Phe Ala Phe Ile
        195                 200                 205

Thr Leu Ser Thr Ile Gly Phe Gly Asp Tyr Val Val Gly Thr Asp Pro
    210                 215                 220

Ser Lys His Tyr Ile Ser Val Tyr Arg Ser Leu Ala Ala Ile Trp Ile
225                 230                 235                 240

Leu Leu Gly Leu Ala Trp Leu Ala Leu Ile Leu Pro Leu Gly Pro Leu
                245                 250                 255

Leu Leu His Arg Cys Cys Gln Leu Trp Leu Leu Ser Leu Arg Gln Gly
            260                 265                 270

Cys Gly Ala Lys Ala Ala Pro Gly Arg Arg Pro Arg Arg Gly Ser Thr
        275                 280                 285

Ala Ala Arg Gly Val Gln Val Thr Pro Gln Asp Phe Pro Ile Ser Lys
    290                 295                 300

Lys Gly Leu Gly Ser
305

<210> 173
<211> 343
<212> PRT
<213> Homo sapiens

<400> 173

<210> 174
<211> 332
<212> PRT
<213> Homo sapiens

<400> 174
Met Tyr Arg Pro Arg Ala Arg Ala Ala Pro Glu Gly Arg Val Arg Gly
1               5                   10                  15

Cys Ala Val Pro Ser Thr Val Leu Leu Leu Leu Ala Tyr Leu Ala Tyr
            20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Asp
65                  70                  75                  80

Val Val Gln Ala Tyr Lys Asn Gly Ala Ser Leu Leu Ser Asn Thr Thr
                85                  90                  95

Ser Met Gly Arg Trp Glu Leu Val Gly Ser Phe Phe Phe Ser Val Ser
            100                 105                 110

Thr Ile Thr Thr Ile Gly Tyr Gly Asn Leu Ser Pro Asn Thr Met Ala
        115                 120                 125

Ala Arg Leu Phe Cys Ile Phe Phe Ala Leu Val Gly Ile Pro Leu Asn
    130                 135                 140

Leu Val Val Leu Asn Arg Leu Gly His Leu Met Gln Gln Gly Val Asn
145                 150                 155                 160

His Trp Ala Ser Arg Leu Gly Gly Thr Trp Gln Asp Pro Asp Lys Ala
                165                 170                 175

Arg Trp Leu Ala Gly Ser Gly Ala Leu Leu Ser Gly Leu Leu Leu Phe
            180                 185                 190

Leu Leu Leu Pro Pro Leu Leu Phe Ser His Met Glu Gly Trp Ser Tyr
        195                 200                 205

Thr Glu Gly Phe Tyr Phe Ala Phe Ile Thr Leu Ser Thr Val Gly Phe
    210                 215                 220

Gly Asp Tyr Val Ile Gly Met Asn Pro Ser Gln Arg Tyr Pro Leu Trp
225                 230                 235                 240

Tyr Lys Asn Met Val Ser Leu Trp Ile Leu Phe Gly Met Ala Trp Leu
                245                 250                 255

Ala Leu Ile Ile Lys Leu Ile Leu Ser Gln Leu Glu Thr Pro Gly Arg
            260                 265                 270

Val Cys Ser Cys Cys His His Ser Ser Lys Glu Asp Phe Lys Ser Gln
        275                 280                 285

Ser Trp Arg Gln Gly Pro Asp Arg Glu Pro Glu Ser His Ser Pro Gln
    290                 295                 300

Gln Gly Cys Tyr Pro Glu Gly Pro Met Gly Ile Ile Gln His Leu Glu
305                 310                 315                 320

Pro Ser Ala His Ala Ala Gly Cys Gly Lys Asp Ser
                325                 330

<210> 175
<211> 229
<212> PRT
<213> Homo sapiens

<400> 175
Met Tyr Arg Pro Arg Ala Arg Ala Ala Pro Glu Gly Arg Val Arg Gly
1               5                   10                  15

Cys Ala Val Pro Ser Thr Val Leu Leu Leu Leu Ala Tyr Leu Ala Tyr
            20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Asp
65                  70                  75                  80

Val Val Gln Ala Tyr Lys Asn Gly Ala Ser Leu Leu Ser Asn Thr Thr
                85                  90                  95

Ser Met Gly Arg Trp Glu Leu Val Gly Ser Phe Phe Phe Ser Val Ser
            100                 105                 110

Thr Ile Thr Thr Ile Gly Tyr Gly Asn Leu Ser Pro Asn Thr Met Ala
        115                 120                 125

Ala Arg Leu Phe Cys Ile Phe Phe Ala Leu Val Gly Ile Pro Leu Asn
    130                 135                 140

Leu Val Val Leu Asn Arg Leu Gly His Leu Met Gln Gln Gly Val Asn
145                 150                 155                 160

His Trp Ala Ser Arg Leu Gly Gly Thr Trp Gln Asp Pro Asp Lys Ala
                165                 170                 175

Arg Trp Leu Ala Gly Ser Gly Ala Leu Leu Ser Gly Leu Leu Leu Phe
            180                 185                 190

Leu Leu Leu Pro Pro Leu Leu Phe Ser His Met Glu Gly Trp Ser Tyr
        195                 200                 205

Thr Glu Gly Phe Tyr Phe Ala Phe Ile Thr Leu Ser Thr Val Gly Phe
    210                 215                 220

Gly Asp Tyr Val Ile
225

<210> 176
<211> 229
<212> PRT
<213> Homo sapiens

<400> 176
Met Tyr Arg Pro Arg Ala Arg Ala Ala Pro Glu Gly Arg Val Arg Gly
1               5                   10                  15

Cys Ala Val Pro Ser Thr Val Leu Leu Leu Leu Ala Tyr Leu Ala Tyr

20                  25                  30

Leu Ala Leu Gly Thr Gly Val Phe Trp Thr Leu Glu Gly Arg Ala Ala
        35                  40                  45

Gln Asp Ser Ser Arg Ser Phe Gln Arg Asp Lys Trp Glu Leu Leu Gln
    50                  55                  60

Asn Phe Thr Cys Leu Asp Arg Pro Ala Leu Asp Ser Leu Ile Arg Asp
65                  70                  75                  80

Val Val Gln Ala Tyr Lys Asn Gly Ala Ser Leu Leu Ser Asn Thr Thr
                85                  90                  95

Ser Met Gly Arg Trp Glu Leu Val Gly Ser Phe Phe Phe Ser Val Ser
            100                 105                 110

Thr Ile Thr Thr Ile Gly Tyr Gly Asn Leu Ser Pro Asn Thr Met Ala
        115                 120                 125

Ala Arg Leu Phe Cys Ile Phe Phe Ala Leu Val Gly Ile Pro Leu Asn
    130                 135                 140

Leu Val Val Leu Asn Arg Leu Gly His Leu Met Gln Gln Gly Val Asn
145                 150                 155                 160

His Trp Ala Ser Arg Leu Gly Gly Thr Trp Gln Asp Pro Asp Lys Ala
                165                 170                 175

Arg Trp Leu Ala Gly Ser Gly Ala Leu Leu Ser Gly Leu Leu Leu Phe
            180                 185                 190

Leu Leu Leu Pro Pro Leu Leu Phe Ser His Met Glu Gly Trp Ser Tyr
        195                 200                 205

Thr Glu Gly Phe Tyr Phe Ala Phe Ile Thr Leu Ser Thr Val Gly Phe
    210                 215                 220

Gly Asp Tyr Val Ile
225


<210> 177
<211> 309
<212> PRT
<213> Homo sapiens

<400> 177
Met Pro Ser Ala Gly Leu Cys Ser Cys Trp Gly Gly Arg Val Leu Pro
1               5                   10                  15

Leu Leu Leu Ala Tyr Val Cys Tyr Leu Leu Leu Gly Ala Thr Ile Phe
            20                  25                  30

Gln Leu Leu Glu Arg Gln Ala Glu Ala Gln Ser Arg Asp Gln Phe Gln
        35                  40                  45

Leu Glu Lys Leu Arg Phe Leu Glu Asn Tyr Thr Cys Leu Asp Gln Trp
    50                  55                  60

Ala Met Glu Gln Phe Val Gln Val Ile Met Glu Ala Trp Val Lys Gly
65                  70                  75                  80

Val Asn Pro Lys Gly Asn Ser Thr Asn Pro Ser Asn Trp Asp Phe Gly
                85                  90                  95

Ser Ser Phe Phe Phe Ala Gly Thr Val Val Thr Thr Ile Gly Tyr Gly
            100                 105                 110

Asn Leu Ala Pro Ser Thr Glu Ala Gly Gln Val Phe Cys Val Phe Tyr
        115                 120                 125

Ala Leu Leu Gly Ile Pro Leu Asn Val Ile Phe Leu Asn His Leu Gly
    130                 135                 140

Thr Gly Leu Arg Ala His Leu Ala Ala Ile Glu Arg Trp Glu Asp Arg
145                 150                 155                 160

Pro Arg Arg Ser Gln Val Leu Gln Val Leu Gly Leu Ala Leu Phe Leu
                165                 170                 175

Thr Leu Gly Thr Leu Val Ile Leu Ile Phe Pro Pro Met Val Phe Ser
            180                 185                 190

His Val Glu Gly Trp Ser Phe Ser Glu Gly Phe Tyr Phe Ala Phe Ile
        195                 200                 205

Thr Leu Ser Thr Ile Gly Phe Gly Asp Tyr Val Val Gly Thr Asp Pro
    210                 215                 220

Ser Lys His Tyr Ile Ser Val Tyr Arg Ser Leu Ala Ala Ile Trp Ile
225                 230                 235                 240

Leu Leu Gly Leu Ala Trp Leu Ala Leu Ile Leu Pro Leu Gly Pro Leu
                245                 250                 255

Leu Leu His Arg Cys Cys Gln Leu Trp Leu Leu Ser Leu Arg Gln Gly
            260                 265                 270

Cys Gly Ala Lys Ala Ala Pro Gly Arg Arg Pro Arg Arg Gly Ser Thr
        275                 280                 285

Ala Ala Arg Gly Val Gln Val Thr Pro Gln Asp Phe Pro Ile Ser Lys
    290                 295                 300

Lys Gly Leu Gly Ser
305


<210> 178
<211> 190
<212> PRT
<213> Homo sapiens

<400> 178

<210> 179
<211> 189
<212> PRT
<213> Sus scrofa

<400> 179
Met Asp His Ser Ala His Met Gly Met Ser Thr His Met Gly Met Ser
1               5                   10                  15

Asp Met Asn His Ser Thr Thr Met Pro Pro Ser His His His Pro Thr
            20                  25                  30

Ser Ser Gly Ser His Glu Ser Met Met Met Pro Met Thr Phe Tyr Phe
        35                  40                  45

Gly Phe Lys Lys Val Glu Val Leu Phe Ala Gly Leu Val Ile Asn Thr
    50                  55                  60

Ala Gly Glu Met Ala Gly Ala Phe Val Ala Val Phe Leu Leu Ala Met
65                  70                  75                  80

Phe Tyr Glu Gly Leu Lys Ile Ala Arg Glu Gly Leu Leu Arg Lys Ser
                85                  90                  95

Gln Val Ser Ile Arg Tyr Asn Ser Met Pro Val Pro Gly Pro Asn Gly
            100                 105                 110

Thr Ile Leu Met Glu Thr His Lys Thr Val Gly Gln Gln Met Leu Ser
        115                 120                 125

Phe Pro His Leu Leu Gln Thr Val Leu His Ile Ile Gln Val Val Ile
    130                 135                 140

Ser Tyr Phe Leu Met Leu Ile Phe Met Thr Tyr Asn Gly Tyr Leu Cys
145                 150                 155                 160

Ile Ala Val Ala Ala Gly Ala Gly Thr Gly Tyr Phe Leu Phe Ser Trp
                165                 170                 175

Lys Lys Ala Val Val Val Asp Ile Thr Glu His Cys His
            180                 185

<210> 180
<211> 187
<212> PRT
<213> Rattus norvegicus

<400> 180
Met Arg Met Asn His Met Glu Met His His Met Gly Met Asn His Thr
1               5                   10                  15

Asp Asp Asn Ile Thr Met Pro Pro His Gln His Pro Thr Thr Ser Ala
            20                  25                  30

Ser His Ser His Glu Met Met Met Pro Met Thr Phe Tyr Phe Gly Phe
        35                  40                  45

Lys Asn Val Asp Leu Leu Phe Ser Ser Leu Val Ile Asn Thr Pro Gly
    50                  55                  60

Glu Met Ala Gly Ala Phe Val Ala Val Phe Leu Leu Ala Met Phe Tyr
65                  70                  75                  80

Glu Gly Leu Lys Ile Ala Arg Glu Gly Leu Leu Arg Lys Ser Gln Val
                85                  90                  95

Ser Ile Arg Tyr Asn Ser Met Pro Val Pro Gly Pro Asn Gly Thr Ile
            100                 105                 110

Leu Met Glu Thr His Lys Thr Val Gly Gln Gln Met Leu Ser Phe Pro
        115                 120                 125

His Leu Leu Gln Thr Val Leu His Ile Ile Gln Val Val Ile Ser Tyr
    130                 135                 140

Phe Leu Met Leu Ile Phe Met Thr Tyr Asn Gly Tyr Leu Cys Ile Ala
145                 150                 155                 160

Val Ala Ala Gly Ala Gly Thr Gly Tyr Phe Leu Phe Ser Trp Lys Lys
                165                 170                 175

Ala Val Val Val Asp Ile Thr Glu His Cys His
            180                 185


<210> 181
<211> 188
<212> PRT
<213> Danio rerio

<400> 181
Met Asp Ser Ser His Ser His His His Val Glu Glu Thr Thr Met Pro
1               5                   10                  15

Ser Pro Ala Ser Gly Asp His Gly Gly His Leu Thr Thr Thr Gly Asn
            20                  25                  30

Gly His Gly Asp His Met Met Met Met Gln Met Thr Phe Tyr Phe Gly
        35                  40                  45

Tyr Lys Asn Val Glu Leu Leu Phe Ala Gly Leu Val Ile Asn Thr Pro
    50                  55                  60

Gly Glu Met Ala Gly Ala Cys Ile Gly Val Phe Leu Leu Ala Val Leu
65                  70                  75                  80

Tyr Glu Gly Leu Lys Ile Gly Arg Glu Val Leu Leu Arg Arg Asn Gln
                85                  90                  95

Val Asn Val Arg Tyr Asn Ser Met Pro Val Pro Gly Ser Asp Gly Thr
            100                 105                 110

Val Leu Met Glu Thr His Lys Thr Val Gly Gln Arg Met Leu Ser Met
        115                 120                 125

Ala His Phe Leu Gln Thr Leu Leu His Ile Ile Gln Val Val Val Ser
    130                 135                 140

Tyr Phe Leu Met Leu Val Phe Met Thr Tyr Asn Gly Tyr Leu Cys Ile
145                 150                 155                 160

Ala Val Ala Ala Gly Ala Gly Leu Gly Tyr Phe Leu Phe Ser Trp Lys
                165                 170                 175

Lys Ala Val Val Val Asp Ile Thr Glu His Cys His
            180                 185

<210> 182
<211> 175
<212> PRT
<213> Homo sapiens

<400> 182
Met Asp His Ser His His Thr Gly Met Ser Cys Met Asp Ser Asn Ser
1               5                   10                  15

Thr Met Gln Arg Pro His His His Pro Thr Thr Ser Ala Ser His Ser
            20                  25                  30

Arg Gly Gly Gly Asp Ser Asn Met Met Met Met Met Pro Met Thr Phe
        35                  40                  45

Tyr Phe Gly Phe Lys Asn Val Glu Leu Leu Phe Ser Ser Leu Val Ile
    50                  55                  60

Asn Thr Ala Gly Glu Met Ala Gly Ala Phe Val Ala Val Ile Ile Leu
65                  70                  75                  80

Ala Val Phe Tyr Glu Gly Leu Asp Ser Pro Arg Glu Pro Ala Val Thr
                85                  90                  95

Lys Trp Asn His Pro Tyr Val Asp Thr Gln Asn Cys Trp Ala Ala Asp
            100                 105                 110

Ala Glu Leu Ser Ser Pro Pro Ala Asn Met Leu His Ile Ile Gln Val
        115                 120                 125

Val Ile Ser Tyr Leu Leu Met Leu Ile Phe Met Thr Tyr Asn Gly Tyr
    130                 135                 140

Leu Cys Ile Ala Val Thr Ala Gly Ala Gly Thr Arg Tyr Phe Leu Phe
145                 150                 155                 160

Ser Trp Lys Lys Ala Val Val Val Asp Ile Thr Glu Tyr Cys His
                165                 170                 175

<210> 183
<211> 483
<212> PRT
<213> Homo sapiens

<400> 183
Met Ser Ile Arg Val Thr Gln Lys Ser Tyr Lys Val Ser Thr Ser Gly
1               5                   10                  15

Pro Arg Ala Phe Ser Ser Arg Ser Tyr Thr Ser Gly Pro Gly Ser Arg
            20                  25                  30

Ile Ser Ser Ser Ser Phe Ser Arg Val Gly Ser Ser Asn Phe Arg Gly
        35                  40                  45

Gly Leu Gly Gly Gly Tyr Gly Gly Ala Ser Gly Met Gly Gly Ile Thr
    50                  55                  60

Ala Val Thr Val Asn Gln Ser Leu Leu Ser Pro Leu Ser Leu Glu Val
65                  70                  75                  80

Asp Pro Asn Ile Gln Ala Val Arg Thr Gln Glu Lys Glu Gln Ile Lys
                85                  90                  95

Thr Leu Asn Asn Lys Phe Ala Ser Phe Ile Asp Lys Val Arg Phe Leu
            100                 105                 110

Glu Gln Gln Asn Lys Met Leu Glu Thr Lys Trp Ser Leu Leu Gln Gln
        115                 120                 125

Gln Lys Thr Ala Arg Ser Asn Met Asp Asn Met Phe Glu Ser Tyr Ile
    130                 135                 140

Asn Asn Leu Arg Arg Gln Leu Glu Thr Leu Gly Gln Glu Lys Leu Lys
145                 150                 155                 160

Leu Glu Ala Glu Leu Gly Asn Met Gln Gly Leu Val Glu Asp Phe Lys
                165                 170                 175

Asn Lys Tyr Glu Asp Glu Ile Asn Lys Arg Thr Glu Met Glu Asn Glu
            180                 185                 190

Phe Val Leu Ile Lys Lys Asp Val Asp Glu Ala Tyr Met Asn Lys Val
        195                 200                 205

Glu Leu Glu Ser Arg Leu Glu Gly Leu Thr Asp Glu Ile Asn Phe Leu
    210                 215                 220

Arg Gln Leu Tyr Glu Glu Glu Ile Arg Glu Leu Gln Ser Gln Ile Ser
225                 230                 235                 240

Asp Thr Ser Val Val Leu Ser Met Asp Asn Ser Arg Ser Leu Asp Met
                245                 250                 255

Asp Ser Ile Ile Ala Glu Val Lys Ala Gln Tyr Glu Asp Ile Ala Asn
            260                 265                 270

Arg Ser Arg Ala Glu Ala Glu Ser Met Tyr Gln Ile Lys Tyr Glu Glu
        275                 280                 285

Leu Gln Ser Leu Ala Gly Lys His Gly Asp Asp Leu Arg Arg Thr Lys
    290                 295                 300

Thr Glu Ile Ser Glu Met Asn Arg Asn Ile Ser Arg Leu Gln Ala Glu
305                 310                 315                 320

Ile Glu Gly Leu Lys Gly Gln Arg Ala Ser Leu Glu Ala Ala Ile Ala
                325                 330                 335

Asp Ala Glu Gln Arg Gly Glu Leu Ala Ile Lys Asp Ala Asn Ala Lys
            340                 345                 350

Leu Ser Glu Leu Glu Ala Ala Leu Gln Arg Ala Lys Gln Asp Met Ala
        355                 360                 365

Arg Gln Leu Arg Glu Tyr Gln Glu Leu Met Asn Val Lys Leu Ala Leu
    370                 375                 380

Asp Ile Glu Ile Ala Thr Tyr Arg Lys Leu Leu Glu Gly Glu Glu Ser
385                 390                 395                 400

Arg Leu Glu Ser Gly Met Gln Asn Met Ser Ile His Thr Lys Thr Thr
                405                 410                 415

Gly Gly Tyr Ala Gly Gly Leu Ser Ser Ala Tyr Gly Asp Leu Thr Asp
            420                 425                 430

Pro Gly Leu Ser Tyr Ser Leu Gly Ser Ser Phe Gly Ser Gly Ala Gly
        435                 440                 445

Ser Ser Ser Phe Ser Arg Thr Ser Ser Ser Arg Ala Val Val Val Lys
    450                 455                 460

Lys Ile Glu Thr Arg Asp Gly Lys Leu Val Ser Glu Ser Ser Asp Val
465                 470                 475                 480

Leu Pro Lys

<210> 184
<211> 483
<212> PRT
<213> Homo sapiens

<400> 184
Met Ser Ile Arg Val Thr Gln Lys Ser Tyr Lys Val Ser Thr Ser Gly
1               5                   10                  15

Pro Arg Ala Phe Ser Ser Arg Ser Tyr Thr Ser Gly Pro Gly Ser Arg
            20                  25                  30

Ile Ser Ser Ser Ser Phe Ser Arg Val Gly Ser Ser Asn Phe Arg Gly
        35                  40                  45

Gly Leu Gly Gly Gly Tyr Gly Gly Ala Ser Gly Met Gly Gly Ile Thr
    50                  55                  60

Ala Val Thr Val Asn Gln Ser Leu Leu Ser Pro Leu Val Leu Glu Val
65                  70                  75                  80

Asp Pro Asn Ile Gln Ala Val Arg Thr Gln Glu Lys Glu Gln Ile Lys
                85                  90                  95

Thr Leu Asn Asn Lys Phe Ala Ser Phe Ile Asp Lys Val Arg Phe Leu
            100                 105                 110

Glu Gln Gln Asn Lys Met Leu Glu Thr Lys Trp Ser Leu Leu Gln Gln
        115                 120                 125

Gln Lys Thr Ala Arg Ser Asn Met Asp Asn Met Phe Glu Ser Tyr Ile
    130                 135                 140

Asn Asn Leu Arg Arg Gln Leu Glu Thr Leu Gly Gln Glu Lys Leu Lys
145                 150                 155                 160

Leu Glu Ala Glu Leu Gly Asn Met Gln Gly Leu Val Glu Asp Phe Lys
                165                 170                 175

Asn Lys Tyr Glu Asp Glu Ile Asn Lys Arg Thr Glu Met Glu Asn Glu
            180                 185                 190

Phe Val Leu Ile Lys Lys Asp Val Asp Glu Ala Tyr Met Asn Lys Val
        195                 200                 205

Glu Leu Glu Ser Arg Leu Glu Gly Leu Thr Asp Glu Ile Asn Phe Leu
    210                 215                 220

Arg Gln Leu Tyr Glu Glu Glu Ile Arg Glu Leu Gln Ser Gln Ile Ser
225                 230                 235                 240

Asp Thr Ser Val Val Leu Ser Met Asp Asn Ser Arg Ser Leu Asp Met
                245                 250                 255

Asp Ser Ile Ile Ala Glu Val Lys Ala Gln Tyr Glu Asp Ile Ala Asn
            260                 265                 270

Arg Ser Arg Ala Glu Ala Glu Ser Met Tyr Gln Ile Lys Tyr Glu Glu
        275                 280                 285

Leu Gln Ser Leu Ala Gly Lys His Gly Asp Asp Leu Arg Arg Thr Lys
    290                 295                 300

Thr Glu Ile Ser Glu Met Asn Arg Asn Ile Ser Arg Leu Gln Ala Glu
305                 310                 315                 320

Ile Glu Gly Leu Lys Gly Gln Arg Ala Ser Leu Glu Ala Ala Ile Ala
                325                 330                 335

Asp Ala Glu Gln Arg Gly Glu Leu Ala Ile Lys Asp Ala Asn Ala Lys
            340                 345                 350

Leu Ser Glu Leu Glu Ala Ala Leu Gln Arg Ala Lys Gln Asp Met Ala
        355                 360                 365

Arg Gln Leu Arg Glu Tyr Gln Glu Leu Met Asn Val Lys Leu Ala Leu
    370                 375                 380

Asp Ile Glu Ile Ala Thr Tyr Arg Lys Leu Leu Glu Gly Glu Glu Ser
385                 390                 395                 400

Arg Leu Glu Ser Gly Met Gln Asn Met Ser Ile His Thr Lys Thr Thr
                405                 410                 415

Gly Gly Tyr Ala Gly Gly Leu Ser Ser Ala Tyr Gly Gly Leu Thr Ser
            420                 425                 430

Pro Gly Leu Ser Tyr Ser Leu Gly Ser Ser Phe Gly Ser Gly Ala Gly
        435                 440                 445

Ser Ser Ser Phe Ser Arg Thr Ser Ser Ser Arg Ala Val Val Val Lys
    450                 455                 460

Lys Ile Glu Thr Arg Asp Gly Lys Leu Val Ser Glu Ser Ser Asp Val
465                 470                 475                 480

Leu Pro Lys


<210> 185
<211> 483
<212> PRT
<213> Homo sapiens

<400> 185
Met Ser Ile Arg Val Thr Gln Lys Ser Tyr Lys Val Ser Thr Ser Gly
  1               5                  10                  15

Pro Arg Ala Phe Ser Ser Arg Ser Tyr Thr Ser Gly Pro Gly Ser Arg
             20                  25                  30

Ile Ser Ser Ser Ser Phe Ser Arg Val Gly Ser Ser Asn Phe Arg Gly
         35                  40                  45

Gly Leu Gly Gly Gly Tyr Gly Gly Ala Ser Gly Met Gly Gly Ile Thr
     50                  55                  60

Ala Val Thr Val Asn Gln Ser Leu Leu Ser Pro Leu Val Leu Glu Val
 65                  70                  75                  80

Asp Pro Asn Ile Gln Ala Val Arg Thr Gln Glu Lys Glu Gln Ile Lys
                 85                  90                  95

Thr Leu Asn Asn Lys Phe Ala Ser Phe Ile Asp Lys Val Arg Phe Leu
            100                 105                 110

Glu Gln Gln Asn Lys Met Leu Glu Thr Lys Trp Ser Leu Leu Gln Gln
        115                 120                 125

Gln Lys Thr Ala Arg Ser Asn Met Asp Asn Met Phe Glu Ser Tyr Ile

130                 135                 140

Asn Asn Leu Arg Arg Gln Leu Glu Thr Leu Gly Gln Glu Lys Leu Lys
145                 150                 155                 160

Leu Glu Ala Glu Leu Gly Asn Met Gln Gly Leu Val Glu Asp Phe Lys
                165                 170                 175

Asn Lys Tyr Glu Asp Glu Ile Asn Lys Arg Thr Glu Met Glu Asn Glu
            180                 185                 190

Phe Val Leu Ile Lys Lys Asp Val Asp Glu Ala Tyr Met Asn Lys Val
        195                 200                 205

Glu Leu Glu Ser Arg Leu Glu Gly Leu Thr Asp Glu Ile Asn Phe Leu
    210                 215                 220

Arg Gln Leu Tyr Glu Glu Glu Ile Arg Glu Leu Gln Ser Gln Ile Ser
225                 230                 235                 240

Asp Thr Ser Val Val Leu Ser Met Asp Asn Ser Arg Ser Leu Asp Met
                245                 250                 255

Asp Ser Ile Ile Ala Glu Val Lys Ala Gln Tyr Glu Asp Ile Ala Asn
            260                 265                 270

Arg Ser Arg Ala Glu Ala Glu Ser Met Tyr Gln Ile Lys Tyr Glu Glu
        275                 280                 285

Leu Gln Ser Leu Ala Gly Lys His Gly Asp Asp Leu Arg Arg Thr Lys
    290                 295                 300

Thr Glu Ile Ser Glu Met Asn Arg Asn Ile Ser Arg Leu Gln Ala Glu
305                 310                 315                 320

Ile Glu Gly Leu Lys Gly Gln Arg Ala Ser Leu Glu Ala Ala Ile Ala
                325                 330                 335

Asp Ala Glu Gln Arg Gly Glu Leu Ala Ile Lys Asp Ala Asn Ala Lys
            340                 345                 350

Leu Ser Glu Leu Glu Ala Ala Leu Gln Arg Ala Lys Gln Asp Met Ala
        355                 360                 365

Arg Gln Leu Arg Glu Tyr Gln Glu Leu Met Asn Val Lys Leu Ala Leu
    370                 375                 380

Asp Ile Glu Ile Ala Thr Tyr Arg Lys Leu Leu Glu Gly Glu Glu Ser
385                 390                 395                 400

Arg Leu Glu Ser Gly Met Gln Asn Met Ser Ile His Thr Lys Thr Thr
                405                 410                 415

Ser Gly Tyr Ala Gly Gly Leu Ser Ser Ala Tyr Gly Gly Leu Thr Ser
            420                 425                 430

Pro Gly Leu Ser Tyr Ser Leu Gly Ser Ser Phe Gly Ser Gly Ala Gly
        435                 440                 445

Ser Ser Ser Phe Ser Arg Thr Ser Ser Ser Arg Ala Val Val Val Lys
    450                 455                 460

Lys Ile Glu Thr Arg Asp Gly Lys Leu Val Ser Glu Ser Ser Asp Val
465                 470                 475                 480

Leu Pro Lys

<210> 186
<211> 483
<212> PRT
<213> Homo sapiens

<400> 186

<210> 187
<211> 482
<212> PRT
<213> Homo sapiens

<400> 187


<210> 188
<211> 312
<212> PRT
<213> Homo sapiens

<400> 188
Asn Glu Lys Glu Gln Met Gln Asn Leu Asn Asp Arg Leu Ala Ser Tyr

1                 5                   10                  15

Ile Asp Lys Val Arg Phe Leu Glu Gln Gln Asn Lys Glu Leu Glu Val
                20                  25                  30

Lys Ile Glu Glu Leu Arg Gln Lys Gln Ala Pro Ser Val Ser Arg Leu
            35                  40                  45

Tyr Ser Leu Tyr Glu Thr Glu Ile Glu Glu Leu Arg Arg Gln Ile Asp
    50                  55                  60

Gln Leu Thr Asn Glu Arg Ala Arg Leu Gln Leu Glu Ile Asp Asn Leu
65                  70                  75                  80

Arg Glu Ala Ala Glu Asp Phe Arg Lys Lys Tyr Glu Asp Glu Ile Asn
                85                  90                  95

Leu Arg Gln Glu Ala Glu Asn Asp Leu Val Gly Leu Arg Lys Asp Leu
            100                 105                 110

Asp Glu Ala Thr Leu Ala Arg Val Asp Leu Glu Asn Lys Val Glu Ser
        115                 120                 125

Leu Gln Glu Glu Leu Glu Phe Leu Lys Lys Asn His Glu Glu Glu Val
    130                 135                 140

Lys Glu Leu Gln Ala Gln Ile Gln Asp Thr Val Asn Val Glu Met Asp
145                 150                 155                 160

Ala Ala Arg Lys Leu Asp Leu Thr Lys Ala Leu Arg Glu Ile Arg Ala
                165                 170                 175

Gln Tyr Glu Glu Ile Ala Lys Lys Asn Arg Gln Glu Ala Glu Glu Trp
            180                 185                 190

Tyr Lys Ser Lys Leu Glu Glu Leu Gln Thr Ala Ala Ala Arg Asn Gly
        195                 200                 205

Glu Ala Leu Arg Ser Ala Lys Glu Glu Ile Thr Glu Leu Arg Arg Gln
    210                 215                 220

Ile Gln Ser Leu Glu Ile Glu Leu Gln Ser Leu Lys Ala Gln Asn Ala
225                 230                 235                 240

Ser Leu Glu Arg Gln Leu Ala Glu Leu Glu Glu Arg Tyr Glu Leu Glu
                245                 250                 255

Leu Arg Gln Tyr Gln Ala Leu Ile Ser Gln Leu Glu Glu Glu Leu Gln
            260                 265                 270

Gln Leu Arg Glu Glu Met Ala Arg Gln Leu Arg Glu Tyr Gln Glu Leu
        275                 280                 285

Leu Asp Val Lys Leu Ala Leu Asp Ile Glu Ile Ala Thr Tyr Arg Lys
    290                 295                 300

Leu Leu Glu Gly Glu Glu Ser Arg
305                 310


<210> 189
<211> 947
<212> PRT
<213> Homo sapiens

<400> 189
Gly Asn Ala Arg Ser Ser Gln Ala Leu Gly Val Ser Trp Cys Pro Ala
1               5                   10                  15

Thr Trp His Pro Arg Leu Asp Met Gly Arg Leu His Arg Pro Arg Ser
            20                  25                  30

Ser Thr Ser Tyr Arg Asn Leu Pro His Leu Phe Leu Phe Phe Leu Phe
        35                  40                  45

Val Gly Pro Phe Ser Cys Leu Gly Ser Tyr Ser Arg Ala Thr Glu Leu
    50                  55                  60

Leu Tyr Ser Leu Asn Glu Gly Leu Pro Ala Gly Val Leu Ile Gly Ser
65                  70                  75                  80

Leu Ala Glu Asp Leu Arg Leu Leu Pro Arg Ser Ala Gly Arg Pro Asp
                85                  90                  95

Pro Gln Ser Gln Leu Pro Glu Arg Thr Gly Ala Glu Trp Asn Pro Pro
            100                 105                 110

Leu Ser Phe Ser Leu Ala Ser Arg Gly Leu Ser Gly Gln Tyr Val Thr
        115                 120                 125

Leu Asp Asn Arg Ser Gly Glu Leu His Thr Ser Ala Gln Glu Ile Asp
    130                 135                 140

Arg Glu Ala Leu Cys Val Glu Gly Gly Gly Gly Thr Ala Trp Ser Gly
145                 150                 155                 160

Ser Val Ser Ile Ser Ser Ser Pro Ser Asp Ser Cys Leu Leu Leu Leu
                165                 170                 175

Asp Val Leu Val Leu Pro Gln Glu Tyr Phe Arg Phe Val Lys Val Lys
            180                 185                 190

Ile Ala Ile Arg Asp Ile Asn Asp Asn Ala Pro Gln Phe Pro Val Ser
        195                 200                 205

Gln Ile Ser Val Trp Val Pro Glu Asn Ala Pro Val Asn Thr Arg Leu
    210                 215                 220

Ala Ile Glu His Pro Ala Val Asp Pro Asp Val Gly Ile Asn Gly Val
225                 230                 235                 240

Gln Thr Tyr Arg Leu Leu Asp Tyr His Gly Met Phe Thr Leu Asp Val
                245                 250                 255

Glu Glu Asn Glu Asn Gly Glu Arg Thr Pro Tyr Leu Ile Val Met Gly
            260                 265                 270

Ala Leu Asp Arg Glu Thr Gln Asp Gln Tyr Val Ser Ile Ile Thr Ala
        275                 280                 285

Glu Asp Gly Gly Ser Pro Pro Leu Leu Gly Ser Ala Thr Leu Thr Ile
    290                 295                 300

Gly Ile Ser Asp Ile Asn Asp Asn Cys Pro Leu Phe Thr Asp Ser Gln
305                 310                 315                 320

Ile Asn Val Thr Val Tyr Gly Asn Ala Thr Val Gly Thr Pro Ile Ala
                325                 330                 335

Ala Val Gln Ala Val Asp Lys Asp Leu Gly Thr Asn Ala Gln Ile Thr
            340                 345                 350

Tyr Ser Tyr Ser Gln Lys Val Pro Gln Ala Ser Lys Asp Leu Phe His
        355                 360                 365

Leu Asp Glu Asn Thr Gly Val Ile Lys Leu Phe Ser Lys Ile Gly Gly
    370                 375                 380

Ser Val Leu Glu Ser His Lys Leu Thr Ile Leu Ala Asn Gly Pro Gly
385                 390                 395                 400

Cys Ile Pro Ala Val Ile Thr Ala Leu Val Ser Ile Ile Lys Val Ile
                405                 410                 415

Phe Arg Pro Pro Glu Ile Val Pro Arg Tyr Ile Ala Asn Glu Ile Asp
            420                 425                 430

Gly Val Val Tyr Leu Lys Glu Leu Glu Pro Val Asn Thr Pro Ile Ala
        435                 440                 445

Phe Phe Thr Ile Arg Asp Pro Glu Gly Lys Tyr Lys Val Asn Cys Tyr
    450                 455                 460

Leu Asp Gly Glu Gly Pro Phe Arg Leu Ser Pro Tyr Lys Pro Tyr Asn
465                 470                 475                 480

Asn Glu Tyr Leu Leu Glu Thr Thr Lys Pro Met Asp Tyr Glu Leu Gln
                485                 490                 495

Gln Phe Tyr Glu Val Ala Val Val Ala Trp Asn Ser Glu Gly Phe His
            500                 505                 510

Val Lys Arg Val Ile Lys Val Gln Leu Leu Asp Asp Asn Asp Asn Ala
        515                 520                 525

Pro Ile Phe Leu Gln Pro Leu Ile Glu Leu Thr Ile Glu Glu Asn Asn
    530                 535                 540

Ser Pro Asn Ala Phe Leu Thr Lys Leu Tyr Ala Thr Asp Ala Asp Ser
545                 550                 555                 560

Glu Glu Arg Gly Gln Val Ser Tyr Phe Leu Gly Pro Asp Ala Pro Ser
                565                 570                 575

Tyr Phe Ser Leu Asp Ser Val Thr Gly Ile Leu Thr Val Ser Thr Gln
            580                 585                 590

Leu Asp Arg Glu Glu Lys Glu Lys Tyr Arg Tyr Thr Val Arg Ala Val
        595                 600                 605

Asp Cys Gly Lys Pro Pro Arg Glu Ser Val Ala Thr Val Ala Leu Thr
    610                 615                 620

Val Leu Asp Lys Asn Asp Asn Ser Pro Arg Phe Ile Asn Lys Asp Phe
625                 630                 635                 640

Ser Phe Phe Val Pro Glu Asn Phe Pro Gly Tyr Gly Glu Ile Gly Val
                645                 650                 655

Ile Ser Val Thr Asp Ala Asp Ala Gly Arg Asn Gly Trp Val Ala Leu
            660                 665                 670

Ser Val Val Asn Gln Ser Asp Ile Phe Val Ile Asp Thr Gly Lys Gly
        675                 680                 685

Met Leu Arg Ala Lys Val Ser Leu Asp Arg Glu Gln Gln Ser Ser Tyr
    690                 695                 700

Thr Leu Trp Val Glu Ala Val Asp Gly Gly Glu Pro Ala Leu Ser Ser
705                 710                 715                 720

Thr Ala Lys Ile Thr Ile Leu Leu Leu Asp Ile Asn Asp Asn Pro Pro
                725                 730                 735

Leu Val Leu Phe Pro Gln Ser Asn Met Ser Tyr Leu Leu Val Leu Pro
            740                 745                 750

Ser Thr Leu Pro Gly Ser Pro Val Thr Glu Val Tyr Ala Val Asp Lys
        755                 760                 765

Asp Thr Gly Met Asn Ala Val Ile Ala Tyr Ser Ile Ile Gly Arg Arg
    770                 775                 780

Gly Pro Arg Pro Glu Ser Phe Arg Ile Asp Pro Lys Thr Gly Asn Ile
785                 790                 795                 800

Thr Leu Glu Glu Ala Leu Leu Gln Thr Asp Tyr Gly Leu His Arg Leu
                805                 810                 815

Leu Val Lys Val Ser Asp His Gly Tyr Pro Glu Pro Leu His Ser Thr
            820                 825                 830

Val Met Val Asn Leu Phe Val Asn Asp Thr Val Ser Asn Glu Ser Tyr
        835                 840                 845

Ile Glu Ser Leu Leu Arg Lys Glu Pro Glu Ile Asn Ile Glu Glu Lys
    850                 855                 860

Glu Pro Gln Ile Ser Ile Glu Pro Thr His Arg Lys Val Glu Ser Val
865                 870                 875                 880

Ser Cys Met Pro Thr Leu Val Ala Leu Ser Val Ile Ser Leu Gly Ser
                885                 890                 895

Ile Thr Leu Val Thr Gly Met Gly Ile Tyr Ile Cys Leu Arg Lys Gly
            900                 905                 910

Glu Lys His Pro Arg Glu Asp Glu Asn Leu Glu Val Gln Ile Pro Leu
        915                 920                 925

Lys Gly Lys Ile Asp Leu His Met Arg Glu Arg Lys Pro Met Asp Ile
    930                 935                 940

Ser Asn Ile
945


<210> 190
<211> 924
<212> PRT
<213> Homo sapiens

<400> 190

<210> 191
<211> 924
<212> PRT
<213> Homo sapiens

<400> 191
Met Gly Arg Leu His Arg Pro Arg Ser Ser Thr Ser Tyr Arg Asn Leu
  1               5                  10                  15

Pro His Leu Phe Leu Phe Phe Leu Phe Val Gly Pro Phe Ser Cys Leu
                 20                  25                  30

Gly Ser Tyr Ser Arg Ala Thr Glu Leu Leu Tyr Ser Leu Asn Glu Gly
         35                  40                  45

Leu Pro Ala Gly Val Leu Ile Gly Ser Leu Ala Glu Asp Leu Arg Leu
     50                  55                  60

Leu Pro Arg Ser Ala Gly Arg Pro Asp Pro Gln Ser Gln Leu Pro Glu
 65                  70                  75                  80

Arg Thr Gly Ala Glu Trp Asn Pro Pro Leu Ser Phe Ser Leu Ala Ser
                 85                  90                  95

Arg Gly Leu Ser Gly Gln Tyr Val Thr Leu Asp Asn Arg Ser Gly Glu
            100                 105                 110

Leu His Thr Ser Ala Gln Glu Ile Asp Arg Glu Ala Leu Cys Val Glu
        115                 120                 125

Gly Gly Gly Gly Thr Ala Trp Ser Gly Ser Val Ser Ile Ser Ser Ser
    130                 135                 140

Pro Ser Asp Ser Cys Leu Leu Leu Leu Asp Val Leu Val Leu Pro Gln
145                 150                 155                 160

Glu Tyr Phe Arg Phe Val Lys Val Lys Ile Ala Ile Arg Asp Ile Asn
                165                 170                 175

Asp Asn Ala Pro Gln Phe Pro Val Ser Gln Ile Ser Val Trp Val Pro
            180                 185                 190

Glu Asn Ala Pro Val Asn Thr Arg Leu Ala Ile Glu His Pro Ala Val
        195                 200                 205

Asp Pro Asp Val Gly Ile Asn Gly Val Gln Thr Tyr Arg Leu Leu Asp
    210                 215                 220

Tyr His Gly Met Phe Thr Leu Asp Val Glu Glu Asn Glu Asn Gly Glu
225                 230                 235                 240

Arg Thr Pro Tyr Leu Ile Val Met Gly Ala Leu Asp Arg Glu Thr Gln
                245                 250                 255

Asp Gln Tyr Val Ser Ile Ile Thr Ala Glu Asp Gly Gly Ser Pro Pro

260                 265                 270

Leu Leu Gly Ser Ala Thr Leu Thr Ile Gly Ile Ser Asp Ile Asn Asp
        275                 280                 285

Asn Cys Pro Leu Phe Thr Asp Ser Gln Ile Asn Val Thr Val Tyr Gly
    290                 295                 300

Asn Ala Thr Val Gly Thr Pro Ile Ala Ala Val Gln Ala Val Asp Lys
305                 310                 315                 320

Asp Leu Gly Thr Asn Ala Gln Ile Thr Tyr Ser Tyr Ser Gln Lys Val
                325                 330                 335

Pro Gln Ala Ser Lys Asp Leu Phe His Leu Asp Glu Asn Thr Gly Val
            340                 345                 350

Ile Lys Leu Phe Ser Lys Ile Gly Gly Ser Val Leu Glu Ser His Lys
        355                 360                 365

Leu Thr Ile Leu Ala Asn Gly Pro Gly Cys Ile Pro Ala Val Ile Thr
    370                 375                 380

Ala Leu Val Ser Ile Ile Lys Val Ile Phe Arg Pro Pro Glu Ile Val
385                 390                 395                 400

Pro Arg Tyr Ile Ala Asn Glu Ile Asp Gly Val Val Tyr Leu Lys Glu
                405                 410                 415

Leu Glu Pro Val Asn Thr Pro Ile Ala Phe Phe Thr Ile Arg Asp Pro
            420                 425                 430

Glu Gly Lys Tyr Lys Val Asn Cys Tyr Leu Asp Gly Glu Gly Pro Phe
        435                 440                 445

Arg Leu Ser Pro Tyr Lys Pro Tyr Asn Asn Glu Tyr Leu Leu Glu Thr
    450                 455                 460

Thr Lys Pro Met Asp Tyr Glu Leu Gln Gln Phe Tyr Glu Val Ala Val
465                 470                 475                 480

Val Ala Trp Asn Ser Glu Gly Phe His Val Lys Arg Val Ile Lys Val
                485                 490                 495

Gln Leu Leu Asp Asp Asn Asp Asn Ala Pro Ile Phe Leu Gln Pro Leu
            500                 505                 510

Ile Glu Leu Thr Ile Glu Glu Asn Asn Ser Pro Asn Ala Phe Leu Thr
        515                 520                 525

Lys Leu Tyr Ala Thr Asp Ala Asp Ser Glu Glu Arg Gly Gln Val Ser
    530                 535                 540

Tyr Phe Leu Gly Pro Asp Ala Pro Ser Tyr Phe Ser Leu Asp Ser Val
545                 550                 555                 560

Thr Gly Ile Leu Thr Val Ser Thr Gln Leu Asp Arg Glu Glu Lys Glu
                565                 570                 575

Lys Tyr Arg Tyr Thr Val Arg Ala Val Asp Cys Gly Lys Pro Pro Arg
            580                 585                 590

Glu Ser Val Ala Thr Val Ala Leu Thr Val Leu Asp Lys Asn Asp Asn
        595                 600                 605

Ser Pro Arg Phe Ile Asn Lys Asp Phe Ser Phe Phe Val Pro Glu Asn
    610                 615                 620

Phe Pro Gly Tyr Gly Glu Ile Gly Val Ile Ser Val Thr Asp Ala Asp

625                 630                 635                 640

Ala Gly Arg Asn Gly Trp Val Ala Leu Ser Val Val Asn Gln Ser Asp
                645                 650                 655

Ile Phe Val Ile Asp Thr Gly Lys Gly Met Leu Arg Ala Lys Val Ser
            660                 665                 670

Leu Asp Arg Glu Gln Gln Ser Ser Tyr Thr Leu Trp Val Glu Ala Val
        675                 680                 685

Asp Gly Gly Glu Pro Ala Leu Ser Ser Thr Ala Lys Ile Thr Ile Leu
    690                 695                 700

Leu Leu Asp Ile Asn Asp Asn Pro Pro Leu Val Leu Phe Pro Gln Ser
705                 710                 715                 720

Asn Met Ser Tyr Leu Leu Val Leu Pro Ser Thr Leu Pro Gly Ser Pro
                725                 730                 735

Val Thr Glu Val Tyr Ala Val Asp Lys Asp Thr Gly Met Asn Ala Val
            740                 745                 750

Ile Ala Tyr Ser Ile Ile Gly Arg Arg Gly Pro Arg Pro Glu Ser Phe
        755                 760                 765

Arg Ile Asp Pro Lys Thr Gly Asn Ile Thr Leu Glu Glu Ala Leu Leu
    770                 775                 780

Gln Thr Asp Tyr Gly Leu His Arg Leu Leu Val Lys Val Ser Asp His
785                 790                 795                 800

Gly Tyr Pro Glu Pro Leu His Ser Thr Val Met Val Asn Leu Phe Val
                805                 810                 815

Asn Asp Thr Val Ser Asn Glu Ser Tyr Ile Glu Ser Leu Leu Arg Lys
            820                 825                 830

Glu Pro Glu Ile Asn Ile Glu Glu Lys Glu Pro Gln Ile Ser Ile Glu
        835                 840                 845

Pro Thr His Arg Lys Val Glu Ser Val Ser Cys Met Pro Thr Leu Val
    850                 855                 860

Ala Leu Ser Val Ile Ser Leu Gly Ser Ile Thr Leu Val Thr Gly Met
865                 870                 875                 880

Gly Ile Tyr Ile Cys Leu Arg Lys Gly Glu Lys His Pro Arg Glu Asp
                885                 890                 895

Glu Asn Leu Glu Val Gln Ile Pro Leu Lys Gly Lys Ile Asp Leu His
            900                 905                 910

Met Arg Glu Arg Lys Pro Met Asp Ile Ser Asn Ile
        915                 920


<210> 192
<211> 1203
<212> PRT
<213> Homo sapiens

<400> 192
Met Asp Leu Arg Asp Phe Tyr Leu Leu Ala Ala Leu Ile Ala Cys Leu
1               5                   10                  15

Arg Leu Asp Ser Ala Ile Ala Gln Glu Leu Ile Tyr Thr Ile Arg Glu
            20                  25                  30

Glu Leu Pro Glu Asn Val Pro Ile Gly Asn Ile Pro Lys Asp Leu Asn
        35                  40                  45

Ile Ser His Ile Asn Ala Ala Thr Gly Thr Ser Ala Ser Leu Val Tyr
    50                  55                  60

Arg Leu Val Ser Lys Ala Gly Asp Ala Pro Leu Val Lys Val Ser Ser
65                  70                  75                  80

Ser Thr Gly Glu Ile Phe Thr Thr Ser Asn Arg Ile Asp Arg Glu Lys
                85                  90                  95

Leu Cys Ala Gly Ala Ser Tyr Ala Glu Glu Asn Glu Cys Phe Phe Glu
            100                 105                 110

Leu Glu Val Val Ile Leu Pro Asn Asp Phe Phe Arg Leu Ile Lys Ile
        115                 120                 125

Lys Ile Ile Val Lys Asp Thr Asn Asp Asn Ala Pro Met Phe Pro Ser
    130                 135                 140

Pro Val Ile Asn Ile Ser Ile Pro Glu Asn Thr Leu Ile Asn Ser Arg
145                 150                 155                 160

Phe Pro Ile Pro Ser Ala Thr Asp Pro Asp Thr Gly Phe Asn Gly Val
                165                 170                 175

Gln His Tyr Glu Leu Leu Asn Gly Gln Ser Val Phe Gly Leu Asp Ile
            180                 185                 190

Val Glu Thr Pro Glu Gly Glu Lys Trp Pro Gln Leu Ile Val Gln Gln
        195                 200                 205

Asn Leu Asp Arg Glu Gln Lys Asp Thr Tyr Val Met Lys Ile Lys Val
    210                 215                 220

Glu Asp Gly Gly Thr Pro Gln Lys Ser Ser Thr Ala Ile Leu Gln Val
225                 230                 235                 240

Thr Val Ser Asp Val Asn Asp Asn Arg Pro Val Phe Lys Glu Gly Gln
                245                 250                 255

Val Glu Val His Ile Pro Glu Asn Ala Pro Val Gly Thr Ser Val Ile
            260                 265                 270

Gln Leu His Ala Thr Asp Ala Asp Ile Gly Ser Asn Ala Glu Ile Arg
        275                 280                 285

Tyr Ile Phe Gly Ala Gln Val Ala Pro Ala Thr Lys Arg Leu Phe Ala
    290                 295                 300

Leu Asn Asn Thr Thr Gly Leu Ile Thr Val Gln Arg Ser Leu Asp Arg
305                 310                 315                 320

Glu Glu Thr Ala Ile His Lys Val Thr Val Leu Ala Ser Asp Gly Ser
                325                 330                 335

Ser Thr Pro Ala Arg Ala Thr Val Thr Ile Asn Val Thr Asp Val Asn
            340                 345                 350

Asp Asn Pro Pro Asn Ile Asp Leu Arg Tyr Ile Ile Ser Pro Ile Asn
        355                 360                 365

Gly Thr Val Tyr Leu Ser Glu Lys Asp Pro Val Asn Thr Lys Ile Ala
    370                 375                 380

Leu Ile Thr Val Ser Asp Lys Asp Thr Asp Val Asn Gly Lys Val Ile
385                 390                 395                 400

Cys Phe Ile Glu Arg Glu Val Pro Phe His Leu Lys Ala Val Tyr Asp
                405                 410                 415

Asn Gln Tyr Leu Leu Glu Thr Ser Ser Leu Leu Asp Tyr Glu Gly Thr
            420                 425                 430

Lys Glu Phe Ser Phe Lys Ile Val Ala Ser Asp Ser Gly Lys Pro Ser
        435                 440                 445

Leu Asn Gln Thr Ala Leu Val Arg Val Lys Leu Glu Asp Glu Asn Asp
    450                 455                 460

Asn Pro Pro Ile Phe Asn Gln Pro Val Ile Glu Leu Ser Val Ser Glu
465                 470                 475                 480

Asn Asn Arg Arg Gly Leu Tyr Leu Thr Thr Ile Ser Ala Thr Asp Glu
                485                 490                 495

Asp Ser Gly Lys Asn Ala Asp Ile Val Tyr Gln Leu Gly Pro Asn Ala
            500                 505                 510

Ser Phe Phe Asp Leu Asp Arg Lys Thr Gly Val Leu Thr Ala Ser Arg
        515                 520                 525

Val Phe Asp Arg Glu Glu Gln Glu Arg Phe Ile Phe Thr Val Thr Ala
    530                 535                 540

Arg Asp Asn Gly Thr Pro Pro Leu Gln Ser Gln Ala Ala Val Ile Val
545                 550                 555                 560

Thr Val Leu Asp Glu Asn Asp Asn Ser Pro Lys Phe Thr His Asn His
                565                 570                 575

Phe Gln Phe Phe Val Ser Glu Asn Leu Pro Lys Tyr Ser Thr Val Gly
            580                 585                 590

Val Ile Thr Val Thr Asp Ala Asp Ala Gly Glu Asn Lys Ala Val Thr
        595                 600                 605

Leu Ser Ile Leu Asn Asp Asn Asp Asn Phe Val Leu Asp Pro Tyr Ser
    610                 615                 620

Gly Val Ile Lys Ser Asn Val Ser Phe Asp Arg Glu Gln Gln Ser Ser
625                 630                 635                 640

Tyr Thr Phe Asp Val Lys Ala Thr Asp Gly Gly Gln Pro Pro Arg Ser
                645                 650                 655

Ser Thr Ala Lys Val Thr Ile Asn Val Met Asp Val Asn Asp Asn Ser
            660                 665                 670

Pro Val Val Ile Ser Pro Pro Ser Asn Thr Ser Phe Lys Leu Val Pro
        675                 680                 685

Leu Ser Ala Ile Pro Gly Ser Val Val Ala Glu Val Phe Ala Val Asp
    690                 695                 700

Val Asp Thr Gly Met Asn Ala Glu Leu Lys Tyr Thr Ile Val Ser Gly
705                 710                 715                 720

Asn Asn Lys Gly Leu Phe Arg Ile Asp Pro Val Thr Gly Asn Ile Thr
                725                 730                 735

Leu Glu Glu Lys Pro Ala Pro Thr Asp Val Gly Leu His Arg Leu Val
            740                 745                 750

Val Asn Ile Ser Asp Leu Gly Tyr Pro Lys Ser Leu His Thr Leu Val
        755                 760                 765

Leu Val Phe Leu Tyr Val Asn Asp Thr Ala Gly Asn Ala Ser Tyr Ile
    770                 775                 780

Tyr Asp Leu Ile Arg Arg Thr Met Glu Thr Pro Leu Asp Arg Asn Ile
785                 790                 795                 800

Gly Asp Ser Ser Gln Pro Tyr Gln Asn Glu Asp Tyr Leu Thr Ile Met
                805                 810                 815

Ile Ala Ile Ile Ala Gly Ala Met Val Val Ile Val Val Ile Phe Val
            820                 825                 830

Thr Val Leu Val Arg Cys Arg His Ala Ser Arg Phe Lys Ala Ala Gln
        835                 840                 845

Arg Ser Lys Gln Gly Ala Glu Trp Met Ser Pro Asn Gln Glu Asn Lys
    850                 855                 860

Gln Asn Lys Lys Lys Lys Arg Lys Lys Arg Lys Ser Pro Lys Ser Ser
865                 870                 875                 880

Leu Leu Asn Phe Val Thr Ile Glu Glu Ser Lys Pro Asp Asp Ala Val
                885                 890                 895

His Glu Pro Ile Asn Gly Thr Ile Ser Leu Pro Ala Glu Leu Glu Glu
            900                 905                 910

Gln Ser Ile Gly Arg Phe Asp Trp Gly Pro Ala Pro Pro Thr Thr Phe
        915                 920                 925

Lys Pro Asn Ser Pro Asp Leu Ala Lys His Tyr Lys Ser Ala Ser Pro
    930                 935                 940

Gln Pro Ala Phe His Leu Lys Pro Asp Thr Pro Val Ser Val Lys Lys
945                 950                 955                 960

His His Val Ile Gln Glu Leu Pro Leu Asp Asn Thr Phe Val Gly Gly
                965                 970                 975

Cys Asp Thr Leu Ser Lys Arg Ser Ser Thr Ser Ser Asp His Phe Ser
            980                 985                 990

Ala Ser Glu Cys Ser Ser Gln Gly Gly Phe Lys Thr Lys Gly Pro Leu
        995                 1000                1005

His Thr Arg Gln Ser Gln Arg Arg Val Thr Phe His Leu Pro Asp Gly
    1010                1015                1020

Ser Gln Glu Ser Cys Ser Asp Ser Gly Leu Gly Asp His Glu Pro Val
1025                1030                1035                1040

Gly Ser Gly Thr Leu Ile Ser His Pro Leu Pro Leu Val Gln Pro Gln
                1045                1050                1055

Asp Glu Phe Tyr Asp Gln Ala Ser Pro Asp Lys Arg Thr Glu Ala Asp
            1060                1065                1070

Gly Asn Ser Asp Pro Asn Ser Asp Gly Pro Leu Gly Pro Arg Gly Leu
        1075                1080                1085

Ala Glu Ala Thr Glu Met Cys Thr Gln Glu Cys Leu Val Leu Gly His
    1090                1095                1100

Ser Asp Asn Cys Trp Met Pro Pro Gly Leu Gly Pro Tyr Gln His Pro
1105                1110                1115                1120

Lys Ser Pro Leu Ser Thr Phe Ala Pro Gln Lys Glu Trp Val Lys Lys
                1125                1130                1135

Asp Lys Leu Val Asn Gly His Thr Leu Thr Arg Ala Trp Lys Glu Asp
            1140                1145                1150

Ser Asn Arg Asn Gln Phe Asn Asp Arg Lys Gln Tyr Gly Ser Asn Glu
        1155                1160                1165

Gly His Phe Asn Asn Gly Ser His Met Thr Asp Ile Pro Leu Ala Asn
    1170                1175                1180

Leu Lys Ser Tyr Lys Gln Ala Gly Gly Ala Thr Glu Ser Pro Lys Glu
1185                1190                1195                1200

His Gln Leu

<210> 193
<211> 1032
<212> PRT
<213> Macaca fascicularis

<400> 193
Met Asp Leu Arg Asp Phe Tyr Leu Leu Ala Ala Leu Ile Ala Cys Leu
1               5                   10                  15

Arg Leu Asp Ser Ala Ile Ala Gln Glu Leu Ile Tyr Thr Ile Arg Glu
            20                  25                  30

Glu Leu Pro Glu Asn Val Pro Ile Gly Asn Ile Pro Lys Asp Leu Asn
        35                  40                  45

Ile Ser His Ile Asn Ala Ala Thr Gly Thr Ser Ala Ser Leu Val Tyr
    50                  55                  60

Arg Leu Val Ser Lys Ala Gly Asp Ala Pro Leu Val Lys Val Ser Ser
65                  70                  75                  80

Ser Thr Gly Glu Ile Phe Thr Thr Ser Asn Arg Ile Asp Arg Glu Lys
                85                  90                  95

Leu Cys Ala Gly Ala Ser Tyr Ala Glu Glu Asn Glu Cys Phe Phe Glu
            100                 105                 110

Leu Glu Val Val Ile Leu Pro Asn Asp Phe Phe Arg Leu Ile Lys Ile
        115                 120                 125

Lys Ile Ile Val Lys Asp Thr Asn Asp Asn Ala Pro Met Phe Pro Pro
    130                 135                 140

Pro Val Ile Asn Ile Ser Ile Pro Glu Asn Thr Leu Ile Asn Ser Arg
145                 150                 155                 160

Phe Pro Ile Pro Ser Ala Thr Asp Pro Asp Thr Gly Phe Asn Gly Val
                165                 170                 175

Gln His Tyr Glu Leu Leu Asn Gly Gln Ser Val Phe Gly Leu Asp Ile
            180                 185                 190

Val Glu Thr Pro Glu Gly Glu Lys Trp Pro Gln Leu Ile Val Gln Gln
        195                 200                 205

Asn Leu Asp Arg Glu Gln Lys Asp Thr Tyr Val Met Lys Ile Lys Val
    210                 215                 220

Glu Asp Gly Gly Thr Pro Gln Lys Ser Ser Thr Ala Ile Leu Gln Val
225                 230                 235                 240

Thr Val Ser Asp Val Asn Asp Asn Arg Pro Val Phe Lys Glu Gly Gln
                245                 250                 255

Val Glu Val His Ile Pro Glu Asn Ala Pro Val Gly Thr Ser Val Ile
            260                 265                 270

Gln Leu His Ala Thr Asp Ala Asp Ile Gly Ser Asn Ala Glu Ile Arg
        275                 280                 285

Tyr Ile Phe Gly Ala Gln Val Ala Pro Ala Thr Lys Arg Leu Phe Ala
    290                 295                 300

Leu Asn Asn Thr Thr Gly Leu Ile Thr Val Gln Arg Ser Leu Asp Arg
305                 310                 315                 320

Glu Glu Thr Ala Ile His Lys Val Thr Val Leu Ala Ser Asp Gly Ser
                325                 330                 335

Ser Thr Pro Ala Arg Ala Thr Val Thr Ile Asn Val Thr Asp Val Asn
            340                 345                 350

Asp Asn Pro Pro Asn Ile Asp Leu Arg Tyr Ile Ile Ser Pro Ile Asn
        355                 360                 365

Gly Thr Val Tyr Leu Ser Glu Lys Asp Pro Val Asn Thr Lys Ile Ala
    370                 375                 380

Leu Ile Thr Val Ser Asp Lys Asp Thr Asp Val Asn Gly Lys Val Ile
385                 390                 395                 400

Cys Phe Ile Glu Arg Glu Val Pro Phe His Leu Lys Ala Val Tyr Asp
                405                 410                 415

Asn Gln Tyr Leu Leu Glu Thr Ser Ser Leu Leu Asp Tyr Glu Gly Thr
            420                 425                 430

Lys Glu Phe Ser Phe Lys Ile Val Ala Ser Asp Ser Gly Lys Pro Ser
        435                 440                 445

Leu Asn Gln Thr Ala Leu Val Arg Val Lys Leu Glu Asp Glu Asn Asp
    450                 455                 460

Asn Pro Pro Val Phe Asn Gln Pro Val Ile Glu Leu Ser Val Ser Glu
465                 470                 475                 480

Asn Asn Arg Arg Gly Leu Tyr Leu Thr Thr Ile Ser Ala Thr Asp Glu
                485                 490                 495

Asp Ser Gly Lys Asn Ala Asp Ile Val Tyr Gln Leu Gly Pro Asn Ala
            500                 505                 510

Ser Phe Phe Asp Leu Asp Arg Lys Thr Gly Val Leu Thr Ala Ser Arg
        515                 520                 525

Val Phe Asp Arg Glu Glu Gln Glu Arg Phe Ile Phe Thr Val Thr Ala
    530                 535                 540

Arg Asp Asn Gly Thr Pro Pro Leu Gln Ser Gln Ala Ala Val Ile Val
545                 550                 555                 560

Thr Val Leu Asp Glu Asn Asp Asn Ser Pro Lys Phe Thr His Asn His
                565                 570                 575

Phe Gln Phe Phe Val Ser Glu Asn Leu Pro Lys Tyr Ser Thr Val Gly
            580                 585                 590

Val Ile Thr Val Thr Asp Ala Asp Ala Gly Glu Asn Lys Ala Val Thr
        595                 600                 605

Leu Ser Ile Leu Asn Asp Asn Asp Asn Phe Val Leu Asp Pro Tyr Ser
    610                 615                 620

Gly Val Ile Lys Ser Asn Val Ser Phe Asp Arg Glu Gln Gln Ser Ser
625                 630                 635                 640

Tyr Thr Phe Asp Val Lys Ala Thr Asp Gly Gly Gln Pro Pro Arg Ser
                645                 650                 655

Ser Thr Ala Lys Val Thr Ile Asn Val Met Asp Val Asn Asp Asn Ser
            660                 665                 670

Pro Val Val Ile Ser Pro Pro Ser Asn Thr Ser Phe Lys Leu Val Pro
        675                 680                 685

Leu Ser Ala Ile Pro Gly Ser Val Val Ala Glu Val Phe Ala Val Asp
    690                 695                 700

Val Asp Thr Gly Met Asn Ala Glu Leu Lys Tyr Thr Ile Val Ser Gly
705                 710                 715                 720

Asn Asn Lys Gly Leu Phe Arg Ile Asp Pro Val Thr Gly Asn Ile Thr
                725                 730                 735

Leu Glu Glu Lys Pro Ala Pro Thr Asp Val Gly Leu His Arg Leu Val
            740                 745                 750

Val Asn Ile Ser Asp Leu Gly Tyr Pro Lys Ser Leu His Thr Leu Val
        755                 760                 765

Leu Val Phe Leu Tyr Val Asn Asp Thr Ala Gly Asn Ala Ser Tyr Ile
    770                 775                 780

Tyr Asp Leu Ile Arg Arg Thr Met Glu Thr Pro Leu Asp Arg Asn Ile
785                 790                 795                 800

Gly Asp Ser Ser Gln Pro Tyr Gln Asn Glu Asp Tyr Leu Thr Ile Met
                805                 810                 815

Ile Ala Ile Ile Ala Gly Ala Met Val Val Ile Val Val Ile Phe Val
            820                 825                 830

Thr Val Leu Val Arg Cys Arg His Ala Ser Arg Phe Lys Ala Ala Gln
        835                 840                 845

Arg Ser Lys Gln Gly Ala Glu Trp Met Ser Pro Asn Gln Glu Asn Lys
    850                 855                 860

Gln Asn Lys Lys Lys Lys Arg Lys Lys Arg Lys Ser Pro Lys Ser Ser
865                 870                 875                 880

Leu Leu Asn Phe Val Thr Ile Glu Glu Ser Lys Pro Asp Asp Ala Val
                885                 890                 895

His Glu Pro Ile Asn Gly Thr Ile Ser Leu Pro Ala Glu Leu Glu Glu
            900                 905                 910

Gln Ser Ile Gly Arg Phe Asp Trp Gly Pro Ala Pro Pro Thr Thr Phe
        915                 920                 925

Lys Pro Asn Ser Pro Asp Leu Ala Lys His Tyr Lys Ser Ala Ser Pro
    930                 935                 940

Gln Pro Ala Phe His Leu Lys Pro Asp Thr Pro Val Ser Val Lys Lys
945                 950                 955                 960

His His Val Ile Gln Glu Leu Pro Leu Asp Asn Thr Phe Val Gly Gly
                965                 970                 975

Cys Asp Thr Leu Ser Lys Arg Ser Ser Thr Ser Ser Asp His Phe Ser
            980                 985                 990

Ala Ser Glu Cys Ser Ser Gln Gly Gly Phe Lys Thr Lys Gly Pro Leu
        995                 1000                1005

His Thr Arg Gln Val Asn Glu His Phe Tyr Trp Ser Ile Ser Thr Ala
    1010                1015                1020

Tyr Lys Cys Pro Val Asn Gln Tyr
1025                1030


<210> 194
<211> 82
<212> PRT
<213> Homo sapiens

<400> 194
Val Ser Ala Thr Asp Ala Asp Ser Gly Glu Asn Gly Lys Val Thr Tyr
  1               5                  10                  15

Ser Ile Leu Ser Gly Asn Asp Gly Gly Leu Phe Ser Ile Asp Pro Glu
             20                  25                  30

Thr Gly Ile Ile Thr Thr Thr Lys Pro Leu Asp Arg Glu Glu Gln Ser
         35                  40                  45

Glu Tyr Thr Leu Thr Val Glu Ala Thr Asp Gly Gly Gly Pro Pro Leu
     50                  55                  60

Ser Ser Thr Ala Thr Val Thr Val Thr Val Leu Asp Val Asn Asp Asn
 65                  70                  75                  80

Ala Pro


<210> 195
<211> 92
<212> PRT
<213> Homo sapiens

<400> 195
Tyr Ser Ala Ser Val Pro Glu Asn Ala Pro Val Gly Thr Glu Val Leu
  1               5                  10                  15

Thr Val Thr Ala Thr Asp Ala Asp Leu Gly Pro Asn Gly Arg Ile Phe
             20                  25                  30

Tyr Ser Ile Leu Gly Gly Gly Pro Gly Gly Trp Phe Arg Ile Asp Pro
         35                  40                  45

Asp Thr Gly Asp Leu Ser Thr Thr Lys Pro Leu Asp Arg Glu Ser Ile
     50                  55                  60

Gly Glu Tyr Glu Leu Thr Val Leu Ala Thr Asp Ser Gly Gly Pro Pro
 65                  70                  75                  80

Leu Ser Gly Thr Thr Thr Val Thr Ile Thr Val Leu
                 85                  90


<210> 196
<211> 3399
<212> PRT
<213> Homo sapiens

<400> 196
Met Ser Leu Arg Ala Gly Gly Lys Thr Arg Arg Trp Pro Gly Pro Glu
  1               5                  10                  15

Gln Ala Ala Arg Gly Gly Arg Ser Gly Gly Glu Gln Ser Pro Arg Arg
            20                  25                  30

Gly Glu Ala Arg Arg Arg Cys Thr His Ala His Gly Ala Met Gly Arg
        35                  40                  45

His Val Ala Thr Ser Cys His Val Ala Trp Leu Leu Val Leu Ile Ser
    50                  55                  60

Gly Cys Trp Gly Gln Val Asn Arg Leu Pro Phe Phe Thr Asn His Phe
65                  70                  75                  80

Phe Asp Thr Tyr Leu Leu Ile Ser Glu Asp Thr Pro Val Gly Ser Ser
                85                  90                  95

Val Thr Gln Leu Leu Ala Gln Asp Met Asp Asn Asp Pro Leu Val Phe
            100                 105                 110

Gly Val Ser Gly Glu Glu Ala Ser Arg Phe Phe Ala Val Glu Pro Asp
        115                 120                 125

Thr Gly Val Val Trp Leu Arg Gln Pro Leu Asp Arg Glu Thr Lys Ser
    130                 135                 140

Glu Phe Thr Val Glu Phe Ser Val Ser Asp His Gln Gly Val Ile Thr
145                 150                 155                 160

Arg Lys Val Asn Ile Gln Val Gly Asp Val Asn Asp Asn Ala Pro Thr
                165                 170                 175

Phe His Asn Gln Pro Tyr Ser Val Arg Ile Pro Glu Asn Thr Pro Val
            180                 185                 190

Gly Thr Pro Ile Phe Ile Val Asn Ala Thr Asp Pro Asp Leu Gly Ala
        195                 200                 205

Gly Gly Ser Val Leu Tyr Ser Phe Gln Pro Pro Ser Gln Phe Phe Ala
    210                 215                 220

Ile Asp Ser Ala Arg Gly Ile Val Thr Val Ile Arg Glu Leu Asp Tyr
225                 230                 235                 240

Glu Thr Thr Gln Ala Tyr Gln Leu Thr Val Asn Ala Thr Asp Gln Asp
                245                 250                 255

Lys Thr Arg Pro Leu Ser Thr Leu Ala Asn Leu Ala Ile Ile Ile Thr
            260                 265                 270

Asp Val Gln Asp Met Asp Pro Ile Phe Ile Asn Leu Pro Tyr Ser Thr
        275                 280                 285

Asn Ile Tyr Glu His Ser Pro Pro Gly Thr Thr Val Arg Ile Ile Thr
    290                 295                 300

Ala Ile Asp Gln Asp Lys Gly Arg Pro Arg Gly Ile Gly Tyr Thr Ile
305                 310                 315                 320

Val Ser Gly Asn Thr Asn Ser Ile Phe Ala Leu Asp Tyr Ile Ser Gly
                325                 330                 335

Val Leu Thr Leu Asn Gly Leu Leu Asp Arg Glu Asn Pro Leu Tyr Ser
            340                 345                 350

His Gly Phe Ile Leu Thr Val Lys Gly Thr Glu Leu Asn Asp Asp Arg
        355                 360                 365

Thr Pro Ser Asp Ala Thr Val Thr Thr Thr Phe Asn Ile Leu Val Ile
    370                 375                 380

Asp Ile Asn Asp Asn Ala Pro Glu Phe Asn Ser Ser Glu Tyr Ser Val
385                 390                 395                 400

Ala Ile Thr Glu Leu Ala Gln Val Gly Phe Ala Leu Pro Leu Phe Ile
                405                 410                 415

Gln Val Val Asp Lys Asp Glu Thr Gln Gly Leu Asn Ser Met Phe Glu
            420                 425                 430

Val Tyr Leu Val Gly Asn Asn Ser His His Phe Ile Ile Ser Pro Thr
        435                 440                 445

Ser Val Gln Gly Lys Ala Asp Ile Arg Ile Arg Val Ala Ile Pro Leu
    450                 455                 460

Asp Tyr Glu Thr Val Asp Arg Tyr Asp Phe Asp Leu Phe Ala Asn Glu
465                 470                 475                 480

Ser Val Pro Asp His Val Gly Tyr Ala Lys Val Lys Ile Thr Leu Ile
                485                 490                 495

Asn Glu Asn Asp Asn Arg Pro Ile Phe Ser Gln Pro Leu Tyr Asn Ile
            500                 505                 510

Ser Leu Tyr Glu Asn Val Thr Val Gly Thr Ser Val Leu Thr Val Leu
        515                 520                 525

Ala Thr Asp Asn Asp Ala Ala Thr Phe Gly Glu Val Ser Tyr Phe Phe
    530                 535                 540

Ser Asp Asp Pro Asp Arg Phe Ser Leu Asp Lys Asp Thr Gly Leu Ile
545                 550                 555                 560

Met Leu Ile Ala Arg Leu Asp Tyr Glu Leu Ile Gln Arg Phe Thr Leu
                565                 570                 575

Thr Ile Ile Ala Arg Asp Gly Gly Gly Glu Glu Thr Thr Gly Arg Val
            580                 585                 590

Arg Ile Asn Val Leu Asp Val Asn Asp Asn Val Pro Thr Phe Gln Lys
        595                 600                 605

Asp Ala Tyr Val Gly Ala Leu Arg Glu Asn Glu Pro Ser Val Thr Gln
    610                 615                 620

Leu Val Arg Leu Arg Ala Thr Asp Glu Asp Ser Pro Pro Asn Asn Gln
625                 630                 635                 640

Ile Thr Tyr Ser Ile Val Ser Ala Ser Ala Phe Gly Ser Tyr Phe Asp
                645                 650                 655

Ile Ser Leu Tyr Glu Gly Tyr Gly Val Ile Ser Val Ser Arg Pro Leu
            660                 665                 670

Asp Tyr Glu Gln Ile Ser Asn Gly Leu Ile Tyr Leu Thr Val Met Ala
        675                 680                 685

Met Asp Ala Gly Asn Pro Pro Leu Asn Ser Thr Val Pro Val Thr Ile
    690                 695                 700

Glu Val Phe Asp Glu Asn Asp Asn Pro Pro Thr Phe Ser Lys Pro Ala
705                 710                 715                 720

Tyr Phe Val Ser Val Val Glu Asn Ile Met Ala Gly Ala Thr Val Leu
                725                 730                 735

Phe Leu Asn Ala Thr Asp Leu Asp Arg Ser Arg Glu Tyr Gly Gln Glu
            740                 745                 750

Ser Ile Ile Tyr Ser Leu Glu Gly Ser Thr Gln Phe Arg Ile Asn Ala
        755                 760                 765

Arg Ser Gly Glu Ile Thr Thr Thr Ser Leu Leu Asp Arg Glu Thr Lys
    770                 775                 780

Ser Glu Tyr Ile Leu Ile Val Arg Ala Val Asp Gly Gly Val Gly His
785                 790                 795                 800

Asn Gln Lys Thr Gly Ile Ala Thr Val Asn Ile Thr Leu Leu Asp Ile
                805                 810                 815

Asn Asp Asn His Pro Thr Trp Lys Asp Ala Pro Tyr Tyr Ile Asn Leu
            820                 825                 830

Val Glu Met Thr Pro Pro Asp Ser Asp Val Thr Thr Val Val Ala Val
        835                 840                 845

Asp Pro Asp Leu Gly Glu Asn Gly Thr Leu Val Tyr Ser Ile Gln Pro
    850                 855                 860

Pro Asn Lys Phe Tyr Ser Leu Asn Ser Thr Thr Gly Lys Ile Arg Thr
865                 870                 875                 880

Thr His Ala Met Leu Asp Arg Glu Asn Pro Asp Pro His Glu Ala Glu
                885                 890                 895

Leu Met Arg Lys Ile Val Val Ser Val Thr Asp Cys Gly Arg Pro Pro
            900                 905                 910

Leu Lys Ala Thr Ser Ser Ala Thr Val Phe Val Asn Leu Leu Asp Leu
        915                 920                 925

Asn Asp Asn Asp Pro Thr Phe Gln Asn Leu Pro Phe Val Ala Glu Val
    930                 935                 940

Leu Glu Gly Ile Pro Ala Gly Val Ser Ile Tyr Gln Val Val Ala Ile
945                 950                 955                 960

Asp Leu Asp Glu Gly Leu Asn Gly Leu Val Ser Tyr Arg Met Pro Val
                965                 970                 975

Gly Met Pro Arg Met Asp Phe Leu Ile Asn Ser Ser Ser Gly Val Val
            980                 985                 990

Val Thr Thr Thr Glu Leu Asp Arg Glu Arg Ile Ala Glu Tyr Gln Leu
        995                 1000                1005

Arg Val Val Ala Ser Asp Ala Gly Thr Pro Thr Lys Ser Ser Thr Ser
    1010                1015                1020

Thr Leu Thr Ile His Val Leu Asp Val Asn Asp Glu Thr Pro Thr Phe
1025                1030                1035                1040

Phe Pro Ala Val Tyr Asn Val Ser Val Ser Glu Asp Val Pro Arg Glu
                1045                1050                1055

Phe Arg Val Val Trp Leu Asn Cys Thr Asp Asn Asp Val Gly Leu Asn
            1060                1065                1070

Ala Glu Leu Ser Tyr Phe Ile Thr Gly Gly Asn Val Asp Gly Lys Phe
        1075                1080                1085

Ser Val Gly Tyr Arg Asp Ala Val Val Arg Thr Val Val Gly Leu Asp
    1090                1095                1100

Arg Glu Thr Thr Ala Ala Tyr Met Leu Ile Leu Glu Ala Ile Asp Asn
1105                1110                1115                1120

Gly Pro Val Gly Lys Arg His Thr Gly Thr Ala Thr Val Phe Val Thr
                1125                1130                1135

Val Leu Asp Val Asn Asp Asn Arg Pro Ile Phe Leu Gln Ser Ser Tyr
            1140                1145                1150

Glu Ala Ser Val Pro Glu Asp Ile Pro Glu Gly His Ser Ile Leu Gln
        1155                1160                1165

Leu Lys Ala Thr Asp Ala Asp Glu Gly Glu Phe Gly Arg Val Trp Tyr
    1170                1175                1180

Arg Ile Leu His Gly Asn His Gly Asn Asn Phe Arg Ile His Val Ser
1185                1190                1195                1200

Asn Gly Leu Leu Met Arg Gly Pro Arg Pro Leu Asp Arg Glu Arg Asn
                1205                1210                1215

Ser Ser His Val Leu Ile Val Glu Ala Tyr Asn His Asp Leu Gly Pro
            1220                1225                1230

Met Arg Ser Ser Val Arg Val Ile Val Tyr Val Glu Asp Ile Asn Asp
        1235                1240                1245

Glu Ala Pro Val Phe Thr Gln Gln Gln Tyr Ser Arg Leu Gly Leu Arg
    1250                1255                1260

Glu Thr Ala Gly Ile Gly Thr Ser Val Ile Val Val Gln Ala Thr Asp
1265                1270                1275                1280

Arg Asp Ser Gly Asp Gly Gly Leu Val Asn Tyr Arg Ile Leu Ser Gly
                1285                1290                1295

Ala Glu Gly Lys Phe Glu Ile Asp Glu Ser Thr Gly Leu Ile Ile Thr
            1300                1305                1310

Val Asn Tyr Leu Asp Tyr Glu Thr Lys Thr Ser Tyr Met Met Asn Val
        1315                1320                1325

Ser Ala Thr Asp Gln Ala Pro Pro Phe Asn Gln Gly Phe Cys Ser Val
    1330                1335                1340

Tyr Ile Thr Leu Leu Asn Glu Leu Asp Glu Ala Val Gln Phe Ser Asn
1345                1350                1355                1360

Ala Ser Tyr Glu Ala Ala Ile Leu Glu Asn Leu Ala Leu Gly Thr Glu
                1365                1370                1375

Ile Val Arg Val Gln Ala Tyr Ser Ile Asp Asn Leu Asn Gln Ile Thr
            1380                1385                1390

Tyr Arg Phe Asn Ala Tyr Thr Ser Thr Gln Ala Lys Ala Leu Phe Lys
        1395                1400                1405

Ile Asp Ala Ile Thr Gly Val Ile Thr Val Gln Gly Leu Val Asp Arg
    1410                1415                1420

Glu Lys Gly Asp Phe Tyr Thr Leu Thr Val Val Ala Asp Asp Gly Gly
1425                1430                1435                1440

Pro Lys Val Asp Ser Thr Val Gln Val Tyr Ile Thr Val Leu Asp Glu
                1445                1450                1455

Asn Asp Asn Ser Pro Arg Phe Asp Phe Thr Ser Asp Ser Ala Val Ser
            1460                1465                1470

Ile Pro Glu Asp Cys Pro Val Gly Gln Arg Val Ala Thr Val Lys Ala
        1475                1480                1485

Trp Asp Pro Asp Ala Gly Ser Asn Gly Gln Val Val Phe Ser Leu Ala
    1490                1495                1500

Ser Gly Asn Ile Ala Gly Ala Phe Glu Ile Val Thr Thr Asn Asp Ser
1505                1510                1515                1520

Ile Gly Glu Val Phe Val Ala Arg Pro Leu Asp Arg Glu Glu Leu Asp
                1525                1530                1535

His Tyr Ile Leu Gln Val Val Ala Ser Asp Arg Gly Thr Pro Pro Arg
            1540                1545                1550

Lys Lys Asp His Ile Leu Gln Val Thr Ile Leu Asp Ile Asn Asp Asn
        1555                1560                1565

Pro Pro Val Ile Glu Ser Pro Phe Gly Tyr Asn Val Ser Val Asn Glu
    1570                1575                1580

Asn Val Gly Gly Gly Thr Ala Val Val Gln Val Arg Ala Thr Asp Arg
1585                1590                1595                1600

Asp Ile Gly Ile Asn Ser Val Leu Ser Tyr Tyr Ile Thr Glu Gly Asn
                1605                1610                1615

Lys Asp Met Ala Phe Arg Met Asp Arg Ile Ser Gly Glu Ile Ala Thr
            1620                1625                1630

Arg Pro Ala Pro Pro Asp Arg Glu Arg Gln Ser Phe Tyr His Leu Val
        1635                1640                1645

Ala Thr Val Glu Asp Glu Gly Thr Pro Thr Leu Ser Ala Thr Thr His
    1650                1655                1660

Val Tyr Val Thr Ile Val Asp Glu Asn Asp Asn Ala Pro Met Phe Gln
1665                1670                1675                1680

Gln Pro His Tyr Glu Val Leu Leu Asp Glu Gly Pro Asp Thr Leu Asn
                1685                1690                1695

Thr Ser Leu Ile Thr Ile Gln Ala Leu Asp Leu Asp Glu Gly Pro Asn
            1700                1705                1710

Gly Thr Val Thr Tyr Ala Ile Val Ala Gly Asn Ile Val Asn Thr Phe
        1715                1720                1725

Arg Ile Asp Arg His Met Gly Val Ile Thr Ala Ala Lys Glu Leu Asp
    1730                1735                1740

Tyr Glu Ile Ser His Gly Arg Tyr Thr Leu Ile Val Thr Ala Thr Asp
1745                1750                1755                1760

Gln Cys Pro Ile Leu Ser His Arg Leu Thr Ser Thr Thr Thr Val Leu
                1765                1770                1775

Val Asn Val Asn Asp Ile Asn Asp Asn Val Pro Thr Phe Pro Arg Asp
            1780                1785                1790

Tyr Glu Gly Pro Phe Glu Val Thr Glu Gly Gln Pro Gly Pro Arg Val
        1795                1800                1805

Trp Thr Phe Leu Ala His Asp Arg Asp Ser Gly Pro Asn Gly Gln Val
    1810                1815                1820

Glu Tyr Ser Ile Met Asp Gly Asp Pro Leu Gly Glu Phe Val Ile Ser
1825                1830                1835                1840

Pro Val Glu Gly Val Leu Arg Val Arg Lys Asp Val Glu Leu Asp Arg
                1845                1850                1855

Glu Thr Ile Ala Phe Tyr Asn Leu Thr Ile Cys Ala Arg Asp Arg Gly
            1860                1865                1870

Met Pro Pro Leu Ser Ser Thr Met Leu Val Gly Ile Arg Val Leu Asp
        1875                1880                1885

Ile Asn Asp Asn Asp Pro Val Leu Leu Asn Leu Pro Met Asn Ile Thr
    1890                1895                1900

Ile Ser Glu Asn Ser Pro Val Ser Ser Phe Val Ala His Val Leu Ala
1905                1910                1915                1920

Ser Asp Ala Asp Ser Gly Cys Asn Ala Arg Leu Thr Phe Asn Ile Thr
                1925                1930                1935

Ala Gly Asn Arg Glu Arg Ala Phe Phe Ile Asn Ala Thr Thr Gly Ile
            1940                1945                1950

Val Thr Val Asn Arg Pro Leu Asp Arg Glu Arg Ile Pro Glu Tyr Lys
        1955                1960                1965

Leu Thr Ile Ser Val Lys Asp Asn Pro Glu Asn Pro Arg Ile Ala Arg
    1970                1975                1980

Arg Asp Tyr Asp Leu Leu Leu Ile Phe Leu Ser Asp Glu Asn Asp Asn
1985                1990                1995                2000

His Pro Leu Phe Thr Lys Ser Thr Tyr Gln Ala Glu Val Met Glu Asn
                2005                2010                2015

Ser Pro Ala Gly Thr Pro Leu Thr Val Leu Asn Gly Pro Ile Leu Ala
            2020                2025                2030

Leu Asp Ala Asp Gln Asp Ile Tyr Ala Val Val Thr Tyr Gln Leu Leu
        2035                2040                2045

Gly Ala Gln Ser Gly Leu Phe Asp Ile Asn Ser Ser Thr Gly Val Val
    2050                2055                2060

Thr Val Arg Ser Gly Val Ile Ile Asp Arg Glu Ala Phe Ser Pro Pro
2065                2070                2075                2080

Ile Leu Glu Leu Leu Leu Leu Ala Glu Asp Ile Gly Leu Leu Asn Ser
                2085                2090                2095

Thr Ala His Leu Leu Ile Thr Ile Leu Asp Asp Asn Asp Asn Arg Pro
            2100                2105                2110

Thr Phe Ser Pro Ala Thr Leu Thr Val His Leu Leu Glu Asn Cys Pro
        2115                2120                2125

Pro Gly Phe Ser Val Leu Gln Val Thr Ala Thr Asp Glu Asp Ser Gly
    2130                2135                2140

Leu Asn Gly Glu Leu Val Tyr Arg Ile Glu Ala Gly Ala Gln Asp Arg
2145                2150                2155                2160

Phe Leu Ile His Leu Val Thr Gly Val Ile Arg Val Gly Asn Ala Thr
                2165                2170                2175

Ile Asp Arg Glu Glu Gln Glu Ser Tyr Arg Leu Thr Val Val Ala Thr
            2180                2185                2190

Asp Arg Gly Thr Val Pro Leu Ser Gly Thr Ala Ile Val Thr Ile Leu
        2195                2200                2205

Ile Asp Asp Ile Asn Asp Ser Arg Pro Glu Phe Leu Asn Pro Ile Gln
    2210                2215                2220

Thr Val Ser Val Leu Glu Ser Ala Glu Pro Gly Thr Val Ile Ala Asn
2225                2230                2235                2240

Ile Thr Ala Ile Asp His Asp Leu Asn Pro Lys Leu Glu Tyr His Ile
                2245                2250                2255

Val Gly Ile Val Ala Lys Asp Asp Thr Asp Arg Leu Val Pro Asn Gln
            2260                2265                2270

Glu Asp Ala Phe Ala Val Asn Ile Asn Thr Gly Ser Val Met Val Lys
        2275                2280                2285

Ser Pro Met Asn Arg Glu Leu Val Ala Thr Tyr Glu Val Thr Leu Ser
    2290                2295                2300

Val Ile Asp Asn Ala Ser Asp Leu Pro Glu Arg Ser Val Ser Val Pro
2305                2310                2315                2320

Asn Ala Lys Leu Thr Val Asn Val Leu Asp Val Asn Asp Asn Thr Pro
                2325                2330                2335

Gln Phe Lys Pro Phe Gly Ile Thr Tyr Tyr Met Glu Arg Ile Leu Glu
            2340                2345                2350

Gly Ala Thr Pro Gly Thr Thr Leu Ile Ala Val Ala Ala Val Asp Pro
        2355                2360                2365

Asp Lys Gly Leu Asn Gly Leu Val Thr Tyr Thr Leu Leu Asp Leu Val
    2370                2375                2380

Pro Pro Gly Tyr Val Gln Leu Glu Asp Ser Ser Ala Gly Lys Val Ile
2385                2390                2395                2400

Ala Asn Arg Thr Val Asp Tyr Glu Glu Val His Trp Leu Asn Phe Thr
                2405                2410                2415

Val Arg Ala Ser Asp Asn Gly Ser Pro Pro Arg Ala Ala Glu Ile Pro
            2420                2425                2430

Val Tyr Leu Glu Ile Val Asp Ile Asn Asp Asn Asn Pro Ile Phe Asp
        2435                2440                2445

Gln Pro Ser Tyr Gln Glu Ala Val Phe Glu Asp Val Pro Val Gly Thr
    2450                2455                2460

Ile Ile Leu Thr Val Thr Ala Thr Asp Ala Asp Ser Gly Asn Phe Ala
2465                2470                2475                2480

Leu Ile Glu Tyr Ser Leu Gly Asp Gly Glu Ser Lys Phe Ala Ile Asn
                2485                2490                2495

Pro Thr Thr Gly Asp Ile Tyr Val Leu Ser Ser Leu Asp Arg Glu Lys
            2500                2505                2510

Lys Asp His Tyr Ile Leu Thr Ala Leu Ala Lys Asp Asn Pro Gly Asp
        2515                2520                2525

Val Ala Ser Asn Arg Arg Glu Asn Ser Val Gln Val Val Ile Gln Val
    2530                2535                2540

Leu Asp Val Asn Asp Cys Arg Pro Gln Phe Ser Lys Pro Gln Phe Ser
2545                2550                2555                2560

Thr Ser Val Tyr Glu Asn Glu Pro Ala Gly Thr Ser Val Ile Thr Met
                2565                2570                2575

Met Ala Thr Asp Gln Asp Glu Gly Pro Asn Gly Glu Leu Thr Tyr Ser
            2580                2585                2590

Leu Glu Gly Pro Gly Val Glu Ala Phe His Val Asp Met Asp Ser Gly
        2595                2600                2605

Leu Val Thr Thr Gln Arg Pro Leu Gln Ser Tyr Glu Lys Phe Ser Leu
    2610                2615                2620

Thr Val Val Ala Thr Asp Gly Gly Glu Pro Pro Leu Trp Gly Thr Thr
2625                2630                2635                2640

Met Leu Leu Val Glu Val Ile Asp Val Asn Asp Asn Arg Pro Val Phe
                2645                2650                2655

Val Arg Pro Pro Asn Gly Thr Ile Leu His Ile Arg Glu Glu Ile Pro
            2660                2665                2670

Leu Arg Ser Asn Val Tyr Glu Val Tyr Ala Thr Asp Lys Asp Glu Gly
        2675                2680                2685

Leu Asn Gly Ala Val Arg Tyr Ser Phe Leu Lys Thr Ala Gly Asn Arg
    2690                2695                2700

Asp Trp Glu Phe Phe Ile Ile Asp Pro Ile Ser Gly Leu Ile Gln Thr
2705                2710                2715                2720

Ala Gln Arg Leu Asp Arg Glu Ser Gln Ala Val Tyr Ser Leu Ile Leu
                2725                2730                2735

Val Ala Ser Asp Leu Gly Gln Pro Val Pro Tyr Glu Thr Met Gln Pro
            2740                2745                2750

Leu Gln Val Ala Leu Glu Asp Ile Asp Asp Asn Glu Pro Leu Phe Val
        2755                2760                2765

Arg Pro Pro Lys Gly Ser Pro Gln Tyr Gln Leu Leu Thr Val Pro Glu
    2770                2775                2780

His Ser Pro Arg Gly Thr Leu Val Gly Asn Val Thr Gly Ala Val Asp
2785                2790                2795                2800

Ala Asp Glu Gly Pro Asn Ala Ile Val Tyr Tyr Phe Ile Ala Ala Gly
                2805                2810                2815

Asn Glu Glu Lys Asn Phe His Leu Gln Pro Asp Gly Cys Leu Leu Val
            2820                2825                2830

Leu Arg Asp Leu Asp Arg Glu Arg Glu Ala Ile Phe Ser Phe Ile Val
        2835                2840                2845

Lys Ala Ser Ser Asn Arg Ser Trp Thr Pro Pro Arg Gly Pro Ser Pro
    2850                2855                2860

Thr Leu Asp Leu Val Ala Asp Leu Thr Leu Gln Glu Val Arg Val Val
2865                2870                2875                2880

Leu Glu Asp Ile Asn Asp Gln Pro Pro Arg Phe Thr Lys Ala Glu Tyr
                2885                2890                2895

Thr Ala Gly Val Ala Thr Asp Ala Lys Val Gly Ser Glu Leu Ile Gln
            2900                2905                2910

Val Leu Ala Leu Asp Ala Asp Ile Gly Asn Asn Ser Leu Val Phe Tyr
        2915                2920                2925

Ser Ile Leu Ala Ile His Tyr Phe Arg Ala Leu Ala Asn Asp Ser Glu
    2930                2935                2940

Asp Val Gly Gln Val Phe Thr Met Gly Ser Met Asp Gly Ile Leu Arg
2945                2950                2955                2960

Thr Phe Asp Leu Phe Met Ala Tyr Ser Pro Gly Tyr Phe Val Val Asp
                2965                2970                2975

Ile Val Ala Arg Asp Leu Ala Gly His Asn Asp Thr Ala Ile Ile Gly
            2980                2985                2990

Ile Tyr Ile Leu Arg Asp Asp Gln Arg Val Lys Ile Val Ile Asn Glu
        2995                3000                3005

Ile Pro Asp Arg Val Arg Gly Phe Glu Glu Glu Phe Ile His Leu Leu
    3010                3015                3020

Ser Asn Ile Thr Gly Ala Ile Val Asn Thr Asp Asn Val Gln Phe His
3025                3030                3035                3040

Val Asp Lys Lys Gly Arg Val Asn Phe Ala Gln Thr Glu Leu Leu Ile
                3045                3050                3055

His Val Val Asn Arg Asp Thr Asn Arg Ile Leu Asp Val Asp Arg Val
            3060                3065                3070

Ile Gln Met Ile Asp Glu Asn Lys Glu Gln Leu Arg Asn Leu Phe Arg
        3075                3080                3085

Asn Tyr Asn Val Leu Asp Val Gln Pro Ala Ile Ser Val Arg Leu Pro
    3090                3095                3100

Asp Asp Met Ser Ala Leu Gln Met Ala Ile Ile Val Leu Ala Ile Leu
3105                3110                3115                3120

Leu Phe Leu Ala Ala Met Leu Phe Val Leu Met Asn Trp Tyr Tyr Arg
                3125                3130                3135

Thr Val His Lys Arg Lys Leu Lys Ala Ile Val Ala Gly Ser Ala Gly
            3140                3145                3150

Asn Arg Gly Phe Ile Asp Ile Met Asp Met Pro Asn Thr Asn Lys Tyr
        3155                3160                3165

Ser Phe Asp Gly Ala Asn Pro Val Trp Leu Asp Pro Phe Cys Arg Asn
    3170                3175                3180

Leu Glu Leu Ala Ala Gln Ala Glu His Glu Asp Asp Leu Pro Glu Asn
3185                3190                3195                3200

Leu Ser Glu Ile Ala Asp Leu Trp Asn Ser Pro Thr Arg Thr His Gly
                3205                3210                3215

Thr Phe Gly Arg Glu Pro Ala Ala Val Lys Pro Asp Asp Asp Arg Tyr
            3220                3225                3230

Leu Arg Ala Ala Ile Gln Glu Tyr Asp Asn Ile Ala Lys Leu Gly Gln
        3235                3240                3245

Ile Ile Arg Glu Gly Pro Ile Lys Gly Ser Leu Leu Lys Val Val Leu
    3250                3255                3260

Glu Asp Tyr Leu Arg Leu Lys Lys Leu Phe Ala Gln Arg Met Val Gln
3265                3270                3275                3280

Lys Ala Ser Ser Cys His Ser Ser Ile Ser Glu Leu Ile Gln Thr Glu
                3285                3290                3295

Leu Asp Glu Glu Pro Gly Asp His Ser Pro Gly Gln Gly Ser Leu Arg
            3300                3305                3310

Phe Arg His Lys Pro Pro Val Glu Leu Lys Gly Pro Asp Gly Ile His
        3315                3320                3325

Val Val His Gly Ser Thr Gly Thr Leu Leu Ala Thr Asp Leu Asn Ser
    3330                3335                3340

Leu Pro Glu Glu Asp Gln Lys Gly Leu Gly Arg Ser Leu Glu Thr Leu
3345                3350                3355                3360

Thr Ala Ala Glu Ala Thr Ala Phe Glu Arg Asn Ala Arg Thr Glu Ser
                3365                3370                3375

Ala Lys Ser Thr Pro Leu His Lys Leu Arg Asp Val Ile Met Glu Thr
            3380                3385                3390

Pro Leu Glu Ile Thr Glu Leu
        3395

<210> 197
<211> 3354
<212> PRT
<213> Homo sapiens

<400> 197
Met Gly Arg His Val Ala Thr Ser Cys His Val Ala Trp Leu Leu Val
  1               5                  10                  15

Leu Ile Ser Gly Cys Trp Gly Gln Val Asn Arg Leu Pro Phe Phe Thr
             20                  25                  30

Asn His Phe Phe Asp Thr Tyr Leu Leu Ile Ser Glu Asp Thr Pro Val
         35                  40                  45

Gly Ser Ser Val Thr Gln Leu Leu Ala Gln Asp Met Asp Asn Asp Pro
     50                  55                  60

Leu Val Phe Gly Val Ser Gly Glu Glu Ala Ser Arg Phe Phe Ala Val
 65                  70                  75                  80

Glu Pro Asp Thr Gly Val Val Trp Leu Arg Gln Pro Leu Asp Arg Glu
                 85                  90                  95

Thr Lys Ser Glu Phe Thr Val Glu Phe Ser Val Ser Asp His Gln Gly
            100                 105                 110

Val Ile Thr Arg Lys Val Asn Ile Gln Val Gly Asp Val Asn Asp Asn
        115                 120                 125

Ala Pro Thr Phe His Asn Gln Pro Tyr Ser Val Arg Ile Pro Glu Asn
    130                 135                 140

Thr Pro Val Gly Thr Pro Ile Phe Ile Val Asn Ala Thr Asp Pro Asp
145                 150                 155                 160

Leu Gly Ala Gly Gly Ser Val Leu Tyr Ser Phe Gln Pro Pro Ser Gln
                165                 170                 175

Phe Phe Ala Ile Asp Ser Ala Arg Gly Ile Val Thr Val Ile Arg Glu
            180                 185                 190

Leu Asp Tyr Glu Thr Thr Gln Ala Tyr Gln Leu Thr Val Asn Ala Thr
        195                 200                 205

Asp Gln Asp Lys Thr Arg Pro Leu Ser Thr Leu Ala Asn Leu Ala Ile
    210                 215                 220

Ile Ile Thr Asp Val Gln Asp Met Asp Pro Ile Phe Ile Asn Leu Pro
225                 230                 235                 240

Tyr Ser Thr Asn Ile Tyr Glu His Ser Pro Pro Gly Thr Thr Val Arg

                245                 250                 255

Ile Ile Thr Ala Ile Asp Gln Asp Lys Gly Arg Pro Arg Gly Ile Gly
            260                 265                 270

Tyr Thr Ile Val Ser Gly Asn Thr Asn Ser Ile Phe Ala Leu Asp Tyr
        275                 280                 285

Ile Ser Gly Val Leu Thr Leu Asn Gly Leu Leu Asp Arg Glu Asn Pro
    290                 295                 300

Leu Tyr Ser His Gly Phe Ile Leu Thr Val Lys Gly Thr Glu Leu Asn
305                 310                 315                 320

Asp Asp Arg Thr Pro Ser Asp Ala Thr Val Thr Thr Thr Phe Asn Ile
                325                 330                 335

Leu Val Ile Asp Ile Asn Asp Asn Ala Pro Glu Phe Asn Ser Ser Glu
            340                 345                 350

Tyr Ser Val Ala Ile Thr Glu Leu Ala Gln Val Gly Phe Ala Leu Pro
        355                 360                 365

Leu Phe Ile Gln Val Val Asp Lys Asp Glu Asn Leu Gly Leu Asn Ser
    370                 375                 380

Met Phe Glu Val Tyr Leu Val Gly Asn Asn Ser His His Phe Ile Ile
385                 390                 395                 400

Ser Pro Thr Ser Val Gln Gly Lys Ala Asp Ile Arg Ile Arg Val Ala
                405                 410                 415

Ile Pro Leu Asp Tyr Glu Thr Val Asp Arg Tyr Asp Phe Asp Leu Phe
            420                 425                 430

Ala Asn Glu Ser Val Pro Asp His Val Gly Tyr Ala Lys Val Lys Ile
        435                 440                 445

Thr Leu Ile Asn Glu Asn Asp Asn Arg Pro Ile Phe Ser Gln Pro Leu
    450                 455                 460

Tyr Asn Ile Ser Leu Tyr Glu Asn Val Thr Val Gly Thr Ser Val Leu
465                 470                 475                 480

Thr Val Leu Ala Thr Asp Asn Asp Ala Gly Thr Phe Gly Glu Val Ser
                485                 490                 495

Tyr Phe Phe Ser Asp Asp Pro Asp Arg Phe Ser Leu Asp Lys Asp Thr
            500                 505                 510

Gly Leu Ile Met Leu Ile Ala Arg Leu Asp Tyr Glu Leu Ile Gln Arg
        515                 520                 525

Phe Thr Leu Thr Ile Ile Ala Arg Asp Gly Gly Gly Glu Glu Thr Thr
    530                 535                 540

Gly Arg Val Arg Ile Asn Val Leu Asp Val Asn Asp Asn Val Pro Thr
545                 550                 555                 560

Phe Gln Lys Asp Ala Tyr Val Gly Ala Leu Arg Glu Asn Glu Pro Ser
                565                 570                 575

Val Thr Gln Leu Val Arg Leu Arg Ala Thr Asp Glu Asp Ser Pro Pro
            580                 585                 590

Asn Asn Gln Ile Thr Tyr Ser Ile Val Ser Ala Ser Ala Phe Gly Ser
        595                 600                 605

Tyr Phe Asp Ile Ser Leu Tyr Glu Gly Tyr Gly Val Ile Ser Val Ser

610                 615                 620

Arg Pro Leu Asp Tyr Glu Gln Ile Ser Asn Gly Leu Ile Tyr Leu Thr
625                 630                 635                 640

Val Met Ala Met Asp Ala Gly Asn Pro Pro Leu Asn Ser Thr Val Pro
                645                 650                 655

Val Thr Ile Glu Val Phe Asp Glu Asn Asp Asn Pro Pro Thr Phe Ser
            660                 665                 670

Lys Pro Ala Tyr Phe Val Ser Val Val Glu Asn Ile Met Ala Gly Ala
        675                 680                 685

Thr Val Leu Phe Leu Asn Ala Thr Asp Leu Asp Arg Ser Arg Glu Tyr
    690                 695                 700

Gly Gln Glu Ser Ile Ile Tyr Ser Leu Glu Gly Ser Thr Gln Phe Arg
705                 710                 715                 720

Ile Asn Ala Arg Ser Gly Glu Ile Thr Thr Thr Ser Leu Leu Asp Arg
                725                 730                 735

Glu Thr Lys Ser Glu Tyr Ile Leu Ile Val Arg Ala Val Asp Gly Gly
            740                 745                 750

Val Gly His Asn Gln Lys Thr Gly Ile Ala Thr Val Asn Ile Thr Leu
        755                 760                 765

Leu Asp Ile Asn Asp Asn His Pro Thr Trp Lys Asp Ala Pro Tyr Tyr
    770                 775                 780

Ile Asn Leu Val Glu Met Thr Pro Pro Asp Ser Asp Val Thr Thr Val
785                 790                 795                 800

Val Ala Val Asp Pro Asp Leu Gly Glu Asn Gly Thr Leu Val Tyr Ser
                805                 810                 815

Ile Gln Pro Pro Asn Lys Phe Tyr Ser Leu Asn Ser Thr Thr Gly Lys
            820                 825                 830

Ile Arg Thr Thr His Ala Met Leu Asp Arg Glu Asn Pro Asp Pro His
        835                 840                 845

Glu Ala Glu Leu Met Arg Lys Ile Val Val Ser Val Thr Asp Cys Gly
    850                 855                 860

Arg Pro Pro Leu Lys Ala Thr Ser Ser Ala Thr Val Phe Val Asn Leu
865                 870                 875                 880

Leu Asp Leu Asn Asp Asn Asp Pro Thr Phe Gln Asn Leu Pro Phe Val
                885                 890                 895

Ala Glu Val Leu Glu Gly Ile Pro Ala Gly Val Ser Ile Tyr Gln Val
            900                 905                 910

Val Ala Ile Asp Leu Asp Glu Gly Leu Asn Gly Leu Val Ser Tyr Arg
        915                 920                 925

Met Pro Val Gly Met Pro Arg Met Asp Phe Leu Ile Asn Ser Ser Ser
    930                 935                 940

Gly Val Val Val Thr Thr Thr Glu Leu Asp Arg Glu Arg Ile Ala Glu
945                 950                 955                 960

Tyr Gln Leu Arg Val Val Ala Ser Asp Ala Gly Thr Pro Thr Lys Ser
                965                 970                 975

Ser Thr Ser Thr Leu Thr Ile His Val Leu Asp Val Asn Asp Glu Thr

980                 985                 990

Pro Thr Phe Phe Pro Ala Val Tyr Asn Val Ser Val Ser Glu Asp Val
        995                 1000                1005

Pro Arg Glu Phe Arg Val Val Trp Leu Asn Cys Thr Asp Asn Asp Val
    1010                1015                1020

Gly Leu Asn Ala Glu Leu Ser Tyr Phe Ile Thr Gly Gly Asn Val Asp
1025                1030                1035                1040

Gly Lys Phe Ser Val Gly Tyr Arg Asp Ala Val Val Arg Thr Val Val
                1045                1050                1055

Gly Leu Asp Arg Glu Thr Thr Ala Ala Tyr Met Leu Ile Leu Glu Ala
            1060                1065                1070

Ile Asp Asn Gly Pro Val Gly Lys Arg His Thr Gly Thr Ala Thr Val
        1075                1080                1085

Phe Val Thr Val Leu Asp Val Asn Asp Asn Arg Pro Ile Phe Leu Gln
    1090                1095                1100

Ser Ser Tyr Glu Ala Ser Val Pro Glu Asp Ile Pro Glu Gly His Ser
1105                1110                1115                1120

Ile Leu Gln Leu Lys Ala Thr Asp Ala Asp Glu Gly Glu Phe Gly Arg
                1125                1130                1135

Val Trp Tyr Arg Ile Leu His Gly Asn His Gly Asn Asn Phe Arg Ile
            1140                1145                1150

His Val Ser Asn Gly Leu Leu Met Arg Gly Pro Arg Pro Leu Asp Arg
        1155                1160                1165

Glu Arg Asn Ser Ser His Val Leu Ile Val Glu Ala Tyr Asn His Asp
    1170                1175                1180

Leu Gly Pro Met Arg Ser Ser Val Arg Val Ile Val Tyr Val Glu Asp
1185                1190                1195                1200

Ile Asn Asp Glu Ala Pro Val Phe Thr Gln Gln Gln Tyr Ser Arg Leu
                1205                1210                1215

Gly Leu Arg Glu Thr Ala Gly Ile Gly Thr Ser Val Ile Val Val Gln
            1220                1225                1230

Ala Thr Asp Arg Asp Ser Gly Asp Gly Gly Leu Val Asn Tyr Arg Ile
        1235                1240                1245

Leu Ser Gly Ala Glu Gly Lys Phe Glu Ile Asp Glu Ser Thr Gly Leu
    1250                1255                1260

Ile Ile Thr Val Asn Tyr Leu Asp Tyr Glu Thr Lys Thr Ser Tyr Met
1265                1270                1275                1280

Met Asn Val Ser Ala Thr Asp Gln Ala Pro Pro Phe Asn Gln Gly Phe
                1285                1290                1295

Cys Ser Val Tyr Ile Thr Leu Leu Asn Glu Leu Asp Glu Ala Val Gln
            1300                1305                1310

Phe Ser Asn Ala Ser Tyr Glu Ala Ala Ile Leu Glu Asn Leu Ala Leu
        1315                1320                1325

Gly Thr Glu Ile Val Arg Val Gln Ala Tyr Ser Ile Asp Asn Leu Asn
    1330                1335                1340

Gln Ile Thr Tyr Arg Phe Asn Ala Tyr Thr Ser Thr Gln Ala Lys Ala

1345                1350                1355                1360

Leu Phe Lys Ile Asp Ala Ile Thr Gly Val Ile Thr Val Gln Gly Leu
                1365                1370                1375

Val Asp Arg Glu Lys Gly Asp Phe Tyr Thr Leu Thr Val Val Ala Asp
            1380                1385                1390

Asp Gly Gly Pro Lys Val Asp Ser Thr Val Lys Val Tyr Ile Thr Val
        1395                1400                1405

Leu Asp Glu Asn Asp Asn Ser Pro Arg Phe Asp Phe Thr Ser Asp Ser
    1410                1415                1420

Ala Val Ser Ile Pro Glu Asp Cys Pro Val Gly Gln Arg Val Ala Thr
1425                1430                1435                1440

Val Lys Ala Trp Asp Pro Asp Ala Gly Ser Asn Gly Gln Val Val Phe
                1445                1450                1455

Ser Leu Ala Ser Gly Asn Ile Ala Gly Ala Phe Glu Ile Val Thr Thr
            1460                1465                1470

Asn Asp Ser Ile Gly Glu Val Phe Val Ala Arg Pro Leu Asp Arg Glu
        1475                1480                1485

Glu Leu Asp His Tyr Ile Leu Gln Val Val Ala Ser Asp Arg Gly Thr
    1490                1495                1500

Pro Pro Arg Lys Lys Asp His Ile Leu Gln Val Thr Ile Leu Asp Ile
1505                1510                1515                1520

Asn Asp Asn Pro Pro Val Ile Glu Ser Pro Phe Gly Tyr Asn Val Ser
                1525                1530                1535

Val Asn Glu Asn Val Gly Gly Gly Thr Ala Val Val Gln Val Arg Ala
            1540                1545                1550

Thr Asp Arg Asp Ile Gly Ile Asn Ser Val Leu Ser Tyr Tyr Ile Thr
        1555                1560                1565

Glu Gly Asn Lys Asp Met Ala Phe Arg Met Asp Arg Ile Ser Gly Glu
    1570                1575                1580

Ile Ala Thr Arg Pro Ala Pro Pro Asp Arg Glu Arg Gln Ser Phe Tyr
1585                1590                1595                1600

His Leu Val Ala Thr Val Glu Asp Glu Gly Thr Pro Thr Leu Ser Ala
                1605                1610                1615

Thr Thr His Val Tyr Val Thr Ile Val Asp Glu Asn Asp Asn Ala Pro
            1620                1625                1630

Met Phe Gln Gln Pro His Tyr Glu Val Leu Leu Asp Glu Gly Pro Asp
        1635                1640                1645

Thr Leu Asn Thr Ser Leu Ile Thr Ile Gln Ala Leu Asp Leu Asp Glu
    1650                1655                1660

Gly Pro Asn Gly Thr Val Thr Tyr Ala Ile Val Ala Gly Asn Ile Val
1665                1670                1675                1680

Asn Thr Phe Arg Ile Asp Arg His Met Gly Val Ile Thr Ala Ala Lys
                1685                1690                1695

Glu Leu Asp Tyr Glu Ile Ser His Gly Arg Tyr Thr Leu Ile Val Thr
            1700                1705                1710

Ala Thr Asp Gln Cys Pro Ile Leu Ser His Arg Leu Thr Ser Thr Thr

1715                1720                1725

Thr Val Leu Val Asn Val Asn Asp Ile Asn Asp Asn Val Pro Thr Phe
    1730                1735                1740

Pro Arg Asp Tyr Glu Gly Pro Phe Glu Val Thr Glu Gly Gln Pro Gly
1745                1750                1755                1760

Pro Arg Val Trp Thr Phe Leu Ala His Asp Arg Asp Ser Gly Pro Asn
                1765                1770                1775

Gly Gln Val Glu Tyr Ser Ile Met Asp Gly Asp Pro Leu Gly Glu Phe
            1780                1785                1790

Val Ile Ser Pro Val Glu Gly Val Leu Arg Val Arg Lys Asp Val Glu
        1795                1800                1805

Leu Asp Arg Glu Thr Ile Ala Phe Tyr Asn Leu Thr Ile Cys Ala Arg
    1810                1815                1820

Asp Arg Gly Met Pro Pro Leu Ser Ser Thr Met Leu Val Gly Ile Arg
1825                1830                1835                1840

Val Leu Asp Ile Asn Asp Asn Asp Pro Val Leu Leu Asn Leu Pro Met
                1845                1850                1855

Asn Ile Thr Ile Ser Glu Asn Ser Pro Val Ser Ser Phe Val Ala His
            1860                1865                1870

Val Leu Ala Ser Asp Ala Asp Ser Gly Cys Asn Ala Arg Leu Thr Phe
        1875                1880                1885

Asn Ile Thr Ala Gly Asn Arg Glu Arg Ala Phe Phe Ile Asn Ala Thr
    1890                1895                1900

Thr Gly Ile Val Thr Val Asn Arg Pro Leu Asp Arg Glu Arg Ile Pro
1905                1910                1915                1920

Glu Tyr Lys Leu Thr Ile Ser Val Lys Asp Asn Pro Glu Asn Pro Arg
                1925                1930                1935

Ile Ala Arg Arg Asp Tyr Asp Leu Leu Leu Ile Phe Leu Ser Asp Glu
            1940                1945                1950

Asn Asp Asn His Pro Leu Phe Thr Lys Ser Thr Tyr Gln Ala Glu Val
        1955                1960                1965

Met Glu Asn Ser Pro Ala Gly Thr Pro Leu Thr Val Leu Asn Gly Pro
    1970                1975                1980

Ile Leu Ala Leu Asp Ala Asp Gln Asp Ile Tyr Ala Val Val Thr Tyr
1985                1990                1995                2000

Gln Leu Leu Gly Ala Gln Ser Gly Leu Phe Asp Ile Asn Ser Ser Thr
                2005                2010                2015

Gly Val Val Thr Val Arg Ser Gly Val Ile Ile Asp Arg Glu Ala Phe
            2020                2025                2030

Ser Pro Pro Ile Leu Glu Leu Leu Leu Leu Ala Glu Asp Ile Gly Leu
        2035                2040                2045

Leu Asn Ser Thr Ala His Leu Leu Ile Thr Ile Leu Asp Asp Asn Asp
    2050                2055                2060

Asn Arg Pro Thr Phe Ser Pro Ala Thr Leu Thr Val His Leu Leu Glu
2065                2070                2075                2080

Asn Cys Pro Pro Gly Phe Ser Val Leu Gln Val Thr Ala Thr Asp Glu

2085                    2090                    2095

Asp Ser Gly Leu Asn Gly Glu Leu Val Tyr Arg Ile Glu Ala Gly Ala
            2100                    2105                    2110

Gln Asp Arg Phe Leu Ile His Leu Val Thr Gly Val Ile Arg Val Gly
        2115                    2120                    2125

Asn Ala Thr Ile Asp Arg Glu Glu Gln Glu Ser Tyr Arg Leu Thr Val
    2130                    2135                    2140

Val Ala Thr Asp Arg Gly Thr Val Pro Leu Ser Gly Thr Ala Ile Val
2145                    2150                    2155                    2160

Thr Ile Leu Ile Asp Asp Ile Asn Asp Ser Arg Pro Glu Phe Leu Asn
                2165                    2170                    2175

Pro Ile Gln Thr Val Ser Val Leu Glu Ser Ala Glu Pro Gly Thr Val
            2180                    2185                    2190

Ile Ala Asn Ile Thr Ala Ile Asp His Asp Leu Asn Pro Lys Leu Glu
        2195                    2200                    2205

Tyr His Ile Val Gly Ile Val Ala Lys Asp Asp Thr Asp Arg Leu Val
    2210                    2215                    2220

Pro Asn Gln Glu Asp Ala Phe Ala Val Asn Ile Asn Thr Gly Ser Val
2225                    2230                    2235                    2240

Met Val Lys Ser Pro Met Asn Arg Glu Leu Val Ala Thr Tyr Glu Val
                2245                    2250                    2255

Thr Leu Ser Val Ile Asp Asn Ala Ser Asp Leu Pro Glu Arg Ser Val
            2260                    2265                    2270

Ser Val Pro Asn Ala Lys Leu Thr Val Asn Val Leu Asp Val Asn Asp
        2275                    2280                    2285

Asn Thr Pro Gln Phe Lys Pro Phe Gly Ile Thr Tyr Tyr Met Glu Arg
    2290                    2295                    2300

Ile Leu Glu Gly Ala Thr Pro Gly Thr Thr Leu Ile Ala Val Ala Ala
2305                    2310                    2315                    2320

Val Asp Pro Asp Lys Gly Leu Asn Gly Leu Val Thr Tyr Thr Leu Leu
                2325                    2330                    2335

Asp Leu Val Pro Pro Gly Tyr Val Gln Leu Glu Asp Ser Ser Ala Gly
            2340                    2345                    2350

Lys Val Ile Ala Asn Arg Thr Val Asp Tyr Glu Glu Val His Trp Leu
        2355                    2360                    2365

Asn Phe Thr Val Arg Ala Ser Asp Asn Gly Ser Pro Pro Arg Ala Ala
    2370                    2375                    2380

Glu Ile Pro Val Tyr Leu Glu Ile Val Asp Ile Asn Asp Asn Asn Pro
2385                    2390                    2395                    2400

Ile Phe Asp Gln Pro Ser Tyr Gln Glu Ala Val Phe Glu Asp Val Pro
                2405                    2410                    2415

Val Gly Thr Ile Ile Leu Thr Val Thr Ala Thr Asp Ala Asp Ser Gly
            2420                    2425                    2430

Asn Phe Ala Leu Ile Glu Tyr Ser Leu Gly Asp Gly Glu Ser Lys Phe
        2435                    2440                    2445

Ala Ile Asn Pro Thr Thr Gly Asp Ile Tyr Val Leu Ser Ser Leu Asp

2450                    2455                    2460

Arg Glu Lys Lys Asp His Tyr Ile Leu Thr Ala Leu Ala Lys Asp Asn
2465                2470                2475                2480

Pro Gly Asp Val Ala Ser Asn Arg Arg Glu Asn Ser Val Gln Val Val
                2485                2490                2495

Ile Gln Val Leu Asp Val Asn Asp Cys Arg Pro Gln Phe Ser Lys Pro
            2500                2505                2510

Gln Phe Ser Thr Ser Val Tyr Glu Asn Glu Pro Ala Gly Thr Ser Val
        2515                2520                2525

Ile Thr Met Met Ala Thr Asp Gln Asp Glu Gly Pro Asn Gly Glu Leu
    2530                2535                2540

Thr Tyr Ser Leu Glu Gly Pro Gly Val Glu Ala Phe His Val Asp Met
2545                2550                2555                2560

Asp Ser Gly Leu Val Thr Thr Gln Arg Pro Leu Gln Ser Tyr Glu Lys
                2565                2570                2575

Phe Ser Leu Thr Val Val Ala Thr Asp Gly Gly Glu Pro Pro Leu Trp
            2580                2585                2590

Gly Thr Thr Met Leu Leu Val Glu Val Ile Asp Val Asn Asp Asn Arg
        2595                2600                2605

Pro Val Phe Val Arg Pro Pro Asn Gly Thr Ile Leu His Ile Arg Glu
    2610                2615                2620

Glu Ile Pro Leu Arg Ser Asn Val Tyr Glu Val Tyr Ala Thr Asp Lys
2625                2630                2635                2640

Asp Glu Gly Leu Asn Gly Ala Val Arg Tyr Ser Phe Leu Lys Thr Ala
                2645                2650                2655

Gly Asn Arg Asp Trp Glu Phe Phe Ile Ile Asp Pro Ile Ser Gly Leu
            2660                2665                2670

Ile Gln Thr Ala Gln Arg Leu Asp Arg Glu Ser Gln Ala Val Tyr Ser
        2675                2680                2685

Leu Ile Leu Val Ala Ser Asp Leu Gly Gln Pro Val Pro Tyr Glu Thr
    2690                2695                2700

Met Gln Pro Leu Gln Val Ala Leu Glu Asp Ile Asp Asp Asn Glu Pro
2705                2710                2715                2720

Leu Phe Val Arg Pro Pro Lys Gly Ser Pro Gln Tyr Gln Leu Leu Thr
                2725                2730                2735

Val Pro Glu His Ser Pro Arg Gly Thr Leu Val Gly Asn Val Thr Gly
            2740                2745                2750

Ala Val Asp Ala Asp Glu Gly Pro Asn Ala Ile Val Tyr Tyr Phe Ile
        2755                2760                2765

Ala Ala Gly Asn Glu Glu Lys Asn Phe His Leu Gln Pro Asp Gly Cys
    2770                2775                2780

Leu Leu Val Leu Arg Asp Leu Asp Arg Glu Arg Glu Ala Ile Phe Ser
2785                2790                2795                2800

Phe Ile Val Lys Ala Ser Ser Asn Arg Ser Trp Thr Pro Pro Arg Gly
                2805                2810                2815

Pro Ser Pro Thr Leu Asp Leu Val Ala Asp Leu Thr Leu Gln Glu Val

2820                    2825                    2830

Arg Val Val Leu Glu Asp Ile Asn Asp Gln Pro Pro Arg Phe Thr Lys
        2835                    2840                    2845

Ala Glu Tyr Thr Ala Gly Val Ala Thr Asp Ala Lys Val Gly Ser Glu
    2850                    2855                    2860

Leu Ile Gln Val Leu Ala Leu Asp Ala Asp Ile Gly Asn Asn Ser Leu
2865                    2870                    2875                    2880

Val Phe Tyr Ser Ile Leu Ala Ile His Tyr Phe Arg Ala Leu Ala Asn
                2885                    2890                    2895

Asp Ser Glu Asp Val Gly Gln Val Phe Thr Met Gly Ser Met Asp Gly
            2900                    2905                    2910

Ile Leu Arg Thr Phe Asp Leu Phe Met Ala Tyr Ser Pro Gly Tyr Phe
        2915                    2920                    2925

Val Val Asp Ile Val Ala Arg Asp Leu Ala Gly His Asn Asp Thr Ala
    2930                    2935                    2940

Ile Ile Gly Ile Tyr Ile Leu Arg Asp Asp Gln Arg Val Lys Ile Val
2945                    2950                    2955                    2960

Ile Asn Glu Ile Pro Asp Arg Val Arg Gly Phe Glu Glu Glu Phe Ile
                2965                    2970                    2975

His Leu Leu Ser Asn Ile Thr Gly Ala Ile Val Asn Thr Asp Asn Val
            2980                    2985                    2990

Gln Phe His Val Asp Lys Lys Gly Arg Val Asn Phe Ala Gln Thr Glu
        2995                    3000                    3005

Leu Leu Ile His Val Val Asn Arg Asp Thr Asn Arg Ile Leu Asp Val
    3010                    3015                    3020

Asp Arg Val Ile Gln Met Ile Asp Glu Asn Lys Glu Gln Leu Arg Asn
3025                    3030                    3035                    3040

Leu Phe Arg Asn Tyr Asn Val Leu Asp Val Gln Pro Ala Ile Ser Val
                3045                    3050                    3055

Arg Leu Pro Asp Asp Met Ser Ala Leu Gln Met Ala Ile Ile Val Leu
            3060                    3065                    3070

Ala Ile Leu Leu Phe Leu Ala Ala Met Leu Phe Val Leu Met Asn Trp
        3075                    3080                    3085

Tyr Tyr Arg Thr Val His Lys Arg Lys Leu Lys Ala Ile Val Ala Gly
    3090                    3095                    3100

Ser Ala Gly Asn Arg Gly Phe Ile Asp Ile Met Asp Met Pro Asn Thr
3105                    3110                    3115                    3120

Asn Lys Tyr Ser Phe Asp Gly Ala Asn Pro Val Trp Leu Asp Pro Phe
                3125                    3130                    3135

Cys Arg Asn Leu Glu Leu Ala Ala Gln Ala Glu His Glu Asp Asp Leu
            3140                    3145                    3150

Pro Glu Asn Leu Ser Glu Ile Ala Asp Leu Trp Asn Ser Pro Thr Arg
        3155                    3160                    3165

Thr Gln Gly Thr Phe Gly Arg Glu Pro Ala Ala Val Lys Pro Asp Asp
    3170                    3175                    3180

Asp Arg Tyr Leu Arg Ala Ala Ile Gln Glu Tyr Asp Asn Ile Ala Lys

3185                3190                3195                3200

Leu Gly Gln Ile Ile Arg Glu Gly Pro Ile Lys Gly Ser Leu Leu Lys
                3205                3210                3215

Val Val Leu Glu Asp Tyr Leu Arg Leu Lys Lys Leu Phe Ala Gln Arg
            3220                3225                3230

Met Val Gln Lys Ala Ser Ser Cys His Ser Ser Ile Ser Glu Leu Ile
        3235                3240                3245

Gln Thr Glu Leu Asp Glu Glu Pro Gly Asp His Ser Pro Gly Gln Gly
    3250                3255                3260

Ser Leu Arg Phe Arg His Lys Pro Pro Val Glu Leu Lys Gly Pro Asp
3265                3270                3275                3280

Gly Ile His Val Val His Gly Ser Thr Gly Thr Leu Leu Ala Thr Asp
                3285                3290                3295

Leu Asn Ser Leu Pro Glu Glu Asp Gln Lys Gly Leu Gly Arg Ser Leu
            3300                3305                3310

Glu Thr Leu Thr Ala Ala Glu Ala Thr Ala Phe Glu Arg Asn Ala Arg
        3315                3320                3325

Thr Glu Ser Ala Lys Ser Thr Pro Leu His Lys Leu Arg Asp Val Ile
    3330                3335                3340

Met Glu Thr Pro Leu Glu Ile Thr Glu Leu
3345                3350


<210> 198
<211> 3354
<212> PRT
<213> Homo sapiens

<400> 198
Met Gly Arg His Val Ala Thr Ser Cys His Val Ala Trp Leu Leu Val
1               5                   10                  15

Leu Ile Ser Gly Cys Trp Gly Gln Val Asn Arg Leu Pro Phe Phe Thr
            20                  25                  30

Asn His Phe Phe Asp Thr Tyr Leu Leu Ile Ser Glu Asp Thr Pro Val
        35                  40                  45

Gly Ser Ser Val Thr Gln Leu Leu Ala Gln Asp Met Asp Asn Asp Pro
    50                  55                  60

Leu Val Phe Gly Val Ser Gly Glu Glu Ala Ser Arg Phe Phe Ala Val
65                  70                  75                  80

Glu Pro Asp Thr Gly Val Val Trp Leu Arg Gln Pro Leu Asp Arg Glu
                85                  90                  95

Thr Lys Ser Glu Phe Thr Val Glu Phe Ser Val Ser Asp His Gln Gly
            100                 105                 110

Val Ile Thr Arg Lys Val Asn Ile Gln Val Gly Asp Val Asn Asp Asn
        115                 120                 125

Ala Pro Thr Phe His Asn Gln Pro Tyr Ser Val Arg Ile Pro Glu Asn
    130                 135                 140

Thr Pro Val Gly Thr Pro Ile Phe Ile Val Asn Ala Thr Asp Pro Asp
145                 150                 155                 160

Leu Gly Ala Gly Gly Ser Val Leu Tyr Ser Phe Gln Pro Pro Ser Gln
                165                 170                 175

Phe Phe Ala Ile Asp Ser Ala Arg Gly Ile Val Thr Val Ile Arg Glu
            180                 185                 190

Leu Asp Tyr Glu Thr Thr Gln Ala Tyr Gln Leu Thr Val Asn Ala Thr
        195                 200                 205

Asp Gln Asp Lys Thr Arg Pro Leu Ser Thr Leu Ala Asn Leu Ala Ile
    210                 215                 220

Ile Ile Thr Asp Val Gln Asp Met Asp Pro Ile Phe Ile Asn Leu Pro
225                 230                 235                 240

Tyr Ser Thr Asn Ile Tyr Glu His Ser Pro Pro Gly Thr Thr Val Arg
                245                 250                 255

Ile Ile Thr Ala Ile Asp Gln Asp Lys Gly Arg Pro Arg Gly Ile Gly
            260                 265                 270

Tyr Thr Ile Val Ser Gly Asn Thr Asn Ser Ile Phe Ala Leu Asp Tyr
        275                 280                 285

Ile Ser Gly Val Leu Thr Leu Asn Gly Leu Leu Asp Arg Glu Asn Pro
    290                 295                 300

Leu Tyr Ser His Gly Phe Ile Leu Thr Val Lys Gly Thr Glu Leu Asn
305                 310                 315                 320

Asp Asp Arg Thr Pro Ser Asp Ala Thr Val Thr Thr Thr Phe Asn Ile
                325                 330                 335

Leu Val Ile Asp Ile Asn Asp Asn Ala Pro Glu Phe Asn Ser Ser Glu
            340                 345                 350

Tyr Ser Val Ala Ile Thr Glu Leu Ala Gln Val Gly Phe Ala Leu Pro
        355                 360                 365

Leu Phe Ile Gln Val Val Asp Lys Asp Glu Asn Leu Gly Leu Asn Ser
    370                 375                 380

Met Phe Glu Val Tyr Leu Val Gly Asn Asn Ser His His Phe Ile Ile
385                 390                 395                 400

Ser Pro Thr Ser Val Gln Gly Lys Ala Asp Ile Arg Ile Arg Val Ala
                405                 410                 415

Ile Pro Leu Asp Tyr Glu Thr Val Asp Arg Tyr Asp Phe Asp Leu Phe
            420                 425                 430

Ala Asn Glu Ser Val Pro Asp His Val Gly Tyr Ala Lys Val Lys Ile
        435                 440                 445

Thr Leu Ile Asn Glu Asn Asp Asn Arg Pro Ile Phe Ser Gln Pro Leu
    450                 455                 460

Tyr Asn Ile Ser Leu Tyr Glu Asn Val Thr Val Gly Thr Ser Val Leu
465                 470                 475                 480

Thr Val Leu Ala Thr Asp Asn Asp Ala Gly Thr Phe Gly Glu Val Ser
                485                 490                 495

Tyr Phe Phe Ser Asp Asp Pro Asp Arg Phe Ser Leu Asp Lys Asp Thr
            500                 505                 510

Gly Leu Ile Met Leu Ile Ala Arg Leu Asp Tyr Glu Leu Ile Gln Arg
        515                 520                 525

Phe Thr Leu Thr Ile Ile Ala Arg Asp Gly Gly Gly Glu Glu Thr Thr
    530                 535                 540

Gly Arg Val Arg Ile Asn Val Leu Asp Val Asn Asp Asn Val Pro Thr
545                 550                 555                 560

Phe Gln Lys Asp Ala Tyr Val Gly Ala Leu Arg Glu Asn Glu Pro Ser
                565                 570                 575

Val Thr Gln Leu Val Arg Leu Arg Ala Thr Asp Glu Asp Ser Pro Pro
            580                 585                 590

Asn Asn Gln Ile Thr Tyr Ser Ile Val Ser Ala Ser Ala Phe Gly Ser
        595                 600                 605

Tyr Phe Asp Ile Ser Leu Tyr Glu Gly Tyr Gly Val Ile Ser Val Ser
    610                 615                 620

Arg Pro Leu Asp Tyr Glu Gln Ile Ser Asn Gly Leu Ile Tyr Leu Thr
625                 630                 635                 640

Val Met Ala Met Asp Ala Gly Asn Pro Pro Leu Asn Ser Thr Val Pro
                645                 650                 655

Val Thr Ile Glu Val Phe Asp Glu Asn Asp Asn Pro Pro Thr Phe Ser
            660                 665                 670

Lys Pro Ala Tyr Phe Val Ser Val Val Glu Asn Ile Met Ala Gly Ala
        675                 680                 685

Thr Val Leu Phe Leu Asn Ala Thr Asp Leu Asp Arg Ser Arg Glu Tyr
    690                 695                 700

Gly Gln Glu Ser Ile Ile Tyr Ser Leu Glu Gly Ser Thr Gln Phe Arg
705                 710                 715                 720

Ile Asn Ala Arg Ser Gly Glu Ile Thr Thr Thr Ser Leu Leu Asp Arg
                725                 730                 735

Glu Thr Lys Ser Glu Tyr Ile Leu Ile Val Arg Ala Val Asp Gly Gly
            740                 745                 750

Val Gly His Asn Gln Lys Thr Gly Ile Ala Thr Val Asn Ile Thr Leu
        755                 760                 765

Leu Asp Ile Asn Asp Asn His Pro Thr Trp Lys Asp Ala Pro Tyr Tyr
    770                 775                 780

Ile Asn Leu Val Glu Met Thr Pro Pro Asp Ser Asp Val Thr Thr Val
785                 790                 795                 800

Val Ala Val Asp Pro Asp Leu Gly Glu Asn Gly Thr Leu Val Tyr Ser
                805                 810                 815

Ile Gln Pro Pro Asn Lys Phe Tyr Ser Leu Asn Ser Thr Thr Gly Lys
            820                 825                 830

Ile Arg Thr Thr His Ala Met Leu Asp Arg Glu Asn Pro Asp Pro His
        835                 840                 845

Glu Ala Glu Leu Met Arg Lys Ile Val Val Ser Val Thr Asp Cys Gly
    850                 855                 860

Arg Pro Pro Leu Lys Ala Thr Ser Ser Ala Thr Val Phe Val Asn Leu
865                 870                 875                 880

Leu Asp Leu Asn Asp Asn Asp Pro Thr Phe Gln Asn Leu Pro Phe Val
                885                 890                 895

Ala Glu Val Leu Glu Gly Ile Pro Ala Gly Val Ser Ile Tyr Gln Val
            900                 905                 910

Val Ala Ile Asp Leu Asp Glu Gly Leu Asn Gly Leu Val Ser Tyr Arg
        915                 920                 925

Met Pro Val Gly Met Pro Arg Met Asp Phe Leu Ile Asn Ser Ser Ser
    930                 935                 940

Gly Val Val Val Thr Thr Thr Glu Leu Asp Arg Glu Arg Ile Ala Glu
945                 950                 955                 960

Tyr Gln Leu Arg Val Val Ala Ser Asp Ala Gly Thr Pro Thr Lys Ser
                965                 970                 975

Ser Thr Ser Thr Leu Thr Ile His Val Leu Asp Val Asn Asp Glu Thr
            980                 985                 990

Pro Thr Phe Phe Pro Ala Val Tyr Asn Val Ser Val Ser Glu Asp Val
        995                 1000                1005

Pro Arg Glu Phe Arg Val Val Trp Leu Asn Cys Thr Asp Asn Asp Val
    1010                1015                1020

Gly Leu Asn Ala Glu Leu Ser Tyr Phe Ile Thr Gly Gly Asn Val Asp
1025                1030                1035                1040

Gly Lys Phe Ser Val Gly Tyr Arg Asp Ala Val Val Arg Thr Val Val
                1045                1050                1055

Gly Leu Asp Arg Glu Thr Thr Ala Ala Tyr Met Leu Ile Leu Glu Ala
            1060                1065                1070

Ile Asp Asn Gly Pro Val Gly Lys Arg His Thr Gly Thr Ala Thr Val
        1075                1080                1085

Phe Val Thr Val Leu Asp Val Asn Asp Asn Arg Pro Ile Phe Leu Gln
    1090                1095                1100

Ser Ser Tyr Glu Ala Ser Val Pro Glu Asp Ile Pro Glu Gly His Ser
1105                1110                1115                1120

Ile Leu Gln Leu Lys Ala Thr Asp Ala Asp Glu Gly Glu Phe Gly Arg
                1125                1130                1135

Val Trp Tyr Arg Ile Leu His Gly Asn His Gly Asn Asn Phe Arg Ile
            1140                1145                1150

His Val Ser Asn Gly Leu Leu Met Arg Gly Pro Arg Pro Leu Asp Arg
        1155                1160                1165

Glu Arg Asn Ser Ser His Val Leu Ile Val Glu Ala Tyr Asn His Asp
    1170                1175                1180

Leu Gly Pro Met Arg Ser Ser Val Arg Val Ile Val Tyr Val Glu Asp
1185                1190                1195                1200

Ile Asn Asp Glu Ala Pro Val Phe Thr Gln Gln Gln Tyr Ser Arg Leu
                1205                1210                1215

Gly Leu Arg Glu Thr Ala Gly Ile Gly Thr Ser Val Ile Val Val Gln
            1220                1225                1230

Ala Thr Asp Arg Asp Ser Gly Asp Gly Gly Leu Val Asn Tyr Arg Ile
        1235                1240                1245

Leu Ser Gly Ala Glu Gly Lys Phe Glu Ile Asp Glu Ser Thr Gly Leu
    1250                1255                1260

Ile Ile Thr Val Asn Tyr Leu Asp Tyr Glu Thr Lys Thr Ser Tyr Met
1265                1270                1275                1280

Met Asn Val Ser Ala Thr Asp Gln Ala Pro Pro Phe Asn Gln Gly Phe
                1285                1290                1295

Cys Ser Val Tyr Ile Thr Leu Leu Asn Glu Leu Asp Glu Ala Val Gln
            1300                1305                1310

Phe Ser Asn Ala Ser Tyr Glu Ala Ala Ile Leu Glu Asn Leu Ala Leu
        1315                1320                1325

Gly Thr Glu Ile Val Arg Val Gln Ala Tyr Ser Ile Asp Asn Leu Asn
    1330                1335                1340

Gln Ile Thr Tyr Arg Phe Asp Ala Tyr Thr Ser Thr Gln Ala Lys Ala
1345                1350                1355                1360

Leu Phe Lys Ile Asp Ala Ile Thr Gly Val Ile Thr Val Gln Gly Leu
                1365                1370                1375

Val Asp Arg Glu Lys Gly Asp Phe Tyr Thr Leu Thr Val Val Ala Asp
            1380                1385                1390

Asp Gly Gly Pro Lys Val Asp Ser Thr Val Lys Val Tyr Ile Thr Val
        1395                1400                1405

Leu Asp Glu Asn Asp Asn Ser Pro Arg Phe Asp Phe Thr Ser Asp Ser
    1410                1415                1420

Ala Val Ser Ile Pro Glu Asp Cys Pro Val Gly Gln Arg Val Ala Thr
1425                1430                1435                1440

Val Lys Ala Trp Asp Pro Asp Ala Gly Ser Asn Gly Gln Val Val Phe
                1445                1450                1455

Ser Leu Ala Ser Gly Asn Ile Ala Gly Ala Phe Glu Ile Val Thr Thr
            1460                1465                1470

Asn Asp Ser Ile Gly Glu Val Phe Val Ala Arg Pro Leu Asp Arg Glu
        1475                1480                1485

Glu Leu Asp His Tyr Ile Leu Gln Val Val Ala Ser Asp Arg Gly Thr
    1490                1495                1500

Pro Pro Arg Lys Lys Asp His Ile Leu Gln Val Thr Ile Leu Asp Ile
1505                1510                1515                1520

Asn Asp Asn Pro Pro Val Ile Glu Ser Pro Phe Gly Tyr Asn Val Ser
                1525                1530                1535

Val Asn Glu Asn Val Gly Gly Gly Thr Ala Val Val Gln Val Arg Ala
            1540                1545                1550

Thr Asp Arg Asp Ile Gly Ile Asn Ser Val Leu Ser Tyr Tyr Ile Thr
        1555                1560                1565

Glu Gly Asn Lys Asp Met Thr Phe Arg Met Asp Arg Ile Ser Gly Glu
    1570                1575                1580

Ile Ala Thr Arg Pro Ala Pro Pro Asp Arg Glu Arg Gln Ser Phe Tyr
1585                1590                1595                1600

His Leu Val Ala Thr Val Glu Asp Glu Gly Thr Pro Thr Leu Ser Ala
                1605                1610                1615

Thr Thr His Val Tyr Val Thr Ile Val Asp Glu Asn Asp Asn Ala Pro
            1620                1625                1630

Met Phe Gln Gln Pro His Tyr Glu Val Leu Leu Asp Glu Gly Pro Asp
        1635                1640                1645

Thr Leu Asn Thr Ser Leu Ile Thr Ile Gln Ala Leu Asp Leu Asp Glu
    1650                1655                1660

Gly Pro Asn Gly Thr Val Thr Tyr Ala Ile Val Ala Gly Asn Ile Val
1665                1670                1675                1680

Asn Thr Phe Arg Ile Asp Arg His Met Gly Val Ile Thr Ala Ala Lys
                1685                1690                1695

Glu Leu Asp Tyr Glu Ile Ser His Gly Arg Tyr Thr Leu Ile Val Thr
            1700                1705                1710

Ala Thr Asp Gln Cys Pro Ile Leu Ser His Arg Leu Thr Ser Thr Thr
        1715                1720                1725

Thr Val Leu Val Asn Val Asn Asp Ile Asn Asp Asn Val Pro Thr Phe
    1730                1735                1740

Pro Arg Asp Tyr Glu Gly Pro Phe Glu Val Thr Glu Gly Gln Pro Gly
1745                1750                1755                1760

Pro Arg Val Trp Thr Phe Leu Ala His Asp Arg Asp Ser Gly Pro Asn
                1765                1770                1775

Gly Gln Val Glu Tyr Ser Ile Met Asp Gly Asp Pro Leu Gly Glu Phe
            1780                1785                1790

Val Ile Ser Pro Val Glu Gly Val Leu Arg Val Arg Lys Asp Val Glu
        1795                1800                1805

Leu Asp Arg Glu Thr Ile Ala Phe Tyr Asn Leu Thr Ile Cys Ala Arg
    1810                1815                1820

Asp Arg Gly Met Pro Pro Leu Ser Ser Thr Met Leu Val Gly Ile Arg
1825                1830                1835                1840

Val Leu Asp Ile Asn Asp Asn Asp Pro Val Leu Leu Asn Leu Pro Met
                1845                1850                1855

Asn Ile Thr Ile Ser Glu Asn Ser Pro Val Ser Ser Phe Val Ala His
            1860                1865                1870

Val Leu Ala Ser Asp Ala Asp Ser Gly Cys Asn Ala Arg Leu Thr Phe
        1875                1880                1885

Asn Ile Thr Ala Gly Asn Arg Glu Arg Ala Phe Phe Ile Asn Ala Thr
    1890                1895                1900

Thr Gly Ile Val Thr Val Asn Arg Pro Leu Asp Arg Glu Arg Ile Pro
1905                1910                1915                1920

Glu Tyr Lys Leu Thr Ile Ser Val Lys Asp Asn Pro Glu Asn Pro Arg
                1925                1930                1935

Ile Ala Arg Arg Asp Tyr Asp Leu Leu Leu Ile Phe Leu Ser Asp Glu
            1940                1945                1950

Asn Asp Asn His Pro Leu Phe Thr Lys Ser Thr Tyr Gln Ala Glu Val
        1955                1960                1965

Met Glu Asn Ser Pro Ala Gly Thr Pro Leu Thr Val Leu Asn Gly Pro
    1970                1975                1980

Ile Leu Ala Leu Asp Ala Asp Gln Asp Ile Tyr Ala Val Val Thr Tyr
1985                1990                1995                2000

Gln Leu Leu Gly Ala Gln Ser Gly Leu Phe Asp Ile Asn Ser Ser Thr
                2005                2010                2015

Gly Val Val Thr Val Arg Ser Gly Val Ile Ile Asp Arg Glu Ala Phe
            2020                2025                2030

Ser Pro Pro Ile Leu Glu Leu Leu Leu Leu Ala Glu Asp Ile Gly Leu
        2035                2040                2045

Leu Asn Ser Thr Ala His Leu Leu Ile Thr Ile Leu Asp Asp Asn Asp
    2050                2055                2060

Asn Arg Pro Thr Phe Ser Pro Ala Thr Leu Thr Val His Leu Leu Glu
2065                2070                2075                2080

Asn Cys Pro Pro Gly Phe Ser Val Leu Gln Val Thr Ala Thr Asp Glu
                2085                2090                2095

Asp Ser Gly Leu Asn Gly Glu Leu Val Tyr Arg Ile Glu Ala Gly Ala
            2100                2105                2110

Gln Asp Arg Phe Leu Ile His Leu Val Thr Gly Val Ile Arg Val Gly
        2115                2120                2125

Asn Ala Thr Ile Asp Arg Glu Glu Gln Glu Ser Tyr Arg Leu Thr Val
    2130                2135                2140

Val Ala Thr Asp Arg Gly Thr Val Pro Leu Ser Gly Thr Ala Ile Val
2145                2150                2155                2160

Thr Ile Leu Ile Asp Asp Ile Asn Asp Ser Arg Pro Glu Phe Leu Asn
                2165                2170                2175

Pro Ile Gln Thr Val Ser Val Leu Glu Ser Ala Glu Pro Gly Thr Val
            2180                2185                2190

Ile Ala Asn Ile Thr Ala Ile Asp His Asp Leu Asn Pro Lys Leu Glu
        2195                2200                2205

Tyr His Ile Val Gly Ile Val Ala Lys Asp Asp Thr Asp Arg Leu Val
    2210                2215                2220

Pro Asn Gln Glu Asp Ala Phe Ala Val Asn Ile Asn Thr Gly Ser Val
2225                2230                2235                2240

Met Val Lys Ser Pro Met Asn Arg Glu Leu Val Ala Thr Tyr Glu Val
                2245                2250                2255

Thr Leu Ser Val Ile Asp Asn Ala Ser Asp Leu Pro Glu Arg Ser Val
            2260                2265                2270

Ser Val Pro Asn Ala Lys Leu Thr Val Asn Val Leu Asp Val Asn Asp
        2275                2280                2285

Asn Thr Pro Gln Phe Lys Pro Phe Gly Ile Thr Tyr Tyr Met Glu Arg
    2290                2295                2300

Ile Leu Glu Gly Ala Thr Pro Gly Thr Thr Leu Ile Ala Val Ala Ala
2305                2310                2315                2320

Val Asp Pro Asp Lys Gly Leu Asn Gly Leu Val Thr Tyr Thr Leu Leu
                2325                2330                2335

Asp Leu Val Pro Pro Gly Tyr Val Gln Leu Glu Asp Ser Ser Ala Gly
            2340                2345                2350

Lys Val Ile Ala Asn Arg Thr Val Asp Tyr Glu Glu Val His Trp Leu
        2355                2360                2365

Asn Phe Thr Val Arg Ala Ser Asp Asn Gly Ser Pro Pro Arg Ala Ala
    2370                2375                2380

Glu Ile Pro Val Tyr Leu Glu Ile Val Asp Ile Asn Asp Asn Asn Pro
2385                2390                2395                2400

Ile Phe Asp Gln Pro Ser Tyr Gln Glu Ala Val Phe Glu Asp Val Pro
                2405                2410                2415

Val Gly Thr Ile Ile Leu Thr Val Thr Ala Thr Asp Ala Asp Ser Gly
            2420                2425                2430

Asn Phe Ala Leu Ile Glu Tyr Ser Leu Gly Asp Gly Glu Ser Lys Phe
        2435                2440                2445

Ala Ile Asn Pro Thr Thr Gly Asp Ile Tyr Val Leu Ser Ser Leu Asp
    2450                2455                2460

Arg Glu Lys Lys Asp His Tyr Ile Leu Thr Ala Leu Ala Lys Asp Asn
2465                2470                2475                2480

Pro Gly Asp Val Ala Ser Asn Arg Arg Glu Asn Ser Val Gln Val Val
                2485                2490                2495

Ile Gln Val Leu Asp Val Asn Asp Cys Arg Pro Gln Phe Ser Lys Pro
            2500                2505                2510

Gln Phe Ser Thr Ser Val Tyr Glu Asn Glu Pro Ala Gly Thr Ser Val
        2515                2520                2525

Ile Thr Met Met Ala Thr Asp Gln Asp Glu Gly Pro Asn Gly Glu Leu
    2530                2535                2540

Thr Tyr Ser Leu Glu Gly Pro Gly Val Glu Ala Phe His Val Asp Met
2545                2550                2555                2560

Asp Ser Gly Leu Val Thr Thr Gln Arg Pro Leu Gln Ser Tyr Glu Lys
                2565                2570                2575

Phe Ser Leu Thr Val Val Ala Thr Asp Gly Gly Glu Pro Pro Leu Trp
            2580                2585                2590

Gly Thr Thr Met Leu Leu Val Glu Val Ile Asp Val Asn Asp Asn Arg
        2595                2600                2605

Pro Val Phe Val Arg Pro Pro Asn Gly Thr Ile Leu His Ile Arg Glu
    2610                2615                2620

Glu Ile Pro Leu Arg Ser Asn Val Tyr Glu Val Tyr Ala Thr Asp Lys
2625                2630                2635                2640

Asp Glu Gly Leu Asn Gly Ala Val Arg Tyr Ser Phe Leu Lys Thr Ala
                2645                2650                2655

Gly Asn Arg Asp Trp Glu Phe Phe Ile Ile Asp Pro Ile Ser Gly Leu
            2660                2665                2670

Ile Gln Thr Ala Gln Arg Leu Asp Arg Glu Ser Gln Ala Val Tyr Ser
        2675                2680                2685

Leu Ile Leu Val Ala Ser Asp Leu Gly Gln Pro Val Pro Tyr Glu Thr
    2690                2695                2700

Met Gln Pro Leu Gln Val Ala Leu Glu Asp Ile Asp Asp Asn Glu Pro
2705                2710                2715                2720

Leu Phe Val Arg Pro Pro Lys Gly Ser Pro Gln Tyr Gln Leu Leu Thr
                2725                2730                2735

Val Pro Glu His Ser Pro Arg Gly Thr Leu Val Gly Asn Val Thr Gly
        2740                2745                2750

Ala Val Asp Ala Asp Glu Gly Pro Asn Ala Ile Val Tyr Tyr Phe Ile
    2755                2760                2765

Ala Ala Gly Asn Glu Glu Lys Asn Phe His Leu Gln Pro Asp Gly Cys
    2770                2775                2780

Leu Leu Val Leu Arg Asp Leu Asp Arg Glu Arg Glu Ala Ile Phe Ser
2785                2790                2795                2800

Phe Ile Val Lys Ala Ser Ser Asn Arg Ser Trp Thr Pro Pro Arg Gly
                2805                2810                2815

Pro Ser Pro Thr Leu Asp Leu Val Ala Asp Leu Thr Leu Gln Glu Val
            2820                2825                2830

Arg Val Val Leu Glu Asp Ile Asn Asp Gln Pro Pro Arg Phe Thr Lys
        2835                2840                2845

Ala Glu Tyr Thr Ala Gly Val Ala Thr Asp Ala Lys Val Gly Ser Glu
    2850                2855                2860

Leu Ile Gln Val Leu Ala Leu Asp Ala Asp Ile Gly Asn Asn Ser Leu
2865                2870                2875                2880

Val Phe Tyr Ser Ile Leu Ala Ile His Tyr Phe Arg Ala Leu Ala Asn
                2885                2890                2895

Asp Ser Glu Asp Val Gly Gln Val Phe Thr Met Gly Ser Met Asp Gly
            2900                2905                2910

Ile Leu Arg Thr Phe Asp Leu Phe Met Ala Tyr Ser Pro Gly Tyr Phe
        2915                2920                2925

Val Val Asp Ile Val Ala Arg Asp Leu Ala Gly His Asn Asp Thr Ala
    2930                2935                2940

Ile Ile Gly Ile Tyr Ile Leu Arg Asp Asp Gln Arg Val Lys Ile Val
2945                2950                2955                2960

Ile Asn Glu Ile Pro Asp Arg Val Arg Gly Phe Glu Glu Glu Phe Ile
                2965                2970                2975

His Leu Leu Ser Asn Ile Thr Gly Ala Ile Val Asn Thr Asp Asn Val
            2980                2985                2990

Gln Phe His Val Asp Lys Lys Gly Arg Val Asn Phe Ala Gln Thr Glu
        2995                3000                3005

Leu Leu Ile His Val Val Asn Arg Asp Thr Asn Arg Ile Leu Asp Val
    3010                3015                3020

Asp Arg Val Ile Gln Met Ile Asp Glu Asn Lys Glu Gln Leu Arg Asn
3025                3030                3035                3040

Leu Phe Arg Asn Tyr Asn Val Leu Asp Val Gln Pro Ala Ile Ser Val
                3045                3050                3055

Arg Leu Pro Asp Asp Met Ser Ala Leu Gln Met Ala Ile Ile Val Leu
            3060                3065                3070

Ala Ile Leu Leu Phe Leu Ala Ala Met Leu Phe Val Leu Met Asn Trp
        3075                3080                3085

Tyr Tyr Arg Thr Val His Lys Arg Lys Leu Lys Ala Ile Val Ala Gly
    3090                3095                3100

Ser Ala Gly Asn Arg Gly Phe Ile Asp Ile Met Asp Met Pro Asn Thr
3105                3110                3115                3120

Asn Lys Tyr Ser Phe Asp Gly Ala Asn Pro Val Trp Leu Asp Pro Phe
                3125                3130                3135

Cys Arg Asn Leu Glu Leu Ala Ala Gln Ala Glu His Glu Asp Asp Leu
            3140                3145                3150

Pro Glu Asn Leu Ser Glu Ile Ala Asp Leu Trp Asn Ser Pro Thr Arg
        3155                3160                3165

Thr His Gly Thr Phe Gly Arg Glu Pro Ala Ala Val Lys Pro Asp Asp
    3170                3175                3180

Asp Arg Tyr Leu Arg Ala Ala Ile Gln Glu Tyr Asp Asn Ile Ala Lys
3185                3190                3195                3200

Leu Gly Gln Ile Ile Arg Glu Gly Pro Ile Lys Gly Ser Leu Leu Lys
                3205                3210                3215

Val Val Leu Glu Asp Tyr Leu Arg Leu Lys Lys Leu Phe Ala Gln Arg
            3220                3225                3230

Met Val Gln Lys Ala Ser Ser Cys His Ser Ser Ile Ser Glu Leu Ile
        3235                3240                3245

Gln Thr Glu Leu Asp Glu Glu Pro Gly Asp His Ser Pro Gly Gln Gly
    3250                3255                3260

Ser Leu Arg Phe Arg His Lys Pro Pro Val Glu Leu Lys Gly Pro Asp
3265                3270                3275                3280

Gly Ile His Val Val His Gly Ser Thr Gly Thr Leu Leu Ala Thr Asp
                3285                3290                3295

Leu Asn Ser Leu Pro Glu Glu Asp Gln Lys Gly Leu Gly Arg Ser Leu
            3300                3305                3310

Glu Thr Leu Thr Ala Ala Glu Ala Thr Ala Phe Glu Arg Asn Ala Arg
        3315                3320                3325

Thr Glu Ser Ala Lys Ser Thr Pro Leu His Lys Leu Arg Asp Val Ile
    3330                3335                3340

Met Glu Thr Pro Leu Glu Ile Thr Glu Leu
3345                3350


<210> 199
<211> 803
<212> PRT
<213> Homo sapiens

<400> 199
Asp Ile Arg Ile Arg Val Ala Ile Pro Leu Asp Tyr Glu Thr Val Asp
1               5                   10                  15

Arg Tyr Asp Phe Asp Leu Phe Ala Asn Glu Ser Val Pro Asp His Val
            20                  25                  30

Gly Tyr Ala Lys Val Lys Ile Thr Leu Ile Asn Glu Asn Asp Asn Arg
        35                  40                  45

Pro Ile Phe Ser Gln Pro Leu Tyr Asn Ile Ser Leu Tyr Glu Asn Val
    50                  55                  60

Thr Val Gly Thr Ser Val Leu Thr Val Leu Ala Thr Asp Asn Asp Ala
65                  70                  75                  80

Gly Thr Phe Gly Glu Val Asn Tyr Phe Phe Ser Asp Asp Pro Asp Arg
                85                  90                  95

Phe Ser Leu Asp Lys Asp Thr Gly Leu Ile Met Leu Ile Ala Arg Leu
            100                 105                 110

Asp Tyr Glu Leu Ile Gln Arg Phe Thr Leu Thr Ile Ile Ala Arg Asp
        115                 120                 125

Gly Gly Gly Glu Glu Thr Thr Gly Arg Val Arg Ile Asn Val Leu Asp
    130                 135                 140

Val Asn Asp Asn Val Pro Thr Phe Gln Lys Asp Ala Tyr Val Gly Ala
145                 150                 155                 160

Leu Arg Glu Asn Glu Pro Ser Val Thr Gln Leu Val Arg Leu Arg Ala
                165                 170                 175

Thr Asp Glu Asp Ser Pro Pro Asn Asn Gln Ile Thr Tyr Ser Ile Val
            180                 185                 190

Ser Ala Ser Ala Phe Gly Ser Tyr Phe Asp Ile Ser Leu Tyr Glu Gly
        195                 200                 205

Tyr Gly Val Ile Ser Val Ser Arg Pro Leu Asp Tyr Glu Gln Ile Ser
    210                 215                 220

Asn Gly Leu Ile Tyr Leu Thr Val Met Ala Met Asp Ala Gly Asn Pro
225                 230                 235                 240

Pro Leu Asn Ser Thr Val Pro Val Thr Ile Glu Val Phe Asp Glu Asn
                245                 250                 255

Asp Asn Pro Pro Thr Phe Ser Lys Pro Ala Tyr Phe Val Ser Val Val
            260                 265                 270

Glu Asn Ile Met Ala Gly Ala Thr Val Leu Phe Leu Asn Ala Thr Asp
        275                 280                 285

Leu Asp Arg Ser Arg Glu Tyr Gly Gln Glu Ser Ile Ile Tyr Ser Leu
    290                 295                 300

Glu Gly Ser Thr Gln Phe Arg Ile Asn Ala Arg Ser Gly Glu Ile Thr
305                 310                 315                 320

Thr Thr Ser Leu Leu Asp Arg Glu Thr Lys Ser Glu Tyr Ile Leu Ile
                325                 330                 335

Val Arg Ala Val Asp Gly Gly Val Gly His Asn Gln Lys Thr Gly Ile
            340                 345                 350

Ala Thr Val Asn Ile Thr Leu Leu Asp Ile Asn Asp Asn His Pro Thr
        355                 360                 365

Trp Lys Asp Ala Pro Tyr Tyr Ile Asn Leu Val Glu Met Thr Pro Pro
    370                 375                 380

Asp Ser Asp Val Thr Thr Val Val Ala Val Asp Pro Asp Leu Gly Glu
385                 390                 395                 400

Asn Gly Thr Leu Val Tyr Ser Ile Gln Pro Pro Asn Lys Phe Tyr Ser
                405                 410                 415

Leu Asn Ser Thr Thr Gly Lys Ile Arg Thr Thr His Ala Met Leu Asp
            420                 425                 430

Arg Glu Asn Pro Asp Pro His Glu Ala Glu Leu Met Arg Lys Ile Val
        435                 440                 445

Val Ser Val Thr Asp Cys Gly Arg Pro Pro Leu Lys Ala Thr Ser Ser
    450                 455                 460

Ala Thr Val Phe Val Asn Leu Leu Asp Leu Asn Asp Asn Asp Pro Thr
465                 470                 475                 480

Phe Gln Asn Leu Pro Phe Val Ala Glu Val Leu Glu Gly Ile Pro Ala
                485                 490                 495

Gly Val Ser Ile Tyr Gln Val Val Ala Ile Asp Leu Asp Glu Gly Leu
            500                 505                 510

Asn Gly Leu Val Ser Tyr Arg Met Pro Val Gly Met Pro Arg Met Asp
        515                 520                 525

Phe Leu Ile Asn Ser Ser Ser Gly Val Val Val Thr Thr Thr Glu Leu
    530                 535                 540

Asp Arg Glu Arg Ile Ala Glu Tyr Gln Leu Arg Val Val Ala Ser Asp
545                 550                 555                 560

Ala Gly Thr Pro Thr Lys Ser Ser Thr Ser Thr Leu Thr Ile His Val
                565                 570                 575

Leu Asp Val Asn Asp Glu Thr Pro Thr Phe Phe Pro Ala Val Tyr Asn
            580                 585                 590

Val Ser Val Ser Glu Asp Val Pro Arg Glu Phe Arg Val Val Trp Leu
        595                 600                 605

Asn Cys Thr Asp Asn Asp Val Gly Leu Asn Ala Glu Leu Ser Tyr Phe
    610                 615                 620

Ile Thr Gly Gly Asn Val Asp Gly Lys Phe Ser Val Gly Tyr Arg Asp
625                 630                 635                 640

Ala Val Val Arg Thr Val Val Gly Leu Asp Arg Glu Thr Thr Ala Ala
                645                 650                 655

Tyr Met Leu Ile Leu Glu Ala Ile Asp Asn Gly Pro Val Gly Lys Arg
            660                 665                 670

His Thr Gly Thr Ala Thr Val Phe Val Thr Val Leu Asp Val Asn Asp
        675                 680                 685

Asn Arg Pro Ile Phe Leu Gln Ser Ser Tyr Glu Ala Ser Val Pro Glu
    690                 695                 700

Asp Ile Pro Glu Gly His Ser Ile Leu Gln Glu Glu Asp Leu Ala Ser
705                 710                 715                 720

Pro Cys Ile Ser Pro Ala Pro Pro Arg Arg Ala Phe Gln Ser Ser Gly
                725                 730                 735

Glu Lys Glu Thr Ser Gln Phe Pro Gly Lys Glu Leu Arg Arg Glu Pro
            740                 745                 750

Gly Pro Ser Lys Ala Gln Asn Arg Ala Ala Phe Thr Glu Pro Leu Ala
        755                 760                 765

Glu Ala Pro Leu Leu Gly Ser Lys Gln Ala Gln Glu Glu Arg Ala Pro
    770                 775                 780

Leu Pro Arg Glu Gln Ala Gln Gln Leu Gln Gly Ser Glu Gly Glu Lys
785                 790                 795                 800

Gly Gly Pro

<210> 200
<211> 3317
<212> PRT
<213> Rattus norvegicus

<400> 200
Met Arg His Pro Pro Val Thr Trp Cys Ala Met Leu Trp Leu Leu Met
1               5                   10                  15

Leu Val Ser Gly Ser Trp Gly Gln Val Asn Arg Leu Pro Phe Phe Thr
            20                  25                  30

Asn His Phe Phe Asp Thr Tyr Leu Leu Ile Ser Glu Asp Thr Pro Val
        35                  40                  45

Gly Ser Ser Val Thr Gln Leu Leu Ala Arg Asp Met Asp Asn Asp Pro
    50                  55                  60

Leu Val Phe Gly Val Ser Gly Glu Glu Ala Ser Arg Phe Phe Ala Val
65                  70                  75                  80

Glu Pro Asp Thr Gly Val Val Trp Leu Arg Gln Pro Leu Asp Arg Glu
                85                  90                  95

Thr Lys Ser Glu Phe Thr Val Glu Phe Ser Val Ser Asp His Gln Gly
            100                 105                 110

Val Ile Thr Arg Lys Val Asn Ile Gln Val Gly Asp Val Asn Asp Asn
        115                 120                 125

Ala Pro Thr Phe His Asn Gln Pro Tyr Ser Val Arg Ile Pro Glu Asn
    130                 135                 140

Thr Pro Val Gly Thr Pro Ile Phe Ile Val Asn Ala Thr Asp Pro Asp
145                 150                 155                 160

Leu Gly Ala Gly Gly Ser Val Leu Tyr Ser Phe Gln Pro Pro Ser Gln
                165                 170                 175

Phe Phe Ala Ile Asp Ser Ala Arg Gly Ile Val Thr Val Ile Arg Glu
            180                 185                 190

Leu Asp Tyr Glu Val Thr Gln Ala Tyr Gln Leu Thr Val Asn Ala Thr
        195                 200                 205

Asp Gln Asp Lys Thr Arg Pro Leu Ser Thr Leu Ala Asn Leu Ala Ile
    210                 215                 220

Ile Ile Thr Asp Val Gln Asp Met Asp Pro Ile Phe Ile Asn Leu Pro
225                 230                 235                 240

Tyr Ser Thr Asn Ile Tyr Glu His Ser Pro Pro Gly Thr Thr Val Arg
                245                 250                 255

Val Ile Thr Ala Val Asp Gln Asp Lys Gly Arg Pro Arg Gly Ile Gly
            260                 265                 270

Tyr Thr Ile Val Ser Gly Asn Thr Asn Ser Ile Phe Ala Leu Asp Tyr
        275                 280                 285

Ile Ser Gly Ala Leu Thr Leu Asn Gly Leu Leu Asp Arg Glu Asn Pro
    290                 295                 300

Leu Tyr Ser His Gly Phe Ile Leu Thr Val Lys Gly Thr Glu Leu Asn
305                 310                 315                 320

Asp Asp Arg Ser Pro Ser Asp Ala Thr Val Thr Thr Thr Phe Asn Ile

                325                 330                 335

Leu Val Ile Asp Ile Asn Asp Asn Ala Pro Glu Phe Asn Ser Ser Glu
            340                 345                 350

Tyr Ser Val Ala Ile Thr Glu Leu Ala Gln Val Gly Phe Ala Leu Pro
        355                 360                 365

Leu Phe Ile Gln Val Val Asp Lys Asp Glu Gly Leu Asn Ser Met Phe
    370                 375                 380

Glu Val Tyr Leu Val Gly Asn Asn Ser His His Phe Ile Ile Ser Pro
385                 390                 395                 400

Thr Ser Val Gln Gly Lys Ala Asp Ile Arg Ile Arg Val Ala Ile Pro
                405                 410                 415

Leu Asp Tyr Glu Thr Val Asp Arg Tyr Asp Phe Asp Leu Phe Ala Asn
            420                 425                 430

Glu Ser Val Pro Asp His Val Gly Tyr Ala Lys Val Lys Ile Thr Leu
        435                 440                 445

Ile Asn Glu Asn Asp Asn Arg Pro Ile Phe Ser Gln Pro Leu Tyr Asn
    450                 455                 460

Val Ser Leu Tyr Glu Asn Ile Thr Val Gly Thr Ser Val Leu Thr Val
465                 470                 475                 480

Leu Ala Thr Asp Asn Asp Val Gly Thr Phe Gly Glu Val Asn Tyr Phe
                485                 490                 495

Phe Ser Asp Asp Pro Asp Arg Phe Ser Leu Asp Lys Asp Thr Gly Leu
            500                 505                 510

Ile Met Leu Ile Ala Arg Leu Asp Tyr Glu Leu Ile Gln Arg Phe Thr
        515                 520                 525

Leu Thr Val Ile Ala Arg Asp Gly Gly Gly Glu Glu Thr Thr Gly Arg
    530                 535                 540

Val Arg Ile Asn Val Leu Asp Val Asn Asp Asn Val Pro Thr Phe Gln
545                 550                 555                 560

Lys Asp Ala Tyr Val Gly Ala Leu Arg Glu Asn Glu Pro Ser Val Thr
                565                 570                 575

Gln Leu Val Arg Leu Arg Ala Thr Asp Glu Asp Ser Pro Pro Asn Asn
            580                 585                 590

Leu Ile Thr Tyr Ser Ile Val Asn Ala Ser Ala Phe Gly Ser Tyr Phe
        595                 600                 605

Asp Ile Ser Val Tyr Glu Gly Tyr Gly Val Ile Ser Val Ser Arg Pro
    610                 615                 620

Leu Asp Tyr Glu Gln Ile Pro Asn Gly Leu Ile Tyr Leu Thr Val Met
625                 630                 635                 640

Ala Lys Asp Ala Gly Asn Pro Pro Leu Tyr Ser Thr Val Pro Val Thr
                645                 650                 655

Ile Glu Val Phe Asp Glu Asn Asp Asn Pro Pro Thr Phe Ser Lys Pro
            660                 665                 670

Ala Tyr Phe Val Ser Val Val Glu Asn Ile Met Ala Gly Ala Thr Val
        675                 680                 685

Leu Phe Leu Asn Ala Thr Asp Leu Asp Arg Ser Arg Glu Tyr Gly Gln

690                 695                 700

Glu Ser Ile Ile Tyr Ser Leu Glu Gly Ser Ser Gln Phe Arg Ile Asn
705                 710                 715                 720

Ala Arg Ser Gly Glu Ile Thr Thr Thr Ser Leu Leu Asp Arg Glu Thr
                725                 730                 735

Lys Ala Glu Tyr Ile Leu Ile Val Arg Ala Val Asp Gly Gly Val Gly
            740                 745                 750

His Asn Gln Lys Thr Gly Ile Ala Thr Val Asn Val Thr Leu Leu Asp
        755                 760                 765

Ile Asn Asp Asn His Pro Thr Trp Lys Asp Ala Pro Tyr Tyr Ile Asn
    770                 775                 780

Leu Val Glu Met Thr Pro Pro Asp Ser Asp Val Thr Thr Val Val Ala
785                 790                 795                 800

Val Asp Pro Asp Leu Gly Lys Asn Gly Thr Leu Val Tyr Ser Ile Gln
                805                 810                 815

Pro Pro Asn Lys Phe Tyr Ser Leu Asn Ser Thr Thr Gly Lys Ile Arg
            820                 825                 830

Thr Thr His Val Met Leu Asp Arg Glu Asn Pro Asp Pro Val Glu Ala
        835                 840                 845

Glu Leu Met Arg Lys Ile Ile Val Ser Val Thr Asp Cys Gly Arg Pro
    850                 855                 860

Pro Leu Lys Ala Thr Ser Ser Ala Thr Val Phe Val Asn Leu Leu Asp
865                 870                 875                 880

Leu Asn Asp Asn Asp Pro Thr Phe Gln Asn Leu Pro Phe Val Ala Glu
                885                 890                 895

Val Leu Glu Gly Thr Pro Ala Gly Val Ser Val Tyr Gln Val Val Ala
            900                 905                 910

Ile Asp Leu Asp Glu Gly Leu Asn Gly Leu Val Ser Tyr Arg Met Gln
        915                 920                 925

Val Gly Met Pro Arg Met Asp Phe Val Ile Asn Ser Thr Ser Gly Val
    930                 935                 940

Val Thr Thr Thr Ala Glu Leu Asp Arg Glu Arg Ile Ala Glu Tyr Gln
945                 950                 955                 960

Leu Arg Val Val Ala Ser Asp Ala Gly Thr Pro Thr Lys Ser Ser Thr
                965                 970                 975

Ser Thr Leu Thr Ile Arg Val Leu Asp Val Asn Asp Glu Thr Pro Thr
            980                 985                 990

Phe Phe Pro Ala Val Tyr Asn Val Ser Val Ser Glu Asp Val Pro Arg
        995                 1000                1005

Glu Phe Arg Val Val Trp Leu Asn Cys Thr Asp Asn Asp Val Gly Leu
    1010                1015                1020

Asn Ala Glu Leu Ser Tyr Phe Ile Thr Ala Gly Asn Val Asp Gly Lys
1025                1030                1035                1040

Phe Ser Val Gly Tyr Arg Asp Ala Val Val Arg Thr Val Val Gly Leu
                1045                1050                1055

Asp Arg Glu Thr Thr Ala Ala Tyr Thr Leu Val Leu Glu Ala Ile Asp

1060                    1065                    1070

Asn Val Pro Val Gly Lys Arg Arg Thr Gly Thr Ala Thr Val Phe Val
        1075                    1080                    1085

Thr Val Leu Asp Val Asn Asp Asn Arg Pro Ile Phe Leu Gln Ser Ser
    1090                    1095                    1100

Tyr Glu Ala Ser Val Pro Glu Asp Ile Pro Glu Gly His Ser Ile Val
1105                    1110                    1115                    1120

Gln Leu Lys Ala Thr Asp Ala Asp Glu Gly Glu Phe Gly Arg Val Trp
                1125                    1130                    1135

Tyr Arg Ile Leu His Gly Asn His Gly Asn Asn Phe Arg Leu His Val
            1140                    1145                    1150

Ser Ser Gly Leu Leu Val Arg Gly Pro Arg Pro Leu Asp Arg Glu Arg
        1155                    1160                    1165

Asn Ser Ser His Val Leu Met Ala Glu Ala Tyr Asn His Asp Leu Gly
    1170                    1175                    1180

Pro Met Arg Ser Ser Val Arg Val Ile Val Tyr Val Glu Asp Val Asn
1185                    1190                    1195                    1200

Asp Glu Ala Pro Val Phe Thr Gln Gln Gln Tyr Asn Arg Leu Gly Leu
                1205                    1210                    1215

Arg Glu Thr Ala Gly Ile Gly Thr Ser Val Ile Val Val Arg Ala Thr
            1220                    1225                    1230

Asp Arg Asp Thr Gly Asp Gly Gly Leu Val Asn Tyr Arg Ile Leu Ser
        1235                    1240                    1245

Gly Ala Glu Gly Lys Phe Glu Ile Asp Glu Ser Thr Gly Leu Ile Val
    1250                    1255                    1260

Thr Val Asp Tyr Leu Asp Tyr Glu Thr Lys Thr Ser Tyr Leu Met Asn
1265                    1270                    1275                    1280

Val Ser Ala Thr Asp Gly Ala Pro Pro Phe Asn Gln Gly Phe Cys Ser
                1285                    1290                    1295

Val Tyr Val Thr Leu Leu Asn Glu Leu Asp Glu Ala Val Gln Phe Ser
            1300                    1305                    1310

Asn Ala Ser Tyr Glu Ala Val Ile Met Glu Asn Leu Ala Leu Gly Thr
        1315                    1320                    1325

Glu Ile Val Arg Val Gln Ala Tyr Ser Ile Asp Asn Leu Asn Gln Ile
    1330                    1335                    1340

Thr Tyr Arg Phe Asp Ala Tyr Thr Ser Ala Gln Ala Lys Ala Leu Phe
1345                    1350                    1355                    1360

Lys Ile Asp Ala Ile Thr Gly Val Ile Thr Val Lys Gly Leu Val Asp
                1365                    1370                    1375

Arg Glu Lys Gly Asp Phe Tyr Thr Leu Thr Val Val Ala Asp Asp Gly
            1380                    1385                    1390

Gly Pro Lys Val Asp Ser Thr Val Lys Val Tyr Val Thr Val Leu Asp
        1395                    1400                    1405

Glu Asn Asp Asn Ser Pro Arg Phe Asp Phe Thr Ser Asp Ser Ala Ile
    1410                    1415                    1420

Ser Val Pro Glu Asp Cys Pro Val Gly Gln Arg Val Ala Thr Val Lys

1425                1430                1435                1440

Ala Arg Asp Pro Asp Ala Gly Ser Asn Gly Gln Val Val Phe Ser Leu
                1445                1450                1455

Ala Ser Gly Asn Ile Ala Gly Ala Phe Glu Ile Ile Thr Ser Asn Asp
            1460                1465                1470

Ser Ile Gly Glu Val Phe Val Ala Lys Pro Leu Asp Arg Glu Glu Leu
        1475                1480                1485

Asp His Tyr Ile Leu Lys Ile Val Ala Ser Asp Arg Gly Thr Pro Pro
    1490                1495                1500

Arg Lys Lys Asp His Ile Leu Gln Val Thr Ile Leu Asp Val Asn Asp
1505                1510                1515                1520

Asn Pro Pro Val Ile Glu Ser Pro Phe Gly Tyr Asn Val Ser Val Asn
                1525                1530                1535

Glu Asn Val Gly Gly Gly Thr Ser Val Val Gln Val Arg Ala Thr Asp
            1540                1545                1550

Arg Asp Ile Gly Ile Asn Ser Val Leu Ser Tyr Tyr Ile Thr Glu Gly
        1555                1560                1565

Asn Glu Asp Met Thr Phe Arg Met Asp Arg Ile Ser Gly Glu Ile Ala
    1570                1575                1580

Thr Arg Pro Ala Pro Pro Asp Arg Glu Arg Gln Asn Phe Tyr His Leu
1585                1590                1595                1600

Val Val Thr Val Glu Asp Glu Gly Thr Pro Thr Leu Ser Ala Thr Thr
                1605                1610                1615

His Val Tyr Val Thr Ile Val Asp Glu Asn Asp Asn Ala Pro Val Phe
            1620                1625                1630

Gln Gln Pro His Tyr Glu Val Val Leu Asp Glu Gly Pro Asp Thr Val
        1635                1640                1645

Asn Thr Ser Leu Ile Thr Val Gln Ala Leu Asp Leu Asp Glu Gly Pro
    1650                1655                1660

Asn Gly Thr Val Thr Tyr Ala Ile Val Ala Gly Asn Ile Ile Asn Thr
1665                1670                1675                1680

Phe Arg Ile Asn Arg Arg Thr Gly Val Ile Thr Ala Ala Lys Glu Leu
                1685                1690                1695

Asp Tyr Glu Ile Ser His Gly Arg Tyr Thr Leu Ile Val Thr Ala Thr
            1700                1705                1710

Asp Gln Cys Pro Ile Leu Ser His Arg Leu Thr Ser Thr Thr Thr Val
        1715                1720                1725

Leu Val Asn Val Asn Asp Ile Asn Asp Asn Val Pro Thr Phe Pro Arg
    1730                1735                1740

Asp Tyr Glu Gly Pro Phe Asp Val Thr Glu Gly Gln Pro Gly Pro Arg
1745                1750                1755                1760

Val Trp Thr Phe Leu Ala His Asp Arg Asp Ser Gly Pro Asn Gly Gln
                1765                1770                1775

Val Glu Tyr Ser Val Val Asp Gly Asp Pro Leu Gly Glu Phe Val Ile
            1780                1785                1790

Ser Pro Val Glu Gly Val Leu Arg Val Arg Lys Asp Val Glu Leu Asp

1795                1800                1805

Arg Glu Thr Ile Ala Phe Tyr Asn Leu Thr Ile Cys Ala Arg Asp Arg
    1810                1815                1820

Gly Val Pro Pro Leu Ser Ser Thr Met Leu Val Gly Ile Arg Val Leu
1825                1830                1835                1840

Asp Ile Asn Asp Asn Asp Pro Val Leu Leu Asn Leu Pro Met Asn Ile
                1845                1850                1855

Thr Ile Ser Glu Asn Ser Pro Val Ser Ser Phe Val Ala His Val Leu
            1860                1865                1870

Ala Ser Asp Ala Asp Ser Gly Cys Asn Ala Leu Leu Thr Phe Asn Ile
        1875                1880                1885

Thr Ala Gly Asn Arg Glu Arg Ala Phe Phe Ile Asn Ala Thr Thr Gly
    1890                1895                1900

Ile Val Thr Val Asn Arg Pro Leu Asp Arg Glu Arg Ile Pro Glu Tyr
1905                1910                1915                1920

Arg Leu Thr Val Ser Val Lys Asp Asn Pro Glu Asn Pro Arg Ile Ala
                1925                1930                1935

Arg Lys Asp Phe Asp Leu Leu Leu Val Ser Leu Ala Asp Glu Asn Asp
            1940                1945                1950

Asn His Pro Leu Phe Thr Glu Gly Thr Tyr Gln Ala Glu Val Met Glu
        1955                1960                1965

Asn Ser Pro Ala Gly Thr Pro Leu Thr Val Leu Asn Gly Pro Ile Leu
    1970                1975                1980

Ala Leu Asp Ala Asp Glu Asp Val Tyr Ala Val Val Thr Tyr Gln Leu
1985                1990                1995                2000

Leu Gly Thr His Ser Asp Leu Phe Val Ile Asp Asn Ser Thr Gly Val
                2005                2010                2015

Val Thr Val Arg Ser Gly Val Ile Ile Asp Arg Glu Ala Phe Ser Pro
            2020                2025                2030

Pro Phe Leu Glu Leu Leu Leu Leu Ala Glu Asp Val Gly Gln Leu Asn
        2035                2040                2045

Gly Thr Ala Tyr Leu Phe Ile Thr Ile Leu Asp Asp Asn Asp Asn Trp
    2050                2055                2060

Pro Thr Phe Ser Pro Pro Ala Tyr Thr Val His Leu Leu Glu Asn Cys
2065                2070                2075                2080

Pro Pro Gly Phe Ser Val Leu Gln Ile Thr Ala Thr Asp Glu Asp Ser
                2085                2090                2095

Gly Leu Asn Gly Glu Leu Val Tyr Arg Ile Glu Ala Gly Ala Gln Asp
            2100                2105                2110

Arg Phe Leu Ile His Pro Val Thr Gly Val Ile Arg Val Gly Asn Ala
        2115                2120                2125

Thr Ile Asp Arg Glu Glu Gln Glu Ser Tyr Arg Leu Thr Val Val Ala
    2130                2135                2140

Thr Asp Arg Gly Thr Val Pro Leu Ser Gly Thr Ala Thr Val Thr Ile
2145                2150                2155                2160

Leu Ile Asp Asp Ile Asn Asp Ser Arg Pro Glu Phe Leu Asn Pro Ile

2165                    2170                    2175

Gln Thr Val Ser Val Leu Glu Ser Thr Glu Pro Gly Thr Val Ile Ala
            2180                2185                2190

Asn Val Thr Ala Ile Asp Leu Asp Leu Asn Pro Lys Leu Glu Tyr His
        2195                2200                2205

Ile Leu Ser Ile Val Ala Lys Asp Asp Thr Asp Arg Leu Val Pro Asp
    2210                2215                2220

Gln Glu Asp Ala Phe Ala Val Asn Ile Asn Thr Gly Ser Val Ile Val
2225                2230                2235                2240

Lys Ser Pro Leu Asn Arg Glu Leu Val Ala Thr Tyr Glu Val Thr Leu
                2245                2250                2255

Ser Val Ile Asp Asn Ala Ser Asp Leu Pro Glu Arg Ser Val Ser Val
            2260                2265                2270

Pro Asn Ala Lys Leu Thr Val Asn Ile Leu Asp Val Asn Asp Asn Thr
        2275                2280                2285

Pro Gln Phe Lys Pro Phe Gly Ile Thr Tyr Tyr Thr Glu Arg Val Leu
    2290                2295                2300

Glu Gly Ala Thr Pro Gly Thr Thr Leu Ile Ala Val Ala Ala Val Asp
2305                2310                2315                2320

Pro Asp Lys Gly Leu Asn Gly Leu Ile Thr Tyr Thr Leu Leu Asp Leu
                2325                2330                2335

Ile Pro Pro Gly Tyr Val Gln Leu Glu Asp Ser Ser Ala Gly Lys Val
            2340                2345                2350

Ile Ala Asn Arg Thr Val Asp Tyr Glu Glu Val His Trp Leu Asn Phe
        2355                2360                2365

Thr Val Arg Ala Ser Asp Asn Gly Ser Pro Pro Arg Ala Ala Glu Ile
    2370                2375                2380

Pro Val Tyr Leu Glu Ile Val Asp Ile Asn Asp Asn Asn Pro Ile Phe
2385                2390                2395                2400

Asp Gln Leu Ser Tyr Gln Glu Ala Val Phe Glu Asp Val Ala Val Gly
                2405                2410                2415

Thr Val Ile Leu Arg Val Thr Ala Thr Asp Ala Asp Ser Gly Asn Phe
            2420                2425                2430

Ala Leu Ile Glu Tyr Ser Leu Val Asp Gly Glu Gly Lys Phe Ala Ile
        2435                2440                2445

Asn Pro Asn Thr Gly Asp Ile Tyr Val Leu Ser Ser Leu Asp Arg Glu
    2450                2455                2460

Lys Lys Asp His Tyr Ile Leu Thr Ala Leu Ala Lys Asp Asn Pro Gly
2465                2470                2475                2480

Asp Val Ala Ser Asn Arg Arg Glu Asn Ser Val Gln Val Val Ile Arg
                2485                2490                2495

Val Leu Asp Val Asn Asp Cys Arg Pro Gln Phe Ser Lys Pro Gln Phe
            2500                2505                2510

Ser Thr Ser Val Tyr Glu Asn Glu Pro Ala Gly Thr Ser Val Ile Thr
        2515                2520                2525

Met Leu Ala Thr Asp Gln Asp Glu Gly Ser Asn Gly Gln Leu Thr Tyr

2530                    2535                    2540

Ser Leu Glu Gly Pro Gly Met Glu Ala Phe Ser Val Asp Met Asp Ser
2545                    2550                    2555                    2560

Gly Leu Val Thr Thr Gln Arg Pro Leu Gln Ser Tyr Glu Arg Phe Asn
                2565                    2570                    2575

Leu Thr Val Val Ala Thr Asp Gly Gly Glu Pro Pro Leu Trp Gly Thr
            2580                    2585                    2590

Thr Met Leu Leu Val Glu Val Ile Asp Val Asn Asp Asn Arg Pro Val
        2595                    2600                    2605

Phe Val Arg Pro Pro Asn Gly Thr Ile Leu His Ile Lys Glu Glu Ile
    2610                    2615                    2620

Pro Leu Arg Ser Asn Val Tyr Glu Val Tyr Ala Thr Asp Lys Asp Glu
2625                    2630                    2635                    2640

Gly Leu Asn Gly Ala Val Arg Tyr Ser Phe Leu Lys Ser Thr Gly Asn
                2645                    2650                    2655

Arg Asp Trp Glu Tyr Phe Thr Ile Asp Pro Ile Ser Gly Leu Ile Gln
            2660                    2665                    2670

Thr Ala Gln Arg Leu Asp Arg Glu Lys Gln Ala Val Tyr Ser Leu Ile
        2675                    2680                    2685

Leu Val Ala Ser Asp Leu Gly Gln Pro Val Pro Tyr Glu Thr Met Gln
    2690                    2695                    2700

Pro Leu Gln Val Ala Leu Glu Asp Ile Asp Asp Asn Glu Pro Leu Phe
2705                    2710                    2715                    2720

Val Arg Pro Pro Lys Gly Ser Pro Gln Tyr Gln Leu Leu Thr Val Pro
                2725                    2730                    2735

Glu His Ser Pro Arg Gly Thr Leu Val Gly Asn Val Thr Gly Ala Val
            2740                    2745                    2750

Asp Ala Asp Glu Gly Pro Asn Ala Ile Val Tyr Tyr Phe Ile Ala Ala
        2755                    2760                    2765

Gly Asn Glu Asp Lys Asn Phe His Leu Gln Pro Asp Gly Arg Leu Leu
    2770                    2775                    2780

Val Leu Arg Asp Leu Asp Arg Glu Thr Glu Ala Ile Phe Ser Phe Ile
2785                    2790                    2795                    2800

Val Lys Ala Ser Ser Asn Arg Ser Trp Thr Pro Pro Arg Gly Pro Ser
                2805                    2810                    2815

Pro Ala Leu Asp Leu Leu Thr Asp Leu Thr Leu Gln Glu Val Arg Val
            2820                    2825                    2830

Val Leu Glu Asp Ile Asn Asp Gln Pro Pro Arg Phe Thr Lys Ala Glu
        2835                    2840                    2845

Tyr Thr Ala Gly Val Ala Thr Asp Ala Lys Val Gly Ser Glu Leu Ile
    2850                    2855                    2860

Gln Val Leu Ala Leu Asp Ala Asp Ile Gly Asn Asn Ser Leu Val Phe
2865                    2870                    2875                    2880

Tyr Gly Ile Leu Ala Ile His Tyr Phe Arg Ala Leu Ala Asn Asp Ser
                2885                    2890                    2895

Glu Asp Val Gly Gln Val Phe Thr Met Gly Ser Val Asp Gly Ile Leu

2900                    2905                    2910

Arg Thr Phe Asp Leu Phe Met Ala Tyr Ser Pro Gly Tyr Phe Val Val
        2915                    2920                    2925

Asp Ile Val Ala Arg Asp Leu Ala Gly His Asn Asp Thr Ala Ile Ile
    2930                    2935                    2940

Gly Ile Tyr Ile Leu Arg Asp Asp Gln Arg Val Lys Ile Val Ile Asn
2945                    2950                    2955                    2960

Glu Ile Pro Asp Arg Val Arg Gly Phe Glu Glu Glu Phe Ile Arg Leu
                2965                    2970                    2975

Leu Ser Asn Ile Thr Gly Ala Ile Val Asn Thr Asp Asp Val Gln Phe
            2980                    2985                    2990

His Val Asp Met Lys Gly Arg Val Asn Phe Ala Gln Thr Glu Leu Leu
        2995                    3000                    3005

Ile His Val Val Asn Arg Asp Thr Asn Arg Ile Leu Asp Val Asp Arg
    3010                    3015                    3020

Val Ile Gln Met Ile Asp Glu Asn Lys Glu Gln Leu Arg Asn Leu Phe
3025                    3030                    3035                    3040

Arg Asn Tyr Asn Val Leu Asp Val Gln Pro Ala Ile Ser Val Gln Leu
                3045                    3050                    3055

Pro Asp Asp Met Ser Ala Leu Gln Met Ala Ile Ile Val Leu Ala Ile
            3060                    3065                    3070

Leu Leu Phe Leu Ala Ala Met Leu Phe Val Leu Met Asn Trp Tyr Tyr
        3075                    3080                    3085

Arg Thr Ile His Lys Arg Lys Leu Lys Ala Ile Val Ala Gly Ser Ala
    3090                    3095                    3100

Gly Asn Arg Gly Phe Ile Asp Ile Met Asp Met Pro Asn Thr Asn Lys
3105                    3110                    3115                    3120

Tyr Ser Phe Asp Gly Ala Asn Pro Val Trp Leu Asp Pro Phe Cys Arg
                3125                    3130                    3135

Asn Leu Glu Leu Ala Ala Gln Ala Glu His Glu Asp Asp Leu Pro Glu
            3140                    3145                    3150

Asn Leu Ser Glu Ile Ala Asp Leu Trp Asn Ser Pro Thr Arg Thr His
        3155                    3160                    3165

Gly Thr Phe Gly Arg Glu Pro Ala Ala Val Lys Pro Glu Asp Asp Arg
    3170                    3175                    3180

Tyr Leu Arg Ala Ala Ile Gln Glu Tyr Asp Asn Ile Ala Lys Leu Gly
3185                    3190                    3195                    3200

Gln Ile Ile Arg Glu Gly Pro Ile Lys Leu Ile His Thr Asp Leu Glu
                3205                    3210                    3215

Glu Glu Pro Gly Asp His Ser Pro Gly Gln Gly Ser Leu Arg Phe Arg
            3220                    3225                    3230

His Lys Pro Pro Thr Glu Leu Lys Gly Pro Asp Gly Ile His Ile Val
        3235                    3240                    3245

His Gly Ser Thr Gly Thr Leu Leu Ala Thr Asp Leu Asn Ser Leu Pro
    3250                    3255                    3260

Glu Asp Asp Gln Lys Gly Leu Asp Arg Ser Leu Glu Thr Leu Thr Ala

3265                3270                3275                3280

Ser Glu Ala Thr Ala Phe Glu Arg Asn Ala Arg Thr Glu Ser Ala Lys
                3285                3290                3295

Ser Thr Pro Leu His Lys Leu Arg Asp Val Ile Met Glu Ser Pro Leu
            3300                3305                3310

Glu Ile Thr Glu Leu
        3315


<210> 201
<211> 82
<212> PRT
<213> Homo sapiens

<400> 201
Val Ser Ala Thr Asp Ala Asp Ser Gly Glu Asn Gly Lys Val Thr Tyr
1               5                   10                  15

Ser Ile Leu Ser Gly Asn Asp Gly Gly Leu Phe Ser Ile Asp Pro Glu
            20                  25                  30

Thr Gly Ile Ile Thr Thr Thr Lys Pro Leu Asp Arg Glu Glu Gln Ser
        35                  40                  45

Glu Tyr Thr Leu Thr Val Glu Ala Thr Asp Gly Gly Gly Pro Pro Leu
    50                  55                  60

Ser Ser Thr Ala Thr Val Thr Val Thr Val Leu Asp Val Asn Asp Asn
65                  70                  75                  80

Ala Pro


<210> 202
<211> 92
<212> PRT
<213> Homo sapiens

<400> 202
Tyr Ser Ala Ser Val Pro Glu Asn Ala Pro Val Gly Thr Glu Val Leu
1               5                   10                  15

Thr Val Thr Ala Thr Asp Ala Asp Leu Gly Pro Asn Gly Arg Ile Phe
            20                  25                  30

Tyr Ser Ile Leu Gly Gly Gly Pro Gly Gly Trp Phe Arg Ile Asp Pro
        35                  40                  45

Asp Thr Gly Asp Leu Ser Thr Thr Lys Pro Leu Asp Arg Glu Ser Ile
    50                  55                  60

Gly Glu Tyr Glu Leu Thr Val Leu Ala Thr Asp Ser Gly Gly Pro Pro
65                  70                  75                  80

Leu Ser Gly Thr Thr Thr Val Thr Ile Thr Val Leu
                85                  90


<210> 203
<211> 442
<212> PRT
<213> Homo sapiens

<400> 203
Met His Tyr Cys Val Leu Ser Ala Phe Leu Ile Leu His Leu Val Thr

1               5                   10                  15

Val Ala Leu Ser Leu Ser Thr Cys Ser Thr Leu Asp Met Asp Gln Phe
            20                  25                  30

Met Arg Lys Arg Ile Glu Ala Ile Arg Gly Gln Ile Leu Ser Lys Leu
        35                  40                  45

Lys Leu Thr Ser Pro Pro Glu Asp Tyr Pro Glu Pro Glu Glu Val Pro
    50                  55                  60

Pro Glu Val Ile Ser Ile Tyr Asn Ser Thr Arg Asp Leu Leu Gln Glu
65                  70                  75                  80

Lys Ala Ser Arg Arg Ala Ala Ala Cys Glu Arg Glu Arg Ser Asp Glu
                85                  90                  95

Glu Tyr Tyr Ala Lys Glu Val Tyr Lys Ile Asp Met Pro Pro Phe Phe
            100                 105                 110

Pro Ser Glu Thr Val Cys Pro Val Val Thr Thr Pro Ser Gly Ser Val
        115                 120                 125

Gly Ser Leu Cys Ser Arg Gln Ser Gln Val Leu Cys Gly Tyr Leu Asp
    130                 135                 140

Ala Ile Pro Pro Thr Phe Tyr Arg Pro Tyr Phe Arg Ile Val Arg Phe
145                 150                 155                 160

Asp Val Ser Ala Met Glu Lys Asn Ala Ser Asn Leu Val Lys Ala Glu
                165                 170                 175

Phe Arg Val Phe Arg Leu Gln Asn Pro Lys Ala Arg Val Pro Glu Gln
            180                 185                 190

Arg Ile Glu Leu Tyr Gln Ile Leu Lys Ser Lys Asp Leu Thr Ser Pro
        195                 200                 205

Thr Gln Arg Tyr Ile Asp Ser Lys Val Val Lys Thr Arg Ala Glu Gly
    210                 215                 220

Glu Trp Leu Ser Phe Asp Val Thr Asp Ala Val His Glu Trp Leu His
225                 230                 235                 240

His Lys Asp Arg Asn Leu Gly Phe Lys Ile Ser Leu His Cys Pro Cys
                245                 250                 255

Cys Thr Phe Val Pro Ser Asn Asn Tyr Ile Ile Pro Asn Lys Ser Glu
            260                 265                 270

Glu Leu Glu Ala Arg Phe Ala Gly Ile Asp Gly Thr Ser Thr Tyr Thr
        275                 280                 285

Ser Gly Asp Gln Lys Thr Ile Lys Ser Thr Arg Lys Lys Asn Ser Gly
    290                 295                 300

Lys Thr Pro His Leu Leu Leu Met Leu Leu Pro Ser Tyr Arg Leu Glu
305                 310                 315                 320

Ser Gln Gln Thr Asn Arg Arg Lys Lys Arg Ala Leu Asp Ala Ala Tyr
                325                 330                 335

Cys Phe Arg Asn Val Gln Asp Asn Cys Cys Leu Arg Pro Leu Tyr Ile
            340                 345                 350

Asp Phe Lys Arg Asp Leu Gly Trp Lys Trp Ile His Glu Pro Lys Gly
        355                 360                 365

Tyr Asn Ala Asn Phe Cys Ala Gly Ala Cys Pro Tyr Leu Trp Ser Ser

370                 375                 380

Asp Thr Gln His Ser Arg Val Leu Ser Leu Tyr Asn Thr Ile Asn Pro
385                 390                 395                 400

Glu Ala Ser Ala Ser Pro Cys Cys Val Ser Gln Asp Leu Glu Pro Leu
                405                 410                 415

Thr Ile Leu Tyr Tyr Ile Gly Lys Thr Pro Lys Ile Glu Gln Leu Ser
            420                 425                 430

Asn Met Ile Val Lys Ser Cys Lys Cys Ser
        435                 440

<210> 204
<211> 414
<212> PRT
<213> Homo sapiens

<400> 204


<210> 205
<211> 435
<212> PRT
<213> Sus scrofa

<400> 205
Met His Tyr Cys Val Leu Ser Ala Phe Leu Leu Leu His Leu Val Thr
  1               5                  10                  15

Val Ala Leu Ser Leu Ser Thr Cys Ser Thr Leu Asp Met Asp Gln Phe
             20                  25                  30

Met Arg Lys Arg Ile Glu Ala Ile Arg Gly Gln Ile Leu Ser Lys Leu
         35                  40                  45

Lys Leu Thr Ser Pro Pro Glu Asp Tyr Pro Glu Pro Glu Glu Val Pro
     50                  55                  60

Pro Glu Val Ile Ser Ile Tyr Asn Ser Thr Arg Asp Leu Leu Gln Glu
 65                  70                  75                  80

Lys Ala Ser Arg Arg Ala Ala Ala Cys Glu Arg Glu Arg Ser Asp Glu
                 85                  90                  95

Glu Tyr Tyr Ala Lys Glu Val Tyr Lys Ile Asp Met Pro Pro Phe Phe
            100                 105                 110

Pro Ser Glu Asn Ala Ile Pro Pro Thr Phe Tyr Arg Pro Tyr Phe Arg
        115                 120                 125

Ile Val Arg Phe Asp Val Ser Ala Met Glu Lys Asn Ala Ser Asn Leu
    130                 135                 140

Val Lys Ala Glu Phe Arg Val Phe Arg Leu Gln Asn Pro Lys Ala Arg
145                 150                 155                 160

Val Ala Glu Gln Arg Ile Glu Leu Tyr Gln Ile Leu Lys Ser Lys Asp
                165                 170                 175

Leu Thr Ser Pro Thr Gln Arg Tyr Ile Asp Ser Lys Val Val Lys Thr
            180                 185                 190

Arg Ala Glu Gly Glu Trp Leu Ser Phe Asp Val Thr Asp Ala Val His
        195                 200                 205

Glu Trp Leu His His Lys Asp Arg Asn Leu Gly Phe Lys Ile Ser Leu
    210                 215                 220

His Cys Pro Cys Cys Thr Phe Val Pro Ser Asn Asn Tyr Ile Ile Pro
225                 230                 235                 240

Asn Lys Ser Glu Glu Leu Glu Ala Arg Phe Ala Gly Ile Asp Gly Thr
                245                 250                 255

Ser Thr Tyr Thr Ser Gly Asp Gln Lys Thr Ile Lys Ser Thr Arg Lys
            260                 265                 270

Lys Asn Ser Gly Lys Thr Pro His Leu Leu Leu Met Leu Leu Pro Ser
        275                 280                 285

Tyr Gly Leu Glu Ser Gln Gln Ser Asn Arg Arg Lys Lys Arg Ala Leu
    290                 295                 300

Asp Ala Ala Tyr Cys Phe Arg Asn Val Gln Asp Asn Cys Cys Leu Arg
305                 310                 315                 320

Pro Leu Tyr Ile Asp Phe Lys Arg Asp Leu Gly Trp Lys Trp Ile His
                325                 330                 335

Glu Pro Lys Gly Tyr Asn Ala Asn Phe Cys Ala Gly Ala Cys Pro Tyr
            340                 345                 350

Leu Trp Ser Ser Asp Thr Gln His Ser Arg Val Leu Ser Leu Tyr Asn
        355                 360                 365

Thr Ile Asn Pro Glu Ala Ser Ala Ser Pro Cys Cys Val Ser Gln Asp
    370                 375                 380

Leu Glu Pro Leu Thr Ile Leu Tyr Tyr Ile Gly Lys Thr Pro Lys Ile
385                 390                 395                 400

Glu Gln Leu Ser Asn Met Ile Val Lys Ser Cys Lys Cys Ser Lys Thr
                405                 410                 415

Lys Leu Ala Ala Phe Ala Arg Leu Tyr His Ser His Ser Asn Leu Gly
            420                 425                 430

Ser Glu Thr
        435


<210> 206
<211> 434
<212> PRT
<213> Mus musculus

<400> 206
His Tyr Cys Val Leu Ser Ala Phe Leu Leu Leu His Leu Val Thr Val
  1               5                  10                  15

Ala Leu Ser Leu Ser Thr Cys Ser Thr Leu Asp Met Asp Gln Phe Met
             20                  25                  30

Arg Lys Arg Ile Glu Ala Ile Arg Gly Gln Ile Leu Ser Lys Leu Lys
         35                  40                  45

Leu Thr Ser Pro Pro Glu Asp Tyr Pro Glu Pro Glu Glu Val Pro Pro

50                  55                  60

Glu Val Ile Ser Ile Tyr Asn Ser Thr Arg Asp Leu Leu Gln Glu Lys
65                  70                  75                  80

Ala Ser Arg Arg Ala Ala Ala Cys Glu Arg Glu Arg Ser Asp Glu Glu
                85                  90                  95

Tyr Tyr Ala Lys Glu Val Tyr Lys Ile Asp Met Pro Pro Phe Phe Pro
            100                 105                 110

Ser Glu Asn Ala Ile Pro Pro Thr Phe Tyr Arg Pro Tyr Phe Arg Ile
        115                 120                 125

Val Arg Phe Asp Val Ser Ala Met Glu Lys Asn Ala Ser Asn Leu Val
    130                 135                 140

Lys Ala Glu Phe Arg Val Phe Arg Leu Gln Asn Pro Lys Ala Arg Val
145                 150                 155                 160

Ala Glu Gln Arg Ile Glu Leu Tyr Gln Ile Leu Lys Ser Lys Asp Leu
                165                 170                 175

Thr Ser Pro Thr Gln Arg Tyr Ile Asp Ser Lys Val Val Lys Thr Arg
            180                 185                 190

Ala Glu Gly Glu Trp Leu Ser Phe Asp Val Thr Asp Ala Val His Glu
        195                 200                 205

Trp Leu His His Lys Asp Arg Asn Leu Gly Phe Lys Ile Ser Leu His
    210                 215                 220

Cys Pro Cys Cys Thr Phe Val Pro Ser Asn Asn Tyr Ile Ile Pro Asn
225                 230                 235                 240

Lys Ser Glu Glu Leu Glu Ala Arg Phe Ala Gly Ile Asp Gly Thr Ser
                245                 250                 255

Thr Tyr Thr Ser Gly Asp Gln Lys Thr Ile Lys Ser Thr Arg Lys Lys
            260                 265                 270

Asn Ser Gly Lys Thr Pro His Leu Leu Leu Met Leu Leu Pro Ser Tyr
        275                 280                 285

Gly Leu Glu Ser Gln Gln Ser Asn Arg Arg Lys Lys Arg Ala Leu Asp
    290                 295                 300

Ala Ala Tyr Cys Phe Arg Asn Val Gln Asp Asn Cys Cys Leu Arg Pro
305                 310                 315                 320

Leu Tyr Ile Asp Phe Lys Arg Asp Leu Gly Trp Lys Trp Ile His Glu
                325                 330                 335

Pro Lys Gly Tyr Asn Ala Asn Phe Cys Ala Gly Ala Cys Pro Tyr Leu
            340                 345                 350

Trp Ser Ser Asp Thr Gln His Ser Arg Val Leu Ser Leu Tyr Asn Thr
        355                 360                 365

Ile Asn Pro Glu Ala Ser Ala Ser Pro Cys Cys Val Ser Gln Asp Leu
    370                 375                 380

Glu Pro Leu Thr Ile Leu Tyr Tyr Ile Gly Lys Thr Pro Lys Ile Glu
385                 390                 395                 400

Gln Leu Ser Asn Met Ile Val Lys Ser Cys Lys Cys Ser Lys Thr Lys
                405                 410                 415

Leu Ala Ala Phe Ala Arg Leu Tyr His Ser His Ser Asn Leu Gly Ser

420                 425                 430

Glu Thr

<210> 207
<211> 414
<212> PRT
<213> Homo sapiens

<400> 207
Met His Tyr Cys Val Leu Ser Thr Phe Leu Leu Leu His Leu Val Pro
1               5                   10                  15

Val Ala Leu Ser Leu Ser Thr Cys Ser Thr Leu Asp Met Asp Gln Phe
            20                  25                  30

Met Arg Lys Arg Ile Glu Ala Ile Arg Gly Gln Ile Leu Ser Lys Leu
        35                  40                  45

Lys Leu Thr Ser Pro Pro Glu Asp Tyr Pro Glu Pro Asp Glu Val Pro
    50                  55                  60

Pro Glu Val Ile Ser Ile Tyr Asn Ser Thr Arg Asp Leu Leu Gln Glu
65                  70                  75                  80

Lys Ala Ser Arg Arg Ala Ala Ala Cys Glu Arg Glu Arg Ser Asp Glu
                85                  90                  95

Glu Tyr Tyr Ala Lys Glu Val Tyr Lys Ile Asp Met Pro Ser His Leu
            100                 105                 110

Pro Ser Glu Asn Ala Ile Pro Pro Thr Phe Tyr Arg Pro Tyr Phe Arg
        115                 120                 125

Ile Val Arg Phe Asp Val Ser Thr Met Glu Lys Asn Ala Ser Asn Leu
    130                 135                 140

Val Lys Ala Glu Phe Arg Val Phe Arg Leu Gln Asn Pro Lys Ala Arg
145                 150                 155                 160

Val Ala Glu Gln Arg Ile Glu Leu Tyr Gln Ile Leu Lys Ser Lys Asp
                165                 170                 175

Leu Thr Ser Pro Thr Gln Arg Tyr Ile Asp Ser Lys Val Val Lys Thr
            180                 185                 190

Arg Ala Glu Gly Glu Trp Leu Ser Phe Asp Val Thr Asp Ala Val Gln
        195                 200                 205

Glu Trp Leu His His Lys Asp Arg Asn Leu Gly Phe Lys Ile Ser Leu
    210                 215                 220

His Cys Pro Cys Cys Thr Phe Val Pro Ser Asn Asn Tyr Ile Ile Pro
225                 230                 235                 240

Asn Lys Ser Glu Glu Leu Glu Ala Arg Phe Ala Gly Ile Asp Gly Thr
                245                 250                 255

Ser Thr Tyr Ala Ser Gly Asp Gln Lys Thr Ile Lys Ser Thr Arg Lys
            260                 265                 270

Lys Thr Ser Gly Lys Thr Pro His Leu Leu Leu Met Leu Leu Pro Ser
        275                 280                 285

Tyr Arg Leu Glu Ser Gln Gln Ser Ser Arg Arg Lys Lys Arg Ala Leu
    290                 295                 300

Asp Ala Ala Tyr Cys Phe Arg Asn Val Gln Asp Asn Cys Cys Leu Arg
305                 310                 315                 320

Pro Leu Tyr Ile Asp Phe Lys Arg Asp Leu Gly Trp Lys Trp Ile His
                325                 330                 335

Glu Pro Lys Gly Tyr Asn Ala Asn Phe Cys Ala Gly Ala Cys Pro Tyr
            340                 345                 350

Leu Trp Ser Ser Asp Thr Gln His Thr Lys Val Leu Ser Leu Tyr Asn
        355                 360                 365

Thr Ile Asn Pro Glu Ala Ser Ala Ser Pro Cys Cys Val Ser Gln Asp
    370                 375                 380

Leu Glu Pro Leu Thr Ile Leu Tyr Tyr Ile Gly Asn Thr Pro Lys Ile
385                 390                 395                 400

Glu Gln Leu Ser Asn Met Ile Val Lys Ser Cys Lys Cys Ser
                405                 410

<210> 208
<211> 227
<212> PRT
<213> Homo sapiens

<400> 208
Cys Arg Pro Leu Asp Leu Arg Arg Ser Gln Lys Gln Asp Arg Leu Glu
1               5                   10                  15

Ala Ile Glu Gly Gln Ile Leu Ser Lys Leu Gly Leu Arg Arg Arg Pro
            20                  25                  30

Arg Pro Ser Lys Glu Pro Met Val Val Pro Glu Tyr Met Leu Asp Leu
        35                  40                  45

Tyr Asn Ala Leu Ser Glu Leu Glu Glu Gly Lys Val Gly Arg Val Pro
    50                  55                  60

Glu Ile Ser Asp Tyr Asp Gly Arg Glu Ala Gly Arg Ala Asn Thr Ile
65                  70                  75                  80

Arg Ser Phe Ser His Leu Glu Ser Asp Asp Phe Glu Glu Ser Thr Pro
                85                  90                  95

Glu Ser His Arg Lys Arg Phe Arg Phe Asn Leu Ser Ser Ile Pro Glu
            100                 105                 110

Gly Glu Thr Leu Thr Ala Ala Glu Leu Arg Leu Tyr Arg Asp Pro Leu
        115                 120                 125

Ala Leu Arg Ser Arg Ala Thr Val Arg Val Glu Ile Tyr Gln Leu Leu
    130                 135                 140

Lys Pro Gly Ser Asp Gly Ser Pro Asp Thr Arg Leu Leu Asp Ser Arg
145                 150                 155                 160

Leu Val Asp Ala Arg Asp Ser Gly Gly Trp Leu Ser Phe Asp Val Thr
                165                 170                 175

Ser Ala Val Asn Arg Trp Leu Ser Asn Pro Glu Ser Asn Leu Gly Leu
            180                 185                 190

Gln Leu Glu Val Glu Cys Leu Cys Gly His Val Arg Pro Ser Arg Ala
        195                 200                 205

Gly Leu Ile Gly Glu Pro Gly Pro Glu Gln Leu Gln Pro Leu Leu Val
    210                 215                 220

Thr Phe Phe
225

<210> 209
<211> 105
<212> PRT
<213> Homo sapiens

<400> 209
Ala Arg Pro Cys Arg Leu Arg Ser Leu Tyr Val Asp Phe Arg Asp Leu
1               5                   10                  15

Gly Trp Gly Asp Trp Ile Ile Ala Pro Glu Gly Tyr Ile Ala Asn Tyr
            20                  25                  30

Cys Ser Gly Ser Cys Pro Phe Pro Leu Arg Asp Asp Leu Asn Leu Ser
        35                  40                  45

Asn His Ala Ile Leu Gln Thr Leu Val Arg Leu Arg Asn Pro Arg Ala
    50                  55                  60

Val Pro Gln Pro Cys Cys Val Pro Thr Lys Leu Ser Pro Leu Ser Met
65                  70                  75                  80

Leu Tyr Leu Asp Asp Asn Ser Asn Val Val Leu Arg Leu Tyr Pro Asn
                85                  90                  95

Met Ser Val Lys Glu Cys Gly Cys Arg
            100                 105

<210> 210
<211> 102
<212> PRT
<213> Homo sapiens

<400> 210
Cys Arg Arg His Asp Leu Tyr Val Asp Phe Lys Asp Leu Gly Trp Asp
1               5                   10                  15

Asp Trp Ile Ile Ala Pro Lys Gly Tyr Asn Ala Tyr Tyr Cys Glu Gly
            20                  25                  30

Glu Cys Pro Phe Pro Leu Ser Glu Arg Leu Asn Ala Thr Asn His Ala
        35                  40                  45

Ile Val Gln Ser Leu Val His Ala Leu Asp Pro Gly Ala Val Pro Lys
    50                  55                  60

Pro Cys Cys Val Pro Thr Lys Leu Ser Pro Leu Ser Met Leu Tyr Tyr
65                  70                  75                  80

Asp Asp Asp Gly Asn Val Val Leu Arg Asn Tyr Pro Asn Met Val Val
                85                  90                  95

Glu Glu Cys Gly Cys Arg
            100

<210> 211
<211> 470
<212> PRT
<213> Gallus gallus

<400> 211
Met Arg Phe Ile Leu Leu Pro Cys Leu Trp Ala Ser Leu Ala Gly Val
1               5                   10                  15

Leu Leu Ala Glu Asp Val Asp Thr Ser Thr Ala Glu Val Arg Leu Val
            20                  25                  30

Asp Gly Pro Asn Arg Cys Ser Gly Arg Val Glu Val Leu His Asn Asp
        35                  40                  45

Val Trp Gly Thr Val Cys Asp Glu Gly Trp Asp Leu Arg Glu Ala Arg
    50                  55                  60

Val Val Cys Arg Gln Leu Gly Cys Gly Thr Ala Leu Ser Ser Pro Lys
65                  70                  75                  80

Lys Ser Lys Tyr Gly Glu Gly Lys Gly Gln Ile Trp Leu Ser Asp Leu
                85                  90                  95

Asp Cys Lys Gly Thr Glu Gly Ser Leu Ser Asn Cys Lys Ser Lys Pro
            100                 105                 110

Trp Gly Glu Asn Ile Cys Asn His Val Glu Asp Ala Ser Val Glu Cys
        115                 120                 125

Ser Gly Thr Glu Ile Pro Glu Pro Gly Pro Leu Arg Leu Val Gly Gly
    130                 135                 140

Pro Asn Arg Cys Ala Gly Arg Val Glu Val Leu His Glu Glu Gln Trp
145                 150                 155                 160

Gly Ser Val Cys His Asp Glu Trp Asp Ile Asn Asp Ala Gln Val Val
                165                 170                 175

Cys Lys Gln Leu Gly Cys Gly Asp Ala Val Leu Ala Pro Ile Ala Ala
            180                 185                 190

Lys Phe Gly Arg Gly Thr Asp Thr Ile Trp Leu Asp Asp Val Asn Cys
        195                 200                 205

Thr Gly Ser Glu Ala Ser Leu Ser Glu Cys Gln Ala Arg Pro Trp Gly
    210                 215                 220

Asp His Asn Cys Tyr His Gly Glu Asp Ala Ser Ala Ile Cys Ser Asp
225                 230                 235                 240

Ser Gly Ile Ser Ile Ser Thr Ser Val Arg Leu Val Gly Gly Pro Asn
                245                 250                 255

Arg Cys Ser Gly Arg Val Glu Val Leu His Asn Asn Val Trp Gly Thr
            260                 265                 270

Val Cys Asp Asp Asn Trp Asp Leu Arg Glu Ala Lys Val Val Cys Lys
        275                 280                 285

Gln Leu Gly Cys Gly Thr Ala Leu Ser Ala Leu Pro Glu Ser Lys Tyr
    290                 295                 300

Gly Glu Gly Lys Gly Gln Ile Trp Leu Ser Asp Leu Asn Cys Thr Gly
305                 310                 315                 320

Thr Glu Gly Ser Leu Thr Glu Cys Glu Ala Lys Pro Trp Gly Glu Asn
                325                 330                 335

Val Cys Asn His Val Glu Asp Ala Ser Val Glu Cys Ser Glu Thr Asp
            340                 345                 350

Ile Ser Glu Ile Gly Pro Val Arg Leu Val Asp Gly Pro Asn Gln Cys
        355                 360                 365

Ala Gly Arg Val Glu Val Phe His Glu Asn Arg Trp Gly Ser Val Cys
    370                 375                 380

Asp Asp Asn Trp Asp Met Lys Asp Ala Lys Val Val Cys Lys Gln Val
385                 390                 395                 400

Gly Cys Gly Ser Pro Leu Ser Ala Leu Gly Ser Ala Arg Tyr Gly Arg
                405                 410                 415

Gly Pro Asp Val Ile Trp Leu Asp Asp Val Asn Cys Glu Gly Thr Glu
            420                 425                 430

Glu Ser Ile Phe Asp Cys Lys Ala Arg Pro Trp Gly Glu His Asn Cys
        435                 440                 445

Tyr His Gly Glu Asp Ala Ser Val Phe Cys Thr Val Asn Lys Asn Leu
    450                 455                 460

Glu Glu Thr Glu Thr Ser
465                 470

<210> 212
<211> 1594
<212> PRT
<213> Orycctolagus cuniculus

<400> 212
Met Gly Ile Ser Thr Val Leu Ala Leu Ser Leu Leu Trp Gly Pro Ala
1               5                   10                  15

Leu Ser Gln Gly Gln Trp Ile Pro Tyr Thr Thr Tyr His Asp Ser Val
            20                  25                  30

Ser Ser Pro Gly Ala Pro Val Glu Thr Thr Thr Thr Glu Val Phe Pro
        35                  40                  45

Ser Gly Leu Glu Leu Arg Leu Ala Asn Gly Gly Asp Arg Cys Gln Gly
    50                  55                  60

Arg Val Glu Val Leu Tyr Gln Gly Ser Trp Gly Thr Val Cys Asp Asp
65                  70                  75                  80

Gly Trp Asp Ile Asn Asp Ala Gln Val Val Cys Arg Gln Leu Gly Cys
                85                  90                  95

Gly Met Ala Val Ser Ala Pro Gly Ser Ala Arg Phe Gly Gln Gly Pro
            100                 105                 110

Gly Gln Ile Val Leu Asp Asp Val Ser Cys Ser Gly Gln Glu Pro Tyr
        115                 120                 125

Leu Trp Ser Cys His His Arg Gly Trp Leu Ser His Asn Cys Gly His
    130                 135                 140

Gln Glu Asp Ala Gly Val Ile Cys Ser Asp Ala Met Ala Met Thr Ser
145                 150                 155                 160

Pro Pro Pro Asp Thr Trp Pro Thr Thr Val Ile Tyr Glu Ser Thr Pro
                165                 170                 175

His Phe Pro Ser Gly Leu Glu Leu Val Phe Pro Ser Gly Leu Glu Leu
            180                 185                 190

Arg Leu Ala Asn Gly Ser Asp Arg Cys Gln Gly Arg Val Glu Val Leu
        195                 200                 205

Tyr Gln Gly Ser Trp Gly Thr Val Cys Asp Asp Gly Trp Asp Ile Asn
    210                 215                 220

Asp Ala Gln Val Val Cys Arg Gln Leu Gly Cys Gly Met Ala Val Ser

225                 230                 235                 240

Ala Pro Gly Ser Ala Arg Phe Gly Gln Gly Pro Gly Gln Ile Val Leu
                245                 250                 255

Asp Asp Val Ser Cys Ser Gly Gln Glu Pro Tyr Leu Trp Ser Cys His
            260                 265                 270

His Arg Gly Trp Leu Ser His Asn Cys Gly His Gln Glu Asp Ala Gly
        275                 280                 285

Val Ile Cys Ser Asp Ala Val Pro Thr Thr Thr Pro Pro Pro Asp Thr
    290                 295                 300

Trp Pro Thr Thr Val Ile Tyr Glu Ser Ser Pro Val Phe Pro Ser Gly
305                 310                 315                 320

Leu Glu Leu Arg Leu Ala Asn Gly Ser Asp Arg Cys Gln Gly Arg Val
                325                 330                 335

Glu Val Leu Tyr Gln Gly Ser Trp Gly Thr Val Cys Asp Asp Gly Trp
            340                 345                 350

Asp Ile Asn Asp Ala Gln Val Val Cys Arg Gln Leu Gly Cys Gly Thr
        355                 360                 365

Ala Val Ser Ala Pro Gly Ser Ala Arg Phe Gly Gln Gly Pro Gly Gln
    370                 375                 380

Ile Val Leu Asp Asp Val Ser Cys Ser Gly Gln Glu Pro Tyr Leu Trp
385                 390                 395                 400

Ser Cys His His Arg Gly Trp Leu Ser His Asn Cys Gly His Gln Glu
                405                 410                 415

Asp Ala Gly Val Ile Cys Ser Asp Ala Met Ala Met Thr Thr Pro Leu
            420                 425                 430

Pro Asp Thr Trp Pro Thr Thr Val Ile His Glu Ser Thr Pro Val Phe
        435                 440                 445

Pro Ser Gly Leu Glu Leu Gln Leu Ala Asn Gly Ser Asp Arg Cys Gln
    450                 455                 460

Gly Arg Val Glu Val Leu Tyr Gln Gly Thr Val Cys Asp Asp Gly Trp
465                 470                 475                 480

Asp Ile Asn Asp Ala Gln Val Val Cys Arg Gln Leu Gly Cys Gly Met
                485                 490                 495

Ala Val Ser Ala Pro Gly Ser Ala Arg Phe Gly Gln Gly Pro Gly Gln
            500                 505                 510

Ile Val Leu Asp Asp Val Ser Cys Ser Gly Gln Glu Pro Tyr Leu Trp
        515                 520                 525

Ser Cys His His Arg Gly Trp Leu Ser His Asn Cys Gly His Gln Glu
    530                 535                 540

Asp Ala Gly Val Ile Cys Ser Asp Ala Met Ala Met Thr Thr Pro Pro
545                 550                 555                 560

Pro Asp Thr Trp Pro Thr Thr Val Ile Tyr Glu Ser Thr Pro His Phe
                565                 570                 575

Pro Ser Gly Leu Glu Leu Arg Leu Ala Asn Gly Ser Asp Arg Cys Gln
            580                 585                 590

Gly Arg Val Glu Val Leu Tyr Gln Gly Ser Trp Gly Thr Val Cys Asp

        595                 600                 605

Asp Gly Trp Asp Ile Asn Asp Ala Gln Val Val Cys Arg Gln Leu Gly
    610                 615                 620

Cys Gly Thr Ala Val Ser Ala Pro Gly Ser Ala Arg Phe Gly Gln Gly
625                 630                 635                 640

Pro Gly Gln Ile Val Leu Asp Asp Val Ser Cys Ser Gly Gln Glu Pro
                645                 650                 655

Tyr Leu Trp Ser Cys His His Arg Gly Trp Leu Ser His Asn Cys Gly
            660                 665                 670

His Gln Glu Asp Ala Gly Val Ile Cys Ser Gly Ala Met Asp Thr Thr
        675                 680                 685

Thr Pro Leu Pro Asp Thr Trp Pro Thr Thr Val Ile Tyr Glu Ser Thr
    690                 695                 700

Pro Val His Ile Ser Gly Leu Gln Leu Arg Leu Val Asn Gly Ser Asp
705                 710                 715                 720

Arg Cys Glu Gly Arg Val Glu Val Leu Tyr Gln Gly Ser Trp Gly Thr
                725                 730                 735

Val Cys Asp Asp Ser Trp Asp Leu Asn Asp Ala Ser Val Val Cys Arg
            740                 745                 750

Gln Leu Gly Cys Gly Thr Ala Leu Ser Ala Pro Ala Ser Ala Gln Phe
        755                 760                 765

Gly Gln Ser Ser Gly Ser Ile Val Leu Asp Asp Val Ser Cys Ser Gly
    770                 775                 780

Ser Glu Pro Asn Leu Trp Ser Cys Ser His Arg Gly Trp Leu Ser His
785                 790                 795                 800

Asn Cys Gly His His Glu Asp Ala Gly Val Val Cys Ser Gly Pro Asp
                805                 810                 815

Ser Arg Leu Ala Val Arg Leu Val Asn Gly Ser Thr Arg Cys Gln Gly
            820                 825                 830

Arg Val Glu Val Leu Tyr Arg Gly Ser Trp Gly Thr Val Cys Asp Asp
        835                 840                 845

Ser Trp Asp Ile Asn Asp Ala Ser Val Val Cys Arg Gln Leu Gly Cys
    850                 855                 860

Gly Trp Ala Val Ser Ala Pro Gly Ser Ala Arg Phe Gly Gln Gly Ser
865                 870                 875                 880

Gly Ser Ile Phe Leu Asp Glu Val Ser Cys Ser Gly Gln Glu Pro Tyr
                885                 890                 895

Leu Trp Asn Cys Ser His Arg Gly Trp Leu Ser His Asn Cys Gly His
            900                 905                 910

Tyr Glu Asp Ala Gly Val Ile Cys Ser Asp Gly Trp Thr Thr Val Thr
        915                 920                 925

Pro Pro Ala Pro Thr Thr Asp Trp Trp Glu Pro Thr Val Thr Thr Thr
    930                 935                 940

Val Gly Pro Ser Ser Asn Cys Gly Gly Phe Leu Tyr Asn Ala Thr Gly
945                 950                 955                 960

Ser Phe Ser Ser Pro Ser Tyr Pro Gly Tyr Tyr Pro Asn Asn Ala Leu

                965                 970                 975
Cys Val Trp Glu Ile Ala Val Pro Ser Gly Tyr Leu Ile Asn Leu Gly
            980                 985                 990
Phe Ser Gln Leu Arg Leu Glu Gln His Ser Tyr Cys Asn Phe Asp Tyr
        995                 1000                1005
Val Glu Ile Phe Asp Gly Ser Thr Asp Ser Ser Leu Leu Gly Lys Ile
    1010                1015                1020
Cys Asn Asp Ser Gly Gln Ile Phe Thr Thr Ser Ser Asn Arg Met Thr
1025                1030                1035                1040
Val Leu Phe Arg Ser Asp Ile Ser Val Gln Asn Thr Gly Phe Leu Ala
                1045                1050                1055
Trp Tyr Asn Ser Phe Pro Arg Asp Ala Ser Leu Arg Leu Val Ser Gly
            1060                1065                1070
Asn Ser Ser Tyr Gly Ala Cys Ala Gly Arg Val Glu Ile Tyr His Gly
        1075                1080                1085
Gly Arg Trp Gly Thr Val Cys Asp Asp Ser Trp Asp Thr Gln Asp Ala
    1090                1095                1100
Gln Val Val Cys Arg Gln Leu Gln Cys Gly Asp Ala Val Ser Ala Pro
1105                1110                1115                1120
Gly Gly Ala Tyr Phe Gly Ser Gly Ser Gly Pro Ile Thr Leu Asp Asp
                1125                1130                1135
Val Asn Cys Ser Gly Thr Glu Ala Thr Leu Trp Gln Cys Arg Ser Gln
            1140                1145                1150
Ser Trp Phe Ser His Asn Cys Gly His His Glu Asp Ala Ser Val Ile
        1155                1160                1165
Cys Thr Gly Asn Tyr Gly Thr Thr Thr Ala Ser Val Pro Asn Ile Ser
    1170                1175                1180
Thr Ser Asn Ala Ser Tyr Ser Cys Gly Gly Phe Leu Ser Gln His Ser
1185                1190                1195                1200
Gly Arg Phe Ser Ser Pro Phe Tyr Pro Gly Asn Tyr Pro Asn Asn Ala
                1205                1210                1215
Arg Cys Val Trp Asp Ile Glu Val Gln Asn Asn Tyr Gln Val Thr Val
            1220                1225                1230
Thr Phe Thr Asp Val Gln Leu Glu Gly Gly Cys Gln Tyr Asp Tyr Ile
        1235                1240                1245
Glu Val Phe Asp Gly Pro Tyr His Ser Ser Pro Leu Ile Ala Arg Val
    1250                1255                1260
Cys Asp Gly Ala Arg Gly Ser Phe Thr Ser Ser Ser Asn Phe Leu Ser
1265                1270                1275                1280
Val Arg Phe Val Ser Asp Gly Ser Ile Thr Arg Arg Gly Phe Gln Ala
                1285                1290                1295
Glu Phe Tyr Ser Leu Pro Ser Asn Asp Ser Thr Asn Leu Leu Cys Leu
            1300                1305                1310
Met Asn His Met Gln Ala Ser Val Ser Arg Ala Tyr Leu Gln Ser Leu
        1315                1320                1325
Gly Phe Ser Ala Trp Glu Leu Val Val Ser Gly Trp Asn Gly Asn Tyr

1330                    1335                    1340

Gln Cys Gln Arg Gln Ile Thr Pro Ser Gln Val Ile Phe Thr Ile Pro
1345                1350                1355                1360

Tyr Ser Gly Cys Gly Thr Ile Lys Gln Val Asp Asn Glu Thr Ile Thr
                1365                1370                1375

Tyr Ser Asn Phe Leu Lys Ala Ala Val Ser Ser Gly Val Ile Lys Arg
            1380                1385                1390

Lys Lys Asp Leu His Ile His Val Ser Cys Arg Met Leu Gln Asp Ser
        1395                1400                1405

Trp Val His Thr Met Tyr Ile Ala Asn Asp Thr Ile Glu Val Ser Glu
    1410                1415                1420

Val Gln Tyr Ser Asn Phe Asn Val Asn Val Ser Phe Tyr Thr Ser Ser
1425                1430                1435                1440

Ser Phe Ser Tyr Pro Val Thr Ser Ser Pro Tyr Tyr Val Asp Leu Asp
                1445                1450                1455

Gln Asn Leu Tyr Leu Gln Ala Glu Ile Leu His Ser Asp Ala Ser Leu
            1460                1465                1470

Ala Leu Phe Val Asp Thr Cys Val Ala Ser Pro Asn Pro Asn Asp Phe
        1475                1480                1485

Thr Ser Val Thr Tyr Asp Leu Ile Arg Ser Gly Cys Val Arg Asp Glu
    1490                1495                1500

Thr Tyr Arg Ser Tyr Ala Gln Pro Ser Pro Arg Val Val Arg Phe Arg
1505                1510                1515                1520

Phe Asn Ser Phe His Phe Leu Asn Arg Phe Pro Ala Val Tyr Leu Arg
                1525                1530                1535

Cys Lys Met Val Val Cys Arg Ala Tyr Asp Tyr Ser Ser Arg Cys Tyr
            1540                1545                1550

Arg Gly Cys Val Val Arg Ser Lys Arg Asp Val Gly Ser Tyr Gln Glu
        1555                1560                1565

Arg Val Asp Val Val Leu Gly Pro Ile Gln Leu Leu Asp Pro Pro Ala
    1570                1575                1580

Gly Lys Lys Ser Pro Gly Lys Gly Ser Pro
1585                1590


<210> 213
<211> 575
<212> PRT
<213> Homo sapiens

<400> 213
Met His Lys Glu Ala Glu Met Leu Ile Gly Pro Gln Leu Asp Glu Lys
1               5                   10                  15

Arg Trp Gly Trp Arg Leu Gly Asp Gly Ser Ala Ala Pro Pro Phe Leu
            20                  25                  30

Pro Gln Ala Leu Ser Phe Leu Leu Leu Leu Pro Leu Ala Ser Ala Leu
        35                  40                  45

Gln Pro Thr Pro Leu Pro Phe Gln Glu Leu Arg Leu Val Gly Gly Pro
    50                  55                  60

Ser Arg Cys Arg Gly Arg Leu Glu Val Met His Gly Gly Ser Trp Gly
65                  70                  75                  80

Ser Val Cys Asp Asp Asp Trp Asp Val Val Asp Ala Asn Val Val Cys
                85                  90                  95

Arg Gln Leu Gly Cys Gly Leu Ala Leu Pro Val Pro Arg Pro Leu Ala
            100                 105                 110

Phe Gly Gln Gly Arg Gly Pro Ile Leu Leu Asp Asn Val Glu Cys Arg
        115                 120                 125

Gly Gln Glu Ala Ala Leu Ser Glu Cys Gly Ser Arg Gly Trp Gly Val
    130                 135                 140

His Asn Cys Phe His Tyr Glu Asp Val Ala Val Leu Cys Asp Glu Phe
145                 150                 155                 160

Leu Pro Thr Gln Pro Pro Thr Arg Lys Met Leu Thr Ser Arg Ala Pro
                165                 170                 175

Pro Thr Thr Leu Pro Asn Gly Lys Ser Glu Gly Ser Val Arg Leu Val
            180                 185                 190

Gly Gly Ala Asn Leu Cys Gln Gly Arg Val Glu Ile Leu His Ser Gly
        195                 200                 205

Leu Trp Gly Thr Val Cys Asp Asp Asp Trp Gly Leu Pro Asp Ala Ala
    210                 215                 220

Val Val Cys Arg Gln Leu Gly Cys Gly Ala Ala Met Ala Ala Thr Thr
225                 230                 235                 240

Asn Ala Phe Phe Gly Tyr Gly Thr Gly His Ile Leu Leu Asp Asn Val
                245                 250                 255

His Cys Glu Gly Gly Glu Pro Arg Leu Ala Ala Cys Gln Ser Leu Gly
            260                 265                 270

Trp Gly Val His Asn Cys Gly His His Glu Asp Ala Gly Ala Leu Cys
        275                 280                 285

Ala Gly Leu Gly Pro Pro Thr Leu Thr Ala Leu Pro Ser Ser Ala Thr
    290                 295                 300

Arg Glu Asp Trp Ala Trp Gln Thr Asp Pro Ser Ala Thr Gly Val Gly
305                 310                 315                 320

Pro Gln Pro Ser Arg Glu Thr Ala Leu Leu Thr Thr Ala Ala Trp Ala
                325                 330                 335

Ala Gly Lys Lys Ser Gly Arg Leu Arg Leu Val Gly Gly Pro Gly Pro
            340                 345                 350

Cys Arg Gly Arg Val Glu Val Leu His Ala Gly Gly Trp Gly Thr Val
        355                 360                 365

Cys Asp Asp Asp Trp Asp Phe Ala Asp Ala Arg Val Ala Cys Arg Glu
    370                 375                 380

Ala Gly Cys Gly Pro Ala Leu Gly Ala Thr Gly Leu Gly His Phe Gly
385                 390                 395                 400

Tyr Gly Arg Gly Pro Val Leu Leu Asp Asn Val Gly Cys Ala Gly Thr
                405                 410                 415

Glu Ala Arg Leu Ser Asp Cys Phe His Leu Gly Trp Gly Gln His Asn
            420                 425                 430

Cys Gly His His Glu Asp Ala Gly Ala Leu Cys Ala Gly Pro Glu Glu
        435                 440                 445

Leu Gly Leu Gln Val Gln Gln Asp Gly Ser Glu Thr Thr Arg Val Pro
    450                 455                 460

Thr Pro Arg Pro Arg Asp Gly His Leu Arg Leu Val Asn Gly Ala His
465                 470                 475                 480

Arg Cys Glu Gly Arg Val Glu Leu Tyr Leu Gly Gln Arg Trp Gly Thr
                485                 490                 495

Val Cys Asp Asp Ala Trp Asp Leu Arg Ala Ala Gly Val Leu Cys Arg
            500                 505                 510

Gln Leu Gly Cys Gly Gln Ala Leu Ala Ala Pro Gly Glu Ala His Phe
        515                 520                 525

Gly Pro Gly Arg Gly Pro Ile Leu Leu Asp Asn Val Lys Cys Arg Gly
    530                 535                 540

Glu Glu Ser Ala Leu Leu Leu Cys Ser His Ile Arg Trp Asp Ala His
545                 550                 555                 560

Asn Cys Asp His Ser Glu Asp Ala Ser Val Leu Cys Gln Pro Ser
                565                 570                 575


<210> 214
<211> 1051
<212> PRT
<213> Homo sapiens

<400> 214
Met Asp Glu Ala Phe Leu Cys Ser Pro Val Gly Ser Glu Ser Ser Leu
1               5                   10                  15

Ala Leu Arg Leu Val Asn Gly Gly Asp Arg Cys Gln Gly Arg Val Glu
            20                  25                  30

Val Leu Tyr Arg Gly Ser Trp Gly Thr Val Cys Asp Asp Tyr Trp Asp
        35                  40                  45

Thr Asn Asp Ala Asn Val Val Cys Arg Gln Leu Gly Cys Gly Trp Ala
    50                  55                  60

Thr Ser Ala Pro Gly Asn Ala Arg Phe Gly Gln Gly Ser Gly Pro Ile
65                  70                  75                  80

Val Leu Asp Asp Val Arg Cys Ser Gly His Glu Ser Tyr Leu Trp Ser
                85                  90                  95

Cys Pro His Asn Gly Trp Leu Ser His Asn Cys Gly His His Glu Asp
            100                 105                 110

Ala Gly Val Ile Cys Ser Ala Ser Gln Ser Gln Pro Thr Pro Ser Pro
        115                 120                 125

Asp Thr Trp Pro Thr Ser Arg Ala Ser Thr Ala Gly Ser Glu Ser Thr
    130                 135                 140

Leu Ala Leu Arg Leu Val Asn Gly Gly Asp Arg Cys Arg Gly Arg Val
145                 150                 155                 160

Glu Val Leu Tyr Gln Gly Ser Trp Gly Thr Val Cys Asp Asp Tyr Trp
                165                 170                 175

Asp Thr Asn Asp Ala Asn Val Val Cys Arg Gln Leu Gly Cys Gly Trp
            180                 185                 190

Ala Met Ser Ala Pro Gly Asn Ala Gln Phe Gly Gln Gly Ser Gly Pro
        195                 200                 205

Ile Val Leu Asp Asp Val Arg Cys Ser Gly His Glu Ser Tyr Leu Trp
    210                 215                 220

Ser Cys Pro His Asn Gly Trp Leu Ser His Asn Cys Gly His His Glu
225                 230                 235                 240

Asp Ala Gly Val Ile Cys Ser Ala Ala Gln Ser Gln Ser Thr Pro Arg
                245                 250                 255

Pro Asp Thr Trp Leu Thr Thr Asn Leu Pro Ala Leu Thr Val Gly Ser
            260                 265                 270

Glu Ser Ser Leu Ala Leu Arg Leu Val Asn Gly Gly Asp Arg Cys Arg
        275                 280                 285

Gly Arg Val Glu Val Leu Tyr Arg Gly Ser Trp Gly Thr Val Cys Asp
    290                 295                 300

Asp Ser Trp Asp Thr Asn Asp Ala Asn Val Val Cys Arg Gln Leu Gly
305                 310                 315                 320

Cys Gly Trp Ala Met Ser Ala Pro Gly Asn Ala Arg Phe Gly Gln Gly
                325                 330                 335

Ser Gly Pro Ile Val Leu Asp Asp Val Arg Cys Ser Gly Asn Glu Ser
            340                 345                 350

Tyr Leu Trp Ser Cys Pro His Lys Gly Trp Leu Thr His Asn Cys Gly
        355                 360                 365

His His Glu Asp Ala Gly Val Ile Cys Ser Ala Thr Gln Ile Asn Ser
    370                 375                 380

Thr Thr Thr Asp Trp Trp His Pro Thr Thr Thr Thr Thr Ala Arg Pro
385                 390                 395                 400

Ser Ser Asn Cys Gly Gly Phe Leu Phe Tyr Ala Ser Gly Thr Phe Ser
                405                 410                 415

Ser Pro Ser Tyr Pro Ala Tyr Tyr Pro Asn Asn Ala Lys Cys Val Trp
            420                 425                 430

Glu Ile Glu Val Asn Ser Gly Tyr Arg Ile Asn Leu Gly Phe Ser Asn
        435                 440                 445

Leu Lys Leu Glu Ala His His Asn Cys Ser Phe Asp Tyr Val Glu Ile
    450                 455                 460

Phe Asp Gly Ser Leu Asn Ser Ser Leu Leu Leu Gly Lys Ile Cys Asn
465                 470                 475                 480

Asp Thr Arg Gln Ile Phe Thr Ser Ser Tyr Asn Arg Met Thr Ile His
                485                 490                 495

Phe Arg Ser Asp Ile Ser Phe Gln Asn Thr Gly Phe Leu Ala Trp Tyr
            500                 505                 510

Asn Ser Phe Pro Ser Asp Ala Thr Leu Arg Leu Val Asn Leu Asn Ser
        515                 520                 525

Ser Tyr Gly Leu Cys Ala Gly Arg Val Glu Ile Tyr His Gly Gly Thr
    530                 535                 540

Trp Gly Thr Val Cys Asp Asp Ser Trp Thr Ile Gln Glu Ala Glu Val
545                 550                 555                 560

Val Cys Arg Gln Leu Gly Cys Gly Arg Ala Val Ser Ala Leu Gly Asn
                565                 570                 575

Ala Tyr Phe Gly Ser Gly Ser Gly Pro Ile Thr Leu Asp Asp Val Glu
            580                 585                 590

Cys Ser Gly Thr Glu Ser Thr Leu Trp Gln Cys Arg Asn Arg Gly Trp
        595                 600                 605

Phe Ser His Asn Cys Asn His Arg Glu Asp Ala Gly Val Ile Cys Ser
    610                 615                 620

Gly Asn His Leu Ser Thr Pro Ala Pro Phe Leu Asn Ile Thr Arg Pro
625                 630                 635                 640

Asn Thr Asp Tyr Ser Cys Gly Gly Phe Leu Ser Gln Pro Ser Gly Asp
                645                 650                 655

Phe Ser Ser Pro Phe Tyr Pro Gly Asn Tyr Pro Asn Asn Ala Lys Cys
            660                 665                 670

Val Trp Asp Ile Glu Val Gln Asn Asn Tyr Arg Val Thr Val Ile Phe
        675                 680                 685

Arg Asp Val Gln Leu Glu Gly Gly Cys Asn Tyr Asp Tyr Ile Glu Val
    690                 695                 700

Phe Asp Gly Pro Tyr Arg Ser Ser Pro Leu Ile Ala Arg Val Cys Asp
705                 710                 715                 720

Gly Ala Arg Gly Ser Phe Thr Ser Ser Ser Asn Phe Met Ser Ile Arg
                725                 730                 735

Phe Ile Ser Asp His Ser Ile Thr Arg Arg Gly Phe Arg Ala Glu Tyr
            740                 745                 750

Tyr Ser Ser Pro Ser Asn Asp Ser Thr Asn Leu Leu Cys Leu Pro Asn
        755                 760                 765

His Met Gln Ala Ser Val Ser Arg Ser Tyr Leu Gln Ser Leu Gly Phe
    770                 775                 780

Ser Ala Ser Asp Leu Val Ile Ser Thr Trp Asn Gly Tyr Tyr Glu Cys
785                 790                 795                 800

Arg Pro Gln Ile Thr Pro Asn Leu Val Ile Phe Thr Ile Pro Tyr Ser
                805                 810                 815

Gly Cys Gly Thr Phe Lys Gln Ala Asp Asn Asp Thr Ile Asp Tyr Ser
            820                 825                 830

Asn Phe Leu Thr Ala Ala Val Ser Gly Gly Ile Ile Lys Arg Arg Thr
        835                 840                 845

Asp Leu Arg Ile His Val Ser Cys Arg Met Leu Gln Asn Thr Trp Val
    850                 855                 860

Asp Thr Met Tyr Ile Ala Asn Asp Thr Ile His Val Ala Asn Asn Thr
865                 870                 875                 880

Ile Gln Val Glu Glu Val Gln Tyr Gly Asn Phe Asp Val Asn Ile Ser
                885                 890                 895

Phe Tyr Thr Ser Ser Ser Phe Leu Tyr Pro Val Thr Ser Arg Pro Tyr
            900                 905                 910

Tyr Val Asp Leu Asn Gln Asp Leu Tyr Val Gln Ala Glu Ile Leu His
        915                 920                 925

Ser Asp Ala Val Leu Thr Leu Phe Val Asp Thr Cys Val Ala Ser Pro
    930                 935                 940

Tyr Ser Asn Asp Phe Thr Ser Leu Thr Tyr Asp Leu Ile Arg Ser Gly
945                 950                 955                 960

Cys Val Arg Asp Asp Thr Tyr Gly Pro Tyr Ser Ser Pro Ser Leu Arg
                965                 970                 975

Ile Ala Arg Phe Arg Phe Arg Ala Phe His Phe Leu Asn Arg Phe Pro
            980                 985                 990

Ser Val Tyr Leu Arg Cys Lys Met Val Val Cys Arg Ala Tyr Asp Pro
        995                 1000                1005

Ser Ser Arg Cys Tyr Arg Gly Cys Val Leu Arg Ser Lys Arg Asp Val
   1010                1015                1020

Gly Ser Tyr Gln Glu Lys Val Asp Val Val Leu Gly Pro Ile Gln Leu
1025                1030                1035                1040

Gln Thr Pro Pro Arg Arg Glu Glu Glu Pro Arg
                1045                1050

<210> 215
<211> 1156
<212> PRT
<213> Homo sapiens

<400> 215
Met Val Leu Leu Glu Asp Ser Gly Ser Ala Asp Phe Arg Arg His Phe
  1               5                  10                  15

Val Asn Leu Ser Pro Phe Thr Ile Thr Val Val Leu Leu Leu Ser Ala
            20                  25                  30

Cys Phe Val Thr Ser Ser Leu Gly Gly Thr Asp Lys Glu Leu Arg Leu
        35                  40                  45

Val Asp Gly Glu Asn Lys Cys Ser Gly Arg Val Glu Val Lys Val Gln
    50                  55                  60

Glu Glu Trp Gly Thr Val Cys Asn Asn Gly Trp Ser Met Glu Ala Val
 65                  70                  75                  80

Ser Val Ile Cys Asn Gln Leu Gly Cys Pro Thr Ala Ile Lys Ala Pro
                85                  90                  95

Gly Trp Ala Asn Ser Ser Ala Gly Ser Gly Arg Ile Trp Met Asp His
            100                 105                 110

Val Ser Cys Arg Gly Asn Glu Ser Ala Leu Trp Asp Cys Lys His Asp
        115                 120                 125

Gly Trp Gly Lys His Ser Asn Cys Thr His Gln Gln Asp Ala Gly Val
    130                 135                 140

Thr Cys Ser Asp Gly Ser Asn Leu Glu Met Arg Leu Thr Arg Gly Gly
145                 150                 155                 160

Asn Met Cys Ser Gly Arg Ile Glu Ile Lys Phe Gln Gly Arg Trp Gly
                165                 170                 175

Thr Val Cys Asp Asp Asn Phe Asn Ile Asp His Ala Ser Val Ile Cys
            180                 185                 190

Arg Gln Leu Glu Cys Gly Ser Ala Val Ser Phe Ser Gly Ser Ser Asn

195                 200                 205

Phe Gly Glu Gly Ser Gly Pro Ile Trp Phe Asp Asp Leu Ile Cys Asn
    210                 215                 220

Gly Asn Glu Ser Ala Leu Trp Asn Cys Lys His Gln Gly Trp Gly Lys
225                 230                 235                 240

His Asn Cys Asp His Ala Glu Asp Ala Gly Val Ile Cys Ser Lys Gly
                245                 250                 255

Ala Asp Leu Ser Leu Arg Leu Val Asp Gly Val Thr Glu Cys Ser Gly
            260                 265                 270

Arg Leu Glu Val Arg Phe Gln Gly Glu Trp Gly Thr Ile Cys Asp Asp
        275                 280                 285

Gly Trp Asp Ser Tyr Asp Ala Ala Val Ala Cys Lys Gln Leu Gly Cys
    290                 295                 300

Pro Thr Ala Val Thr Ala Ile Gly Arg Val Asn Ala Ser Lys Gly Phe
305                 310                 315                 320

Gly His Ile Trp Leu Asp Ser Val Ser Cys Gln Gly His Glu Pro Ala
                325                 330                 335

Val Trp Gln Cys Lys His His Glu Trp Gly Lys His Tyr Cys Asn His
            340                 345                 350

Asn Glu Asp Ala Gly Val Thr Cys Ser Asp Gly Ser Asp Leu Glu Leu
        355                 360                 365

Arg Leu Arg Gly Gly Gly Ser Arg Cys Ala Gly Thr Val Glu Val Glu
    370                 375                 380

Ile Gln Arg Leu Leu Gly Lys Val Cys Asp Arg Gly Trp Gly Leu Lys
385                 390                 395                 400

Glu Ala Asp Val Val Cys Arg Gln Leu Gly Cys Gly Ser Ala Leu Lys
                405                 410                 415

Thr Ser Tyr Gln Val Tyr Ser Lys Ile Gln Ala Thr Asn Thr Trp Leu
            420                 425                 430

Phe Leu Ser Ser Cys Asn Gly Asn Glu Thr Ser Leu Trp Asp Cys Lys
        435                 440                 445

Asn Trp Gln Trp Gly Gly Leu Thr Cys Asp His Tyr Glu Glu Ala Lys
    450                 455                 460

Ile Thr Cys Ser Ala His Arg Glu Pro Arg Leu Val Gly Gly Asp Ile
465                 470                 475                 480

Pro Cys Ser Gly Arg Val Glu Val Lys His Gly Asp Thr Trp Gly Ser
                485                 490                 495

Ile Cys Asp Ser Asp Phe Ser Leu Glu Ala Ala Ser Val Leu Cys Arg
            500                 505                 510

Glu Leu Gln Cys Gly Thr Val Val Ser Ile Leu Gly Gly Ala His Phe
        515                 520                 525

Gly Glu Gly Asn Gly Gln Ile Trp Ala Glu Glu Phe Gln Cys Glu Gly
    530                 535                 540

His Glu Ser His Leu Ser Leu Cys Pro Val Ala Pro Arg Pro Glu Gly
545                 550                 555                 560

Thr Cys Ser His Ser Arg Asp Val Gly Val Val Cys Ser Arg Tyr Thr

                565                 570                 575

Glu Ile Arg Leu Val Asn Gly Lys Thr Pro Cys Glu Gly Arg Val Glu
            580                 585                 590

Leu Lys Thr Leu Gly Ala Trp Gly Ser Leu Cys Asn Ser His Trp Asp
        595                 600                 605

Ile Glu Asp Ala His Val Leu Cys Gln Gln Leu Lys Cys Gly Val Ala
    610                 615                 620

Leu Ser Thr Pro Gly Gly Ala Arg Phe Gly Lys Gly Asn Gly Gln Ile
625                 630                 635                 640

Trp Arg His Met Phe His Cys Thr Gly Thr Glu Gln His Met Gly Asp
                645                 650                 655

Cys Pro Val Thr Ala Leu Gly Ala Ser Leu Cys Pro Ser Glu Gln Val
            660                 665                 670

Ala Ser Val Ile Cys Ser Gly Asn Gln Ser Gln Thr Leu Ser Ser Cys
        675                 680                 685

Asn Ser Ser Ser Leu Gly Pro Thr Arg Pro Thr Ile Pro Glu Glu Ser
    690                 695                 700

Ala Val Ala Cys Ile Glu Ser Gly Gln Leu Arg Leu Val Asn Gly Gly
705                 710                 715                 720

Gly Arg Cys Ala Gly Arg Val Glu Ile Tyr His Glu Gly Ser Trp Gly
                725                 730                 735

Thr Ile Cys Asp Asp Ser Trp Asp Leu Ser Asp Ala His Val Val Cys
            740                 745                 750

Arg Gln Leu Gly Cys Gly Glu Ala Ile Asn Ala Thr Gly Ser Ala His
        755                 760                 765

Phe Gly Glu Gly Thr Gly Pro Ile Trp Leu Asp Glu Met Lys Cys Asn
    770                 775                 780

Gly Lys Glu Ser Arg Ile Trp Gln Cys His Ser His Gly Trp Gly Gln
785                 790                 795                 800

Gln Asn Cys Arg His Lys Glu Asp Ala Gly Val Ile Cys Ser Glu Phe
                805                 810                 815

Met Ser Leu Arg Leu Thr Ser Glu Ala Ser Arg Glu Ala Cys Ala Gly
            820                 825                 830

Arg Leu Glu Val Phe Tyr Asn Gly Ala Trp Gly Thr Val Gly Lys Ser
        835                 840                 845

Ser Met Ser Glu Thr Thr Val Gly Val Val Cys Arg Gln Leu Gly Cys
    850                 855                 860

Ala Asp Lys Gly Lys Ile Asn Pro Ala Ser Leu Asp Lys Ala Met Ser
865                 870                 875                 880

Ile Pro Met Trp Val Asp Asn Val Gln Cys Pro Lys Gly Pro Asp Thr
                885                 890                 895

Leu Trp Gln Cys Pro Ser Ser Pro Trp Glu Lys Arg Leu Ala Ser Pro
            900                 905                 910

Ser Glu Glu Thr Trp Ile Thr Cys Asp Asn Lys Ile Arg Leu Gln Glu
        915                 920                 925

Gly Pro Thr Ser Cys Ser Gly Arg Val Glu Ile Trp His Gly Gly Ser

930                 935                 940

Trp Gly Thr Val Cys Asp Asp Ser Trp Asp Leu Asp Asp Ala Gln Val
945                 950                 955                 960

Val Cys Gln Gln Leu Gly Cys Gly Pro Ala Leu Lys Ala Phe Lys Glu
                965                 970                 975

Ala Glu Phe Gly Gln Gly Thr Gly Pro Ile Trp Leu Asn Glu Val Lys
            980                 985                 990

Cys Lys Gly Asn Glu Ser Ser Leu Trp Asp Cys Pro Ala Arg Arg Trp
        995                 1000                1005

Gly His Ser Glu Cys Gly His Lys Glu Asp Ala Ala Val Asn Cys Thr
    1010                1015                1020

Asp Ile Ser Val Gln Lys Thr Pro Gln Lys Ala Thr Thr Gly Arg Ser
1025                1030                1035                1040

Ser Arg Gln Ser Ser Phe Ile Ala Val Gly Ile Leu Gly Val Val Leu
                1045                1050                1055

Leu Ala Ile Phe Val Ala Leu Phe Phe Leu Thr Lys Lys Arg Arg Gln
            1060                1065                1070

Arg Gln Arg Leu Ala Val Ser Ser Arg Gly Glu Asn Leu Val His Gln
        1075                1080                1085

Ile Gln Tyr Arg Glu Met Asn Ser Cys Leu Asn Ala Asp Asp Leu Asp
    1090                1095                1100

Leu Met Asn Ser Ser Gly Leu Trp Val Leu Gly Gly Ser Ile Ala Gln
1105                1110                1115                1120

Gly Phe Arg Ser Val Ala Ala Val Glu Ala Gln Thr Phe Tyr Phe Asp
                1125                1130                1135

Lys Gln Leu Lys Lys Ser Lys Asn Val Ile Gly Ser Leu Asp Ala Tyr
            1140                1145                1150

Asn Gly Gln Glu
        1155


<210> 216
<211> 101
<212> PRT
<213> Homo sapiens

<400> 216
Val Arg Leu Val Gly Gly Gly Ser Pro Cys Glu Gly Arg Val Glu Val
1               5                   10                  15

Tyr His Asn Gly Gln Trp Gly Thr Val Cys Asp Asp Gly Trp Asp Leu
            20                  25                  30

Arg Asp Ala Asn Val Val Cys Arg Gln Leu Gly Phe Gly Gly Ala Val
        35                  40                  45

Ser Ala Ser Gly Ser Ala Tyr Phe Gly Pro Gly Ser Gly Pro Ile Trp
    50                  55                  60

Leu Asp Asn Val Arg Cys Thr Gly Thr Glu Ala Ser Leu Ser Asp Cys
65                  70                  75                  80

Pro His Ser Gly Trp Gly Ser His Asn Cys Ser His Ser Glu Asp Ala
                85                  90                  95

Gly Val Val Cys Ser
            100

<210> 217
<211> 95
<212> PRT
<213> Homo sapiens

<400> 217
Val Gly Gly Ser Ser Arg Cys Glu Gly Arg Val Glu Val Arg Asp Gly
1               5                   10                  15

Ser Lys Trp Gly Thr Val Cys Asp Ser Ser Trp Thr Leu Arg Asp Ala
            20                  25                  30

Asn Val Val Cys Arg Gln Leu Gly Cys Gly Gly Ala Leu Ser Ser Leu
        35                  40                  45

Gly Gly Pro Tyr Phe Ser Glu Gly Gly Gly Pro Ile Pro Leu Asp Gly
    50                  55                  60

Val Asn Cys Ser Gly Asn Glu Ser Ser Leu Ser Gln Cys Pro His Arg
65                  70                  75                  80

Ser Arg Gln Cys Ser His Gly Glu Asp Ala Gly Val Val Cys Ser
                85                  90                  95

<210> 218
<211> 132
<212> PRT
<213> Homo sapiens

<400> 218
Met Val Glu Ala Phe Cys Ala Thr Trp Lys Leu Thr Asn Ser Gln Asn
1               5                   10                  15

Phe Asp Glu Tyr Met Lys Ala Leu Gly Val Gly Phe Ala Thr Arg Gln
            20                  25                  30

Val Gly Asn Val Thr Lys Pro Thr Val Ile Ile Ser Gln Glu Gly Asp
        35                  40                  45

Lys Val Val Ile Arg Thr Leu Ser Thr Phe Lys Asn Thr Glu Ile Ser
    50                  55                  60

Phe Gln Leu Gly Glu Glu Phe Asp Glu Thr Thr Ala Asp Asp Arg Asn
65                  70                  75                  80

Cys Lys Ser Val Val Ser Leu Asp Gly Asp Lys Leu Val His Ile Gln
                85                  90                  95

Lys Trp Asp Gly Lys Glu Thr Asn Phe Val Arg Glu Ile Lys Asp Gly
            100                 105                 110

Lys Met Val Met Thr Leu Thr Phe Gly Asp Val Val Ala Val Arg His
        115                 120                 125

Tyr Glu Lys Ala
    130

<210> 219
<211> 131
<212> PRT
<213> Homo sapiens

<400> 219

Val Glu Ala Phe Cys Ala Thr Trp Lys Leu Thr Asn Ser Gln Asn Phe
1               5                   10                  15

Asp Glu Tyr Met Lys Ala Leu Gly Val Gly Phe Ala Thr Arg Gln Val
            20                  25                  30

Gly Asn Val Thr Lys Pro Thr Val Ile Ile Ser Gln Glu Gly Asp Lys
        35                  40                  45

Val Val Ile Arg Thr Leu Ser Thr Phe Lys Asn Thr Glu Ile Ser Phe
    50                  55                  60

Gln Leu Gly Glu Glu Phe Asp Glu Thr Thr Ala Asp Asp Arg Asn Cys
65                  70                  75                  80

Lys Ser Val Val Ser Leu Asp Gly Asp Lys Leu Val His Ile Gln Lys
                85                  90                  95

Trp Asp Gly Lys Glu Thr Asn Phe Val Arg Glu Ile Lys Asp Gly Lys
            100                 105                 110

Met Val Met Thr Leu Thr Phe Gly Asp Val Val Ala Val Arg His Tyr
        115                 120                 125

Glu Lys Ala
    130


<210> 220
<211> 132
<212> PRT
<213> Homo sapiens

<400> 220


<210> 221
<211> 166
<212> PRT
<213> Homo sapiens

<400> 221
Met Val Glu Ala Phe Cys Ala Thr Trp Lys Leu Thr Asn Ser Gln Asn

1                   5                   10                  15

Phe Asp Glu Tyr Met Lys Ala Leu Gly Val Gly Phe Ala Thr Arg Gln
            20                  25                  30

Val Gly Asn Val Thr Lys Pro Thr Val Ile Ile Ser Gln Glu Gly Asp
        35                  40                  45

Lys Val Val Ile Arg Thr Leu Ser Thr Phe Lys Asn Thr Glu Ile Ser
    50                  55                  60

Phe Gln Leu Gly Glu Glu Phe Asp Glu Thr Thr Ala Asp Asp Arg Asn
65                  70                  75                  80

Cys Lys Ser Val Val Ser Leu Asp Gly Asp Lys Leu Val His Ile Gln
                85                  90                  95

Lys Trp Asp Gly Lys Glu Thr Asn Phe Val Arg Glu Ile Lys Asp Gly
            100                 105                 110

Lys Met Val Met Val Ser Asn Asp Asn Ser Pro Phe Phe Leu Val Phe
        115                 120                 125

Phe Ser Ser Pro His Thr Ser His Leu Leu Pro Ser Ser Ser Leu Leu
    130                 135                 140

Leu Pro Phe Phe Leu Leu Pro Ser Phe Phe Asn Asn Thr Ser Leu Ala
145                 150                 155                 160

Arg Phe Phe Asn Tyr Met
                165

<210> 222
<211> 132
<212> PRT
<213> Gallus gallus

<400> 222
Met Val Glu Ala Phe Cys Ala Thr Trp Lys Leu Ala Asp Ser His Asn
1               5                   10                  15

Phe Asp Glu Tyr Met Lys Ala Leu Gly Val Gly Phe Ala Met Arg Gln
            20                  25                  30

Val Gly Asn Val Thr Lys Pro Thr Val Ile Ile Ser Ser Glu Gly Asp
        35                  40                  45

Lys Val Val Ile Arg Thr Gln Ser Thr Phe Lys Asn Thr Glu Ile Ser
    50                  55                  60

Phe Lys Leu Gly Glu Glu Phe Asp Glu Thr Thr Pro Asp Asp Arg Asn
65                  70                  75                  80

Cys Lys Ser Val Val Thr Leu Asp Gly Asp Lys Leu Val His Val Gln
                85                  90                  95

Lys Trp Asp Gly Lys Glu Thr Asn Phe Val Arg Glu Ile Lys Asp Gly
            100                 105                 110

Arg Met Val Met Thr Leu Thr Phe Gly Asp Val Val Ala Val Arg His
        115                 120                 125

Tyr Glu Lys Ala
    130

<210> 223
<211> 145

<212> PRT
<213> Homo sapiens

<400> 223
Lys Phe Ala Gly Lys Trp Tyr Leu Val Ala Ser Ala Asn Phe Asp Pro
1               5                   10                  15

Glu Leu Lys Glu Glu Leu Gly Val Leu Glu Ala Thr Arg Lys Glu Ile
            20                  25                  30

Thr Pro Leu Lys Glu Gly Asn Leu Glu Ile Val Phe Asp Gly Asp Lys
        35                  40                  45

Asn Gly Ile Cys Glu Glu Thr Phe Gly Lys Leu Glu Lys Thr Lys Lys
    50                  55                  60

Leu Gly Val Glu Phe Asp Tyr Tyr Thr Gly Asp Asn Arg Phe Val Val
65                  70                  75                  80

Leu Asp Thr Asp Tyr Asp Asn Tyr Leu Leu Val Cys Val Gln Lys Gly
                85                  90                  95

Asp Gly Asn Glu Thr Ser Arg Thr Ala Glu Leu Tyr Gly Arg Thr Pro
            100                 105                 110

Glu Leu Ser Pro Glu Ala Leu Glu Leu Phe Glu Thr Ala Thr Lys Glu
        115                 120                 125

Leu Gly Ile Pro Glu Asp Asn Val Val Cys Thr Arg Gln Thr Glu Arg
    130                 135                 140

Cys
145


<210> 224
<211> 787
<212> PRT
<213> Homo sapiens

<400> 224
Met Ser Arg Met Ser Arg His Pro Asp Lys Asp Leu Ala Gln Gly Pro
1               5                   10                  15

Phe Asn Thr Cys Cys Gly Cys Thr Leu Met Ala Ser Pro Ala Asn Leu
            20                  25                  30

Pro Pro Asn Thr Gln Ala Ala Ala Glu Arg Ala Leu Ser Gln Ser Arg
        35                  40                  45

Trp Lys Arg Val Gln Val Pro Ala Pro Ala Ser Leu Ser Pro Phe Pro
    50                  55                  60

Leu Ala Met Ala Ser Val Ala Phe Trp Ile Ser Ile Leu Ile Gly Cys
65                  70                  75                  80

Glu Glu Gln Thr Leu Cys Arg Gly Trp Arg Ser Pro Val Gly Asp Gly
                85                  90                  95

Cys Ala His Val Pro Pro Gln Glu Arg Ala Thr Ala Glu Ala Asp Pro
            100                 105                 110

Pro Gly Arg Cys Ser Thr Ser Thr Ala Ser Ser Thr Ile Cys Gly Leu
        115                 120                 125

Trp His Leu Ser Pro Arg Leu Gln Leu Leu Pro Pro Leu His Ser Arg
    130                 135                 140

Gln Gly Glu Glu Ser Gly Lys Thr Glu Lys Val Leu Leu Trp Gly Arg

145                 150                 155                 160

Glu Gly Leu His Val Trp Lys Pro Gly Val Leu Gln Pro Asp Val His
                165                 170                 175

Gly Thr Ser Asn Leu Gly Asn Cys Ser Phe Leu His Gly Leu Val Thr
            180                 185                 190

Ala Pro Ser Cys Pro Arg Arg Ala Gly Ala Glu Leu Leu Ala Met Pro
        195                 200                 205

Leu Lys His Tyr Leu Leu Leu Leu Val Gly Cys Gln Ala Trp Gly Ala
    210                 215                 220

Gly Leu Ala Tyr His Gly Cys Pro Ser Glu Cys Thr Cys Ser Arg Ala
225                 230                 235                 240

Ser Gln Val Glu Cys Thr Gly Ala Arg Ile Val Ala Val Pro Thr Pro
                245                 250                 255

Leu Pro Trp Asn Ala Met Ser Leu Gln Ile Leu Asn Thr His Ile Thr
            260                 265                 270

Glu Leu Asn Glu Ser Pro Phe Leu Asn Ile Ser Ala Leu Ile Ala Leu
        275                 280                 285

Arg Ile Glu Lys Asn Glu Leu Ser Arg Ile Thr Pro Gly Ala Phe Arg
    290                 295                 300

Asn Leu Gly Ser Leu Arg Tyr Leu Ser Leu Ala Asn Asn Lys Leu Gln
305                 310                 315                 320

Val Leu Pro Ile Gly Leu Phe Gln Gly Leu Asp Ser Leu Glu Ser Leu
                325                 330                 335

Leu Leu Ser Ser Asn Gln Leu Leu Gln Ile Gln Pro Ala His Phe Ser
            340                 345                 350

Gln Cys Ser Asn Leu Lys Glu Leu Gln Leu His Gly Asn His Leu Glu
        355                 360                 365

Tyr Ile Pro Asp Gly Ala Phe Asp His Leu Val Gly Leu Thr Lys Leu
    370                 375                 380

Asn Leu Gly Lys Asn Ser Leu Thr His Ile Ser Pro Arg Val Phe Gln
385                 390                 395                 400

His Leu Gly Asn Leu Gln Val Leu Arg Leu Tyr Glu Asn Arg Leu Thr
                405                 410                 415

Asp Ile Pro Met Gly Thr Phe Asp Gly Leu Val Asn Leu Gln Glu Leu
            420                 425                 430

Ala Leu Gln Gln Asn Gln Ile Gly Leu Leu Ser Pro Gly Leu Phe His
        435                 440                 445

Asn Asn His Asn Leu Gln Arg Leu Tyr Leu Ser Asn Asn His Ile Ser
    450                 455                 460

Gln Leu Pro Pro Ser Ile Phe Met Gln Leu Pro Gln Leu Asn Arg Leu
465                 470                 475                 480

Thr Leu Phe Gly Asn Ser Leu Lys Glu Leu Ser Leu Gly Ile Phe Gly
                485                 490                 495

Pro Met Pro Asn Leu Arg Glu Leu Trp Leu Tyr Asp Asn His Ile Ser
            500                 505                 510

Ser Leu Pro Asp Asn Val Phe Ser Asn Leu Arg Gln Leu Gln Val Leu

515                 520                 525

Ile Leu Ser Arg Asn Gln Ile Ser Phe Ile Ser Pro Gly Ala Phe Asn
    530                 535                 540

Gly Leu Thr Glu Leu Arg Glu Leu Ser Leu His Thr Asn Ala Leu Gln
545                 550                 555                 560

Asp Leu Asp Gly Asn Val Phe Arg Met Leu Ala Asn Leu Gln Asn Ile
                565                 570                 575

Ser Leu Gln Asn Asn Arg Leu Arg Gln Leu Pro Gly Asn Ile Phe Ala
            580                 585                 590

Asn Val Asn Gly Leu Met Ala Ile Gln Leu Gln Asn Asn Gln Leu Glu
        595                 600                 605

Asn Leu Pro Leu Gly Ile Phe Asp His Leu Gly Lys Leu Cys Glu Leu
    610                 615                 620

Arg Leu Tyr Asp Asn Pro Trp Arg Cys Asp Ser Asp Ile Leu Pro Leu
625                 630                 635                 640

Arg Asn Trp Leu Leu Leu Asn Gln Pro Arg Leu Gly Thr Asp Thr Val
                645                 650                 655

Pro Val Cys Phe Ser Pro Ala Asn Val Arg Gly Gln Ser Leu Ile Ile
            660                 665                 670

Ile Asn Val Asn Val Ala Val Pro Ser Val His Val Pro Glu Val Pro
        675                 680                 685

Ser Tyr Pro Glu Thr Pro Trp Tyr Pro Asp Thr Pro Ser Tyr Pro Asp
    690                 695                 700

Thr Thr Ser Val Ser Ser Thr Thr Glu Leu Thr Ser Pro Val Glu Asp
705                 710                 715                 720

Tyr Thr Asp Leu Thr Thr Ile Gln Val Thr Asp Asp Arg Ser Val Trp
                725                 730                 735

Gly Met Thr His Ala His Ser Gly Leu Ala Ile Ala Ala Ile Val Ile
            740                 745                 750

Gly Ile Val Ala Leu Ala Cys Ser Leu Ala Ala Cys Val Gly Cys Cys
        755                 760                 765

Cys Cys Lys Lys Arg Ser Gln Ala Val Leu Met Gln Met Lys Ala Pro
    770                 775                 780

Asn Glu Cys
785


<210> 225
<211> 581
<212> PRT
<213> Homo sapiens

<400> 225
Met Pro Leu Lys His Tyr Leu Leu Leu Leu Val Gly Cys Gln Ala Trp
1               5                   10                  15

Gly Ala Gly Leu Ala Tyr His Gly Cys Pro Ser Glu Cys Thr Cys Ser
            20                  25                  30

Arg Ala Ser Gln Val Glu Cys Thr Gly Ala Arg Ile Val Ala Val Pro
        35                  40                  45

Thr Pro Leu Pro Trp Asn Ala Met Ser Leu Gln Ile Leu Asn Thr His
    50                  55                  60

Ile Thr Glu Leu Asn Glu Ser Pro Phe Leu Asn Ile Ser Ala Leu Ile
65                  70                  75                  80

Ala Leu Arg Ile Glu Lys Asn Glu Leu Ser Arg Ile Thr Pro Gly Ala
                85                  90                  95

Phe Arg Asn Leu Gly Ser Leu Arg Tyr Leu Ser Leu Ala Asn Asn Lys
            100                 105                 110

Leu Gln Val Leu Pro Ile Gly Leu Phe Gln Gly Leu Asp Ser Leu Glu
        115                 120                 125

Ser Leu Leu Leu Ser Ser Asn Gln Leu Leu Gln Ile Gln Pro Ala His
    130                 135                 140

Phe Ser Gln Cys Ser Asn Leu Lys Glu Leu Gln Leu His Gly Asn His
145                 150                 155                 160

Leu Glu Tyr Ile Pro Asp Gly Ala Phe Asp His Leu Val Gly Leu Thr
                165                 170                 175

Lys Leu Asn Leu Gly Lys Asn Ser Leu Thr His Ile Ser Pro Arg Val
            180                 185                 190

Phe Gln His Leu Gly Asn Leu Gln Val Leu Arg Leu Tyr Glu Asn Arg
        195                 200                 205

Leu Thr Asp Ile Pro Met Gly Thr Phe Asp Gly Leu Val Asn Leu Gln
    210                 215                 220

Glu Leu Ala Leu Gln Gln Asn Gln Ile Gly Leu Leu Ser Pro Gly Leu
225                 230                 235                 240

Phe His Asn Asn His Asn Leu Gln Arg Leu Tyr Leu Ser Asn Asn His
                245                 250                 255

Ile Ser Gln Leu Pro Pro Ser Ile Phe Met Gln Leu Pro Gln Leu Asn
            260                 265                 270

Arg Leu Thr Leu Phe Gly Asn Ser Leu Lys Glu Leu Ser Leu Gly Ile
        275                 280                 285

Phe Gly Pro Met Pro Asn Leu Arg Glu Leu Trp Leu Tyr Asp Asn His
    290                 295                 300

Ile Ser Ser Leu Pro Asp Asn Val Phe Ser Asn Leu Arg Gln Leu Gln
305                 310                 315                 320

Val Leu Ile Leu Ser Arg Asn Gln Ile Ser Phe Ile Ser Pro Gly Ala
                325                 330                 335

Phe Asn Gly Leu Thr Glu Leu Arg Glu Leu Ser Leu His Thr Asn Ala
            340                 345                 350

Leu Gln Asp Leu Asp Gly Asn Val Phe Arg Met Leu Ala Asn Leu Gln
        355                 360                 365

Asn Ile Ser Leu Gln Asn Asn Arg Leu Arg Gln Leu Pro Gly Asn Ile
    370                 375                 380

Phe Ala Asn Val Asn Gly Leu Met Ala Ile Gln Leu Gln Asn Asn Gln
385                 390                 395                 400

Leu Glu Asn Leu Pro Leu Gly Ile Phe Asp His Leu Gly Lys Leu Cys
                405                 410                 415

Glu Leu Arg Leu Tyr Asp Asn Pro Trp Arg Cys Asp Ser Asp Ile Leu
            420                 425                 430

Pro Leu Arg Asn Trp Leu Leu Leu Asn Gln Pro Arg Leu Gly Thr Asp
        435                 440                 445

Thr Val Pro Val Cys Phe Ser Pro Ala Asn Val Arg Gly Gln Ser Leu
    450                 455                 460

Ile Ile Ile Asn Val Asn Val Ala Val Pro Ser Val His Val Pro Glu
465                 470                 475                 480

Val Pro Ser Tyr Pro Glu Thr Pro Trp Tyr Pro Asp Thr Pro Ser Tyr
                485                 490                 495

Pro Asp Thr Thr Ser Val Ser Ser Thr Thr Glu Leu Thr Ser Pro Val
            500                 505                 510

Glu Asp Tyr Thr Asp Leu Thr Thr Ile Gln Val Thr Asp Asp Arg Ser
        515                 520                 525

Val Trp Gly Met Thr Gln Ala Gln Ser Gly Leu Ala Ile Ala Ala Ile
    530                 535                 540

Val Ile Gly Ile Val Ala Leu Ala Cys Ser Leu Ala Ala Cys Val Gly
545                 550                 555                 560

Cys Cys Cys Cys Lys Lys Arg Ser Gln Ala Val Leu Met Gln Met Lys
                565                 570                 575

Ala Pro Asn Glu Cys
            580

<210> 226
<211> 578
<212> PRT
<213> Rattus norvegicus

<400> 226
Met Pro Leu Lys His Tyr Leu Leu Leu Leu Val Gly Cys Gln Ala Trp
1               5                   10                  15

Ala Leu Gly Leu Ala Tyr Tyr Gly Cys Pro Ser Glu Cys Thr Cys Ser
            20                  25                  30

Arg Ala Ser Gln Val Glu Cys Thr Gly Ala Arg Ile Val Ala Met Pro
        35                  40                  45

Thr Pro Leu Pro Trp Asn Ala Met Ser Leu Gln Val Val Asn Thr His
    50                  55                  60

Ile Thr Glu Leu Pro Glu Asn Leu Phe Leu Asn Ile Ser Ala Leu Ile
65                  70                  75                  80

Ala Leu Lys Met Glu Lys Asn Glu Leu Ser Thr Ile Met Pro Gly Ala
                85                  90                  95

Phe Arg Asn Leu Gly Ser Leu Arg Tyr Leu Ser Leu Ala Asn Asn Lys
            100                 105                 110

Leu Arg Met Leu Pro Ile Arg Val Phe Gln Asp Val Asn Asn Leu Glu
        115                 120                 125

Ser Leu Leu Leu Ser Asn Asn Gln Leu Val Gln Ile Gln Pro Ala Gln
    130                 135                 140

Phe Ser Gln Phe Ser Asn Leu Arg Glu Leu Gln Leu His Gly Asn Asn
145                 150                 155                 160

Leu Glu Ser Ile Pro Glu Glu Ala Phe Asp His Leu Val Gly Leu Thr
                165                 170                 175

Lys Leu Asn Leu Gly Arg Asn Ser Phe Thr His Leu Ser Pro Arg Leu
            180                 185                 190

Phe Gln His Leu Gly Asn Leu Gln Val Leu Arg Leu His Glu Asn Arg
        195                 200                 205

Leu Ser Asp Ile Pro Met Gly Thr Phe Asp Ala Leu Gly Asn Leu Gln
    210                 215                 220

Glu Leu Ala Leu Gln Glu Asn Gln Ile Gly Thr Leu Ser Pro Gly Leu
225                 230                 235                 240

Phe His Asn Asn Arg Asn Leu Gln Arg Leu Tyr Leu Ser Asn Asn His
                245                 250                 255

Ile Ser Gln Leu Pro Pro Gly Ile Phe Met Gln Leu Pro Gln Leu Asn
            260                 265                 270

Lys Leu Thr Leu Phe Gly Asn Ser Leu Arg Glu Leu Ser Pro Gly Val
        275                 280                 285

Phe Gly Pro Met Pro Asn Leu Arg Glu Leu Trp Leu Tyr Asn Asn His
    290                 295                 300

Ile Thr Ser Leu Ala Asp Asn Thr Phe Ser His Leu Asn Gln Leu Gln
305                 310                 315                 320

Val Leu Ile Leu Ser His Asn Gln Leu Thr Tyr Ile Ser Pro Gly Ala
                325                 330                 335

Phe Asn Gly Leu Thr Asn Leu Arg Glu Leu Ser Leu His Thr Asn Ala
            340                 345                 350

Leu Gln Asp Leu Asp Ser Asn Val Phe Arg Ser Leu Ala Asn Leu Gln
        355                 360                 365

Asn Ile Ser Leu Gln Ser Asn Arg Leu Arg Gln Leu Pro Gly Ser Ile
    370                 375                 380

Phe Ala Asn Val Asn Gly Leu Thr Thr Ile Gln Leu Gln Asn Asn Asn
385                 390                 395                 400

Leu Glu Asn Leu Pro Leu Gly Ile Phe Asp His Leu Val Asn Leu Cys
                405                 410                 415

Glu Leu Arg Leu Tyr Asp Asn Pro Trp Arg Cys Asp Ser Asp Ile Leu
            420                 425                 430

Pro Leu His Asn Trp Leu Leu Leu Asn Arg Ala Arg Leu Gly Thr Asp
        435                 440                 445

Thr Leu Pro Val Cys Ser Ser Pro Ala Asn Val Arg Gly Gln Ser Leu
    450                 455                 460

Val Ile Ile Asn Ile Asn Phe Pro Gly Pro Ser Val Gln Gly Pro Glu
465                 470                 475                 480

Thr Pro Glu Val Pro Ser Tyr Pro Asp Thr Pro Ser Tyr Pro Asp Thr
                485                 490                 495

Thr Ser Val Ser Ser Thr Thr Glu Ile Thr Ser Ala Val Asp Asp Tyr
            500                 505                 510

Thr Asp Leu Thr Thr Ile Glu Ala Thr Asp Asp Arg Asn Thr Trp Gly
        515                 520                 525

Met Thr Glu Ala Gln Ser Gly Leu Ala Ile Ala Ala Ile Val Ile Gly
    530                 535                 540

Ile Ile Ala Leu Ala Cys Ser Leu Ala Ala Cys Ile Cys Cys Cys Cys
545                 550                 555                 560

Cys Lys Lys Arg Ser Gln Ala Val Leu Met Gln Met Lys Ala Pro Asn
                565                 570                 575

Glu Cys

<210> 227
<211> 391
<212> PRT
<213> Mus musculus

<400> 227
Met Pro Leu Lys His Tyr Leu Leu Leu Leu Val Ser Cys Gln Ala Trp
1               5                   10                  15

Ala Ala Gly Leu Ala Tyr Tyr Gly Cys Pro Ser Glu Cys Thr Cys Ser
            20                  25                  30

Arg Ala Ser Gln Val Glu Cys Thr Gly Ala Gln Ile Val Ala Met Pro
        35                  40                  45

Ser Pro Leu Pro Trp Asn Ala Met Ser Leu Gln Ile Leu Asn Thr His
    50                  55                  60

Ile Thr Glu Leu Pro Glu Asp Lys Phe Leu Asn Ile Ser Ala Leu Ile
65                  70                  75                  80

Ala Leu Lys Met Glu Lys Asn Glu Leu Ala Asn Ile Met Pro Gly Ala
                85                  90                  95

Phe Arg Asn Leu Gly Ser Leu Arg His Leu Ser Leu Ala Asn Asn Lys
            100                 105                 110

Leu Lys Asn Leu Pro Val Arg Leu Phe Gln Asp Val Asn Asn Leu Glu
        115                 120                 125

Thr Leu Leu Leu Ser Asn Asn Gln Leu Val Gln Ile Gln Pro Ala Gln
    130                 135                 140

Phe Ser Gln Phe Ser Asn Leu Lys Glu Leu Gln Leu Tyr Gly Asn Asn
145                 150                 155                 160

Leu Glu Tyr Ile Pro Glu Gly Val Phe Asp His Leu Val Gly Leu Thr
                165                 170                 175

Lys Leu Asn Leu Gly Asn Asn Gly Phe Thr His Leu Ser Pro Arg Val
            180                 185                 190

Phe Gln His Leu Gly Asn Leu Gln Val Leu Arg Leu Tyr Glu Asn Arg
        195                 200                 205

Leu Ser Asp Ile Pro Met Gly Thr Phe Asp Ala Leu Gly Asn Leu Gln
    210                 215                 220

Glu Leu Ala Leu Gln Glu Asn Gln Ile Gly Thr Leu Ser Pro Gly Leu
225                 230                 235                 240

Phe His Asn Asn Arg Asn Leu Gln Arg Leu Tyr Leu Ser Asn Asn His
                245                 250                 255

Ile Ser His Leu Pro Pro Gly Ile Phe Met Gln Leu Pro His Leu Asn

260                 265                 270

Lys Leu Thr Leu Phe Gly Asn Ser Leu Lys Glu Leu Ser Pro Gly Val
        275                 280                 285

Phe Gly Pro Met Pro Asn Leu Arg Glu Leu Trp Leu Tyr Asn Asn His
    290                 295                 300

Ile Thr Ser Leu Pro Asp Asn Ala Phe Ser His Leu Asn Gln Leu Gln
305                 310                 315                 320

Val Leu Ile Leu Ser His Asn Gln Leu Ser Tyr Ile Ser Pro Gly Ala
                325                 330                 335

Phe Asn Gly Leu Thr Asn Leu Arg Glu Leu Ser Leu His Thr Asn Ala
            340                 345                 350

Leu Gln Asp Leu Asp Gly Asn Val Phe Arg Ser Leu Ala Asn Leu Gly
        355                 360                 365

Thr Ser His Ser Arg Ile Thr Ala Phe Asp Ser Ser Leu Ala Ala Ser
    370                 375                 380

Ser Pro Thr Ser Met Val Ser
385                 390


<210> 228
<211> 567
<212> PRT
<213> Rattus norvegicus

<400> 228
Met Leu Arg Ser Val Leu Leu Ser Ala Val Leu Ser Leu Val Gly Ala
1               5                   10                  15

Gln Pro Phe Pro Cys Pro Lys Thr Cys Lys Cys Val Val Arg Asp Ala
            20                  25                  30

Val Gln Cys Ser Gly Gly Ser Val Ala His Ile Ala Glu Leu Gly Leu
        35                  40                  45

Pro Thr Asn Leu Thr His Ile Leu Leu Phe Arg Met Asp Arg Gly Val
    50                  55                  60

Leu Gln Ser His Ser Phe Ser Gly Met Thr Val Leu Gln Arg Leu Met
65                  70                  75                  80

Leu Ser Asp Ser His Ile Ser Ala Ile Asp Pro Gly Thr Phe Asn Asp
                85                  90                  95

Leu Val Lys Leu Lys Thr Leu Arg Leu Thr Arg Asn Lys Ile Ser His
            100                 105                 110

Leu Pro Arg Ala Ile Leu Asp Lys Met Val Leu Leu Glu Gln Leu Phe
        115                 120                 125

Leu Asp His Asn Ala Leu Arg Asp Leu Asp Gln Asn Leu Phe Gln Lys
    130                 135                 140

Leu Leu Asn Leu Arg Asp Leu Cys Leu Asn Gln Asn Gln Leu Ser Phe
145                 150                 155                 160

Leu Pro Ala Asn Leu Phe Ser Ser Leu Gly Lys Leu Lys Val Leu Asp
                165                 170                 175

Leu Ser Arg Asn Asn Leu Thr His Leu Pro Gln Gly Leu Leu Gly Ala
            180                 185                 190

Gln Ile Lys Leu Glu Lys Leu Leu Leu Tyr Ser Asn Arg Leu Met Ser
        195                 200                 205

Leu Asp Ser Gly Leu Leu Ala Asn Leu Gly Ala Leu Thr Glu Leu Arg
    210                 215                 220

Leu Glu Arg Asn His Leu Arg Ser Ile Ala Pro Gly Ala Phe Asp Ser
225                 230                 235                 240

Leu Gly Asn Leu Ser Thr Leu Thr Leu Ser Gly Asn Leu Leu Glu Ser
                245                 250                 255

Leu Pro Pro Ala Leu Phe Leu His Val Ser Trp Leu Thr Arg Leu Thr
            260                 265                 270

Leu Phe Glu Asn Pro Leu Glu Glu Leu Pro Glu Val Leu Phe Gly Glu
        275                 280                 285

Met Ala Gly Leu Arg Glu Leu Trp Leu Asn Gly Thr His Leu Arg Thr
    290                 295                 300

Leu Pro Ala Ala Ala Phe Arg Asn Leu Ser Gly Leu Gln Thr Leu Gly
305                 310                 315                 320

Leu Thr Arg Asn Pro Leu Leu Ser Ala Leu Pro Pro Gly Met Phe His
                325                 330                 335

Gly Leu Thr Glu Leu Arg Val Leu Ala Val His Thr Asn Ala Leu Glu
            340                 345                 350

Glu Leu Pro Glu Asp Ala Leu Arg Gly Leu Gly Arg Leu Arg Gln Val
        355                 360                 365

Ser Leu Arg His Asn Arg Leu Arg Ala Leu Pro Arg Thr Leu Phe Arg
    370                 375                 380

Asn Leu Ser Ser Leu Val Thr Val Gln Leu Glu His Asn Gln Leu Lys
385                 390                 395                 400

Thr Leu Pro Gly Asp Val Phe Ala Ala Leu Pro Gln Leu Thr Arg Val
                405                 410                 415

Leu Leu Gly His Asn Pro Trp Leu Cys Asp Cys Gly Leu Trp Pro Phe
            420                 425                 430

Leu Gln Trp Leu Arg His His Leu Glu Leu Leu Gly Arg Asp Glu Pro
        435                 440                 445

Pro Gln Cys Asn Gly Pro Glu Ser Arg Ala Ser Leu Thr Phe Trp Glu
    450                 455                 460

Leu Leu Gln Gly Asp Gln Trp Cys Pro Ser Ser Arg Gly Leu Pro Pro
465                 470                 475                 480

Asp Pro Pro Thr Glu Asn Ala Leu Lys Ala Pro Asp Pro Thr Gln Arg
                485                 490                 495

Pro Asn Ser Ser Gln Ser Trp Ala Trp Val Gln Leu Val Ala Arg Gly
            500                 505                 510

Glu Ser Pro Asp Asn Arg Phe Tyr Trp Asn Leu Tyr Ile Leu Leu Leu
        515                 520                 525

Ile Ala Gln Ala Thr Ile Ala Gly Phe Ile Val Phe Ala Met Ile Lys
    530                 535                 540

Ile Gly Gln Leu Phe Arg Thr Leu Ile Arg Glu Glu Leu Leu Phe Glu
545                 550                 555                 560

Ala Met Gly Lys Ser Ser Asn
                565

<210> 229
<211> 51
<212> PRT
<213> Homo sapiens

<400> 229
Asn Pro Phe Ile Cys Asp Cys Glu Leu Arg Trp Leu Leu Arg Trp Leu
1               5                   10                  15

Gln Ala Asn Arg His Leu Gln Asp Pro Val Asp Leu Arg Cys Ala Ser
            20                  25                  30

Pro Glu Ser Leu Arg Gly Pro Leu Leu Leu Leu Leu Pro Ser Ser Phe
        35                  40                  45

Lys Cys Pro
    50

<210> 230
<211> 1558
<212> PRT
<213> Homo sapiens

<400> 230


<210> 231
<211> 662
<212> PRT
<213> Homo sapiens

<400> 231
Met Arg Pro Gln Ile Leu Leu Leu Leu Ala Leu Leu Thr Leu Gly Leu
1               5                   10                  15

Ala Ala Gln His Gln Asp Lys Val Pro Cys Lys Met Val Asp Lys Lys
            20                  25                  30

Val Ser Cys Gln Val Leu Gly Leu Leu Gln Val Pro Ser Val Leu Pro
        35                  40                  45

Pro Asp Thr Glu Thr Leu Asp Leu Ser Gly Asn Gln Leu Arg Ser Ile
    50                  55                  60

Leu Ala Ser Pro Leu Gly Phe Tyr Thr Ala Leu Arg His Leu Asp Leu
65                  70                  75                  80

Ser Thr Asn Glu Ile Ser Phe Leu Gln Pro Gly Ala Phe Gln Ala Leu
                85                  90                  95

Thr His Leu Glu His Leu Ser Leu Ala His Asn Arg Leu Ala Met Ala
            100                 105                 110

Thr Ala Leu Ser Ala Gly Gly Leu Gly Pro Leu Pro Arg Val Thr Ser
        115                 120                 125

Leu Asp Leu Ser Gly Asn Ser Leu Tyr Ser Gly Leu Leu Glu Arg Leu
    130                 135                 140

Leu Gly Glu Ala Pro Ser Leu His Thr Leu Ser Leu Ala Glu Asn Ser
145                 150                 155                 160

Leu Thr Arg Leu Thr Arg His Thr Phe Arg Asp Met Pro Ala Leu Glu
                165                 170                 175

Gln Leu Asp Leu His Ser Asn Val Leu Met Asp Ile Glu Asp Gly Ala
            180                 185                 190

Phe Glu Gly Leu Pro Arg Leu Thr His Leu Asn Leu Ser Arg Asn Ser
        195                 200                 205

Leu Thr Cys Ile Ser Asp Phe Ser Leu Gln Gln Leu Arg Val Leu Asp
    210                 215                 220

Leu Ser Cys Asn Ser Ile Glu Ala Phe Gln Thr Ala Ser Gln Pro Gln
225                 230                 235                 240

Ala Glu Phe Gln Leu Thr Trp Leu Asp Leu Arg Glu Asn Lys Leu Leu
                245                 250                 255

His Phe Pro Asp Leu Ala Ala Leu Pro Arg Leu Ile Tyr Leu Asn Leu
            260                 265                 270

Ser Asn Asn Leu Ile Arg Leu Pro Thr Gly Pro Pro Gln Asp Ser Lys
        275                 280                 285

Gly Ile His Ala Pro Ser Glu Gly Trp Ser Ala Leu Pro Leu Ser Ala
    290                 295                 300

Pro Ser Gly Asn Ala Ser Gly Arg Pro Leu Ser Gln Leu Leu Asn Leu
305                 310                 315                 320

Asp Leu Ser Tyr Asn Glu Ile Glu Leu Ile Pro Asp Ser Phe Leu Glu
                325                 330                 335

His Leu Thr Ser Leu Cys Phe Leu Asn Leu Ser Arg Asn Cys Leu Arg
            340                 345                 350

Thr Phe Glu Ala Arg Arg Leu Gly Ser Leu Pro Cys Leu Met Leu Leu
        355                 360                 365

Asp Leu Ser His Asn Ala Leu Glu Thr Leu Glu Leu Gly Ala Arg Ala
    370                 375                 380

Leu Gly Ser Leu Arg Thr Leu Leu Leu Gln Gly Asn Ala Leu Arg Asp
385                 390                 395                 400

Leu Pro Pro Tyr Thr Phe Ala Asn Leu Ala Ser Leu Gln Arg Leu Asn
                405                 410                 415

Leu Gln Gly Asn Arg Val Ser Pro Cys Gly Gly Pro Asp Glu Pro Gly
            420                 425                 430

Pro Ser Gly Cys Val Ala Phe Ser Gly Ile Thr Ser Leu Arg Ser Leu
        435                 440                 445

Ser Leu Val Asp Asn Glu Ile Glu Leu Leu Arg Ala Gly Ala Phe Leu
    450                 455                 460

His Thr Pro Leu Thr Glu Leu Asp Leu Ser Ser Asn Pro Gly Leu Glu
465                 470                 475                 480

Val Ala Thr Gly Ala Leu Gly Gly Leu Glu Ala Ser Leu Glu Val Leu
                485                 490                 495

Ala Leu Gln Gly Asn Gly Leu Met Val Leu Gln Val Asp Leu Pro Cys
            500                 505                 510

Phe Ile Cys Leu Lys Arg Leu Asn Leu Ala Glu Asn Arg Leu Ser His
        515                 520                 525

Leu Pro Ala Trp Thr Gln Ala Val Ser Leu Glu Val Leu Asp Leu Arg
    530                 535                 540

Asn Asn Ser Phe Ser Leu Leu Pro Gly Ser Ala Met Gly Gly Leu Glu
545                 550                 555                 560

Thr Ser Leu Arg Arg Leu Tyr Leu Gln Gly Asn Pro Leu Ser Cys Cys
                565                 570                 575

Gly Asn Gly Trp Leu Ala Ala Gln Leu His Gln Gly Arg Val Asp Val
            580                 585                 590

Asp Ala Thr Gln Asp Leu Ile Cys Arg Phe Ser Ser Gln Glu Glu Val
        595                 600                 605

Ser Leu Ser His Val Arg Pro Glu Asp Cys Glu Lys Gly Gly Leu Lys
    610                 615                 620

Asn Ile Asn Leu Ile Ile Ile Leu Thr Phe Ile Leu Val Ser Ala Ile
625                 630                 635                 640

Leu Leu Thr Thr Leu Ala Ala Cys Cys Cys Val Arg Arg Gln Lys Phe
                645                 650                 655

Asn Gln Gln Tyr Lys Ala
            660


<210> 232
<211> 517
<212> PRT
<213> Drosophila melanogaster

<400> 232
Met Gln Arg Thr Asn Ile Glu Leu Glu Arg Gln Arg His Leu Ala Asn
  1               5                  10                  15

Trp Leu Met Asn Ser Arg Pro His Met Pro Gly Gly Pro Ala Ala Ala
             20                  25                  30

Thr Thr Thr Ser Ala Ala Ala Ala Thr Thr Pro Thr Ala Ser Ala Thr
         35                  40                  45

Lys Gly Ala Thr Thr Thr Thr Ala Thr Thr Thr Asn Pro Gly Ser Thr
     50                  55                  60

Val Gly Gly Ala Gly Ala Gly Pro Val Gly Gly Asn Gly Val Ala Thr
 65                  70                  75                  80

Ala Ala Ala Ala Gly Ala Pro Ser Asn Ser Val Ala Ala Ala Ala Ala
                 85                  90                  95

Ala Ala Cys Asn Gly Gly Ala Ala Val Gly Ala His His Gly His His
            100                 105                 110

Ser Ala Ala Gly Ala Arg Lys Pro Lys Leu Arg Arg Phe Asn Ser His
        115                 120                 125

Asp Thr Ser Ser Asn Met Phe Ser Val Ala Asp Phe Glu Asn Ala Arg
    130                 135                 140

Leu Ala Arg Arg Asn Glu Ile Glu Leu Asn Gln Arg Arg Ala Arg Arg
145                 150                 155                 160

Val Arg Gly Ser Ala Asn Asn Ser Thr Tyr Gly Leu Gly Ser Gly Ser
                165                 170                 175

Gly Leu Leu Gly Gly Ala His Ser Gly Tyr Cys Asp Ala Leu Asn Gly
            180                 185                 190

Ser Gly Asp Tyr Ser Thr Gly Asp Ser Lys Ala Ser Lys Gly Ser Ser
        195                 200                 205

Glu Gly Ala Leu Thr Ala Ile Glu Phe Leu Glu Arg Asn Ser Leu Pro
    210                 215                 220

Arg Val Val Arg Ile Leu His Ala Ser Lys Ser Ser Asn Glu Thr Leu
225                 230                 235                 240

Gln Ser Ser Ser Ser His Gly Ser Ser Thr Ala Ala Ser Ser Ser Ser
                245                 250                 255

Ser Thr Gly Ala Asn Pro Phe Gly Ser Gly Gly Ser Gly Gly Gly Asn
            260                 265                 270

Ile Ser Ser Gly Asn Ile Asp Thr Gly Thr His Ser Ser Ser Ser Gly
        275                 280                 285

Gly Ser Ala Thr Gly Asn Gly Thr Gly Ser Gly Pro Gly Leu Ser Ser
    290                 295                 300

Gly Ser Gly Ser Gly Ser Gly Ser Gly His Gln Asn Asn Asn Gly Arg
305                 310                 315                 320

His Ser Ser Asn Gly Gly Thr Pro Pro Gly His Asp Glu Leu Phe Leu
                325                 330                 335

Leu Tyr Arg Leu Val Arg Gln Arg Asn Ile Tyr His Gly His Asn Ala
            340                 345                 350

Lys Thr Gln Ala Ser Gln Arg Lys Lys Thr Leu Leu Ile Pro Gln Glu
        355                 360                 365

Phe Pro Gly Tyr Phe Ser Met Leu Ser Glu Lys Gly Leu Pro Thr Ala
    370                 375                 380

Thr Gln Tyr Gly Ser Leu Ile Gln Leu Val Arg Glu Arg Val Tyr Lys
385                 390                 395                 400

Phe Val Ser Val Asp Asn Met Pro Ala Phe Thr Glu Ser Ser Pro Asn
                405                 410                 415

Ser Thr Ser Ser Pro Thr His Glu Gly Cys Leu Pro Ile Asn Lys Gly
            420                 425                 430

Arg Pro Gln Tyr Val Lys Thr Thr Ala Arg Gly Gly Gln Val Phe Arg
        435                 440                 445

Leu Leu Ala Val Phe Glu Asp Gly Lys Gln Asp Gln Val Asn Met Ser
    450                 455                 460

Gln His Gly Asn Gly Asn Ala Thr Arg Ser His Gly Ser Gly Tyr Met
465                 470                 475                 480

Gly Arg Glu Lys Glu Lys Asn Arg Tyr Ala Gln Leu Leu Asn Glu Asn
                485                 490                 495

Arg Gln Val Leu Tyr Val Pro Leu Ser Thr Lys Gly Lys Phe Tyr Glu
            500                 505                 510

Ile Glu Pro Gly Ile
        515


<210> 233
<211> 189
<212> PRT
<213> Drosophila melanogaster

<400> 233
Met Glu Ile Pro Asp Thr Tyr Tyr Ser Lys Asp Glu Tyr Val Glu Thr
  1               5                  10                  15

Ala Ser Gly Asn Lys Val Ser Arg His Thr Val Leu Cys Gly Ser Gln
             20                  25                  30

Asn Ile Ile Leu Asn Gly Lys Val Ile Val Gln Ser Gly Ala Ile Ile
         35                  40                  45

Arg Gly Asp Leu Ala Asn Val Arg Thr Gly Arg Tyr Cys Val Ile Gly
     50                  55                  60

Lys Asn Ser Val Ile Arg Pro Pro Tyr Lys Gln Phe Ser Lys Gly Ile
 65                  70                  75                  80

Ala Phe Phe Pro Met His Val Gly Glu His Val Phe Val Gly Glu Gly
                 85                  90                  95

Ala Val Val Ser Ala Ala Thr Ile Gly Ser Tyr Val Tyr Ile Gly Lys
            100                 105                 110

Asn Ala Ile Ile Gly Arg Arg Cys Val Leu Lys Asp Cys Cys Val Ile
        115                 120                 125

Glu Asp Gly Ala Val Leu Pro Pro Glu Thr Thr Val Ser Ser Tyr Met
    130                 135                 140

Arg Tyr Thr Ala Arg Gly Thr Ile Glu Gly Gly Gln Gly Asn Pro Tyr
145                 150                 155                 160

Phe Val Pro Ala Ala Met Gln Asp Glu Met Ile Asn Tyr Thr Lys Ser
                165                 170                 175

Phe Tyr Glu His Phe Val Arg Ala Pro Ala Pro Ala Ser
            180                 185


<210> 234
<211> 1348
<212> PRT
<213> Drosophila melanogaster

<400> 234
Met Gly Gly Lys Pro Val Ser Pro Thr Glu Arg Leu Gln Arg Arg Glu
  1               5                  10                  15

Leu Ala Gln Lys Arg Ser Pro Arg Gln Lys Gln Lys Thr Ala Lys Leu
             20                  25                  30

Pro Ser Ser Gln Ala Thr Lys Ser Leu Lys Cys Asn Leu Gln Ala Ala
         35                  40                  45

Pro Lys Thr Glu Thr Glu Asn Thr Phe Gly Gln Leu Lys Leu Thr Ile
    50                  55                  60

Glu Glu Leu Asp Leu Ser Tyr Asn Leu Ile Arg Arg Ile Pro Glu Lys
65                  70                  75                  80

Ala Phe Asp Gly Leu Lys Asp Ser Leu Asn Glu Leu Arg Leu Ala Asn
                85                  90                  95

Asn Leu Leu Gly Asp Asn Leu Asn Pro Ile Phe Ser Thr Ala Glu Leu
            100                 105                 110

His Val Leu Lys Asn Leu Arg Leu Leu Asp Leu Ser Gly Asn Lys Ile
        115                 120                 125

Lys Leu Ile Glu Glu Gly Leu Leu Lys Gly Cys Met Asp Leu Lys Glu
    130                 135                 140

Phe Tyr Ile Asp Arg Asn Ser Leu Thr Ser Val Pro Thr Asn Ser Leu
145                 150                 155                 160

Asn Gly Pro Ser Ala Leu Arg His Leu Ser Leu Arg Gln Asn Gln Ile
                165                 170                 175

Gly Ser Leu Leu Ala Asp Ser Phe Asn Ala Gln Arg Gln Leu Glu Ile
            180                 185                 190

Ile Asp Leu Arg His Asn Val Ile Arg Ser Ile Asp Ser Leu Ala Phe
        195                 200                 205

Lys Gly Leu Gln Lys Ile Arg Glu Ile Lys Leu Ala Gly Asn Arg Ile
    210                 215                 220

Ser His Leu Asn Ser Asp Val Phe Glu Lys Leu Gln Ser Leu Gln Lys
225                 230                 235                 240

Leu Asp Leu Ser Glu Asn Phe Phe Gly Gln Phe Pro Thr Val Ala Leu
                245                 250                 255

Ala Ala Val Pro Gly Leu Lys His Leu Asn Leu Ser Ser Asn Met Leu
            260                 265                 270

Gln Gln Leu Asp Tyr Thr His Met Gln Val Val Arg Ser Leu Glu Ser
        275                 280                 285

Leu Asp Ile Ser Arg Asn Thr Ile Thr Thr Ile Thr Pro Gly Thr Phe
    290                 295                 300

Arg Glu Met Gly Ala Leu Lys Tyr Leu Asp Leu Ser Leu Asn Ser Leu
305                 310                 315                 320

Arg Thr Ile Glu Asp Asp Ala Leu Glu Gly Leu Asp Ser Leu Gln Thr
                325                 330                 335

Leu Ile Ile Lys Asp Asn Asn Ile Leu Leu Val Pro Gly Ser Ala Leu
            340                 345                 350

Gly Arg Leu Pro Gln Leu Thr Ser Leu Gln Leu Asp Tyr Asn Arg Val
        355                 360                 365

Ala Ala Leu Ser Ala Glu Ile Leu Gly Ser Leu Gln Ala Gly Asp Ile
    370                 375                 380

Thr Thr Leu Ser Leu Ser Arg Asn Val Ile Arg Glu Leu Pro Pro Gly
385                 390                 395                 400

Ser Phe Gln Met Phe Ser Ser Leu His Thr Leu Asp Leu Ser Gly Asn
                405                 410                 415

Ser Leu Ala Val Ile Asn Ala Asp Thr Phe Ala Gly Leu Glu Ser Thr
            420                 425                 430

Leu Met Ala Leu Lys Leu Ser Gln Asn Arg Leu Thr Gly Leu Gly Gly
        435                 440                 445

Ala Pro Trp Val Leu Pro Glu Leu Arg Ser Leu Asp Leu Ser Gly Asn
    450                 455                 460

Thr Leu Thr Glu Leu Pro Ser Thr Ile Phe Glu Glu Leu Glu Asn Val
465                 470                 475                 480

Gln Ser Leu Asn Leu Ser Gly Asn His Leu Thr Pro Leu Thr Gly Ala
                485                 490                 495

Leu Phe Lys Pro Leu Asp Arg Leu Gln Val Ile Asp Leu Ser Gly Cys
            500                 505                 510

Asn Ile Arg Gln Ile Ser Gly Asp Leu Leu Ala Gly Leu Gln Asp Leu
        515                 520                 525

Lys His Ile Tyr Leu Asn Asp Asn Gln Leu Gln Glu Leu Gln Asp Gly
    530                 535                 540

Ser Phe Val Asn Leu Trp Asn Ile Ser Ser Ile Asp Leu Ser Asn Asn
545                 550                 555                 560

Arg Ile Gly Ser Ile Arg Ser Gly Ala Phe Val Asn Val Met Lys Leu
                565                 570                 575

Gln Lys Leu Asp Leu His Gly Asn Gln Leu Ser Ala Phe Lys Gly Glu
            580                 585                 590

Tyr Phe Asn Thr Gly Thr Gly Ile Glu Glu Leu Asp Ile Ser Asp Asn
        595                 600                 605

Gln Leu Ser Tyr Leu Phe Pro Ser Ser Phe Arg Ile His Pro Arg Leu
    610                 615                 620

Arg Glu Ile Arg Ala Ala Asn Asn Lys Phe Ser Phe Phe Pro Ala Glu
625                 630                 635                 640

Leu Ile Ser Thr Leu Gln Tyr Leu Glu His Ile Asp Leu Ser His Asn
                645                 650                 655

Gln Leu Lys Thr Ile Glu Glu Leu Asp Phe Ala Arg Leu Pro Arg Leu
            660                 665                 670

Arg Val Leu Leu Val Ala Asn Asn Gln Leu Asp Met Val Ser Glu Met
        675                 680                 685

Ala Phe His Asn Ser Thr Gln Leu Gln Ile Leu Asp Leu Ala His Asn
    690                 695                 700

Asn Leu Asp Arg Ile Gly Glu Arg Thr Phe Glu Gly Leu Val Arg Leu
705                 710                 715                 720

Glu Gln Leu Asn Leu Glu Gly Asn Arg Leu Ser Glu Leu Ser Asp Gly
                725                 730                 735

Val Phe Glu Arg Thr Lys Leu Gln Met Leu Glu Asn Ile Asn Leu Ala
            740                 745                 750

His Asn Arg Phe Glu Tyr Ala Pro Leu Asn Ala Leu Gln Arg Gln Phe
        755                 760                 765

Phe Phe Val Ser Ser Val Asp Leu Ser His Asn Lys Ile Lys Glu Leu
    770                 775                 780

Pro Gly Asp Asp Ser Ile Met Val Asn Ile Lys Arg Ile Asp Leu Ser
785                 790                 795                 800

Phe Asn Pro Leu Ser Ser Lys Ala Val His Asn Val Leu Asn Glu Pro
                805                 810                 815

Lys Thr Val Arg Glu Leu Ser Leu Ala Gly Thr Gly Ile Glu Asn Leu
            820                 825                 830

Glu Leu Leu Glu Thr Pro Phe Leu Gln Phe Leu Asn Leu Ser His Asn
        835                 840                 845

Lys Leu Lys Asn Val Lys Pro Glu Val Phe Gln Arg Val Thr Leu Leu
    850                 855                 860

Glu Thr Leu Asp Leu Ser Ser Asn Gln Leu Glu Ser Leu Glu Asp Leu
865                 870                 875                 880

Ser Met Ala Trp Pro Gln Leu Gln Val Leu Gln Ser Leu Asp Val Ser
                885                 890                 895

Asn Asn Ser Phe Glu Ile Val Ser Gln Ser Asn Phe Gly Lys Leu Glu
            900                 905                 910

Met Leu Arg Ser Leu Arg Leu Ser His Leu Pro Gln Cys Thr Arg Ile
        915                 920                 925

Glu Lys Asn Ala Phe Lys Gln Leu Pro Asn Leu Val Ser Leu Glu Ala
    930                 935                 940

Tyr Asp Leu Pro Leu Leu Gly Tyr Leu Asp Leu Gln Gly Ile Leu Glu
945                 950                 955                 960

Leu Leu Pro Gly Leu Glu Val Leu Asp Ile Glu Val Lys Asp Ser Ser
                965                 970                 975

Ile Gly Ser Glu Gln Ile Gln Pro Leu Lys His Pro Arg Leu Lys Ser
            980                 985                 990

Leu Gly Ile Arg Gly Asp Arg Leu Lys Ser Ile Ser Ser Gly Thr Leu
        995                 1000                1005

Ala Gly Leu Lys Ser Asn Asp Leu Ser Val Gln Leu Arg Asn Thr Ser
    1010                1015                1020

Leu Asn Ala Leu Pro Pro Ala Leu Leu Phe Pro Val Pro Arg Ser Ser
1025                1030                1035                1040

His Leu Ser Leu Asn Val Glu Gly Ser Lys Ile Thr Val Leu Val Pro
                1045                1050                1055

Gln Phe Leu Asn Ala Leu Glu Asp Arg Arg Ala Ser Leu Gln Leu Gln
            1060                1065                1070

Gly Leu Ala Ser Asn Pro Ile Val Cys Asp Cys Asn Ala Arg Ala Leu
        1075                1080                1085

Arg Arg Trp Leu Pro Ser Ser Gly Met Pro Asp Val Thr Cys Ala Ser
    1090                1095                1100

Pro Ala Tyr Leu Leu Asn Arg Lys Leu Ile Glu Val Gly Asp Asp Glu
1105                1110                1115                1120

Leu Thr Cys Asp Ala Arg Arg Met Thr Ser Ser Thr Ser Arg Pro Thr
                1125                1130                1135

Ala Ser Val Pro His Leu Leu Lys Thr Ser Ser Gln Leu Val Thr Arg
            1140                1145                1150

Ser Ser Ser Thr Thr Glu Glu Pro Leu Ile Ile Trp Ser Leu Glu Pro
        1155                1160                1165

Thr Gln Pro Pro Ser Leu Lys Lys Met Lys Thr Lys Ala Pro Leu Met
    1170                1175                1180

Lys Ala Gln Ser Pro Ile Ile Ser Asn Asp Asp Thr Leu Ile Ile Gly
1185                1190                1195                1200

Ile Val Gly Gly Val Val Ala Phe Ile Ala Ile Leu Ile Ile Ile Ile
                1205                1210                1215

Cys Ile Ile Arg Leu Arg Met Ser Asn Ala Glu Tyr Gln Gln Asn Ala
            1220                1225                1230

Thr Met Ile Gly Ile Pro Ala Gly Met Gln Met Gly Ala His Asn Ala
        1235                1240                1245

Ala Tyr Asn Tyr Lys Asn Gly Ala Gly Ala Ala Leu Tyr Ala Val Pro
    1250                1255                1260

Pro Tyr His Ala Thr Leu Pro His Lys Ala Ala Ser Ile His Gln Ser
1265                1270                1275                1280

Ser Gln Asn Leu Ser Gln Arg Gln Gln Gln Gln Gln Gln Gln Gln Gln
                1285                1290                1295

Val Ala Ala Ala Ala Ala Ala Tyr Ser Thr Met Ser Arg Met Ser Tyr
            1300                1305                1310

Phe Ser Gly Ala Gly Gly Gly Asn Gly Asp Gly Ala Glu Ser Leu Thr
        1315                1320                1325

His Gln His Pro His Gln His Gln Pro Tyr Ile Ile Tyr Ser Asp Asp
    1330                1335                1340

Lys Ala Tyr Arg
1345


<210> 235
<211> 804
<212> PRT
<213> Homo sapiens

<400> 235
Ser Pro Glu Gly Ala Glu Ala Ala Gly Asp Pro Ser Gln Pro Arg Ser
1               5                   10                  15

Cys Phe Arg Ala Thr Ile Asp Glu Val Glu Thr Asp Val Val Glu Ile
            20                  25                  30

Glu Ala Lys Leu Asp Lys Leu Val Lys Leu Cys Ser Gly Met Val Glu
        35                  40                  45

Ala Gly Lys Ala Tyr Val Ser Thr Ser Arg Leu Phe Val Ser Gly Val
    50                  55                  60

Arg Asp Leu Ser Gln Gln Cys Gln Gly Asp Thr Val Ile Ser Glu Cys
65                  70                  75                  80

Leu Gln Arg Phe Ala Asp Ser Leu Gln Glu Val Val Asn Tyr His Met
                85                  90                  95

Ile Leu Phe Asp Gln Ala Gln Arg Ser Val Arg Gln Gln Leu Gln Ser
            100                 105                 110

Phe Val Lys Glu Asp Val Arg Lys Phe Lys Glu Thr Lys Lys Gln Phe
        115                 120                 125

Asp Lys Val Arg Glu Asp Leu Glu Leu Ser Leu Val Arg Asn Ala Gln
    130                 135                 140

Ala Pro Arg His Arg Pro His Glu Val Glu Glu Ala Thr Gly Ala Leu
145                 150                 155                 160

Thr Leu Thr Arg Lys Cys Phe Arg His Leu Ala Leu Asp Tyr Val Leu
                165                 170                 175

Gln Ile Asn Val Leu Gln Ala Lys Lys Lys Phe Glu Ile Leu Asp Ser
            180                 185                 190

Met Leu Ser Phe Met His Ala Gln Ser Ser Phe Phe Gln Gln Gly Tyr
        195                 200                 205

Ser Leu Leu His Gln Leu Asp Pro Tyr Met Lys Lys Leu Ala Ala Glu
    210                 215                 220

Leu Asp Gln Leu Val Ile Asp Ser Ala Val Glu Lys Arg Glu Met Glu
225                 230                 235                 240

Arg Lys His Ala Ala Ile Gln Gln Arg Thr Leu Leu Gln Asp Phe Ser
                245                 250                 255

Tyr Asp Glu Ser Lys Val Glu Phe Asp Val Asp Ala Pro Ser Gly Val
            260                 265                 270

Val Met Glu Gly Tyr Leu Phe Lys Arg Ala Ser Asn Ala Phe Lys Thr
        275                 280                 285

Trp Asn Arg Arg Trp Phe Ser Ile Gln Asn Ser Gln Leu Val Tyr Gln
    290                 295                 300

Lys Lys Leu Lys Asp Ala Leu Thr Val Val Val Asp Asp Leu Arg Leu
305                 310                 315                 320

Cys Ser Val Lys Pro Cys Glu Asp Ile Glu Arg Arg Phe Cys Phe Glu
                325                 330                 335

Val Leu Ser Pro Thr Lys Ser Cys Met Leu Gln Ala Asp Ser Glu Lys
            340                 345                 350

Leu Arg Gln Ala Trp Val Gln Ala Val Gln Ala Ser Ile Ala Ser Ala
        355                 360                 365

Tyr Arg Glu Ser Pro Asp Ser Cys Tyr Ser Glu Arg Leu Asp Arg Thr
    370                 375                 380

Ala Ser Pro Ser Thr Ser Ser Ile Asp Ser Ala Thr Asp Thr Arg Glu
385                 390                 395                 400

Arg Gly Val Lys Gly Glu Ser Val Leu Gln Arg Val Gln Ser Val Ala
                405                 410                 415

Gly Asn Ser Gln Cys Gly Asp Cys Gly Gln Pro Asp Pro Arg Trp Ala
            420                 425                 430

Ser Ile Asn Leu Gly Val Leu Leu Cys Ile Glu Cys Ser Gly Ile His
        435                 440                 445

Arg Ser Leu Gly Val His Cys Ser Lys Val Arg Ser Leu Thr Leu Asp
    450                 455                 460

Ser Trp Glu Pro Glu Leu Leu Lys Leu Met Cys Glu Leu Gly Asn Ser
465                 470                 475                 480

Ala Val Asn Gln Ile Tyr Glu Ala Gln Cys Glu Gly Ala Gly Ser Arg
                485                 490                 495

Lys Pro Thr Ala Ser Ser Ser Arg Gln Asp Lys Glu Ala Trp Ile Lys
            500                 505                 510

Asp Lys Tyr Val Glu Lys Lys Phe Leu Arg Lys Ala Pro Met Ala Pro
        515                 520                 525

Ala Leu Glu Ala Pro Arg Arg Trp Arg Val Gln Lys Cys Leu Arg Pro
    530                 535                 540

His Ser Ser Pro Arg Ala Pro Thr Ala Arg Arg Lys Val Arg Leu Glu
545                 550                 555                 560

Pro Val Leu Pro Cys Val Ala Ala Leu Ser Ser Val Gly Thr Leu Asp
                565                 570                 575

Arg Lys Phe Arg Arg Asp Ser Leu Phe Cys Pro Asp Glu Leu Asp Ser
            580                 585                 590

Leu Phe Ser Tyr Phe Asp Ala Gly Ala Ala Gly Ala Gly Pro Arg Lys
        595                 600                 605

Gly Ala Glu Ser Glu Glu Ser Ser Gly Glu Ala Asp Gly Asp Thr Glu
    610                 615                 620

Ala Glu Ala Trp Gly Leu Ala Asp Val Arg Glu Leu His Pro Gly Leu
625                 630                 635                 640

Leu Ala His Arg Ala Ala Arg Ala Arg Asp Leu Pro Ala Leu Ala Ala
                645                 650                 655

Ala Leu Ala His Gly Ala Glu Val Asn Trp Ala Asp Ala Glu Asp Glu
            660                 665                 670

Gly Lys Thr Pro Leu Val Gln Ala Val Leu Gly Gly Ser Leu Ile Val
        675                 680                 685

Cys Glu Phe Leu Leu Gln Asn Gly Ala Asp Val Asn Gln Arg Asp Ser
    690                 695                 700

Arg Gly Arg Ala Pro Leu His His Ala Thr Leu Leu Gly Arg Thr Gly
705                 710                 715                 720

Gln Val Cys Leu Phe Leu Lys Arg Gly Ala Asp Gln His Ala Leu Asp
                725                 730                 735

Gln Glu Gln Arg Asp Pro Leu Ala Ile Ala Val Gln Ala Ala Asn Ala
            740                 745                 750

Asp Ile Val Thr Leu Leu Arg Leu Ala Arg Met Ala Glu Glu Met Arg
        755                 760                 765

Glu Ala Glu Ala Ala Pro Gly Pro Pro Gly Ala Leu Ala Gly Ser Pro
    770                 775                 780

Thr Glu Leu Gln Phe Arg Arg Cys Ile Gln Glu Phe Ile Ser Leu His
785                 790                 795                 800

Leu Glu Glu Ser


<210> 236
<211> 759
<212> PRT
<213> Homo sapiens

<400> 236
Met Val Glu Ala Gly Lys Ala Tyr Val Ser Thr Ser Arg Leu Phe Val

1               5                   10                  15

Ser Gly Val Arg Asp Leu Ser Gln Gln Cys Gln Gly Asp Thr Val Ile
                20                  25                  30

Ser Glu Cys Leu Gln Arg Phe Ala Asp Ser Leu Gln Glu Val Val Asn
            35                  40                  45

Tyr His Met Ile Leu Phe Asp Gln Ala Gln Arg Ser Val Arg Gln Gln
        50                  55                  60

Leu Gln Ser Phe Val Lys Glu Asp Val Arg Lys Phe Lys Glu Thr Lys
65                  70                  75                  80

Lys Gln Phe Asp Lys Val Arg Glu Asp Leu Glu Leu Ser Leu Val Arg
                85                  90                  95

Asn Ala Gln Ala Pro Arg His Arg Pro His Glu Val Glu Glu Ala Thr
            100                 105                 110

Gly Ala Leu Thr Leu Thr Arg Lys Cys Phe Arg His Leu Ala Leu Asp
        115                 120                 125

Tyr Val Leu Gln Ile Asn Val Leu Gln Ala Lys Lys Lys Phe Glu Ile
    130                 135                 140

Leu Asp Ser Met Leu Ser Phe Met His Ala Gln Ser Ser Phe Phe Gln
145                 150                 155                 160

Gln Gly Tyr Ser Leu Leu His Gln Leu Asp Pro Tyr Met Lys Lys Leu
                165                 170                 175

Ala Ala Glu Leu Asp Gln Leu Val Ile Asp Ser Ala Val Glu Lys Arg
            180                 185                 190

Glu Met Glu Arg Lys His Ala Ala Ile Gln Gln Arg Thr Leu Leu Gln
        195                 200                 205

Asp Phe Ser Tyr Asp Glu Ser Lys Val Glu Phe Asp Val Asp Ala Pro
    210                 215                 220

Ser Gly Val Val Met Glu Gly Tyr Leu Phe Lys Arg Ala Ser Asn Ala
225                 230                 235                 240

Phe Lys Thr Trp Asn Arg Arg Trp Phe Ser Ile Gln Asn Ser Gln Leu
                245                 250                 255

Val Tyr Gln Lys Lys Leu Lys Asp Ala Leu Thr Val Val Val Asp Asp
            260                 265                 270

Leu Arg Leu Cys Ser Val Lys Pro Cys Glu Asp Ile Glu Arg Arg Phe
        275                 280                 285

Cys Phe Glu Val Leu Ser Pro Thr Lys Ser Cys Met Leu Gln Ala Asp
    290                 295                 300

Ser Glu Lys Leu Arg Gln Ala Trp Val Gln Ala Val Gln Ala Ser Ile
305                 310                 315                 320

Ala Ser Ala Tyr Arg Glu Ser Pro Asp Ser Cys Tyr Ser Glu Arg Leu
                325                 330                 335

Asp Arg Thr Ala Ser Pro Ser Thr Ser Ser Ile Asp Ser Ala Thr Asp
            340                 345                 350

Thr Arg Glu Arg Gly Val Lys Gly Glu Ser Val Leu Gln Arg Val Gln
        355                 360                 365

Ser Val Ala Gly Asn Ser Gln Cys Gly Asp Cys Gly Gln Pro Asp Pro

370                 375                 380

Arg Trp Ala Ser Ile Asn Leu Gly Val Leu Leu Cys Ile Glu Cys Ser
385                 390                 395                 400

Gly Ile His Arg Ser Leu Gly Val His Cys Ser Lys Val Arg Ser Leu
                405                 410                 415

Thr Leu Asp Ser Trp Glu Pro Glu Leu Leu Lys Leu Met Cys Glu Leu
            420                 425                 430

Gly Asn Ser Ala Val Asn Gln Ile Tyr Glu Ala Gln Cys Glu Gly Ala
        435                 440                 445

Gly Ser Arg Lys Pro Thr Ala Ser Ser Ser Arg Gln Asp Lys Glu Ala
    450                 455                 460

Trp Ile Lys Asp Lys Tyr Val Glu Lys Lys Phe Leu Arg Lys Ala Pro
465                 470                 475                 480

Met Ala Pro Ala Leu Glu Ala Pro Arg Arg Trp Arg Val Gln Lys Cys
                485                 490                 495

Leu Arg Pro His Ser Ser Pro Arg Ala Pro Thr Ala Arg Arg Lys Val
            500                 505                 510

Arg Leu Glu Pro Val Leu Pro Cys Val Ala Ala Leu Ser Ser Val Gly
        515                 520                 525

Thr Leu Asp Arg Lys Phe Arg Arg Asp Ser Leu Phe Cys Pro Asp Glu
    530                 535                 540

Leu Asp Ser Leu Phe Ser Tyr Phe Asp Ala Gly Ala Ala Gly Ala Gly
545                 550                 555                 560

Pro Arg Lys Gly Ala Glu Ser Glu Glu Ser Ser Gly Glu Ala Asp Gly
                565                 570                 575

Asp Thr Glu Ala Glu Ala Trp Gly Leu Ala Asp Val Arg Glu Leu His
            580                 585                 590

Pro Gly Leu Leu Ala His Arg Ala Ala Arg Ala Arg Asp Leu Pro Ala
        595                 600                 605

Leu Ala Ala Ala Leu Ala His Gly Ala Glu Val Asn Trp Ala Asp Ala
    610                 615                 620

Glu Asp Glu Gly Lys Thr Pro Leu Val Gln Ala Val Leu Gly Gly Ser
625                 630                 635                 640

Leu Ile Val Cys Glu Phe Leu Leu Gln Asn Gly Ala Asp Val Asn Gln
                645                 650                 655

Arg Asp Ser Arg Gly Arg Ala Pro Leu His His Ala Thr Leu Leu Gly
            660                 665                 670

Arg Thr Gly Gln Val Cys Leu Phe Leu Lys Arg Gly Ala Asp Gln His
        675                 680                 685

Ala Leu Asp Gln Glu Gln Arg Asp Pro Leu Ala Ile Ala Val Gln Ala
    690                 695                 700

Ala Asn Ala Asp Ile Val Thr Leu Leu Arg Leu Ala Arg Met Ala Glu
705                 710                 715                 720

Glu Met Arg Glu Ala Glu Ala Ala Pro Gly Pro Pro Gly Ala Leu Ala
                725                 730                 735

Gly Ser Pro Thr Glu Leu Gln Phe Arg Arg Cys Ile Gln Glu Phe Ile

740                 745                 750

Ser Leu His Leu Glu Glu Ser
        755

<210> 237
<211> 834
<212> PRT
<213> Homo sapiens

<400> 237
Met Thr Val Glu Phe Glu Glu Cys Val Lys Asp Ser Pro Arg Phe Arg
1               5                   10                  15

Ala Thr Ile Asp Glu Val Glu Thr Asp Val Val Glu Ile Glu Ala Lys
            20                  25                  30

Leu Asp Lys Leu Val Lys Leu Cys Ser Gly Met Val Glu Ala Gly Lys
        35                  40                  45

Ala Tyr Val Ser Thr Ser Arg Leu Phe Val Ser Gly Val Arg Asp Leu
    50                  55                  60

Ser Gln Gln Cys Gln Gly Asp Thr Val Ile Ser Glu Cys Leu Gln Arg
65                  70                  75                  80

Phe Ala Asp Ser Leu Gln Glu Val Val Asn Tyr His Met Ile Leu Phe
                85                  90                  95

Asp Gln Ala Gln Arg Ser Val Arg Gln Gln Leu Gln Ser Phe Val Lys
            100                 105                 110

Glu Asp Val Arg Lys Phe Lys Glu Thr Lys Lys Gln Phe Asp Lys Val
        115                 120                 125

Arg Glu Asp Leu Glu Leu Ser Leu Val Arg Asn Ala Gln Ala Pro Arg
    130                 135                 140

His Arg Pro His Glu Val Glu Glu Ala Thr Gly Ala Leu Thr Leu Thr
145                 150                 155                 160

Arg Lys Cys Phe Arg His Leu Ala Leu Asp Tyr Val Leu Gln Ile Asn
                165                 170                 175

Val Leu Gln Ala Lys Lys Lys Phe Glu Ile Leu Asp Ser Met Leu Ser
            180                 185                 190

Phe Met His Ala Gln Ser Ser Phe Phe Gln Gln Gly Tyr Ser Leu Leu
        195                 200                 205

His Gln Leu Asp Pro Tyr Met Lys Lys Leu Ala Ala Glu Leu Asp Gln
    210                 215                 220

Leu Val Ile Asp Ser Ala Val Glu Lys Arg Glu Met Glu Arg Lys His
225                 230                 235                 240

Ala Ala Ile Gln Gln Arg Thr Leu Leu Gln Asp Phe Ser Tyr Asp Glu
                245                 250                 255

Ser Lys Val Glu Phe Asp Val Asp Ala Pro Ser Gly Val Val Met Glu
            260                 265                 270

Gly Tyr Leu Phe Lys Arg Ala Ser Asn Ala Phe Lys Thr Trp Asn Arg
        275                 280                 285

Arg Trp Phe Ser Ile Gln Asn Ser Gln Leu Val Tyr Gln Lys Lys Leu
    290                 295                 300

Lys Asp Ala Leu Thr Val Val Val Asp Asp Leu Arg Leu Cys Ser Val
305                 310                 315                 320

Lys Pro Cys Glu Asp Ile Glu Arg Arg Phe Cys Phe Glu Val Leu Ser
                325                 330                 335

Pro Thr Lys Ser Cys Met Leu Gln Ala Asp Ser Glu Lys Leu Arg Gln
            340                 345                 350

Ala Trp Val Gln Ala Val Gln Ala Ser Ile Ala Ser Ala Tyr Arg Glu
        355                 360                 365

Ser Pro Asp Ser Cys Tyr Ser Glu Arg Leu Asp Arg Thr Ala Ser Pro
    370                 375                 380

Ser Thr Ser Ser Ile Asp Ser Ala Thr Asp Thr Arg Glu Arg Gly Val
385                 390                 395                 400

Lys Gly Glu Ser Val Leu Gln Arg Val Gln Ser Val Ala Gly Asn Ser
                405                 410                 415

Gln Cys Gly Asp Cys Gly Gln Pro Asp Pro Arg Trp Ala Ser Ile Asn
            420                 425                 430

Leu Gly Val Leu Leu Cys Ile Glu Cys Ser Gly Ile His Arg Ser Leu
        435                 440                 445

Gly Val His Cys Ser Lys Val Arg Ser Leu Thr Leu Asp Ser Trp Glu
    450                 455                 460

Pro Glu Leu Leu Lys Leu Met Cys Glu Leu Gly Asn Ser Ala Val Asn
465                 470                 475                 480

Gln Ile Tyr Glu Ala Gln Cys Glu Gly Ala Gly Ser Arg Lys Pro Thr
                485                 490                 495

Ala Ser Ser Ser Arg Gln Asp Lys Glu Ala Trp Ile Lys Asp Lys Tyr
            500                 505                 510

Val Glu Lys Lys Phe Leu Arg Lys Ala Pro Met Ala Pro Ala Leu Glu
        515                 520                 525

Ala Pro Arg Arg Trp Arg Val Gln Lys Cys Leu Arg Pro His Ser Ser
    530                 535                 540

Pro Arg Ala Pro Thr Ala Arg Arg Lys Val Arg Leu Glu Pro Val Leu
545                 550                 555                 560

Pro Cys Val Ala Ala Leu Ser Ser Val Gly Thr Leu Asp Arg Lys Phe
                565                 570                 575

Arg Arg Asp Ser Leu Phe Cys Pro Asp Glu Leu Asp Ser Leu Phe Ser
            580                 585                 590

Tyr Phe Asp Ala Gly Ala Ala Gly Ala Gly Pro Arg Ser Leu Ser Ser
        595                 600                 605

Asp Ser Gly Leu Gly Gly Ser Ser Asp Gly Ser Ser Asp Val Leu Ala
    610                 615                 620

Phe Gly Ser Gly Ser Val Val Asp Ser Val Thr Glu Glu Glu Gly Ala
625                 630                 635                 640

Glu Ser Glu Glu Ser Ser Gly Glu Ala Asp Gly Asp Thr Glu Ala Glu
                645                 650                 655

Ala Trp Gly Leu Ala Asp Val Arg Glu Leu His Pro Gly Leu Leu Ala
            660                 665                 670

His Arg Ala Ala Arg Ala Arg Asp Leu Pro Ala Leu Ala Ala Ala Leu
        675                 680                 685

Ala His Gly Ala Glu Val Asn Trp Ala Asp Ala Glu Asp Glu Gly Lys
    690                 695                 700

Thr Pro Leu Val Gln Ala Val Leu Gly Gly Ser Leu Ile Val Cys Glu
705                 710                 715                 720

Phe Leu Leu Gln Asn Gly Ala Asp Val Asn Gln Arg Asp Ser Arg Gly
                725                 730                 735

Arg Ala Pro Leu His His Ala Thr Leu Leu Gly Arg Thr Gly Gln Val
            740                 745                 750

Cys Leu Phe Leu Lys Arg Gly Ala Asp Gln His Ala Leu Asp Gln Glu
        755                 760                 765

Gln Arg Asp Pro Leu Ala Ile Ala Val Gln Ala Ala Asn Ala Asp Ile
    770                 775                 780

Val Thr Leu Leu Arg Leu Ala Arg Met Ala Glu Glu Met Arg Glu Ala
785                 790                 795                 800

Glu Ala Ala Pro Gly Pro Pro Gly Ala Leu Ala Gly Ser Pro Thr Glu
                805                 810                 815

Leu Gln Phe Arg Arg Cys Ile Gln Glu Phe Ile Ser Leu His Leu Glu
            820                 825                 830

Glu Ser

<210> 238
<211> 778
<212> PRT
<213> Homo sapiens

<400> 238
Met Lys Met Thr Gly Asp Phe Glu Glu Cys Leu Lys Asp Ser Pro Arg
1               5                   10                  15

Phe Arg Ala Ala Leu Glu Glu Val Glu Gly Asp Val Ala Glu Leu Glu
            20                  25                  30

Leu Lys Leu Asp Lys Leu Val Lys Leu Gly Ile Ala Met Ile Asp Thr
        35                  40                  45

Gly Lys Ala Phe Cys Val Ala Asn Lys Gln Phe Met Asn Gly Ile Arg
    50                  55                  60

Asp Leu Ala Gln Tyr Ser Ser Asn Asp Ala Val Val Glu Thr Ser Leu
65                  70                  75                  80

Thr Lys Phe Ser Asp Ser Leu Gln Glu Met Ile Asn Phe His Thr Ile
                85                  90                  95

Leu Leu Pro Asn Ser Glu Ile Asn Leu Arg His Ser Phe Ser Asn Phe
            100                 105                 110

Val Lys Glu Asp Leu Arg Lys Phe Lys Asp Ala Lys Lys Gln Phe Glu
        115                 120                 125

Lys Val Ser Glu Glu Lys Glu Asn Ala Leu Val Lys Asn Ala Gln Val
    130                 135                 140

Gln Arg Asn Lys Gln His Glu Val Glu Glu Ala Thr Asn Ile Leu Thr
145                 150                 155                 160

Ala Thr Arg Lys Cys Phe Arg His Ile Ala Leu Asp Tyr Val Leu Gln
                165                 170                 175

Ile Asn Val Leu Gln Ser Lys Arg Arg Ser Glu Ile Leu Lys Ser Met
            180                 185                 190

Leu Ser Phe Met Tyr Ala His Leu Ala Phe Phe His Gln Gly Tyr Asp
        195                 200                 205

Leu Phe Ser Glu Leu Gly Pro Tyr Met Lys Asp Leu Gly Ala Gln Leu
    210                 215                 220

Asp Arg Leu Val Gly Asp Ala Ala Lys Glu Lys Arg Glu Met Glu Gln
225                 230                 235                 240

Lys His Ser Thr Ile Gln Gln Lys Asp Phe Ser Arg Asp Asp Ser Lys
                245                 250                 255

Leu Lys Tyr Asn Val Asp Ala Ala Asn Gly Ile Val Met Glu Gly Tyr
            260                 265                 270

Leu Phe Lys Arg Ala Ser Asn Ala Phe Lys Thr Trp Asn Arg Arg Trp
        275                 280                 285

Phe Ser Ile Gln Asn Asn Gln Val Val Tyr Gln Lys Lys Phe Lys Asp
    290                 295                 300

Asn Pro Thr Val Val Val Glu Asp Leu Arg Leu Cys Thr Val Lys His
305                 310                 315                 320

Cys Glu Asp Ile Glu Arg Arg Phe Cys Phe Glu Val Val Ser Pro Thr
                325                 330                 335

Lys Ser Cys Met Leu Gln Ala Asp Ser Glu Lys Leu Arg Gln Ala Trp
            340                 345                 350

Ile Lys Ala Val Gln Thr Ser Ile Ala Thr Ala Tyr Arg Glu Lys Gly
        355                 360                 365

Asp Glu Ser Glu Lys Leu Asp Lys Lys Ser Ser Pro Ser Thr Gly Ser
    370                 375                 380

Leu Asp Ser Gly Asn Glu Ser Lys Glu Lys Leu Leu Lys Gly Glu Ser
385                 390                 395                 400

Ala Leu Gln Arg Val Gln Cys Ile Pro Gly Asn Ala Ser Cys Cys Asp
                405                 410                 415

Cys Gly Leu Ala Asp Pro Arg Trp Ala Ser Ile Asn Leu Gly Ile Thr
            420                 425                 430

Leu Cys Ile Glu Cys Ser Gly Ile His Arg Ser Leu Gly Val His Phe
        435                 440                 445

Ser Lys Val Arg Ser Leu Thr Leu Asp Thr Trp Glu Pro Glu Leu Leu
    450                 455                 460

Lys Leu Met Cys Glu Leu Gly Asn Asp Val Ile Asn Arg Val Tyr Glu
465                 470                 475                 480

Ala Asn Val Glu Lys Met Gly Ile Lys Lys Pro Gln Pro Gly Gln Arg
                485                 490                 495

Gln Glu Lys Glu Ala Tyr Ile Arg Ala Lys Tyr Val Glu Arg Lys Phe
            500                 505                 510

Val Asp Lys Tyr Ser Ile Ser Leu Ser Pro Pro Glu Gln Gln Lys Lys
        515                 520                 525

Phe Val Ser Lys Ser Ser Glu Glu Lys Arg Leu Ser Ile Ser Lys Phe
    530                 535                 540

Gly Pro Gly Asp Gln Val Arg Ala Ser Ala Gln Ser Ser Val Arg Ser
545                 550                 555                 560

Asn Asp Ser Gly Ile Gln Gln Ser Ser Asp Asp Gly Arg Glu Ser Leu
                565                 570                 575

Pro Ser Thr Val Ser Ala Asn Ser Leu Tyr Glu Pro Glu Gly Glu Arg
            580                 585                 590

Gln Asp Ser Ser Met Phe Leu Asp Ser Lys His Leu Asn Pro Gly Leu
        595                 600                 605

Gln Leu Tyr Arg Ala Ser Tyr Glu Lys Asn Leu Pro Lys Met Ala Glu
    610                 615                 620

Ala Leu Ala His Gly Ala Asp Val Asn Trp Ala Asn Ser Glu Glu Asn
625                 630                 635                 640

Lys Ala Thr Pro Leu Ile Gln Ala Val Leu Gly Gly Ser Leu Val Thr
                645                 650                 655

Cys Glu Phe Leu Leu Gln Asn Gly Ala Asn Val Asn Gln Arg Asp Val
            660                 665                 670

Gln Gly Arg Gly Pro Leu His His Ala Thr Val Leu Gly His Thr Gly
        675                 680                 685

Gln Val Cys Leu Phe Leu Lys Arg Gly Ala Asn Gln His Ala Thr Asp
    690                 695                 700

Glu Glu Gly Lys Asp Pro Leu Ser Ile Ala Val Glu Ala Ala Asn Ala
705                 710                 715                 720

Asp Ile Val Thr Leu Leu Arg Leu Ala Arg Met Asn Glu Glu Met Arg
                725                 730                 735

Glu Ser Glu Gly Leu Tyr Gly Gln Pro Gly Asp Glu Thr Tyr Gln Asp
            740                 745                 750

Ile Phe Arg Asp Phe Ser Gln Met Ala Ser Asn Asn Pro Glu Lys Leu
        755                 760                 765

Asn Arg Phe Gln Gln Asp Ser Gln Lys Phe
    770                 775


<210> 239
<211> 740
<212> PRT
<213> Homo sapiens

<400> 239
Met Thr Val Lys Leu Asp Phe Glu Glu Cys Leu Lys Asp Ser Pro Arg
1               5                   10                  15

Phe Arg Ala Ser Ile Glu Leu Val Glu Ala Glu Val Ser Glu Leu Glu
            20                  25                  30

Thr Arg Leu Glu Lys Leu Leu Lys Leu Gly Thr Gly Leu Leu Glu Ser
        35                  40                  45

Gly Arg His Tyr Leu Ala Ala Ser Arg Ala Phe Val Val Gly Ile Cys
    50                  55                  60

Asp Leu Ala Arg Leu Gly Pro Pro Glu Pro Met Met Ala Glu Cys Leu

65                  70                  75                  80

Glu Lys Phe Thr Val Ser Leu Asn His Lys Leu Asp Ser His Ala Glu
                85                  90                  95

Leu Leu Asp Ala Thr Gln His Thr Leu Gln Gln Gln Ile Gln Thr Leu
            100                 105                 110

Val Lys Glu Gly Leu Arg Gly Phe Arg Glu Ala Arg Arg Asp Phe Trp
        115                 120                 125

Arg Gly Ala Glu Ser Leu Glu Ala Ala Leu Thr His Asn Ala Glu Val
    130                 135                 140

Pro Arg Arg Arg Ala Gln Glu Ala Glu Glu Ala Gly Ala Ala Leu Arg
145                 150                 155                 160

Thr Ala Arg Ala Gly Tyr Arg Gly Arg Ala Leu Asp Tyr Ala Leu Gln
                165                 170                 175

Ile Asn Val Ile Glu Asp Lys Arg Lys Phe Asp Ile Met Glu Phe Val
            180                 185                 190

Leu Arg Leu Val Glu Ala Gln Ala Thr His Phe Gln Gln Gly His Glu
        195                 200                 205

Glu Leu Ser Arg Leu Ser Gln Tyr Arg Lys Glu Leu Gly Ala Gln Leu
    210                 215                 220

His Gln Leu Val Leu Asn Ser Ala Arg Glu Lys Arg Asp Met Glu Gln
225                 230                 235                 240

Arg His Val Leu Leu Lys Gln Lys Glu Leu Gly Gly Glu Glu Pro Glu
                245                 250                 255

Pro Ser Leu Arg Glu Gly Pro Gly Gly Leu Val Met Glu Gly His Leu
            260                 265                 270

Phe Lys Arg Ala Ser Asn Ala Phe Lys Thr Trp Ser Arg Arg Trp Phe
        275                 280                 285

Thr Ile Gln Ser Asn Gln Leu Val Tyr Gln Lys Lys Tyr Lys Asp Pro
    290                 295                 300

Val Thr Val Val Val Asp Asp Leu Arg Leu Cys Thr Val Lys Leu Cys
305                 310                 315                 320

Pro Asp Ser Glu Arg Arg Phe Cys Phe Glu Val Val Ser Thr Ser Lys
                325                 330                 335

Ser Cys Leu Leu Gln Ala Asp Ser Glu Arg Leu Leu Gln Leu Trp Val
            340                 345                 350

Ser Ala Val Gln Ser Ser Ile Ala Ser Ala Phe Ser Gln Ala Arg Leu
        355                 360                 365

Asp Asp Ser Pro Arg Gly Pro Gly Gln Gly Ser Gly His Leu Ala Ile
    370                 375                 380

Gly Ser Ala Ala Thr Leu Gly Ser Gly Gly Met Ala Arg Gly Arg Glu
385                 390                 395                 400

Pro Gly Gly Val Gly His Val Val Ala Gln Val Gln Ser Val Asp Gly
                405                 410                 415

Asn Ala Gln Cys Cys Asp Cys Arg Glu Pro Ala Pro Glu Trp Ala Ser
            420                 425                 430

Ile Asn Leu Gly Val Thr Leu Cys Ile Gln Cys Ser Gly Ile His Arg

435                 440                 445

Ser Leu Gly Val His Phe Ser Lys Val Arg Ser Leu Thr Leu Asp Ser
    450                 455                 460

Trp Glu Pro Glu Leu Val Lys Leu Met Cys Glu Leu Gly Asn Val Ile
465                 470                 475                 480

Ile Asn Gln Ile Tyr Glu Ala Arg Val Glu Ala Met Ala Val Lys Lys
                485                 490                 495

Pro Gly Pro Ser Cys Ser Arg Gln Glu Lys Glu Ala Trp Ile His Ala
            500                 505                 510

Lys Tyr Val Glu Lys Lys Phe Leu Thr Lys Leu Pro Glu Ile Arg Gly
        515                 520                 525

Arg Arg Gly Gly Arg Gly Arg Pro Arg Gly Gln Pro Pro Val Pro Pro
    530                 535                 540

Lys Pro Ser Ile Arg Pro Arg Pro Gly Ser Leu Arg Ser Lys Pro Glu
545                 550                 555                 560

Pro Pro Ser Glu Asp Leu Gly Ser Leu His Pro Gly Ala Leu Leu Phe
                565                 570                 575

Arg Ala Ser Gly His Pro Pro Ser Leu Pro Thr Met Ala Asp Ala Leu
            580                 585                 590

Ala His Gly Ala Asp Val Asn Trp Val Asn Gly Gly Gln Asp Asn Ala
        595                 600                 605

Thr Pro Leu Ile Gln Ala Thr Ala Ala Asn Ser Leu Leu Ala Cys Glu
    610                 615                 620

Phe Leu Leu Gln Asn Gly Ala Asn Val Asn Gln Ala Asp Ser Ala Gly
625                 630                 635                 640

Arg Gly Pro Leu His His Ala Thr Ile Leu Gly His Thr Gly Leu Ala
                645                 650                 655

Cys Leu Phe Leu Lys Arg Gly Ala Asp Leu Gly Ala Arg Asp Ser Glu
            660                 665                 670

Gly Arg Asp Pro Leu Thr Ile Ala Met Glu Thr Ala Asn Ala Asp Ile
        675                 680                 685

Val Thr Leu Leu Arg Leu Ala Lys Met Arg Glu Ala Glu Ala Ala Gln
    690                 695                 700

Gly Gln Ala Gly Asp Glu Thr Tyr Leu Asp Ile Phe Arg Asp Phe Ser
705                 710                 715                 720

Leu Met Ala Ser Asp Asp Pro Glu Lys Leu Ser Arg Arg Ser His Asp
                725                 730                 735

Leu His Thr Leu
            740


<210> 240
<211> 122
<212> PRT
<213> Homo sapiens

<400> 240
Arg Arg Val Leu Lys Leu Leu Arg Ser Leu Asp Gly Asn Lys Lys Cys
1               5                   10                  15

Phe Asp Cys Gly Ala Pro Asn Pro Thr Trp Ala Ser Val Asn Leu Gly
            20                  25                  30

Val Phe Ile Cys Ile Glu Cys Ser Gly Ile His Arg Ser Leu Gly Val
        35                  40                  45

His Ile Ser Lys Val Arg Ser Leu Thr Leu Asp Thr Trp Thr Pro Glu
    50                  55                  60

Glu Leu Arg Lys Met Glu Lys Gly Gly Asn Glu Asn Ala Asn Ser Phe
65                  70                  75                  80

Trp Glu Ser Asn Leu Asp Asp Phe Ser Leu Lys Pro Lys Asp Ser Asp
                85                  90                  95

Asp Arg Gln Lys Tyr Glu Ser Phe Ile Ala Ala Lys Tyr Glu Glu Lys
            100                 105                 110

Leu Phe Val Leu Lys Glu Gly Lys Glu Glu
        115                 120


<210> 241
<211> 104
<212> PRT
<213> Homo sapiens

<400> 241
Val Ile Lys Glu Gly Trp Leu Leu Lys Lys Ser Ser Gly Gly Lys Lys
1               5                   10                  15

Ser Trp Lys Lys Arg Tyr Phe Val Leu Phe Asn Gly Val Leu Leu Tyr
            20                  25                  30

Tyr Lys Ser Lys Lys Lys Lys Ser Ser Ser Lys Pro Lys Gly Ser Ile
        35                  40                  45

Pro Leu Ser Gly Cys Thr Val Arg Glu Ala Pro Asp Ser Asp Ser Asp
    50                  55                  60

Lys Lys Lys Asn Cys Phe Glu Ile Val Thr Pro Asp Arg Lys Thr Leu
65                  70                  75                  80

Leu Leu Gln Ala Glu Ser Glu Glu Glu Arg Lys Glu Trp Val Glu Ala
                85                  90                  95

Leu Arg Lys Ala Ile Ala Lys Leu
            100


<210> 242
<211> 33
<212> PRT
<213> Homo sapiens

<400> 242
Asp Gly Asn Thr Pro Leu His Leu Ala Ala Arg Asn Gly His Leu Glu
1               5                   10                  15

Val Val Lys Leu Leu Leu Glu Ala Gly Ala Asp Val Asn Ala Arg Asp
            20                  25                  30

Lys


<210> 243
<211> 628
<212> PRT

<213> Homo sapiens

<400> 243
Met Ala Leu Arg Ala Arg Ala Leu Tyr Asp Phe Arg Ser Glu Asn Pro
1               5                   10                  15

Gly Glu Ile Ser Leu Arg Glu His Glu Val Leu Ser Leu Cys Ser Glu
            20                  25                  30

Gln Asp Ile Glu Gly Trp Leu Glu Gly Val Asn Ser Arg Gly Asp Arg
        35                  40                  45

Gly Leu Phe Pro Ala Ser Tyr Val Gln Val Ile Arg Ala Pro Glu Pro
    50                  55                  60

Gly Pro Ala Gly Asp Gly Gly Pro Gly Ala Pro Ala Arg Tyr Ala Asn
65                  70                  75                  80

Val Pro Pro Gly Gly Phe Glu Pro Leu Pro Val Ala Pro Pro Ala Ser
                85                  90                  95

Phe Lys Pro Pro Pro Asp Ala Phe Gln Ala Leu Leu Gln Pro Gln Gln
            100                 105                 110

Ala Pro Pro Pro Ser Thr Phe Gln Pro Pro Gly Ala Gly Phe Pro Tyr
        115                 120                 125

Gly Gly Gly Ala Leu Gln Pro Ser Pro Gln Gln Leu Tyr Gly Gly Tyr
    130                 135                 140

Gln Ala Ser Gln Gly Ser Asp Asp Asp Trp Asp Asp Glu Trp Asp Asp
145                 150                 155                 160

Ser Ser Thr Val Ala Asp Glu Pro Gly Ala Leu Gly Ser Gly Ala Tyr
                165                 170                 175

Pro Asp Leu Asp Gly Ser Ser Ser Ala Gly Val Gly Ala Ala Gly Arg
            180                 185                 190

Tyr Arg Leu Ser Thr Arg Ser Asp Leu Ser Leu Gly Ser Arg Gly Gly
        195                 200                 205

Ser Val Pro Pro Gln His His Pro Ser Gly Pro Lys Ser Ser Ala Thr
    210                 215                 220

Val Ser Arg Asn Leu Asn Arg Phe Ser Thr Phe Val Lys Ser Gly Gly
225                 230                 235                 240

Glu Ala Phe Val Leu Gly Glu Ala Ser Gly Phe Val Lys Asp Gly Asp
                245                 250                 255

Lys Leu Cys Val Val Leu Gly Pro Tyr Gly Pro Glu Trp Gln Glu Asn
            260                 265                 270

Pro Tyr Pro Phe Gln Cys Thr Ile Asp Asp Pro Thr Lys Gln Thr Lys
        275                 280                 285

Phe Lys Gly Met Lys Ser Tyr Ile Ser Tyr Lys Leu Val Pro Thr His
    290                 295                 300

Thr Gln Val Pro Val His Arg Arg Tyr Lys His Phe Asp Trp Leu Tyr
305                 310                 315                 320

Ala Arg Leu Ala Glu Lys Phe Pro Val Ile Ser Val Pro His Leu Pro
                325                 330                 335

Glu Lys Gln Ala Thr Gly Arg Phe Glu Glu Asp Phe Ile Ser Lys Arg
            340                 345                 350

Arg Lys Gly Leu Ile Trp Trp Met Asn His Met Ala Ser His Pro Val
        355                 360                 365

Leu Ala Gln Cys Asp Val Phe Gln His Phe Leu Thr Cys Pro Ser Ser
    370                 375                 380

Thr Asp Glu Lys Ala Trp Lys Gln Gly Lys Arg Lys Ala Glu Lys Asp
385                 390                 395                 400

Glu Met Val Gly Ala Asn Phe Phe Leu Thr Leu Ser Thr Pro Pro Ala
                405                 410                 415

Ala Ala Leu Asp Leu Gln Glu Val Glu Ser Lys Ile Asp Gly Phe Lys
            420                 425                 430

Cys Phe Thr Lys Lys Met Asp Asp Ser Ala Leu Gln Leu Asn His Thr
        435                 440                 445

Ala Asn Glu Phe Ala Arg Lys Gln Val Thr Gly Phe Lys Lys Glu Tyr
    450                 455                 460

Gln Lys Val Gly Gln Ser Phe Arg Gly Leu Ser Gln Ala Phe Glu Leu
465                 470                 475                 480

Asp Gln Gln Ala Phe Ser Val Gly Leu Asn Gln Ala Ile Ala Phe Thr
                485                 490                 495

Gly Asp Ala Tyr Asp Ala Ile Gly Glu Leu Phe Ala Glu Gln Pro Arg
            500                 505                 510

Gln Asp Leu Asp Pro Val Met Asp Leu Leu Ala Leu Tyr Gln Gly His
        515                 520                 525

Leu Ala Asn Phe Pro Asp Ile Ile His Val Gln Lys Gly Lys Ala Trp
    530                 535                 540

Pro Leu Glu Gln Val Ile Trp Ser Val Leu Cys Arg Leu Lys Gly Ala
545                 550                 555                 560

Thr Leu Thr Ala Val Pro Leu Trp Val Ser Asp Ser Tyr Ser Thr Gly
                565                 570                 575

Glu Glu Ala Ser Arg Asp Val Asp Ala Trp Val Phe Ser Leu Glu Cys
            580                 585                 590

Lys Leu Asp Cys Ser Thr Gly Ser Phe Leu Leu Glu Tyr Leu Ala Leu
        595                 600                 605

Gly Asn Glu Tyr Ser Phe Ser Lys Val Gln Arg Val Pro Leu Met Thr
    610                 615                 620

Val Leu Ser Phe
625

<210> 244
<211> 614
<212> PRT
<213> Mus musculus

<400> 244
Met Ala Leu Arg Ala Arg Ala Leu Tyr Asp Phe Lys Ser Glu Asn Pro
1               5                   10                  15

Gly Glu Ile Ser Leu Arg Glu His Glu Val Leu Ser Leu Cys Ser Glu
            20                  25                  30

Gln Asp Ile Glu Gly Trp Leu Glu Gly Ile Asn Ser Arg Gly Asp Arg
        35                  40                  45

Gly Leu Phe Pro Ala Ser Tyr Val Gln Val Ile Arg Ala Pro Glu Pro
    50                  55                  60

Gly Pro Pro Ala Asp Gly Gly Pro Gly Ala Pro Ala Arg Tyr Ala Asn
65                  70                  75                  80

Val Pro Pro Gly Gly Phe Glu Pro Leu Pro Ala Ala Pro Pro Ala Ala
                85                  90                  95

Phe Pro Pro Leu Leu Gln Pro Gln Ala Ser Pro Gly Ser Phe Gln Pro
            100                 105                 110

Pro Gly Ala Gly Phe Pro Tyr Gly Gly Gly Ala Leu Gln Pro Ser Pro
        115                 120                 125

Gln Gln Leu Tyr Gly Gly Tyr Gln Ala Ser Leu Gly Ser Asp Asp Asp
    130                 135                 140

Trp Asp Asp Glu Trp Asp Asp Ser Ser Thr Val Ala Asp Glu Pro Gly
145                 150                 155                 160

Ala Leu Gly Ser Gly Ala Tyr Pro Asp Leu Asp Gly Ser Ser Ser Ala
                165                 170                 175

Gly Gly Gly Ala Ala Gly Arg Tyr Arg Leu Ser Thr Arg Ser Asp Leu
            180                 185                 190

Ser Leu Gly Ser Arg Gly Val Ser Ala Pro Pro Ala Pro Ser Val Trp
        195                 200                 205

Ser Gln Glu Leu Gly His Gly Glu Pro Gln Pro Gln Ser Leu Leu His
    210                 215                 220

Leu Arg Gln Val Gly Arg Gly Gly Leu Arg Ala Gly Arg Gly Val Arg
225                 230                 235                 240

Leu Arg Glu Gly Trp Gly Gln Ala Val Arg Gly Ala Gly Ser Tyr Gly
                245                 250                 255

Pro Glu Trp Gln Glu Asn Pro Tyr Pro Phe Gln Cys Thr Ile Asp Asp
            260                 265                 270

Pro Thr Lys Gln Thr Lys Phe Lys Gly Met Lys Ser Tyr Ile Ser Tyr
        275                 280                 285

Lys Leu Val Pro Thr His Thr Gln Val Pro Val His Arg Arg Tyr Lys
    290                 295                 300

His Phe Asp Trp Leu Tyr Ala Arg Leu Ala Glu Lys Phe Pro Val Ile
305                 310                 315                 320

Ser Val Pro His Leu Pro Glu Lys Gln Ala Thr Gly Arg Phe Glu Glu
                325                 330                 335

Asp Phe Ile Ser Lys Arg Arg Lys Gly Leu Ile Trp Trp Met Asn His
            340                 345                 350

Met Ala Ser His Pro Val Leu Ala Gln Cys Asp Val Phe Gln His Phe
        355                 360                 365

Leu Thr Cys Pro Ser Ser Thr Asp Glu Lys Ala Trp Lys Gln Gly Lys
    370                 375                 380

Arg Lys Ala Glu Lys Asp Glu Met Val Gly Ala Asn Phe Phe Leu Thr
385                 390                 395                 400

Leu Ser Thr Pro Pro Ala Ala Ala Leu Asp Leu Gln Glu Val Glu Ser
                405                 410                 415

Lys Ile Asp Gly Phe Lys Cys Phe Thr Lys Lys Met Asp Asp Ser Ala
            420                 425                 430

Leu Gln Leu Asn His Thr Ala Asn Glu Phe Ala Arg Lys Gln Val Thr
        435                 440                 445

Gly Phe Lys Lys Glu Tyr Gln Lys Val Gly Gln Ser Phe Arg Gly Leu
    450                 455                 460

Ser Gln Ala Phe Glu Leu Asp Gln Gln Ala Phe Ser Val Gly Leu Asn
465                 470                 475                 480

Gln Ala Ile Ala Phe Thr Gly Asp Ala Tyr Asp Ala Ile Gly Glu Leu
                485                 490                 495

Phe Ala Glu Gln Pro Arg Gln Asp Leu Asp Pro Val Met Asp Leu Leu
            500                 505                 510

Ala Leu Tyr Gln Gly His Leu Ala Asn Phe Pro Asp Ile Ile His Val
        515                 520                 525

Gln Lys Gly Ala Leu Thr Lys Val Lys Glu Ser Arg Arg His Val Glu
    530                 535                 540

Glu Gly Lys Met Glu Val Gln Lys Ala Asp Gly Ile Gln Asp Arg Cys
545                 550                 555                 560

Asn Thr Ile Ser Phe Ala Thr Leu Ala Glu Ile His His Phe His Gln
                565                 570                 575

Ile Arg Val Arg Asp Phe Lys Ser Gln Met Gln His Phe Leu Gln Gln
            580                 585                 590

Gln Ile Ile Phe Phe Gln Lys Val Thr Gln Lys Leu Glu Glu Ala Leu
        595                 600                 605

His Lys Tyr Asp Ser Val
    610


<210> 245
<211> 574
<212> PRT
<213> Homo sapiens

<400> 245
Met Ala Leu Lys Gly Arg Ala Leu Tyr Asp Phe His Ser Glu Asn Lys
1               5                   10                  15

Glu Glu Ile Ser Ile Gln Gln Asp Glu Asp Leu Val Ile Phe Ser Glu
            20                  25                  30

Thr Ser Leu Asp Gly Trp Leu Gln Gly Gln Asn Ser Arg Gly Glu Thr
        35                  40                  45

Gly Leu Phe Pro Ala Ser Tyr Val Glu Ile Val Arg Ser Gly Ile Ser
    50                  55                  60

Thr Asn His Ala Asp Tyr Ser Ser Ser Pro Ala Gly Ser Pro Gly Ala
65                  70                  75                  80

Gln Val Ser Leu Tyr Asn Ser Pro Ser Val Ala Ser Pro Ala Arg Ser
                85                  90                  95

Gly Gly Gly Ser Gly Phe Leu Ser Asn Gln Gly Ser Phe Glu Glu Asp
            100                 105                 110

Asp Asp Asp Asp Trp Asp Asp Trp Asp Asp Gly Cys Thr Val Val Glu

115                 120                 125

Glu Pro Arg Ala Gly Gly Leu Gly Thr Asn Gly His Pro Pro Leu Asn
    130                 135                 140

Leu Ser Tyr Pro Gly Ala Tyr Pro Ser Gln His Met Ala Phe Arg Pro
145                 150                 155                 160

Lys Pro Pro Leu Glu Arg Gln Asp Ser Leu Ala Ser Ala Lys Arg Gly
                165                 170                 175

Ser Val Val Gly Arg Asn Leu Asn Arg Phe Ser Cys Phe Val Arg Ser
            180                 185                 190

Gly Val Glu Ala Phe Ile Leu Gly Asp Val Pro Met Met Ala Lys Ile
        195                 200                 205

Ala Glu Thr Tyr Ser Ile Glu Met Gly Pro Arg Gly Pro Gln Trp Lys
    210                 215                 220

Ala Asn Pro His Pro Phe Ala Cys Ser Val Glu Asp Pro Thr Lys Gln
225                 230                 235                 240

Thr Lys Phe Lys Gly Ile Lys Ser Tyr Ile Ser Tyr Lys Leu Thr Pro
                245                 250                 255

Thr His Ala Ala Ser Pro Val Tyr Arg Arg Tyr Lys His Phe Asp Trp
            260                 265                 270

Leu Tyr Asn Arg Leu Leu His Lys Phe Thr Val Ile Ser Val Pro His
        275                 280                 285

Leu Pro Glu Lys Gln Ala Thr Gly Arg Phe Glu Glu Asp Phe Ile Glu
    290                 295                 300

Lys Arg Lys Arg Arg Leu Ile Leu Trp Met Asp His Met Thr Ser His
305                 310                 315                 320

Pro Val Leu Ser Gln Tyr Glu Gly Phe Gln His Phe Leu Ser Cys Leu
                325                 330                 335

Asp Asp Lys Gln Trp Lys Met Gly Lys Arg Arg Ala Glu Lys Asp Glu
            340                 345                 350

Met Val Gly Ala Ser Phe Leu Leu Thr Phe Gln Ile Pro Thr Glu His
        355                 360                 365

Gln Asp Leu Gln Asp Val Glu Asp Arg Val Asp Thr Phe Lys Ala Phe
    370                 375                 380

Ser Lys Lys Met Asp Asp Ser Val Leu Gln Leu Ser Thr Val Ala Ser
385                 390                 395                 400

Glu Leu Val Arg Lys His Val Gly Gly Phe Arg Lys Glu Phe Gln Lys
                405                 410                 415

Leu Gly Ser Ala Phe Gln Ala Ile Ser His Ser Phe Gln Met Asp Pro
            420                 425                 430

Pro Phe Cys Ser Glu Ala Leu Asn Ser Ala Ile Ser His Thr Gly Arg
        435                 440                 445

Thr Tyr Glu Ala Ile Gly Glu Met Phe Ala Glu Gln Pro Lys Asn Asp
    450                 455                 460

Leu Phe Gln Met Leu Asp Thr Leu Ser Leu Tyr Gln Gly Leu Leu Ser
465                 470                 475                 480

Asn Phe Pro Asp Ile Ile His Leu Gln Lys Gly Ala Phe Ala Lys Val

                485                 490                 495

Lys Glu Ser Gln Arg Met Ser Asp Glu Gly Arg Met Val Gln Asp Glu
            500                 505                 510

Ala Asp Gly Ile Arg Arg Arg Cys Arg Val Val Gly Phe Ala Leu Gln
        515                 520                 525

Ala Glu Met Asn His Phe His Gln Arg Arg Glu Leu Asp Phe Lys His
    530                 535                 540

Met Met Gln Asn Tyr Leu Arg Gln Gln Ile Leu Phe Tyr Gln Arg Val
545                 550                 555                 560

Gly Gln Gln Leu Glu Lys Thr Leu Arg Met Tyr Asp Asn Leu
                565                 570

<210> 246
<211> 395
<212> PRT
<213> Mus musculus

<400> 246
Met Lys Leu Pro Leu Asn Lys Phe Pro Gly Phe Ala Lys Pro Gly Met
1               5                   10                  15

Glu Gln Tyr Leu Leu Ala Lys Gln Leu Ala Lys Pro Lys Glu Lys Ile
            20                  25                  30

Ala Ile Ile Val Gly Asp Tyr Gly Pro Met Trp Val Tyr Pro Thr Ser
        35                  40                  45

Thr Phe Asp Cys Val Val Ala Asp Pro Arg Lys Gly Ser Lys Met Tyr
    50                  55                  60

Gly Leu Lys Ser Tyr Ile Glu Tyr Gln Leu Thr Pro Thr Asn Thr Asn
65                  70                  75                  80

Arg Ser Val Asn His Arg Tyr Lys His Phe Asp Trp Leu Tyr Glu Arg
                85                  90                  95

Leu Leu Val Lys Phe Gly Ser Ala Ile Pro Ile Pro Ser Leu Pro Asp
            100                 105                 110

Lys Gln Val Thr Gly Arg Phe Glu Glu Glu Phe Ile Lys Met Arg Met
        115                 120                 125

Glu Arg Leu Gln Ala Trp Met Thr Arg Met Cys Arg His Pro Val Val
    130                 135                 140

Ser Glu Ser Glu Val Phe Gln Gln Phe Leu Asn Phe Arg Asp Glu Lys
145                 150                 155                 160

Glu Trp Lys Thr Gly Lys Arg Lys Ala Glu Lys Asp Glu Leu Val Gly
                165                 170                 175

Val Met Ile Phe Ser Thr Met Glu Pro Glu Ala Pro Asp Leu Asp Leu
            180                 185                 190

Ile Glu Ile Glu Gln Lys Cys Asp Ala Val Gly Lys Phe Thr Lys Ala
        195                 200                 205

Met Asp Asp Gly Val Lys Glu Leu Leu Thr Val Gly Gln Glu His Trp
    210                 215                 220

Lys Arg Cys Thr Gly Thr Leu Pro Lys Glu Tyr Gln Lys Ile Gly Lys
225                 230                 235                 240

Ala Leu Gln Ser Leu Ala Ala Val Phe Ser Ser Ser Gly Tyr Gln Gly
                245                 250                 255

Glu Thr Asp Leu Asn Asp Ala Ile Thr Glu Ala Gly Lys Thr Tyr Glu
            260                 265                 270

Glu Ile Ala Ser Leu Val Ala Glu Gln Pro Lys Lys Asp Leu His Phe
        275                 280                 285

Leu Met Glu Cys Asn His Glu Tyr Lys Gly Phe Leu Gly Cys Phe Pro
    290                 295                 300

Asp Ile Ile Gly Ala His Lys Gly Ala Ile Glu Lys Val Lys Glu Ser
305                 310                 315                 320

Asp Lys Leu Val Ala Thr Ser Lys Ile Thr Pro Gln Asp Lys Gln Thr
                325                 330                 335

Met Val Lys Arg Val Gly Thr Met Ser Tyr Ala Leu Gln Ala Glu Met
            340                 345                 350

Asn His Phe His Ser Asn Arg Ile Tyr Asp Tyr Asn Ser Val Ile Arg
        355                 360                 365

Leu Tyr Leu Glu Gln Gln Val Gln Phe Tyr Glu Thr Ile Ala Glu Lys
    370                 375                 380

Leu Arg Gln Ala Leu Ser Arg Phe Pro Val Met
385                 390                 395

<210> 247
<211> 595
<212> PRT
<213> Mus musculus

<400> 247
Met Ala Thr Lys Ala Arg Val Met Tyr Asp Phe Ala Ala Glu Pro Gly
1               5                   10                  15

Asn Asn Glu Leu Thr Val Thr Glu Gly Glu Ile Ile Thr Val Thr Asn
            20                  25                  30

Pro Asn Val Gly Gly Gly Trp Leu Glu Gly Lys Asn Asn Lys Gly Glu
        35                  40                  45

Gln Gly Leu Val Pro Thr Asp Tyr Val Glu Ile Leu Pro Asn Asp Gly
    50                  55                  60

Lys Asp Pro Phe Ser Cys Gly Asn Ser Val Ala Asp Gln Ala Phe Leu
65                  70                  75                  80

Asp Ser Leu Thr Ala Ser Thr Ala Gln Thr Asn Ser Ser Ser Ala Asn
                85                  90                  95

Ser Asn Asn Gln Val Gly Gly Gly Asn Asp Pro Trp Thr Ala Trp Asn
            100                 105                 110

Ala Pro Lys Pro Gly Asn Trp Asp Ser Ser Asp Ala Trp Gly Ser Arg
        115                 120                 125

Thr Asp Gly Thr Ser Ala Gln Arg Asn Ser Ser Ala Asn Asn Trp Asp
    130                 135                 140

Thr Gly Phe Gly His Pro Gln Ala Tyr Gln Gly Pro Ala Thr Gly Asp
145                 150                 155                 160

Asp Asp Glu Trp Asp Glu Asp Trp Asp Asp Pro Lys Ser Ser Ser Pro
                165                 170                 175

Tyr Phe Lys Asp Ser Glu Pro Ala Glu Ala Gly Gly Ile Gln Arg Gly
            180                 185                 190

Asn Ser Arg Ala Gly Ala Ser Ser Met Lys Leu Pro Leu Asn Lys Phe
        195                 200                 205

Pro Gly Phe Ala Lys Pro Gly Met Glu Gln Tyr Leu Leu Ala Lys Gln
    210                 215                 220

Leu Ala Lys Pro Lys Glu Lys Ile Ala Ile Ile Val Gly Asp Tyr Gly
225                 230                 235                 240

Pro Met Trp Val Tyr Pro Thr Ser Thr Phe Asp Cys Val Val Ala Asp
                245                 250                 255

Pro Arg Lys Gly Ser Lys Met Tyr Gly Leu Lys Ser Tyr Ile Glu Tyr
            260                 265                 270

Gln Leu Thr Pro Thr Asn Thr Asn Arg Ser Val Asn His Arg Tyr Lys
        275                 280                 285

His Phe Asp Trp Leu Tyr Glu Arg Leu Leu Val Lys Phe Gly Ser Ala
    290                 295                 300

Ile Pro Ile Pro Ser Leu Pro Asp Lys Gln Val Thr Gly Arg Phe Glu
305                 310                 315                 320

Glu Glu Phe Ile Lys Met Arg Met Glu Arg Leu Gln Ala Trp Met Thr
                325                 330                 335

Arg Met Cys Arg His Pro Val Val Ser Glu Ser Glu Val Phe Gln Gln
            340                 345                 350

Phe Leu Asn Phe Arg Asp Glu Lys Glu Trp Lys Thr Gly Lys Arg Lys
        355                 360                 365

Ala Glu Lys Asp Glu Leu Val Gly Val Met Ile Phe Ser Thr Met Glu
    370                 375                 380

Pro Glu Ala Pro Asp Leu Asp Leu Ile Glu Ile Glu Gln Lys Cys Asp
385                 390                 395                 400

Ala Val Gly Lys Phe Thr Lys Ala Met Asp Asp Gly Val Lys Glu Leu
                405                 410                 415

Leu Thr Val Gly Gln Glu His Trp Lys Arg Cys Thr Gly Pro Leu Pro
            420                 425                 430

Lys Glu Tyr Gln Lys Ile Gly Lys Ala Leu Gln Ser Leu Ala Ala Val
        435                 440                 445

Phe Ser Ser Ser Gly Tyr Gln Gly Glu Thr Asp Leu Asn Asp Ala Ile
    450                 455                 460

Thr Glu Ala Gly Lys Thr Tyr Glu Glu Ile Ala Ser Leu Val Ala Glu
465                 470                 475                 480

Gln Pro Lys Lys Asp Leu His Phe Leu Met Glu Cys Asn His Glu Tyr
                485                 490                 495

Lys Gly Phe Leu Gly Cys Phe Pro Asp Ile Ile Gly Ala His Lys Gly
            500                 505                 510

Ala Ile Glu Lys Val Lys Glu Ser Asp Lys Leu Val Ala Thr Ser Lys
        515                 520                 525

Ile Thr Pro Gln Asp Lys Gln Thr Met Val Lys Arg Val Gly Thr Met
    530                 535                 540

Ser Tyr Ala Leu Gln Ala Glu Met Asn His Phe His Ser Asn Arg Ile
545                 550                 555                 560

Tyr Asp Tyr Asn Ser Val Ile Arg Leu Tyr Leu Glu Gln Gln Val Gln
                565                 570                 575

Phe Tyr Glu Thr Ile Ala Glu Lys Leu Arg Gln Ala Leu Ser Arg Phe
            580                 585                 590

Pro Val Met
        595


<210> 248
<211> 112
<212> PRT
<213> Homo sapiens

<400> 248
Asp Asn Ile Leu Ile Val Thr Val Val Glu Phe Glu Thr Tyr Gly Asp
1               5                   10                  15

Gly Lys His Tyr Tyr Tyr Val Ile Glu Ile Glu Thr Ser Thr Gly Leu
            20                  25                  30

Lys Glu Trp Thr Val Lys Arg Arg Tyr Ser Asp Phe Leu Glu Leu His
        35                  40                  45

Ser Lys Leu Lys Arg Lys Phe Pro Arg Arg Ile Leu Pro Pro Leu Pro
    50                  55                  60

Gly Lys Lys Leu Phe Val Arg Tyr Leu Ser Glu Glu Phe Ile Glu Lys
65                  70                  75                  80

Arg Arg Arg Gly Leu Glu Lys Tyr Leu Gln Lys Leu Leu Asn His Pro
                85                  90                  95

Glu Leu Ile Asn His Ser Glu Val Val Leu Glu Phe Leu Glu Ser Ser
            100                 105                 110


<210> 249
<211> 57
<212> PRT
<213> Homo sapiens

<400> 249
Pro Lys Val Val Ala Leu Tyr Asp Tyr Gln Ala Arg Glu Ser Asp Glu
1               5                   10                  15

Leu Ser Phe Lys Lys Gly Asp Ile Ile Ile Val Leu Glu Lys Ser Asp
            20                  25                  30

Asp Gly Gly Trp Trp Lys Gly Arg Leu Lys Gly Thr Lys Glu Gly Leu
        35                  40                  45

Ile Pro Ser Asn Tyr Val Glu Pro Val
    50                  55


<210> 250
<211> 490
<212> PRT
<213> Homo sapiens

<400> 250
Met Asn Leu Ala Glu Ile Cys Asp Asn Ala Lys Lys Gly Arg Glu Tyr
1               5                   10                  15

Ala Leu Leu Gly Asn Tyr Asp Ser Ser Met Val Tyr Tyr Gln Gly Val
            20                  25                  30

Met Gln Gln Ile Gln Arg His Cys Gln Ser Val Arg Asp Pro Ala Ile
        35                  40                  45

Lys Gly Lys Trp Gln Gln Val Arg Gln Glu Leu Leu Glu Glu Tyr Glu
    50                  55                  60

Gln Val Lys Ser Ile Val Ser Thr Leu Glu Ser Phe Lys Ile Asp Lys
65                  70                  75                  80

Pro Pro Asp Phe Pro Val Ser Cys Gln Asp Glu Pro Phe Arg Asp Pro
                85                  90                  95

Ala Val Trp Pro Pro Pro Val Pro Ala Glu His Arg Ala Pro Pro Gln
            100                 105                 110

Ile Arg Arg Pro Asn Arg Glu Val Arg Pro Leu Arg Lys Glu Met Ala
        115                 120                 125

Gly Val Gly Ala Arg Gly Pro Val Gly Arg Ala His Pro Ile Ser Lys
    130                 135                 140

Ser Glu Lys Pro Ser Thr Ser Arg Asp Lys Asp Tyr Arg Ala Arg Gly
145                 150                 155                 160

Arg Asp Asp Lys Gly Arg Lys Asn Met Gln Asp Gly Ala Ser Asp Gly
                165                 170                 175

Glu Met Pro Lys Phe Asp Gly Ala Gly Tyr Asp Lys Asp Leu Val Glu
            180                 185                 190

Ala Leu Glu Arg Asp Ile Val Ser Arg Asn Pro Ser Ile His Trp Asp
        195                 200                 205

Asp Ile Ala Asp Leu Glu Glu Ala Lys Lys Leu Leu Arg Glu Ala Val
    210                 215                 220

Val Leu Pro Met Trp Met Pro Asp Phe Phe Lys Gly Ile Arg Arg Pro
225                 230                 235                 240

Trp Lys Gly Val Leu Met Val Gly Pro Pro Gly Thr Gly Lys Thr Met
                245                 250                 255

Leu Ala Lys Ala Val Ala Thr Glu Cys Gly Thr Thr Phe Phe Asn Val
            260                 265                 270

Ser Ser Ser Thr Leu Thr Ser Lys Tyr Arg Gly Glu Ser Glu Lys Leu
        275                 280                 285

Val Arg Leu Leu Phe Glu Met Ala Arg Phe Tyr Ala Pro Thr Thr Ile
    290                 295                 300

Phe Ile Asp Glu Ile Asp Ser Ile Cys Ser Arg Arg Gly Thr Ser Asp
305                 310                 315                 320

Glu His Glu Ala Ser Arg Arg Val Lys Ser Glu Leu Leu Ile Gln Met
                325                 330                 335

Asp Gly Val Gly Gly Ala Leu Glu Asn Asp Asp Pro Ser Lys Met Val
            340                 345                 350

Met Val Leu Ala Ala Thr Asn Phe Pro Trp Asp Ile Asp Glu Ala Leu
        355                 360                 365

Arg Arg Arg Leu Glu Lys Arg Ile Tyr Ile Pro Leu Pro Thr Ala Lys
    370                 375                 380

Gly Arg Ala Glu Leu Leu Lys Ile Asn Leu Arg Glu Val Glu Leu Asp
385                 390                 395                 400

Pro Asp Ile Gln Leu Glu Asp Ile Ala Glu Lys Ile Glu Gly Tyr Ser
                405                 410                 415

Gly Ala Asp Ile Thr Asn Val Cys Arg Asp Ala Ser Leu Met Ala Met
            420                 425                 430

Arg Arg Arg Ile Asn Gly Leu Ser Pro Glu Glu Ile Arg Ala Leu Ser
        435                 440                 445

Lys Glu Glu Leu Gln Met Pro Val Thr Lys Gly Asp Phe Glu Leu Ala
    450                 455                 460

Leu Lys Lys Ile Ala Lys Ser Val Ser Ala Ala Asp Leu Glu Lys Tyr
465                 470                 475                 480

Glu Lys Trp Met Val Glu Phe Gly Ser Ala
                485                 490


<210> 251
<211> 486
<212> PRT
<213> Xenopus laevis

<400> 251
Met Ser Leu Leu Met Ile Ser Glu Asn Val Lys Leu Ala Arg Glu Tyr
1               5                   10                  15

Ala Leu Leu Gly Asn Tyr Asp Ser Ala Met Val Tyr Tyr Gln Gly Val
            20                  25                  30

Leu Asp Gln Met Asn Lys Tyr Leu Tyr Ser Val Lys Asp Thr Phe Leu
        35                  40                  45

Gln Gln Lys Trp Gln Gln Val Trp Gln Glu Ile Asn Met Glu Cys Lys
    50                  55                  60

His Val Lys Asp Ile Met Ser Thr Leu Glu Gly Phe Lys Leu Asp Ser
65                  70                  75                  80

Ser Pro Val Lys Thr Thr Gln His Glu Phe Pro Ser His Asp Gly Glu
                85                  90                  95

Val Trp Ser Leu Pro Val Pro Val Glu Arg Arg Pro Ser Pro Gly Pro
            100                 105                 110

Arg Lys Arg Gln Ser Val Gln Cys Asn Asp Asn Lys Ser His Asn Asn
        115                 120                 125

Arg Phe Ser Ala Ala Ala Lys Gly Pro Asn Leu Pro Ser Ala Arg Asn
    130                 135                 140

Ala Asn Asn Val Lys Met Lys Pro Val Arg Ala Arg Glu Lys Lys Asp
145                 150                 155                 160

Ala Leu Ile Lys Asn Lys Ser Ser Ala Asp Val Ser Glu Thr Glu Val
                165                 170                 175

Lys Arg Phe Asp Gly Ser Gly Tyr Asp Lys Asp Leu Ile Glu Ala Leu
            180                 185                 190

Glu Arg Asp Ile Ile Ser Gln Asn Pro Asn Ile Arg Trp Asp Asp Ile

195                 200                 205

Ala Asp Leu Glu Glu Ala Lys Lys Leu Leu Lys Glu Ala Val Val Leu
    210                 215                 220

Pro Met Trp Met Pro Glu Phe Phe Lys Gly Ile Arg Arg Pro Trp Lys
225                 230                 235                 240

Gly Val Leu Met Val Gly Pro Pro Gly Thr Gly Lys Thr Leu Leu Ala
                245                 250                 255

Lys Ala Val Ala Thr Glu Cys Lys Thr Thr Phe Phe Asn Ile Ser Ser
            260                 265                 270

Ser Thr Leu Thr Ser Lys Tyr Arg Gly Glu Ser Glu Lys Leu Val Arg
        275                 280                 285

Leu Leu Phe Glu Met Ala Arg Phe Tyr Ala Pro Thr Thr Ile Phe Ile
    290                 295                 300

Asp Glu Ile Asp Ser Ile Cys Ser Arg Arg Gly Thr Ser Glu Glu His
305                 310                 315                 320

Glu Ala Ser Arg Arg Val Lys Ala Glu Leu Leu Val Gln Met Asp Gly
                325                 330                 335

Val Gly Gly Ala Ser Glu Asn Glu Asp Pro Ser Lys Met Val Met Val
            340                 345                 350

Leu Ala Ala Thr Asn Phe Pro Trp Asp Ile Asp Glu Ala Leu Arg Arg
        355                 360                 365

Arg Leu Glu Lys Arg Ile Tyr Ile Pro Leu Pro Ser Ala Lys Gly Arg
    370                 375                 380

Glu Glu Leu Leu Arg Ile Asn Leu Lys Glu Leu Glu Leu Ala Asp Asp
385                 390                 395                 400

Val Asn Ile Glu Cys Ile Ala Glu Asn Met Asp Gly Tyr Ser Gly Ala
                405                 410                 415

Asp Ile Thr Asn Val Cys Arg Asp Ala Ser Leu Met Ala Met Arg Arg
            420                 425                 430

Arg Ile Glu Gly Leu Thr Pro Glu Glu Ile Arg Asn Leu Ser Arg Asp
        435                 440                 445

Asp Met His Met Pro Thr Thr Met Glu Asp Phe Glu Met Ala Leu Lys
    450                 455                 460

Lys Val Ser Lys Ser Val Ser Ala Ser Asp Ile Glu Lys Tyr Glu Lys
465                 470                 475                 480

Trp Ile Phe Glu Phe Gly
                485


<210> 252
<211> 491
<212> PRT
<213> Homo sapiens

<400> 252
Met Ser Leu Leu Met Ile Ser Glu Asn Val Lys Leu Ala Arg Glu Tyr
1               5                   10                  15

Ala Leu Leu Gly Asn Tyr Asp Ser Ala Met Val Tyr Tyr Gln Gly Val
            20                  25                  30

Leu Asp Gln Met Asn Lys Tyr Leu Tyr Ser Val Lys Asp Thr Tyr Leu
        35                  40                  45

Gln Gln Lys Trp Gln Gln Val Trp Gln Glu Ile Asn Val Glu Ala Lys
    50                  55                  60

His Val Lys Asp Ile Met Lys Thr Leu Glu Ser Phe Lys Leu Asp Ser
65                  70                  75                  80

Thr Pro Leu Lys Ala Ala Gln His Asp Leu Pro Ala Ser Glu Gly Glu
                85                  90                  95

Val Trp Ser Met Pro Val Pro Val Glu Arg Arg Pro Ser Pro Gly Pro
            100                 105                 110

Arg Lys Arg Gln Ser Ser Gln Tyr Ser Asp Pro Lys Ser His Gly Asn
        115                 120                 125

Arg Pro Ser Thr Thr Val Arg Val His Arg Ser Ser Ala Gln Asn Val
    130                 135                 140

His Asn Asp Arg Gly Lys Ala Val Arg Cys Arg Glu Lys Lys Glu Gln
145                 150                 155                 160

Asn Lys Gly Arg Glu Glu Lys Asn Lys Ser Pro Ala Ala Val Thr Glu
                165                 170                 175

Pro Glu Thr Asn Lys Phe Asp Ser Thr Gly Tyr Asp Lys Asp Leu Val
            180                 185                 190

Glu Ala Leu Glu Arg Asp Ile Ile Ser Gln Asn Pro Asn Val Arg Trp
        195                 200                 205

Asp Asp Ile Ala Asp Leu Val Glu Ala Lys Lys Leu Leu Lys Glu Ala
    210                 215                 220

Val Val Leu Pro Met Trp Met Pro Glu Phe Phe Lys Gly Ile Arg Arg
225                 230                 235                 240

Pro Trp Lys Gly Val Leu Met Val Gly Pro Pro Gly Thr Gly Lys Thr
                245                 250                 255

Leu Leu Ala Lys Ala Val Ala Thr Glu Cys Lys Thr Thr Phe Phe Asn
            260                 265                 270

Val Ser Ser Ser Thr Leu Thr Ser Lys Tyr Arg Gly Glu Ser Glu Lys
        275                 280                 285

Leu Val Arg Leu Leu Phe Glu Met Ala Arg Phe Tyr Ser Pro Ala Thr
    290                 295                 300

Ile Phe Ile Asp Glu Ile Asp Ser Ile Cys Ser Arg Arg Gly Thr Ser
305                 310                 315                 320

Glu Glu His Glu Ala Ser Arg Arg Val Lys Ala Glu Leu Leu Val Gln
                325                 330                 335

Met Asp Gly Val Gly Gly Thr Ser Glu Asn Asp Asp Pro Ser Lys Met
            340                 345                 350

Val Met Val Leu Ala Ala Thr Asn Phe Pro Trp Asp Ile Asp Glu Ala
        355                 360                 365

Leu Arg Arg Arg Leu Glu Lys Arg Ile Tyr Ile Pro Leu Pro Ser Ala
    370                 375                 380

Lys Gly Arg Glu Glu Leu Leu Arg Ile Ser Leu Arg Glu Leu Glu Leu
385                 390                 395                 400

Ala Asp Asp Val Asp Leu Ala Ser Ile Ala Glu Asn Met Glu Gly Tyr
                405                 410                 415

Ser Gly Ala Asp Ile Thr Asn Val Cys Arg Asp Ala Ser Leu Met Ala
            420                 425                 430

Met Arg Arg Arg Ile Glu Gly Leu Thr Pro Glu Glu Ile Arg Asn Leu
        435                 440                 445

Ser Lys Glu Glu Met His Met Pro Thr Thr Met Glu Asp Phe Glu Met
    450                 455                 460

Ala Leu Lys Lys Val Ser Lys Ser Val Ser Ala Ala Asp Ile Glu Arg
465                 470                 475                 480

Tyr Glu Lys Trp Ile Phe Glu Phe Gly Ser Cys
                485                 490

<210> 253
<211> 290
<212> PRT
<213> Homo sapiens

<400> 253
Met Ala Val Leu Leu Glu Thr Thr Leu Gly Asp Val Val Ile Asp Leu
1               5                   10                  15

Tyr Thr Glu Glu Arg Pro Arg Ala Cys Leu Asn Phe Leu Lys Leu Cys
            20                  25                  30

Lys Ile Lys Tyr Tyr Asn Tyr Cys Leu Ile His Asn Val Gln Arg Asp
        35                  40                  45

Phe Ile Ile Gln Thr Gly Asp Pro Thr Gly Thr Gly Arg Gly Gly Glu
    50                  55                  60

Ser Ile Phe Gly Gln Leu Tyr Gly Asp Gln Ala Ser Phe Phe Glu Ala
65                  70                  75                  80

Glu Lys Val Pro Arg Ile Lys His Lys Lys Lys Gly Thr Val Ser Met
                85                  90                  95

Val Asn Asn Gly Ser Asp Gln His Gly Ser Gln Phe Leu Ile Thr Thr
            100                 105                 110

Gly Glu Asn Leu Asp Tyr Leu Asp Gly Val His Thr Val Phe Gly Glu
        115                 120                 125

Val Thr Glu Gly Met Asp Ile Ile Lys Lys Ile Asn Glu Thr Phe Val
    130                 135                 140

Asp Lys Asp Phe Val Pro Tyr Gln Asp Ile Arg Ile Asn His Thr Val
145                 150                 155                 160

Ile Leu Asp Asp Pro Phe Asp Asp Pro Pro Asp Leu Leu Ile Pro Asp
                165                 170                 175

Arg Ser Pro Glu Pro Thr Arg Glu Gln Leu Asp Ser Gly Arg Ile Gly
            180                 185                 190

Ala Asp Glu Glu Ile Asp Asp Phe Lys Gly Arg Ser Ala Glu Glu Val
        195                 200                 205

Glu Glu Ile Lys Ala Glu Lys Glu Ala Lys Thr Gln Ala Ile Leu Leu
    210                 215                 220

Glu Met Val Gly Asp Leu Pro Asp Ala Asp Ile Lys Pro Pro Glu Asn
225                 230                 235                 240

Val Leu Phe Val Cys Lys Leu Asn Pro Val Thr Thr Asp Glu Asp Leu
                245                 250                 255

Glu Ile Ile Phe Ser Arg Phe Gly Pro Ile Arg Ser Cys Glu Val Ile
            260                 265                 270

Arg Asp Trp Lys Thr Gly Glu Ser Leu Cys Tyr Ala Phe Ile Glu Phe
        275                 280                 285

Glu Lys
    290


<210> 254
<211> 491
<212> PRT
<213> Mus musculus

<400> 254
Met Ser Leu Gln Met Ile Val Glu Asn Val Lys Leu Ala Arg Glu Tyr
  1               5                  10                  15

Ala Leu Leu Gly Asn Tyr Asp Ser Ala Met Val Tyr Tyr Gln Gly Val
             20                  25                  30

Leu Asp Gln Met Asn Lys Tyr Leu Tyr Ser Val Lys Asp Thr His Leu
         35                  40                  45

Arg Gln Lys Trp Gln Gln Val Trp Gln Glu Ile Asn Val Glu Ala Lys
     50                  55                  60

Gln Val Lys Asp Ile Met Lys Thr Leu Glu Ser Phe Lys Leu Asp Ile
 65                  70                  75                  80

Thr Ser Leu Gln Ala Ala Gln His Glu Leu Pro Ala Ala Glu Gly Glu
                 85                  90                  95

Val Trp Ser Leu Pro Val Pro Val Glu Arg Arg Pro Leu Pro Gly Pro
            100                 105                 110

Arg Lys Arg Gln Ser Ser Gln His Ser Asp Pro Lys Pro His Ser Asn
        115                 120                 125

Arg Pro Ser Thr Val Val Arg Ala His Arg Pro Ser Pro Gln Asn Leu
    130                 135                 140

His Asn Asp Arg Gly Lys Ala Val Arg Ser Arg Glu Lys Lys Glu Gln
145                 150                 155                 160

Ser Lys Gly Arg Glu Glu Lys Asn Lys Leu Pro Ala Ala Val Thr Glu
                165                 170                 175

Pro Glu Ala Asn Lys Phe Asp Gly Thr Gly Tyr Asp Lys Asp Leu Val
            180                 185                 190

Glu Ala Leu Glu Arg Asp Ile Ile Ser Gln Asn Pro Asn Val Arg Trp
        195                 200                 205

Tyr Asp Ile Ala Asp Leu Val Glu Ala Lys Lys Leu Leu Gln Glu Ala
    210                 215                 220

Val Val Leu Pro Met Trp Met Pro Glu Phe Phe Lys Gly Ile Arg Arg
225                 230                 235                 240

Pro Trp Lys Gly Val Leu Met Val Gly Pro Pro Gly Thr Gly Lys Thr
                245                 250                 255

Leu Leu Ala Lys Ala Val Ala Thr Glu Cys Lys Thr Thr Phe Phe Asn

260                 265                 270

Val Ser Ser Ser Thr Leu Thr Ser Lys Tyr Arg Gly Glu Ser Glu Lys
        275                 280                 285

Leu Val Arg Leu Leu Phe Glu Met Ala Arg Phe Tyr Ser Pro Ala Thr
    290                 295                 300

Ile Phe Ile Asp Glu Ile Asp Ser Ile Cys Ser Arg Arg Gly Thr Ser
305                 310                 315                 320

Glu Glu His Glu Ala Ser Arg Arg Met Lys Ala Glu Leu Leu Val Gln
                325                 330                 335

Met Asp Gly Val Gly Gly Ala Ser Glu Asn Asp Asp Pro Ser Lys Met
            340                 345                 350

Val Met Val Leu Ala Ala Thr Asn Phe Pro Trp Asp Ile Asp Glu Ala
        355                 360                 365

Leu Arg Arg Arg Leu Glu Lys Arg Ile Tyr Ile Pro Leu Pro Ser Ala
    370                 375                 380

Lys Gly Arg Glu Glu Leu Leu Arg Ile Ser Leu Arg Glu Leu Glu Leu
385                 390                 395                 400

Ala Asp Asp Val Asn Leu Ala Ser Ile Ala Glu Asn Met Glu Gly Tyr
                405                 410                 415

Ser Gly Ala Asp Ile Thr Asn Val Cys Arg Asp Ala Ser Leu Met Ala
            420                 425                 430

Met Arg Arg Arg Ile Glu Gly Leu Thr Pro Glu Glu Ile Arg Asn Leu
        435                 440                 445

Ser Arg Glu Ala Met His Met Pro Thr Thr Met Glu Asp Phe Glu Met
    450                 455                 460

Ala Leu Lys Lys Ile Ser Lys Ser Val Ser Ala Ala Asp Ile Glu Arg
465                 470                 475                 480

Tyr Glu Lys Trp Ile Val Glu Phe Gly Ser Cys
                485                 490


<210> 255
<211> 186
<212> PRT
<213> Homo sapiens

<400> 255
Gly Ile Leu Leu Tyr Gly Pro Pro Gly Thr Gly Lys Thr Leu Leu Ala
1               5                   10                  15

Lys Ala Val Ala Lys Glu Leu Gly Val Pro Phe Ile Glu Ile Ser Gly
            20                  25                  30

Ser Glu Leu Leu Ser Lys Tyr Val Gly Glu Ser Glu Lys Leu Val Arg
        35                  40                  45

Ala Leu Phe Ser Leu Ala Arg Lys Ser Ala Pro Cys Ile Ile Phe Ile
    50                  55                  60

Asp Glu Ile Asp Ala Leu Ala Pro Lys Arg Gly Asp Val Gly Thr Gly
65                  70                  75                  80

Asp Val Ser Ser Arg Val Val Asn Gln Leu Leu Thr Glu Met Asp Gly
                85                  90                  95

Phe Glu Lys Leu Ser Asn Val Ile Val Ile Gly Ala Thr Asn Arg Pro
            100                 105                 110

Asp Leu Leu Asp Pro Ala Leu Leu Arg Pro Gly Arg Phe Asp Arg Arg
        115                 120                 125

Ile Glu Val Pro Leu Pro Asp Glu Glu Glu Arg Leu Glu Ile Leu Lys
    130                 135                 140

Ile His Leu Lys Lys Lys Pro Leu Glu Lys Asp Val Asp Leu Asp Glu
145                 150                 155                 160

Ile Ala Arg Arg Thr Pro Gly Phe Ser Gly Ala Asp Leu Ala Ala Leu
                165                 170                 175

Cys Arg Glu Ala Ala Leu Arg Ala Ile Arg
            180                 185

<210> 256
<211> 821
<212> PRT
<213> Homo sapiens

<400> 256

<210> 257
<211> 815
<212> PRT
<213> Homo sapiens

<400> 257
Met Pro Thr Val Ile Ser Ala Ser Val Ala Pro Arg Thr Ala Ala Glu
1               5                   10                  15

Pro Arg Ser Pro Gly Pro Val Pro His Pro Ala Gln Ser Lys Ala Thr
            20                  25                  30

Glu Ala Gly Gly Gly Asn Pro Ser Gly Ile Tyr Ser Ala Ile Ile Ser
        35                  40                  45

Arg Asn Phe Pro Ile Ile Gly Val Lys Glu Lys Thr Phe Glu Gln Leu
    50                  55                  60

His Lys Lys Cys Leu Glu Lys Lys Val Leu Tyr Val Asp Pro Glu Phe
65                  70                  75                  80

Pro Pro Asp Glu Thr Ser Leu Phe Tyr Ser Gln Lys Phe Pro Ile Gln
                85                  90                  95

Phe Val Trp Lys Arg Pro Pro Glu Ile Cys Glu Asn Pro Arg Phe Ile

            100                 105                 110

Ile Asp Gly Ala Asn Arg Thr Asp Ile Cys Gln Gly Glu Leu Gly Asp
        115                 120                 125

Cys Trp Phe Leu Ala Ala Ile Ala Cys Leu Thr Leu Asn Gln His Leu
    130                 135                 140

Leu Phe Arg Val Ile Pro His Asp Gln Ser Phe Ile Glu Asn Tyr Ala
145                 150                 155                 160

Gly Ile Phe His Phe Gln Phe Trp Arg Tyr Gly Glu Trp Val Asp Val
                165                 170                 175

Val Ile Asp Asp Cys Leu Pro Thr Tyr Asn Asn Gln Leu Val Phe Thr
            180                 185                 190

Lys Ser Asn His Arg Asn Glu Phe Trp Ser Ala Leu Leu Glu Lys Ala
        195                 200                 205

Tyr Ala Lys Leu His Gly Ser Tyr Glu Ala Leu Lys Gly Gly Asn Thr
    210                 215                 220

Thr Glu Ala Met Glu Asp Phe Thr Gly Gly Val Ala Glu Phe Phe Glu
225                 230                 235                 240

Ile Arg Asp Ala Pro Ser Asp Met Tyr Lys Ile Met Lys Lys Ala Ile
                245                 250                 255

Glu Arg Gly Ser Leu Met Gly Cys Ser Ile Asp Asp Gly Thr Asn Met
            260                 265                 270

Thr Tyr Gly Thr Ser Pro Ser Gly Leu Asn Met Gly Glu Leu Ile Ala
        275                 280                 285

Arg Met Val Arg Asn Met Asp Asn Ser Leu Leu Gln Asp Ser Asp Leu
    290                 295                 300

Asp Pro Arg Gly Ser Asp Glu Arg Pro Thr Arg Thr Ile Ile Pro Val
305                 310                 315                 320

Gln Tyr Glu Thr Arg Met Ala Cys Gly Leu Val Arg Gly His Ala Tyr
                325                 330                 335

Ser Val Thr Gly Leu Asp Glu Val Pro Phe Lys Gly Glu Lys Val Lys
            340                 345                 350

Leu Val Arg Leu Arg Asn Pro Trp Gly Gln Val Glu Trp Asn Gly Ser
        355                 360                 365

Trp Ser Asp Arg Trp Lys Asp Trp Ser Phe Val Asp Lys Asp Glu Lys
    370                 375                 380

Ala Arg Leu Gln His Gln Val Thr Glu Asp Gly Glu Phe Trp Met Ser
385                 390                 395                 400

Tyr Glu Asp Phe Ile Tyr His Phe Thr Lys Leu Glu Ile Cys Asn Leu
                405                 410                 415

Thr Ala Asp Ala Leu Gln Ser Asp Lys Leu Gln Thr Trp Thr Val Ser
            420                 425                 430

Val Asn Glu Gly Arg Trp Val Arg Gly Cys Ser Ala Gly Gly Cys Arg
        435                 440                 445

Asn Phe Pro Asp Thr Phe Trp Thr Asn Pro Gln Tyr Arg Leu Lys Leu
    450                 455                 460

Leu Glu Glu Asp Asp Asp Pro Asp Asp Ser Glu Val Ile Cys Ser Phe

465                 470                 475                 480

Leu Val Ala Leu Met Gln Lys Asn Arg Arg Lys Asp Arg Lys Leu Gly
                485                 490                 495

Ala Ser Leu Phe Thr Ile Gly Phe Ala Ile Tyr Glu Val Pro Lys Glu
            500                 505                 510

Met His Gly Asn Lys Gln His Leu Gln Lys Asp Phe Phe Leu Tyr Asn
        515                 520                 525

Ala Ser Lys Ala Arg Ser Lys Thr Tyr Ile Asn Met Arg Glu Val Ser
    530                 535                 540

Gln Arg Phe Arg Leu Pro Pro Ser Glu Tyr Val Ile Val Pro Ser Thr
545                 550                 555                 560

Tyr Glu Pro His Gln Glu Gly Glu Phe Ile Leu Arg Val Phe Ser Glu
                565                 570                 575

Lys Arg Asn Leu Ser Glu Glu Val Glu Asn Thr Ile Ser Val Asp Arg
            580                 585                 590

Pro Val Pro Ile Ile Phe Val Ser Asp Arg Ala Asn Ser Asn Lys Glu
        595                 600                 605

Leu Gly Val Asp Gln Glu Ser Glu Glu Gly Lys Gly Lys Thr Ser Pro
    610                 615                 620

Asp Lys Gln Lys Gln Ser Pro Gln Pro Gln Pro Gly Ser Ser Asp Gln
625                 630                 635                 640

Glu Ser Glu Glu Gln Gln Gln Phe Arg Asn Ile Phe Lys Gln Ile Ala
                645                 650                 655

Gly Asp Asp Met Glu Ile Cys Ala Asp Glu Leu Lys Lys Val Leu Asn
            660                 665                 670

Thr Val Val Asn Lys His Lys Asp Leu Lys Thr His Gly Phe Thr Leu
        675                 680                 685

Glu Ser Cys Arg Ser Met Ile Ala Leu Met Asp Thr Asp Gly Ser Gly
    690                 695                 700

Lys Leu Asn Leu Gln Glu Phe His His Leu Trp Asn Lys Ile Lys Ala
705                 710                 715                 720

Trp Gln Lys Ile Phe Lys His Tyr Asp Thr Asp Gln Ser Gly Thr Ile
                725                 730                 735

Asn Ser Tyr Glu Met Arg Asn Ala Val Asn Asp Ala Gly Phe His Leu
            740                 745                 750

Asn Asn Gln Leu Tyr Asp Ile Ile Thr Met Arg Tyr Ala Asp Lys His
        755                 760                 765

Met Asn Ile Asp Phe Asp Ser Phe Ile Cys Cys Phe Val Arg Leu Glu
    770                 775                 780

Gly Met Phe Arg Ala Phe His Ala Phe Asp Lys Asp Gly Asp Gly Ile
785                 790                 795                 800

Ile Lys Leu Asn Val Leu Glu Trp Leu Gln Leu Thr Met Tyr Ala
                805                 810                 815

<210> 258
<211> 815
<212> PRT

<213> Macaca fascicularis

<400> 258
Met Pro Thr Val Ile Ser Ala Ser Val Ala Pro Arg Thr Ala Ala Glu
1               5                   10                  15

Pro Arg Ser Pro Gly Pro Val Pro His Pro Ala Gln Ser Lys Ala Thr
            20                  25                  30

Glu Ala Gly Gly Gly Asn Ala Ser Gly Ile Tyr Ser Ala Ile Ile Ser
        35                  40                  45

Arg Asn Phe Pro Ile Ile Gly Val Lys Glu Lys Thr Phe Glu Gln Leu
    50                  55                  60

His Lys Lys Cys Leu Glu Lys Lys Val Leu Tyr Val Asp Pro Glu Phe
65                  70                  75                  80

Pro Pro Asp Glu Thr Ser Leu Phe Tyr Ser Gln Lys Phe Pro Ile Gln
                85                  90                  95

Phe Ile Trp Lys Arg Pro Pro Glu Ile Cys Glu Asn Pro Arg Phe Ile
            100                 105                 110

Ile Asp Gly Ala Asn Arg Thr Asp Ile Cys Gln Gly Asp Leu Gly Asp
        115                 120                 125

Cys Trp Phe Leu Ala Ala Ile Ala Cys Leu Thr Leu Asn Gln Arg Leu
    130                 135                 140

Leu Phe Arg Val Ile Pro His Asp Gln Ser Phe Ile Glu Asn Tyr Ala
145                 150                 155                 160

Gly Ile Phe His Phe Gln Phe Trp Arg Tyr Gly Glu Trp Val Asp Val
                165                 170                 175

Val Ile Asp Asp Cys Leu Pro Thr Tyr Asn Asn Gln Leu Val Phe Thr
            180                 185                 190

Lys Ser Asn His Arg Asn Glu Phe Trp Ser Ala Leu Leu Glu Lys Ala
        195                 200                 205

Tyr Ala Lys Leu His Gly Ser Tyr Glu Ala Leu Lys Gly Gly Asn Thr
    210                 215                 220

Thr Glu Ala Met Glu Asp Phe Thr Gly Gly Val Thr Glu Phe Phe Glu
225                 230                 235                 240

Ile Arg Asp Ala Pro Ser Asp Met His Lys Ile Met Lys Lys Ala Ile
                245                 250                 255

Glu Arg Gly Ser Leu Met Gly Cys Ser Ile Asp Asp Gly Thr Asn Met
            260                 265                 270

Thr Tyr Gly Thr Ser Pro Ser Gly Leu Asn Met Gly Glu Leu Ile Ala
        275                 280                 285

Arg Met Val Arg Asn Met Asp Asn Ser Leu Phe Arg Asp Ser Asp Leu
    290                 295                 300

Asp Pro Arg Ala Ser Val Glu Arg Pro Thr Arg Thr Ile Val Pro Val
305                 310                 315                 320

Gln Tyr Glu Thr Arg Met Ala Cys Gly Leu Val Arg Gly His Ala Tyr
                325                 330                 335

Ser Val Thr Gly Leu Asp Glu Val Leu Phe Lys Gly Glu Lys Val Lys
            340                 345                 350

Leu Val Arg Leu Arg Asn Pro Trp Gly Gln Val Glu Trp Asn Gly Ser
        355                 360                 365

Trp Ser Asp Gly Trp Lys Asp Trp Ser Phe Val Asp Lys Asp Glu Lys
    370                 375                 380

Ala Arg Leu Gln His Gln Val Thr Glu Asp Gly Glu Phe Trp Met Ser
385                 390                 395                 400

Tyr Glu Asp Phe Ile Tyr His Phe Thr Lys Leu Glu Ile Cys Asn Leu
                405                 410                 415

Thr Ala Asp Ala Leu Gln Ser Asp Lys Leu Gln Thr Trp Thr Val Ser
            420                 425                 430

Val Asn Glu Gly Arg Trp Val Arg Gly Cys Ser Ala Gly Gly Cys Arg
        435                 440                 445

Asn Phe Pro Asp Thr Phe Trp Thr Asn Pro Gln Tyr Arg Leu Lys Leu
    450                 455                 460

Leu Glu Glu Asp Asp Asp Pro Asp Asp Ser Glu Val Ile Cys Ser Phe
465                 470                 475                 480

Leu Val Ala Leu Met Gln Lys Asn Arg Arg Lys Asp Arg Lys Leu Gly
                485                 490                 495

Ala Asn Leu Phe Thr Ile Gly Phe Ala Ile Tyr Glu Val Pro Lys Glu
            500                 505                 510

Met His Gly Asn Arg Gln His Leu Gln Lys Asp Phe Phe Leu Tyr Asn
        515                 520                 525

Ala Ser Arg Ala Arg Ser Lys Thr Tyr Ile Asn Met Arg Glu Val Ser
    530                 535                 540

Gln Arg Phe Arg Leu Pro Pro Ser Glu Tyr Val Ile Val Pro Ser Thr
545                 550                 555                 560

Tyr Glu Pro His Gln Glu Gly Glu Phe Ile Leu Arg Val Phe Ser Glu
                565                 570                 575

Lys Arg Asn Leu Ser Glu Glu Val Glu Asn Thr Ile Ser Val Asp Arg
            580                 585                 590

Pro Val Pro Ile Ile Phe Val Ser Asp Arg Ala Asn Ser Asn Lys Glu
        595                 600                 605

Leu Gly Val Asp Gln Glu Ser Glu Glu Gly Lys Gly Lys Thr Ser Pro
    610                 615                 620

Asp Lys Gln Glu Gln Ser Pro Gln Pro Gln Pro Gly Ser Ser Asp Gln
625                 630                 635                 640

Glu Ser Glu Glu Gln Gln Gln Phe Arg Asn Ile Phe Lys Gln Ile Ala
                645                 650                 655

Gly Asp Asp Met Glu Ile Cys Ala Asp Glu Leu Lys Lys Val Leu Asn
            660                 665                 670

Thr Val Val Asn Lys His Lys Asp Leu Lys Thr His Gly Phe Thr Leu
        675                 680                 685

Glu Ser Cys Arg Ser Met Ile Ala Leu Met Asp Thr Asp Gly Ser Gly
    690                 695                 700

Lys Leu Asn Leu Gln Glu Phe His His Leu Trp Asn Lys Ile Lys Ala
705                 710                 715                 720

Trp Gln Lys Ile Phe Lys His Tyr Asp Thr Asp Gln Ser Gly Thr Ile
                725                 730                 735

Asn Ser Tyr Glu Met Arg Asn Ala Val Asn Asp Ala Gly Phe His Leu
            740                 745                 750

Asn Asn Gln Leu Tyr Asp Ile Ile Thr Met Arg Tyr Ala Asp Lys His
        755                 760                 765

Met Asn Ile Asp Phe Asp Ser Phe Ile Cys Cys Phe Val Arg Leu Glu
    770                 775                 780

Gly Met Phe Arg Ala Phe His Ala Phe Asp Lys Asp Gly Asp Gly Ile
785                 790                 795                 800

Ile Lys Leu Asn Val Leu Glu Trp Leu Gln Leu Thr Met Tyr Ala
                805                 810                 815

<210> 259
<211> 729
<212> PRT
<213> Homo sapiens

<400> 259
Met Pro Thr Val Ile Ser Ala Ser Val Ala Pro Arg Thr Ala Ala Glu
  1               5                  10                  15

Pro Arg Ser Pro Gly Pro Val Pro His Pro Ala Gln Ser Lys Ala Thr
             20                  25                  30

Glu Ala Gly Gly Gly Asn Pro Ser Gly Ile Tyr Ser Ala Ile Ile Ser
         35                  40                  45

Arg Asn Phe Pro Ile Ile Gly Val Lys Glu Lys Thr Phe Glu Gln Leu
     50                  55                  60

His Lys Lys Cys Leu Glu Lys Lys Val Leu Tyr Val Asp Pro Glu Phe
 65                  70                  75                  80

Pro Pro Asp Glu Thr Ser Leu Phe Tyr Ser Gln Lys Phe Pro Ile Gln
                 85                  90                  95

Phe Val Trp Lys Arg Pro Pro Glu Ile Cys Glu Asn Pro Arg Phe Ile
            100                 105                 110

Ile Asp Gly Ala Asn Arg Thr Asp Ile Cys Gln Gly Glu Leu Gly Asp
        115                 120                 125

Cys Trp Phe Leu Ala Ala Ile Ala Cys Leu Thr Leu Asn Gln His Leu
    130                 135                 140

Leu Phe Arg Val Ile Pro His Asp Gln Ser Phe Ile Glu Asn Tyr Ala
145                 150                 155                 160

Gly Ile Phe His Phe Gln Phe Trp Arg Tyr Gly Glu Trp Val Asp Val
                165                 170                 175

Val Ile Asp Asp Cys Leu Pro Thr Tyr Asn Asn Gln Leu Val Phe Thr
            180                 185                 190

Lys Ser Asn His Arg Asn Glu Phe Trp Ser Ala Leu Leu Glu Lys Ala
        195                 200                 205

Tyr Ala Lys Leu His Gly Ser Tyr Glu Ala Leu Lys Gly Gly Asn Thr
    210                 215                 220

Thr Glu Ala Met Glu Asp Phe Thr Gly Gly Val Ala Glu Phe Phe Glu
225                 230                 235                 240

Ile Arg Asp Ala Pro Ser Asp Met Tyr Lys Ile Met Lys Lys Ala Ile
                245                 250                 255

Glu Arg Gly Ser Leu Met Gly Cys Ser Ile Asp Thr Ile Ile Pro Val
            260                 265                 270

Gln Tyr Glu Thr Arg Met Ala Cys Gly Leu Val Arg Gly His Ala Tyr
        275                 280                 285

Ser Val Thr Gly Leu Asp Glu Val Pro Phe Lys Gly Glu Lys Val Lys
    290                 295                 300

Leu Val Arg Leu Arg Asn Pro Trp Gly Gln Val Glu Trp Asn Gly Ser
305                 310                 315                 320

Trp Ser Asp Arg Trp Lys Asp Trp Ser Phe Val Asp Lys Asp Glu Lys
                325                 330                 335

Ala Arg Leu Gln His Gln Val Thr Glu Asp Gly Glu Phe Trp Met Ser
            340                 345                 350

Tyr Glu Asp Phe Ile Tyr His Phe Thr Lys Leu Glu Ile Cys Asn Leu
        355                 360                 365

Thr Ala Asp Ala Leu Gln Ser Asp Lys Leu Gln Thr Trp Thr Val Ser
    370                 375                 380

Val Asn Glu Gly Arg Trp Val Arg Gly Cys Ser Ala Gly Gly Cys Arg
385                 390                 395                 400

Asn Phe Pro Asp Thr Phe Trp Thr Asn Pro Gln Tyr Arg Leu Lys Leu
                405                 410                 415

Leu Glu Glu Asp Asp Asp Pro Asp Asp Ser Glu Val Ile Cys Ser Phe
            420                 425                 430

Leu Val Ala Leu Met Gln Lys Asn Arg Arg Lys Asp Arg Lys Leu Gly
        435                 440                 445

Ala Ser Leu Phe Thr Ile Gly Phe Ala Ile Tyr Glu Val Pro Lys Glu
    450                 455                 460

Met His Gly Asn Lys Gln His Leu Gln Lys Asp Phe Phe Leu Tyr Asn
465                 470                 475                 480

Ala Ser Lys Ala Arg Ser Lys Thr Tyr Ile Asn Met Arg Glu Val Ser
                485                 490                 495

Gln Arg Phe Arg Leu Pro Pro Ser Glu Tyr Val Ile Val Pro Ser Thr
            500                 505                 510

Tyr Glu Pro His Gln Glu Gly Glu Phe Ile Leu Arg Val Phe Ser Glu
        515                 520                 525

Lys Arg Asn Leu Ser Glu Glu Val Glu Asn Thr Ile Ser Val Asp Arg
    530                 535                 540

Pro Val Pro Gln Pro Gly Ser Ser Asp Gln Glu Ser Glu Glu Gln Gln
545                 550                 555                 560

Gln Phe Arg Asn Ile Phe Lys Gln Ile Ala Gly Asp Asp Met Glu Ile
                565                 570                 575

Cys Ala Asp Glu Leu Lys Lys Val Leu Asn Thr Val Val Asn Lys His
            580                 585                 590

Lys Asp Leu Lys Thr His Gly Phe Thr Leu Glu Ser Cys Arg Ser Met
        595                 600                 605

Ile Ala Leu Met Asp Thr Asp Gly Ser Gly Lys Leu Asn Leu Gln Glu
    610                 615                 620

Phe His His Leu Trp Asn Lys Ile Lys Ala Trp Gln Lys Ile Phe Lys
625                 630                 635                 640

His Tyr Asp Thr Asp Gln Ser Gly Thr Ile Asn Ser Tyr Glu Met Arg
                645                 650                 655

Asn Ala Val Asn Asp Ala Gly Phe His Leu Asn Asn Gln Leu Tyr Asp
            660                 665                 670

Ile Ile Thr Met Arg Tyr Ala Asp Lys His Met Asn Ile Asp Phe Asp
        675                 680                 685

Ser Phe Ile Cys Cys Phe Val Arg Leu Glu Gly Met Phe Arg Ala Phe
    690                 695                 700

His Ala Phe Asp Lys Asp Gly Asp Gly Ile Ile Lys Leu Asn Val Leu
705                 710                 715                 720

Glu Trp Leu Gln Leu Thr Met Tyr Ala
                725

<210> 260
<211> 821
<212> PRT
<213> Rattus norvegicus

<400> 260
Met Pro Thr Val Ile Ser Pro Thr Val Ala Pro Arg Thr Gly Ala Glu
  1               5                  10                  15

Pro Arg Ser Pro Gly Pro Val Pro His Pro Ala Gln Gly Lys Thr Thr
             20                  25                  30

Glu Ala Gly Gly Gly His Pro Gly Gly Ile Tyr Ser Ala Ile Ile Ser
         35                  40                  45

Arg Asn Phe Pro Ile Ile Gly Val Lys Glu Lys Thr Phe Glu Gln Leu
     50                  55                  60

His Lys Lys Cys Leu Glu Lys Lys Val Leu Tyr Leu Asp Pro Glu Phe
 65                  70                  75                  80

Pro Pro Asp Glu Thr Ser Leu Phe Tyr Ser Gln Lys Phe Pro Ile Gln
                 85                  90                  95

Phe Val Trp Lys Arg Pro Pro Glu Ile Cys Glu Asn Pro Arg Phe Ile
            100                 105                 110

Ile Gly Gly Ala Asn Arg Thr Asp Ile Cys Gln Gly Asp Leu Gly Asp
        115                 120                 125

Cys Trp Leu Leu Ala Ala Ile Ala Cys Leu Thr Leu Asn Glu Arg Leu
    130                 135                 140

Leu Phe Arg Val Ile Pro His Asp Gln Ser Phe Thr Glu Asn Tyr Ala
145                 150                 155                 160

Gly Ile Phe His Phe Gln Phe Trp Arg Tyr Gly Asp Trp Val Asp Val
                165                 170                 175

Val Ile Asp Asp Cys Leu Pro Thr Tyr Asn Asn Gln Leu Val Phe Thr
            180                 185                 190

Lys Ser Asn His Arg Asn Glu Phe Trp Ser Ala Leu Leu Glu Lys Ala

        195                 200                 205

Tyr Ala Lys Leu His Gly Ser Tyr Glu Ala Leu Lys Gly Gly Asn Thr
    210                 215                 220

Thr Glu Ala Met Glu Asp Phe Thr Gly Gly Val Thr Glu Phe Phe Glu
225                 230                 235                 240

Ile Lys Asp Ala Pro Ser Asp Met Tyr Lys Ile Met Arg Lys Ala Ile
                245                 250                 255

Glu Arg Gly Ser Leu Met Gly Cys Ser Ile Asp Asp Gly Thr Asn Met
            260                 265                 270

Thr Tyr Gly Thr Ser Pro Ser Gly Leu Asn Met Gly Glu Leu Ile Ala
        275                 280                 285

Arg Met Val Arg Asn Met Asp Asn Ser Leu Leu Arg Asp Ser Asp Leu
    290                 295                 300

Asp Pro Arg Ala Ser Asp Asp Arg Pro Ser Arg Thr Ile Val Pro Val
305                 310                 315                 320

Gln Tyr Glu Thr Arg Met Ala Cys Gly Leu Val Lys Gly His Ala Tyr
                325                 330                 335

Ser Val Thr Gly Leu Glu Glu Ala Leu Phe Lys Gly Glu Lys Val Lys
            340                 345                 350

Leu Val Arg Leu Arg Asn Pro Trp Gly Gln Val Glu Trp Asn Gly Ser
        355                 360                 365

Trp Ser Asp Gly Trp Lys Asp Trp Ser Phe Val Asp Lys Asp Glu Lys
    370                 375                 380

Ala Arg Leu Gln His Gln Val Thr Glu Asp Gly Glu Phe Trp Met Ser
385                 390                 395                 400

Tyr Asp Asp Phe Val Tyr His Phe Thr Lys Leu Glu Ile Cys Asn Leu
                405                 410                 415

Thr Ala Asp Ala Leu Glu Ser Asp Lys Leu Gln Thr Trp Thr Val Ser
            420                 425                 430

Val Asn Glu Gly Arg Trp Val Arg Gly Cys Ser Ala Gly Gly Cys Arg
        435                 440                 445

Asn Phe Pro Asp Thr Phe Trp Thr Asn Pro Gln Tyr Arg Leu Lys Leu
    450                 455                 460

Leu Glu Glu Asp Asp Asp Pro Asp Asp Ser Glu Val Ile Cys Ser Phe
465                 470                 475                 480

Leu Val Ala Leu Met Gln Lys Asn Arg Arg Lys Asp Arg Lys Leu Gly
                485                 490                 495

Ala Asn Leu Phe Thr Ile Gly Phe Ala Ile Tyr Glu Val Pro Lys Glu
            500                 505                 510

Met His Gly Asn Lys Gln His Leu Gln Lys Asp Phe Phe Leu Tyr Asn
        515                 520                 525

Ala Ser Lys Ala Arg Ser Lys Thr Tyr Ile Asn Met Arg Glu Val Ser
    530                 535                 540

Gln Arg Phe Arg Leu Pro Pro Ser Glu Tyr Val Ile Val Pro Ser Thr
545                 550                 555                 560

Tyr Glu Pro His Gln Glu Gly Glu Phe Ile Leu Arg Val Phe Ser Glu

                565                 570                 575

Lys Arg Asn Leu Ser Glu Glu Ala Glu Asn Thr Ile Ser Val Asp Arg
            580                 585                 590

Pro Val Lys Lys Lys Lys Asn Lys Pro Ile Ile Phe Val Ser Asp Arg
        595                 600                 605

Ala Asn Ser Asn Lys Glu Leu Gly Val Asp Gln Glu Ala Glu Glu Gly
    610                 615                 620

Lys Asp Lys Thr Gly Pro Asp Lys Gln Gly Glu Ser Pro Gln Pro Arg
625                 630                 635                 640

Pro Gly His Thr Asp Gln Glu Ser Glu Glu Gln Gln Gln Phe Arg Asn
                645                 650                 655

Ile Phe Arg Gln Ile Ala Gly Asp Asp Met Glu Ile Cys Ala Asp Glu
            660                 665                 670

Leu Lys Asn Val Leu Asn Thr Val Val Asn Lys His Lys Asp Leu Lys
        675                 680                 685

Thr Gln Gly Phe Thr Leu Glu Ser Cys Arg Ser Met Ile Ala Leu Met
    690                 695                 700

Asp Thr Asp Gly Ser Gly Arg Leu Asn Leu Gln Glu Phe His His Leu
705                 710                 715                 720

Trp Lys Lys Ile Lys Ala Trp Gln Lys Ile Phe Lys His Tyr Asp Thr
                725                 730                 735

Asp His Ser Gly Thr Ile Asn Ser Tyr Glu Met Arg Asn Ala Val Asn
            740                 745                 750

Asp Ala Gly Phe His Leu Asn Ser Gln Leu Tyr Asp Ile Ile Thr Met
        755                 760                 765

Arg Tyr Ala Asp Lys His Met Asn Ile Asp Phe Asp Ser Phe Ile Cys
    770                 775                 780

Cys Phe Val Arg Leu Glu Gly Met Phe Arg Ala Phe His Ala Phe Asp
785                 790                 795                 800

Lys Asp Gly Asp Gly Ile Ile Lys Leu Asn Val Leu Glu Trp Leu Gln
                805                 810                 815

Leu Thr Met Tyr Ala
            820


<210> 261
<211> 298
<212> PRT
<213> Homo sapiens

<400> 261
Leu Phe Val Asp Pro Ser Phe Pro Ala Ala Pro Lys Ser Leu Gly Tyr
1               5                   10                  15

Lys Pro Leu Gly Pro Arg Gly Ile Glu Trp Lys Arg Pro His Glu Ile
            20                  25                  30

Asn Glu Asn Pro Gln Phe Ile Val Gly Gly Ala Thr Arg Thr Asp Ile
        35                  40                  45

Cys Gln Gly Ala Leu Gly Asp Cys Trp Leu Leu Ala Ala Leu Ala Ser
    50                  55                  60

Leu Thr Leu Asn Glu Pro Leu Leu Leu Arg Val Val Pro His Asp Gln
65                  70                  75                  80

Ser Phe Gln Glu Asn Tyr Ala Gly Ile Phe His Phe Arg Phe Trp Gln
                85                  90                  95

Phe Gly Glu Trp Val Asp Val Val Val Asp Asp Leu Leu Pro Thr Lys
            100                 105                 110

Asp Gly Lys Leu Leu Phe Val His Ser Ala Glu Arg Asn Glu Phe Trp
        115                 120                 125

Ser Ala Leu Leu Glu Lys Ala Tyr Ala Lys Leu Asn Gly Cys Tyr Glu
    130                 135                 140

Ala Leu Ser Gly Gly Ser Thr Thr Glu Ala Leu Glu Asp Leu Thr Gly
145                 150                 155                 160

Gly Val Cys Glu Ser Tyr Glu Leu Lys Leu Ala Pro Ser Ser Met Leu
                165                 170                 175

Asn Leu Gly Asn Ile Ile Lys Lys Met Leu Glu Arg Gly Ser Leu Leu
            180                 185                 190

Gly Cys Ser Ile Asp Ile Thr Ser Pro Val Asp Met Glu Ala Arg Met
        195                 200                 205

Ala Lys Gly Leu Val Lys Gly His Ala Tyr Ser Val Thr Gly Val Lys
    210                 215                 220

Glu Val Asn Tyr Arg Gly Glu Gly Val Lys Leu Ile Arg Leu Arg Asn
225                 230                 235                 240

Pro Trp Gly Gln Val Glu Trp Thr Gly Asp Trp Ser Asp Ser Ser Pro
                245                 250                 255

Asp Trp Asn Ile Val Asp Pro Asp Glu Lys Ala Arg Leu Gln Leu Lys
            260                 265                 270

Phe Glu Asp Gly Glu Phe Trp Met Ser Phe Glu Asp Phe Leu Arg His
        275                 280                 285

Phe Ser Arg Leu Glu Ile Cys Asn Leu Thr
    290                 295


<210> 262
<211> 323
<212> PRT
<213> Homo sapiens

<400> 262
Phe Glu Asn Gln Asp Tyr Glu Glu Leu Arg Gln Glu Cys Leu Glu Glu
1               5                   10                  15

Gly Gly Leu Phe Val Asp Pro Leu Phe Pro Ala Lys Pro Ser Ser Leu
            20                  25                  30

Phe Phe Ser Gln Leu Gln Arg Lys Phe Val Val Trp Lys Arg Pro His
        35                  40                  45

Glu Ile Phe Glu Asp Pro Pro Leu Ile Val Gly Gly Ala Ser Arg Thr
    50                  55                  60

Asp Ile Cys Gln Gly Val Leu Gly Asp Cys Trp Leu Leu Ala Ala Leu
65                  70                  75                  80

Ala Ala Leu Thr Leu Arg Glu Glu Leu Leu Ala Arg Val Ile Pro Lys
                85                  90                  95

Asp Gln Glu Phe Ser Glu Asn Tyr Ala Gly Ile Tyr His Phe Arg Phe
            100                 105                 110

Trp Arg Tyr Gly Lys Trp Val Asp Val Val Ile Asp Asp Arg Leu Pro
        115                 120                 125

Thr Tyr Asn Gly Asp Leu Leu Phe Met His Ser Asn Ser Arg Asn Glu
    130                 135                 140

Phe Trp Ser Ala Leu Leu Glu Lys Ala Tyr Ala Lys Leu Arg Gly Cys
145                 150                 155                 160

Tyr Glu Ala Leu Lys Gly Gly Ser Thr Thr Glu Ala Leu Glu Asp Leu
                165                 170                 175

Thr Gly Gly Val Ala Glu Ser Ile Glu Leu Lys Lys Ile Ser Lys Asp
            180                 185                 190

Pro Asp Glu Leu Phe Lys Asp Leu Lys Lys Ala Phe Glu Arg Gly Ser
        195                 200                 205

Leu Met Gly Cys Ser Ile Gly Ala Gly Thr Ala Val Glu Glu Glu Glu
    210                 215                 220

Gln Lys Arg Asn Gly Leu Val Lys Gly His Ala Tyr Ser Val Thr Asp
225                 230                 235                 240

Val Arg Glu Val Asp Gly Arg Arg Arg Gln Lys Leu Leu Arg Leu Arg
                245                 250                 255

Asn Pro Trp Gly Glu Ser Glu Trp Asn Gly Pro Trp Ser Asp Asp Ser
            260                 265                 270

Pro Glu Trp Arg Ser Val Ser Ala Glu Glu Lys Lys Asn Leu Gly Leu
        275                 280                 285

Thr Met Asp Asp Asp Gly Glu Phe Trp Met Ser Phe Glu Asp Phe Leu
    290                 295                 300

Arg His Phe Thr Lys Val Glu Ile Cys Asn Leu Arg Pro Asp Trp Phe
305                 310                 315                 320

Glu Tyr Arg



<210> 263
<211> 148
<212> PRT
<213> Homo sapiens

<400> 263
Lys Trp Glu Glu Ala Ile Val Glu Gly Glu Trp Thr Arg Gly Ser Thr
1               5                   10                  15

Ala Gly Gly Cys Arg Asn Tyr Pro Asp Thr Phe Trp Thr Asn Pro Gln
            20                  25                  30

Tyr Ile Ile Thr Leu Pro Glu Pro Asp Asp Asp Asp Glu Asp Ser Cys
        35                  40                  45

Ser Val Leu Val Ala Leu Met Gln Lys Asp Arg Arg Arg Glu Arg Arg
    50                  55                  60

Met Gly Ala Asp Met Leu Thr Ile Gly Phe Ala Val Tyr Lys Val Pro
65                  70                  75                  80

Asp Glu Leu Asn His Leu Ser Arg Asp Phe Phe Leu Tyr Asn Gln Ser

85                  90                  95

Arg Ala Arg Ser Ser Thr Tyr Ile Asn Leu Arg Glu Val Ser Leu Arg
            100                 105                 110

Phe Arg Leu Pro Pro Gly Glu Tyr Val Leu Val Pro Ser Thr Phe Glu
        115                 120                 125

Pro Asn Glu Glu Gly Glu Phe Ile Leu Arg Val Phe Ser Glu Lys Pro
    130                 135                 140

Ile Asn Thr Arg
145


<210> 264
<211> 29
<212> PRT
<213> Homo sapiens

<400> 264
Glu Leu Lys Glu Ile Phe Lys Glu Phe Asp Lys Asp Gly Asp Gly Lys
 1               5                  10                  15

Ile Ser Phe Glu Glu Phe Lys Glu Leu Leu Lys Lys Leu
            20                  25


<210> 265
<211> 430
<212> PRT
<213> Homo sapiens

<400> 265
Met Ser Phe Thr Thr Arg Ser Thr Phe Ser Thr Asn Tyr Arg Ser Leu
 1               5                  10                  15

Gly Ser Val Gln Ala Pro Ser Tyr Gly Ala Arg Pro Val Ser Ser Ala
            20                  25                  30

Ala Ser Val Tyr Ala Gly Ala Gly Gly Ser Gly Ser Arg Ile Ser Val
        35                  40                  45

Ser Arg Ser Thr Ser Phe Arg Gly Gly Met Gly Ser Gly Gly Leu Ala
    50                  55                  60

Thr Gly Ile Ala Gly Gly Leu Ala Gly Met Gly Gly Ile Gln Asn Glu
65                  70                  75                  80

Lys Glu Thr Met Gln Ser Leu Asn Asp Arg Leu Ala Ser Tyr Leu Asp
                85                  90                  95

Arg Val Arg Ser Leu Glu Thr Glu Asn Arg Arg Leu Glu Ser Lys Ile
            100                 105                 110

Arg Glu His Leu Glu Lys Lys Gly Pro Gln Val Arg Asp Trp Ser His
        115                 120                 125

Tyr Phe Lys Ile Ile Glu Asp Leu Arg Ala Gln Ile Phe Ala Asn Thr
    130                 135                 140

Val Asp Asn Ala Arg Ile Val Leu Gln Ile Asp Asn Ala Arg Leu Ala
145                 150                 155                 160

Ala Asp Asp Phe Arg Val Lys Tyr Glu Thr Glu Leu Ala Met Arg Gln
                165                 170                 175

Ser Val Glu Asn Asp Ile His Gly Leu Arg Lys Val Ile Asp Asp Thr
            180                 185                 190

Asn Ile Thr Arg Leu Gln Leu Glu Thr Glu Ile Glu Ala Leu Lys Glu
        195                 200                 205

Glu Leu Leu Phe Met Lys Lys Asn His Glu Glu Glu Val Lys Gly Leu
    210                 215                 220

Gln Ala Gln Ile Ala Ser Ser Gly Leu Thr Val Glu Val Asp Ala Pro
225                 230                 235                 240

Lys Ser Gln Asp Leu Ala Lys Ile Met Ala Asp Ile Arg Ala Gln Tyr
                245                 250                 255

Asp Glu Leu Ala Arg Lys Asn Arg Glu Glu Leu Asp Lys Tyr Trp Ser
            260                 265                 270

Gln Gln Ile Glu Glu Ser Thr Thr Val Val Thr Thr Gln Ser Ala Glu
        275                 280                 285

Val Gly Ala Ala Glu Thr Thr Leu Thr Glu Leu Arg Arg Thr Val Gln
    290                 295                 300

Ser Leu Glu Ile Asp Leu Asp Ser Met Arg Asn Leu Lys Ala Ser Leu
305                 310                 315                 320

Glu Asn Ser Leu Arg Glu Val Glu Ala Arg Tyr Ala Leu Gln Met Glu
                325                 330                 335

Gln Leu Asn Gly Ile Leu Leu His Leu Glu Ser Glu Leu Ala Gln Thr
            340                 345                 350

Arg Ala Glu Gly Gln Arg Gln Ala Gln Glu Tyr Glu Ala Leu Leu Asn
        355                 360                 365

Ile Lys Val Lys Leu Glu Ala Glu Ile Ala Thr Tyr Arg Arg Leu Leu
    370                 375                 380

Glu Asp Gly Glu Asp Phe Asn Leu Gly Asp Ala Leu Asp Ser Ser Asn
385                 390                 395                 400

Ser Met Gln Thr Ile Gln Lys Thr Thr Thr Arg Arg Ile Val Asp Gly
                405                 410                 415

Lys Val Val Ser Glu Thr Asn Asp Thr Lys Val Leu Arg His
            420                 425                 430


<210> 266
<211> 424
<212> PRT
<213> Homo sapiens

<400> 266
Ser Thr Phe Ser Thr Asn Tyr Arg Ser Leu Gly Ser Val Gln Ala Pro
  1               5                  10                  15

Ser Tyr Gly Ala Arg Pro Val Ser Ser Ala Ala Ser Val Tyr Ala Gly
             20                  25                  30

Ala Gly Gly Ser Gly Ser Arg Ile Ser Val Ser Arg Ser Thr Ser Phe
         35                  40                  45

Arg Gly Gly Met Gly Ser Gly Gly Leu Ala Thr Gly Ile Ala Gly Gly
     50                  55                  60

Leu Ala Gly Met Gly Gly Ile Gln Asn Glu Lys Glu Thr Met Gln Ser
 65                  70                  75                  80

Leu Asn Asp Arg Leu Ala Ser Tyr Leu Asp Arg Val Arg Ser Leu Glu

85                  90                  95

Thr Glu Asn Arg Arg Leu Glu Ser Lys Ile Arg Glu His Leu Glu Lys
            100                 105                 110

Lys Gly Pro Gln Val Arg Asp Trp Ser His Tyr Phe Lys Ile Ile Glu
        115                 120                 125

Asp Leu Arg Ala Gln Ile Phe Ala Asn Thr Val Asp Asn Ala Arg Ile
    130                 135                 140

Val Leu Gln Ile Asp Asn Ala Arg Leu Ala Ala Asp Asp Phe Arg Val
145                 150                 155                 160

Lys Tyr Glu Thr Glu Leu Ala Met Arg Gln Ser Val Glu Asn Asp Ile
                165                 170                 175

His Gly Leu Arg Lys Val Ile Asp Asp Thr Asn Ile Thr Arg Leu Gln
            180                 185                 190

Leu Glu Thr Glu Ile Glu Ala Leu Lys Glu Glu Leu Leu Phe Met Lys
        195                 200                 205

Lys Asn His Glu Glu Glu Val Lys Gly Leu Gln Ala Gln Ile Ala Ser
    210                 215                 220

Ser Gly Leu Thr Val Glu Val Asp Ala Pro Lys Ser Gln Asp Leu Ala
225                 230                 235                 240

Lys Ile Met Ala Asp Ile Arg Ala Gln Tyr Asp Glu Leu Ala Arg Lys
                245                 250                 255

Asn Arg Glu Glu Leu Asp Lys Tyr Trp Ser Gln Gln Ile Glu Glu Ser
            260                 265                 270

Thr Thr Val Val Thr Thr Gln Ser Ala Glu Val Gly Ala Ala Glu Thr
        275                 280                 285

Thr Leu Thr Glu Leu Arg Arg Thr Val Gln Ser Leu Glu Ile Asp Leu
    290                 295                 300

Asp Ser Met Arg Asn Leu Lys Ala Ser Leu Glu Asn Ser Leu Arg Glu
305                 310                 315                 320

Val Glu Ala Arg Tyr Ala Leu Gln Met Glu Gln Leu Asn Gly Ile Leu
                325                 330                 335

Leu His Leu Glu Ser Glu Leu Ala Gln Thr Arg Ala Glu Gly Gln Arg
            340                 345                 350

Gln Ala Gln Glu Tyr Glu Ala Leu Leu Asn Ile Lys Val Lys Leu Glu
        355                 360                 365

Ala Glu Ile Ala Thr Tyr Arg Arg Leu Leu Glu Asp Gly Glu Asp Phe
    370                 375                 380

Asn Leu Gly Asp Ala Leu Asp Ser Ser Asn Ser Met Gln Thr Ile Gln
385                 390                 395                 400

Lys Thr Thr Thr Arg Arg Ile Val Asp Gly Lys Val Val Ser Glu Thr
                405                 410                 415

Asn Asp Thr Lys Val Leu Arg His
            420

<210> 267
<211> 430
<212> PRT

<213> Homo sapiens

<400> 267
Met Ser Phe Thr Thr Arg Ser Thr Phe Ser Thr Asn Tyr Arg Ser Leu
1               5                   10                  15

Gly Ser Val Gln Ala Pro Ser Tyr Gly Ala Arg Pro Val Ser Ser Ala
            20                  25                  30

Ala Ser Val Tyr Ala Gly Ala Gly Gly Ser Gly Ser Arg Ile Ser Val
        35                  40                  45

Ser Arg Ser Thr Ser Phe Arg Gly Gly Met Gly Ser Gly Gly Leu Ala
    50                  55                  60

Thr Gly Ile Ala Gly Gly Leu Ala Gly Met Gly Gly Ile Gln Asn Glu
65                  70                  75                  80

Lys Glu Thr Met Gln Ser Leu Asn Asp Arg Leu Ala Ser Tyr Leu Asp
                85                  90                  95

Arg Val Arg Ser Leu Glu Thr Glu Asn Arg Arg Leu Glu Ser Lys Ile
            100                 105                 110

Arg Glu His Leu Glu Lys Lys Gly Pro Gln Val Arg Asp Trp Ser His
        115                 120                 125

Tyr Phe Lys Ile Ile Glu Asp Leu Arg Ala Gln Ile Phe Ala Asn Thr
    130                 135                 140

Val Asp Asn Ala Arg Ile Val Leu Gln Ile Asp Asn Ala Arg Leu Ala
145                 150                 155                 160

Ala Asp Asp Phe Arg Val Lys His Glu Thr Glu Leu Ala Met Arg Gln
                165                 170                 175

Ser Val Glu Asn Asp Ile His Gly Leu Arg Lys Val Ile Asp Asp Thr
            180                 185                 190

Asn Ile Thr Arg Leu Gln Leu Glu Thr Glu Ile Glu Ala Leu Lys Glu
        195                 200                 205

Glu Leu Leu Phe Met Lys Lys Asn His Glu Glu Glu Val Lys Gly Leu
    210                 215                 220

Gln Ala Gln Ile Ala Ser Ser Gly Leu Thr Val Glu Val Asp Ala Pro
225                 230                 235                 240

Lys Ser Gln Asp Leu Ala Lys Ile Met Ala Asp Ile Arg Ala Gln Tyr
                245                 250                 255

Asp Glu Leu Ala Arg Lys Asn Arg Glu Glu Leu Asp Lys Tyr Trp Ser
            260                 265                 270

Gln Gln Ile Glu Glu Ser Thr Thr Val Val Thr Thr Gln Ser Ala Glu
        275                 280                 285

Val Gly Ala Ala Glu Thr Thr Leu Thr Glu Leu Arg Arg Thr Val Gln
    290                 295                 300

Ser Leu Glu Ile Asp Leu Asp Ser Met Arg Asn Leu Lys Ala Ser Leu
305                 310                 315                 320

Glu Asn Ser Leu Arg Glu Val Glu Ala Arg Tyr Ala Leu Gln Met Glu
                325                 330                 335

Gln Leu Asn Gly Ile Leu Leu His Leu Glu Ser Glu Leu Ala Gln Thr
            340                 345                 350

Arg Ala Glu Gly Gln Arg Gln Ala Gln Glu Tyr Glu Ala Leu Leu Asn
        355                 360                 365

Ile Lys Val Lys Leu Glu Ala Glu Ile Ala Thr Tyr Arg Arg Leu Leu
    370                 375                 380

Glu Asp Gly Glu Asp Phe Asn Leu Gly Asp Ala Leu Asp Ser Ser Asn
385                 390                 395                 400

Ser Met Gln Thr Ile Gln Lys Thr Thr Thr Arg Arg Ile Val Asp Gly
                405                 410                 415

Lys Val Val Ser Glu Thr Asn Asp Thr Lys Val Leu Arg His
            420                 425                 430

<210> 268
<211> 375
<212> PRT
<213> Homo sapiens

<400> 268
Ala Arg Gly Gly Ser Gly Gly Leu Ala Thr Gly Ile Ala Gly Gly Leu
  1               5                  10                  15

Ala Gly Met Gly Gly Ile Gln Asn Glu Lys Glu Thr Met Gln Ser Leu
             20                  25                  30

Asn Asp Arg Leu Ala Ser Tyr Leu Asp Arg Val Arg Ser Leu Glu Thr
         35                  40                  45

Glu Asn Arg Arg Leu Glu Ser Lys Ile Arg Glu His Leu Glu Lys Lys
     50                  55                  60

Gly Pro Gln Val Arg Asp Trp Ser His Tyr Phe Lys Ile Ile Glu Asp
 65                  70                  75                  80

Leu Arg Ala Gln Ile Phe Ala Asn Thr Val Asp Asn Ala Arg Ile Val
                 85                  90                  95

Leu Gln Ile Asp Asn Ala Arg Leu Ala Ala Asp Asp Phe Arg Val Lys
            100                 105                 110

Tyr Glu Thr Glu Leu Ala Met Arg Gln Ser Val Glu Asn Asp Ile His
        115                 120                 125

Gly Leu Arg Lys Val Ile Asp Asp Thr Asn Ile Thr Arg Leu Gln Leu
    130                 135                 140

Glu Thr Glu Ile Glu Ala Leu Lys Glu Glu Leu Leu Phe Met Lys Lys
145                 150                 155                 160

Asn His Glu Glu Glu Val Lys Gly Leu Gln Ala Gln Ile Ala Ser Ser
                165                 170                 175

Gly Leu Thr Val Glu Val Asp Ala Pro Lys Ser Gln Asp Leu Ala Lys
            180                 185                 190

Ile Met Ala Asp Ile Arg Ala Gln Tyr Asp Glu Leu Ala Arg Lys Asn
        195                 200                 205

Arg Glu Glu Leu Asp Lys Tyr Trp Ser Gln Gln Ile Glu Glu Ser Thr
    210                 215                 220

Thr Val Val Thr Thr Gln Ser Ala Glu Val Gly Ala Ala Glu Thr Thr
225                 230                 235                 240

Leu Thr Glu Leu Arg Arg Thr Val Gln Ser Leu Glu Ile Asp Leu Asp
                245                 250                 255

Ser Met Arg Asn Leu Lys Ala Ser Leu Glu Asn Ser Leu Arg Glu Val
            260                 265                 270

Glu Ala Arg Tyr Ala Leu Gln Met Glu Gln Leu Asn Gly Ile Leu Leu
        275                 280                 285

His Leu Glu Ser Glu Leu Ala Gln Thr Arg Ala Glu Gly Gln Arg Gln
    290                 295                 300

Ala Gln Glu Tyr Glu Ala Leu Leu Asn Ile Lys Val Lys Leu Glu Ala
305                 310                 315                 320

Glu Ile Ala Thr Tyr Arg Arg Leu Leu Glu Asp Gly Glu Asp Phe Asn
                325                 330                 335

Leu Gly Asp Ala Leu Asp Ser Ser Asn Ser Met Gln Thr Ile Gln Lys
            340                 345                 350

Thr Thr Thr Arg Arg Ile Val Asp Gly Lys Val Val Ser Glu Thr Asn
        355                 360                 365

Asp Thr Lys Val Leu Arg His
    370                 375


<210> 269
<211> 423
<212> PRT
<213> Mus musculus

<400> 269
Met Ser Phe Thr Thr Arg Ser Thr Thr Phe Ser Thr Asn Tyr Arg Ser
  1               5                  10                  15

Leu Gly Ser Val Arg Thr Pro Ser Gln Arg Val Arg Pro Ala Ser Ser
             20                  25                  30

Ala Ala Ser Val Tyr Ala Gly Ala Gly Gly Ser Gly Ser Arg Ile Ser
         35                  40                  45

Val Ser Arg Ser Val Trp Gly Gly Ser Val Gly Ser Ala Gly Leu Ala
     50                  55                  60

Gly Met Gly Gly Ile Gln Thr Glu Lys Glu Thr Met Gln Asp Leu Asn
 65                  70                  75                  80

Asp Arg Leu Ala Ser Tyr Leu Asp Lys Val Lys Ser Leu Glu Thr Glu
                 85                  90                  95

Asn Arg Arg Leu Glu Ser Lys Ile Arg Glu His Leu Glu Lys Lys Gly
            100                 105                 110

Pro Gln Gly Val Arg Asp Trp Gly His Tyr Phe Lys Ile Ile Glu Asp
        115                 120                 125

Leu Arg Ala Gln Ile Phe Ala Asn Ser Val Asp Asn Ala Arg Ile Val
    130                 135                 140

Leu Gln Ile Asp Asn Ala Arg Leu Ala Ala Asp Asp Phe Arg Val Lys
145                 150                 155                 160

Tyr Glu Thr Glu Leu Ala Met Arg Gln Ser Val Glu Ser Asp Ile His
                165                 170                 175

Gly Leu Arg Lys Val Val Asp Asp Thr Asn Ile Thr Arg Leu Gln Leu
            180                 185                 190

Glu Thr Glu Ile Glu Ala Leu Lys Glu Glu Leu Leu Phe Met Lys Lys

195                 200                 205

Asn His Glu Glu Glu Val Gln Gly Leu Glu Ala Gln Ile Ala Ser Ser
    210                 215                 220

Gly Leu Thr Val Glu Val Asp Ala Pro Lys Ser Gln Asp Leu Ser Lys
225                 230                 235                 240

Ile Met Ala Asp Ile Arg Ala Gln Tyr Glu Ala Leu Ala Gln Lys Asn
                245                 250                 255

Arg Glu Glu Leu Asp Lys Tyr Trp Ser Gln Gln Ile Glu Glu Ser Thr
            260                 265                 270

Thr Val Val Thr Thr Lys Ser Ala Glu Ile Arg Asp Ala Glu Thr Thr
        275                 280                 285

Leu Thr Glu Leu Arg Arg Thr Leu Gln Thr Leu Glu Ile Asp Leu Asp
    290                 295                 300

Ser Met Lys Asn Gln Asn Ile Asn Leu Glu Asn Ser Leu Gly Asp Val
305                 310                 315                 320

Glu Ala Arg Tyr Lys Ala Gln Met Glu Gln Leu Asn Gly Val Leu Leu
                325                 330                 335

His Leu Glu Ser Glu Leu Ala Gln Thr Arg Ala Glu Gly Gln Arg Gln
            340                 345                 350

Ala Gln Glu Tyr Glu Ala Leu Leu Asn Ile Lys Val Lys Leu Glu Ala
        355                 360                 365

Glu Ile Ala Thr Tyr Arg Arg Leu Leu Glu Asp Gly Glu Asp Phe Ser
    370                 375                 380

Leu Asn Asp Ala Leu Asp Ser Ser Asn Ser Met Gln Thr Val Gln Lys
385                 390                 395                 400

Thr Thr Thr Arg Lys Ile Val Asp Gly Arg Val Val Ser Glu Thr Asn
                405                 410                 415

Asp Thr Arg Val Leu Arg His
            420


<210> 270
<211> 312
<212> PRT
<213> Homo sapiens

<400> 270
Asn Glu Lys Glu Gln Met Gln Asn Leu Asn Asp Arg Leu Ala Ser Tyr
1               5                   10                  15

Ile Asp Lys Val Arg Phe Leu Glu Gln Gln Asn Lys Glu Leu Glu Val
            20                  25                  30

Lys Ile Glu Glu Leu Arg Gln Lys Gln Ala Pro Ser Val Ser Arg Leu
        35                  40                  45

Tyr Ser Leu Tyr Glu Thr Glu Ile Glu Glu Leu Arg Arg Gln Ile Asp
    50                  55                  60

Gln Leu Thr Asn Glu Arg Ala Arg Leu Gln Leu Glu Ile Asp Asn Leu
65                  70                  75                  80

Arg Glu Ala Ala Glu Asp Phe Arg Lys Lys Tyr Glu Asp Glu Ile Asn
                85                  90                  95

Leu Arg Gln Glu Ala Glu Asn Asp Leu Val Gly Leu Arg Lys Asp Leu
            100                 105                 110

Asp Glu Ala Thr Leu Ala Arg Val Asp Leu Glu Asn Lys Val Glu Ser
        115                 120                 125

Leu Gln Glu Glu Leu Glu Phe Leu Lys Lys Asn His Glu Glu Glu Val
    130                 135                 140

Lys Glu Leu Gln Ala Gln Ile Gln Asp Thr Val Asn Val Glu Met Asp
145                 150                 155                 160

Ala Ala Arg Lys Leu Asp Leu Thr Lys Ala Leu Arg Glu Ile Arg Ala
                165                 170                 175

Gln Tyr Glu Glu Ile Ala Lys Lys Asn Arg Gln Glu Ala Glu Glu Trp
            180                 185                 190

Tyr Lys Ser Lys Leu Glu Glu Leu Gln Thr Ala Ala Ala Arg Asn Gly
        195                 200                 205

Glu Ala Leu Arg Ser Ala Lys Glu Glu Ile Thr Glu Leu Arg Arg Gln
    210                 215                 220

Ile Gln Ser Leu Glu Ile Glu Leu Gln Ser Leu Lys Ala Gln Asn Ala
225                 230                 235                 240

Ser Leu Glu Arg Gln Leu Ala Glu Leu Glu Glu Arg Tyr Glu Leu Glu
                245                 250                 255

Leu Arg Gln Tyr Gln Ala Leu Ile Ser Gln Leu Glu Glu Glu Leu Gln
            260                 265                 270

Gln Leu Arg Glu Glu Met Ala Arg Gln Leu Arg Glu Tyr Gln Glu Leu
        275                 280                 285

Leu Asp Val Lys Leu Ala Leu Asp Ile Glu Ile Ala Thr Tyr Arg Lys
    290                 295                 300

Leu Leu Glu Gly Glu Glu Ser Arg
305                 310


<210> 271
<211> 4302
<212> PRT
<213> Homo sapiens

<400> 271

<210> 272
<211> 4302
<212> PRT
<213> Homo sapiens

<400> 272


<210> 273
<211> 4292
<212> PRT
<213> Homo sapiens

<400> 273

<210> 274
<211> 4303
<212> PRT
<213> Homo sapiens

<400> 274


<210> 275
<211> 4283
<212> PRT
<213> Rattus norvegicus

<400> 275
Ala Leu Ala Leu Gly Leu Gly Leu Trp Leu Gly Ala Leu Ala Gly Asp
1               5                   10                  15

Pro Gly Arg Gly Cys Gly Pro Cys Pro Leu Pro Cys Phe Cys Ser Pro
            20                  25                  30

Ala Pro Asp Ala Ala Cys Arg Val Asn Cys Ser Gly Arg Trp Leu Gln

        35                  40                  45

Thr Leu Gly Pro Ser Leu Arg Ile Pro Ala Asp Ala Thr Ala Leu Asp
    50                  55                  60

Leu Ser His Asn Leu Leu Gln Thr Leu Asp Ile Arg Leu Leu Val Asn
65                  70                  75                  80

Leu Ser Gly Leu Val Glu Leu Asp Leu Ser Asn Asn Arg Ile Ser Thr
                85                  90                  95

Leu Glu Glu Gly Val Phe Ala Asn Leu Phe Asn Leu Ser Glu Ile Asn
            100                 105                 110

Leu Ser Gly Asn Pro Phe Glu Cys Asn Cys Gly Leu Ala Trp Leu Pro
        115                 120                 125

Arg Trp Ala Lys Glu Gln Gln Val His Val Val Gln Ser Glu Ala Thr
    130                 135                 140

Thr Cys Arg Gly Pro Val Pro Leu Ala Gly Arg Pro Leu Leu Ser Thr
145                 150                 155                 160

Pro Leu Leu Asp Asn Ala Cys Gly Glu Glu Tyr Val Ala Cys Leu Pro
                165                 170                 175

Asp Asn Ser Ser Gly Ala Val Ala Ala Val Pro Phe Phe Phe Ala His
            180                 185                 190

Gln Gly Pro Leu Glu Thr Ala Ala Cys Ser Ala Phe Cys Phe Ser Ala
        195                 200                 205

Gly Glu Gly Leu Ala Ala Leu Ser Glu Gln Asn Gln Cys Leu Cys Gly
    210                 215                 220

Ala Gly Gln Pro Ser Asn Thr Ser Ala Ala Cys Ser Ser Trp Cys Ser
225                 230                 235                 240

Ser Ile Leu Leu Ser Phe Asn Ser Ala Cys Gly Gly Pro Thr Leu Leu
                245                 250                 255

Gln His Thr Phe Pro Ala Ser Pro Gly Ala Ala Leu Val Gly Pro His
            260                 265                 270

Gly Pro Leu Ala Ser Gly Gln Pro Ala Asp Phe His Ile Asn Ser Pro
        275                 280                 285

Leu Pro Ile Ser Ser Thr Cys Trp Asn Phe Gly Asp Gly Ser Pro Glu
    290                 295                 300

Val Asp Met Ala Gly Pro Ala Ala Thr His Ser Tyr Val Leu Pro Gly
305                 310                 315                 320

Gly Tyr His Val Thr Val Val Leu Thr Leu Gly Ala Gly Ser Ala Leu
                325                 330                 335

Leu Glu Thr Glu Val Gln Val Glu Val Ala Pro Thr Val Leu Glu Leu
            340                 345                 350

Val Cys Pro Ser Phe Val His Ser Asp Glu Ser Leu Asp Leu Gly Ile
        355                 360                 365

Arg His Arg Gly Gly Ser Ala Leu Glu Val Thr Tyr Ser Ile Leu Ala
    370                 375                 380

Leu Asp Lys Glu Pro Ala Gln Val Val His Pro Leu Cys Pro Ser Asp
385                 390                 395                 400

Thr Glu Ile Phe Pro Gly Asn Gly His Cys Tyr Arg Leu Val Ala Glu

                405                 410                 415

Lys Ala Pro Trp Leu Gln Ala Gln Glu Gln Cys Arg Thr Trp Ala Gly
            420                 425                 430

Ala Ala Leu Ala Met Val Asp Ser Pro Ala Ile Gln His Phe Leu Val
        435                 440                 445

Ser Lys Val Thr Arg Ser Leu Asp Val Trp Ile Gly Phe Ser Ser Val
    450                 455                 460

Glu Gly Lys Glu Gly Leu Asp Pro Gln Gly Glu Ala Phe Ser Leu Glu
465                 470                 475                 480

Ser Cys Gln Asn Trp Leu Pro Gly Glu Pro His Pro Ala Thr Glu Glu
                485                 490                 495

His Cys Val Arg Leu Gly Pro Ala Gly Gln Cys Asn Thr Asp Leu Cys
            500                 505                 510

Ser Ala Pro His Ser Tyr Val Cys Glu Leu Arg Pro Gly Gly Pro Val
        515                 520                 525

Gly Asp Ala Asp Asn Phe Leu Leu Gly Val Ser Gly Gly Gly Arg Ser
    530                 535                 540

Gly Pro Leu Gln Pro Leu Ala Gln Gln Gly Thr Leu Gln Gly Pro Leu
545                 550                 555                 560

Gln Pro Val Glu Val Met Val Phe Pro Gly Leu Ser Pro Ser Arg Glu
                565                 570                 575

Ala Phe Leu Thr Ala Ala Glu Phe Glu Thr Gln Glu Leu Glu Asp Pro
            580                 585                 590

Val Gln Leu Arg Leu Gln Val Tyr Arg His Ser Arg Glu Ala Val Ala
        595                 600                 605

Pro Glu Gly Ser Ser Glu Leu Asp Asn Ser Thr Glu Pro Ala Pro Lys
    610                 615                 620

Cys Val Pro Glu Glu Leu Trp Cys Pro Gly Ala Asn Val Cys Val Pro
625                 630                 635                 640

Phe Asp Ala Leu Cys Asn Ser His Val Cys Ile Asn Gly Ser Ala Ser
                645                 650                 655

Arg Leu Gly Leu Pro Arg Ala Ser Tyr Thr Leu Trp Lys Glu Phe Phe
            660                 665                 670

Phe Ser Val Pro Ala Gly Pro Pro Thr Gln Tyr Leu Val Thr Leu His
        675                 680                 685

Gly Gln Asp Val Pro Met Leu Pro Gly Asp Leu Val Ala Leu Gln His
    690                 695                 700

Asp Ala Gly Pro Gly Thr Phe Leu His Cys Pro Leu Ala Ser Ser Cys
705                 710                 715                 720

Pro Gly Gln Ala Leu Tyr Leu Ser Thr Asn Ala Ser Asp Trp Met Thr
                725                 730                 735

Asn Leu Pro Val His Leu Glu Glu Ala Trp Ala Gly Pro Val Cys Ser
            740                 745                 750

Leu Gln Leu Leu Leu Val Thr Glu Gln Leu Thr Pro Leu Leu Gly Leu
        755                 760                 765

Gly Ser Asn Pro Gly Leu Gln His Pro Gly His Tyr Glu Val Arg Ala

770                 775                 780

Thr Val Gly Asn Ser Ile Ser Arg Gln Asn Leu Ser Cys Ser Phe Ser
785                 790                 795                 800

Val Val Ser Pro Val Ala Gly Leu Arg Val Ile His Pro Ile Pro Leu
                805                 810                 815

Asp Gly His Ile Tyr Val Pro Thr Asn Gly Ser Ile Leu Val Leu Gln
            820                 825                 830

Val Asp Ser Gly Ala Asn Ala Thr Ala Thr Ala His Trp Phe Gly Gly
        835                 840                 845

Asn Val Ser Ala Pro Phe Glu Asp Ala Cys Pro Pro Glu Val Asp Phe
    850                 855                 860

Leu Lys Gln Asp Cys Thr Glu Glu Ala Asn Ala Thr Leu Phe Ser Val
865                 870                 875                 880

Leu Val Leu Pro Arg Leu Lys Glu Gly Asp His Thr Val Glu Ile Val
                885                 890                 895

Ala Gln Asn Gly Ala Ser Gln Ala Asn Leu Ser Leu Arg Val Thr Ala
            900                 905                 910

Glu Glu Pro Ile Cys Gly Leu Arg Ala Val Pro Ser Pro Glu Ala Arg
        915                 920                 925

Val Leu Gln Gly Ile Leu Val Arg Tyr Ser Pro Met Val Glu Ala Gly
    930                 935                 940

Ser Asp Val Ala Phe Arg Trp Thr Ile Asp Asp Lys Gln Ser Leu Thr
945                 950                 955                 960

Phe His Asn Thr Val Phe Asn Val Ile Tyr Gln Thr Ala Ala Val Phe
                965                 970                 975

Lys Leu Ser Leu Thr Ala Ser Asn His Val Ser Asn Ile Thr Val Asn
            980                 985                 990

Tyr Asn Val Thr Val Glu Arg Met Asn Lys Met His Gly Leu Trp Val
        995                 1000                1005

Ser Ala Val Pro Ala Val Leu Pro Pro Asn Ala Thr Leu Ala Leu Thr
    1010                1015                1020

Gly Gly Val Leu Val Asp Ser Ala Val Glu Val Ala Phe Leu Trp Asn
1025                1030                1035                1040

Phe Gly Asp Gly Glu Gln Val Leu Arg Gln Phe Lys Pro Pro Tyr Asp
                1045                1050                1055

Glu Ser Phe Gln Val Pro Asp Pro Thr Val Ala Gln Val Leu Val Glu
            1060                1065                1070

His Asn Thr Thr His Ile Tyr Asn Thr Pro Gly Glu Tyr Asn Leu Thr
        1075                1080                1085

Val Thr Val Ser Asn Thr Tyr Glu Asn Leu Thr Gln Gln Val Pro Val
    1090                1095                1100

Ser Val Arg Thr Val Leu Pro Asn Val Thr Ile Gly Met Ser Ser Asn
1105                1110                1115                1120

Val Leu Leu Ala Gly Gln Pro Ile Thr Phe Phe Pro Tyr Pro Leu Pro
                1125                1130                1135

Ser Ala Asp Gly Val Leu Tyr Thr Trp Asp Phe Gly Asp Gly Ser Pro

1140                1145                1150

Val Leu Ile Gln Ser Gln Pro Val Leu Asn His Thr Tyr Ser Met Thr
        1155                1160                1165

Gly Thr Tyr Arg Ile Ser Leu Glu Val Asn Asn Thr Val Ser Ser Val
    1170                1175                1180

Ala Ala His Val Asp Ile Cys Val Phe Gln Glu Leu His Gly Leu Thr
1185                1190                1195                1200

Val Tyr Leu Asn Gln Ser Val Glu Gln Gly Ala Pro Met Val Val Asn
                1205                1210                1215

Ala Ser Val Glu Ser Gly Asp Asn Ile Thr Trp Thr Phe Asp Met Gly
            1220                1225                1230

Asp Gly Thr Val Phe Thr Gly Pro Glu Ala Thr Val Glu His Val Tyr
        1235                1240                1245

Leu Arg Ala Gln Asn Phe Thr Val Thr Val Gly Ala Thr Ser Pro Ala
    1250                1255                1260

Gly His Leu Ser Gln Ser Leu His Val Gln Val Phe Val Leu Glu Val
1265                1270                1275                1280

Leu Arg Ile Glu Pro Ser Thr Cys Ile Pro Thr Gln Pro Ser Ala Gln
                1285                1290                1295

Ile Met Ala His Val Thr Gly Asp Pro Ala His Tyr Leu Phe Asp Trp
            1300                1305                1310

Thr Phe Gly Asp Gly Ser Ser Asn Val Thr Val His Gly His Pro Ser
        1315                1320                1325

Val Thr His Asn Phe Thr Arg Ser Gly Ile Phe Pro Leu Ala Leu Val
    1330                1335                1340

Leu Ser Ser His Val Asn Lys Ala His Tyr Phe Thr Ser Ile Cys Val
1345                1350                1355                1360

Glu Pro Glu Ile Cys Asn Val Thr Leu Gln Pro Glu Arg Gln Val Val
                1365                1370                1375

Lys Leu Gly Asp Glu Ala Trp Leu Val Ala Tyr Pro Trp Pro Pro Phe
            1380                1385                1390

Pro Tyr Arg Tyr Thr Trp Asp Phe Gly Thr Glu Asp Ser Ile His Thr
        1395                1400                1405

Gln Thr Gly Gly Ser Glu Val Thr Phe Ile Tyr Arg Glu Pro Gly Ser
    1410                1415                1420

Tyr Leu Val Ile Val Thr Val Ser Asn Asn Ile Ser Ser Thr Asn Asp
1425                1430                1435                1440

Ser Ala Phe Val Asp Val Gln Glu Pro Val Ser Val Thr Gly Ile Arg
                1445                1450                1455

Ile Asn Gly Ser His Val Leu Glu Leu Gln Gln Pro Tyr Leu Phe Ser
            1460                1465                1470

Ala Met Gly Asn Gly Ser Pro Ala Ala Tyr Leu Trp Glu Leu Gly Asp
        1475                1480                1485

Gly Ser Gln His Glu Gly Pro Glu Val Thr His Ile Tyr Ser Ser Thr
    1490                1495                1500

Gly Asp Phe Thr Val Arg Val Ser Gly Trp Asn Glu Val Ser Arg Ser

1505                1510                1515                1520

Glu Ala Gln Leu Asn Ile Thr Val Lys Gln Arg Val Arg Gly Leu Thr
                1525                1530                1535

Ile Asn Ala Ser Pro Thr Val Val Pro Leu Asn Gly Ser Val Ser Phe
            1540                1545                1550

Ser Thr Leu Leu Glu Val Gly Ser Asp Val His Tyr Ser Trp Val Leu
        1555                1560                1565

Cys Asp Arg Cys Thr Pro Ile Pro Gly Gly Pro Thr Ile Ser Tyr Thr
    1570                1575                1580

Phe Arg Ser Val Gly Thr Phe Asn Ile Ile Val Thr Ala Glu Asn Glu
1585                1590                1595                1600

Val Gly Ser Ala Gln Asp Ser Ile Phe Ile Tyr Val Leu Gln Leu Ile
                1605                1610                1615

Glu Gly Leu Gln Val Val Gly Gly Asp Gly Gly Cys Cys Phe Pro Thr
            1620                1625                1630

Asn Tyr Thr Leu Gln Leu Gln Ala Ala Val Arg Asp Gly Thr Asn Ile
        1635                1640                1645

Ser Tyr Ser Trp Thr Ala Gln Gln Asp Gly Gly Pro Thr Leu Ile Ser
    1650                1655                1660

Ser Gly Lys Ser Phe Ser Leu Thr Ala Leu Lys Ala Ser Thr Tyr Tyr
1665                1670                1675                1680

Val His Leu Arg Ala Thr Asn Met Leu Gly Ser Ala Ser Ala Asn Arg
                1685                1690                1695

Thr Ile Asp Phe Val Glu Pro Val Glu Ser Leu Ile Leu Ser Ala Ser
            1700                1705                1710

Pro Asn Pro Ala Ala Val Asn Thr Ser Leu Thr Leu Gly Ala Glu Leu
        1715                1720                1725

Ala Gly Gly Ser Gly Val Val Tyr Thr Trp Tyr Leu Glu Glu Gly Leu
    1730                1735                1740

Ser Arg Glu Thr Ser Met Pro Ser Thr Thr His Thr Phe Ala Ala Pro
1745                1750                1755                1760

Gly Leu His Leu Val Arg Val Thr Ala Glu Asn Gln Leu Gly Ser Val
                1765                1770                1775

Asn Ala Thr Ile Glu Val Ala Val His Gly Pro Val Gly Gly Leu Ser
            1780                1785                1790

Ile Arg Thr Ser Glu Pro Asp Ser Ile Phe Val Ala Ala Gly Ser Thr
        1795                1800                1805

Val Pro Phe Trp Gly Gln Leu Ala Glu Gly Thr Asn Val Thr Trp Cys
    1810                1815                1820

Trp Thr Leu Pro Gly Gly Ser Lys Tyr Ser Gln Tyr Ile Asp Val Arg
1825                1830                1835                1840

Phe Pro Ala Ala Gly His Phe Ser Leu Trp Leu Asn Ala Ser Asn Ala
                1845                1850                1855

Val Ser Trp Val Ser Ala Val Tyr Asn Leu Thr Val Glu Glu Pro Ile
            1860                1865                1870

Met Asn Leu Val Leu Trp Ala Ser Ser Lys Val Val Ala Pro Gly Gln

1875                1880                1885

Pro Val His Phe Gln Ile Leu Leu Ala Ala Gly Ser Ala Val Thr Phe
    1890                1895                1900

Arg Leu Gln Val Gly Gly Ser Ile Pro Glu Val Leu Pro Ser Leu His
1905                1910                1915                1920

Phe Ser His Ser Phe Phe Arg Val Gly Asp His Met Val Ser Val Gln
                1925                1930                1935

Ala Glu Asn His Val Ser Arg Ala Gln Ala Gln Val Arg Ile Leu Val
            1940                1945                1950

Leu Glu Ala Ile Val Gly Leu Gln Val Pro Asn Cys Cys Glu Pro Gly
        1955                1960                1965

Met Ala Thr Gly Thr Glu Lys Asn Phe Thr Ala Arg Val Gln Arg Gly
    1970                1975                1980

Ser Arg Val Ala Tyr Ala Trp Tyr Phe Ser Leu Gln Lys Val Gln Gly
1985                1990                1995                2000

Asp Ser Leu Val Ile Leu Ser Gly Arg Asp Val Thr Tyr Thr Pro Val
                2005                2010                2015

Ala Ala Gly Ile Leu Glu Ile His Val Arg Ala Phe Asn Glu Leu Gly
            2020                2025                2030

Gly Val Asn Leu Thr Leu Val Val Glu Val Gln Asp Ile Ile Gln Tyr
        2035                2040                2045

Val Thr Leu Gln Ser Gly Arg Cys Phe Thr Asn Arg Ser Ala Arg Phe
    2050                2055                2060

Glu Ala Ala Thr Ser Pro Ser Pro Arg Arg Val Thr Tyr His Trp Asp
2065                2070                2075                2080

Phe Gly Asp Gly Thr Pro Val Gln Glu Thr Glu Glu Ser Trp Thr Asp
                2085                2090                2095

His Tyr Tyr Leu His Pro Gly Asp Tyr Arg Val Glu Val Asn Ala Thr
            2100                2105                2110

Asn Leu Val Ser Phe Phe Val Ala Gln Ala Gln Val Thr Val Gln Val
        2115                2120                2125

Leu Ala Cys Arg Glu Pro Glu Val Glu Val Ala Leu Pro Leu Gln Val
    2130                2135                2140

Leu Met Arg Arg Ser Gln Arg Asn Tyr Leu Glu Ala His Val Asp Leu
2145                2150                2155                2160

Arg Asn Cys Val Ser Tyr Gln Thr Glu Tyr Arg Trp Glu Val Tyr Arg
                2165                2170                2175

Thr Thr Ser Cys Gln Arg Ala Gly Arg Met Thr Gln Met Val Leu Pro
            2180                2185                2190

Gly Val Asp Val Ser Arg Pro Gln Leu Val Val Pro Arg Leu Ala Leu
        2195                2200                2205

Pro Val Gly His Tyr Cys Phe Val Phe Val Val Ser Phe Gly Asp Thr
    2210                2215                2220

Pro Leu Ala Arg Ser Ile Gln Ala Asn Val Thr Val Ala Ala Glu Arg
2225                2230                2235                2240

Leu Val Pro Ile Ile Glu Gly Gly Ser Tyr Arg Val Trp Ser Asp Thr

2245                    2250                    2255

Gln Asp Leu Val Leu Asp Gly Ser Lys Ser Tyr Asp Pro Asn Leu Glu
        2260                    2265                    2270

Asp Gly Asp Gln Thr Pro Leu Asn Phe His Trp Ala Cys Val Ala Ser
    2275                    2280                    2285

Thr Gln Ser Glu Thr Gly Gly Cys Val Leu Lys Leu Trp Ala Arg Gly
    2290                    2295                    2300

Ser Ser Val Val Thr Ile Pro Leu Glu Arg Leu Glu Ala Gly Val Glu
2305                    2310                    2315                    2320

Tyr Thr Phe Asn Leu Ile Val Trp Lys Ala Gly Arg Lys Glu Glu Ala
                2325                    2330                    2335

Thr Asn Gln Thr Val Leu Ile Arg Ser Gly Arg Val Pro Ile Val Ser
            2340                    2345                    2350

Leu Glu Cys Val Ser Cys Lys Ala Gln Ala Val Tyr Glu Val Ser Arg
        2355                    2360                    2365

Ser Ser Tyr Val Tyr Leu Glu Gly His Cys His Asn Cys Ser Ser Gly
    2370                    2375                    2380

Ser Lys Gln Gly Arg Trp Ala Ala Arg Thr Phe Ser Asn Lys Thr Leu
2385                    2390                    2395                    2400

Met Leu Asn Glu Asn Thr Thr Ser Thr Gly Ser Thr Gly Met His Leu
                2405                    2410                    2415

Val Val Arg Pro Gly Ala Leu His Asp Gly Glu Gly Tyr Ile Phe Thr
            2420                    2425                    2430

Leu Thr Val Leu Gly His Ser Gly Glu Glu Glu Gly Cys Arg Ser Ile
        2435                    2440                    2445

Arg Leu Leu Pro Asn Arg Pro Pro Leu Gly Gly Ser Cys Arg Leu Phe
    2450                    2455                    2460

Pro Leu Glu Ser Val Arg Gly Leu Thr Thr Lys Val His Phe Glu Cys
2465                    2470                    2475                    2480

Thr Gly Trp Arg Asp Ala Glu Asp Gly Gly Ala Pro Leu Val Tyr Ala
                2485                    2490                    2495

Leu Arg Leu Lys Arg Cys His Gln Asn Tyr Cys Glu Asp Phe Cys Ile
            2500                    2505                    2510

Tyr Lys Gly Ser Leu Ser Thr Tyr Gly Ala Val Leu Pro Pro Gly Phe
        2515                    2520                    2525

Gln Pro Leu Phe Val Val Ser Leu Thr Val Val Val Glu Asp Gln Leu
    2530                    2535                    2540

Gly Ala Ser Val Val Ala Leu Asn Arg Ser Leu Thr Ile Val Leu Pro
2545                    2550                    2555                    2560

Glu Pro Ser Gly Asn Pro Ala Asp Leu Ile Pro Trp Leu His Ser Leu
                2565                    2570                    2575

Thr Ala Ser Val Leu Pro Gly Leu Leu Arg Gln Ala Asp Pro Gln His
            2580                    2585                    2590

Val Ile Glu Tyr Ser Leu Ala Leu Ile Thr Val Leu Asn Glu Tyr Glu
        2595                    2600                    2605

Gln Ala Pro Asp Met Val Ser Glu Pro Asn Leu Glu Gln Gln Leu Arg

2610                    2615                    2620

Ala Gln Met Arg Lys Asn Ile Thr Glu Thr Leu Ile Ser Leu Arg Val
2625                    2630                    2635                    2640

Asn Thr Val Asp Asp Ile Gln Gln Ile Thr Ala Ala Leu Ala Gln Cys
                2645                    2650                    2655

Met Val Ser Ser Arg Glu Leu Met Cys Arg Ser Cys Leu Lys Lys Met
            2660                    2665                    2670

Leu Gln Lys Leu Glu Gly Met Met Arg Ile Leu Gln Ala Glu Thr Thr
        2675                    2680                    2685

Glu Gly Thr Leu Thr Pro Thr Thr Ile Ala Asp Ser Ile Leu Asn Ile
    2690                    2695                    2700

Thr Gly Asp Leu Ile His Leu Ala Ser Leu Asp Met Gln Gly Pro Gln
2705                    2710                    2715                    2720

Pro Leu Glu Leu Gly Ala Glu Pro Pro Ser Leu Met Val Ala Ser Lys
                2725                    2730                    2735

Ala Tyr Asn Leu Ser Ser Ala Leu Met Cys Ile Leu Met Arg Ser Arg
            2740                    2745                    2750

Val Leu Asn Glu Glu Pro Leu Thr Leu Ala Gly Glu Glu Ile Val Ala
        2755                    2760                    2765

Leu Gly Lys Arg Ala Asp Pro Leu Ser Leu Leu Cys Tyr Gly Lys Ala
    2770                    2775                    2780

Trp Gly Pro Ser Cys His Phe Ser Ile Pro Glu Ala Phe Ser Gly Ala
2785                    2790                    2795                    2800

Leu Ser Asp Leu Ser Asp Val Val Gln Leu Ile Leu Leu Val Asp Ser
                2805                    2810                    2815

Asn Pro Phe Pro Phe Gly Tyr Ile Ser Asn Tyr Thr Val Ser Thr Lys
            2820                    2825                    2830

Val Ala Ser Met Ala Phe Gln Thr Gln Thr Gly Thr Gln Ile Pro Ile
        2835                    2840                    2845

Glu Gln Leu Ala Ala Asp Gly Ser Ile Thr Val Lys Val Pro Asn Asn
    2850                    2855                    2860

Ser Asp Gln Ala Ala Gln Ser Ser His Ser Pro Val Gly Ser Thr Ile
2865                    2870                    2875                    2880

Val Gln Pro Arg Ala Ser Val Ser Ala Val Val Thr Ala Asp Asn Ser
                2885                    2890                    2895

Asn Pro Gln Ala Gly Leu His Leu Arg Ile Thr Tyr Thr Val Leu Asn
            2900                    2905                    2910

Ala Arg Tyr Leu Ser Glu Glu Arg Glu Pro Tyr Leu Ala Val Tyr Leu
        2915                    2920                    2925

His Ser Val Ser Gln Pro Asn Glu Tyr Asn Cys Ser Ala Ser Arg Arg
    2930                    2935                    2940

Ile Ser Leu Glu Val Leu Glu Gly Ala Asp His Arg Pro Tyr Thr Phe
2945                    2950                    2955                    2960

Phe Ile Ala Pro Gly Thr Gly Thr Leu Gly Arg Ser Tyr Tyr Leu Asn
                2965                    2970                    2975

Leu Thr Ser His Phe His Trp Ser Ala Leu Glu Val Ser Val Gly Leu

2980                    2985                    2990

Tyr Thr Ser Leu Cys Gln Tyr Phe Ser Glu Glu Ala Met Met Trp Arg
        2995                    3000                    3005

Thr Glu Gly Ile Val Pro Leu Glu Glu Thr Ser Pro Ser Gln Ala Val
    3010                    3015                    3020

Cys Leu Thr Arg His Leu Thr Ala Phe Gly Ala Ser Leu Phe Val Pro
3025                    3030                    3035                    3040

Pro Ser His Val Gln Phe Ile Phe Pro Glu Pro Ser Val Ser Ile Asn
                3045                    3050                    3055

Tyr Ile Val Leu Leu Thr Cys Val Ile Cys Leu Val Thr Tyr Val Ile
            3060                    3065                    3070

Met Ala Met Ile Leu Arg Lys Leu Asp Gln Leu Asp Val Ser Arg Val
        3075                    3080                    3085

Arg Val Ile Pro Phe Cys Gly Lys Gly Gly Arg Phe Lys Tyr Glu Ile
    3090                    3095                    3100

Leu Val Lys Thr Gly Trp Ser Arg Gly Ser Gly Thr Thr Ala His Val
3105                    3110                    3115                    3120

Gly Ile Met Leu Tyr Gly Glu Asp Asn Arg Ser Gly His Arg His Leu
                3125                    3130                    3135

Asp Gly Asp Arg Ala Phe His Arg Asn Ser Leu Asp Ile Phe Gln Ile
            3140                    3145                    3150

Ala Thr Pro Gln Ser Leu Gly Ser Val Trp Lys Ile Arg Val Trp His
        3155                    3160                    3165

Asp Asn Lys Gly Leu Ser Pro Ala Trp Phe Leu Gln His Ile Ile Val
    3170                    3175                    3180

Arg Asp Leu Gln Ser Ala Arg Ser Thr Phe Phe Leu Val Asn Asp Trp
3185                    3190                    3195                    3200

Leu Ser Val Glu Thr Glu Ala Asn Gly Gly Leu Val Glu Lys Glu Val
                3205                    3210                    3215

Leu Ala Ala Asn Glu Ala Ala Leu Trp Gln Phe Gln Arg Leu Leu Val
            3220                    3225                    3230

Ala Glu Leu Gln Arg Gly Phe Phe Asp Lys His Ile Trp Leu Ser Ile
        3235                    3240                    3245

Trp Asp Arg Pro Pro Arg Ser Arg Phe Thr Arg Val Gln Arg Val Thr
    3250                    3255                    3260

Cys Cys Val Leu Leu Leu Cys Leu Phe Leu Ala Ala Asn Ala Val Trp
3265                    3270                    3275                    3280

Tyr Gly Val Val Gly Asp Thr Thr Tyr Ser Met Gly Pro Val Ser Ser
                3285                    3290                    3295

Leu Met Ser Pro Ser Val Asp Thr Val Ala Ile Gly Leu Val Ser Ser
            3300                    3305                    3310

Val Val Val Tyr Pro Val Tyr Leu Ala Val Leu Phe Leu Phe Arg Met
        3315                    3320                    3325

Ser Arg Ser Lys Val Ser Gly Asp Gln Asn Pro Thr Pro Thr Gly Gln
    3330                    3335                    3340

Gln Ala Leu Asp Val Asp Ser Tyr Leu Asp Pro Ser Val Leu Asp Ser

3345                3350                3355                3360

Ser Leu Leu Thr Leu Ser Gly Leu Thr Glu Ala Phe Ser Gly Gln Val
                3365                3370                3375

Lys Asn Asp Leu Phe Leu Glu Asp Ala Lys Ser Leu Val Cys Trp Pro
            3380                3385                3390

Ser Ser Glu Gly Thr Leu Ser Trp Pro Asp Leu Leu Ser Asp Pro Ser
        3395                3400                3405

Val Met Ser Ser Thr Leu Gln Arg Leu Ala Gln Gly Arg Pro Gly Cys
    3410                3415                3420

Met Leu Gly Ser Glu Glu Asp Gly Ala Ser Leu Val Ser Pro Ser Leu
3425                3430                3435                3440

Pro Ala Lys Tyr Leu Ser Ala Ser Asp Glu Asp Leu Ile His Gln Val
                3445                3450                3455

Leu Ala Asp Gly Ala Asn Asn Pro Asp Pro Thr Gln Asp Ala Leu Ile
            3460                3465                3470

Glu Arg Asp Leu Leu Thr Ser Leu Ser Ser Ala Pro Gly Glu Lys Thr
        3475                3480                3485

Glu Thr Leu Ile Leu Gln Thr Met Gly Glu Lys Arg Pro Pro Ser Met
    3490                3495                3500

Gly Leu Thr Trp Glu Gln Ser Pro Val Thr Arg Leu Ser Arg Thr Gly
3505                3510                3515                3520

Leu Val Glu Gly Leu Arg Lys Arg Leu Leu Pro Thr Trp Cys Ala Pro
                3525                3530                3535

Leu Ala His Gly Leu Ser Leu Leu Leu Val Ala Val Ala Val Ala Val
            3540                3545                3550

Ser Gly Trp Ile Gly Ala Ser Phe Pro Pro Ser Val Ser Val Met Trp
        3555                3560                3565

Leu Leu Ser Ser Ser Ser Ser Phe Leu Ala Ser Phe Leu Gly Trp Glu
    3570                3575                3580

Pro Leu Lys Val Leu Leu Glu Ala Leu Tyr Phe Ser Leu Val Ala Lys
3585                3590                3595                3600

Arg Leu His Pro Asp Glu Asp Asp Thr Leu Val Glu Ser Pro Ala Val
                3605                3610                3615

Thr Pro Val Ser Glu Arg Val Pro Arg Val Arg Pro Pro His Gly Phe
            3620                3625                3630

Ala Leu Phe Leu Ala Lys Glu Glu Ala Arg Lys Val Lys Arg Leu His
        3635                3640                3645

Asp Met Leu Lys Ser Leu Leu Val Tyr Met Leu Phe Leu Leu Val Thr
    3650                3655                3660

Leu Leu Ala Asn Tyr Gly Asp Ala Ser Cys His Gly His Ala Tyr Arg
3665                3670                3675                3680

Leu Gln Ser Ala Ile Lys Gln Glu Leu Asp Ser Gln Ala Phe Leu Ala
                3685                3690                3695

Ile Thr Arg Ser Asp Glu Phe Trp Pro Trp Met Ser His Val Leu Leu
            3700                3705                3710

Pro Tyr Val His Gly Asn Gln Ser Ser Pro Glu Leu Gly Pro Pro Arg

3715                    3720                    3725

Leu Arg Gln Val Arg Leu Gln Glu Ala Phe Cys Pro Asp Pro Ser Ser
    3730                    3735                    3740

Ser Glu His Met Cys Ser Ala Thr Gly Ser Leu Ser Thr Ser Asp Tyr
3745                    3750                    3755                    3760

Gly Ile Gly Trp Gln Ser Val Val Gln Asn Gly Ser Glu Thr Trp Ala
                3765                    3770                    3775

Tyr Ser Ala Pro Asp Leu Leu Gly Ala Trp Tyr Trp Gly Tyr Cys Ala
            3780                    3785                    3790

Val Tyr Asp Ser Gly Gly Tyr Ile Gln Glu Leu Gly Leu Ser Leu Glu
        3795                    3800                    3805

Glu Ser Arg Ala Arg Leu Gly Phe Leu Gln Leu His Asn Trp Leu Asp
    3810                    3815                    3820

Ser Arg Ser Arg Ala Val Phe Val Glu Leu Thr Arg Tyr Ser Pro Ala
3825                    3830                    3835                    3840

Val Gly Leu His Ala Ala Val Thr Leu Arg Leu Glu Phe Pro Val Ala
                3845                    3850                    3855

Gly His Ala Leu Ala Ala Phe Ser Val Arg Pro Phe Ala Leu Arg Arg
            3860                    3865                    3870

Leu Ser Thr Gly Leu Ser Leu Pro Leu Leu Thr Ser Val Cys Leu Leu
        3875                    3880                    3885

Leu Phe Ala Leu Tyr Phe Ser Val Ala Glu Val His Thr Trp Arg Arg
    3890                    3895                    3900

Glu Gly Cys Ala Arg Thr Ala Arg Pro Asp Val Gly Ala Arg Trp Leu
3905                    3910                    3915                    3920

Leu Val Met Leu Thr Ala Ala Thr Gly Leu Val Arg Leu Ala Gln Leu
                3925                    3930                    3935

Arg Ile Ala Asp Arg Gln Trp Thr Arg Phe Val His Asp His Pro His
            3940                    3945                    3950

His Phe Thr Ser Phe Asp Gln Val Ala Gln Leu Gly Ser Val Ala Arg
        3955                    3960                    3965

Gly Leu Ala Ala Ser Leu Leu Phe Leu Leu Leu Val Lys Ala Ala Gln
    3970                    3975                    3980

His Val Arg Phe Val Arg Gln Trp Ser Val Phe Gly Lys Thr Leu Cys
3985                    3990                    3995                    4000

Arg Ala Leu Pro Glu Leu Met Gly Ala Thr Leu Gly Leu Val Leu Leu
                4005                    4010                    4015

Gly Val Ala Tyr Ala Gln Met Ala Ile Leu Leu Ile Ser Ser Gly Ala
            4020                    4025                    4030

Asp Thr Leu Tyr Ser Met Ala Arg Ala Phe Leu Val Leu Cys Pro Gly
        4035                    4040                    4045

Ala Arg Val Pro Thr Leu Cys Pro Ser Glu Ser Trp Tyr Leu Ser Pro
    4050                    4055                    4060

Leu Leu Cys Val Gly Leu Trp Ala Leu Arg Val Trp Gly Ala Leu Arg
4065                    4070                    4075                    4080

Leu Gly Ala Val Leu Leu Arg Trp Arg Tyr His Ala Leu Arg Gly Glu

4085                4090                4095

Leu Tyr Arg Pro Ala Trp Glu Pro Gln Asp Tyr Glu Met Val Glu Leu
            4100                4105                4110

Phe Leu Arg Arg Leu Arg Leu Trp Met Gly Phe Thr Lys Val Lys Glu
        4115                4120                4125

Phe Arg His Lys Val Arg Phe Glu Gly Met Asp Pro Leu Pro Ser Arg
    4130                4135                4140

Ser Ser Arg Gly Ser Lys Ser Ser Pro Val Val Pro Pro Pro Ser Ala
4145                4150                4155                4160

Gly Ser Glu Ala Ser His Pro Ser Thr Ser Ser Ser Gln Pro Asp Gly
                4165                4170                4175

Leu Ser Ala Gly Leu Arg Arg Ser Ala Leu Arg Leu Glu Pro Glu Pro
            4180                4185                4190

Ser Arg Leu His Ala Val Phe Glu Ser Leu Leu Val Gln Phe Asp Arg
        4195                4200                4205

Leu Asn Gln Ala Thr Glu Asp Val Tyr Gln Leu Glu Gln Gln Leu Gln
    4210                4215                4220

Ser Leu Arg Gly His Gly His Ser Gly Pro Pro Ser Ser Pro Ser Pro
4225                4230                4235                4240

Gly Gly Phe Pro Ala Ser Gln Pro Ala Leu Pro Ser Arg Leu Ala Arg
                4245                4250                4255

Ala Ser Gln Gly Pro Asp Gln Thr Thr Gly Pro Ser Arg Val Ser Leu
            4260                4265                4270

Trp Pro Asn Asn Lys Val His Pro Ser Ser Thr
        4275                4280


<210> 276
<211> 547
<212> PRT
<213> Homo sapiens

<400> 276
Thr Leu Glu Ala Ser Val Asp Leu Lys Cys Cys Asp Ala Tyr Thr Ile
1               5                   10                  15

Asp Tyr Lys Trp Gln Ile Phe Ser Val Pro Ser Cys Asp Asp Asp Pro
            20                  25                  30

Gly Arg Thr Thr Pro Ile Pro Leu Pro Ala Val Asp Val Ser Arg Thr
        35                  40                  45

Arg Gln Leu His Ile Pro Arg Gly Ala Leu Pro Tyr Gly Val Tyr Val
    50                  55                  60

Phe Asn Phe Thr Leu Ser Ile Arg Leu Lys Thr Ser Gly Glu Pro Thr
65                  70                  75                  80

Leu Lys Lys Ser Ile Ile Ser Tyr Leu Trp Val Gln Ala Pro Pro Leu
                85                  90                  95

Val Ala Val Ile Glu Gly Gly Ala Tyr Arg Val Trp Ser Phe Thr Gln
            100                 105                 110

Asp Leu Ile Leu Asp Gly Ser Asn Ser Tyr Asp Pro Asp Ala Asp Pro
        115                 120                 125

Gly Ser Gln Ser Asn Leu Gln Phe His Trp Tyr Cys Val Thr Val Pro
    130                 135                 140

Arg Asp Tyr Met Gly Ser Ser Leu Asp Glu Ala Ser Gln Asn Val Cys
145                 150                 155                 160

His Pro Cys Gln Leu Ser Phe Gly Trp Pro Gly Ser Ser Gly Ser Val
                165                 170                 175

Leu Thr Ile Pro Pro Glu Thr Leu Glu Ala Asn Val Glu Tyr Thr Phe
            180                 185                 190

Arg Leu Val Ile Ser Lys Glu Gly Arg Ile Thr Glu Phe Thr Asp Gln
        195                 200                 205

Thr Val His Val Leu Ser Gly Leu Leu Pro Ile Val His Ile Ser Cys
    210                 215                 220

Ile Ser Asn Cys Ala Gln Tyr Leu Tyr Glu Val Ser Arg Phe Ser Leu
225                 230                 235                 240

Val Tyr Leu Cys Thr Asn Cys Asp Gly Asp Asp Ser Gly Arg Trp Ser
                245                 250                 255

Ala Leu Ser Leu Ser Asn Thr Thr Val Val Leu Asp Trp Ser Gly Gln
            260                 265                 270

Thr Thr Thr Gly Ser Asn Gly Pro Tyr Leu Val Leu Lys Pro Gly Ala
        275                 280                 285

Phe Arg Ser Gly Glu Glu Tyr Ser Phe Thr Leu Ser Val Thr Gly Ser
    290                 295                 300

Ser Trp Asp Ser Glu Gly Tyr Ala Ser Ile Ser Leu His Pro Pro Ile
305                 310                 315                 320

Asn His Pro Pro Ala Gly Gly Ser Cys Lys Leu Asn Pro Ala Glu Gly
                325                 330                 335

Ile Ala Leu Gln Thr Lys Phe Thr Val Glu Cys Ser Asn Phe Arg Asp
            340                 345                 350

Val Asp Glu Pro Leu Thr Tyr Lys Ile Ile Val Ser Arg Cys Arg Ser
        355                 360                 365

Val Gly Met Ile Ser Ser Tyr Cys Glu Asn Phe Leu Leu Tyr Glu Gly
    370                 375                 380

Ser Ala Pro Ile Lys Pro Gly Ala Phe Leu Pro Val Gly Phe Gly Thr
385                 390                 395                 400

Asp Gln His Asp Val Ser Leu Tyr Val Gln Val Tyr Asp Ser Leu Gly
                405                 410                 415

Ala Ala Ser Gln Val Leu Asn Ser Ala Thr Val His Val Pro Thr Asp
            420                 425                 430

Pro Ala Ser Ser Lys Asn Val Leu Gln Gln Leu Leu Ser Met Leu Leu
        435                 440                 445

Leu Pro Glu Ser Leu Leu Ser Thr Leu Leu Gln Gln Gly Asp Pro Gln
    450                 455                 460

Gln Ala Gly Glu Leu Ser Leu Ala Leu Ile Ser Val Leu Asn Glu Ile
465                 470                 475                 480

Glu Gln Glu Asp Asp Ser Glu Val Glu Arg Asp Asp Arg Ala Arg Leu
                485                 490                 495

Arg Lys Asn Leu Val Asp Gln Leu Ala Ala Leu Pro Val Asn Thr Val
            500                 505                 510

Asp Asp Ile Gln Gln Ser Ser Ala Ala Leu Ala Gln Cys Thr Gln Lys
        515                 520                 525

Ser Arg Glu Phe Ser Cys His Val Gln Met Leu Ala Thr Leu Arg Leu
    530                 535                 540

Leu Glu Ala
545


<210> 277
<211> 113
<212> PRT
<213> Homo sapiens

<400> 277
Val Arg Tyr Gln Leu Val Val Ala Thr Gly Gly Asp Glu Gly Ala Gly
  1               5                  10                  15

Thr Thr Gly Lys Val Ser Ile Ser Leu Tyr Gly Glu Glu Gly Glu Ser
             20                  25                  30

Gly Lys Ile Pro Leu Leu Lys Gly Glu Gly Ala Gly Pro Gly Ser Thr
         35                  40                  45

Phe Ser Phe Thr Phe Asp Val Asp Glu Asp Leu Gly Glu Leu Gly Ala
     50                  55                  60

Val Arg Ile Arg Asn Glu His Ser Gly Leu Phe Ser Pro Glu Trp Phe
 65                  70                  75                  80

Leu Lys Ser Ile Thr Val Glu Asp Gly Gly Thr Gln Gly Lys Val His
                 85                  90                  95

Phe Pro Cys Asn Ser Trp Val Tyr Gly Lys Asp Glu Thr Leu Phe Leu
            100                 105                 110

Arg



<210> 278
<211> 120
<212> PRT
<213> Homo sapiens

<400> 278
Ala Lys Tyr Lys Val Thr Val Thr Thr Gly Val Leu Asp Phe Ala Gly
  1               5                  10                  15

Thr Thr Ala Ser Val Ser Leu Ser Leu Ile Gly Ala Glu Gly Arg Gly
             20                  25                  30

Lys Glu Ser Lys Leu Asp Tyr Leu Glu Arg Pro Leu Leu Phe Ala Arg
         35                  40                  45

Gly Ser Thr Tyr Ser Phe Thr Phe Asp Val Asp Val Asp Phe Gly Glu
     50                  55                  60

Leu Gly Ala Val Lys Ile Lys Asn Glu His Ala Gly Leu His Pro Glu
 65                  70                  75                  80

Trp Phe Leu Lys Ser Ile Thr Val Lys Asp Gly Pro Thr Gly Gly Lys
                 85                  90                  95

Val His Phe Pro Cys Asn Ser Trp Val Tyr Pro Lys Lys Lys Tyr Pro

            100                 105                 110

Gly Glu Arg Ile Phe Phe Ala Asn
        115                 120

<210> 279
<211> 84
<212> PRT
<213> Homo sapiens

<400> 279
Glu Pro Val Pro Gly Leu Ser Val Ser Ala Ser Pro Ser Val Val Ala
1               5                   10                  15

Leu Gly Leu Thr Val Thr Phe Thr Ala Thr Ser Pro Asp Gly Ser Ile
            20                  25                  30

Val Ser Tyr Leu Trp Asp Phe Gly Asp Ser Pro Gly Leu Thr Ser Thr
        35                  40                  45

Gly Pro Asn Val Thr His Thr Tyr Leu Lys Pro Gly Thr Tyr Thr Val
    50                  55                  60

Thr Leu Thr Ala Thr Asn Asp Val Gly Ser Ala Ser Ala Thr Leu Thr
65                  70                  75                  80

Val Thr Val Val

<210> 280
<211> 389
<212> PRT
<213> Homo sapiens

<400> 280
Met Ile Arg Asn Gly Arg Gly Ala Ala Gly Gly Ala Glu Gln Pro Gly
1               5                   10                  15

Pro Gly Gly Arg Arg Ala Val Arg Val Trp Cys Asp Gly Cys Tyr Asp
            20                  25                  30

Met Val His Tyr Gly His Ser Asn Gln Leu Arg Gln Ala Arg Ala Met
        35                  40                  45

Gly Asp Tyr Leu Ile Val Gly Val His Thr Asp Glu Glu Ile Ala Lys
    50                  55                  60

His Lys Gly Pro Pro Val Phe Thr Gln Glu Glu Arg Tyr Lys Met Val
65                  70                  75                  80

Gln Ala Ile Lys Trp Val Asp Glu Val Val Pro Ala Ala Pro Tyr Val
                85                  90                  95

Thr Thr Leu Glu Thr Leu Asp Lys Tyr Asn Cys Asp Phe Cys Val His
            100                 105                 110

Gly Asn Asp Ile Thr Leu Thr Val Asp Gly Arg Asp Thr Tyr Glu Glu
        115                 120                 125

Val Lys Gln Ala Gly Arg Tyr Arg Glu Cys Lys Arg Thr Gln Gly Val
    130                 135                 140

Ser Thr Thr Asp Leu Val Gly Arg Met Leu Leu Val Thr Lys Ala His
145                 150                 155                 160

His Ser Ser Gln Glu Met Ser Ser Glu Tyr Arg Glu Tyr Ala Asp Ser
                165                 170                 175

Phe Gly Lys Cys Pro Gly Gly Arg Asn Pro Trp Thr Gly Val Ser Gln
            180                 185                 190

Phe Leu Gln Thr Ser Gln Lys Ile Ile Gln Phe Ala Ser Gly Lys Glu
        195                 200                 205

Pro Gln Pro Gly Glu Thr Val Ile Tyr Val Ala Gly Ala Phe Asp Leu
    210                 215                 220

Phe His Ile Gly His Val Asp Phe Leu Glu Lys Val His Arg Leu Ala
225                 230                 235                 240

Glu Arg Pro Tyr Ile Ile Ala Gly Leu His Phe Asp Gln Glu Val Asn
                245                 250                 255

His Tyr Lys Gly Lys Asn Tyr Pro Ile Met Asn Leu His Glu Arg Thr
            260                 265                 270

Leu Ser Val Leu Ala Cys Arg Tyr Val Ser Glu Val Val Ile Gly Ala
        275                 280                 285

Pro Tyr Ala Val Thr Ala Glu Leu Leu Ser His Phe Lys Val Asp Leu
    290                 295                 300

Val Cys His Gly Lys Thr Glu Ile Ile Pro Asp Arg Asp Gly Ser Asp
305                 310                 315                 320

Pro Tyr Gln Glu Pro Lys Arg Arg Gly Ile Phe Arg Gln Ile Asp Ser
                325                 330                 335

Gly Ser Asn Leu Thr Thr Asp Leu Ile Val Gln Arg Ile Ile Thr Asn
            340                 345                 350

Arg Leu Glu Tyr Glu Ala Arg Asn Gln Lys Lys Glu Ala Lys Glu Leu
        355                 360                 365

Ala Phe Leu Glu Ala Ala Arg Gln Gln Ala Ala Gln Pro Leu Gly Glu
    370                 375                 380

Arg Asp Gly Asp Phe
385

<210> 281
<211> 389
<212> PRT
<213> Homo sapiens

<400> 281

<210> 282
<211> 404
<212> PRT
<213> Rattus norvegicus

<400> 282
Met Ile Arg Asn Gly His Gly Ala Gly Gly Ala Ala Gly Leu Lys Gly
1               5                   10                  15

Pro Gly Gly Gln Arg Thr Val Arg Val Trp Cys Asp Gly Cys Tyr Asp
            20                  25                  30

Met Val His Tyr Gly His Ser Asn Gln Leu Arg Gln Ala Arg Ala Met
        35                  40                  45

Gly Asp Tyr Leu Ile Val Gly Val His Thr Asp Glu Glu Ile Ala Lys
    50                  55                  60

His Lys Gly Pro Pro Val Phe Thr Gln Glu Glu Arg Tyr Lys Met Val
65                  70                  75                  80

Gln Ala Ile Lys Trp Val Asp Glu Val Val Pro Ala Ala Pro Tyr Val
                85                  90                  95

Thr Thr Leu Glu Thr Leu Asp Lys His Asn Cys Asp Phe Cys Val His
            100                 105                 110

Gly Asn Asp Ile Thr Leu Thr Val Asp Gly Arg Asp Thr Tyr Glu Glu
        115                 120                 125

Val Lys Gln Ala Gly Arg Tyr Arg Glu Cys Lys Arg Thr Gln Gly Val
    130                 135                 140

Ser Thr Thr Asp Leu Val Gly Arg Met Leu Leu Val Thr Lys Ala His
145                 150                 155                 160

His Ser Ser Gln Glu Met Ser Ser Glu Tyr Arg Glu Tyr Ala Asp Ser
                165                 170                 175

Phe Gly Lys Pro Pro His Pro Thr Pro Ala Gly Asp Thr Leu Ser Ser
            180                 185                 190

Glu Val Ser Ser Gln Cys Pro Gly Gly Gln Ser Pro Trp Thr Gly Val
        195                 200                 205

Ser Gln Phe Leu Gln Thr Ser Gln Lys Ile Ile Gln Phe Ala Ser Gly
    210                 215                 220

Lys Glu Pro Gln Pro Gly Glu Thr Val Ile Tyr Val Ala Gly Ala Phe
225                 230                 235                 240

Asp Leu Phe His Ile Gly His Val Asp Phe Leu Gln Glu Val His Lys
                245                 250                 255

Leu Ala Lys Arg Pro Tyr Val Ile Ala Gly Leu His Phe Asp Gln Glu
            260                 265                 270

Val Asn Arg Tyr Lys Gly Lys Asn Tyr Pro Ile Met Asn Leu His Glu
        275                 280                 285

Arg Thr Leu Ser Val Leu Ala Cys Arg Tyr Val Ser Glu Val Val Ile
    290                 295                 300

Gly Ala Pro Tyr Ser Val Thr Ala Glu Leu Leu Asn His Phe Lys Val
305                 310                 315                 320

Asp Leu Val Cys His Gly Lys Thr Glu Ile Val Pro Asp Arg Asp Gly
                325                 330                 335

Ser Asp Pro Tyr Glu Glu Pro Lys Arg Arg Gly Ile Phe Cys Gln Ile
            340                 345                 350

Asp Ser Gly Ser Asp Leu Thr Thr Asp Leu Ile Val Gln Arg Ile Ile
        355                 360                 365

Lys Asn Arg Leu Glu Tyr Glu Ala Arg Asn Gln Lys Lys Glu Ala Lys
    370                 375                 380

Glu Leu Ala Phe Leu Glu Ala Leu Arg Gln Gln Glu Ala Gln Pro Arg
385                 390                 395                 400

Gly Glu Thr Asp

<210> 283
<211> 404
<212> PRT
<213> Mus musculus

<400> 283
Met Ile Arg Asn Gly His Gly Ala Ala Ser Ala Ala Gly Leu Lys Gly
1               5                   10                  15

Pro Gly Asp Gln Arg Ile Val Arg Val Trp Cys Asp Gly Cys Tyr Asp
            20                  25                  30

Met Val His Tyr Gly His Ser Asn Gln Leu Arg Gln Ala Arg Ala Met
        35                  40                  45

Gly Asp Tyr Leu Ile Val Gly Val His Thr Asp Glu Glu Ile Ala Lys
    50                  55                  60

His Lys Gly Pro Pro Val Phe Thr Gln Glu Glu Arg Tyr Lys Met Val
65                  70                  75                  80

Gln Ala Ile Lys Trp Val Asp Glu Val Val Pro Ala Ala Pro Tyr Val
                85                  90                  95

Thr Thr Leu Glu Thr Leu Asp Lys His Asn Cys Asp Phe Cys Val His
            100                 105                 110

Gly Asn Asp Ile Thr Leu Thr Val Asp Gly Arg Asp Thr Tyr Glu Glu
        115                 120                 125

Val Lys Gln Ala Gly Arg Tyr Arg Glu Cys Lys Arg Thr Gln Gly Val
    130                 135                 140

Ser Thr Thr Asp Leu Val Gly Arg Met Leu Leu Val Thr Lys Ala His
145                 150                 155                 160

His Ser Ser Gln Glu Met Ser Ser Glu Tyr Arg Glu Tyr Ala Asp Ser
                165                 170                 175

Phe Gly Lys Pro Pro His Pro Thr Pro Ala Gly Asp Thr Leu Ser Ser
            180                 185                 190

Glu Val Ser Ser Gln Cys Pro Gly Gly Gln Ser Pro Trp Thr Gly Val
        195                 200                 205

Ser Gln Phe Leu Gln Thr Ser Gln Lys Ile Ile Gln Phe Ala Ser Gly
    210                 215                 220

Lys Glu Pro Gln Pro Gly Glu Thr Val Ile Tyr Val Ala Gly Ala Phe
225                 230                 235                 240

Asp Leu Phe His Ile Gly His Val Asp Phe Leu Gln Glu Val His Lys
                245                 250                 255

Leu Ala Lys Arg Pro Tyr Val Ile Ala Gly Leu His Phe Asp Gln Glu
            260                 265                 270

Val Asn Arg Tyr Lys Gly Lys Asn Tyr Pro Ile Met Asn Leu His Glu
        275                 280                 285

Arg Thr Leu Ser Val Leu Ala Cys Arg Tyr Val Ser Glu Val Val Ile
    290                 295                 300

Gly Ala Pro Tyr Ser Val Thr Ala Glu Leu Leu Asn His Phe Lys Val
305                 310                 315                 320

Asp Leu Val Cys His Gly Lys Thr Glu Ile Val Pro Asp Arg Asp Gly
                325                 330                 335

Ser Asp Pro Tyr Gln Glu Pro Lys Arg Arg Gly Ile Phe Tyr Gln Ile
            340                 345                 350

Asp Ser Gly Ser Asp Leu Thr Thr Asp Leu Ile Val Gln Arg Ile Ile
        355                 360                 365

Lys Asn Arg Leu Glu Tyr Glu Ala Arg Asn Gln Lys Lys Glu Ala Lys
    370                 375                 380

Glu Leu Ala Phe Leu Glu Ala Thr Lys Gln Gln Glu Ala Pro Pro Gly
385                 390                 395                 400

Gly Glu Ile Asp

<210> 284
<211> 404
<212> PRT
<213> Mus musculus

<400> 284
Met Ile Arg Asn Gly His Gly Ala Ala Ser Ala Ala Gly Leu Lys Gly
1               5                   10                  15

Pro Gly Asp Gln Arg Ile Val Arg Val Trp Cys Asp Gly Cys Tyr Asp
            20                  25                  30

Met Val His Tyr Gly His Ser Asn Gln Leu Arg Gln Ala Arg Ala Met
        35                  40                  45

Gly Asp Tyr Leu Ile Val Gly Val His Thr Asp Glu Glu Ile Ala Lys
    50                  55                  60

His Lys Gly Pro Pro Val Phe Thr Gln Glu Glu Arg Tyr Lys Met Val
65                  70                  75                  80

Gln Ala Ile Lys Trp Val Asp Glu Val Val Pro Ala Ala Pro Tyr Val
                85                  90                  95

Thr Thr Leu Glu Thr Leu Asp Lys His Asn Cys Asp Phe Ser Val His
            100                 105                 110

Gly Asn Asp Ile Thr Leu Thr Val Asp Gly Arg Asp Thr Tyr Glu Glu
        115                 120                 125

Val Lys Gln Ala Gly Arg Tyr Arg Glu Cys Lys Arg Thr Gln Gly Val
    130                 135                 140

Ser Thr Thr Asp Leu Val Gly Arg Met Leu Leu Val Thr Lys Ala His
145                 150                 155                 160

His Ser Ser Gln Glu Met Ser Ser Glu Tyr Arg Glu Tyr Ala Asp Ser
                165                 170                 175

Phe Gly Lys Pro Pro His Pro Thr Pro Ala Gly Asp Thr Leu Ser Ser
            180                 185                 190

Glu Val Ser Ser Gln Cys Pro Gly Gly Gln Ser Pro Trp Thr Gly Val
        195                 200                 205

Ser Gln Phe Leu Gln Thr Ser Gln Lys Ile Ile Gln Phe Ala Ser Gly
    210                 215                 220

Lys Glu Pro Gln Pro Gly Glu Thr Val Ile Tyr Val Ala Gly Ala Phe

225                 230                 235                 240

Asp Leu Phe His Ile Gly His Val Asp Phe Leu Gln Glu Val His Lys
                245                 250                 255

Leu Ala Lys Arg Pro Tyr Val Ile Ala Gly Leu His Phe Asp Gln Glu
            260                 265                 270

Val Asn Arg Tyr Lys Gly Lys Asn Tyr Pro Ile Met Asn Leu His Glu
        275                 280                 285

Arg Thr Leu Ser Val Leu Ala Cys Arg Tyr Val Ser Glu Val Val Ile
    290                 295                 300

Gly Ala Pro Tyr Ser Val Thr Ala Glu Leu Leu Asn His Phe Lys Val
305                 310                 315                 320

Asp Leu Val Cys His Gly Lys Thr Glu Ile Val Pro Asp Arg Asp Gly
                325                 330                 335

Ser Asp Pro Tyr Gln Glu Pro Lys Arg Arg Gly Ile Phe Tyr Gln Ile
            340                 345                 350

Asp Ser Gly Ser Asp Leu Thr Thr Asp Leu Ile Val Gln Arg Ile Ile
        355                 360                 365

Lys Asn Arg Leu Glu Tyr Glu Ala Arg Asn Gln Lys Lys Glu Ala Lys
    370                 375                 380

Glu Leu Ala Phe Leu Glu Ala Thr Lys Gln Gln Glu Ala Pro Pro Gly
385                 390                 395                 400

Gly Glu Ile Asp


<210> 285
<211> 129
<212> PRT
<213> Homo sapiens

<400> 285
Pro Lys Arg Val Ile Tyr Gly Gly Thr Phe Asp Pro Phe His Leu Gly
1               5                   10                  15

His Leu Asp Leu Leu Glu Arg Ala Lys Glu Leu Gly Asp Glu Leu Ile
            20                  25                  30

Val Gly Val Ala Ser Asp Glu Ser Lys Lys Lys Leu Lys Pro Phe Thr
        35                  40                  45

Ala Glu Glu Arg Arg Lys Met Leu Leu Glu Ala Leu Lys Asp Val Asp
    50                  55                  60

Glu Val Tyr Val Phe Ala Pro Asp Asp Leu Thr Val Glu Phe Ile Lys
65                  70                  75                  80

Glu Ile Lys Pro Asp Val Ile Val Arg Gly Leu Asp Val Val Ser Phe
                85                  90                  95

Glu Tyr Glu Leu Val Tyr Ala Leu Val Lys Arg Ala Gly Leu Glu Val
            100                 105                 110

Val Phe Leu Pro Arg Thr Glu Gly Phe Ser Thr Ser Asp Ile Val Lys
        115                 120                 125

Arg

<210> 286
<211> 1025
<212> PRT
<213> Homo sapiens

<400> 286
Met Lys Ala Ser Ser Gly Asp Gln Gly Ser Pro Pro Cys Phe Leu Arg
1               5                   10                  15

Phe Pro Arg Pro Val Arg Val Val Ser Gly Ala Glu Ala Glu Leu Lys
            20                  25                  30

Cys Val Val Leu Gly Glu Pro Pro Pro Val Val Val Trp Glu Lys Gly
        35                  40                  45

Gly Gln Gln Leu Ala Ala Ser Glu Arg Leu Ser Phe Pro Ala Asp Gly
    50                  55                  60

Ala Glu His Gly Leu Leu Leu Thr Ala Ala Leu Pro Thr Asp Ala Gly
65                  70                  75                  80

Val Tyr Val Cys Arg Ala Arg Asn Ala Ala Gly Glu Ala Tyr Ala Ala
                85                  90                  95

Ala Ala Val Thr Val Leu Glu Pro Pro Ala Ser Asp Pro Glu Leu Gln
            100                 105                 110

Pro Ala Glu Arg Pro Leu Pro Ser Pro Gly Ser Gly Glu Gly Ala Pro
        115                 120                 125

Val Phe Leu Thr Gly Pro Arg Ser Gln Trp Val Leu Arg Gly Ala Glu
    130                 135                 140

Val Val Leu Thr Cys Arg Ala Gly Gly Leu Pro Glu Pro Thr Leu Tyr
145                 150                 155                 160

Trp Glu Lys Asp Gly Met Ala Leu Asp Glu Val Trp Asp Ser Ser His
                165                 170                 175

Phe Ala Leu Gln Pro Gly Arg Ala Glu Asp Gly Pro Gly Ala Ser Leu
            180                 185                 190

Ala Leu Arg Ile Leu Ala Ala Arg Leu Pro Asp Ser Gly Val Tyr Val
        195                 200                 205

Cys His Ala Arg Asn Ala His Gly His Ala Gln Ala Gly Ala Leu Leu
    210                 215                 220

Gln Val His Gln Pro Pro Glu Ser Pro Pro Ala Asp Pro Asp Glu Ala
225                 230                 235                 240

Pro Ala Pro Val Val Glu Pro Leu Lys Cys Ala Pro Lys Thr Phe Trp
                245                 250                 255

Val Asn Glu Gly Lys His Ala Lys Phe Arg Cys Tyr Val Met Gly Lys
            260                 265                 270

Pro Glu Pro Glu Ile Glu Trp His Trp Glu Gly Arg Pro Leu Leu Pro
        275                 280                 285

Asp Arg Arg Arg Leu Met Tyr Arg Asp Arg Asp Gly Gly Phe Val Leu
    290                 295                 300

Lys Val Leu Tyr Cys Gln Ala Lys Asp Arg Gly Leu Tyr Val Cys Ala
305                 310                 315                 320

Ala Arg Asn Ser Ala Gly Gln Thr Leu Ser Ala Val Gln Leu His Val
                325                 330                 335

Lys Glu Pro Arg Leu Arg Phe Thr Arg Pro Leu Gln Asp Val Glu Gly
        340                 345                 350

Arg Glu His Gly Ile Ala Val Leu Glu Cys Lys Val Pro Asn Ser Arg
        355                 360                 365

Ile Pro Thr Ala Trp Phe Arg Glu Asp Gln Arg Leu Leu Pro Cys Arg
    370                 375                 380

Lys Tyr Glu Gln Ile Glu Glu Gly Thr Val Arg Arg Leu Ile Ile His
385                 390                 395                 400

Arg Leu Lys Ala Asp Asp Asp Gly Ile Tyr Leu Cys Glu Met Arg Gly
                405                 410                 415

Arg Val Arg Thr Val Ala Asn Val Thr Val Lys Gly Pro Ile Leu Lys
            420                 425                 430

Arg Leu Pro Arg Lys Leu Asp Val Leu Glu Gly Glu Asn Ala Val Leu
        435                 440                 445

Leu Val Glu Thr Leu Glu Ala Gly Val Glu Gly Arg Trp Ser Arg Asp
    450                 455                 460

Gly Glu Glu Leu Pro Val Ile Cys Gln Ser Ser Ser Gly His Met His
465                 470                 475                 480

Ala Leu Val Leu Pro Gly Val Thr Arg Glu Asp Ala Gly Glu Val Thr
                485                 490                 495

Phe Ser Leu Gly Asn Ser Arg Thr Thr Thr Leu Leu Arg Val Lys Cys
            500                 505                 510

Val Lys His Ser Pro Pro Gly Pro Pro Ile Leu Ala Glu Met Phe Lys
        515                 520                 525

Gly His Lys Asn Thr Val Leu Leu Thr Trp Lys Pro Pro Glu Pro Ala
    530                 535                 540

Pro Glu Thr Pro Phe Ile Tyr Arg Leu Glu Arg Gln Glu Val Gly Ser
545                 550                 555                 560

Glu Asp Trp Ile Gln Cys Phe Ser Ile Glu Lys Ala Gly Ala Val Glu
                565                 570                 575

Val Pro Gly Asp Cys Val Pro Ser Glu Gly Asp Tyr Arg Phe Arg Ile
            580                 585                 590

Cys Thr Val Ser Gly His Gly Arg Ser Pro His Val Val Phe His Gly
        595                 600                 605

Ser Ala His Leu Val Pro Thr Ala Arg Leu Val Ala Gly Leu Glu Asp
    610                 615                 620

Val Gln Val Tyr Asp Gly Glu Asp Ala Val Phe Ser Leu Asp Leu Ser
625                 630                 635                 640

Thr Ile Ile Gln Gly Thr Trp Phe Leu Asn Gly Glu Glu Leu Lys Ser
                645                 650                 655

Asn Glu Pro Glu Gly Gln Val Glu Pro Gly Ala Leu Arg Tyr Arg Ile
            660                 665                 670

Glu Gln Lys Gly Leu Gln His Arg Leu Ile Leu His Ala Val Lys His
        675                 680                 685

Gln Asp Ser Gly Ala Leu Val Gly Phe Ser Cys Pro Gly Val Gln Asp
    690                 695                 700

Ser Ala Ala Leu Thr Ile Gln Glu Ser Pro Val His Ile Leu Ser Pro
705                 710                 715                 720

Gln Asp Arg Val Ser Leu Thr Phe Thr Thr Ser Glu Arg Val Val Leu
                725                 730                 735

Thr Cys Glu Leu Ser Arg Val Asp Phe Pro Ala Thr Trp Tyr Lys Asp
            740                 745                 750

Gly Gln Lys Val Glu Glu Ser Glu Leu Leu Val Val Lys Met Asp Gly
        755                 760                 765

Arg Lys His Arg Leu Ile Leu Pro Glu Ala Lys Val Gln Asp Ser Gly
    770                 775                 780

Glu Phe Glu Cys Arg Thr Glu Gly Val Ser Ala Phe Phe Gly Val Thr
785                 790                 795                 800

Val Gln Asp Pro Pro Val His Ile Val Asp Pro Arg Glu His Val Phe
                805                 810                 815

Val His Ala Ile Thr Ser Glu Cys Val Met Leu Ala Cys Glu Val Asp
            820                 825                 830

Arg Glu Asp Ala Pro Val Arg Trp Tyr Lys Asp Gly Gln Glu Val Glu
        835                 840                 845

Glu Ser Asp Phe Val Val Leu Glu Asn Glu Gly Pro His Arg Arg Leu
    850                 855                 860

Val Leu Pro Ala Thr Gln Pro Ser Asp Gly Gly Glu Phe Gln Cys Val
865                 870                 875                 880

Ala Gly Asp Glu Cys Ala Tyr Phe Thr Val Thr Ile Thr Asp Val Ser
                885                 890                 895

Ser Trp Ile Val Tyr Pro Ser Gly Lys Val Tyr Val Ala Ala Val Arg
            900                 905                 910

Leu Glu Arg Val Val Leu Thr Cys Glu Leu Cys Arg Pro Trp Ala Glu
        915                 920                 925

Val Arg Trp Thr Lys Asp Gly Glu Glu Val Val Glu Ser Pro Ala Leu
    930                 935                 940

Leu Leu Gln Lys Glu Asp Thr Val Arg Arg Leu Val Leu Pro Ala Val
945                 950                 955                 960

Gln Leu Glu Asp Ser Gly Glu Tyr Leu Cys Glu Ile Asp Asp Glu Ser
                965                 970                 975

Ala Ser Phe Thr Val Thr Val Thr Glu Ser Tyr Gln Ser Gln Asp Ser
            980                 985                 990

Ser Asn Asn Asn Pro Glu Leu Cys Val Leu Leu Lys Lys Pro Lys Thr
        995                 1000                1005

Arg Arg Leu Trp Ser Arg Phe Pro Pro Trp Arg Arg Thr Ala Gly Thr
    1010                1015                1020

Glu
1025


<210> 287
<211> 1044
<212> PRT
<213> Homo sapiens

<400> 287
Leu Arg Thr Pro Pro Cys Pro Pro Thr Val Arg Arg Cys Arg Arg Ser
1               5                   10                  15

Ala Ala Ala Met Lys Ala Ser Ser Gly Asp Gln Gly Ser Pro Pro Cys
            20                  25                  30

Phe Leu Arg Phe Pro Arg Pro Val Arg Val Val Ser Gly Ala Glu Ala
        35                  40                  45

Glu Leu Lys Cys Val Val Leu Gly Glu Pro Pro Pro Val Val Val Trp
    50                  55                  60

Glu Lys Gly Gly Gln Gln Leu Ala Ala Ser Glu Arg Leu Ser Phe Pro
65                  70                  75                  80

Ala Asp Gly Ala Glu His Gly Leu Leu Leu Thr Ala Ala Leu Pro Thr
                85                  90                  95

Asp Ala Gly Val Tyr Val Cys Arg Ala Arg Asn Ala Ala Gly Glu Ala
            100                 105                 110

Tyr Ala Ala Ala Ala Val Thr Val Leu Glu Pro Pro Ala Ser Asp Pro
        115                 120                 125

Glu Leu Gln Pro Ala Glu Arg Pro Leu Pro Ser Pro Gly Ser Gly Glu
    130                 135                 140

Gly Ala Pro Val Phe Leu Thr Gly Pro Arg Ser Gln Trp Val Leu Arg
145                 150                 155                 160

Gly Ala Glu Val Val Leu Thr Cys Arg Ala Gly Gly Leu Pro Glu Pro
                165                 170                 175

Thr Leu Tyr Trp Glu Lys Asp Gly Met Ala Leu Asp Glu Val Trp Asp
            180                 185                 190

Ser Ser His Phe Ala Leu Gln Pro Gly Arg Ala Glu Asp Gly Pro Gly
        195                 200                 205

Ala Ser Leu Ala Leu Arg Ile Leu Ala Ala Arg Leu Pro Asp Ser Gly
    210                 215                 220

Val Tyr Val Cys His Ala Arg Asn Ala His Gly His Ala Gln Ala Gly
225                 230                 235                 240

Ala Leu Leu Gln Val His Gln Pro Pro Glu Ser Pro Pro Ala Asp Pro
                245                 250                 255

Asp Glu Ala Pro Ala Pro Val Val Glu Pro Leu Lys Cys Ala Pro Lys
            260                 265                 270

Thr Phe Trp Val Asn Glu Gly Lys His Ala Lys Phe Arg Cys Tyr Val
        275                 280                 285

Met Gly Lys Pro Glu Pro Glu Ile Glu Trp His Trp Glu Gly Arg Pro
    290                 295                 300

Leu Leu Pro Asp Arg Arg Arg Leu Met Tyr Arg Asp Arg Asp Gly Gly
305                 310                 315                 320

Phe Val Leu Lys Val Leu Tyr Cys Gln Ala Lys Asp Arg Gly Leu Tyr
                325                 330                 335

Val Cys Ala Ala Arg Asn Ser Ala Gly Gln Thr Leu Ser Ala Val Gln
            340                 345                 350

Leu His Val Lys Glu Pro Arg Leu Arg Phe Thr Arg Pro Leu Gln Asp

        355                 360                 365

Val Glu Gly Arg Glu His Gly Ile Ala Val Leu Glu Cys Lys Val Pro
    370                 375                 380

Asn Ser Arg Ile Pro Thr Ala Trp Phe Arg Glu Asp Gln Arg Leu Leu
385                 390                 395                 400

Pro Cys Arg Lys Tyr Glu Gln Ile Glu Glu Gly Thr Val Arg Arg Leu
                405                 410                 415

Ile Ile His Arg Leu Lys Ala Asp Asp Asp Gly Ile Tyr Leu Cys Glu
            420                 425                 430

Met Arg Gly Arg Val Arg Thr Val Ala Asn Val Thr Val Lys Gly Pro
        435                 440                 445

Ile Leu Lys Arg Leu Pro Arg Lys Leu Asp Val Leu Glu Gly Glu Asn
    450                 455                 460

Ala Val Leu Leu Val Glu Thr Leu Glu Ala Gly Val Glu Gly Arg Trp
465                 470                 475                 480

Ser Arg Asp Gly Glu Glu Leu Pro Val Ile Cys Gln Ser Ser Ser Gly
                485                 490                 495

His Met His Ala Leu Val Leu Pro Gly Val Thr Arg Glu Asp Ala Gly
            500                 505                 510

Glu Val Thr Phe Ser Leu Gly Asn Ser Arg Thr Thr Thr Leu Leu Arg
        515                 520                 525

Val Lys Cys Val Lys His Ser Pro Pro Gly Pro Pro Ile Leu Ala Glu
    530                 535                 540

Met Phe Lys Gly His Lys Asn Thr Val Leu Leu Thr Trp Lys Pro Pro
545                 550                 555                 560

Glu Pro Ala Pro Glu Thr Pro Phe Ile Tyr Arg Leu Glu Arg Gln Glu
                565                 570                 575

Val Gly Ser Glu Asp Trp Ile Gln Cys Phe Ser Ile Glu Lys Ala Gly
            580                 585                 590

Ala Val Glu Val Pro Gly Asp Cys Val Pro Ser Glu Gly Asp Tyr Arg
        595                 600                 605

Phe Arg Ile Cys Thr Val Ser Gly His Gly Arg Ser Pro His Val Val
    610                 615                 620

Phe His Gly Ser Ala His Leu Val Pro Thr Ala Arg Leu Val Ala Gly
625                 630                 635                 640

Leu Glu Asp Val Gln Val Tyr Asp Gly Glu Asp Ala Val Phe Ser Leu
                645                 650                 655

Asp Leu Ser Thr Ile Ile Gln Gly Thr Trp Phe Leu Asn Gly Glu Glu
            660                 665                 670

Leu Lys Ser Asn Glu Pro Glu Gly Gln Val Glu Pro Gly Ala Leu Arg
        675                 680                 685

Tyr Arg Ile Glu Gln Lys Gly Leu Gln His Arg Leu Ile Leu His Ala
    690                 695                 700

Val Lys His Gln Asp Ser Gly Ala Leu Val Gly Phe Ser Cys Pro Gly
705                 710                 715                 720

Val Gln Asp Ser Ala Ala Leu Thr Ile Gln Glu Ser Pro Val His Ile

                725                 730                 735

Leu Ser Pro Gln Asp Lys Val Ser Leu Thr Phe Thr Thr Ser Glu Arg
            740                 745                 750

Val Val Leu Thr Cys Glu Leu Ser Arg Val Asp Phe Pro Ala Thr Trp
        755                 760                 765

Tyr Lys Asp Gly Gln Lys Val Glu Glu Ser Glu Leu Leu Val Val Lys
    770                 775                 780

Met Asp Gly Arg Lys His Arg Leu Ile Leu Pro Glu Ala Lys Val Gln
785                 790                 795                 800

Asp Ser Gly Glu Phe Glu Cys Arg Thr Glu Gly Val Ser Ala Phe Phe
                805                 810                 815

Gly Val Thr Val Gln Asp Pro Pro Val His Ile Val Asp Pro Arg Glu
            820                 825                 830

His Val Phe Val His Ala Ile Thr Ser Glu Cys Val Met Leu Ala Cys
        835                 840                 845

Glu Val Asp Arg Glu Asp Ala Pro Val Arg Trp Tyr Lys Asp Gly Gln
    850                 855                 860

Glu Val Glu Glu Ser Asp Phe Val Val Leu Glu Asn Glu Gly Pro His
865                 870                 875                 880

Arg Arg Leu Val Leu Pro Ala Thr Gln Pro Ser Asp Gly Gly Glu Phe
                885                 890                 895

Gln Cys Val Ala Gly Asp Glu Cys Ala Tyr Phe Thr Val Thr Ile Thr
            900                 905                 910

Asp Val Ser Ser Trp Ile Val Tyr Pro Ser Gly Lys Val Tyr Val Ala
        915                 920                 925

Ala Val Arg Leu Glu Arg Val Val Leu Thr Cys Glu Leu Cys Arg Pro
    930                 935                 940

Trp Ala Glu Val Arg Trp Thr Lys Asp Gly Glu Glu Val Val Glu Ser
945                 950                 955                 960

Pro Ala Leu Leu Leu Gln Lys Glu Asp Thr Val Arg Arg Leu Val Leu
                965                 970                 975

Pro Ala Val Gln Leu Glu Asp Ser Gly Glu Tyr Leu Cys Glu Ile Asp
            980                 985                 990

Asp Glu Ser Ala Ser Phe Thr Val Thr Val Thr Glu Ser Tyr Gln Ser
        995                 1000                1005

Gln Asp Ser Ser Asn Asn Asn Pro Glu Leu Cys Val Leu Leu Lys Lys
    1010                1015                1020

Pro Lys Thr Arg Arg Leu Trp Ser Arg Phe Pro Pro Trp Arg Arg Thr
1025                1030                1035                1040

Ala Gly Thr Glu


<210> 288
<211> 180
<212> PRT
<213> Homo sapiens

<400> 288

Met Ser Ala Arg Leu Arg Ala Arg Glu Ser Pro Pro Pro Pro Pro Gln
1               5                   10                  15

Ser Cys Ser Ser Gly Pro Arg Pro Gly Arg Ala Glu Asp Gly Pro Gly
            20                  25                  30

Ala Ser Leu Ala Leu Arg Ile Leu Ala Ala Arg Leu Pro Asp Ser Gly
        35                  40                  45

Val Tyr Val Cys His Ala Arg Asn Ala His Gly His Ala Gln Ala Gly
    50                  55                  60

Ala Leu Leu Gln Val His Gln Pro Pro Glu Ser Pro Pro Ala Asp Pro
65                  70                  75                  80

Asp Glu Ala Pro Ala Pro Val Val Glu Pro Leu Lys Cys Ala Pro Lys
                85                  90                  95

Thr Phe Trp Val Asn Glu Gly Lys His Ala Lys Phe Arg Cys Tyr Val
            100                 105                 110

Met Gly Lys Pro Glu Pro Glu Ile Glu Trp His Trp Glu Gly Arg Pro
        115                 120                 125

Leu Leu Pro Asp Arg Arg Arg Leu Met Tyr Arg Asp Arg Asp Gly Gly
    130                 135                 140

Phe Val Leu Lys Val Leu Tyr Cys Gln Ala Lys Asp Arg Gly Leu Tyr
145                 150                 155                 160

Val Cys Ala Ala Arg Asn Ser Ala Gly Gln Thr Leu Ser Ala Val Gln
                165                 170                 175

Leu His Val Lys
            180


<210> 289
<211> 304
<212> PRT
<213> Homo sapiens

<400> 289
Met Leu Pro Pro Pro Arg Pro Ala Ala Ala Leu Ala Leu Pro Val Leu
1               5                   10                  15

Leu Leu Leu Leu Val Val Leu Thr Pro Pro Pro Thr Gly Ala Arg Pro
            20                  25                  30

Ser Pro Gly Pro Asp Tyr Leu Arg Arg Gly Trp Met Arg Leu Leu Ala
        35                  40                  45

Glu Gly Glu Gly Cys Ala Pro Cys Arg Pro Glu Glu Cys Ala Ala Pro
    50                  55                  60

Arg Gly Cys Leu Ala Gly Arg Val Arg Asp Ala Cys Gly Cys Cys Trp
65                  70                  75                  80

Glu Cys Ala Asn Leu Glu Gly Gln Leu Cys Asp Leu Asp Pro Ser Ala
                85                  90                  95

His Phe Tyr Gly His Cys Gly Glu Gln Leu Glu Cys Arg Leu Asp Thr
            100                 105                 110

Gly Gly Asp Leu Ser Arg Gly Glu Val Pro Glu Pro Leu Cys Ala Cys
        115                 120                 125

Arg Ser Gln Ser Pro Leu Cys Gly Ser Asp Gly His Thr Tyr Ser Gln
    130                 135                 140

Ile Cys Arg Leu Gln Glu Ala Ala Arg Ala Arg Pro Asp Ala Asn Leu
145                 150                 155                 160

Thr Val Ala His Pro Gly Pro Cys Glu Ser Gly Pro Gln Ile Val Ser
                165                 170                 175

His Pro Tyr Asp Thr Trp Asn Val Thr Gly Gln Asp Val Ile Phe Gly
            180                 185                 190

Cys Glu Val Phe Ala Tyr Pro Met Ala Ser Ile Glu Trp Arg Lys Asp
        195                 200                 205

Gly Leu Asp Ile Gln Leu Pro Gly Asp Asp Pro His Ile Ser Val Gln
    210                 215                 220

Phe Arg Gly Gly Pro Gln Arg Phe Glu Val Thr Gly Trp Leu Gln Ile
225                 230                 235                 240

Gln Ala Val Arg Pro Ser Asp Glu Gly Thr Tyr Arg Cys Leu Gly Arg
                245                 250                 255

Asn Ala Leu Gly Gln Val Glu Ala Pro Ala Ser Leu Thr Val Leu Thr
            260                 265                 270

Pro Asp Gln Leu Asn Ser Thr Gly Ile Pro Gln Leu Arg Ser Leu Asn
        275                 280                 285

Leu Val Pro Glu Glu Glu Ala Glu Ser Glu Glu Asn Asp Asp Tyr Tyr
    290                 295                 300

<210> 290
<211> 6620
<212> PRT
<213> Homo sapiens

<400> 290
Met Asp Gln Pro Gln Phe Ser Gly Ala Pro Arg Phe Leu Thr Arg Pro
1               5                   10                  15

Lys Ala Phe Val Val Ser Val Gly Lys Asp Ala Thr Leu Ser Cys Gln
            20                  25                  30

Ile Val Gly Asn Pro Thr Pro Gln Val Ser Trp Glu Lys Asp Gln Gln
        35                  40                  45

Pro Val Thr Ala Gly Ala Arg Phe Arg Leu Ala Gln Asp Gly Asp Leu
    50                  55                  60

Tyr Arg Leu Thr Ile Leu Asp Leu Ala Leu Gly Asp Ser Gly Gln Tyr
65                  70                  75                  80

Val Cys Arg Ala Arg Asn Ala Ile Gly Glu Ala Phe Ala Ala Val Gly
                85                  90                  95

Leu Gln Val Asp Ala Glu Ala Ala Cys Ala Glu Gln Ala Pro His Phe
            100                 105                 110

Leu Leu Arg Pro Thr Ser Ile Arg Val Arg Glu Gly Ser Glu Ala Thr
        115                 120                 125

Phe Arg Cys Arg Val Gly Gly Ser Pro Arg Pro Ala Val Ser Trp Ser
    130                 135                 140

Lys Asp Gly Arg Arg Leu Gly Glu Pro Asp Gly Pro Arg Val Arg Val

145                 150                 155                 160

Glu Glu Leu Gly Glu Ala Ser Ala Leu Arg Ile Arg Ala Ala Arg Pro
                165                 170                 175

Arg Asp Gly Gly Thr Tyr Glu Val Arg Ala Glu Asn Pro Leu Gly Ala
            180                 185                 190

Ala Ser Ala Ala Ala Ala Leu Val Val Asp Ser Asp Ala Ala Asp Thr
        195                 200                 205

Ala Ser Arg Pro Gly Thr Ser Thr Ala Ala Leu Leu Ala His Leu Gln
    210                 215                 220

Arg Arg Arg Glu Ala Met Arg Ala Glu Gly Ala Pro Ala Ser Pro Pro
225                 230                 235                 240

Ser Thr Gly Thr Arg Thr Cys Thr Val Thr Glu Gly Lys His Ala Arg
                245                 250                 255

Leu Ser Cys Tyr Val Thr Gly Glu Pro Lys Pro Glu Thr Val Trp Lys
            260                 265                 270

Lys Asp Gly Gln Leu Val Thr Glu Gly Arg Arg His Val Val Tyr Glu
        275                 280                 285

Asp Ala Gln Glu Asn Phe Val Leu Lys Ile Leu Phe Cys Lys Gln Ser
    290                 295                 300

Asp Arg Gly Leu Tyr Thr Cys Thr Ala Ser Asn Leu Val Gly Gln Thr
305                 310                 315                 320

Tyr Ser Ser Val Leu Val Val Val Arg Glu Pro Ala Val Pro Phe Lys
                325                 330                 335

Lys Arg Leu Gln Asp Leu Glu Val Arg Glu Lys Glu Ser Ala Thr Phe
            340                 345                 350

Leu Cys Glu Val Pro Gln Pro Ser Thr Glu Ala Ala Trp Phe Lys Glu
        355                 360                 365

Glu Thr Arg Leu Trp Ala Ser Ala Lys Tyr Gly Ile Glu Glu Glu Gly
    370                 375                 380

Thr Glu Arg Arg Leu Thr Val Arg Asn Val Ser Ala Asp Asp Asp Ala
385                 390                 395                 400

Val Tyr Ile Cys Glu Thr Pro Glu Gly Ser Arg Thr Val Ala Glu Leu
                405                 410                 415

Ala Val Gln Gly Asn Leu Leu Arg Lys Leu Pro Arg Lys Thr Ala Val
            420                 425                 430

Arg Val Gly Asp Thr Ala Met Phe Cys Val Glu Leu Ala Val Pro Val
        435                 440                 445

Gly Pro Val His Trp Leu Arg Asn Gln Glu Glu Val Val Ala Gly Gly
    450                 455                 460

Arg Val Ala Ile Ser Ala Glu Gly Thr Arg His Thr Leu Thr Ile Ser
465                 470                 475                 480

Gln Cys Cys Leu Glu Asp Val Gly Gln Val Ala Phe Met Ala Gly Asp
                485                 490                 495

Cys Gln Thr Ser Thr Arg Phe Cys Val Ser Ala Pro Arg Lys Pro Pro
            500                 505                 510

Leu Gln Pro Pro Val Asp Pro Val Val Lys Ala Arg Met Glu Ser Ser

515                 520                 525

Val Ile Leu Ser Trp Ser Pro Pro Pro His Gly Glu Arg Pro Val Thr
    530                 535                 540

Ile Asp Gly Tyr Leu Val Glu Lys Lys Lys Leu Gly Thr Tyr Thr Trp
545                 550                 555                 560

Ile Arg Cys His Glu Ala Glu Trp Val Ala Thr Pro Glu Leu Thr Val
                565                 570                 575

Ala Asp Val Ala Glu Glu Gly Asn Phe Gln Phe Arg Val Ser Ala Leu
            580                 585                 590

Asn Ser Phe Gly Gln Ser Pro Tyr Leu Glu Phe Pro Gly Thr Val His
        595                 600                 605

Leu Ala Pro Lys Leu Ala Val Arg Thr Pro Leu Lys Ala Val Gln Ala
    610                 615                 620

Val Glu Gly Gly Glu Val Thr Phe Ser Val Asp Leu Thr Val Ala Ser
625                 630                 635                 640

Ala Gly Glu Trp Phe Leu Asp Gly Gln Ala Leu Lys Ala Ser Ser Val
                645                 650                 655

Tyr Glu Ile His Cys Asp Arg Thr Arg His Thr Leu Thr Ile Arg Glu
            660                 665                 670

Val Pro Ala Ser Leu His Gly Ala Gln Leu Lys Phe Val Ala Asn Gly
        675                 680                 685

Ile Glu Ser Ser Ile Arg Met Glu Val Arg Ala Ala Pro Gly Leu Thr
    690                 695                 700

Ala Asn Lys Pro Pro Ala Ala Ala Ala Arg Glu Val Leu Ala Arg Leu
705                 710                 715                 720

His Glu Glu Ala Gln Leu Leu Ala Glu Leu Ser Asp Gln Ala Ala Ala
                725                 730                 735

Val Thr Trp Leu Lys Asp Gly Arg Thr Leu Ser Pro Gly Pro Lys Tyr
            740                 745                 750

Glu Val Gln Ala Ser Ala Gly Arg Arg Val Leu Leu Val Arg Asp Val
        755                 760                 765

Ala Arg Asp Asp Ala Gly Leu Tyr Glu Cys Val Ser Arg Gly Gly Arg
    770                 775                 780

Ile Ala Tyr Gln Leu Ser Val Gln Gly Leu Ala Arg Phe Leu His Lys
785                 790                 795                 800

Asp Met Ala Gly Ser Cys Val Asp Ala Val Ala Gly Gly Pro Ala Gln
                805                 810                 815

Phe Glu Cys Glu Thr Ser Glu Ala His Val His Val His Trp Tyr Lys
            820                 825                 830

Asp Gly Met Glu Leu Gly His Ser Gly Glu Arg Phe Leu Gln Glu Asp
        835                 840                 845

Val Gly Thr Arg His Arg Leu Val Ala Ala Thr Val Thr Arg Gln Asp
    850                 855                 860

Glu Gly Thr Tyr Ser Cys Arg Val Gly Glu Asp Ser Val Asp Phe Arg
865                 870                 875                 880

Leu Arg Val Ser Glu Pro Lys Val Val Phe Ala Lys Glu Gln Leu Ala

                885                 890                 895

Arg Arg Lys Leu Gln Ala Glu Ala Gly Ala Ser Ala Thr Leu Ser Cys
            900                 905                 910

Glu Val Ala Gln Ala Gln Thr Glu Val Thr Trp Tyr Lys Asp Gly Lys
        915                 920                 925

Lys Leu Ser Ser Ser Ser Lys Val Cys Met Glu Ala Thr Gly Cys Thr
    930                 935                 940

Arg Arg Leu Val Val Gln Gln Ala Gly Gln Ala Asp Ala Gly Glu Tyr
945                 950                 955                 960

Ser Cys Glu Ala Gly Gly Gln Arg Leu Ser Phe His Leu Asp Val Lys
                965                 970                 975

Glu Pro Lys Val Val Phe Ala Lys Asp Gln Val Ala His Ser Glu Val
            980                 985                 990

Gln Ala Glu Ala Gly Ala Asn Ala Thr Leu Ser Cys Glu Val Ala Gln
        995                 1000                1005

Ala Gln Ala Glu Val Met Trp Tyr Lys Asp Gly Lys Lys Leu Ser Ser
    1010                1015                1020

Ser Leu Lys Val His Val Glu Ala Lys Gly Cys Arg Arg Arg Leu Val
1025                1030                1035                1040

Val Gln Gln Ala Gly Lys Thr Asp Ala Gly Asp Tyr Ser Cys Glu Ala
                1045                1050                1055

Arg Gly Gln Arg Val Ser Phe Arg Leu His Ile Thr Glu Pro Lys Met
            1060                1065                1070

Met Phe Ala Lys Glu Gln Ser Val His Asn Glu Val Gln Ala Glu Ala
        1075                1080                1085

Gly Ala Ser Ala Met Leu Ser Cys Glu Val Ala Gln Ala Gln Thr Glu
    1090                1095                1100

Val Thr Trp Tyr Lys Asp Gly Lys Lys Leu Ser Ser Ser Ser Lys Val
1105                1110                1115                1120

Gly Met Glu Val Lys Gly Cys Thr Arg Arg Leu Val Leu Pro Gln Ala
                1125                1130                1135

Gly Lys Ala Asp Ala Gly Glu Tyr Ser Cys Glu Ala Gly Gly Gln Arg
            1140                1145                1150

Val Ser Phe His Leu His Ile Thr Glu Pro Lys Gly Val Phe Ala Lys
        1155                1160                1165

Glu Gln Ser Val His Asn Glu Val Gln Ala Glu Ala Gly Thr Thr Ala
    1170                1175                1180

Met Leu Ser Cys Glu Val Ala Gln Pro Gln Thr Glu Val Thr Trp Tyr
1185                1190                1195                1200

Lys Asp Gly Lys Lys Leu Ser Ser Ser Ser Lys Val Arg Met Glu Val
                1205                1210                1215

Lys Gly Cys Thr Arg Arg Leu Val Val Gln Gln Val Gly Lys Ala Asp
            1220                1225                1230

Ala Gly Glu Tyr Ser Cys Glu Ala Gly Gly Gln Arg Val Ser Phe Gln
        1235                1240                1245

Leu His Ile Thr Glu Pro Lys Ala Val Phe Ala Lys Glu Gln Leu Val

1250                    1255                    1260

His Asn Glu Val Arg Thr Glu Ala Gly Ala Ser Ala Thr Leu Ser Cys
1265                    1270                    1275                    1280

Glu Val Ala Gln Ala Gln Thr Glu Val Thr Trp Tyr Lys Asp Gly Lys
                1285                    1290                    1295

Lys Leu Ser Ser Ser Ser Lys Val Arg Ile Glu Ala Ala Gly Cys Met
            1300                    1305                    1310

Arg Gln Leu Val Val Gln Gln Ala Gly Gln Ala Asp Ala Gly Glu Tyr
        1315                    1320                    1325

Thr Cys Glu Ala Gly Gly Gln Arg Leu Ser Phe His Leu Asp Val Ser
    1330                    1335                    1340

Glu Pro Lys Ala Val Phe Ala Lys Glu Gln Leu Ala His Arg Lys Val
1345                    1350                    1355                    1360

Gln Ala Glu Ala Gly Ala Ile Ala Thr Leu Ser Cys Glu Val Ala Gln
                1365                    1370                    1375

Ala Gln Thr Glu Val Thr Trp Tyr Lys Asp Gly Lys Lys Leu Ser Ser
            1380                    1385                    1390

Ser Ser Lys Val Arg Met Glu Ala Val Gly Cys Thr Arg Arg Leu Val
        1395                    1400                    1405

Val Gln Gln Ala Cys Gln Ala Asp Thr Gly Glu Tyr Ser Cys Glu Ala
    1410                    1415                    1420

Gly Gly Gln Arg Leu Ser Phe Ser Leu Asp Val Ala Glu Pro Lys Val
1425                    1430                    1435                    1440

Val Phe Ala Lys Glu Gln Pro Val His Arg Glu Val Gln Ala Gln Ala
                1445                    1450                    1455

Gly Ala Ser Thr Thr Leu Ser Cys Glu Val Ala Gln Ala Gln Thr Glu
            1460                    1465                    1470

Val Met Trp Tyr Lys Asp Gly Lys Lys Leu Ser Phe Ser Ser Lys Val
        1475                    1480                    1485

Arg Met Glu Ala Val Gly Cys Thr Arg Arg Leu Val Val Gln Gln Ala
    1490                    1495                    1500

Gly Gln Ala Asp Ala Gly Glu Tyr Ser Cys Glu Ala Gly Ser Gln Arg
1505                    1510                    1515                    1520

Leu Ser Phe His Leu His Val Ala Glu Pro Lys Ala Val Phe Ala Lys
                1525                    1530                    1535

Glu Gln Pro Ala Ser Arg Glu Val Gln Ala Glu Ala Gly Thr Ser Ala
            1540                    1545                    1550

Thr Leu Ser Cys Glu Val Ala Gln Ala Gln Thr Glu Val Thr Trp Tyr
        1555                    1560                    1565

Lys Asp Gly Lys Lys Leu Ser Ser Ser Ser Lys Val Arg Met Glu Ala
    1570                    1575                    1580

Val Gly Cys Thr Arg Arg Leu Val Val Gln Glu Ala Gly Gln Ala Asp
1585                    1590                    1595                    1600

Ala Gly Glu Tyr Ser Cys Lys Ala Gly Asp Gln Arg Leu Ser Phe His
                1605                    1610                    1615

Leu His Val Ala Glu Pro Lys Val Val Phe Ala Lys Glu Gln Pro Ala

1620                1625                1630

His Arg Glu Val Gln Ala Glu Ala Gly Ala Ser Ala Thr Leu Ser Cys
        1635                1640                1645

Glu Val Ala Gln Ala Gln Thr Glu Val Thr Trp Tyr Lys Asp Gly Lys
    1650                1655                1660

Lys Leu Ser Ser Ser Ser Lys Val Arg Val Glu Ala Val Gly Cys Thr
1665                1670                1675                1680

Arg Arg Leu Val Val Gln Gln Ala Gly Gln Ala Asp Ala Gly Glu Tyr
                1685                1690                1695

Ser Cys Glu Ala Gly Gly Gln Arg Leu Ser Phe Arg Leu His Val Ala
            1700                1705                1710

Glu Leu Glu Pro Gln Ile Ser Glu Arg Pro Cys Arg Arg Glu Pro Leu
        1715                1720                1725

Val Val Lys Glu His Glu Asp Ile Ile Leu Thr Ala Thr Leu Ala Thr
    1730                1735                1740

Pro Ser Ala Ala Thr Val Thr Trp Leu Lys Asp Gly Val Glu Ile Arg
1745                1750                1755                1760

Arg Ser Lys Arg His Glu Thr Ala Ser Gln Gly Asp Thr His Thr Leu
                1765                1770                1775

Thr Val His Gly Ala Gln Val Leu Asp Ser Ala Ile Tyr Ser Cys Arg
            1780                1785                1790

Val Gly Ala Glu Gly Gln Asp Phe Pro Val Gln Val Glu Glu Val Ala
        1795                1800                1805

Ala Lys Phe Cys Arg Leu Leu Glu Pro Val Cys Gly Glu Leu Gly Gly
    1810                1815                1820

Thr Val Thr Leu Ala Cys Glu Leu Ser Pro Ala Cys Ala Glu Val Val
1825                1830                1835                1840

Trp Arg Cys Gly Asn Thr Gln Pro Arg Val Gly Lys Arg Phe Gln Met
                1845                1850                1855

Val Ala Glu Gly Pro Val Arg Ser Leu Thr Val Leu Gly Leu Arg Ala
            1860                1865                1870

Glu Asp Ala Gly Glu Tyr Val Cys Glu Ser Arg Asp Asp His Thr Ser
        1875                1880                1885

Ala Gln Leu Thr Val Ser Val Pro Arg Val Val Lys Phe Met Ser Gly
    1890                1895                1900

Leu Ser Thr Val Val Ala Glu Glu Gly Gly Glu Ala Thr Phe Gln Cys
1905                1910                1915                1920

Val Val Ser Pro Ser Asp Val Ala Val Val Trp Phe Arg Asp Gly Ala
                1925                1930                1935

Leu Leu Gln Pro Ser Glu Lys Phe Ala Ile Ser Gln Ser Gly Ala Ser
            1940                1945                1950

His Ser Leu Thr Ile Ser Asp Leu Val Leu Glu Asp Ala Gly Gln Ile
        1955                1960                1965

Thr Val Glu Ala Glu Gly Ala Ser Ser Ser Ala Ala Leu Arg Val Arg
    1970                1975                1980

Glu Ala Pro Val Leu Phe Lys Lys Lys Leu Glu Pro Gln Thr Val Glu

1985                1990                1995                2000

Glu Arg Ser Ser Val Thr Leu Glu Val Glu Leu Thr Arg Pro Trp Pro
                2005                2010                2015

Glu Leu Arg Trp Thr Arg Asn Ala Thr Ala Leu Ala Pro Gly Lys Asn
            2020                2025                2030

Val Glu Ile His Ala Glu Gly Ala Arg His Arg Leu Val Leu His Asn
        2035                2040                2045

Val Gly Phe Ala Asp Arg Gly Phe Phe Gly Cys Glu Thr Pro Asp Asp
    2050                2055                2060

Lys Thr Gln Ala Lys Leu Thr Val Glu Met Arg Gln Val Arg Leu Val
2065                2070                2075                2080

Arg Gly Leu Gln Ala Val Glu Ala Arg Glu Gln Gly Thr Ala Thr Met
                2085                2090                2095

Glu Val Gln Leu Ser His Ala Asp Val Asp Gly Ser Trp Thr Arg Asp
            2100                2105                2110

Gly Leu Arg Phe Gln Gln Gly Pro Thr Cys His Leu Ala Val Arg Gly
        2115                2120                2125

Pro Met His Thr Leu Thr Leu Ser Gly Leu Arg Pro Glu Asp Ser Gly
    2130                2135                2140

Leu Met Val Phe Lys Ala Glu Gly Val His Thr Ser Ala Arg Leu Val
2145                2150                2155                2160

Val Thr Glu Leu Pro Val Ser Phe Ser Arg Pro Leu Gln Asp Val Val
                2165                2170                2175

Thr Thr Glu Lys Glu Lys Val Thr Leu Glu Cys Glu Leu Ser Arg Pro
            2180                2185                2190

Asn Val Asp Val Arg Trp Leu Lys Asp Gly Val Glu Leu Arg Ala Gly
        2195                2200                2205

Lys Thr Met Ala Ile Ala Ala Gln Gly Ala Cys Arg Ser Leu Thr Ile
    2210                2215                2220

Tyr Arg Cys Glu Phe Ala Asp Gln Gly Val Tyr Val Cys Asp Ala His
2225                2230                2235                2240

Asp Ala Gln Ser Ser Ala Ser Val Lys Val Gln Gly Arg Thr Tyr Thr
                2245                2250                2255

Leu Ile Tyr Arg Arg Val Leu Ala Glu Asp Ala Gly Glu Ile Gln Phe
            2260                2265                2270

Val Ala Glu Asn Ala Glu Ser Arg Ala Gln Leu Arg Val Lys Glu Leu
        2275                2280                2285

Pro Val Thr Leu Val Arg Pro Leu Arg Asp Lys Ile Ala Met Glu Lys
    2290                2295                2300

His Arg Gly Val Leu Glu Cys Gln Val Ser Arg Ala Ser Ala Gln Val
2305                2310                2315                2320

Arg Trp Phe Lys Gly Ser Gln Glu Leu Gln Pro Gly Pro Lys Tyr Glu
                2325                2330                2335

Leu Val Ser Asp Gly Leu Tyr Arg Lys Leu Ile Ile Ser Asp Val His
            2340                2345                2350

Ala Glu Asp Glu Asp Thr Tyr Thr Cys Asp Ala Gly Asp Val Lys Thr

2355                2360                2365

Ser Ala Gln Phe Phe Val Glu Glu Gln Ser Ile Thr Ile Val Arg Gly
    2370                2375                2380

Leu Gln Asp Val Thr Val Met Glu Pro Ala Pro Ala Trp Phe Glu Cys
2385                2390                2395                2400

Glu Thr Ser Ile Pro Ser Val Arg Pro Pro Lys Trp Leu Leu Gly Lys
                2405                2410                2415

Thr Val Leu Gln Ala Gly Gly Asn Val Gly Leu Glu Gln Glu Gly Thr
            2420                2425                2430

Val His Arg Leu Met Leu Arg Arg Thr Cys Ser Thr Met Thr Gly Pro
        2435                2440                2445

Val His Phe Thr Val Gly Lys Ser Arg Ser Ser Ala Arg Leu Val Val
    2450                2455                2460

Ser Asp Ile Pro Val Val Leu Thr Arg Pro Leu Glu Pro Lys Thr Gly
2465                2470                2475                2480

Arg Glu Leu Gln Ser Val Val Leu Ser Cys Asp Phe Arg Pro Ala Pro
                2485                2490                2495

Lys Ala Val Gln Trp Tyr Lys Asp Asp Thr Pro Leu Ser Pro Ser Glu
            2500                2505                2510

Lys Phe Lys Met Ser Leu Glu Gly Gln Met Ala Glu Leu Arg Ile Leu
        2515                2520                2525

Arg Leu Met Pro Ala Asp Ala Gly Val Tyr Arg Cys Gln Ala Gly Ser
    2530                2535                2540

Ala His Ser Ser Thr Glu Val Thr Val Glu Ala Arg Glu Val Thr Val
2545                2550                2555                2560

Thr Gly Pro Leu Gln Asp Ala Glu Ala Thr Glu Glu Gly Trp Ala Ser
                2565                2570                2575

Phe Ser Cys Glu Leu Ser His Glu Asp Glu Glu Val Glu Trp Ser Leu
            2580                2585                2590

Asn Gly Met Pro Leu Tyr Asn Asp Ser Phe His Glu Ile Ser His Lys
        2595                2600                2605

Gly Arg Arg His Thr Leu Val Leu Lys Ser Ile Gln Arg Ala Asp Ala
    2610                2615                2620

Gly Ile Val Arg Ala Ser Ser Leu Lys Val Ser Thr Ser Ala Arg Leu
2625                2630                2635                2640

Glu Val Arg Val Lys Pro Val Val Phe Leu Lys Ala Leu Asp Asp Leu
                2645                2650                2655

Ser Ala Glu Glu Arg Gly Thr Leu Ala Leu Gln Cys Glu Val Ser Asp
            2660                2665                2670

Pro Glu Ala His Val Val Trp Arg Lys Asp Gly Val Gln Leu Gly Pro
        2675                2680                2685

Ser Asp Lys Tyr Asp Phe Leu His Thr Ala Gly Thr Arg Gly Leu Val
    2690                2695                2700

Val His Asp Val Ser Pro Glu Asp Ala Gly Leu Tyr Thr Cys His Val
2705                2710                2715                2720

Gly Ser Glu Glu Thr Arg Ala Arg Val Arg Val His Asp Leu His Val

2725                    2730                    2735

Gly Ile Thr Lys Arg Leu Lys Thr Met Glu Val Leu Glu Gly Glu Ser
            2740                    2745                    2750

Cys Ser Phe Glu Cys Val Leu Ser His Glu Ser Ala Ser Asp Pro Ala
        2755                    2760                    2765

Met Trp Thr Val Gly Gly Lys Thr Val Gly Ser Ser Ser Arg Phe Gln
    2770                    2775                    2780

Ala Thr Arg Gln Gly Arg Lys Tyr Ile Leu Val Val Arg Glu Ala Ala
2785                    2790                    2795                    2800

Pro Ser Asp Ala Gly Glu Val Val Phe Ser Val Arg Gly Leu Thr Ser
                2805                    2810                    2815

Lys Ala Ser Leu Ile Val Arg Glu Arg Pro Ala Ala Ile Ile Lys Pro
            2820                    2825                    2830

Leu Glu Asp Gln Trp Val Ala Pro Gly Glu Asp Val Glu Leu Arg Cys
        2835                    2840                    2845

Glu Leu Ser Arg Ala Gly Thr Pro Val His Trp Leu Lys Asp Arg Lys
    2850                    2855                    2860

Ala Ile Arg Lys Ser Gln Lys Tyr Asp Val Val Cys Glu Gly Thr Met
2865                    2870                    2875                    2880

Ala Met Leu Val Ile Arg Gly Ala Ser Leu Lys Asp Ala Gly Glu Tyr
                2885                    2890                    2895

Thr Cys Glu Val Glu Ala Ser Lys Ser Thr Ala Ser Leu His Val Glu
            2900                    2905                    2910

Glu Lys Ala Asn Cys Phe Thr Glu Glu Leu Thr Asn Leu Gln Val Glu
        2915                    2920                    2925

Glu Lys Gly Thr Ala Val Phe Thr Cys Lys Thr Glu His Pro Ala Ala
    2930                    2935                    2940

Thr Val Thr Trp Arg Lys Gly Leu Leu Glu Leu Arg Ala Ser Gly Lys
2945                    2950                    2955                    2960

His Gln Pro Ser Gln Glu Gly Leu Thr Leu Arg Leu Thr Ile Ser Ala
                2965                    2970                    2975

Leu Glu Lys Ala Asp Ser Asp Thr Tyr Thr Cys Asp Ile Gly Gln Ala
            2980                    2985                    2990

Gln Ser Arg Ala Gln Leu Leu Val Gln Gly Arg Arg Val His Ile Ile
        2995                    3000                    3005

Glu Asp Leu Glu Asp Val Asp Val Gln Glu Gly Ser Ser Ala Thr Phe
    3010                    3015                    3020

Arg Cys Arg Ile Ser Pro Ala Asn Tyr Glu Pro Val His Trp Phe Leu
3025                    3030                    3035                    3040

Asp Lys Thr Pro Leu His Ala Asn Glu Leu Asn Glu Ile Asp Ala Gln
                3045                    3050                    3055

Pro Gly Gly Tyr His Val Leu Thr Leu Arg Gln Leu Ala Leu Lys Asp
            3060                    3065                    3070

Ser Gly Thr Ile Tyr Phe Glu Ala Gly Asp Gln Arg Ala Ser Ala Ala
        3075                    3080                    3085

Leu Arg Val Thr Glu Lys Pro Ser Val Phe Ser Arg Glu Leu Thr Asp

3090                3095                3100

Ala Thr Ile Thr Glu Gly Glu Asp Leu Thr Leu Val Cys Glu Thr Ser
3105                3110                3115                3120

Thr Cys Asp Ile Pro Met Cys Trp Thr Lys Asp Gly Lys Thr Leu Arg
                3125                3130                3135

Gly Ser Ala Arg Cys Gln Leu Ser His Glu Gly His Arg Ala Gln Leu
            3140                3145                3150

Leu Ile Thr Gly Ala Thr Leu Gln Asp Ser Gly Arg Tyr Lys Cys Glu
        3155                3160                3165

Ala Gly Gly Ala Cys Ser Ser Ser Ile Val Arg Val His Ala Arg Pro
    3170                3175                3180

Val Arg Phe Gln Glu Ala Leu Lys Asp Leu Glu Val Leu Glu Gly Gly
3185                3190                3195                3200

Ala Ala Thr Leu Arg Cys Val Leu Ser Ser Val Ala Ala Pro Val Lys
                3205                3210                3215

Trp Cys Tyr Gly Asn Asn Val Leu Arg Pro Gly Asp Lys Tyr Ser Leu
            3220                3225                3230

Arg Gln Glu Gly Ala Met Leu Glu Leu Val Val Arg Asn Leu Arg Pro
        3235                3240                3245

Gln Asp Ser Gly Arg Tyr Ser Cys Ser Phe Gly Asp Gln Thr Thr Ser
    3250                3255                3260

Ala Thr Leu Thr Val Thr Ala Leu Pro Ala Gln Phe Ile Gly Lys Leu
3265                3270                3275                3280

Arg Asn Lys Glu Ala Thr Glu Gly Ala Thr Ala Thr Leu Arg Cys Glu
                3285                3290                3295

Leu Ser Lys Thr Ala Pro Val Glu Trp Arg Lys Gly Ser Glu Thr Leu
            3300                3305                3310

Arg Asp Gly Asp Arg Tyr Cys Leu Arg Gln Asp Gly Ala Met Cys Glu
        3315                3320                3325

Leu Gln Ile Arg Gly Leu Ala Met Val Asp Ala Ala Glu Tyr Ser Cys
    3330                3335                3340

Val Cys Gly Glu Glu Arg Thr Ser Ala Ser Leu Thr Ile Arg Pro Met
3345                3350                3355                3360

Pro Ala His Phe Ile Gly Arg Leu Arg His Gln Glu Ser Ile Glu Gly
                3365                3370                3375

Ala Thr Ala Thr Leu Arg Cys Glu Leu Ser Lys Ala Ala Pro Val Glu
            3380                3385                3390

Trp Arg Lys Gly Arg Glu Ser Leu Arg Asp Gly Asp Arg His Ser Leu
        3395                3400                3405

Arg Gln Asp Gly Ala Val Cys Glu Leu Gln Ile Cys Gly Leu Ala Val
    3410                3415                3420

Ala Asp Ala Gly Glu Tyr Ser Cys Val Cys Gly Glu Glu Arg Thr Ser
3425                3430                3435                3440

Ala Thr Leu Thr Val Lys Ala Leu Pro Ala Lys Phe Thr Glu Gly Leu
                3445                3450                3455

Arg Asn Glu Glu Ala Val Glu Gly Ala Thr Ala Met Leu Trp Cys Glu

3460                    3465                    3470

Leu Ser Lys Val Ala Pro Val Glu Trp Arg Lys Gly Pro Glu Asn Leu
        3475                    3480                    3485

Arg Asp Gly Asp Arg Tyr Ile Leu Arg Gln Glu Gly Thr Arg Cys Glu
    3490                    3495                    3500

Leu Gln Ile Cys Gly Leu Ala Met Ala Asp Ala Gly Glu Tyr Leu Cys
3505                    3510                    3515                    3520

Val Cys Gly Gln Glu Arg Thr Ser Ala Thr Leu Thr Ile Arg Ala Leu
                3525                    3530                    3535

Pro Ala Arg Phe Ile Glu Asp Val Lys Asn Gln Glu Ala Arg Glu Gly
            3540                    3545                    3550

Ala Thr Ala Val Leu Gln Cys Glu Leu Asn Ser Ala Ala Pro Val Glu
        3555                    3560                    3565

Trp Arg Lys Gly Ser Glu Thr Leu Arg Asp Gly Asp Arg Tyr Ser Leu
    3570                    3575                    3580

Arg Gln Asp Gly Thr Lys Cys Glu Leu Gln Ile Arg Gly Leu Ala Met
3585                    3590                    3595                    3600

Ala Asp Thr Gly Glu Tyr Ser Cys Val Cys Gly Gln Glu Arg Thr Ser
                3605                    3610                    3615

Ala Met Leu Thr Val Arg Ala Leu Pro Ile Lys Phe Thr Glu Gly Leu
            3620                    3625                    3630

Arg Asn Glu Glu Ala Thr Glu Gly Ala Thr Ala Val Leu Arg Cys Glu
        3635                    3640                    3645

Leu Ser Lys Met Ala Pro Val Glu Trp Trp Lys Gly His Glu Thr Leu
    3650                    3655                    3660

Arg Asp Gly Asp Arg His Ser Leu Arg Gln Asp Gly Ala Arg Cys Glu
3665                    3670                    3675                    3680

Leu Gln Ile Arg Gly Leu Val Ala Glu Asp Ala Gly Glu Tyr Leu Cys
                3685                    3690                    3695

Met Cys Gly Lys Glu Arg Thr Ser Ala Met Leu Thr Val Arg Ala Met
            3700                    3705                    3710

Pro Ser Lys Phe Ile Glu Gly Leu Arg Asn Glu Glu Ala Thr Glu Gly
        3715                    3720                    3725

Asp Thr Ala Thr Leu Trp Cys Glu Leu Ser Lys Ala Ala Pro Val Glu
    3730                    3735                    3740

Trp Arg Lys Gly His Glu Thr Leu Arg Asp Gly Asp Arg His Ser Leu
3745                    3750                    3755                    3760

Arg Gln Asp Gly Ser Arg Cys Glu Leu Gln Ile Arg Gly Leu Ala Val
                3765                    3770                    3775

Val Asp Ala Gly Glu Tyr Ser Cys Val Cys Gly Gln Glu Arg Thr Ser
            3780                    3785                    3790

Ala Thr Leu Thr Val Arg Ala Leu Pro Ala Arg Phe Ile Glu Asp Val
        3795                    3800                    3805

Lys Asn Gln Glu Ala Arg Glu Gly Ala Thr Ala Val Leu Gln Cys Glu
    3810                    3815                    3820

Leu Ser Lys Ala Ala Pro Val Glu Trp Arg Lys Gly Ser Glu Thr Leu

3825                3830                3835                3840

Arg Gly Gly Asp Arg Tyr Ser Leu Arg Gln Asp Gly Thr Arg Cys Glu
                3845                3850                3855

Leu Gln Ile His Gly Leu Ser Val Ala Asp Thr Gly Glu Tyr Ser Cys
            3860                3865                3870

Val Cys Gly Gln Glu Arg Thr Ser Ala Thr Leu Thr Val Arg Ala Pro
        3875                3880                3885

Gln Pro Val Phe Arg Glu Pro Leu Gln Ser Leu Gln Ala Glu Glu Gly
    3890                3895                3900

Ser Thr Ala Thr Leu Gln Cys Glu Leu Ser Glu Pro Thr Ala Thr Val
3905                3910                3915                3920

Val Trp Ser Lys Gly Gly Leu Gln Leu Gln Ala Asn Gly Arg Arg Glu
                3925                3930                3935

Pro Arg Leu Gln Gly Cys Thr Ala Glu Leu Val Leu Gln Asp Leu Gln
            3940                3945                3950

Arg Glu Asp Thr Gly Glu Tyr Thr Cys Thr Cys Gly Ser Gln Ala Thr
        3955                3960                3965

Ser Ala Thr Leu Thr Val Thr Ala Ala Pro Val Arg Phe Leu Arg Glu
    3970                3975                3980

Leu Gln His Gln Glu Val Asp Glu Gly Gly Thr Ala His Leu Cys Cys
3985                3990                3995                4000

Glu Leu Ser Arg Ala Gly Ala Ser Val Glu Trp Arg Lys Gly Ser Leu
                4005                4010                4015

Gln Leu Phe Pro Cys Ala Lys Tyr Gln Met Val Gln Asp Gly Ala Ala
            4020                4025                4030

Ala Glu Leu Leu Val Arg Gly Val Glu Gln Glu Asp Ala Gly Asp Tyr
        4035                4040                4045

Thr Cys Asp Thr Gly His Thr Gln Ser Met Ala Ser Leu Ser Val Arg
    4050                4055                4060

Val Pro Arg Pro Lys Phe Lys Thr Arg Leu Gln Ser Leu Glu Gln Glu
4065                4070                4075                4080

Thr Gly Asp Ile Ala Arg Leu Cys Cys Gln Leu Ser Asp Ala Glu Ser
                4085                4090                4095

Gly Ala Val Val Gln Trp Leu Lys Glu Gly Val Glu Leu His Ala Gly
            4100                4105                4110

Pro Lys Tyr Glu Met Arg Ser Gln Gly Ala Thr Arg Glu Leu Leu Ile
        4115                4120                4125

His Gln Leu Glu Ala Lys Asp Thr Gly Glu Tyr Ala Cys Val Thr Gly
    4130                4135                4140

Gly Gln Lys Thr Ala Ala Ser Leu Arg Val Thr Glu Pro Glu Val Thr
4145                4150                4155                4160

Ile Val Arg Gly Leu Val Asp Ala Glu Val Thr Ala Asp Glu Asp Val
                4165                4170                4175

Glu Phe Ser Cys Glu Val Ser Arg Ala Gly Ala Thr Gly Val Gln Trp
            4180                4185                4190

Cys Leu Gln Gly Leu Pro Leu Gln Ser Asn Glu Val Thr Glu Val Ala

4195                4200                4205

Val Arg Asp Gly Arg Ile His Thr Leu Arg Leu Lys Gly Val Thr Pro
    4210                4215                4220

Glu Asp Ala Gly Thr Val Ser Phe His Leu Gly Asn His Ala Ser Ser
4225                4230                4235                4240

Ala Gln Leu Thr Val Arg Ala Pro Glu Val Thr Ile Leu Glu Pro Leu
                4245                4250                4255

Gln Asp Val Gln Leu Ser Glu Gly Gln Asp Ala Ser Phe Gln Cys Arg
            4260                4265                4270

Leu Ser Arg Ala Ser Gly Gln Glu Ala Arg Trp Ala Leu Gly Gly Val
        4275                4280                4285

Pro Leu Gln Ala Asn Glu Met Asn Asp Ile Thr Val Glu Gln Gly Thr
    4290                4295                4300

Leu His Leu Leu Thr Leu His Lys Val Thr Leu Glu Asp Ala Gly Thr
4305                4310                4315                4320

Val Ser Phe His Val Gly Thr Cys Ser Ser Glu Ala Gln Leu Lys Val
                4325                4330                4335

Thr Ala Lys Asn Thr Val Val Arg Gly Leu Glu Asn Val Glu Ala Leu
            4340                4345                4350

Glu Gly Gly Glu Ala Leu Phe Glu Cys Gln Leu Ser Gln Pro Glu Val
        4355                4360                4365

Ala Ala His Thr Trp Leu Leu Asp Asp Glu Pro Val Arg Thr Ser Glu
    4370                4375                4380

Asn Ala Glu Val Val Phe Phe Glu Asn Gly Leu Arg His Leu Leu Leu
4385                4390                4395                4400

Leu Lys Asn Leu Arg Pro Gln Asp Ser Cys Arg Val Thr Phe Leu Ala
                4405                4410                4415

Gly Asp Met Val Thr Ser Ala Phe Leu Thr Val Arg Gly Trp Arg Leu
            4420                4425                4430

Glu Ile Leu Glu Pro Leu Lys Asn Ala Ala Val Arg Ala Gly Ala Gln
        4435                4440                4445

Ala Arg Phe Thr Cys Thr Leu Ser Glu Ala Val Pro Val Gly Glu Ala
    4450                4455                4460

Ser Trp Tyr Ile Asn Gly Ala Ala Val Gln Pro Asp Asp Ser Asp Trp
4465                4470                4475                4480

Thr Val Thr Ala Asp Gly Ser His Gln Ala Leu Leu Leu Arg Ser Ala
                4485                4490                4495

Gln Pro His His Ala Gly Glu Val Thr Phe Ala Cys Arg Asp Ala Val
            4500                4505                4510

Ala Ser Ala Arg Leu Thr Val Leu Gly Leu Pro Asp Pro Pro Glu Asp
        4515                4520                4525

Ala Glu Val Val Ala His Ser Ser His Thr Val Thr Leu Ser Trp Ala
    4530                4535                4540

Ala Pro Met Ser Asp Gly Gly Gly Gly Leu Cys Gly Tyr Arg Val Glu
4545                4550                4555                4560

Val Lys Glu Gly Ala Thr Gly Gln Trp Arg Leu Cys His Glu Leu Val

4565                    4570                    4575

Pro Gly Pro Glu Cys Val Val Asp Gly Leu Ala Pro Gly Glu Thr Tyr
            4580                    4585                    4590

Arg Phe Arg Val Ala Ala Val Gly Pro Val Gly Ala Gly Glu Pro Val
        4595                    4600                    4605

His Leu Pro Gln Thr Val Arg Leu Ala Glu Pro Pro Lys Pro Val Pro
    4610                    4615                    4620

Pro Gln Pro Ser Ala Pro Glu Ser Arg Gln Val Ala Ala Gly Glu Asp
4625                    4630                    4635                    4640

Val Ser Leu Glu Leu Glu Val Val Ala Glu Ala Gly Glu Val Ile Trp
                4645                    4650                    4655

His Lys Gly Met Glu Arg Ile Gln Pro Gly Gly Arg Phe Glu Val Val
            4660                    4665                    4670

Ser Gln Gly Arg Gln Gln Met Leu Val Ile Lys Gly Phe Thr Ala Glu
        4675                    4680                    4685

Asp Gln Gly Glu Tyr His Cys Gly Leu Ala Gln Gly Ser Ile Cys Pro
    4690                    4695                    4700

Ala Ala Ala Thr Phe Gln Val Ala Leu Ser Pro Ala Ser Val Asp Glu
4705                    4710                    4715                    4720

Ala Pro Gln Pro Ser Leu Pro Pro Glu Ala Ala Gln Glu Gly Asp Leu
                4725                    4730                    4735

His Leu Leu Trp Glu Ala Leu Ala Arg Lys Arg Arg Met Ser Arg Glu
            4740                    4745                    4750

Pro Thr Leu Asp Ser Ile Ser Glu Leu Pro Glu Glu Asp Gly Arg Ser
        4755                    4760                    4765

Gln Arg Leu Pro Gln Glu Ala Glu Glu Val Ala Pro Asp Leu Ser Glu
    4770                    4775                    4780

Gly Tyr Ser Thr Ala Asp Glu Leu Ala Arg Thr Gly Asp Ala Asp Leu
4785                    4790                    4795                    4800

Ser His Thr Ser Ser Asp Asp Glu Ser Arg Ala Gly Thr Pro Ser Leu
                4805                    4810                    4815

Val Thr Tyr Leu Lys Lys Ala Gly Arg Pro Gly Thr Ser Pro Leu Ala
            4820                    4825                    4830

Ser Lys Val Gly Ala Pro Ala Ala Pro Ser Val Lys Pro Gln Gln Gln
        4835                    4840                    4845

Gln Glu Pro Leu Ala Ala Val Arg Pro Pro Leu Gly Asp Leu Ser Thr
    4850                    4855                    4860

Lys Asp Leu Gly Asp Pro Ser Met Asp Lys Ala Ala Val Lys Ile Gln
4865                    4870                    4875                    4880

Ala Ala Phe Lys Gly Tyr Lys Val Arg Lys Glu Met Lys Gln Gln Glu
                4885                    4890                    4895

Gly Pro Met Phe Ser His Thr Phe Gly Asp Thr Glu Ala Gln Val Gly
            4900                    4905                    4910

Asp Ala Leu Arg Leu Glu Cys Val Val Ala Ser Lys Ala Asp Val Arg
        4915                    4920                    4925

Ala Arg Trp Leu Lys Asp Gly Val Glu Leu Thr Asp Gly Arg His His

4930                    4935                    4940

His Ile Asp Gln Leu Gly Asp Gly Thr Cys Ser Leu Leu Ile Ala Gly
4945                4950                4955                4960

Leu Asp Arg Ala Asp Ala Gly Cys Tyr Thr Cys Gln Val Ser Asn Lys
                4965                4970                4975

Phe Gly Gln Val Thr His Ser Ala Cys Val Val Val Ser Gly Ser Glu
            4980                4985                4990

Ser Glu Ala Glu Ser Ser Ser Gly Gly Glu Leu Asp Asp Ala Phe Arg
        4995                5000                5005

Arg Ala Ala Arg Arg Leu His Arg Leu Phe Arg Thr Lys Ser Pro Ala
    5010                5015                5020

Glu Val Ser Asp Glu Glu Leu Phe Leu Ser Ala Asp Glu Gly Pro Ala
5025                5030                5035                5040

Glu Pro Glu Glu Pro Ala Asp Trp Gln Thr Tyr Arg Glu Asp Glu His
                5045                5050                5055

Phe Ile Cys Ile Arg Phe Glu Ala Leu Thr Glu Ala Arg Gln Ala Val
            5060                5065                5070

Thr Arg Phe Gln Glu Met Phe Ala Thr Leu Gly Ile Gly Val Glu Ile
        5075                5080                5085

Lys Leu Val Glu Gln Gly Pro Arg Arg Val Glu Met Cys Ile Ser Lys
    5090                5095                5100

Glu Thr Pro Ala Pro Val Val Pro Pro Glu Pro Leu Pro Ser Leu Leu
5105                5110                5115                5120

Thr Ser Asp Ala Ala Pro Val Phe Leu Thr Glu Leu Gln Asn Gln Glu
                5125                5130                5135

Val Gln Asp Gly Tyr Pro Val Ser Phe Asp Cys Val Val Thr Gly Gln
            5140                5145                5150

Pro Met Pro Ser Val Arg Trp Phe Lys Asp Gly Lys Leu Leu Glu Glu
        5155                5160                5165

Asp Asp His Tyr Met Ile Asn Glu Asp Gln Gln Gly Gly His Gln Leu
    5170                5175                5180

Ile Ile Thr Ala Val Val Pro Ala Asp Met Gly Val Tyr Arg Cys Leu
5185                5190                5195                5200

Ala Glu Asn Ser Met Gly Val Ser Ser Thr Lys Ala Glu Leu Arg Val
                5205                5210                5215

Asp Leu Thr Ser Thr Asp Tyr Asp Thr Ala Ala Asp Ala Thr Glu Ser
            5220                5225                5230

Ser Ser Tyr Phe Ser Ala Gln Gly Tyr Leu Ser Ser Arg Glu Gln Glu
        5235                5240                5245

Gly Thr Glu Ser Thr Thr Asp Glu Gly Gln Leu Pro Gln Val Val Glu
    5250                5255                5260

Glu Leu Arg Asp Leu Gln Val Ala Pro Gly Thr Arg Leu Ala Lys Phe
5265                5270                5275                5280

Gln Leu Lys Val Lys Gly Tyr Pro Ala Pro Arg Leu Tyr Trp Phe Lys
                5285                5290                5295

Asp Gly Gln Pro Leu Thr Ala Ser Ala His Ile Arg Met Thr Asp Lys

5300                5305                5310

Lys Ile Leu His Thr Leu Glu Ile Ile Ser Val Thr Arg Glu Asp Ser
        5315                5320                5325

Gly Gln Tyr Ala Ala Tyr Ile Ser Asn Ala Met Gly Ala Ala Tyr Ser
    5330                5335                5340

Ser Ala Arg Leu Leu Val Arg Gly Pro Asp Glu Pro Glu Glu Lys Pro
5345                5350                5355                5360

Ala Ser Asp Val His Glu Gln Leu Val Pro Pro Arg Met Leu Glu Arg
                5365                5370                5375

Phe Thr Pro Lys Lys Val Lys Lys Gly Ser Ser Ile Thr Phe Ser Val
            5380                5385                5390

Lys Val Glu Gly Arg Pro Val Pro Thr Val His Trp Leu Arg Glu Glu
        5395                5400                5405

Ala Glu Arg Gly Val Leu Trp Ile Gly Pro Asp Thr Pro Gly Tyr Thr
    5410                5415                5420

Val Ala Ser Ser Ala Gln Gln His Ser Leu Val Leu Leu Asp Val Gly
5425                5430                5435                5440

Arg Gln His Gln Gly Thr Tyr Thr Cys Ile Ala Ser Asn Ala Ala Gly
                5445                5450                5455

Gln Ala Leu Cys Ser Ala Ser Leu His Val Ser Gly Leu Pro Lys Val
            5460                5465                5470

Glu Glu Gln Glu Lys Val Lys Glu Ala Leu Ile Ser Thr Phe Leu Gln
        5475                5480                5485

Gly Thr Thr Gln Ala Ile Ser Ala Gln Gly Phe Gln Thr Ala Ser Phe
    5490                5495                5500

Ala Asp Leu Gly Gly Gln Arg Lys Glu Glu Pro Leu Ala Ala Lys Glu
5505                5510                5515                5520

Ala Leu Gly His Leu Ser Leu Ala Glu Val Gly Thr Glu Glu Phe Leu
                5525                5530                5535

Gln Lys Leu Thr Ser Gln Ile Thr Glu Met Val Ser Ala Lys Ile Thr
            5540                5545                5550

Gln Ala Lys Leu Gln Val Pro Gly Gly Asp Ser Asp Glu Asp Ser Lys
        5555                5560                5565

Thr Pro Ser Ala Ser Pro Arg His Gly Arg Ser Arg Pro Ser Ser Ser
    5570                5575                5580

Ile Gln Glu Ser Ser Ser Glu Ser Glu Asp Gly Asp Ala Arg Gly Glu
5585                5590                5595                5600

Ile Phe Asp Ile Tyr Val Val Thr Ala Asp Tyr Leu Pro Leu Gly Ala
                5605                5610                5615

Glu Gln Asp Ala Ile Thr Leu Arg Glu Gly Gln Tyr Val Glu Val Leu
            5620                5625                5630

Asp Ala Ala His Pro Leu Arg Trp Leu Val Arg Thr Lys Pro Thr Lys
        5635                5640                5645

Ser Ser Pro Ser Arg Gln Gly Trp Val Ser Pro Ala Tyr Leu Asp Arg
    5650                5655                5660

Arg Leu Lys Leu Ser Pro Glu Trp Gly Ala Ala Glu Ala Pro Glu Phe

5665                5670                5675                5680

Pro Gly Glu Ala Val Ser Glu Asp Glu Tyr Lys Ala Arg Leu Ser Ser
                5685                5690                5695

Val Ile Gln Glu Leu Leu Ser Ser Glu Gln Ala Phe Val Glu Glu Leu
            5700                5705                5710

Gln Phe Leu Gln Ser His His Leu Gln His Leu Glu Arg Cys Pro His
        5715                5720                5725

Val Pro Ile Ala Val Ala Gly Gln Lys Ala Val Ile Phe Arg Asn Val
    5730                5735                5740

Arg Asp Ile Gly Arg Phe His Ser Ser Phe Leu Gln Glu Leu Gln Gln
5745                5750                5755                5760

Cys Asp Thr Asp Asp Asp Val Ala Met Cys Phe Ile Lys Asn Gln Ala
                5765                5770                5775

Ala Phe Glu Gln Tyr Leu Glu Phe Leu Val Gly Arg Val Gln Ala Glu
            5780                5785                5790

Ser Val Val Val Ser Thr Ala Ile Gln Glu Phe Tyr Lys Lys Tyr Ala
        5795                5800                5805

Glu Glu Ala Leu Leu Ala Gly Asp Pro Ser Gln Pro Pro Pro Pro Pro
    5810                5815                5820

Leu Gln His Tyr Leu Glu Gln Pro Val Glu Arg Val Gln Arg Tyr Gln
5825                5830                5835                5840

Ala Leu Leu Lys Glu Leu Ile Arg Asn Lys Ala Arg Asn Arg Gln Asn
                5845                5850                5855

Cys Ala Leu Leu Glu Gln Ala Tyr Ala Val Val Ser Ala Leu Pro Gln
            5860                5865                5870

Arg Ala Glu Asn Lys Leu His Val Ser Leu Met Glu Asn Tyr Pro Gly
        5875                5880                5885

Thr Leu Gln Ala Leu Gly Glu Pro Ile Arg Gln Gly His Phe Ile Val
    5890                5895                5900

Trp Glu Gly Ala Pro Gly Ala Arg Met Pro Trp Lys Gly His Asn Arg
5905                5910                5915                5920

His Val Phe Leu Phe Arg Asn His Leu Val Ile Cys Lys Pro Arg Arg
                5925                5930                5935

Asp Ser Arg Thr Asp Thr Val Ser Tyr Val Phe Arg Asn Met Met Lys
            5940                5945                5950

Leu Ser Ser Ile Asp Leu Asn Asp Gln Val Glu Gly Asp Asp Arg Ala
        5955                5960                5965

Phe Glu Val Trp Gln Glu Arg Glu Asp Ser Val Arg Lys Tyr Leu Leu
    5970                5975                5980

Gln Ala Arg Thr Ala Ile Ile Lys Ser Ser Trp Val Lys Glu Ile Cys
5985                5990                5995                6000

Gly Ile Gln Gln Arg Leu Ala Leu Pro Val Trp Arg Pro Pro Asp Phe
                6005                6010                6015

Glu Glu Glu Leu Ala Asp Cys Thr Ala Glu Leu Gly Glu Thr Val Lys
            6020                6025                6030

Leu Ala Cys Arg Val Thr Gly Thr Pro Lys Pro Val Ile Ser Trp Tyr

6035                6040                6045

Lys Asp Gly Lys Ala Val Gln Val Asp Pro His His Ile Leu Ile Glu
    6050                6055                6060

Asp Pro Asp Gly Ser Cys Ala Leu Ile Leu Asp Ser Leu Thr Gly Val
6065                6070                6075                6080

Asp Ser Gly Gln Tyr Met Cys Phe Ala Ala Ser Ala Ala Gly Asn Cys
                6085                6090                6095

Ser Thr Leu Gly Lys Ile Leu Val Gln Val Pro Pro Arg Phe Val Asn
            6100                6105                6110

Lys Val Leu Ala Ser Pro Phe Val Glu Gly Glu Asp Ala Gln Phe Thr
        6115                6120                6125

Cys Thr Ile Glu Gly Ala Pro Tyr Pro Gln Ile Arg Trp Tyr Lys Asp
    6130                6135                6140

Gly Ala Leu Leu Thr Thr Gly Asn Lys Phe Gln Thr Leu Ser Glu Pro
6145                6150                6155                6160

Arg Ser Gly Leu Leu Val Leu Val Ile Arg Ala Ala Ser Lys Glu Asp
                6165                6170                6175

Leu Gly Leu Tyr Glu Cys Glu Leu Val Asn Arg Leu Gly Ser Ala Arg
            6180                6185                6190

Ala Ser Ala Glu Leu Arg Ile Gln Ser Pro Met Leu Gln Ala Gln Glu
        6195                6200                6205

Gln Cys His Arg Glu Gln Leu Val Ala Ala Val Glu Val Thr Glu Gln
    6210                6215                6220

Glu Thr Lys Val Pro Lys Lys Thr Val Ile Ile Glu Glu Thr Ile Thr
6225                6230                6235                6240

Thr Val Val Lys Ser Pro Arg Gly Gln Arg Arg Ser Pro Ser Lys Ser
                6245                6250                6255

Pro Ser Arg Ser Pro Ser Arg Cys Ser Ala Ser Pro Leu Arg Pro Gly
            6260                6265                6270

Leu Leu Ala Pro Asp Leu Leu Tyr Leu Pro Gly Ala Gly Gln Pro Arg
        6275                6280                6285

Arg Pro Glu Ala Glu Pro Gly Gln Lys Pro Val Val Pro Thr Leu Tyr
    6290                6295                6300

Val Thr Glu Ala Glu Ala His Ser Pro Ala Leu Pro Gly Leu Ser Gly
6305                6310                6315                6320

Pro Gln Pro Lys Trp Val Glu Val Glu Glu Thr Ile Glu Val Arg Val
                6325                6330                6335

Lys Lys Met Gly Pro Gln Gly Val Ser Pro Thr Thr Glu Val Pro Arg
            6340                6345                6350

Ser Ser Ser Gly His Leu Phe Thr Leu Pro Gly Ala Thr Pro Gly Gly
        6355                6360                6365

Asp Pro Asn Ser Asn Asn Ser Asn Asn Lys Leu Leu Ala Gln Glu Ala
    6370                6375                6380

Trp Ala Gln Gly Thr Ala Met Val Gly Val Arg Glu Pro Leu Val Phe
6385                6390                6395                6400

Arg Val Asp Ala Arg Gly Ser Val Asp Trp Ala Ala Ser Gly Met Gly

6405                    6410                    6415

Ser Leu Glu Glu Glu Gly Thr Met Glu Glu Ala Gly Glu Glu Glu Gly
        6420                    6425                    6430

Glu Asp Gly Asp Ala Phe Val Thr Glu Glu Ser Gln Asp Thr His Ser
        6435                    6440                    6445

Leu Gly Asp Arg Asp Pro Lys Ile Leu Thr His Asn Gly Arg Met Leu
    6450                    6455                    6460

Thr Leu Ala Asp Leu Glu Asp Tyr Val Pro Gly Glu Gly Glu Thr Phe
6465                    6470                    6475                    6480

His Cys Gly Gly Pro Gly Pro Gly Ala Pro Asp Asp Pro Pro Cys Glu
                6485                    6490                    6495

Val Ser Val Ile Gln Arg Glu Ile Gly Glu Pro Thr Val Gly Gln Pro
            6500                    6505                    6510

Val Leu Leu Ser Val Gly His Ala Leu Gly Pro Arg Gly Pro Leu Gly
        6515                    6520                    6525

Leu Phe Arg Pro Glu Pro Arg Gly Ala Ser Pro Pro Gly Pro Gln Val
    6530                    6535                    6540

Arg Ser Leu Glu Gly Thr Ser Phe Leu Leu Arg Glu Ala Pro Ala Arg
6545                    6550                    6555                    6560

Pro Val Gly Ser Ala Pro Trp Thr Gln Ser Phe Cys Thr Arg Ile Arg
                6565                    6570                    6575

Arg Ser Ala Asp Ser Gly Gln Ser Ser Phe Thr Thr Glu Leu Ser Thr
            6580                    6585                    6590

Gln Thr Val Asn Phe Gly Thr Val Gly Glu Thr Val Thr Leu His Ile
        6595                    6600                    6605

Cys Pro Asp Arg Asp Gly Asp Glu Ala Ala Gln Pro
    6610                    6615                    6620


<210> 291
<211> 86
<212> PRT
<213> Homo sapiens

<400> 291
Pro Pro Ser Val Thr Val Lys Glu Gly Glu Ser Val Thr Leu Ser Cys
1               5                   10                  15

Glu Ala Ser Gly Asn Pro Pro Pro Thr Val Thr Trp Tyr Lys Gln Gly
            20                  25                  30

Gly Lys Leu Leu Ala Glu Ser Gly Arg Phe Ser Val Ser Arg Ser Gly
        35                  40                  45

Gly Asn Ser Thr Leu Thr Ile Ser Asn Val Thr Pro Glu Asp Ser Gly
    50                  55                  60

Thr Tyr Thr Cys Ala Ala Thr Asn Ser Ser Gly Ser Ala Ser Ser Gly
65                  70                  75                  80

Thr Thr Leu Thr Val Leu
                85


<210> 292
<211> 63

<212> PRT
<213> Homo sapiens

<400> 292
Leu Glu Gly Glu Ser Val Thr Leu Thr Cys Pro Ala Ser Gly Asp Pro
1               5                   10                  15

Val Pro Asn Ile Thr Trp Leu Lys Asp Gly Lys Pro Leu Pro Glu Ser
            20                  25                  30

Arg Val Val Ala Ser Gly Ser Thr Leu Thr Ile Lys Asn Val Ser Leu
        35                  40                  45

Glu Asp Ser Gly Leu Tyr Thr Cys Val Ala Arg Asn Ser Val Gly
    50                  55                  60


<210> 293
<211> 22
<212> DNA
<213> Artificial Sequence

<220>
<223> Description of Artificial Sequence: PCR Primer
      Sequence

<400> 293
gatggtacgt gatgctcttg tt                                          22


<210> 294
<211> 26
<212> DNA
<213> Artificial Sequence

<220>
<223> Description of Artificial Sequence: PCR Primer
      Sequence

<400> 294
cccttgccga taggaagttc tttgct                                      26


<210> 295
<211> 22
<212> DNA
<213> Artificial Sequence

<220>
<223> Description of Artificial Sequence: PCR Primer
      Sequence

<400> 295
aggaagctgc acatttgttc ta                                          22


<210> 296
<211> 22
<212> DNA
<213> Artificial Sequence

<220>
<223> Description of Artificial Sequence: PCR Primer
      Sequence

<400> 296
accagggatg tagccataaa ac                                          22

<210> 297
<211> 26
<212> DNA
<213> Artificial Sequence

<220>
<223> Description of Artificial Sequence: PCR Primer
      Sequence

<400> 297
tttcaccaag gatgaagcac ttccct                                          26


<210> 298
<211> 22
<212> DNA
<213> Artificial Sequence

<220>
<223> Description of Artificial Sequence: PCR Primer
      Sequence

<400> 298
gcatgtgttg gtttcttctc at                                              22


<210> 299
<211> 22
<212> DNA
<213> Artificial Sequence

<220>
<223> Description of Artificial Sequence: PCR Primer
      Sequence

<400> 299
accagggatg tagccataaa ac                                              22


<210> 300
<211> 26
<212> DNA
<213> Artificial Sequence

<220>
<223> Description of Artificial Sequence: PCR Primer
      Sequence

<400> 300
tttcaccaag gatgaagcac ttccct                                          26


<210> 301
<211> 22
<212> DNA
<213> Artificial Sequence

<220>
<223> Description of Artificial Sequence: PCR Primer
      Sequence

<400> 301
gcatgtgttg gtttcttctc at                                              22


<210> 302
<211> 22
<212> DNA
<213> Artificial Sequence

<220>
<223> Description of Artificial Sequence: PCR Primer
      Sequence

<400> 302
gggtgtttag gattccctgt ag                                                22


<210> 303
<211> 26
<212> DNA
<213> Artificial Sequence

<220>
<223> Description of Artificial Sequence: PCR Primer
      Sequence

<400> 303
ccaattgaaa gagtctccag gcatca                                            26


<210> 304
<211> 22
<212> DNA
<213> Artificial Sequence

<220>
<223> Description of Artificial Sequence: PCR Primer
      Sequence

<400> 304
tgcagttgat tcagaacatg ag                                                22


<210> 305
<211> 22
<212> DNA
<213> Artificial Sequence

<220>
<223> Description of Artificial Sequence: PCR Primer
      Sequence

<400> 305
ttgctctcat gaggtcagag at                                                22


<210> 306
<211> 30
<212> DNA
<213> Artificial Sequence

<220>
<223> Description of Artificial Sequence: PCR Primer
      Sequence

<400> 306
ccataggatg agtcttctgt tcttaaaccg                                        30


<210> 307
<211> 22
<212> DNA
<213> Artificial Sequence

<220>
<223> Description of Artificial Sequence: PCR Primer
      Sequence

<400> 307
ccgacagcct tatccttaca ct                                              22


<210> 308
<211> 20
<212> DNA
<213> Artificial Sequence

<220>
<223> Description of Artificial Sequence: PCR Primer
      Sequence

<400> 308
actgggaaca ggcactgtct                                                 20


<210> 309
<211> 23
<212> DNA
<213> Artificial Sequence

<220>
<223> Description of Artificial Sequence: PCR Primer
      Sequence

<400> 309
cagcagaact gtccagccca tgg                                             23


<210> 310
<211> 22
<212> DNA
<213> Artificial Sequence

<220>
<223> Description of Artificial Sequence: PCR Primer
      Sequence

<400> 310
gggtctgtca gagcatcagt ac                                              22


<210> 311
<211> 22
<212> DNA
<213> Artificial Sequence

<220>
<223> Description of Artificial Sequence: PCR Primer
      Sequence

<400> 311
tgcaatttgg agctaatatc ca                                              22


<210> 312
<211> 26
<212> DNA
<213> Artificial Sequence

<220>
<223> Description of Artificial Sequence: PCR Primer
      Sequence

<400> 312
taaccctcaa cctgcccttg taccag                                          26

<210> 313
<211> 22
<212> DNA
<213> Artificial Sequence

<220>
<223> Description of Artificial Sequence: PCR Primer
      Sequence

<400> 313
cgatggagta cagcagctta ac                                                22

<210> 314
<211> 22
<212> DNA
<213> Artificial Sequence

<220>
<223> Description of Artificial Sequence: PCR Primer
      Sequence

<400> 314
ggaaaacaaa atgaaggatc ct                                                22

<210> 315
<211> 26
<212> DNA
<213> Artificial Sequence

<220>
<223> Description of Artificial Sequence: PCR Primer
      Sequence

<400> 315
cggaagttcc cactcatcct gtacct                                            26

<210> 316
<211> 22
<212> DNA
<213> Artificial Sequence

<220>
<223> Description of Artificial Sequence: PCR Primer
      Sequence

<400> 316
atgtagagga tggtgacgat ga                                                22

<210> 317
<211> 19
<212> DNA
<213> Artificial Sequence

<220>
<223> Description of Artificial Sequence: PCR Primer
      Sequence

<400> 317
cagaccaagg tcttccaca                                                    19

<210> 318
<211> 27
<212> DNA

<213> Artificial Sequence

<220>
<223> Description of Artificial Sequence: PCR Primer
      Sequence

<400> 318
agagcaggtt cctctctgtc ttcagca                                          27


<210> 319
<211> 21
<212> DNA
<213> Artificial Sequence

<220>
<223> Description of Artificial Sequence: PCR Primer
      Sequence

<400> 319
actgttccat ttgagactcc a                                                21


<210> 320
<211> 19
<212> DNA
<213> Artificial Sequence

<220>
<223> Description of Artificial Sequence: PCR Primer
      Sequence

<400> 320
cagtctccag gccatgaag                                                   19


<210> 321
<211> 23
<212> DNA
<213> Artificial Sequence

<220>
<223> Description of Artificial Sequence: PCR Primer
      Sequence

<400> 321
accttgtccc tggtcctgct ggt                                              23


<210> 322
<211> 20
<212> DNA
<213> Artificial Sequence

<220>
<223> Description of Artificial Sequence: PCR Primer
      Sequence

<400> 322
agaccctgag ctctctccat                                                  20


<210> 323
<211> 22
<212> DNA
<213> Artificial Sequence

<220>
<223> Description of Artificial Sequence: PCR Primer

Sequence

<400> 323
gtgttgctag gaagcatgtt ct                                                22

<210> 324
<211> 25
<212> DNA
<213> Artificial Sequence

<220>
<223> Description of Artificial Sequence: PCR Primer
      Sequence

<400> 324
acacgtgcag tagctgcacc tgctt                                             25

<210> 325
<211> 21
<212> DNA
<213> Artificial Sequence

<220>
<223> Description of Artificial Sequence: PCR Primer
      Sequence

<400> 325
atgacacatg cgacgtatga g                                                 21

<210> 326
<211> 22
<212> DNA
<213> Artificial Sequence

<220>
<223> Description of Artificial Sequence: PCR Primer
      Sequence

<400> 326
ctttgtctac accgccctta at                                                22

<210> 327
<211> 27
<212> DNA
<213> Artificial Sequence

<220>
<223> Description of Artificial Sequence: PCR Primer
      Sequence

<400> 327
ccaaccatag attacggctt tcagagg                                           27

<210> 328
<211> 22
<212> DNA
<213> Artificial Sequence

<220>
<223> Description of Artificial Sequence: PCR Primer
      Sequence

<400> 328
gatgtcttgg gatcactttc tg                                                22

<210> 329
<211> 22
<212> DNA
<213> Artificial Sequence

<220>
<223> Description of Artificial Sequence: PCR Primer
      Sequence

<400> 329
attcttacac tgctccgagt ca                                          22


<210> 330
<211> 26
<212> DNA
<213> Artificial Sequence

<220>
<223> Description of Artificial Sequence: PCR Primer
      Sequence

<400> 330
tcatggttac aaattatcga agccaa                                      26


<210> 331
<211> 22
<212> DNA
<213> Artificial Sequence

<220>
<223> Description of Artificial Sequence: PCR Primer
      Sequence

<400> 331
ggtagggatt ggaggaatga ta                                          22


<210> 332
<211> 22
<212> DNA
<213> Artificial Sequence

<220>
<223> Description of Artificial Sequence: PCR Primer
      Sequence

<400> 332
gaggtaccca ctgtggtaca ag                                          22


<210> 333
<211> 26
<212> DNA
<213> Artificial Sequence

<220>
<223> Description of Artificial Sequence: PCR Primer
      Sequence

<400> 333
acatggtgtc cctgtggatc ctcttt                                      26


<210> 334
<211> 22

<212> DNA
<213> Artificial Sequence

<220>
<223> Description of Artificial Sequence: PCR Primer
      Sequence

<400> 334
gggagaggat gagtttgatg at                                              22


<210> 335
<211> 22
<212> DNA
<213> Artificial Sequence

<220>
<223> Description of Artificial Sequence: PCR Primer
      Sequence

<400> 335
ggctccttct tcttttctgt gt                                              22


<210> 336
<211> 26
<212> DNA
<213> Artificial Sequence

<220>
<223> Description of Artificial Sequence: PCR Primer
      Sequence

<400> 336
atcaccacca ttggctatgg caacct                                          26


<210> 337
<211> 21
<212> DNA
<213> Artificial Sequence

<220>
<223> Description of Artificial Sequence: PCR Primer
      Sequence

<400> 337
ggcaaagaag atgcagaaga g                                               21


<210> 338
<211> 21
<212> DNA
<213> Artificial Sequence

<220>
<223> Description of Artificial Sequence: PCR Primer
      Sequence

<400> 338
gctatgcagg tggtctgagt t                                               21


<210> 339
<211> 24
<212> DNA
<213> Artificial Sequence

<220>

<223> Description of Artificial Sequence: PCR Primer
      Sequence

<400> 339
ctcacaagcc ctggcctcag ctat                                          24


<210> 340
<211> 20
<212> DNA
<213> Artificial Sequence

<220>
<223> Description of Artificial Sequence: PCR Primer
      Sequence

<400> 340
agagccaaag ctggagctta                                               20


<210> 341
<211> 20
<212> DNA
<213> Artificial Sequence

<220>
<223> Description of Artificial Sequence: PCR Primer
      Sequence

<400> 341
actttggaag aggcattgct                                               20


<210> 342
<211> 26
<212> DNA
<213> Artificial Sequence

<220>
<223> Description of Artificial Sequence: PCR Primer
      Sequence

<400> 342
cagacagatt atgggctcca tcgctt                                        26


<210> 343
<211> 21
<212> DNA
<213> Artificial Sequence

<220>
<223> Description of Artificial Sequence: PCR Primer
      Sequence

<400> 343
ctcgggataa ccatgatcac t                                             21


<210> 344
<211> 22
<212> DNA
<213> Artificial Sequence

<220>
<223> Description of Artificial Sequence: PCR Primer
      Sequence

<400> 344

gggaccaatg ctcaaattac tt 22

<210> 345
<211> 27
<212> DNA
<213> Artificial Sequence

<220>
<223> Description of Artificial Sequence: PCR Primer
      Sequence

<400> 345
tcagaaagtt ccacaagcat ctaagga 27

<210> 346
<211> 22
<212> DNA
<213> Artificial Sequence

<220>
<223> Description of Artificial Sequence: PCR Primer
      Sequence

<400> 346
atgactccag tgttttcatc ca 22

<210> 347
<211> 19
<212> DNA
<213> Artificial Sequence

<220>
<223> Description of Artificial Sequence: PCR Primer
      Sequence

<400> 347
agacttgggg accaatgct 19

<210> 348
<211> 27
<212> DNA
<213> Artificial Sequence

<220>
<223> Description of Artificial Sequence: PCR Primer
      Sequence

<400> 348
tcagaaagtt ccacaagcat ctaagga 27

<210> 349
<211> 21
<212> DNA
<213> Artificial Sequence

<220>
<223> Description of Artificial Sequence: PCR Primer
      Sequence

<400> 349
tgactccagt gttttcatcc a 21

<210> 350

<211> 22
<212> DNA
<213> Artificial Sequence

<220>
<223> Description of Artificial Sequence: PCR Primer
      Sequence

<400> 350
cttgtgctag tgatcgacat ca                                                22

<210> 351
<211> 27
<212> DNA
<213> Artificial Sequence

<220>
<223> Description of Artificial Sequence: PCR Primer
      Sequence

<400> 351
ccccttattc tcaatcctcc tactacg                                           27

<210> 352
<211> 22
<212> DNA
<213> Artificial Sequence

<220>
<223> Description of Artificial Sequence: PCR Primer
      Sequence

<400> 352
ccgttgtttt cgtttacgta aa                                                22

<210> 353
<211> 22
<212> DNA
<213> Artificial Sequence

<220>
<223> Description of Artificial Sequence: PCR Primer
      Sequence

<400> 353
ggagactgct ttgagtcagt tc                                                22

<210> 354
<211> 23
<212> DNA
<213> Artificial Sequence

<220>
<223> Description of Artificial Sequence: PCR Primer
      Sequence

<400> 354
tctgctccaa gtccggacct gtg                                               23

<210> 355
<211> 22
<212> DNA
<213> Artificial Sequence

<220>
<223> Description of Artificial Sequence: PCR Primer
      Sequence

<400> 355
aattataggc atagggcaac gt                                                22


<210> 356
<211> 22
<212> DNA
<213> Artificial Sequence

<220>
<223> Description of Artificial Sequence: PCR Primer
      Sequence

<400> 356
gcaaaatcgt cgtctctgtt ac                                                22


<210> 357
<211> 23
<212> DNA
<213> Artificial Sequence

<220>
<223> Description of Artificial Sequence: PCR Primer
      Sequence

<400> 357
ccctctgaaa gccaccagca gtg                                               23


<210> 358
<211> 22
<212> DNA
<213> Artificial Sequence

<220>
<223> Description of Artificial Sequence: PCR Primer
      Sequence

<400> 358
ccaagaggtt cacaaacact gt                                                22


<210> 359
<211> 22
<212> DNA
<213> Artificial Sequence

<220>
<223> Description of Artificial Sequence: PCR Primer
      Sequence

<400> 359
gcaaaatcgt cgtctctgtt ac                                                22


<210> 360
<211> 23
<212> DNA
<213> Artificial Sequence

<220>
<223> Description of Artificial Sequence: PCR Primer
      Sequence

<400> 360
ccctctgaaa gccaccagca gtg                                              23

<210> 361
<211> 22
<212> DNA
<213> Artificial Sequence

<220>
<223> Description of Artificial Sequence: PCR Primer
      Sequence

<400> 361
ccaagaggtt cacaaacact gt                                               22

<210> 362
<211> 20
<212> DNA
<213> Artificial Sequence

<220>
<223> Description of Artificial Sequence: PCR Primer
      Sequence

<400> 362
tcgttgtccg tgcagttcag                                                  20

<210> 363
<211> 22
<212> DNA
<213> Artificial Sequence

<220>
<223> Description of Artificial Sequence: PCR Primer
      Sequence

<400> 363
cagaccaccc ggaactcgcg tg                                               22

<210> 364
<211> 20
<212> DNA
<213> Artificial Sequence

<220>
<223> Description of Artificial Sequence: PCR Primer
      Sequence

<400> 364
cggccgtgta caatgtgtct                                                  20

<210> 365
<211> 22
<212> DNA
<213> Artificial Sequence

<220>
<223> Description of Artificial Sequence: PCR Primer
      Sequence

<400> 365
gaggtgtgac tcagacatcc tt                                               22

<210> 366
<211> 24
<212> DNA
<213> Artificial Sequence

<220>
<223> Description of Artificial Sequence: PCR Primer
      Sequence

<400> 366
aactggctcc tgctcaacca gcct                                              24


<210> 367
<211> 22
<212> DNA
<213> Artificial Sequence

<220>
<223> Description of Artificial Sequence: PCR Primer
      Sequence

<400> 367
gtacagtgtc cgtccctaac ct                                                22


<210> 368
<211> 22
<212> DNA
<213> Artificial Sequence

<220>
<223> Description of Artificial Sequence: PCR Primer
      Sequence

<400> 368
acatgagcca caatcagatc tc                                                22


<210> 369
<211> 23
<212> DNA
<213> Artificial Sequence

<220>
<223> Description of Artificial Sequence: PCR Primer
      Sequence

<400> 369
actttgtccc ctgccagctg cct                                               23


<210> 370
<211> 22
<212> DNA
<213> Artificial Sequence

<220>
<223> Description of Artificial Sequence: PCR Primer
      Sequence

<400> 370
attcctgaaa tccacacagc ta                                                22


<210> 371
<211> 20
<212> DNA
<213> Artificial Sequence

<220>
<223> Description of Artificial Sequence: PCR Primer
      Sequence

<400> 371
cagcaacgct ttcaagacat                                                    20


<210> 372
<211> 26
<212> DNA
<213> Artificial Sequence

<220>
<223> Description of Artificial Sequence: PCR Primer
      Sequence

<400> 372
ttctccattc agaacagcca gctggt                                             26


<210> 373
<211> 22
<212> DNA
<213> Artificial Sequence

<220>
<223> Description of Artificial Sequence: PCR Primer
      Sequence

<400> 373
catccttgag cttcttctgg ta                                                 22


<210> 374
<211> 22
<212> DNA
<213> Artificial Sequence

<220>
<223> Description of Artificial Sequence: PCR Primer
      Sequence

<400> 374
tccatcaaat tcgagtgaga ga                                                 22


<210> 375
<211> 29
<212> DNA
<213> Artificial Sequence

<220>
<223> Description of Artificial Sequence: PCR Primer
      Sequence

<400> 375
tcacagatgc agcatttctt acaacaaca                                          29


<210> 376
<211> 22
<212> DNA
<213> Artificial Sequence

<220>
<223> Description of Artificial Sequence: PCR Primer
      Sequence

<400> 376
gcttcttcca acttctgggt aa                                                22

<210> 377
<211> 21
<212> DNA
<213> Artificial Sequence

<220>
<223> Description of Artificial Sequence: PCR Primer
      Sequence

<400> 377
acccaagtgg catctattca g                                                 21

<210> 378
<211> 26
<212> DNA
<213> Artificial Sequence

<220>
<223> Description of Artificial Sequence: PCR Primer
      Sequence

<400> 378
tcagccgcaa ttttcctatt atcgga                                            26

<210> 379
<211> 22
<212> DNA
<213> Artificial Sequence

<220>
<223> Description of Artificial Sequence: PCR Primer
      Sequence

<400> 379
gtgaagttgc tcgaatgtct tc                                                22

<210> 380
<211> 21
<212> DNA
<213> Artificial Sequence

<220>
<223> Description of Artificial Sequence: PCR Primer
      Sequence

<400> 380
gactgaggag agcaccacag t                                                 21

<210> 381
<211> 25
<212> DNA
<213> Artificial Sequence

<220>
<223> Description of Artificial Sequence: PCR Primer
      Sequence

<400> 381
acactctgcc aaggtcagag ctgct                                             25

<210> 382
<211> 21
<212> DNA
<213> Artificial Sequence

<220>
<223> Description of Artificial Sequence: PCR Primer
      Sequence

<400> 382
gtctcagctc cgttgtcatc t                                                21

<210> 383
<211> 22
<212> DNA
<213> Artificial Sequence

<220>
<223> Description of Artificial Sequence: PCR Primer
      Sequence

<400> 383
catcttccgt cagattgaca gt                                               22

<210> 384
<211> 26
<212> DNA
<213> Artificial Sequence

<220>
<223> Description of Artificial Sequence: PCR Primer
      Sequence

<400> 384
agcaacctca ccacagacct catcgt                                           26

<210> 385
<211> 22
<212> DNA
<213> Artificial Sequence

<220>
<223> Description of Artificial Sequence: PCR Primer
      Sequence

<400> 385
atactccaac ctgttggtga tg                                               22

<210> 386
<211> 22
<212> DNA
<213> Artificial Sequence

<220>
<223> Description of Artificial Sequence: PCR Primer
      Sequence

<400> 386
gacaacaaac aagcaaacac aa                                               22

<210> 387
<211> 26
<212> DNA

<213> Artificial Sequence

<220>
<223> Description of Artificial Sequence: PCR Primer
      Sequence

<400> 387
accttccact tccttctggt cctgct                                          26


<210> 388
<211> 21
<212> DNA
<213> Artificial Sequence

<220>
<223> Description of Artificial Sequence: PCR Primer
      Sequence

<400> 388
gcaggagagg aggaagaaga g                                               21

## Description

### FIELD OF THE INVENTION

The invention relates to polynucleotides and the polypeptides encoded by such polynucleotides, as well as vectors, host cells, antibodies and recombinant methods for producing the polypeptides and polynucleotides, as well as methods for using the same.

### BACKGROUND OF THE INVENTION

The present invention is based in part on nucleic acids encoding proteins that are new members of the following protein families: Androgen-Regulated Short-Chain Dehydrogenase /Reductase-like, Aryl-Acylamidase-like, Insulysin-like, Integrin Beta-7 Precursor-like, Membrane protein-like, BCSC-1-like, Amino Acid Transporter-like, Lymphocyte Antigen Precursor-like, Lymphocyte Antigen LY-6F-like, Early B-Cell Factor-like, High-Affinity Camp-Specific and IBMX-Insensitive-like, KIAA0216-like, TWIK 3-like, TASK 4-like, Copper Transporter-like, Cytokeratin-like, Protocadherin-like, Protocadherin Beta-like, Cadherin 23-like, Transforming Growth Factor Beta 2-like, Ebnerin-like, Fatty Acid Binding-like, Platelet glycoprotein V-like, GARPIN-like, Centaurin Beta 2-like, Sorting Nexin 9-like, Katanin-like, Calpain-like, Keratin 18-like, Polycystic Kidney Disease Associated, Cholinephosphate Cytidylyltransferase-like and mac25/IGFBP7-like. More particularly, the invention relates to nucleic acids encoding novel polypeptides, as well as vectors, host cells, antibodies, and recombinant methods for producing these nucleic acids and polypeptides.

### SUMMARY OF THE INVENTION

The invention is based in part upon the discovery of nucleic acid sequences encoding novel polypeptides. The novel nucleic acids and polypeptides are referred to herein as NOVX, or NOV1, NOV2, NOV3, NOV4, NOV5, NOV6, NOV7, NOV8, NOV9, NOV10, NOV11, NOV12, NOV13, NOV14, NOV15, NOV16, NOV17, NOV18, NOV19, NOV20, NOV21, NOV22, NOV23, NOV24, NOV25, NOV26, NOV27, NOV28, NOV29 and NOV30 nucleic acids and polypeptides. These nucleic acids and polypeptides, as well as derivatives, homologs, analogs and fragments thereof, will hereinafter be collectively designated as "NOVX" nucleic acid or polypeptide sequences.

In one aspect, the invention provides an isolated NOVX nucleic acid molecule encoding a NOVX polypeptide that includes a nucleic acid sequence that has identity to the nucleic acids disclosed in SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93. In some embodiments, the NOVX nucleic acid molecule will hybridize under stringent conditions to a nucleic acid sequence complementary to a nucleic acid molecule that includes a protein-coding sequence of a NOVX nucleic acid sequence. The invention also includes an isolated nucleic acid that encodes a NOVX polypeptide, or a fragment, homolog, analog or derivative thereof. For example, the nucleic acid can encode a polypeptide at least 80% identical to a polypeptide comprising the amino acid sequences of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94. The nucleic acid can be, for example, a genomic DNA fragment or a cDNA molecule that includes the nucleic acid sequence of any of SEQ ID NOS:1, 3, 5, 7, 9,11,13,15,17,19,21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93.

Also included in the invention is an oligonucleotide, *e*.*g*., an oligonucleotide which includes at least 6 contiguous nucleotides of a NOVX nucleic acid (*e*.*g*., SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93) or a complement of said oligonucleotide. Also included in the invention are substantially purified NOVX polypeptides (SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94). In certain embodiments, the NOVX polypeptides include an amino acid sequence that is substantially identical to the amino acid sequence of a human NOVX polypeptide.

The invention also features antibodies that immunoselectively bind to NOVX polypeptides, or fragments, homologs, analogs or derivatives thereof.

In another aspect, the invention includes pharmaceutical compositions that include therapeutically- or prophylactically-effective amounts of a therapeutic and a pharmaceutically-acceptable carrier. The therapeutic can be, *e*.*g*., a NOVX nucleic acid, a NOVX polypeptide, or an antibody specific for a NOVX polypeptide. In a further aspect, the invention includes, in one or more containers, a therapeutically- or prophylactically-effective amount of this pharmaceutical composition.

In a further aspect, the invention includes a method of producing a polypeptide by culturing a cell that includes a NOVX nucleic acid, under conditions allowing for expression of the NOVX polypeptide encoded by the DNA. If desired, the NOVX polypeptide can then be recovered.

In another aspect, the invention includes a method of detecting the presence of a NOVX polypeptide in a sample. In the method, a sample is contacted with a compound that selectively binds to the polypeptide under conditions allowing for formation of a complex between the polypeptide and the compound. The complex is detected, if present, thereby identifying the NOVX polypeptide within the sample.

The invention also includes methods to identify specific cell or tissue types based on their expression of a NOVX.

Also included in the invention is a method of detecting the presence of a NOVX nucleic acid molecule in a sample by contacting the sample with a NOVX nucleic acid probe or primer, and detecting whether the nucleic acid probe or primer bound to a NOVX nucleic acid molecule in the sample.

In a further aspect, the invention provides a method for modulating the activity of a NOVX polypeptide by contacting a cell sample that includes the NOVX polypeptide with a compound that binds to the NOVX polypeptide in an amount sufficient to modulate the activity of said polypeptide. The compound can be, *e.g*., a small molecule, such as a nucleic acid, peptide, polypeptide, peptidomimetic, carbohydrate, lipid or other organic (carbon containing) or inorganic molecule, as further described herein.

Also within the scope of the invention is the use of a therapeutic in the manufacture of a medicament for treating or preventing disorders or syndromes including, *e.g*., trauma, regeneration (in vitro and in vivo), viral/bacterial/parasitic infections, Von Hippel-Lindau (VHL) syndrome, Alzheimer's disease, stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, behavioral disorders, addiction, anxiety, pain, actinic keratosis, acne, hair growth diseases, allopecia, pigmentation disorders, endocrine disorders, connective tissue disorders, such as severe neonatal Marfan syndrome, dominant ectopia lentis, familial ascending aortic aneurysm, isolated skeletal features of Marfan syndrome, Shprintzen-Goldberg syndrome, genodermatoses, contractural arachnodactyly, inflammatory disorders such as osteo- and rheumatoid-arthritis, inflammatory bowel disease, Crohn's disease; immunological disorders, AIDS; cancers including but not limited to lung cancer, colon cancer, Neoplasm; adenocarcinoma; lymphoma; prostate cancer, uterus cancer, leukemia or pancreatic cancer; blood disorders; asthma; psoriasis; vascular disorders, hypertension, skin disorders, renal disorders including Alport syndrome, immunological disorders, tissue injury, fibrosis disorders, bone diseases, Ehlers-Danlos syndrome type VI, VII, type IV, S-linked cutis laxa and Ehlers-Danlos syndrome type V, osteogenesis imperfecta, Neurologic diseases, Brain and/or autoimmune disorders like encephalomyelitis, neurodegenerative disorders, immune disorders, hematopoietic disorders, muscle disorders, inflammation and wound repair, bacterial, fungal, protozoal and viral infections (particularly infections caused by HIV-1 or HIV-2), pain, acute heart failure, hypotension, hypertension, urinary retention, osteoporosis, Treatment of Albright Hereditary Ostoeodystrophy, angina pectoris, myocardial infarction, ulcers, benign prostatic hypertrophy, arthrogryposis multiplex congenita, osteogenesis imperfecta, keratoconus, scoliosis, duodenal atresia, esophageal atresia, intestinal malrotation, Pancreatitis, Obesity Systemic lupus erythematosus, Autoimmune disease, Emphysema, Scleroderma, allergy, ARDS, Neuroprotection, Fertility Myasthenia gravis, Diabetes, obesity, Growth and reproductive disorders Hemophilia, Hypercoagulation, Idiopathic thrombocytopenic purpura, Immunodeficiencies, Graft vesus host, Adrenoleukodystrophy , Congenital Adrenal Hyperplasia, Endometriosis, Xerostomia, Ulcers, Cirrhosis, Transplantation, Diverticular disease, Hirschsprung's disease, Appendicitis, Arthritis, Ankylosing spondylitis, Tendinitis, Renal artery stenosis, Interstitial nephritis, Glomerulonephritis, Polycystic kidney disease, erythematosus, Renal tubular acidosis, IgA nephropathy, anorexia, bulimia, psychotic disorders, including anxiety, schizophrenia, manic depression, delirium, dementia, severe mental retardation and dyskinesias, such as Huntington's disease and/or other pathologies and disorders of the like.

The therapeutic can be, *e*.*g*., a NOVX nucleic acid, a NOVX polypeptide, or a NOVX-specific antibody, or biologically-active derivatives or fragments thereof.

For example, the compositions of the present invention will have efficacy for treatment of patients suffering from the diseases and disorders disclosed above and/or other pathologies and disorders of the like. The polypeptides can be used as immunogens to produce antibodies specific for the invention, and as vaccines. They can also be used to screen for potential agonist and antagonist compounds. For example, a cDNA encoding NOVX may be useful in gene therapy, and NOVX may be useful when administered to a subject in need thereof. By way of non-limiting example, the compositions of the present invention will have efficacy for treatment of patients suffering from the diseases and disorders disclosed above and/or other pathologies and disorders of the like.

The invention further includes a method for screening for a modulator of disorders or syndromes including, *e*.*g*., the diseases and disorders disclosed herein and/or other pathologies and disorders of the like. The method includes contacting a test compound with a NOVX polypeptide and determining if the test compound binds to said NOVX polypeptide. Binding of the test compound to the NOVX polypeptide indicates the test compound is a modulator of activity, or of latency or predisposition to the aforementioned disorders or syndromes.

Also within the scope of the invention is a method for screening for a modulator of activity, or of latency or predisposition to disorders or syndromes including, *e*.*g*., the diseases and disorders disclosed above and/or other pathologies and disorders of the like by administering a test compound to a test animal at increased risk for the aforementioned disorders or syndromes. The test animal expresses a recombinant polypeptide encoded by a NOVX nucleic acid. Expression or activity of NOVX polypeptide is then measured in the test animal, as is expression or activity of the protein in a control animal which recombinantly-expresses NOVX polypeptide and is not at increased risk for the disorder or syndrome. Next, the expression of NOVX polypeptide in both the test animal and the control animal is compared. A change in the activity of NOVX polypeptide in the test animal relative to the control animal indicates the test compound is a modulator of latency of the disorder or syndrome.

In yet another aspect, the invention includes a method for determining the presence of or predisposition to a disease associated with altered levels of a NOVX polypeptide, a NOVX nucleic acid, or both, in a subject (*e*.*g*., a human subject). The method includes measuring the amount of the NOVX polypeptide in a test sample from the subject and comparing the amount of the polypeptide in the test sample to the amount of the NOVX polypeptide present in a control sample. An alteration in the level of the NOVX polypeptide in the test sample as compared to the control sample indicates the presence of or predisposition to a disease in the subject. Preferably, the predisposition includes, *e*.*g*., the diseases and disorders disclosed above and/or other pathologies and disorders of the like. Also, the expression levels of the new polypeptides of the invention can be used in a method to screen for various cancers as well as to determine the stage of cancers.

In a further aspect, the invention includes a method of treating or preventing a pathological condition associated with a disorder in a mammal by administering to the subject a NOVX polypeptide, a NOVX nucleic acid, or a NOVX-specific antibody to a subject (*e.g*., a human subject), in an amount sufficient to alleviate or prevent the pathological condition. In preferred embodiments, the disorder, includes, *e.g.*, the diseases and disorders disclosed above and/or other pathologies and disorders of the like.

In yet another aspect, the invention can be used in a method to identity the cellular receptors and downstream effectors of the invention by any one of a number of techniques commonly employed in the art. These include but are not limited to the two-hybrid system, affinity purification, co-precipitation with antibodies or other specific-interacting molecules.

NOVX nucleic acids and polypeptides are further useful in the generation of antibodies that bind immuno-specifically to the novel NOVX substances for use in therapeutic or diagnostic methods. These NOVX antibodies may be generated according to methods known in the art, using prediction from hydrophobicity charts, as described in the "Anti-NOVX Antibodies" section below. The disclosed NOVX proteins have multiple hydrophilic regions, each of which can be used as an immunogen. These NOVX proteins can be used in assay systems for functional analysis of various human disorders, which will help in understanding of pathology of the disease and development of new drug targets for various disorders.

The NOVX nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration in vivo and in vitro of all tissues and cell types composing (but not limited to) those defined here.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION OF THE INVENTION

The present invention provides novel nucleotides and polypeptides encoded thereby. Included in the invention are the novel nucleic acid sequences and their encoded polypeptides. The sequences are collectively referred to herein as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table A provides a summary of the NOVX nucleic acids and their encoded polypeptides.

**TABLE A. Sequences and Corresponding SEQ ID Numbers**

| **NOVX Assignment** | **Internal Identification** | **SEQ ID NO (nucleic acid)** | **SEQ IOD NO (polypeptide)** | **Homology** |
|---|---|---|---|---|
| 1 | CG57413-01 | 1 | 2 | Androgen-Regulated Short-Chain Dehydrogenase / Reductase-like |
| 2a | CG57391-01 | 3 | 4 | Aryl-Acylamidase-like |
| 2b | CG57391-02 | 5 | 6 | Aryl-Acylamidase-like |
| 3a | CG57433-01 | 7 | 8 | Insulysin-like |
| 3b | CG57433-02 | 9 | 10 | Insulysin-like |
| 4a | CG57185-01 | 11 | 12 | Integrin Beta-7 Precursor-like |
| 4b | CG57185-02 | 13 | 14 | Integrin Beta-7 Precursor-like |
| 5 | CG57360-01 | 15 | 16 | Membrane protein-like |
| 6 | CG57362-01 | 17 | 18 | BCSC-1 like |
| 7a | CG57387-01 | 19 | 20 | Amino Acid Transporter-like |
| 7b | CG57387-02 | 21 | 22 | Amino Acid Transporter-like |
| 7c | CG57387-03 | 23 | 24 | Amino Acid Transporter-like |
| 8a | CG56417-01 | 25 | 26 | Lymphocyte Antigen Precursor-like |
| 8b | CG56417-02 | 27 | 28 | Lymphocyte Antigen Precursor-like |
| 8c | CG56417-03 | 29 | 30 | Lymphocyte Antigen Precursor-like |
| 8d | CG56417-04 | 31 | 32 | Lymphocyte Antigen LY-6F-like |
| 8e | 172885384 | 33 | 34 | Lymphocyte Antigen LY-6F-like |
| 9a | CG57480-01 | 35 | 36 | Early B-Cell Factor-like |
| 9b | CG57480-02 | 37 | 38 | Early B-Cell Factor-like |
| 10 | CG57389-01 | 39 | 40 | High-Affinity Camp-Specific and IBMX-Insensitive-like |
| 11 | CG57337-01 | 41 | 42 | KIAA0216-like |
| 12a | CG57220-01 | 43 | 44 | TWIK 3-like |
| 12b | CG57220-02 | 45 | 46 | TWIK 3-like |
| 12c | CG57220-03 | 47 | 48 | TWIK 3-like |
| 13 | CG57220-04 | 49 | 50 | TASK 4-like |
| 14 | CG57458-01 | 51 | 52 | Copper Transporter-like |
| 15 | CG57454-01 | 53 | 54 | Cytokeratin-like |
| 16a | CG57448-01 | 55 | 56 | Protocadherin-like |
| 16b | CG57446-01 | 57 | 58 | Protocadherin-like |
| 16c | CG57444-01 | 59 | 60 | Piotocadherin-like |
| 16d | CG57442-01 | 61 | 62 | Protocadherin Beta-like |
| 17a | CG57429-01 | 63 | 64 | Cadherin 23-like |
| 17b | CG57429-02 | 65 | 66 | Cadherin 23-like |
| 18 | CG55887-02 | 67 | 68 | Transforming Growth Factor Beta 2-like |
| 19 | CG57333-01 | 69 | 70 | Ebnerin-like |
| 20a | CG57556-01 | 71 | 72 | Fatty Acid Binding-like |
| 20b | CG57556-02 | 73 | 74 | Fatty Acid Binding-tike |
| 21 | CG57436-01 | 75 | 76 | Platelet glycoprotein V-like |
| 22 | CG57529-01 | 77 | 78 | GARPIN-like |
| 23 | CG57351-01 | 79 | 80 | Centaurin Beta 2-like |
| 24 | CG57515-01 | 81 | 82 | Sorting Nexin 9-like |
| 25 | CG57568-01 | 83 | 84 | Katanin-like |
| 26 | CG57509-01 | 85 | 86 | Calpain-like |
| 27 | CG57484-01 | 87 | 88 | Keratin 18-like |
| 28 | CG57236-01 | 89 | 90 | Polycystic Kidney Disease Associated-like |
| 29 | CG57589-01 | 91 | 92 | Cholinephosphate Cytidylyltransferase-like |
| 30 | CG57558-01 | 93 | 94 | mac25/IGFBP7-like |

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

The NOVX nucleic acids and polypeptides can also be used to screen for molecules, which inhibit or enhance NOVX activity or function. Specifically, the nucleic acids and polypeptides according to the invention may be used as targets for the identification of small molecules that modulate or inhibit, e.g., neurogenesis, cell differentiation, cell proliferation, hematopoiesis, wound healing and angiogenesis.

Additional utilities for the NOVX nucleic acids and polypeptides according to the invention are disclosed herein.

### NOV1

A disclosed NOV1 nucleic acid of 872 nucleotides (also referred to as CG57413-01) encoding a novel Androgen-Regulated Short-Chain Dehydrogenase/Reductase-like protein is shown in Table 1A. An open reading frame was identified beginning with an ATG initiation codon at nucleotides 11-13 and ending with a TAA codon at nucleotides 845-847. Putative untranslated regions, if any, upstream from the initiation codon and downstream from the termination codon are underlined in Table 1A, and the start and stop codons are in bold letters.

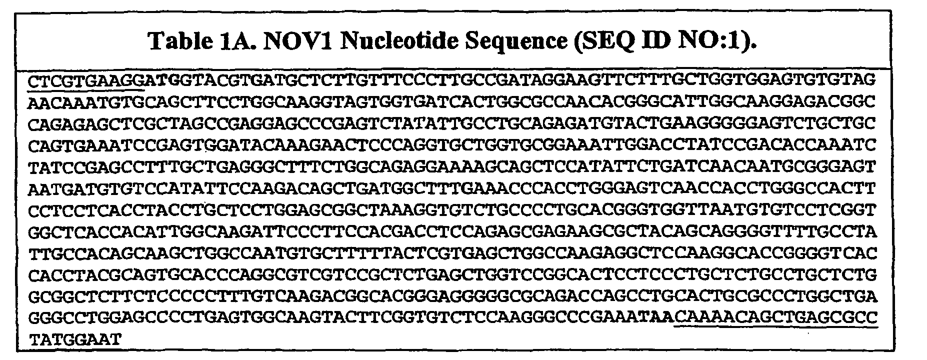

The NOV1 nucleic acid sequence maps to chromosome 14 and has 584 of 797 bases (73%) identical to a gb:GENBANK-ID:AX041971|acc:AX041971.1 mRNA from *Homo sapiens* (Sequence 1 from Patent WO0065067) (E = 2.1e⁻⁸⁴). Similiarity information was assessed using public nucleotide databases including all GenBank databases and the GeneSeq patent database. Chromosome information was assigned using OMIM and the electronic northern tool from Curatools to derive the the chromosomal mapping of the SeqCalling assemblies, Genomic clones, and/or EST sequences that were included in the invention.

In all BLAST alignments herein, the "E-value" or "Expect" value is a numeric indication of the probability that the aligned sequences could have achieved their similarity to the BLAST query sequence by chance alone, within the database that was searched. For example, the probability that the subject ("Sbjct") retrieved from the NOV1 BLAST analysis, *e.g*., mRNA from *Homo sapiens* (Sequence 1 from Patent WO0065067), matched the Query NOV1 sequence purely by chance is 2.1e⁻⁸⁴. The Expect value (E) is a parameter that describes the number of hits one can "expect" to see just by chance when searching a database of a particular size. It decreases exponentially with the Score (S) that is assigned to a match between two sequences. Essentially, the E value describes the random background noise that exists for matches between sequences.

The Expect value is used as a convenient way to create a significance threshold for reporting results. The default value used for blasting is typically set to 0.0001. In BLAST 2.0, the Expect value is also used instead of the P value (probability) to report the significance of matches. For example, an E value of one assigned to a hit can be interpreted as meaning that in a database of the current size one might expect to see one match with a similar score simply by chance. An E value of zero means that one would not expect to see any matches with a similar score simply by chance. See, e.g., http://www.ncbi.nlm.nih.gov/Education/BLASTinfo/. Occasionally, a string of X's or N's will result from a BLAST search. This is a result of automatic filtering of the query for low-complexity sequence that is performed to prevent artifactual hits. The filter substitutes any low-complexity sequence that it finds with the letter "N" in nucleotide sequence (e.g., "NNNNNNNN") or the letter "X" in protein sequences (e.g., "XXX"). Low-complexity regions can result in high scores that reflect compositional bias rather than significant position-by-position alignment. Wootton and Federhen, Methods Enzymol 266:554-571, 1996.

The disclosed NOV1 polypeptide (SEQ ID NO:2) encoded by SEQ ID NO:1 has 278 amino acid residues and is presented in Table 1B using the one-letter amino acid code. Signal P, Psort and/or Hydropathy results predict that NOV1 does not contain a signal peptide and is likely to be localized to the mitochondrial matrix space with a certainty of 0.4843 and to the microbody (peroxisome) with a certainty of 0.3713.

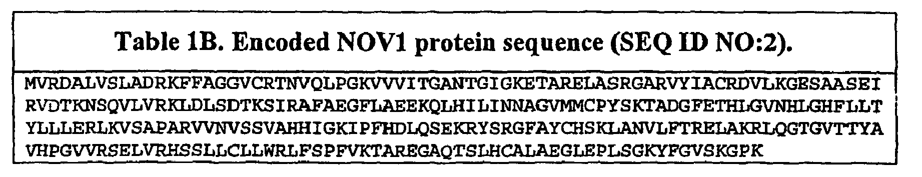

The NOV1 amino acid sequence has 197 of 260 amino acid residues (75%) identical to, and 228 of 260 amino acid residues (87%) similar to, the 318 amino acid residue ptnr:SPTREMBL-ACC:Q9NRW0 protein from Homo sapiens (Androgen-Regulated Short-Chain Dehydrogenase/Reductase 1) (E = 4.6e⁻¹⁰⁵).

NOV1 is expressed in at least the following tissues: kidney, pancreas, retina and vulva. This information was derived by determining the tissue sources of the sequences that were included in the invention including but not limited to SeqCalling sources, public EST sources, and/or RACE sources.

Possible small nucleotide polymorphisms (SNPs) found for NOV1 are listed in Table 1C. Depth, when shown, represents the number of clones covering the region of the SNP. The putative allele frequence (PAF), when shown, is the fraction of these clones containing the SNP. A dash, when shown, means that a base is not present. The sign ">" means "is changed to." Silent, when indicated, means that the SNP did not result in an amino acid change.

| **Table 1C: SNPs** | | | | |
|---|---|---|---|---|
| **Variant** | **Nucleotide** Position | **Base** Change | **Amino** Acid Position | **Base** Change |
| 13375950 | 327 | A > G | 106 | Lys > Arg |

NOV1 has homology to the amino acid sequences shown in the BLASTP data listed in Table 1D.

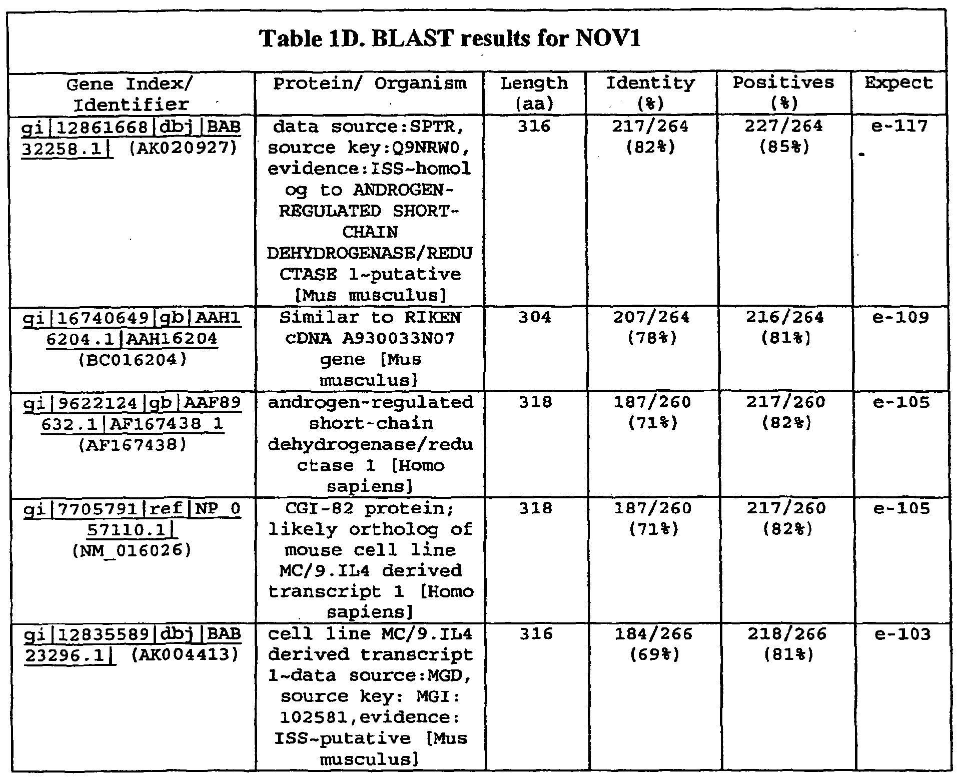

The homology between these and other sequences is shown graphically in the ClustalW analysis shown in Table 1E. In the ClustalW alignment of the NOV1 protein, as well as all other ClustalW analyses herein, the black outlined amino acid residues indicate regions of conserved sequence (*i*.*e*., regions that may be required to preserve structural or functional properties), whereas non-highlighted amino acid residues are less conserved and can potentially be altered to a much broader extent without altering protein structure or function.

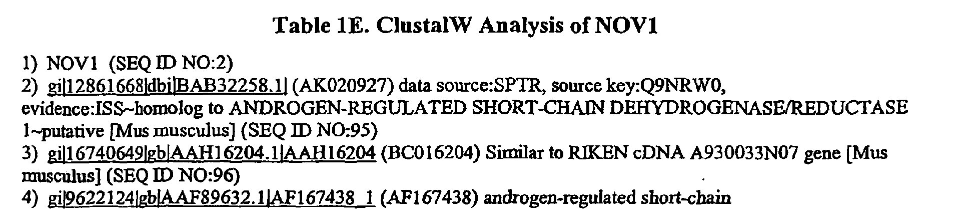

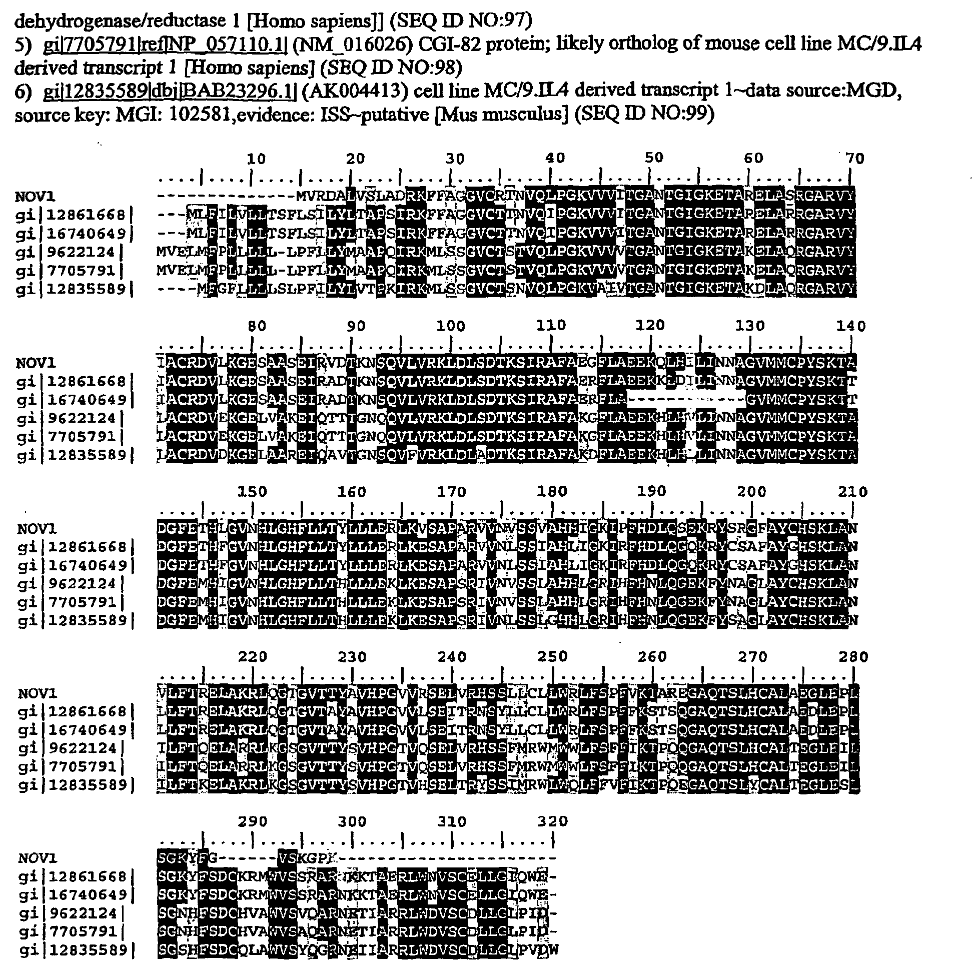

The presence of identifiable domains in NOV 1, as well as all other NOVX proteins, was determined by searches using software algorithms such as PROSITE, DOMAIN, Blocks, Pfam, ProDomain, and Prints, and then determining the Interpro number by crossing the domain match (or numbers) using the Interpro website (http:www.ebi.ac.uk/ interpro). DOMAIN results for NOV1, as disclosed in Table 1F, were collected from the Conserved Domain Database (CDD) with Reverse Position Specific BLAST analyses. This BLAST analysis software samples domains found in the Smart and Pfam collections. For Table 1F and all successive DOMAIN sequence alignments, fully conserved single residues are indicated by black shading or by the sign (|) and "strong" semi-conserved residues are indicated by grey shading or by the sign (+). The "strong" group of conserved amino acid residues may be any one of the following groups of amino acids: STA, NEQK, NHQY, NDEQ, QHRK, MILV, MILF, HY, FYW.

Table 1F lists the domain description from DOMAIN analysis results against NOV1. This indicates that the NOV1 sequence has properties similar to those of other proteins known to contain these domains.

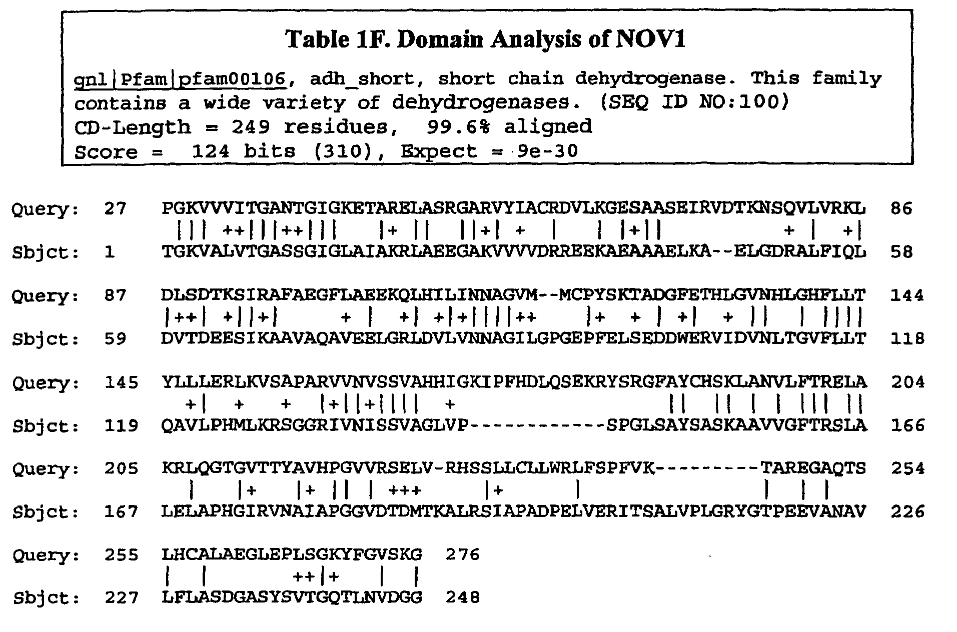

Most mammalian hydroxysteroid dehydrogenases known thus far belong to the protein superfamilies of short-chain dehydrogenases/reductases (SDR) and aldo-keto reductases (AKR). Whereas members of the AKR family are soluble, cytoplasmic enzymes, SDR-type hydroxysteroid dehydrogenases are also located to other subcellular compartments, i.e. endoplasmic reticulum, mitochondria or peroxisomes. Differential localization might play an important role in influencing the reaction direction of hydroxy dehydrogenaseloxo reductase pathways by determining the available nucleotide cofactor pool. Targeting signals for different subcellular organelles in human hydroxysteroid dehydrogenases have been identified, however, in several enzymes localization signals remain to be determined. The short-chain dehydrogenases/reductases family (SDR) is a very large family of enzymes, most of which are known to be NAD- or NADP-dependent oxidoreductases. As the first member of this family to be characterized was Drosophila alcohol dehydrogenase, this family used to be called 'insect-type', or 'short-chain' alcohol dehydrogenases. Most members of this family are proteins of about 250 to 300 amino acid residues. Most dehydrogenases possess at least 2 domains, the first binding the coenzyme, often NAD, and the second binding the substrate. This latter domain determines the substrate specificity and contains amino acids involved in catalysis. Little sequence similarity has been found in the coenzyme binding domain although there is a large degree of structural similarity, and it has therefore been suggested that the structure of dehydrogenases has arisen through gene fusion of a common ancestral coenzyme nucleotide sequence with various substrate specific domains.

The NOV1 nucleic acid of the invention encoding a Androgen-Regulated Short-Chain Dehydrogenase/Reductase-like protein includes the nucleic acid whose sequence is provided in Table 1A, or a fragment thereof. The invention also includes a mutant or variant nucleic acid any of whose bases may be changed from the corresponding base shown in Table 1A while still encoding a protein that maintains its Androgen-Regulated Short-Chain Dehydrogenase/Reductase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 27% of the residues may be so changed.

The NOV1 protein of the invention includes the Androgen-Regulated Short-Chain Dehydrogenase/Reductase-like protein whose sequence is provided in Table 1B. The invention also includes a mutant or variant protein any of whose residues may be changed from the corresponding residue shown in Table 1B while still encoding a protein that maintains its Androgen-Regulated Short-Chain Dehydrogenase/Reductase-like activities and physiological functions, or a functional fragment thereof. In the mutant or variant protein, up to about 25% of the bases may be so changed.

The NOV1 nucleic acids and proteins of the invention are useful in potential diagnostic and therapeutic applications implicated in various diseases and disorders described below and/or other pathologies. For example, the compositions of the present invention will have efficacy for treatment of patients suffering from: diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, Von Hippel-Lindau (VHL) syndrome, pancreatitis, obesity, tuberous sclerosis, fertility and other diseases, disorders and conditions of the like.

NOV1 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV1 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV2

NOV2 includes two novel Aryl-acylamidase-like proteins disclosed below. The disclosed proteins have been named NOV2a and NOV2b.

### NOV2a

A disclosed NOV2a nucleic acid of 1527 nucleotides (also referred to as CG57391-01) encoding a novel Aryl-acylamidase-like protein is shown in Table 2A. An open reading frame was identified beginning with an ATG initiation codon at nucleotides 37-39 and ending with a TAA codon at nucleotides 1240-1242. Putative untranslated regions, if any, upstream from the initiation codon and downstream from the termination codon are underlined in Table 2A, and the start and stop codons are in bold letters.

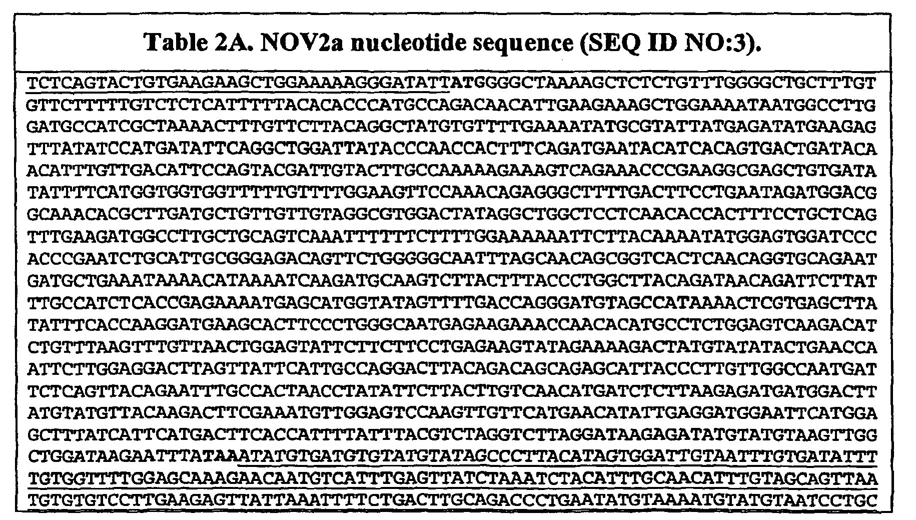

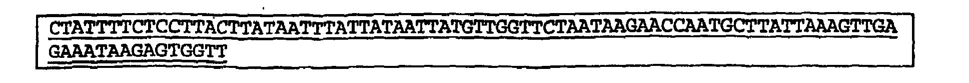

The disclosed NOV2a nucleic acid sequence, localized to chromsome 3, has 770 of 1205 bases (63%) identical to a gb:GENBANK-ID:HUMARDE|acc:L32179.1 mRNA from *Homo sapiens* (Human arylacetamide deacetylase mRNA, complete cds) (E = 1.3e⁻⁷⁵).

A NOV2a polypeptide (SEQ ID NO:4) encoded by SEQ ID NO:3 has 401 amino acid residues and is presented using the one-letter code in Table 2B. Signal P, Psort and/or Hydropathy results predict that NOV2a contains a signal peptide and is likely to be localized to the cytoplasm with a certainty of 0.8524 or extracellularly with a certainty of 0.7714. The most likely cleavage site for a NOV2a peptide is between amino acids 18 and 19: VSH-FY.

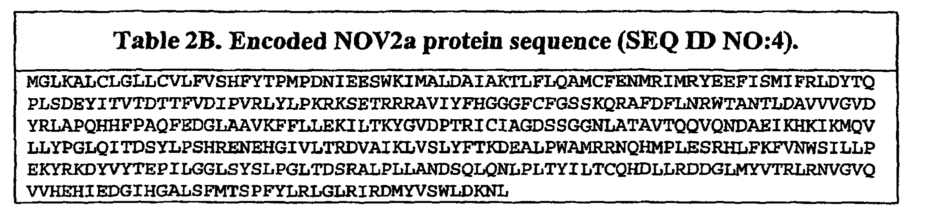

The NOV2a amino acid sequence has 205 of 401 amino acid residues (51 %) identical to, and 282 of 401 amino acid residues (70%) similar to, the 399 amino acid residue ptnr:pir-id:A53856 protein from human (aryl-acylamidase (EC 3.5.1.13)) (E = 2.7e⁻¹⁰⁷).

The disclosed NOV2a is expressed in at least the following tissues: heart, uterus and colon. This information was derived by determining the tissue sources of the sequences that were included in the invention including but not limited to SeqCalling sources, Public EST sources, Literature sources, and/or RACE sources.

### NOV2b

A disclosed NOV2b nucleic acid of 1324 nucleotides (also referred to as CG57391-02) encoding a novel Aryl-acylamidase-like protein is shown in Table 2C. An open reading frame was identified beginning with an ATG initiation codon at nucleotides 34-36 and ending with a TAA codon at nucleotides 1237-1239. Putative untranslated regions, if any, upstream from the initiation codon and downstream from the termination codon are underlined in Table 2C, and the start and stop codons are in bold letters.

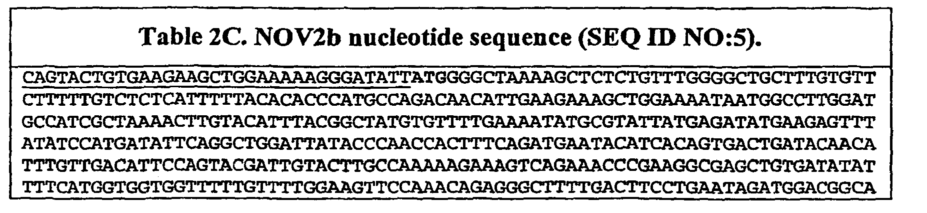

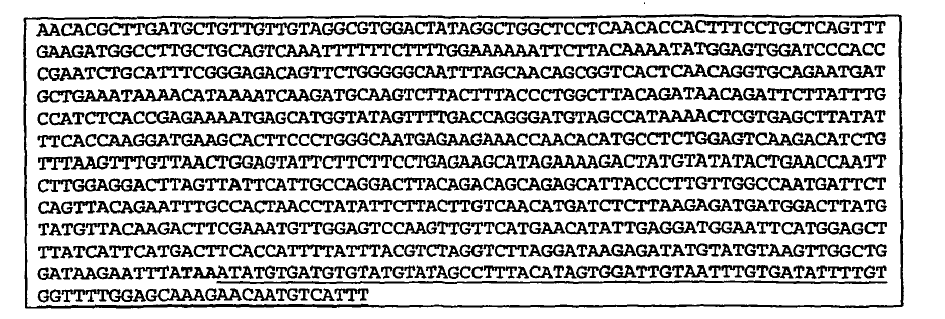

The disclosed NOV2b nucleic acid sequence, localized to chromsome 3, 805 of 1261 bases (63%) identical to a gb:GENBANK-ID:HUMARDE|acc:L32179.1 mRNA from *Homo sapiens* (Human arylacetamide deacetylase mRNA, complete cds) (E = 1.4e⁻⁷⁶).

A NOV2b polypeptide (SEQ ID NO:6) encoded by SEQ ID NO:5 has 401 amino acid residues and is presented using the one-letter code in Table 2D. Signal P, Psort andior Hydropathy results predict that NOV2b contains a signal peptide and is likely to be localized to the cytoplasm with a certainty of 0.8524 or extracellularly with a certainty of 0.7714. The most likely cleavage site for a NOV2b peptide is between amino acids 18 and 19: VSH-FY.

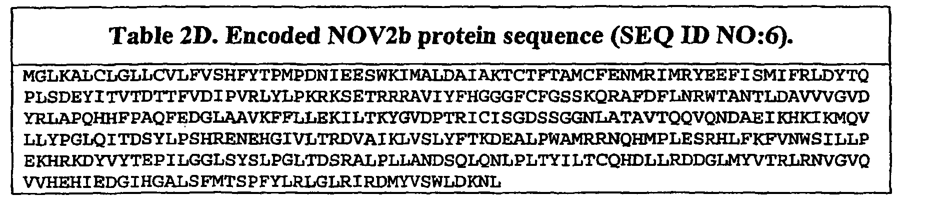

The NOV2b amino acid sequence has 206 of 401 amino acid residues (51 %) identical to, and 280 of 401 amino acid residues (69%) similar to, the 399 amino acid residue ptnr:pir-id:A53856 protein from human (aryl-acylamidase (EC 3.5.1.13)) (E = 3.9e⁻¹⁰⁷).

The disclosed NOV2b is expressed in at least the following tissues: heart, uterus and colon. This information was derived by determining the tissue sources of the sequences that were included in the invention including but not limited to SeqCalling sources, Public EST sources, Literature sources, and/or RACE sources.

Possible small nucleotide polymorphisms (SNPs) found for NOV2a are listed in Table 2E.

| **Table 2E: SNPs** | | | | |
|---|---|---|---|---|
| **Variant** | **Nucleotide Position** | **Base Change** | **Amino Acid Position** | **Base Change** |
| 13377048 | 179 | T > A | 48 | Met>Lys |
| 13377047 | 839 | A > T | 268 | Gln > Leu |
| 13377046 | 853 | G > A | 273 | Glu>Lys |
| 13377045 | 907 | T > C | 291 | Tyr>His |
| 13377044 | 1044 | T > C | Silent | N/A |
| 13377043 | 1140 | T > C | Silent | N/A |
| 13377042 | 1210 | A > G | 392 | Met > Val |
| 13377041 | 1266 | C > T | Silent | N/A |

NOV2a and NOV2b are very closely homologous as is shown in the amino acid alignment in Table 2F.

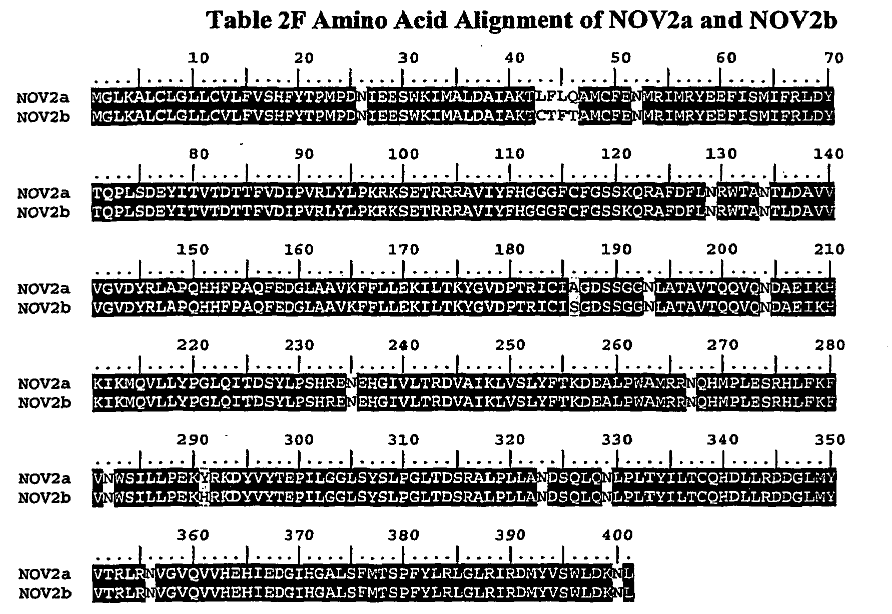

Homologies to any of the above NOV2 proteins will be shared by the other NOV2 proteins insofar as they are homologous to each other as shown above. Any reference to NOV2 is assumed to refer to both of the NOV2 proteins in general, unless otherwise noted.

NOV2a has homology to the amino acid sequences shown in the BLASTP data listed in Table 2G.

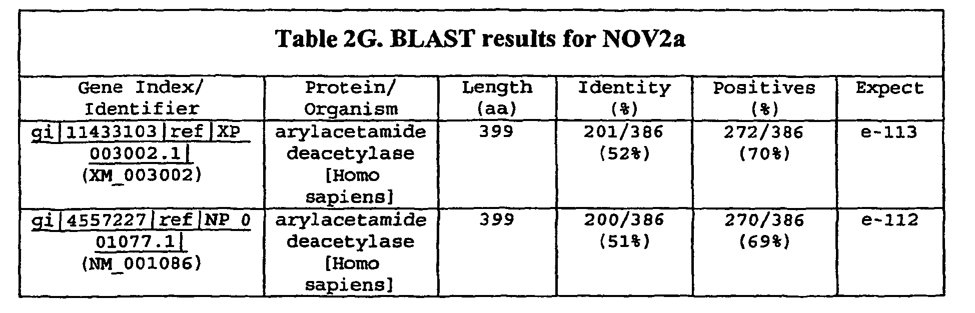

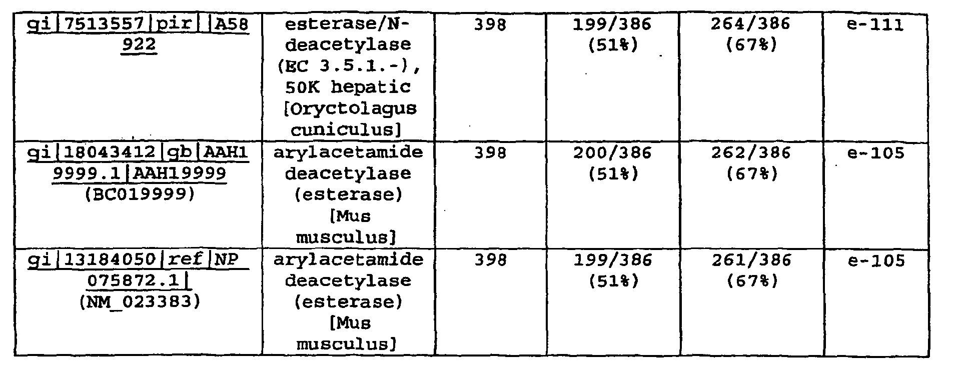

The homology of these sequences is shown graphically in the ClustalW analysis shown in Table 2H.

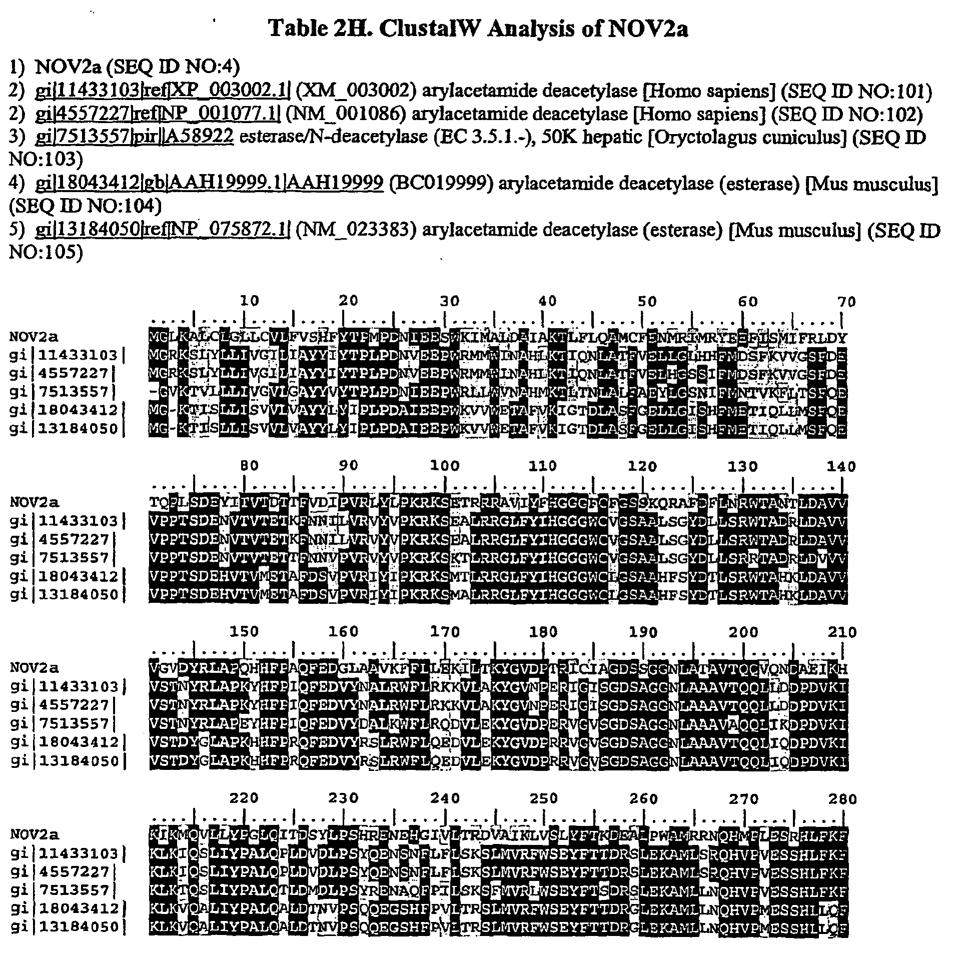

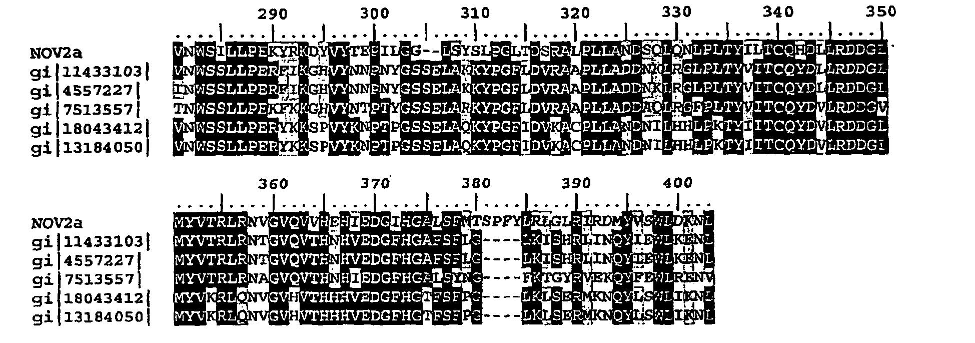

The NOV2 nucleic acid of the invention encoding a Aryl-acylamidase-like protein includes the nucleic acid whose sequence is provided in Tables 2A and 2C, or a fragment thereof. The invention also includes a mutant or variant nucleic acid any of whose bases may be changed from the corresponding base shown in Tables 2A and 2C while still encoding a protein that maintains its Aryl-acylamidase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 37% of the residues may be so changed.

The NOV2 protein of the invention includes the Aryl-acylamidase-like protein whose sequence is provided in Tables 2B and 2D. The invention also includes a mutant or variant protein any of whose residues may be changed from the corresponding residue shown in Tables 2B and 2D while still encoding a protein that maintains its Aryl-acylamidase-like activities and physiological functions, or a functional fragment thereof. In the mutant or variant protein, up to about 49% of the bases may be so changed.

The NOV2 nucleic acids and proteins of the invention are useful in potential diagnostic and therapeutic applications implicated in various diseases and disorders described below and/or other pathologies. For example, the compositions of the present invention will have efficacy for treatment of patients suffering from: Cardiomyopathy, Atherosclerosis, Hypertension, Congenital heart defects, Aortic stenosis, Atrial septal defect (ASD), Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity,Transplantation, Endometriosis, Fertility and other diseases, disorders and conditions of the like.

NOV2 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV2 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV3

NOV3 includes two novel Insulysin-like proteins disclosed below. The disclosed proteins have been named NOV3a and NOV3b.

### NOV3a

A disclosed NOV3a nucleic acid of 4321 nucleotides (also referred to as CG57433-01) encoding a novel Insulysin-like protein is shown in Table 3A. An open reading frame was identified beginning with an ATG initiation codon at nucleotides 58-60 and ending with a TGA codon at nucleotides 3115-3117. Putative untranslated regions, if any, upstream from the initiation codon and downstream from the termination codon are underlined in Table 3A, and the start and stop codons are in bold letters.

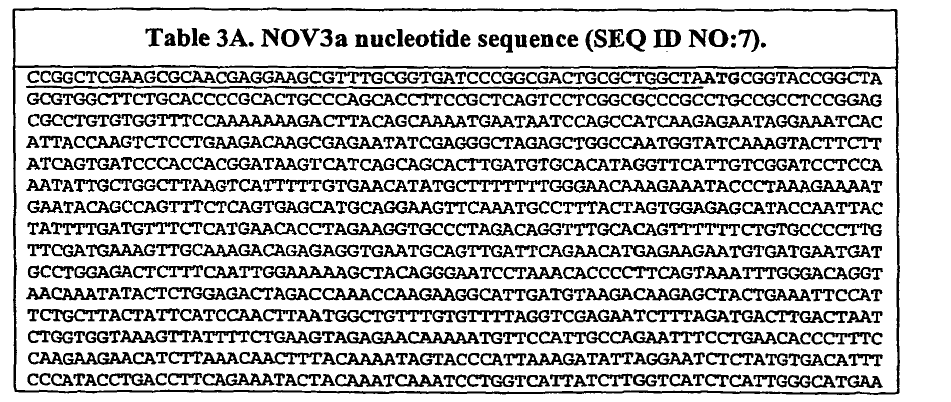

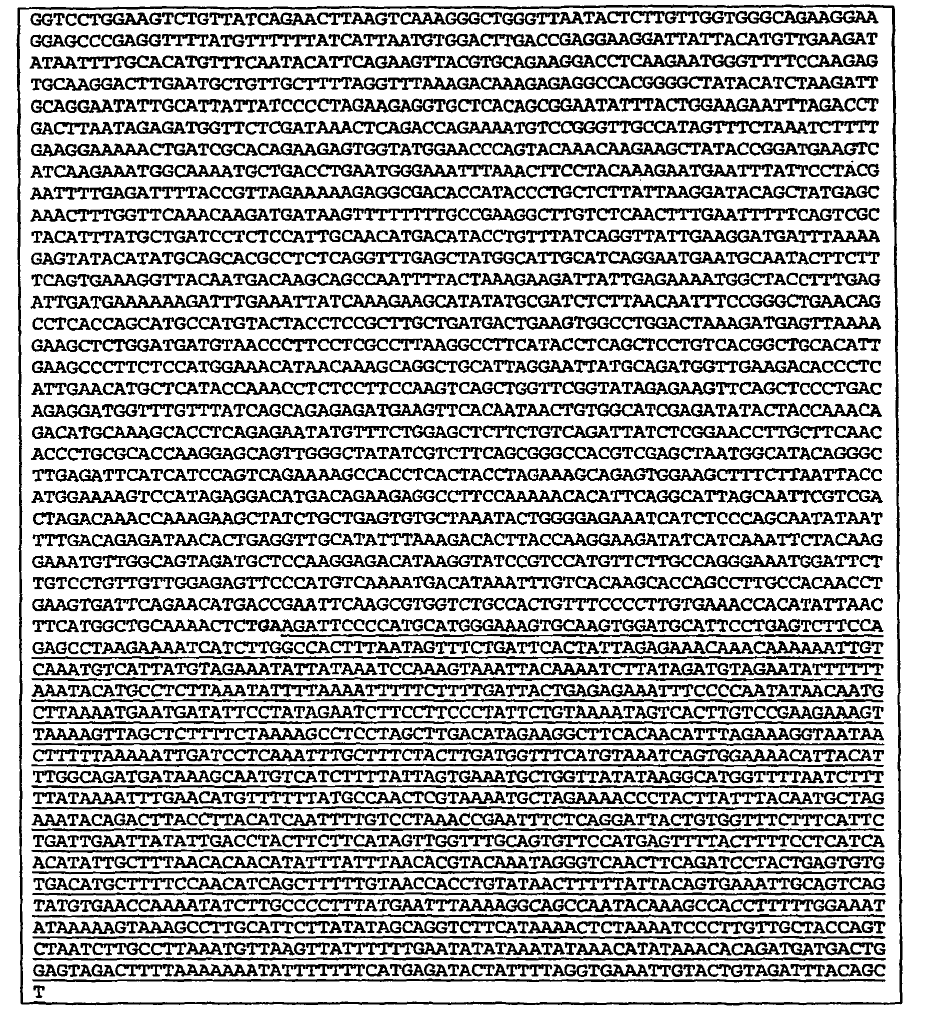

The disclosed NOV3a nucleic acid sequence, localized to chromsome 10, has 3265 of 3336 bases (97%) identical to a gb:GENBANK-ID:HUMIDE|acc:M21188.1 mRNA from *Homo sapiens* (Human insulin-degrading enzyme (IDE) mRNA, complete cds) (E = 0.0).

A NOV3a polypeptide (SEQ ID NO:8) encoded by SEQ ID NO:7 has 1019 amino acid residues and is presented using the one-letter code in Table 3B. Signal P, Psort and/or Hydropathy results predict that NOV3a contains a signal peptide and is likely to be localized to the peroxisome (microbody) with a certainty of 0.8347 or the mitochondrial matrix space with a certainty of 0.6517. The most likely cleavage site for a NOV3a peptide is between amino acids 22 and 23: VLG-AR.

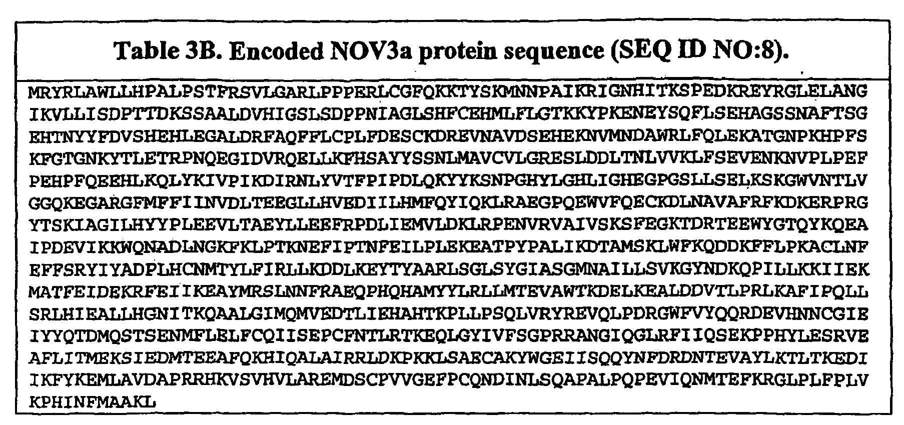

The NOV3a amino acid sequence has 989 of 1019 amino acid residues (97%) identical to, and 999 of 1019 amino acid residues (98%) similar to, the 1019 amino acid residue ptnr:pir-id:SNHUIN protein from human (insulysin (EC 3.4.24.56)) (E = 0.0).

The disclosed NOV3a is expressed in at least the following tissues: Cervix, Liver, Spleen, Testis, Melanocyte, Heart and Uterus. This information was derived by determining the tissue sources of the sequences that were included in the invention including but not limited to SeqCalling sources, Public EST sources, Literature sources, and/or RACE sources.

### NOV3b

A disclosed NOV3b nucleic acid of 3153 nucleotides (also referred to as CG57433-02) encoding a novel Insulysin-like protein is shown in Table 3C. An open reading frame was identified beginning with an ATG initiation codon at nucleotides 37-39 and ending with a TGA codon at nucleotides 3094-3096. Putative untranslated regions, if any, upstream from the initiation codon and downstream from the termination codon are underlined in Table 3C, and the start and stop codons are in bold letters.

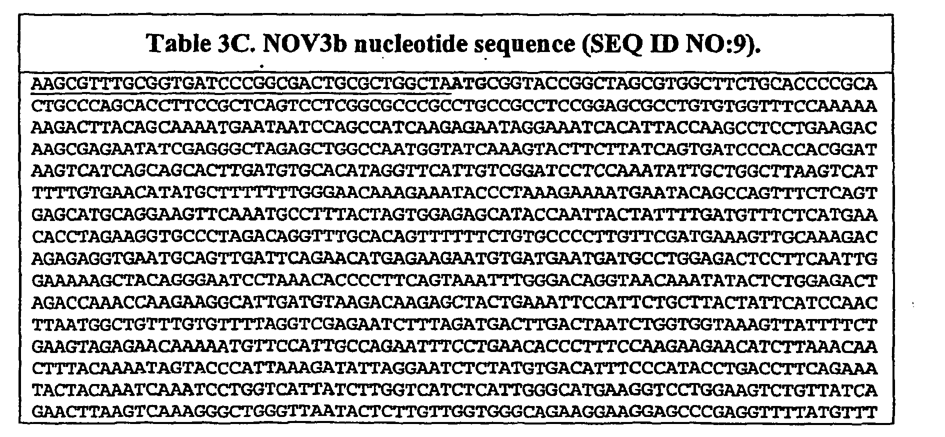

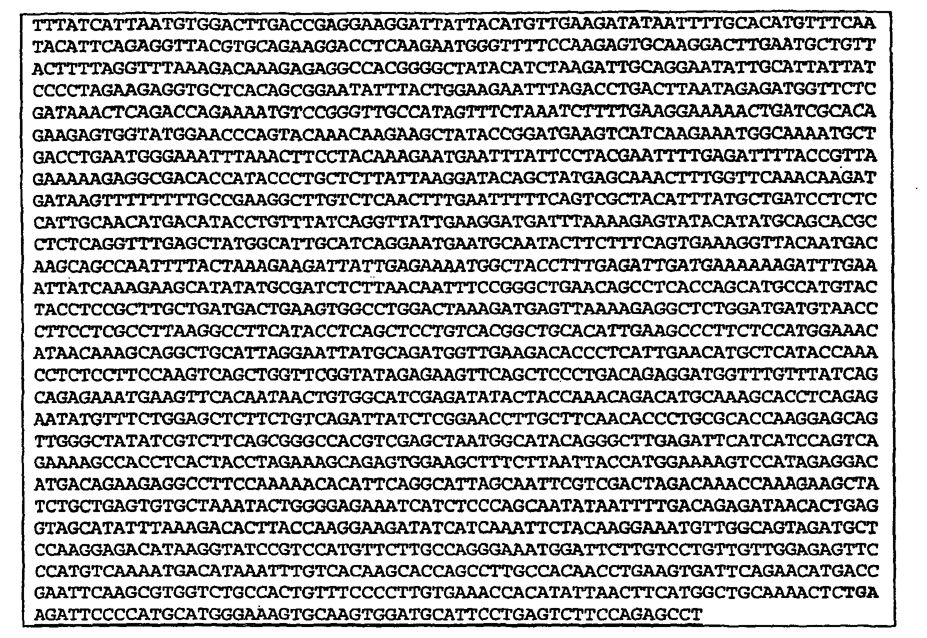

The disclosed NOV3b nucleic acid sequence, localized to chromsome 10q23-q25, 3078 of 3153 bases (97%) identical to a gb:GENBANK-ID:HUMIDE|acc:M21188.1 mRNA from *Homo sapiens* (Human insulin-degrading enzyme (IDE) mRNA, complete cds) (E = 0.0).

A NOV3b polypeptide (SEQ ID NO:10) encoded by SEQ ID NO:9 has 1019 amino acid residues and is presented using the one-letter code in Table 3D. Signal P, Psort and/or Hydropathy results predict that NOV3b contains a signal peptide and is likely to be localized to the peroxisome (microbody) with a certainty of 0.8347 or the mitochondrial matrix space with a certainty of 0.6517. The most likely cleavage site for a NOV3b peptide is between amino acids 22 and 23: VLG-AR.

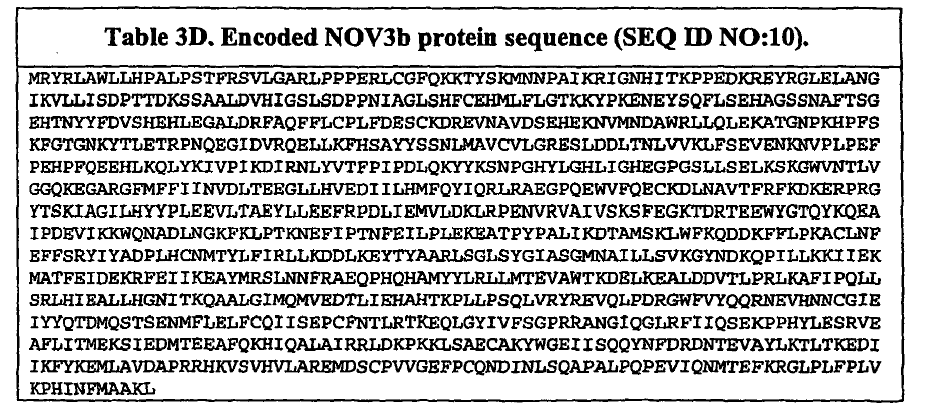

The NOV3b amino acid sequence has 986 of 1019 amino acid residues (96%) identical to, and 996 of 1019 amino acid residues (97%) similar to, the 1019 amino acid residue ptnr:pir-id:SNHCTIN protein from human (insulysin (EC 3.4.24.56)) (E = 0.0).

The disclosed NOV3b is expressed in at least the following tissues: Lung, skin, uterus, colon, breast, liver, spleen, testis, and pediatric pre-B cell acute lymphoblastic leukemia. This information was derived by determining the tissue sources of the sequences that were included in the invention including but not limited to SeqCalling sources, Public EST sources, Literature sources, and/or RACE sources.

Possible SNPs found for NOV3a are listed in Table 3E and possible SNPs found for NOV3b are listed in Table 3F.

| **Table 3E: SNPs** | | | | |
|---|---|---|---|---|
| **Variant** | **Nucleotide Position** | **Base Change** | **Amino Acid Position** | **Base Change** |
| 13375689 | 177 | C > T | Silent | N/A |
| 13375688 | 184 | A > G | 43 | Asn > Asp |
| 13375672 | 223 | A > T | 56 | Lys > End |
| 13375687 | 226 | T > C | 57 | Ser > Pro |
| 13375686 | 270 | T > C | Silent | N/A |
| 13375685 | 322 | G > T | 89 | Ala > Ser |
| 13375684 | 330 | T > C | Silent | N/A |
| 13375683 | 355 | C > T | 100 | Pro > Ser |
| 13375682 | 435 | A > G | Silent | N/A |
| 13375681 | 450 | C > T | Silent | N/A |
| 13375326 | 661 | T > C | 202 | Phe > Leu |
| 13375680 | 685 | A > G | 210 | Asn > Asp |
| 13375679 | 905 | T > C | 283 | Val > Ala |
| 13375327 | 1256 | A > G | 400 | Lys > Arg |
| 13375328 | 1318 | G > A | 421 | Ala > Thr |
| 13375329 | 1412 | C > T | 452 | Ala > Val |
| 13375678 | 2686 | A > G | 877 | Met > Val |
| 13375677 | 2751 | G > A | Silent | N/A |
| 13375676 | 2815 | A > G | 920 | Arg > Gly |
| 13375675 | 2895 | A > G | Silent | N/A |
| 13375674 | 2910 | A > G | Silent | N/A |
| 13375673 | 2984 | A > G | 976 | Asn > Ser |
| 13377049 | 3077 | T > A | 1007 | Leu > His |

| **Table 3F: SNPs** | | | |
|---|---|---|---|
| **Consensus Position** | **Depth** | **Base Change** | **PAF** |
| 656 | 7 | C > T | 0.429 |

NOV3a and NOV3b are very closely homologous as is shown in the amino acid alignment in Table 3 G.

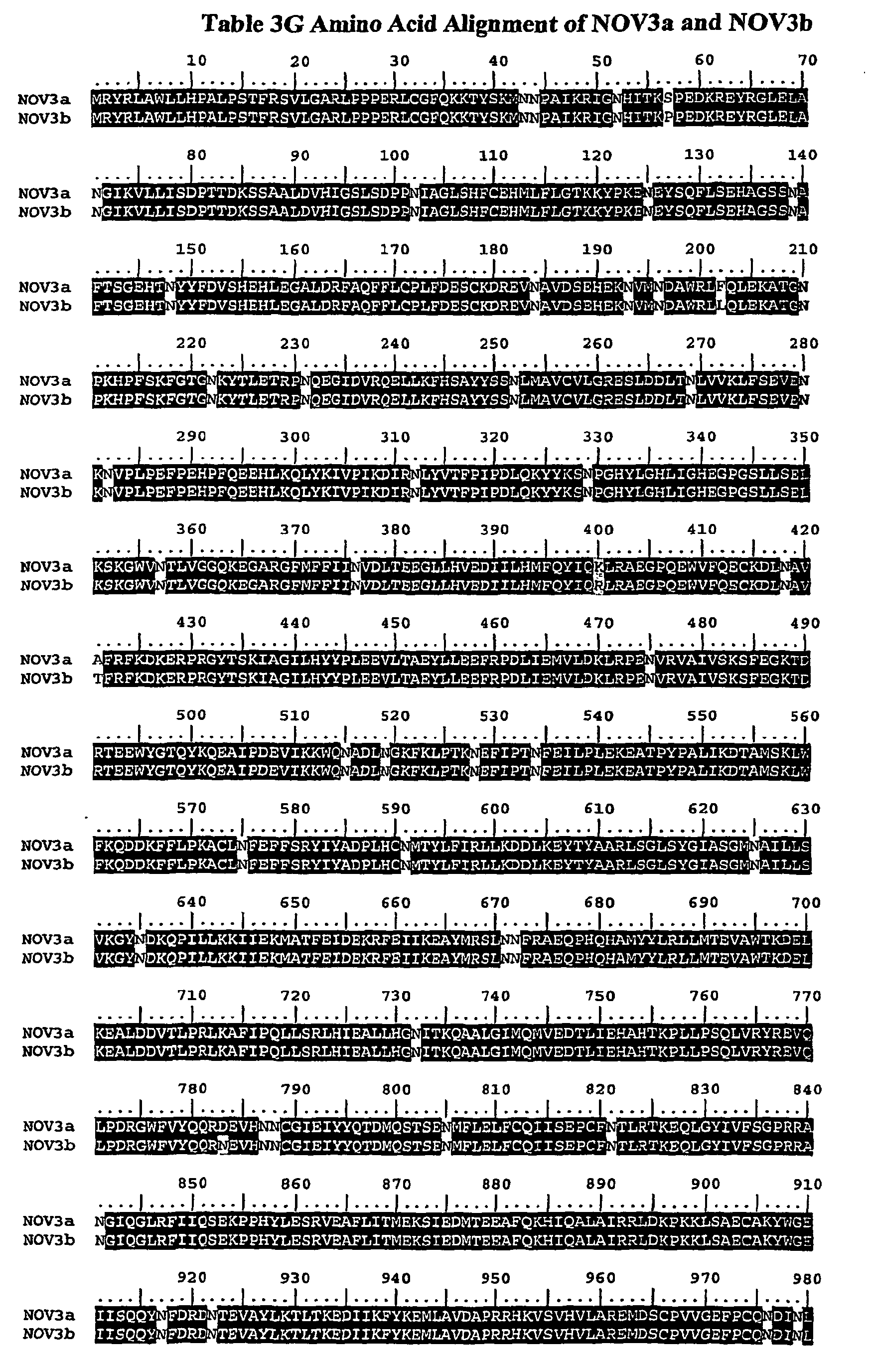

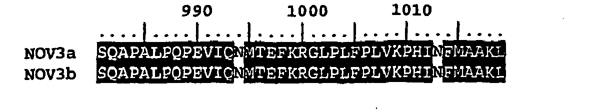

Homologies to any of the above NOV3 proteins will be shared by the other NOV3 proteins insofar as they are homologous to each other as shown above. Any reference to NOV3 is assumed to refer to both of the NOV3 proteins in general, unless otherwise noted.

NOV3a has homology to the amino acid sequences shown in the BLASTP data listed in Table 3H.

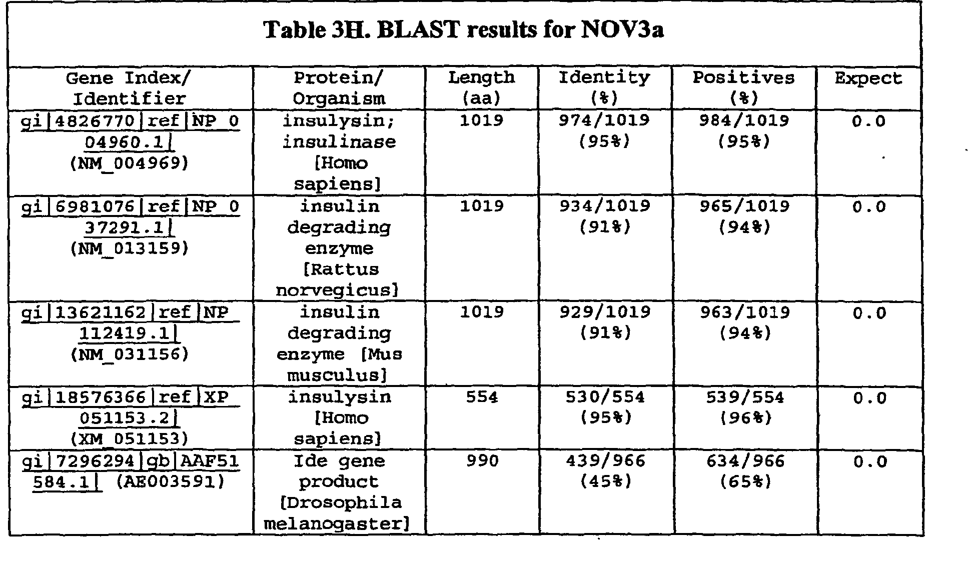

The homology of these sequences is shown graphically in the ClustalW analysis shown in Table 3I.

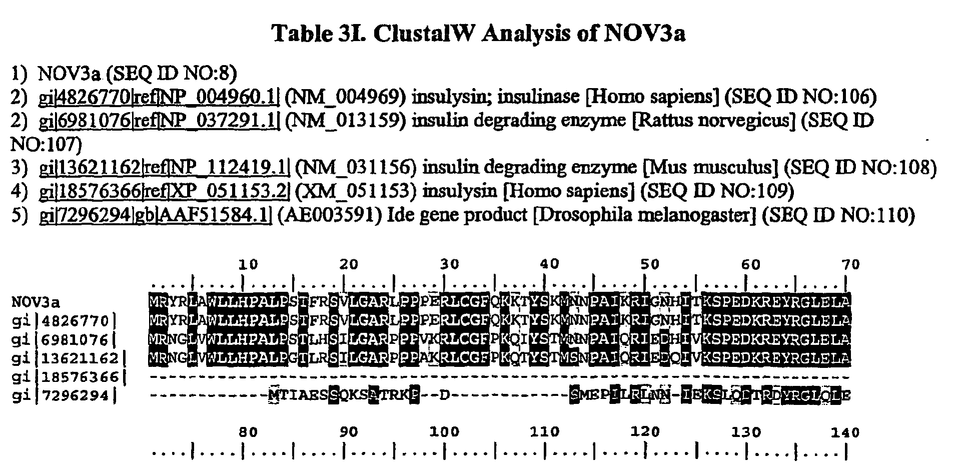

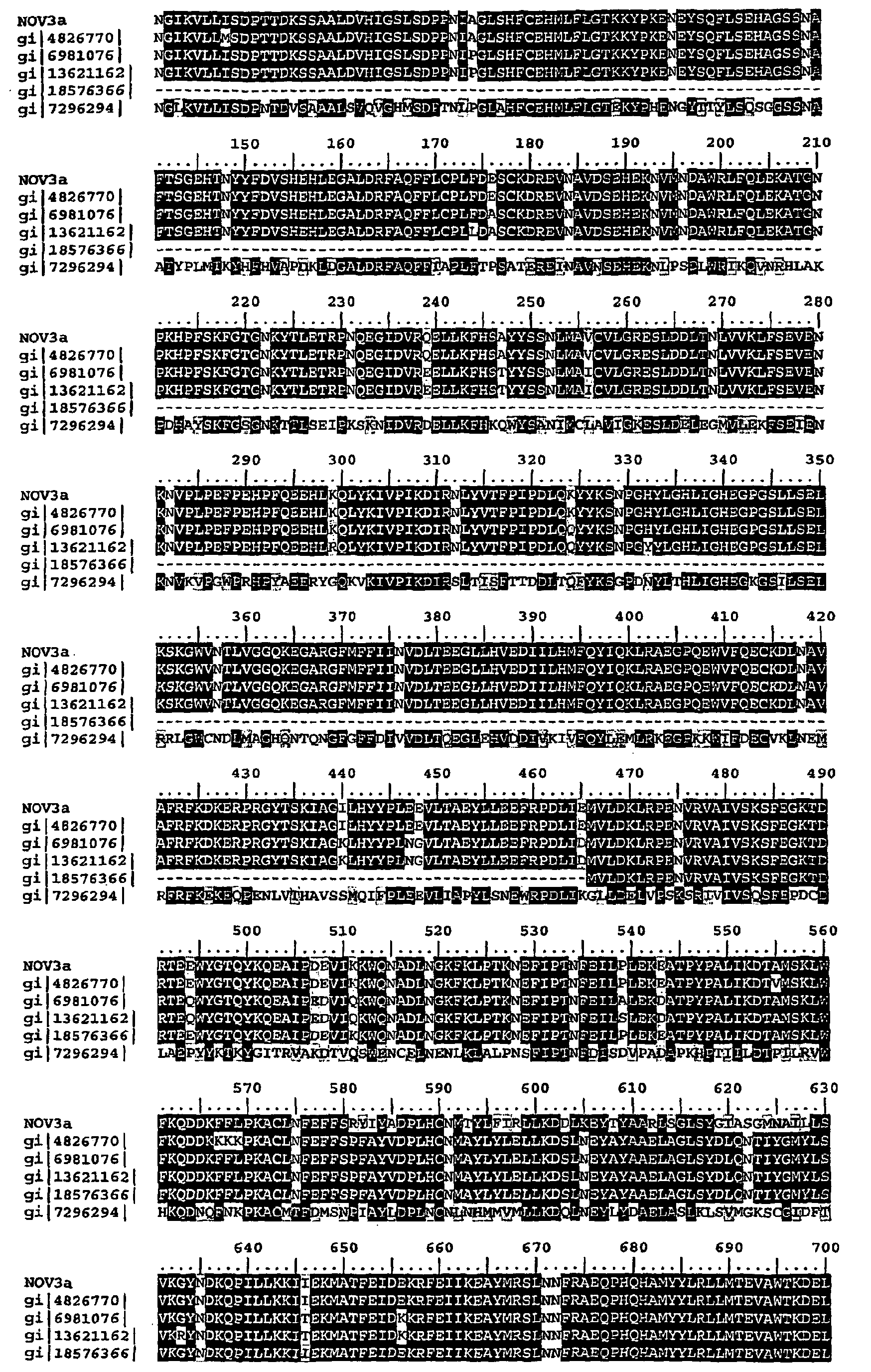

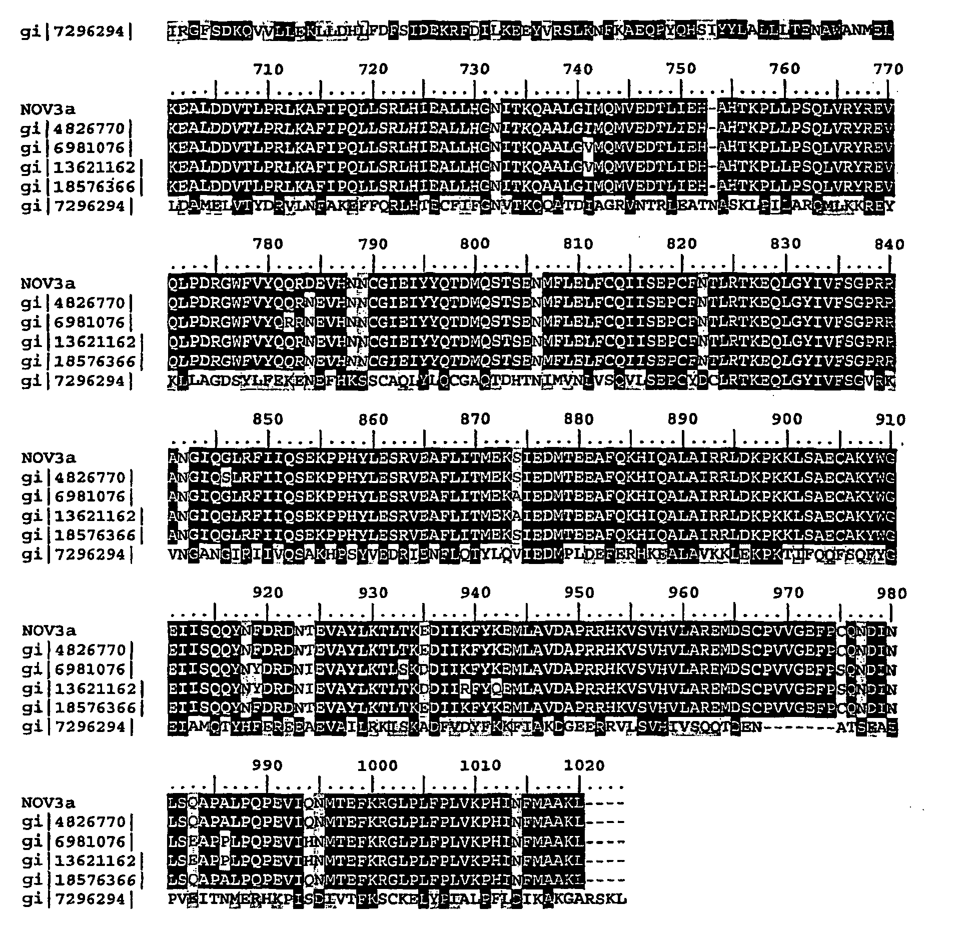

Table 3J lists the domain description from DOMAIN analysis results against NOV3a. This indicates that the NOV3a sequence has properties similar to those of other proteins known to contain these domains.

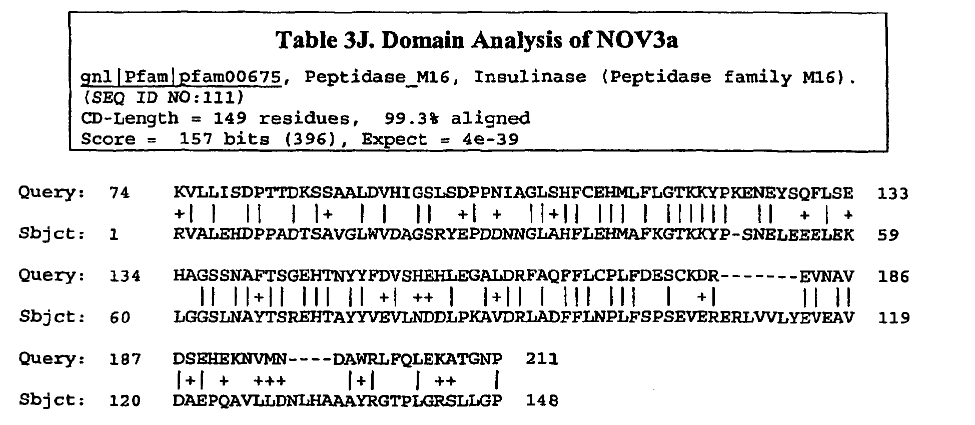

After binding to its receptor on the cell surface, insulin is internalized by receptor-mediated endocytosis and degraded within components of the endosomal apparatus. Degradation of insulin is important in the termination of signaling and clearance of the circulating hormone. It has been proposed that insulin-degrading enzyme (IDE), an evolutionarily conserved, neutral thiol-metalloendopeptidase, plays a crucial role in the degradation of internalized insulin in many types of cells. Despite the substantial evidence supporting the importance of IDE in cellular insulin degradation, there is controversy over its mode and site of action, mainly because of its cytosolic location. Its physiological location in cells has recently been elucidated through subcellular fractionation of liver parenchyma and through immunofluorescence microscopy of stably transfected Chinese hamster ovary cells that overexpress IDE. These experiments have excluded the presence of the enzyme in endosomes and have defined a peroxisomal location, consistent with the presence of a peroxisomal targeting sequence at the carboxyl terminus of the protein. Recently, researchers have demonstrated the functional significance of peroxisome-associated IDE (type I peroxisomal enzyme) in degrading cleaved leader peptides of peroxisomal proteins targeted by the type II motif. IDE is the first cloned and characterized proteinase to be localized to peroxisomes. Moreover, IDE appears to be a member of a newly identified superfamily of metalloendopeptidases that has an HXXEH active-site motif. Although fundamental questions concerning the biological role of IDE remain, its high degree of evolutionary conservation suggests that it must have important functions and multifaceted biological significance (Authier et al., Clin Invest Med 19(3):149-60, 1996)

Insulin degradation is a regulated process that plays a role in controlling insulin action by removing and inactivating the hormone. Abnormalities in insulin clearance and degradation are present in various pathological conditions including type 2 diabetes and obesity and may be important in producing clinical problems. The uptake, processing, and degradation of insulin by cells is a complex process with multiple intracellular pathways. Most evidence supports IDE as the primary degradative mechanism, but other systems (PDI, lysosomes, and other enzymes) undoubtedly contribute to insulin metabolism. Recent studies support a multifunctional role for IDE, as an intracellular binding, regulatory, and degradative protein. IDE increases proteasome and steroid hormone receptor activity, and this activation is reversed by insulin. This raises the possibility of a direct intracellular interaction of insulin with IDE that could modulate protein and fat metabolism. The recent findings would place intracellular insulin-IDE interaction into the insulin signal transduction pathway for mediating the intermediate effects of insulin on fat and protein turnover (Duckworth et al., Endocr Rev 19(5):608-24, 1998).

A number of proteases dependent on divalent cations for their activity have been shown to belong to one family, on the basis of sequence similarity. These enzymes include those listed below.

Insulinase (EC 3.4.24.56) (also known as insulysin or insulin-degrading enzyme or IDE), a cytoplasmic enzyme which seems to be involved in the cellular processing of insulin, glucagon and other small polypeptides.

Escherichia coli protease III (EC 3.4.24.55) (pitrilysin) (gene ptr), a periplasmic enzyme that degrades small peptides.

Mitochondrial processing peptidase (EC 3.4.24.64) (MPP). This enzyme removes the transit peptide from the precursor form of proteins imported from the cytoplasm across the mitochondrial inner membrane. It is composed of two nonidentical homologous subunits termed alpha and beta. The beta subunit seems to be catalytically active while the alpha subunit has probably lost its activity.

Nardilysin (EC 3.4.24.61) (N-arginine dibasic convertase or NRD convertase) this mammalian enzyme cleaves peptide substrates on the N-terminus of Arg residues in dibasic stretches.

Klebsiella pneumoniae protein pqqF. This protein is required for the biosynthesis of the coenzyme pyrrolo-quinoline-quinone (PQQ). It is thought to be protease that cleaves peptide bonds in a small peptide (gene pqqA) thus providing the glutamate and tyrosine residues necessary for the synthesis of PQQ.

Yeast protein AXL1, which is involved in axial budding.

Eimeria bovis sporozoite developmental protein.

Escherichia coli hypothetical protein yddC and HI1368, the corresponding Haemophilus influenzae protein.

Bacillus subtilis hypothetical protein ymxG. - Caenorhabditis elegans hypothetical proteins C28F5.4 and F56D2.1.

It should be noted that in addition to the above enzymes, this family also includes the core proteins I and II of the mitochondrial bc1 complex (also called cytochrome c reductase or complex III), but the situation as to the activity or lack of activity of these subunits is quite complex. In mammals and yeast, core proteins I and II lack enzymatic activity. In Neurospora crassa and in potato core protein I is equivalent to the beta subunit of MPP. In Euglena gracilis, core protein I seems to be active, while subunit II is inactive.

These proteins do not share many regions of sequence similarity; the most noticeable is in the N-terminal section. This region includes a conserved histidine followed, two residues later by a glutamate and another histidine. In pitrilysin, it has been shown that this H-x-x-E-H motif is involved in enzyme activity; the two histidines bind zinc and the glutamate is necessary for catalytic activity. Non active members of this family have lost from one to three of these active site residues. A signature pattern has been developed that detect active , members of this family as well as some inactive members.

The NOV3 nucleic acid of the invention encoding a Insulysin-like protein includes the nucleic acid whose sequence is provided in Tables 3A and 3C, or a fragment thereof. The invention also includes a mutant or variant nucleic acid any of whose bases may be changed from the corresponding base shown in Tables 3A and 3C while still encoding a protein that maintains its Insulysin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 3% of the residues may be so changed.

The NOV3 protein of the invention includes the Insulysin-like protein whose sequence is provided in Tables 3B and 3D. The invention also includes a mutant or variant protein any of whose residues may be changed from the corresponding residue shown in Tables 3B and 3D while still encoding a protein that maintains its Insulysin-like activities and physiological functions, or a functional fragment thereof. In the mutant or variant protein, up to about 4% of the bases may be so changed.

The NOV3 nucleic acids and proteins of the invention are useful in potential diagnostic and therapeutic applications implicated in various diseases and disorders described below and/or other pathologies. For example, the compositions of the present invention will have efficacy for treatment of patients suffering from: Cardiomyopathy, Atherosclerosis, Hypertension, Congenital heart defects, Aortic stenosis, Atrial septal defect (ASD), Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity,Transplantation, Endometriosis, Fertility, Von Hippel-Lindau (VHL) syndrome, Cirrhosis, Transplantation, Hemophilia, Hypercoagulation, Idiopathic thrombocytopenic purpura, Immunodeficiencies, Retinitis pigmentosa, autosomal dominant; Retinitis pigmentosa, autosomal recessive; SEMD, Pakistani type; Urofacial syndrome; Alzheimer disease 6; Cholesteryl ester storage disease; Corneal dystrophy, Thiel-Behnke type; Dubin-Johnson syndrome; Leukemia, T-cell acute lymphocytic; Leukemia, T-cell acute lymphocytic; Spinocerebellar ataxia, infantile-onset, with sensory neuropathy; Split hand/foot malformation, type 3; Tolbutamide poor metabolizer; Warfarin sensitivity; Wolman disease; Anterior segment mesenchymal dysgenesis and cataract; Cataract, congenital; Neurofibrosarcoma; Diabetes mellitus, insulin-dependent, 17; Diabetes mellitus, insulin-dependent, 17; obesity, insulin resistance, Graft vesus host and other diseases, disorders and conditions of the like.

NOV3 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV3 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV4

NOV4 includes two novel Integrin Beta-7 Precursor-like proteins disclosed below. The disclosed proteins have been named NOV4a and NOV4b.

### NOV4a

A disclosed NOV4a nucleic acid of 2798 nucleotides (designated CuraGen Acc. No. CG57185-01) encoding a novel Integrin Beta-7 Precursor-like protein is shown in Table 4A. An open reading frame was identified beginning with an ATG initiation codon at nucleotides 152-154 and ending with a TAA codon at nucleotides 2545-2547. Putative untranslated regions upstream from the initiation codon and downstream from the termination codon are underlined in Table 4A, and the start and stop codons are in bold letters.

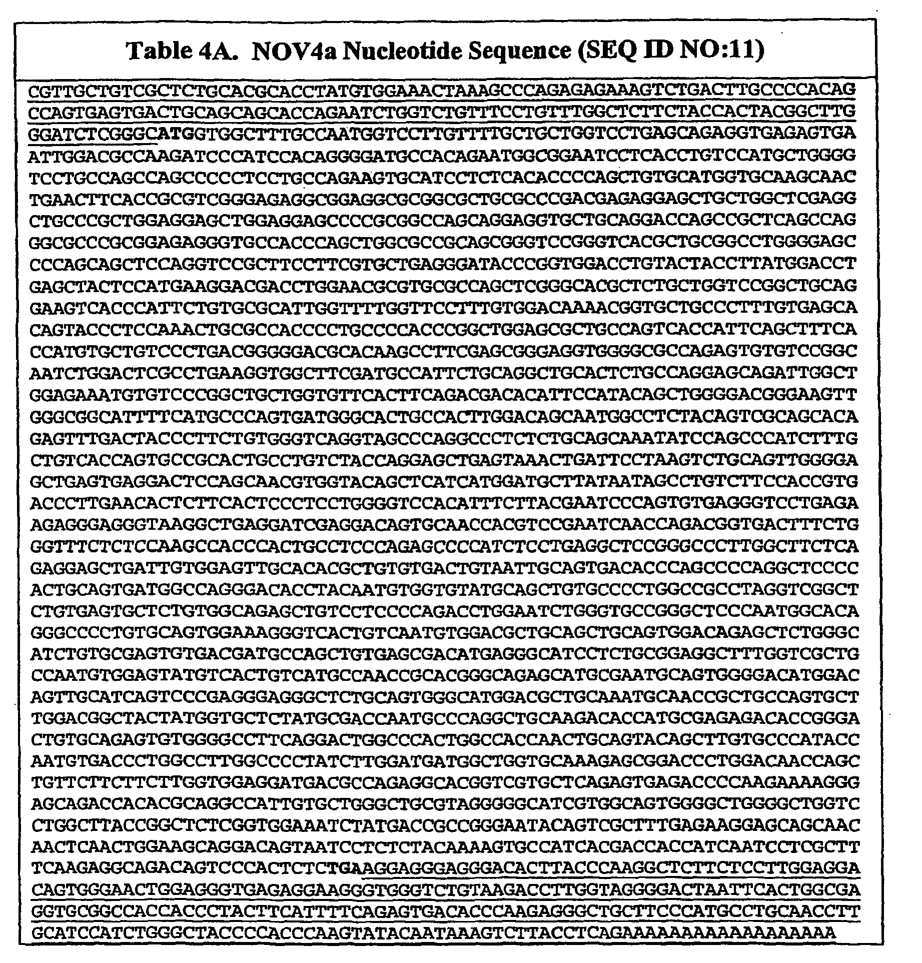

A NOV4a polypeptide (SEQ ID NO:12) encoded by SEQ ID NO:11 is 798 amino acid residues and is presented using the one letter code in Table 4B.

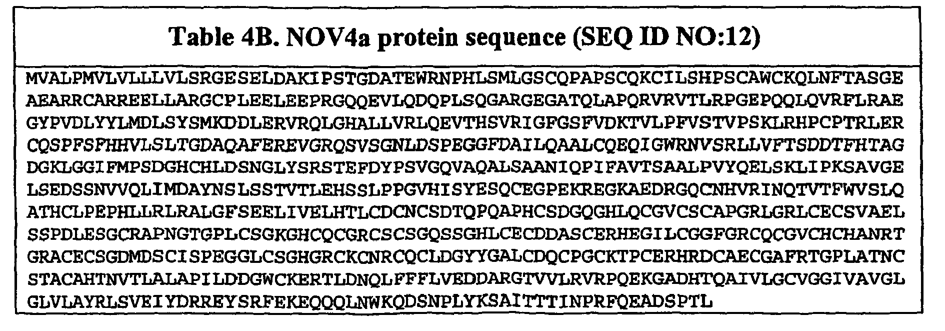

### NOV4b

A disclosed NOV4b nucleic acid of 2211 nucleotides (designated CuraGen Acc. No. CG57185-02) encoding a novel Integrin Beta-7 Precursor-like protein is shown in Table 4C. An open reading frame was identified beginning with an ATG initiation codon at nucleotides 13-15 and ending with a TAA codon at nucleotides 1801-1803. Putative untranslated regions upstream from the initiation codon and downstream from the termination codon are underlined in Table 4C, and the start and stop codons are in bold letters.

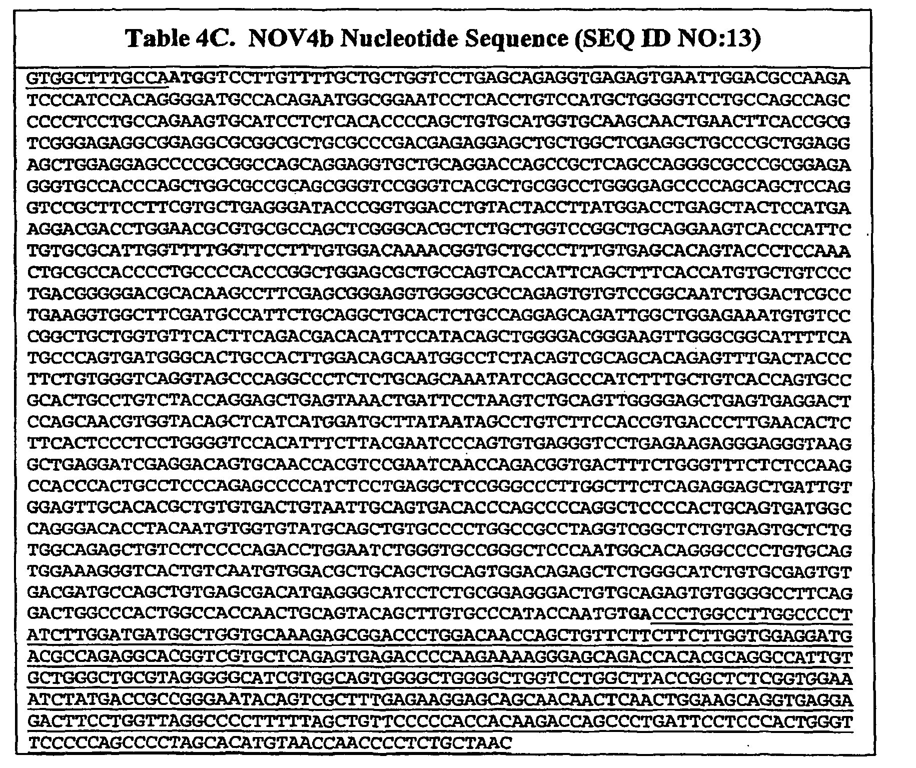

The nucleic acid sequence of NOV4b maps to chromosome 12 and has 1813 of 1881 bases (96%) identical to a gb:GBNBANK-ID:HUMINTB7|acc:M68892.1 mRNA from *Homo sapiens* (Human integrin beta-7 subunit mRNA, complete cds) (E = 0.0).

A NOV4b polypeptide (SEQ ID NO:14) encoded by SEQ ID NO:13 is 596 amino acid residues and is presented using the one letter code in Table 4D. Signal P, Psort and/or Hydropathy results predict that NOV4b contains a signal peptide and is likely to be localized to the mitochondrial matrix space with a certainty of 0.4542 and the mitochondrial intermembrane space with a certainty of 0.4030. The most likely cleavage site for a NOV4b polypeptide is between amino acids 14 and 15: GES-EL.

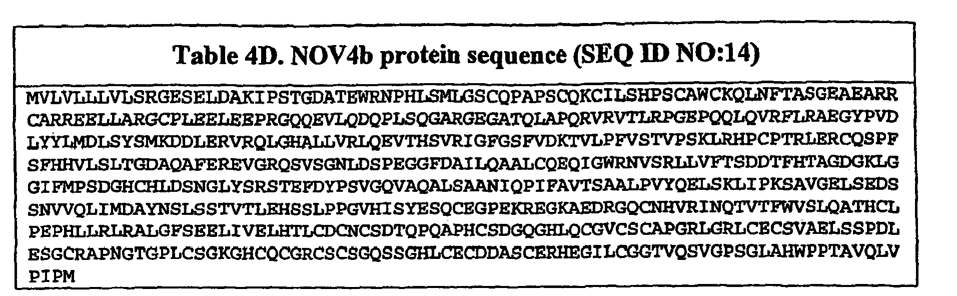

The NOV4b amino acid sequence has 571 of 571 amino acid residues (100%) identical to, and 571 of 571 amino acid residues (100%) similar to, the 798 amino acid residue ptnr:SWISSNEW-ACC:P26010 protein from *Homo sapiens* (Human) (Integrin Beta-7 Precursor) (E = 5.9e⁻³¹⁸).

NOV4b is expressed in at least the following tissues: Colon, Lung, Lymph node, Lymphoid tissue, Peripheral Blood, Placenta, Salivary Glands, Spleen, Thyroid and Tonsils. Expression information was derived from the tissue sources of the sequences that were included in the derivation of NOV4b.

Possible small nucleotide polymorphisms (SNPs) found for NOV4a are listed in Table 4E.

| **Table 4E: SNPs** | | | | |
|---|---|---|---|---|
| **Variant** | **Nucleotide Position** | **Base Change** | **Amino Acid Position** | **Base Change** |
| 13377057 | 423 | G > A | 91 | Cys > Tyr |
| 13377058 | 1133 | C > T | 328 | Pro > Ser |
| 13377059 | 1261 | G > A | Silent | N/A |
| 13377056 | 2126 | A> G | 659 | Thr > Ala |
| 13377055 | 2241 | T > C | 697 | Leu > Pro |
| 13377054 | 2285 | C > T | 712 | Leu > Phe |
| 13377053 | 2303 | G > A | 718 | Glu > Lys |
| 13377052 | 2414 | C > T | 755 | Arg > Cys |
| 13377051 | 2443 | G > A | Silent | N/A |
| 13377050 | 2447 | C > T | 766 | Gln > End |

NOV4a and NOV4b are very closely homologous as is shown in the amino acid alignment in Table 4F.

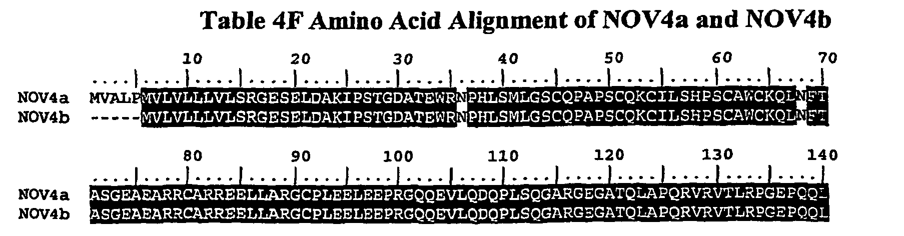

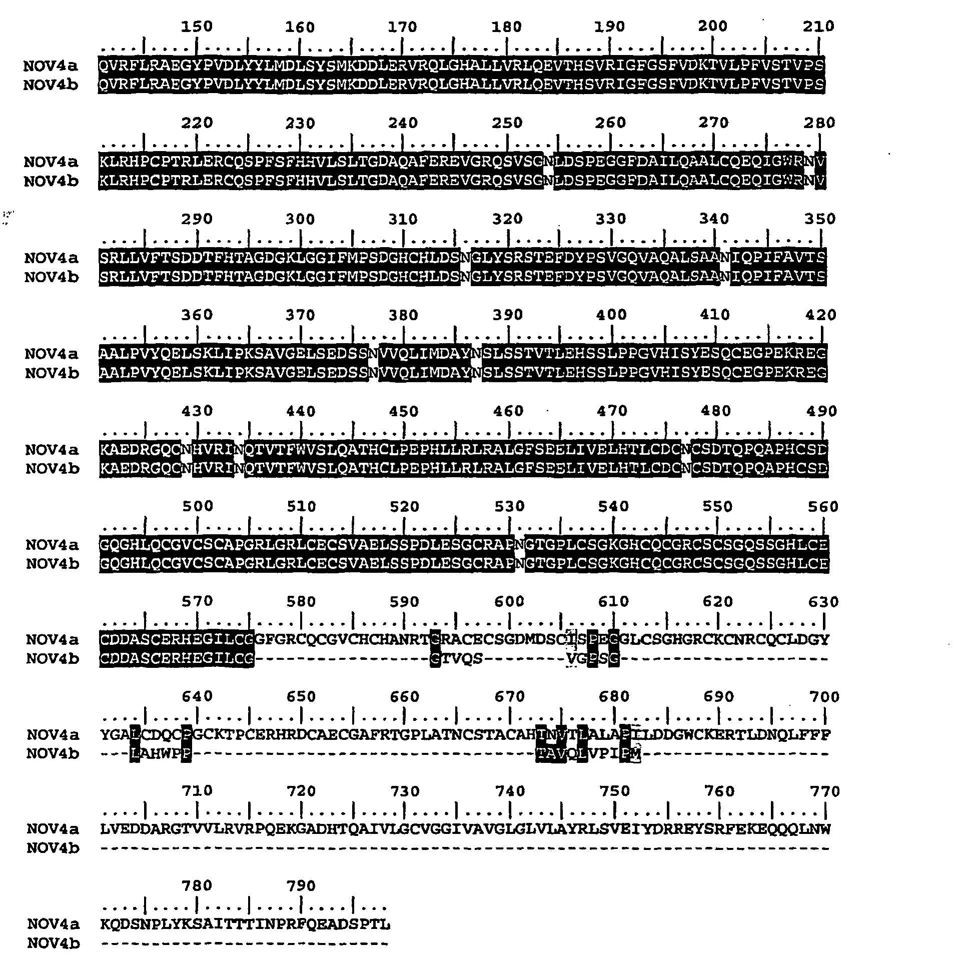

Homologies to any of the above NOV4 proteins will be shared by the other NOV4 proteins insofar as they are homologous to each other as shown above. Any reference to NOV4 is assumed to refer to both of the NOV4 proteins in general, unless otherwise noted.

NOV4a also has homology to the amino acid sequences shown in the BLASTP data listed in Table 4G.

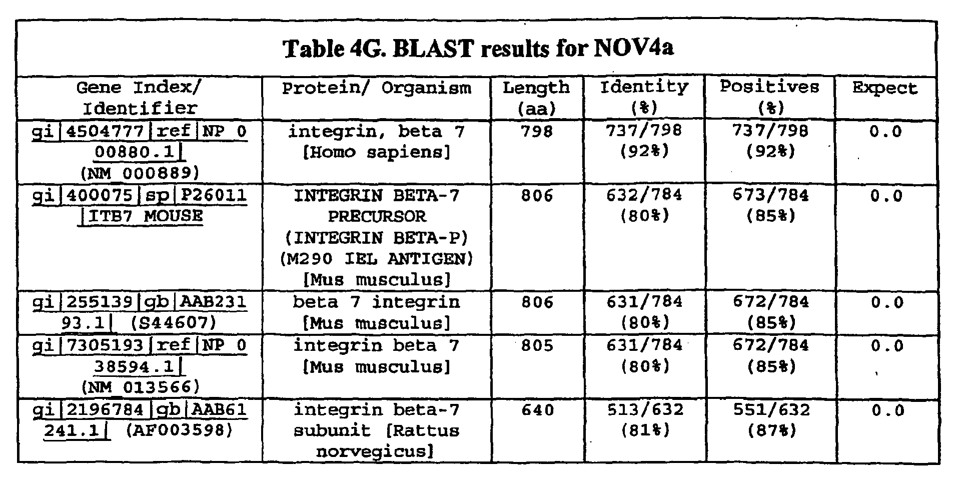

The homology of these sequences is shown graphically in the ClustalW analysis shown in Table 4H.

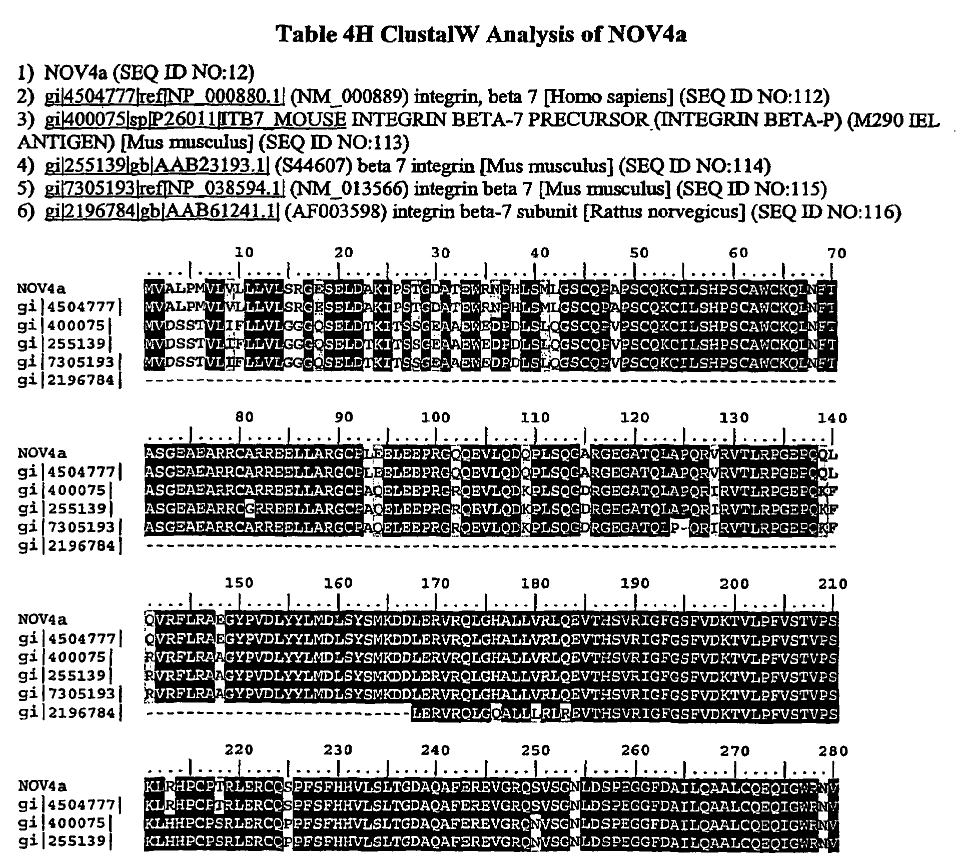

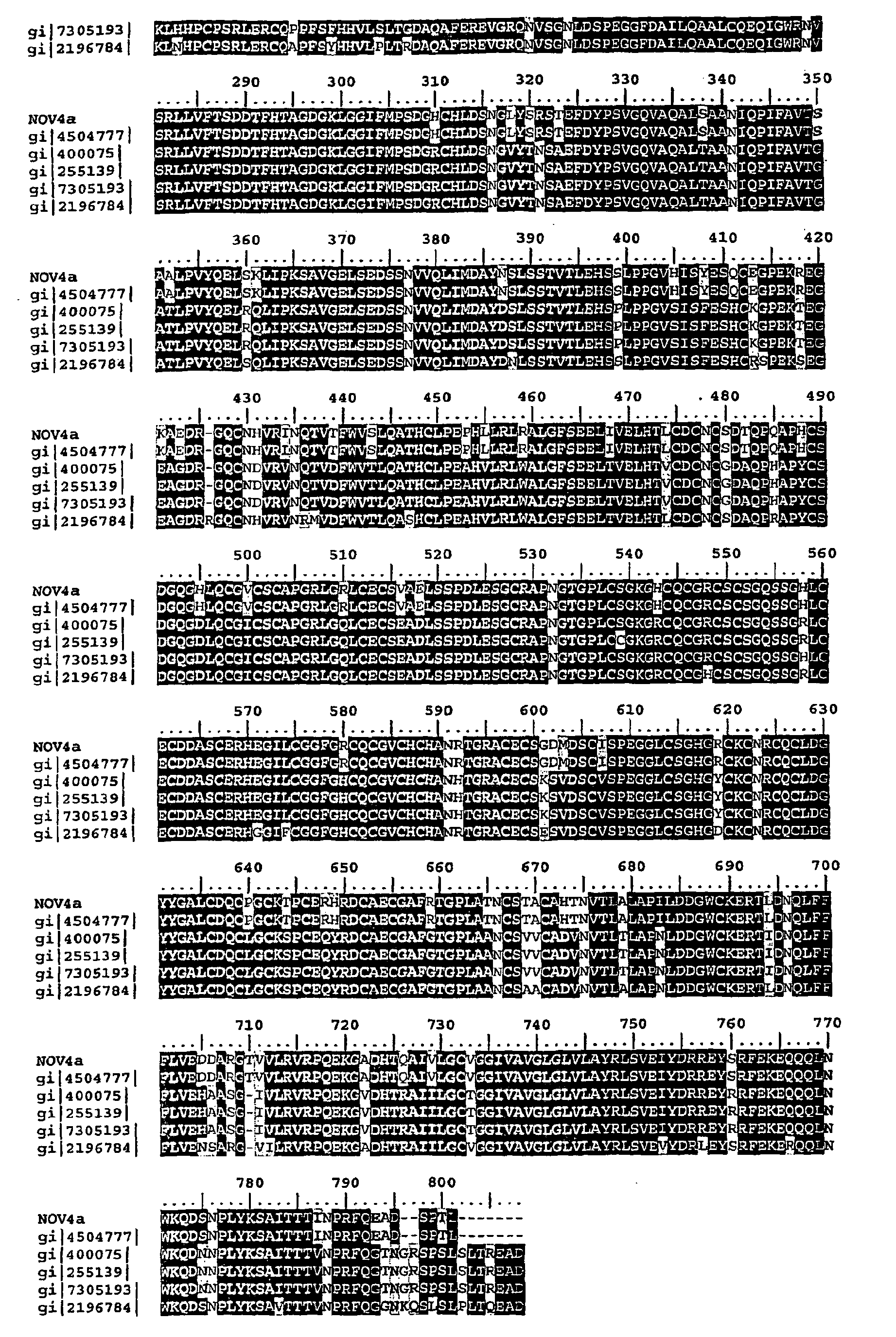

Tables 4I and 4J lists the domain description from DOMAIN analysis results against NOV4a. This indicates that the NOV4a sequence has properties similar to those of other proteins known to contain these domains.

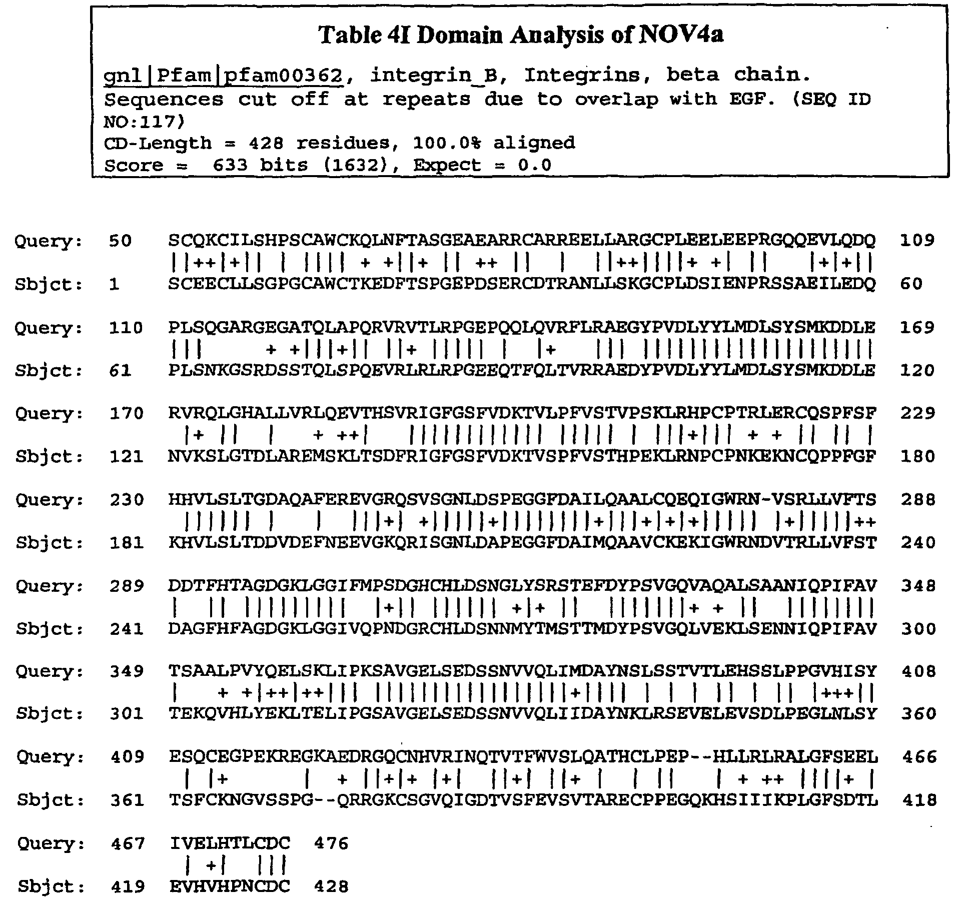

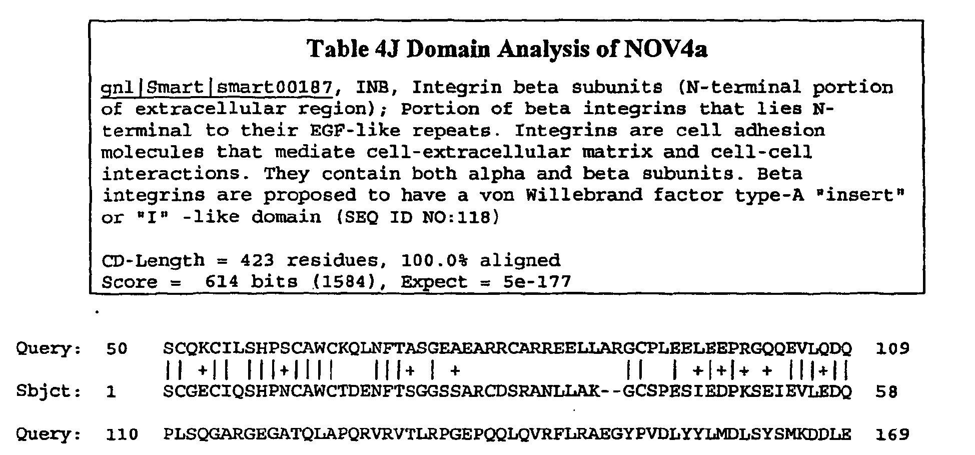

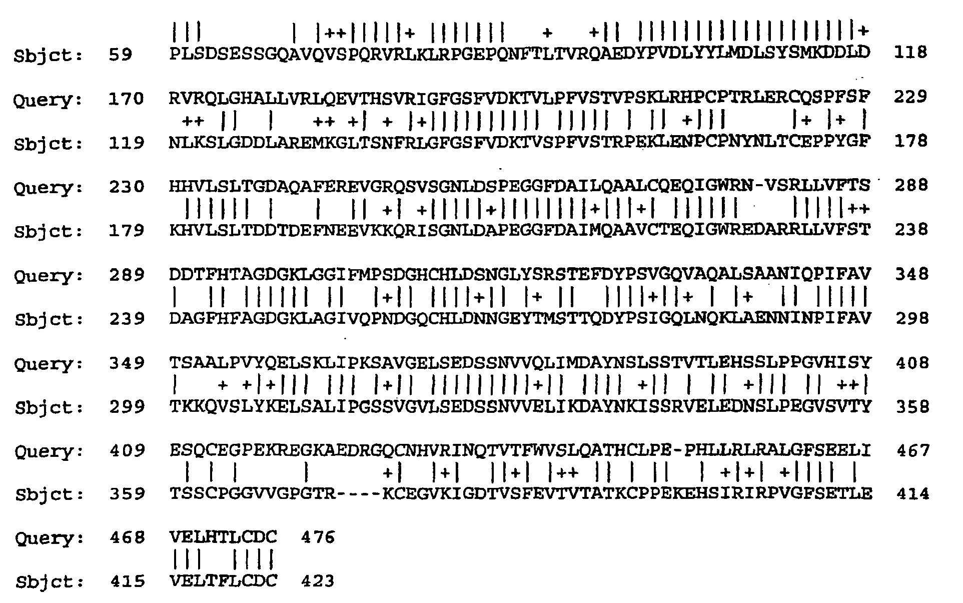

The integrins are a large family of heterodimeric cell-surface glycoproteins that play key roles in the adherence of cells to other cells and to extracellular matrix proteins. Leukocytes express a variety of integrins that are crucial participants in the inflammatory and immune responses. All integrins are composed of 1 alpha subunit and 1 beta subunit that are translated separately and are noncovalently associated. The cDNA sequences of 6 mammalian beta subunits, numbered beta-1 through beta-6, had previously been reported. Erle et al. (J. Biol. Chem. 266: 11009-11016, 1991) determined the complete sequence of a novel integrin beta subunit from leukocytes, designated beta-7. The cDNA contained a single large open reading frame predicted to encode a 798-amino acid protein precursor (signal peptide plus mature protein). Like other beta-subunit proteins, it was predicted to contain a large extracellular domain, a transmembrane domain, and a cytoplasmic tail. The deduced beta-7 amino acid sequence was 32 to 46% identical to the 6 previously sequenced subunits. It was most similar to the leukocyte integrin common beta subunit (ITGB2; CD18; 116920). Erle et al. (1991) predicted that integrin beta-7 plays a role in adhesive interactions of leukocytes. Krissansen et al. (Immunogenetics 35: 58-61, 1992) assigned the ITGB7 gene to human chromosome 12 by analysis of somatic cell hybrids. Krissansen et al. (1992) commented on the fact that the gene encoding the alpha subunit of the fibronectin receptor (FNRA; 135620) resides on chromosome 12 also. They stated that the 'extent and evolutionary significance of linkages between integrin genes...remains to be determined.' Baker et al. (Mammalian Genome 2: 272-273, 1992) assigned the ITGB7 gene to 12q13.13 by nonisotopic in situ hybridization. Yuan et al. (Immunogenetics 35: 403-407, 1992) mapped the mouse homolog Itgb-7 to chromosome 15 by study of mouse-hamster somatic cell hybrids and by interspecific backcrosses.

The NOV4 nucleic acid of the invention encoding a Integrin Beta-7 Precursor-like protein includes the nucleic acid whose sequence is provided in Tables 4A and 4C, or a fragment thereof. The invention also includes a mutant or variant nucleic acid any of whose bases may be changed from the corresponding base shown in Tables 4A and 4C while still encoding a protein that maintains its Integrin Beta-7 Precursor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 4% of the residues may be so changed.

The NOV4 protein of the invention includes the Integrin Beta-7 Precursor-like protein whose sequence is provided in Tables 4B and 4D. The invention also includes a mutant or variant protein any of whose residues may be changed from the corresponding residue shown in Tables 4B and 4D while still encoding a protein that maintains its Integrin Beta-7 Precursor-like activities and physiological functions, or a functional fragment thereof.

The NOV4 nucleic acids and proteins of the invention are useful in potential diagnostic and therapeutic applications implicated in various diseases and disorders described below and/or other pathologies. For example, the compositions of the present invention will have efficacy for treatment of patients suffering from: systemic lupus erythematosus, autoimmune disease, asthma, emphysema, scleroderma, allergy, ARDS; atherosclerosis, thrombosis, cancer, asthma, lymphedema; fertility, xerostomia, hyperthyroidism, hypothyroidism and other diseases, disorders and conditions of the like.

NOV4 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV4 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV5

A disclosed NOV5 nucleic acid of 1360 nucleotides (also referred to as CG57360-01) encoding a novel membrane protein-like protein is shown in Table 5A. An open reading frame was identified beginning with an ATG initiation codon at nucleotides 244-246 and ending with a TGA codon at nucleotides 1282-1284. Putative untranslated regions upstream from the intitation codon and downstream from the termination codon are underlined in Table 5A, and the start and stop codons are in bold letters.

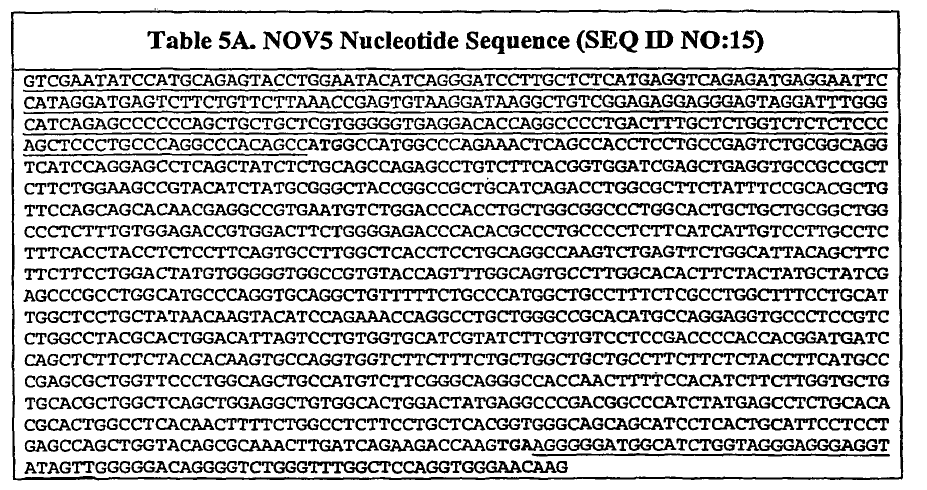

The NOV5 nucleic acid was identified on chromosome 1 and has 308 of 508 bases (60%) identical to a gb:GENBANK-ID:RNU87960|acc:U87960.1 mRNA from *Rattus norvegicus* (Rattus norvegicus leukocyte common antigen receptor (LAR) gene, trans-spliced alternative untranslated exon) (E = 1.5e⁻⁰⁶).

A disclosed NOV5 polypeptide (SEQ ID NO:16) encoded by SEQ ID NO:15 is 346 amino acid residues and is presented using the one-letter code in Table 5B. Signal P, Psort and/or Hydropathy results predict that NOV5 does not contain a signal peptide and is likely to be localized at the plasma membrane with a certainty of 0.6000 and the Golgi body with a certainty of 0.4000.

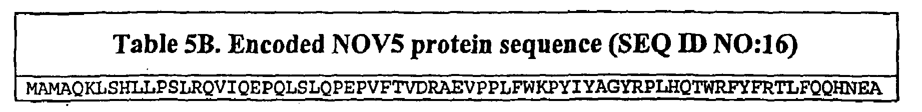

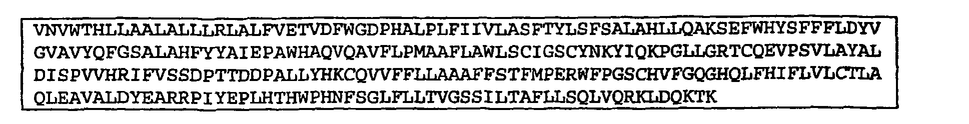

The NOV5 amino acid sequence has 171 of 349 amino acid residues (48%) identical to, and 230 of 349 amino acid residues (65%) similar to, the 359 amino acid residue ptnr:TREMBLNEW-ACC:CAC05478 protein from Homo sapiens (Human) (DJ304B14.1 (NOVEL PROTEIN) (E = 3.4e⁻⁸⁴).

NOV5 is expressed in at least the following tissues: Adrenal Gland/Suprarenal gland, Bone Marrow, Kidney, Liver, Prostate and Testis. This information was derived by determining the tissue sources of the sequences that were included in the invention including but not limited to SeqCalling sources, Public EST sources, genomic clone sources, literature sources, and/or RACE sources. The NOV5 sequence is also predicted to be expressed in the following tissues because of the expression pattern of (GENBANK-ID: gb:GENBANK-ID:RNU87960|acc:U87960.1) a closely *related Rattus norvegicus* leukocyte common antigen receptor (LAR) gene, trans-spliced alternative untranslated exon homolog in species *Rattus norvegicus:* leukocyte.

Possible small nucleotide polymorphisms (SNPs) found for NOV5 are listed in Table 5C.

| **Table 5C: SNPs** | | | | |
|---|---|---|---|---|
| **Variant** | **Nucleotide Position** | **Base Change** | **Amino Acid Position** | **Base Change** |
| 13377060 | 967 | A>G | 242 | Lys > Glu |
| 13377061 | 1233 | A>G | Silent | N/A |

NOV5 has homology to the amino acid sequences shown in the BLASTP data listed in Table 5D.

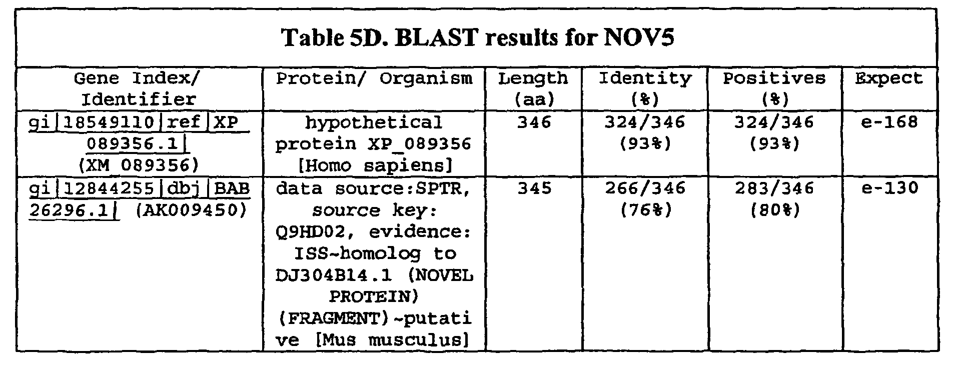

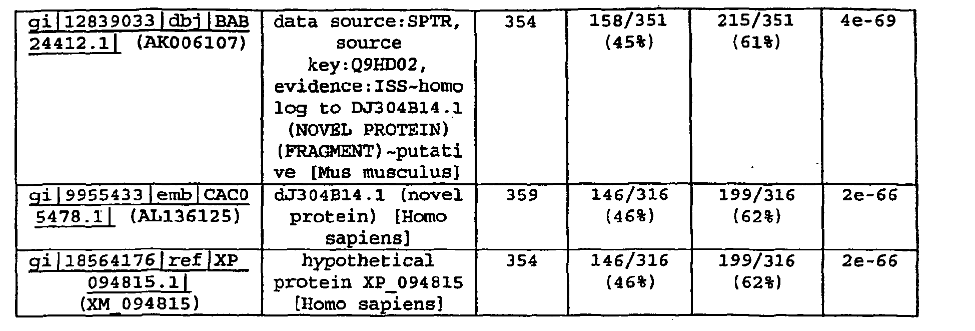

The homology of these sequences is shown graphically in the ClustalW analysis shown in Table 5E.

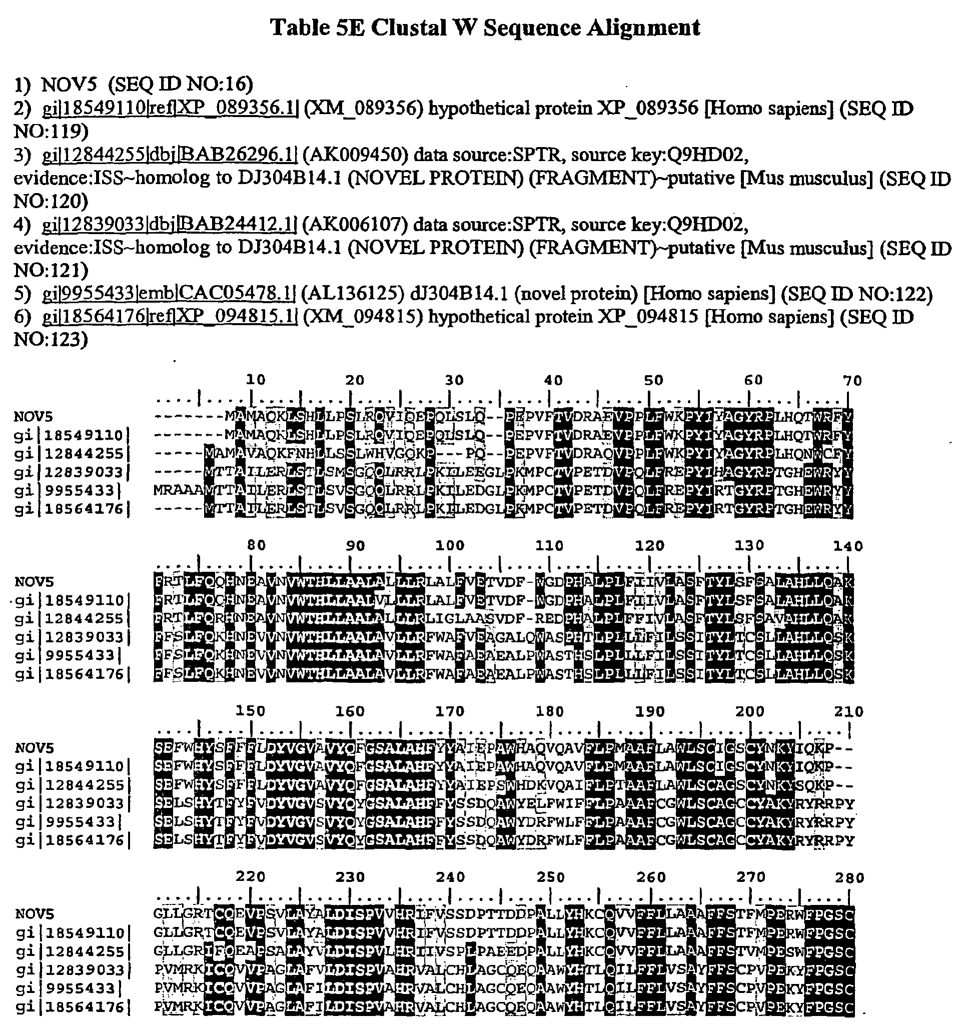

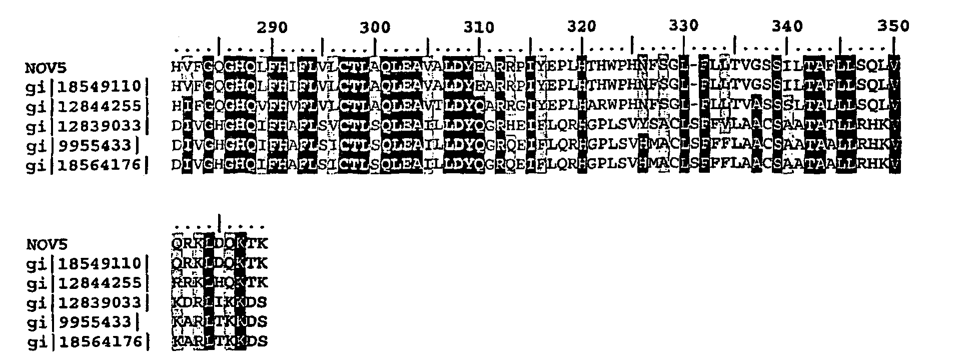

Table 5F list the domain description from DOMAIN analysis results against NOV5. This indicates that the NOV5 sequence has properties similar to those of other proteins known to contain these domains.

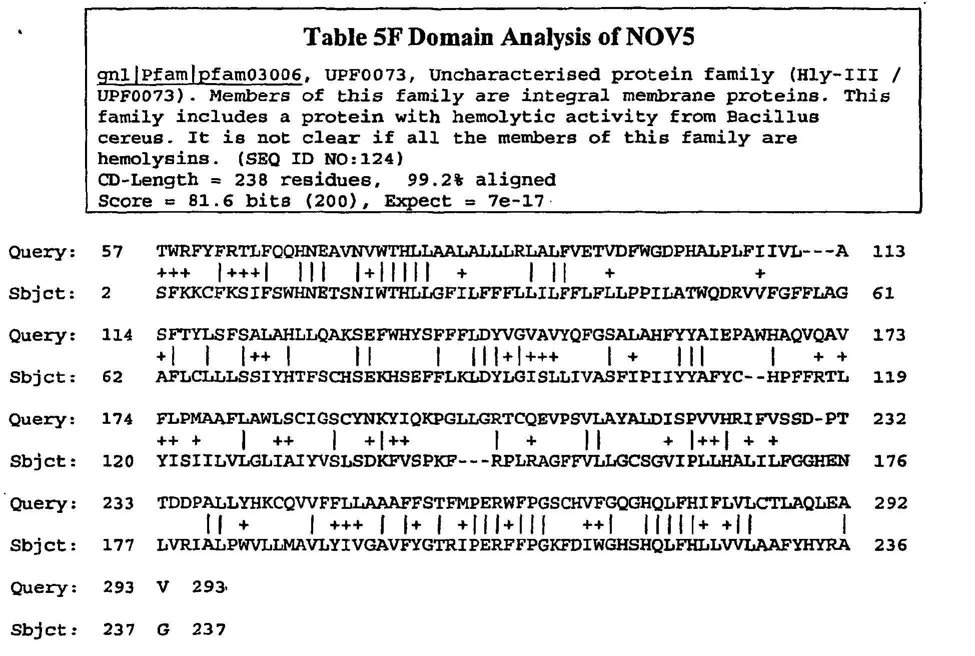

The NOV5 nucleic acid of the invention encoding a membrane protein-like protein includes the nucleic acid whose sequence is provided in Table 5A, or a fragment thereof. The invention also includes a mutant or variant nucleic acid any of whose bases may be changed from the corresponding base shown in Table 5A while still encoding a protein that maintains its membrane protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 40% of the residues may be so changed.

The NOV5 protein of the invention includes the membrane protein-like protein whose sequence is provided in Table 5B. The invention also includes a mutant or variant protein any of whose residues may be changed from the corresponding residue shown in Table 5B while still encoding a protein that maintains its membrane protein-like activities and physiological functions, or a functional fragment thereof. In the mutant or variant protein, up to about 52% of the bases may be so changed.

The NOV5 nucleic acids and proteins of the invention are useful in potential diagnostic and therapeutic applications implicated in various diseases and disorders described below and/or other pathologies. For example, the compositions of the present invention will have efficacy for treatment of patients suffering from: cancer, trauma, regeneration (in vitro and in vivo), viral/bacterial/parasitic infections, adrenoleukodystrophy , congenital adrenal hyperplasia, Von Hippel-Lindau (VHL) syndrome, cirrhosis, transplantation, hemophilia, hypercoagulation, idiopathic thrombocytopenic purpura, autoimmune disease, allergies, immunodeficiencies, fertility, hypogonadism, diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome and other diseases, disorders and conditions of the like.

NOV5 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV5 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV6

A disclosed NOV6 nucleic acid of 3509 nucleotides (also referred to as CG57362-01) encoding a novel BCSC-1-like protein is shown in Table 6A. An open reading frame was identified beginning with an ATG initiation codon at nucleotides 155-157 and ending with a TGA codon at nucleotides 3206-3208. Putative untranslated regions upstream from the intitation codon and downstream from the termination codon are underlined in Table 6A, and the start and stop codons are in bold letters.

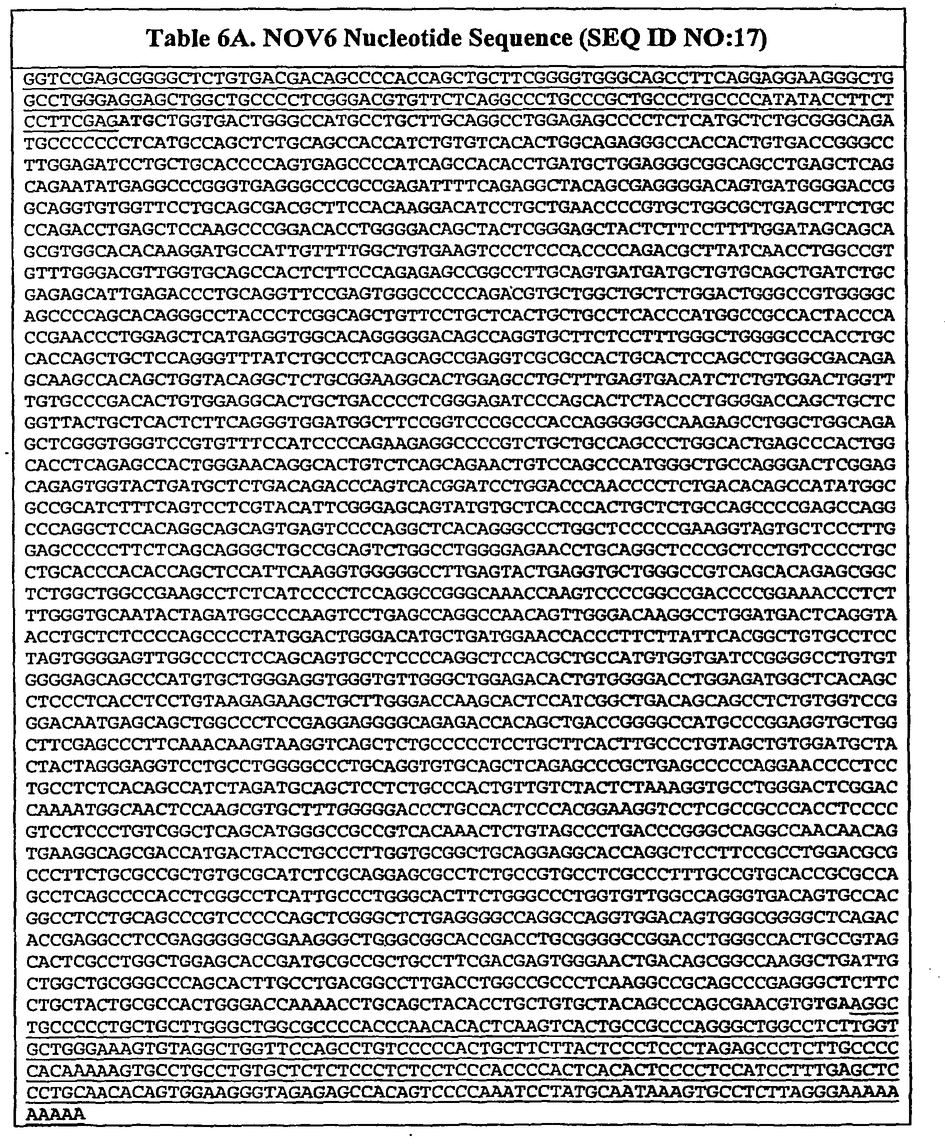

The NOV6 nucleic acid was identified on chromosome 3 and has 2669 of 2799 bases (95%) identical to a gb:GENBANK-ID:AB047829|acc:AB047829.1 mRNA from *Macaca fascicularis* (*Macaca fascicularis* brain cDNA, clone:QccE-10361) (E = 0.0).

A disclosed NOV6 polypeptide (SEQ ID NO:18) encoded by SEQ ID NO:17 is 1017 amino acid residues and is presented using the one-letter code in Table 6B. Signal P, Psort and/or Hydropathy results predict that NOV6 contains a signal peptide and is likely to be localized to the nucleus with a certainty of 0.6000. The most likely cleavage site is between amino acidsl8 and 19: SHA-LR.

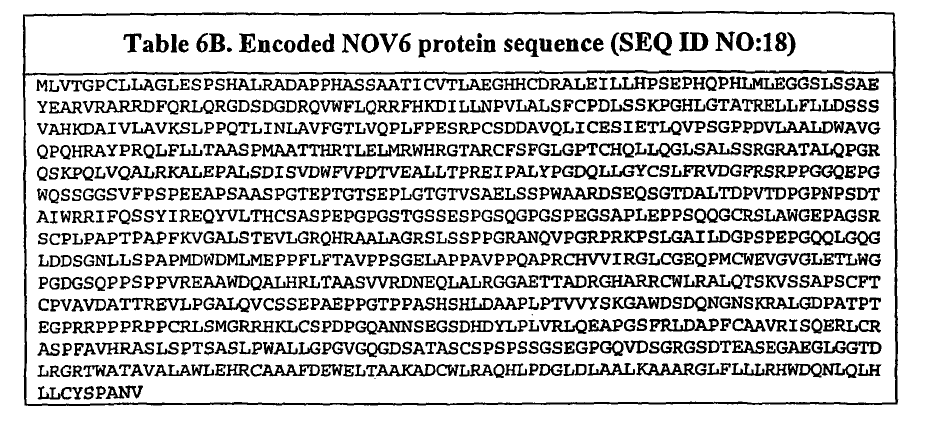

The NOV6 amino acid sequence has 767 of 803 amino acid residues (95%) identical to, and 779 of 803 amino acid residues (97%) similar to, the 803 amino acid residue ptnr:TREMBLNEW-ACC:BAB12255 protein from *Macaca fascicularis* (Crab eating macaque) (Cynomolgus monkey) (Hypothetical 84.7 Kda Protein) (E = 0.0).

NOV6 is expressed in at least the following tissues: lung. This information was derived by determining the tissue sources of the sequences that were included in the invention including but not limited to SeqCalling sources, Public EST sources, genomic clone sources, literature sources, and/or RACE sources. The NOV6 sequence is also predicted to be expressed in the following tissues because of the expression pattern of (GENBANK-ID: gb:GENBANK-ID:AB047829|acc:AB047829.1) a closely related *Macaca fascicularis* brain cDNA, clone:QccE-10361 homolog in species *Macaca fascicularis:* brain.

Possible small nucleotide polymorphisms (SNPs) found for NOV6 are listed inTable 6C.

| **Table 6C: SNPs** | | | | |
|---|---|---|---|---|
| **Variant** | **Nucleotide Position** | **Base Change** | **Amino Acid Position** | **Base Change** |
| 13377063 | 3196 | A > G | Silent | N/A |

NOV6 has homology to the amino acid sequences shown in the BLASTP data listed in Table 6D.

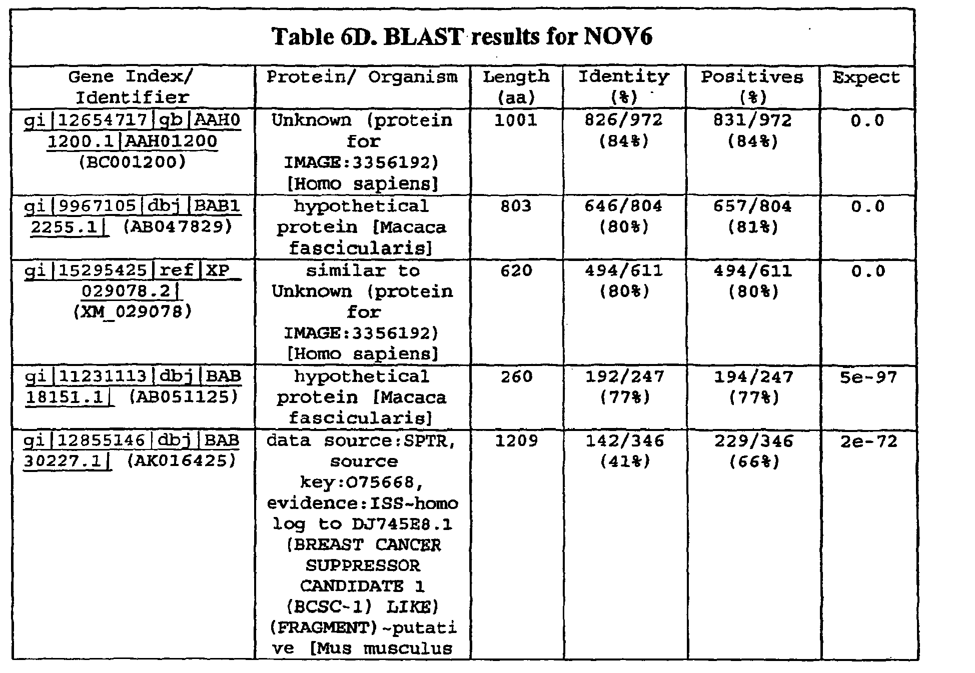

The homology of these sequences is shown graphically in the ClustalW analysis shown in Table 6E.

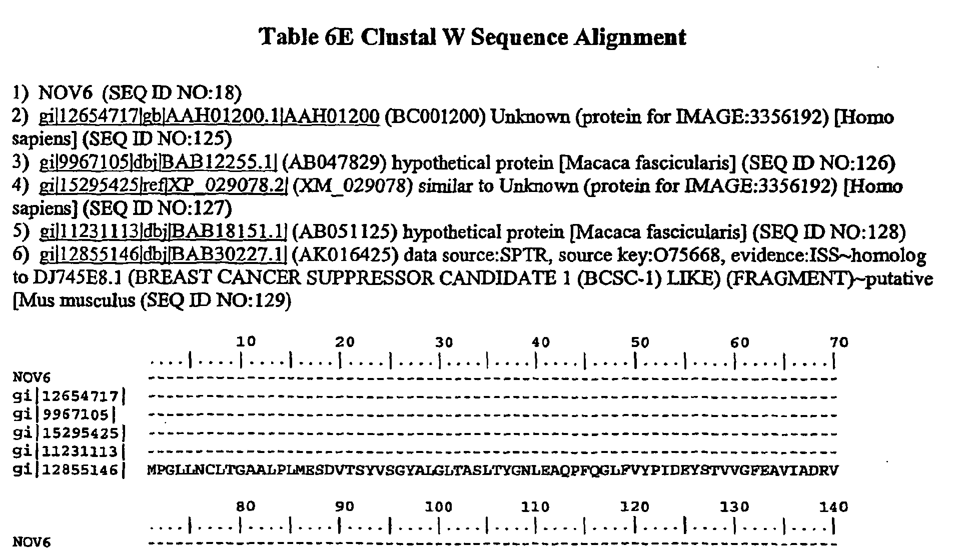

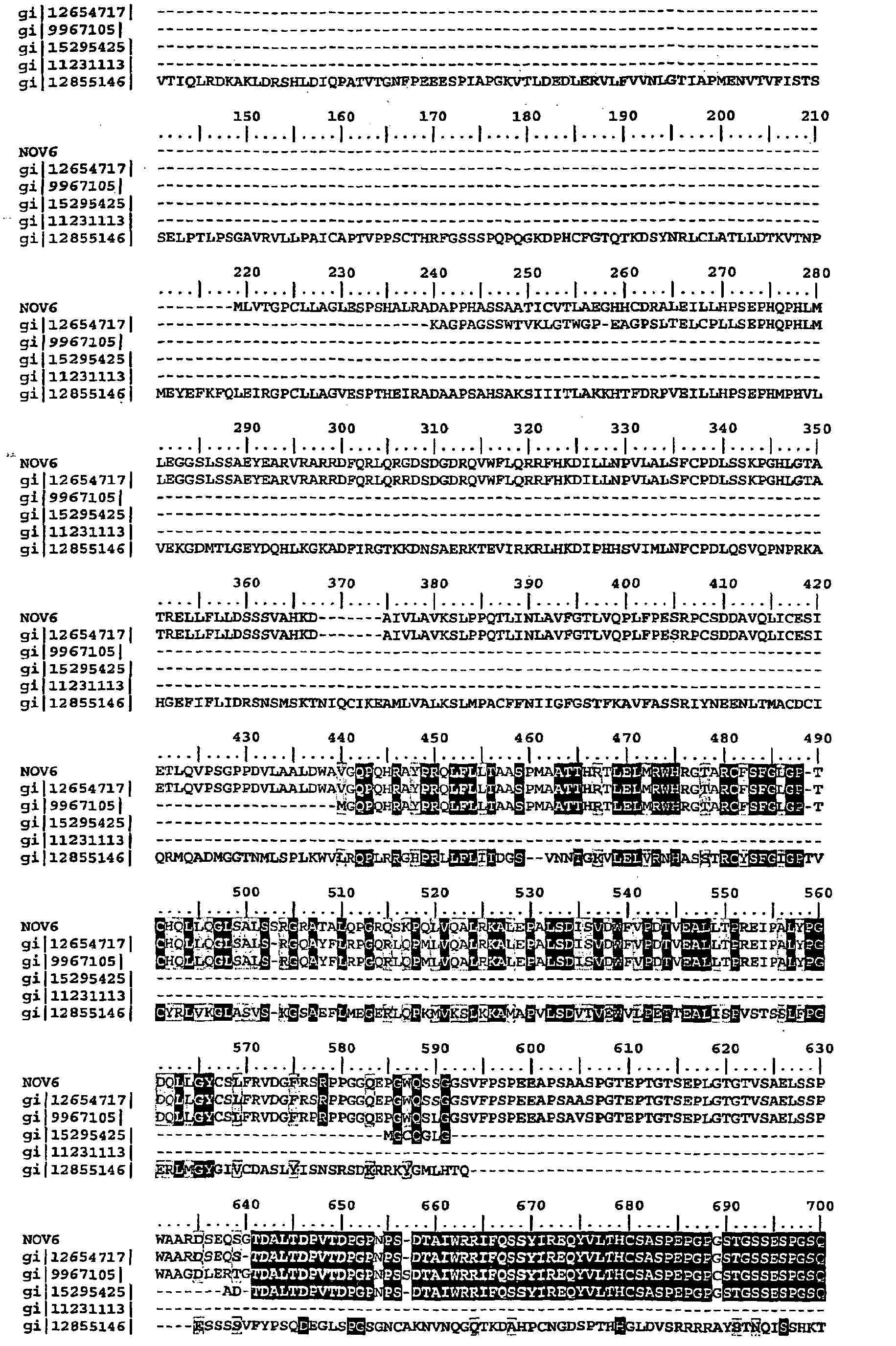

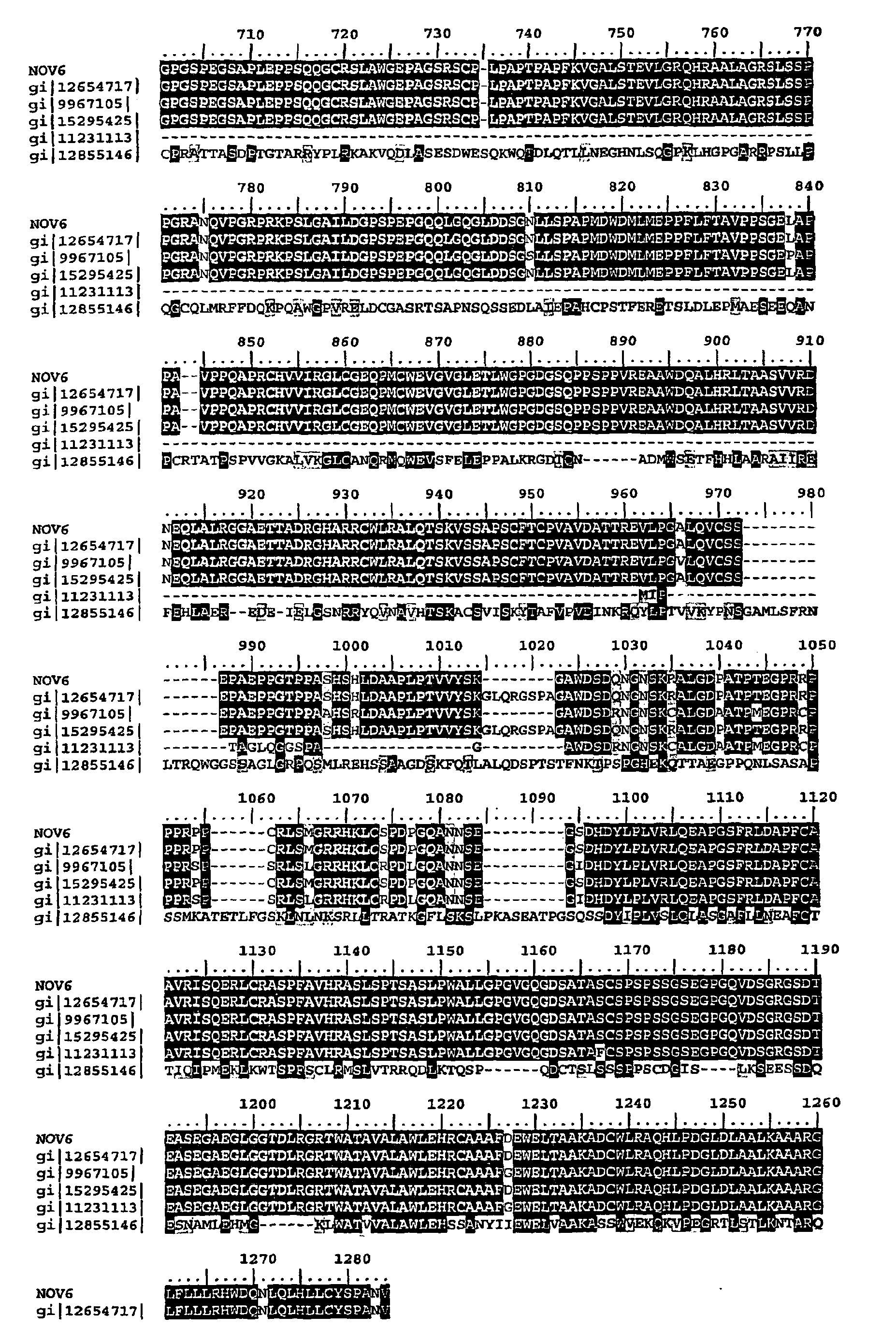

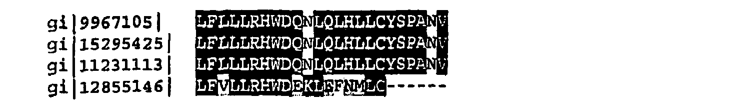

Table 6F list the domain description from DOMAIN analysis results against NOV6. This indicates that the NOV6 sequence has properties similar to those of other proteins known to contain these domains.

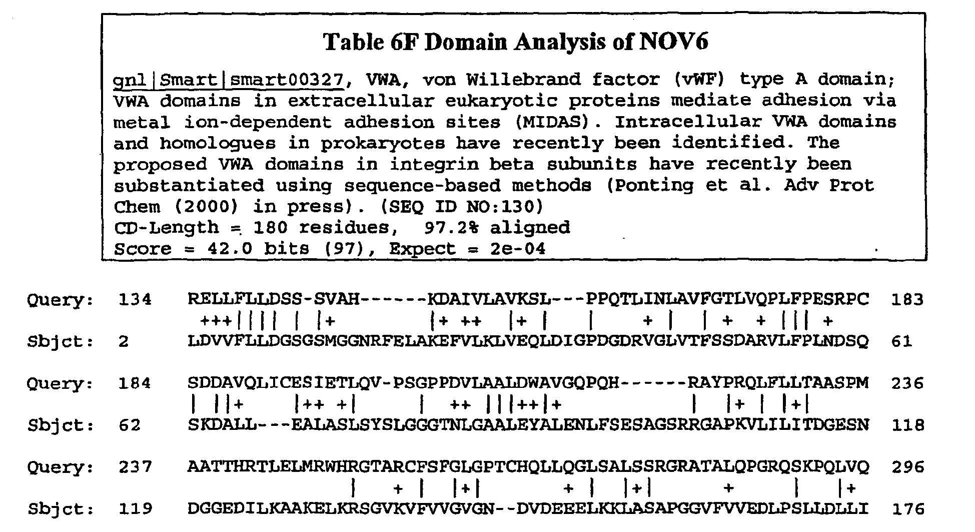

NOV6 described herein bears resemblance to BCSC-1 a molecule that is a candidate for suppression of breast cancer and this molecule may play a similar role in the lung and brain.

The NOV6 nucleic acid of the invention encoding a BCSC-1-like protein includes the nucleic acid whose sequence is provided in Table 6A, or a fragment thereof. The invention also includes a mutant or variant nucleic acid any of whose bases may be changed from the corresponding base shown in Table 6A while still encoding a protein that maintains its BCSC-1-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5% of the residues may be so changed.

The NOV6 protein of the invention includes the BCSC-1-like protein whose sequence is provided in Table 6B. The invention also includes a mutant or variant protein any of whose residues may be changed from the corresponding residue shown in Table 6B while still encoding a protein that maintains its BCSC-1-like activities and physiological functions, or a functional fragment thereof. In the mutant or variant protein, up to about 5% of the bases may be so changed.

The NOV6 nucleic acids and proteins of the invention are useful in potential diagnostic and therapeutic applications implicated in various diseases and disorders described below and/or other pathologies. For example, the compositions of the present invention will have efficacy for treatment of patients suffering from: cancer, trauma, regeneration (in vitro and in vivo), viral/bacterial/parasitic infections, systemic lupus erythematosus, autoimmune disease, asthma, emphysema, scleroderma, allergy, ARDS and other diseases, disorders and conditions of the like.

NOV6 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV6 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV7

NOV7 includes three novel Amino Acid Transporter-like proteins disclosed below. The disclosed proteins have been named NOV7a, NOV7b and NOV7c.

### NOV7a

A disclosed NOV7a nucleic acid of 1513 nucleotides (also referred to as CG57387-01) encoding a novel Amino Acid Transporter-like protein is shown in Table 7A. An open reading frame was identified beginning with an ATG initiation codon at nucleotides 2-4 and ending with a TAG codon at nucleotides 1502-1504. Putative untranslated regions, if any, upstream from the initiation codon and downstream from the termination codon are underlined in Table 7A, and the start and stop codons are in bold letters.

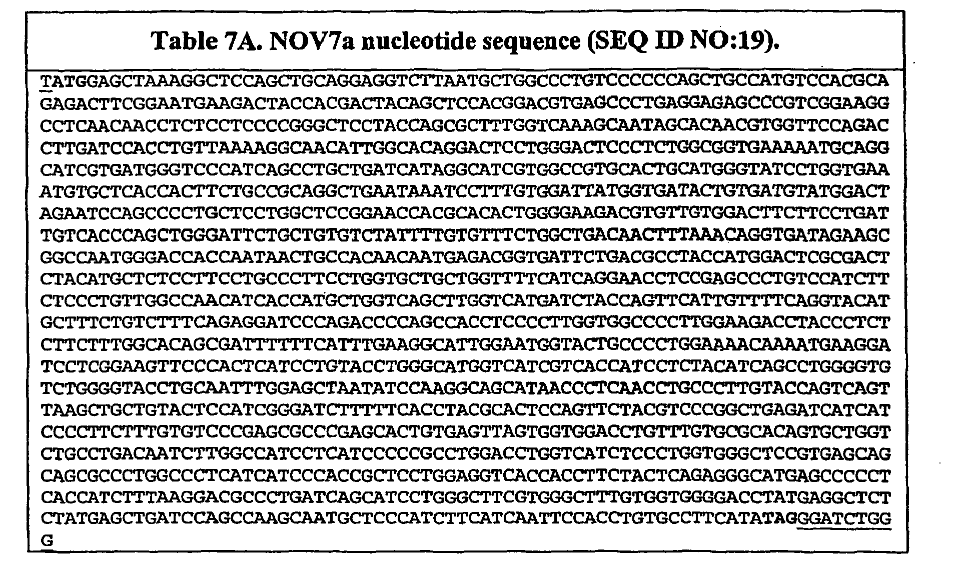

The disclosed NOV7a nucleic acid sequence, localized to chromsome 5, has 94 of 104 bases (90%) identical to a gb:GENBANK-ID:HSU42412|acc:U42412.1 mRNA from *Homo sapiens* (Human 5'-AMP-activated protein kinase, gamma-1 subunit mRNA, complete cds) (E = 2.4e⁻⁰⁸).

A NOV7a polypeptide (SEQ ID NO:20) encoded by SEQ ID NO:19 has 500 amino acid residues and is presented using the one-letter code in Table 7B. Signal P, Psort and/or Hydropathy results predict that NOV7a does not contain a signal peptide and is likely to be localized to the plasma membrane with a certainty of 0.6000.

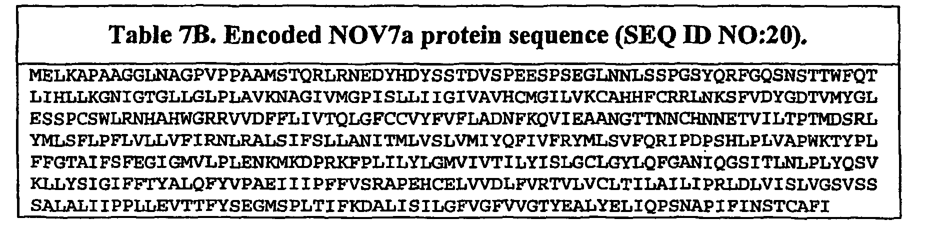

The NOV7a amino acid sequence has 127 of 276 amino acid residues (46%) identical to, and 175 of 276 amino acid residues (63%) similar to, the 486 amino acid residue ptnr:SPTREMBL-ACC:Q9VLM4 protein from *Drosophila melanogaster* (Fruit fly) (CG13384 PROTEIN) (E = 9.3e⁻⁸⁴).

The disclosed NOV7a is expressed in at least the following tissues: Adrenal Gland/Suprarenal gland, Cerebral Medulla/Cerebral white matter, Ovary and Synovium. This information was derived by determining the tissue sources of the sequences that were included in the invention including but not limited to SeqCalling sources, Public EST sources, Literature sources, and/or RACE sources.

### NOV7b

A disclosed NOV7b nucleic acid of 1501 nucleotides (also referred to as CG57387-02) encoding a novel Amino Acid Transporter-like protein is shown in Table 7C. An open reading frame was identified beginning with an ATG initiation codon at nucleotides 2-4 and ending with a TAG codon at nucleotides 1490-1492. Putative untranslated regions, if any, upstream from the initiation codon and downstream from the termination codon are underlined in Table 7C, and the start and stop codons are in bold letters.

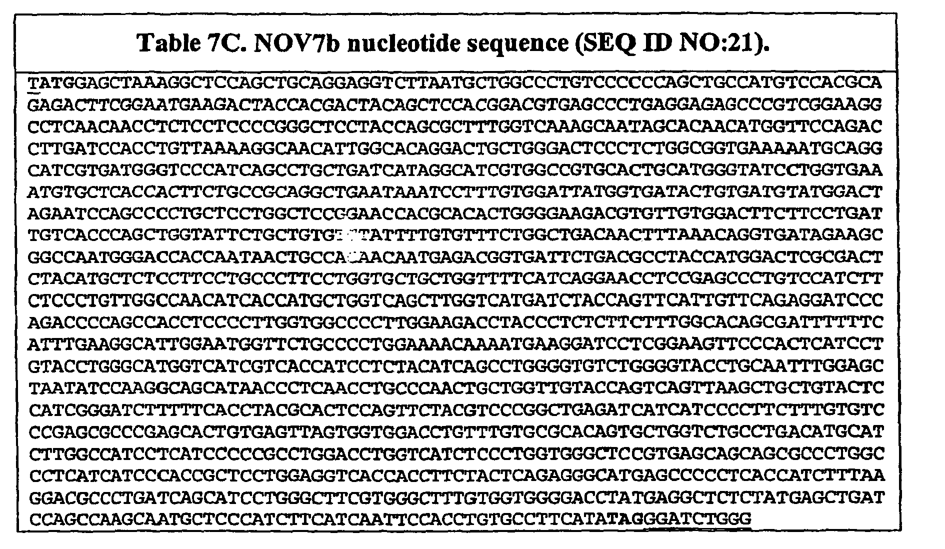

The disclosed NOV7b nucleic acid sequence, localized to chromsome 5, has 1364 of 1428 bases (95%) identical to a gb:GENBANK-ID:AX049362|acc:AX049362.1 mRNA from *Homo sapiens* (Sequence 32 from Patent W00071709) (E = 1.4e⁻²⁹⁰).

A NOV7b polypeptide (SEQ ID NO:22) encoded by SEQ ID NO:21 has 496 amino acid residues and is presented using the one-letter code in Table 7D. Signal P, Psort and/or Hydropathy results predict that NOV7b does not contain a signal peptide and is likely to be localized to the plasma membrane with a certainty of 0.6000.

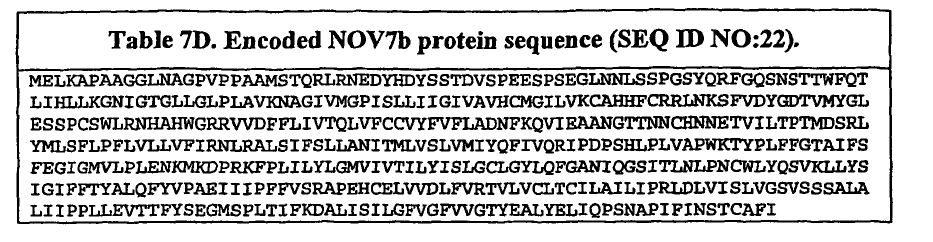

The NOV7b amino acid sequence has 407 of 476 amino acid residues (85%) identical to, and 443 of 476 amino acid residues (93%) similar to, the 475 amino acid residue ptnr:TREMBLNEW-ACC:AAK67316 protein from *Rattus norvegicus* (Rat) (Lysosomal Amino Acid Transporter 1) (E = 3.1e⁻²²¹).

The disclosed NOV7b is expressed in at least the following tissues: Adrenal Gland/Suprarenal gland, Cerebral Medulla/Cerebral white matter, Ovary and Synovium. This information was derived by determining the tissue sources of the sequences that were included in the invention including but not limited to SeqCalling sources, Public EST sources, Literature sources, and/or RACE sources.

### NOV7c

A disclosed NOV7c nucleic acid of 953 nucleotides (also referred to as CG57387-03) encoding a novel Amino Acid Transporter-like protein is shown in Table 7E. An open reading frame was identified beginning with an ATG initiation codon at nucleotides 2-4 and ending with a TGA codon at nucleotides 791-793. Putative untranslated regions, if any, upstream from the initiation codon and downstream from the termination codon are underlined in Table 7E, and the start and stop codons are in bold letters.

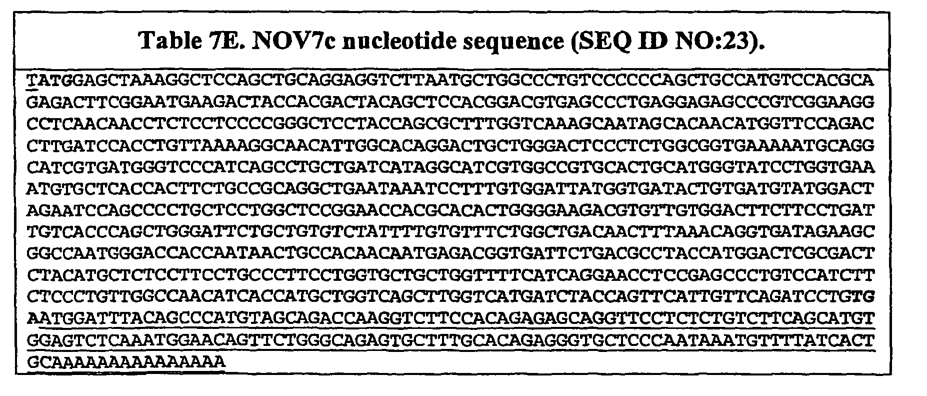

The disclosed NOV7c nucleic acid sequence, localized to chromsome 5, has 723 of 724 bases (99%) identical to a gb:GENBANK-ID:AX049362|acc:AX049362.1 mRNA from *Homo sapiens* (Sequence 32 from Patent W00071709) (E = 3.5e⁻¹⁵⁷).

A NOV7c polypeptide (SEQ ID NO:24) encoded by SEQ ID NO:23 has 263 amino acid residues and is presented using the one-letter code in Table 7F. Signal P, Psort and/or Hydropathy results predict that NOV7c does not contain a signal peptide and is likely to be localized to the plasma membrane with a certainty of 0.6000.

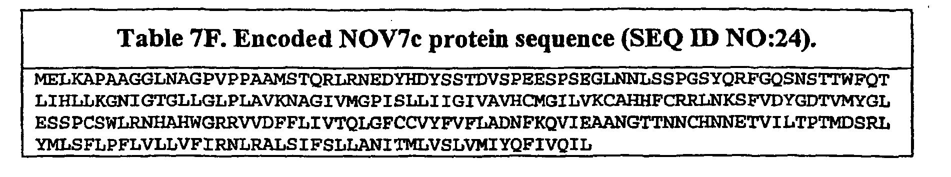

The NOV7c amino acid sequence has 209 of 241 amino acid residues (86%) identical to, and 228 of 241 amino acid residues (94%) similar to, the 475 amino acid residue ptnr:TREMBLNEW-ACC:AAK67316 protein from *Rattus norvegicus* (Rat) (Lysosomal Amino Acid Transporter 1) (E = 6.8e⁻¹¹⁴).

The disclosed NOV7c is expressed in at least the following tissues: Adrenal Gland/Suprarenal gland, Cerebral Medulla/Cerebral white matter, Ovary and Synovium. This information was derived by determining the tissue sources of the sequences that were included in the invention including but not limited to SeqCalling sources, Public EST sources, Literature sources, and/or RACE sources.

Possible SNPs found for NOV7a are listed in Table 7G.

| **Table 7G: SNPs** | | | | |
|---|---|---|---|---|
| **Variant** | **Nucleotide Position** | **Base Change** | **Amino Acid Position** | **Base Change** |
| 13377064 | 256 | C > G | Silent | N/A |
| 13377065 | 519 | G > T | 173 | Gly > Val |
| 13377066 | 1153 | C > T | Silent | N/A |

NOV7a, NOV7b and NOV7c are very closely homologous as is shown in the amino acid alignment in Table 7H.

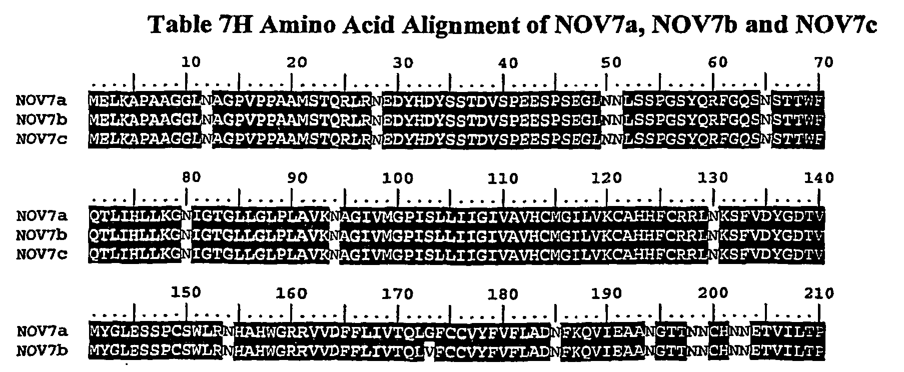

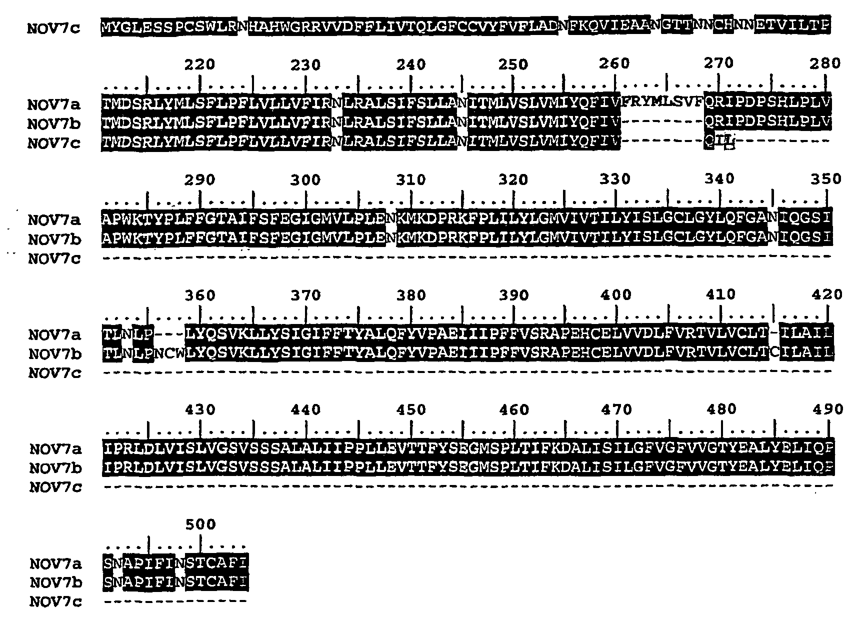

Homologies to any of the above NOV7 proteins will be shared by the other NOV7 proteins insofar as they are homologous to each other as shown above. Any reference to NOV7 is assumed to refer to the NOV7 proteins in general, unless otherwise noted.

NOV7a has homology to the amino acid sequences shown in the BLASTP data listed in Table 7I.

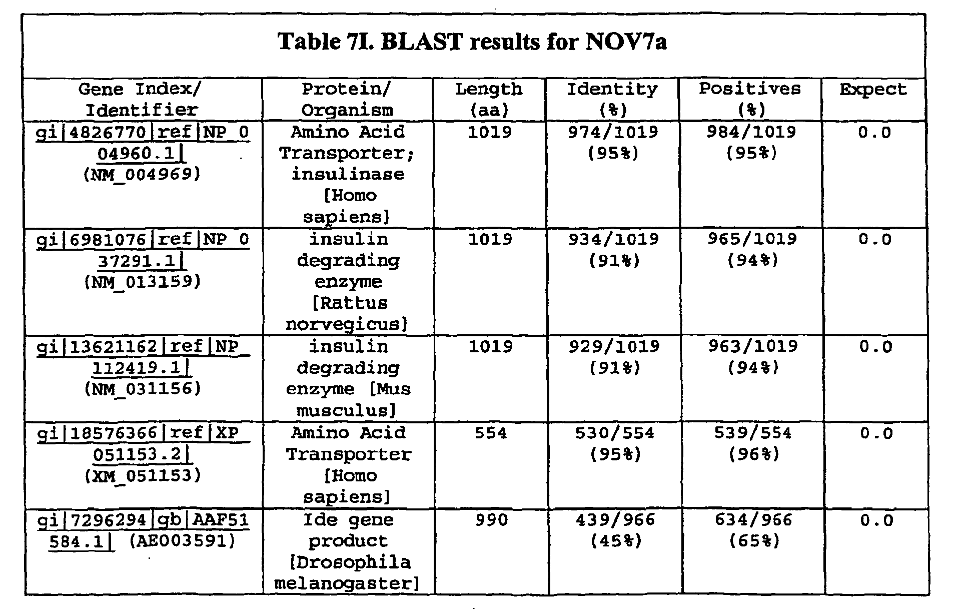

The homology of these sequences is shown graphically in the ClustalW analysis shown in Table 7J.

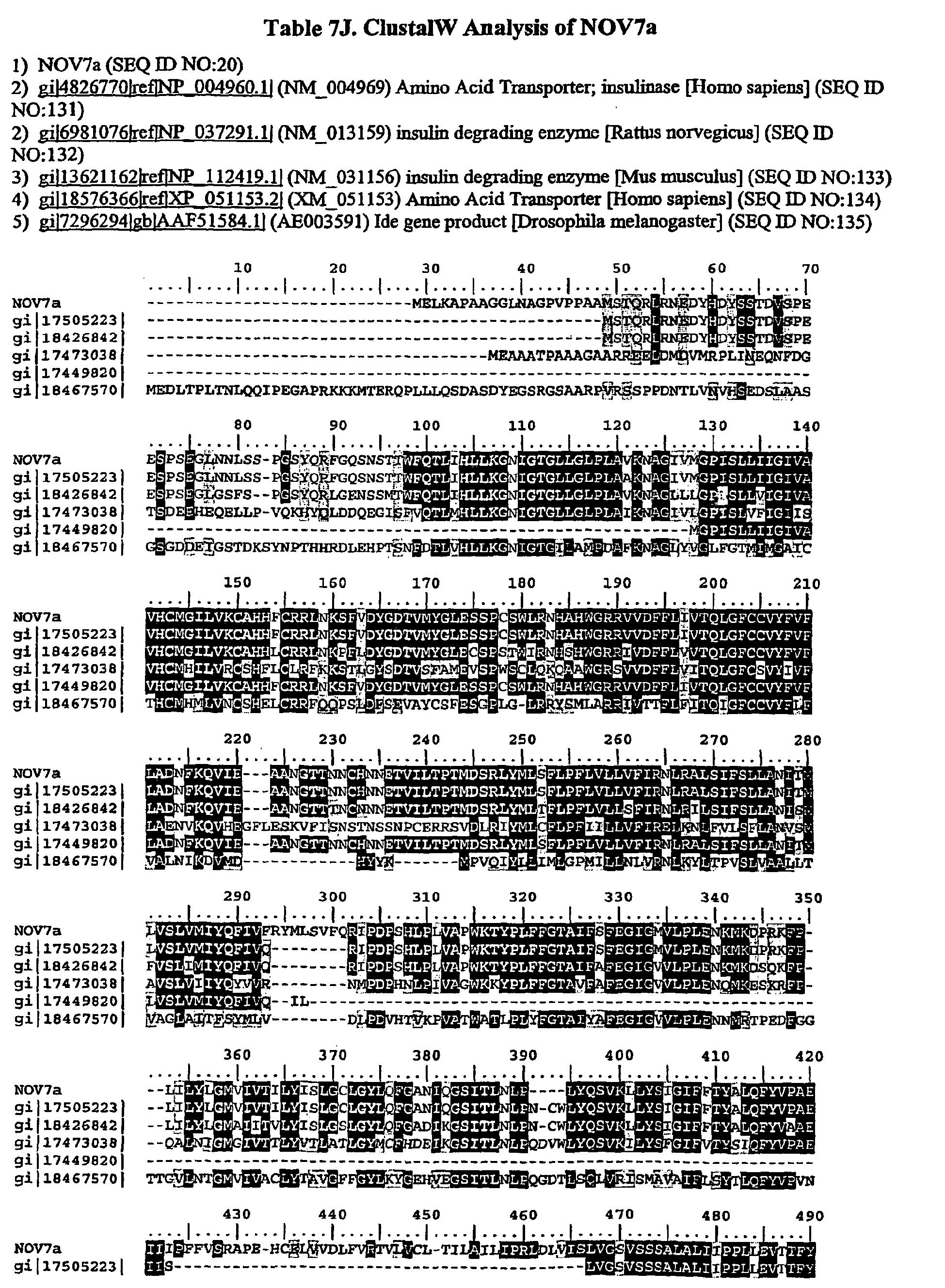

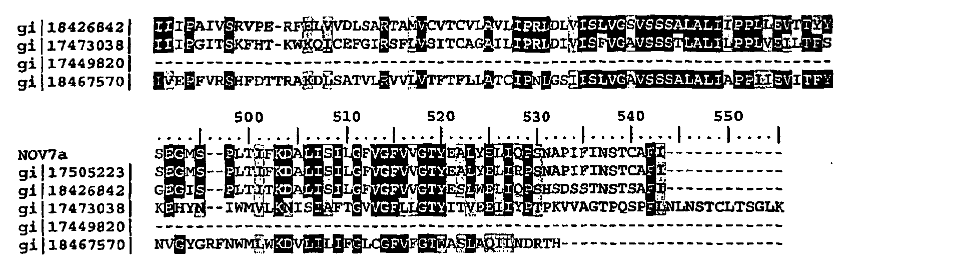

Table 7K lists the domain description from DOMAIN analysis results against NOV7a. This indicates that the NOV7a sequence has properties similar to those of other proteins known to contain these domains.

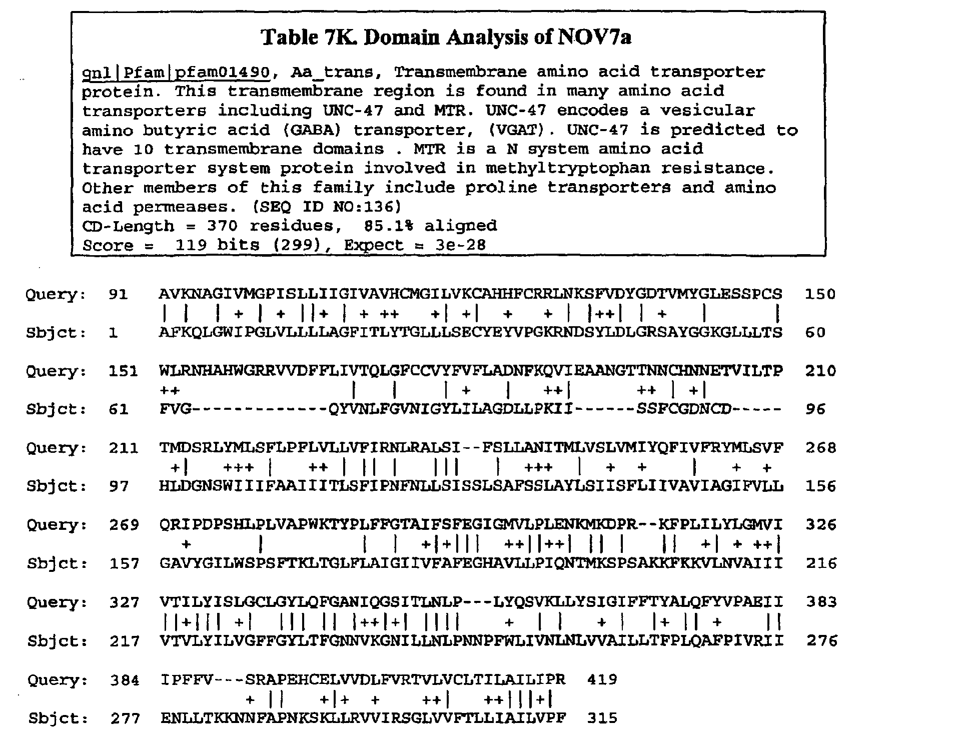

NOV7 has significant homology in the transmembrane region found in many amino acid transporters including UNC-47 and MTR. UNC-47 encodes a vesicular amino butyric acid(GABA) transporter, (VGAT). UNC-47 is predicted to have 10 transmembrane domains. MTR is a N system amino acid transporter system protein involved in methyltryptophan resistance. Other members of this family include proline transporters and amino acid permeases. Amino acid transporters may play an important role in a number of diseases as exemplified below.

Because most cancer deaths result from disseminated disease, understanding the regulation of tumor invasion and metastasis is a central theme in tumor cell biology. Interactions between extracellular matrices (ECM) and cellular microenvironment play a crucial role in this process. Selected amino acids and polyamines have been tested for their ability to regulate RL95-2 cell invasion through both intact human amniotic basement membrane and a novel human ECM (Amgel). Three major systems for neutral amino acid transport, systems L, A, and ASC, are operational in these neoplastic cells. Amino acids entering the cell via transport system A or N, i.e., (methyl amino)-isobutyrate (MeAIB) or Asn, markedly enhanced invasiveness of these human adenocarcinoma cells as measured by a standard 72-hr amnion or Amgel invasion assay. Addition of 2-amino-2-norborane carboxylic acid (BCH; 1 mM), a model substrate of the L transport system, caused a significant decrease in invasive activity when tested in the Amgel assay. Interestingly, Val lowers steady-state levels of MeAIB uptake and blocks the increase in cell invasion elicited by MeAIB. At the same time, these amino acids do not influence cell proliferation activity. Neither the charged amino acid Lys or Asp (not transported by A/N/L systems) nor the polyamines putrescine, spermidine, or spermine modulate invasiveness under similar experimental conditions. Moreover, the observed time-dependent stimulation of system A activity (cellular influx of MeAIB) by substrate depletion is prevented by the addition of actinomycin D (5 microM) or cycloheximide (100 microM), suggesting the involvement of de novo RNA and protein synthesis events in these processes. MeAIB treatment of tumor cells selectively increased the activities of key invasion-associated type IV collagenases/gelatinases. These results indicate that in the absence of defined regulators (growth factors or hormones), certain amino acids may contribute to the epigenetic control of human tumor cell invasion and, by extension, metastasis. This study suggests that amino acids, acting via specific signaling pathways, modulate phenotypic cell behavior by modulating the levels of key regulatory enzymatic proteins (Singh et al., Cancer Invest 14(1):6-18, 1996).

To investigate the function of a basic and neutral amino acid transporter-like protein (rBAT) which is a candidate gene for cystinuria, the rBAT gene was analysed in cystinuric patients. Patient 1 is a compound heterozygote with mutations in the rBAT gene causing a glutamine-to-lysine transition at amino acid 268, and a threonine-to-alanine transition at amino acid 341, who inherited these alleles from his mother (E268K) and father (T341A), respectively. Injection of T341A and E268K mutant cRNAs into oocytes decreased transport activity to 53.9% and 62.5% of control (L-cystine transport activity in oocytes injected with wild-type rBAT cRNA), respectively. Co-injection of E268K and T341A into oocytes strongly decreased amino acid transport activity to 28% of control. On the other hand, co-injection of wild-type and mutant rBAT did not decrease transport activity. Furthermore, immunological studies have demonstrated that the reduction of amino acid transport is not due to a decrease in the amount of rBAT protein expressed in oocyte membranes. These results indicate that mutations in the rBAT gene are crucial disease-causing lesions in cystinuria. In addition, co-injection experiments suggest that rBAT may function as a transport activator orregulatory subunit by homo- or hetero-multimer complex formation (Miyamoto et al., Biochem J 310 (Pt 3):951-5, 1995).

The spinal cord of 20 patients with amyotrophic lateral sclerosis (ALS) and 5 patients with lower motor neuron disease (LMND) were investigated immunohistochemically using anti-human excitatory amino acid transporter 1 (EAAT1) and EAAT2 antibodies which are the astrocytic transporters. The purpose of the study was to examine relationships between EAAT1 and EAAT2 immunoreactivity and degeneration of anterior horn neurons. Specimens from 20 patients without any neurological disease served as controls. In controls, spinal cord gray matter was densely immunostained by antibodies, whereas the white matter was generally not immunostained. In motor neuron disease (MND) patients, EAAT1 immunoreactivity was relatively well preserved in the gray matter despite neuronal loss of anterior horn cells. On the other hand, EAAT2 immunoreactivity in anterior horns correlated with the degree of neuronal loss of anterior horn cells: in the patients with mild neuronal depletion, anterior horns were densely immunostained by the antibody, whereas in the patients with severe neuronal loss, EAAT2 expression was markedly reduced. Degenerated anterior horn cells frequently showed a much denser EAAT1 and EAAT2 immunoreactivity around the surface of the neurons and their neuronal processes than that observed in normal-appearing neurons. There was no difference in the expression of EAAT1 and EAAT2 immunoreactivity between LMND and ALS patients. These findings suggest that in the early stage of degeneration of anterior horn cells, EAAT1 and EAAT2 immunoreactivity is preserved in the astrocytic foot directly attached to normal-appearing neurons, whereas levels of EAAT1 and EAAT2 protein rather increase in the astrocytic foot directly attached to degenerated anterior horn neurons; the latter effect most probably reduces the elevated glutamate level, compensates for the reduced function of astroglial glutamate transporters, or represents a condensation of EAAT1 and EAAT2 immunoreactivity secondary to loss of neurites and greater condensation of astrocytic processes. Thus, the study demonstrates a difference in EAAT1 and EAAT2 immunoreactivity in different stages of progression in ALS, as a feature of the pathomechanism of this disease (Sasaki et al., Acta Neuropathol (Ber1) 100(2):138-44, 2000).

The NOV7 nucleic acid of the invention encoding a Amino Acid Transporter-like protein includes the nucleic acid whose sequence is provided in Tables 7A, 7C and 7E, or a fragment thereof. The invention also includes a mutant or variant nucleic acid any of whose bases may be changed from the corresponding base shown in Tables 7A, 7C and 7E while still encoding a protein that maintains its Amino Acid Transporter-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 10% of the NOV7a residues, about 5% of the NOV7b residues and about 1% of the NOV7c residues may be so changed.

The NOV7 protein of the invention includes the Amino Acid Transporter-like protein whose sequence is provided in Tables 7B, 7D and 7F. The invention also includes a mutant or variant protein any of whose residues may be changed from the corresponding residue shown in Tables 7B, 7D and 7F while still encoding a protein that maintains its Amino Acid Transporter-like activities and physiological functions, or a functional fragment thereof. In the mutant or variant protein, up to about 54% of the NOV7a bases, about 15% of the NOV7b bases and about 14% of the NOV7c bases may be so changed.

The NOV7 nucleic acids and proteins of the invention are useful in potential diagnostic and therapeutic applications implicated in various diseases and disorders described below and/or other pathologies. For example, the compositions of the present invention will have efficacy for treatment of patients suffering from: cancer, trauma, regeneration (in vitro and in vivo), viral/bacterial/parasitic infections,Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, fertility, adrenoleukodystrophy, congenital adrenal hyperplasia and other diseases, disorders and conditions of the like.

NOV7 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV7 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV8

NOV8 includes five novel Lymphocyte antigen precursor-like or Lymphocyte antigen LY-6F-like proteins disclosed below. The disclosed proteins have been named NOVBa-NOV8e.

### NOV8a

A disclosed NOV8a nucleic acid of 468 nucleotides (also referred to as CG56417-01) encoding a novel Lymphocyte antigen precursor-like protein is shown in Table 8A. An open reading frame was identified beginning with an ATG initiation codon at nucleotides 31-33 and ending with a TGA codon at nucleotides 439-441. Putative untranslated regions, if any, upstream from the initiation codon and downstream from the termination codon are underlined in Table 8A, and the start and stop codons are in bold letters.

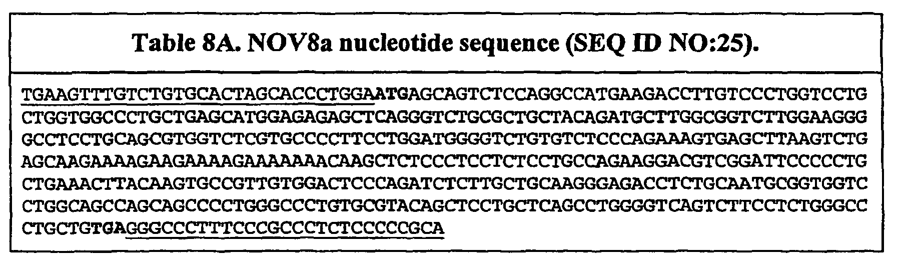

A NOV8a polypeptide (SEQ ID NO:26) encoded by SEQ ID NO:25 has 136 amino acid residues and is presented using the one-letter code in Table 8B.

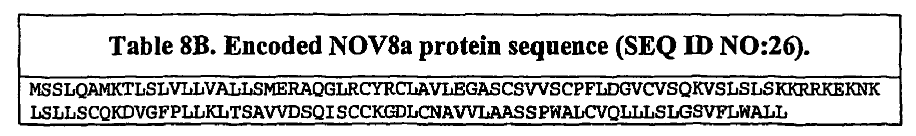

### NOV8b

A disclosed NOV8b nucleic acid of 610 nucleotides (also referred to as CG56417-02) encoding a novel Lymphocyte antigen precursor-like protein is shown in Table 8C. An open reading frame was identified beginning with an ATG initiation codon at nucleotides 47-49 and ending with a TAG codon at nucleotides 515-517. Putative untranslated regions, if any, upstream from the initiation codon and downstream from the termination codon are underlined in Table 8C, and the start and stop codons are in bold letters.

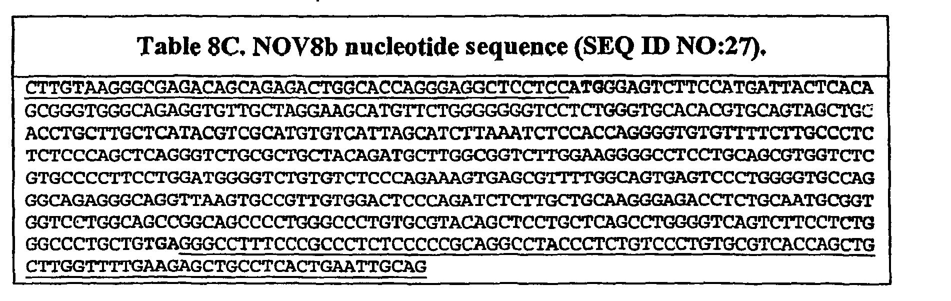

The disclosed NOV8b nucleic acid sequence, localized to chromsome 8, has 192 of 249 bases (77%) identical to a gb:GENBANK-ID:HSU85047|acc:U85047.1 mRNA from *Homo sapiens* (Human FRA3B region corresponding to the breakpoint junction in lung tumor cell line NCIH211) (E = 1.1e⁻²²).

A NOV8b polypeptide (SEQ ID NO:28) encoded by SEQ ID NO:27 has 156 amino acid residues and is presented using the one-letter code in Table 8D. Signal P, Psort and/or Hydropathy results predict that NOV8b does not contain a signal peptide and is likely to be localized to the plasma membrane with a certainty of 0.6000.

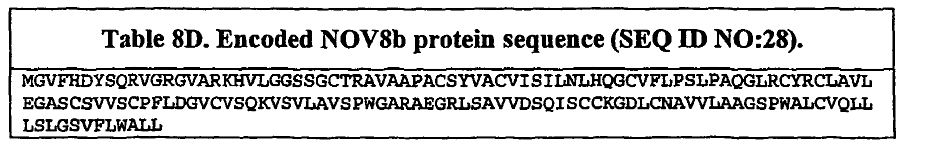

The NOV8b amino acid sequence has 53 of 126 amino acid residues (42%) identical to, and 69 of 126 amino acid residues (54%) similar to, the 134 amino acid residue ptnr:SWISSNEW-ACC:P35460 protein from *Mus musculus* (Mouse) (Lymphocyte Antigen LY-6F.1 Precursor) (E = 2.3e⁻¹⁶).

The disclosed NOV8b is expressed in at least the following tissues: Adrenal Gland/Suprarenal gland, Brain, Kidney, Lung, Pituitary Gland, Placenta, Trachea and Whole Organism. This information was derived by determining the tissue sources of the sequences that were included in the invention including but not limited to SeqCalling sources, Public EST sources, Literature sources, and/or RACE sources.

### NOV8c

A disclosed NOV8c nucleic acid of 406 nucleotides (also referred to as CG56417-03) encoding a novel Lymphocyte antigen precursor-like protein is shown in Table 8E. An open reading frame was identified beginning with an ATG initiation codon at nucleotides 2-4 and ending with a TGA codon at nucleotides 404-406. Putative untranslated regions, if any, upstream from the initiation codon and downstream from the termination codon are underlined in Table 8E, and the start and stop codons are in bold letters.

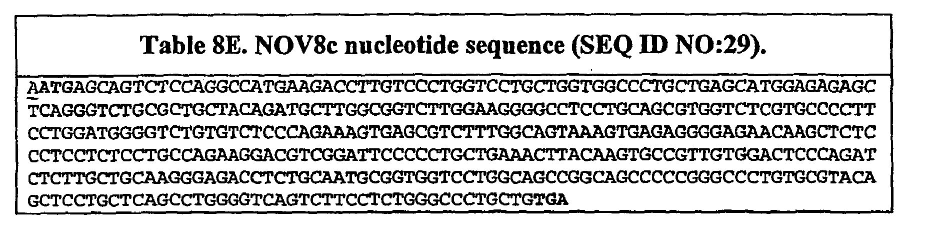

The disclosed NOV8c nucleic acid sequence, localized to chromsome 8, has 260 of 403 bases (64%) identical to a gb:GENBANK-ID:RATLY6CA|acc:M30691.1 mRNA from *Rattus norvegicus* (Rat Ly6-C antigen mRNA, exon 2) (E =1.2e⁻¹⁶).

A NOV8c polypeptide (SEQ ID NO:30) encoded by SEQ ID NO:29 has 134 amino acid residues and is presented using the one-letter code in Table 8F. Signal P, Psort and/or Hydropathy results predict that NOV8c contains a signal peptide and is likely to be localized to the plasma membrane with a certainty of 0.9190. The most likely cleavage site for a NOV8c polypeptide is between amino acids 26 and 27: AQG-LR

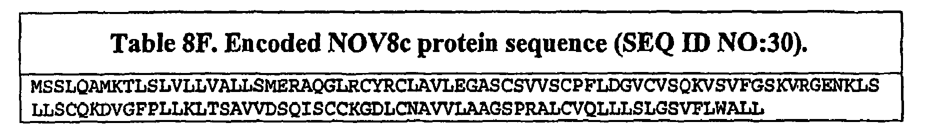

The NOV8c amino acid sequence has 61 of 134 amino acid residues (45%) identical to, and 79 of 134 amino acid residues (58%) similar to, the 134 amino acid residue ptnr:SPTREMBL-ACC:Q63318 protein from *Rattus norvegicus* (Rat) (RAT LY6-C ANTIGEN) (E = 4.4e⁻²²).

### NOV8d

A disclosed NOV8d nucleic acid of 611 nucleotides (also referred to as CG56417-04) encoding a novel Lymphocyte antigen LY-6F-like protein is shown in Table 8G. An open reading frame was identified beginning with an ATG initiation codon at nucleotides 47-49 and ending with a TGA codon at nucleotides 602-604. Putative untranslated regions, if any, upstream from the initiation codon and downstream from the termination codon are underlined in Table 8G, and the start and stop codons are in bold letters.

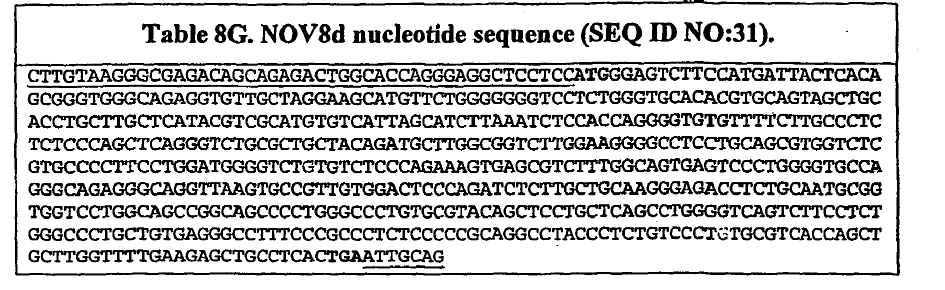

The disclosed NOV8d nucleic acid sequence, localized to chromsome 8, has 198 of 256 bases (77%) identical to a gb:GENBANK-ID:AP000509|acc:AP000509.1 mRNA from *Homo sapiens* (Homo sapiens genomic DNA, chromosome 6p21.3, HLA Class I region, section 8/20) (E = 5.4e⁻²³).

A NOV8d polypeptide (SEQ ID NO:32) encoded by SEQ ID NO:31 has 185 amino acid residues and is presented using the one-letter code in Table 8H. Signal P, Psort and/or Hydropathy results predict that NOV8d does not contain a signal peptide and is likely to be localized to the plasma membrane with a certainty of 0.7900.

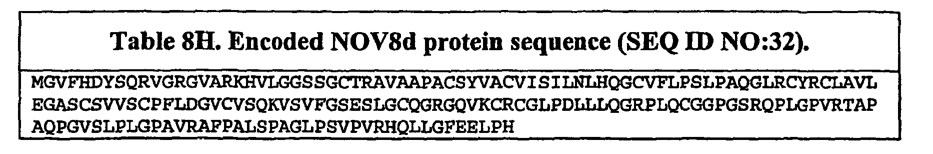

The NOV8d amino acid sequence has 24 of 57 amino acid residues (42%) identical to, and 35 of 57 amino acid residues (61%) similar to, the 134 amino acid residue ptnr:SWISSNEW-ACC:P35460 protein from *Mus musculus* (Mouse) (Lymphocyte Antigen LY-6F.1 Precursor) (E = 2.4e⁻⁰⁷).

The disclosed NOV8d is expressed in at least the following tissues: Brain, Pituitary Gland, Placenta, Lung, Trachea, Kidney, Colon and Whole Organism. This information was derived by determining the tissue sources of the sequences that were included in the invention including but not limited to SeqCalling sources, Public EST sources, Literature sources, and/or RACE sources.

### NOV8e

A disclosed NOV8e nucleic acid assembly (also referred to as 172885384) ofNOV8d (CG56417-04) encoding a novel Lymphocyte antigen LY-6F-like protein is shown in Table 8I. The cDNA coding for the mature form of the full length NOV8d from residue 49 to 183 was targeted for "in-frame" cloning by PCR. The insert 172885384 was found to encode an open reading frame between residues 49 and 183 of the target sequence of NOV8d.

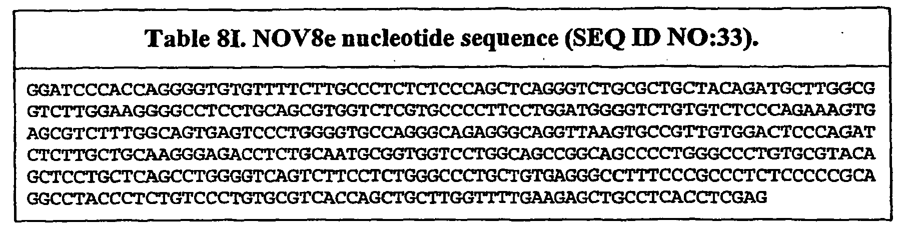

A NOV8e polypeptide (SEQ ID NO:34) encoded by SEQ ID NO:33 has 136 amino acid residues and is presented using the one-letter code in Table 8J.

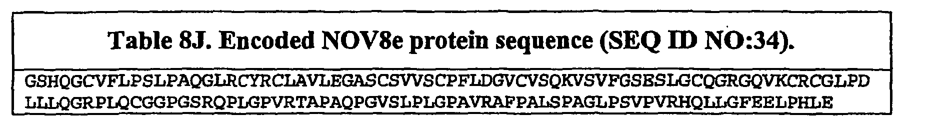

Possible SNPs found for NOV8a are listed in Table 8K and possible SNPs for NOV8d are listed in Table 8L.

| **Table 8K: SNPs** | | | | |
|---|---|---|---|---|
| **Variant** | **Nucleotide Position** | **Base Change** | **Amino Acid Position** | **Base Change** |
| 13377070 | 53 | A > G | 8 | Lys > Arg |
| 13377074 | 161 | T > C | 44 | Val > Ala |
| 13377075 | 166 | T > C | 46 | Cys > Arg |
| 13377076 | 194 | C > A | 55 | Ser > Tyr |
| 13377077 | 196 | C > T | 56 | Gln > End |
| 13377078 | 300 | A > G | Silent | N/A |
| 13377068 | 362 | T > C | 111 | Leu > Pro |
| 13377079 | 370 | A > G | 114 | Ser > Gly |
| 13377067 | 371 | G > A | 114 | Ser > Asn |
| 13377080 | 431 | C > T | 134 | Ala > Val |
| 13377081 | 468 | A > G | Silent | N/A |

| **Table 8L: SNPs** | | | |
|---|---|---|---|
| **Consensus Position** | **Depth** | **Base Change** | **PAF** |
| 61 | 31 | G > - | 0.065 |
| 79 | 32 | A > G | 0.062 |
| 89 | 33 | A > G | 0.061 |
| 105 | 48 | G > A | 0.042 |
| 166 | 51 | C > T | 0.157 |
| 174 | 51 | A > G | 0.039 |
| 192 | 51 | A>C | 0.039 |
| 207 | 51 | C>T | 0.039 |
| 236 | 51 | T>C | 0.059 |
| 237 | 51 | A>G | 0.039 |
| 265 | 52 | T>A | 0.038 |
| 294 | 49 | G>T | 0.327 |
| 322 | 49 | A>G | 0.041 |
| 327 | 49 | A>G | 0.061 |
| 360 | 48 | A>C | 0.042 |
| 373 | 48 | A>G | 0.042 |
| 419 | 47 | G>A | 0.043 |
| 448 | 47 | G>A | 0.064 |

NOV8a - NOV8e are very closely homologous as is shown in the amino acid alignment in Table 8M.

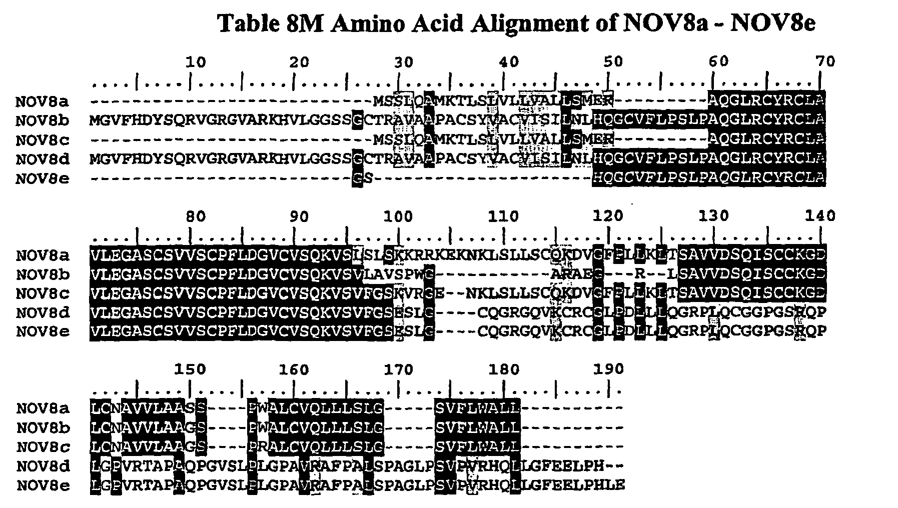

Homologies to any of the above NOV8 proteins will be shared by the other NOV8 proteins insofar as they are homologous to each other as shown above. Any reference to NOV8 is assumed to refer to the NOV8 proteins in general, unless otherwise noted.

NOV8a has homology to the amino acid sequences shown in the BLASTP data listed in Table 8N.

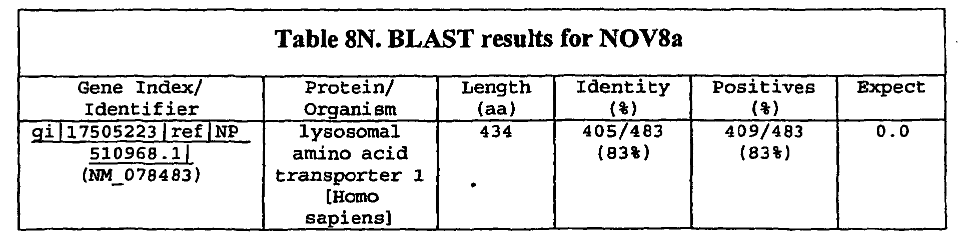

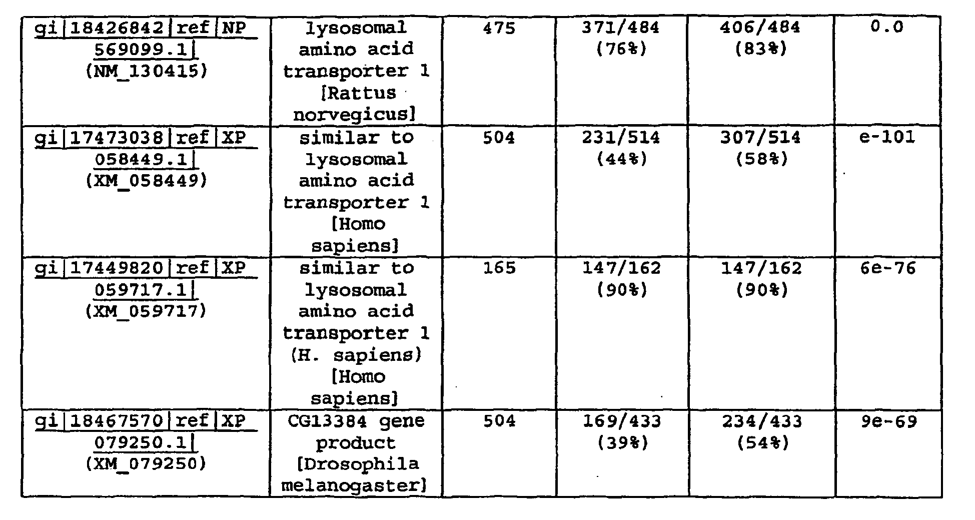

The homology of these sequences is shown graphically in the ClustalW analysis shown in Table 80.

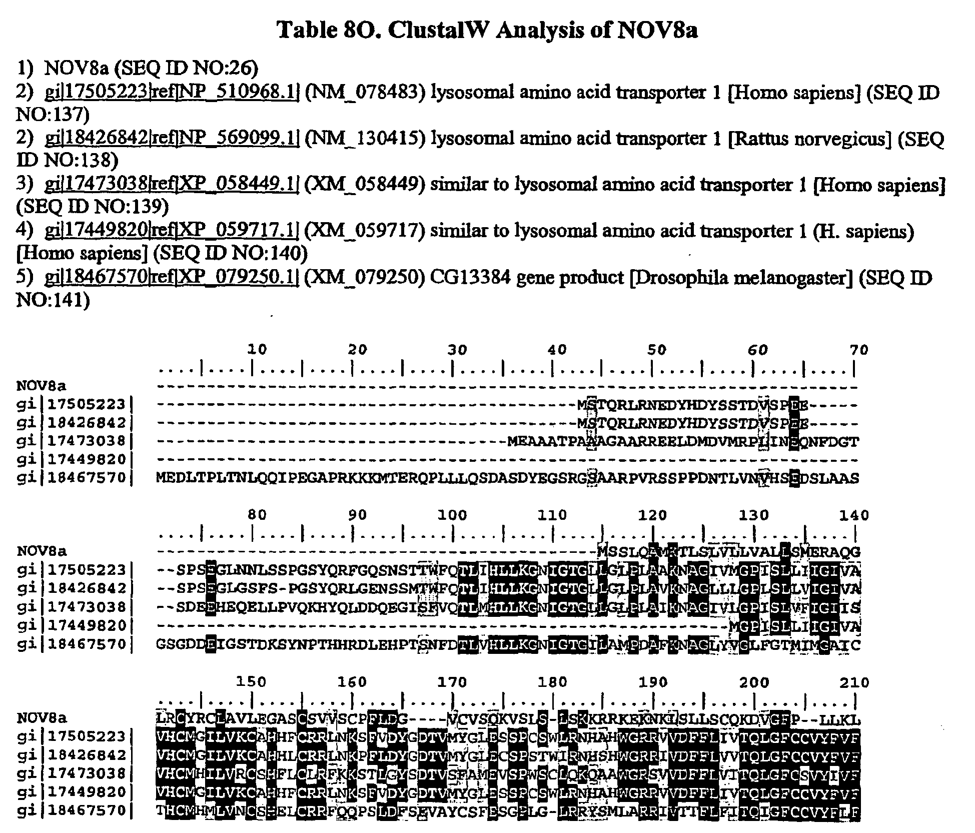

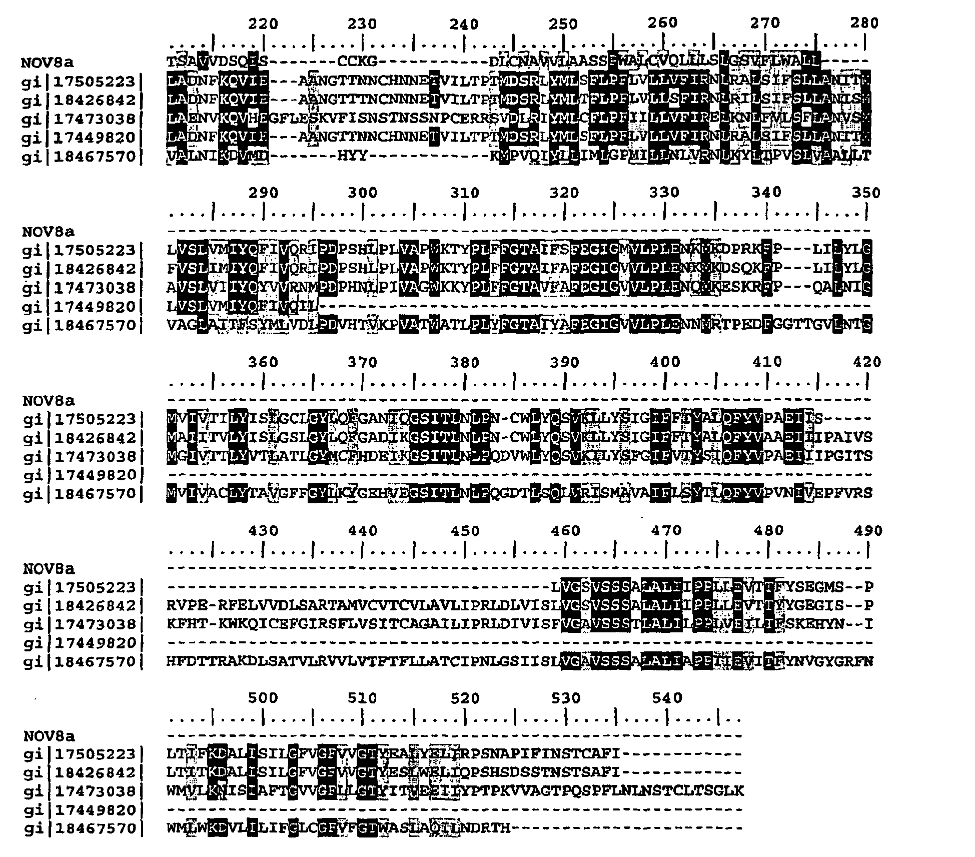

Table 8P lists the domain description from DOMAIN analysis results against NOV8a. This indicates that the NOV8a sequence has properties similar to those of other proteins known to contain these domains.

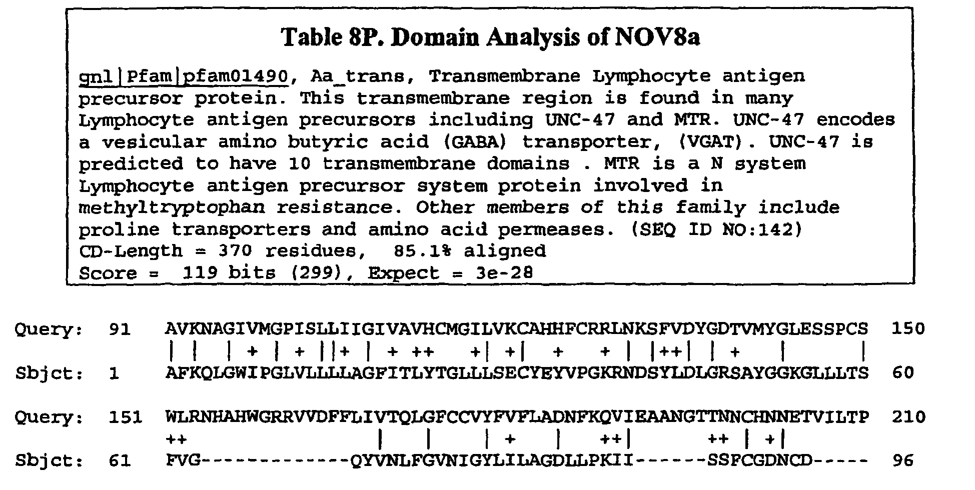

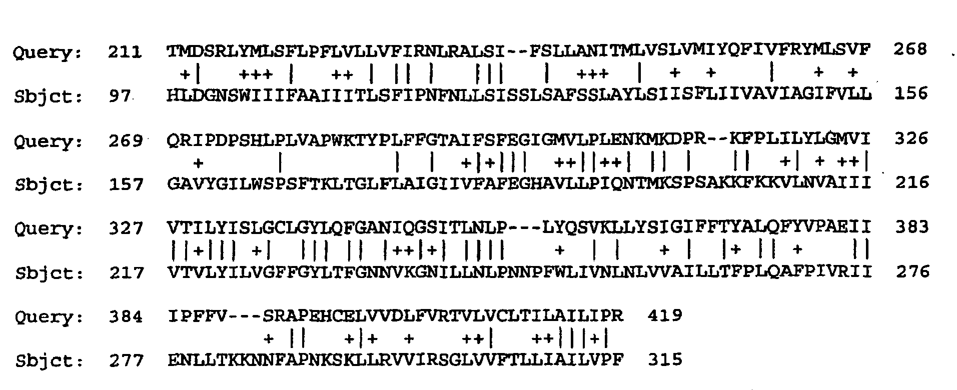

Murine Ly-6 molecules are a family of cell surface glycoproteins which have interesting patterns of tissue expression during haematopoiesis from multipotential stem cells to lineage committed precursor cells, and on specific leucocyte subpopulations in the peripheral lymphoid tissues. These interesting patterns of tissue expression suggest an intimate association between the regulation of Ly-6 expression and the development and homeostasis of the immune system. Ly-6 molecules are low molecular weight phosphatidyl inositol anchored glycoproteins with remarkable amino acid homology throughout a distinctive cysteine rich protein domain that is associated predominantly with O-linked carbohydrate. The in vivo functions of Ly-6 molecules are not known although in vitro studies suggest a role in cellular activation (Gumley et al., Immunol Cell Biol.;73(4):277-96, 1995).

The Ly-6 alloantigens have been shown to play a critical role in T lymphocyte activation. LeClair et al. (LeClair et al., EMBO J. 5(12):3227-34, 1986) isolated a Ly-6 cDNA, synthetic oligonucleotides, based on the partial amino acid sequence of purified Ly-6E.1 protein, were used to probe a cDNA library. The synthetic oligonucleotides or the isolated cDNA detected a 1.1-kb RNA species. Sequence analysis of the cDNA clone revealed that the Ly-6E.1 protein consists of a 26-amino acid leader followed by a 108-residue, cysteine-rich, core protein with no N-linked glycosylation sites. Southern blot analysis of genomic DNAs revealed multiple bands indicating a family of related genes. Using recombinant inbred and Ly-6 congenic strains of mice, restriction fragment length polymorphisms were demonstrable, and correlated with the Ly-6 allotype of the DNA donors. This probe will enable further molecular genetic analysis of the role of Ly-6-linked proteins in the process of T lymphocyte activation. Isolation of Ly-6 genomic clones may promote a further understanding of the complex tissue-specific expression patterns characteristic of Ly-6-linked genes (LeClair et al., 1986).

NOV8 will have similar properties as other lymphocyte antigen precursor and Ly-6 proteins and may be involved in the regulation of T lymphocyte activation.

The NOV8 nucleic acid of the invention encoding a Lymphocyte antigen precursor-like protein includes the nucleic acid whose sequence is provided in Tables 8A, 8C, 8E, 8G and 8I or a fragment thereof. The invention also includes a mutant or variant nucleic acid any of whose bases may be changed from the corresponding base shown in Tables 8A, 8C, 8E, 8G and 8I while still encoding a protein that maintains its Lymphocyte antigen precursor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 23% of the NOV8b residues, about 36% of the NOV8c residues and about 23% of the NOV8d residues may be so changed.

The NOV8 protein of the invention includes the Lymphocyte antigen precursor-like protein whose sequence is provided in Tables 8B, 8D, 8F, 8H and 8J. The invention also includes a mutant or variant protein any of whose residues may be changed from the corresponding residue shown in Tables 8B, 8D, 8F, 8H and 8J while still encoding a protein that maintains its Lymphocyte antigen precursor-like activities and physiological functions, or a functional fragment thereof. In the mutant or variant protein, up to about 58% of the NOV8b bases, about 55% of the NOV8c bases and about 58% of the NOV8d bases may be so changed.

The NOV8 nucleic acids and proteins of the invention are useful in potential diagnostic and therapeutic applications implicated in various diseases and disorders described below and/or other pathologies. For example, the compositions of the present invention will have efficacy for treatment of patients suffering from: adrenoleukodystrophy, congenital adrenal hyperplasia, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, growth and reproductive disorders, systemic lupus erythematosus, autoimmune disease, asthma, emphysema, scleroderma, allergy, ARDS and other diseases, disorders and conditions of the like.

NOV8 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV8 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV9

NOV9 includes two novel Early B-Cell Factor-like proteins disclosed below. The disclosed proteins have been named NOV9a and NOV9b,

### NOV9a

A disclosed NOV9a nucleic acid of 1873 nucleotides (also referred to as CG57480-01) encoding a novel Early B-Cell Factor-like protein is shown in Table 9A. An open reading frame was identified beginning with an ATG initiation codon at nucleotides 25-27 and ending with a TGA codon at nucleotides 1813-1815. Putative untranslated regions, if any, upstream from the initiation codon and downstream from the termination codon are underlined in Table 9A, and the start and stop codons are in bold letters.

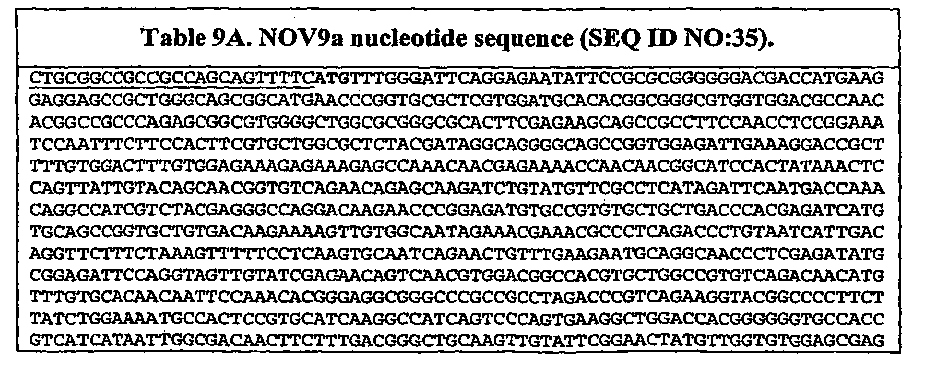

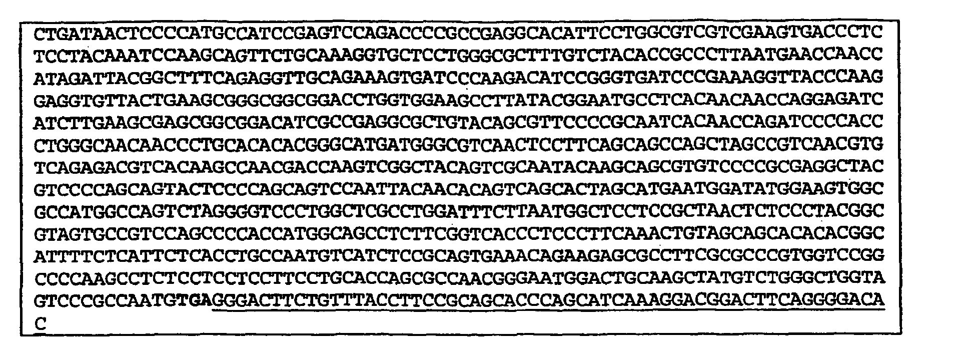

The disclosed NOV9a nucleic acid sequence, localized to chromsome 10, has 1769 of 1871 bases (94%) identical to a gb:GENBANK-ID:MMU92702|acc:U92702.1 mRNA from *Mus musculus* (Mus musculus Olf-1/EBF-like-2(9L) transcription factor (O/E-2(9L)) mRNA, complete cds) (E = 0.0).

A NOV9a polypeptide (SEQ ID NO:36) encoded by SEQ ID NO:35 has 596 amino acid residues and is presented using the one-letter code in Table 9B. Signal P, Psort and/or Hydropathy results predict that NOV9a does not contain a signal peptide and is likely to be localized to the cytoplasm with a certainty of 0.6500.

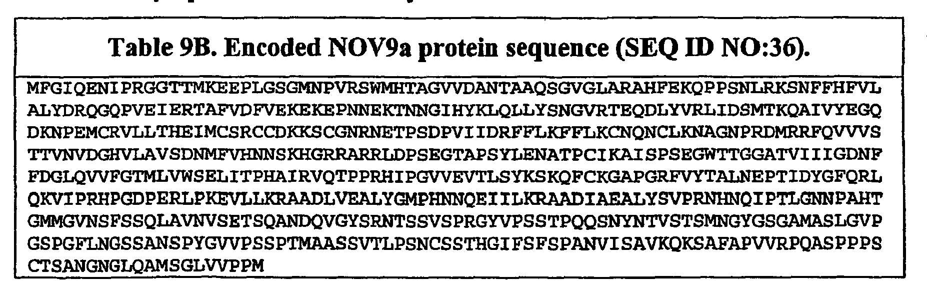

The NOV9a amino acid sequence has 593 of 596 amino acid residues (99%) identical to, and 595 of 596 amino acid residues (99%) similar to, the 596 amino acid residue ptnr:SWISSPROT-ACC:008791 protein from *Mus musculus* (Mouse) (Early B-Cell Factor-3 (Early B-Cell Factor 3) (EBF-3) (OLF-1/EBF- Like 2) (OE-2) (O/E-2)) (E = 0.0).

The disclosed NOV9a is expressed in at least the following tissue: because of the expression pattern of (GENBANK-ID: gb:GENBANK-ID:MMU92702|acc:U92702.1) a closely related *Mus musculus* Olf-1/EBF-like-2(9L) transcription factor (O/E-2(9L)) mRNA, complete cds homolog in species *Mus musculus:* adult olfactory epithelium; in embryo, expressed in epithalamus, hypothalamus, throughout the brainstem and near the ventricular zones of mesencephalon, in rostral rhombencephalon, in vomeronasal organ, at high level in developing retina, developing spinal cord, at low level in dorsal root ganglia, trigeminal ganglia, glossopharyngeal nerve ganglia.

### NOV9b

A disclosed NOV9b nucleic acid of 1667 nucleotides (also referred to as CG57480-02) encoding a novel Early B-Cell Factor-like protein is shown in Table 9C. An open reading frame was identified beginning with an ATG initiation codon at nucleotides 5-7 and ending with a TAA codon at nucleotides 1625-1627. Putative untranslated regions, if any, upstream from the initiation codon and downstream from the termination codon are underlined in Table 9C, and the start and stop codons are in bold letters.

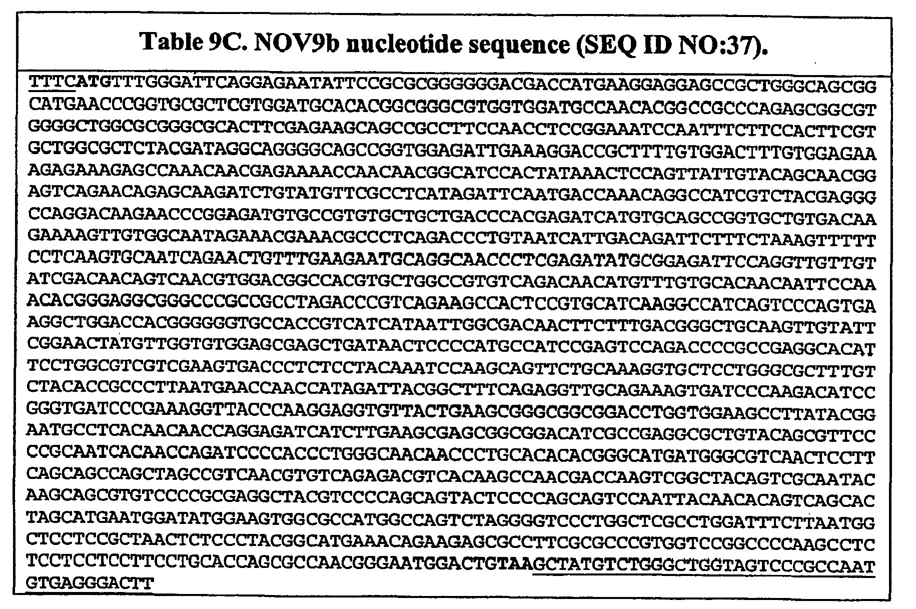

The disclosed NOV9b nucleic acid sequence, localized to chromsome 10, has 1568 of 1665 bases (94%) identical to a gb:GENBANK-ID:MMU92704|acc:U92704.1 mRNA from *Mus musculus (Mus musculus* Olf-1/EBF-like-2(0S) transcription factor (O/E-2(0S)) mRNA, alternative splice variant, complete cds) (E = 0.0).

A NOV9b polypeptide (SEQ ID NO:38) encoded by SEQ ID NO:37 has 540 amino acid residues and is presented using the one-letter code in Table 9D. Signal P, Psort and/or Hydropathy results predict that NOV9b does not contain a signal peptide and is likely to be localized to the plasma membrane with a certainty of 0.6500.

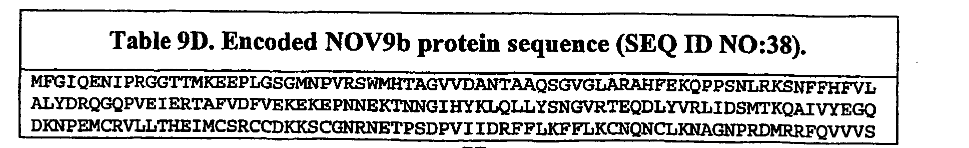

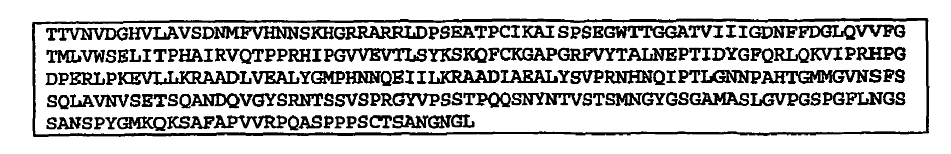

The NOV9b amino acid sequence has 526 of 526 amino acid residues (100%) identical to, and 526 of 526 amino acid residues (100%) similar to, the 537 amino acid residue ptnr:TREMBLNEW-ACC:CAC16113 protein from *Homo sapiens* (Human) (BA234G16.1.1 (Novel Protein Similar Early B-Cell Factor (EBF)) (E = 6.5e⁻²⁸⁹).

The disclosed NOV9b is expressed in at least the following tissues: olfactory tissue. This information was derived by determining the tissue sources of the sequences that were included in the invention including but not limited to SeqCalling sources, Public EST sources, Literature sources, and/or RACE sources.

Possible SNPs found for NOV9a are listed in Table 9E and possible SNPs for NOV9b are listed in Table 9F.

| **Table 9E: SNPs** | | | | |
|---|---|---|---|---|
| **Variant** | **Nucleotide Position** | **Base Change** | **Amino Acid Position** | **Base Change** |
| 13377086 | 138 | C > T | Silent | N/A |
| 13377087 | 172 | G > A | 50 | Ala > Thr |
| 13377088 | 506 | G > A | 161 | Cys > Tyr |
| 13377089 | 1088 | A > C | 355 | Tyr > Ser |
| 13377084 | 1394 | A > T | 457 | Gln > Leu |
| 13377083 | 1671 | A > G | Silent | N/A |
| 13377085 | 1780 | C > T | 586 | Gln > End |

| **Table 9F: SNPs** | | | |
|---|---|---|---|
| **Consensus Position** | **Depth** | **Base Change** | **PAF** |
| 133 | 41 | A > G | 0.049 |
| 268 | 41 | A > G | 0.049 |
| 324 | 41 | A > G | 0.049 |
| 372 | 41 | A > G | 0.049 |
| 376 | 41 | A > - | 0.049 |
| 456 | 40 | T > C | 0.050 |
| 488 | 32 | A > G | 0.344 |

NOV9a and NOV9b are very closely homologous as is shown in the amino acid alignment in Table 9G.

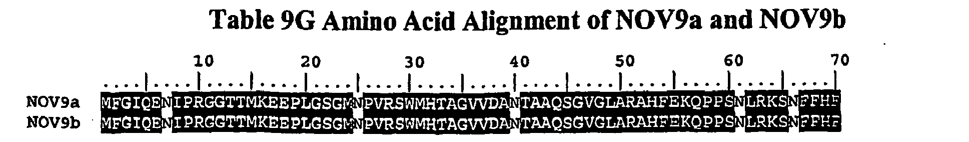

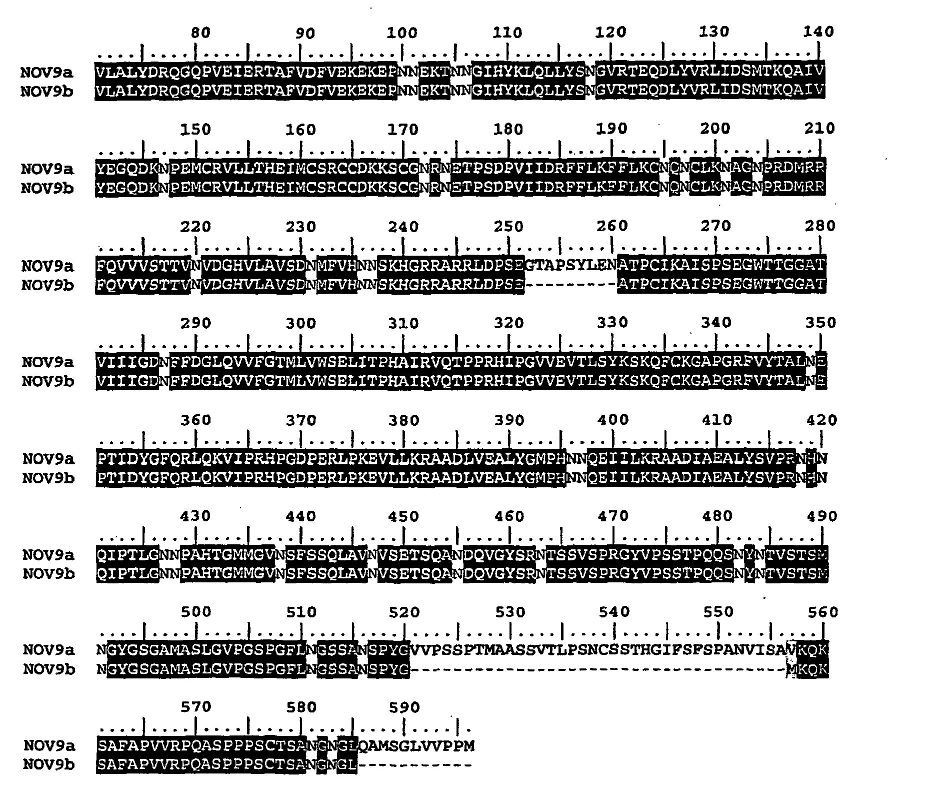

Homologies to any of the above NOV9 proteins will be shared by the other NOV9 proteins insofar as they are homologous to each other as shown above. Any reference to NOV9 is assumed to refer to both of the NOV9 proteins in general, unless otherwise noted.

NOV9a has homology to the amino acid sequences shown in the BLASTP data listed in Table 9H.

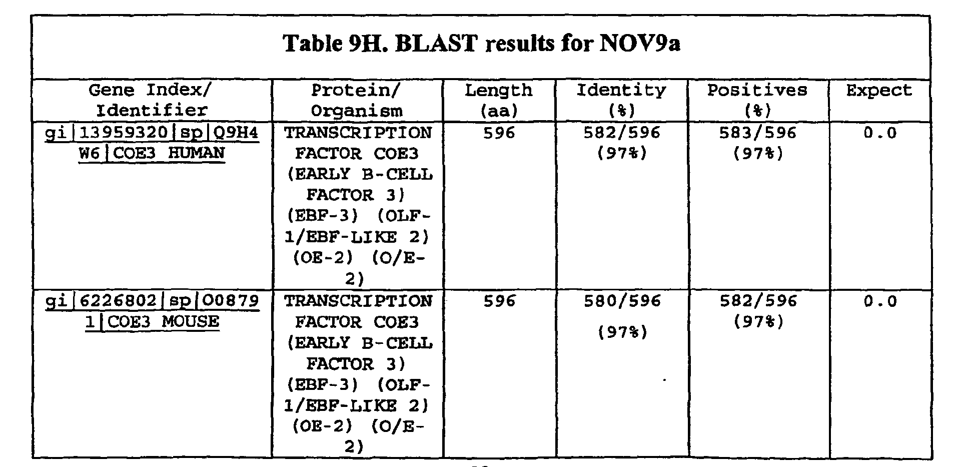

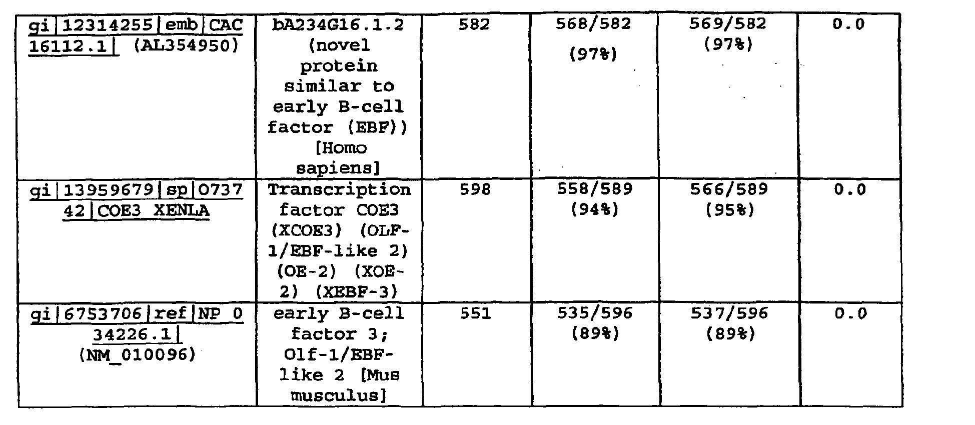

The homology of these sequences is shown graphically in the ClustalW analysis shown in Table 9I.

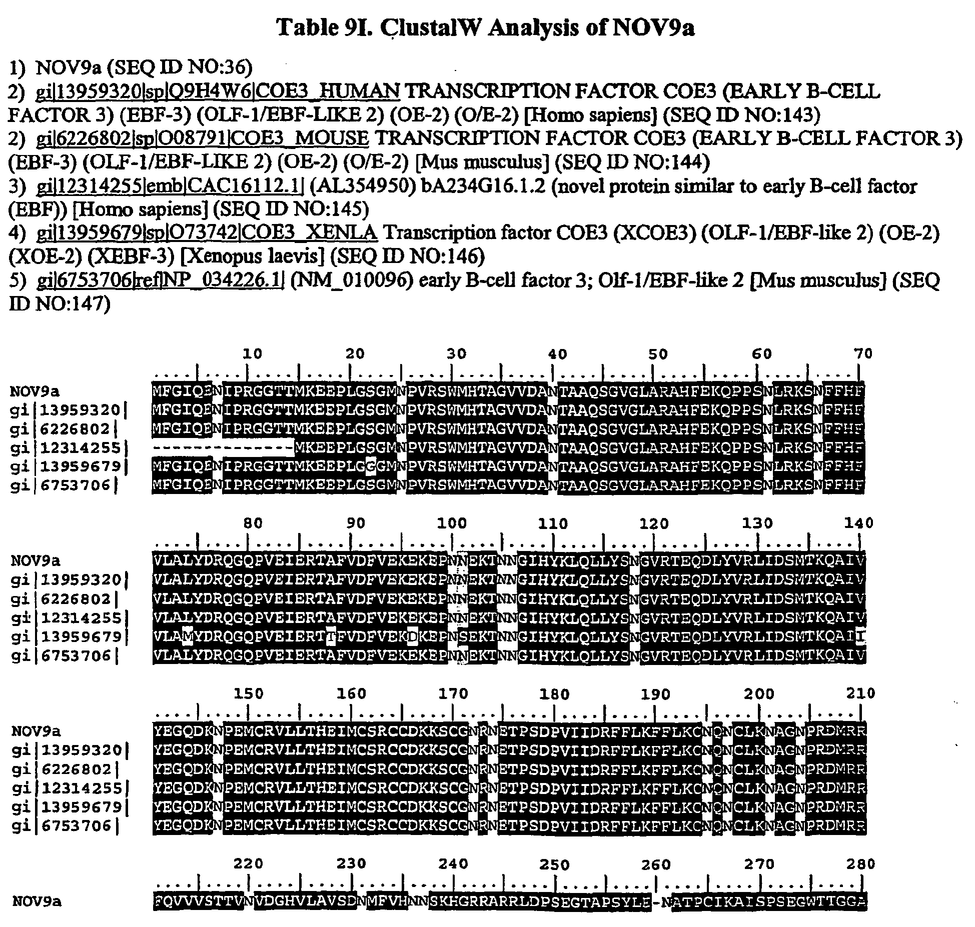

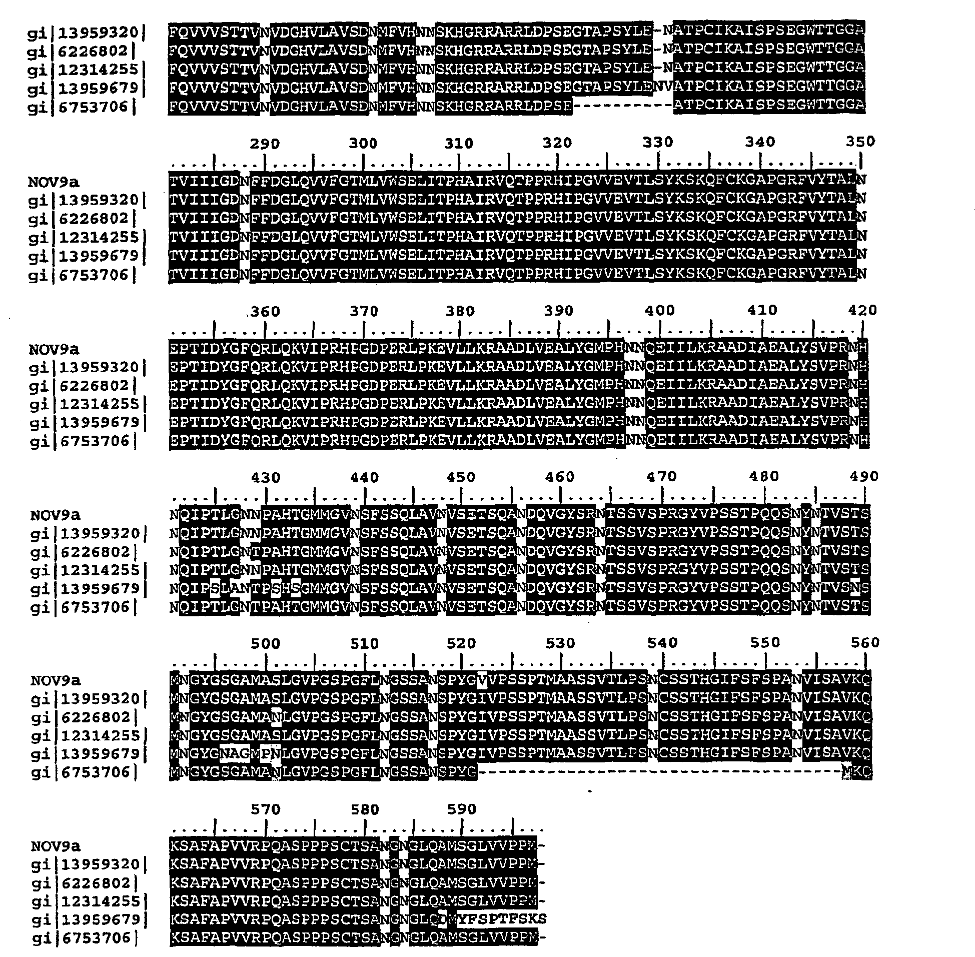

Tables 9J, 9K and 9L lists the domain description from DOMAIN analysis results against NOV9a. This indicates that the NOV9a sequence has properties similar to those of other proteins known to contain these domains.

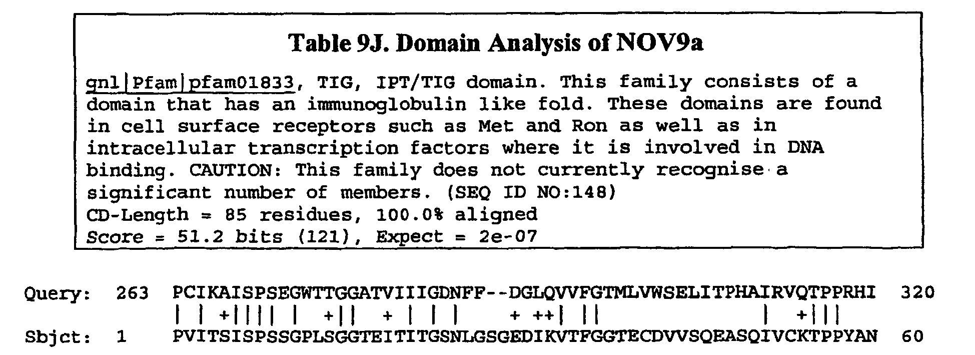

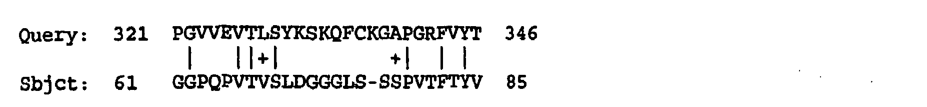

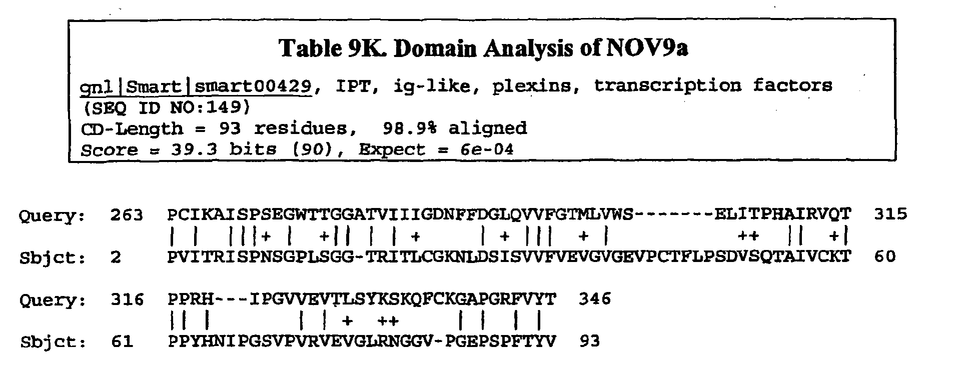

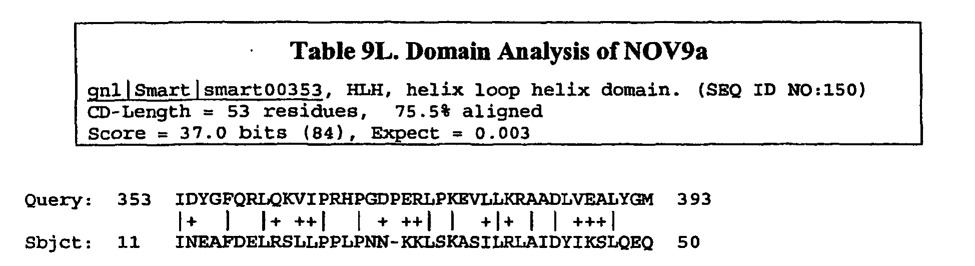

Murine B lymphocytes, adipocytes, and olfactory neurons contain a DNA-binding protein that participates in the regulation of genes encoding tissue-specific components of signal transduction. Purification and cloning of this protein, termed early B-cell factor (EBF), from murine B lymphocytes and independent cloning of a protein, termed Olf-1, from olfactory neuronal cells revealed virtual complete amino acid sequence identity between these proteins.

Early B-cell factor (EBF) is a tissue-specific and differentiation stage-specific DNA-binding protein that participates in the regulation of the pre-B and B lymphocyte-specific MB1 gene. Travis et al. (Molec. Cell. Biol. 13: 3392-3400, 1993) purified the mouse Ebf protein from pre-B cells and found that it is composed of two 62- to 65-kD subunits. Hagman et al. (Genes Dev. 7: 760-773, 1993) determined partial amino acid sequences of Ebf and used them to isolate mouse pre-B-cell cDNAs encoding Ebf. The predicted 591-amino acid protein has 2 functional domains: an N-terminal cysteine-rich region essential for DNA binding, and a C-terminal dimerization region containing two 15-amino acid repeats with similarity to the dimerization domains of basic helix-loop-helix (bHLH) proteins. The calculated molecular mass of the encoded Ebf protein is 64.4 kD. The authors found that recombinant Ebf binds to DNA as a homodimer, forms complexes with the Mb1 promoter, and is a strong activator of transcription. Northern blot analysis detected multiple Ebf transcripts in pre-B- and early B-cell lines but not in other hematopoietic cells. S 1 nuclease protection analysis of adult mouse RNAs showed high levels of Ebf expression in lymph node, spleen, and adipose tissues and low levels in several nonlymphoid tissues. By Southern blot analysis of somatic cell hybrid DNAs using a murine Ebf cDNA as a probe, and by fluorescence in situ hybridization using human genomic cosmids, Milatovich et al. (Genome 5: 211-215, 1994) mapped the human EBF gene to 5q34. The study mapped the mouse Ebf gene to proximal chromosome 11 by Southern blot analysis of somatic cell hybrid DNAs and by analysis of recombinant inbred strains.

The mammalian olfactory system has the remarkable ability to detect odorants with high sensitivity and specificity. The initial events in the olfactory signal transduction pathway occur in the specialized cilia of the sensory neurons. Unlike other neurons, the olfactory sensory cells are continually replaced throughout adult life. Cells within the olfactory epithelium follow an orderly developmental program resulting in the high level of expression of gene products essential for odorant signal transduction. The mature neurons express several olfactory-specific genes, some of which appear to mediate the odorant signal transduction cascade. Evidence supporting the involvement of a G protein-coupled receptor pathway in odorant signal transduction was provided by the isolation of olfactory-specific components that correspond to each step in the pathway, e.g., G-alpha-olf. Additional olfactory neuron-specific genes have been identified. The establishment of the mature olfactory neuronal phenotype probably results from the coordinated expression of olfactory-specific genes. In the investigation of these genes, Wang and Reed (Nature 364: 121-126, 1993) found that each contains at least 1 binding site for the DNA-binding protein Olf1. The binding of an olfactory-specific factor, Olf1, was first described in the olfactory marker protein gene (OMP). Olf1 activity was detectable in nuclear extracts from nasal epithelium and absent from nuclear extracts of a variety of other tissues. Using a novel genetic selection in yeast, Wang and Reed (1993) isolated a cDNA for the rat transcriptional activator Olf1, which binds to the regulatory sequences of several olfactory-specific genes. Expressed exclusively in the olfactory receptor neurons and their precursors, the Olf1 protein contains a new helix-loop-helix motif and functions as an apparent homodimer. They suggested that Olf1 may be the first member of a family of related proteins that may direct cellular differentiation in a variety of neuronal tissues.

Using homology screening methods, two additional Olf-1/EBF-like cDNAs were identified from a mouse embryonic cDNA library. The Olf-1/EBF-like (O/E) proteins O/E-1, O/E-2, and O/E-3 define a family of transcription factors that share structural similarities and biochemical activities. Although these O/E genes are expressed within olfactory epithelium in an identical pattern, they exhibit different patterns of expression in the developing nervous system. Although O/E-1 mRNA is present in several tissues in addition to olfactory neurons and developing B-cells, O/E-2 and O/E-3 are expressed at high levels only in olfactory tissue. In O/E-1 knock-out animals, the presence of two additional O/E family members in olfactory neurons may provide redundancy and allow normal olfactory neurodevelopment. Further, the identification of the O/E family of HLH transcription factors and their embryonic expression patterns suggest that the O/E proteins may have a more general function in neuronal development.

The independent cloning of rodent EBF/Olf-1 and Drosophila Collier has defined a family of transcription factors, the Collier or COE family. COE proteins have various functions in different organisms. Proteins currently known to belong to this family include: mammalian COE1 (OLF-1; EBF-1; OE-1), COE2 (EBF-2; OE-3) and COE3 (EBF-3; OE-2); Xenopus XCOE2 and XCOE3; zebrafish ZCOE2; Drosophila Collier; and Caenorhabditis elegans UNC-3. In mouse, COE1 has a role in B-cell differentiation, and could also perform a role in neuronal differentiation. All three COE are expressed in immature olfactory neuronal precursors and mature olfactory neurons as well as in developping nervous system during embryogenesis. In Drosophila, Collier is involved in the formation of the embryonic somatic muscle DA3, in the patterning of the wing by mediating Hedgehog activity. It could also act as a second-level regulator in the patterning of embryonic head. In Xenopus, XCOE2 is expressed in precursors of primary neurons, and may play a pivotal role in the transcriptional cascade specifying primary neurons in embryo. It promotes neuronal differentiation by activating XNeuroD expression. In Zebrafish, ZCOE2 is expressed in a subset of primary neuroblasts in the spinal cord and at later stages is a marker of the olfactory placodes. Caenorhabditis elegans mutants for UNC-3 move abnormally, suggesting that UNC-3 might regulate expression of genes involved in growth cone pioneering long the ventral cord or in fasciculation.

The NOV9 nucleic acid of the invention encoding a Early B-Cell Factor-like protein includes the nucleic acid whose sequence is provided in Tables 9A and 9C, or a fragment thereof. The invention also includes a mutant or variant nucleic acid any of whose bases may be changed from the corresponding base shown in Tables 9A and 9C while still encoding a protein that maintains its Early B-Cell Factor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 6% of the NOV9 residues may be so changed.

The NOV9 protein of the invention includes the Early B-Cell Factor-like protein whose sequence is provided in Tables 9B and 9D. The invention also includes a mutant or variant protein any of whose residues may be changed from the corresponding residue shown in Tables 9B and 9D while still encoding a protein that maintains its Early B-Cell Factor-like activities and physiological functions, or a functional fragment thereof. In the mutant or variant protein, up to about 1% of the NOV9 bases may be so changed.

The NOV9 nucleic acids and proteins of the invention are useful in potential diagnostic and therapeutic applications implicated in various diseases and disorders described below and/or other pathologies. For example, the compositions of the present invention will have efficacy for treatment of patients suffering from: Von Hippel-Lindau (VHL) syndrome, Alzheimer's disease, stroke, tuberous sclerosis, hypercalceimia, Parlanson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neuroprotection, multiple sclerosis, myasthenia gravis, diabetes, obesity, neuronal development and other diseases, disorders and conditions of the like.

NOV9 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV9 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV10

A disclosed NOV10 nucleic acid of 3892 nucleotides (also referred to as CG57389-01) encoding a novel High-Affinity CAMP Specific and IBMX-Insensitive-like protein is shown in Table 10A. An open reading frame was identified beginning with an ATG initiation codon at nucleotides 137-139 and ending with a TAG codon at nucleotides 2624-2626. Putative untranslated regions upstream from the intitation codon and downstream from the termination codon are underlined in Table 10A, and the start and stop codons are in bold letters.

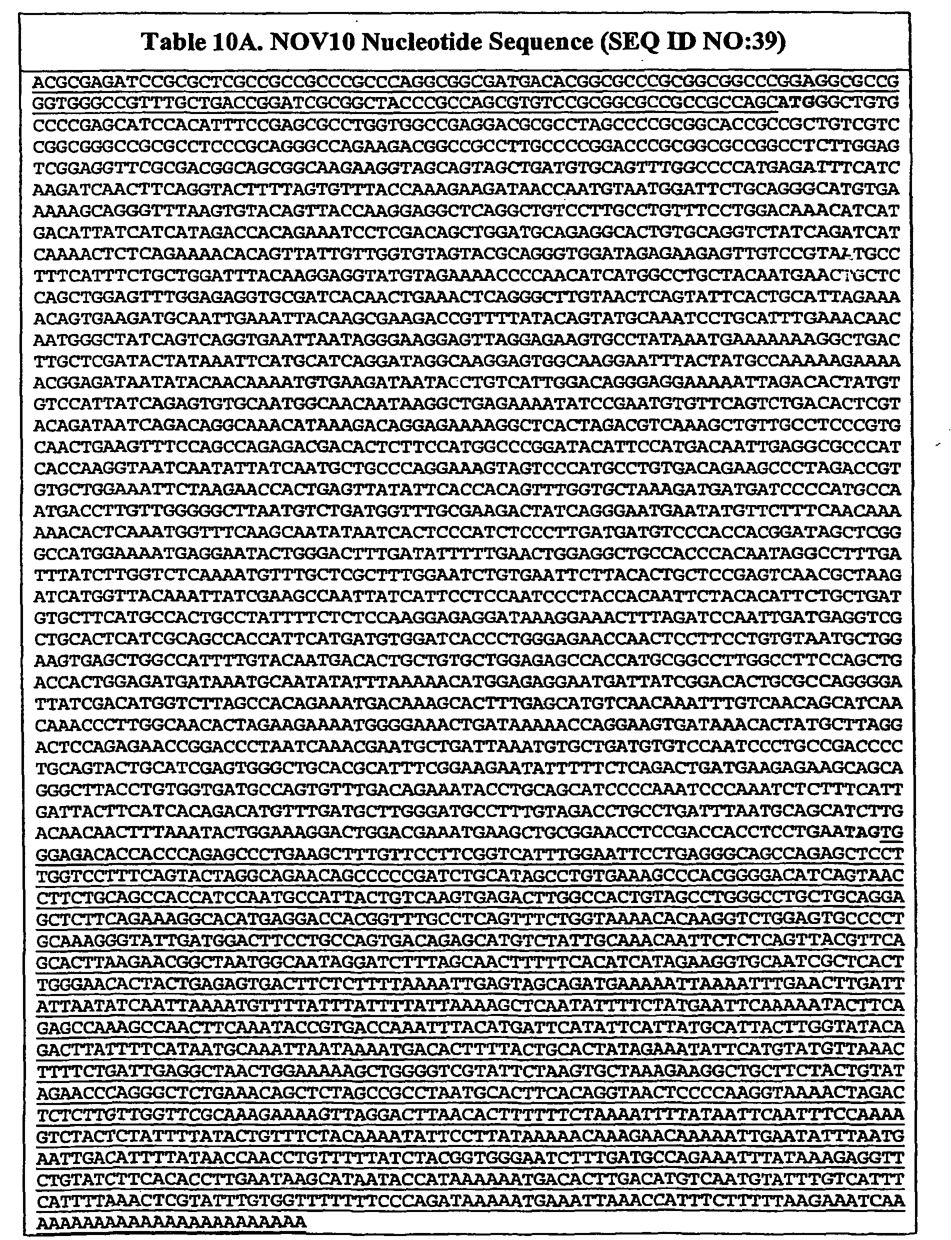

The NOV10 nucleic acid was identified on chromosome 15 and has 3393 of 3396 bases (99%) identical to a gb:GENBANK-ID:AF056490|acc:AF056490.1 mRNA from *Homo sapiens* (Homo sapiens cAMP-specific phosphodiesterase 8A (PDE8A) mRNA, partial cds) (E = 0.0).

A disclosed NOV10 polypeptide (SEQ ID NO:40) encoded by SEQ ID NO:39 is 829 amino acid residues and is presented using the one-letter code in Table 10B. Signal P, Psort and/or Hydropathy results predict that NOV10 contains a signal peptide and is likely to be localized to the cytoplasm with a certainty of 0.4500.

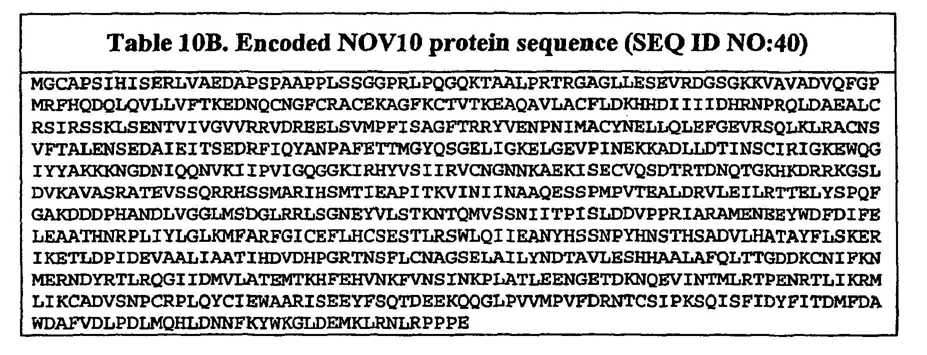

The NOV10 amino acid sequence has 712 of 713 amino acid residues (99%) identical to, and 713 of 713 amino acid residues (100%) similar to, the 713 amino acid residue ptnr:SWISSNEW-ACC:O60658 protein from *Homo sapiens* (Human) (High-Affinity CAMP-Specific And IBMX-Insensitive 3',5'-Cyclic Phosphodiesterase 8A (EC 3.1.4.17) (E = 0.0).

NOV10 is expressed in at least the following tissues: kidney, lung, uterus, ovary and heart. This information was derived by determining the tissue sources of the sequences that were included in the invention including but not limited to SeqCalling sources, Public EST sources, genomic clone sources, literature sources, and/or RACE sources.

NOV10 has homology to the amino acid sequences shown in the BLASTP data listed in Table 10C.

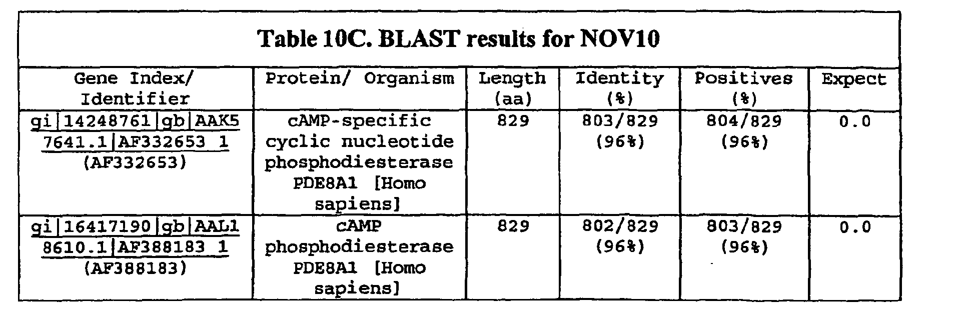

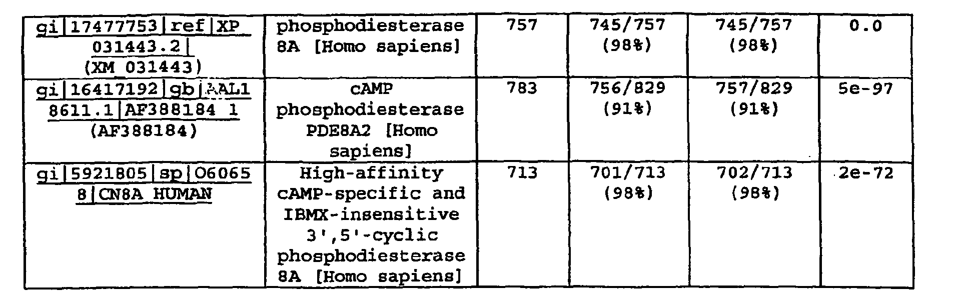

The homology of these sequences is shown graphically in the ClustalW analysis shown in Table 10D.

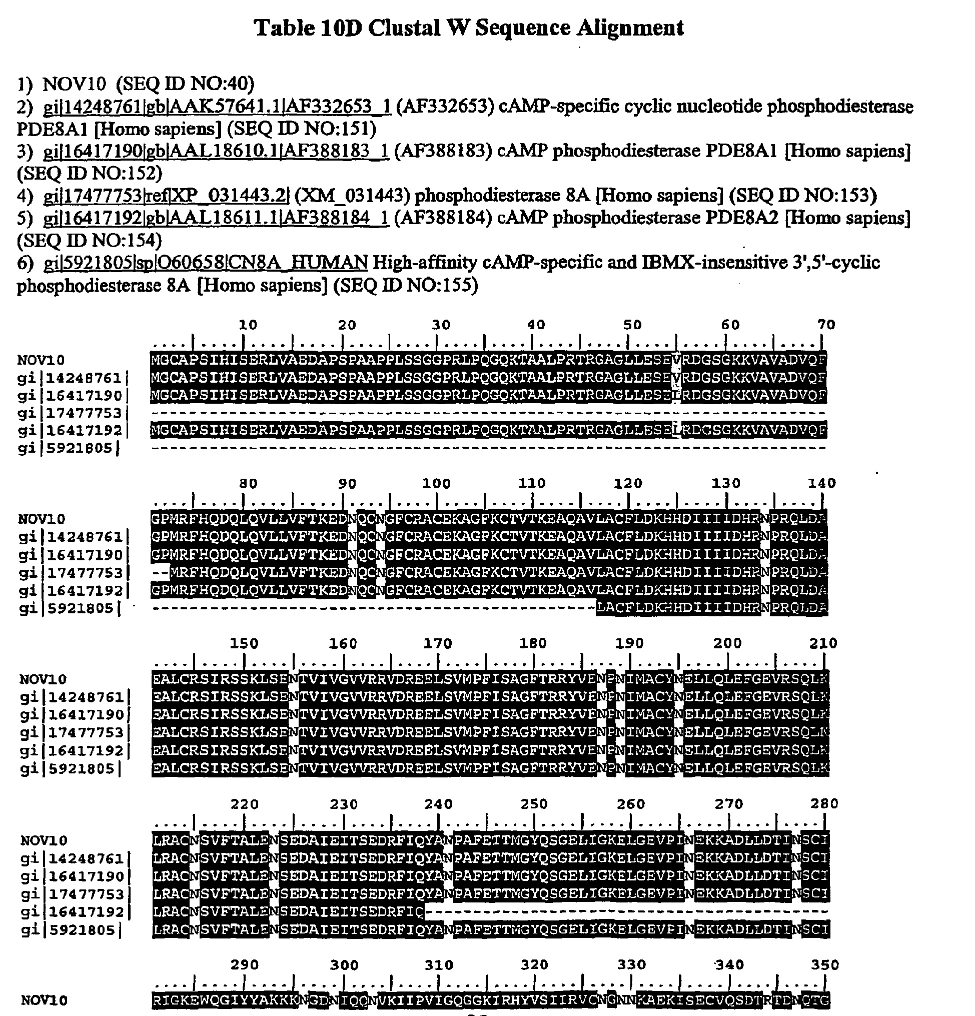

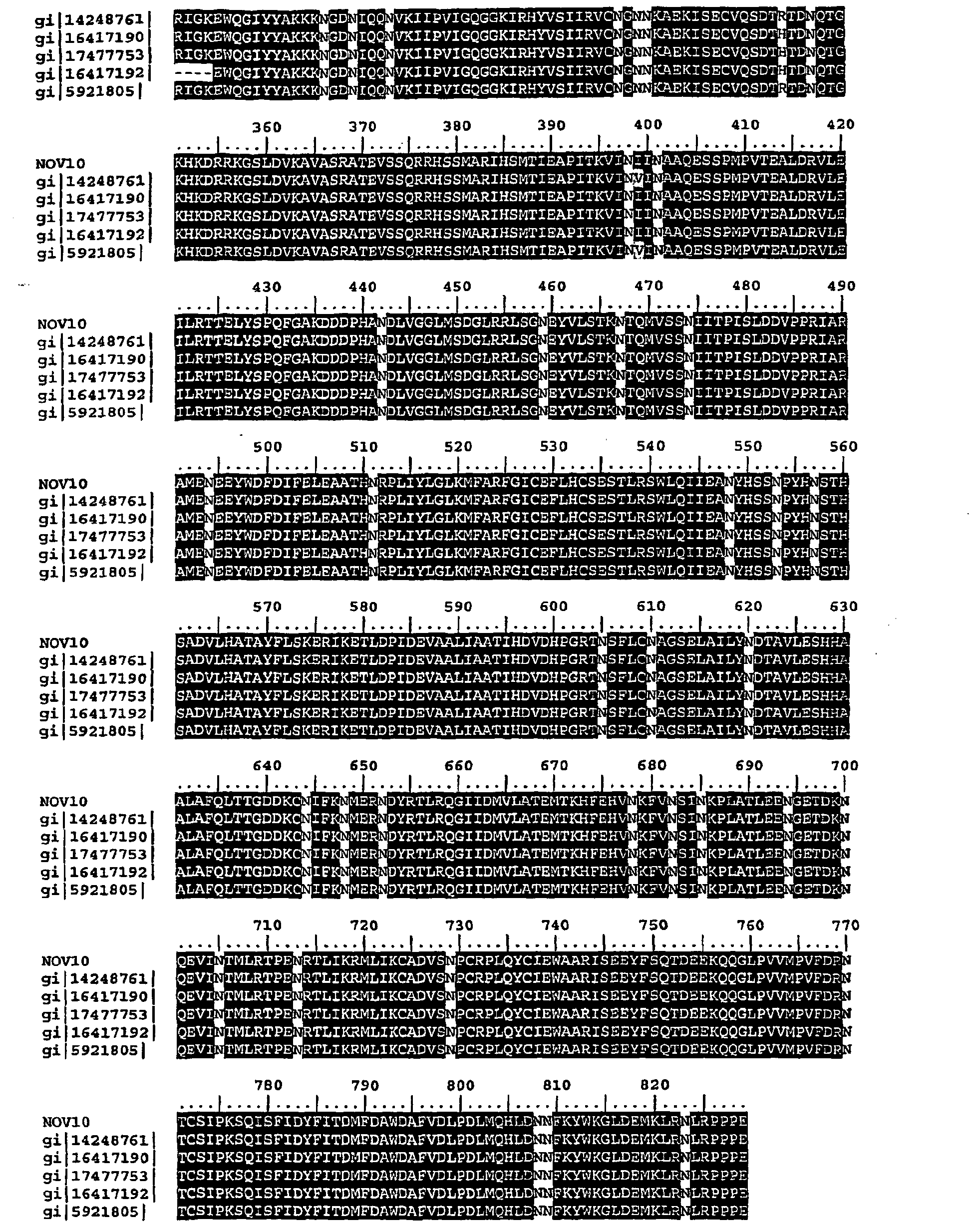

Tables 10E, 10F and 10G list the domain description from DOMAIN analysis results against NOV10. This indicates that the NOV10 sequence has properties similar to those of other proteins known to contain these domains.

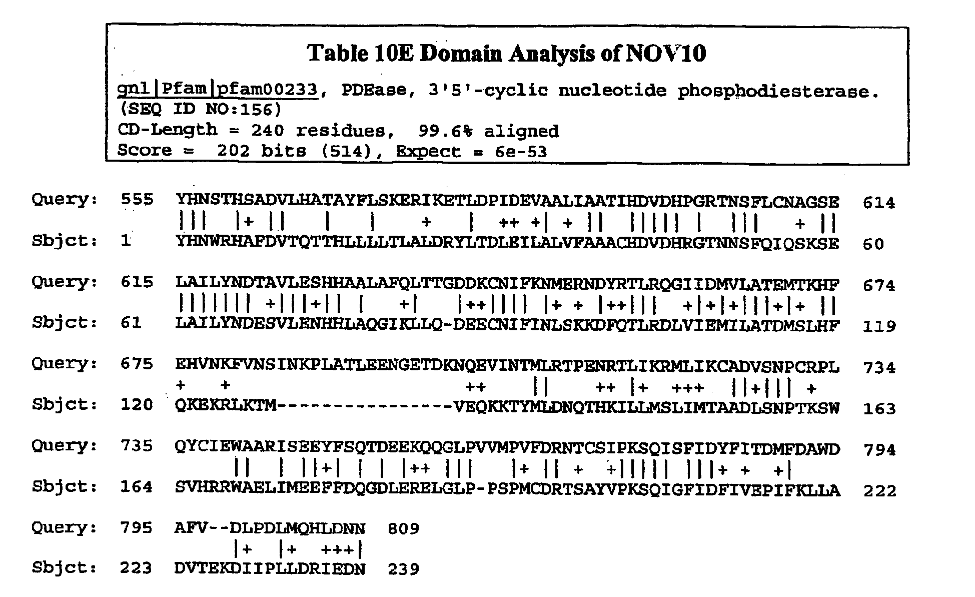

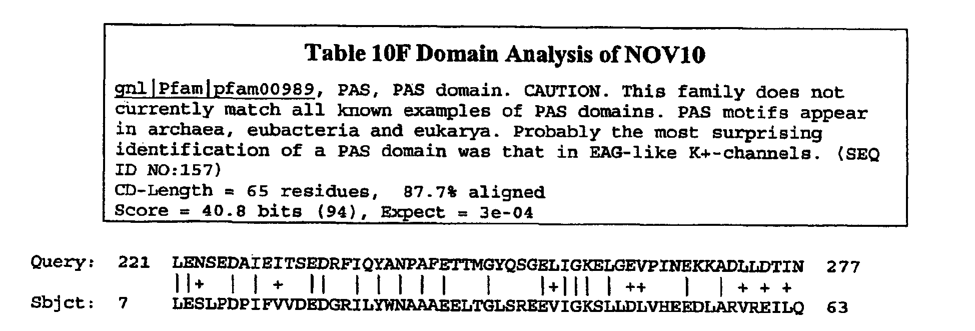

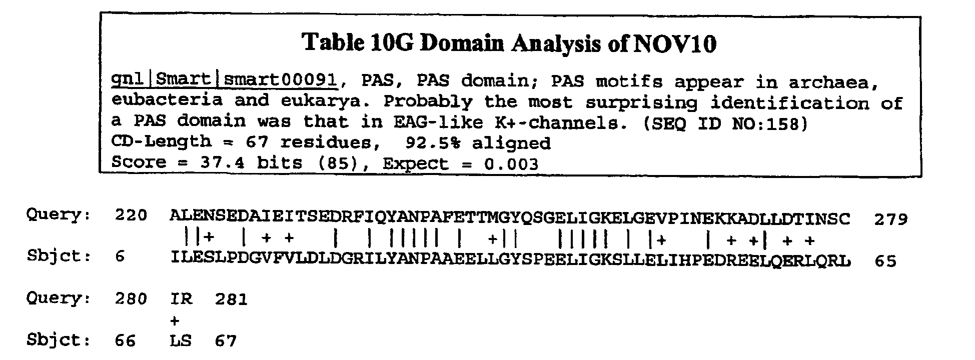

The NOV10 nucleic acid of the invention encoding a High-Affinity CAMP Specific and IBMX-Insensitive-like protein includes the nucleic acid whose sequence is provided in Table 10A, or a fragment thereof. The invention also includes a mutant or variant nucleic acid any of whose bases may be changed from the corresponding base shown in Table 10A while still encoding a protein that maintains its High-Affinity CAMP Specific and IBMX-Insensitive-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1% of the residues may be so changed.

The NOV10 protein of the invention includes the High-Affnity CAMP Specific and IBMX-Insensidve-like protein whose sequence is provided in Table 10B. The invention also includes a mutant or variant protein any of whose residues may be changed from the corresponding residue shown in Table 10B while still encoding a protein that maintains its High-Affinity CAMP Specific and IBMX-Insensitive-like activities and physiological functions, or a functional fragment thereof. In the mutant or variant protein, up to about 1% of the bases may be so changed.

The NOV10 nucleic acids and proteins of the invention are useful in potential diagnostic and therapeutic applications implicated in various diseases and disorders described below and/or other pathologies. For example, the compositions of the present invention will have efficacy for treatment of patients suffering from: Cardiomyopathy, Atherosclerosis, Hypertension, Congenital heart defects, Aortic stenosis, Atrial septal defect (ASD), Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity,Transplantation, Endometriosis, Fertility, Systemic lupus erythematosus , Autoimmune disease, Asthma, Emphysema, Scleroderma, allergy, Diabetes, Autoimmune disease, Renal artery stenosis, Interstitial nephritis, Glomerulonephritis, Polycystic kidney disease, Systemic lupus erythematosus, Renal tubular acidosis, IgA nephropathy, Hypercalceimia, Lesch-Nyhan syndrome and other diseases, disorders and conditions of the like.

NOV 10 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV10 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV11

A disclosed NOV11 nucleic acid of 6524 nucleotides (also referred to as CG57337-01) encoding a novel KIAA0216-like protein is shown in Table 11A. An open reading frame was identified beginning with an ATG initiation codon at nucleotides 485-487 and ending with a TAG codon at nucleotides 5273-5275. Putative untranslated regions upstream from the intitation codon and downstream from the termination codon are underlined in Table 11A, and the start and stop codons are in bold letters.

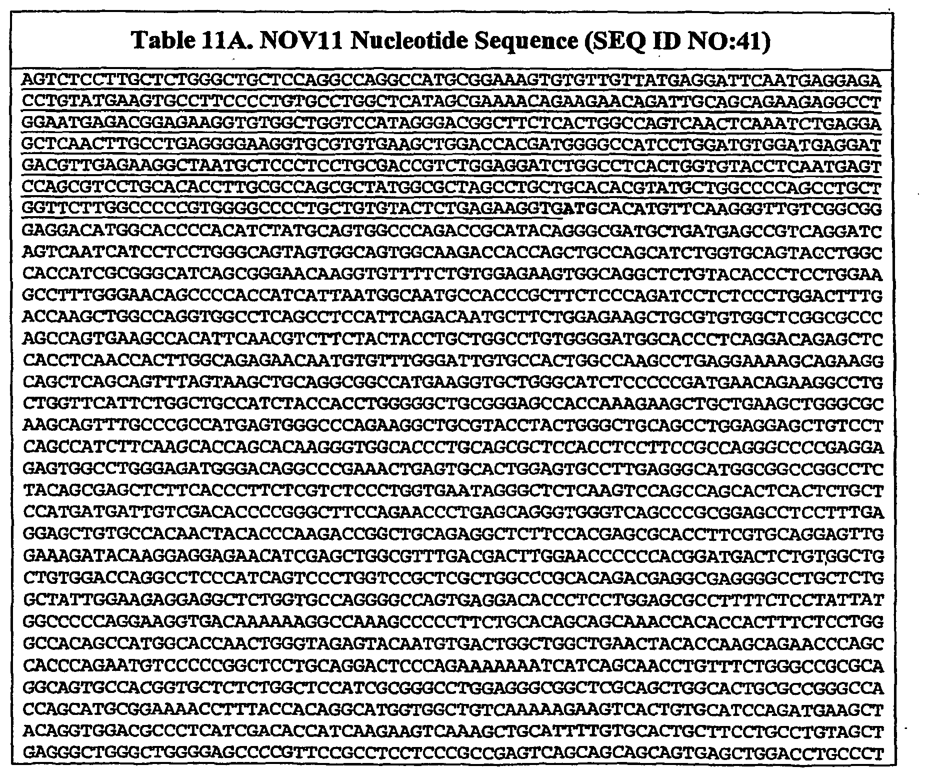

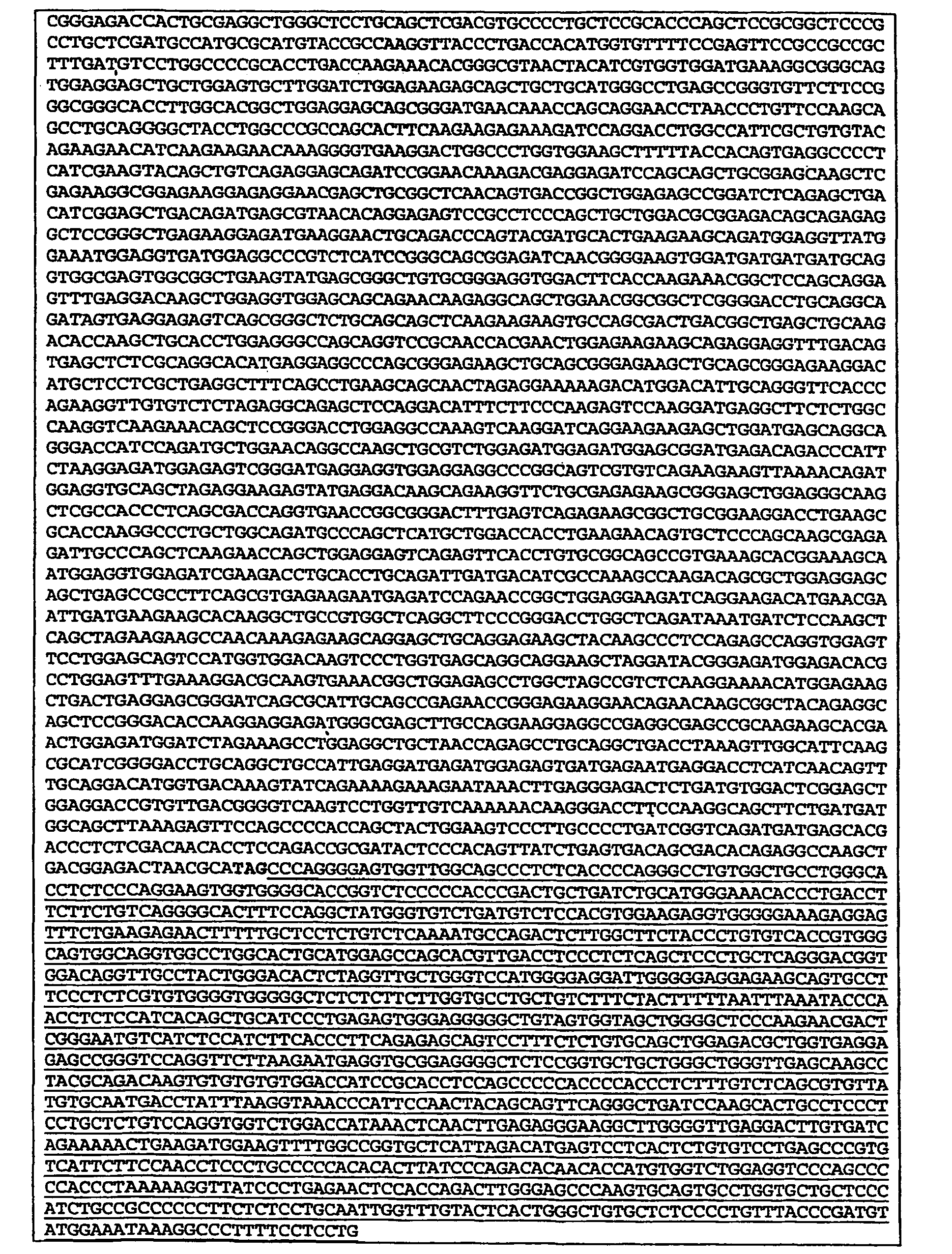

The NOV11 nucleic acid was identified on chromosome 17 and has 4969 of 4979 bases (99%) identical to a gb:GENBANK-ID:D86970|acc:D86970.1 mRNA from *Homo sapiens* (Human mRNA for KIAA0216 gene, complete cds) (E = 0.0).

A disclosed NOV11 polypeptide (SEQ ID NO:42) encoded by SEQ ID NO:41 is 1596 amino acid residues and is presented using the one-letter code in Table 11B. Signal P, Psort and/or Hydropathy results predict that NOV11 contains a signal peptide and is likely to be localized to the nucleus with a certainty of 0.9800. Although SignalP, Psort and/or hydropathy suggest that the NOV11 protein may be localized in the nucleus, the NOV11 protein is similar to the myosin family, some members of which are expected to have intracellular sub-cellular localization (Trends Cell Biol 1998 Apr;8(4):138-41). Therefore it is likely that NOV11 is available at the same sub-cellular localization and hence accessible to a diagnostic probe and for various therapeutic applications.

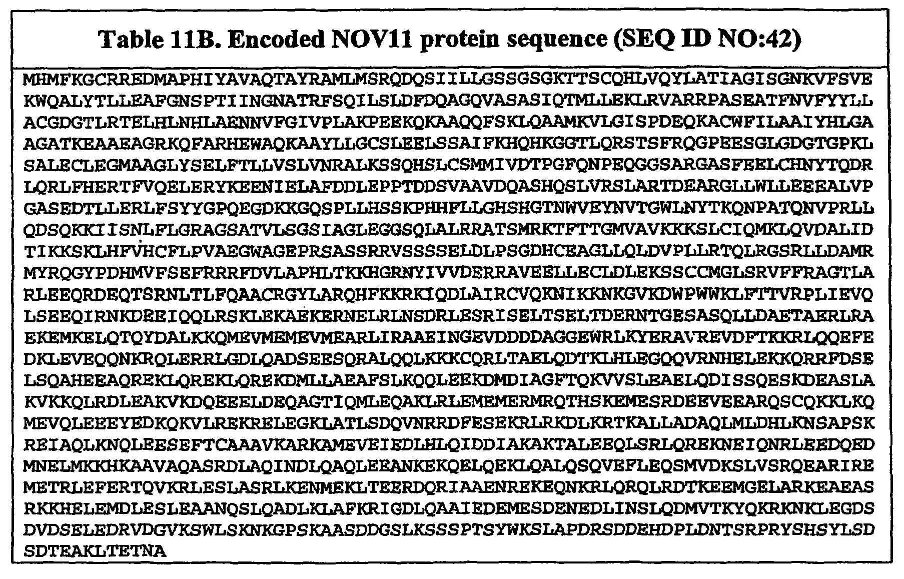

The NOV11 amino acid sequence has 1579 of 1596 amino acid residues (98%) identical to, and 1581 of 1596 amino acid residues (99%) similar to, the 1581 amino acid residue ptnr:SPTREMBL-ACC:Q92614 protein from *Homo sapiens* (Human) (MYELOBLAST KIAA0216) (E = 0.0). The NOV11 amino acid sequence has additional 15 internal amino acids, when compared to ptnr:SPTREMBL-ACC:Q92614 protein from *Homo sapiens* (Human) (MYELOBLAST KIAA0216).

NOV11 is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus, aorta, ascending colon, bronchus, cervix, colon, coronary artery, epidermis, gall bladder, hypothalamus, lung, lymph node, lymphoid tissue, muscle, ovary, peripheral blood, pharynx, pineal gland, retina, right cerebellum, tonsils and whole organism. This information was derived by determining the tissue sources of the sequences that were included in the invention including but not limited to SeqCalling sources, Public EST sources, genomic clone sources, literature sources, and/or RACE sources.

Possible SNPs found for NOV11 are listed in Tables 11C and 11D.

| **Table 11C: SNPs** | | | | |
|---|---|---|---|---|
| **Variant** | **Nucleotide Position** | **Base Change** | **Amino Acid Position** | **Base Change** |
| 13377093 | 491 | A > G | 3 | Met > Val |
| 13377092 | 570 | T > C | 29 | Met > Thr |
| 13377091 | 1783 | G > A | Silent | N/A |
| 13377090 | 3657 | T > C | 1058 | Val > Ala |
| 13374495 | 4848 | C > T | 1455 | Ala > Val |
| 13374496 | 4939 | T > C | Silent | N/A |
| 13374497 | 4998 | A > T | 1505 | Lys > Met |
| 13374498 | 5114 | A > G | 1544 | Ser > Gly |

| **Table 11D: SNPs** | | | |
|---|---|---|---|
| **Consensus Position** | **Depth** | **Base Change** | **PAF** |
| 455 | 24 | C > T | 0.083 |
| 546 | 27 | T > C | 0.074 |
| 605 | 27 | A > T | 0.074 |
| 721 | 27 | A > G | 0.074 |
| 753 | 26 | C > T | 0.077 |
| 891 | 13 | C > T | 0.154 |

NOV11 has homology to the amino acid sequences shown in the BLASTP data listed in Table 11E.

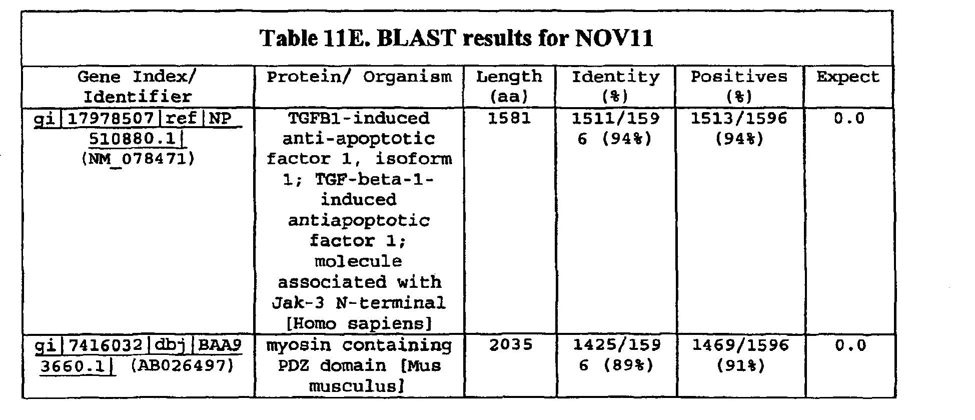

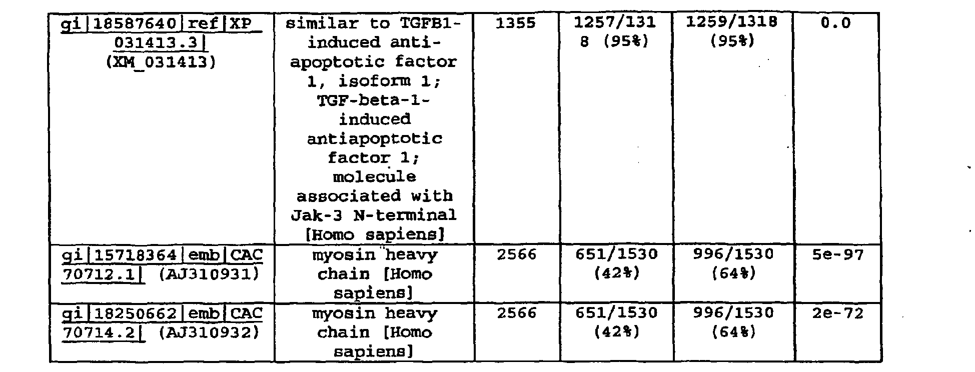

The homology of these sequences is shown graphically in the ClustalW analysis shown in Table 11F.

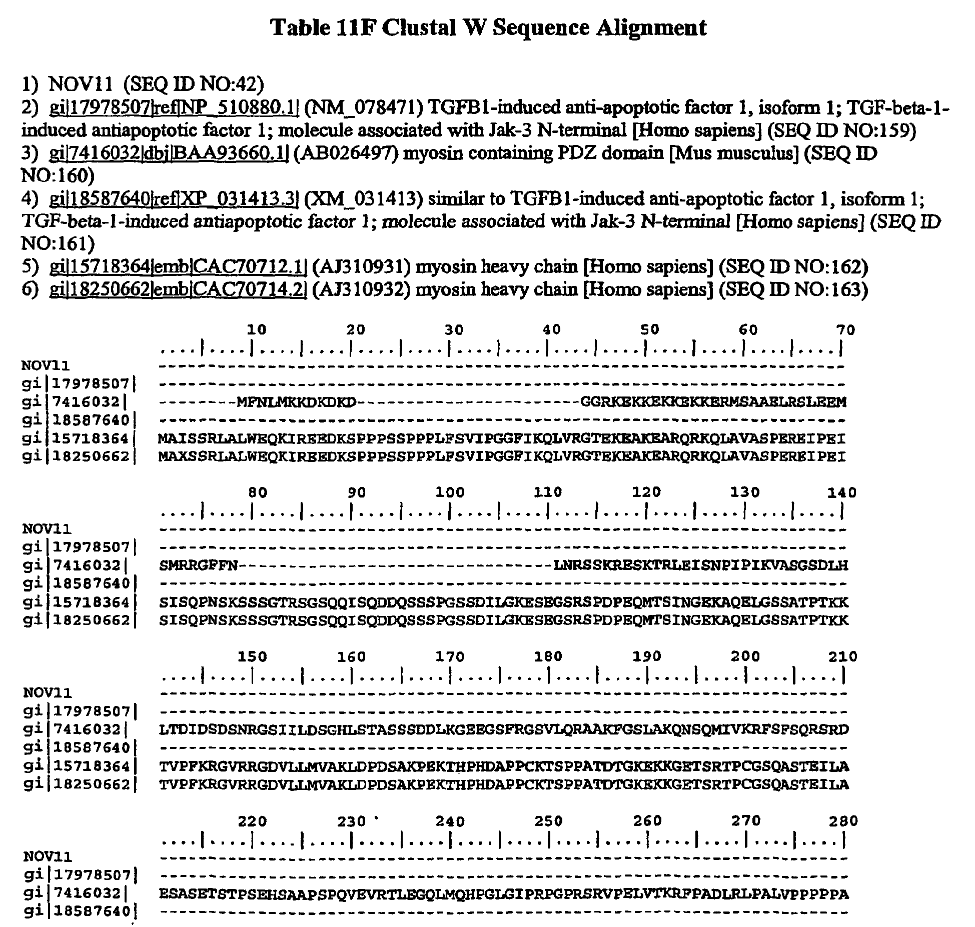

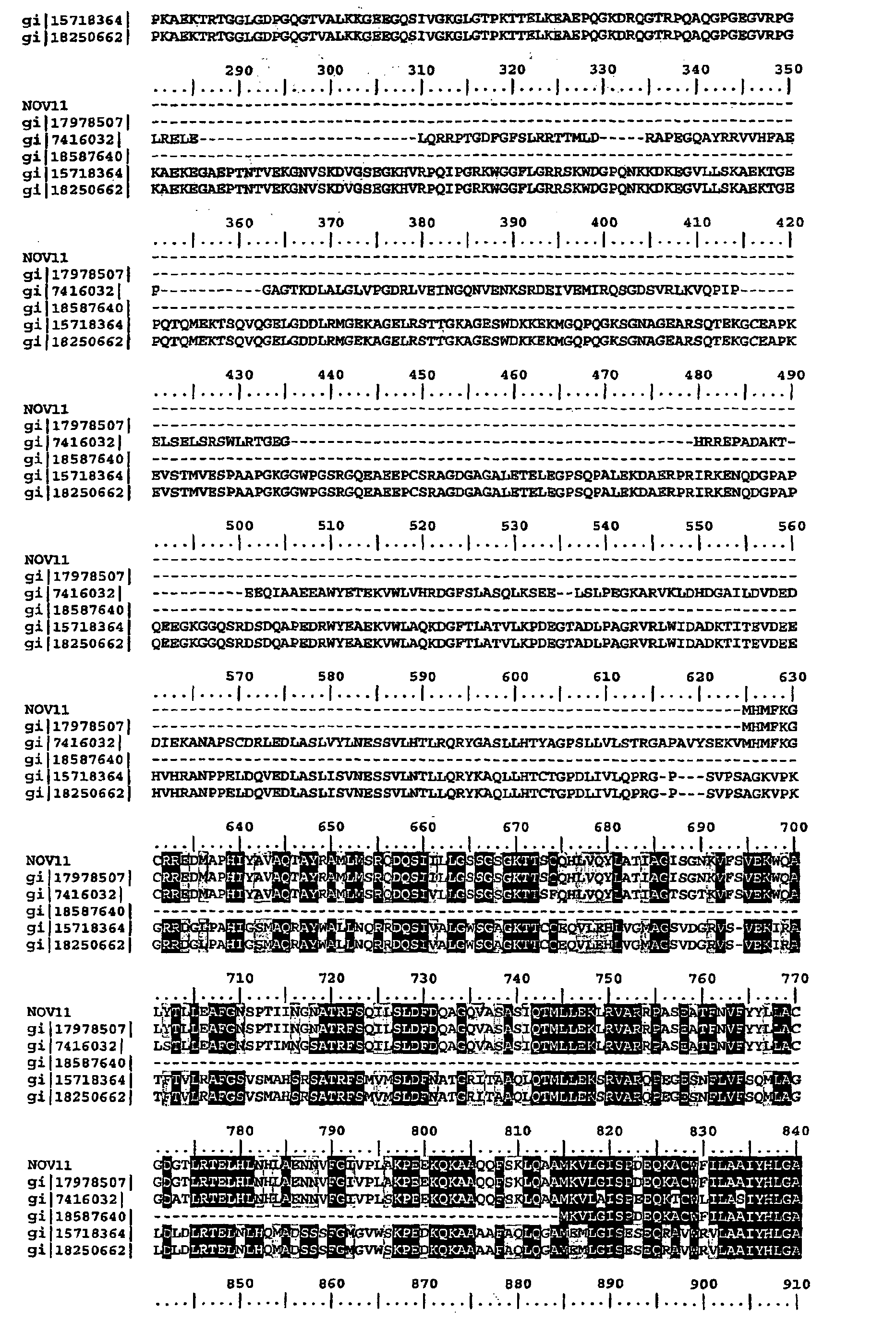

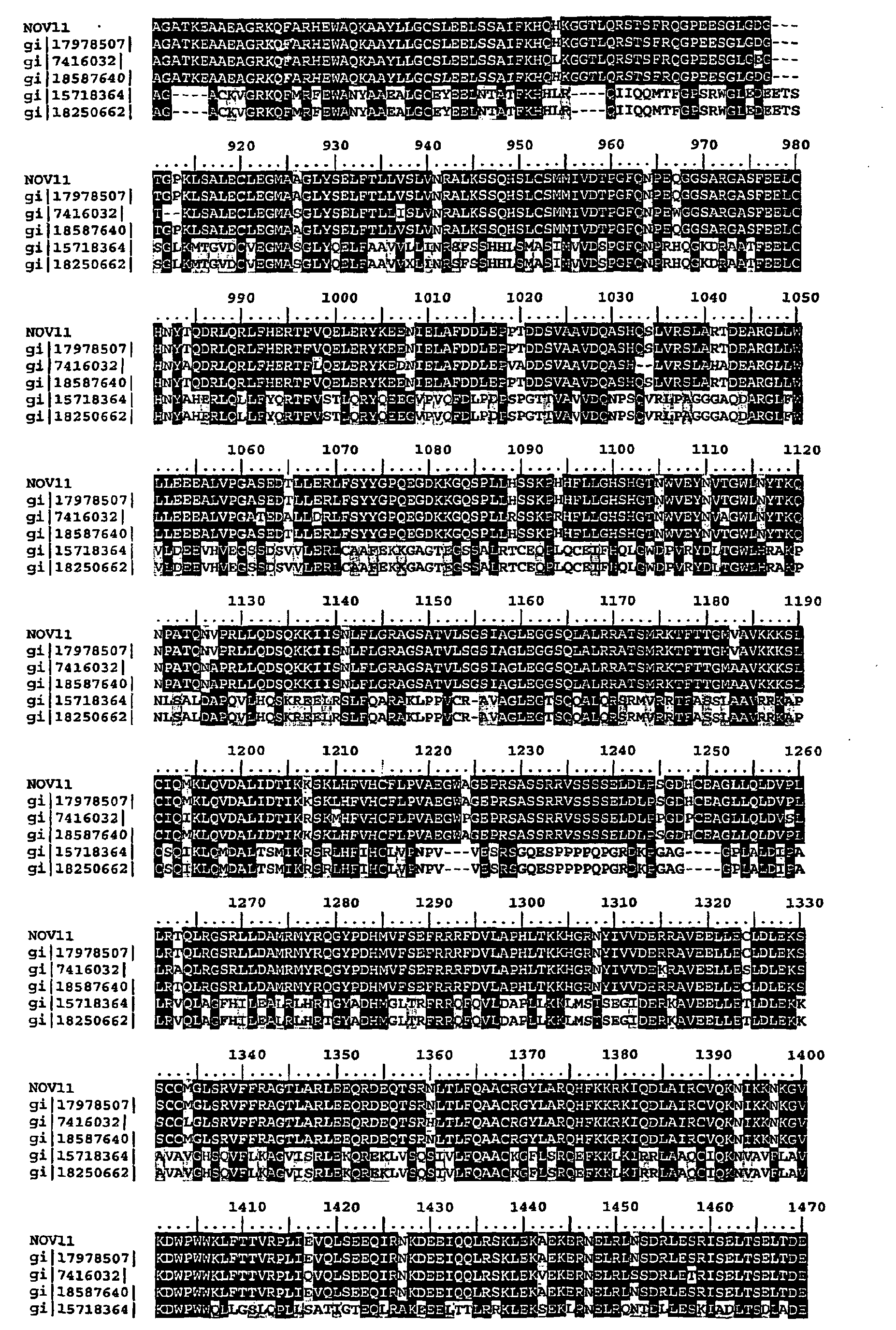

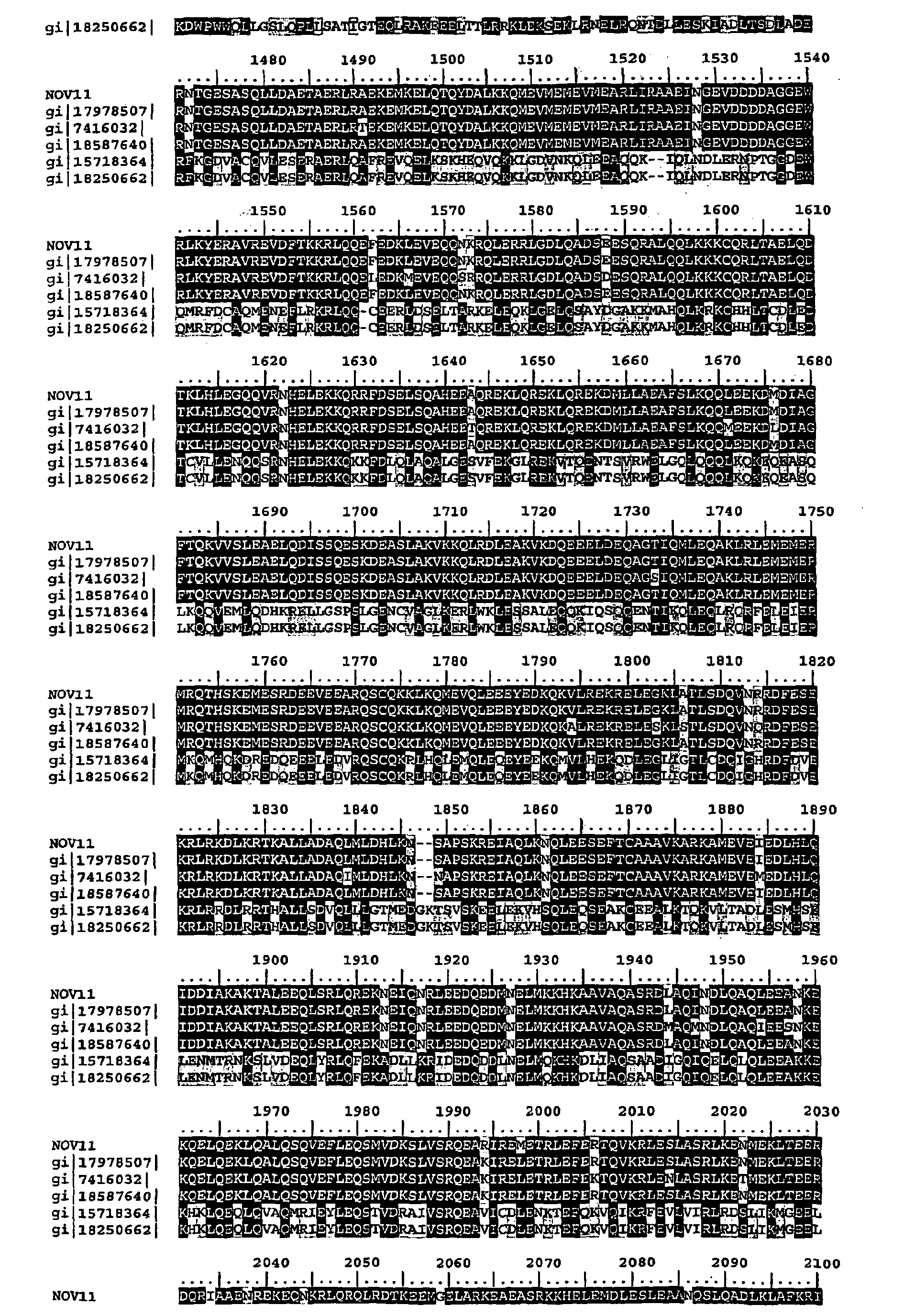

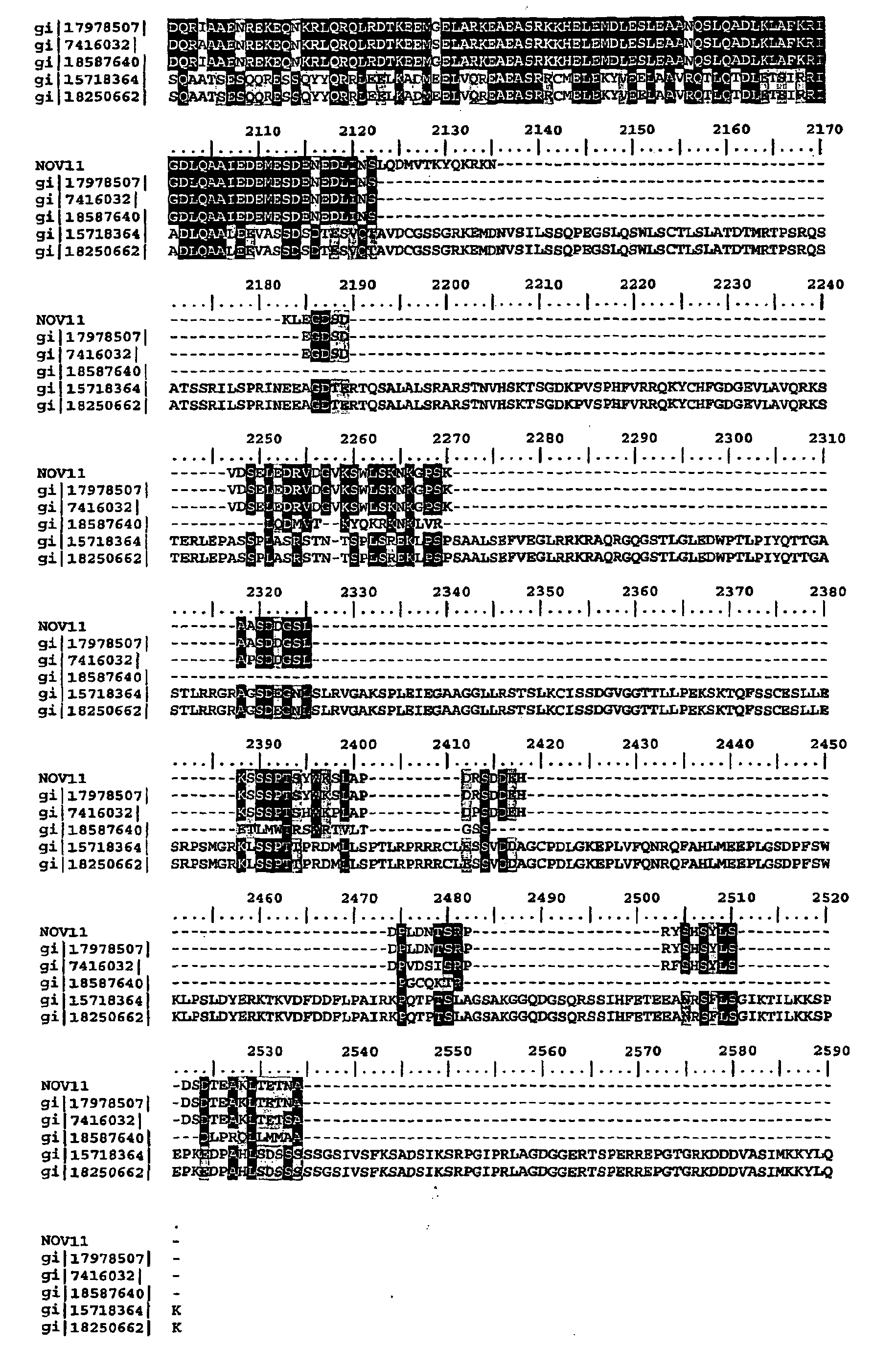

Tables 11G, 11H, 11I and 11J list the domain description from DOMAIN analysis results against NOV11. This indicates that the NOV11 sequence has properties similar to those of other proteins known to contain these domains.

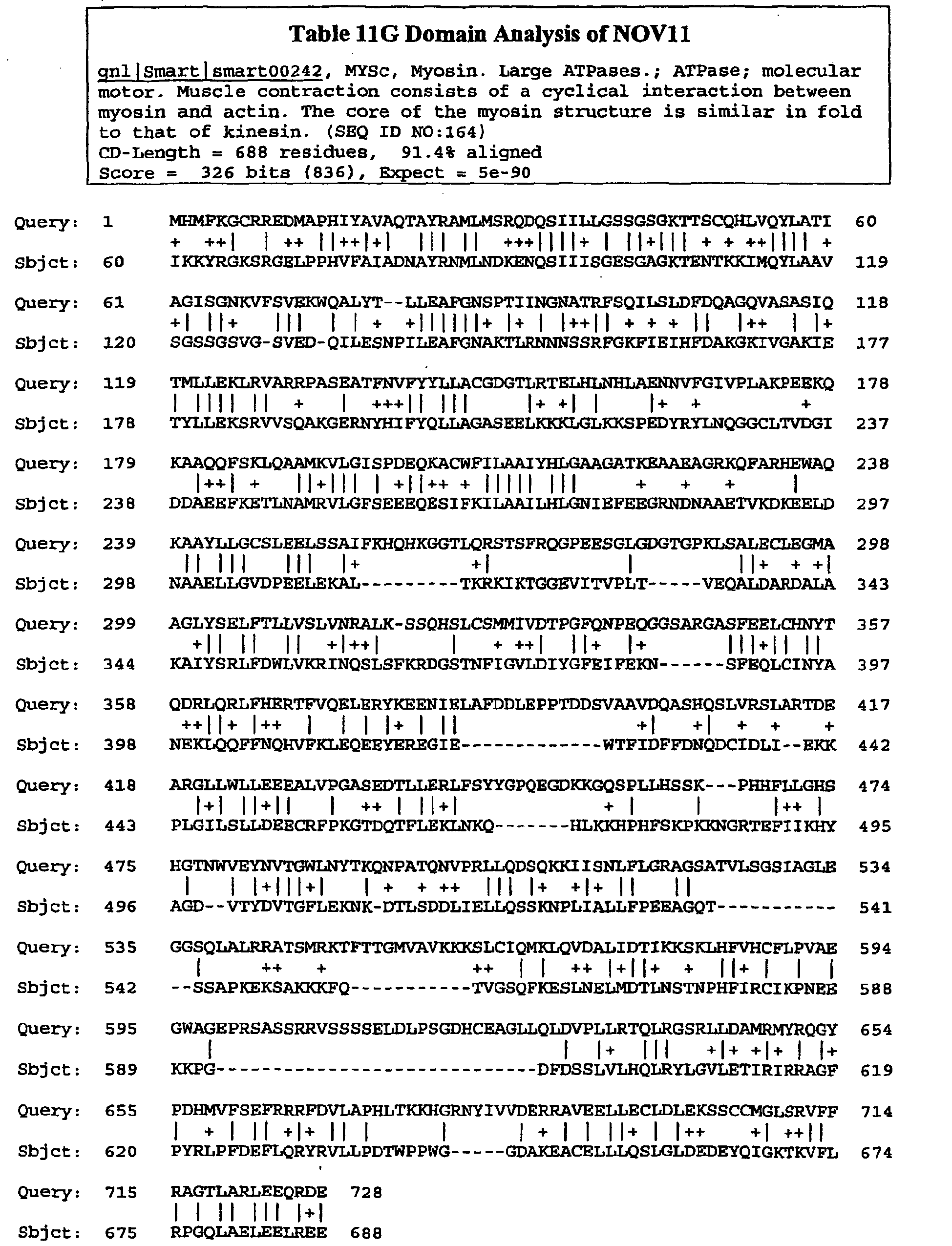

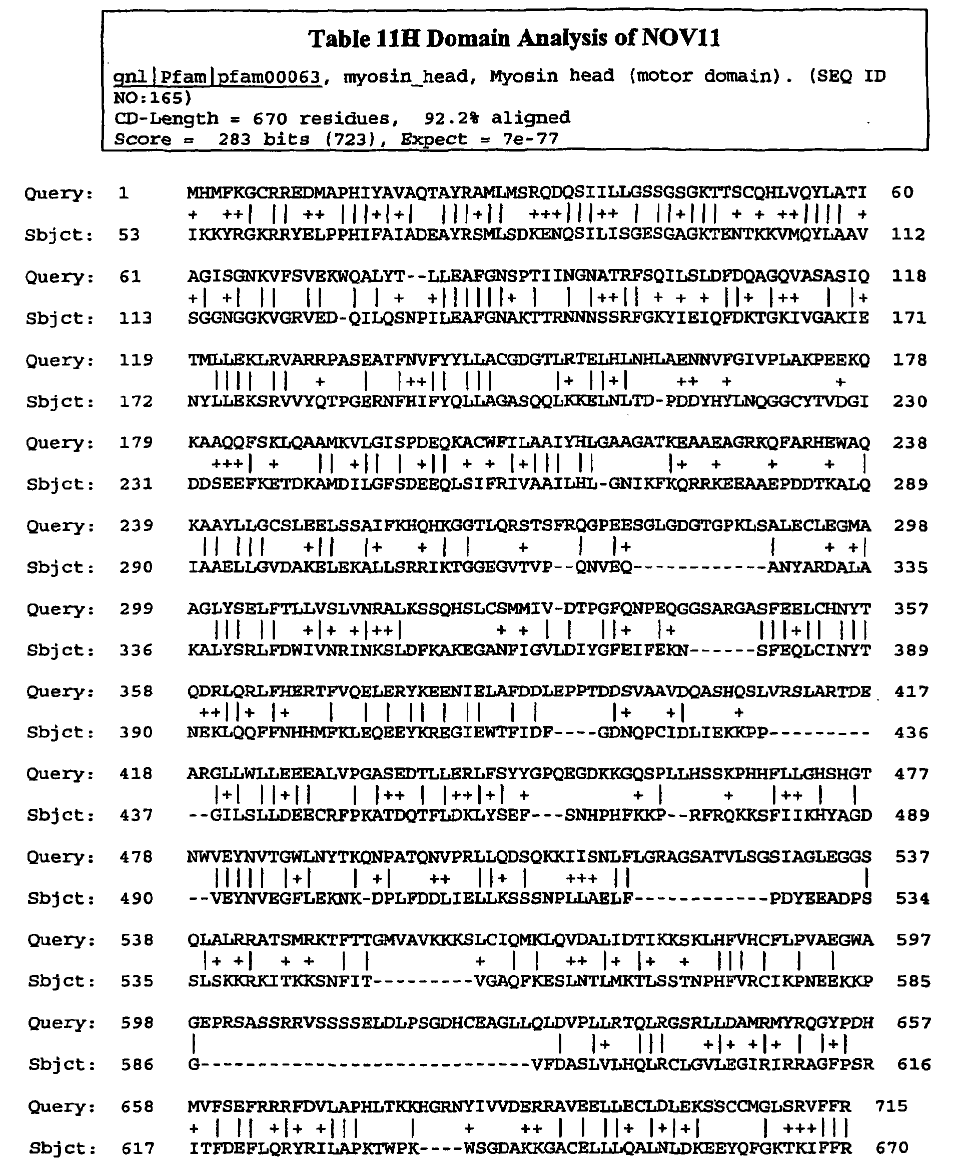

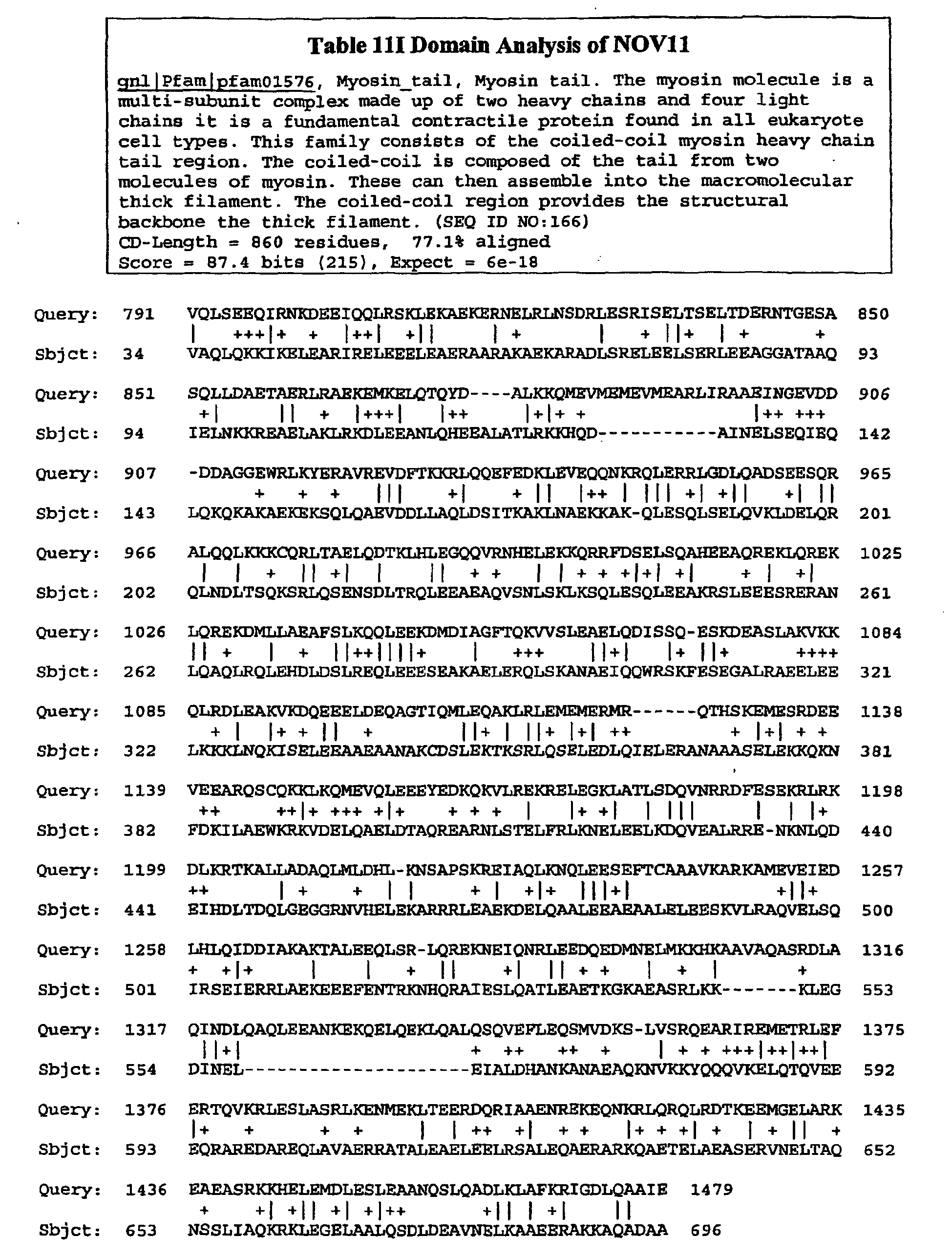

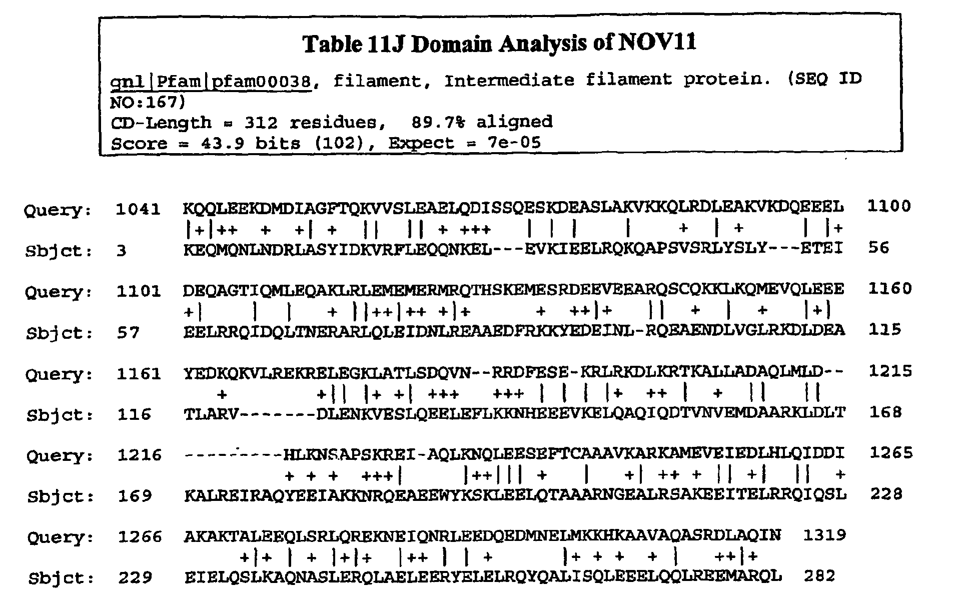

In an effort to sequence human cDNA clones which correspond to relatively long and nearly full-length transcripts, the sequence of KIAA0216 has been determined. It has been obtained from human immature myeloid cell line KG-1. KIAA0216 contains a myosin head (motor domain). Myosins are molecular motors that upon interaction with actin filaments convert energy from ATP hydrolysis into mechanical force. Evidence has emerged for the existence of a large, widely expressed and evolutionarily ancient superfamily of myosin genes.

In addition to the well-catheterized conventional, filament-forming, two-headed myosin-II of muscle and nonmuscle cells, at least ten additional classes of myosins have been identified. In vertebrates, at least seven classes are expressed, and many myosins can be expressed in a single cell type. Distance matrix and maximum parsimony methods have been used to study the evolutionary relationships between members of the myosin superfamily of molecular motors. Amino acid sequences of the conserved core of the motor region have been used in the analysis.

Myosins can be divided into at least three main classes, with two types of unconventional myosin being no more related to each other than they are to conventional myosin. Myosins have traditionally been classified as conventional or unconventional, with many of the unconventional myosin proteins thought to be distributed in a narrow range of organisms. It has been found that members of all three of these main classes are likely to be present in most (or all) eukaryotes. The structure of the trees suggests that these ungrouped proteins and some of the subclasses of the main classes are also likely to be widely distributed, implying that most eukaryotic cells contain many different myosin proteins. The groupings derived from phylogenetic analysis of myosin head sequences agree strongly with those based on tail structure, developmental expression, and (where available) enzymology, suggesting that specific head sequences have been tightly coupled to specific tail sequences throughout evolution.

Analysis of the relationships within each class has interesting implications. For example, smooth muscle myosin and striated muscle myosin seem to have independently evolved from nonmuscle myosin. Furthermore, brush border myosin I, a type of protein initially thought to be specific to specialized metazoan tissues, probably has relatives that are much more broadly distributed. Myosin II, the conventional two-headed myosin that forms bipolar filaments, is directly involved in regulating cytokinesis, cell motility and cell morphology in nonmuscle cells. To understand the mechanisms by which nonmuscle myosin-II regulates these processes, investigators are looking at the regulation of this molecule in vertebrate nonmuscle cells. The identification of multiple isoforms of nonmuscle myosin-II, whose activities and regulation differ from that of smooth muscle myosin-II, suggests that, in addition to regulatory light chain phosphorylation, other regulatory mechanisms control vertebrate nonmuscle myosin-II activity. It has been shown that nonmuscle myosin II, along with other myosins and cytoskeletal proteins, assembles on Golgi membranes. Nonmuscle myosin II associates transiently with membranes of the trans-Golgi network during the budding of a subpopulation of transport vesicles. The exact role of myosin II in vesicular trafficking is not yet understood, but its participation heralds a novel role for actin-based motors in vesicle budding.

In the aortic wall of mammalian species, the maturation phase of smooth muscle cell (SMC) lineage is characterized by two temporally correlated but opposite regulatory processes of gene expression: upregulation of SM type SM2 myosin isoform and downregulation of brain (myosin heavy chain B)- and platelet (myosin heavy chain A(pla))-type nonmuscle myosins. There is propensity of the immature type SMC population to be activated in experimental models and human vascular diseases that are characterized by proliferation and migration of medial SMCs into the subendothelial space. Neointimal proliferation leading to restenosis frequently develops after coronary angioplasty. This process is associated with a change in vascular smooth-muscle cells from a contractile (quiescent) phenotype to a synthetic or proliferating (activated) one. The expression of the B isoform of nonmuscle myosin heavy chain is increased in some coronary atherosclerotic plaques and that this increase in expression identifies a group of lesions at high risk for restenosis after atherectomy. The human homologue of the mouse dilute gene combines elements from both nonmuscle myosin type I and nonmuscle myosin type II. Mutations in the mouse dilute gene result not only in the lightening of coat color, but also in the onset of severe neurological defects shortly after birth, indicating that this gene is important in maintaining the normal neuronal function.

The NOV11 nucleic acid of the invention encoding a KIAA0216-like protein includes the nucleic acid whose sequence is provided in Table 11A, or a fragment thereof. The invention also includes a mutant or variant nucleic acid any of whose bases may be changed from the corresponding base shown in Table 11A while still encoding a protein that maintains its KIAA0216-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1% of the residues may be so changed.

The NOV11 protein of the invention includes the KIAA0216-like protein whose sequence is provided in Table 11B. The invention also includes a mutant or variant protein any of whose residues may be changed from the corresponding residue shown in Table 11B while still encoding a protein that maintains its KIAA0216-like activities and physiological functions, or a functional fragment thereof. In the mutant or variant protein, up to about 2% of the bases may be so changed.

The NOV11 nucleic acids and proteins of the invention are useful in potential diagnostic and therapeutic applications implicated in various diseases and disorders described below and/or other pathologies. For example, the compositions of the present invention will have efficacy for treatment of patients suffering from: restenosis, neurological, glomerular diseases and other diseases, disorders and conditions of the like. Enhanced embryonic nonmuscle myosin heavy chain isoform and matrix metalloproteinase expression in aortic abdominal aneurysm with rapid progression has been found (Cardiovasc Pathol 1999 Sep-Oct;8(5):291-5). In addition, the expression of a nonmuscle myosin heavy chain in glomerular cells differentiates various types of glomerular disease in rats. The nonmuscle type myosin heavy chain, Smemb, is especially useful to detect both mesangial and glomerular epithelial cell activation in these glomerular disease models. Understanding the functional difference and regulatory mechanisms of these cytoskeletal proteins will provide insight into the pathogenesis and progression of glomerular diseases (Kidney Int 1996 May;49(5):1231-41). The expression of the B isoform of nomnuscle myosin heavy chain is increased in some coronary atherosclerotic plaques and this increase in expression identifies a group of lesions at high risk for restenosis after atherectomy (N Engl J Med 1993 Mar 4;328(9):608-13). The human homologue of the mouse dilute gene, which may be important in maintaining the normal neuronal function in the mouse, combines elements from both nonmuscle myosin type I and nonmuscle myosin type II (Genomics 1994 Feb;19(3):407-16).

NOV11 nucleic acids and polypeptides are further useful in the generation of antibodies that bind immunospecincally to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV11 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV12

NOV12 includes three novel TWIK 3-like proteins disclosed below. The disclosed proteins have been named NOV12a, NOV12b and NOV12c.

### NOV12a

A disclosed NOV12a nucleic acid of 1011 nucleotides (also referred to as CG57220-O1) encoding a novel TWIK 3-like protein is shown in Table 12A. An open reading frame was identified beginning with an ATG initiation codon at nucleotides 17-19 and ending with a TAG codon at nucleotides 953-955. Putative untranslated regions, if any, upstream from the initiation codon and downstream from the termination codon are underlined in Table 12A, and the start and stop codons are in bold letters.

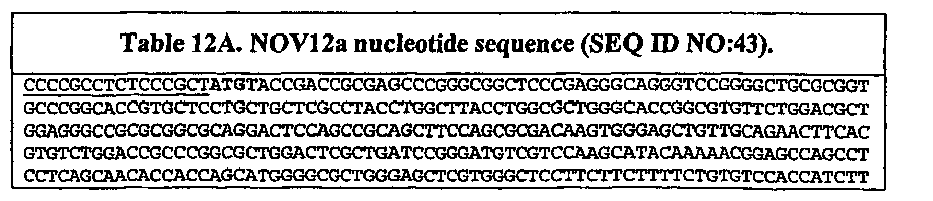

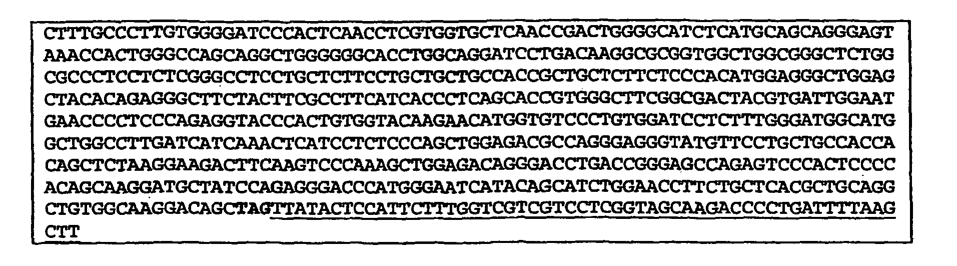

The disclosed NOV12a nucleic acid sequence, localized to chromsome 6p21.1-21.2, has 384 of 641 bases (59%) identical to a gb:GENBANK-ID:AF006823|acc:AF006823.1 mRNA from *Homo sapiens (Homo sapiens* TWIK-related acid-sensitive K+ channel (TASK) mRNA, complete cds) (E = 3.1e⁻¹⁵).

A NOV12a polypeptide (SEQ ID NO:44) encoded by SEQ ID NO:43 has 312 amino acid residues and is presented using the one-letter code in Table 12B. Signal P, Psort and/or Hydropathy results predict that NOV12a contains a signal peptide and is likely to be localized to the plasma membrane with a certainty of 0.6400. The most likely cleavage site for a NOV12a polypeptide is between amino acids 48 and 49: RAA-QD.

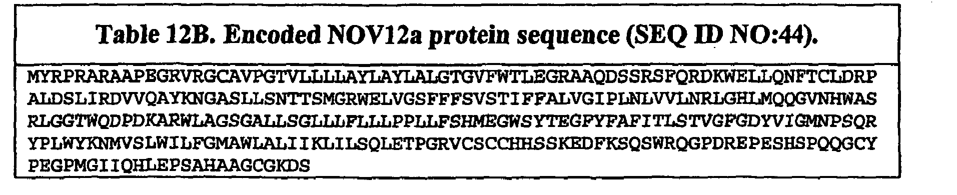

The NOV12a amino acid sequence has 113 of 114 amino acid residues (99%) identical to, and 113 of 114 amino acid residues (99%) similar to, the 229 amino acid residue ptnr:TREMBLNEW-ACC:CAC07335 protein from *Homo sapiens* (Human) (DJ137F1.1 (Novel Member Of The Potassium Channel Subfamily K)) (E = 2.4e⁻¹⁰⁸).

The disclosed NOV12a is expressed in at least the following tissues: amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, Literature sources, and/or RACE sources.

### NOV12b

A disclosed NOV12b nucleic acid of 1083 nucleotides (also referred to as CG57220-02) encoding a novel TWIK 3-like protein is shown in Table 12C. An open reading frame was identified beginning with an ATG initiation codon at nucleotides 17-19 and ending with a TAG codon at nucleotides 1025-1027. Putative untranslated regions, if any, upstream from the initiation codon and downstream from the termination codon are underlined in Table 12C, and the start and stop codons are in bold letters.

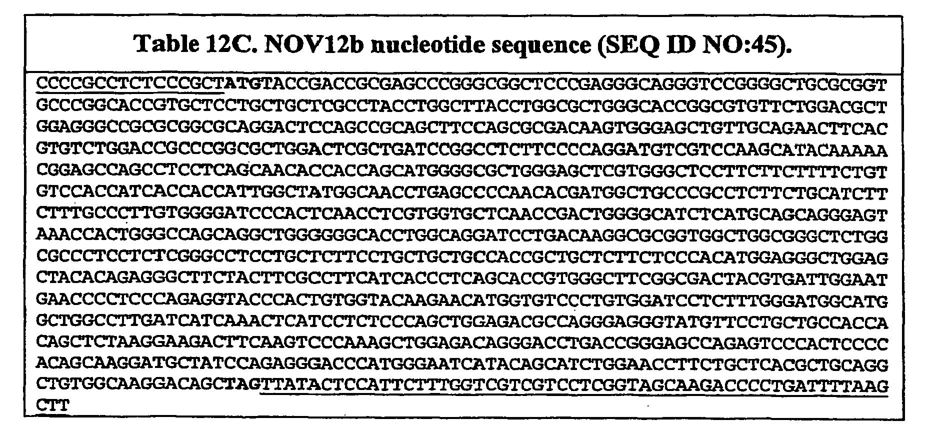

The disclosed NOV12b nucleic acid sequence, localized to chromsome 6p21.1-21.2, has 496 of 795 bases (62%) identical to a gb:GENBANK-ID:AFOO6823|acc:AF006823.1 mRNA from *Homo sapiens (Homo sapiens* TWIK-related acid-sensitive K+ channel (TASK) mRNA, complete cds) (E = 1.2e⁻²⁷).

A NOV12b polypeptide (SEQ ID NO:46) encoded by SEQ ID NO:45 has 336 amino acid residues and is presented using the one-letter code in Table 12D. Signal P, Psort and/or Hydropathy results predict that NOV12b contains a signal peptide and is likely to be localized to the plasma membrane with a certainty of 0.6400. The most likely cleavage site for a NOV12a polypeptide is between amino acids 48 and 49: RAA-QD.

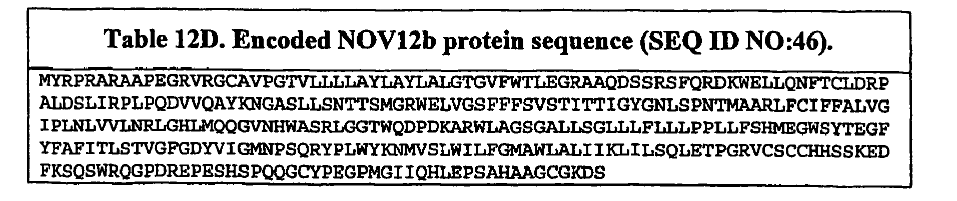

The NOV12b amino acid sequence has 228 of 233 amino acid residues (97%) identical to, and 228 of 233 amino acid residues (97%) similar to, the 229 amino acid residue ptnr:TREMBLNEW-ACC:CAC07335 protein from *Homo sapiens* (Human) (DJ137F1.1 (Novel Member Of The Potassium Channel Subfamily K)) (E = 1.1e⁻¹¹⁹).

The disclosed NOV12b is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, Literature sources, and/or RACE sources.

### NOV12c

A disclosed NOV12c nucleic acid of 1256 nucleotides (also referred to as CG57220-03) encoding a novel TWIK 3-like protein is shown in Table 12E. An open reading frame was identified beginning with an ATG initiation codon at nucleotides 17-19 and ending with a TAG codon at nucleotides 953-955. Putative untranslated regions, if any, upstream from the initiation codon and downstream from the termination codon are underlined in Table 12E, and the start and stop codons are in bold letters.

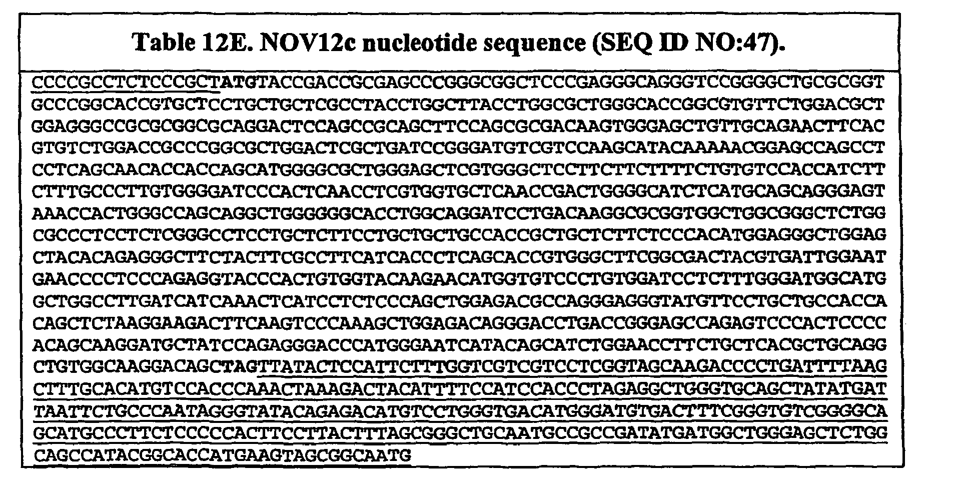

The disclosed NOV12c nucleic acid sequence, localized to chromsome 6p21.1-21.2, has 384 of 641 bases (59%) identical to a gb:GBNBANK-ID:AF006823|acc:AF006823.1 mRNA from *Homo sapiens (Homo sapiens* TWIK-related acid-sensitive K+ channel (TASK) mRNA, complete cds) (E = 1.3e⁻¹⁴).

A NOV12c polypeptide (SEQ ID NO:48) encoded by SEQ ID NO:47 has 312 amino acid residues and is presented using the one-letter code in Table 12F. Signal P, Psort and/or Hydropathy results predict that NOV12c contains a signal peptide and is likely to be localized to the plasma membrane with a certainty of 0.6400. The most likely cleavage site for a NOV12a polypeptide is between amino acids 48 and 49: RAA-QD.

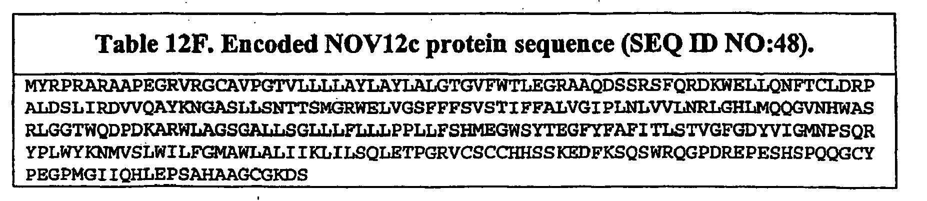

The NOV12c amino acid sequence has 67 of 132 amino acid residues (50%) identical to, and 97 of 132 amino acid residues (73%) similar to, the 294 amino acid residue ptnr:SPTREMBL-ACC:Q9H591 protein from *Homo sapiens* (Human) (DJ137F1.2 (Novel Member Of The Potassium Channel Subfamily K)) (E = 2.1 e⁻⁴⁷).

The disclosed NOV12c is expressed in at least the following tissues: amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, Literature sources, and/or RACE sources.

Possible SNPs found for NOV12a are listed in Table 12G.

| Table 12G: SNPs | | | | |
|---|---|---|---|---|
| **Variant** | **Nucleotide Position** | **Base Change** | **Amino Acid Position** | **Base Change** |
| 13377098 | 77 | G > A | 21 | Gly > Ser |
| 13377097 | 593 | T > C | 193 | Tyr > His |
| 13377096 | 718 | A > G | Silent | N/A |
| 13377095 | 856 | C > T | Silent | N/A |

NOV12a, NOV12b and NOV12c are very closely homologous as is shown in the amino acid alignment in Table 12H.

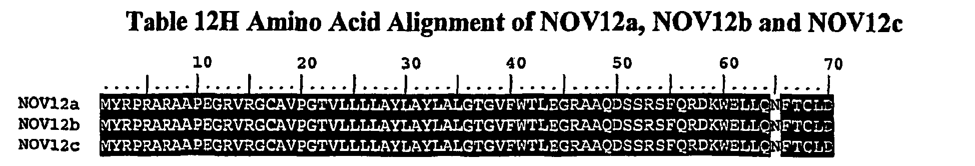

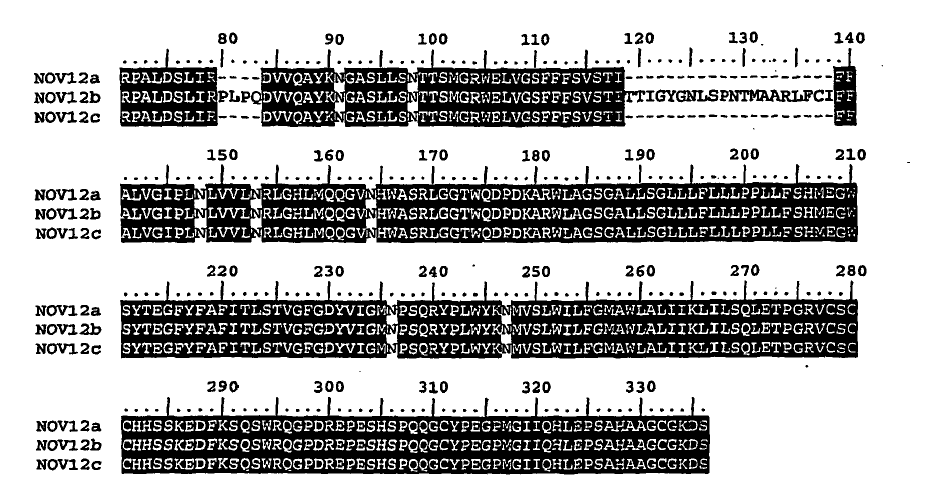

Homologies to any of the above NOV12 proteins will be shared by the other NOV12 proteins insofar as they are homologous to each other as shown above. Any reference to NOV 12 is assumed to refer to the NOV 12 proteins in general, unless otherwise noted.

NOV12a has homology to the amino acid sequences shown in the BLASTP data listed in Table 12I.

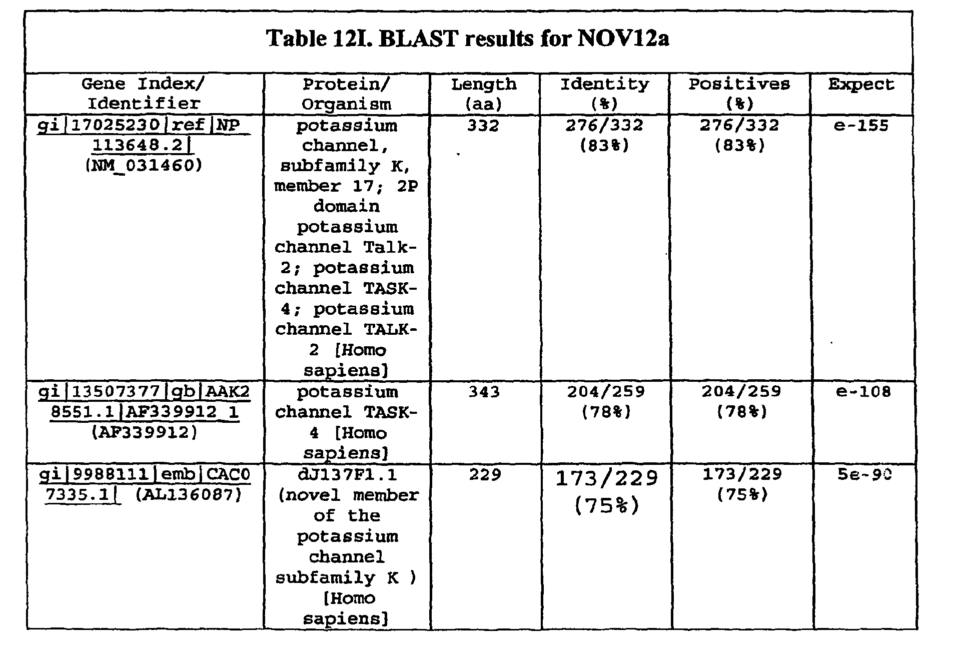

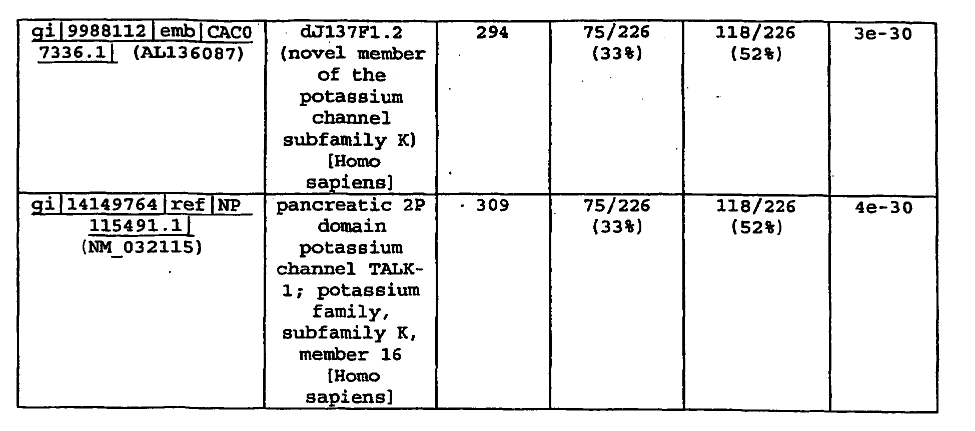

The homology of these sequences is shown graphically in the ClustalW analysis shown in Table 12J.

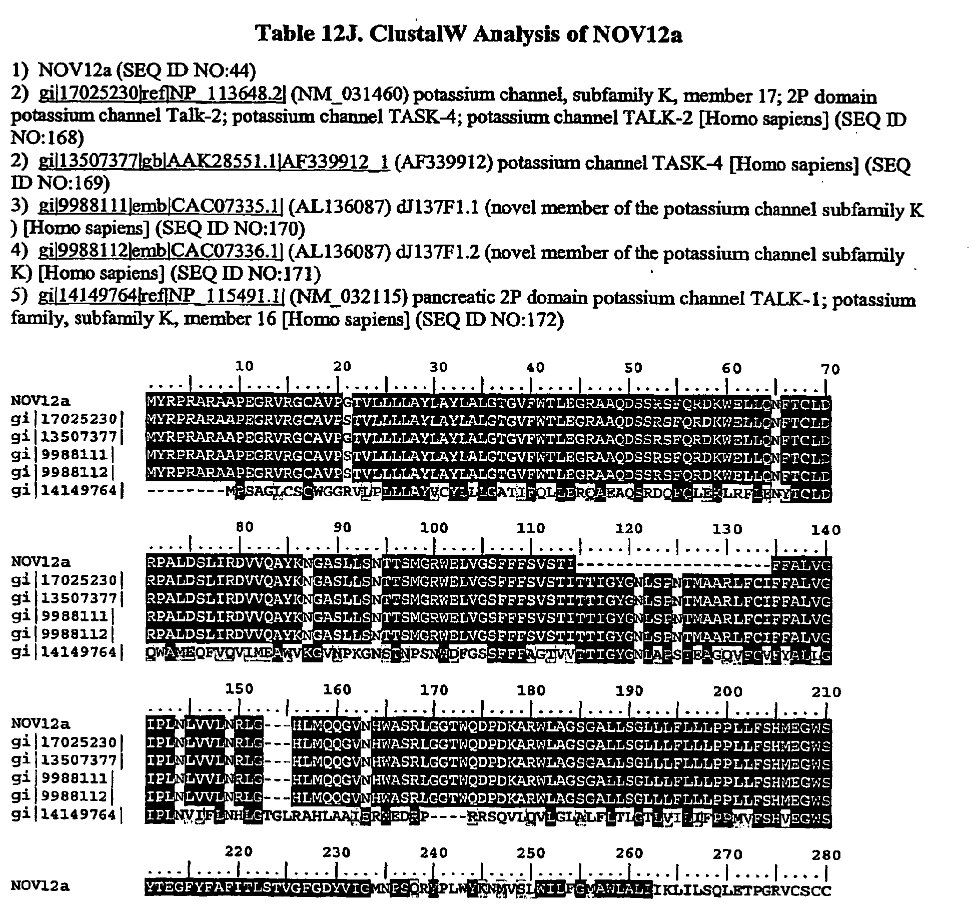

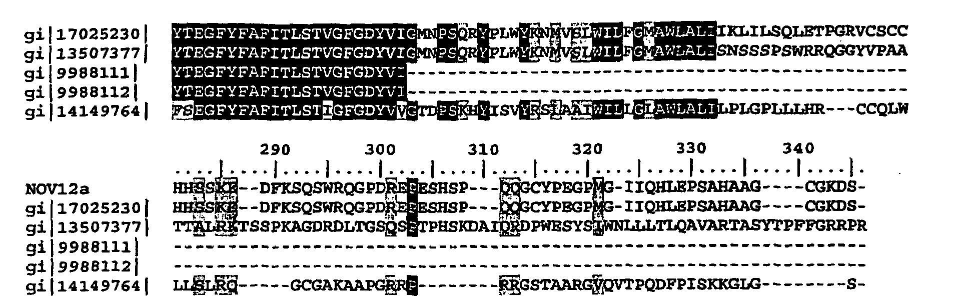

Potassium channels are amongst the most heterogeneous class of ion channels known and are responsible for mediating a diverse range of biological functions. The most recently described family of K(+) channels, the 'two pore-domain family', contain four membrane spanning domains and two pore-forming domains, suggesting that two channel subunits associate to form a functional K(+) pore. Several sub-families of the two pore domain potassium channel family have been described, including the weakly inward rectifying K(+) channel (TWIK), the acid-sensitive K(+) channel (TASK), the TWIK-related K(+) channel (TREK) and the TWIK-related arachidonic acid stimulated K(+) channel (TRAAK). TWIK-1 and the TWIK-1-like channel KCNK7 were predominantly expressed in the CNS, in contrast to TWIK-2 which was preferentially expressed in peripheral tissues such as pancreas, stomach, spleen and uterus. TASK-1 was expressed in the CNS and some peripheral tissues, whereas TASK-2 was exclusively expressed in the periphery except for mRNA expression observed in dorsal root ganglion and spinal cord. In addition, mRNA expression of the recently identified TASK-3, was almost completely exclusive to cerebellum with little or no mRNA detected in any other tissues. TREK-1 and TRAAK mRNA expression was predominantly CNS specific in contrast to the closely related TREK-2, which was expressed in both CNS and peripheral tissues. (Medhurst et al., Brain Res Mol Brain Res 86:101-114).

The two-pore-domain K(+) channels, or K(2P) channels produce currents with unusual characteristics. They are quasi-instantaneous and noninactivating, and they are active at all membrane potentials and insensitive to the classic K(+) channel blockers. These properties designate them as background K(+) channels. They are expected to play a major role in setting the resting membrane potential in many cell types. Another salient feature of K(2P) channels is the diversity of their regulatory mechanisms. The weak inward rectifiers TWIK-1 and TWIK-2 are stimulated by activators of protein kinase C and decreased by internal acidification, the baseline TWIK-related acid-sensitive K(+) (TASK)-1 and TASK-2 channels are sensitive to external pH changes in a narrow range near physiological pH, and the TWIK-related (TREK)-1 and TWIK-related arachidonic acid-stimulated K(+) (TRAAK) channels are the first cloned polyunsaturated fatty acids-activated and mechanogated K(+) channels. The recent demonstration that TASK-1 and TREK-1 channels are activated by inhalational general anesthetics, and that TRAAK is activated by the neuroprotective agent riluzole, indicates that this novel class of K(+) channels is an interesting target for new therapeutic developments (Lesage and Lazdunski, Am J Physiol Renal Physiol 2000, 279(5):F793-801).

TWIK-1, the founding member of the 2P domain mammalian family, is widely expressed in human tissues and is particularly abundant in brain and heart. hTWIK-1 currents expressed in Xenopus oocytes are K+-selective, are time-independent, and present a nearly linear I-V relationship that rectifies for depolarizations positive to 0 mV . TWIK-1 is blocked by Ba2+, quinine, and quinidine (Lesage et al., 1996 EMBO J. 15: 1004-1011). Recently, hTWIK-2 (also called hTOSS), a TWIK-1-related gene, was cloned by two independent groups . Although both hTWIK-2 and hTOSS sequences are identical, conflicting results were published concerning functional expression in heterologous systems (Chavez, et al.,1999 J. Biol. Chem. 274: 7887-7892; Pountney, et al., 1999 FEBS Lett. 450: 191-196). hTWIK-2 expressed in Xenopus oocytes was shown to be a noninactivating, time-independent, weak inward rectifier with biophysical properties identical to TWIK-1. Pharmacologically, hTWIK-2 was reported to be different from TWIK-1 with a lack of sensitivity to quinine, quinidine, and Ba2+. On the contrary, no significant current was observed in hTOSS cRNA-injected Xenopus laevis oocytes or in hTOSS cDNA-transfected HEK293T cells. Co-injection of equimolar concentrations of hTWIK-1 and hTOSS cRNA also failed to generate currents in Xenopus oocytes. These negative findings have led these authors and others to propose that hTOSS may be targeted to locations other than the plasma membrane or that it may possess a regulatory function, modulating the properties of other principal channel-forming subunits with tissue-specific implications (Chavez, et al.,1999, J. Biol. Chem. 274: 7887-7892; Pountney, et al., 1999 FEBS Lett. 450: 191-196; Rajan, et al., 2000 J. Biol. Chem. 275: 16650-16657).

The NOV12 nucleic acid of the invention encoding a TWIK 3-like protein includes the nucleic acid whose sequence is provided in Tables 12A, 12C and 12E, or a fragment thereof. The invention also includes a mutant or variant nucleic acid any of whose bases may be changed from the corresponding base shown in Tables 12A, 12C and 12E while still encoding a protein that maintains its TWIK 3-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 41% of the NOV12a residues, about 38% of the NOV12b residues and about 41% of the NOV12c residues may be so changed.

The NOV12 protein of the invention includes the TWIK 3-like protein whose sequence is provided in Tables 12B, 12D and 12F. The invention also includes a mutant or variant protein any of whose residues may be changed from the corresponding residue shown in Tables 12B, 12D and 12F while still encoding a protein that maintains its TWIK 3-like activities and physiological functions, or a functional fragment thereof. In the mutant or variant protein, up to about 1% of the NOV12a, about 3% of the NOV12b and about 50% of the NOV12c bases may be so changed.

The NOV12 nucleic acids and proteins of the invention are useful in potential diagnostic and therapeutic applications implicated in various diseases and disorders described below and/or other pathologies. For example, the compositions of the present invention will have efficacy for treatment of patients suffering from: central nervous system disorder; dementia; epilepsy; Alzheimer's disease; Parkinson's disease; multiple sclerosis; depression; amyotrophic lateral sclerosis; progressive supranuclear palsy; mania; Creutzfeldt-Jacob disease; psychiatric disorder; schizophrenic disorder; Korsakoff's psychosis; anxiety disorder; phobic disorder; amnesia; learning disorder; memory disorder; age-related memory loss; obesity; neurological disorder; cardiac disorder and other diseases, disorders and conditions of the like.

NOV12 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV12 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV13

A disclosed NOV 13 nucleic acid of 1260 nucleotides (also referred to as CG57220-04) encoding a novel TASK 4-like protein is shown in Table 13A. An open reading frame was identified beginning with an ATG initiation codon at nucleotides 107-109 and ending with a TAG codon at nucleotides 1163-1165. Putative untranslated regions upstream from the intitation codon and downstream from the termination codon are underlined in Table 13A, and the start and stop codons are in bold letters.

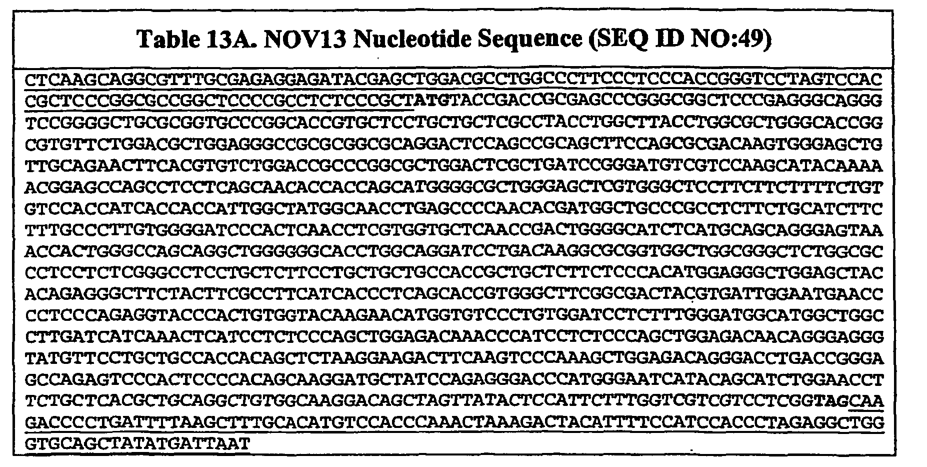

The NOV13 nucleic acid was identified on chromosome 6 and has 508 of 827 bases (61%) identical to a gb:GENBANK-ID:AF006823|acc:AF006823.1 mRNA from *Homo sapiens (Homo sapiens* TWIK-related acid-sensitive K+ channel (TASK) mRNA, complete cds) (E = 1.2e⁻²⁹).

A disclosed NOV13 polypeptide (SEQ ID NO:50) encoded by SEQ ID NO:49 is 352 amino acid residues and is presented using the one-letter code in Table 13B. Signal P, Psort and/or Hydropathy results predict that NOV13 contains a signal peptide and is likely to be localized to the extracellularly with a certainty of 0.6400. The most likely cleavage site for a NOV 13 polypeptide is between amino acids 48 and 49: RAA-QD.

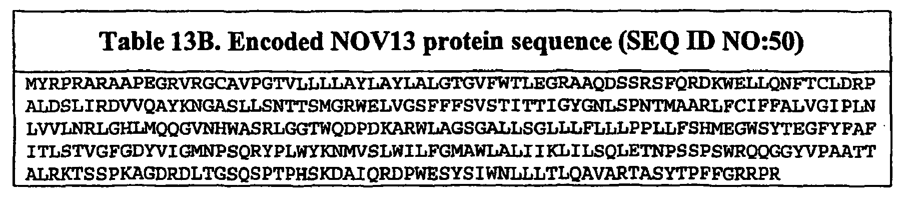

The NOV13 amino acid sequence has 340 of 352 amino acid residues (96%) identical to, and 342 of 352 amino acid residues (97%) similar to, the 343 amino acid residue ptnr: TREMBLNEW-ACC:AAK28551 protein from *Homo sapiens* (Human) (Potassium Channel TASK-4) (E = 5.0e⁻¹⁸³).

NOV13 is expressed in at least the following tissues: liver, lung, pancreas, placenta, aorta and heart. This information was derived by determining the tissue sources of the sequences that were included in the invention including but not limited to SeqCalling sources, Public EST sources, genomic clone sources, literature sources, and/or RACE sources. The sequence is predicted to be expressed in the following tissues because of the expression pattern of (GENBANK-ID: gb:GENBANK-ID:AF006823|acc:AF006823.1) a closely related *Homo sapiens* TWIK-related acid-sensitive K+ channel (TASK) mRNA, complete cds homolog in species *Homo sapiens:* pancreas, placenta, brain, lung, prostate, heart, kidney, uterus, small intestine and colon.

NOV13 has homology to the amino acid sequences shown in the BLASTP data listed in Table 13C.

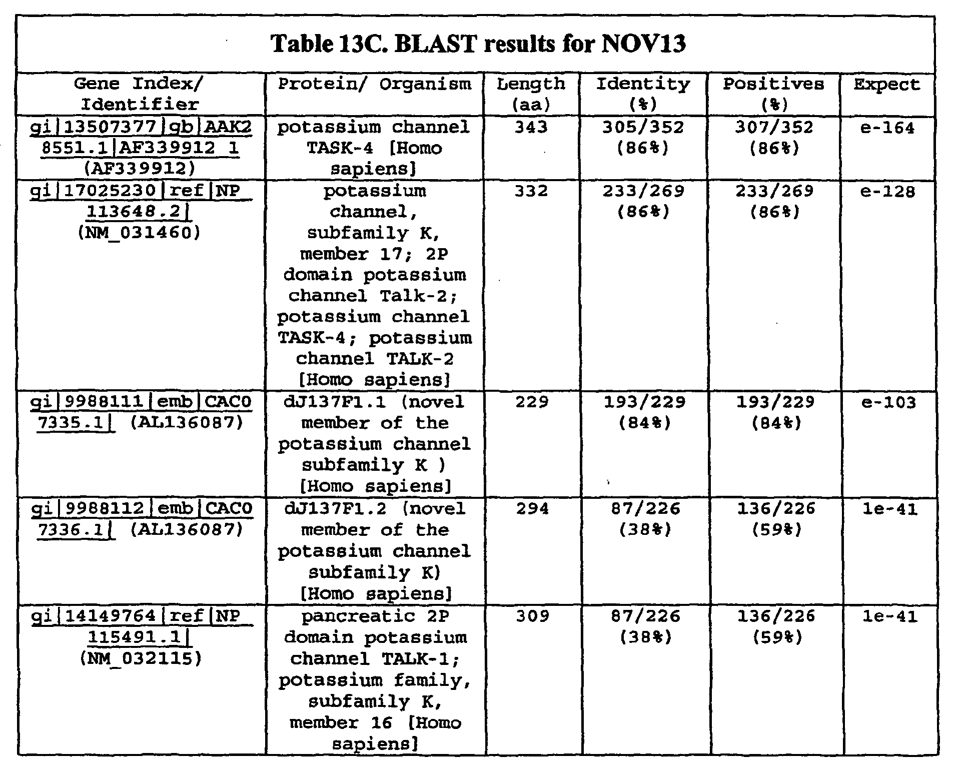

The homology of these sequences is shown graphically in the ClustalW analysis shown in Table 13D.

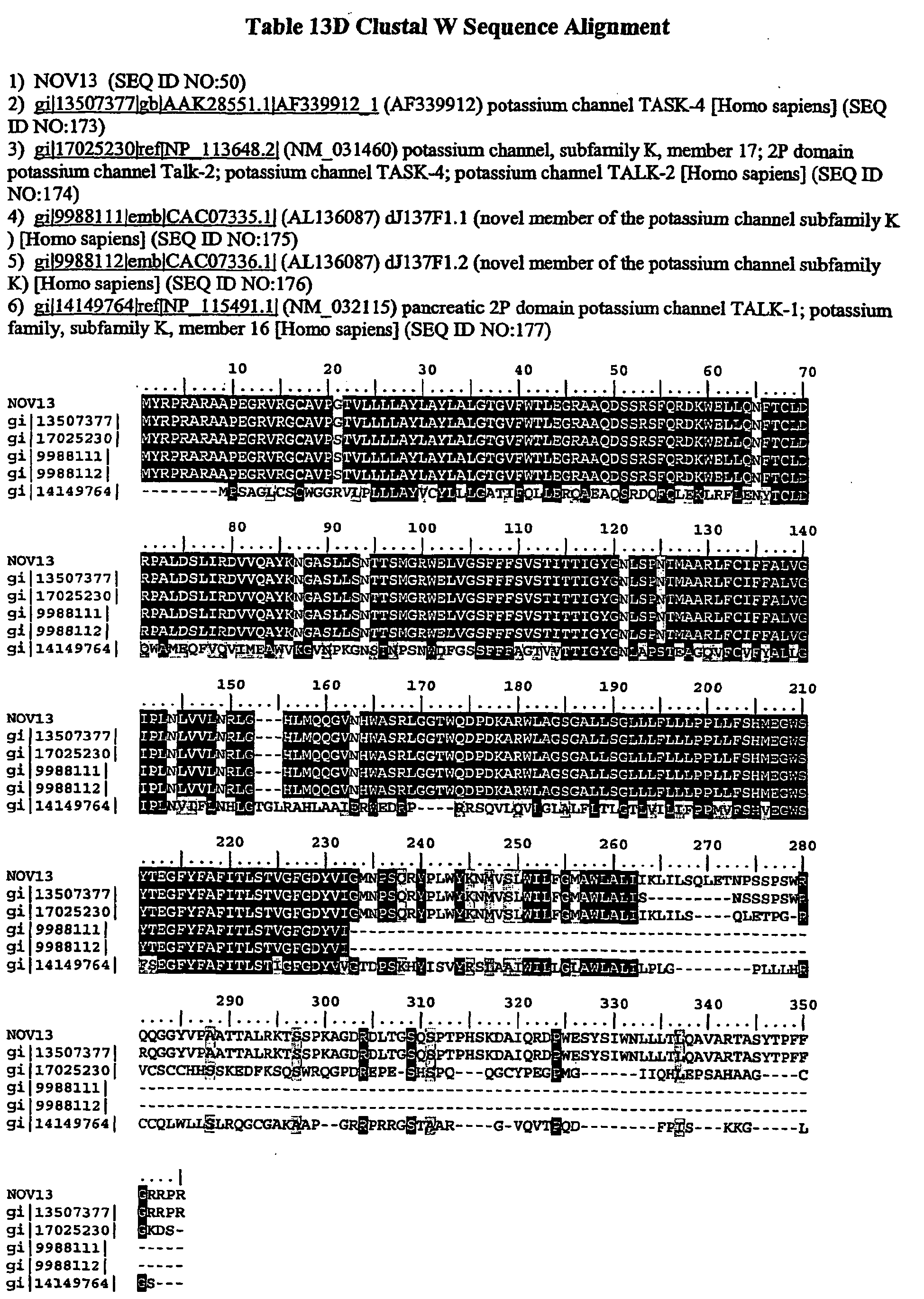

The NOV13 nucleic acid of the invention encoding a TASK 4-like protein includes the nucleic acid whose sequence is provided in Table 13A, or a fragment thereof. The invention also includes a mutant or variant nucleic acid any of whose bases may be changed from the corresponding base shown in Table 13A while still encoding a protein that maintains its TASK 4-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 39% of the residues may be so changed.

The NOV13 protein of the invention includes the TASK 4-like protein whose sequence is provided in Table 13B. The invention also includes a mutant or variant protein any of whose residues may be changed from the corresponding residue shown in Table 13B while still encoding a protein that maintains its TASK 4-like activities and physiological functions, or a functional fragment thereof In the mutant or variant protein, up to about 4% of the bases may be so changed.

The NOV13 nucleic acids and proteins of the invention are useful in potential diagnostic and therapeutic applications implicated in various diseases and disorders described below and/or other pathologies. For example, the compositions of the present invention will have efficacy for treatment of patients suffering from: cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, aneurysm, fibromuscular dysplasia, stroke, systemic lupus erythematosus, autoimmune disease, asthma, emphysema, allergy, ARDS, Von Hippel-Lindau (VHL) syndrome, cirrhosis, diabetes, pancreatitis, fertility, cancer, tissue degeneration, bacterial/viral/parasitic infections and other diseases, disorders and conditions of the like.

NOV13 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV13 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV14

A disclosed NOV14 nucleic acid of 627 nucleotides (also referred to as CG57458-01) encoding a novel Copper transporter-like protein is shown in Table 14A. An open reading frame was identified beginning with an ATG initiation codon at nucleotides 31-33 and ending with a TGA codon at nucleotides 598-600. Putative untranslated regions upstream from the intitation codon and downstream from the termination codon are underlined in Table 14A, and the start and stop codons are in bold letters.

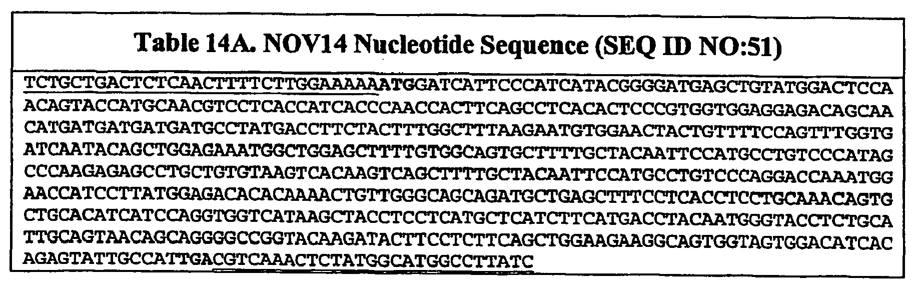

The NOV14 nucleic acid was identified on chromosome 3.

A disclosed NOV14 polypeptide (SEQ ID NO:52) encoded by SEQ ID NO:51 is 189 amino acid residues and is presented using the one-letter code in Table 14B. Signal P, Psort and/or Hydropathy results predict that NOV14 contains a signal peptide and is likely to be localized to the plasma membrane with a certainty of 0.6000. The most likely cleavage site for a NOV14 polypeptide is between amino acids 68 and 69: TAG-EM.

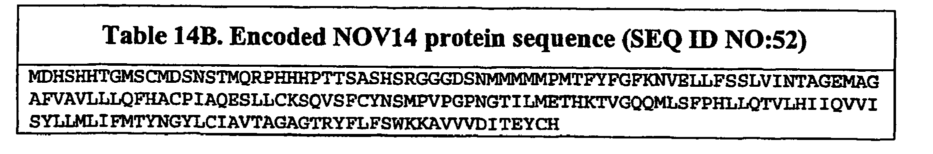

The NOV14 amino acid sequence has 166 of 190 amino acid residues (87%) identical to, and 170 of 190 amino acid residues (89%) similar to, the 190 amino acid residue ptnr:SWISSNEW-ACC:015431 protein from *Homo sapiens* (Human) (High-Affinity Copper Uptake Protein 1 (HCTR1) (E = 1.9e⁻⁸³).

NOV14 is expressed in at least the following tissues: brain. This information was derived by determining the tissue sources of the sequences that were included in the invention including but not limited to SeqCalling sources, Public EST sources, genomic clone sources, literature sources, and/or RACE sources.

NOV14 has homology to the amino acid sequences shown in the BLASTP data listed in Table 14C.

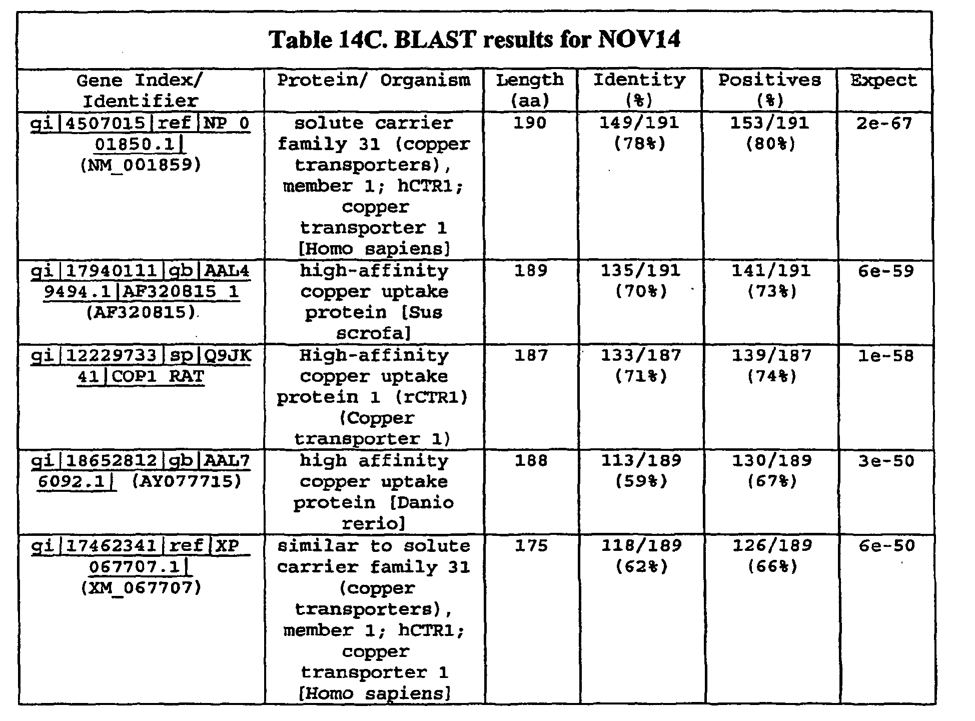

The homology of these sequences is shown graphically in the ClustalW analysis shown in Table 14D.

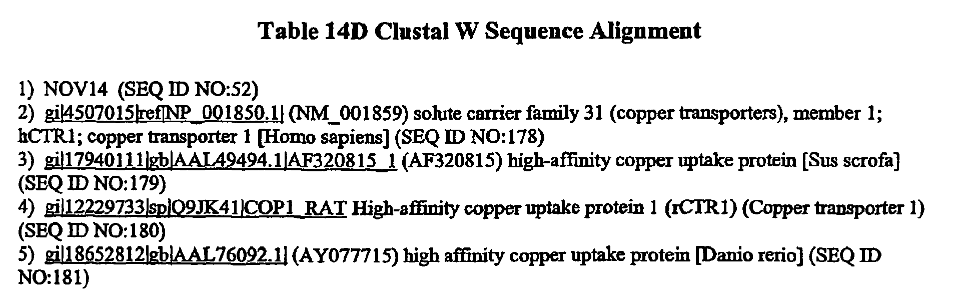

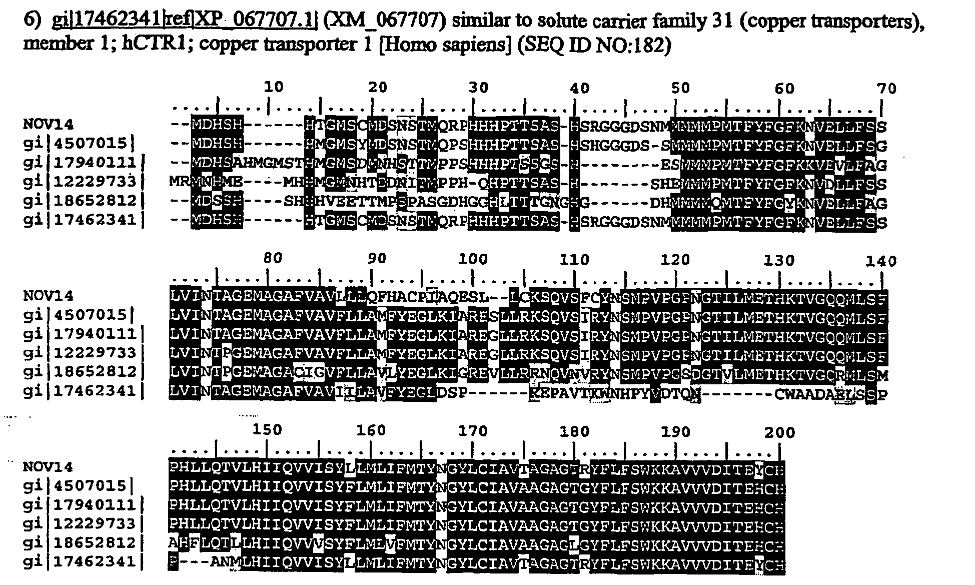

Copper is an element essential for life, but excessive copper can be toxic or even lethal to the cell. Therefore, cells have developed sophisticated ways to maintain a critical copper balance, with the intake, export, and intracellular compartmentalization or buffering of copper strictly regulated. The two related genes ATP7A and ATP7B, responsible for the human diseases Menkes syndrome and Wilson disease (WND), respectively, are involved in copper export. In *S. cerevisiae*, three copper uptake genes CTR1, CTR2, and CTR3 have been identified.

In mammals, however, the molecular basis for copper uptake is unknown. Zhou and Gitschier (Proc Natl Acad Sci U S A. 94(14):7481-6, 1997) isolated a human cDNA encoding COPT1, which they called CTR1, by functional complementation of the yeast high-affinity copper uptake mutant ctr1. The deduced 190-amino acid human CTR1 protein is similar to yeast CTR1 and Arabidopsis COPT1, a copper transporter also isolated by functional complementation of yeast ctr1. All 3 predicted proteins have 3 transmembrane domains and an N terminus that is rich in methionine and serine residues; the N terminus of human CTR1 is also abundant in histidines. The authors proposed that human CTR1 is a high-affinity copper uptake gene because it can complement the yeast ctr1 mutation, it can rescue multiple defects in ctr1 yeast, its expression in ctr1 yeast increases the concentration of cellular copper, and its overexpression in yeast leads to a vulnerability to the toxicity of copper overload. Northern blot analysis detected 2 major CTR1 transcripts of approximately 2 kb and 5.5 kb and a less abundant transcript of about 8.5 kb in all human organs and tissues examined. Zhou and Gitschier (1997) found that the 3-prime untranslated region of the human CTR1 gene contains a CA repeat marker (D9S262) that had been previously mapped to 9q31-q32. By analysis of YAC clones, they showed that CTR1 and CTR2 (COPT2) which is also located in 9q31-q32, are not adjacent genes. The molecular mechanisms responsible for the cellular uptake of copper in mammalian cells are unknown. The isolation of a human gene involved in this process by complementation of the yeast high-affinity copper uptake mutant, ctr1 is described. Besides complementing ctr1 growth defect on nonfermentable media, the human gene also rescues iron transport and SOD1 defects in ctr1 yeast. Overexpression of the gene in yeast leads to vulnerability to the toxicity of copper overload. In addition, its expression in ctr1 yeast significantly increases the level of cellular copper, as demonstrated by atomic absorption. It is proposed that this gene is a candidate for high-affinity copper uptake in humans and by analogy have named it hCTR1. The hCTR1 and yeast CTR1 predicted transmembrane proteins are 29% identical, but the human protein is substantially smaller in both the extracellular metal-binding and intracellular domains. An additional human gene similar to hCTR1, here named hCTR2, was identified in a database search. Both hCTR1 and hCTR2 are expressed in all human tissues examined, and both genes are located in 9q31/32. These studies, together with the previously recognized functional and sequence similarity between the Menkes/Wilson copper export proteins and CCC2 in yeast, demonstrate that similar copper homeostatic mechanisms are used in these evolutionarily divergent organisms.

The NOV14 nucleic acid of the invention encoding a Copper transporter-like protein includes the nucleic acid whose sequence is provided in Table 14A, or a fragment thereof. The invention also includes a mutant or variant nucleic acid any of whose bases may be changed from the corresponding base shown in Table 14A while still encoding a protein that maintains its Copper transporter-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 13% of the residues may be so changed.

The NOV14 protein of the invention includes the Copper transporter-like protein whose sequence is provided in Table 14B. The invention also includes a mutant or variant protein any ofwhose residues may be changed from the corresponding residue shown in Table 14B while still encoding a protein that maintains its Copper transporter-like activities and physiological functions, or a functional fragment thereof. In the mutant or variant protein, up to about 13% of the bases may be so changed.

The NOV14 nucleic acids and proteins of the invention are useful in potential diagnostic and therapeutic applications implicated in various diseases and disorders described below and/or other pathologies. For example, the compositions of the present invention will have efficacy for treatment of patients suffering from: Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection and other diseases, disorders and conditions of the like.

NOV 14 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV14 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV15

A disclosed NOV15 nucleic acid of 1478 nucleotides (also referred to as CG57454-01) encoding a novel Cytokeratin-like protein is shown in Table 15A. An open reading frame was identified beginning with an ATG initiation codon at nucleotides 21-23 and ending with a TGA codon at nucleotides 1464-1466. Putative untranslated regions upstream from the intitation codon and downstream from the termination codon are underlined in Table 15A, and the start and stop codons are in bold letters.

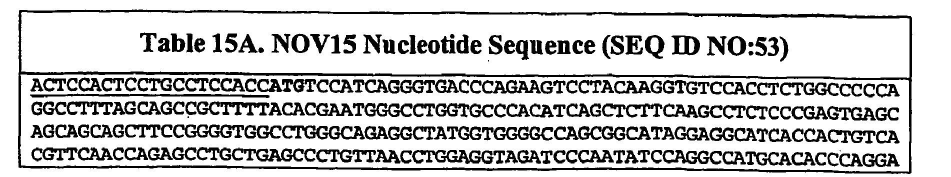

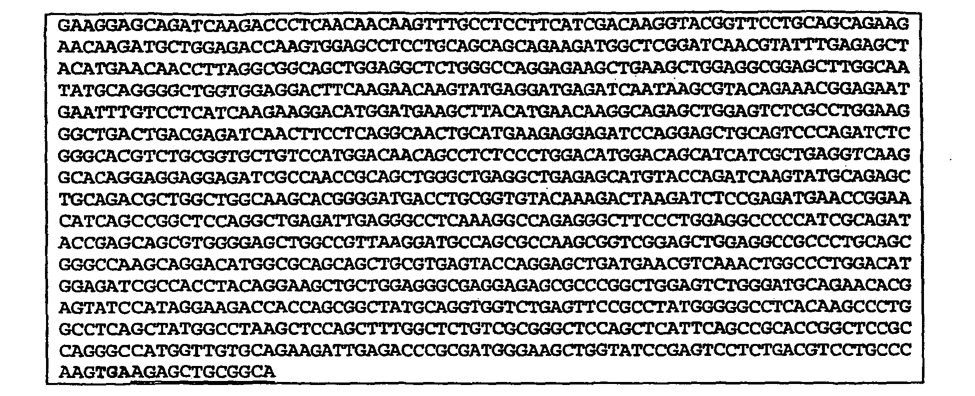

The NOV15 nucleic acid was identified on chromosome 3 and has 1187 of 1344 bases (88%) identical to a gb:GENBANK-ID:AF213884S2|acc:AF224669.1 mRNA from *Homo sapiens (Homo sapiens* mannosidase, beta A, lysosomal (MANBA) gene, and ubiquitin-conjugating enzyme E2D 3 (UBE2D3) genes, complete cds) (E = 7.3e⁻²²⁸).

A disclosed NOV15 polypeptide (SEQ ID NO:54) encoded by SEQ ID NO:53 is 481 amino acid residues and is presented using the one-letter code in Table 15B. Signal P, Psort and/or Hydropathy results predict that NOV15 contains a signal peptide and is likely to be localized to the plasma membrane with a certainty of 0.3600.

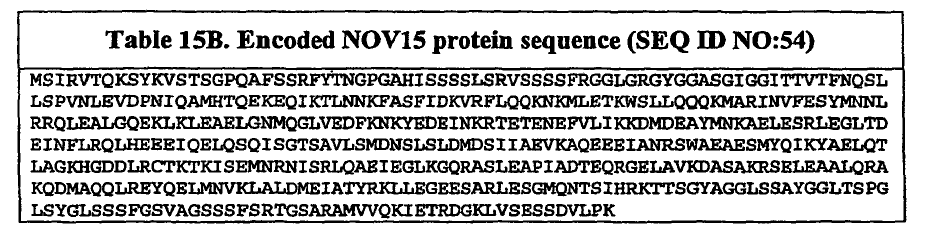

The NOV15 amino acid sequence has 424 of 482 amino acid residues (87%) identical to, and 448 of 482 amino acid residues (92%) similar to, the 482 amino acid residue ptnr:SWISSPROT-ACC:P05787 protein from Homo sapiens (Human) (Keratin, Type II Cytoskeletal 8 (Cytokeratin 8) (K8) (CK 8)) (E = 4.9e⁻²¹³).

NOV15 is expressed in at least the following tissues: Heart, Kidney, Liver, Lung, Muscle, Nose, Ovary, Pancreas, Parathyroid, Placenta, Pooled, Prostate, Stomach, Synovial membrane, Testis, Thyroid, Tonsil and Uterus. This information was derived by determining the tissue sources of the sequences that were included in the invention including but not limited to SeqCalling sources, Public EST sources, genomic clone sources, literature sources, and/or RACE sources.

NOV15 has homology to the amino acid sequences shown in the BLASTP data listed in Table 15C.

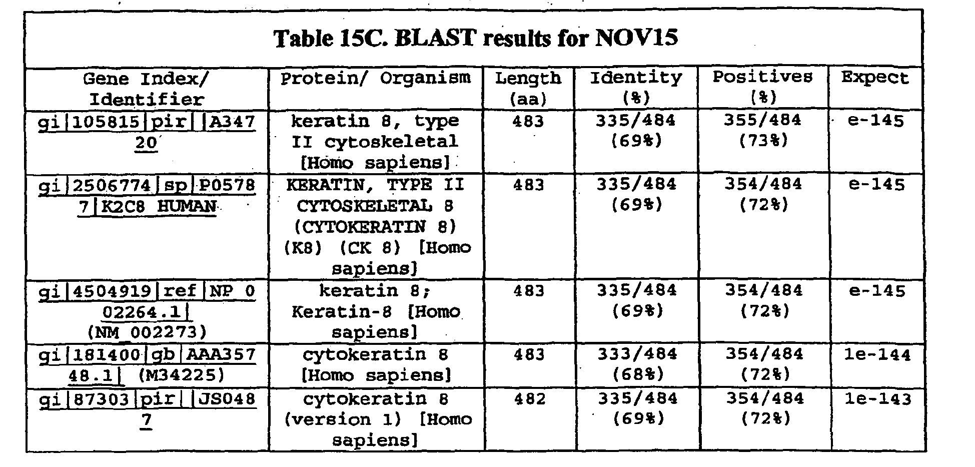

The homology of these sequences is shown graphically in the ClustalW analysis shown in Table 15D.

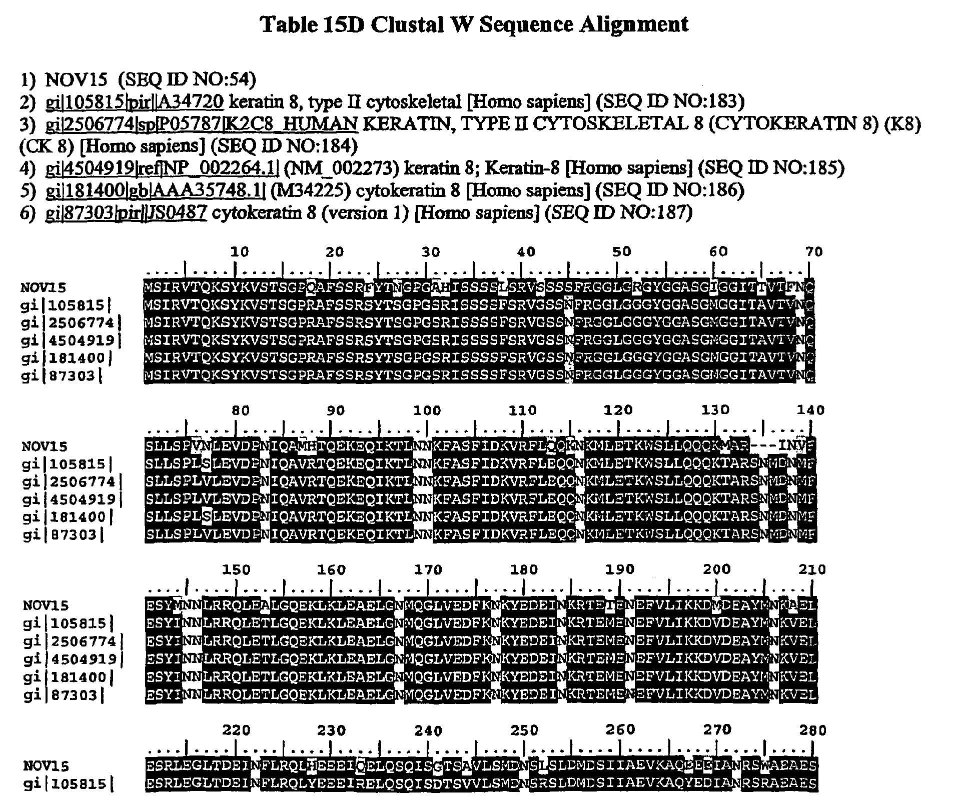

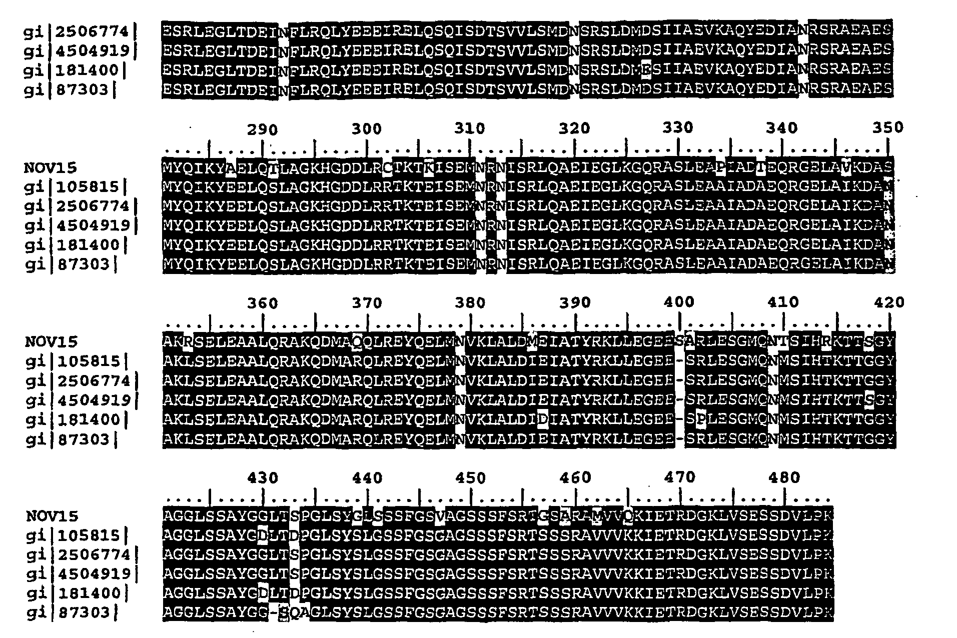

Table 15E lists the domain description from DOMAIN analysis results against NOV15. This indicates that the NOV15 sequence has properties similar to those of other proteins known to contain these domains.

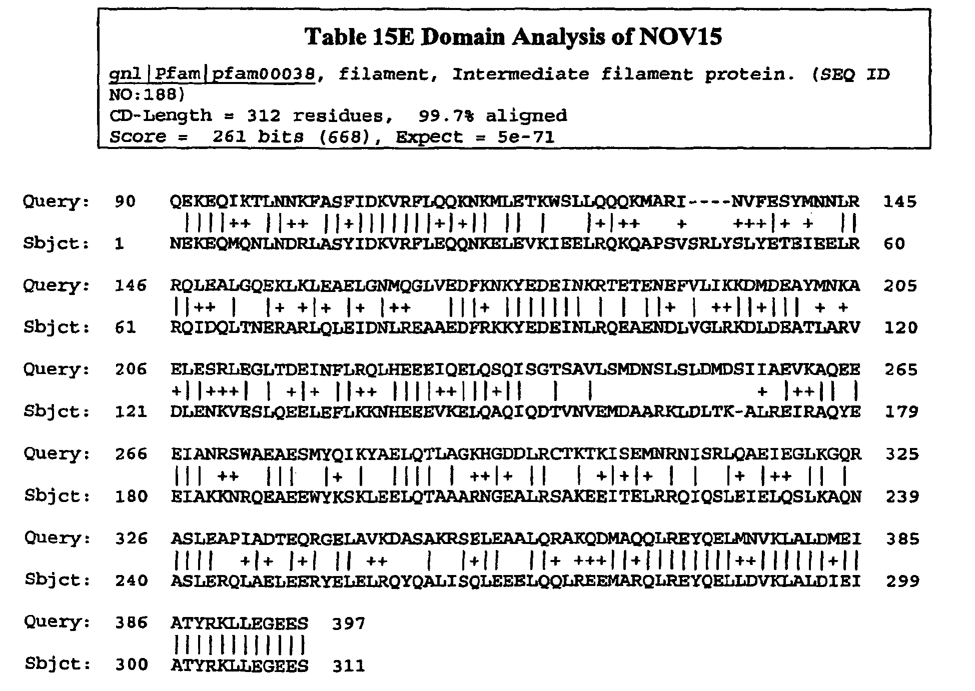

Keratin 8 is a type II keratin (Moll et al., 1982). Endo A is the mouse equivalent. Endo B, which is the equivalent of human keratin 18, a type I keratin, is coexpressed with Endo A; the 2 appear to be the first intermediate filament (IF) proteins expressed during murine development (Jackson et al., Differentiation. 17(3):161-79, 1980). Yamamoto et al. (Mol Endocrinol. 4(3):370-4, 1990) studied a full-length cDNA for cytokeratin 8 from placenta. They determined the distribution of cytokeratin 8 mRNA in various fetal tissues and in placentae of different gestational ages. Keratins 8 and 18 of simple epithelia differ from the keratins of stratified epithelium in tissue expression and regulation.

Using PCR to study DNAs from somatic cell hybrids, Waseem et al. (Genomics. 7(2):188-94, 1990) located a single active gene for keratin 8 on chromosome 12. This chromosome contains several genes for type II keratins and also the gene for keratin 18, the type I keratin that is coexpressed with keratin 8. This location of both members of a keratin pair on a single chromosome is unique among keratin genes; it is consistent with the hypothesis that keratins 8 and 18 may be closer to an ancestral gene than the keratins of more highly differentiated epithelia. Casanova et al. (J Clin Invest. 103(11):1587-95, 1999) generated mice expressing the human KRT8 gene, leading to a moderate increase in the content of keratin in simple epithelia. These mice displayed progressive exocrine pancreas alterations, including dysplasia and loss of acinar architecture, redifferentiation of acinar to ductal cells, inflammation, fibrosis, and substitution of exocrine by adipose tissue, as well as increased cell proliferation and apoptosis. The phenotype was very similar to that reported for transgenic mice expressing a dominant-negative mutant TGF-beta type II receptor. Casanova et al. (1999) showed that these Tgfbr2 mutant mice also had elevated KRT8/KRT18 levels. The results indicated that simple epithelial keratins play a relevant role in the regulation of exocrine pancreas homeostasis and supported the idea that disruption of mechanisms that normally regulate keratin expression in vivo could be related to inflammatory and neoplastic pancreatic disorders.

The NOV15 nucleic acid of the invention encoding a Cytokeratin-like protein includes the nucleic acid whose sequence is provided in Table 15A, or a fragment thereof. The invention also includes a mutant or variant nucleic acid any of whose bases may be changed from the corresponding base shown in Table 15A while still encoding a protein that maintains its Cytokeratin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applicifions in a subject. In the mutant or variant nucleic acids, and their complements, up to about 12% of the residues may be so changed.

The NOV15 protein of the invention includes the Cytokeratin-like protein whose sequence is provided in Table 15B. The invention also includes a mutant or variant protein any of whose residues may be changed from the corresponding residue shown in Table 15B while still encoding a protein that maintains its Cytokeratin-like activities and physiological functions, or a functional fragment thereof. In the mutant or variant protein, up to about 13% of the bases may be so changed.

The NOV15 nucleic acids and proteins of the invention are useful in potential diagnostic and therapeutic applications implicated in various diseases and disorders described below and/or other pathologies. For example, the compositions of the present invention will have efficacy for treatment of patients suffering from: Cardiomyopathy, Atherosclerosis, Hypertension, Congenital heart defects, Aortic stenosis, Atrial septal defect (ASD), Atrioventricular (A-V) canal defect, Ductus arteriosus, Pulmonary stenosis, Subaortic stenosis, Ventricular septal defect (VSD), valve diseases, Tuberous sclerosis, Scleroderma, Obesity, Transplantation, Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection and other diseases, disorders and conditions of the like.

NOV15 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV15 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV16

NOV16 includes four novel Protocadherin-like proteins disclosed below. The disclosed proteins have been named NOV16a, NOV16b, NOV16c and NOV16d.

### NOV16a

A disclosed NOV16a nucleic acid of 2907 nucleotides (also referred to as CG57448-01) encoding a novel Protocadherin-like protein is shown in Table 16A. An open reading frame was identified beginning with an ATG initiation codon at nucleotides 10-12 and ending with a TGA codon at nucleotides 2896-2898. Putative untranslated regions, if any, upstream from the initiation codon and downstream from the termination codon are underlined in Table 16A, and the start and stop codons are in bold letters.

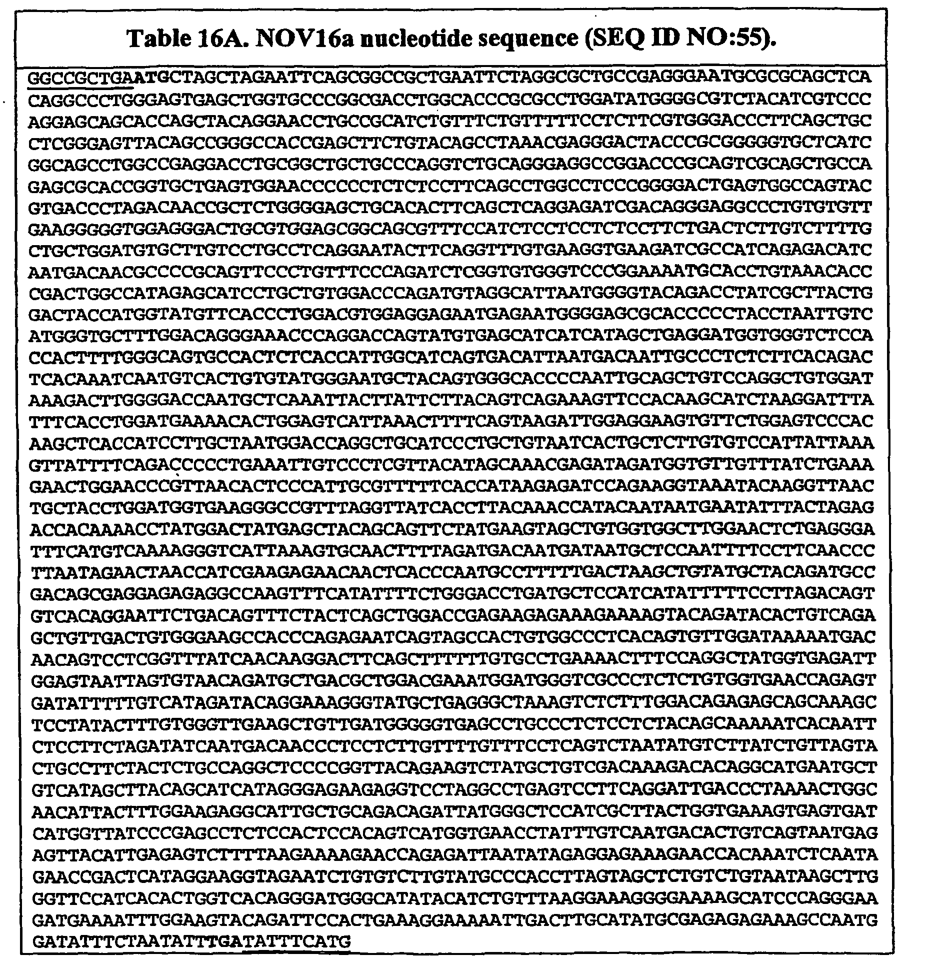

The disclosed NOV16a nucleic acid sequence was localized to chromsome 5.

A NOV16a polypeptide (SEQ ID NO:56) encoded by SEQ ID NO:55 has 962 amino acid residues and is presented using the one-letter code in Table 16B. Signal P, Psort and/or Hydropathy results predict that NOV16a contains a signal peptide and is likely to be localized to the mitochondrial inner membrane with a certainty of 0.6916, to the mitochondrial intermembrane space with a certainty of 0.6185, to the plasma membrane with a certainty of 0.6000 and to the mitochondrial matrix space with a certainty of 0.5077. The most likely cleavage site for a NOV16a polypeptide is between amino acids 13 and 14: SRR-CR

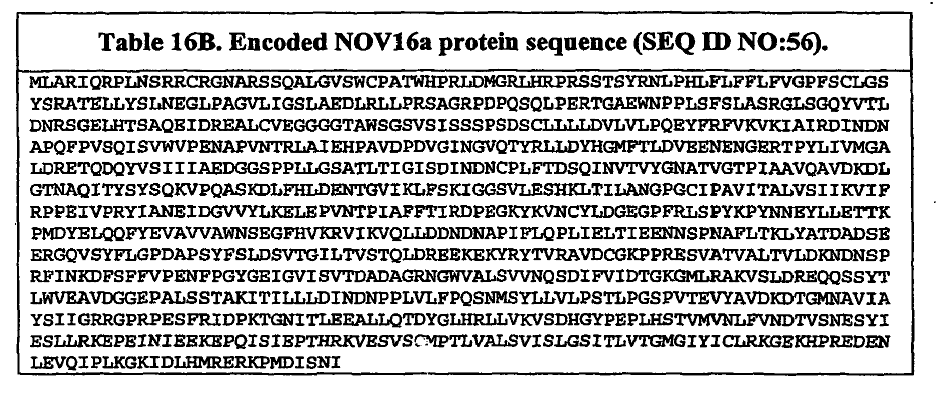

The NOV16a amino acid sequence has 946 of 947 amino acid residues (99%) identical to, and 946 of 947 amino acid residues (99%) similar to, the 947 amino acid residue ptnr:SPTREMBIrACC:Q9NRT9 protein from *Homo sapiens* (Human) (Protocadherin 10) (E = 0.0).

The disclosed NOV16a is expressed in at least the following tissues: Uterus, colon, eye and retina. This information was derived by determining the tissue sources of the sequences that were included in the invention including but not limited to SeqCalling sources, Public EST sources, Literature sources, and/or RACE sources.

### NOV16b

A disclosed NOV 16b nucleic acid of 2801 nucleotides (also referred to as CG57446-01) encoding a novel Protocadherin-like protein is shown in Table 16C. An open reading frame was identified beginning with an ATG initiation codon at nucleotides 6-8 and ending with a TAA codon at nucleotides 2724-2726. Putative untranslated regions, if any, upstream from the initiation codon and downstream from the termination codon are underlined in Table 16C, and the start and stop codons are in bold letters.

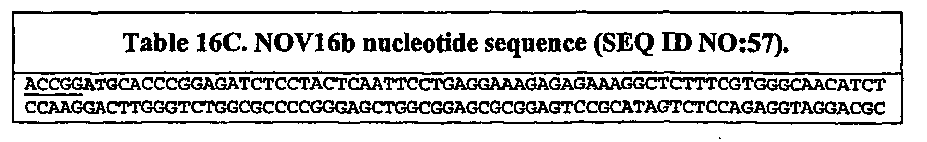

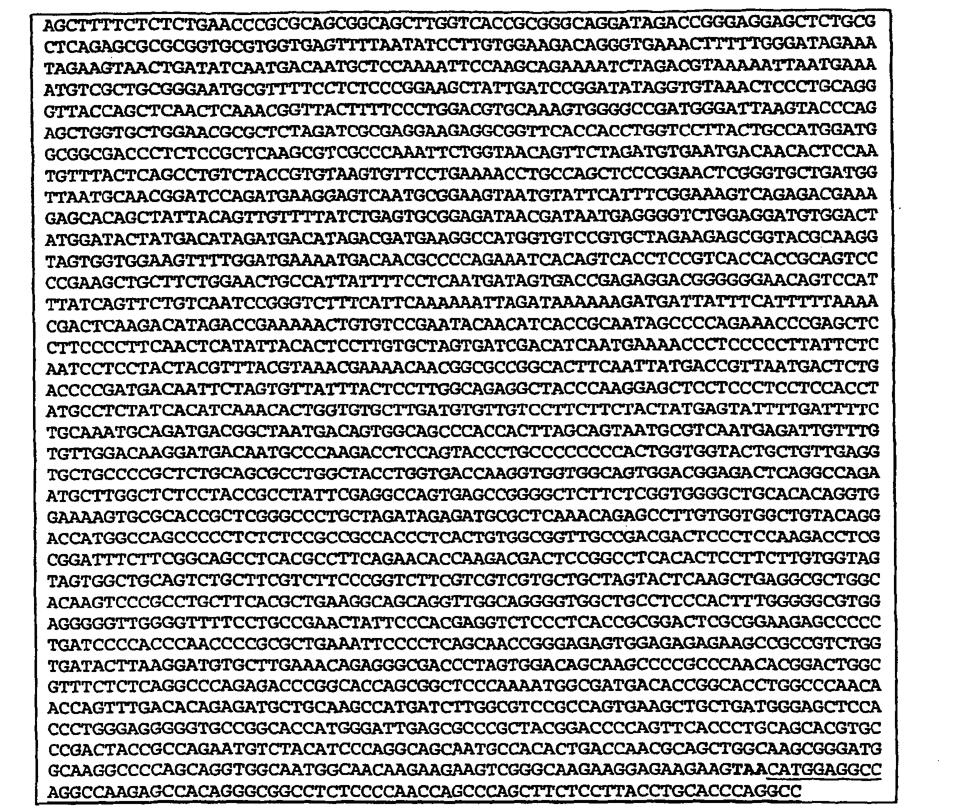

The disclosed NOV16b nucleic acid sequence, localized to chromsome 5, has 2214 of 2786 bases (79%) identical to a gb:GENBANK-ID:AF152324|acc:AF152324.1 mRNA from *Homo sapiens* (protocadherin gamma A4 (PCDH-gamma-A4) mRNA, complete cds) (E = 0.0).

A NOV16b polypeptide (SEQ ID NO:58) encoded by SEQ ID NO:57 has 906 amino acid residues and is presented using the one-letter code in Table 16D. Signal P, Psort and/or Hydropathy results predict that NOV16b does not contain a signal peptide and is likely to be localized to the plasma membrane with a certainty of 0.7000 and to the nucleus with a certainty of 0.6000.

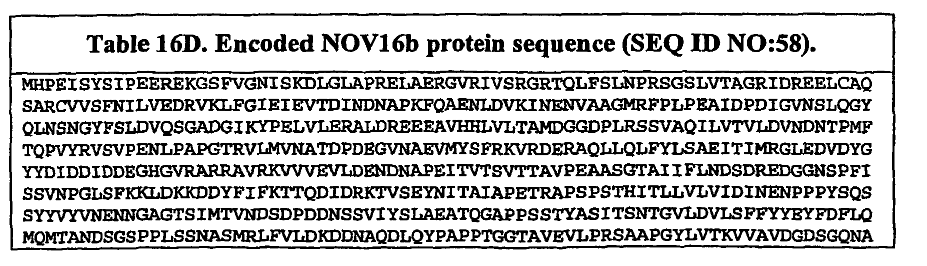

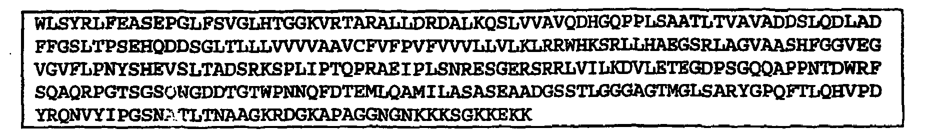

The NOV16b amino acid sequence has 635 of 903 amino acid residues (70%) identical to, and 721 of 903 amino acid residues (79%) similar to, the 931 amino acid residue ptar:SPTREMBL-ACC:Q9Y5G9 protein from *Homo sapiens* (Human) (Protocadherin Gamma A4) (E = 0.0).

The disclosed NOV16b is expressed in at least the following tissues: Uterus, colon, eye and retina. This information was derived by determining the tissue sources of the sequences that were included in the invention including but not limited to SeqCalling sources, Public EST sources, Literature sources, and/or RACE sources.

### NOV16c

A disclosed NOV16c nucleic acid of 2836 nucleotides (also referred to as CG57444-01) encoding a novel Protocadherin-like protein is shown in Table 16E. An open reading frame was identified beginning with an ATG initiation codon at nucleotides 15-17 and ending with a TAA codon at nucleotides 2802-2804. Putative untranslated regions, if any, upstream from the initiation codon and downstream from the termination codon are underlined in Table 16E, and the start and stop codons are in bold letters.

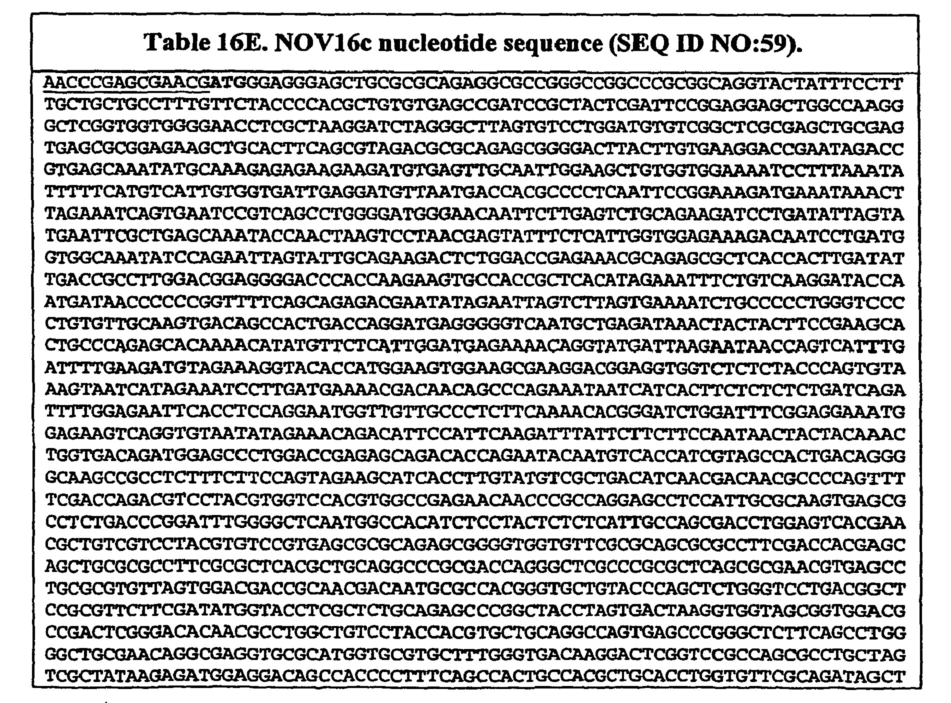

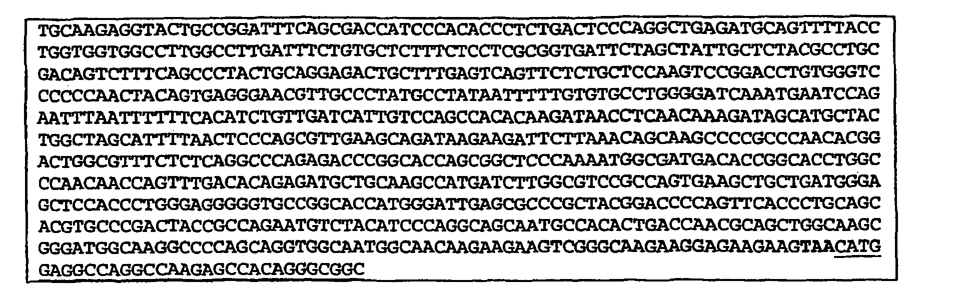

The disclosed NOV16c nucleic acid sequence, localized to chromsome 5, has 2584 of 2822 bases (91%) identical to a gb:GENBANK-ID:AF152336|acc:AF152336.1 mRNA from *Homo sapiens* (protocadherin gamma B7 (PCDH-gamma-B7) mRNA, complete cds) (E = 0.0).

A NOV16c polypeptide (SEQ ID NO:60) encoded by SEQ ID NO:59 has 929 amino acid residues and is presented using the one-letter code in Table 16F. Signal P, Psort and/or Hydropathy results predict that NOV16c does not contain a signal peptide and is likely to be localized to the plasma membrane with a certainty of 0.5140. The most likely cleavage site for a NOV16c polypeptide is between amino acids 30 and 31: TLC-EP

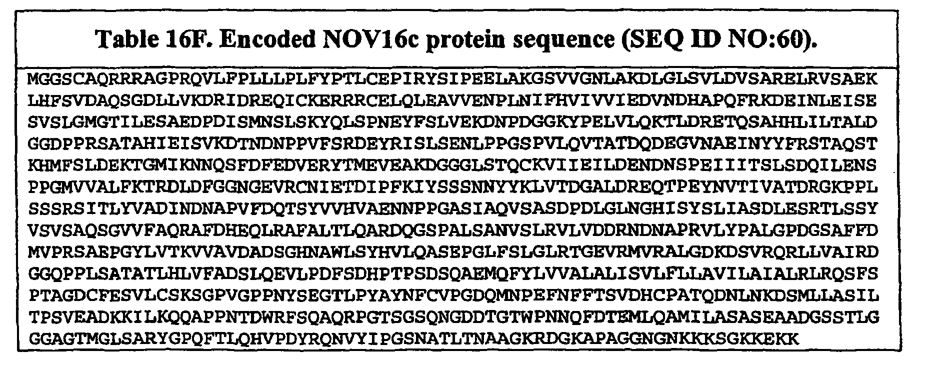

The NOV16c amino acid sequence has 842 of 929 amino acid residues (90%) identical to, and 881 of 929 amino acid residues (94%) similar to, the 929 amino acid residue ptnr:SPTREMBL-ACC:Q9Y5F8 protein from *Homo sapiens* (Human) (Protocadherin Gamma B7) (E = 0.0).

The disclosed NOV16c is expressed in at least the following tissues: Uterus, colon, eye and retina. This information was derived by determining the tissue sources of the sequences that were included in the invention including but not limited to SeqCalling sources, Public EST sources, Literature sources, and/or RACE sources.

### NOV16d

A disclosed NOV 1 6d nucleic acid of 2436 nucleotides (also referred to as CG57442-01) encoding a novel Protocadherin Beta-like protein is shown in Table 16G. An open reading frame was identified beginning with an ATG initiation codon at nucleotides 1-3 and ending with a TGA codon at nucleotides 2398-2400. Putative untranslated regions, if any, upstream from the initiation codon and downstream from the termination codon are underlined in Table 16G, and the start and stop codons are in bold letters.

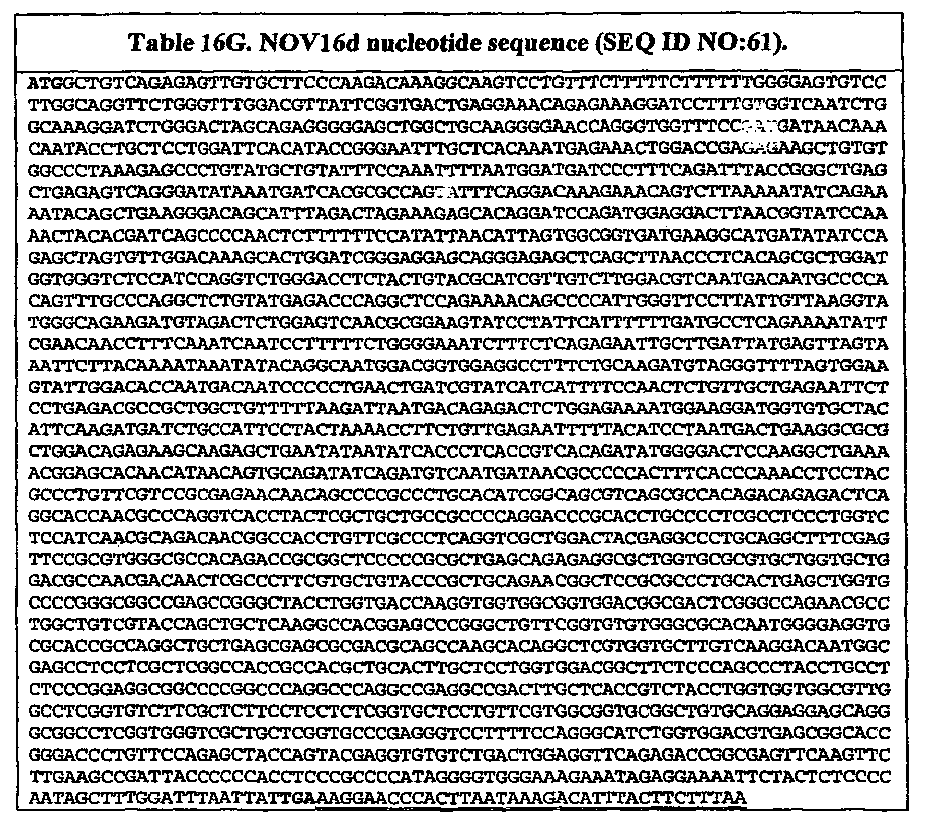

The disclosed NOV16d nucleic acid sequence, localized to chromsome 5, has 2319 of 2400 bases (96%) identical to a gb:GENBANK-m:AF152489|acc:AF152489.1 mRNA from *Homo sapiens (Homo sapiens* protocadherin beta 10 (PCDH-beta10) mRNA, complete cds) (E = 0.0).

A NOV16d polypeptide (SEQ ID NO:62) encoded by SEQ ID NO:61 has 799 amino acid residues and is presented using the one-letter code in Table 16H. Signal P, Psort and/or Hydropathy results predict that NOV16d does not contain a signal peptide and is likely to be localized to the plasma membrane with a certainty of 0.4600. The most likely cleavage site for a NOV16d polypeptide is between amino acids 26 and 27: SLA-GS.

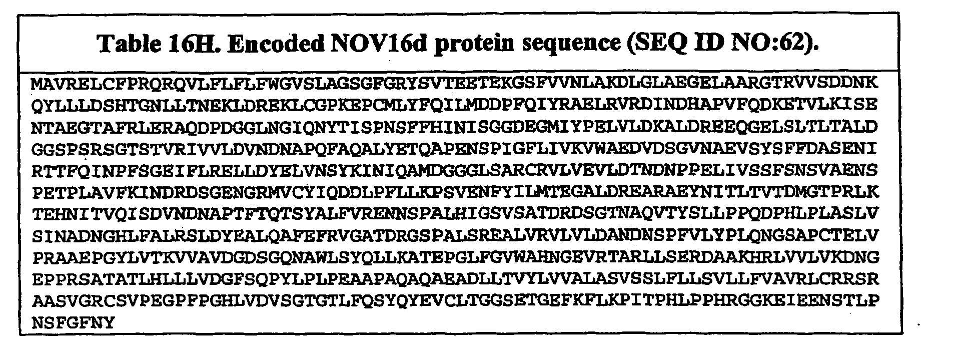

The NOV16d amino acid sequence has 765 of 798 amino acid residues (95%) identical to, and 776 of 798 amino acid residues (97%) similar to, the 800 amino acid residue ptnr:SPTREMBL-ACC:Q9UN67 protein from *Homo sapiens* (Human) (Protocadherin Beta 10) (E = 0.0).

The disclosed NOV16d is expressed in at least the following tissues: Uterus, colon, eye and retina. This information was derived by determining the tissue sources of the sequences that were included in the invention including but not limited to SeqCalling sources, Public EST sources, Literature sources, and/or RACE sources.

Possible SNPs found for NOV 1 6c are listed in Table 16L

| **Table 16I: SNPs** | | | | |
|---|---|---|---|---|
| **Variant** | **Nucleotide Position** | **Base Change** | **Amino Acid Position** | **Base Change** |
| 13377099 | 218 | G > T | Silent | N/A |

NOV16a - NOV 16d are very closely homologous as is shown in the amino acid alignment in Table 16J.

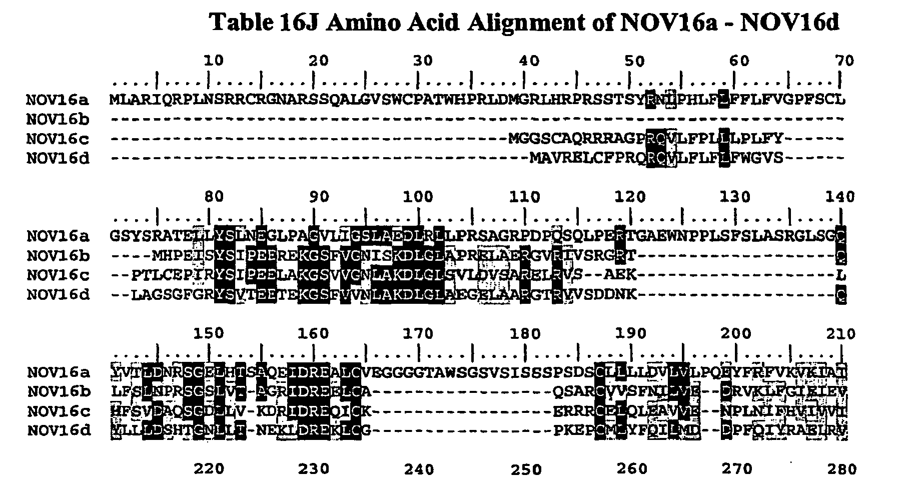

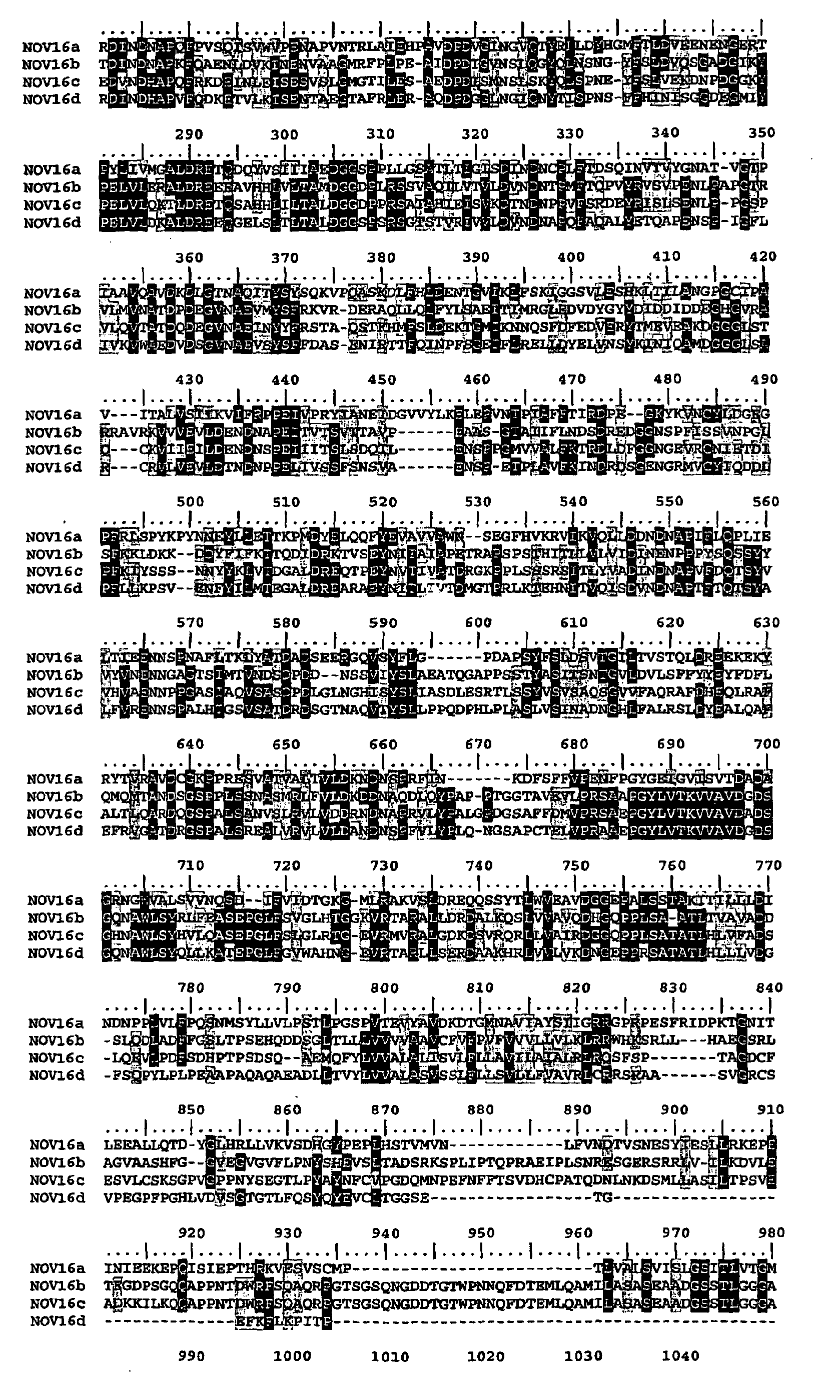

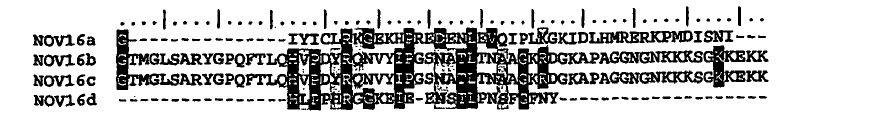

Homologies to any of the above NOV16 proteins will be shared by the other NOV16 proteins insofar as they are homologous to each other as shown above. Any reference to NOV 16 is assumed to refer to the NOV 16 proteins in general, unless otherwise noted.

NOV16a has homology to the amino acid sequences shown in the BLASTP data listed in Table 16K.

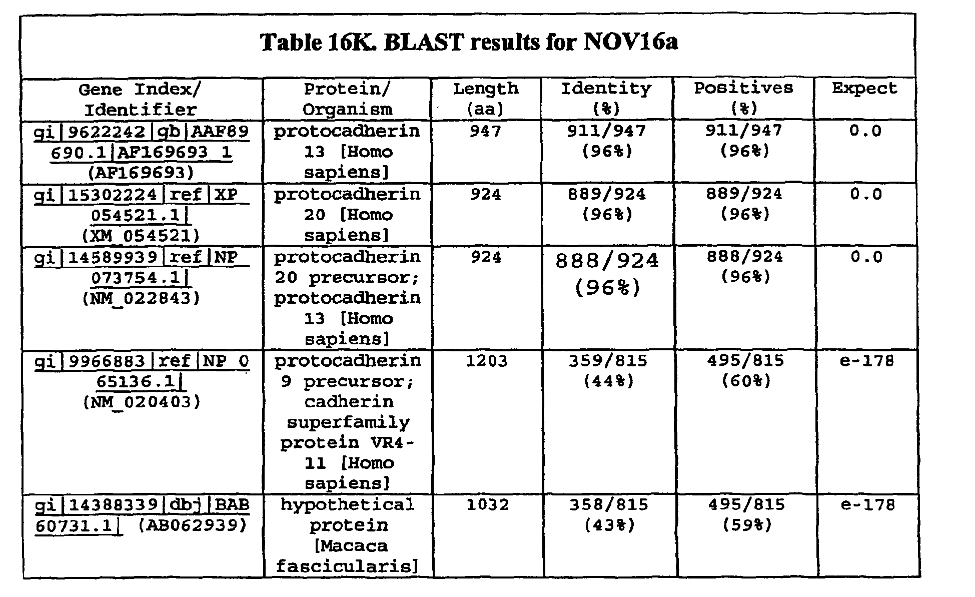

The homology of these sequences is shown graphically in the ClustalW analysis shown in Table 16L.

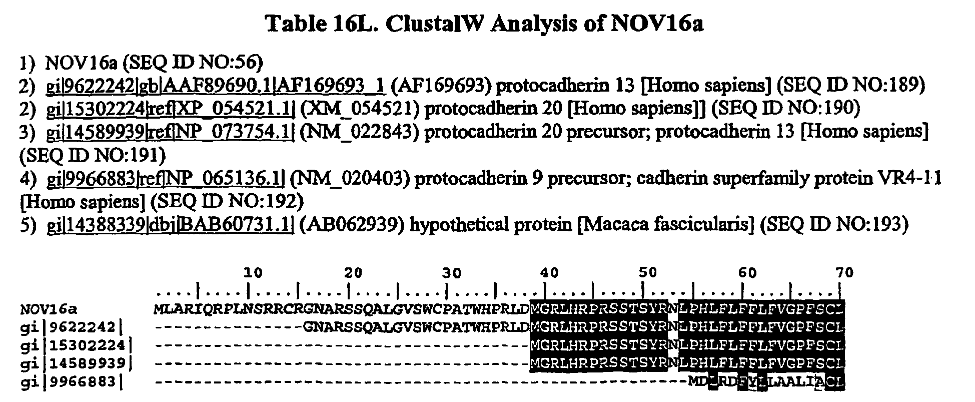

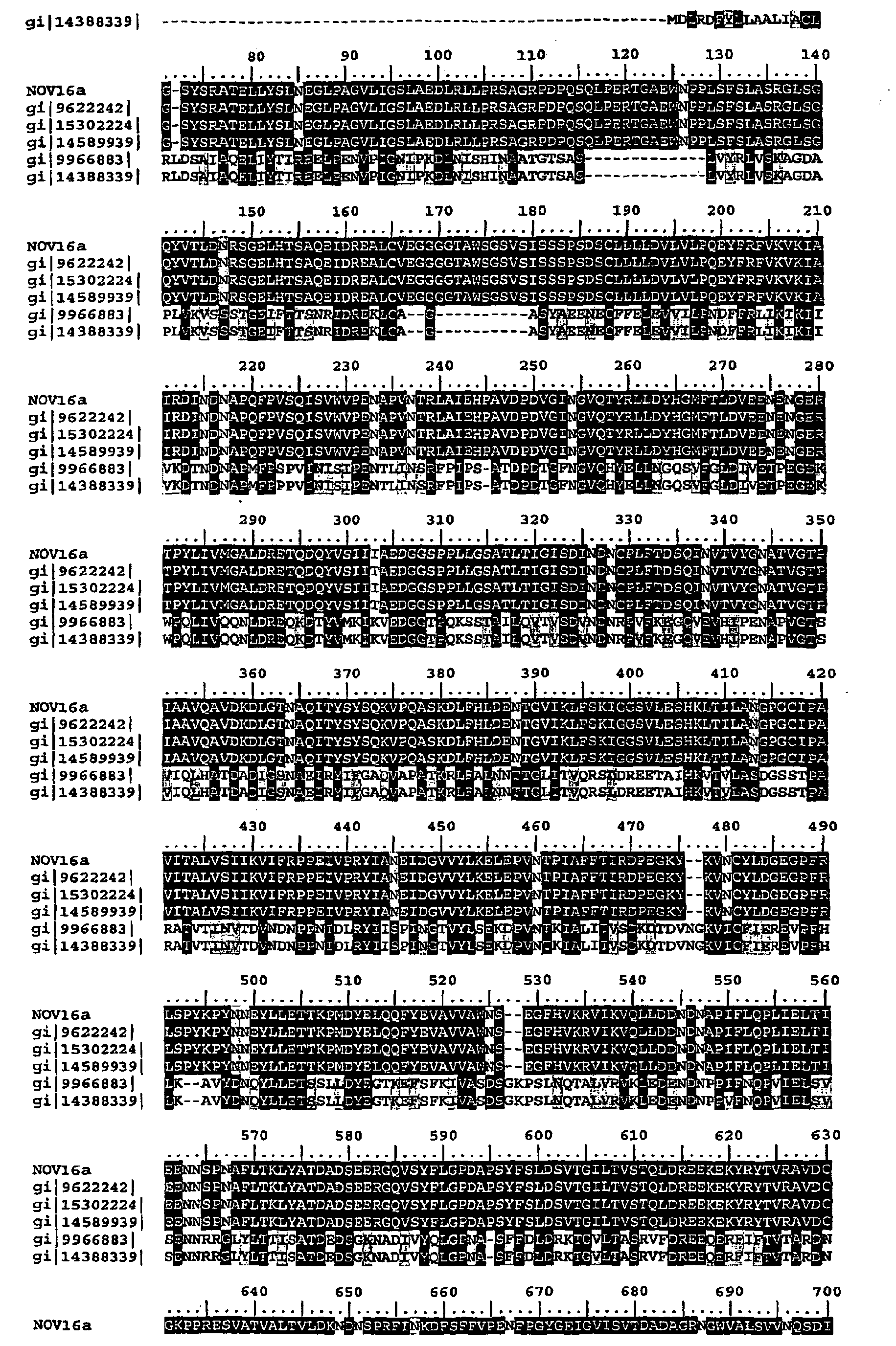

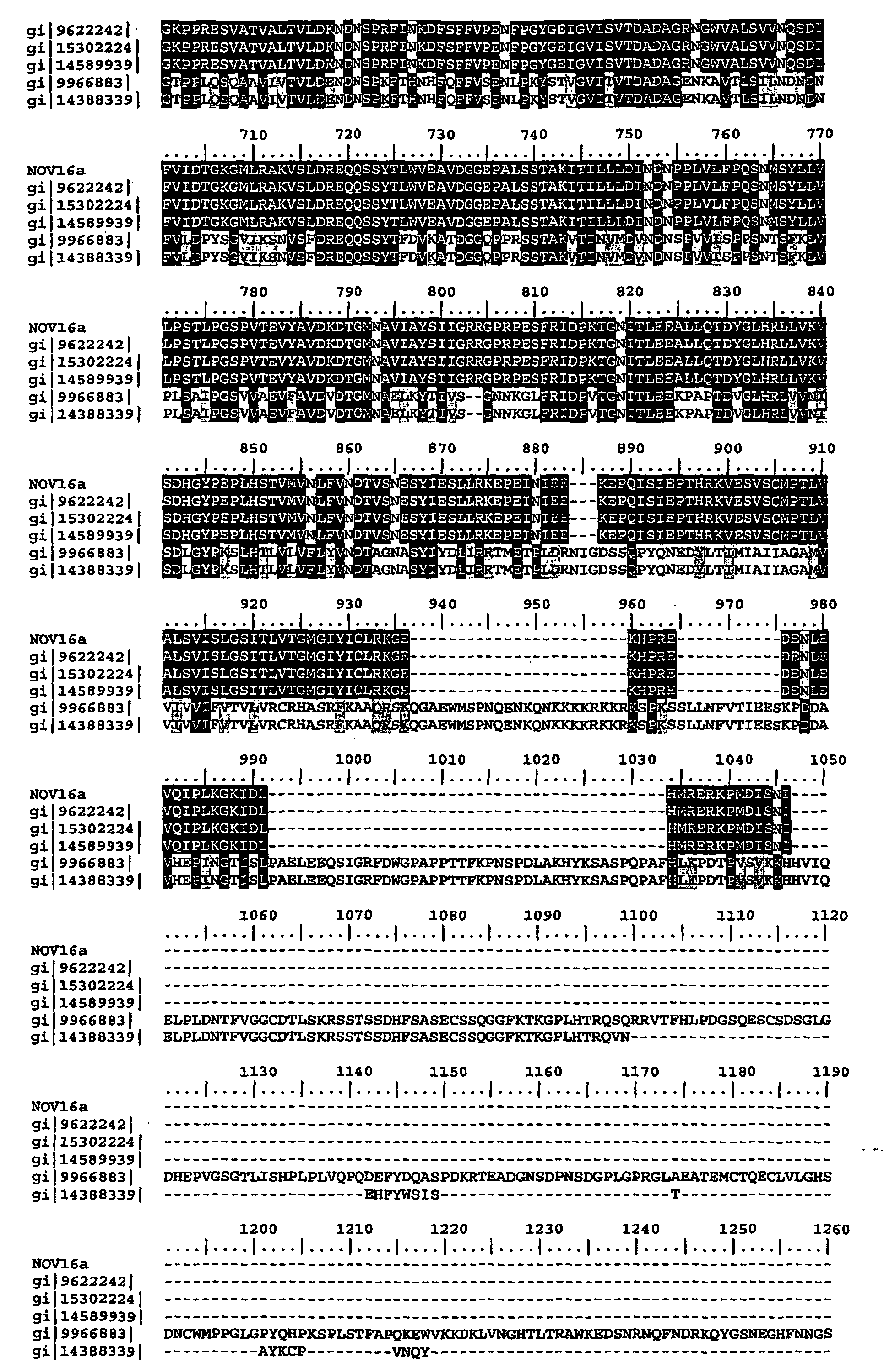

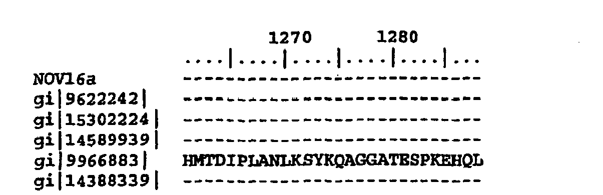

Tables 16M and 16N list the domain description from DOMAIN analysis results against NOV16a. This indicates that the NOV16a sequence has properties similar to those of other proteins known to contain these domains.

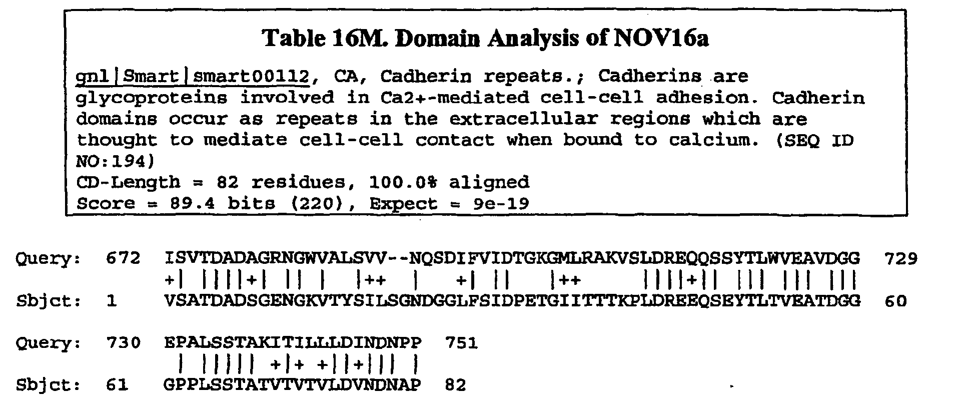

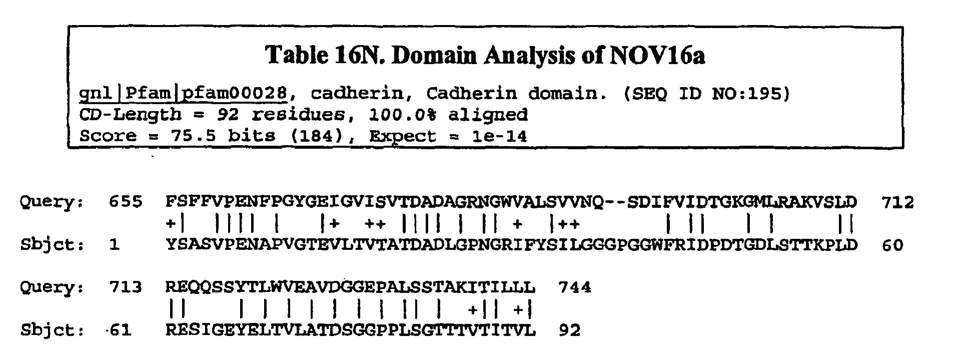

Cadherins, a family of calcium-dependent cell-cell adhesion molecules, mediate neural cell-cell interactions. Sperry (1963) proposed that neurons recognize their synaptic partners through lock-and-key interactions mediated by millions of specifier molecules. Cadherins were likely candidates for components of the lock-and-key mechanism based on their neural location, their adhesive diversity, and the structural biology of their adhesive interactions maintaining a synapse gap size of approximately 200 angstroms (Shapiro and Colman, Neuron. 23(3):427-30, 1999). Neural cadherins (CDH2, 114020), as well as epithelial (CDH1, 192090), placental (CDH3, 114021), and retinal (CDH4, 603006) cadherins, have homophilic binding specificities in that they preferentially adhere to cells expressing the same cadherin type. Cadherins of the 'classic' type have a highly conserved extracellular sequence motif of approximately 110 amino acids that is repeated 5 times as well as a highly conserved cytoplasmic domain of approximately 200 amino acids that associates with intracellular actin microfilaments via catenins (see CTNNA1). 'Nonclassic' cadherins differ in that they may have 6 or 7 repeated extracellular domains or have cytoplasmic domains that connect to intermediate filaments instead of actin.

Protocadherins constitute a subfamily of the nonclassic cadherins. Kohmura et al. (Neuron. 20(6):1137-51, 1998) described cadherin-related neuronal receptors (CNR) in the mouse. By EST database searching for cadherin-like sequences, Wu and Maniatis (Cell 97(6):779-90, 1999) identified 52 novel genes organized into 3 closely linked tandem clusters on human chromosome 5q31. A distinct large exon of approximately 2,400 nucleotides encodes the 6 N-terminal extracellular domains and the transmembrane domain of each cadherin. In contrast, the C terminus of each of these proteins is identical within each cluster and is encoded by 3 small exons located downstream from the array of N-terminal exons. Wu and Maniatis (1999) designated the clusters alpha, beta and gamma. Each large exon is independently spliced to the first exon encoding the intracellular domain. The authors also denoted the extracellular portion as the variable region and the cytoplasmic portion as the constant region. The alpha cluster contains at least 15 proteins encoded by large, uninterrupted exons whose sequences most closely resemble those of the mouse CNR proteins. Wu and Maniatis (1999) proposed 4 models to explain protocadherin gene regulation and noted that several neurologic disorders map to chromosome 5q31.

The NOV 16 nucleic acid of the invention encoding a Protocadherin-like protein includes the nucleic acid whose sequence is provided in Tables 16A, 16C, 16E and 16G, or a fragment thereof. The invention also includes a mutant or variant nucleic acid any of whose bases may be changed from the corresponding base shown in Tables 16A, 16C, 16E and 16G while still encoding a protein that maintains its Protocadherin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 21 % of the NOV16b residues, about 9% of the NOV16c residues and about 4% of the NOV16d residues may be so changed.

The NOV 16 protein of the invention includes the Protocadherin-like protein whose sequence is provided in Tables 16B, 16D, 16F and 16H. The invention also includes a mutant or variant protein any of whose residues may be changed from the corresponding residue shown in Tables 16B, 16D, 16F and 16H while still encoding a protein that maintains its Protocadherin-like activities and physiological functions, or a functional fragment thereof. In the mutant or variant protein, up to about 1% of the NOV16a bases, about 30% of the NOV16b bases, about 10% of the NOV16c bases and about 5% of the NOV16d bases may be so changed.

The NOV16 nucleic acids and proteins of the invention are useful in potential diagnostic and therapeutic applications implicated in various diseases and disorders described below and/or other pathologies. For example, the compositions of the present invention will have efficacy for treatment of patients suffering from: Hirschsprung's disease, Crohn's Disease, Appendicitis, Von Hippel-Lindau (VHL) syndrome, Diabetes, Tuberous sclerosis, Endometriosis, Fertility and other diseases, disorders and conditions of the like.

NOV16 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV16 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV17

NOV17 includes two novel Cadherin 23-like proteins disclosed below. The disclosed proteins have been named NOV17a and NOV17b.

### NOV17a

A disclosed NOV17a nucleic acid of 1303 nucleotides (also referred to as CG57429-01) encoding a novel Cadherin 23-like protein is shown in Table 17A. An open reading frame was identified beginning with an ATG initiation codon at nucleotides 31-33 and ending with a TAG codon at nucleotides 1291-1293. Putative untranslated regions, if any, upstream from the initiation codon and downstream from the termination codon are underlined in Table 17A, and the start and stop codons are in bold letters.

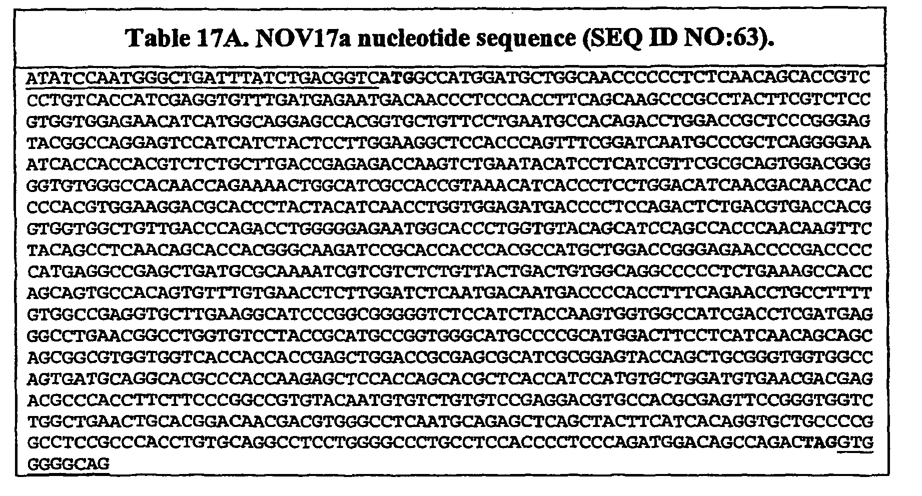

The disclosed NOV17a nucleic acid sequence, localized to chromsome 10, has 702 of 703 bases (99%) identical to a gb:GENBANK-ID:AY010111|acc:AY010111.1 mRNA from *Homo sapiens (Homo sapiens* cadherin-23 (CDH23) mRNA, partial cds) (E = 6.9e⁻¹⁵²).

A NOV17a polypeptide (SEQ ID NO:64) encoded by SEQ ID NO:63 has 420 amino acid residues and is presented using the one-letter code in Table 17B. Signal P, Psort and/or Hydropathy results predict that NOV17a does not contain a signal peptide and is likely to be localized to the plasma membrane with a certainty of 0.7900.

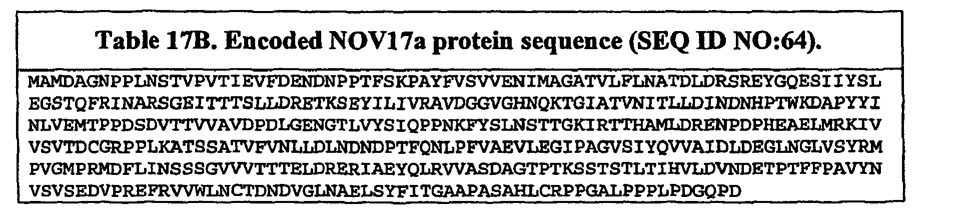

The NOV17a amino acid sequence has 233 of 234 amino acid residues (99%) identical to, and 234 of 234 amino acid residues (100%) similar to, the 2552 amino acid residue ptnr:TREMBLNEW-ACC:AAG27034 protein from *Homo sapiens* (Human) (CADHERIN-23) (E = 1.8e⁻¹²⁰).

The disclosed NOV17a is expressed in at least the following tissues: lung, pancreas, spinal chord and testis. This information was derived by determining the tissue sources of the sequences that were included in the invention including but not limited to SeqCalling sources, Public EST sources, Literature sources, and/or RACE sources.

### NOV17b

A disclosed NOV17b nucleic acid of 1278 nucleotides (also referred to as CG57429-02) encoding a novel Cadherin 23-like protein is shown in Table 17C. An open reading frame was identified beginning with an ATG initiation codon at nucleotides 6-8 and ending with a TAG codon at nucleotides 1266-1268. Putative untranslated regions, if any, upstream from the initiation codon and downstream from the termination codon are underlined in Table 17C, and the start and stop codons are in bold letters.

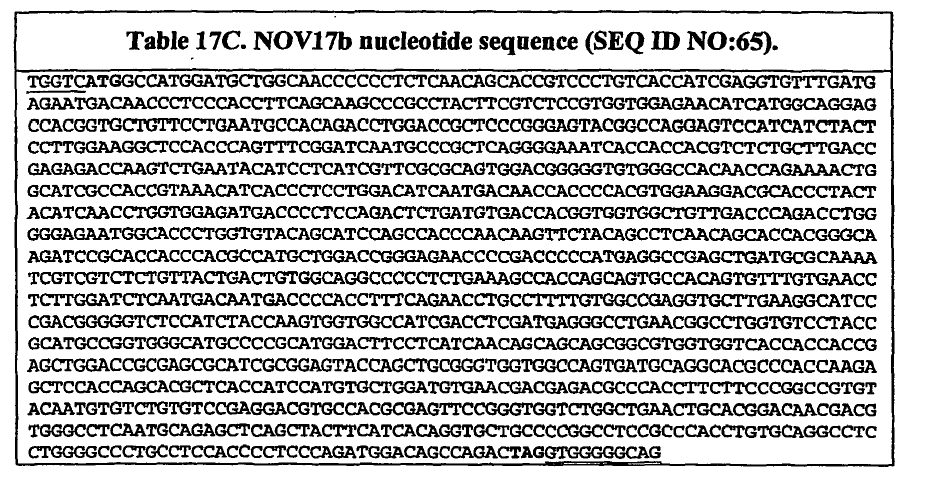

The disclosed NOV17b nucleic acid sequence, localized to chromsome 10, has 1188 of 1190 bases (99%) identical to a gb:GENBANK-ID:AF312024|acc:AF312024.1 mRNA from *Homo sapiens* (cadherin related 23 (CDH23) mRNA, complete cds) (E = 6.6e⁻²⁶³).

A NOV17b polypeptide (SEQ ID NO:66) encoded by SEQ ID NO:65 has 420 amino acid residues and is presented using the one-letter code in Table 17D. Signal P, Psort and/or Hydropathy results predict that NOV17b does not contain a signal peptide and is likely to be localized to the plasma membrane with a certainty of 0.7900.

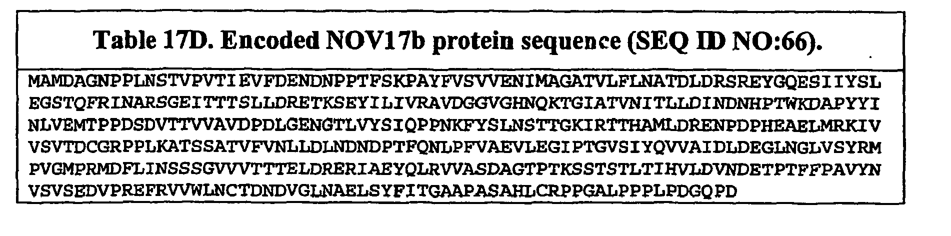

The NOV17b amino acid sequence has 394 of 395 amino acid residues (99%) identical to, and 394 of 395 amino acid residues (99%) similar to, the 3354 amino acid residue ptnr:SPTREMBL-ACC:Q9H251 protein from *Homo sapiens* (Human) (Cadherin Related 23) (E = 3.9e⁻²⁰⁸).

The disclosed NOV17b is expressed in at least the following tissues: lung, pancreas, spinal chord and testis. This information was derived by determining the tissue sources of the sequences that were included in the invention including but not limited to SeqCalling sources, Public EST sources, Literature sources, and/or RACE sources.

Possible SNPs found for NOV17a are listed in Table 17E.

| **Table 17E: SNPs** | | | | |
|---|---|---|---|---|
| **Variant** | **Nucleotide Position** | **Base Change** | **Amino Acid Position** | **Base Change** |
| 13377100 | 495 | C > T | Silent | N/A |
| 13377100 | 820 | G > Z | 264 | Ala > Thr |

NOV17a and NOV17b are very closely homologous as is shown in the nucleic acid alignment in Table 17F.

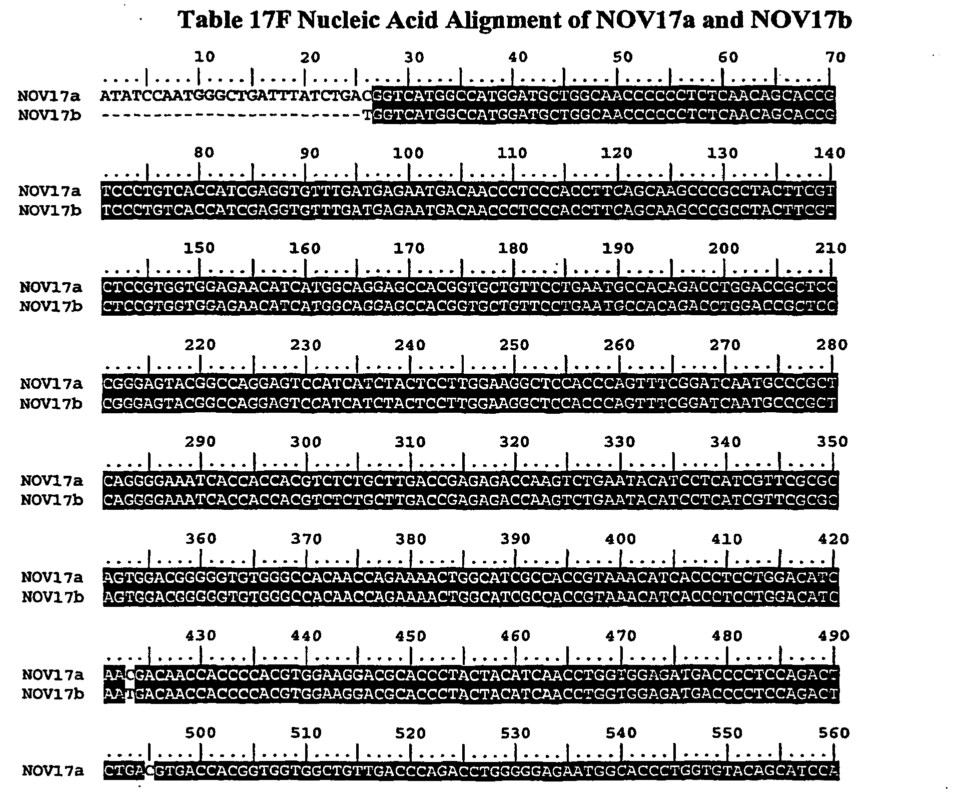

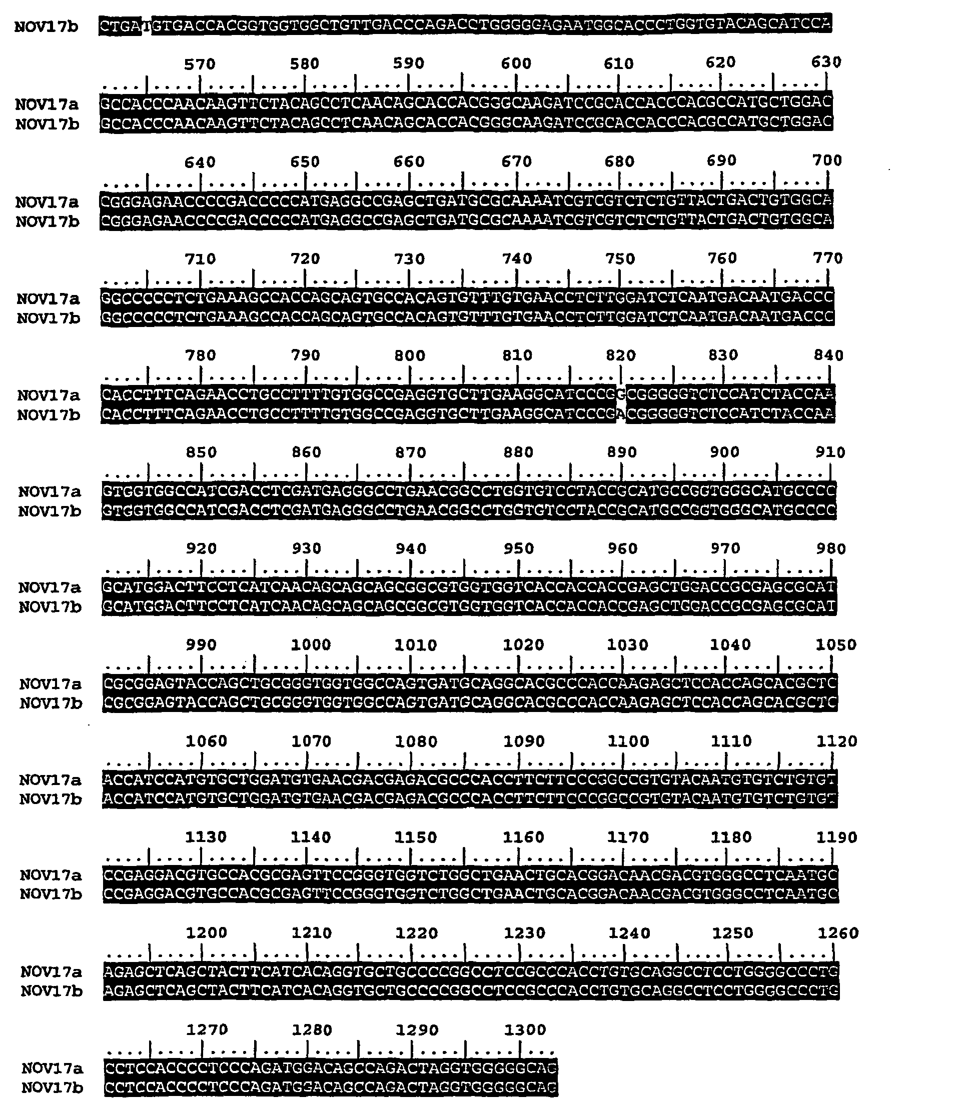

Homologies to any of the above NOV17 nucleic acids and encoded proteins will be shared by the other NOV17 nucleic acids and encoded proteins insofar as they are homologous to each other as shown above. Any reference to NOV1⁻ is assumed to refer to both of the NOV 17 nucleic acids and encoded proteins in general, unless otherwise noted.

NOV17a has homology to the amino acid sequences shown in the BLASTP data listed in Table 17G.

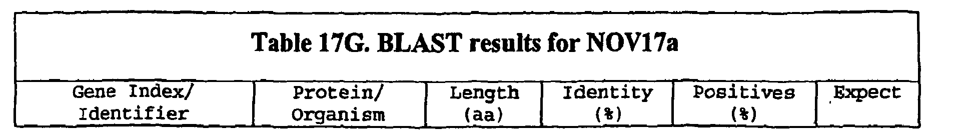

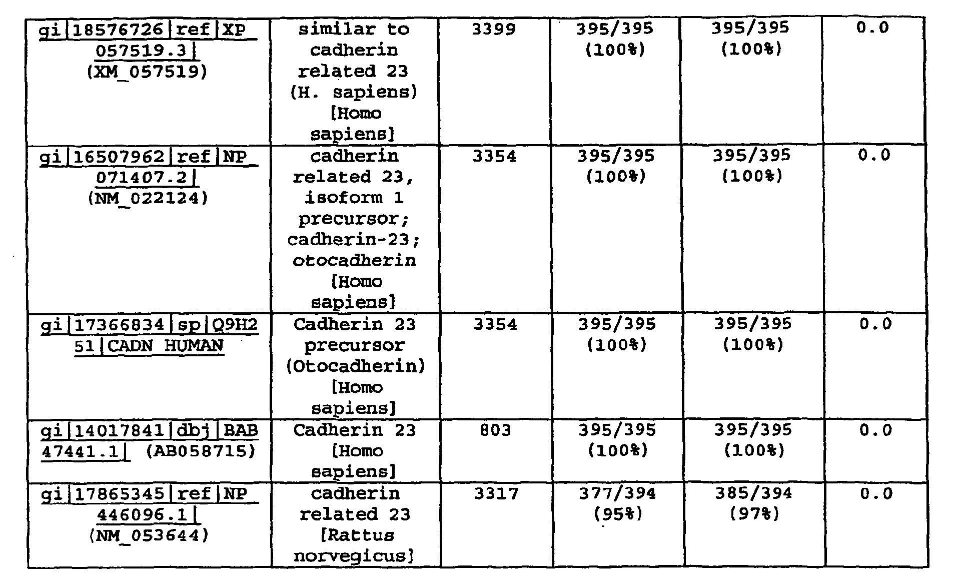

The homology of these sequences is shown graphically in the ClustalW analysis shown in Table 17H.

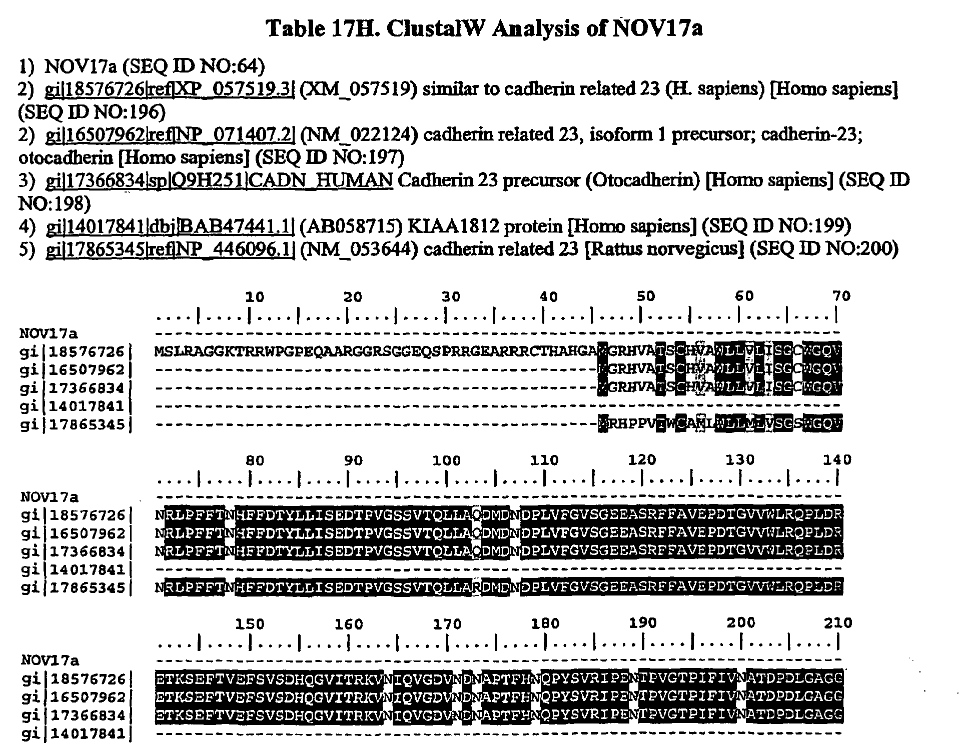

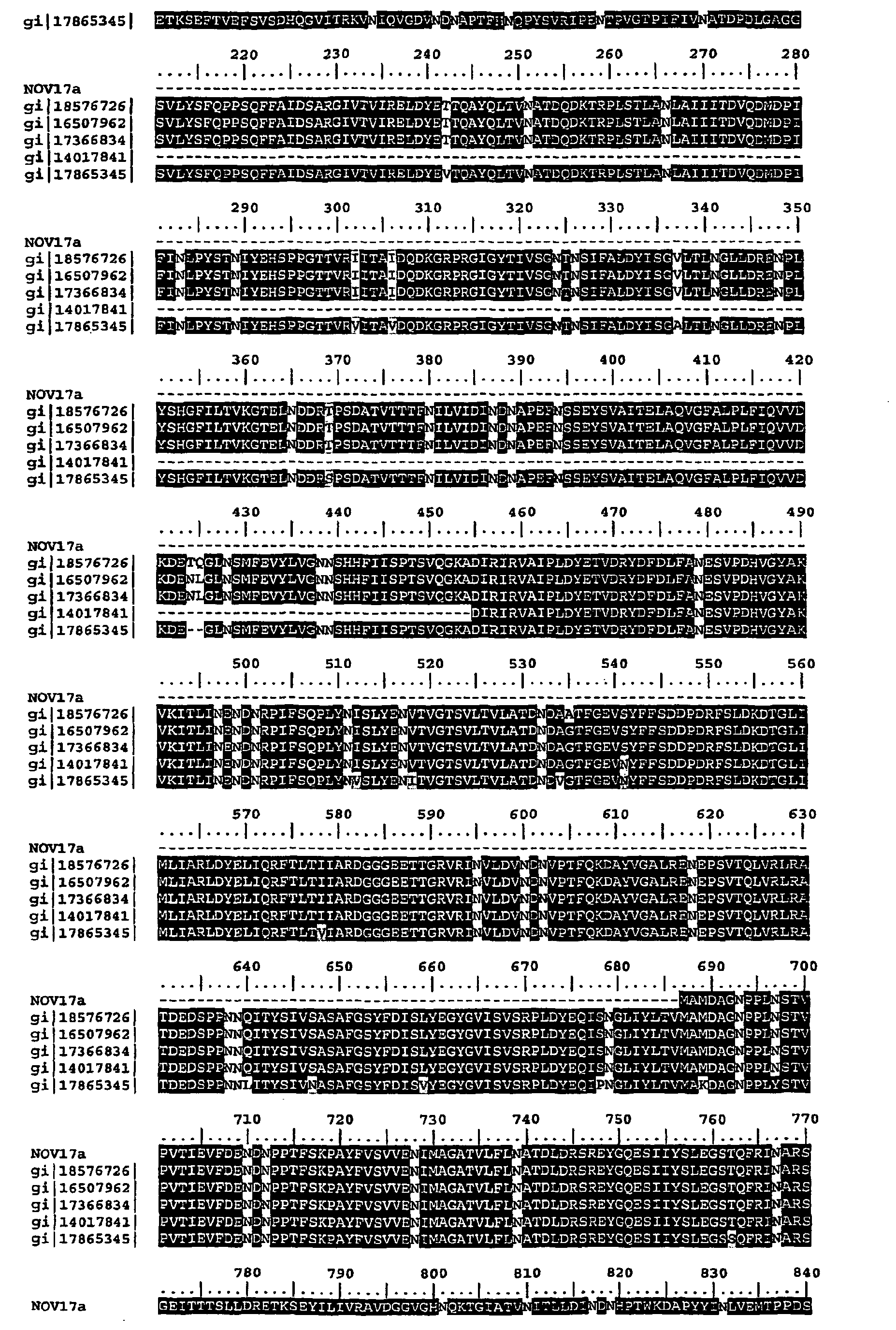

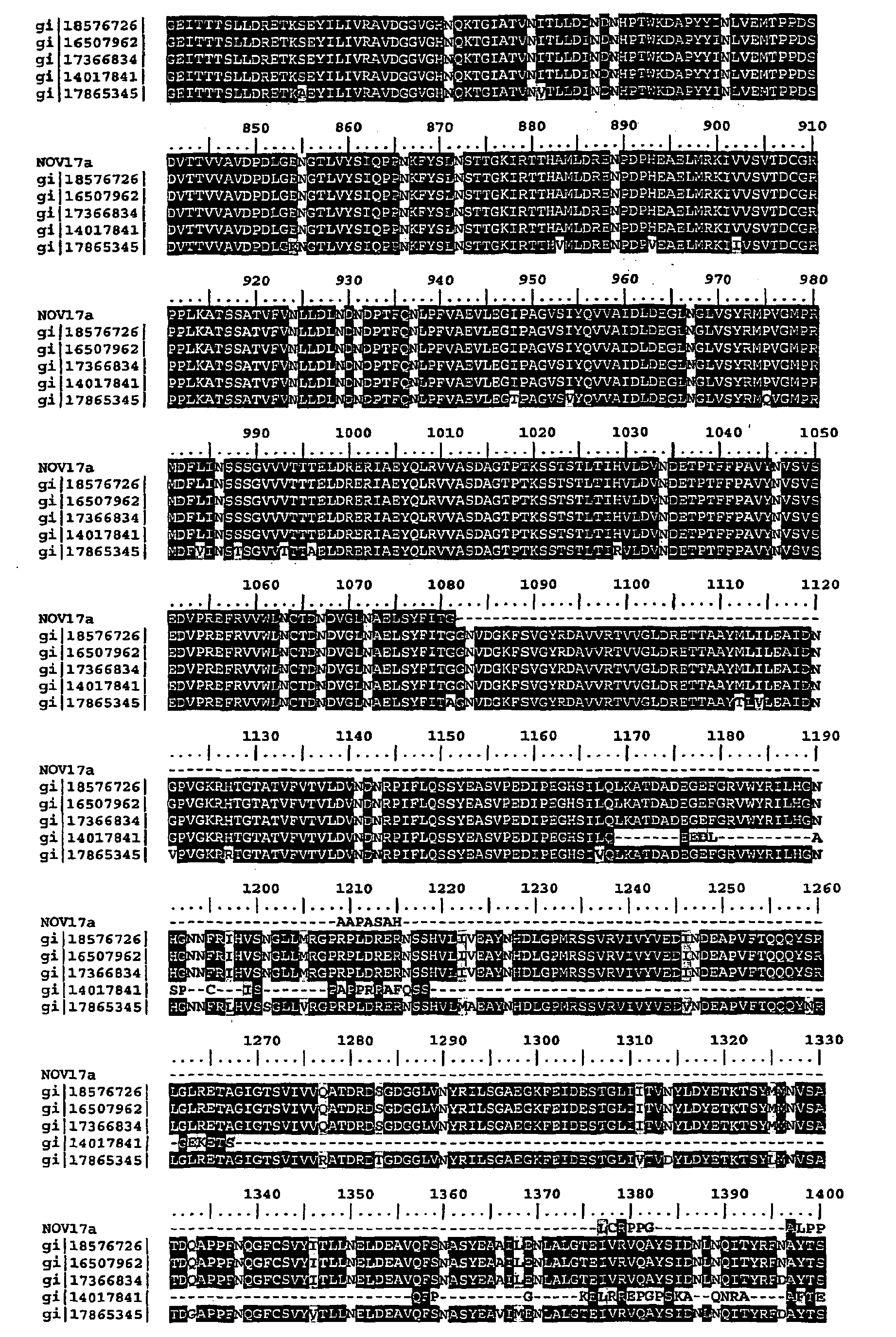

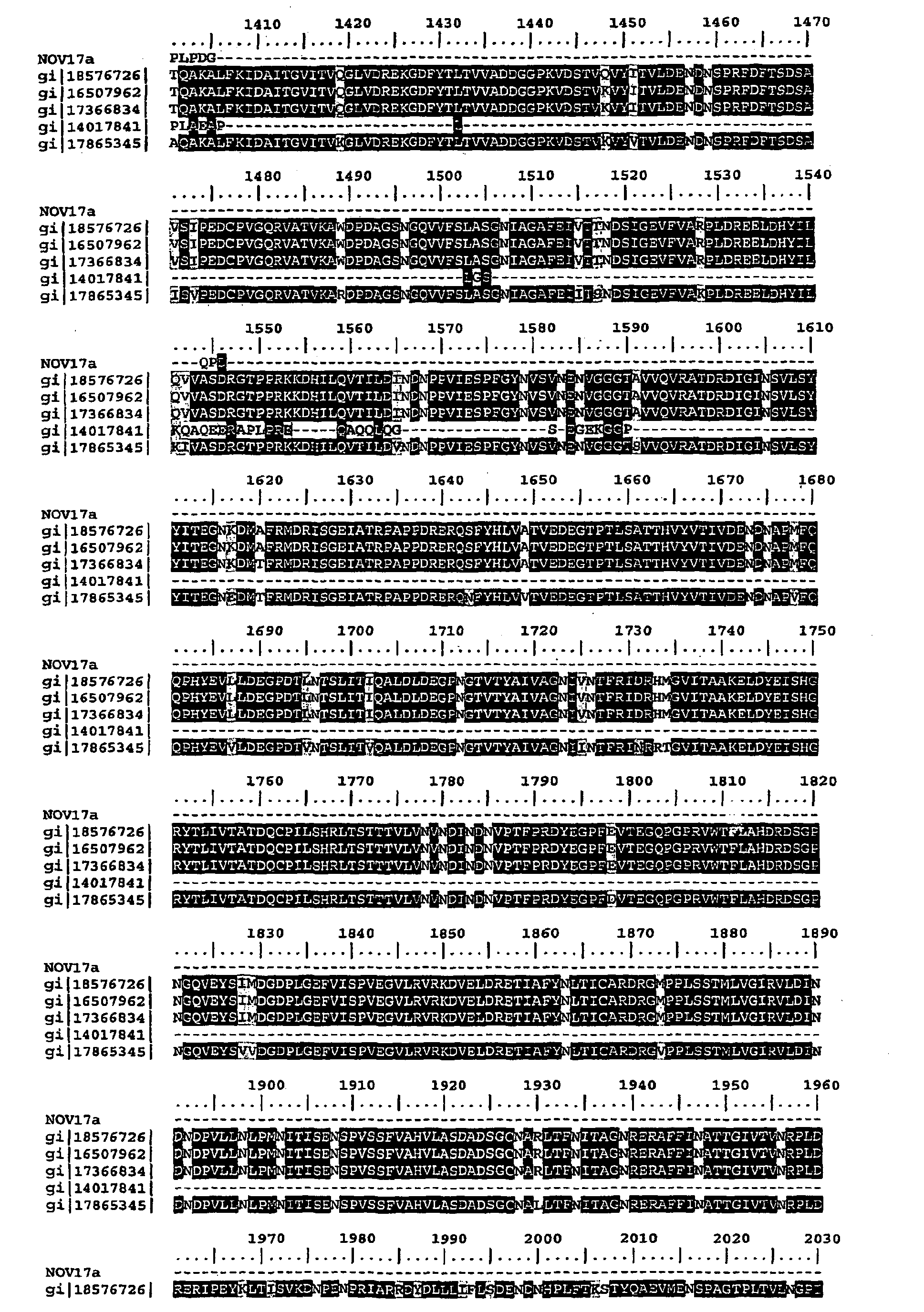

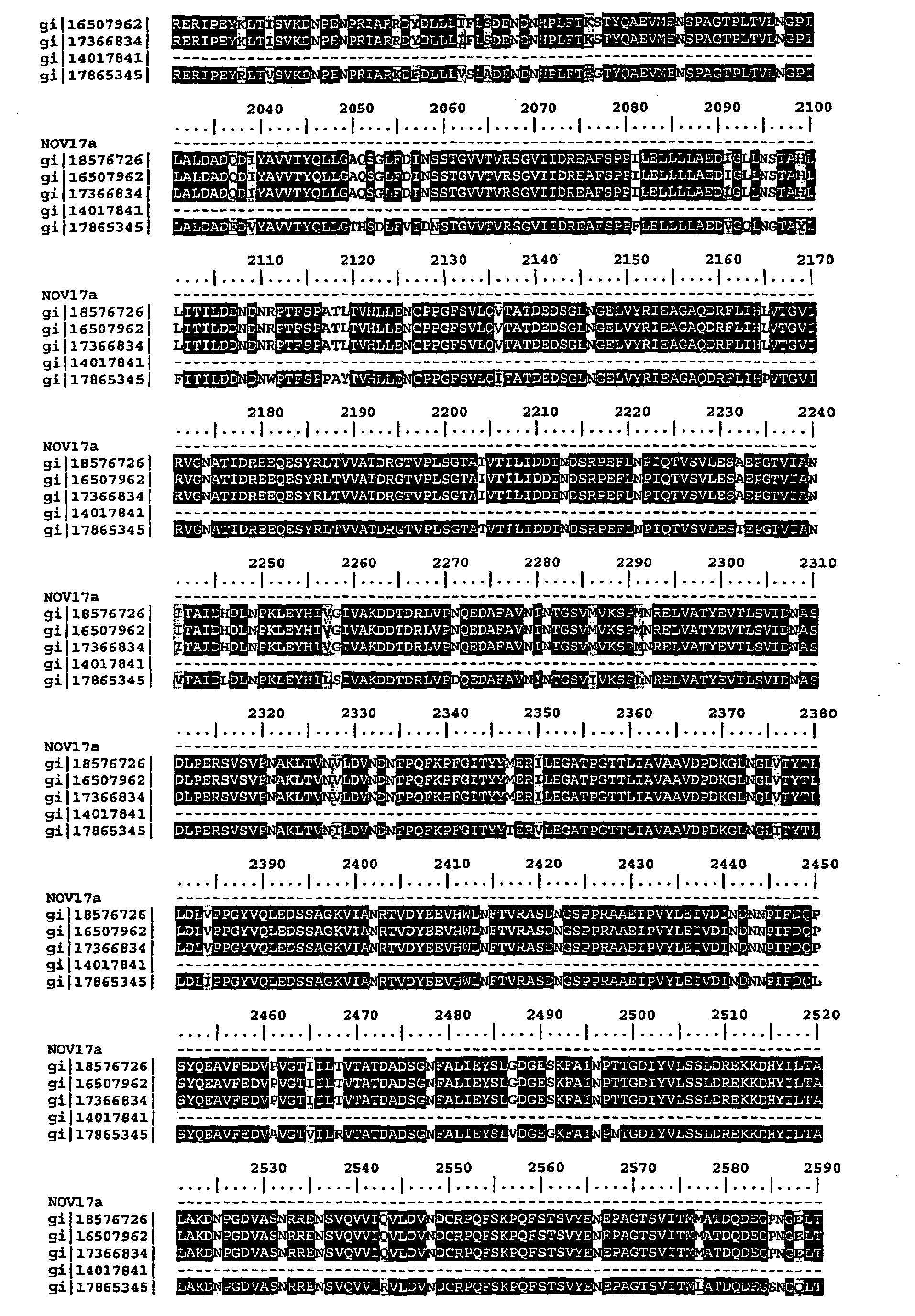

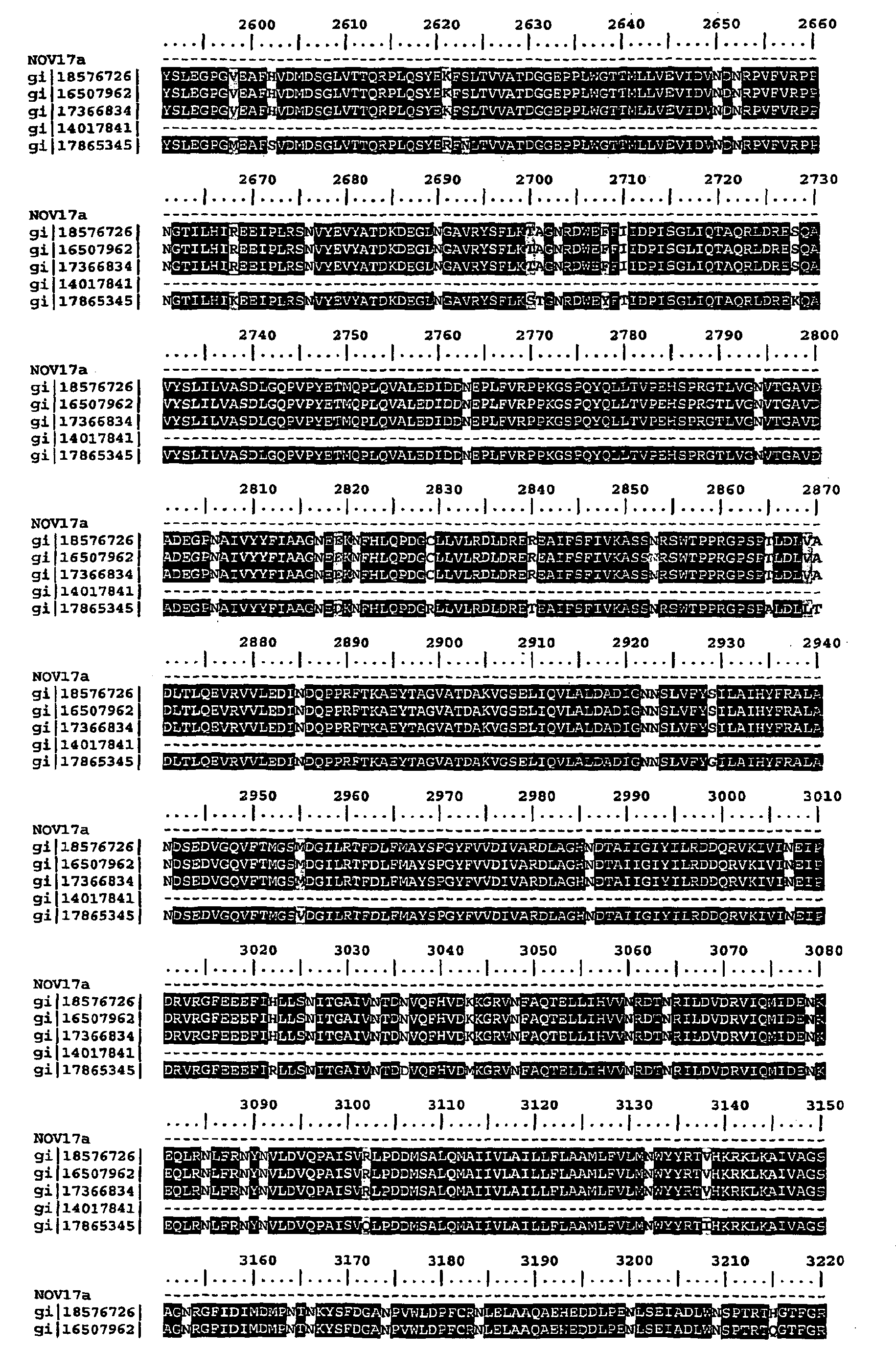

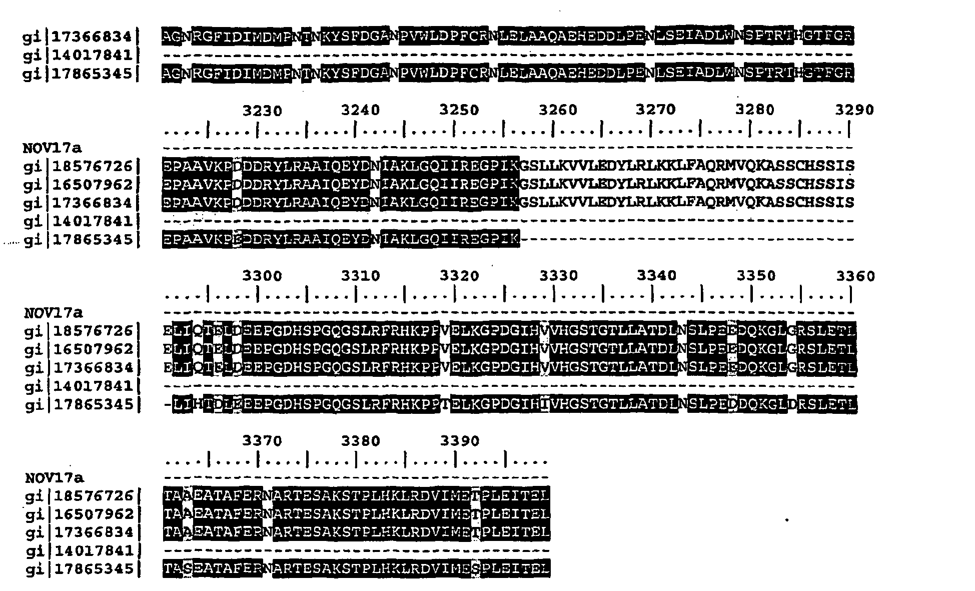

Tables 17I and 17J list the domain description from DOMAIN analysis results against NOV17a. This indicates that the NOV17a sequence has properties similar to those of other proteins known to contain these domains.

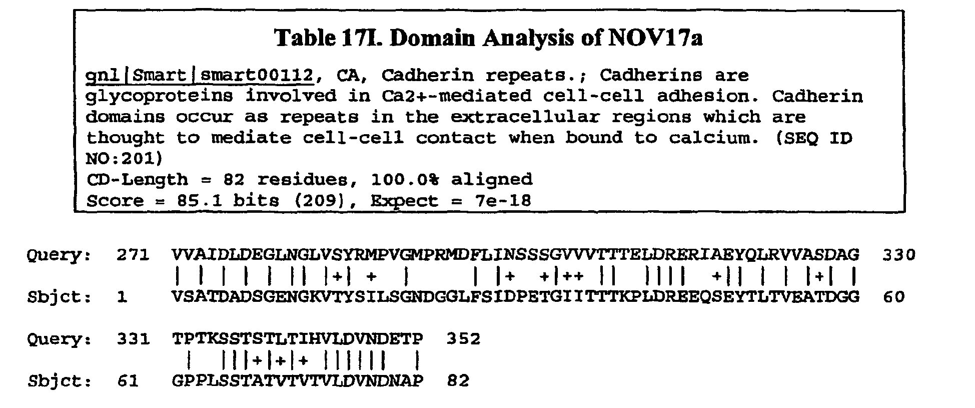

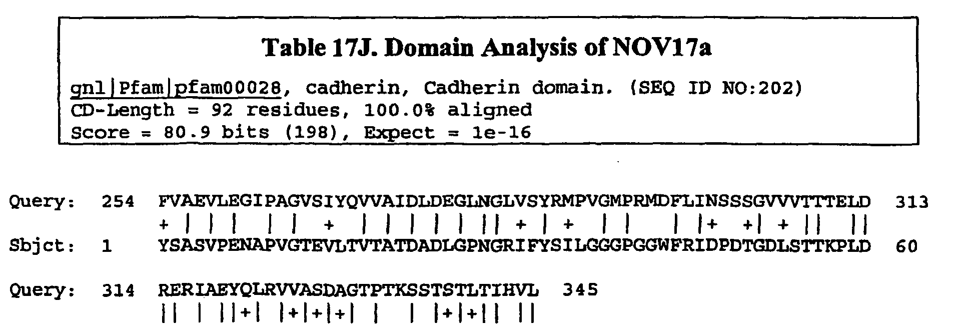

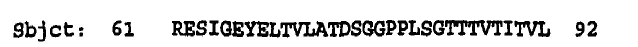

Cadherins are a family of animal glycoproteins responsible for calcium-dependent cell-cell adhesion. Cadherins preferentially interact with themselves in a homophilic manner in connecting cells; thus acting as both receptor and ligand. A wide number of tissue-specific forms of cadherins are known, including epithelial (E-cadherin, also known as uvomorulin or L-CAM; CDH1), neural (N-cadherin; CDH2), placental (P-cadherin; CDH3), retinal (R-cadherin; CDH4), vascular endothelial (VE-cadherin; CDH5), kidney (K-cadherin; CDH6), cadherin-8 (CDH8), osteoblast (OB-cadherin; CDH11), brain (BR-cadherin; CDH12), T-cadherin (truncated cadherin; CDH13), muscle (M-cadherin; CDH14), liver-intestine (LI-cadherin), and EP-cadherin.

Structurally, cadherins are built of the following domains: a signal sequence, followed by a propeptide of about 130 residues, then an extracellular domain of around 600 residues, then a transmembrane region, and finally a C-terminal cytoplasmic domain of about 150 residues. The extracellular domain can be sub-divided into five parts: there are four repeats of about 110 residues followed by a region that contains four conserved cysteines. It is suggested that the calcium-binding region of cadherins is located in the extracellular repeats. Cadherins are evolutionary related to the desmogleins which are component of intercellular desmosome junctions involved in the interaction of plaque proteins.

A form of nonsyndromic autosomal recessive deafness is caused by mutation in the cadherin-23 gene (CDH23), which is also the site of mutation in a form of Usher syndrome, USH1D. Chaib et al. (Hum Mol Genet. 5(7):1061-4,1996) reviewed the frequency and inheritance of congenital isolated deafness and causes for difficulties associated with mapping of deafness genes. They noted that in the U.S., deafness affects 1 in 1000 children at birth or during infancy. About 75% of the inherited forms of congenital isolated deafness have an autosomal recessive mode of transmission. Chaib et al. (1996) noted that difficulties in localization of deafness genes are due to several factors, including extreme genetic heterogeneity of the defect (there are an estimated 40 deafness genes segregating in the population); the absence of clinical criteria to allow differentiation between inner ear defects; and the high proportion of marriages between deaf persons in Western countries leading to coexistence of several defective genes responsible for clinically indistinguishable phenotypes in one family. In an effort to simplify mapping of deafness genes, Chaib et al. (1996) carried out studies in consanguineous families living in isolated regions. Marker data from these families was initially analyzed using a lod-score approach. Subsequently they performed homozygosity mapping. They localized a 'new' gene for nonsyndromal recessive deafness (symbolized DFNB 12) to chromosome 10q21-q22 in a consanguineous Sunni family who lived in an isolated village in Syria. Affected members in this family have profound prelingual sensorineural hearing impairment. A significant lod score (6.40 at theta = 0.00) was obtained with the marker D10S535. Analysis of adjacent markers placed the gene distal to D10S529 and proximal to D10S532 in a 11- to 15-cM region. All affected individuals were homozygous for polymorphic markers located in this region. Chaib et al. (1996) stated that the gene encoding the mitochondrial transcription factor 6-like 2 is a possible candidate gene for DFNB12. They also noted that the homologous murine region for DFNB12 contains 3 deaf mouse mutants, including Jackson circler (jc), Waltzer (v), and Ames Waltzer (av). Bork et al. (Am J Hum Genet. 68(1):26-37,2001) demonstrated that DFNB12 and a form of Usher syndrome characterized by deafness associated with retinitis pigmentosa and vestibular dysfimction (USH1D) are allelic disorders due to different mutations in the cadherin-23 gene.

Usher syndrome type I is an autosomal recessive disorder characterized by profound congenital hearing impairment with unintelligible speech, early retinitis pigmentosa, and constant vestibular dysfunction. Three different loci had been found by linkage analysis: USH1A on 14q34, USH1B on 11q13, and USH1C on 11p15. Gerber et al. (J Med Genet. 33(1):77-9, 1996) suggested the existence of yet a fourth form of Usher syndrome type I from the fact that the 3 previously reported loci were excluded by linkage studies in 2 families of Moroccan and Pakistani ancestry. Wayne et al. (Hum Mol Genet. 5(10):1689-92, 1996) reported that a first-cousin union in a family of Pakistani origin produced 4 children with clinical signs of Usher syndrome, including profound prelingual auditory impairment of sensorineural type, congenital vestibular dysfunction, and progressive pigmentary retinopathy. Wayne et al. (1996) prepared 2 genomic DNA pools, one from the affected children and the other from the parents, and screened 161 polymorphic markers evenly spaced across the autosomal genome. The only region showing homozygosity by descent in the affected sibs was a 15-cM interval on chromosome 10 bounded by D10S529 and D10S573. Wayne et al. (1996) concluded that this was the location of the gene responsible for Usher syndrome in this family. They symbolized the locus USH1D. Bolz et al. (Nat Genet. 27(1):108-12, 2001) identified mutations in the CDH23 gene in a Cuban family and a German patient with USH1D. Di Palma et al. (Nat Genet. 27(1):103-7, 2001; Gene. 281(1-2):31-41, 2001) demonstrated that mutations in the mouse Cdh23 gene are responsible for the 'waltzer' mutation, thus establishing it as a model for USH1D. Bork et al. (2001) showed that USH1D and DFNB12 are allelic disorders due to different mutations in the cadherin-23 gene.

Truncated or alternatively spliced cadherins have been identified, and some may play roles in disease. Berx et al. (Hum Mutat. 12(4):226-37, 1998) found reports of 69 somatic mutations of the CDH1 gene. These comprised, in addition to a few missense mutations, mainly splice site mutations and truncation mutations caused by insertions, deletions, and nonsense mutations. There was a major difference in mutation type between diffuse gastric and infiltrative lobular breast cancers. In diffuse gastric tumors, the predominant defects were exon skippings, which caused in-frame deletions. By contrast, most mutations found in infiltrating lobular breast cancers were out-of-frame mutations, which were predicted to yield secreted truncated E-cadherin fragments. Two different forms of human OB-cadherin cDNA were cloned; one was a counterpart of the mouse gene and the other encoded a protein with a truncated cytoplasmic domain. Sequence analysis demonstrated that Ksp-cadherin, like LI-cadherin (CDH17), lacks the prosequence and tripeptide HAV adhesion recognition sequence typical of most classical cadherins, and possesses a truncated cytoplasmic domain.

The NOV 17 nucleic acid of the invention encoding a Cadherin 23-like protein includes the nucleic acid whose sequence is provided in Tables 17A and 17C, or a fragment thereof. The invention also includes a mutant or variant nucleic acid any of whose bases may be changed from the corresponding base shown in Tables 17A and 17C while still encoding a protein that maintains its Cadherin 23-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1% of the NOV17 residues may be so changed.

The NOV17 protein of the invention includes the Cadherin 23-like protein whose sequence is provided in Tables 17B and 17D. The invention also includes a mutant or variant protein any of whose residues may be changed from the corresponding residue shown in Tables 17B and 17D while still encoding a protein that maintains its Cadherin 23-like activities and physiological functions, or a functional fragment thereof. In the mutant or variant protein, up to about 1% of the NOV17 bases may be so changed.

The NOV17 nucleic acids and proteins of the invention are useful in potential diagnostic and therapeutic applications implicated in various diseases and disorders described below and/or other pathologies. For example, the compositions of the present invention will have efficacy for treatment of patients suffering from: nonsyndromic autosomal recessive deafness, Usher syndrome type I, systemic lupus erythematosus, autoimmune disease, asthma, emphysema, scleroderma, allergy, ARDS, diabetes, Von Hippel-Lindau (VHL) syndrome, pancreatitis, obesity, multiple sclerosis, leukodystrophies, pain, neuroprotection, fertility and other diseases, disorders and conditions of the like.

NOV17 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV17 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV18

A disclosed NOV18 nucleic acid of 1614 nucleotides (also referred to as CG55887-02) encoding a novel Transforming Growth Factor Beta 2 (TGF Beta 2)-like protein is shown in Table 18A. An open reading frame was identified beginning with an ATG initiation codon at nucleotides 182-184 and ending with a TAA codon at nucleotides 1343-1345. Putative untranslated regions upstream from the intitation codon and downstream from the termination codon are underlined in Table 18A, and the start and stop codons are in bold letters.

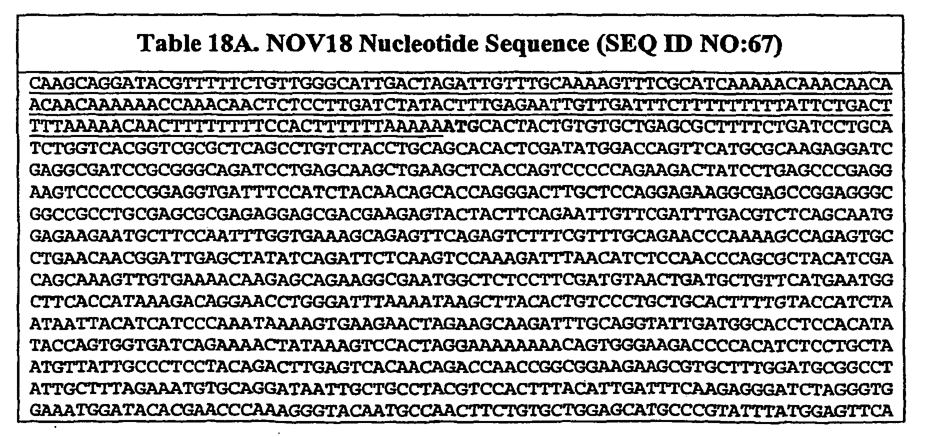

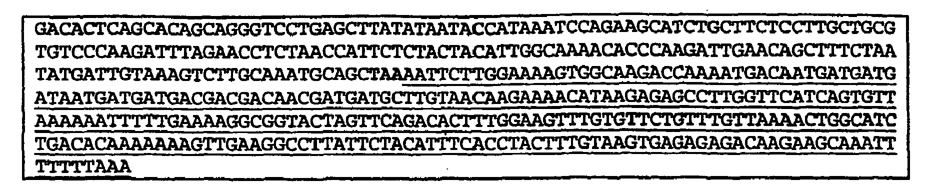

The NOV18 nucleic acid was identified on chromosome 1 and has 1140 of 1140 bases (100%) identical to a gb:GENBANK-ID:HSGTSF|acc:Y00083.1 mRNA from *Homo sapiens* (Human mRNA for glioblastoma-derived T-cell suppressor factor G-TsF (transforming growth factor-beta2, TGF-beta2)) (E = 0.0).

A disclosed NOV18 polypeptide (SEQ ID NO:68) encoded by SEQ ID NO:67 is 387 amino acid residues and is presented using the one-letter code in Table 18B. Signal P, Psort and/or Hydropathy results predict that NOV18 contains a signal peptide and is likely to be localized to the extracellularly with a certainty of 0.8200. The most likely cleavage site for a NOV18 polypeptide is between amino acids 20 and 21: ALS-LS.

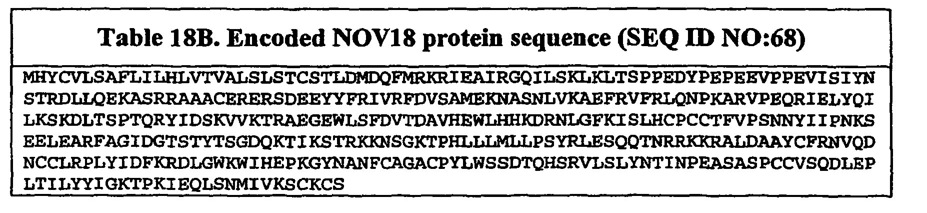

The NOV 18 amino acid sequence 289 of 289 amino acid residues (100%) identical to, and 289 of 289 amino acid residues (100%) similar to, the 414 amino acid residue ptnr:SWISSPROT-ACC:P08112 protein from *Homo sapiens* (Human), and (Transforming Growth Factor Beta 2 Precursor (TGF-BETA 2) (Glioblastoma -Derived T-Cell Suppressor Factor) (G-TSF) (BSC-1 Cell Growth Inhibitor) (Polyergin) (Cetermin)) (E =1.2e⁻²¹⁰).

NOV18 is expressed in at least the following tissues: Lung, Mammary gland/Breast, Placenta, Thymus, Uterus, Whole OrganismAorta, Brain, Cervix, Foreskin, Heart, Kidney, Prostate, Retina, Right Cerebellum and Spinal Chord. This information was derived by determining the tissue sources of the sequences that were included in the invention including but not limited to SeqCalling sources, Public EST sources, genomic clone sources, literature sources, and/or RACE sources.

Possible SNPs found for NOV18 are listed in Table 18C.

| **Table 18C: SNPs** | | | | |
|---|---|---|---|---|
| **Variant** | **Nucleotide Position** | **Base Change** | **Amino Acid Position** | **Base Change** |
| 13377104 | 785 | T > C | 202 | Cys > Arg |

NOV18 has homology to the amino acid sequences shown in the BLASTP data listed in Table 18D.

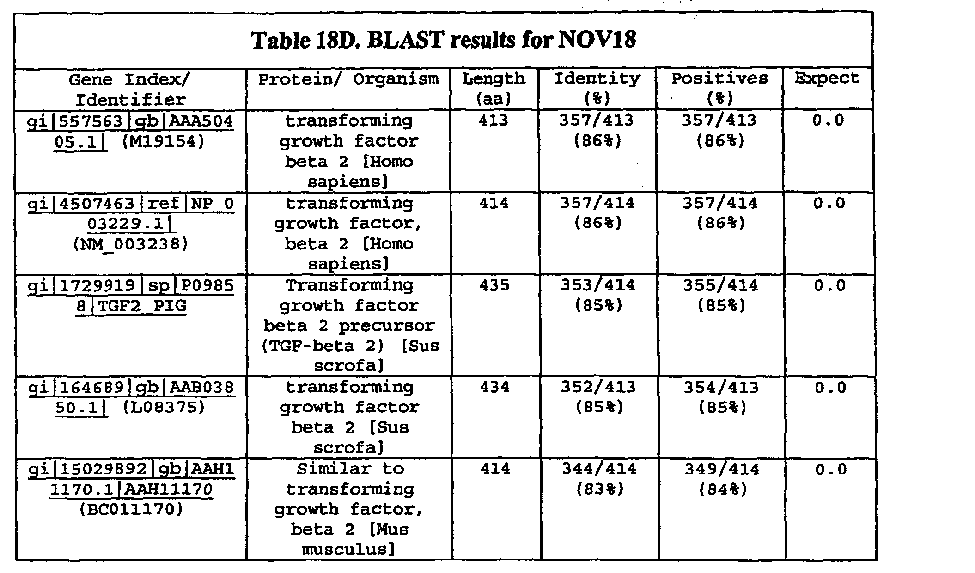

The homology of these sequences is shown graphically in the ClustalW analysis shown in Table 18E.

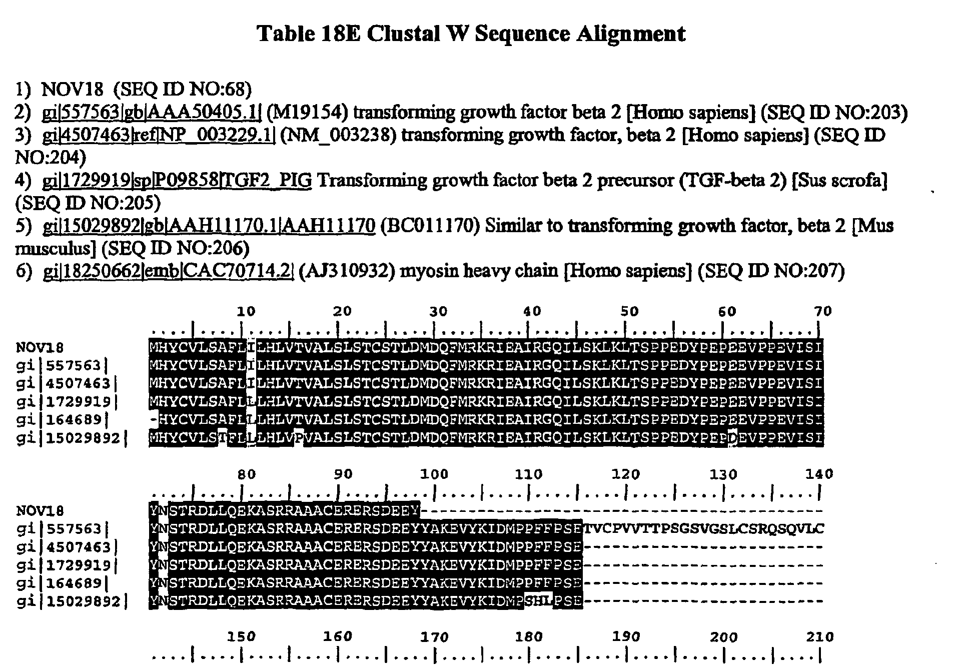

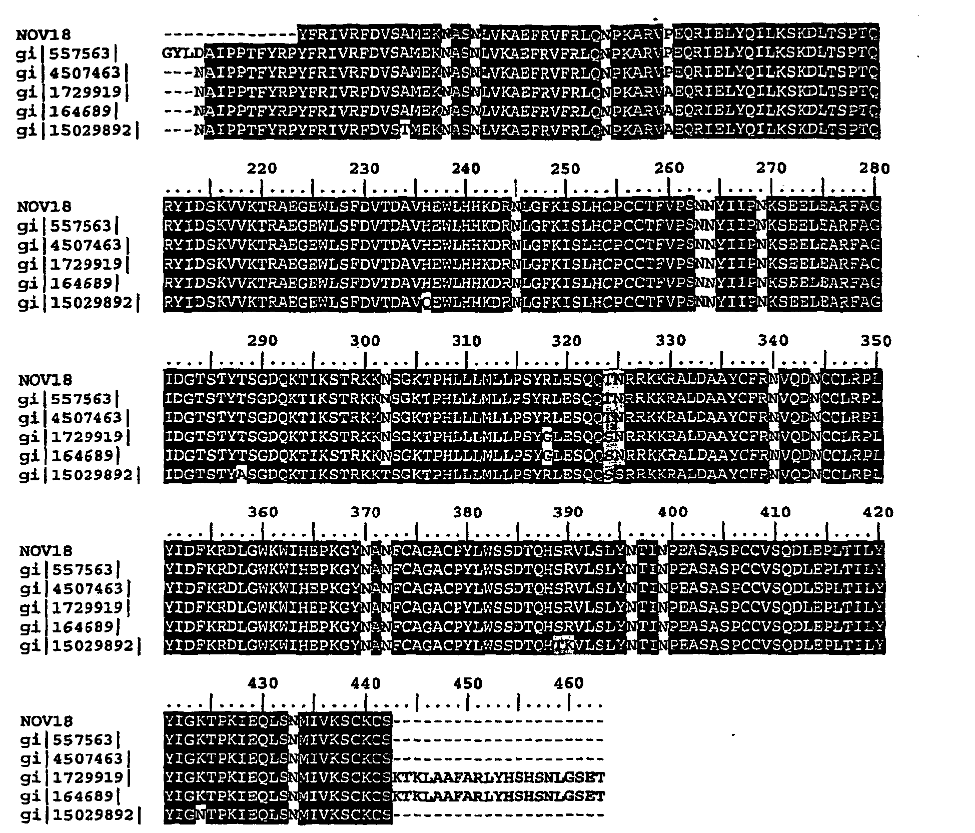

Tables 18F, 18G and 18H list the domain description from DOMAIN analysis results against NOV18. This indicates that the NOV18 sequence has properties similar to those of other proteins known to contain these domains.

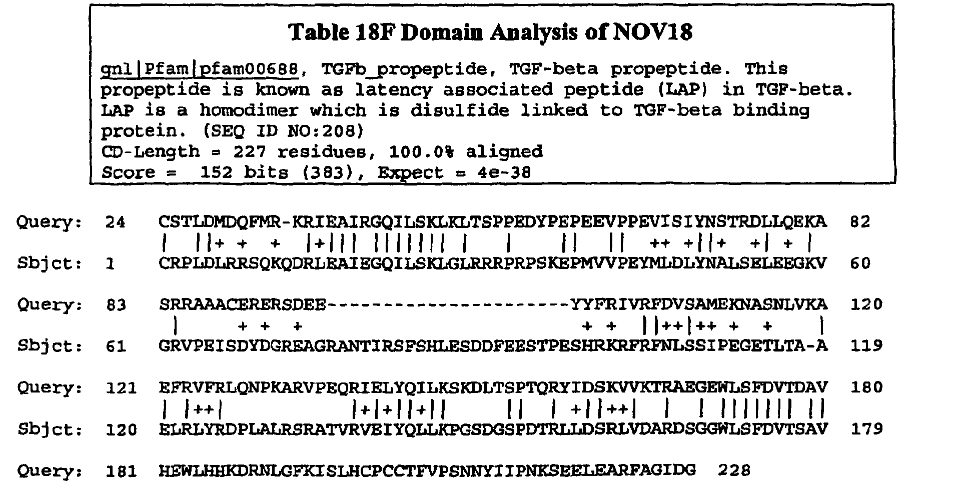

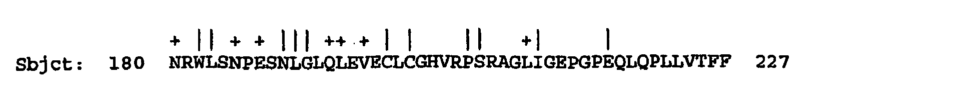

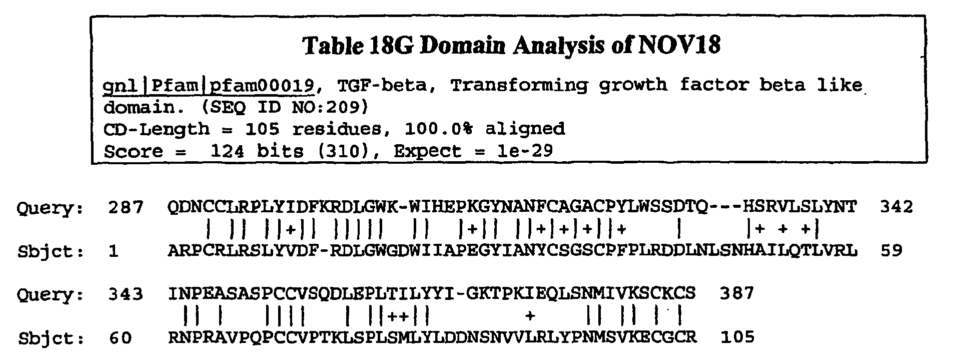

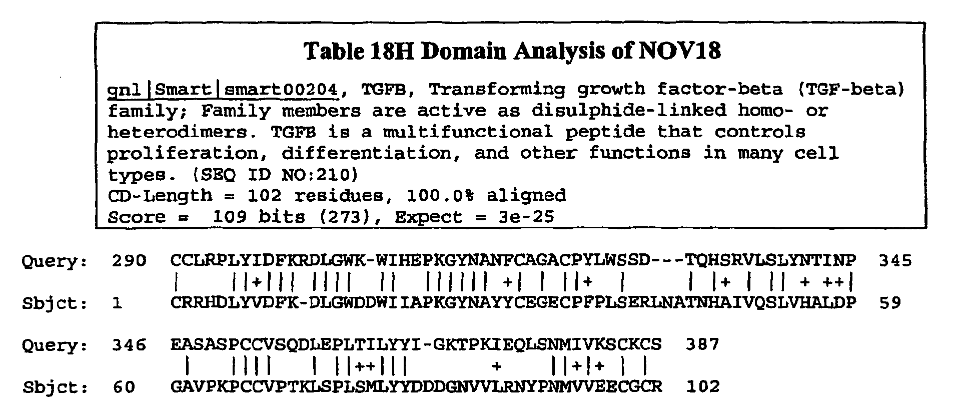

The human (de Martin et al., EMBO J. 6(6):1633-6, 1987; de Martin et al., EMBO J. 6(12):3673-7, 1987; Madisen et al., DNA. 7(1):1-8, 1988) and simian (Hanks et al., Proc Natl Acad Sci U S A. 85(1):79-82, 1988) forms of TGF-beta-2 have identical C-terminal 112 amino acid residues. The beta-2 form was derived from human glioblastoma cells. It has suppressive effects on interleukin-2-dependent T-cell growth. For these reasons it is also called glioblastoma-derived T-cell suppressor factor (G-TSF). It may have an autocrine function in enhancing tumor growth and/or reducing immunosurveillance of tumor development. By Southern blot analysis of somatic cell hybrid lines and, for the human locus, also by in situ chromosomal hybridization, Barton et al. (Oncogene Res. 3(4):323-31, 1988) mapped TGFB2 to 1q41 in the human and to chromosome 1 in the mouse, most likely in the known conserved syntenic region. Dickinson et al. (Genomics. 6(3):505-20, 1990) also assigned the mouse Tgfb-2 gene to chromosome 1. Nishimura et al. (Genomics. 15(2):357-64, 1993) identified 4 RFLPs and SSCPs (single strand conformation polymorphisms) for TGFB2 in humans and gorillas. Using these, they localized the gene within a framework map of distal 1q and showed close linkage to homeo box gene HLX1 (142995); maximum lod score of 14.49 at theta = 0.031.

The NOV18 nucleic acid of the invention encoding a Transforming Growth Factor Beta 2-like protein includes the nucleic acid whose sequence is provided in Table 18A, or a fragment thereof. The invention also includes a mutant or variant nucleic acid any of whose bases may be changed from the corresponding base shown in Table 18A while still encoding a protein that maintains its Transforming Growth Factor Beta 2-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

The NOV18 protein of the invention includes the Transforming Growth Factor Beta 2-like protein whose sequence is provided in Table 18B. The invention also includes a mutant or variant protein any of whose residues may be changed from the corresponding residue shown in Table 18B while still encoding a protein that maintains its Transforming Growth Factor Beta 2-like activities and physiological functions, or a functional fragment thereof.

The NOV18 nucleic acids and proteins of the invention are useful in potential diagnostic and therapeutic applications implicated in various diseases and disorders described below and/or other pathologies. For example, the compositions of the present invention will have efficacy for treatment of patients suffering from: cancer and other diseases, disorders and conditions of the like.

NOV 18 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV 18 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV19

A disclosed NOV19 nucleic acid of 2939 nucleotides (also referred to as CG57333-01) encoding a novel Ebnerin-like protein is shown in Table 19A. An open reading frame was identified beginning with an ATG initiation codon at nucleotides 20-22 and ending with a TAG codon at nucleotides 2912-2914. Putative untranslated regions upstream from the intitation codon and downstream from the termination codon are underlined in Table 19A, and the start and stop codons are in bold letters.

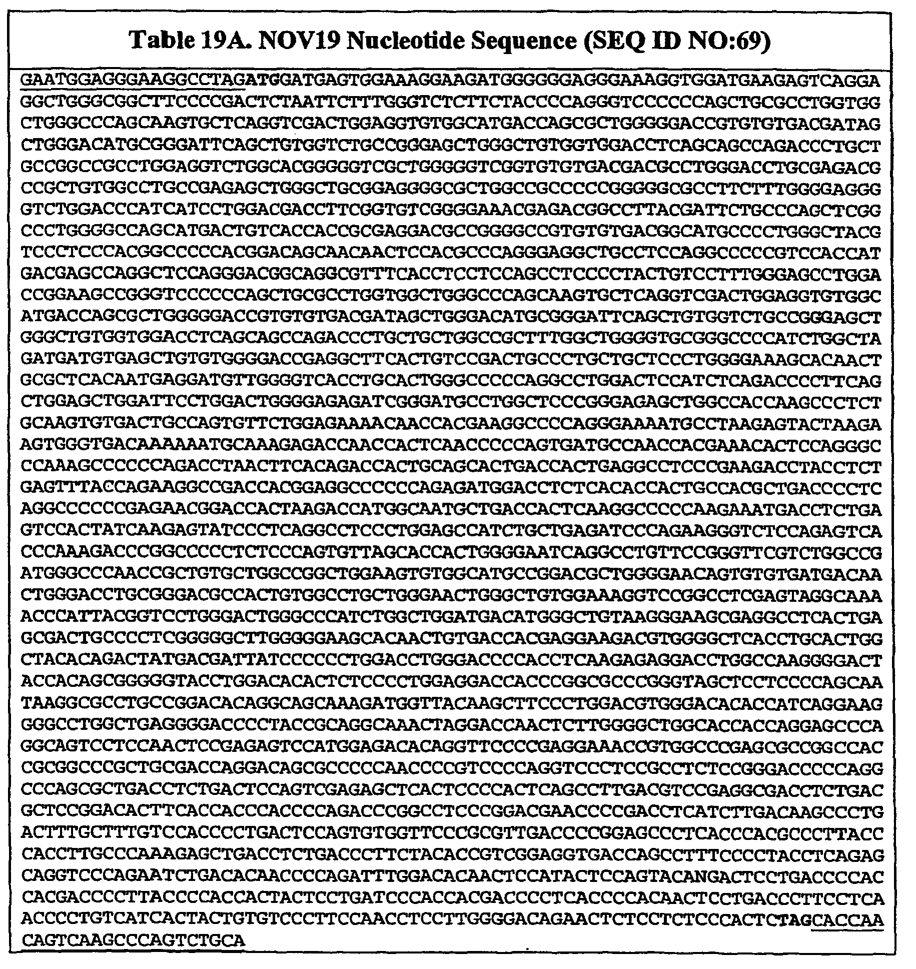

The NOV19 nucleic acid and has 313 of 490 bases (63%) identical to a gb:GENBANK-ID:HSA243224|acc:AJ243224.1 mRNA from *Homo sapiens (Homo sapiens* mRNA for DMBT1 protein 8kb transcript variant 1 (DMBT1/8kb.1)) (E = 7.0e⁻⁴⁶).

A disclosed NOV19 polypeptide (SEQ ID NO:70) encoded by SEQ ID NO:69 is 964 amino acid residues and is presented using the one-letter code in Table 19B. Signal P, Psort and/or Hydropathy results predict that NOV19 does not contain a signal peptide and is likely to be localized to the cytoplasm with a certainty of 0.4500.

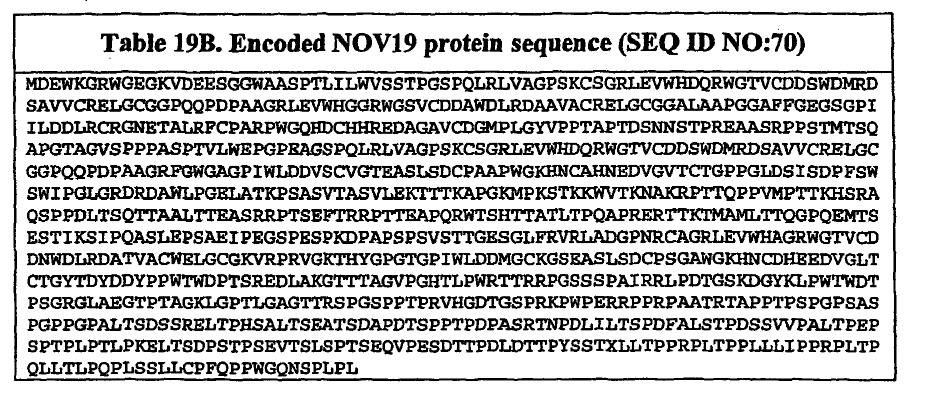

The NOV19 amino acid sequence has 57 of 109 amino acid residues (52%) identical to, and 75 of 109 amino acid residues (68%) similar to, the 1594 amino acid residue ptnr:SPTREMBL-ACC:Q95218 protein from *Oryctolagus cuniculus* (Rabbit) (HENSIN) (E = 2.2e⁻⁴⁰).

NOV19 is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, genomic clone sources, literature sources, and/or RACE sources.

NOV19 has homology to the amino acid sequences shown in the BLASTP data listed in Table 19C.

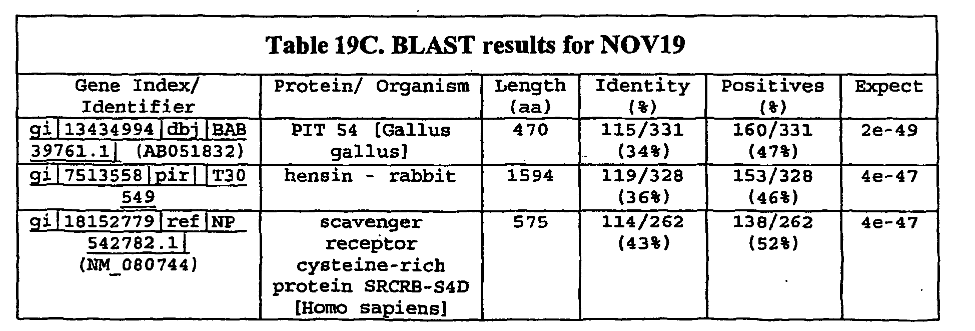

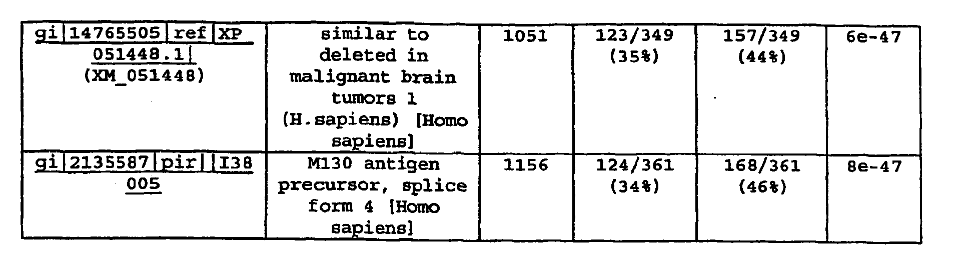

The homology of these sequences is shown graphically in the ClustalW analysis shown in Table 19D.

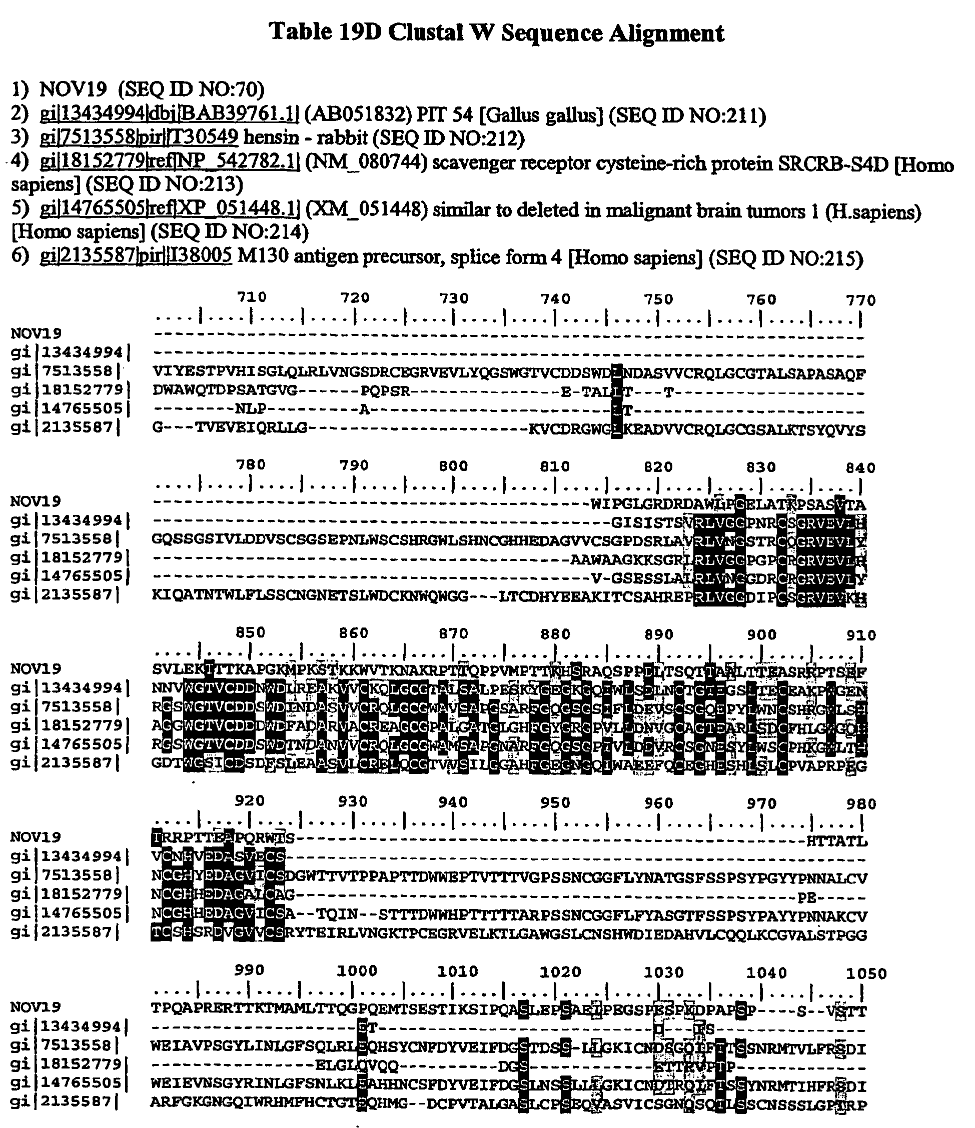

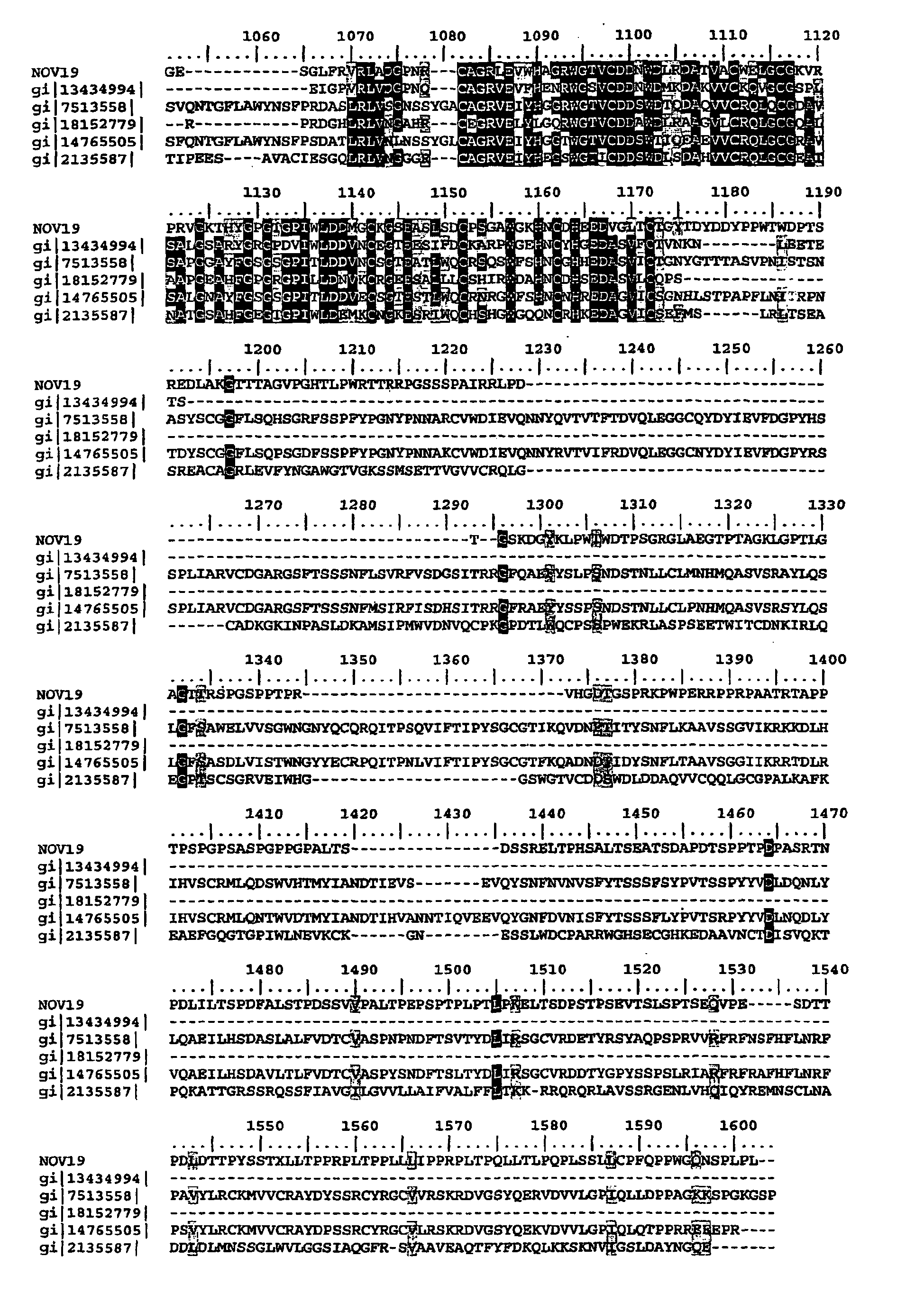

Tables 19E and 19F list the domain description from DOMAIN analysis results against NOV19. This indicates that the NOV19 sequence has properties similar to those of other proteins known to contain these domains.

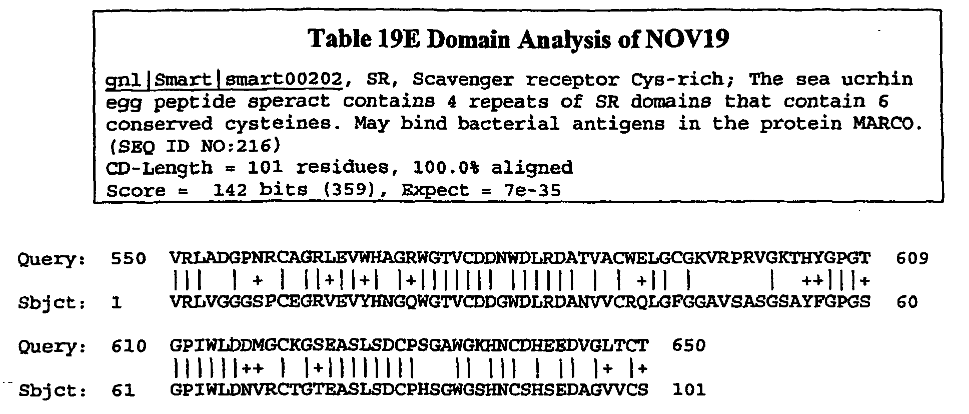

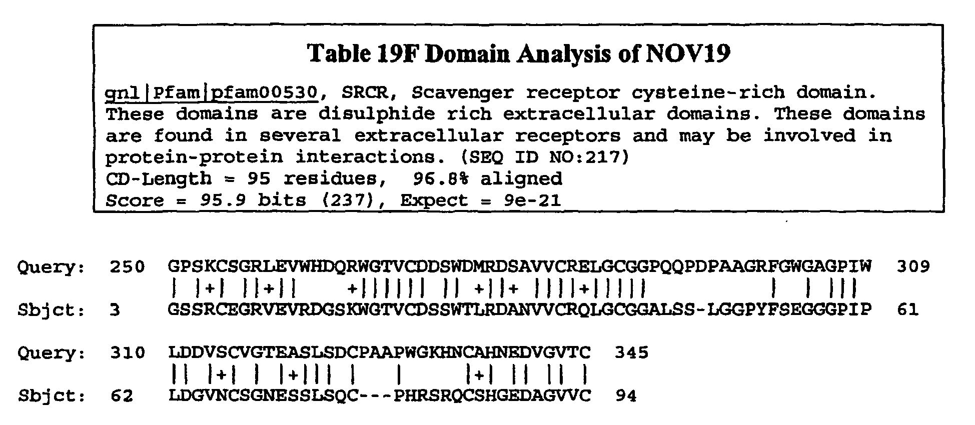

Saliva is the first digestive fluid secreted by the gastrointestinal pathway and performs a variety of functions. It is essential in the formation of small boluses of food, provides lubrication for swallowing and speech, dissolves a number of chemicals in food substances, and provides digestive enzymes such as amylase and lipase (Hamosh & Scow, 1973, J. Clin. Invest.52:88-95; Field & Hand, 1987, Am. J. Physio1.253:G217-G225). About 90% of saliva is produced by three major glands, the parotid, the submaxillary, and the sublingual glands, whose secretions drain into the oral cavity.

von Ebner's glands are unique salivary glands contained within the tongue that drain directly into the clefts of the circumvallate and foliate papillae, which contain the major taste buds. Secretions of von Ebner's gland directly modulate taste perception (Gurkan & Bradley, 1988, Chem. Senses 13: 655-661). Xiao-Jiang Li and Solomon H. Snyder (1995, J Biol Chem 270 :17674-9) have identified a novel von Ebner's gland (VEG) protein, designated Ebnerin, which is formed in the ducts of von Ebner's gland and released into fluid bathing the taste buds contained in the taste papillae. Ebnerin possesses a putative single transmembrane domain at the C terminus with 17 amino acids in the cytoplasmic area. The extracellular region of Ebnerin contains a number of repeated domains with homology to the scavenger receptor cysteine-rich domain and to a repeated domain of bone morphogenetic protein-I and other related proteins. Western blot analysis reveals that Ebnerin exists in particulate and soluble forms in VEG and is present in secretions from VEG. The unique structure and localization of Ebnerin suggest that it may function as a binding protein in saliva for the regulation of taste sensation.

Ebnerin is a modular protein containing a signal peptide, SRCR ("scavenger receptor, cysteine rich") domains. Other proteins known to have this domain include CRP-ductin, a cDNA expressed at high levels in mouse intestine (8 SRCR), hensin, the polarity reversal protein cloned from a rabbit kidney (8SRCR), and DMBT1, a sequence in human chromosome 10q25-26 frequently deleted in malignant gliomas (9 SRCR) (Cheng, et al., 1996, Anat. Rec. 244: 327-343; Mollenhauer, 1997, Nat. Genet. 17: 32-39; Takito et al., 1999, Am J Physiol 277:F277-89). Hensin, DMBT1, CRP-ductin, and ebnerin are alternately spliced products from a single gene located on human chromosome 10q25-26, a region often deleted in several cancers, especially malignant gliomas. Hensin is expressed in many epithelial cell types, and it plays a critical role in terminal differentiation of the intercalated cell and perhaps other epithelia (Al-Awqati et al., 2000, Exp Nephrol 8:66-71).

Vomeroglandin, a subform of mouse CRP-ductin, is a protein strongly expressed in the glands of mouse vomeronasal system. Both the proteins contain several of scavenger receptor cysteine-rich and CUB domains and one ZP domain. This domain arrangement is similar to those of rat Ebnerin, human DMBT1, and rabbit hensin. In situ hybridization analysis shows strong expression of vomeroglandin mRNA in the glands of vomeronasal system. Immunological analyses detect both membrane-bound and secreted forms of vomeroglandin. The secreted protein seems to be localized in the lumen of the vomeronasal organ, playing a certain role in the pheromone perception (Matsushita et al., 2000, Biochem Biophys Res Commun 268:275-81).

The NOV19 nucleic acid of the invention encoding a Ebnerin-like protein includes the nucleic acid whose sequence is provided in Table 19A, or a fragment thereof. The invention also includes a mutant or variant nucleic acid any of whose bases may be changed from the corresponding base shown in Table 19A while still encoding a protein that maintains its Ebnerin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 37% of the residues may be so changed.

The NOV19 protein of the invention includes the Ebnerin-like protein whose sequence is provided in Table 19B. The invention also includes a mutant or variant protein any of whose residues may be changed from the corresponding residue shown in Table 19B while still encoding a protein that maintains its Ebnerin-like activities and physiological functions, or a functional fragment thereof. In the mutant or variant protein, up to about 48% of the bases may be so changed.

The NOV 19 nucleic acids and proteins of the invention are useful in potential diagnostic and therapeutic applications implicated in various diseases and disorders described below and/or other pathologies. For example, the compositions of the present invention will have efficacy for treatment of patients suffering from: several cancers, especially malignant gliomas, glioblastoma multiforme, medulloblastoma and other diseases, disorders and conditions of the like.

NOV19 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV 19 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV20

NOV20 includes two novel Fatty Acid Binding-like proteins disclosed below. The disclosed proteins have been named NOV20a and NOV20b.

### NOV20a

A disclosed NOV20a nucleic acid of 400 nucleotides (also referred to as CG57556-01) encoding a novel Fatty Acid Binding-like protein is shown in Table 20A. An open reading frame was identified beginning with an ATG initiation codon at nucleotides 2-4 and ending with a TAG codon at nucleotides 398-400. Putative untranslated regions, if any, upstream from the initiation codon and downstream from the termination codon are underlined in Table 20A, and the start and stop codons are in bold letters.

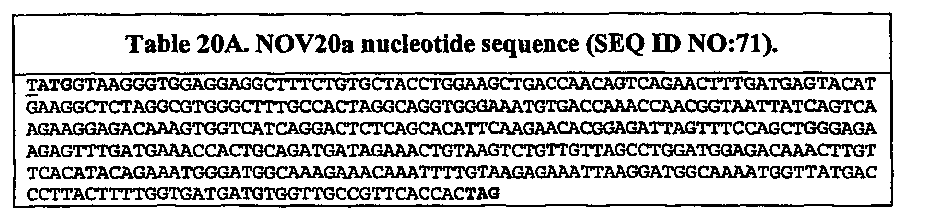

The disclosed NOV20a nucleic acid sequence, localized to chromsome 6q22-23, has 389 of 398 bases (97%) identical to a gb:GBNBANK-ID:HSAJ2962|acc:AJ002962.1 mRNA from *Homo sapiens (Homo sapiens* mRNA for hB-FABP) (E = 2.5e⁻⁷⁹).

A NOV20a polypeptide (SEQ ID NO:72) encoded by SEQ ID NO:71 has 132 amino acid residues and is presented using the one-letter code in Table 20B. Signal P, Psort and/or Hydropathy results predict that NOV20a does not contain a signal peptide and is likely to be localized to the microbody (peroxisome) with a certainty of 0.4849 and to the mitochondrial matrix space with a certainty of 0.4750. Although PSORT suggests that the NOV20a protein may be localized in the intracellularly, the NOV20a protein is similar to the Fatty Acid Binding Protein family, some members of which are localized at the plasma membrane. Therefore it is likely that this novel Fatty Acid Binding Protein -like protein is localized to the same sub-cellular compartment.

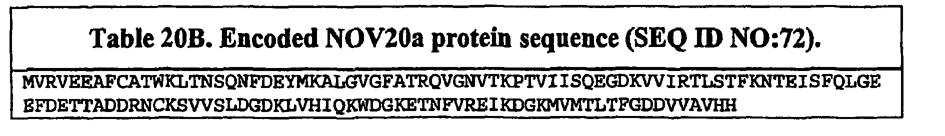

The NOV20a amino acid sequence has 125 of 127 amino acid residues (98%) identical to, and 125 of 127 amino acid residues (98%) similar to, the 131 amino acid residue ptnr:SWISSNEW-ACC:O15540 protein from *Homo sapiens* (Human) (Fatty Acid-Binding Protein, Brain (B-FABP) (Brain Lipid-Binding Protein) (BLBP) (Mammary Derived Growth Inhibitor Related)) (E = 1.9e⁻⁶²).

The disclosed NOV20a is expressed in at least the following tissues: Bone, Brain, Heart, Kidney, Lung, Retina, Temporal Lobe and Uterus. This information was derived by determining the tissue sources of the sequences that were included in the invention including but not limited to SeqCalling sources, Public EST sources, Literature sources, and/or RACE sources. The disclosed NOV20a is also predicted to be expressed in the following tissues because of the expression pattern of (gb:GENBANK-ID:HSAJ2962|acc:AJ002962.1) a closely related *Homo sapiens* mRNA for hB-FABP homolog in species *Homo sapiens:* brain.

### NOV20b

A disclosed NOV20b nucleic acid of 417 nucleotides (also referred to as CG57556-02) encoding a novel Fatty Acid Binding-like protein is shown in Table 20C. An open reading frame was identified beginning with an ATG initiation codon at nucleotides 5-7 and ending with a TAA codon at nucleotides 410-412. Putative untranslated regions, if any, upstream from the initiation codon and downstream from the termination codon are underlined in Table 20C, and the start and stop codons are in bold letters.

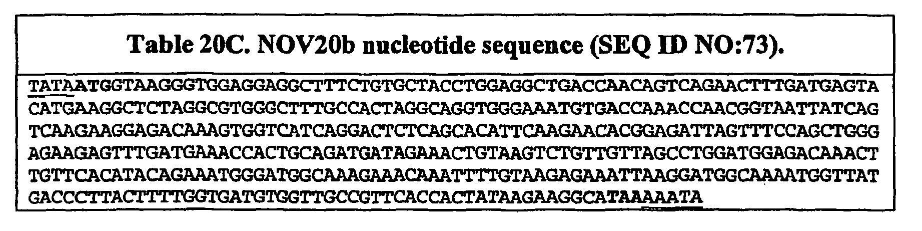

The disclosed NOV20b nucleic acid sequence, localized to chromsome 6q22-23, has 405 of 413 bases (98%) identical to a gb:GENBANK-ID:HSAJ2962|acc:AJ002962.1 mRNA from *Homo sapiens (Homo sapiens* mRNA for hB-FABP) (E = 6.6e⁻⁸⁴).

A NOV20b polypeptide (SEQ ID NO:74) encoded by SEQ ID NO:73 has 135 amino acid residues and is presented using the one-letter code in Table 20D. Signal P, Psort and/or Hydropathy results predict that NOV20b does not contain a signal peptide and is likely to be localized to the mitochondrial matrix space with a certainty of 0.5834 and to the peroxisome (microbody) with a certainty of 0.4915.

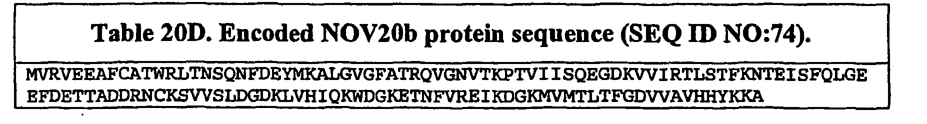

The NOV20b amino acid sequence has 127 of 130 amino acid residues (97%) identical to, and 129 of 130 amino acid residues (99%) similar to, the 131 amino acid residue ptnr:SWISSNEW-ACC:O15540 protein from *Homo sapiens* (Human) (Fatty Acid-Binding Protein, Brain (B-FABP) (Brain Lipid-Binding Protein) (BLBP) (Mammary Derived Growth Inhibitor Related)) (E = 6.8e⁻⁶⁵).

The disclosed NOV20b is expressed in at least the following tissues: Bone, Brain, Heart, Kidney, Lung, Retina, Temporal Lobe and Uterus. This information was derived by determining the tissue sources of the sequences that were included in the invention including but not limited to SeqCalling sources, Public EST sources, Literature sources, and/or RACE sources. The disclosed NOV20b is also predicted to be expressed in the following tissues because of the expression pattern of (gb:GENBANK-ID:HSAJ2962|acc:AJ002962.1) a closely related *Homo sapiens* mRNA for hB-FABP homolog in species *Homo sapiens:* brain.

Possible SNPs found for NOV20a are listed in Table 20E.

| **Table 20E: SNPs** | | | |
|---|---|---|---|
| **Consensus Position** | **Depth** | **Base Change** | **PAF** |
| 42 | 6 | A > G | 0.333 |
| 170 | 6 | A > G | 0.333 |
| 388 | 6 | C > T | 0.333 |

NOV20a and NOV20b are very closely homologous as is shown in the amino acid alignment in Table 20F.

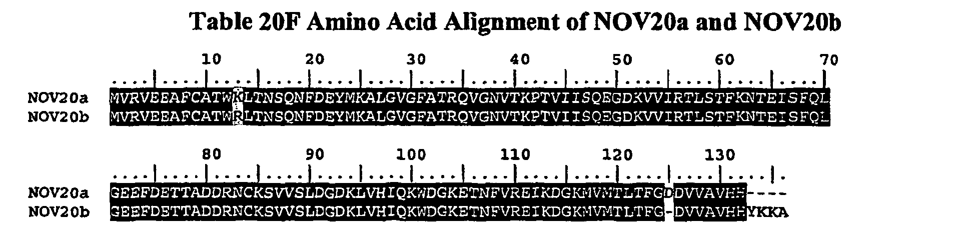

Homologies to any of the above NOV20 proteins will be shared by the other NOV20 proteins insofar as they are homologous to each other as shown above. Any reference to NOV20 is assumed to refer to the NOV20 proteins in general, unless otherwise noted.

NOV20a has homology to the amino acid sequences shown in the BLASTP data listed in Table 20G.

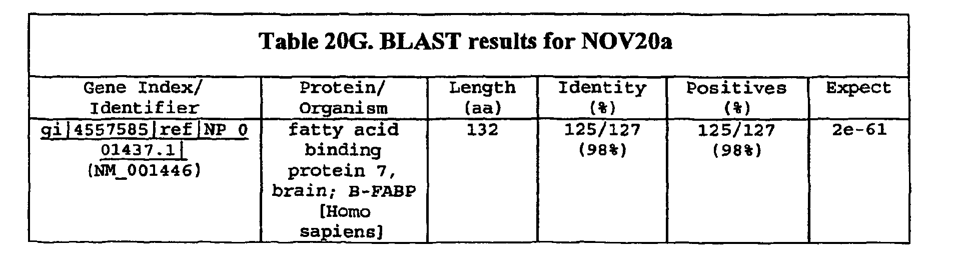

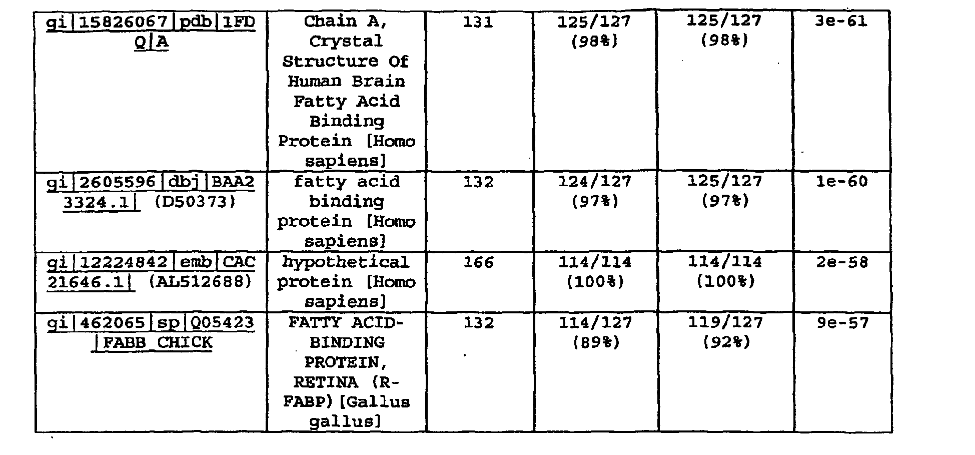

The homology of these sequences is shown graphically in the ClustalW analysis shown in Table 20H.

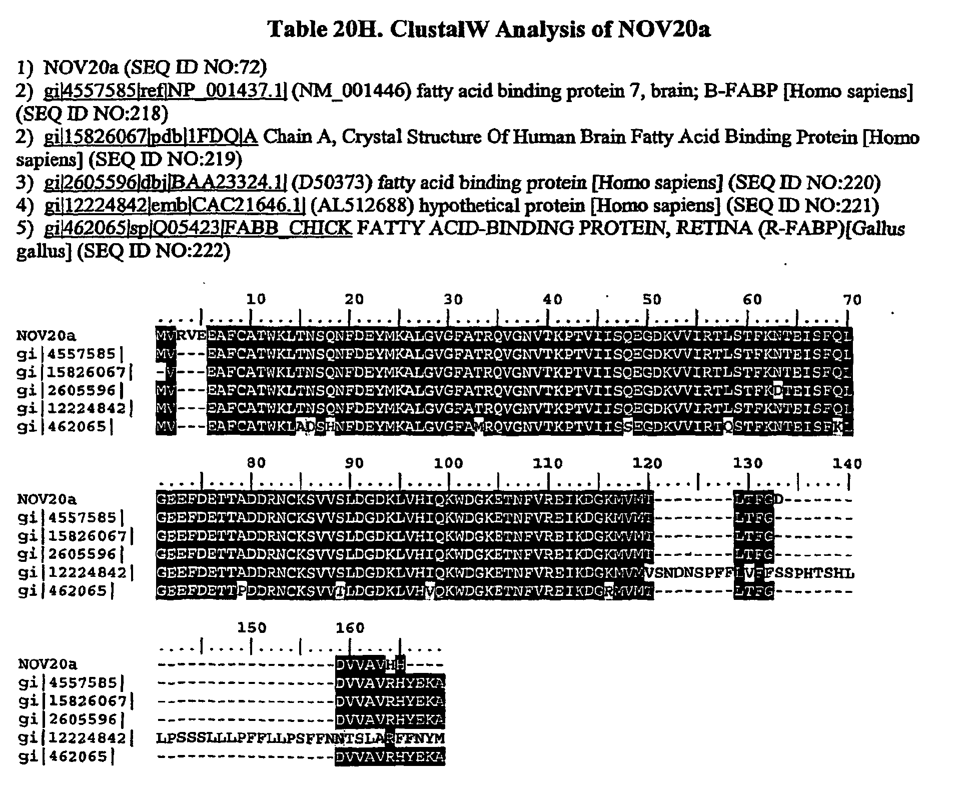

Table 20I lists the domain description from DOMAIN analysis results against NOV20a. This indicates that the NOV20a sequence has properties similar to those of other proteins known to contain these domains.

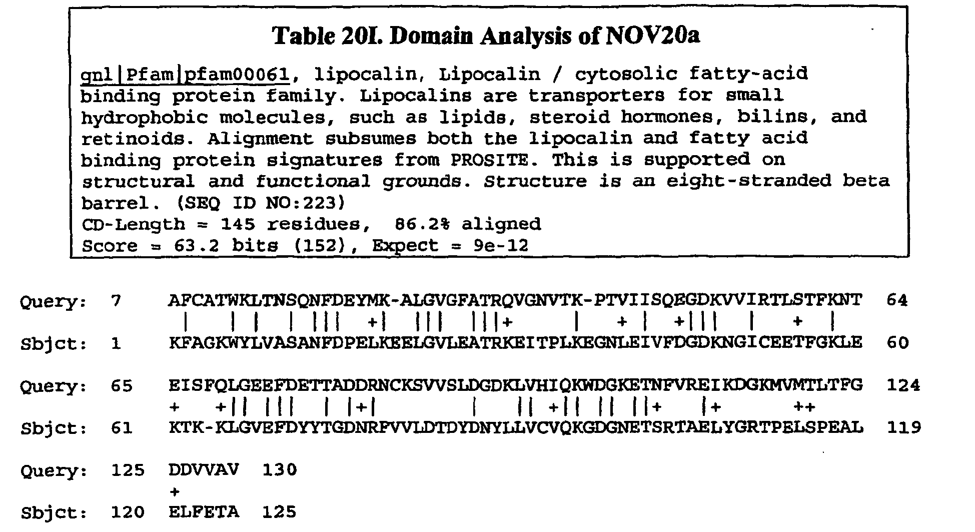

The murine brain fatty acid binding protein (B-FABP) is encoded by a developmentally regulated gene that is expressed in radial glial cells and immature astrocytes. The human B-FABP gene has been cloned and mapped to chromosome 6q22-23. B-FABP mRNA is expressed in human malignant glioma tumor biopsies and in a subset of malignant glioma cell lines, as well as in human fetal retina and brain. Malignant glioma tumors are characterized by cytoplasmic bundles of glial fibrillary acidic protein (GFAP), a protein normally expressed in mature astrocytes. Establishment of malignant glioma cell lines often results in loss of GFAP. The subset of malignant glioma cell lines that express GFAP mRNA also express B-FABP mRNA. Co-localization experiments in cell lines indicate that the same cells produce both GFAP and B-FABP. These data suggest that some malignant gliomas may be derived from astrocytic precursor cells which can express proteins that are normally produced at different developmental stages in the astrocytic differentiation pathway (Godbout et al., Oncogene. 16(15):1955-62, 1998).

The NOV20 nucleic acid of the invention encoding a Fatty Acid Binding-like protein includes the nucleic acid whose sequence is provided in Tables 20A and 20C, or a fragment thereof. The invention also includes a mutant or variant nucleic acid any of whose bases may be changed from the corresponding base shown in Tables 20A and 20C while still encoding a protein that maintains its Fatty Acid Binding-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject In the mutant or variant nucleic acids, and their complements, up to about 3% of the NOV20a residues and up to about 2% of the NOV20b residues may be so changed.

The NOV20 protein of the invention includes the Fatty Acid Binding-like protein whose sequence is provided in Tables 20B and 20D. The invention also includes a mutant or variant protein any of whose residues may be changed from the corresponding residue shown in Tables 20B and 20D while still encoding a protein that maintains its Fatty Acid Binding-like activities and physiological functions, or a functional fragment thereof. In the mutant or variant protein, up to about 2% of the NOV20a bases and up to about 3% of the NOV20b bases may be so changed.

The NOV20 nucleic acids and proteins of the invention are useful in potential diagnostic and therapeutic applications implicated in various diseases and disorders described below and/or other pathologies. For example, the compositions of the present invention will have efficacy for treatment of patients suffering from: malignant glioma tumors, Arthropathy, progressive pseudorheumatoid, of childhood; Muscular dystrophy, congenital merosin-deficient; Nephropathy, IgA type; Oculodentodigital dysplasia; Ossification of posterior longitudinal ligament of spine; Syndactyly, type III; Hepatic fibrosis susceptibility due to Schistosoma mansoni infection; Hepatic fibrosis susceptibility due to Schistosoma mansoni infection; endometriosis, fertility; Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration; cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation; diabetes, autoimmune disease, renal artery stenosis, interstitial nephritis, glomerulonephritis, polycystic kidney disease, systemic lupus erythematosus, renal tubular acidosis, IgA nephropathy, hypercalceimia, systemic lupus erythematosus, autoimmune disease, asthma, emphysema, scleroderma, allergy, ARDS and other diseases, disorders and conditions of the like.

NOV20 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV20 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV21

A disclosed NOV21 nucleic acid of 1931 nucleotides (also referred to as CG57436-01) encoding a novel Platelet Glycoprotein V-like protein is shown in Table 21A. An open reading frame was identified beginning with an ATG initiation codon at nucleotides 135-137 and ending with a TAA codon at nucleotides 1878-1880. Putative untranslated regions upstream from the intitation codon and downstream from the termination codon are underlined in Table 21A, and the start and stop codons are in bold letters.

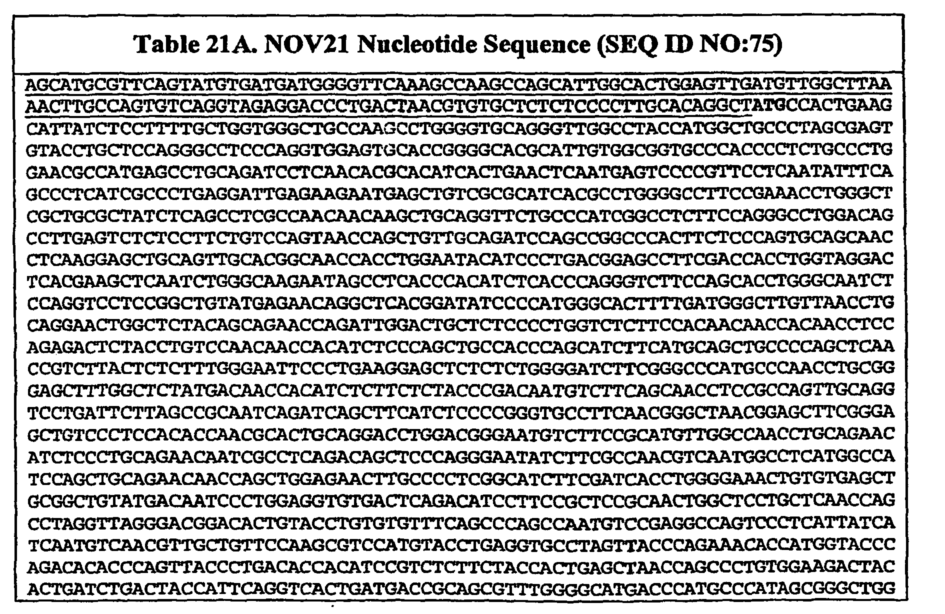

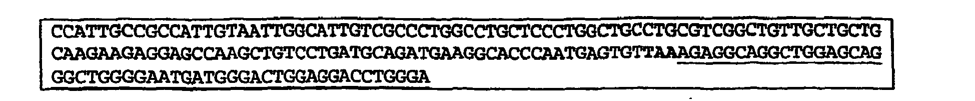

The NOV21 nucleic acid was identified on chromosome 1 and has 779 of 1317 bases (59%) identical to a gb:GENBANK-ID:AF163101|acc:AF163101.1 mRNA from *Mus musculus (Mus musculus* platelet glycoprotein V gene, partial cds) (E = 4.4e⁻⁴³).

A disclosed NOV21 polypeptide (SEQ ID NO:76) encoded by SEQ ID NO:75 is 581 amino acid residues and is presented using the one-letter code in Table 21B. Signal P, Psort and/or Hydropathy results predict that NOV21 contains a signal peptide and is likely to be localized to the plasma membrane with a certainty of 0.4600. The most likely cleavage site for a NOV21 polypeptide is between amino acids 21 and 22: GLA-YH.

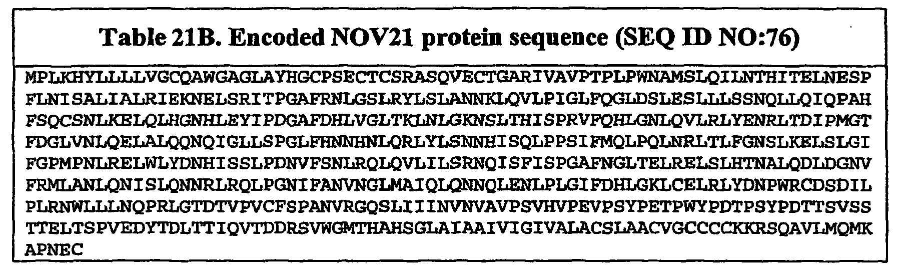

The NOV21 amino acid sequence 165 of 434 amino acid residues (38%) identical to, and 235 of 434 amino acid residues (54%) similar to, the 567 amino acid residue ptnr:SPTREMBL-ACC:Q9QZU3 protein from *Mus musculus* (Mouse) (platelet Glycoprotein V) (E = 7.5e⁻⁷³).

NOV21 is expressed in at least the following tissues: Mammary Gland/Breast. This information was derived by determining the tissue sources of the sequences that were included in the invention including but not limited to SeqCalling sources, Public EST sources, genomic clone sources, literature sources, and/or RACE sources.

Possible SNPs found for NOV21 are listed in Table 21C.

| **Table 21C: SNPs** | | | | |
|---|---|---|---|---|
| **Variant** | **Nucleotide Position** | **Base Change** | **Amino Acid Position** | **Base Change** |
| 13377106 | 166 | T > C | 11 | Val > Ala |
| 13377107 | 287 | G> A | Silent | N/A |
| 13377108 | 399 | C > T | Silent | N/A |
| 13377109 | 576 | T > C | 148 | Cys > Arg |
| 13377110 | 1423 | A > G | 430 | Asp > Gly |
| 13377111 | 1517 | C > T | Silent | N/A |
| 13377112 | 1595 | A > G | Silent | N/A |
| 13377113 | 1618 | G > A | 495 | Ser > Asn |
| 13377114 | 1749 | C > T | Silent | N/A |

NOV21 has homology to the amino acid sequences shown in the BLASTP data listed in Table 21D.

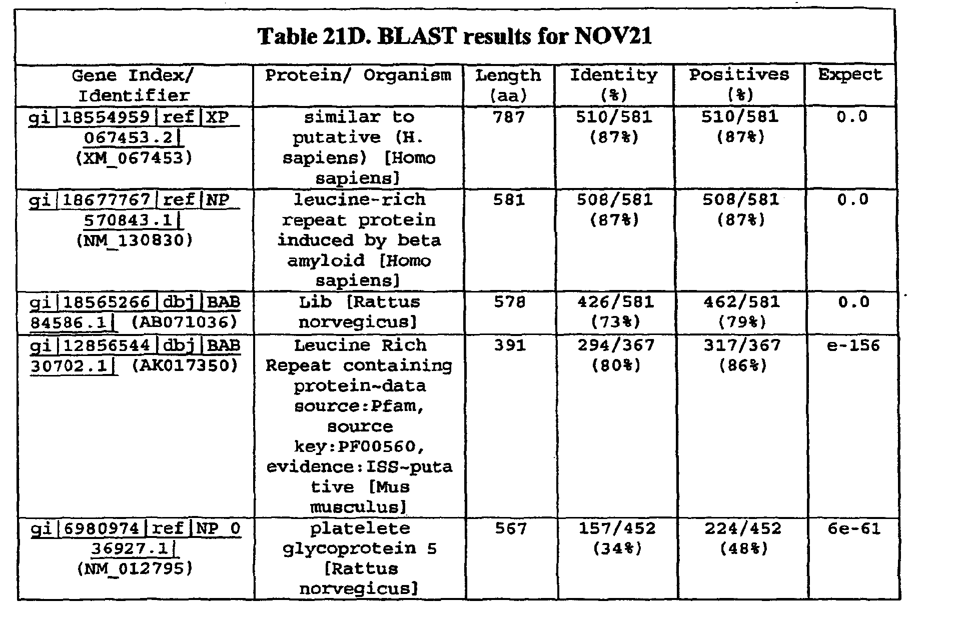

The homology of these sequences is shown graphically in the ClustalW analysis shown in Table 21E.

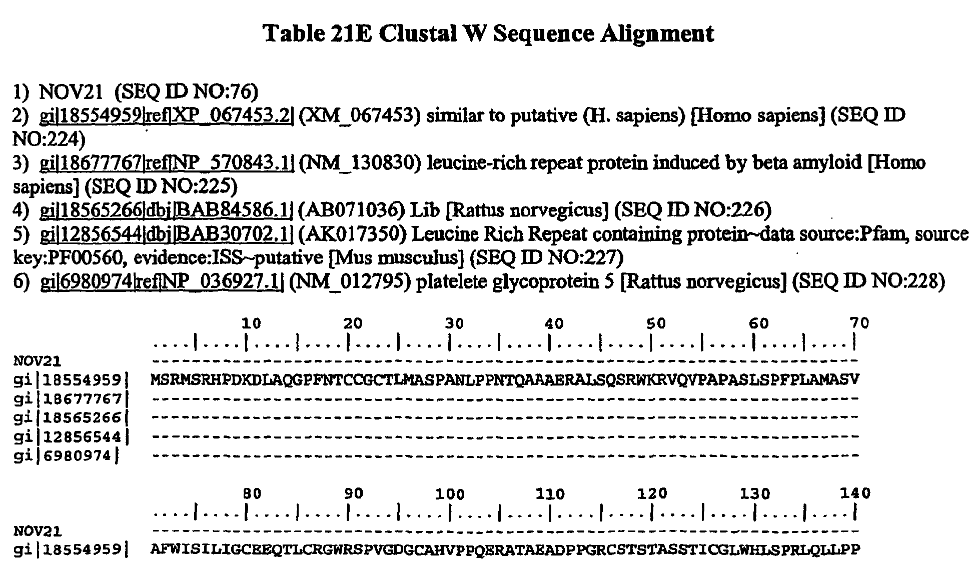

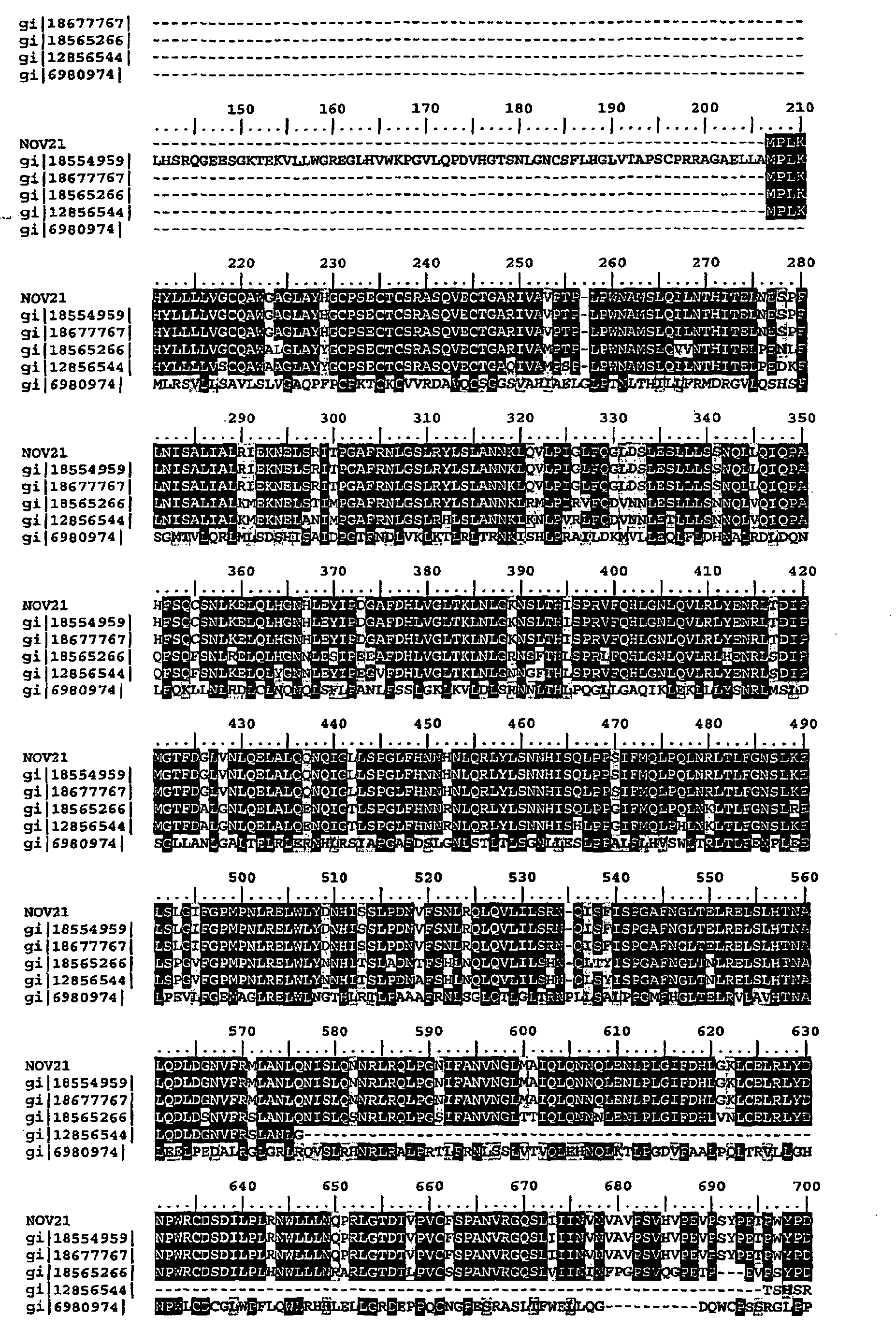

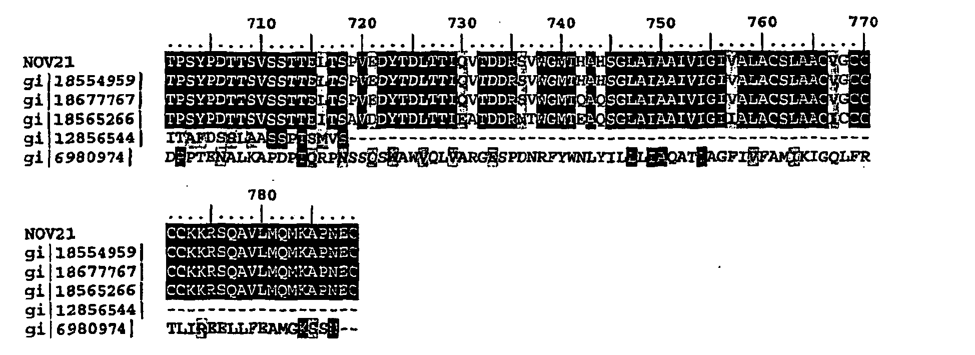

Table 21F lists the domain description from DOMAIN analysis results against NOV21. This indicates that the NOV21 sequence has properties similar to those of other proteins known to contain these domains.

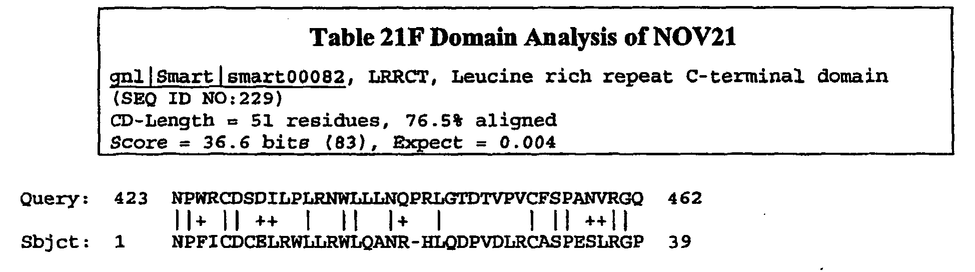

Following injury to a blood vessel, platelets rapidly adhere to the site of exposed subendothelial matrix in the vessel wall, become activated, and secrete granule contents that will recruit and activate other platelets, which then aggregate at the site (thrombus) and seal the defect in the vessel wall. The clot that forms prevents further blood loss (Lopez and Dong, Curr Opin Hematol 4(5):323-9, 1997). Cell-cell and cell-extracellular matrix adhesion are critical aspects of platelet function, regulating interactions between circulating platelets in the bloodstream with the blood vessel wall. The platelet glycoprotein Ib-IX-V complex plays crucial roles in both of these processes by mediating platelet adhesion to sites of blood vessel injury and by participating in the thrombin induced aggregation of platelets in both haemostasis and thrombosis under conditions of high shear blood flow (Lopez and Dong, 1997; Andrews and Berndt, Histol Histopathol 13(3):837-44,1998). Emerging evidence suggests GP Ib-IX-V-dependent signalling may involve receptor cross-linking and the cytoplasmic signalling protein, 14-3-3 zeta. (Andrews and Berndt, 1998; Williamson et al., Aust N Z J Med 29(3):452-61, 1999).

In addition to this normal physiological response, platelet adhesion is critical in the pathological process of thrombosis, where circulating platelets adhere to sclerotic lesions or undergo shear-induced aggregation within vessels occluded by atherosclerotic plaque (Andrews and Berndt, 1998). Under these circumstances, the resulting thrombus may result in acute myocardial infarction or stroke. The critical role of platelets in the development of the acute coronary syndromes is now well recognised, and a great deal of effort has therefore focused on elucidating the key adhesion receptors mediating platelet-vessel wall and platelet-platelet interactions. The vascular adhesion protein von Willebrand factor (vWf) plays a key role in supporting platelet adhesion to the damaged vessel wall and binds to two adhesion receptors on the platelet surface, the glycoprotein (GP) Ib-V-IX complex and glycoprotein IIb-IIIa (Lopez and Dong, 1997; Andrews et al., Thromb Haemost 82(2):357-64, 1999). The GP Ib-V-IX complex is a unique adhesion receptor which enables platelets to roll on a vWf matrix under conditions of rapid blood flow as well as transducing signals leading to the activation of GP IIb-IIIa (Andrews and Berndt, 1998).

Genetic defects of the blood platelet membrane glycoproteins, GPIIb-IIIa (alpha IIb/beta 3; CD41/CD61) and GPIb-V-IX (CD42) are the origin of several rare bleeding disorders, the best known of which are Glanzmann's thrombasthenia, Bernard-Soulier syndrome, and platelet-type von Willebrand's disease (Hayashi and Suzuki, Semin Thromb Hemost 26(1):53-9, 2000; Clemetson and Clemetson, Curr Opin Hematol 1(5):388-93,1994). In Glanzmann's thrombasthenia, GPIIb-IIIa are missing or defective and platelet aggregation is lacking or reduced. Either gene can be affected and mutations leading to lack of expression or to expression of poorly functional form have been described. In Bernard-Soulier syndrome, GPIb-V-IX are missing or defective, leading to poor platelet adhesion at high-shear stress to damaged vessel wall and reduced platelet response to thrombin. Mutations in both GPIb alpha (CD42b) and GPIX (CD42a) have been described. Mutations in GPIb alpha can also lead to platelet-type von Willebrand's disease in which GPIb-V-IX are expressed normally but bind von Willebrand's factor spontaneously, which leads to platelet aggregation and thrombocytopenia. (Clemetson and Clemetson, 1994). Given the homology of the novel protein in this invention to platelet glycoprotein V, which has a critical role in thrombosis, the novel protein in this invention is an excellent antibody target in thrombosis, arterial thrombotic disorders, bleeding disorders, stroke and atherosclerosis.

The NOV21 nucleic acid of the invention encoding a Platelet Glycoprotein V-like protein includes the nucleic acid whose sequence is provided in Table 21A, or a fragment thereof. The invention also includes a mutant or variant nucleic acid any of whose bases may be changed from the corresponding base shown in Table 21 A while still encoding a protein that maintains its Platelet Glycoprotein V-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 41 % of the NOV21 residues may be so changed.

The NOV21 protein of the invention includes the Platelet Glycoprotein V-like protein whose sequence is provided in Table 21B. The invention also includes a mutant or variant protein any of whose residues may be changed from the corresponding residue shown in Table 21B while still encoding a protein that maintains its Platelet Glycoprotein V-like activities and physiological functions, or a functional fragment thereof. In the mutant or variant protein, up to about 62% of the NOV21 bases may be so changed.

The NOV21 nucleic acids and proteins of the invention are useful in potential diagnostic and therapeutic applications implicated in various diseases and disorders described below and/or other pathologies. For example, the compositions of the present invention will have efficacy for treatment of patients suffering from: cancer, fertility and other diseases, disorders and conditions of the like.

NOV21 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV21 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV22

A disclosed NOV22 nucleic acid of 2189 nucleotides (also referred to as CG57529-01) encoding a novel GARPIN-like protein is shown in Table 22A. An open reading frame was identified beginning with an ATG initiation codon at nucleotides 10-12 and ending with a TGA codon at nucleotides 2056-2058. Putative untranslated regions upstream from the intitation codon and downstream from the termination codon are underlined in Table 22A, and the start and stop codons are in bold letters.

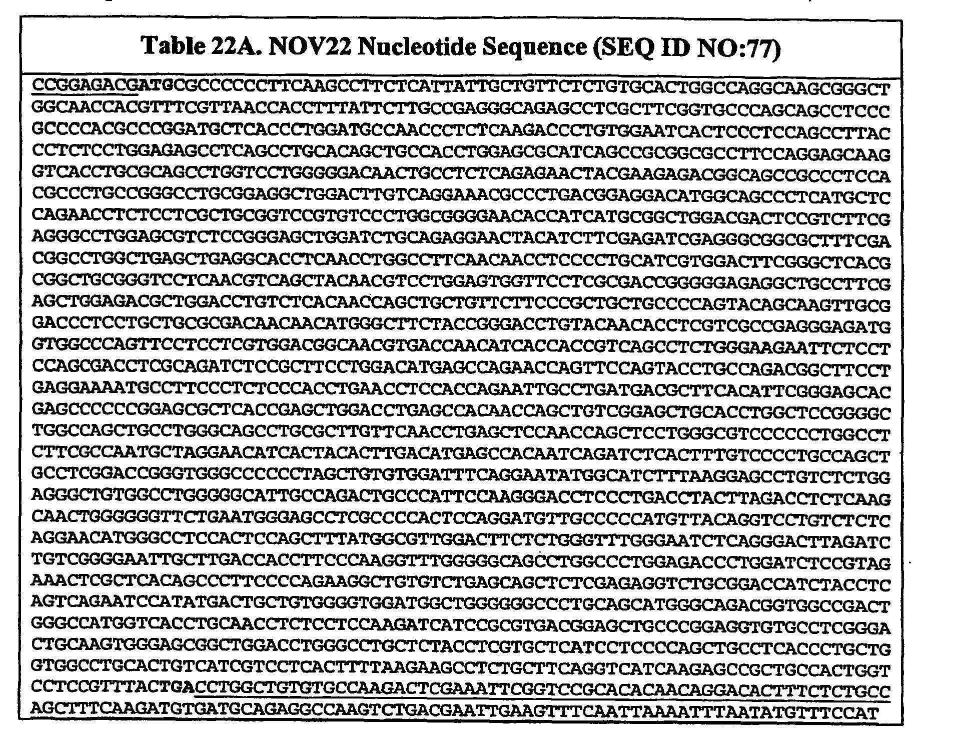

The NOV22 nucleic acid was identified on chromosome 3 and has 671 of 1132 bases (59%) identical to a gb:GENBANK-ID:HUMIGFACID|acc:M86826.1 mRNA from *Homo sapiens* (Human IGF binding protein complex acid-labile subunit a mRNA, complete cds) (E = 2.5e⁻⁰⁸).

A disclosed NOV22 polypeptide (SEQ ID NO:78) encoded by SEQ ID NO:77 is 682 amino acid residues and is presented using the one-letter code in Table 22B. Signal P, Psort and/or Hydropathy results predict that NOV22 contains a signal peptide and is likely to be localized to the plasma membrane with a certainty of 0.4600. The most likely cleavage site for a NOV22 polypeptide is between amino acids 22 and 23: AGW-QP.

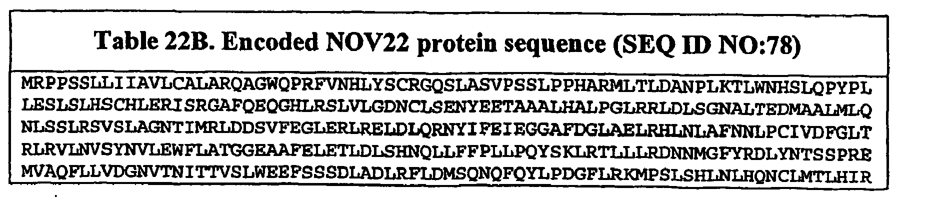

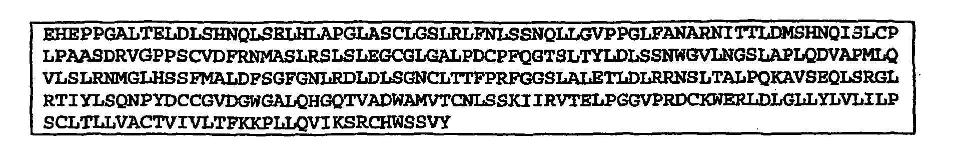

The NOV22 amino acid sequence 253 of 660 amino acid residues (38%) identical to, and 341 of 660 amino acid residues (51%) similar to, the 662 amino acid residue ptnr:SWISSNEW-ACC:Q14392 protein from *Homo sapiens* (Human) (GARP Protein Precursor (GARPIN) (Glycoprotein A Repetitions Predominant) (E = 1.7e⁻⁸²).

NOV22 is expressed in at least the following tissues: ovary, colon, breast and testis. This information was derived by determining the tissue sources of the sequences that were included in the invention including but not limited to SeqCalling sources, Public EST sources, genomic clone sources, literature sources, and/or RACE sources and because of the expression pattern of (gb:GENBANK-ID:HUMIGFACID|acc:M86826.1) a closely related Human IGF binding protein complex acid-labile subunit a mRNA, complete cds homolog in species *Homo sapiens*.

Possible SNPs found for NOV22 are listed in Table 22C.

| **Table 22C: SNPs** | | | | |
|---|---|---|---|---|
| **Variant** | **Nucleotide Position** | **Base Change** | **Amino Acid Position** | **Base Change** |
| 13377115 | 1038 | C > T | Silent | N/A |

NOV22 has homology to the amino acid sequences shown in the BLASTP data listed in Table 22D.

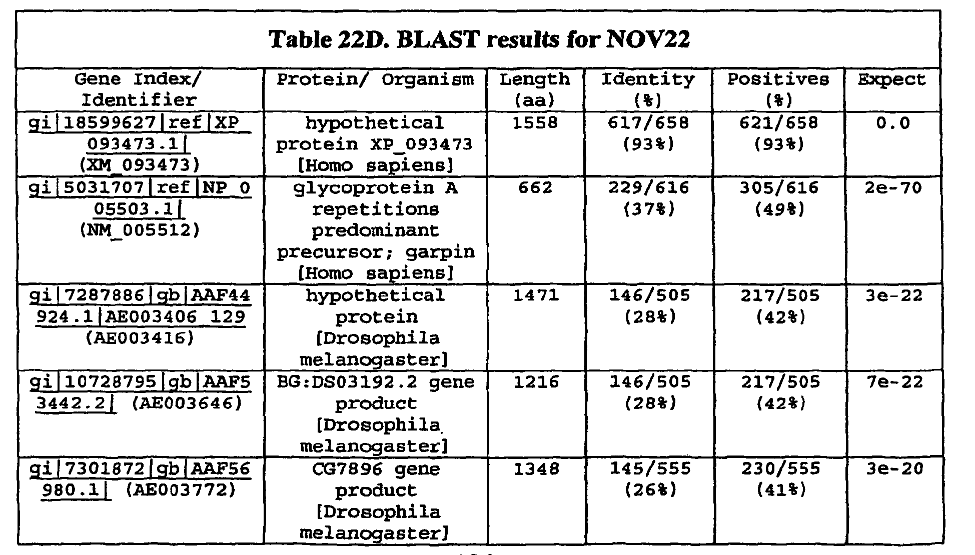

The homology of these sequences is shown graphically in the ClustalW analysis shown in Table 22E.

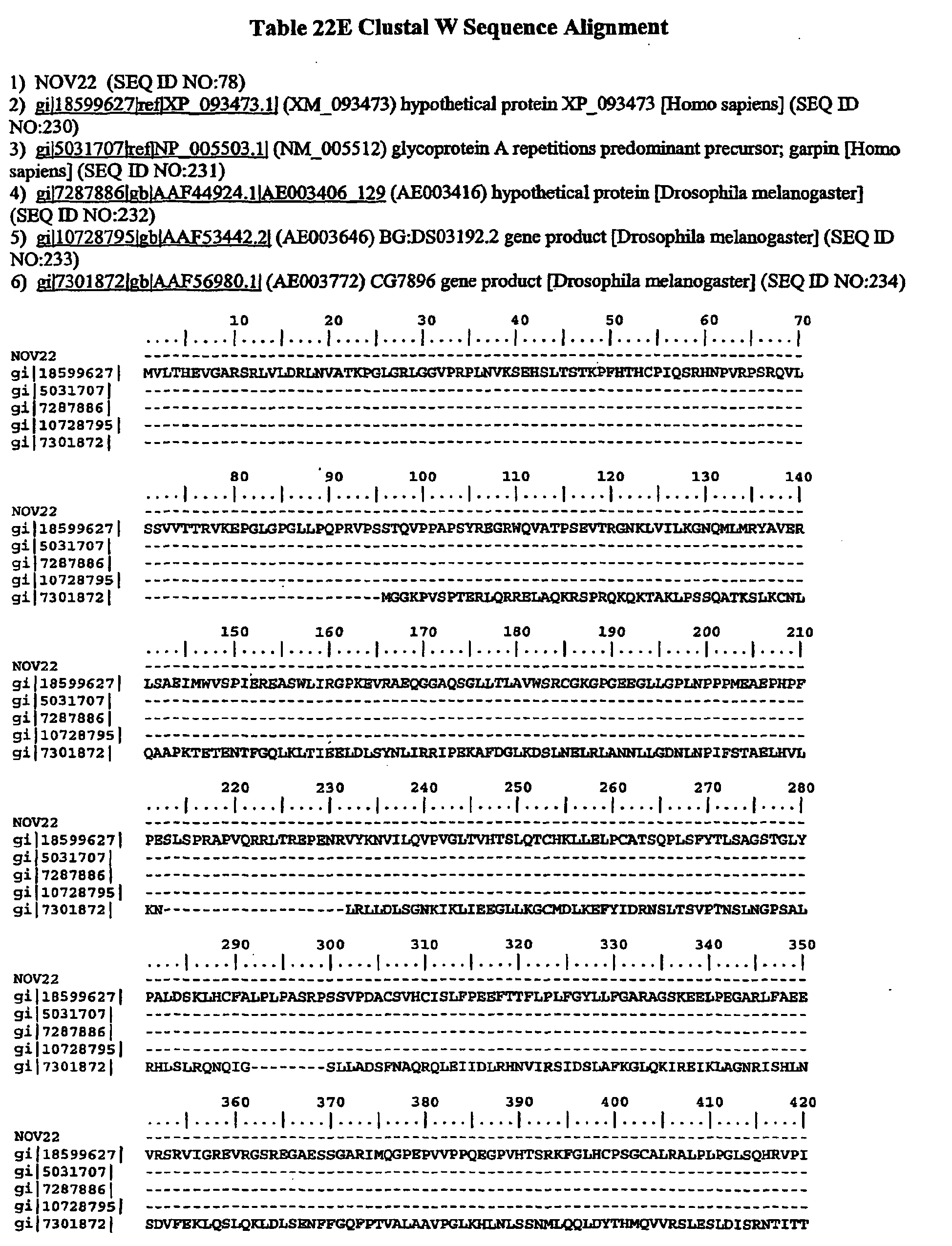

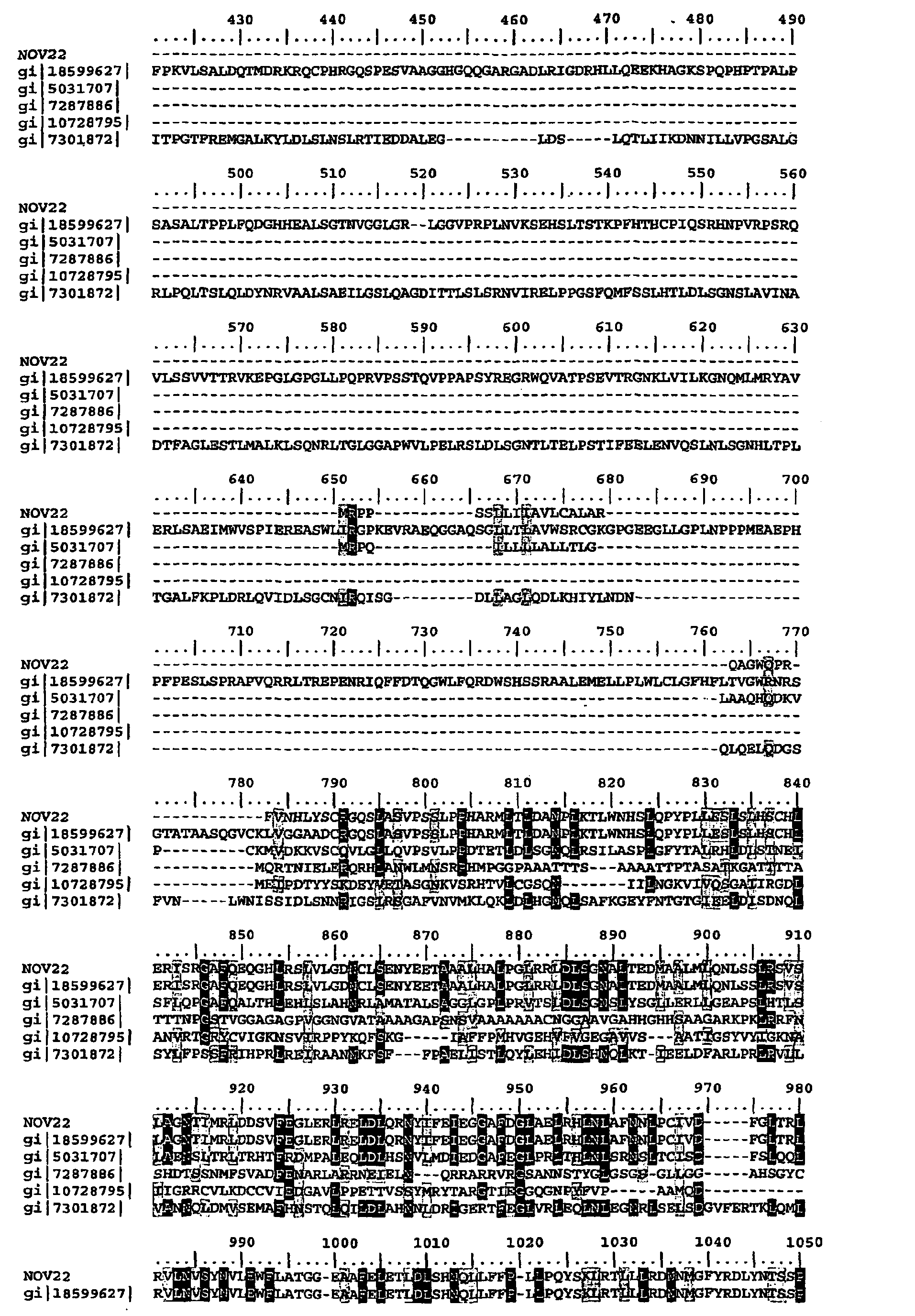

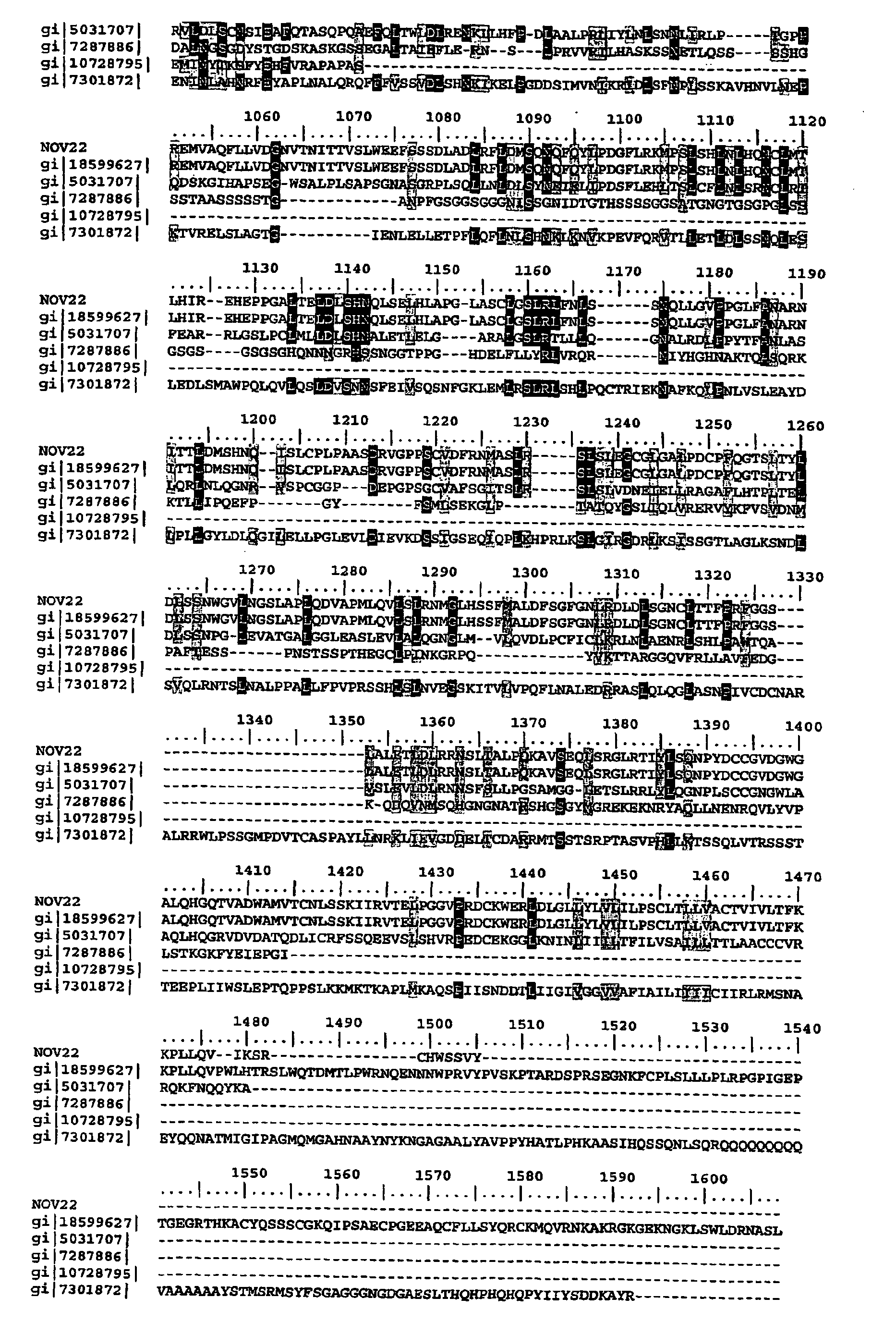

The NOV22 protein described herein belongs to the leucine-rich repeat protein family. It is homologous to GARPIN, insulin-like growth factor-binding protein, and platelet glycoprotein V. The leucine-rich repeat (INTERPRO IPR001611) is a recently characterized structural motif used in molecular recognition processes as diverse as signal transduction, cell adhesion, cell development, DNA repair and RNA processing. All proteins containing these repeats are thought to be involved in protein-protein interactions (Deisenhofer and Kobe, Trends Biochem. Sci. 19: 415-421, 1994).

Ollendorff et al. reported a new human gene, named GARP, encodes a putative transmembrane protein of 662 amino acids, the extracellular portion of which is almost entirely made of leucine-rich repeats. The molecular weight of the protein immunoprecipitated from transfected cells is 80,000. The GARP protein has structural similarities with the human GP Ib alpha and GP V platelet proteins, and with the Chaoptin, Toll, and Connectin adhesion molecules of Drosophila (Ollendorff et al., Cell Growth Differ 5(2):213-9, 1994).

Human platelet glycoprotein (GP) V , is a part of the Ib-V-IX system of surface glycoproteins (GPs Ib alpha, Ib beta, V, IX) that constitute the receptor for von Willebrand factor (vWf) and mediate the adhesion of platelets to injured vascular surfaces in the arterial circulation, a critical initiating event in hemostasis. System members share physical associations, leucine-rich glycoprotein (LRG) structures, and a congenital deficiency state, Bernard-Soulier syndrome (Hickey et al., Proc Natl Acad Sci U S A 90(18):8327-31, 1993). A 16-amino acid signal peptide is present. Mature GP V is a 544-amino acid transmembrane protein with a 504-amino acid extracellular domain that encompasses a set of 15 tandem LRG repeats in a "flank-LRG center-flank" array (Roth, Blood 77, 5-19, 1991) along with eight putative N-linked glycosylation sites and cleavage sites for thrombin and calpain. GP V is a transmembrane, adhesive LRG protein that plays an undefined, but potentially critical, role in the expression and/or function of the Ib-V-IX receptor for vWf/shear-dependent platelet adhesion in arteries (Hickey et al., Proc Natl Acad Sci U S A 90(18):8327-31, 1993).

Nearly all of the insulin-like growth factor (IGF) in the circulation is bound in a heterotrimeric complex composed of IGF, IGF-binding protein-3, and the acid-labile subunit (ALS). Full-length clones encoding ALS have been isolated from human liver cDNA libraries by using probes based on amino acid sequence data from the purified protein. These clones encode a mature protein of 578 amino acids preceded by a 27-amino acid hydrophobic sequence indicative of a secretion signal. Expression of the cDNA clones in mammalian tissue culture cells results in the secretion into the culture medium of ALS activity that can form the expected complex with IGF-I and IGF-binding protein-3. The amino acid sequence of ALS is largely composed of 18-20 leucine-rich repeats of 24 amino acids. These repeats are found in a number of diverse proteins that, like ALS, participate in protein-protein interactions (Leong et al., Mol Endocrinol 6(6):870-6, 1992).

Because of the presence of the Leucine rich repeat domains and the homology to the GARPIN, platelet glycoprotein (GP) V, and IGFBP, it is anticipated that NOV22 described here will have useful properties and functions similar to these genes.

The NOV22 nucleic acid of the invention encoding a GARPIN-like protein includes the nucleic acid whose sequence is provided in Table 22A, or a fragment thereof. The invention also includes a mutant or variant nucleic acid any of whose bases may be changed from the corresponding base shown in Table 22A while still encoding a protein that maintains its GARPIN-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 41% of the NOV22 residues may be so changed.

The NOV22 protein of the invention includes the GARPIN-like protein whose sequence is provided in Table 22B. The invention also includes a mutant or variant protein any of whose residues may be changed from the corresponding residue shown in Table 22B while still encoding a protein that maintains its GARPIN-like activities and physiological functions, or a functional fragment thereof. In the mutant or variant protein, up to about 62% of the NOV22 bases may be so changed.

The NOV22 nucleic acids and proteins of the invention are useful in potential diagnostic and therapeutic applications implicated in various diseases and disorders described below and/or other pathologies. For example, the compositions of the present invention will have effcacy for treatment of patients suffering from: fertility, hypogonadism, endometriosis and other diseases, disorders and conditions of the like.

NOV22 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV22 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV23

A disclosed NOV23 nucleic acid of 2484 nucleotides (also referred to as CG57351-01) encoding a novel Centaurin Beta 2-like protein is shown in Table 23A. An open reading frame was identified beginning with an ATG initiation codon at nucleotides 15-17 and ending with a TAG codon at nucleotides 2307-2309. Putative untranslated regions upstream from the intitation codon and downstream from the termination codon are underlined in Table 23A, and the start and stop codons are in bold letters.

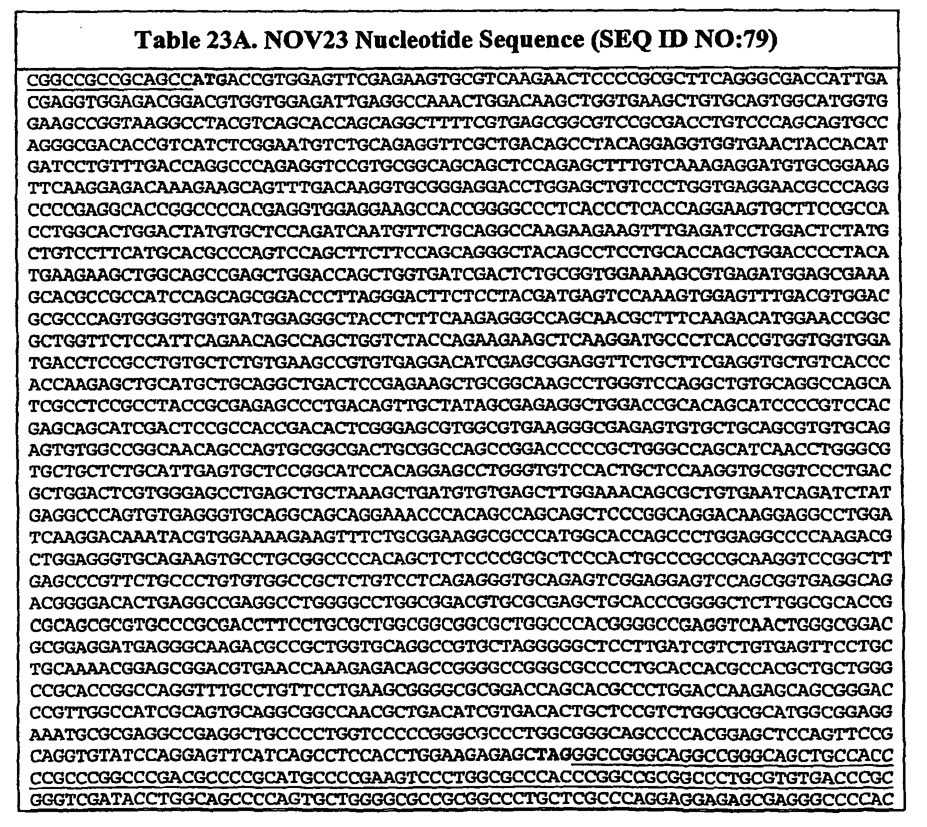

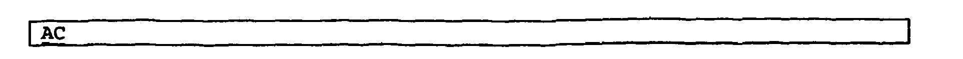

The NOV23 nucleic acid was identified on chromosome 1p36.33 and has 608 of 891 bases (68%) identical to a gb:GENBANK-ID:HSA238248|acc:AJ238248.1 mRNA from *Homo sapiens (Homo sapiens* mRNA for centaurin beta2) (E = 5.9e⁻¹⁴⁹).

A disclosed NOV23 polypeptide (SEQ ID NO:80) encoded by SEQ ID NO:79 is 764 amino acid residues and is presented using the one-letter code in Table 23B. Signal P, Psort and/or Hydropathy results predict that NOV23 contains a signal peptide and is likely to be localized to the plasma membrane with a certainty of 0.7000 and to the nucleus with a certainty of 0.6000.

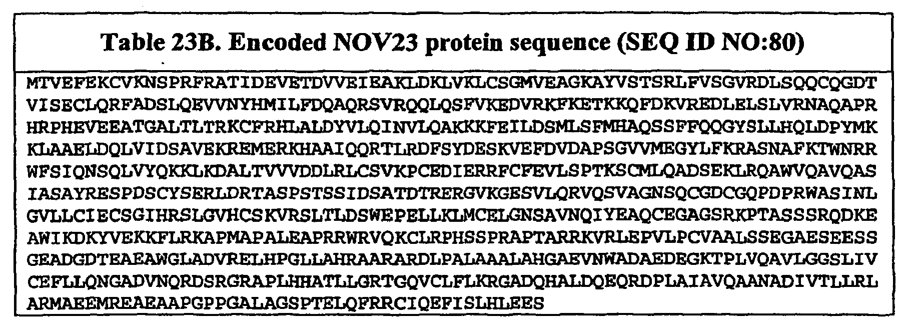

The NOV23 amino acid sequence 456 of 744 amino acid residues (61 %) identical to, and 576 of 744 amino acid residues (77%) similar to, the 778 amino acid residue ptnr:SPTREMBL-ACC:Q9UQR3 protein from *Homo sapiens* (Human) (CENTAURIN BETA2) (E = 3.4e⁻²³⁷).

NOV23 is expressed in at least the following tissues: Brain, mammary gland/breast, pituitary gland, germ cell, ovary, testis and muscle. This information was derived by determining the tissue sources of the sequences that were included in the invention including but not limited to SeqCalling sources, Public EST sources, genomic clone sources, literature sources, and/or RACE sources.

Possible SNPs found for NOV23 are listed in Table 23C.

| **Table 23C: SNPs** | | | | |
|---|---|---|---|---|
| **Variant** | **Nucleotide Position** | **Base Change** | **Amino Acid Position** | **Base Change** |
| 13377116 | 1390 | T > C | 459 | Leu > Pro |
| 13376624 | 1464 | G > A | 484 | Ala > Thr |
| 13376623 | 1480 | C > T | 489 | Ala > Val |
| 13376622 | 1576 | C > T | 521 | Pro> Leu |

NOV23 has homology to the amino acid sequences shown in the BLASTP data listed in Table 23D.

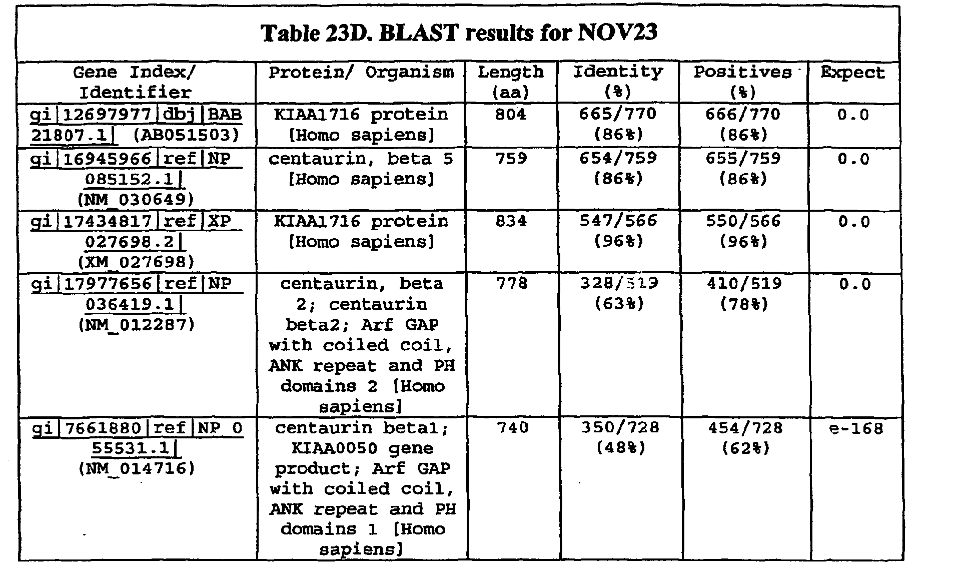

The homology of these sequences is shown graphically in the ClustalW analysis shown in Table 23E.

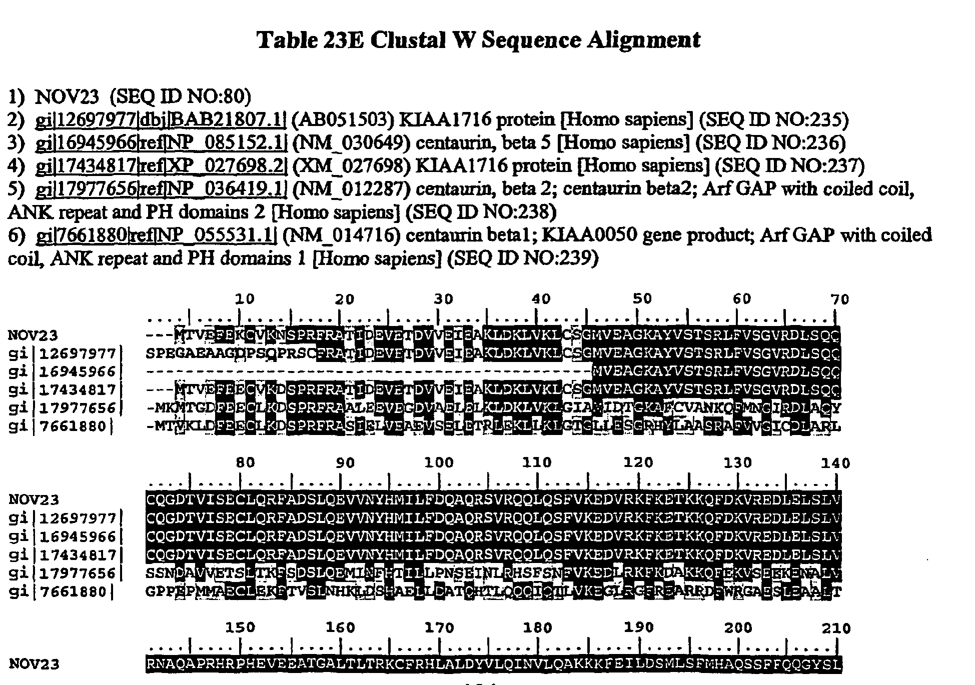

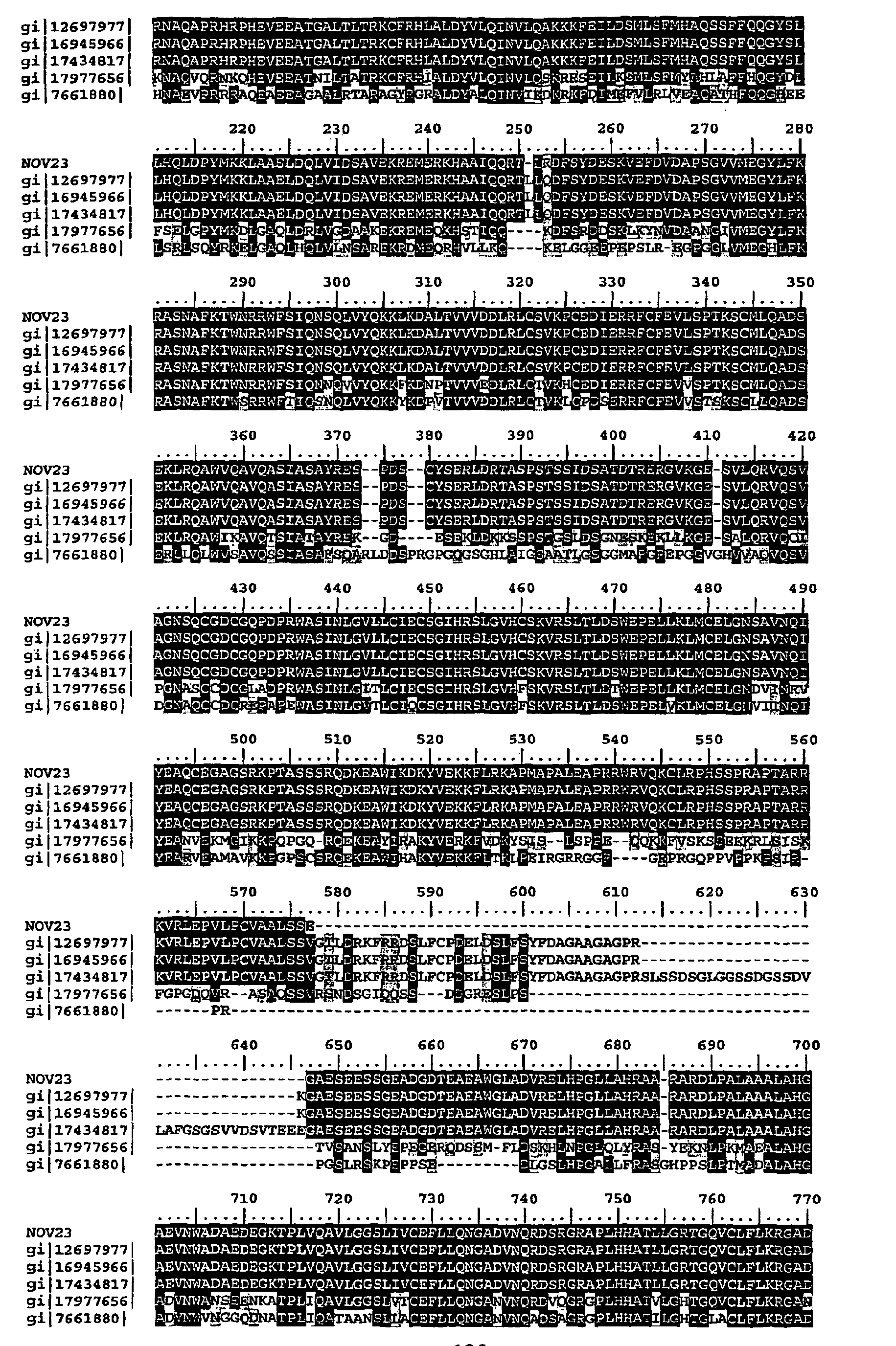

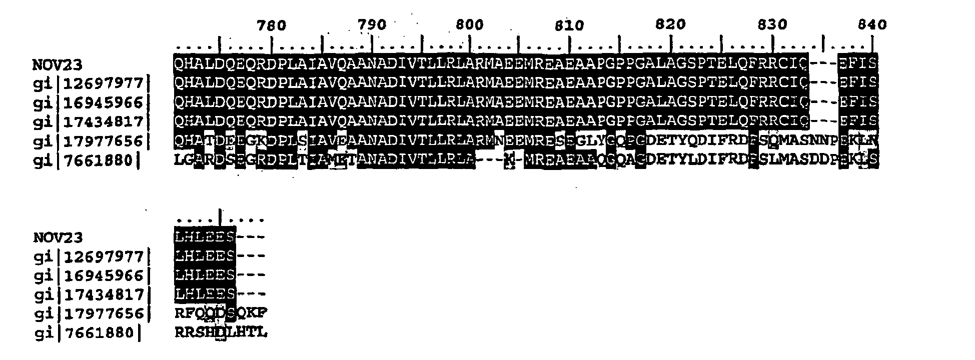

Tables 23F, 23G and 23H list the domain description from DOMAIN analysis results against NOV23. This indicates that the NOV23 sequence has properties similar to those of other proteins known to contain these domains.

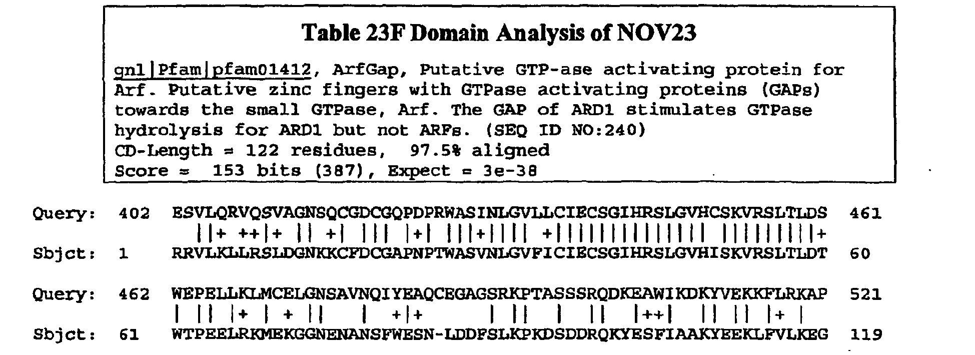

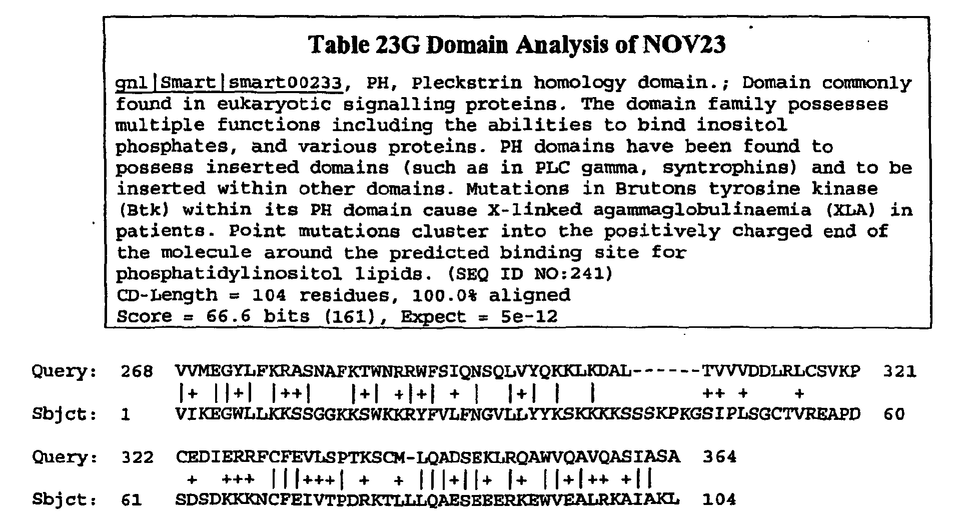

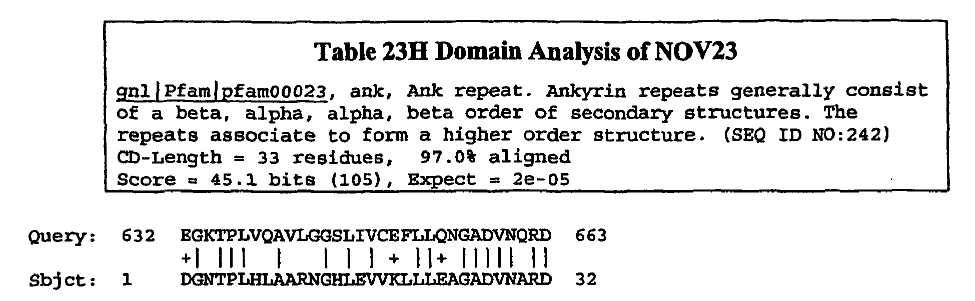

Phosphoinositide (PI) 3-kinases play a role in the regulation of cell growth and survival, metabolism, transcription, vesicular trafficking and cytoskeletal organization. Receptor-regulated (class I) PI 3-kinases phosphorylate phosphatidylinositol 4,5-bisphosphate [PtdIns(4,5)P2] to phosphatidylinositol 3,4,5-trisphosphate [PtdIns(3,4,5)P3]. Proteins in the centaurin family are regulated by phosphoinositide and are regulatory proteins for Arfs (ADP ribosylation factors) (Jackson et al., Trends Biochem. Sci. 25: 489-95, 2000). Arfs are small GTP-binding proteins that function in vesicular trafficking and cytoskeletal regulation. Centaurin family members are GTPase-activating proteins (GAPs) for Arf or have homology to Arf GAPs and Arf effectors. ARFs are essential and ubiquitous in eukaryotes, being involved in vesicular transport and functioning as activators of phospholipase D. The functions of ARF proteins in membrane traffic and organelle integrity are intimately tied to their reversible association with membranes and specific interactions with membrane phospholipids.

The NOV23 protein described herein is predicted to share the attributes of the other centaurin family members and is thus implicated in signal transduction pathways that regulate cell growth and survival, metabolism and transcription. Its potential role in the regulation of ARF activity suggests that the centaurin beta 2-like protein may play an important role in vesicular trafficking and cytoskeletal regulation. Therefore, the NOV23 protein is an attractive target for drug intervention in the treatment of human metabolic diseases, central nervous system disorders, and cancer, among others.

The NOV23 nucleic acid of the invention encoding a Centaurin Beta 2-like protein includes the nucleic acid whose sequence is provided in Table 23A, or a fragment thereof. The invention also includes a mutant or variant nucleic acid any of whose bases may be changed from the corresponding base shown in Table 23A while still encoding a protein that maintains its Centaurin Beta 2-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 32% of the NOV23 residues may be so changed.

The NOV23 protein of the invention includes the Centaurin Beta 2-like protein whose sequence is provided in Table 23B. The invention also includes a mutant or variant protein any of whose residues may be changed from the corresponding residue shown in Table 23B while still encoding a protein that maintains its Centaurin Beta 2-like activities and physiological functions, or a functional fragment thereof. In the mutant or variant protein, up to about 39% of the NOV23 bases may be so changed.

The NOV23 nucleic acids and proteins of the invention are useful in potential diagnostic and therapeutic applications implicated in various diseases and disorders described below and/or other pathologies. For example, the compositions of the present invention will have efficacy for treatment of patients suffering from: cancer, trauma, bacterial and viral infections, in vitro and in vivo regeneration, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration endocrine dysfunctions, diabetes, obesity, growth and reproductive disorders, endometriosis, fertility, hypogonadism, muscular dystrophy, Lesch-Nyhan syndrome, and myasthenia gravis and other diseases, disorders and conditions of the like.

NOV23 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV23 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV24

A disclosed NOV24 nucleic acid of 1930 nucleotides (also referred to as CG57515-01) encoding a novel Sorting Nexin 9-like protein is shown in Table 24A. An open reading frame was identified beginning with an ATG initiation codon at nucleotides 32-34 and ending with a TAA codon at nucleotides 1904-1906. Putative untranslated regions upstream from the intitation codon and downstream from the termination codon are underlined in Table 24A, and the start and stop codons are in bold letters.

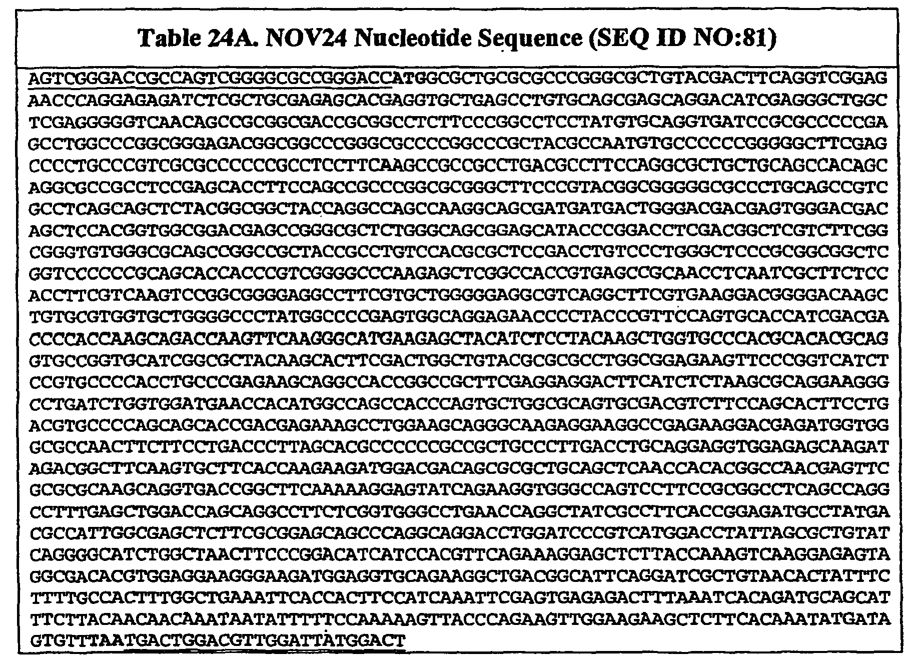

The NOV24 nucleic acid was identified on chromosome 5 and has 275 of 435 bases (63%) identical to a gb:GENBANK-ID:AF121859|acc:AF121859.1 mRNA from *Homo sapiens (Homo sapiens* sorting nexin 9 (SNX9) mRNA, complete cds) (E = 1.7e⁻¹⁴).

A disclosed NOV24 polypeptide (SEQ ID NO:82) encoded by SEQ ID NO:81 is 624 amino acid residues and is presented using the one-letter code in Table 24B. Signal P, Psort and/or Hydropathy results predict that NOV24 contains a signal peptide and is likely to be localized to the cytoplasm with a certainty of 0.7284.

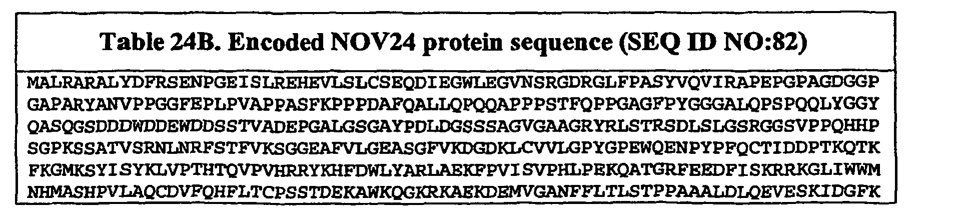

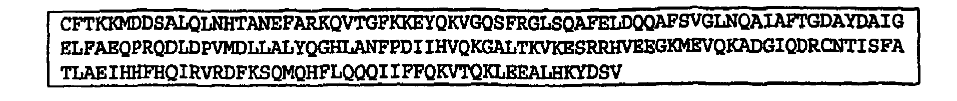

The NOV24 amino acid sequence 171 of 401 amino acid residues (42%) identical to, and 239 of 401 amino acid residues (59%) similar to, the 595 amino acid residue ptnr:SWISSNEW-ACC:Q9Y5X1 protein from *Homo sapiens* (Human) (Sorting Nexin 9 (SH3 and PX Domain-Containing Protein 1) (SDP1 Protein)) (E = 1.0e⁻⁹⁹).

NOV24 is expressed in at least the following tissues: lung, colon, mammary gland/breast, peripheral blood, pituitary gland, thyroid gland, and vulva. This information was derived by determining the tissue sources of the sequences that were included in the invention including but not limited to SeqCalling sources, Public EST sources, genomic clone sources, literature sources, and/or RACE sources.

NOV24 has homology to the amino acid sequences shown in the BLASTP data listed in Table 24C.

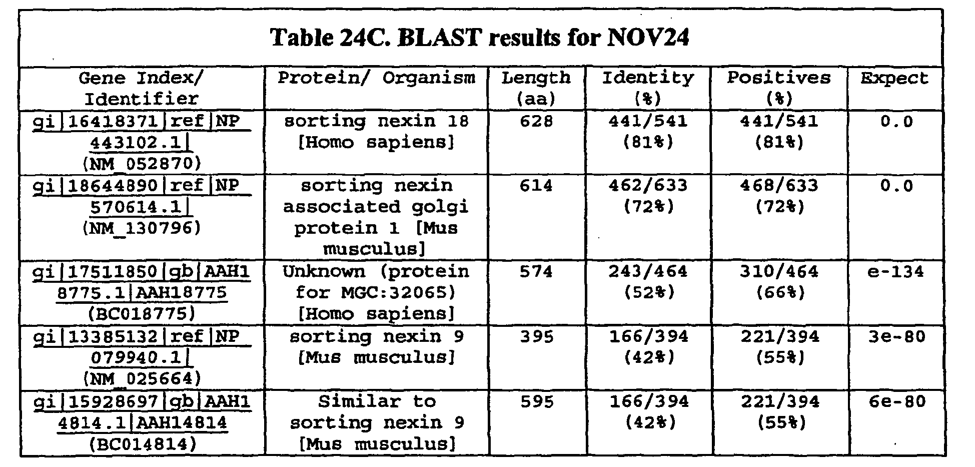

The homology of these sequences is shown graphically in the ClustalW analysis shown in Table 24D.

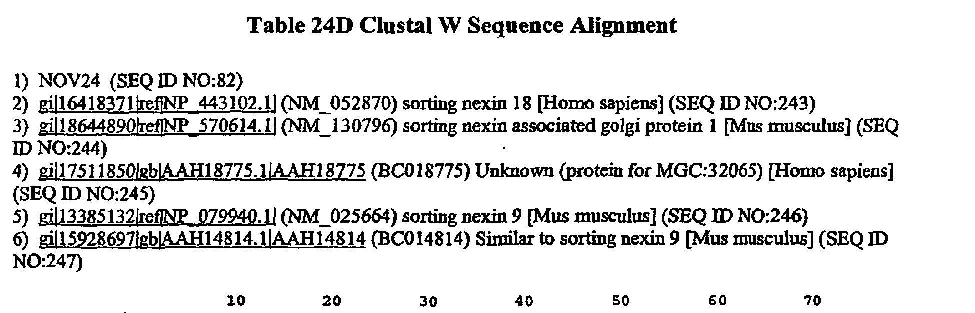

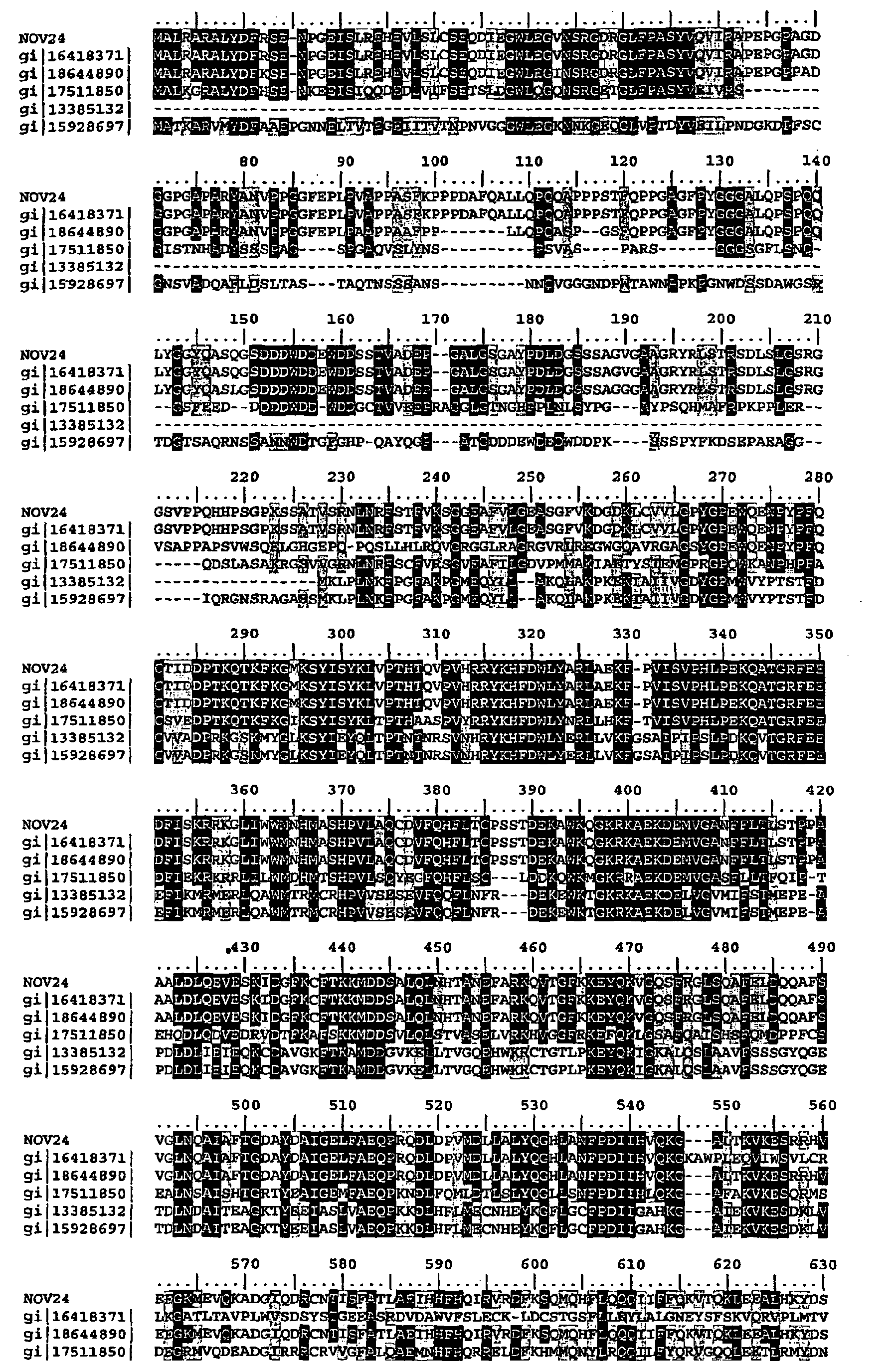

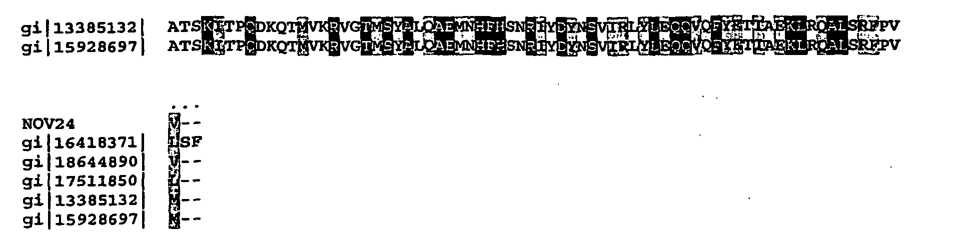

Tables 24E and 24F list the domain description from DOMAIN analysis results against NOV24. This indicates that the NOV24 sequence has properties similar to those of other proteins known to contain these domains.

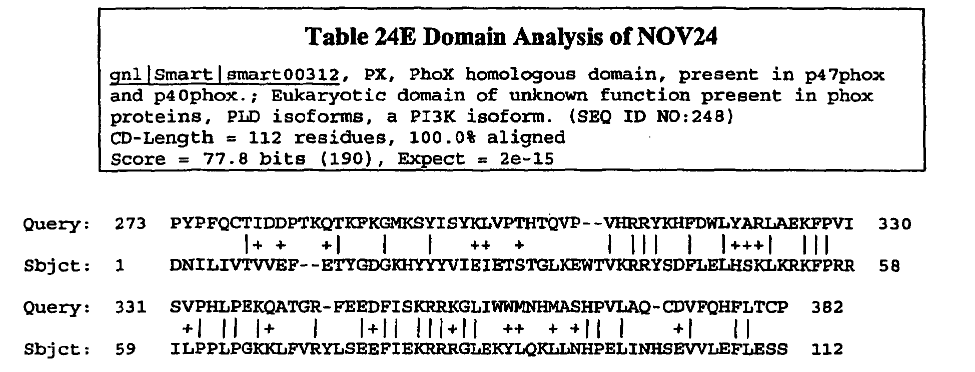

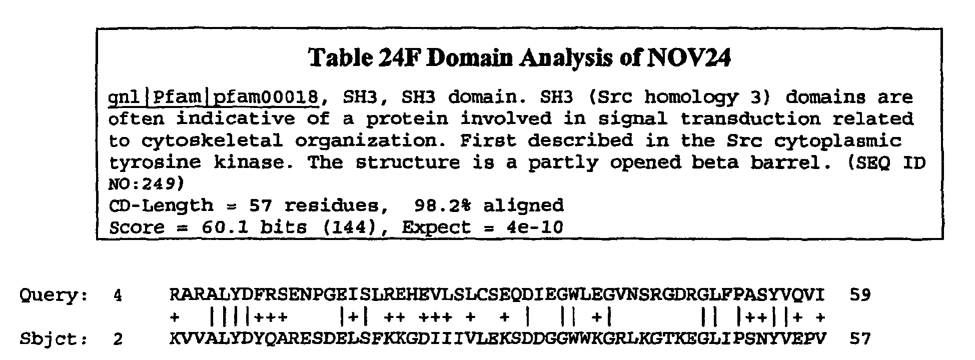

Sorting nexins are a family of phox homology (PX) domain containing proteins that are homologous to yeast proteins involved in protein trafficking. Sorting nexin-1 (SNX1) was identified based on its ability to bind to a region of the epidermal growth factor receptor (EGFR) containing the lysosomal targeting code (Kurten et al., Science 272:1008-10, 1996). The SNX1 protein contains a region of homology to a yeast vacuolar sorting protein, and overexpression of SNX1 decreased the amount of EGFR on the cell surface as a result of enhanced rates of constitutive and ligand-induced degradation. Human SNX1, SNX1A, SNX2, SNX3, and SNX4 are part of a larger family of hydrophilic molecules including proteins identified in Caenorhabditis elegans and Saccharomyces cerevisiae (Haft et al., MoL Cell. Biol. 18:7278-87, 1998). Despite their hydrophilic nature, these sorting nexins are found partially associated with cellular membranes. They are widely expressed, although the tissue distribution of each sorting nexin mRNA varies. The sorting nexins have been demonstrated to interact with a variety of receptor types (including EGFR, platelet derived growth factor receptor, and leptin) suggesting that these proteins may be involved in several stages of intracellular trafficking in mammalian cells.

The NOV24 protein described in this invention is predicted to share the attributes of the other sorting nexin family members and is thus implicated in protein trafficking. Its potential role in the regulation of growth factor receptor localization and levels suggests that the NOV24 protein may play an important role in cell growth and differentiation. Therefore, the NOV24 protein is an attractive target for drug intervention in the treatment of human metabolic diseases, immune disorders, and cancer, among others.

The NOV24 nucleic acid of the invention encoding a Sorting Nexin 9-like protein includes the nucleic acid whose sequence is provided in Table 24A, or a fragment thereof. The invention also includes a mutant or variant nucleic acid any of whose bases may be changed from the corresponding base shown in Table 24A while still encoding a protein that maintains its Sorting Nexin 9-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 37% of the NOV24 residues may be so changed.

The NOV24 protein of the invention includes the Sorting Nexin 9-like protein whose sequence is provided in Table 24B. The invention also includes a mutant or variant protein any of whose residues may be changed from the corresponding residue shown in Table 24B while still encoding a protein that maintains its Sorting Nexin 9-like activities and physiological functions, or a functional fragment thereof. In the mutant or variant protein, up to about 58% of the NOV24 bases may be so changed.

The NOV24 nucleic acids and proteins of the invention are useful in potential diagnostic and therapeutic applications implicated in various diseases and disorders described below and/or other pathologies. For example, the compositions of the present invention will have efficacy for treatment of patients suffering from: cancer, trauma, bacterial and viral infections, in vitro and in vivo regeneration, systemic lupus erythematosus, autoimmune disease, asthma, emphysema, scleroderma, allergy, ARDS, Hirschsprung's disease, Crohn's Disease, appendicitis, hyperthyroidism, hypothyroidism, fertility, anemia, ataxia-telangiectasia, immunodeficiencies, endocrine dysfunctions, diabetes, obesity, growth and reproductive disorders and other diseases, disorders and conditions of the like.

NOV24 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV24 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV25

A disclosed NOV25 nucleic acid of 1514 nucleotides (also referred to as CG57568-01) encoding a novel Katanin-like protein is shown in Table 25A. An open reading frame was identified beginning with an ATG initiation codon at nucleotides 102-104 and ending with a TGA codon at nucleotides 1191-1193. Putative untranslated regions upstream from the intitation codon and downstream from the termination codon are underlined in Table 25A, and the start and stop codons are in bold letters.

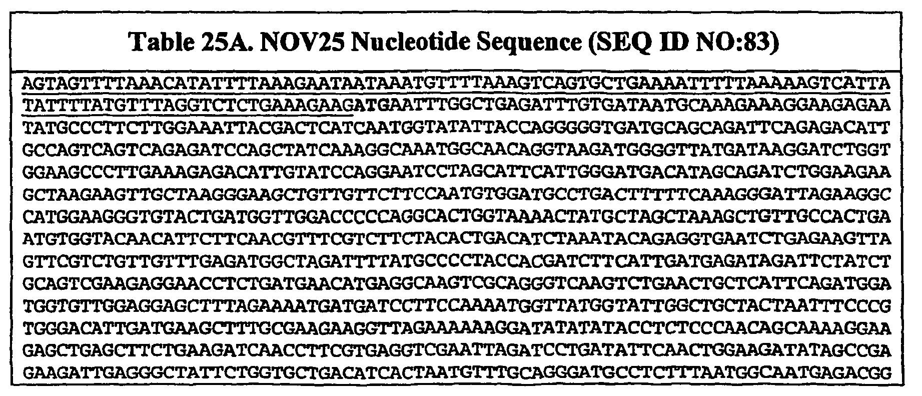

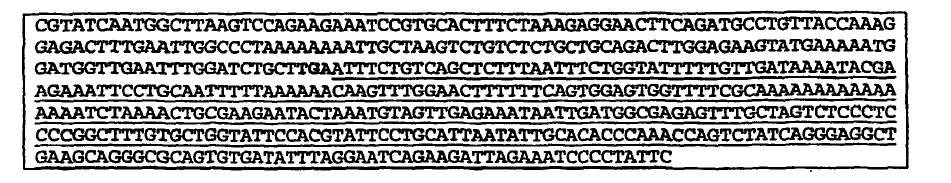

The NOV25 nucleic acid was identified on chromosome 13 and has 720 of 953 bases (75%) identical to a gb:GENBANK-ID:AF056022|acc:AF056022.1 mRNA from *Homo sapiens (Homo sapiens* p60 katanin mRNA, complete cds) (E = 1.6e⁻¹²³).

A disclosed NOV25 polypeptide (SEQ ID NO:84) encoded by SEQ ID NO:83 is 363 amino acid residues and is presented using the one-letter code in Table 25B. Signal P, Psort and/or Hydropathy results predict that NOV25 does not contain a signal peptide and is likely to be localized to the cytoplasm with a certainty of 0.6500.

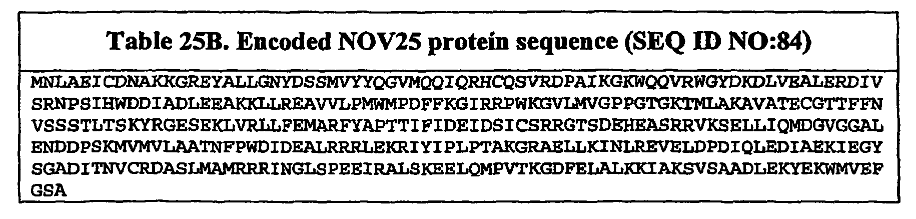

The NOV25 amino acid sequence 260 of 305 amino acid residues (85%) identical to, and 285 of 305 amino acid residues (93%) similar to, the 491 amino acid residue ptnr:SPTREMBL-ACC:O75449 protein from *Homo sapiens* (Human) (P60 Katanin) (E = 1.2e⁻¹⁵⁵).

NOV25 is expressed in at least the following tissues: lung, testis and b-cell. This information was derived by determining the tissue sources of the sequences that were included in the invention including but not limited to SeqCalling sources, Public EST sources, genomic clone sources, literature sources, and/or RACE sources.

Possible SNPs found for NOV25 are listed in Table 25C.

| **Table 25C: SNPs** | | | | |
|---|---|---|---|---|
| **Variant** | **Nucleotide Position** | **Base Change** | **Amino Acid Position** | **Base Change** |
| 13377117 | 1050 | A > G | 317 | Ile > Val |
| 13377118 | 1061 | T > C | Silent | N/A |
| 13377119 | 1073 | A > G | Silent | N/A |
| 13377120 | 1074 | C > T | 325 | Leu > Phe |
| 13377121 | 1085 | T > C | Silent | N/A |
| 13377122 | 1111 | C > T | 337 | Ala > Val |
| 13377123 | 1127 | T > C | Silent | N/A |
| 13377124 | 1178 | A > G | Silent | N/A |
| 13377125 | 1190 | T>C | Silent | N/A |
| 13377126 | 1297 | T>C | Silent | N/A |

NOV25 has homology to the amino acid sequences shown in the BLASTP data listed in Table 25D.

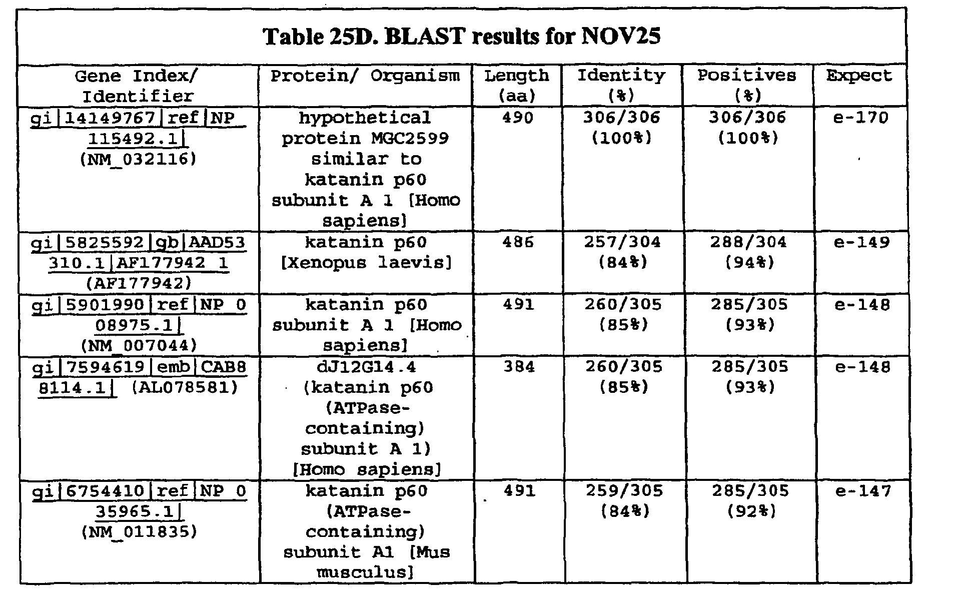

The homology of these sequences is shown graphically in the ClustalW analysis shown in Table 25E.

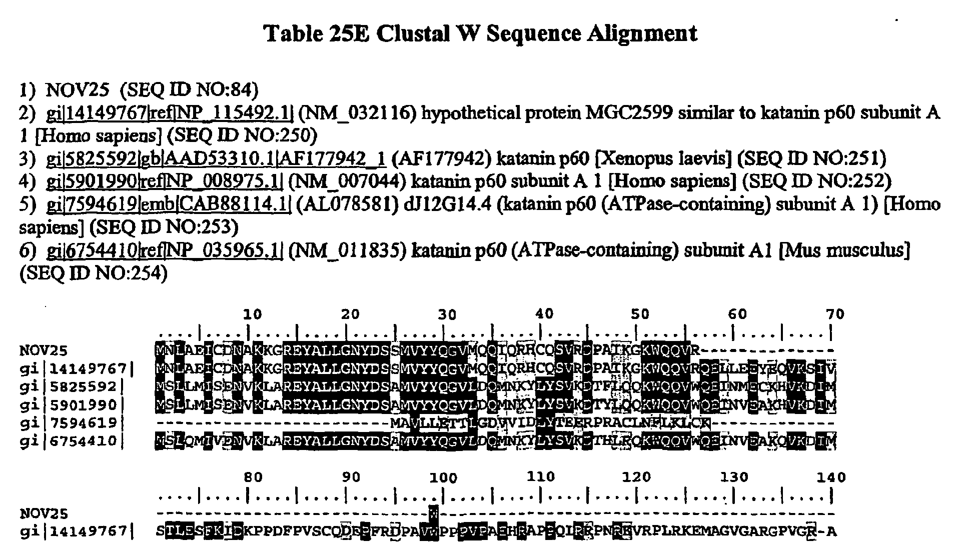

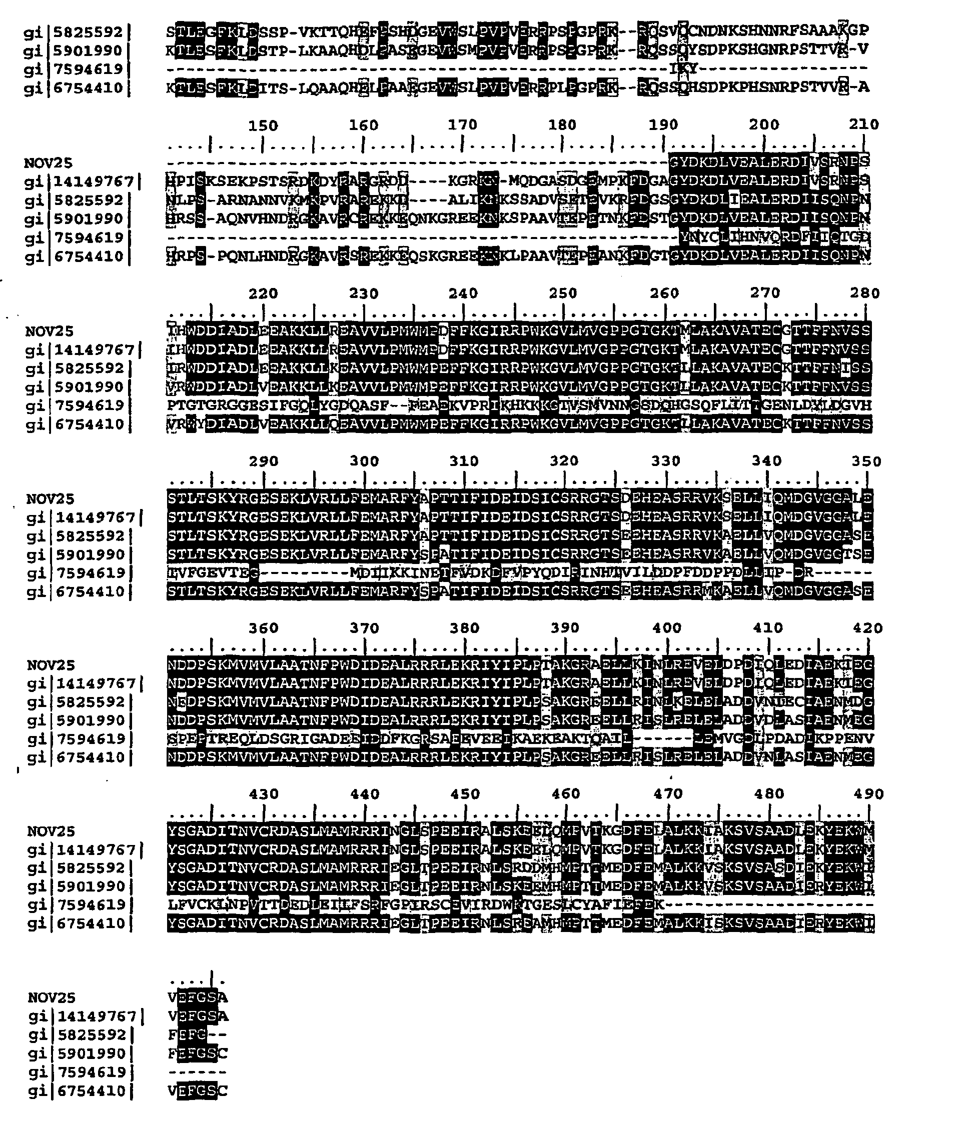

Table 25F lists the domain description from DOMAIN analysis results against NOV25. This indicates that the NOV25 sequence has properties similar to those of other proteins known to contain these domains.

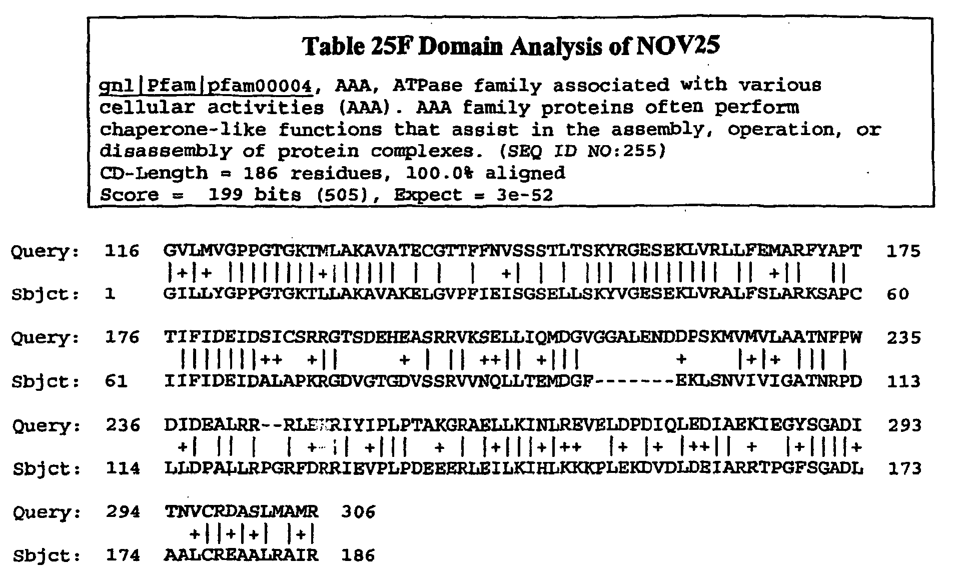

Microtubule disassembly at centrosomes is involved in mitotic spindle function. The microtubule-severing protein katanin, a heterodimer of 60- and 80-kD subunits, was purified and shown to localize to centrosomes in vivo (McNally and Vale, Cell 75: 419-429, 1993; McNally et al., J. Cell Sci. 109: 561-567, 1996). By homology to the sequence of S. purpuratus, Hartman et al. (Cell 93: 277-287, 1998) cloned the cDNA for the human p80 subunit. This protein contains WD40 repeats, which are frequently involved in protein-protein interactions. The p80 WD40 domain does not participate in p60 dimerization, but localizes to centrosomes in transfected mammalian cells. The results of the studies of Hartman et al. (1998) in yeast indicated that the activities of katanin are segregated into a subunit (p60) that possesses enzymatic activity and a subunit (p80) that targets the enzyme to the centrosome.

Microtubule disassembly at centrosomes is involved in mitotic spindle function. The microtubule-severing protein katanin, a heterodimer of 60 and 80 kDa subunits, was previously purified and shown to localize to centrosomes in vivo. The sequences and activities of the katanin subunits were reported. p60 is a new member of the AAA family of ATPases, and expressed p60 has microtubule-stimulated ATPase and microtubule-severing activities in the absence of p80. p80 is a novel protein containing WD40 repeats, which are frequently involved in protein-protein interactions. The p80 WD40 domain does not participate in p60 dimerization, but localizes to centrosomes in transfected mammalian cells. These results indicate katanin's activities are segregated into a subunit (p60) that possesses enzymatic activity and a subunit (p80) that targets the enzyme to the centrosome (Hartman et al., Cell 93: 277-287,1998).

The assembly and function of the mitotic spindle involve specific changes in the dynamic properties of microtubules. One such change results in the poleward flux of tubulin in which spindle microtubules polymerize at their kinetochore-attached plus ends while they shorten at their centrosome-attached minus ends. Since free microtubule minus ends do not depolymerize in vivo, the poleward flux of tubulin suggests that spindle microtubules are actively disassembled at or near their centrosomal attachment points. The microtubule-severing ATPase, katanin, has the ability actively to sever and disassemble microtubules and is thus a candidate for the role of a protein mediating the poleward flux of tubulin. The subcellular localization of katanin by immunofluorescence was identified as a preliminary step in determining whether katanin mediates the poleward flux of tubulin. Katanin is highly concentrated at centrosomes throughout the cell cycle. Katanin's localization is different from that of gamma-tubulin in that microtubules are required to maintain the centrosomal localization of katanin. Direct comparison of the localization of katanin and gamma-tubulin reveals that katanin is localized in a region surrounding the gamma-tubufin-containing pericentriolar region in detergent-extracted mitotic spindles. The centrosomal localization of katanin is consistent with the hypothesis that katanin mediates the disassembly of microtubule minus ends during poleward flux (McNally and Vale, Cell 75: 419-429, 1993).

The NOV25 nucleic acid of the invention encoding a Katanin-like protein includes the nucleic acid whose sequence is provided in Table 25A, or a fragment thereof. The invention also includes a mutant or variant nucleic acid any of whose bases may be changed from the corresponding base shown in Table 25A while still encoding a protein that maintains its Katanin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 25% of the NOV25 residues may be so changed.

The NOV25 protein of the invention includes the Katauin-like protein whose sequence is provided in Table 25B. The invention also includes a mutant or variant protein any of whose residues may be changed from the corresponding residue shown in Table 25B while still encoding a protein that maintains its Katanin-like activities and physiological functions, or a functional fragment thereof. In the mutant or variant protein, up to about 15% of the NOV25 bases may be so changed.

The NOV25 nucleic acids and proteins of the invention are useful in potential diagnostic and therapeutic applications implicated in various diseases and disorders described below and/or other pathologies. For example, the compositions of the present invention will have efficacy for treatment of patients suffering from: developmental disorders, fertility, hypogonadism, systemic lupus erythematosus, autoimmune disease, asthma, emphysema, scleroderma, allergy, ARDS, immunological diseases and other diseases, disorders and conditions of the like.

NOV25 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV25 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV26

A disclosed NOV26 nucleic acid of 2442 nucleotides (also referred to as CG57509-01) encoding a novel Calpain-like protein is shown in Table 26A. An open reading frame was identified beginning with an ATG initiation codon at nucleotides 77-79 and ending with a TGA codon at nucleotides 2396-2398. Putative untranslated regions upstream from the intitation codon and downstream from the termination codon are underlined in Table 26A, and the start and stop codons are in bold letters.

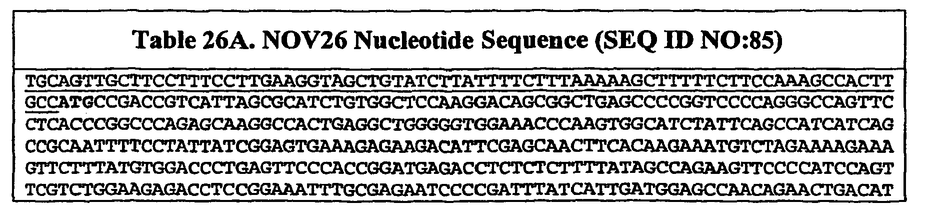

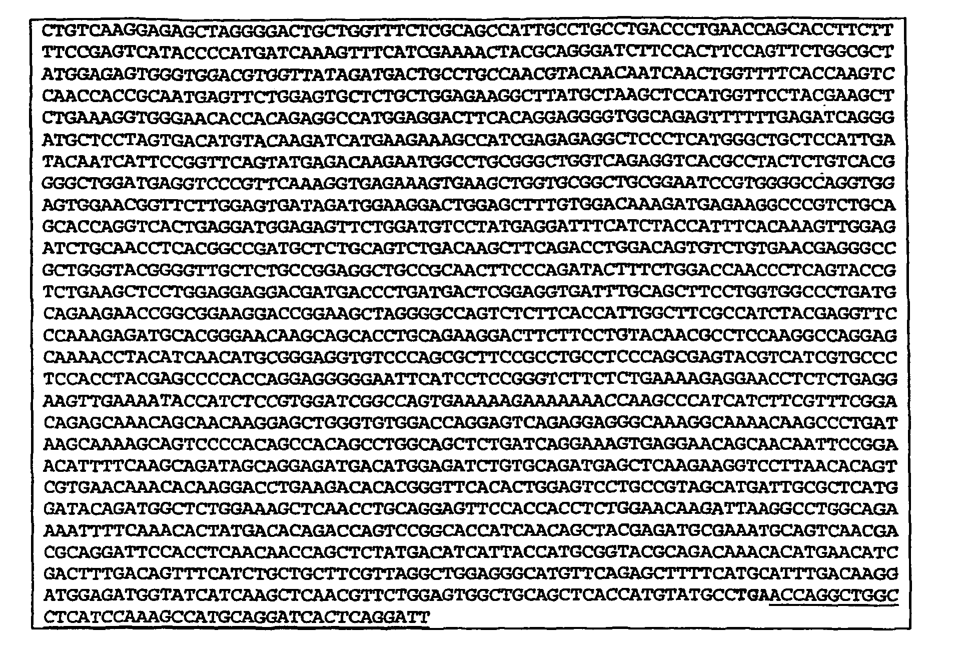

The NOV26 nucleic acid was identified on chromosome 15 and has 1717 of 1720 bases (99%) identical to a gb:GENBANK-ID:AF127765|acc:AF127765.3 mRNA from *Homo sapiens (Homo sapiens* calpain 3 (CAPN3) mRNA, complete cds, alternatively spliced) (E = 0.0).

A disclosed NOV26 polypeptide (SEQ ID NO:86) encoded by SEQ ID NO:85 is 773 amino acid residues and is presented using the one-letter code in Table 26B. Signal P, Psort and/or Hydropathy results predict that NOV26 does not contain a signal peptide and is likely to be localized to the cytoplasm with a certainty of 0.6526. The most likely cleavage site for a NOV26 polypeptide is between amino acids 15 and 16: TAA-EP

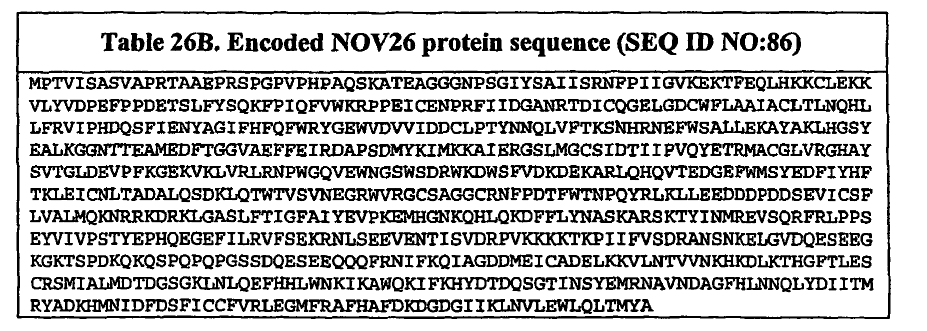

The NOV26 amino acid sequence 506 of 506 amino acid residues (100%) identical to, and 506 of 506 amino acid residues (100%) similar to, the 821 amino acid residue ptnr:SWISSNEW-ACC:P20807 protein from *Homo sapiens* (Human) (Calpain 3 Large Subunit (EC 3.4.22.17) (Calpain L3) (Calpain P94, Large [Catalytic] Subunit) (Calcium-Activated Neutral Proteinase 3) (CANP 3) (Muscle-Specific Calcium-Activated Neutral Protease 3 Large Subunit)) (E = 0.0). The NOV26 amino acid lacks 48 internal amino acids when compared to ptnr:SWISSNEW-ACC.P20807 protein from *Homo sapiens* (Human) (Calpain 3 Large Subunit (EC 3.4.22.17) (Calpain L3) (Calpain P94, Large [Catalytic] Subunit) (Calcium-Activated Neutral Proteinase 3) (CANP 3) (Muscle-Specific Calcium-Activated Neutral Protease 3 Large Subunit)).

NOV26 is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus, aorta, epidermis, foreskin, heart, hypothalamus, liver, lung, lymph node, lymphoid tissue, muscle, peripheral blood, skin and whole organism. This information was derived by determining the tissue sources of the sequences that were included in the invention including but not limited to SeqCalling sources, Public EST sources, genomic clone sources, literature sources, and/or RACE sources. NOV26 is predicted to be expressed in leukocytes because of the expression pattern of (gb:GENBANK-ID:AF127765|acc:AF127765.3) a closely related *Homo sapiens* calpain 3 (CAPN3) mRNA, complete cds, alternatively spliced homolog in species *Homo sapiens.*

Possible SNPs found for NOV26 are listed in Table 26C and Table 26D.

| **Table 26C: SNPs** | | | | |
|---|---|---|---|---|
| **Variant** | **Nucleotide Position** | **Base Change** | **Amino Acid Position** | **Base Change** |
| 13377127 | 72 | T > C | Silent | N/A |
| 13377128 | 78 | T > C | 1 | Met > Thr |
| 13377129 | 201 | T > C | 42 | De>Thr |
| 13377130 | 412 | C > T | Silent | N/A |
| 13377131 | 2253 | A > G | 726 | His > Arg |

| **Table 26D: SNPs** | | | |
|---|---|---|---|
| **Consensus Position** | **Depth** | **Base Change** | **PAF** |
| 2258 | 29 | A > G | 0.379 |

NOV26 has homology to the amino acid sequences shown in the BLASTP data listed in Table 26E.

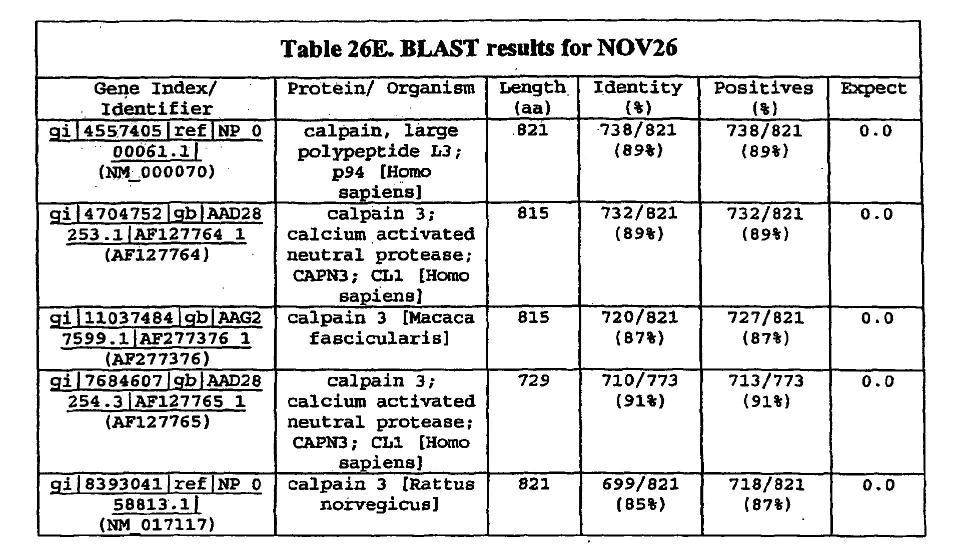

The homology of these sequences is shown graphically in the ClustalW analysis shown in Table 26F.

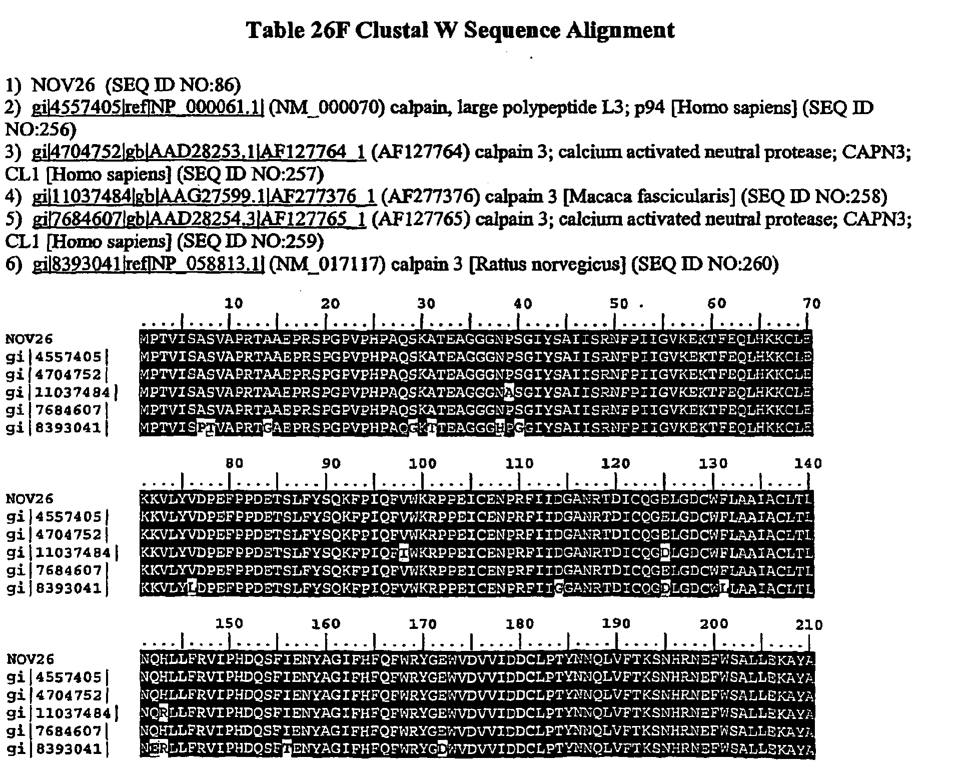

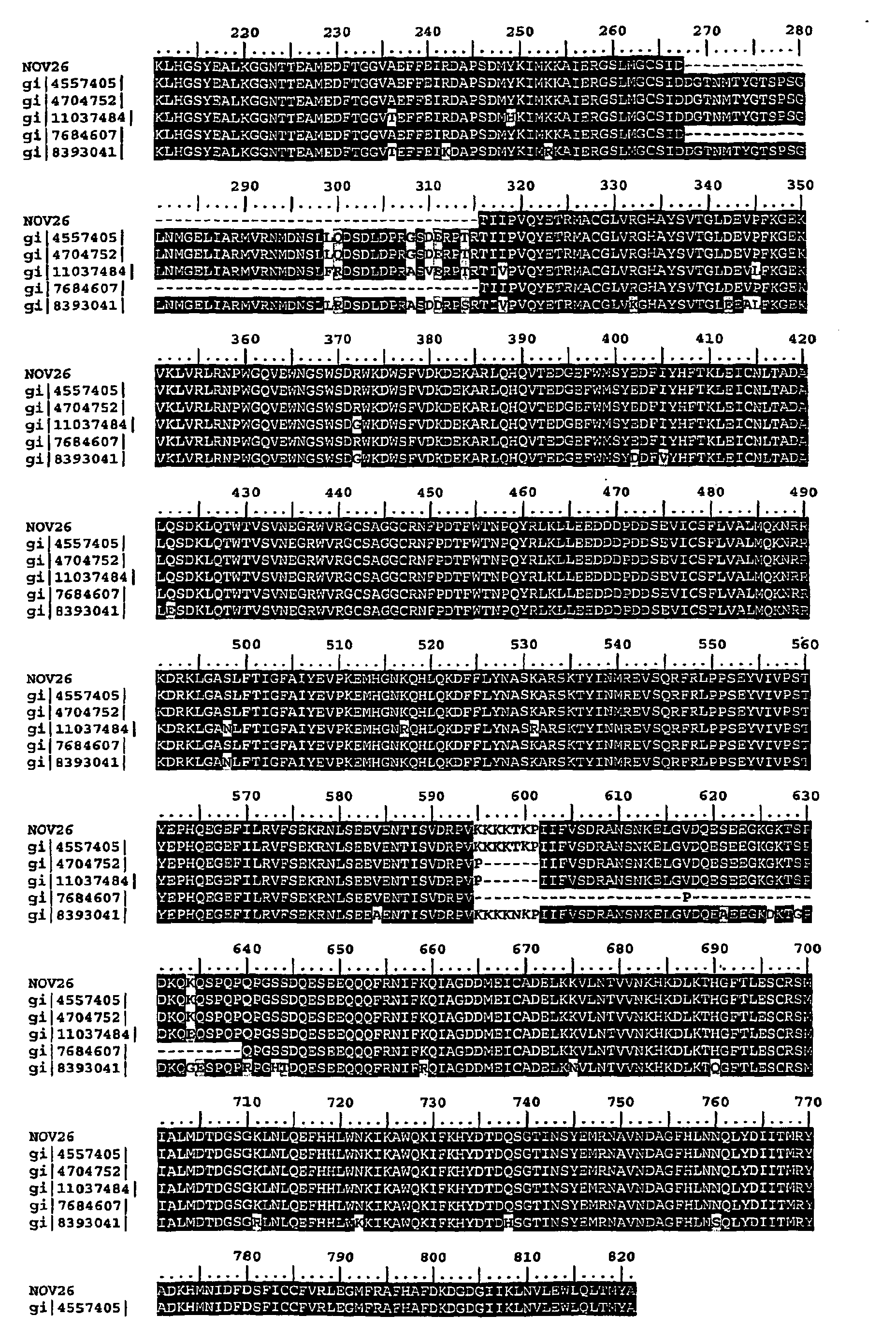

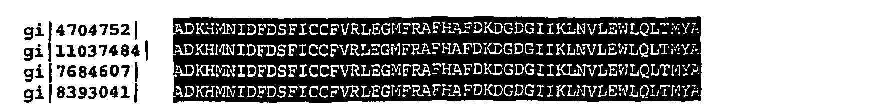

Tables 26G, 26H, 26I and 26J list the domain description from DOMAIN analysis results against NOV26. This indicates that the NOV26 sequence has properties similar to those of other proteins known to contain these domains.

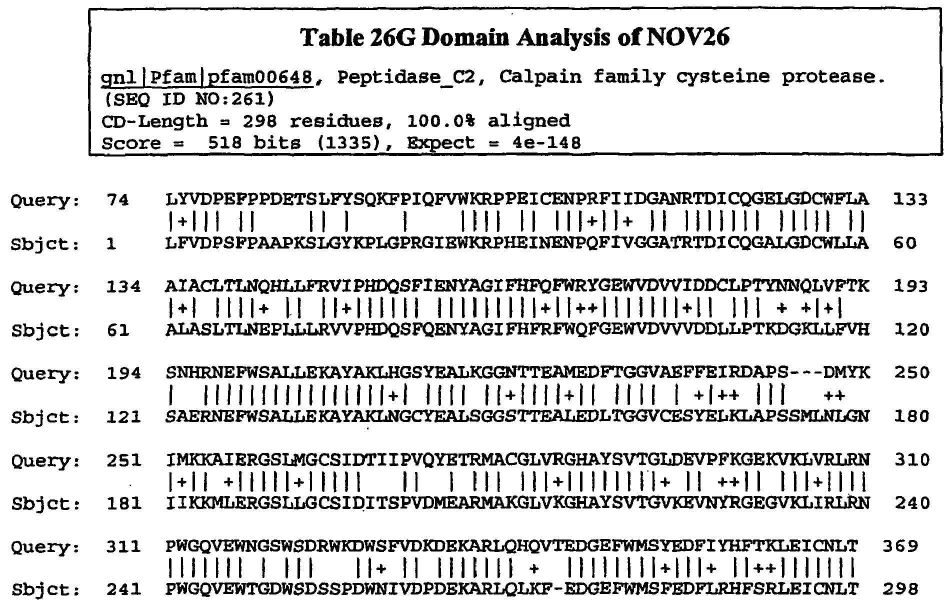

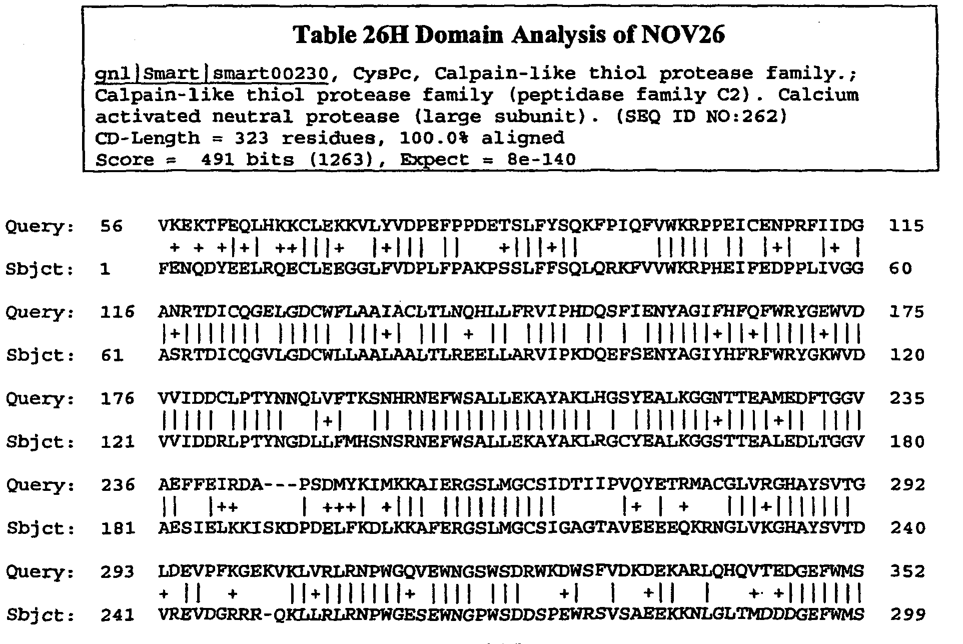

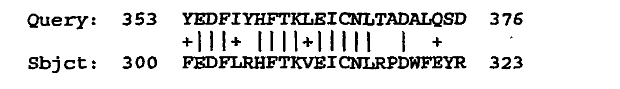

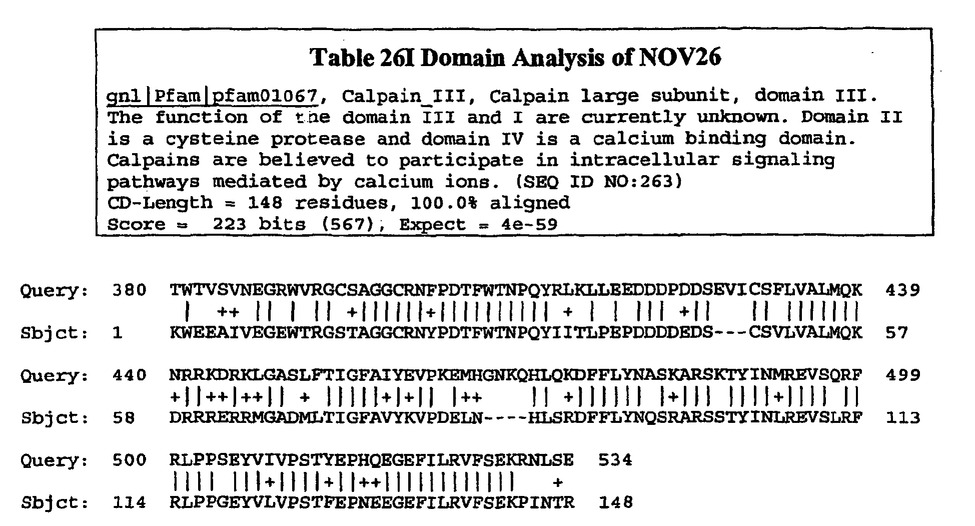

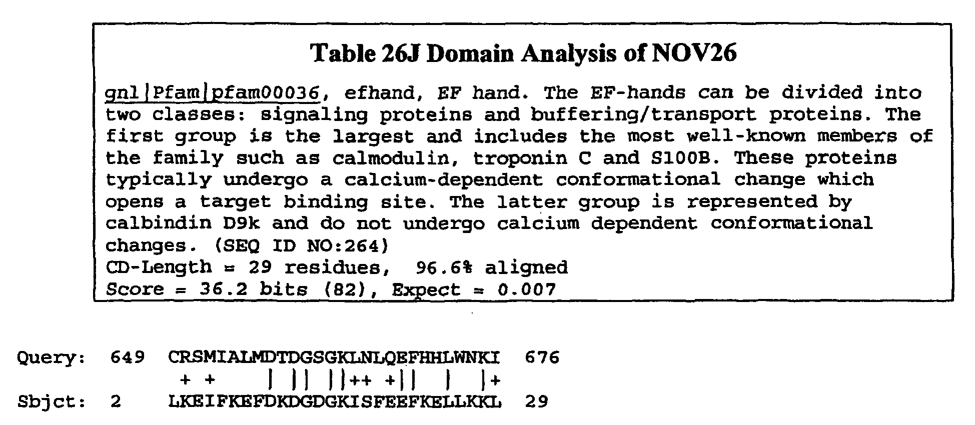

Calpains are intracellular cysteine proteases that are regulated by calcium. Cysteine protease activity is dependent on an active dyad of cysteine and histidine, the order and spacing of these residues varying in the 20 or so known families. Families C1, C2 and C10 are loosely termed papain-like, and nearly half of all cysteine proteases are found exclusively in viruses. Calpain is an intracellular protease involved in many important cellular functions that are regulated by calcium.

The protein is a complex of 2 polypeptide chains (light and heavy), with three known forms in mammals: a highly calcium-sensitive (i.e., micro-molar range) form known as mucalpain, mu-CANP or calpain I; a form sensitive to calcium in the milli-molar range, known as m-calpain, m-CANP or calpain II; and a third form, known as p94, which is found in skeletal muscle only. All three forms have identical light but different heavy chains. The heavy chain comprises four domains: domain 2 contains the catalytic region; domain 4 binds calcium and regulates activity. Domain 2 shows low levels of sequence similarity to papain; although the catalytic His has not been located by biochemical means, it is likely that calpain and papain are related. Domain 4 has four EF hand calcium-binding regions and is simmilar to sorcin and the Ca²⁺-binding region of calpain light chain. Calpain shows preferential cleavage for Tyr- with leucine or valine as the P2 residue.

The product of the Drosophila gene sol has also been shown to be similar to calpain. Many calcium-binding proteins belong to the same evolutionary family and share a type of calcium-binding domain known as the EF-hand. This type of domain consists of a twelve residue loop flanked on both side by a twelve residue alpha-helical domain. In an EF-hand loop the calcium ion is coordinated in a pentagonal bipyramidal configuration. The six residues involved in the binding are in positions 1, 3, 5, 7, 9 and 12; these residues are denoted by X, Y, Z, -Y, -X and -Z. The invariant Glu or Asp at position 12 provides two oxygens for liganding Ca (bidentate ligand).

Calpains are known to be involved in a number of cellular processes, such as apoptosis, protein processing, cell differentiation, metabolism etc. As such, their role in pathophysiologies extends to - but is not restricted to - tissue remodeling and regeneration (in response to a variety of injury models in the eye, brain, spinal cord, kidney etc.), fertility, tumorigenesis and myopathies. One of the genes identified in susceptibility to type II diabetes is a calpain (calpain-10). Polymorphisms within this gene are correlated with insulin resistance. Therapies targeting calpain are relevant to disease areas such as cataract, spinal cord injury, Alzheimer's disease, muscular dystrophy, acoustic trauma, diabetes, cancer, learning and memory defects and infertility. Knockout and transgenic models of various calpains also point to a potential role for this family of proteases in a number of cellular and disease processes.

The NOV26 nucleic acid of the invention encoding a Calpain-like protein includes the nucleic acid whose sequence is provided in Table 26A, or a fragment thereof. The invention also includes a mutant or variant nucleic acid any of whose bases may be changed from the corresponding base shown in Table 26A while still encoding a protein that maintains its Calpain-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivadzed. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1% of the NOV26 residues may be so changed.

The NOV26 protein of the invention includes the Calpain-like protein whose sequence is provided in Table 26B. The invention also includes a mutant or variant protein any of whose residues may be changed from the corresponding residue shown in Table 26B while still encoding a protein that maintains its Calpain-like activities and physiological functions, or a functional fragment thereof. In the mutant or variant protein, up to about 1% of the NOV26 bases may be so changed.

The NOV26 nucleic acids and proteins of the invention are useful in potential diagnostic and therapeutic applications implicated in various diseases and disorders described below and/or other pathologies. For example, the compositions of the present invention will have efficacy for treatment of patients suffering from: obesity, diabetes, autoimmune disease, Von Hippel-Lindau (VHL) syndrome, cirrhosis, transplantation disorders, pancreatitis, renal artery stenosis, interstitial nephritis, glomerulonephritis, polycystic kidney disease, systemic lupus erythematosus, renal tubular acidosis, IgA nephropathy, hypercalcemia, Lesch-Nyhan syndrome, developmental defects, cataract, spinal cord injury, Alzheimer's disease, muscular dystrophy, acoustic trauma, cancer, learning and memory defects, infertility and other diseases, disorders and conditions of the like.

NOV26 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV26 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV27

A disclosed NOV27 nucleic acid of 1327 nucleotides (also referred to as CG57484-01) encoding a novel Keratin 18-like protein is shown in Table 27A. An open reading frame was identified beginning with an ATG initiation codon at nucleotides 67-69 and ending with a TAA codon at nucleotides 1273-1275. Putative untranslated regions upstream from the intitation codon and downstream from the termination codon are underlined in Table 27A, and the start and stop codons are in bold letters.

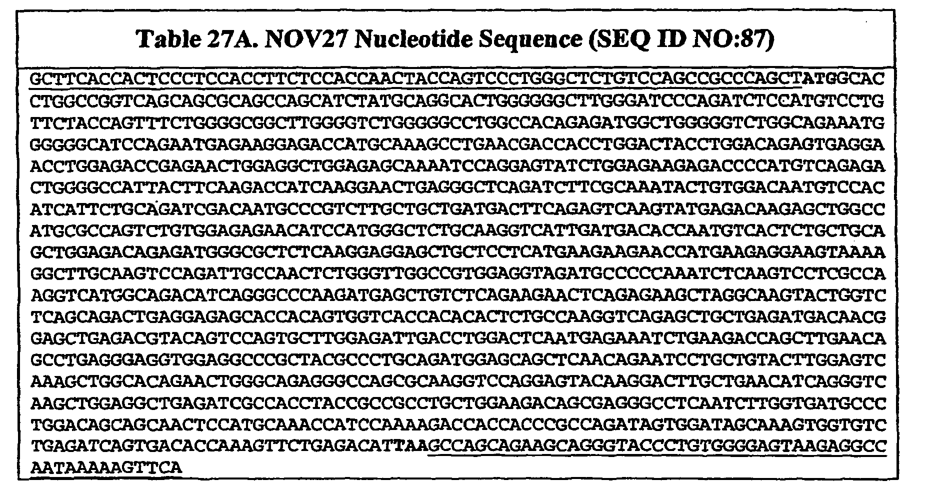

The NOV27 nucleic acid was identified on chromosome 12 and has 367 of 415 bases (88%) identical to a gb:GENBANK-ID:AF179904|acc:AF179904.1 mRNA from *Homo sapiens (Homo sapiens* keratin 18 (KRT18) gene, complete cds) (E = 5.1e⁻¹⁸⁰).

A disclosed NOV27 polypeptide (SEQ ID NO:88) encoded by SEQ ID NO:87 is 402 amino acid residues and is presented using the one-letter code in Table 27B. Signal P, Psort and/or Hydropathy results predict that NOV27 contains a signal peptide and is likely to be localized to the cytoplasm with a certainty of 0.4500.

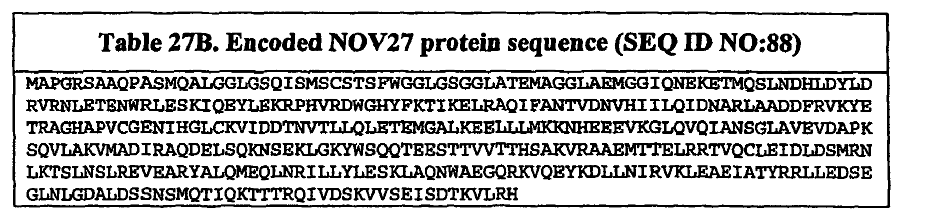

The NOV27 amino acid sequence 315 of 404 amino acid residues (77%) identical to, and 344 of 404 amino acid residues (85%) similar to, the 429 amino acid residue ptnr:SWISSNEW-ACC:P05783 protein from *Homo sapiens* (Human) (Keratin, Type I Cytoskeletal 18 (Cytokeratin 18) (K18) (CK 18)) (E = 2.9e⁻¹⁵¹).

NOV27 is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, genomic clone sources, literature sources, and/or RACE sources.

NOV27 has homology to the amino acid sequences shown in the BLASTP data listed in Table 27C.

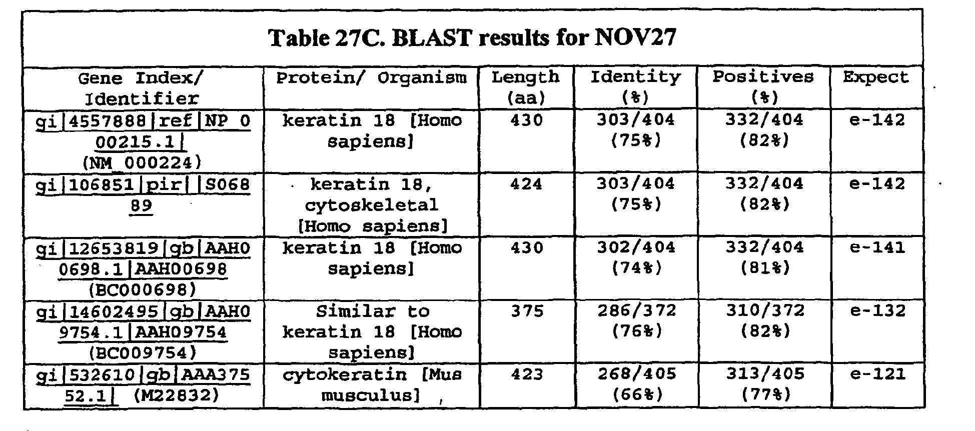

The homology of these sequences is shown graphically in the ClustalW analysis shown in Table 27D.

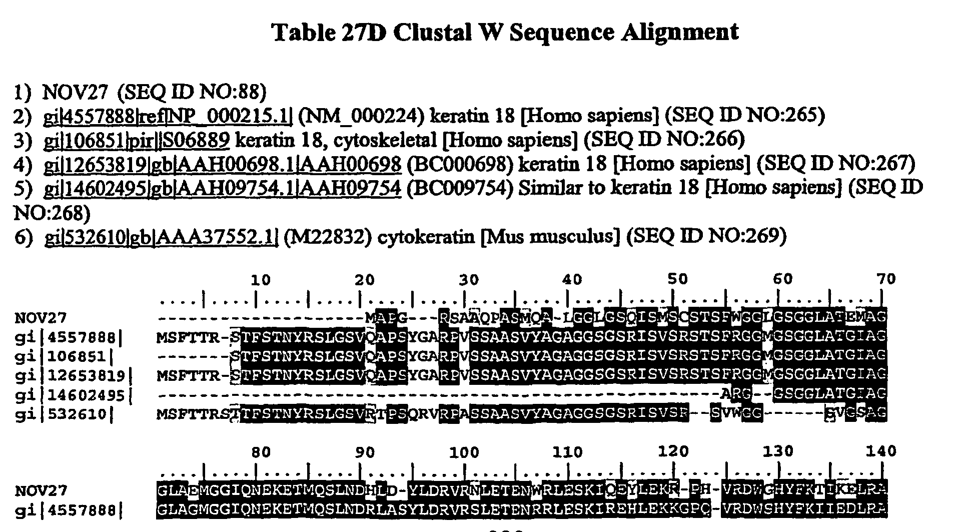

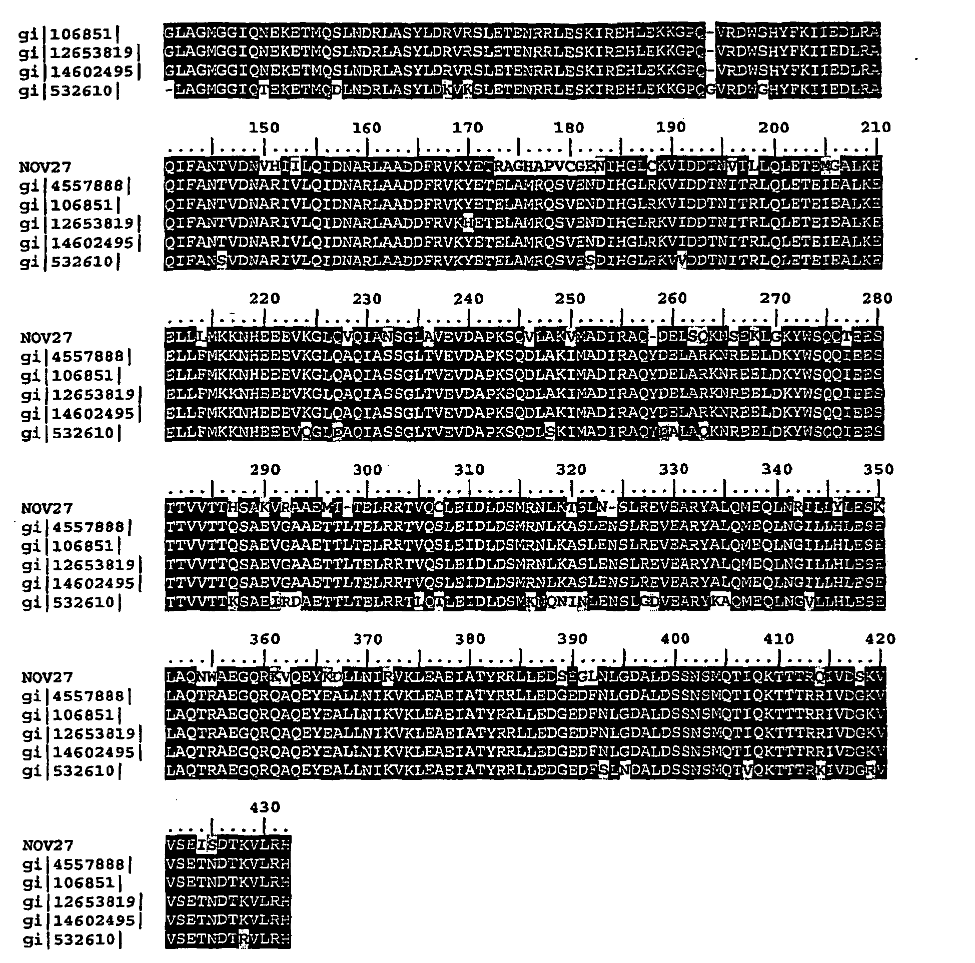

Table 27E lists the domain description from DOMAIN analysis results against NOV27. This indicates that the NOV27 sequence has properties similar to those of other proteins known to contain these domains.

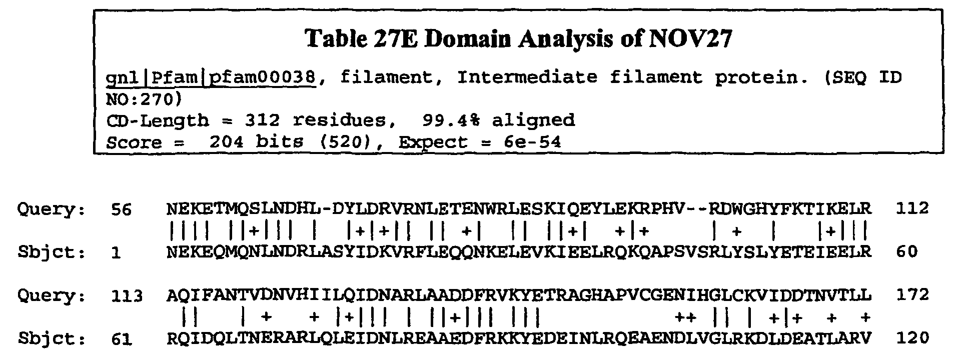

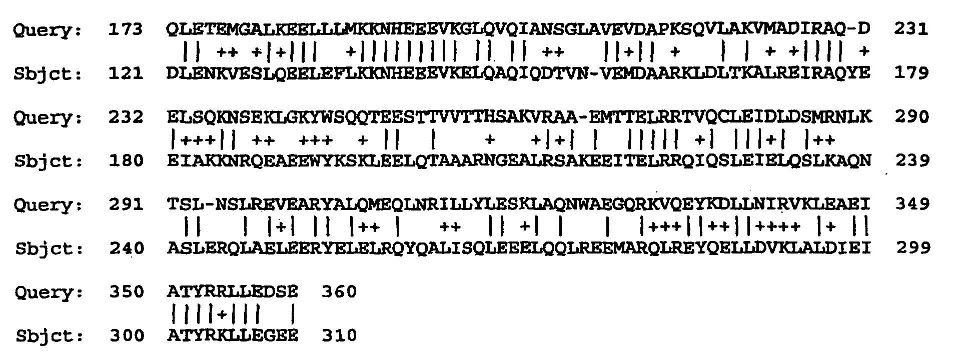

Keratin 8 (K8) and keratin 18 (K18) are the most common and characteristic members of the large intermediate filament gene family expressed in 'simple' or single layer epithelial tissues of the body. Their persistent expression in tumor cells derived from these epithelia has led to the wide spread use of keratin monoclonal antibodies as aids in the detection and identification of carcinomas. Oncogenes which activate ras signal transduction pathways stimulate expression of the K18 gene through transcription factors including members of the AP-1 (jun and fos) and ETS families. The persistent expression of K8 and K18 may reflect the integrated transcriptional activation of such transcription factors and, in the cases of ectopic expression, an escape from the suppressive epigenetic mechanisms of DNA methylation and chromatin condensation. Comparison of the mechanisms of transcriptional control of K18 expression with expression patterns documented in both normal and pathological conditions leads to the proposal that persistent K8 and K18 expression is a reflection of the action of multiple different oncogenes converging on the nucleus through a limited number of transcription factors to then influence the expression of a large number of genes including these keratins. Furthermore, correlation of various tumor cell characteristics including invasive behavior and drug sensitivity with K8 and K18 expression has stimulated consideration of the possible functions of these proteins in both normal development and in tumorigenesis. Recent developments in the analysis of the functions of these intermediate filament proteins provide new insights into diverse functions influenced by K8 and K18 (Oshima et al., Cancer Metastasis Rev 15:445-71, 1996).

Keratin 8 (K8) and keratin 18 (K18) form intermediate filaments characteristic of liver and other single cell layered, internal epithelia and their derivative cancers. K8-deficient (K8(-)) mice, which escape embryonic lethality, develop inflammatory colorectal hyperplasia, mild liver abnormalities, and tolerate hepatectomy poorly. Normal and malignant epithelial cells deficient in K8 and K18 have been shown to be approximately 100 times more sensitive to TNF-induced death. K8 and K18 both bind the cytoplasmic domain of TNFR2 and moderate TNF-induced, Jun NH(2)-terminal kinase (JNK) intracellular signaling and NFkappaB activation. Furthermore, K8(-) and K18(-) mice are much more sensitive to TNF dependent, apoptotic liver damage induced by the injection of concanavalin A. This moderation of the effects of TNF may be the fundamental function of K8 and K18 common to liver regeneration, inflammatory bowel disease, hepatotoxin sensitivity, and the diagnostic, persistent expression of these keratins in many carcinomas (Caulin et al., J Cell Biol 149:17-22, 2000).

The NOV27 nucleic acid of the invention encoding a Keratin 18-like protein includes the nucleic acid whose sequence is provided in Table 27A, or a fragment thereof. The invention also includes a mutant or variant nucleic acid any of whose bases may be changed from the corresponding base shown in Table 27A while still encoding a protein that maintains its Keratin 18-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 12% of the NOV27 residues may be so changed.

The NOV27 protein of the invention includes the Keratin 18-like protein whose sequence is provided in Table 27B. The invention also includes a mutant or variant protein any of whose residues may be changed from the corresponding residue shown in Table 27B while still encoding a protein that maintains its Keratin 18-like activities and physiological functions, or a functional fragment thereof. In the mutant or variant protein, up to about 23% of the NOV27 bases may be so changed.

The NOV27 nucleic acids and proteins of the invention are useful in potential diagnostic and therapeutic applications implicated in various diseases and disorders described below and/or other pathologies. For example, the compositions of the present invention will have efficacy for treatment of patients suffering from: inflammatory colorectal hyperplasia, mild liver abnormalities, skin abnormalities, inflammatory bowel disease, hepatotoxin sensitivity, chronic hepatitis, breast cancer, pancreatic cancer, mammary gland tumours, benign prostatic hyperplasia, prostate cancer, cryptogenic cirrhosis and other diseases, disorders and conditions of the like.

NOV27 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV27 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV28

A disclosed NOV28 nucleic acid of 12992 nucleotides (also referred to as CG57236-01) encoding a novel Polycystic Kidney Disease Associated-like protein is shown in Table 28A. An open reading frame was identified beginning with an ATG initiation codon at nucleotides 53-55 and ending with a TAG codon at nucleotides 12950-12952. Putative untranslated regions upstream from the intitation codon and downstream from the termination codon are underlined in Table 28A, and the start and stop codons are in bold letters.

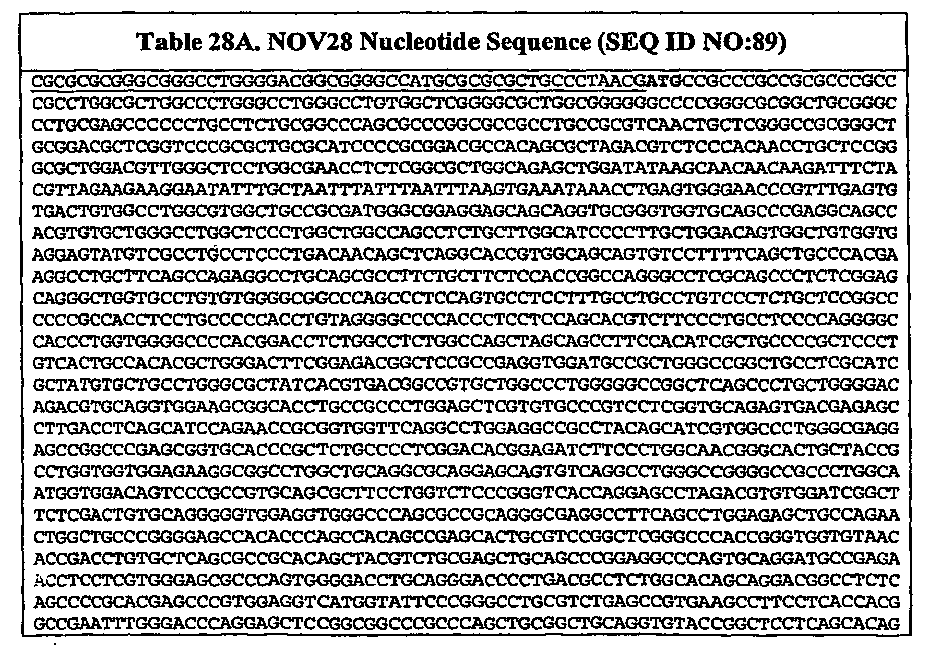

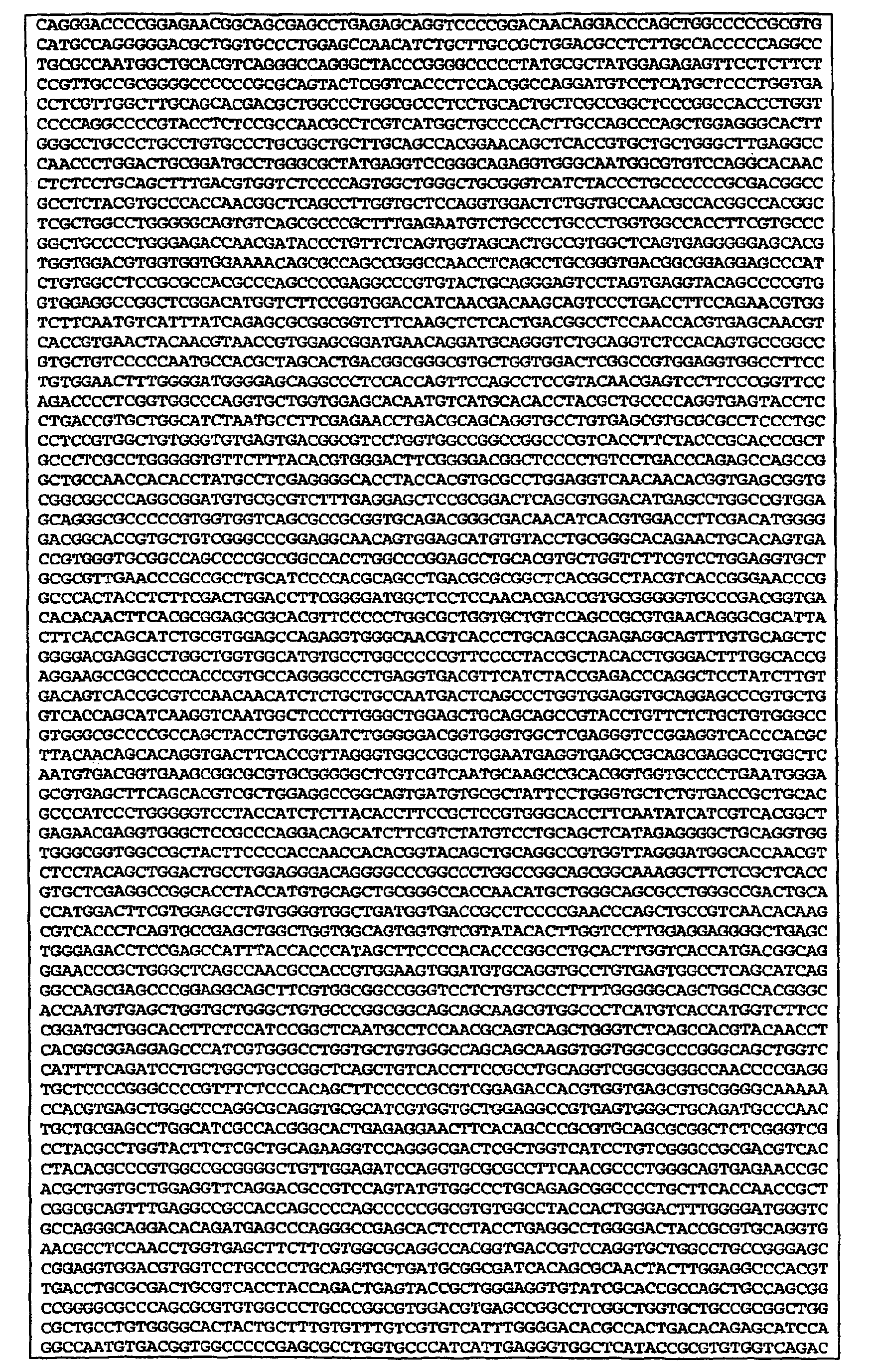

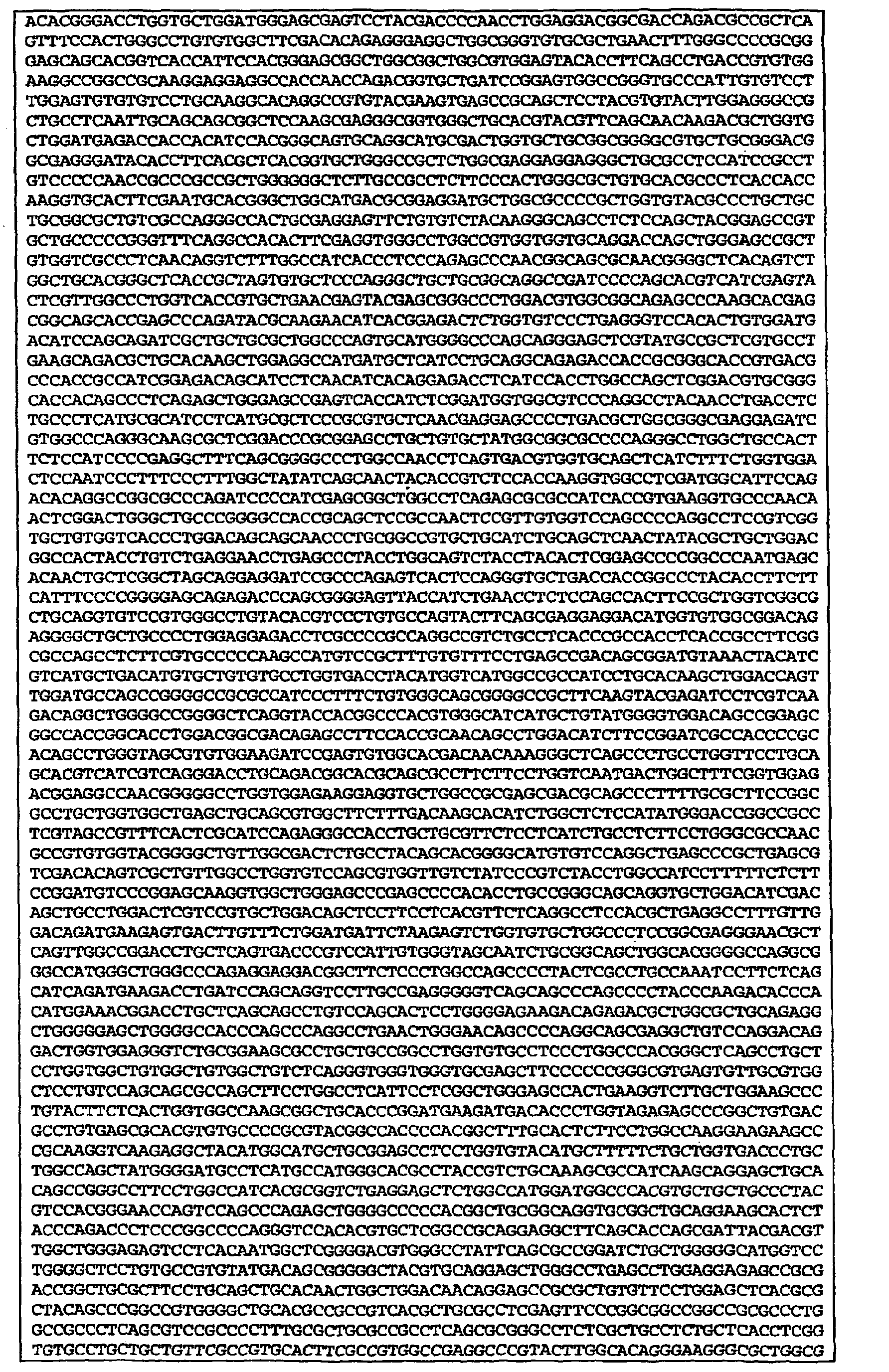

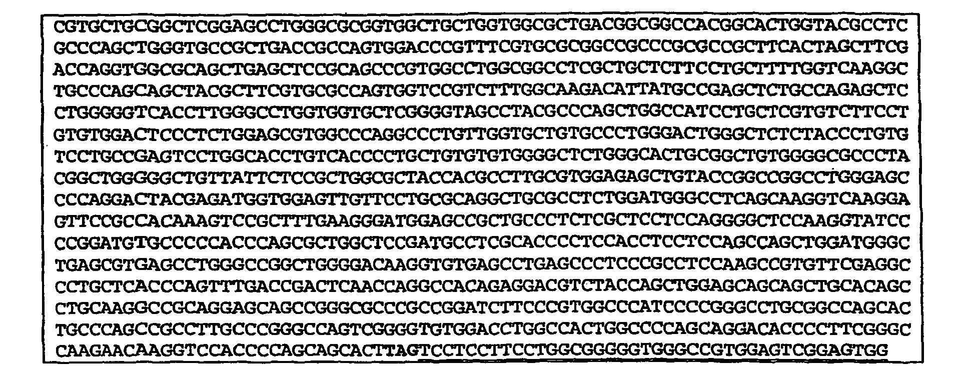

The NOV28 nucleic acid was identified on chromosome 16p13.3 and has 8732 of 8732 bases (100%) identical to a gb:GENBANK-ID:HUMPKD1A|acc:L33243.1 mRNA from *Homo sapiens* (*Homo sapiens* polycystic kidney disease 1 protein (PKD1) mRNA, complete cds) (E = 0.0).

A disclosed NOV28 polypeptide (SEQ ID NO:90) encoded by SEQ ID NO:89 is 4299 amino acid residues and is presented using the one-letter code in Table 28B. Signal P, Psort and/or Hydropathy results predict that NOV28 contains a signal peptide and is likely to be localized to the plasma membrane with a certainty of 0.8200. The most likely cleaveage site for a NOV28 polypeptide is between amino acids 23 and 24: ALA-GG.

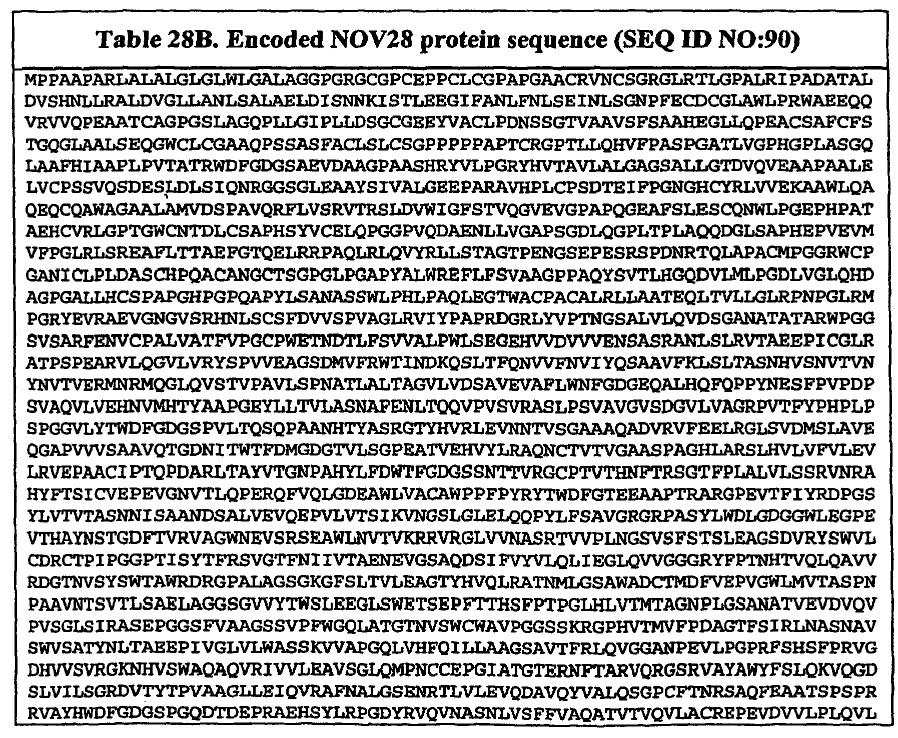

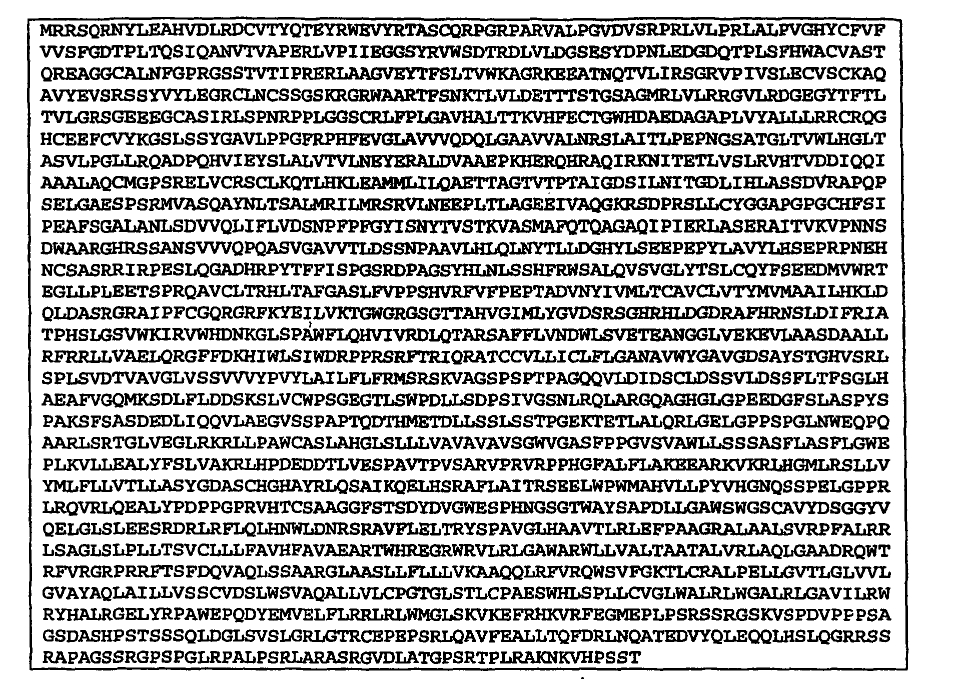

The NOV28 amino acid sequence 4298 of 4302 amino acid residues (99%) identical to, and 4298 of 4302 amino acid residues (99%) similar to, the 4302 amino acid residue ptnr:SPTREMBL-ACC:Q15140 protein from *Homo sapiens* (Human) (Polycystic Kidney Disease 1 Protein) (E = 0.0).

NOV28 is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, genomic clone sources, literature sources, and/or RACE sources. NOV28 is also predicted to be expressed in the following tissues because of the expression pattern of (gb:GBNBANK-ID:HUMPKD1A|acc:L33243.1) a closely related *Homo sapiens* polycystic kidney disease 1 protein (PKD1) mRNA, complete cds homolog in species *Homo sapiens*: kidney.

NOV28 has homology to the amino acid sequences shown in the BLASTP data listed in Table 28C.

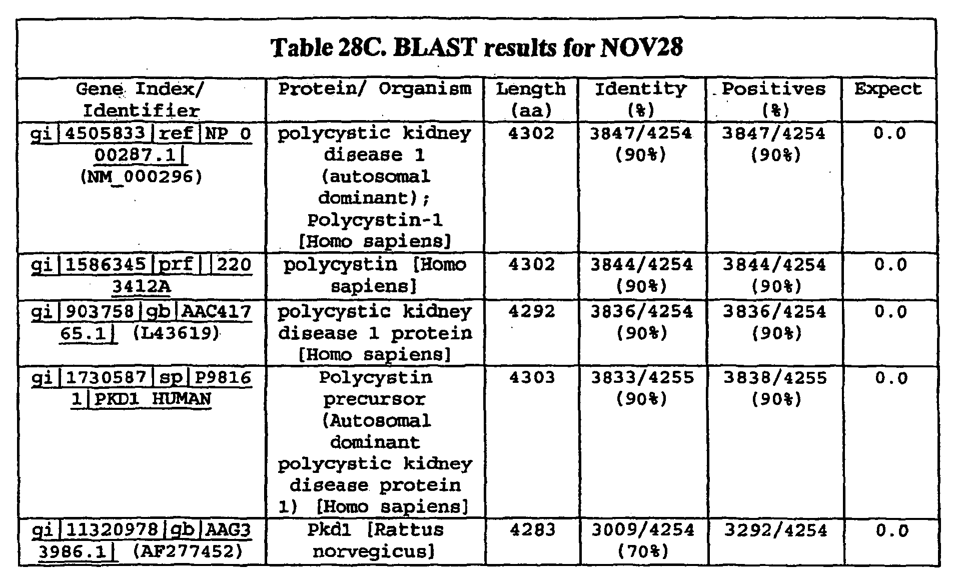

The homology of these sequences is shown graphically in the ClustalW analysis shown in Table 28D.

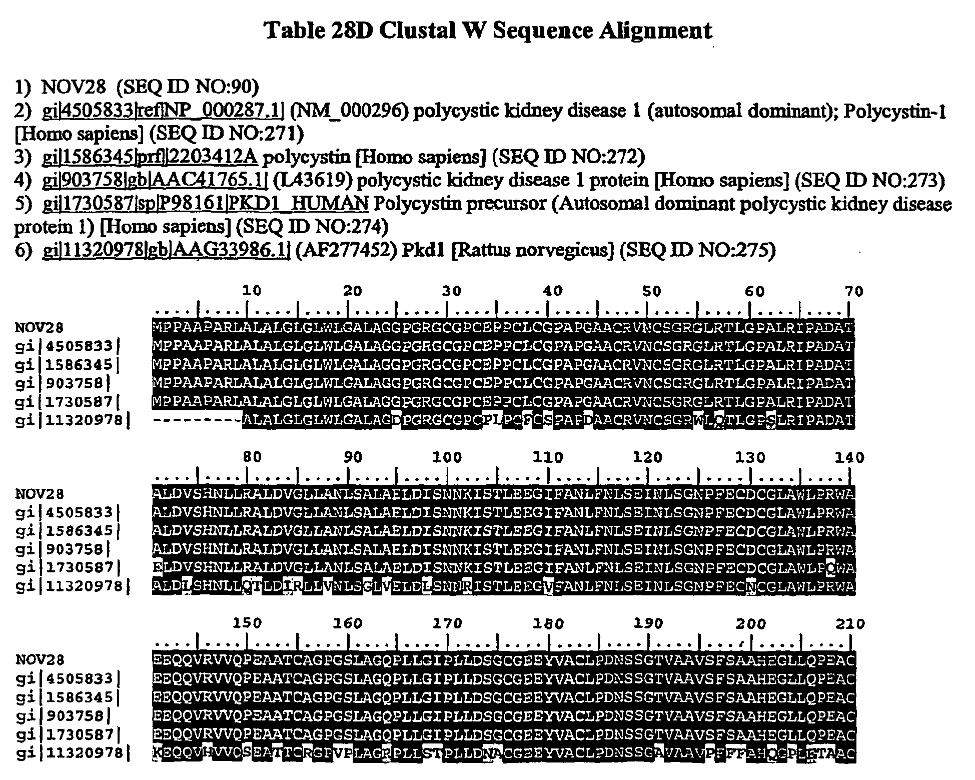

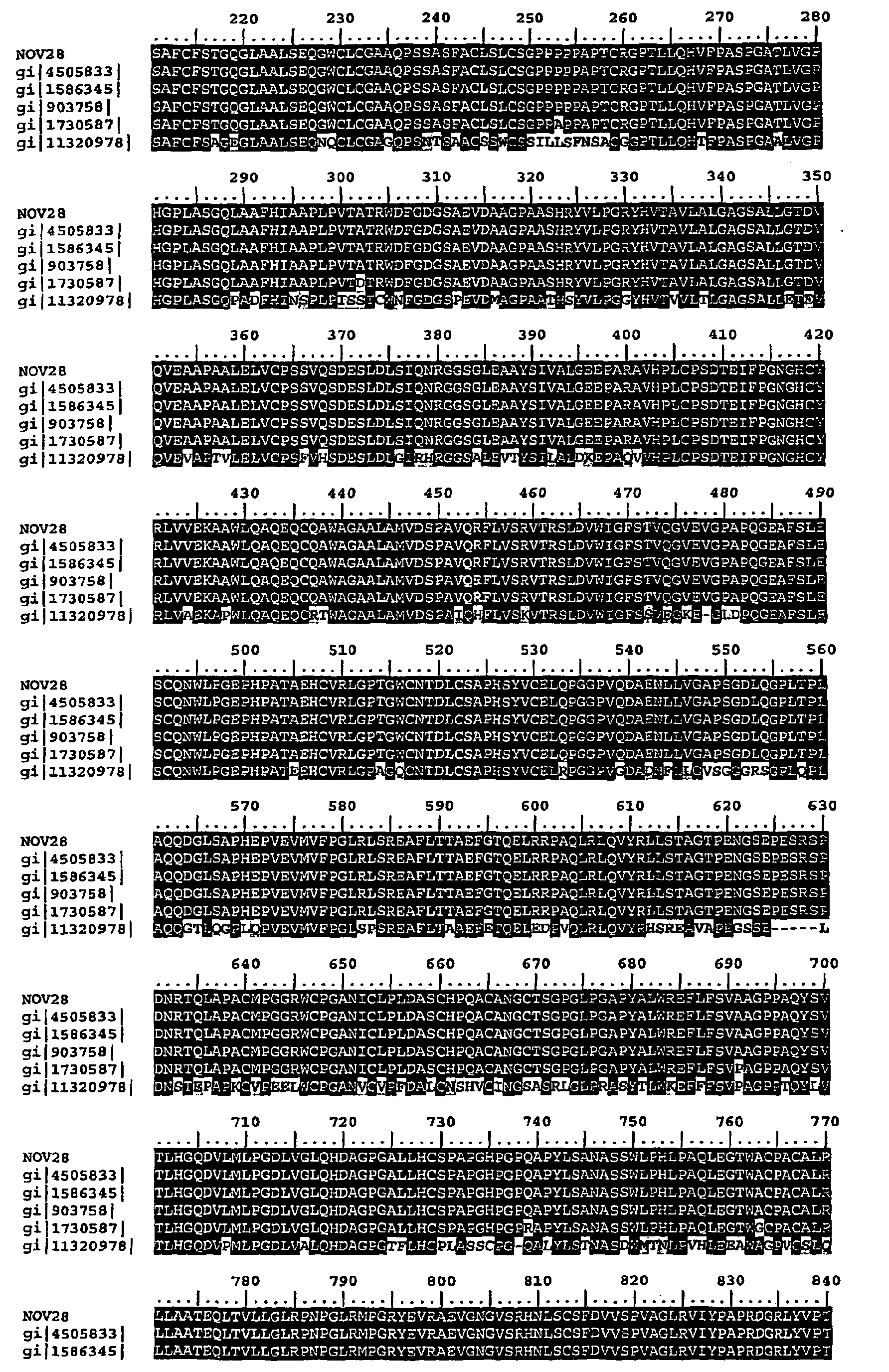

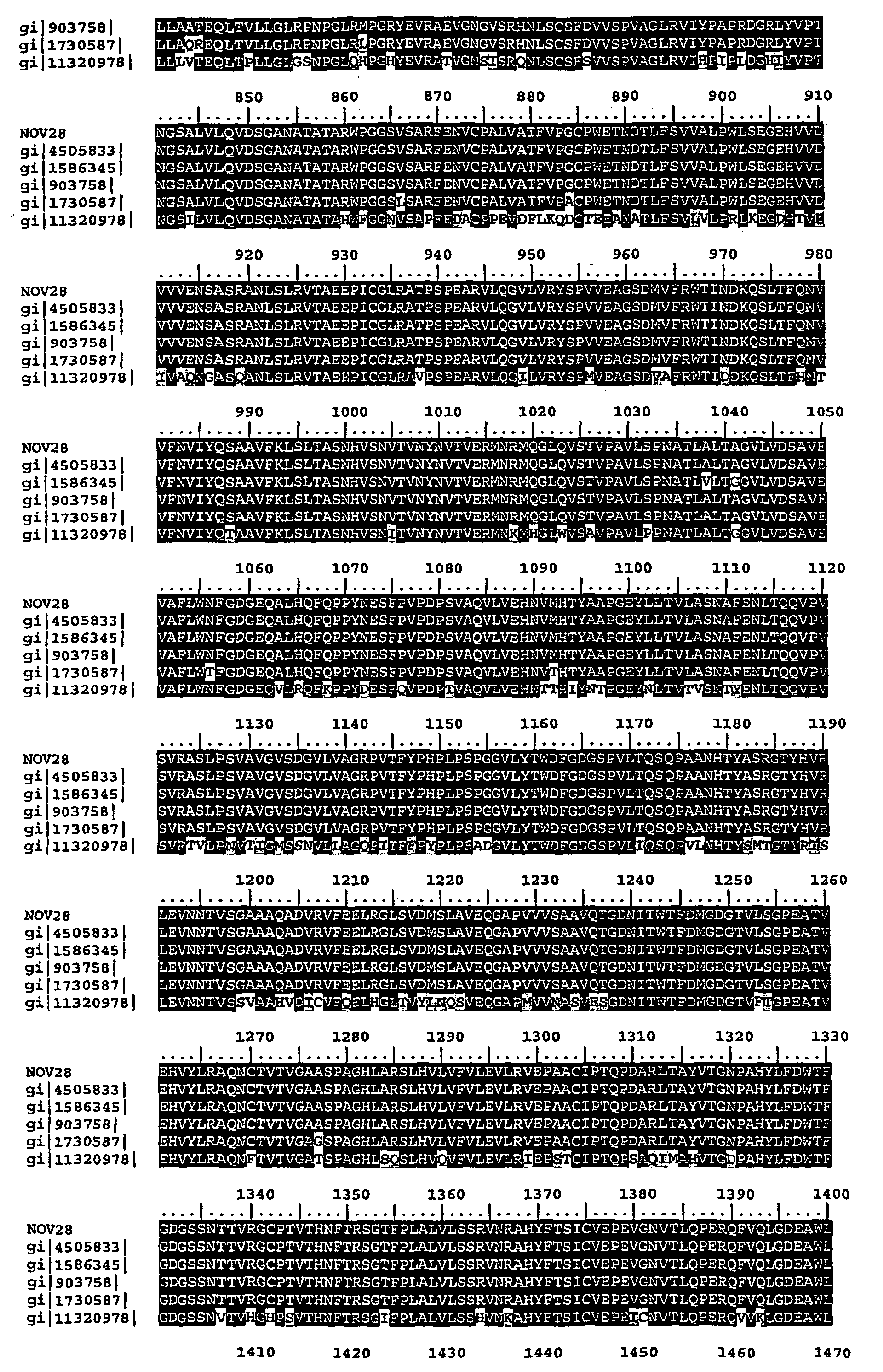

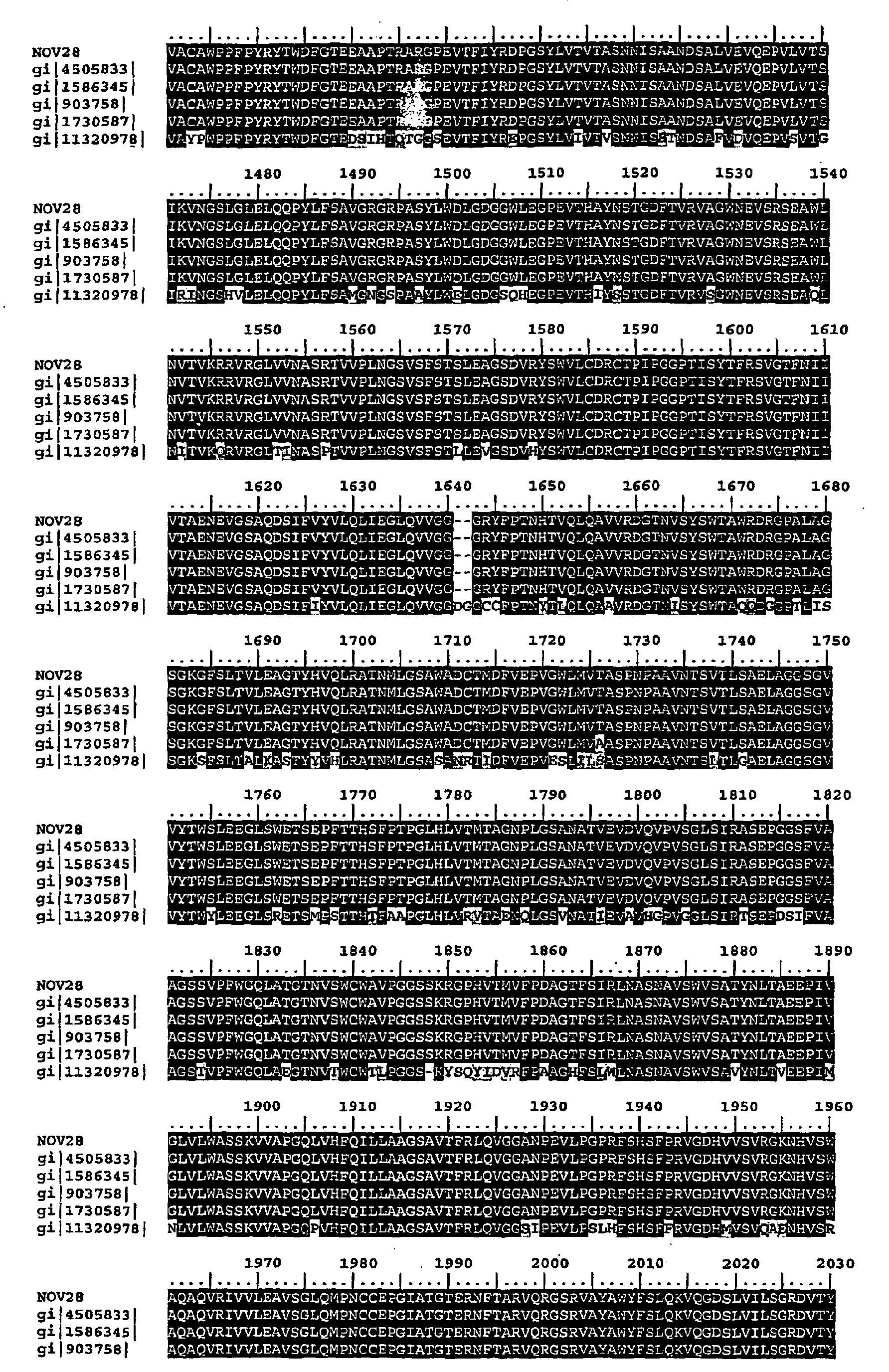

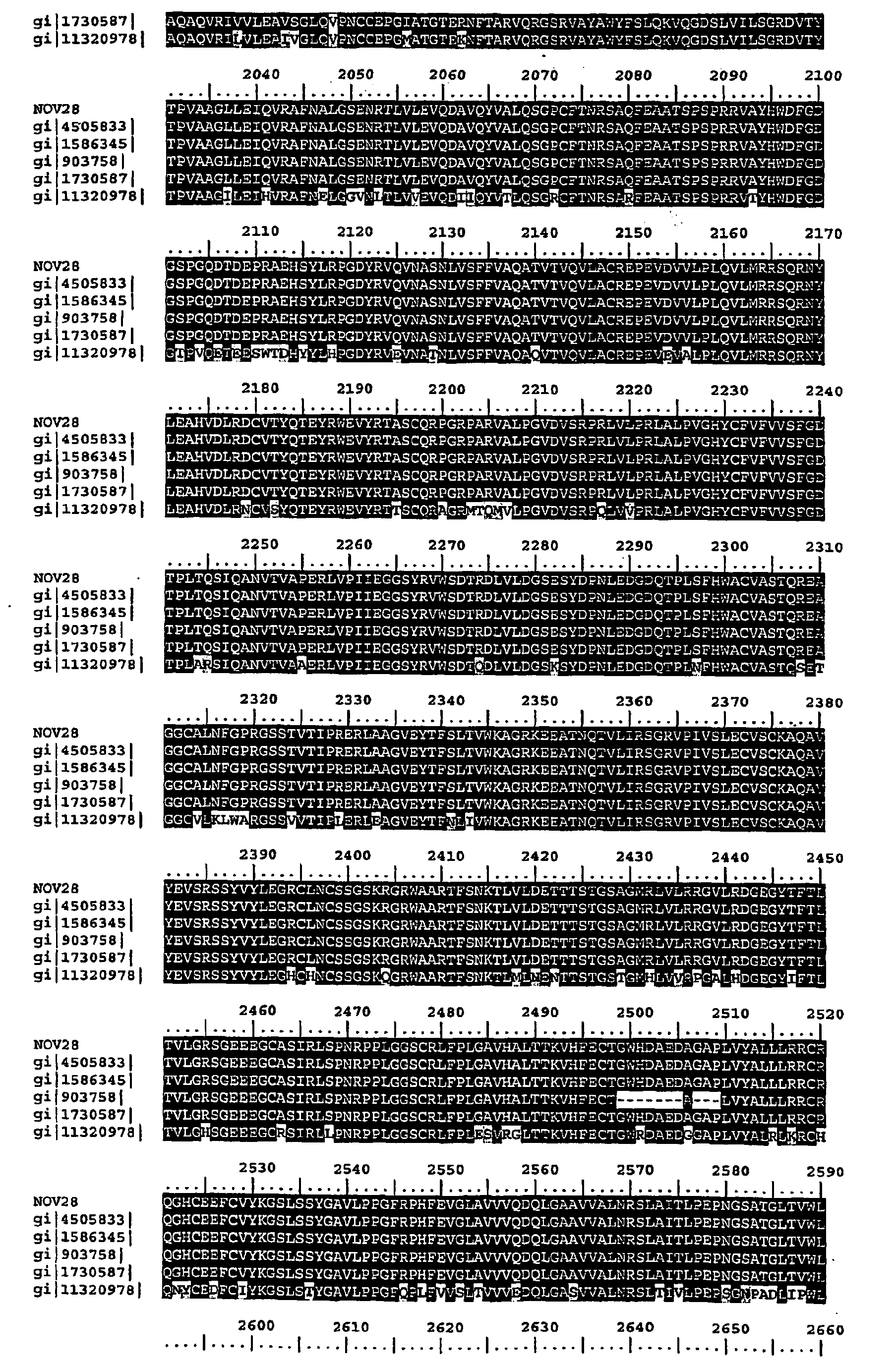

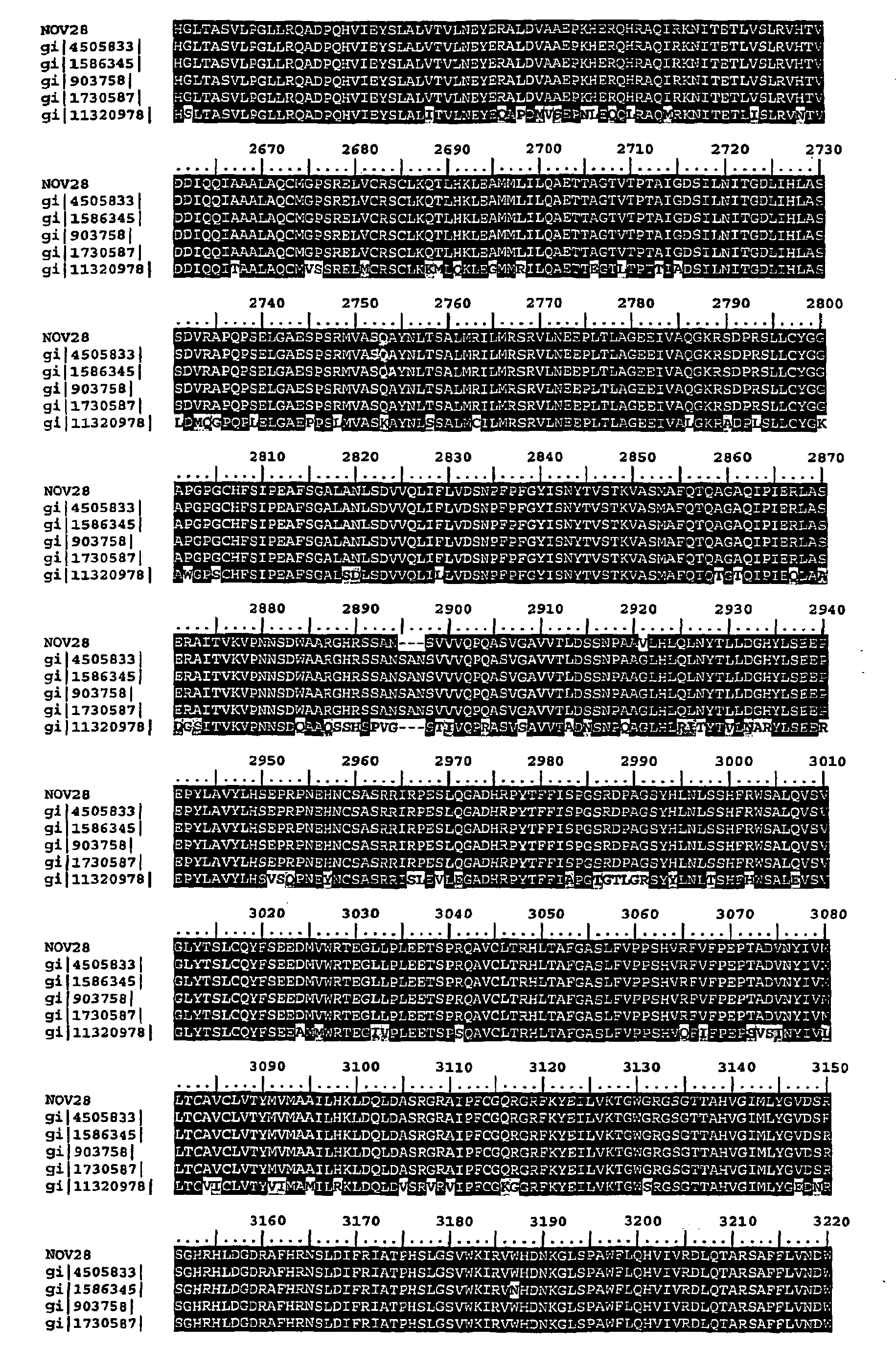

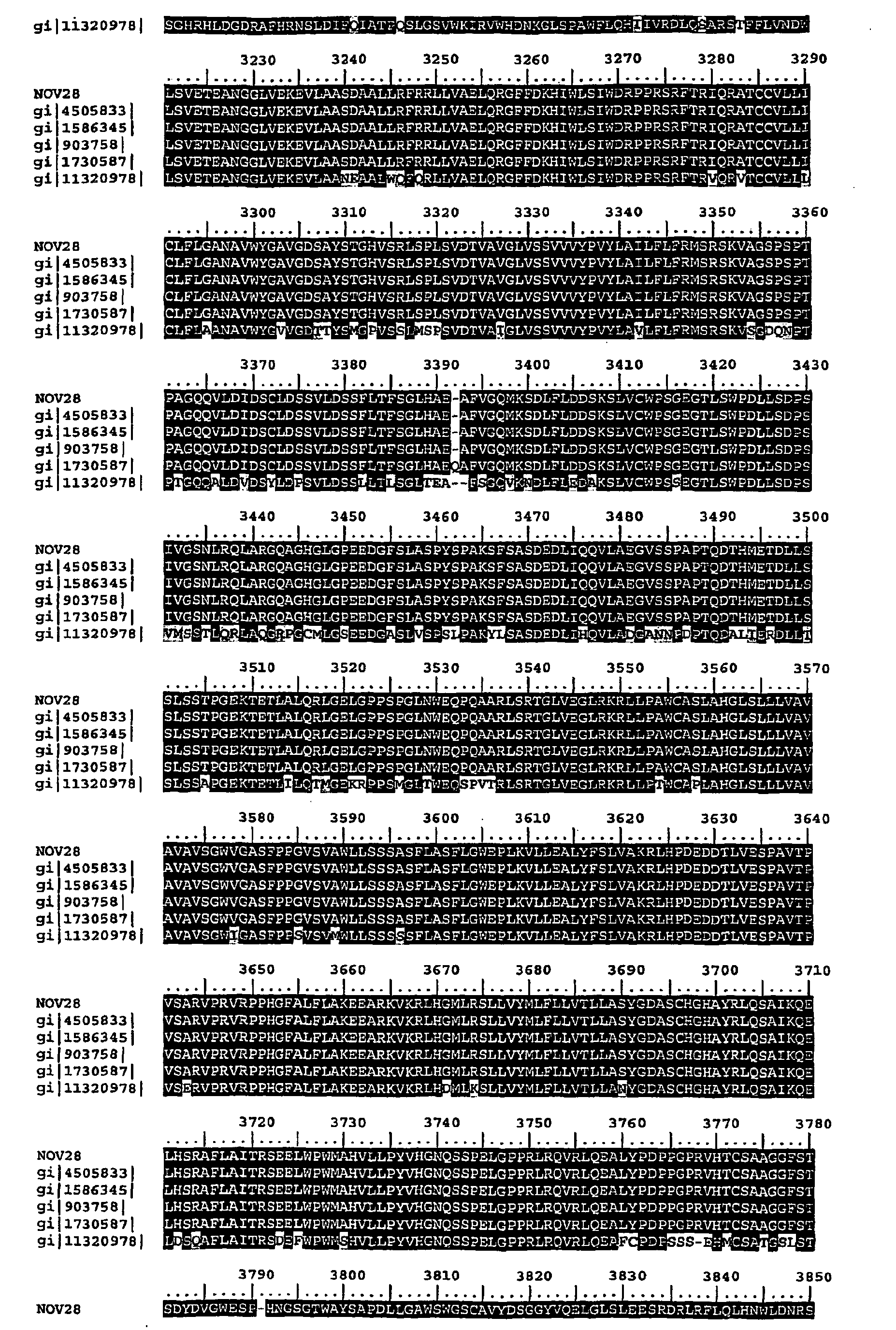

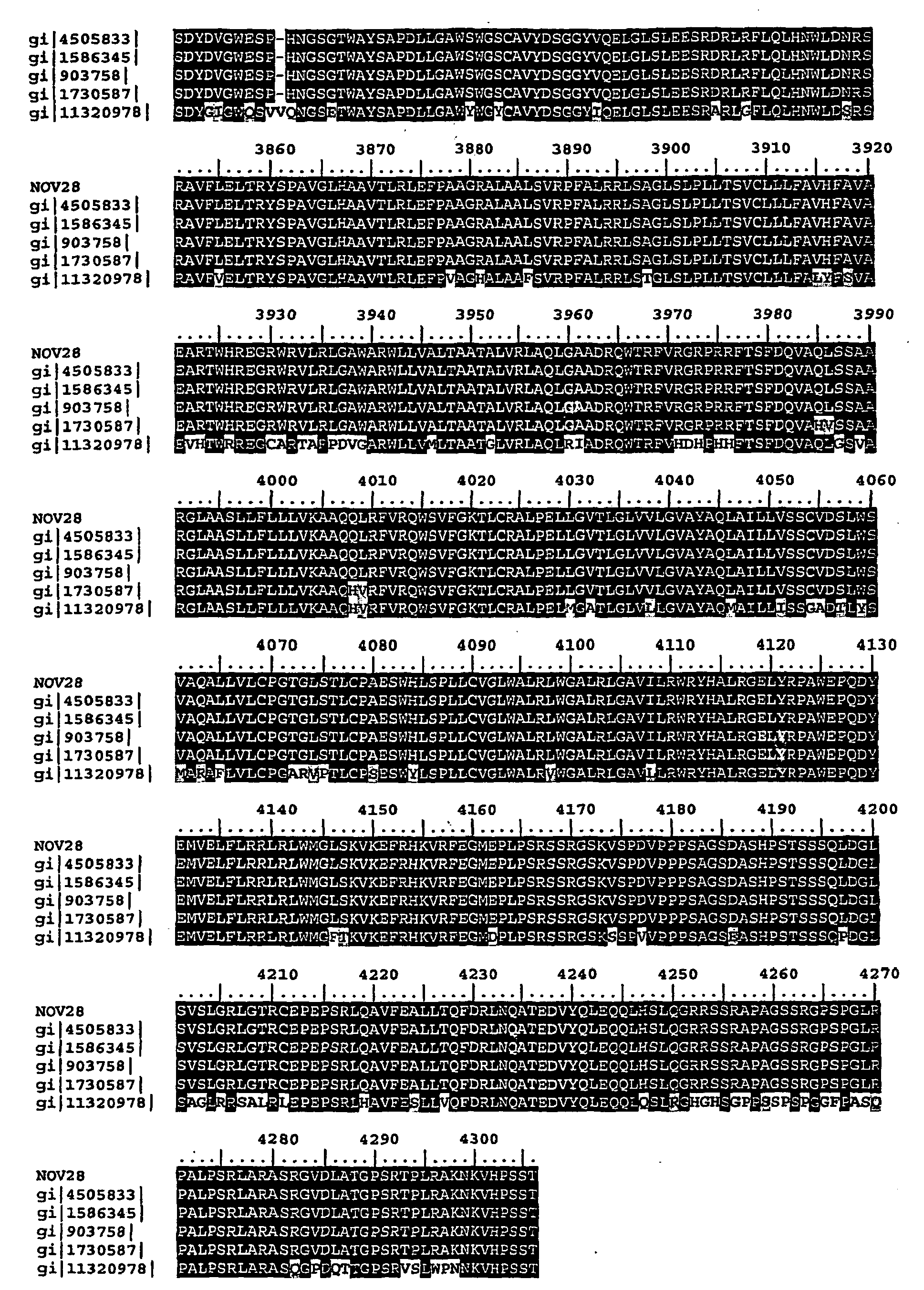

Tables 28E, 28F, 28G and 28H list the domain description from DOMAIN analysis results against NOV28. This indicates that the NOV28 sequence has properties similar to those of other proteins known to contain these domains.

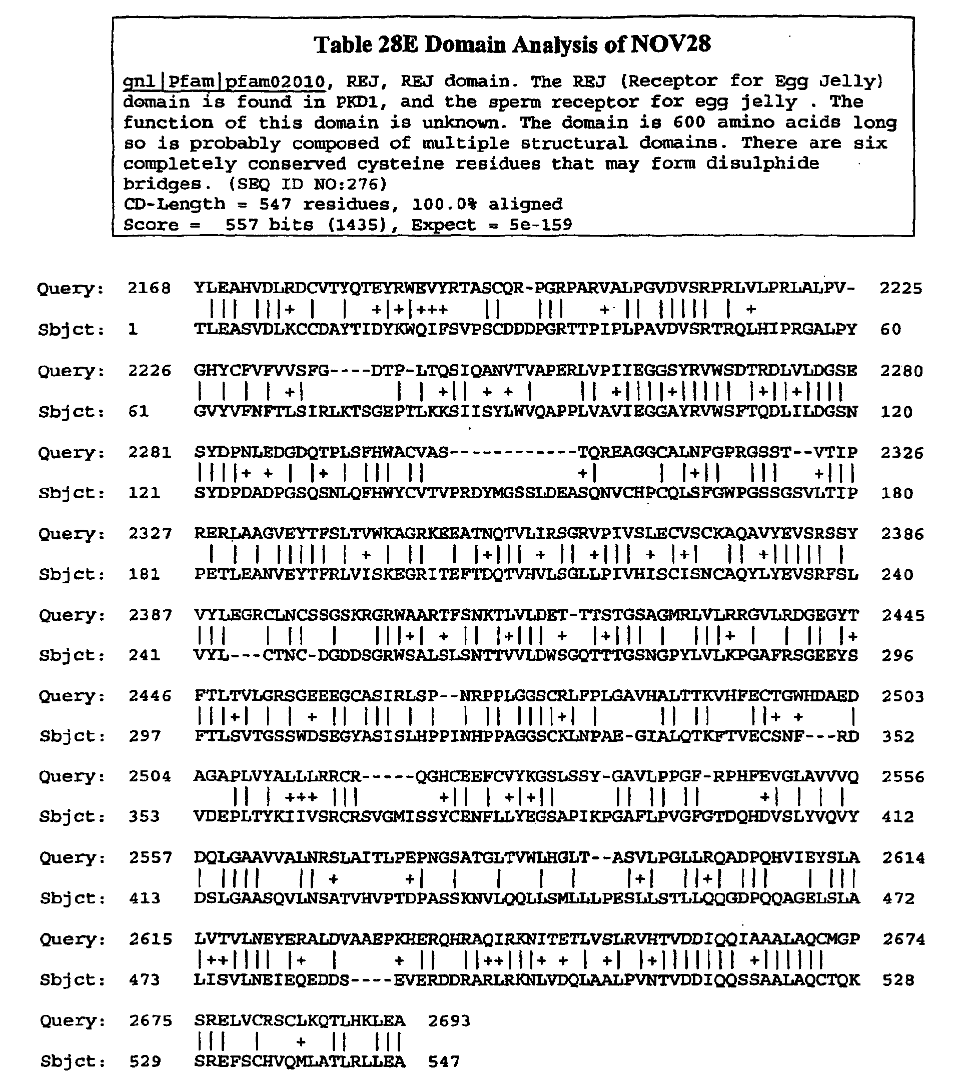

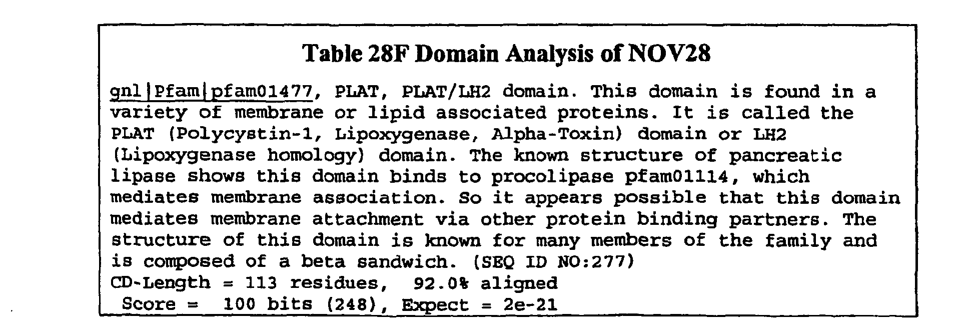

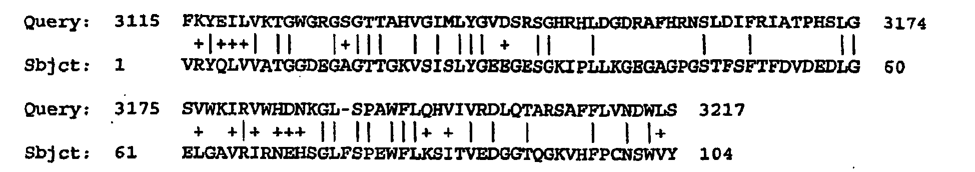

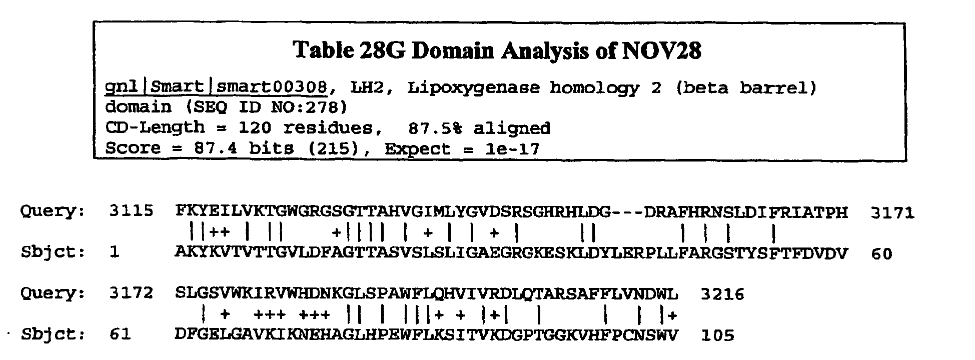

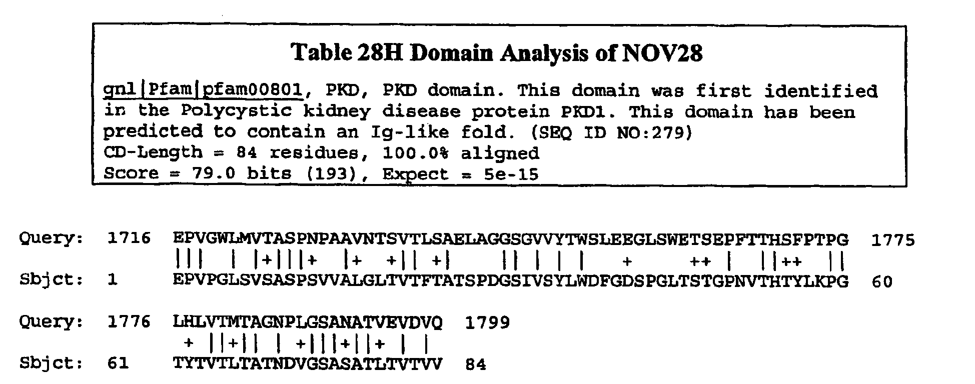

Polycystic Kidney Disease Associated 8 (K8) and keratin 18 (K18) are the most common and characteristic members of the large intermediate filament gene family expressed in 'simple' or single layer epithelial tissues of the body. Their persistent expression in tumor cells derived from these epithelia has led to the wide spread use of keratin monoclonal antibodies as aids in the detection and identification of carcinomas. Oncogenes which activate ras signal transduction pathways stimulate expression of the K18 gene through transcription factors including members of the AP-1 (jun and fos) and ETS families. The persistent expression of K8 and K18 may reflect the integrated transcriptional activation of such transcription factors and, in the cases of ectopic expression, an escape from the suppressive epigenetic mechanisms of DNA methylation and chromatin condensation. Comparison of the mechanisms of transcriptional control of K18 expression with expression patterns documented in both normal and pathological conditions leads to the proposal that persistent K8 and K18 expression is a reflection of the action of multiple different oncogenes converging on the nucleus through a limited number of transcription factors to then influence the expression of a large number of genes including these keratins. Furthermore, correlation of various tumor cell characteristics including invasive behavior and drug sensitivity with K8 and K18 expression has stimulated consideration of the possible functions of these proteins in both normal development and in tumorigenesis. Recent developments in the analysis of the functions of these intermediate filament proteins provide new insights into diverse functions influenced by K8 and K18 (Oshima et al., Cancer Metastasis Rev 15:445-71, 1996).

Polycystic Kidney Disease Associated 8 (K8) and keratin 18 (K18) form intermediate filaments characteristic of liver and other single cell layered, internal epithelia and their derivative cancers. K8-deficient (K8(-)) mice, which escape embryonic lethality, develop inflammatory colorectal hyperplasia, mild liver abnormalities, and tolerate hepatectomy poorly. Normal and malignant epithelial cells deficient in K8 and K18 have been shown to be approximately 100 times more sensitive to TNF-induced death. K8 and K18 both bind the cytoplasmic domain of TNFR2 and moderate TNF-induced, Jun NH(2)-terminal kinase (JNK) intracellular signaling and NFkappaB activation. Furthermore, K8(-) and K18(-) mice are much more sensitive to TNF dependent, apoptotic liver damage induced by the injection of concanavalin A. This moderation of the effects of TNF may be the fundamental function of K8 and K18 common to liver regeneration, inflammatory bowel disease, hepatotoxin sensitivity, and the diagnostic, persistent expression of these keratins in many carcinomas (Caulin et al., J Cell Biol 149:17-22, 2000).

The NOV28 nucleic acid of the invention encoding a Polycystic Kidney Disease Associated-like protein includes the nucleic acid whose sequence is provided in Table 28A, or a fragment thereof. The invention also includes a mutant or variant nucleic acid any of whose bases may be changed from the corresponding base shown in Table 28A while still encoding a protein that maintains its Polycystic Kidney Disease Associated-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1% of the NOV28 residues may be so changed.

The NOV28 protein of the invention includes the Polycystic Kidney Disease Associated-like protein whose sequence is provided in Table 28B. The invention also includes a mutant or variant protein any of whose residues may be changed from the corresponding residue shown in Table 28B while still encoding a protein that maintains its Polycystic Kidney Disease Associated-like activities and physiological functions, or a functional fragment thereof. In the mutant or variant protein, up to about 1% of the NOV28 bases may be so changed.

The NOV28 nucleic acids and proteins of the invention are useful in potential diagnostic and therapeutic applications implicated in various diseases and disorders described below and/or other pathologies. For example, the compositions of the present invention will have efficacy for treatment of patients suffering from: polycystic kidney disease and renal carcinoma and other diseases, disorders and conditions of the like.

NOV28 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV28 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV29

A disclosed NOV29 nucleic acid of 1222 nucleotides (also referred to as CG57589-01) encoding a novel Cholinephosphate Cytidylyltransferase-like protein is shown in Table 29A. An open reading frame was identified beginning with an ATG initiation codon at nucleotides 67-69 and ending with a TAA codon at nucleotides 1156-1158. Putative untranslated regions upstream from the intitation codon and downstream from the termination codon are underlined in Table 29A, and the start and stop codons are in bold letters.

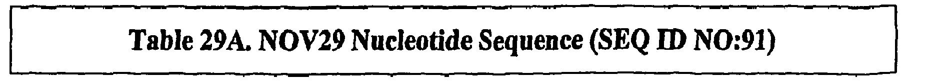

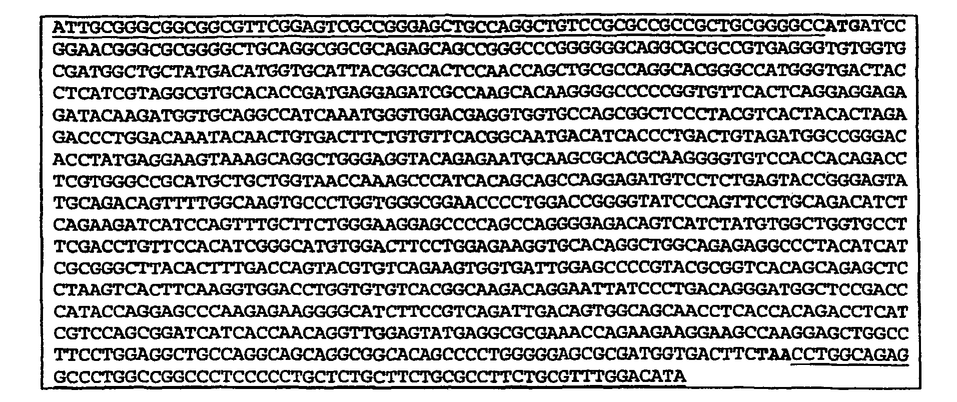

The NOV29 nucleic acid was identified on chromosome 17 and has 828 of 830 bases (99%) identical to a gb:GENBANK-ID:D84307|acc:D84307.1 mRNA from *Homo sapiens* (mRNA for phosphoethanolamine cytidylyltransferase, complete cds) (E = 6.4e⁻¹⁸¹).

A disclosed NOV29 polypeptide (SEQ ID NO:92) encoded by SEQ ID NO:91 is 363 amino acid residues and is presented using the one-letter code in Table 29B. Signal P, Psort and/or Hydropathy results predict that NOV29 contains a signal peptide and is likely to be localized to the nucleus with a certainty of 0.3000.

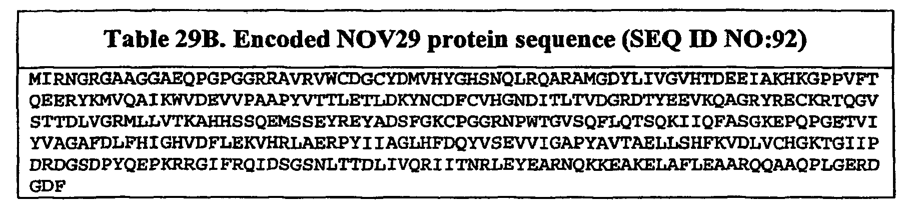

The NOV29 amino acid sequence 254 of 256 amino acid residues (99%) identical to, and 255 of 256 amino acid residues (99%) similar to, the 389 amino acid residue ptnr:SWISSNEW-ACC:Q99447 protein from *Homo sapiens* (Human) (Ethanolamane-Phosphate Cytidylyltransferase (EC 2.7.7.14) (Phosphorylethanolamine Transferase) (CTP:Phosphoethanolamine Cytidylyltransferase)) (E = 2.3e⁻¹⁹⁶).

NOV29 is expressed in at least the following tissues: adrenal gland/suprarenal gland, bone marrow, brain, bronchus, coronary artery, dermis, epidermis, heart, hypothalamus, kidney, left cerebellum, liver, lung pleura, lymphoid tissue, mammary gland/breast, ovary, pancreas, peripheral blood, pituitary gland, placenta, prostate, skeletal muscle, spinal chord, spleen, stomach, substantia nigra, testis, thalamus, thymus, thyroid, uterus and whole organism. This information was derived by determining the tissue sources of the sequences that were included in the invention including but not limited to SeqCalling sources, Public EST sources, genomic clone sources, literature sources, and/or RACE sources.

NOV29 has homology to the amino acid sequences shown in the BLASTP data listed in Table 29C.

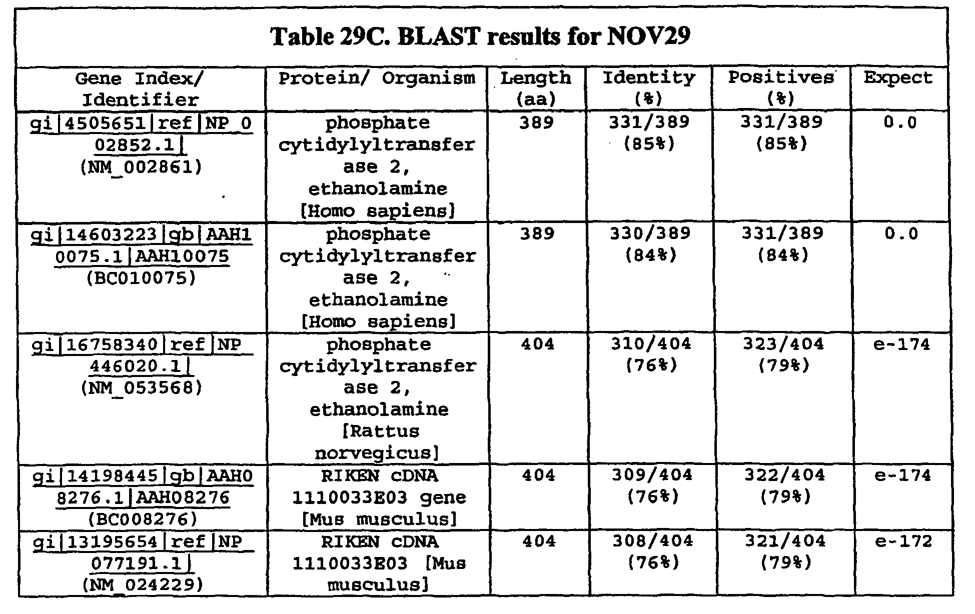

The homology of these sequences is shown graphically in the ClustalW analysis shown in Table 29D.

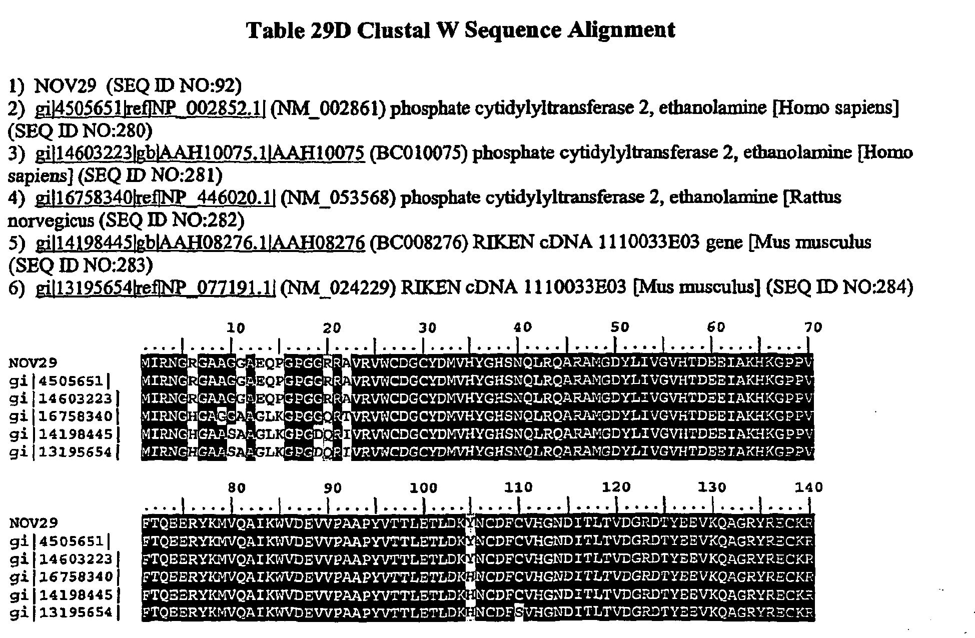

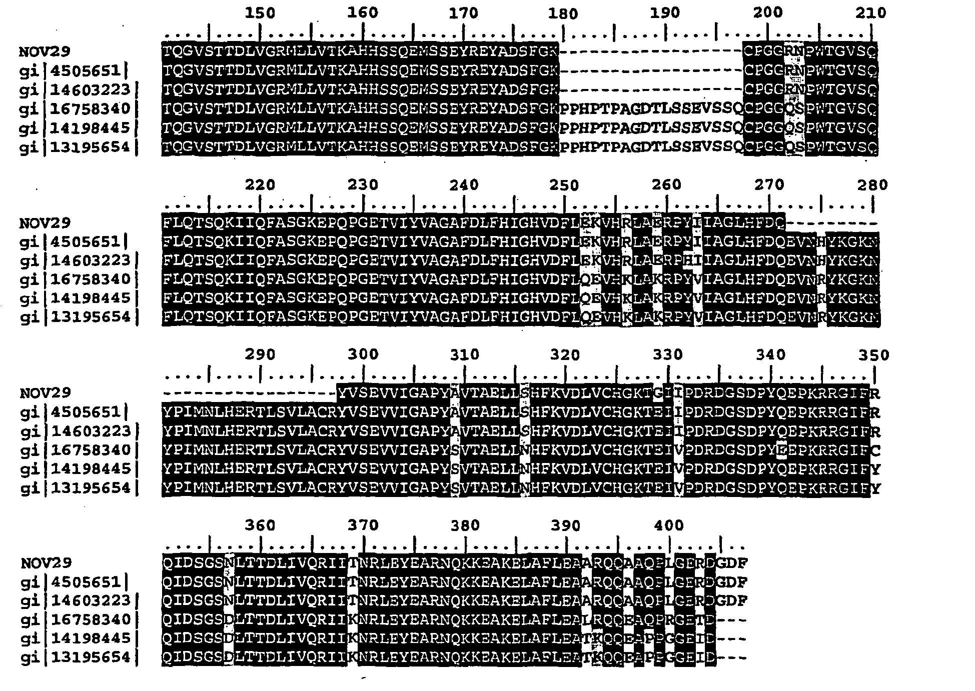

Table 29E lists the domain description from DOMAIN analysis results against NOV29. This indicates that the NOV29 sequence has properties similar to those of other proteins known to contain these domains.

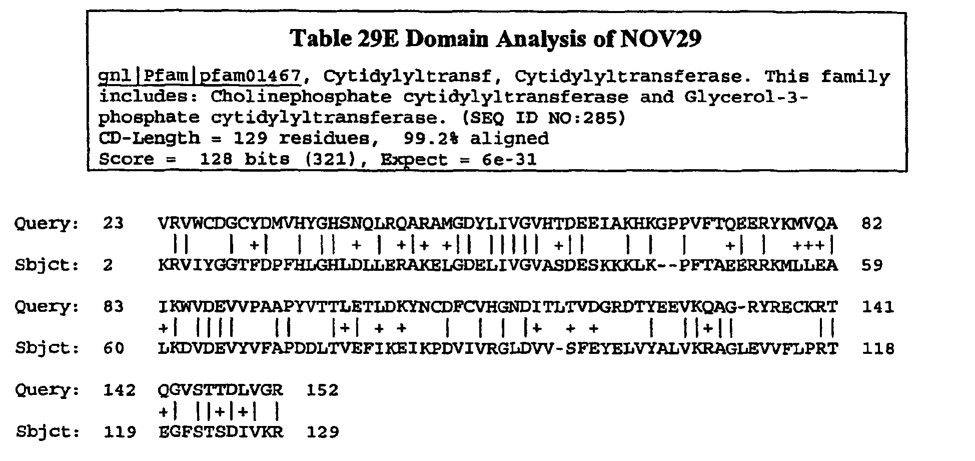

Ross et al. *(Neuroscience* 2001;102:899-904) reported that the activities of phospholipase A(2), phosphocholine cytidylyltransferase and phosphoethanolamine cytidylyltransferase, key phospholipid metabolic enzymes, are low in substantia nigra of normal human brain and that this might reduce the ability of nigral neurons to repair damage to cell membranes. To determine whether adaptive changes in nigral phospholipid metabolism can occur in idiopathic Parkinson's disease they compared activities of 11 catabolic and anabolic enzymes in autopsied brain of 10 patients with Parkinson's disease to those in control subjects. Nigral activity of the catabolic enzyme phospholipase A(2) was normal in the Parkinson's disease group, whereas that of the biosynthetic enzymes phosphoethanolamine cytidylyltransferase, phosphocholine cytidylyltransferase, and phosphatidylserine synthase were elevated 193, 48 and 38%, respectively, possibly representing a compensatory response to repair membrane phospholipids. Enzyme activities were normal in all other brain areas with the exception of increased (+26%) activity of calcium-stimulated phospholipase A(2) in putamen, a change which could be consequent to either decreased dopaminergic striatal input or to a dopamine nerve terminal degenerative process. Their data indicate that the normally low rate of membrane phospholipid synthesis in the substantia nigra, the primary area of neurodegeneration in Parkinson's disease, is increased during the course of the disorder. It was suggested that pharmacotherapies which augment this compensatory response might have utility as a treatment for Parkinson's disease.

The NOV29 nucleic acid of the invention encoding a Cholinephosphate Cytidylyltransferase-like protein includes the nucleic acid whose sequence is provided in Table 29A, or a fragment thereof. The invention also includes a mutant or variant nucleic acid any of whose bases may be changed from the corresponding base shown in Table 29A while still encoding a protein that maintains its Cholinephosphate Cytidylyltransferase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1% of the NOV29 residues may be so changed.

The NOV29 protein of the invention includes the Cholinephosphate Cytidylyltransferase-like protein whose sequence is provided in Table 29B. The invention also includes a mutant or variant protein any of whose residues may be changed from the corresponding residue shown in Table 29B while still encoding a protein that maintains its Cholinephosphate Cytidylyltransferase-like activities and physiological functions, or a functional fragment thereof. In the mutant or variant protein, up to about 1% of the NOV29 bases may be so changed.

The NOV29 nucleic acids and proteins of the invention are useful in potential diagnostic and therapeutic applications implicated in various diseases and disorders described below and/or other pathologies. For example, the compositions of the present invention will have efficacy for treatment of patients suffering from: Parkinson's disease and other diseases, disorders and conditions of the like.

NOV29 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV29 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOV30

A disclosed NOV30 nucleic acid of 893 nucleotides (also referred to as CG57558-01) encoding a novel mac25/IGFBP7-like protein is shown in Table 30A. An open reading frame was identified beginning with an ATG initiation codon at nucleotides 49-51 and ending with a TAA codon at nucleotides 871-873. Putative untranslated regions upstream from the intitation codon and downstream from the termination codon are underlined in Table 30A, and the start and stop codons are in bold letters.

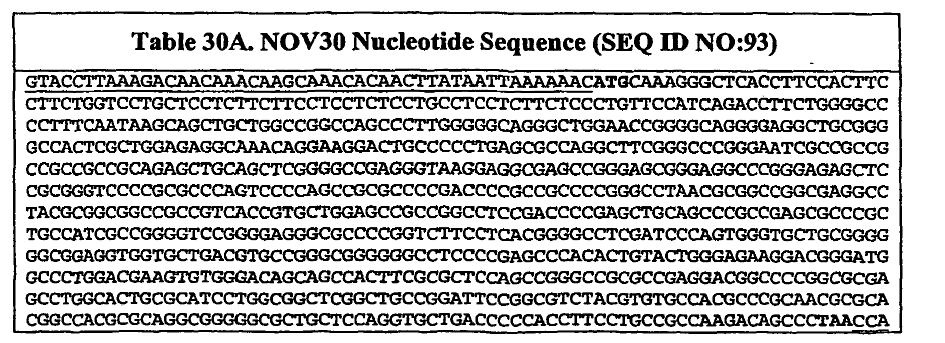

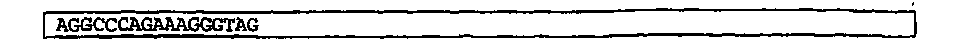

The NOV30 nucleic acid was identified on chromosome 2 and has 564 of 779 bases (72%) identical to a gb:GBNBANK-ID:S56581|acc:S56581.1 mRNA from *Rattus* sp. (alpha inhibin gene {5' region} [rats, Genomic, 2141 nt]) (E = 1.3e⁻⁶⁸).

A disclosed NOV30 polypeptide (SEQ ID NO:94) encoded by SEQ ID NO:93 is 274 amino acid residues and is presented using the one-letter code in Table 30B. Signal P, Psort and/or Hydropathy results predict that NOV30 contains a signal peptide and is likely to be localized to the extracellularly with a certainty of 0.3700. The most likely cleavage site for a NOV30 polypeptide is between amino acids 32-33: LLG-PL.

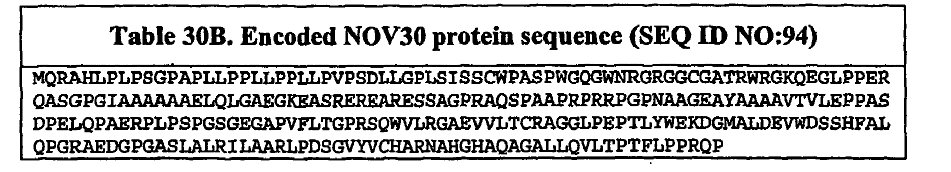

The NOV30 amino acid sequence 80 of 266 amino acid residues (30%) identical to, and 112 of 266 amino acid residues (42%) similar to, the 277 amino acid residue ptnr:SPTREMBL-ACC:Q07822 protein from *Homo sapiens* (Human) (mac25 protein) (E = 2.0e⁻¹⁷).

NOV30 is expressed in at least the following tissues: brain, ovary, breast and testis. This information was derived by determining the tissue sources of the sequences that were included in the invention including but not limited to SeqCalling sources, Public EST sources, genomic clone sources, literature sources, and/or RACE sources and the expression pattern of (gb:GENBANK-ID:S56581|acc:S56581.1) a closely related alpha inhibin gene {5' region} [rats, Genomic, 2141 nt] homolog in species *Rattus* sp.

NOV30 has homology to the amino acid sequences shown in the BLASTP data listed in Table 30C.

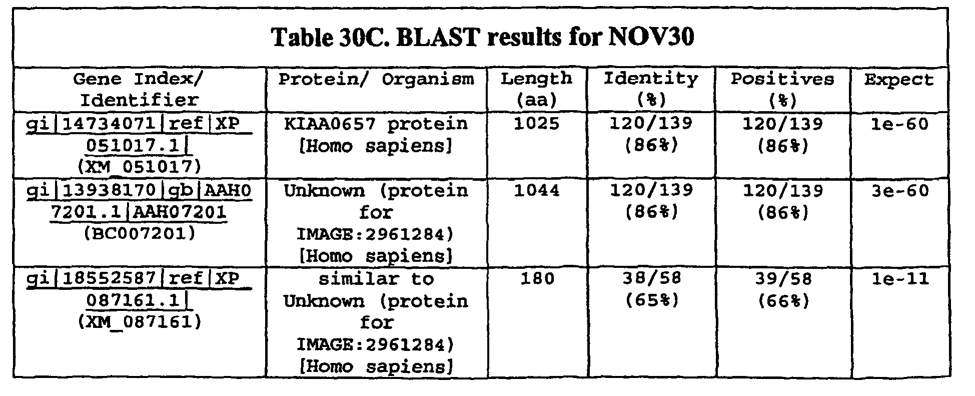

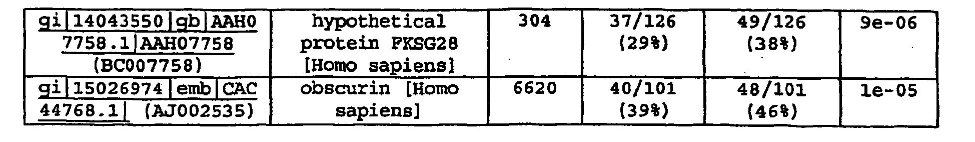

The homology of these sequences is shown graphically in the Clusta1W analysis shown in Table 30D.

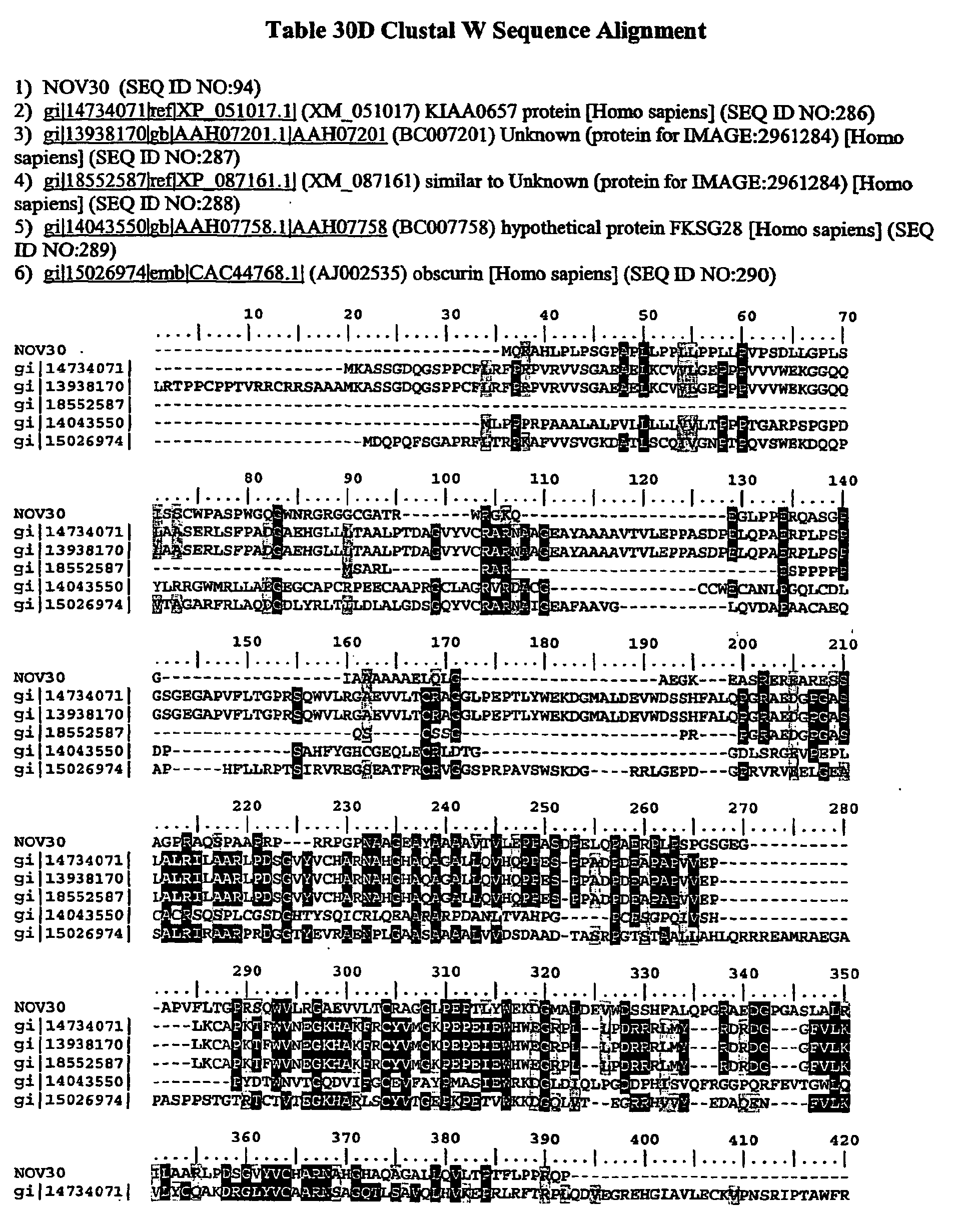

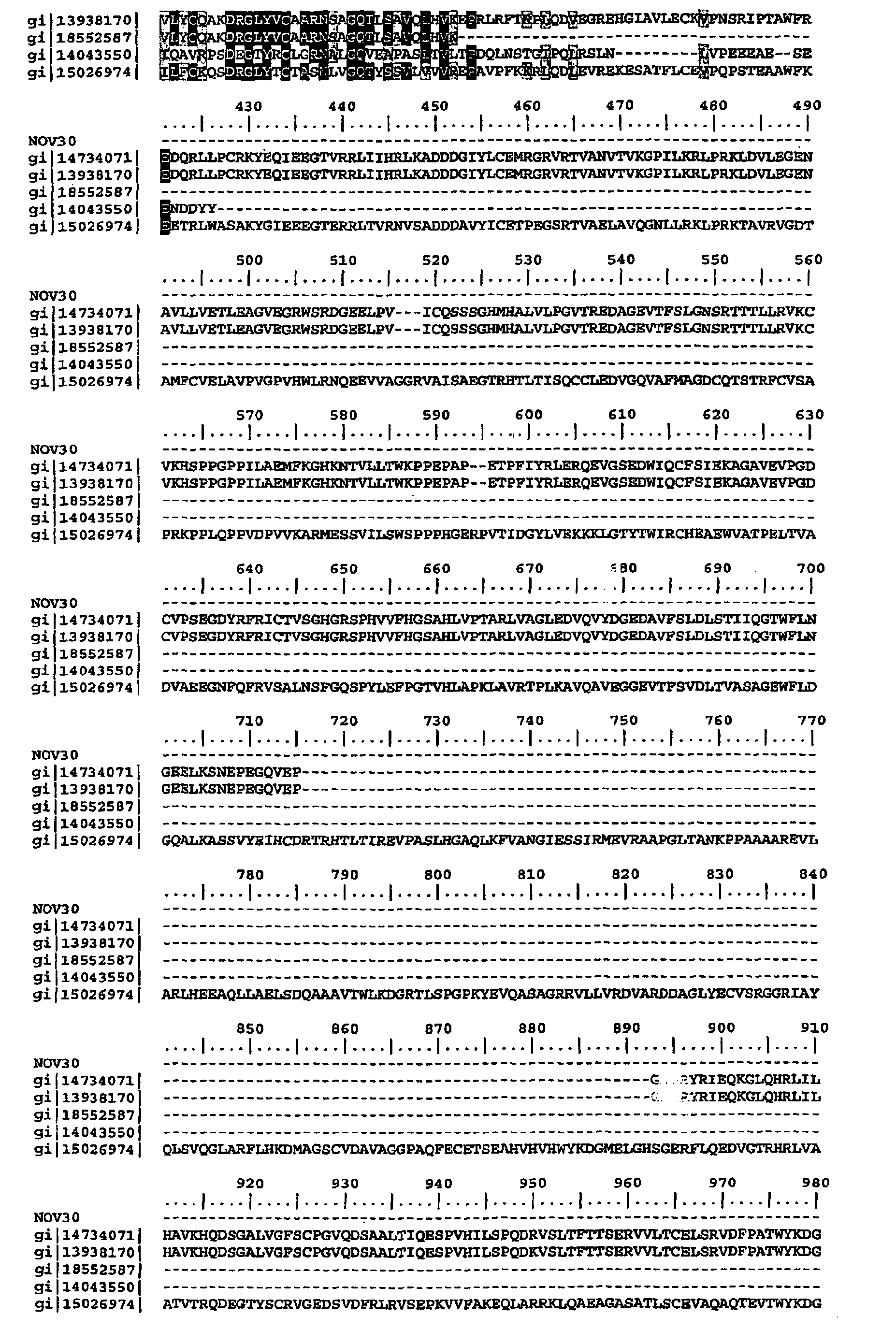

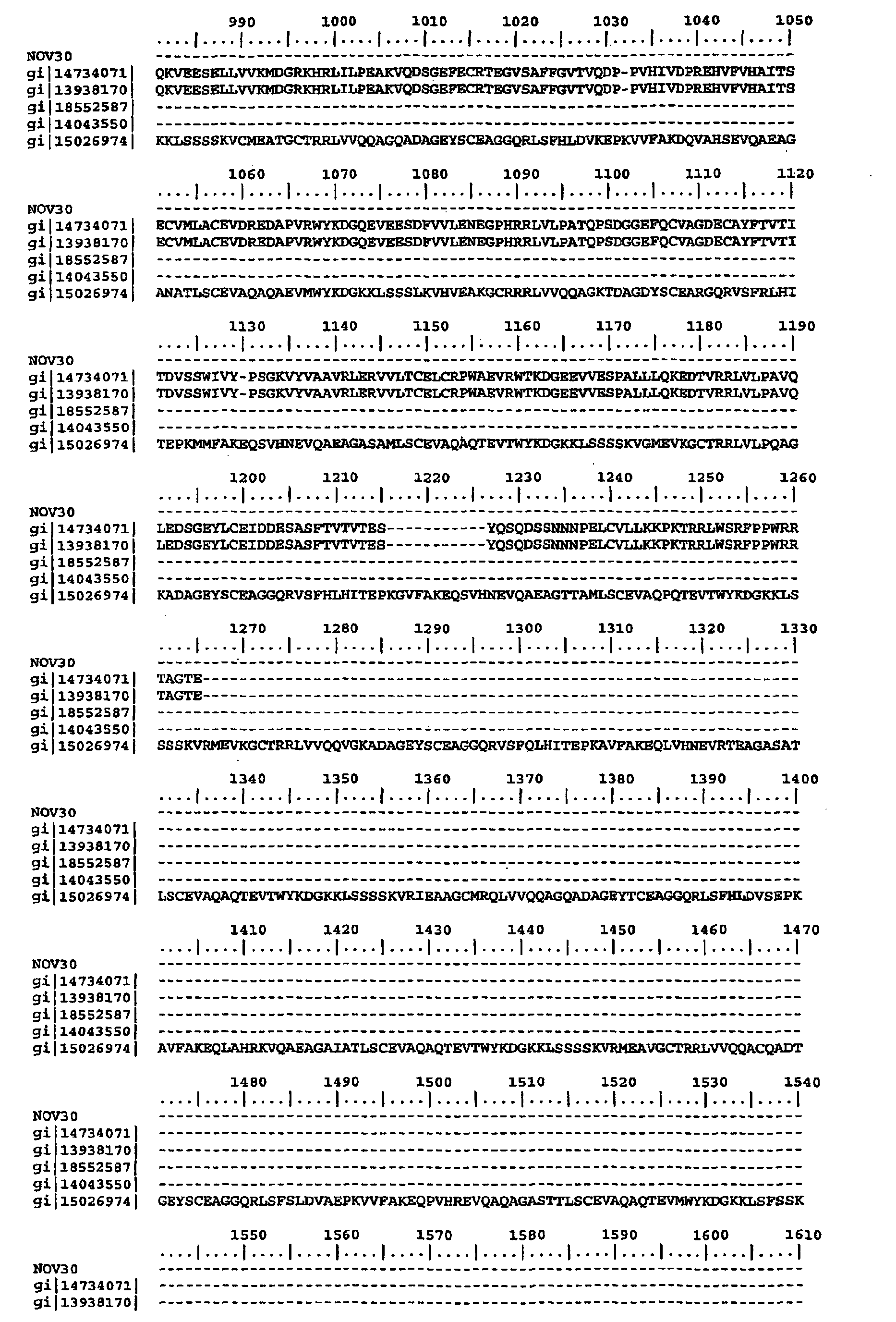

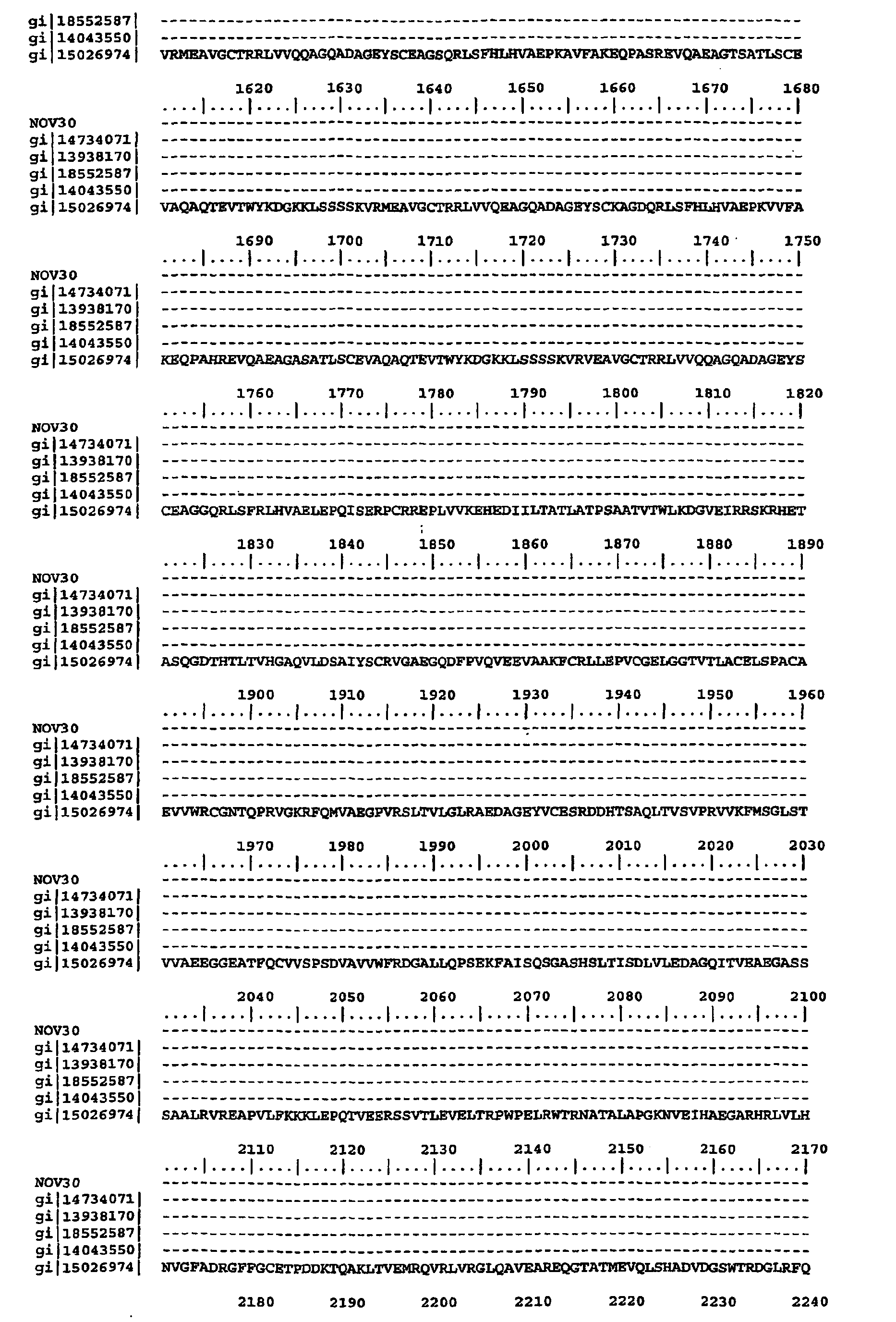

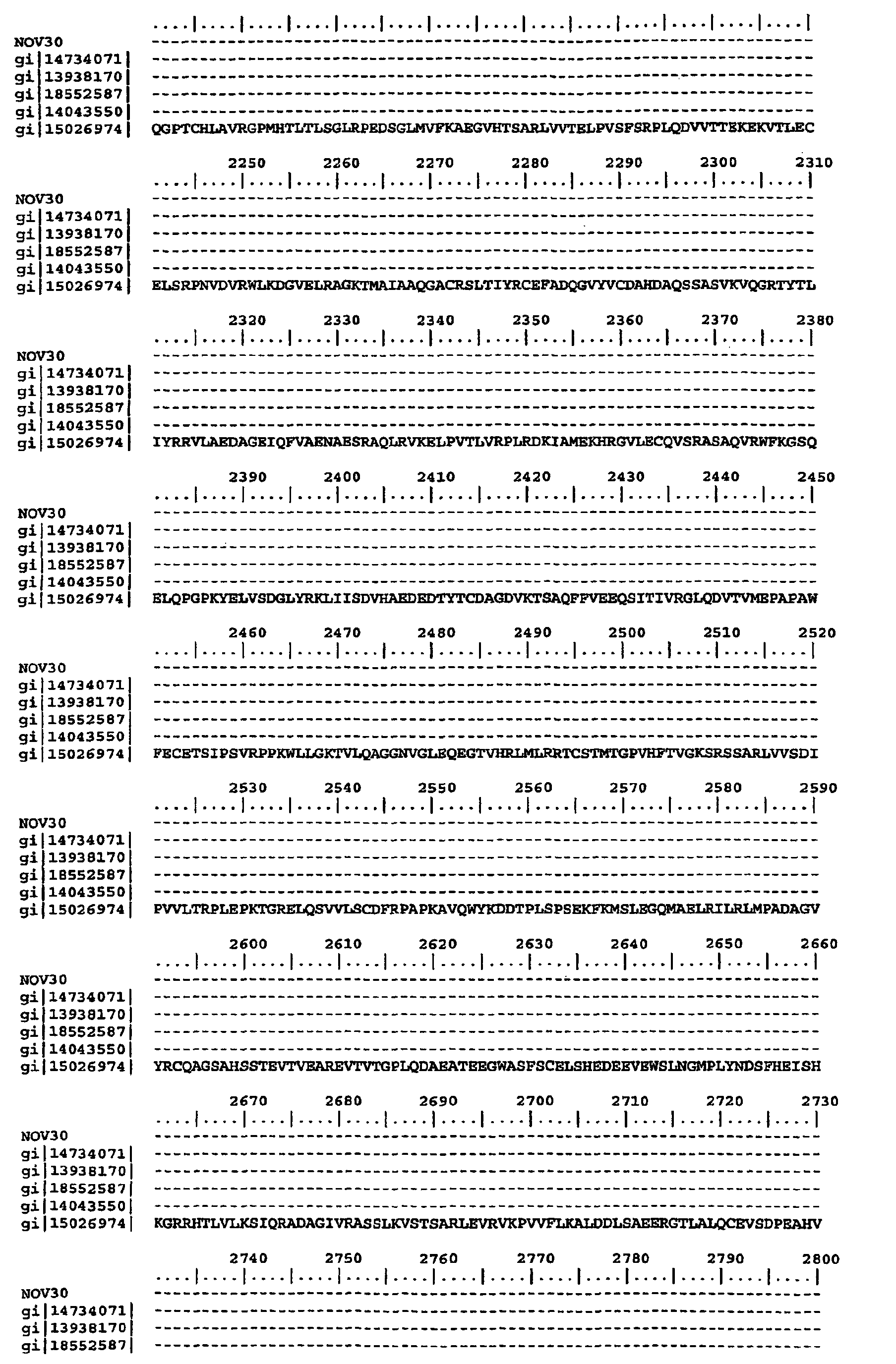

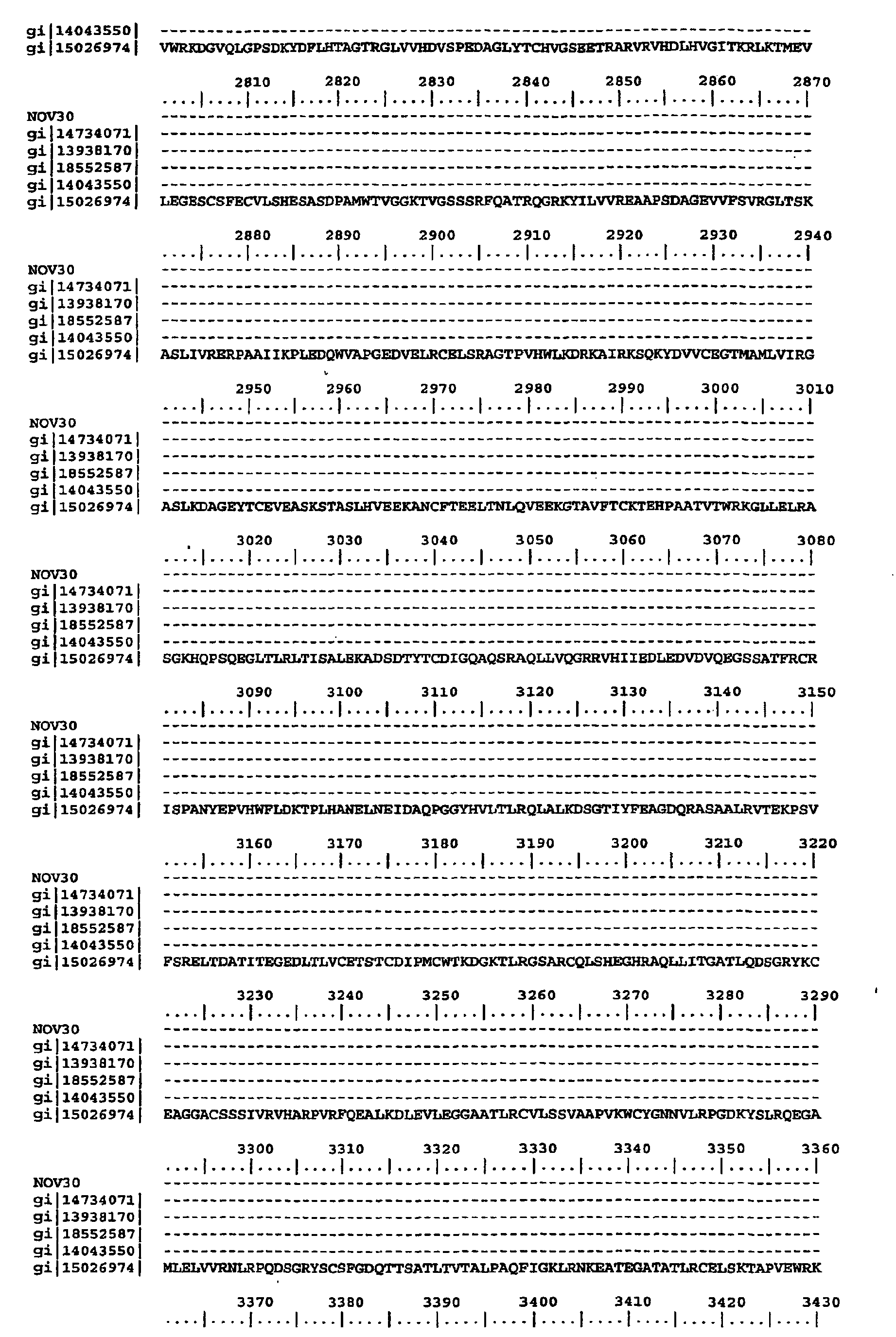

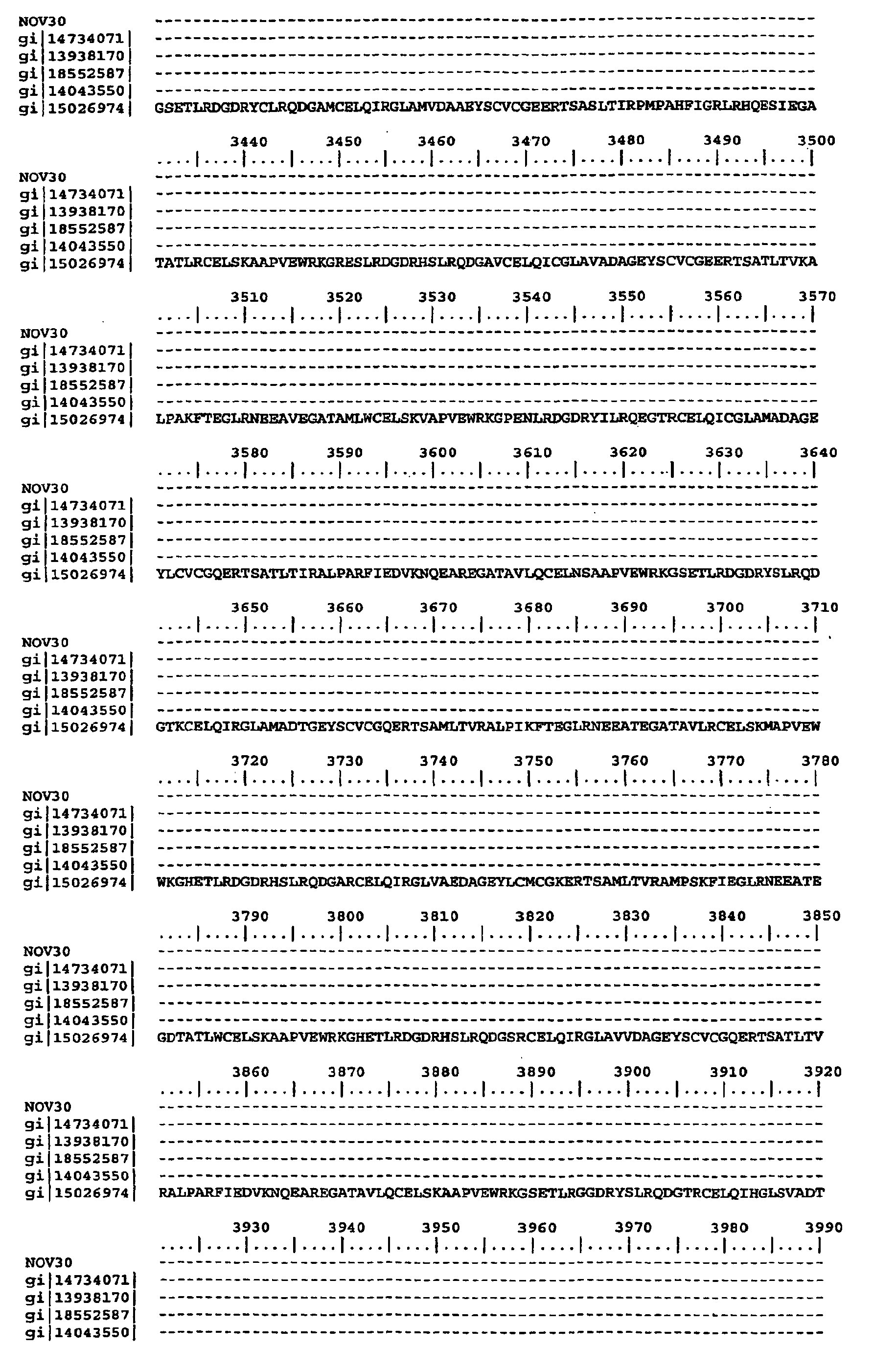

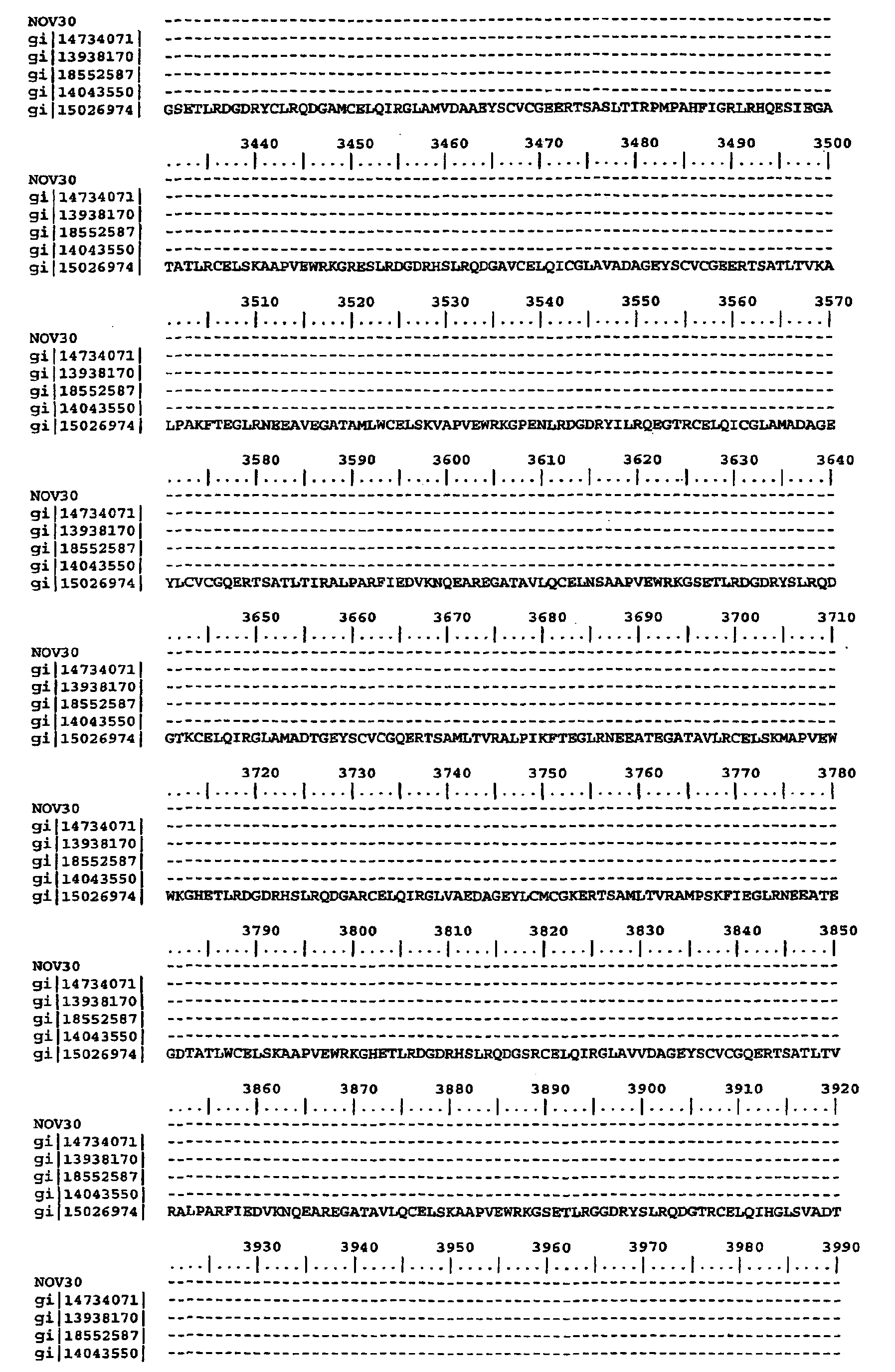

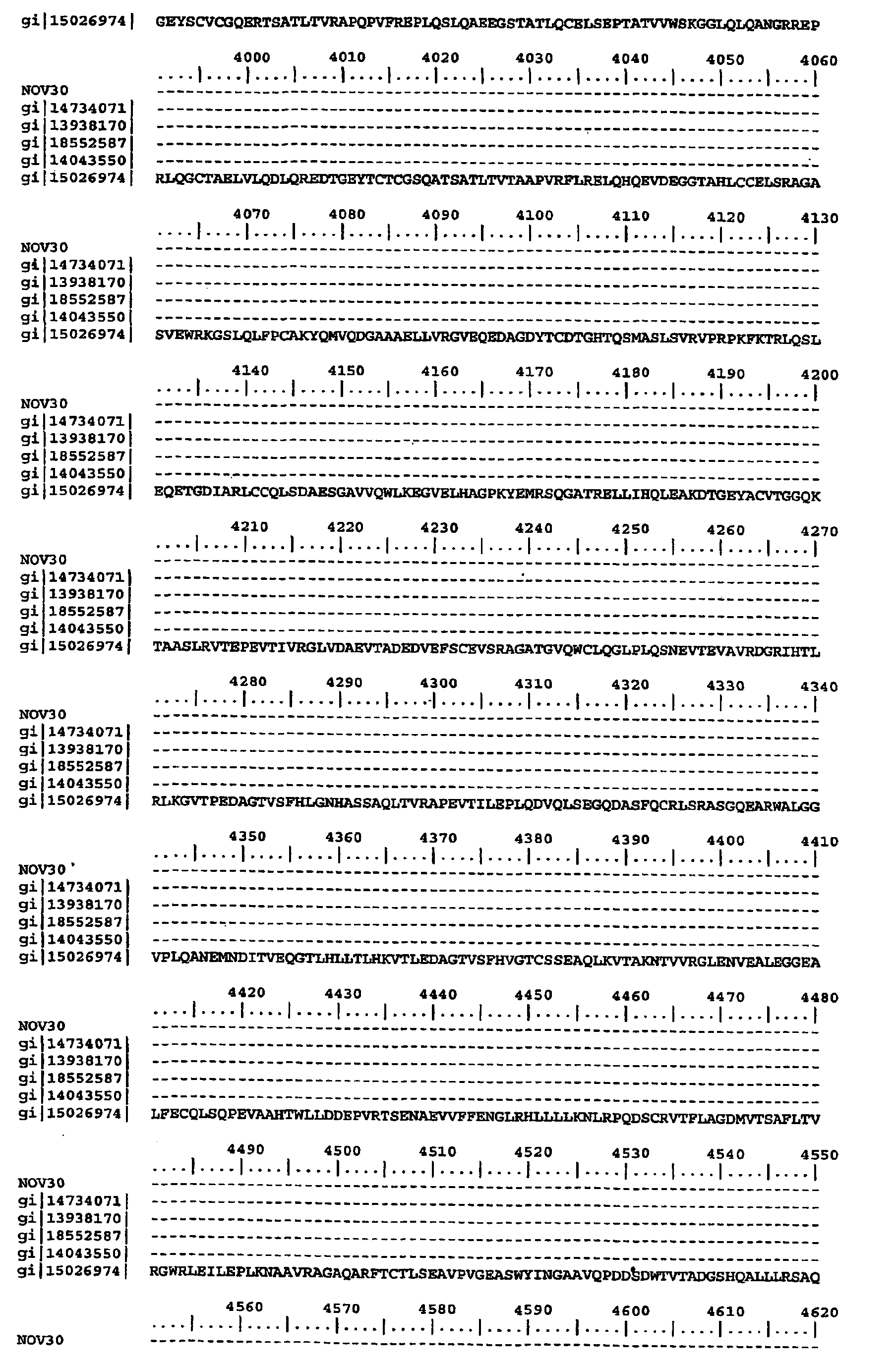

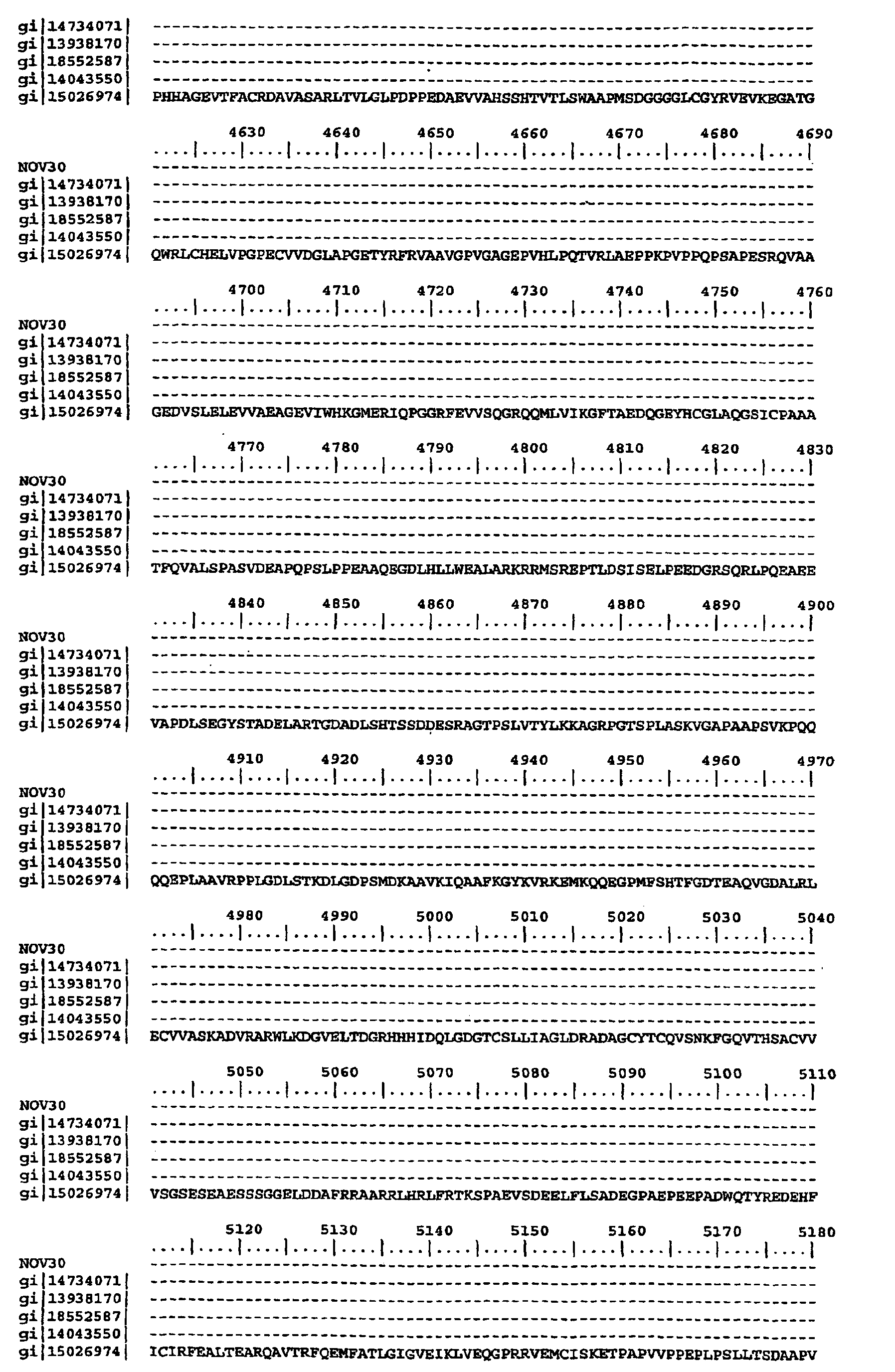

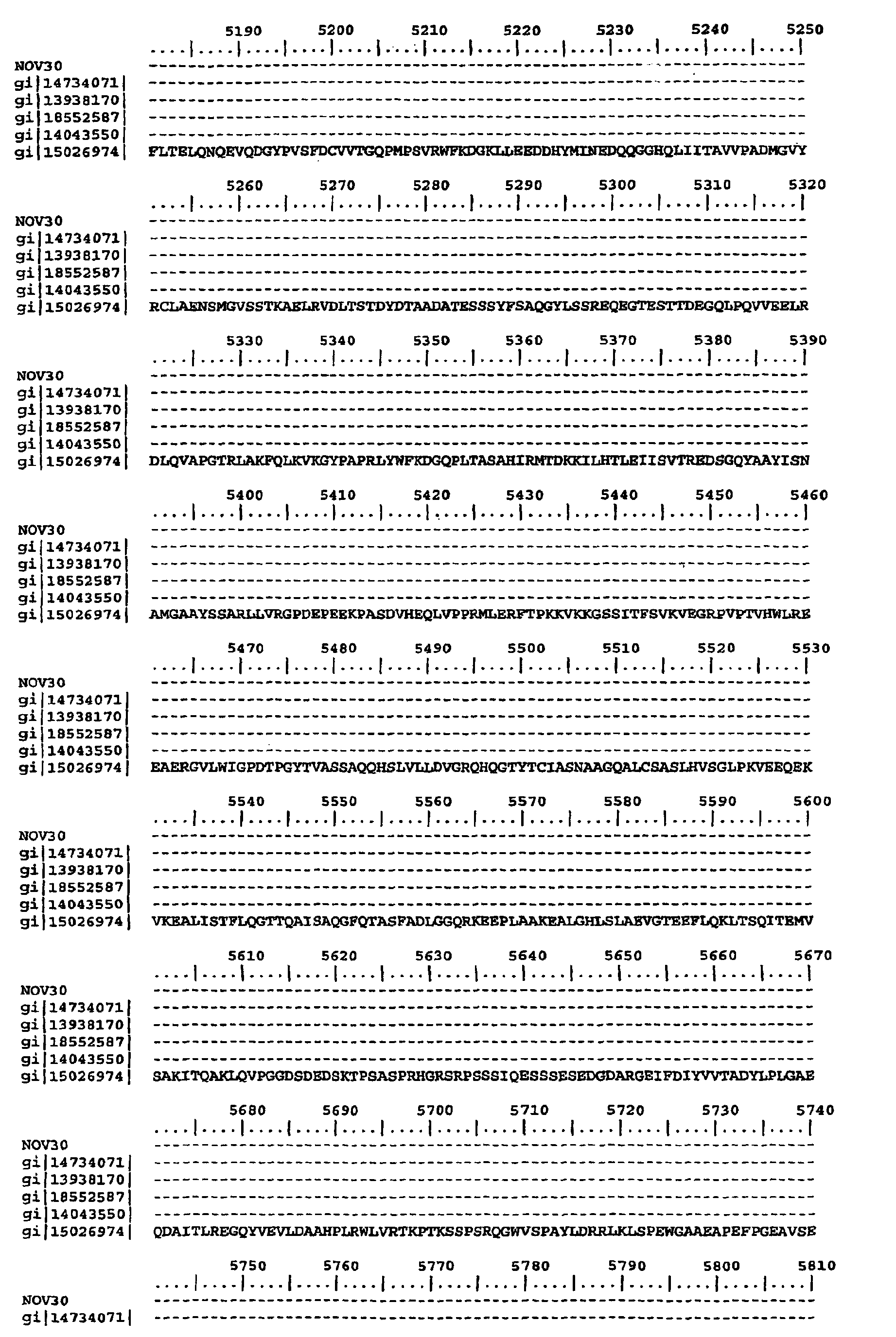

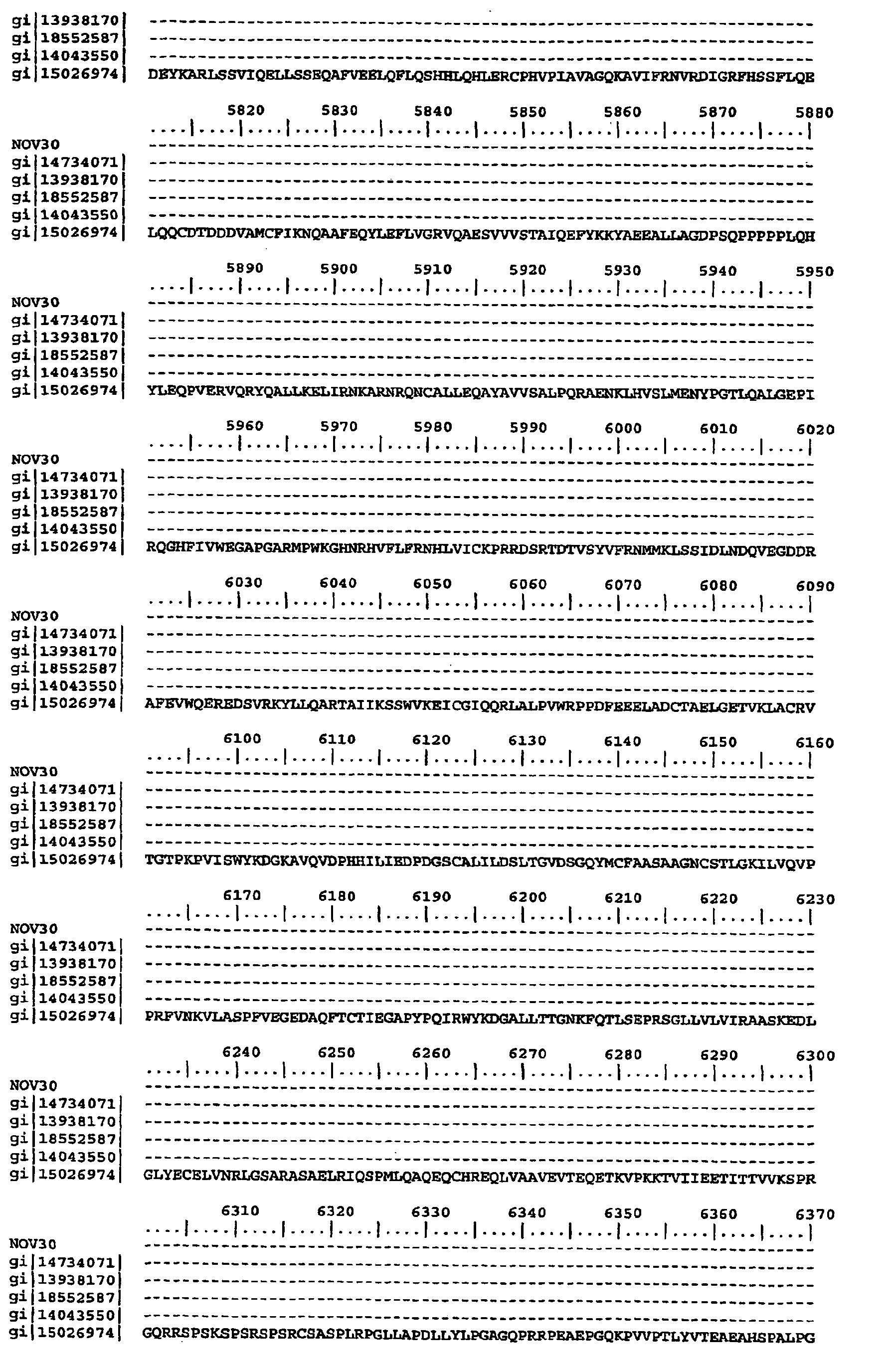

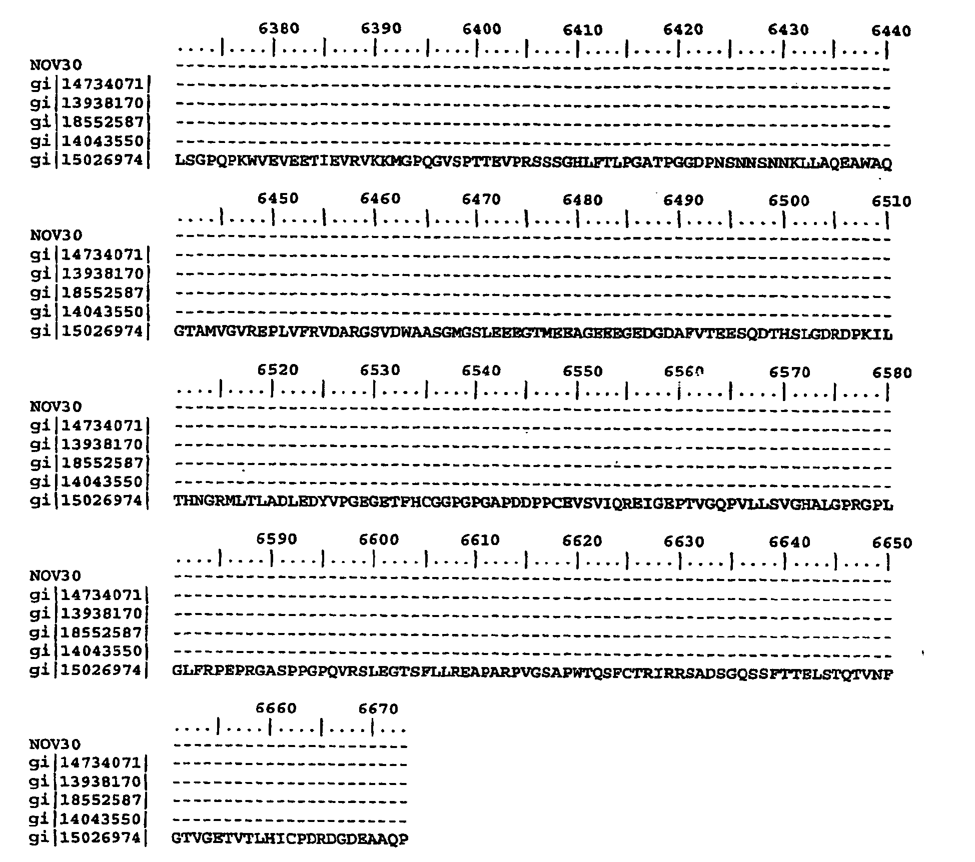

Tables 30E and 30F list the domain description from DOMAIN analysis results against NOV30. This indicates that the NOV30 sequence has properties similar to those of other proteins known to contain these domains.

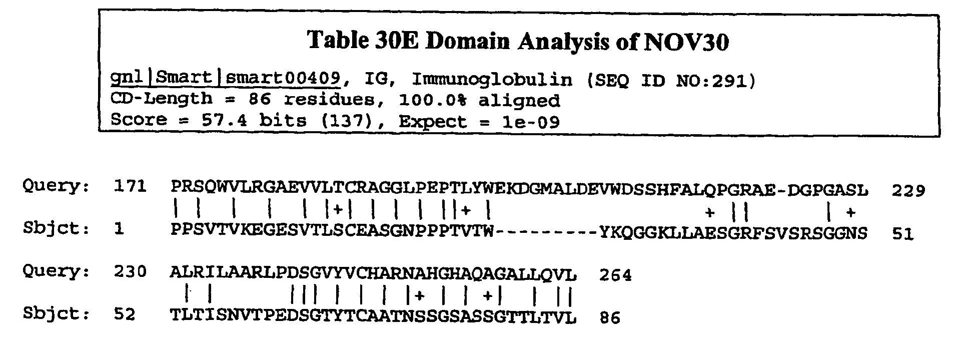

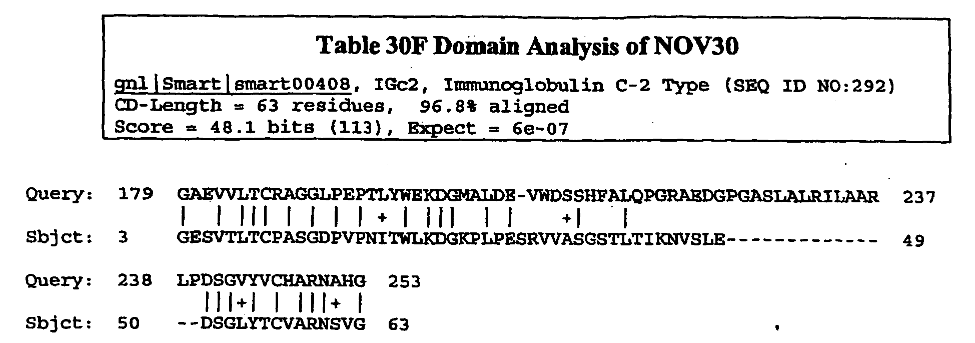

NOV30 described here has homology to mac25, a potent tumor inhibitor. Mac25 is a follistatin (FS)-like protein that has a growth-suppressing effect on a p53-deficient osteosarcoma cell line (Saos-2). The protein exhibits a strong homology to FS, an activin-binding protein, and part of its sequence includes the consensus sequence of the member of the Kazal serine protease inhibitor family. The mac25 protein was localized in the cytoplasm and secreted into culture medium (Kato, Mol Med 6(2):126-35,2000). Addition of recombinant mac25 protein (10-7 M) into the culture medium induced significant suppression of the growth of human cervical carcinoma cells (HeLa) and murine embryonic carcinoma cells (P19), as well as osteosarcoma cells (Saos-2). The mac25 protein was co-immunoprecipitated with activin A, a result that suggests that mac25 may be a secreted tumor-suppressor that binds activin A. The mac25 exhibits homology to insulin-like growth factor-binding proteins (IGFBPs) and to fibroblast growth factor receptor. The multi-functional nature of mac25 protein may be important for growth-suppression and/or cellular senescence (Kato, 2000).

The mac25 is strongly induced in senescent epithelial cells, whereas CTGF stimulates angiogenesis and wound healing. Using in situ hybridization and immunohistochemistry, Wandji et al. examined the possibilities that mac25 is inhibited, whereas CTGF is induced during active periods of follicular development and luteogenesis (Wandji et al., Endocrinology 141(7):2648-57, 2000). Ovaries were collected during the follicular and early luteal phases from prostaglandin F2alpha-treated mature pigs and from slaughterhouse sows. CTGF transcripts were induced during the late preantral stage in granulosa and theca cells concomitantly with the appearance of endothelial cells in the theca. CTGF mRNA expression increased in granulosa cells to a maximum (P < 0.01) in mid-antral follicles but was down regulated (P < 0.01) in preovulatory follicles. In contrast, granulosa cell mac25 mRNA expression was undetectable between the preantral and mid-antral stage but was strongly induced in terminally differentiated granulosa cells of preovulatory follicles. CTGF mRNA and peptide were also detected in the theca externa/interstitium and in vascular endothelial cells of ovarian blood vessels, whereas mac25 transcripts, which were also abundant in ovarian blood vessels increased in the theca interna with follicular development. Transcripts of cyclin D1, a marker of cell proliferation, appeared during the early antral stage and were moderate in granulosa cells but abundant in capillary endothelial cells in the theca interna, underneath the basement membrane. Following ovulation, CTGF and cyclin D1 mRNAs were associated with the migration of endothelial cells into the CL. Subsequently, there was a marked up-regulation of CTGF mRNA expression in granulosa luteins concomitantly with an increase in endothelial cell proliferation within the CL. Those data suggested that CTGF may promote ovarian cell growth and blood vessel formation during follicular and luteal development whereas mac25, a tumor inhibitor, may promote terminal differentiation of granulosa cells in preovulatory follicles (Wandji et al., 2000).

It is important to know the regulation of the expression of the mac25 gene because of its reduced expression in several cancer cells and of its induction by some hormonal factors. Kanemitsu et al. cloned the promoter region of the murine mac25 gene and found five repeats of CCAAT sequences, four Sp1 sites, a TATA-like sequence, and an initiator (INR) sequence. Analysis using luciferase reporter plasmids indicated that CCAAT repeats have a strong enhancer activity and the second to fourth Sp1 sites are essential for basal activity of the expression of the mac25 gene. The 1 kb region that contains the promoter and exon 1 of the mac25 gene was in a typical CpG island. As hypermethylation and reduced expression of the mac25 gene were reported in murine liver tumors, methylation of this CpG island may be directly associated with the expression of the mac25 gene and tumorigenesis (Kanemitsu et al., Biochem Biophys Res Commun 279(1):251-7, 2000).

Komatsu et al., used restriction landmark genomic scanning for methylation (RLGS-M) to detect alterations in DNA methylation associated with murine SV40 T/t antigen-induced hepatocarcinogenesis (Komatsu et al., Biochem Biophys Res Commun 267(1):109-17, 2000). An altered locus/spot (S130) was cloned and found to correspond to sequences in the 5' flanking region and 5' portion of the cDNA for the murine mac25/insulin-like growth factor binding protein-7 (Igfbp-7) gene. IGFBPs are believed to be capable of binding insulin, Igfl, and Igf2 and modulating mitogenic effects. Previous studies have shown that Igf2 has an important role in promoting liver tumorigenesis. Quantitative PCR was used to access the methylation status of the NotI site just 5' to the coding region and the expression level of the mac25/igfbp-7 gene. The results indicated that the degree of methylation was inversely related to the expression level and is consistent with a role for DNA methylation in silencing mac25/Igfbp-7 gene expression and function for mac25/Igfbp-7 as a tumor suppressor gene (Komatsu et al., 2000).

Because of the homology to mac25/IGFBP7, the NOV30 described here will have useful properties and functions similar to these genes.

The NOV30 nucleic acid of the invention encoding a mac25/IGFBP7-Iike protein includes the nucleic acid whose sequence is provided in Table 30A, or a fragment thereof. The invention also includes a mutant or variant nucleic acid any of whose bases may be changed from the corresponding base shown in Table 30A while still encoding a protein that maintains its mac25/IGFBP7-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 28% of the NOV30 residues may be so changed.

The NOV30 protein of the invention includes the mac25/IGFBP7-like protein whose sequence is provided in Table 30B. The invention also includes a mutant or variant protein any of whose residues may be changed from the corresponding residue shown in Table 30B while still encoding a protein that maintains its mac25/IGFBP7-like activities and physiological functions, or a functional fragment thereof. In the mutant or variant protein, up to about 70% of the NOV30 bases may be so changed.

The NOV30 nucleic acids and proteins of the invention are useful in potential diagnostic and therapeutic applications implicated in various diseases and disorders described below and/or other pathologies. For example, the compositions of the present invention will have efficacy for treatment of patients suffering from: Von Hippel-Lindau (VBL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, fertility, hypogonadism, endometriosis, hemophilia, hypercoagulation, idiopathic thrombocytopenic purpura, immunodeficiencies, graft versus host disease and other diseases, disorders and conditions of the like.

NOV30 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV30 protein have multiple hydrophilic regions, each of which can be used as an immunogen. This novel protein also has value in development of powerful assay system for functional analysis of various human disorders, which will help in understanding of pathology of the disease and development of new drug targets for various disorders.

### NOVX Nucleic Acids and Polypeptides

One aspect of the invention pertains to isolated nucleic acid molecules that encode NOVX polypeptides or biologically active portions thereof. Also included in the invention are nucleic acid fragments sufficient for use as hybridization probes to identify NOVX-encoding nucleic acids (*e.g*., NOVX mRNAs) and fragments for use as PCR primers for the amplification and/or mutation of NOVX nucleic acid molecules. As used herein, the term "nucleic acid molecule" is intended to include DNA molecules (*e.g*., cDNA or genomic DNA), RNA molecules (*e.g.,* mRNA), analogs of the DNA or RNA generated using nucleotide analogs, and derivatives, fragments and homologs thereof. The nucleic acid molecule may be single-stranded or double-stranded, but preferably is comprised double-stranded DNA.

An NOVX nucleic acid can encode a mature NOVX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product, encoded by the corresponding gene. Alternatively, it may be defined as the polypeptide, precursor or proprotein encoded by an ORF described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

The term "probes", as utilized herein, refers to nucleic acid sequences of variable length, preferably between at least about 10 nucleotides (nt), 100 nt, or as many as approximately, *e.g*., 6,000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies.

The term "isolated" nucleic acid molecule, as utilized herein, is one, which is separated from other nucleic acid molecules which are present in the natural source of the nucleic acid. Preferably, an "isolated" nucleic acid is free of sequences which naturally flank the nucleic acid (*i*.*e*., sequences located at the 5'- and 3'-termini of the nucleic acid) in the genomic DNA of the organism from which the nucleic acid is derived. For example, in various embodiments, the isolated NOVX nucleic acid molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g*., brain, heart, liver, spleen, etc.). Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the invention, *e*.*g*., a nucleic acid molecule having the nucleotide sequence SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or a complement of this aforementioned nucleotide sequence, can be isolated using standard molecular biology techniques and the sequence information provided herein. Using all or a portion of the nucleic acid sequence of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 as a hybridization probe, NOVX molecules can be isolated using standard hybridization and cloning techniques (*e.g*., as described in Sambrook, *et al*., (eds.), MOLECULAR CLONING: A LABORATORY MANUAL 2^{nd} Ed., Cold Spring Harbor Laboratory Press, Cold Spring Harbor, NY, 1989; and Ausubel, *et al*., (eds.), CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e*.*g*., using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides SEQ ID NOS: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or a complement thereof. Oligonucleotides may be chemically synthesized and may also be used as probes.

In another embodiment, an isolated nucleic acid molecule of the invention comprises a nucleic acid molecule that is a complement of the nucleotide sequence shown in SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or a portion of this nucleotide sequence (*e*.*g*., a fragment that can be used as a probe or primer or a fragment encoding a biologically-active portion of an NOVX polypeptide). A nucleic acid molecule that is complementary to the nucleotide sequence shown NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 is one that is sufficiently complementary to the nucleotide sequence shown NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75,77,79, 81, 83, 85, 87, 89, 91 and 93 that it can hydrogen bond with little or no mismatches to the nucleotide sequence shown SEQ ID NOS:1, 3, 5, 7, 9, 11,13, 15, 17, 19, 21, 23, 25, 27, 29, 31, 33, 35, 37,39,41,43,45,47,49,51,53,55,57,59, 61, 63, 65, 67, 69, 71, 73, 75, 77, 79, 81, 83, 85, 87, 89, 91 and 93, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. *See e*.*g*. Ausubel, *et al*., CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of NOVX polypeptides. Isoforms can be expressed in different tissues of the same organism as a result of, for example, alternative splicing of RNA. Alternatively, isoforms can be encoded by different genes. In the invention, homologous nucleotide sequences include nucleotide sequences encoding for an NOVX polypeptide of species other than humans, including, but not limited to: vertebrates, and thus can include, *e.g*., frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human NOVX protein. Homologous nucleic acid sequences include those nucleic acid sequences that encode conservative amino acid substitutions (see below) in SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, as well as a polypeptide possessing NOVX biological activity. Various biological activities of the NOVX proteins are described below.

An NOVX polypeptide is encoded by the open reading frame ("ORF") of an NOVX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF may be any part of a coding sequence, with or without a start codon, a stop codon, or both. For an ORF to be considered as a good candidate for coding for a *bona fide* cellular protein, a minimum size requirement is often set, *e.g.,* a stretch of DNA that would encode a protein of 50 amino acids or more.

The nucleotide sequences determined from the cloning of the human NOVX genes allows for the generation of probes and primers designed for use in identifying and/or cloning NOVX homologues in other cell types, *e*.*g*. from other tissues, as well as NOVX homologues from other vertebrates. The probe/primer typically comprises substantially purified oligonucleotide. The oligonucleotide typically comprises a region of nucleotide sequence that hybridizes under stringent conditions to at least about 12, 25, 50, 100, 150, 200, 250, 300, 350 or 400 consecutive sense strand nucleotide sequence SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93; or an anti-sense strand nucleotide sequence of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93; or of a naturally occurring mutant of SEQ ID NOS:1, 3, 5, 7, 9, 11, 13, 15, 17, 19, 21,23,25,27,29,31,33,35,37,39,41,43,45,47,49,51,53, 55, 57, 59, 61, 63, 65, 67, 69, 71, 73, 75, 77, 79, 81, 83, 85, 87, 89, 91 and 93.

Probes based on the human NOVX nucleotide sequences can be used to detect transcripts or genomic sequences encoding the same or homologous proteins. In various embodiments, the probe further comprises a label group attached thereto, *e*.*g*. the label group can be a radioisotope, a fluorescent compound, an enzyme, or an enzyme co-factor. Such probes can be used as a part of a diagnostic test kit for identifying cells or tissues which mis-express an NOVX protein, such as by measuring a level of an NOVX-encoding nucleic acid in a sample of cells from a subject *e.g*., detecting NOVX mRNA levels or determining whether a genomic NOVX gene has been mutated or deleted.

"A polypeptide having a biologically-active portion of an NOVX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of NOVX" can be prepared by isolating a portion SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, that encodes a polypeptide having an NOVX biological activity (the biological activities of the NOVX proteins are described below), expressing the encoded portion of NOVX protein (*e*.*g*., by recombinant expression *in vitro*) and assessing the activity of the encoded portion of NOVX.

### NOVX Nucleic Acid and Polypeptide Variants

The invention further encompasses nucleic acid molecules that differ from the nucleotide sequences shown in SEQ ID NOS:1,3,5,7,9,11,13,15,17,19,21,23,25,27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 due to degeneracy of the genetic code and thus encode the same NOVX proteins as that encoded by the nucleotide sequences shown in SEQ ID NOS:1, 3, 5, 7, 9, 11, 13, 15, 17, 19, 21, 23, 25, 27, 29, 31, 33, 35, 37, 39, 41, 43, 4S, 47, 49, 51, 53, 55, 57, 59, 61, 63, 65, 67, 69, 71, 73, 75, 77, 79, 81, 83, 85, 87, 89, 91 and 93. In another embodiment, an isolated nucleic acid molecule of the invention has a nucleotide sequence encoding a protein having an amino acid sequence shown in SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94.

In addition to the human NOVX nucleotide sequences shown in SEQ ID NOS:1,3,5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65,67, 69,71, 73, 75, 77, 79, 81, 83, 85, 87, 89, 91 and 93, it will be appreciated by those skilled in the art that DNA sequence polymorphisms that lead to changes in the amino acid sequences of the NOVX polypeptides may exist within a population (*e.g*., the human population). Such genetic polymorphism in the NOVX genes may exist among individuals within a population due to natural allelic variation. As used herein, the terms "gene" and "recombinant gene" refer to nucleic acid molecules comprising an open reading frame (ORF) encoding an NOVX protein, preferably a vertebrate NOVX protein. Such natural allelic variations can typically result in 1-5% variance in the nucleotide sequence of the NOVX genes. Any and all such nucleotide variations and resulting amino acid polymorphisms in the NOVX polypeptides, which are the result of natural allelic variation and that do not alter the functional activity of the NOVX polypeptides, are intended to be within the scope of the invention.

Moreover, nucleic acid molecules encoding NOVX proteins from other species, and thus that have a nucleotide sequence that differs from the human SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93. In another embodiment, the nucleic acid is at least 10, 25, 50, 100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i.e*., nucleic acids encoding NOVX proteins derived from species other than human) or other related sequences (*e.g*., paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5 °C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e*.*g*., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in Ausubel, *et al*., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. An isolated nucleic acid molecule of the invention that hybridizes under stringent conditions to the sequences SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, corresponds to a naturally-occurring nucleic acid molecule. As used herein, a "naturally-occurring" nucleic acid molecule refers to an RNA or DNA molecule having a nucleotide sequence that occurs in nature (*e*.*g*., encodes a natural protein).

In a second embodiment, a nucleic acid sequence that is hybridizable to the nucleic acid molecule comprising the nucleotide sequence of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well-known within the art. *See, e.g.,* Ausubel, *et al.* (eds.), 1993, CURRENT PROTOCOLS IN MOLECULAR BIOLOGY, John Wiley & Sons, NY, and Kriegler, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e*.*g*., as employed for cross-species hybridizations). *See, e*.*g*., Ausubel, *et al*. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. *Proc Natl Acad Sci USA* **78**: 6789-6792.

### Conservative Mutations

In addition to naturally-occurring allelic variants of NOVX sequences that may exist in the population, the skilled artisan will further appreciate that changes can be introduced by mutation into the nucleotide sequences SEQ ID NOS:1, 3, 5, 7, 9, 11, 13, 15,17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89,91 and 93, thereby leading to changes in the amino acid sequences of the encoded NOVX proteins, without altering the functional ability of said NOVX proteins. For example, nucleotide substitutions leading to amino acid substitutions at "non-essential" amino acid residues can be made in the sequence SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94. A "non-essential" amino acid residue is a residue that can be altered from the wild-type sequences of the NOVX proteins without altering their biological activity, whereas an "essential" amino acid residue is required for such biological activity. For example, amino acid residues that are conserved among the NOVX proteins of the invention are predicted to be particularly non-amenable to alteration. Amino acids for which conservative substitutions can be made are well-known within the art.

Another aspect of the invention pertains to nucleic acid molecules encoding NOVX proteins that contain changes in amino acid residues that are not essential for activity. Such NOVX proteins differ in amino acid sequence from SEQ ID NOS:1, 3, 5, 7, 9,11, 13,15,17, 19, 21, 23, 25, 27, 29, 3I, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 yet retain biological activity. In one embodiment, the isolated nucleic acid molecule comprises a nucleotide sequence encoding a protein, wherein the protein comprises an amino acid sequence at least about 45% homologous to the amino acid sequences SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94. Preferably, the protein encoded by the nucleic acid molecule is at least about 60% homologous to SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94; more preferably at least about 70% homologous SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94; still more preferably at least about 80% homologous to SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94; even more preferably at least about 90% homologous to SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94; and most preferably at least about 95% homologous to SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94.

An isolated nucleic acid molecule encoding an NOVX protein homologous to the protein of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94 can be created by introducing one or more nucleotide substitutions, additions or deletions into the nucleotide sequence of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, such that one or more amino acid substitutions, additions or deletions are introduced into the encoded protein.

Mutations can be introduced into SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 by standard techniques, such as site-directed mutagenesis and PCR-mediated mutagenesis. Preferably, conservative amino acid substitutions are made at one or more predicted, non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains (*e*.*g*., lysine, arginine, histidine), acidic side chains (*e*.*g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e*.*g*., threonine, valine, isoleucine) and aromatic side chains (*e*.*g*., tyrosine, phenylalanine, tryptophan, histidine). Thus, a predicted non-essential amino acid residue in the NOVX protein is replaced with another amino acid residue from the same side chain family. Alternatively, in another embodiment, mutations can be introduced randomly along all or part of an NOVX coding sequence, such as by saturation mutagenesis, and the resultant mutants can be screened for NOVX biological activity to identify mutants that retain activity. Following mutagenesis SEQ ID NOS:1,3,5,7,9,11,13,15,17,19,21,23,25,27,29,31,33,35,37,39,41,43,45, 47, 49, 51, 53, 55, 57, 59, 61, 63, 65, 67, 69, 71, 73, 75, 77, 79, 81, 83, 85, 87, 89, 91 and 93, the encoded protein can be expressed by any recombinant technology known in the art and the activity of the protein can be determined.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MILF, HY, FYW, wherein the single letter amino acid codes are grouped by those amino acids that may be substituted for each other. Likewise, the "weak" group of conserved residues may be any one of the following: CSA, ATV, SAG, STNK, STPA, SGND, SNDEQK, NDEQHK, NEQHRK, HFY, wherein the letters within each group represent the single letter amino acid code.

In one embodiment, a mutant NOVX protein can be assayed for (i) the ability to form protein:protein interactions with other NOVX proteins, other cell-surface proteins, or biologically-active portions thereof, (ii) complex formation between a mutant NOVX protein and an NOVX ligand; or *(iii)* the ability of a mutant NOVX protein to bind to an intracellular target protein or biologically-active portion thereof; *(e.g.* avidin proteins).

In yet another embodiment, a mutant NOVX protein can be assayed for the ability to regulate a specific biological function *(e.g.,* regulation of insulin release).

### Antisense Nucleic Acids

Another aspect of the invention pertains to isolated antisense nucleic acid molecules that are hybridizable to or complementary to the nucleic acid molecule comprising the nucleotide sequence of SEQ ID NOS:1, 3, 5, 7, 9, 11,13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or fragments, analogs or derivatives thereof. An "antisense" nucleic acid comprises a nucleotide sequence that is complementary to a "sense" nucleic acid encoding a protein (*e*.*g*., complementary to the coding strand of a double-stranded cDNA molecule or complementary to an mRNA sequence). In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of an NOVX protein of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, or antisense nucleic acids complementary to an NOVX nucleic acid sequence of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, are additionally provided.

In one embodiment, an antisense nucleic acid molecule is antisense to a "coding region" of the coding strand of a nucleotide sequence encoding an NOVX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the NOVX protein. The term "noncoding region" refers to 5' and 3' sequences which flank the coding region that are not translated into amino acids (*i.e*., also referred to as 5' and 3' untranslated regions).

Given the coding strand sequences encoding the NOVX protein disclosed herein, antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e*.*g*., an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e*.*g*., phosphorothioate derivatives and acridine substituted nucleotides can be used).

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding an NOVX protein to thereby inhibit expression of the protein (*e*.*g*., by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e.g*., by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other. *See, e.g*., Gaultier, *et al*., 1987. *Nucl. Acids Res. **15:*** 6625-6641. The antisense nucleic acid molecule can also comprise a 2'-o-methylribonucleotide *(See, e*.*g*., Inoue, *et al*. 1987. *Nucl. Acids Res.* **15:** 6131-6148) or a chimeric RNA-DNA analogue *(See, e*.*g*., Inoue, *et al*., 1987. *FEBSLett.* **215:** 327-330.

### Ribozymes and PNA Moieties

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e.g.*, hammerhead ribozymes as described in Haselhoff and Gerlach 1988. *Nature* 334: 585-591) can be used to catalytically cleave NOVX mRNA transcripts to thereby inhibit translation of NOVX mRNA. A ribozyme having specificity for an NOVX-encoding nucleic acid can be designed based upon the nucleotide sequence of an NOVX cDNA disclosed herein (*i.e*., SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93). For example, a derivative of a *Tetrahymena* L-19 IVS RNA can be constructed in which the nucleotide sequence of the active site is complementary to the nucleotide sequence to be cleaved in an NOVX-encoding mRNA. *See, e*.*g*., U.S. Patent 4,987,071 to Cech, *et al*. and U.S. Patent 5,116,742 to Cech, *et al*. NOVX mRNA can also be used to select a catalytic RNA having a specific ribonuclease activity from a pool of RNA molecules. *See, e*.*g*., Bartel *et al*., (1993) *Science* 261:1411-1418.

Alternatively, NOVX gene expression can be inhibited by targeting nucleotide sequences complementary to the regulatory region of the NOVX nucleic acid (*e*.*g*., the NOVX promoter and/or enhancers) to form triple helical structures that prevent transcription of the NOVX gene in target cells. *See, e*.*g*., Helene, 1991. *Anticancer Drug Des.* 6: 569-84; Helene, *et al*. 1992. *Ann. N.Y. Acad. Sci.* 660: 27-36; Maher, 1992. *Bioassays* 14: 807-15.

In various embodiments, the NOVX nucleic acids can be modified at the base moiety, sugar moiety or phosphate backbone to improve, *e*.*g*., the stability, hybridization, or solubility of the molecule. For example, the deoxyribose phosphate backbone of the nucleic acids can be modified to generate peptide nucleic acids. *See, e.g.,* Hyrup, *et al.,* 1996. *Bioorg Med Chem* 4: 5-23. As used herein, the terms "peptide nucleic acids" or "PNAs" refer to nucleic acid mimics (*e*.*g*., DNA mimics) in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup, *et al*., 1996. *supra*; Perry-O'Keefe, *et al*., 1996. *Proc. Natl. Acad. Sci. USA* 93: 14670-14675.

PNAs of NOVX can be used in therapeutic and diagnostic applications. For example, PNAs can be used as antisense or antigene agents for sequence-specific modulation of gene expression by, *e*.*g*., inducing transcription or translation arrest or inhibiting replication. PNAs of NOVX can also be used, for example, in the analysis of single base pair mutations in a gene (*e*.*g*., PNA directed PCR clamping; as artificial restriction enzymes when used in combination with other enzymes, *e*.*g*., S₁ nucleases *(See,* Hyrup, *et al*., 1996.*supra*); or as probes or primers for DNA sequence and hybridization *(See,* Hyrup, *et al*., 1996, *supra*; Perry-O'Keefe, *et al*., *1996. supra).*

In another embodiment, PNAs of NOVX can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes (*e*.*g*., RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleobases, and orientation (see, Hyrup, et al., 1996. *supra).* The synthesis of PNA-DNA chimeras can be performed as described in Hyrup, *et al*., 1996. *supra* and Finn, *et al*., 1996. *Nucl Acids Res* 24: 3357-3363. For example, a DNA chain can be synthesized on a solid support using standard phosphoramidite coupling chemistry, and modified nucleoside analogs, *e*.*g*., 5'-(4-methoxytrityl)amino-5'-deoxy-thymidine phosphoramidite, can be used between the PNA and the 5' end of DNA. *See, e*.*g*., Mag, *et al*., *1989. Nucl Acid Res* 17: 5973-5988. PNA monomers are then coupled in a stepwise manner to produce a chimeric molecule with a 5' PNA segment and a 3' DNA segment. *See, e.g*., Finn, *et al*., 1996. *supra.* Alternatively, chimeric molecules can be synthesized with a 5' DNA segment and a 3'PNA segment. *See, e.g.,* Petersen, *et al*., 1975. *Bioorg. Med. Chem. Lett. 5:* 1119-11124.

In other embodiments, the oligonucleotide may include other appended groups such as peptides (*e*.*g*., for targeting host cell receptors *in vivo*), or agents facilitating transport across the cell membrane *(see, e*.*g*., Letsinger, *et al*., 1989. *Proc. Natl. Acad. Sci. U.S.A. 86:* 6553-6556; Lemaitre, *et al*., 1987. *Proc. Natl. Acad. Sci.* 84: 648-652; PCT Publication No. WO88/09810) or the blood-brain barrier *(see, e.g.,* PCT Publication No. WO 89/10134). In addition, oligonucleotides can be modified with hybridization triggered cleavage agents (*see, e.g.,* Krol, *et al*., 1988. *BioTechniques* 6:958-976) or intercalating agents *(see, e.g.,* Zon, 1988. *Pharm. Res.* 5: 539-549). To this end, the oligonucleotide may be conjugated to another molecule, *e.g*., a peptide, a hybridization triggered cross-linking agent, a transport agent, a hybridization-triggered cleavage agent, and the like.

### NOVX Polypeptides

A polypeptide according to the invention includes a polypeptide including the amino acid sequence of NOUX polypeptides whose sequences are provided in SEQ ID NOS:2, 4, 6, 8, 10, 12, 14, 16, 18, 20, 22, 24, 26, 28, 30, 32, 34, 36, 3 8, 40, 42, 44, 46, 48, 50, 52, 54, 56, 58, 60, 62, 64, 66, 68, 70, 72, 74, 76, 78, 80, 82, 84, 86, 88, 90, 92 and 94. The invention also includes a mutant or variant protein any of whose residues may be changed from the corresponding residues shown in SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94 while still encoding a protein that maintains its NOVX activities and physiological functions, or a functional fragment thereof.

In general, an NOVX variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically-active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, an NOVX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX proteins in which the protein is separated from cellular components of the cells from which it is isolated or recombinantly-produced. In one embodiment, the language "substantially free of cellular material" includes preparations of NOVX proteins having less than about 30% (by dry weight) of non-NOVX proteins (also referred to herein as a "contaminating protein"), more preferably less than about 20% of non-NOVX proteins, still more preferably less than about 10% of non-NOVX proteins, and most preferably less than about 5% of non-NOVX proteins. When the NOVX protein or biologically-active portion thereof is recombinantly-produced, it is also preferably substantially free of culture medium, *i*.*e*., culture medium represents less than about 20%, more preferably less than about 10%, and most preferably less than about 5% of the volume of the NOVX protein preparation.

The language "substantially free of chemical precursors or other chemicals" includes preparations of NOVX proteins in which the protein is separated from chemical precursors or other chemicals that are involved in the synthesis of the protein. In one embodiment, the language "substantially free of chemical precursors or other chemicals" includes preparations of NOVX proteins having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically-active portions of NOVX proteins include peptides comprising amino acid sequences sufficiently homologous to or derived from the amino acid sequences of the NOVX proteins (*e*.*g*., the amino acid sequence shown in SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94) that include fewer amino acids than the full-length NOVX proteins, and exhibit at least one activity of an NOVX protein. Typically, biologically-active portions comprise a domain or motif with at least one activity of the NOVX protein. A biologically-active portion of an NOVX protein can be a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native NOVX protein.

In an embodiment, the NOVX protein has an amino acid sequence shown SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94. In other embodiments, the NOVX protein is substantially homologous to SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, and retains the functional activity of the protein of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, yet differs in amino acid sequence due to natural allelic variation or mutagenesis, as described in detail, below. Accordingly, in another embodiment, the NOVX protein is a protein that comprises an amino acid sequence at least about 45% homologous to the amino acid sequence SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, and retains the functional activity of the NOVX proteins of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94.

### Determining Homology Between Two or More Sequences

To determine the percent homology of two amino acid sequences or of two nucleic acids, the sequences are aligned for optimal comparison purposes (*e*.*g*., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i*.*e*., as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See*, Needleman and Wunsch, 1970. *J Mol Biol* 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NOS:1, 3, 5, 7, 9, 11, 13, 15, 17, 19,21,23,25,27,29,31,33,35,37,39,41,43,45,47,49, 51,53,55,57,59, 61, 63, 6S, 67, 69, 71, 73, 75, 77, 79, 81, 83, 8S, 87, 89, 91 and 93.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e*.*g*., A, T, C, G, U, or I, in the case of nucleic acids) occurs in both sequences to yield the number of matched positions, dividing the number of matched positions by the total number of positions in the region of comparison (*i*.*e*., the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides NOVX chimeric or fusion proteins. As used herein, an NOVX "chimeric protein" or "fusion protein" comprises an NOVX polypeptide operatively-linked to a non-NOVX polypeptide. An "NOVX polypeptide" refers to a polypeptide having an amino acid sequence corresponding to an NOVX protein SEQ ID NOS:2, 4, 6, 8, 10, 12, 14, 16, 18, 20, 22, 24, 26, 28, 3 0, 32, 34, 36, 3 8, 40, 42, 44, 46, 48, 50, 52, 54, 56, 5 8, 60, 62, 64, 66, 68, 70, 72, 74, 76, 78, 80, 82, 84, 86, 88, 90, 92 and 94, whereas a "non-NOVX polypeptide" refers to a polypeptide having an amino acid sequence corresponding to a protein that is not substantially homologous to the NOVX protein, *e.g*., a protein that is different from the NOVX protein and that is derived from the same or a different organism. Within an NOVX fusion protein the NOVX polypeptide can correspond to all or a portion of an NOVX protein. In one embodiment, an NOVX fusion protein comprises at least one biologically-active portion of an NOVX protein. In another embodiment, an NOVX fusion protein comprises at least two biologically-active portions of an NOVX protein. In yet another embodiment, an NOVX fusion protein comprises at least three biologically-active portions of an NOVX protein. Within the fusion protein, the term "operatively-linked" is intended to indicate that the NOVX polypeptide and the non-NOVX polypeptide are fused in-frame with one another. The non-NOVX polypeptide can be fused to the N-terminus or C-terminus of the NOVX polypeptide.

In one embodiment, the fusion protein is a GST-NOVX fusion protein in which the NOVX sequences are fused to the C-terminus of the GST (glutathione S-transferase) sequences. Such fusion proteins can facilitate the purification of recombinant NOVX polypeptides.

In another embodiment, the fusion protein is an NOVX protein containing a heterologous signal sequence at its N-terminus. In certain host cells (*e*.*g*., mammalian host cells), expression and/or secretion of NOVX can be increased through use of a heterologous signal sequence.

In yet another embodiment, the fusion protein is an NOVX-immunoglobulin fusion protein in which the NOVX sequences are fused to sequences derived from a member of the immunoglobulin protein family. The NOVX-immunoglobulin fusion proteins of the invention can be incorporated into pharmaceutical compositions and administered to a subject to inhibit an interaction between an NOVX ligand and an NOVX protein on the surface of a cell, to thereby suppress NOVX-mediated signal transduction *in vivo.* The NOVX-immunoglobulin fusion proteins can be used to affect the bioavailability of an NOVX cognate ligand. Inhibition of the NOVX ligand/NOVX interaction may be useful therapeutically for both the treatment of proliferative and differentiative disorders, as well as modulating (*e*.*g*. promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with an NOVX ligand.

An NOVX chimeric or fusion protein of the invention can be produced by standard recombinant DNA techniques. For example, DNA fragments coding for the different polypeptide sequences are ligated together in-frame in accordance with conventional techniques, *e*.*g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence *(see, e.g.,* Ausubel, *et al.* (eds.) CURRENT PROTOCOLS IN MOLECULAR BIOLOGY, John Wiley & Sons, 1992). Moreover, many expression vectors are commercially available that already encode a fusion moiety (*e*.*g*., a GST polypeptide). An NOVX-encoding nucleic acid can be cloned into such an expression vector such that the fusion moiety is linked in-frame to the NOVX protein.

### NOVX Agonists and Antagonists

The invention also pertains to variants of the NOVX proteins that function as either NOVX agonists (*i*.*e*., mimetics) or as NOVX antagonists. Variants of the NOVX protein can be generated by mutagenesis (*e.g.*, discrete point mutation or truncation of the NOVX protein). An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX proteins that function as either NOVX agonists (*i*.*e*., mimetics) or as NOVX antagonists can be identified by screening combinatorial libraries of mutants (*e*.*g*., truncation mutants) of the NOVX proteins for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e*.*g*., for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are well-known within the art. *See, e*.*g*., Narang, 1983. *Tetrahedron* 39: 3; Itakura, *et al.,* 1984. *Annu. Rev. Biochem.* 53: 323; Itakura, *et al.,* 1984. *Science* 198: 1056; Ike, *et al.,* 1983. *Nucl. Acids Res.* 11: 477.

### Polypeptide Libraries

In addition, libraries of fragments of the NOVX protein coding sequences can be used to generate a variegated population of NOVX fragments for screening and subsequent selection of variants of an NOVX protein. In one embodiment, a library of coding sequence fragments can be generated by treating a double stranded PCR fragment of an NOVX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes N-terminal and internal fragments of various sizes of the NOVX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify NOVX variants. *See, e*.*g*., Arkin and Yourvan, 1992. *Proc. Natl. Acad. Sci. USA* 89: 7811-7815; Delgrave, *et al*., 1993. *Protein Engineering* 6:327-331.

### Antibodies

The term "antibody" as used herein refers to immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, F_{ab}, F_{ab'} and F(_{ab'})₂ fragments, and an F_{ab} expression library. In general, antibody molecules obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated protein of the invention intended to serve as an antigen, or a portion or fragment thereof, can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as an amino acid sequence shown in SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of SECX that is located on the surface of the protein, e.g., a hydrophilic region. A hydrophobicity analysis of the human SECX protein sequence will indicate which regions of a SECX polypeptide are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, e.g., Hopp and Woods, 1981, Proc. Nat. Acad. Sci. USA 78: 3824-3828; Kyte and Doolittle 1982, J. Mol. Biol. 157: 105-142, each incorporated herein by reference in their entirety. Antibodies that are specific for one or more domains within an antigenic protein, or derivatives, fragments, analogs or homologs thereof, are also provided herein.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (e.g., rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (e.g., aluminum hydroxide), surface active substances (e.g., lysolecithin, pluronic polyols, polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (e.g., from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). It is an objective, especially important in therapeutic applications of monoclonal antibodies, to identify antibodies having a high degree of specificity and a high binding affinity for the target antigen.

After the desired hybridoma cells are identified, the clones can be subcloned by limiting dilution procedures and grown by standard methods (Goding, 1986). Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### Humanized Antibodies

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

### Human Antibodies

Fully human antibodies essentially relate to antibody molecules in which the entire sequence of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R. Liss, Inc., pp. 77-96).

In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (Bio/Technology 10, 779-783 (1992)); Lonberg et al. (Nature 368 856-859 (1994)); Morrison (Nature 368, 812-13 (1994)); Fishwild et al,(Nature Biotechnology 14, 845-51 (1996)); Neuberger (Nature Biotechnology 14, 826 (1996)); and Lonberg and Huszar (Intern. Rev. Immunol. 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### F_{ab} Fragments and Single Chain Antibodies

According to the invention, techniques can be adapted for the production of single-chain antibodies specific to an antigenic protein of the invention (see e.g*.*, U.S. Patent No. 4,946,778). In addition, methods can be adapted for the construction of F_{ab} expression libraries (see e.g., Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitmbenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD 16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### Effector Function Engineering

It can be desirable to modify the antibody of the invention with respect to effector fimction, so as to enhance, e.g., the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g., avidin) that is in turn conjugated to a cytotoxic agent.

### Immunoliposomes

The antibodies disclosed herein can also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### Diagnostic Applications of Antibodies Directed Against the Proteins of the Invention

Antibodies directed against a protein of the invention may be used in methods known within the art relating to the localization and/or quantitation of the protein (e.g., for use in measuring levels of the protein within appropriate physiological samples, for use in diagnostic methods, for use in imaging the protein, and the like). In a given embodiment, antibodies against the proteins, or derivatives, fragments, analogs or homologs thereof, that contain the antigen binding domain, are utilized as pharmacologically-active compounds (see below).

An antibody specific for a protein of the invention can be used to isolate the protein by standard techniques, such as immunoaffinity chromatography or immunoprecipitation. Such an antibody can facilitate the purification of the natural protein antigen from cells and of recombinantly produced antigen expressed in host cells. Moreover, such an antibody can be used to detect the antigenic protein (e.g., in a cellular lysate or cell supernatant) in order to evaluate the abundance and pattern of expression of the antigenic protein. Antibodies directed against the protein can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### Antibody Therapeutics

Antibodies of the invention, including polyclonal, monoclonal, humanized and fully human antibodies, may used as therapeutic agents. Such agents will generally be employed to treat or prevent a disease or pathology in a subject. An antibody preparation, preferably one having high specificity and high affinity for its target antigen, is administered to the subject and will generally have an effect due to its binding with the target. Such an effect may be one of two kinds, depending on the specific nature of the interaction between the given antibody molecule and the target antigen in question. In the first instance, administration of the antibody may abrogate or inhibit the binding of the target with an endogenous ligand to which it naturally binds. In this case, the antibody binds to the target and masks a binding site of the naturally occurring ligand, wherein the ligand serves as an effector molecule. Thus the receptor mediates a signal transduction pathway for which ligand is responsible.

Alternatively, the effect may be one in which the antibody elicits a physiological result by virtue of binding to an effector binding site on the target molecule. In this case the target, a receptor having an endogenous ligand which may be absent or defective in the disease or pathology, binds the antibody as a surrogate effector ligand, initiating a receptor-based signal transduction event by the receptor.

A therapeutically effective amount of an antibody of the invention relates generally to the amount needed to achieve a therapeutic objective. As noted above, this may be a binding interaction between the antibody and its target antigen that, in certain cases, interferes with the functioning of the target, and in other cases, promotes a physiological response. The amount required to be administered will furthermore depend on the binding affinity of the antibody for its specific antigen, and will also depend on the rate at which an administered antibody is depleted from the free volume other subject to which it is administered. Common ranges for therapeutically effective dosing of an antibody or antibody fragment of the invention may be, by way of nonlimiting example, from about 0.1 mg/kg body weight to about 50 mg/kg body weight Common dosing frequencies may range, for example, from twice daily to once a week.

### Pharmaceutical Compositions of Antibodies

Antibodies specifically binding a protein of the invention, as well as other molecules identified by the screening assays disclosed herein, can be administered for the treatment of various disorders in the form of pharmaceutical compositions. Principles and considerations involved in preparing such compositions, as well as guidance in the choice of components are provided, for example, in Remington: The Science And Practice Of Pharmacy 19th ed. (Alfonso R. Gennaro, et al., editors) Mack Pub. Co., Easton, Pa. : 1995; Drug Absorption Enhancement : Concepts, Possibilities, Limitations, And Trends, Harwood Academic Publishers, Langhorne, Pa., 1994; and Peptide And Protein Drug Delivery (Advances In Parenteral Sciences, Vol. 4), 1991, M. Dekker, New York.

If the antigenic protein is intracellular and whole antibodies are used as inhibitors, internalizing antibodies are preferred. However, liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, e.g., Marasco et al., Proc. Natl. Acad. Sci. USA, 90: 7889-7893 (1993). The formulation herein can also contain more than one active compound as necessary for the particular indication being treated, preferably those with complementary activities that do not adversely affect each other. Alternatively, or in addition, the composition can comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients can also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macmemulsions.

The formulations to be used for in vivo administration must be sterile. This is readily accomplished by filtration through sterile filtration membranes.

Sustained-release preparations can be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

### ELISA Assay

An agent for detecting an analyte protein is an antibody capable of binding to an analyte protein, preferably an antibody with a detectable label. Antibodies can be polyclonal, or more preferably, monoclonal. An intact antibody, or a fragment thereof (e.g., F_{ab} or F_{(ab)2}) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. Included within the usage of the term "biological sample", therefore, is blood and a fraction or component of blood including blood serum, blood plasma, or lymph. That is, the detection method of the invention can be used to detect an analyte mRNA, protein, or genomic DNA in a biological sample in vitro as well as in vivo. For example, in vitro techniques for detection of an analyte mRNA include Northern hybridizations and in situ hybridizations. In vitro techniques for detection of an analyte protein include enzyme linked immunosorbent assays (ELISAs), Western blots, immunoprecipitations, and immunofluorescence. In vitro techniques for detection of an analyte genomic DNA include Southern hybridizations. Procedures for conducting immunoassays are described, for example in "ELISA: Theory and Practice: Methods in Molecular Biology", Vol. 42, J. R. Crowther (Ed.) Human Press, Totowa, NJ, 1995; "Immunoassay", E. Diamandis and T. Christopoulus, Academic Press, Inc., San Diego, CA, 1996; and "Practice and Thory of Enzyme Immunoassays", P. Tijssen, Elsevier Science Publishers, Amsterdam, 1985. Furthermore, in vivo techniques for detection of an analyte protein include introducing into a subject a labeled anti-an analyte protein antibody. For example, the antibody can be labeled with a radioactive marker whose presence and location in a subject can be detected by standard imaging techniques.

### NOVX Recombinant Expression Vectors and Host Cells

Another aspect of the invention pertains to vectors, preferably expression vectors, containing a nucleic acid encoding an NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e*.*g*., bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g.,* in an *in vitro* transcription/translation system or in a host cell when the vector is introduced into the host cell).

The term "regulatory sequence" is intended to includes promoters, enhancers and other expression control elements (*e*.*g*., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e*.*g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e*.*g*., NOVX proteins, mutant forms of NOVX proteins, fusion proteins, etc.).

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. *Gene* 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann *et al.,* (1988) *Gene* 69:301-315) and pET 11d (Studier *et al.,* GENE EXPRESSION TECHNOLOGY: METHODS IN ENZYMOLOGY 185, Academic Press, San Diego, Calif. (1990) 60-89).

One strategy to maximize recombinant protein expression in *E*. *coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli (see, e.g*., Wada, *et al.,* 1992. *Nucl. Acids Res.* 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSec1 (Baldari, *et al.,* 1987. *EMBOJ.* 6: 229-234), pMFa (Kurjan and Herskowitz, 1982. *Cell* 30: 933-943), pJRY88 (Schultz *et al*., 1987. *Gene* 54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells *(e.g.,* SF9 cells) include the pAc series (Smith, *et al.,* 1983. *Mol. Cell. Biol.* 3: 2156-2165) and the pVL series (Lucklow and Summers, 1989. *Virology* 170: 31-39).

In yet another embodiment, a nucleic acid of the invention is expressed in mammalian cells using a mammalian expression vector. Examples of mammalian expression vectors include pCDM8 (Seed, 1987. *Nature* 329: 840) and pMT2PC (Kaumian, *et al*., 1987. *EMBO J.* 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e*.*g*., Chapters 16 and 17 of Sambrook, *et al*., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (*e.g*., tissue-specific regulatory elements are used to express the nucleic acid). Tissue-specific regulatory elements are known in the art. Non-limiting examples of suitable tissue-specific promoters include the albumin promoter (liver-specific; Pinkert, *et al*., 1987. *Genes Dev.* 1: 268-277), lymphoid-specific promoters (Calame and Eaton, 1988. *Adv. Immunol.* 43: 235-275), in particular promoters of T cell receptors (Winoto and Baltimore, 1989. *EMBO J.* 8: 729-733) and immunoglobulins (Banerji, *et al.,* 1983. *Cell* 33: 729-740; Queen and Baltimore, 1983. *Cell* 33: 741-748), neuron-specific promoters (*e*.*g*., the neurofilament promoter; Byrne and Ruddle, 1989. *Proc. Natl. Acad. Sci. USA* 86: 5473-5477), pancreas-specific promoters (Edlund, *et al*., 1985. *Science* 230: 912-916), and mammary gland-specific promoters (*e*.*g*., milk whey promoter; U.S. Pat. No. 4,873,316 and European Application Publication No. 264,166). Developmentally-regulated promoters are also encompassed, *e*.*g*., the murine hox promoters (Kessel and Gruss, 1990. *Science* 249: 374-379) and the α-fetoprotein promoter (Campes and Tilghman, 1989. *Genes Dev*. 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, *et al*., "Antisense RNA as a molecular tool for genetic analysis," *Reviews-Trends in Genetics,* Vol. 1(1) 1986. Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli,* insect cells, yeast or mammalian cells (such as Chinese hamster ovary cells (CHO) or COS cells). Other suitable host cells are known to those skilled in the art. Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al.* (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (*e.g*., cells that have incorporated the selectable marker gene will survive, while the other cells die).

A host cell of the invention, such as a prokaryotic or eukaryotic host cell in culture, can be used to produce (*i*.*e*., express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

### Transgenic NOVX Animals

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g*., an embryonic cell of the animal, prior to development of the animal.

A transgenic animal of the invention can be created by introducing NOVX-encoding nucleic acid into the male pronuclei of a fertilized oocyte (*e.g*., by microinjection, retroviral infection) and allowing the oocyte to develop in a pseudopregnant female foster animal. The human NOVX cDNA sequences SEQ ID NOS:1, 3, 5, 7, 9, 11,13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 can be introduced as a transgene into the genome of a non-human animal. Alternatively, a non-human homologue of the human NOVX gene, such as a mouse NOVX gene, can be isolated based on hybridization to the human NOVX cDNA (described further *supra*) and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene-encoding NOVX protein can further be bred to other transgenic animals carrying other transgenes.

To create a homologous recombinant animal, a vector is prepared which contains at least a portion of an NOVX gene into which a deletion, addition or substitution has been introduced to thereby alter, *e*.*g*., functionally disrupt, the NOVX gene. The NOVX gene can be a human gene (*e*.*g*., the cDNA of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93), but more preferably, is a non-human homologue of a human NOVX gene. For example, a mouse homologue of human NOVX gene of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 can be used to construct a homologous recombination vector suitable for altering an endogenous NOVX gene in the mouse genome. In one embodiment, the vector is designed such that, upon homologous recombination, the endogenous NOVX gene is functionally disrupted (*i*.*e*., no longer encodes a functional protein; also referred to as a "knock out" vector).

Alternatively, the vector can be designed such that, upon homologous recombination, the endogenous NOVX gene is mutated or otherwise altered but still encodes functional protein (*e*.*g*., the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e.g.,* Thomas, *et al*., 1987. *Cell* 51: 503 for a description of homologous recombination vectors. The vector is ten introduced into an embryonic stem cell line (*e*.*g*., by electroporation) and cells in which the introduced NOVX gene has homologously-recombined with the endogenous NOVX gene are selected. *See, e*.*g*., Li, *et al*., 1992. *Cell* 69: 915.

The selected cells are then injected into a blastocyst of an animal (*e*.*g*., a mouse) to form aggregation chimeras. *See, e*.*g*., Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. *Curr. Opin. Biotechnol*. 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e*.*g*., Lakso, *et al*., 1992. *Proc. Natl. Acad. Sci. USA 89:* 6232-6236. Another example of a recombinase system is the FLP recombinase system of *Saccharomyces cerevisiae. See,* O'Gorman, *et al*., 1991. *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e*.*g*., by mating two transgenic animals, one containing a transgene encoding a selected protein and the other containing a transgene encoding a recombinase.

Clones of the non-human transgenic animals described herein can also be produced according to the methods described in Wilmut, *et al*., 1997. *Nature* 385: 810-813. In brief, a cell (*e*.*g*., a somatic cell) from the transgenic animal can be isolated and induced to exit the growth cycle and enter Go phase. The quiescent cell can then be fused, *e*.*g*., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell *(e.g.,* the somatic cell) is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e*.*g*., intravenous, intradermal, subcutaneous, oral (*e*.*g*., inhalation), transdermal (*i*.*e*., topical), transmucosal, and rectal administration. Solutions or suspensions used for parenteral,' intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e*.*g*., an NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof. Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e*.*g*., a gas such as carbon dioxide, or a nebulizer. Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (*see, e.g.,* U.S. Patent No. 5,328,470) or by stereotactic injection (*see, e*.*g*., Chen, *et al*., 1994. *Proc. Natl. Acad Sci. USA* 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e*.*g*., retroviral vectors, the pharmaceutical preparation can include one or more cells that produce the gene delivery system.

The pharmaceutical compositions can be included in a container, pack, or dispenser together with instructions for administration.

### Screening and Detection Methods

The isolated nucleic acid molecules of the invention can be used to express NOVX protein (*e*.*g*., via a recombinant expression vector in a host cell in gene therapy applications), to detect NOVX mRNA (*e*.*g*., in a biological sample) or a genetic lesion in an NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein (*e*.*g*.; diabetes (regulates insulin release); obesity (binds and transport lipids); metabolic disturbances associated with obesity, the metabolic syndrome X as well as anorexia and wasting disorders associated with chronic diseases and various cancers, and infectious disease(possesses anti-microbial activity) and the various dyslipidemias. In addition, the anti-NOVX antibodies of the invention can be used to detect and isolate NOVX proteins and modulate NOVX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra.*

### Screening Assays

The invention provides a method (also referred to herein as a "screening assay") for identifying modulators, *i*.*e*., candidate or test compounds or agents (*e*.*g*., peptides, peptidomimetics, small molecules or other drugs) that bind to NOVX proteins or have a stimulatory or inhibitory effect on, *e*.*g*., NOVX protein expression or NOVX protein activity. The invention also includes compounds identified in the screening assays described herein.

In one embodiment, the invention provides assays for screening candidate or test compounds which bind to or modulate the activity of the membrane-bound form of an NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e*.*g*., Lam, 1997. *Anticancer Drug Design* 12: 145.

A "small molecule" as used herein, is meant to refer to a composition that has a molecular weight of less than about 5 kD and most preferably less than about 4 kD. Small molecules can be, *e.g*., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention. Examples of methods for the synthesis of molecular libraries can be found in the art, for example in: DeWitt, *et al*., 1993. *Proc. Natl. Acad. Sci. U.S.A*. 90: 6909; Erb, *et al.,* 1994. *Proc. Natl. Acad. Sci. U.S.A*. 91: 11422; Zuckermann, *et al*., 1994. *J. Med. Chem.* 37: 2678; Cho, *et al.,* 1993. *Science* 261: 1303; Carrell, *et al*., 1994. *Angew. Chem. Int. Ed. Engl.* 33: 2059; Carell, *et al*., 1994. *Angew. Chem. Int. Ed. Engl.* 33: 2061; and Gallop, *et al.,* 1994. *J. Med. Chem.* 37:1233.

Libraries of compounds may be presented in solution (*e*.*g*., Houghten, 1992. *Biotechniques* 13: 412-421), or on beads (Lam, 1991. *Nature* 354: 82-84), on chips (Fodor, 1993. *Nature* 364: 555-556), bacteria (Ladner, U.S. Patent No. 5,223,409), spores (Ladner, U.S. Patent 5,233,409), plasmids (Cull, *et al*., 1992. *Proc. Natl. Acad. Sci. USA 89:* 1865-1869) or on phage (Scott and Smith, 1990. *Science* 249: 386-390; Devlin, 1990. *Science* 249: 404-406; Cwirla, *et al.,* 1990. *Proc. Natl. Acad. Sci. U.S.A.* 87: 6378-6382; Felici, 1991. *J. Mol. Biol.* 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to an NOVX protein determined. The cell, for example, can of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form ofNOVX protein, or a biologically-active portion thereof, on the cell surface with a known compound which binds NOVX to form an assay mixture, contacting the assay mixture with a test compound, and determining the ability of the test compound to interact with an NOVX protein, wherein determining the ability of the test compound to interact with an NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form of NOVX protein, or a biologically-active portion thereof, on the cell surface with a test compound and determining the ability of the test compound to modulate (*e*.*g*., stimulate or inhibit) the activity of the NOVX protein or biologically-active portion thereof. Determining the ability of the test compound to modulate the activity of NOVX or a biologically-active portion thereof can be accomplished, for example, by determining the ability of the NOVX protein to bind to or interact with an NOVX target molecule. As used herein, a "target molecule" is a molecule with which an NOVX protein binds or interacts in nature, for example, a molecule on the surface of a cell which expresses an NOVX interacting protein, a molecule on the surface of a second cell, a molecule in the extracellular milieu, a molecule associated with the internal surface of a cell membrane or a cytoplasmic molecule. An NOVX target molecule can be a non-NOVX molecule or an NOVX protein or polypeptide of the invention. In one embodiment, an NOVX target molecule is a component of a signal transduction pathway that facilitates transduction of an extracellular signal (*e.g.* a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with an NOVX target molecule can be accomplished by one of the methods described above for determining direct binding. In one embodiment, determining the ability of the NOVX protein to bind to or interact with an NOVX target molecule can be accomplished by determining the activity of the target molecule. For example, the activity of the target molecule can be determined by detecting induction of a cellular second messenger of the target (*i*.*e*. intracellular Ca²⁺, diacylglycerol, IP₃, etc.), detecting catalytic/enzymatic activity of the target an appropriate substrate, detecting the induction of a reporter gene (comprising an NOVX-responsive regulatory element operatively linked to a nucleic acid encoding a detectable marker, *e.g.*, luciferase), or detecting a cellular response, for example, cell survival, cellular differentiation, or cell proliferation.

In yet another embodiment, an assay of the invention is a cell-free assay comprising contacting an NOVX protein or biologically-active portion thereof with a test compound and determining the ability of the test compound to bind to the NOVX protein or biologically-active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with an NOVX protein, wherein determining the ability of the test compound to interact with an NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (*e*.*g*. stimulate or inhibit) the activity of the NOVX protein or biologically-active portion thereof. Determining the ability of the test compound to modulate the activity of NOVX can be accomplished, for example, by determining the ability of the NOVX protein to bind to an NOVX target molecule by one of the methods described above for determining direct binding. In an alternative embodiment, determining the ability of the test compound to modulate the activity of NOVX protein can be accomplished by determining the ability of the NOVX protein further modulate an NOVX target molecule. For example, the catalytic/enzymatic activity of the target molecule on an appropriate substrate can be determined as described, *supra*.

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with an NOVX protein, wherein determining the ability of the test compound to interact with an NOVX protein comprises determining the ability of the NOVX protein to preferentially bind to or modulate the activity of an NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecyhnaltoside, octanoyl-N-methylglucamide, decanoyl-N-methylglucamide, Triton® X-100, Triton® X-114, Thesit®; Isotridecypoly(ethylene glycol ether)ₙ, N-dodecyl--N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (*e*.*g*., at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (*e*.*g*., biotinylation kit, Pierce Chemicals, Rockford, Ill.), and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation. Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX mRNA or protein expression based upon this comparison. For example, when expression of NOVX mRNA or protein is greater (*i.e*., statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay *(see, e.g*., U.S. Patent No. 5,283,317; Zervos, *et al*., 1993. *Cell* 72: 223-232; Madura, *et al*., 1993. *J. Biol. Chem.* 268: 12046-12054; Bartel, *et al*., 1993. *Biotechniques* 14: 920-924; Iwabuchi, *et al.,* 1993. *Oncogene* 8: 1693-1696; and Brent WO 94/10300), to identify other proteins that bind to or interact with NOVX ("NOVX-binding proteins" or "NOVX-bp") and modulate NOVX activity. Such NOVX-binding proteins are also likely to be involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (*e*.*g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo*, forming an NOVX-dependent complex, the DNA-binding and activation domains of the transcription factor are brought into close proximity. This proximity allows transcription of a reporter gene (*e*.*g*., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (*ii*) identify an individual from a minute biological sample (tissue typing); and (*iii*) aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the NOVX sequences, SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or fragments or derivatives thereof, can be used to map the location of the NOVX genes, respectively, on a chromosome. The mapping of the NOVX sequences to chromosomes is an important first step in correlating these sequences with genes associated with disease.

Briefly, NOVX genes can be mapped to chromosomes by preparing PCR primers (preferably 15-25 bp in length) from the NOVX sequences. Computer analysis of the NOVX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the NOVX sequences will yield an amplified fragment.

Somatic cell hybrids are prepared by fusing somatic cells from different mammals (*e*.*g*., human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e*.*g*., D'Eustachio, *et al., 1983. Science* 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the NOVX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, *et al*., HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e.g.,* in McKusick, MENDELIAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e.g*., Egeland, *et al*., 1987. *Nature*, 325: 783-787.

Moreover, differences in the DNA sequences between individuals affected and unaffected with a disease associated with the NOVX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The NOVX sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If predicted coding sequences, such as those in SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93 are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (*e.g*., blood, serum, cells, tissue) to thereby determine whether an individual is afflicted with a disease or disorder, or is at risk of developing a disorder, associated with aberrant NOVX expression or activity. The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers. The invention also provides for prognostic (or predictive) assays for determining whether an individual is at risk of developing a disorder associated with NOVX protein, nucleic acid expression or activity. For example, mutations in an NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents (*e*.*g*., drugs) for therapeutic or prophylactic treatment of an individual based on the genotype of the individual (*e*.*g*., the genotype of the individual examined to determine the ability of the individual to respond to a particular agent.)

Yet another aspect of the invention pertains to monitoring the influence of agents (*e*.*g*., drugs, compounds) on the expression or activity of NOVX in clinical trials.

These and other agents are described in further detail in the following sections.

### Diagnostic Assays

An exemplary method for detecting the presence or absence of NOVX in a biological sample involves obtaining a biological sample from a test subject and contacting the biological sample with a compound or an agent capable of detecting NOVX protein or nucleic acid (*e*.*g*., mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

An agent for detecting NOVX protein is an antibody capable of binding to NOVX protein, preferably an antibody with a detectable label. Antibodies can be polyclonal, or more preferably, monoclonal. An intact antibody, or a fragment thereof *(e.g.,* Fab or F(ab')₂) can be used. The term "labeled", with regard to the probe or antibody, is intended to encompass direct labeling of the probe or antibody by coupling *(i.e.,* physically linking) a detectable substance to the probe or antibody, as well as indirect labeling of the probe or antibody by reactivity with another reagent that is directly labeled. Examples of indirect labeling include detection of a primary antibody using a fluoreseently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example, *in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations, and immunofluorescence. *In vitro* techniques for detection of NOVX genomic DNA include Southern hybridizations. Furthermore, *in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid (*e*.*g*., mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e*.*g*., serum), cell sample, or tissue.

Furthermore, the prognostic assays described herein can be used to determine whether a subject can be administered an agent (*e*.*g*., an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (*e*.*g*., wherein the presence of NOVX protein or nucleic acid is diagnostic for a subject that can be administered the agent to treat a disorder associated with aberrant NOVX expression or activity).

The methods of the invention can also be used to detect genetic lesions in an NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding an NOVX-protein, or the misexpression of the NOVX gene. For example, such genetic lesions can be detected by ascertaining the existence of at least one of: (*i*) a deletion of one or more nucleotides from an NOVX gene; (*ii*) an addition of one or more nucleotides to an NOVX gene; *(iii)* a substitution of one or more nucleotides of an NOVX gene, (iv) a chromosomal rearrangement of an NOVX gene; (v) an alteration in the level of a messenger RNA transcript of an NOVX gene, (vi) aberrant modification of an NOVX gene, such as of the methylation pattern of the genomic DNA, (*vii*) the presence of a non-wild-type splicing pattern of a messenger RNA transcript of an NOVX gene, (*viii*) a non-wild-type level of an NOVX protein, (*ix*) allelic loss of an NOVX gene, and (*x*) inappropriate post-translational modification of an NOVX protein. As described herein, there are a large number of assay techniques known in the art which can be used for detecting lesions in an NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) *(see, e*.*g*., U.S. Patent Nos. 4,683,195 and 4,683,202), such as anchor PCR or RACE PCR, or, alternatively, in a ligation chain reaction (LCR) *(see, e*.*g*., Landegran, *et al*., 1988. *Science* 241: 1077-1080; and Nakazawa, *et al*., 1994. *Proc. Natl. Acad. Sci. USA* 91: 360-364), the latter of which can be particularly useful for detecting point mutations in the NOVX-gene (*see,* Abravaya, *et al*., 1995. *Nucl. Acids Res.* 23: 675-682). This method can include the steps of collecting a sample of cells from a patient, isolating nucleic acid (*e*.*g*., genomic, mRNA or both) from the cells of the sample, contacting the nucleic acid sample with one or more primers that specifically hybridize to an NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication *(see,* Guatelli, *et al.,* 1990. *Proc. Natl. Acad. Sci. USA* 87:1874-1878), transcriptional amplification system *(see,* Kwoh, *et al.,* 1989. *Proc. Natl. Acad. Sci. USA* 86: 1173-1177); Qβ Replicase *(see,* Lizardi, *et al,* 1988. *BioTechnology* 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in an NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes *(see, e.g.,* U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, *e*.*g*., DNA or RNA, to high-density arrays containing hundreds or thousands of oligonucleotides probes. *See, e*.*g*., Cronin, *et al*., 1996. *Human Mutation* 7: 244-255; Kozal, *et al*., 1996. *Nat*. *Med.* 2: 753-759. For example, genetic mutations in NOVX can be identified in two dimensional arrays containing light-generated DNA probes as described in Cronin, *et al*., *supra.* Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. *Proc. Natl. Acad. Sci. USA* 74: 560 or Sanger, 1977. *Proc. Natl. Acad. Sci. USA* 74: 5463. It is also contemplated that any of a variety of automated sequencing procedures can be utilized when performing the diagnostic assays *(see, e.g.,* Naeve, *et al*., 1995. *Biotechniques* 19: 448), including sequencing by mass spectrometry (see, *e.g.,* PCT International Publication No. WO 94/16101; Cohen, *et al.,* 1996. *Adv. Chromatography 36:* 127-162; and Griffin, *et al.,* 1993. *Appl. Biochem. Biotechnol.* 38: 147-159).

Other methods for detecting mutations in the NOVX gene include methods in which protection from cleavage agents is used to detect mismatched bases in RNA/RNA or RNA/DNA heteroduplexes. *See, e.g.,* Myers, *et al.,* 1985. *Science* 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g.,* Cotton, *et al.,* 1988. *Proc. Natl. Acad Sci. USA* 85: 4397; Saleeba, *et al.,* 1992. *Methods Enzymol.* 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E*. *coli* cleaves A at G/A mismatches and the thymidine DNA glycosylase from HeLa cells cleaves T at G/T mismatches. *See, e*.*g*., Hsu, *et al*., 1994. *Carcinogenesis* 15: 1657-1662. According to an exemplary embodiment, a probe based on an NOVX sequence, *e*.*g*., a wild-type NOVX sequence, is hybridized to a cDNA or other DNA product from a test cell(s). The duplex is treated with a DNA mismatch repair enzyme, and the cleavage products, if any, can be detected from electrophoresis protocols or the like. *See, e*.*g*., U.S. Patent No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids. *See, e*.*g*., Orita, *et al*., 1989. *Proc. Natl. Acad. Sci. USA*: 86: 2766; Cotton, 1993. *Mutat. Res*. 285: 125-144; Hayashi, 1992. *Genet. Anal. Tech. Appl.* 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e*.*g*., Keen, *et al*., *1991. Trends Genet.* 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, *et al.,* 1985. *Nature* 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e*.*g*., Rosenbaum and Reissner, 1987. *Biophys. Chem.* 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, *et al.,* 1986. *Nature* 324: 163; Saiki, *et al.,* 1989. *Proc. Natl. Acad. Sci. USA* 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e*.*g*., Gibbs, *et al.,* 1989. *Nucl. Acids Res.* 17: 2437-2448) or at the extreme 3'-terminus of one primer where, under appropriate conditions, mismatch can prevent, or reduce polymerase extension *(see, e.g.,* Prossner, 1993. *Tibtech.* 11: 238). In addition it may be desirable to introduce a novel restriction site in the region of the mutation to create cleavage-based detection. *See, e*.*g*., Gasparini, *et al*., 1992. *Mol. Cell Probes* 6: 1. It is anticipated that in certain embodiments amplification may also be performed using *Taq* ligase for amplification. *See, e*.*g*., Barany, *1991. Proc. Natl. Acad. Sci. USA* 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e*.*g*., in clinical settings to diagnose patients exhibiting symptoms or family history of a disease or illness involving an NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity (*e.g*., NOVX gene expression), as identified by a screening assay described herein can be administered to individuals to treat (prophylactically or therapeutically) disorders (The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers.) In conjunction with such treatment, the pharmacogenomics (*i*.*e*., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e*.*g*., drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g*., Eichelbaum, 1996. *Clin. Exp. Pharmacol. Physiol*., 23: 983-985; Linder, 1997. *Clin. Chem.,* 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e*.*g*., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with an NOVX modulator, such as a modulator identified by one of the exemplary screening assays described herein.

### Monitoring of Effects During Clinical Trials

Monitoring the influence of agents (*e.g*., drugs, compounds) on the expression or activity of NOVX (*e.g*., the ability to modulate aberrant cell proliferation and/or differentiation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (*e.g*., compound, drug or small molecule) that modulates NOVX activity (*e.g*., identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i*.*e*., a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e*.*g*., an agonist, antagonist, protein, peptide, peptidomimetic, nucleic acid, small molecule, or other drug candidate identified by the screening assays described herein) comprising the steps of (*i*) obtaining a pre-administration sample from a subject prior to administration of the agent; (*ii*) detecting the level of expression of an NOVX protein, mRNA, or genomic DNA in the preadministration sample; (*iii*) obtaining one or more post-administration samples from the subject; (*iv*) detecting the level of expression or activity of the NOVX protein, mRNA, or genomic DNA in the post-administration samples; (*v*) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, *i.e.,* to increase the effectiveness of the agent. Alternatively, decreased administration of the agent may be desirable to decrease expression or activity of NOVX to lower levels than detected, *i.e.,* to decrease the effectiveness of the agent.

### Methods of Treatment

The invention provides for both prophylactic and therapeutic methods of treating a subject at risk of (or susceptible to) a disorder or having a disorder associated with aberrant NOVX expression or activity. The disorders include cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, seleroderma, obesity, transplantation, adrenoleukodystrophy, congenital adrenal hyperplasia, prostate cancer, neoplasm; adenocarcinoma, lymphoma, uterus cancer, fertility, hemophilia, hypercoagulation, idiopathic thrombocytopenic purpura, immunodeficiencies, graft versus host disease, AIDS, bronchial asthma, Crohn's disease; multiple sclerosis, treatment of Albright Hereditary Ostoeodystrophy, and other diseases, disorders and conditions of the like.

These methods of treatment will be discussed more fully, below.

### Disease and Disorders

Diseases and disorders that are characterized by increased (relative to a subject not suffering from the disease or disorder) levels or biological activity may be treated with Therapeutics that antagonize (*i.e*., reduce or inhibit) activity. Therapeutics that antagonize activity may be administered in a therapeutic or prophylactic manner. Therapeutics that may be utilized include, but are not limited to: (*i*) an aforementioned peptide, or analogs, derivatives, fragments or homologs thereof; (*ii*) antibodies to an aforementioned peptide; (*iii*) nucleic acids encoding an aforementioned peptide; (iv) administration of antisense nucleic acid and nucleic acids that are "dysfuncfional" (*i*.*e*., due to a heterologous insertion within the coding sequences of coding sequences to an aforementioned peptide) that are utilized to "knockout" endogenous function of an aforementioned peptide by homologous recombination *(see, e*.*g*., Capecchi, 1989. *Science* 244: 1288-1292); or (v) modulators (*i*.*e*., inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase *(i.e.,* are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e*.*g*., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e*.*g*., by Western blot analysis, immunoprecipitation followed by sodium dodecyl sulfate (SDS) polyacrylamide gel electrophoresis, immunocytochemistry, etc.) and/or hybridization assays to detect expression of mRNAs (*e.g*., Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, an NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of an NOVX protein, a peptide, an NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and and-NOVX antibodies. These modulatory methods can be performed in *vitro* (*e*.*g*., by culturing the cell with the agent) or, alternatively, *in vivo* (*e*.*g*., by administering the agent to a subject). As such, the invention provides methods of treating an individual afflicted with a disease or disorder characterized by aberrant expression or activity of an NOVX protein or nucleic acid molecule. In one embodiment, the method involves administering an agent (*e.g*., an agent identified by a screening assay described herein), or combination of agents that modulates (*e*.*g*., up-regulates or down-regulates) NOVX expression or activity. In another embodiment, the method involves administering an NOVX protein or nucleic acid molecule as therapy to compensate for reduced or aberrant NOVX expression or activity.

Stimulation of NOVX activity is desirable in situations in which NOVX is abnormally downregulated and/or in which increased NOVX activity is likely to have a beneficial effect. One example of such a situation is where a subject has a disorder characterized by aberrant cell proliferation and/or differentiation (*e*.*g*., cancer or immune associated disorders). Another example of such a situation is where the subject has a gestational disease (*e*.*g*., preclampsia).

### Determination of the Biological Effect of the Therapeutic

In various embodiments of the invention, suitable *in vitro or in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The NOVX nucleic acids and proteins of the invention are useful in potential prophylactic and therapeutic applications implicated in a variety of disorders including, but not limited to: metabolic disorders, diabetes, obesity, infectious disease, anorexia, cancer-associated cancer, neurodegenerative disorders, Alzheimer's Disease, Parkinson's Disorder, immune disorders, hematopoietic disorders, and the various dyslipidemias, metabolic disturbances associated with obesity, the metabolic syndrome X and wasting disorders associated with chronic diseases and various cancers.

As an example, a cDNA encoding the NOVX protein of the invention may be useful in gene therapy, and the protein may be useful when administered to a subject in need thereof. By way of non-limiting example, the compositions of the invention will have efficacy for treatment of patients suffering from: metabolic disorders, diabetes, obesity, infectious disease, anorexia, cancer-associated cachexia, cancer, neurodegenerative disorders, Alzheimer's Disease, Parkinson's Disorder, immune disorders, hematopoietic disorders, and the various dyslipidemias.

Both the novel nucleic acid encoding the NOVX protein, and the NOVX protein of the invention, or fragments thereof, may also be useful in diagnostic applications, wherein the presence or amount of the nucleic acid or the protein are to be assessed. A further use could be as an anti-bacterial molecule (*i.e.,* some peptides have been found to possess anti-bacterial properties). These materials are further useful in the generation of antibodies, which immunospecifically-bind to the novel substances of the invention for use in therapeutic or diagnostic methods.

The invention will be further described in the following examples, which do not limit the scope of the invention described in the claims.

### Examples

### EXAMPLE 1: Identification of NOVX Nucleic Acids

TblastN using CuraGen Corporation's sequence file for polypeptides or homologs was run against the Genomic Daily Files made available by GenBank or from files downloaded from the individual sequencing centers. Exons were predicted by homology and the intron/exon boundaries were determined using standard genetic rules. Exons were further selected and refined by means of similarity determination using multiple BLAST (for example, tBlastN, BlastX, and BlastN) searches, and, in some instances, GeneScan and Grail. Expressed sequences from both public and proprietary databases were also added when available to further define and complete the gene sequence. The DNA sequence was then manually corrected for apparent inconsistencies thereby obtaining the sequences encoding the full-length protein.

The novel NOVX target sequences identified in the present invention were subjected to the exon linking process to confirm the sequence. PCR primers were designed by starting at the most upstream sequence available, for the forward primer, and at the most downstream sequence available for the reverse primer. PCR primer sequences were used for obtaining different clones. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the predicted exons to closely related human sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The PCR product derived from exon linking was cloned into the pCR2.1 vector from Invitrogen. The resulting bacterial clone has an insert covering the entire open reading frame cloned into the pCR2.1 vector. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. These procedures provide the sequence reported herein.

Physical clone: Exons were predicted by homology and the intron/exon boundaries were determined using standard genetic rules. Exons were further selected and refined by means of similarity determination using multiple BLAST (for example, tBlastN, BlastX, and BlastN) searches, and, in some instances, GeneScan and Grail. Expressed sequences from both public and proprietary databases were also added when available to further define and complete the gene sequence. The DNA sequence was then manually corrected for apparent inconsistencies thereby obtaining the sequences encoding the full-length protein.

### Example 2: Identification of Single Nucleotide Polymorphisms in NOVX nucleic acid sequences

Variant sequences are also included in this application. A variant sequence can include a single nucleotide polymorphism (SNP). A SNP can, in some instances, be referred to as a "cSNP" to denote that the nucleotide sequence containing the SNP originates as a cDNA. A SNP can arise in several ways. For example, a SNP may be due to a substitution of one nucleotide for another at the polymorphic site. Such a substitution can be either a transition or a transversion. A SNP can also arise from a deletion of a nucleotide or an insertion of a nucleotide, relative to a reference allele. In this case, the polymorphic site is a site at which one allele bears a gap with respect to a particular nucleotide in another allele. SNPs occurring within genes may result in an alteration of the amino acid encoded by the gene at the position of the SNP. Intragenic SNPs may also be silent, when a codon including a SNP encodes the same amino acid as a result of the redundancy of the genetic code. SNPs occurring outside the region of a gene, or in an intron within a gene, do not result in changes in any amino acid sequence of a protein but may result in altered regulation of the expression pattern. Examples include alteration in temporal expression, physiological response regulation, cell type expression regulation, intensity of expression, and stability of transcribed message.

SeqCalling assemblies produced by the exon linking process were selected and extended using the following criteria. Genomic clones having regions with 98% identity to all or part of the initial or extended sequence were identified by BLASTN searches using the relevant sequence to query human genomic databases. The genomic clones that resulted were selected for further analysis because this identity indicates that these clones contain the genomic locus for these SeqCalling assemblies. These sequences were analyzed for putative coding regions as well as for similarity to the known DNA and protein sequences. Programs used for these analyses include Grail, Genscan, BLAST, HMMER, FASTA, Hybrid and other relevant programs.

Some additional genomic regions may have also been identified because selected SeqCalling assemblies map to those regions. Such SeqCalling sequences may have overlapped with regions defined by homology or exon prediction. They may also be included because the location of the fragment was in the vicinity of genomic regions identified by similarity or exon prediction that had been included in the original predicted sequence. The sequence so identified was manually assembled and then may have been extended using one or more additional sequences taken from CuraGen Corporation's human SeqCalling database. SeqCalling fragments suitable for inclusion were identified by the CuraTools™ program SeqExtend or by identifying SeqCalling fragments mapping to the appropriate regions of the genomic clones analyzed.

The regions defined by the procedures described above were then manually integrated and corrected for apparent inconsistencies that may have arisen, for example, from miscalled bases in the original fragments or from discrepancies between predicted exon junctions, EST locations and regions of sequence similarity, to derive the final sequence disclosed herein. When necessary, the process to identify and analyze SeqCalling assemblies and genomic clones was reiterated to derive the full length sequence (Alderbom et al., Determination of Single Nucleotide Polymorphisms by Real-time Pyrophosphate DNA Sequencing. Genome Research. 10 (8) 1249-1265, 2000).

### Example 3. Quantitative expression analysis of clones in various cells and tissues

The quantitative expression of various clones was assessed using microtiter plates containing RNA samples from a variety of normal and pathology-derived cells, cell lines and tissues using real time quantitative PCR (RTQ PCR). RTQ PCR was performed on an Applied Biosystems ABI PRISM® 7700 or an ABI PRISM® 7900 HT Sequence Detection System. Various collections of samples are assembled on the plates, and referred to as Panel 1 (containing normal tissues and cancer cell lines), Panel 2 (containing samples derived from tissues from normal and cancer sources), Panel 3 (containing cancer cell lines), Panel 4 (containing cells and cell lines from normal tissues and cells related to inflammatory conditions), Panel 5D/51 (containing human tissues and cell lines with an emphasis on metabolic diseases), AI_comprehensive_panel (containing normal tissue and samples from autoimmune diseases), Panel CNSD.01 (containing central nervous system samples from normal and diseased brains) and CNS_neurodegeneration_panel (containing samples from normal and Alzheimer's diseased brains).

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

First, the RNA samples were normalized to reference nucleic acids such as constitutively expressed genes (for example, β-actin and GAPDH). Normalized RNA (5 ul) was converted to cDNA and analyzed by RTQ-PCR using One Step RT-PCR Master Mix Reagents (Applied Biosystems; Catalog No. 4309169) and gene-specific primers according to the manufacturer's instructions.

In other cases, non-normalized RNA samples were converted to single strand cDNA (sscDNA) using Superscript II (Invitrogen Corporation; Catalog No. 18064-147) and random hexamers according to the manufacturer's instructions. Reactions containing up to 10 µg of total RNA were performed in a volume of 20 µl and incubated for 60 minutes at 42°C. This reaction can be scaled up to 50 µg of total RNA in a final volume of 100 µl. sscDNA samples are then normalized to reference nucleic acids as described previously, using 1X TaqMan® Universal Master mix (Applied Biosystems; catalog No. 4324020), following the manufacturer's instructions.

Probes and primers were designed for each assay according to Applied Biosystems Primer Express Software package (version I for Apple Computer's Macintosh Power PC) or a similar algorithm using the target sequence as input. Default settings were used for reaction conditions and the following parameters were set before selecting primers: primer concentration = 250 nM, primer melting temperature (Tm) range = 58°-60°C, primer optimal Tm = 59°C, maximum primer difference = 2°C, probe does not have 5'G, probe Tm must be 10°C greater than primer Tm, amplicon size 75bp to 100bp. The probes and primers selected (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900nM each, and probe, 200nM.

PCR conditions: When working with RNA samples, normalized RNA from each tissue and each cell line was spotted in each well of either a 96 well or a 384-well PCR plate (Applied Biosystems). PCR cocktails included either a single gene specific probe and primers set, or two multiplexed probe and primers sets (a set specific for the target clone and another gene-specific set multiplexed with the target probe). PCR reactions were set up using TaqMan® One-Step RT-PCR Master Mix (Applied Biosystems, Catalog No. 4313803) following manufacturer's instructions. Reverse transcription was performed at 48°C for 30 minutes followed by amplification/PCR cycles as follows: 95°C 10 min, then 40 cycles of 95°C for 15 seconds, 60°C for 1 minute. Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100.

When working with sscDNA samples, normalized sscDNA was used as described previously for RNA samples. PCR reactions containing one or two sets of probe and primers were set up as described previously, using 1X TaqMan® Universal Master mix (Applied Biosystems; catalog No. 4324020), following the manufacturer's instructions. PCR amplification was performed as follows: 95°C 10 min, then 40 cycles of 95°C for 15 seconds, 60°C for 1 minute. Results were analyzed and processed as described previously.

### Panels 1,1.1,1.2, and 1.3D

The plates for Panels 1, 1.1, 1.2 and 1.3D include 2 control wells (genomic DNA control and chemistry control) and 94 wells containing cDNA from various samples. The samples in these panels are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in these panels are widely available through the American Type Culture Collection (ATCC), a repository for cultured cell lines, and were cultured using the conditions recommended by the ATCC. The normal tissues found on these panels are comprised of samples derived from all major organ systems from single adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose.

In the results for Panels 1, 1.1, 1.2 and 1.3D, the following abbreviations are used:
ca. = carcinoma,
* = established from metastasis,
met = metastasis,
s cell var = small cell variant,
non-s = non-sm = non-small,
squam = squamous,
pl. eff = pl effusion = pleural effusion,
glio = glioma,
astro = astrocytoma, and
neuro = neuroblastoma.

### General_screening_panel_v1.4

The plates for Panel 1.4 include 2 control wells (genomic DNA control and chemistry control) and 94 wells containing cDNA from various samples. The samples in Panel 1.4 are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in Panel 1.4 are widely available through the American Type Culture Collection (ATCC), a repository for cultured cell lines, and were cultured using the conditions recommended by the ATCC. The normal tissues found on Panel 1.4 are comprised of pools of samples derived from all major organ systems from 2 to 5 different adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose. Abbreviations are as described for Panels 1, 1.1, 1.2, and 1.3D.

### Panels 2D and 2.2

The plates for Panels 2D and 2.2 generally include 2 control wells and 94 test samples composed ofRNA or cDNA isolated from human tissue procured by surgeons working in close cooperation with the National Cancer Institute's Cooperative Human Tissue Network (CHTN) or the National Disease Research Initiative (NDRI). The tissues are derived from human malignancies and in cases where indicated many malignant tissues have "matched margins" obtained from noncancerous tissue just adjacent to the tumor. These are termed normal adjacent tissues and are denoted "NAT" in the results below. The tumor tissue and the "matched margins" are evaluated by two independent pathologists (the surgical pathologists and again by a pathologist at NDRI or CHTN). This analysis provides a gross histopathological assessment of tumor differentiation grade. Moreover, most samples include the original surgical pathology report that provides information regarding the clinical stage of the patient. These matched margins are taken from the tissue surrounding (i.e. immediately proximal) to the zone of surgery (designated "NAT", for normal adjacent tissue, in Table RR). In addition, RNA and cDNA samples were obtained from various human tissues derived from autopsies performed on elderly people or sudden death victims (accidents, etc.). These tissues were ascertained to be free of disease and were purchased from various commercial sources such as Clontech (Palo Alto, CA), Research Genetics, and Invitrogen.

### Panel 3D

The plates of Panel 3D are comprised of 94 cDNA samples and two control samples. Specifically, 92 of these samples are derived from cultured human cancer cell lines, 2 samples of human primary cerebellar tissue and 2 controls. The human cell lines are generally obtained from ATCC (American Type Culture Collection), NCI or the German tumor cell bank and fall into the following tissue groups: Squamous cell carcinoma of the tongue, breast cancer, prostate cancer, melanoma, epidermoid carcinoma, sarcomas, bladder carcinomas, pancreatic cancers, kidney cancers, leukemias/lymphomas, ovarian/uterine/cervical, gastric, colon, lung and CNS cancer cell lines. In addition, there are two independent samples of cerebellum. These cells are all cultured under standard recommended conditions and RNA extracted using the standard procedures. The cell lines in panel 3D and 1.3D are of the most common cell lines used in the scientific literature.

### Panels 4D, 4R, and 4.1D

Panel 4 includes samples on a 96 well plate (2 control wells, 94 test samples) composed of RNA (Panel 4R) or cDNA (Panels 4D/4.1D) isolated from various human cell lines or tissues related to inflammatory conditions. Total RNA from control normal tissues such as colon and lung (Stratagene, La Jolla, CA) and thymus and kidney (Clontech) was employed. Total RNA from liver tissue from cirrhosis patients and kidney from lupus patients was obtained from BioChain (Biochain Institute, Inc., Hayward, CA). Intestinal tissue for RNA preparation from patients diagnosed as having Crohn's disease and ulcerative colitis was obtained from the National Disease Research Interchange (NDRI) (Philadelphia, PA).

Astrocytes, lung fibroblasts, dermal fibroblasts, coronary artery smooth muscle cells, small airway epithelium, bronchial epithelium, microvascular dermal endothelial cells, microvascular lung endothelial cells, human pulmonary aortic endothelial cells, human umbilical vein endothelial cells were all purchased from Clonetics (Walkersville, MD) and grown in the media supplied for these cell types by Clonetics. These primary cell types were activated with various cytokines or combinations of cytokines for 6 and/or 12-14 hours, as indicated. The following cytokines were used; EL-1 beta at approximately 1-5ng/ml, TNF alpha at approximately 5-1 Ong/ml, IFN gamma at approximately 20-50ng/ml, IL-4 at approximately 5-10ng/ml, IL-9 at approximately 5-10ng/ml, IL-13 at approximately 5-10ng/ml. Endothelial cells were sometimes starved for various times by culture in the basal media from Clonetics with 0.1 % serum.

Mononuclear cells were prepared from blood of employees at CuraGen Corporation, using Ficoll. LAK cells were prepared from these cells by culture in DMEM 5% FCS (Hyclone), 100µM non essential amino acids (Gibco/Life Technologies, Rockville, MD), 1mM sodium pyruvate (Gibco), mercaptoethanol 5.5x10⁻⁵M (Gibco), and 10mM Hepes (Gibco) and Interleukin 2 for 4-6 days. Cells were then either activated with 10-20ng/ml PMA and 1-2µg/ml ionomycin, IL-12 at 5-10ng/ml, IFN gamma at 20-50ng/ml and IL-18 at 5-10ng/ml for 6 hours. In some cases, mononuclear cells were cultured for 4-5 days in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) with PHA (phytohemagglutinin) or PWM (pokeweed mitogen) at approximately 5µg/ml. Samples were taken at 24, 48 and 72 hours for RNA preparation. MLR (mixed lymphocyte reaction) samples were obtained by taking blood from two donors, isolating the mononuclear cells using Ficoll and mixing the isolated mononuclear cells 1:1 at a final concentration of approximately 2x10⁶cells/ml in DMEM 5% FCS (Hyclone), 100µM non essential amino acids (Gibco), 1mM sodium pyruvate (Gibco), mercaptoethanol (5.5x10⁻⁵M) (Gibco), and 10mM Hepes (Gibco). The MLR was cultured and samples taken at various time points ranging from 1- 7 days for RNA preparation.

Monocytes were isolated from mononuclear cells using CD14 Miltenyi Beads, +ve VS selection columns and a Vario Magnet according to the manufacturer's instructions. Monocytes were differentiated into dendritic cells by culture in DMEM 5% fetal calf serum (FCS) (Hyclone, Logan, UT), 100µM non essential amino acids (Gibco), 1mM sodium pyruvate (Gibco), mercaptoethanol 5.5x10⁻⁵M (Gibco), and 10mM Hepes (Gibco), 50ng/ml GMCSF and 5ng/ml IL-4 for 5-7 days. Macrophages were prepared by culture of monocytes for 5-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and 10% AB Human Serum or MCSF at approximately 50ng/ml. Monocytes, macrophages and dendritic cells were stimulated for 6 and 12-14 hours with lipopolysaccharide (LPS) at 100ng/ml. Dendritic cells were also stimulated with anti-CD40 monoclonal antibody (Phanningen) at 10µg/ml for 6 and 12-14 hours.

CD4 lymphocytes, CD8 lymphocytes and NK cells were also isolated from mononuclear cells using CD4, CD8 and CD56 Miltenyi beads, positive VS selection columns and a Vario Magnet according to the manufacturer's instructions. CD45RA and CD45RO CD4 lymphocytes were isolated by depleting mononuclear cells of CD8, CD56, CD14 and CD19 cells using CD8, CD56, CD14 and CD19 Miltenyi beads and positive selection. CD45RO beads were then used to isolate the CD45RO CD4 lymphocytes with the remaining cells being CD45RA CD4 lymphocytes. CD45RA CD4, CD45RO CD4 and CD8 lymphocytes were placed in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and plated at 10⁶cells/ml onto Falcon 6 well tissue culture plates that had been coated overnight with 0.5µg/ml anti-CD28 (Pharmingen) and 3ug/ml anti-CD3 (OKT3, ATCC) in PBS. After 6 and 24 hours, the cells were harvested for RNA preparation. To prepare chronically activated CD8 lymphocytes, we activated the isolated CD8 lymphocytes for 4 days on anti-CD28 and anti-CD3 coated plates and then harvested the cells and expanded them in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2. The expanded CD8 cells were then activated again with plate bound anti-CD3 and anti-CD28 for 4 days and expanded as before. RNA was isolated 6 and 24 hours after the second activation and after 4 days of the second expansion culture. The isolated NK cells were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2 for 4-6 days before RNA was prepared.

To obtain B cells, tonsils were procured from NDRI. The tonsil was cut up with sterile dissecting scissors and then passed through a sieve. Tonsil cells were then spun down and resupended at 10⁶cells/ml in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). To activate the cells, we used PWM at 5µg/ml or anti-CD40 (Pharmingen) at approximately 10µg/ml and IL-4 at 5-10ng/ml. Cells were harvested for RNA preparation at 24,48 and 72 hours.

To prepare the primary and secondary Th1/Th2 and Tr1 cells, six-well Falcon plates were coated overnight with 10µg/ml anti-CD28 (Pharmingen) and 2µg/ml OKT3 (ATCC), and then washed twice with PBS. Umbilical cord blood CD4 lymphocytes (Poietic Systems, German Town, MD) were cultured at 10⁵-10⁶cells/ml in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (4ng/ml). IL-12 (5ng/ml) and anti-IL4 (1µg/ml) were used to direct to Th1, while EL-4 (5ng/ml) and anti-IFN gamma (1µg/ml) were used to direct to Th2 and IL-10 at 5ng/ml was used to direct to Tr1. After 4-5 days, the activated Th1, Th2 and Tr1 lymphocytes were washed once in DMEM and expanded for 4-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (1ng/ml). Following this, the activated Th1, Th2 and Tr1 lymphocytes were re-stimulated for 5 days with anti-CD28/OKT3 and cytokines as described above, but with the addition of anti-CD95L (1µg/ml) to prevent apoptosis. After 4-5 days, the Th1, Th2 and Tr1 lymphocytes were washed and then expanded again with IL-2 for 4-7 days. Activated Th1 and Th2 lymphocytes were maintained in this way for a maximum of three cycles. RNA was prepared from primary and secondary Th1, Th2 and Tr1 after 6 and 24 hours following the second and third activations with plate bound anti-CD3 and anti-CD28 mAbs and 4 days into the second and third expansion cultures in Interleuldn 2.

The following leukocyte cells lines were obtained from the ATCC: Ramos, EOL-1, KU-812. EOL cells were further differentiated by culture in 0.1mM dbcAMP at 5x10⁵cells/ml for 8 days, changing the media every 3 days and adjusting the cell concentration to 5x10⁵cells/ml. For the culture of these cells, we used DMEM or RPMI (as recommended by the ATCC), with the addition of 5% FCS (Hyclone), 100µM non essential amino acids (Gibco), 1mM sodium pyruvate (Gibco), mercaptoethanol 5.5x10⁻⁵M (Gibco), 10mM Hepes (Gibco). RNA was either prepared from resting cells or cells activated with PMA at 10ng/ml and ionomycin at 1µg/ml for 6 and 14 hours. Keratinocyte line CCD106 and an airway epithelial tumor line NCI-H292 were also obtained from the ATCC. Both were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). CCD1106 cells were activated for 6 and 14 hours with approximately 5 ng/ml TNF alpha and 1ng/ml IL-1 beta, while NCI-H292 cells were activated for 6 and 14 hours with the following cytokines: 5ng/ml IL-4, 5ng/ml IL-9, 5ng/ml IL-13 and 25ng/ml IFN gamma.

For these cell lines and blood cells, RNA was prepared by lysing approximately 10⁷cells/ml using Trizol (Gibco BRL). Briefly, 1/10 volume ofbromochloropropane (Molecular Research Corporation) was added to the RNA sample, vortexed and after 10 minutes at room temperature, the tubes were spun at 14,000 rpm in a Sorvall SS34 rotor. The aqueous phase was removed and placed in a 15ml Falcon Tube. An equal volume of isopropanol was added and left at -20°C overnight. The precipitated RNA was spun down at 9,000 rpm for 15 min in a Sorvall SS34 rotor and washed in 70% ethanol. The pellet was redissolved in 300µl of RNAse-free water and 35µl buffer (Promega) 5µl DTT, 7µl RNAsin and 8µl DNAse were added. The tube was incubated at 37°C for 30 minutes to remove contaminating genomic DNA, extracted once with phenol chloroform and re-precipitated with 1/10 volume of 3M sodium acetate and 2 volumes of 100% ethanol. The RNA was spun down and placed in RNAse free water. RNA was stored at -80°C.

### AI_comprehensive panel_v1.0

The plates for AI_comprehensive panel_v1.0 include two control wells and 89 test samples comprised of cDNA isolated from surgical and postmortem human tissues obtained from the Backus Hospital and Clinomics (Frederick, MD). Total RNA was extracted from tissue samples from the Backus Hospital in the Facility at CuraGen. Total RNA from other tissues was obtained from Clinomics.

Joint tissues including synovial fluid, synovium, bone and cartilage were obtained from patients undergoing total knee or hip replacement surgery at the Backus Hospital. Tissue samples were immediately snap frozen in liquid nitrogen to ensure that isolated RNA was of optimal quality and not degraded. Additional samples of osteoarthritis and rheumatoid arthritis joint tissues were obtained from Clinomics. Normal control tissues were supplied by Clinomics and were obtained during autopsy of trauma victims.

Surgical specimens of psoriatic tissues and adjacent matched tissues were provided as total RNA by Clinomics. Two male and two female patients were selected between the ages of 25 and 47. None of the patients were taking prescription drugs at the time samples were isolated.

Surgical specimens of diseased colon from patients with ulcerative colitis and Crohns disease and adjacent matched tissues were obtained from Clinomics. Bowel tissue from three female and three male Crohn's patients between the ages of 41-69 were used. Two patients were not on prescription medication while the others were taking dexamethasone, phenobarbital, or tylenol. Ulcerative colitis tissue was from three male and four female patients. Four of the patients were taking lebvid and two were on phenobarbital.

Total RNA from post mortem lung tissue from trauma victims with no disease or with emphysema, asthma or COPD was purchased from Clinomics. Emphysema patients ranged in age from 40-70 and all were smokers, this age range was chosen to focus on patients with cigarette-linked emphysema and to avoid those patients with alpha-lanti-trypsin deficiencies. Asthma patients ranged in age from 36-75, and excluded smokers to prevent those patients that could also have COPD. COPD patients ranged in age from 35-80 and included both smokers and non-smokers. Most patients were taking corticosteroids, and bronchodilators.

In the labels employed to identify tissues in the AI_comprehensive panel_v1.0 panel, the following abbreviations are used:
AI = Autoimmunity
Syn = Synovial
Normal = No apparent disease
Rep22 /Rep20 = individual patients
RA = Rheumatoid arthritis
Backus = From Backus Hospital
OA = Osteoarthritis
(SS) (BA) (MF) = Individual patients
Adj = Adjacent tissue
Match control = adjacent tissues
-M = Male
-F = Female
COPD = Chronic obstructive pulmonary disease

### Panels 5D and 5I

The plates for Panel 5D and 5I include two control wells and a variety of cDNAs isolated from human tissues and cell lines with an emphasis on metabolic diseases. Metabolic tissues were obtained from patients enrolled in the Gestational Diabetes study. Cells were obtained during different stages in the differentiation of adipocytes from human mesenchymal stem cells. Human pancreatic islets were also obtained.

In the Gestational Diabetes study subjects are young (18 - 40 years), otherwise healthy women with and without gestational diabetes undergoing routine (elective) Caesarean section. After delivery of the infant, when the surgical incisions were being repaired/closed, the obstetrician removed a small sample (<1 cc) of the exposed metabolic tissues during the closure of each surgical level. The biopsy material was rinsed in sterile saline, blotted and fast frozen within 5 minutes from the time of removal. The tissue was then flash frozen in liquid nitrogen and stored, individually, in sterile screw-top tubes and kept on dry ice for shipment to or to be picked up by CuraGen. The metabolic tissues of interest include uterine wall (smooth muscle), visceral adipose, skeletal muscle (rectus) and subcutaneous adipose. Patient descriptions are as follows:
Patient 2 Diabetic Hispanic, overweight, not on insulin
Patient 7-9 Nondiabetic Caucasian and obese (BMI>30)
Patient 10 Diabetic Hispanic, overweight, on insulin
Patient 11 Nondiabetic African American and overweight
Patient 12 Diabetic Hispanic on insulin

Adipocyte differentiation was induced in donor progenitor cells obtained from Osirus (a division of Clonetics/BioWhittaker) in triplicate, except for Donor 3U which had only two replicates. Scientists at Clonetics isolated, grew and differentiated human mesenchymal stem cells (HuMSCs) for CuraGen based on the published protocol found in Mark F. Pittenger, et al., Multilineage Potential of Adult Human Mesenchymal Stem Cells Science Apr 2 1999: 143-147. Clonetics provided Trizol lysates or frozen pellets suitable for mRNA isolation and ds cDNA production. A general description of each donor is as follows:
Donor 2 and 3 U: Mesenchymal Stem cells, Undifferentiated Adipose
Donor 2 and 3 AM: Adipose, AdiposeMidway Differentiated
Donor 2 and 3 AD: Adipose, Adipose Differentiated

Human cell lines were generally obtained from ATCC (American Type Culture Collection), NCI or the German tumor cell bank and fall into the following tissue groups: kidney proximal convoluted tubule, uterine smooth muscle cells, small intestine, liver HepG2 cancer cells, heart primary stromal cells, and adrenal cortical adenoma cells. These cells are all cultured under standard recommended conditions and RNA extracted using the standard procedures. All samples were processed at CuraGen to produce single stranded cDNA.

Panel 51 contains all samples previously described with the addition of pancreatic islets from a 58 year old female patient obtained from the Diabetes Research Institute at the University of Miami School of Medicine. Islet tissue was processed to total RNA at an outside source and delivered to CuraGen for addition to panel 5I.

In the labels employed to identify tissues in the 5D and 5I panels, the following abbreviations are used:
GO Adipose = Greater Omentum Adipose
SK = Skeletal Muscle
UT = Uterus
PL = Placenta
AD = Adipose Differentiated
AM = Adipose Midway Differentiated
U = Undifferentiated Stem Cells

### Panel CNSD.01

The plates for Panel CNSD.01 include two control wells and 94 test samples comprised of cDNA isolated from postmortem human brain tissue obtained from the Harvard Brain Tissue Resource Center. Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains two brains from each of the following diagnoses: Alzheimer's disease, Parkinson's disease, Huntington's disease, Progressive Supernuclear Palsy, Depression, and "Normal controls". Within each of these brains, the following regions are represented: cingulate gyrus, temporal pole, globus palladus, substantia nigra, Brodman Area 4 (primary motor strip), Brodman Area 7 (parietal cortex), Brodman Area 9 (prefrontal cortex), and Brodman area 17 (occipital cortex). Not all brain regions are represented in all cases; e.g., Huntington's disease is characterized in part by neurodegeneration in the globus palladus, thus this region is impossible to obtain from confirmed Huntington's cases. Likewise Parkinson's disease is characterized by degeneration of the substantia nigra making this region more difficult to obtain. Normal control brains were examined for neuropathology and found to be free of any pathology consistent with neurodegeneration.

In the labels employed to identify tissues in the CNS panel, the following abbreviations are used:
PSP = Progressive supranuclear palsy
Sub Nigra = Substantia nigra
Glob Palladus= Globus palladus
Temp Pole = Temporal pole
Cing Gyr = Cingulate gyrus
BA 4 = Brodman Area 4

### Panel CNS_Neurodegeneration_V1.0

The plates for Panel CNS_Neurodegeneration_V1.0 include two control wells and 47 test samples comprised of cDNA isolated from postmortem human brain tissue obtained from the Harvard Brain Tissue Resource Center (McLean Hospital) and the Human Brain and Spinal Fluid Resource Center (VA Greater Los Angeles Healthcare System). Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains six brains from Alzheimer's disease (AD) patients, and eight brains from "Normal controls" who showed no evidence of dementia prior to death. The eight normal control brains are divided into two categories: Controls with no dementia and no Alzheimer's like pathology (Controls) and controls with no dementia but evidence of severe Alzheimer's like pathology, (specifically senile plaque load rated as level 3 on a scale of 0-3; 0 = no evidence of plaques, 3 = severe AD senile plaque load). Within each of these brains, the following regions are represented: hippocampus, temporal cortex (Brodman Area 21), parietal cortex (Broclinan area 7), and occipital cortex (Brodman area 17). These regions were chosen to encompass all levels of neurodegeneration in AD. The hippocampus is a region of early and severe neuronal loss in AD; the temporal cortex is known to show neurodegeneration in AD after the hippocampus; the parietal cortex shows moderate neuronal death in the late stages of the disease; the occipital cortex is spared in AD and therefore acts as a "control" region within AD patients. Not all brain regions are represented in all cases.

In the labels employed to identify tissues in the CNS_Neurodegeneration_V1.0 panel, the following abbreviations are used:
AD = Alzheimer's disease brain; patient was demented and showed AD-like pathology upon autopsy
Control = Control brains; patient not demented, showing no neuropathology
Control (Path) = Control brains; pateint not demented but showing sever AD-like pathology
SupTemporal Ctx = Superior Temporal Cortex
Inf Temporal Ctx = Inferior Temporal Cortex

### A. NOV1: Androgen-Regulated Short-Chain Dehydrogenase/Reductase

Expression of gene CG57413-01 was assessed using the primer-probe set Ag3230, described in Table AA. Results of the RTQ-PCR runs are shown in Tables AB, AC, AD and AE.

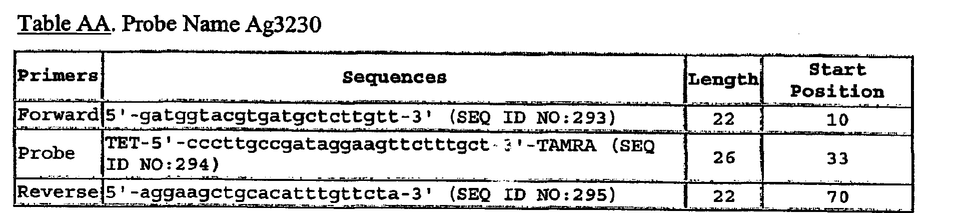

**Table AB. CNS_neurodegeneration_v1.0**

| **Tissue Name** | **Rel. Exp.(%) Ag3230, Run 206533576** | **Tissue Name** | **Rel. Exp.(%) Ag3230, Run 206533576** |
|---|---|---|---|
| AD 1 Hippo | 0.0 | Control (Path) 3 Temporal Ctx | 0.0 |
| AD 2 Hippo | 0.0 | Control (Path) 4 Temporal Ctx | 54.7 |
| AD 3 Hippo | 8.7 | AD 1 Occipital Ctx | 18.2 |
| AD 4 Hippo | 0.0 | AD 2 Occipital Ctx (Missing) | 0.0 |
| AD 5 hippo | 32.1 | AD 3 Occipital Ctx | 0.0 |
| AD 6 Hippo | 95.3 | AD 4 Occipital Ctx | 8.5 |
| Control 2 Hippo | 0.0 | AD 5 Occipital Ctx | 6.6 |
| Control 4 Hippo | 9.4 | AD 6 Occipital Ctx | 22.8 |
| Control (Path) 3 Hippo | 21.6 | Control 1 Occipital Ctx | 0.0 |
| AD 1 Temporal Ctx | 8.2 | Control 2 Occipital Ctx | 36.3 |
| AD 2 Temporal Ctx | 4.3 | Control 3 Occipital Ctx | 0.0 |
| AD 3 Temporal Ctx | 0.0 | Control 4 Occipital Ctx | 0.0 |
| AD 4 Temporal Ctx | 8.2 | Control (Path) 1 Occipital Ctx | **100.0** |
| AD 5 Inf Temporal Ctx | 32.5 | Control (Path) 2 Occipital Ctx | 15.3 |
| AD 5 SupTemporal Ctx | 15.2 | Control (Path) 3 Occipital Ctx | 0.0 |
| AD 6 Inf Temporal Ctx | 66.9 | Control (Path) 4 Occipital Ctx | 25.5 |
| AD 6 Sup Temporal Ctx | 67.8 | Control 1 Parietal Ctx | 0.0 |
| Control 1 Temporal Ctx | 0.0 | Control 2 Parietal Ctx | 5.6 |
| Control 2 Temporal Ctx | 18.0 | Control 3 Parietal Ctx | 20.4 |
| Control 3 Temporal Ctx | 8.1 | Control (Path) 1 Parietal Ctx | 37.9 |
| Control 4 Temporal Ctx | 17.7 | Control (Path) 2 Parietal Ctx | 7.5 |
| Control (Path) 1 Temporal Ctx | 14.8 | Control (Path) 3 Parietal Ctx | 0.0 |
| Control (Path) 2 Temporal Ctx | 34.2 | Control (Path) 4 Parietal Ctx | 43.8 |

**Table AC. Panel 1.3D**

| **Tissue Name** | **Rel. Exp.(%) Ag3230, Run 165524130** | **Tissue Name** | **Rel. Exp.(%) Ag3230, Run 165524130** |
|---|---|---|---|
| Liver adenocarcinoma | 0.0 | Kidney (fetal) | 0.0 |
| Pancreas | 0.0 | Renal ca. 786-0 | 9.8 |
| Pancreatic ca. CAPAN 2 | 0.0 | Renal ca. A498 | 0.0 |
| Adrenal gland | 0.0 | Renal ca. RXF 393 | 0.0 |
| Thyroid | 0.0 | Renal ca. ACHN | 0.0 |
| Salivary gland | 0.0 | Renal ca. UO-31 | 0.0 |
| Pituitary gland | 11.1 | Renal ca. TK-10 | 0.0 |
| Brain (fetal) | 0.0 | Liver | 0.0 |
| Brain (whole) | 20.0 | Liver (fetal) | 0.0 |
| Brain (amygdala) | 33.9 | Liver ca. (hepatoblast) HepG2 | 18.0 |
| Brain (cerebellum) | 0.0 | Lung | 0.0 |
| Brain (hippocampus) | 16.4 | Lung (fetal) | 0.0 |
| Brain (substantia nigra) | 14.1 | Lung ca. (small cell) LX- | 17.9 |
| Brain (thalamus) | 0.0 | Lung ca. (small cell) NCI-H69 | 0.0 |
| Cerebral Cortex | 22.2 | Lung ca. (s.cell var.) SHP-77 | 0.0 |
| Spinal cord | 0.0 | Lung ca. (large cell)NCI-H460 | 0.0 |
| glio/astro U87-MG | 0.0 | Lung ca. (non-sm. cell) | 0.0 |
| glio/astro U-118-MG | 0.0 | Lung ca. (non-s.cell) NCI-H23 | 0.0 |
| astrocytoma SW1783 | 0.0 | Lung ca. (non-s.cell) HOP-62 | 0.0 |
| neuro*; met SK-N-AS | 0.0 | Lung ca. (non-s.cl) NCI-H522 | 0.0 |
| astrocytoma SF-539 | 0.0 | Lung ca. (squam) SW 900 | 0.0 |
| astrocytoma SNB-75 | 13.8 | Lung ca. (squam.) NCI-H596 | 0.0 |
| glioma SNB-19 | 0.0 | Mammary gland | 0.0 |
| glioma U251 | **100.0** | Breast ca.* (pl.ef) MCF-7 | 12.7 |
| glioma SF-295 | 0.0 | Breast ca.* (pl.ef) MDA-MB-231 | 0.0 |
| Heart (fetal) | 0.0 | Breast ca.* (pl.ef) T47D | 0.0 |
| Heart | 0.0 | Breast ca. BT-549 | 0.0 |
| Skeletal muscle (fetal) | 0.0 | Breast ca. MDA-N | 0.0 |
| Skeletal muscle | 0.0 | Ovary | 0.0 |
| Bone marrow | 0.0 | Ovarian ca. OVCAR-3 | 0.0 |
| Thymus | 0.0 | Ovarian ca. OVCAR-4 | 0.0 |
| Spleen | 0.0 | Ovarian ca. OVCAR-5 | 0.0 |
| Lymph node | 39.5 | Ovarian ca. OVCAR-8 | 0.0 |
| Colorectal | 43.2 | Ovarian ca. IGROV-1 | 0.0 |
| Stomach | 43.5 | Ovarian ca.* (ascites) SK-OV-3 | 36.6 |
| Small intestine | 38.7 | Uterus | 57.8 |
| Colon ca. SW480 | 0.0 | Placenta | 0.0 |
| Colon ca.* SW620(SW480 met) | 0.0 | Prostate | 38.2 |
| Colon ca. HT29 | 0.0 | Prostate ca.* (bone met)PC-3 | 0.0 |
| Colon ca. HCT-116 | 0.0 | Testis | 17.9 |
| Colon ca. CaCo-2 | 13.4 | Melanoma Hs688(A).T | 0.0 |
| Colonca. tissue(OD03866) | 0.0 | Melanoma* (met) Hs688(B).T | 0.0 |
| Colon ca. HCC-2998 | 14.3 | Melanoma UACC-62 | 0.0 |
| Gastric ca.* (liver met) NCI-N87 | 79.6 | Melanoma M14 | 18.6 |
| Bladder | 26.8 | Melanoma LOX IMVI | 0.0 |
| Trachea | 0.0 | Melanoma* (met) SK-MEL-5 | 0.0 |
| Kidney | 0.0 | Adipose | 0.0 |

**Table AD. Panel 2.2**

| **Tissue Name** | **Rel. Exp.(%) Ag3230, Run 174442812** | **Tissue Name** | **Rel. Exp.(%) Ag3230, Run 174442812** |
|---|---|---|---|
| Normal Colon | 18.2 | Kidney Margin (OD04348) | 38.4 |
| Colon cancer (OD06064) | 0.0 | Kidney malignant cancer (OD06204B) | 23.2 |
| Colon Margin (OD06064) | 0.0 | Kidney normal adjacent tissue (OD06204E) | 0.0 |
| Colon cancer (OD06159) | 0.0 | Kidney Cancer (OD04450-01) | 0.0 |
| Colon Margin (OD06159) | 0.0 | Kidney Margin (OD04450- | 0.0 |
| Colon cancer (OD06297-04) | 0.0 | Kidney Cancer 8120613 | 22.7 |
| Colon Margin (OD06297-05) | 17.9 | Kidney Margin 8120614 | 0.0 |
| CC Gr.2 ascend colon (ODO3921) | 0.0 | Kidney Cancer 9010320 | 0.0 |
| CC Margin (ODO3921) | 9.0 | Kidney Margin 9010321 | 0.0 |
| Colon cancer metastasis (OD06104) | 0.0 | Kidney Cancer 8120607 | 0.0 |
| Lung Margin (OD06104) | 0.0 | Kidney Margin 8120608 | 20.3 |
| Colon mets to lung (OD04451-01) | 41.5 | Normal Uterus | 12.6 |
| Lung Margin (OD04451-02) | 19.9 | Uterine Cancer 064011 | 0.0 |
| Normal Prostate | 0.0 | Normal Thyroid | 0.0 |
| Prostate Cancer (OD04410) | 12.6 | Thyroid Cancer 064010 | 0.0 |
| Prostate Margin (OD04410) | 20.0 | Thyroid Cancer A302152 | 0.0 |
| Normal Ovary | 0.0 | Thyroid Margin A302153 | 0.0 |
| Ovarian cancer (OD06283-03) | 0.0 | Normal Breast | 44.4 |
| Ovarian Margin (OD06283-07) | 0.0 | Breast Cancer (OD04566) | 0.0 |
| Ovarian Cancer 064008 | 41.8 | Breast Cancer 1024 | 18.9 |
| Ovarian cancer (OD06145) | 0.0 | Breast Cancer (OD04590-01) | 0.0 |
| Ovarian Margin (OD06145) | 4.7 | Breast Cancer Mets (OD04590-03) | 0.0 |
| Ovarian cancer (OD06455-03) | 0.0 | Breast Cancer Metastasis (OD04655-05) | 38.2 |
| Ovarian Margin (OD06455-07) | 0.0 | Breast Cancer 064006 | 15.7 |
| Normal Lung | 32.1 | Breast Cancer 9100266 | 0.0 |
| Invasive poor diff. lung adeno (ODO4945-01 | 0.0 | Breast Margin 9100265 | 0.0 |
| Lung Margin (OD04945-03) | 0.0 | Breast Cancer A209073 | 0.0 |
| Lung Malignant Cancer | 0.0 | Breast Margin A2090734 | 20.4 |
| Lung Margin (OD03126) | 0.0 | Breast cancer (OD06083) | 39.0 |
| Lung Cancer (OD05014A) | 0.0 | Breast cancer node metastasis (OD06083) | 45.1 |
| Lung Margin (OD05014B) | **100.0** | Normal Liver | 6.0 |
| Lung cancer (OD06081) | 0.0 | Liver Cancer 1026 | 0.0 |
| Lung Margin (OD06081) | 0.0 Liver | Cancer 1025 | 27.9 |
| Lung Cancer (OD04237-01) | 0.0 | Liver Cancer 6004-T | 20.0 |
| Lung Margin (OD04237-02) | 0.0 | Liver Tissue 6004-N | 0.0 |
| Ocular Melanoma Metastasis | 12.1 | Liver Cancer 6005-T | 0.0 |
| Ocular Melanoma Margin (Liver) | 0.0 | Liver Tissue 6005-N | 0.0 |
| Melanoma Metastasis | 18.8 | Liver Cancer 064003 | 19.2 |
| Melanoma Margin (Lung) | 0.0 | Normal Bladder | 0.0 |
| Normal Kidney | 20.4 | Bladder Cancer 1023 | 0.0 |
| Kidney Ca, Nuclear grade 2 (OD04338) | 82.4 | Bladder Cancer A302173 | 0.0 |
| Kidney Margin (OD04338) | 9.0 | Normal Stomach | 44.4 |
| Kidney Ca Nuclear grade 1/2 (OD04339) | 53.2 | Gastric Cancer 9060397 | 0.0 |
| Kidney Margin (OD04339) | 18.3 | Stomach Margin 9060396 | 18.7 |
| Kidney Ca, Clear cell type (OD04340) | 38.2 | Gastric Cancer 9060395 Gastric Cancer 9060395 | 33.0 |
| Kidney Margin (OD04340) | 0.0 | Stomach Margin 9060394 | 21.0 |
| Kidney Ca, Nuclear grade 3 (OD04348) | 0.0 | Gastric Cancer 064005 | 0.0 |

**Table AE. Panel 4D**

| **Tissue Name** | **Rel. Exp.(%) Ag3230, Run 164532020** | **Tissue Name** | **Rel.Exp.(%) Ag3230, Run 164532020** |
|---|---|---|---|
| Secondary Th1 act | 0.0 | HUVEC IL-1beta | 7.9 |
| Secondary Th2 act | 0.0 | HUVEC IFN gamma | 9.2 |
| Secondary Tr1 act | 11.1 | HUVEC TNF alpha + IFN gamma | 0.0 |
| Secondary Th1 rest | 0.0 | HUVEC TNF alpha + IL4 | 0.0 |
| Secondary Th2 rest | 13.9 | HUVEC IL-11 | 4.0 |
| Secondary Tr1 rest | 12.2 | Long Microvascular EC none | 15.2 |
| Primary Th1 act | 7.1 | Lung Microvascular EC TNFalpha + IL-1beta | 15.2 |
| Primary Th2 act | 17.7 | Microvascular Dermal EC none | 9.2 |
| Primary Tr1 act | 0.0 | Microsvasular Dermal EC TNFalpha + IL-1beta | 7.2 |
| Primary Th1 rest | 57.0 | Bronchial epithelium TNFalpha + IL1beta | 15.9 |
| Primary Th2 rest | 11.5 | Small airway epithelium none | 22.4 |
| Primary Tr1 rest | 7.9 | Small airway epithelium TNFalpha + IL-1beta | **100.0** |
| CD45RA CD4 lymphocyte act | 1.9 | Coronery artery SMC rest | 0.0 |
| CD45RO CD4 lymphocyte act | 25.2 | Coronery artery SMC TNFalpha + IL-1beta | 0.0 |
| CD8 lymphocyte act | 7.1 | Astrocytes rest | 0.0 |
| Secondary CD8 lymphocyte rest | 53.6 | Astrocytes TNFalpha + IL-1beta | 0.0 |
| Secondary CD8 lymphocyte act | 0.0 | KU-812 (Basophil) rest | 0.0 |
| CD4 lymphocyte none | 41.8 | KU-812 (Basophil) PMA/ionomycin | 58.2 |
| 2ry Th1/Th2/Tr1_anti-CD95 CH11 | 7.0 | CCD1106 (Keratinocytes) none | 0.0 |
| LAK cells rest | 24.8 | CCD1106 (Keratinocytes) TNFalpha + IL-1beta | 0.0 |
| LAK cells IL-2 | 32.5 | Liver cirrhosis | 51.1 |
| LAK cells IL-2+IL-12 | 22.2 | Lupus kidney | 0.0 |
| LAK cells IL-2+IFN gamma | 27.4 | NCI-H292 none | 0.0 |
| LAK cells IL-2+IL-18 | 15.3 | NCl-H292 IL-4 | 31.9 |
| LAK cells PMA/ionomycin | 0.0 | NCI-H292 IL-9 | 12.3 |
| NK Cells IL-2 rest | 12.8 | NCI-H292 IL-13 | 0.0 |
| Two Way MLR 3 day | 28.9 | NCI-H292 IFN gamma | 0.0 |
| Two Way MLR 5 day | 0.0 | HPAEC none | 0.0 |
| Two Way MLR 7 day | 27.5 | HPAEC TNF alpha + IL-1 beta | 3.2 |
| PBMC rest | 9.4 | Lung fibroblast none | 20.9 |
| PBMCPWM | 21.8 | Lung fibroblast TNF alpha + IL-1 beta | 14.9 |
| PBMC PHA-L | 7.9 | Lung fibroblast IL-4 | 13.2 |
| Ramos(B cell) none | 0.0 | Lung fibroblast IL-9 | 0.0 |
| Ramos (B cell) ionomycin | 0.0 | Lung fibroblast IL-13 | 0.0 |
| B lymphocytes PWM | 25.9 | Lung fibroblast IFN gamma | 0.0 |
| B lymphocytes CD40L and IL-4 | 30.8 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 59.5 | Dermal fibroblast IFN gamma | 0.0 |
| Dendritic cells LPS | 0.0 | Dermal fibroblast IL-4 | 7.7 |
| Dendritic cells anti-CD40 | 54.3 | IBD Colitis 2 | 0.0 |
| Monocytes rest | 49.7 | IBD Crohn's | 7.1 |
| Monocytes LPS | 8.7 | Colon | 14.4 |
| Macrophages rest | 21.6 | Lung | 16.2 |
| Macrophages LPS | 8.0 | Thymus | 92.7 |
| HUVEC none | 0.0 | Kidney | 0.0 |
| HUVEC starved | 0.0 | | |

**CNS_neurodegeneration_v1.0 Summary:** Ag3230 This panel does not show differential expression of the CG57413-01 gene in Alzheimer's disease. However, this expression profile confirms the presence of this gene in the brain. This gene encodes a homolog of an androgen regulated short chain dehydrogenase. Members of this family are known to function in the processing of hormones in the brain. Brain hormone regulation mediates numerous clinically significant conditions, including psychiatric disorders such as anxiety, overeating and memory disorders. Therefore, agents that modulate the activity of this gene product have potential utility in the treatment of these disorders. In addition, steroid treatment is used in a number of clinical conditions including Alzheimer's disease (estrogen), menopause associated symptoms (estrogen), multiple sclerosis (glucocorticoids), and spinal cord injury (methylprednisolone). Treatment with an antagonist of this gene product, or reduction of the levels of this gene product could slow steroid degredation and lower the necessary amount given for therapeutic effect, thus reducing peripheral side effects (Biswas and Russell, J Biol Chem 1997 Jun 20;272(25):15959-66; Matsumoto et al., Spine 2001 Feb 15;26(4):426-30; Holinka, Ann N Y Acad Sci 2001 Sep;943:89-108; Burkman et al., Am J Obstet Gynecol 2001 Aug;185(2 Suppl):S13-23; Gaillard et al., Neuroreport 2001 Jul 20;12(10):2189-93; Penning et al., Biochem J 2000 Oct 1;351(Pt 1):67-77).

**Panel 1.3D Summary:** Ag3230 Expression of the CG57413-01 gene is restricted to a sample derived from a brain cancer cell line (CT=34.8). Thus, expression of this gene could be used to differentiate between this sample and other samples on this panel and as a marker to detect the presence of brain cancer. Furthermore, therapeutic modulation of the expression or function of this gene may be effective in the treatment of brain cancer.

**Panel 2.2 Summary:** Ag3230 Expression of the CG57413-01 gene is restricted to a sample derived from a normal lung tissue (CT=34.95). Thus, expression of this gene could be used to differentiate between this sample and other samples on this panel and as a marker of normal lung tissue.

**Panel 4D Summary:** Ag3230 Highest expression of the CG57413-01 gene is seen in Small airway epithelium stimulated with TNFalpha + IL-1beta (CT=33.4). Significant expression is also seen in the basophil cell line KU-812 stimulated with PMA/ionomycin. This cell line constitutes a reasonable model for the inflammatory cells that take part in various inflammatory lung diseases. This expression profile in combination with the exclusive expression in normal lung seen in Panel 2D suggests that the protein encoded by this gene may be involved in the proliferation or activation of airway epithelium. Therefore, therapuetics designed with the protein encoded by this transcript could be important in the treatment of pathological and inflammatory lung disorders, including emphysema, allergies, asthma and COPD.

**Panel 5 Islet Summary:** Ag3230 Expression of the CG57413-01 gene is low/undetectable in all samples on this panel (CTs>35).

### B. NOV2a and NOV2b: Aryl-Acylamidase

Expression of gene CG57391-01 and variant CG57391-02 was assessed using the primer-probe sets Ag3053 and Ag3224, described in Tables BA and BB. Results of the RTQ-PCR runs are shown in Table BC.

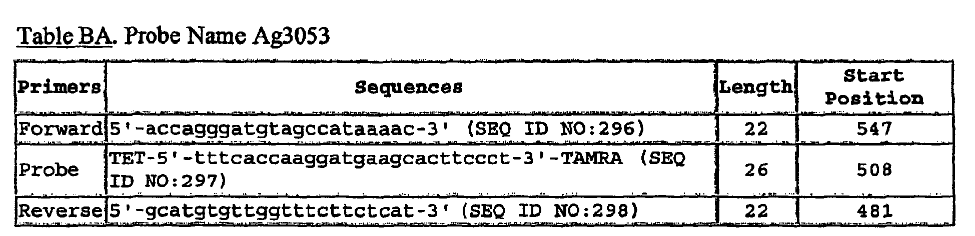

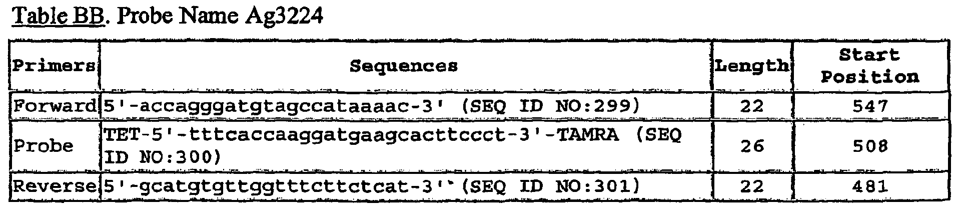

**Table BC. Panel 1.3D**

| **Tissue Name** | **Rel. Exp.(%) Ag3053, Run 167985386** | **Rel. Exp.(%) Ag3224, Run 165524086** | **Tissue Name** | **Rel. Exp.(%) Ag3053,Run 167985386** | **Rel. Exp.(%) Ag3224,Run 165524086** |
|---|---|---|---|---|---|
| Liver adenocarcinoma | 0.0 | 0.0 | Kidney (fetal) | 24.8 | 0.0 |
| Pancreas | 0.0 | 0.0 | Renal ca. 786-0 | 0.0 | 0.0 |
| Pancreatic ca. CAPAN 2 | 0.0 | 0.0 | Renal ca. A498 | 22.4 | 6.1 |
| Adrenal gland | 0.0 | 0.0 | Renal ca. RXF 393 | 0.0 | 0.0 |
| Thyroid | 11.3 | 0.0 | Renal ca. ACHN | 0.0 | 0.0 |
| Salivary gland | 0.0 | 0.0 | Renal ca. UO-31 | 0.0 | 0.0 |
| Pituitary gland | 66.0 | 12.4 | Renal ca. TK-10 | 0.0 | 0.0 |
| Brain (fetal) | 0.0 | 0.0 | Liver | 0.0 | 0.0 |
| Brain (whole) | 0.0 | 0.0 | Liver (fetal) | 0.0 | 0.0 |
| Brain (amygdala) | 0.0 | 0.0 | Liver ca. (hepatoblast) HepG2 | 0.0 | 0.0 |
| Brain (cerebellum) | 0.0 | 0.0 | Lung | 0.0 | 0.0 |
| Brain (hippocampus) | 0.0 | 0.0 | Lung (fetal) | 27.5 | 0.0 |
| Brain (substantia nigra) | 0.0 | 0.0 | Lung ca. (small cell) LX-1 | 9.5 | 0.0 |
| Brain (thalamus) | 0.0 | 0.0 | Lung ca. (small cell) NCI-H69 | 0.0 | 0.0 |
| Cerebral Cortex | 0.0 | 0.0 | Lung ca. (s.cell var.) SHP-77 | 0.0 | 0.0 |
| Spinal cord | 0.0 | 4.8 | Lung ca. (large cell)NCI-H460 | 0.0 | 0.0 |
| glio/astro U87-MG | 0.0 | 0.0 | Lung ca. (non-sm. cell) A549 | **100.0** | 5.8 |
| glio/astro U-118-MG | 0.0 | 0.0 | Lung ca. (non-s.cell) NCI-H23 | 9.3 | 0.0 |
| astrocytoma SW 1783 | 0.0 | 0.0 | Lung ca. (non-s.cell) HOP-62 | 17.0 | 0.0 |
| neuro*; met SK-N-AS | 0.0 | 0.0 | Lung ca. (non-s.cl) NCI-H522 | 10.7 | 0.0 |
| astrocytoma SF-539 | 0.0 | 4.6 | Lung ca. (squam.) SW 900 | 36.3 | 21.9 |
| astrocytoma SNB-75 | 57.0 | 0.0 | Lung ca. (squam.) NCI-H596 | 0.0 | 0.0 |
| glioma SNB-19 | 0.0 | 0.0 | Mammary gland | 0.0 | 0.0 |
| glioma U251 | 0.0 | 11.8 | Breast ca.* (pl.ef) MCF-7 | 0.0 | 0.0 |
| glioma SF-295 | 0.0 | 0.0 | Breast ca.* (pl.ef) MDA-MB-231 | 0.0 | 0.0 |
| Heart (fetal) | 0.0 | 0.0 | Breast ca.* (pl.ef) T47D | 0.0 | 0.0 |
| Heart | 0.0 | 0.0 549 | Breast ca. BT- | 0.0 | 4.0 |
| Skeletal muscle (fetal) | 0.0 | 0.0 | Breast ca. MDA-N | 0.0 | 0.0 |
| Skeletal muscle | 0.0 | 0.0 | Ovary | 0.0 | 0.0 |
| Bone marrow | 0.0 | 0.0 | Ovarian ca. OVCAR-3 | 12.8 | 10.4 |
| Thymus | 7.7 | 0.0 | Ovarian ca. OVCAR-4 | 0.0 | 0.0 |
| Spleen | 0.0 | 0.0 | Ovarian ca. OVCAR-5 | 20.4 | 0.0 |
| Lymph node | 8.4 | 13.0 | Ovarian ca. OVCAR-8 | 0.0 | 0.0 |
| Colorectal | 0.0 | 0.0 | Ovarian ca. IGROV-1 | 0.0 | 0.0 |
| Stomach | 0.0 | 6.8 | Ovarian ca.* (ascites)SK-OV-3 | 0.0 | 0.0 |
| Small intestine | 42.9 | 100.0 | Uterus | 0.0 | 10.7 |
| Colon ca. SW480 | 0.0 | 0.0 | Placenta | 9.7 | 18.2 |
| Colon ca.* SW620(SW480 met) | 0.0 | 0.0 | Prostate | 0.0 | 0.0 |
| Colon ca. HT29 | 7.7 | 0.0 | Prostate ca.* (bone met)PC-3 | 0.0 | 0.0 |
| Colon ca. HCT-116 | 0.0 | 0.0 | Testis | 9.5 | 4.7 |
| Colon ca. CaCo-2 | 7.9 | 0.0 | Melanoma Hs688(A)T | 0.0 | 0.0 |
| Colon ca. tissue(OD03866) | 5.9 | 0.0 | Melanoma* (met) Hs688(B).T | 0.0 | 0.0 |
| Colon ca. HCC-2998 | 0.0 | 5.9 | Melanoma UACC-62 | 0.0 | 0.0 |
| Gastric ca.* (liver met) NCI-N87 | 11.4 | 4.9 | Melanoma M14 | 0.0 | 0.0 |
| Bladder | 0.0 | 0.0 | Melanoma LOX IMVI | 0.0 | 0.0 |
| Trachea | 24.8 | 9.6 Melanoma* | (met) SK-MEL-5 | 0.0 | 0.0 |
| Kidney | 0.0 | 0.0 | Adipose | 57.8 | 20.9 |

**Panel 1.3D Summary:** Ag3053/Ag3224 Two experiments with the same probe and primer set show expression of the CG57391-01 gene restricted to samples derived from the small intestine and a lung cancer cell line (CTs=33-35). Thus, expression of this gene could be used to differentiate between these samples and other samples on this panel and as a marker for intestinal tissue and lung cancer.

**Panel 4D Summary:** Ag3053 Expression of the CG57391-01 gene is low/undetectable in all samples on this panel (CTs>35). (Data not shown.)

### C. NOV3a: Insulysin

Expression of gene CG57433-01 was assessed using the primer-probe set Ag3225, described in Table CA. Results of the RTQ-PCR runs are shown in Tables CB, CC and CD.

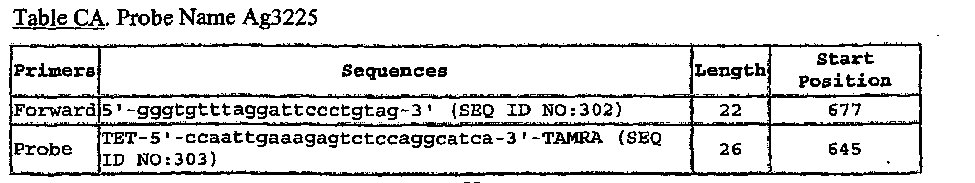

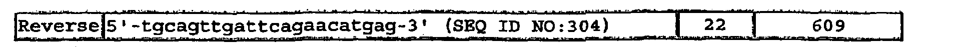

**Table CB. Panel 1.3D**

| **Tissue Name** | **Rel. Exp.(%) Ag3225, Run 165524115** | **Tissue Name** | **Rel. Exp.(%) Ag3225, Run 165524115** |
|---|---|---|---|
| Liver adenocarcinoma | 29.9 | Kidney (fetal) | 6.7 |
| Pancreas | 7.2 | Renal ca. 786-0 | 27.2 |
| Pancreatic ca. CAPAN 2 | 31.0 | Renal ca. A498 | 23.5 |
| Adrenal gland | 8.7 | Renal ca. RXF 393 | 23.3 |
| Thyroid | 10.2 | Renal ca. ACHN | 20.9 |
| Salivary gland | 7.1 | Renal ca. UO-31 | 28.5 |
| Pituitary gland | 10.3 | Renal ca. TK-10 | 7.1 |
| Brain (fetal) | 12.2 | Liver | 3.3 |
| Brain (whole) | 24.1 | Liver (fetal) | 22.2 |
| Brain (amygdala) | 7.7 | Liver ca. (hepatoblast) HepG2 | 47.0 |
| Brain (cerebellum) | 21.5 | Lung | 8.2 |
| Brain (hippocampus) | 11.0 | Lung (fetal) | 2.7 |
| Brain (substantia nigra) | 7.2 | Lung ca. (small cell) LX-1 | 37.1 |
| Brain (thalamus) | 20.6 | Lung ca. (small cell) NCI-H69 | 5.0 |
| Cerebral Cortex | 13.5 | Lung ca. (s.cell var.) SHP-77 | 16.6 |
| Spinal cord | 14.3 | Lung ca. (large cell)NCI- H460 | **100.0** |
| glio/astro U87-MG | 30.1 | Lung ca. (non-sm. cell) A549 | 35.6 |
| glio/astro U-118-MG | 50.3 | Lung ca. (non-s.cell) NCI-H23 | 23.3 |
| astrocytoma SW1783 | 31.0 | Lung ca. (non-s.cell) HOP-62 | 30.1 |
| neuro*; met SK-N-AS | 31.2 | Lung ca. (non-s.cl) NCI-H522 | 13.1 |
| astrocytoma SF-539 | 25.5 | Lung ca. (squam.) SW | 19.9 |
| astrocytoma SNB-75 | 34.6 | Lung ca. (squam.) NCI-H596 | 14.2 |
| glioma SNB-19 | 28.7 | Mammary gland | 12.7 |
| glioma U251 | 92.7 | Breast ca.* (pl.ef) MCF-7 | 31.0 |
| glioma SF-295 | 24.7 | Breast ca.* (pl.ef) MDA-MB-231 | 62.9 |
| Heart (fetal) | 5.1 | Breast ca.* (pl.ef) T47D | 13.9 |
| Heart | 14.2 | Breast ca. BT-549 | 61.1 |
| Skeletal muscle (fetal) | 2.2 | Breast ca. MDA-N | 5.7 |
| Skeletal muscle | 90.1 | Ovary | 2.8 |
| Bone marrow | 7.5 | Ovarian ca. OVCAR-3 | 27.2 |
| Thymus | 7.0 | Ovarian ca. OVCAR-4 | 23.8 |
| Spleen | 12.2 | Ovarian ca. OVCAR-5 | 18.0 |
| Lymph node | 20.3 | Ovarian ca. OVCAR-8 | 8.6 |
| Colorectal | 7.5 | Ovarian ca. IGROV-1 | 10.2 |
| Stomach | 17.6 | Ovarian ca.* (ascites) SK-OV-3 | 20.0 |
| Small intestine | 17.4 | Uterus | 17.8 |
| Colon ca. SW480 | 18.9 | Placenta | 7.1 |
| Colon ca.* SW620(SW480 met) | 25.7 | Prostate | 7.9 |
| Colon ca. HT29 | 9.7 | Prostate ca.* (bone met)PC-3 | 16.6 |
| Colon ca. HCT-116 | 30.6 | Testis | 10.2 |
| Colon ca. CaCo-2 | 29.9 | Melanoma Hs688(A).T | 9.6 |
| Colon ca. tissue(ODO3866) | 24.5 | Melanoma* (met) Hs688(B).T | 10.9 |
| Colon ca. HCC-2998 | 14.3 | Melanoma UACC-62 | 8.0 |
| Gastric ca.* (liver met) NCI-N87 | 36.1 | Melanoma M14 | 47.0 |
| Bladder | 17.4 | Melanoma LOX IMVI | 3.4 |
| Trachea | 7.5 | Melanoma* (met) SK-MEL-5 | 9.2 |
| Kidney | 7.0 | Adipose | 9.2 |

**Table CC. Panel 5 Islet**

| **Tissue Name** | **Rel. Exp.(%) Ag3225, Run 223846465** | **Tissue Name** | **Rel.Exp.(%) Ag3225, Run 223846465** |
|---|---|---|---|
| 97457_Patient-02go_adipose | 12.2 | 94709_Donor 2 AM-A_adipose | 32.5 |
| 97476_Patient-07sk_skeletal muscle | 13.6 | 94710_Donor 2 AM-B_adipose | 16.5 |
| 97477_Patient-07ut_uterus | 10.5 | 94711_Donor 2 AM-C_adipose | 15.8 |
| 97479_Patient-07p1_placenta | 15.5 | 94712_Donor 2 AD-A_adipose | 37.9 |
| 99167_Bayer Patient 1 | 9.7 | 94713_Donor 2 AD-B_adipose | 37.4 |
| 97482_Patient-08ut_uterus | 7.8 | 94714_Donor 2 AD-C_adipose | 41.5 |
| 97483_Patient-08pl_placenta | 13.3 | 94742_Donor 3 U-A_Mesenchymal Stem Cells | 5.7 |
| 97486_Patient-09sk_skeletal muscle | 4.0 | 94743_Donor 3 U-B_Mesenchymal Stem Cells | 25.7 |
| 97487_Patient-09ut_uterus | 18.4 | 94730_Donor 3 AM-A_adipose | 42.0 |
| 97488_Patient-09pl_placenta | 6.9 | 94731_Donor 3 AM-B_adipose | 16.4 |
| 97492_Patient-10ut_uterus | 28.5 | 94732_Donor 3 AM-C_adipose | 26.4 |
| 97493_Patient-10pl_placenta | 23.0 | 94733_Donor 3 AD-A_adipose | 59.5 |
| 97495_Patient-11go_adipose | 8.9 | 94734_Donor 3 AD-B_adipose | 11.7 |
| 97496_Patient-11sk_skeletal muscle | 20.7 | 94735_Donor 3 AD-C_adipose | 35.4 |
| 97497_Patient-11ut_uterus | 23.5 | 77138_Liver_HepG2untreated | **100.0** |
| 97498_Patient-11p1_placenta | 17.0 | 73556_Heart_Cardiac stromal cells (primary) | 10.8 |
| 97500_Patient-12go_adipose | 18.3 | 81735_Small Intestine | 26.8 |
| 97501_Patient-12sk_skeletal muscle | 49.3 | 72409_Kidney_Proximal Convoluted Tubule | 12.1 |
| 97502_Patient-12ut_uterus | 29.7 | 82685_Small intestine_Duodenum | 10.4 |
| 97503_Patient-12pl_placenta | 12.9 | 90650_Adrenal_Adrenocortical adenoma | 9.8 |
| 94721_Donor 2 U - A_Mesenchymal Stem Cells | 14.3 | 72410_Kidney_HRCE | 38.4 |
| 94722 Donor 2 U - B_Mesendiymal Stem Cells | 12.3 | 72411_Kidney_HRE | 32.3 |
| 94723_Donor 2 U - C_Mesenchymal Stem Cells | 23.5 | 73139_Uterus_Uterine smooth muscle cells | 11.0 |

**Table CD. Panel 5D**

| **Tissue Name** | **Rel. Exp.(%) Ag3225, Run 169271478** | **Tissue Name** | **Rel. Exp.(%) Ag3225, Run 169271478** |
|---|---|---|---|
| 97457 Patient-02go_adipose | 9.7 | 94709_Donor 2 AM - A_adipose | 26.2 |
| 97476_Patient-07sk_skeletal muscle | 7.1 | 94710_Donor 2 AM - B_adipose | 19.2 |
| 97477_Patient-07ut_uterus | 9.7 | 94711_Donor 2 AM - C_adipose | 18.4 |
| 97478 Patient-07pl_placenta | 13.9 | 94712 Donor 2 AD - A_adipose | 35.1 |
| 97481_Patient-08sk_skeletal muscle | 7.7 | 94713_Donor 2 AD - B_adipose | 37.9 |
| 97482_Patient-08ut_uterus | 10.2 | 94714_Donor 2 AD - C_adipose | 38.4 |
| 97483_Patient-08pl_placenta | 7.9 | 94742_Donor 3U-A_Mesenchymal Stem Cells | 18.9 |
| 97486_Patient-09sk_skeletal muscle | 7.7 | 94743_Donor 3 U - B_Mesenchymal Stem Cells | 25.7 |
| 97487_Patient-09ut_uterus | 12.4 | 94730_Doaor 3 AM - A_adipose | 40.1 |
| 97488_Patient-09p1_placenta | 8.4 | 94731_Donor 3 AM-B_adipose | 20.7 |
| 97492_Patient-10ut_uterus | 16.0 | 94732_Donor 3 AM - C_adipose | 30.1 |
| 97493_Patient-10pl_placenta | 24.5 | 94733_Donor 3 AD - A_adipose | 43.2 |
| 97495_Patient-11go_adipose | 6.7 | 94734_Donor 3 AD-B adipose | 27.2 |
| 97496_Patient-1 1sk_skeletal muscle | 22.5 | 94735 Donor 3 AD - C_adipose | 37.9 |
| 97497_Patient-11ut_uterus | 22.4 | 77138_Liver_HepG2untreated | 100.0 |
| 97498_Patient-11pl_placenta | 8.2 | 73556_Heart_Cardiac stromal cells (primary) | 9.7 |
| 97500_Patient-12go_adipose | 9.3 | 81735_Small Intestine | 16.5 |
| 97501_Patient-12sk_skeletal muscle | 43.2 | 72409_Kidney_Proximal Convoluted Tubule | 10.2 |
| 97502_Patient-12ut_uterus | 25.2 | 82685_Small intestine_Duodenum | 13.6 |
| 97503_Patient-12pl_placenta | 9.6 | 90650_Adrenal_Adrenocortical adenoma | 8.5 |
| 94721_Donor 2 U - A_Mesenchymal Stem Cells | 15.6 | 72410_Kidney_HRCE | 26.8 |
| 94722_Donor 2 U - B_Mesenchymal Stem Cells | 12.6 | 72411_Kidney_HRE | 27.2 |
| 94723_Donor 2 U - C_Mesenchymal Stem Cells | 13.3 | 73139_Uterus_Uterine smooth muscle cells | 11.0 |

**Panel 1.3D Summary:** 3225 The expression of the CG57433-01 gene appears to be highest in a sample derived from a lung cancer cell line (H460 (CT=29.5). In addition, there appears to be substantial expression in other samples derived from lung cancer cell lines, brain cancer cell lines, kidney cancer cell lines, colon cancer cell lines and breast cancer cell lines. Of note, is the apparent overexpression of this gene in skeletal muscle (CT=30) in comparison to fetal skeletal muscle (CT=35). Thus, the expression of this gene could be used to distinguish H460 cells from other samples in this panel. In addition, the expression of this gene could be used to distinguish adult skeletal muscle and fetal skeletal muscle. Moreover, therapeutic modulation of this gene, through the use of small molecule drugs, protein therapeutics, or antibodies could be of benefit in the treatment of lung, brain, kidney, colon or breast cancer.

Among metabolic tissues, this gene has low levels of expression in pancreas, adrenal, thyroid, pituitary, heart, liver and adipose. Inhibition of this insulin-degrading enzyme may increase insulin signalling and action and thus be a treatment for the prevention and treatment of Type 2 diabetes. This gene product may also be a small molecule target for the treatment of metabolic and endocrine disease, including thyroidopathies, Type 1 diabetes, and obesity.

This gene is also expressed throughout the brain at low levels, indicating a role in CNS processes. Insulysin (also known as Insulin-degrading enyzme) has been shown to rapidly remove the beta-amyloid precursor protein intracellular domain (AICD) and eliminate proteins with amyloidogenic potential, which are thought to mediate of the pathological features of Alzheimer's disease. Potentiation of this process is a likely means of addressing Alzneimer's disease pathology. Therefore, agents that increase the physiological activity of this gene product are likely to have utility as clinically therapeutic treatments for Alzheimer's disease, and potentially other neurodegenerative diseases involving protein aggregation (Edbauer et al., Biol Chem 2002 Jan 23; [epub ahead of print]; Kurochkin, Trends Biochem Sci 2001 Jul;26(7):421-5).

**Panel 5 Islet and 5D Summary:** Ag3225 The CG57433-01 gene is expressed at low levels in islets of Langerhans and mesenchymal stem cells that differentiate in vitro into adipocytes, chondrocytes and osteocytes. Therefore, therapeutic modulation of this enzyme may be a treatment for diseases of bone and cartilage as well as Types 1 and 2 diabetes.

### D. NOV5: Membrane protein

Expression of gene CG57360-01 was assessed using the primer-probe set Ag3215, described in Table DA. Results of the RTQ-PCR runs are shown in Tables DB, DC, DD and DE.

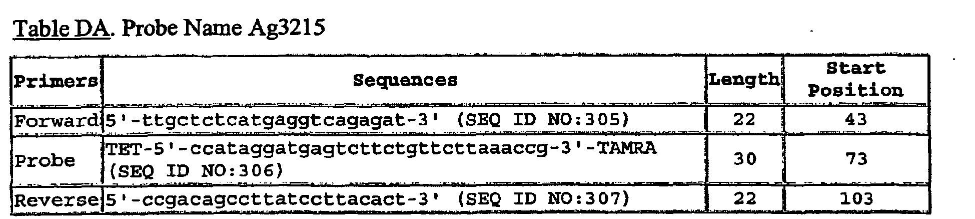

**Table DB. CNS_neurodegeneration_v1.0**

| **Tissue Name** | **Rel. Ezp.(%) Ag3215, Run 209861782** | **Tissue Name** | **Rel. Exp.(%) Ag3215, Run 209861782** |
|---|---|---|---|
| AD 1 Hippo | 5.3 | Control(Path)3 Temporal Ctx | 3.7 |
| AD 2 Hippo | 21.8 | Control (Path) 4 Temporal Ctx | 37.6 |
| AD 3 Hippo | 0.0 | AD 1 Occipital Ctx | 8.1 |
| AD 4 Hippo | 15.5 | AD 2 Occipital Ctx (Missing) | 0.0 |
| AD 5 hippo | 42.0 | AD 3 Occipital Ctx | 2.0 |
| AD 6 Hippo | 51.8 | AD 4 Occipital Ctx | 20.3 |
| Control 2 Hippo | 25.2 | AD 5 Occipital Ctx | 4.1 |
| Control 4 Hippo | 11.8 | AD 6 Occipital Ctx | 33.4 |
| Control (Path) 3 Hippo | 1.3 | Control 1 Occipital Ctx | 5.8 |
| AD 1 Temporal Ctx | 7.5 | Control 2 Occipital Ctx | 62.9 |
| AD 2 Temporal Ctx | 33.0 | Control 3 Occipital Ctx | 14.2 |
| AD 3 Temporal Ctx | 2.7 | Control 4 Occipital Ctx | 6.9 |
| AD 4 Temporal Ctx | 27.2 | Control (Path) 1 Occipital Ctx | **100.0** |
| AD 5 Inf Temporal Ctx | 41.8 | Control (Path) 2 Occipital Ctx | 22.8 |
| AD 5 SupTemporal Ctx | 36.3 | Control (Path) 3 Occipital Ctx | 3.3 |
| AD 6 Inf Temporal Ctx | 56.6 | Control (Path) 4 Occipital Ctx | 20.3 |
| AD 6 Sup Temporal Ctx | 73.2 | Control 1 Parietal Ctx | 4.2 |
| Control 1 Temporal Ctx | 1.5 | Control 2 Parietal Ctx | 25.9 |
| Control 2 Temporal Ctx | 8.7 | Control 3 Parietal Ctx | 20.6 |
| Control 3 Temporal Ctx | 19.5 | Control (Path) 1 Parietal Ctx | 60.7 |
| Control 4 Temporal Ctx | 14.5 | Control (Path) 2 Parietal Ctx | 17.3 |
| Control (Path) 1 Temporal Ctx | 59.5 | Control (Path) 3 Parietal Ctx | 1.5 |
| Control (Path) 2 Temporal Ctx | 54.3 | Control (Path) 4 Parietal Ctx | 33.0 |

**Table DC. Panel 1.3D**

| **Tissue Name** | **Rel. Exp.(%) Ag3215, Run 168012859** | **Tissue Name** | **Rel. Exp.(%) Ag3215, Run 168012859** |
|---|---|---|---|
| Liver adenocarcinoma | 64.6 | Kidney (fetal) | 80.7 |
| Pancreas | 22.4 | Renal ca. 786-0 | 0.0 |
| Pancreatic ca. CAPAN 2 | 79.6 | Renal ca. A498 | 4.3 |
| Adrenal gland | 24.0 | Renal ca. RXF 393 | 20.7 |
| Thyroid | 17.3 | Renal ca. ACHN | 15.8 |
| Salivary gland | 1.6 | Renal ca. UO-31 | 1.6 |
| Pituitary gland | 26.1 | Renal ca. TK-10 | 4.3 |
| Brain (fetal) | 86.5 | Liver | 26.1 |
| Brain(whole) | 55.5 | Liver (fetal) | 20.7 |
| Brain (amygdala) | 69.7 | Liver ca. (hepatoblast) HepG2 | 18.9 |
| Brain (cerebellum) | 37.9 | Lung | 15.1 |
| Brain (hippocampus) | 20.7 | Lung (fetal) | 38.7 |
| Brain (substantia nigra) | 46.3 | Lung ca. (small cell) LX-1 | 6.0 |
| Brain (thalamus) | 23.8 | Lung ca. (small cell) NCI-H69 | 5.5 |
| Cerebral Cortex | 17.6 | Lung ca. (s.cell var.) SHP-77 | 35.4 |
| Spinal cord | 19.3 | Lung ca. (large cell)NCI-H460 | 4.5 |
| glio/astro U87-MG | 20.0 | Lung ca. (non-sm. cell) A549 | 7.6 |
| glio/astro U-118-MG | 27.7 | Lung ca. (non-s.cell) NCI-H23 | 39.0 |
| astrocytoma SW1783 | 28.5 | Lung ca. (non-s.cell) HOP-62 | 29.7 |
| neuro*; met SK-N-AS | 15.2 | Lung ca. (non-s.cl) NCI-H522 | 46.3 |
| astrocytoma SF-539 | 13.8 | Lung ca. (squam.) SW 900 | 17.7 |
| astrocytoma SNB-75 | 13.5 | Lung ca. (squanl)NCI-H596 | 12.7 |
| glioma SNB-19 | 2.3 | Mammary gland | 42.0 |
| glioma U251 | 18.4 | Breast ca.* (pl.ef) MCF- | 0.0 |
| glioma SF-295 glioma SF-295 | 36.6 | Breast ca.*(ple.ef) MDA-MB-231 | 18.7 |
| Heart (fetal) | 2.2 | Breast ca.* (pl.ef) T47D | 15.2 |
| Heart | 2.3 | Breast ca. BT-549 | 13.6 |
| Skeletal muscle (fetal) | 12.8 | Breast ca. MDA-N | 4.2 |
| Skeletal muscle | 4.2 | Ovary | 15.0 |
| Bone marrow | 11.7 | Ovarian ca. OVCAR-3 | 6.0 |
| Thymus | 14.5 | Ovarian ca. OVCAR-4 | 12.2 |
| Spleen | 19.2 | Ovarian ca. OVCAR-5 | 39.5 |
| Lymph node | 17.2 | Ovarian ca. OVCAR-8 | 3.8 |
| Colorectal | 4.4 | Ovarian ca. IGROV-1 | 41.2 |
| Stomach | 26.2 | Ovarian ca.* (ascites) SK-OV-3 | 14.7 |
| Small intestine | 8.8 | Uterus | 24.7 |
| Colon ca. SW480 | 19.3 | Placenta | **100.0** |
| Colon ca.* SW620(SW480 met) | 19.8 | Prostate | 8.0 |
| Colon ca. HT29 | 4.1 | Prostate ca.* (bone met)PC-3 | 11.1 |
| Colon ca. HCT-116 | 7.9 | Testis | 49.3 |
| Colon ca. CaCo-2 | 29.7 | Melanoma Hs688(A).T | 5.7 |
| Colon ca. tissue(ODO3866) | 9.1 | Melanoma* (met) Hs688(B).T | 7.9 |
| Colon ca. HCC-2998 | 9.9 | Melanoma UACC-62 | 4.0 |
| Gastric ca.* (liver met) NCI-N87 | 28.5 | Melanoma M14 | 2.9 |
| Bladder | 14.0 | Melanoma LOX IMVI | 15.8 |
| Trachea | 9.3 | Melanoma* (met) SK- MEL-5 | 2.0 |
| Kidney | 43.8 | Adipose | 8.4 |

**Table DD. Panel 2.2**

| **Tissue Name** | **Rel. Exp.(%) Ag3215, Run 174416485** | **Tissue Name** | **Rel. Exp.(%) Ag3215, Run 174416485** |
|---|---|---|---|
| Normal Colon | 4.4 | Kidney Margin (OD04348) | **100.0** |
| Colon cancer (OD06064) | 10.4 | Kidney malignant cancer (OD06204B) | 47.6 |
| Colon Margin (OD06064) | 2.9 | Kidney normal adjacent tissue (OD06204E) | 29.9 |
| Colon cancer (OD06159) | 0.0 | Kidney Cancer (OD04450-01) | 27.7 |
| Colon Margin (OD06159) | 4.7 | Kidney Margin (OD04450-03) | 31.2 |
| Colon cancer (OD06297-04) | 0.0 | Kidney Cancer 8120613 | 0.0 |
| Colon Margin (OD06297-05) | 4.6 | Kidney Margin 8120614 | 36.6 |
| CC Gr.2 ascend colon (ODO3921) | 21.8 | Kidney Cancer 9010320 | 10.7 |
| CC Margin (OD03921) | 0.0 | Kidney Margin 9010321 | 12.3 |
| Colon cancer metastasis (OD06104) | 0.0 | Kidney Cancer 8120607 | 27.9 |
| Lung Margin (OD06104) | 0.0 | Kidney Margin 8120608 | 4.5 |
| Colon mets to lung (OD04451-01) | 7.9 | Normal Uterus | 21.0 |
| Lung Margin (OD04451-02) | 19.2 | Uterine Cancer 064011 | 18.8 |
| Normal Prostate | 14.6 | Normal Thyroid | 0.0 |
| Prostate Cancer (OD04410) | 2.1 | Thyroid Cancer 064010 | 0.0 |
| Prostate Margin (OD04410) | 9.6 | Thyroid Cancer A302152 | 49.7 |
| Normal Ovary | 18.4 | Thyroid Margin A302153 | 0.0 |
| Ovarian cancer (OD06283-03) | 18.9 | Normal Breast | 0.0 |
| Ovarian Margin (OD06283-07) | 23.5 | Breast Cancer (OD04566) | 0.0 |
| Ovarian Cancer 064008 | 14.4 | Breast Cancer 1024 | 79.0 |
| Ovarian cancer (OD06145) | 5.8 | Breast Cancer (OD04590-01) | 33.7 |
| Ovarian Margin (OD06145) | 39.5 | Breast Cancer Mets (OD04590-03) | 17.2 |
| Ovarian cancer (OD06455-03) | 59.9 | Breast Cancer Metastasis (OD04655-05) | 49.0 |
| Ovarian Margin (OD06455-07) | 0.0 | Breast Cancer 064006 | 27.4 |
| Normal Lung | 14.8 | Breast Cancer 9100266 | 3.8 |
| Invasive poor diff. lung adeno (ODO4945-01 | 3.5 | Breast Margin 9100265 | 10.4 |
| Lung Margin (ODO4945-03) | 3.6 | Breast Cancer A209073 | 0.0 |
| Lung Malignant Cancer (OD03126) | 0.0 | Breast Margin A2090734 | 54.7 |
| Lung Margin (OD03126) | 10.8 | Breast cancer (OD06083) | 17.7 |
| Lung Cancer (OD05014A) | 15.7 | Breast cancer node metastasis (OD06083) | 76.3 |
| Lung Margin (OD05014B) | 2.0 | Normal Liver | 18.3 |
| Lung cancer (OD06081) | 12.4 | Liver Cancer 1026 | 0.0 |
| Lung Margin (OD06081) | 4.4 | Liver Cancer 1025 | 19.6 |
| Lung Cancer (OD04237-01) | 5.6 | Liver Cancer 6004-T | 0.0 |
| Lung Margin (OD04237-02) | 0.0 | Liver Tissue 6004-N | 0.0 |
| Ocular Melanoma Metastasis | 8.4 | Liver Cancer 6005-T | 0.0 |
| Ocular Melanoma Margin (Liver) | 0.0 | Liver Tissue 6005-N | 0.0 |
| Melanoma Metastasis | 0.0 | Liver Cancer 064003 | 18.9 |
| Melanoma Margin (Lung) | 0.0 | Normal Bladder | 33.7 |
| Normal Kidney | 37.4 | Bladder Cancer 1023 | 4.0 |
| Kidney Ca, Nuclear grade 2 (OD04338) | 82.9 | Bladder Cancer A302173 | 20.0 |
| Kidney Margin (OD04338) | 7.6 | Normal Stomach | 20.7 |
| Kidney Ca Nuclear grade 1/2 (OD04339) | 94.6 | Gastric Cancer 9060397 | 0.0 |
| Kidney Margin (OD04339) | 19.1 | Stomach Margin 9060396 | 6.0 |
| Kidney Ca, Clear cell type (OD04340) | 0.0 | Gastric Cancer 9060395 | 6.0 |
| Kidney Margin (OD04340) | 28.7 | Stomach Margin 9060394 | 4.0 |
| Kidney Ca, Nuclear grade 3 (OD04348) | 0.0 | Gastric Cancer 064005 | 11.9 |

**Table DE. Panel 4D**

| **Tissue Name** | **Rel. Exp.(%) Ag3215, Run 164682514** | **Tissue Name** | **Rel. Exp.(%) Ag3215, Run 164682514** |
|---|---|---|---|
| Secondary Th1 act | 6.8 | HUVEC IL-1beta | 5.8 |
| Secondary Th2 act | 7.4 | HUVEC IFN gamma | 29.5 |
| Secondary Tr1 act | 8.7 | HUVEC TNF alpha + IFN gamma | 6.0 |
| Secondary Th1 rest | 2.8 | HUVEC TNF alpha + IL4 | 17.6 |
| Secondary Th2 rest | 4.0 | HUVEC IL-11 | 11.5 |
| Secondary Tr1 rest | 6.3 | Lung Microvascular EC none | 34.4 |
| Primary Th1 act | 12.7 | Lung Microvascular EC TNFalpha + IL-1beta | 42.3 |
| Primary Th2 act | 12.5 | Microvascular Dermal EC none | 14.1 |
| Primary Tr1 act | 11.1 | Microsvasular Dermal EC TNFalpha + IL-1beta | 13.9 |
| Primary Th1 rest | 12.8 | Bronchial epithelium TNFalpha + IL1beta | **100.0** |
| Primary Th2 rest | 5.7 | Small airway epithelium none | 28.1 |
| Primary Tr1 rest | 2.4 | Small airway epithelium TNFalpha + IL-1beta | 74.2 |
| CD45RA CD4 lymphocyte act | 7.9 | Coronery artery SMC rest | 26.8 |
| CD45RO CD4 lymphocyte act | 12.0 | Coronery artery SMC TNFalpha + IL-1beta | 2.8 |
| CD8 lymphocyte act | 19.3 | Astrocytes rest | 17.3 |
| Secondary CD8 lymphocyte rest | 13.5 | Astrocytes TNFalpba + IL-1beta | 2.9 |
| Secondary CD8 lymphocyte act | 10.1 | KU-812 (Basophil) rest | 9.0 |
| CD4 lymphocyte none | 1.6 | KU-812 (Basophil) PMA/ionomycin | 6.3 |
| 2ry Th1/Th2/Tr1_anti-CD95 CH11 | 5.1 | CCD1106 (Keratinocytes) none | 25.2 |
| LAK cells rest | 1.0 | CCD1106 (Keratinocytes) TNFalpha + IL-1beta | 26.2 |
| LAK cells IL-2 | 12.3 | Liver cirrhosis | 13.2 |
| LAK cells IL-2+IL-12 | 5.7 | Lupus kidney | 8.4 |
| LAK cells IL-2+IFN gamma | 5.8 | NCI-H292 none | 31.6 |
| LAK cells IL-2+ IL-18 | 7.2 | NCI-H292 IL-4 | 22.2 |
| LAK cells PMA/ionomycin | 2.9 | NCI-H292 IL-9 | 57.4 |
| NK Cells IL-2 rest | 9.2 | NCI-H292 IL-13 | 26.4 |
| Two Way MLR 3 day | 14.4 | NCI-H292 IFN gamma | 35.1 |
| Two Way MLR 5 day | 1.9 | HPAEC none | 15.0 |
| Two Way MLR 7 day | 12.9 | HPAEC TNF alpha + IL-1 beta | 6.9 |
| PBMC rest | 7.1 | Lung fibroblast none | 17.6 |
| PBMC PWM | 12.5 | Lung fibroblast TNF alpha + IL-1 beta | 12.2 |
| PBMC PHA-L | 18.9 | Lung fibroblast IL-4 | 21.9 |
| Ramos (B cell) none | 2.3 | Lung fibroblast IL-9 | 17.0 |
| Ramos (B cell) ionomycin | 0.0 | Lung fibroblast IL-13 | 16.7 |
| B lymphocytes PWM | 20.3 | Lung fibroblast IFN gamma | 18.8 |
| B lymphocytes CD40L and IL-4 | 8.7 | Dermal fibroblast CCD 1070 rest | 44.8 |
| EOL-1 dbcAMP | 0.0 | Dermal fibroblast CCD1070 TNF alpha | 30.1 |
| EOL-1 dbcAMP PMA/ionomycin | 0.0 | Dermal fibroblast CCD1070 IL-1 beta | 25.3 |
| Dendritic cells none | 8.8 | Dermal fibroblast IFN gamma | 13.3 |
| Dendritic cells LPS | 0.0 | Dermal fibroblast IL-4 | 14.1 |
| Dendhtic cells anti-CD40 | 6.0 | IBD Colitis 2 | 2.6 |
| Monocytes rest | 25.2 | IBD Crohn's | 11.1 |
| Monocytes LPS | 3.3 | Colon | 26.2 |
| Macrophages rest | 11.1 | Lung | 19.3 |
| Macrophages LPS | 6.7 | Thymus | 56.3 |
| HUVEC none | 37.9 | Kidney | 18.9 |
| HUVEC starved | 11.3 | | |

**CNS_neurodegeneration_v1.0 Summary:** Ag3215 This panel does not show differential expression of the CG57360-01 gene in Alzheimer's disease. However, this expression profile confirms the presence of this gene in the brain. Please see Panel 1.3D for discussion of utility of this gene in the central nervous system.

**Panel 1.3D Summary:** Ag3215 Highest expression of the CG57360-01 gene is seen in the placenta (CT=31.7). Significant expression is also seen in samples derived from pancreatic cancer, liver cancer, lung cancer, ovarian cancer, brain cancer, and colon cancer. Thus, expression of this gene could be used to differentiate these samples from other samples on this panel and as a marker to detect the presence of these cancers. Furthermore, therapeutic modulation of the expression or function of this gene may be effective in the treatment of these cancers.

Among tissues with metabolic function, this gene is expressed in fetal and adult liver, pancreas, and the adrenal and thyroid glands. This widespread expression of the gene in these tissues suggests that its protein product may play a role in normal metabolic function. This gene product may also be useful in the treatment of metabolic disorders, including obesity and diabetes.

In addition, this molecule is expressed at moderate to low levels in the CNS and may be useful for the treatment of neurologic diseases.

**Panel 2.2 Summary:** Ag 3215 The expression of the CG57360-01 gene appears to be highest in a sample derived from a normal kidney tissue (CT=33.8). In addition, there appears to be substantial expression in other samples derived from kidney cancers and breast cancers. Thus, the expression of this gene could be used to distinguish normal kidney tissue from other samples in the panel. Moreover, therapeutic modulation of this gene, through the use of small molecule drugs, protein therapeutics, or antibodies could be of benefit in the treatment of kidney or breast cancer.

**Panel 4D Summary:** Ag3215 Highest expression of the CG57360-01 gene is seen in bronchial epithelium treated with TNF-alpha + EL-1 beta (CT=31.2). Significant expression is also seen in TNF-alpha/1L-1 beta stimulated small airway epithelium, IL-4, IL-9, IL-13, IFN gamma activated-NCI-H292 mucoepidermoid cells as well as untreated NCI-H292 cells, IL-4, IL-9, IL-13 and IFN gamma activated lung fibroblasts, and both treated and untreated lung microvascular endothelial cells The expression of this gene in cells derived from or within the lung suggests that this gene may be involved in normal conditions as well as pathological and inflammatory lung disorders, including chronic obstructive pulmonary disease, asthma, allergy and emphysema.

### E. NOV6: BCSC-1

Expression of gene CG57362-01 was assessed using the primer-probe set Ag3217, described in Table EA. Results of the RTQ-PCR runs are shown in Tables EB, EC, ED, EE and EF.

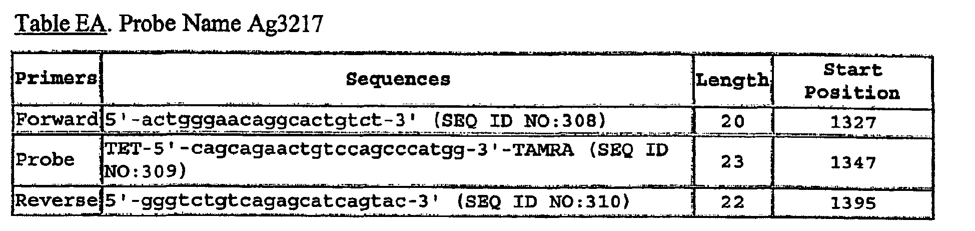

**Table EB. CNS_neurodegeneration_v1.0**

| **Tissue Name** | **Rel.Exp.(%) Ag3217, Run 209861783** | **Rel.Exp.(%) Ag3217, Run 224079666** | **Tissue Name** | **ReL Exp.(%) Ag3217, Run 209861783** | **Rel. Exp.(%) Ag3217, Run 224079666** |
|---|---|---|---|---|---|
| AD 1 Hippo | 18.3 | 24.8 | Control 3 (path) Temporal Ctx | 15.6 | 17.7 |
| AD 2 Hippo | 30.4 | 42.6 | Control (Path) 4 Temporal Ctx | 72.2 | 81.8 |
| AD 3 Hippo | 20.2 | 18.3 | AD 1 Occipital Ctx | 25.2 | 32.5 |
| AD 4 Hippo | 21.3 | 22.2 | AD 2 Occipital Ctx (Missing) | 0.0 | 0.0 |
| AD 5 Hippo | 82.9 | 78.5 | AD 3 Ctx Occipital | 7.2 | 7.4 |
| AD 6 Hippo | 29.9 | 33.2 | AD 4 Occipital Ctx | 52.1 | 36.6 |
| Control 2 Hippo | 43.2 | 45.1 | AD 5 Occipital Ctx | 46.3 | 38.4 |
| Control 4 Hippo | 26.2 | 17.7 | AD 6 Occipital Ctx | 17.2 | 17.9 |
| Control (Path) 3 Hippo | 24.3 | 12.8 | Control 1 Occipital Ctx | 5.1 | 8.0 |
| AD 1 Temporal Ctx | 12.6 | 15.3 | Control 2 Occipital Ctx | 51.8 | 75.8 |
| AD 2 Temporal Ctx | 51.8 | 33.4 | Control 3 Occipital Ctx | 71.7 | 85.9 |
| AD 3 Temporal Ctx | 12.3 | 13.3 | Control 4 Occipital Ctx | 14.4 | 17.6 |
| AD 4 Temporal Ctx | 45.4 | 46.7 | Control (Path) 1 Occipital Ctx | **100.0** | **100.0** |
| AD 5 Inf Temporal Ctx | 56.6 | 51.4 | Control (Path) 2 Occipital Ctx | 46.3 | 53.2 |
| AD 5 Sup Temporal Ctx | 43.8 | 33.2 | Control (Path) 3 Occipital Ctx | 5.9 | 6.3 |
| AD 6 Inf Temporal Ctx | 40.3 | 33.7 | Control (Path) 4 Occipital Ctx | 50.7 | 58.6 |
| AD 6 Sup Temporal Ctx | 38.4 | 46.3 | Control 1 Parietal Ctx | 10.6 | 10.4 |
| Control 1 Temporal Ctx | 12.9 | 11.0 | Control 2 Parietal Ctx | 34.4 | 43.2 |
| Control 2 Temporal Ctx | 45.7 | 41.8 | Control 3 Parietal Ctx | 49.7 | 37.1 |
| Control 3 Temporal Ctx | 57.4 | 71.7 | Control (Path) 1 Parietal Ctx | 82.4 | 86.5 |
| Control 3 Temporal Ctx | 42.3 | 37.1 | Control (Path) 2 Parietal Ctx | 55.5 | 63.7 |
| Control (Path) 1 Temporal Ctx | 87.1 | 99.3 | Control (Path) 3 Parietal Ctx | 12.9 | 9.3 |
| Control (Path) 2 Temporal Ctx | 92.0 | 97.3 | Control (Path) Parietal Ctx | 90.1 | 95.9 |

**Table EC. Panel 1.3D**

| **Tissue Name** | **Rel. Exp.(%) Ag3217, Run 169012860** | **Tissue Name** | **Rel. Exp.(%) Ag3217, Run 168012860** |
|---|---|---|---|
| Liver adenocarcinoma | 3.9 | Kidney (fetal) | 2.6 |
| Pancreas | 8.1 | Renal ca. 786-0 | 0.4 |
| Pancreatic ca. CAPAN 2 | 0.0 | Renal ca. A498 | 0.0 |
| Adrenal gland | 24.3 | Renal ca.RXF 393 | 0.0 |
| Thyroid | 0.0 | Renal ca. ACHN | 0.0 |
| Salivary gland | 5.4 | Renal ca. UO-31 | 0.1 |
| Pituitary gland | 12.9 | Renal ca. TK-10 | 1.2 |
| Brain (fetal) | 10.2 | Liver | 0.9 |
| Brain (whole) | 31.6 | Liver (fetal) | 0.0 |
| Brain (amygdala) | 72.2 | Liver ca. (hepatoblast) HepG2 | 0.0 |
| Brain (cerebellum) | 21.5 | Lung | 0.0 |
| Brain (hippocampus) | 39.5 | Lung(fetal) | 1.4 |
| Brain (substantia nigra) | 113 | Lung ca. (small cell) LX-1 | 1.7 |
| Brain (thalamus) | 31.6 | Lung ca. (small cell) NCI-H69 | 6.6 |
| Cerebral Cortex | 39.8 | Lung ca. (s.cell var.) SHP-77 | 27.7 |
| Spinal cord | 1.4 | Lung ca. (large cell)NCI-H460 | 0.0 |
| glio/astro U87-MG | 0.0 | Lung ca. (non-sm. cell) A549 | 4.4 |
| glio/astro U-118-MG | 0.0 | Lung ca. (non-s.cell) NCI-H23 | 0.4 |
| astrocytoma SW1783 | 0.0 | Lung ca. (non-s.cell) HOP-62 | 0.0 |
| neuro*; met SK-N-AS | 0.3 | Lung ca. (non-s.cl) NCI-H522 | 2.9 |
| astrocytoma SF-539 | 0.1 | Lung ca. (squam.) SW 900 | 1.0 |
| astrocytoma SNB-75 | 0.0 | Lung ca. (squam.) NCI-H596 | **100.0** |
| glioma SNB-19 | 2.4 | Mammary gland | 0.0 |
| glioma U251 | 3.4 | Breast ca.* (pl.ef) MCF-7 | 0.0 |
| glioma SF-295 | 0.8 | Breast ca.* (pl.ef) MDA-MB-231 | 0.0 |
| Heart (fetal) | 0.0 | Breast ca.* (pl.ef) T47D | 4.6 |
| Heart | 0.0 | Breast ca. BT-549 | 0.0 |
| Skeletal muscle (fetal) | 1.1 | Breast ca. MDA-N | 0.2 |
| Skeletal muscle | 0.0 | Ovary | 1.4 |
| Bone marrow | 1.4 | Ovarian ca. OVCAR-3 | 0.0 |
| Thymus | 0.0 | Ovarian ca. OVCAR-4 | 0.8 |
| Spleen | 0.4 | Ovarian ca. OVCAR-5 | 3.2 |
| Lymph node | 0.0 | Ovarian ca. OVCAR-8 | 0.8 |
| Colorectal | 2.6 | Ovarian ca. IGROV-1 | 0.0 |
| Stomach | 3.2 | Ovarian ca.* (ascites) SK-OV-3 | 0.3 |
| Small intestine | 1.3 | Utems | 0.0 |
| Colon ca. SW480 | 1.5 | Placenta | 0.0 |
| Colon ca.* SW620(SW480 met) | 11.9 | Prostate | 1.6 |
| Colon ca. HT29 | 0.0 | Prostate ca.* (bone met)PC-3 | 0.4 |
| Colon ca. HCT-116 | 2.9 | Testis | 2.6 |
| Colon ca. CaCo-2 | 0.0 | Melanoma Hs688(A).T | 0.0 |
| Colon ca. tissue(ODO3866) | 0.0 | Melanoma* (met) Hs688(B).T | 0.0 |
| Colon ca. HCC-2998 | 0.0 | Melanoma UACC-62 | 0.0 |
| Gastric ca.* (liver met) NCI-N87 | 0.0 | Melanoma M14 | 0.0 |
| Bladder | 0.8 | Melanoma LOX IMVI | 0.0 |
| Trachea | 0.0 | Melanoma* (met) SK-MEL-5 | 0.0 |
| Kidney | 0.0 | Adipose | 0.0 |

**Table ED. Panel 2.2**

| **Tissue Name** | **Rel. Exp.(%) Ag3217, Run 174416493** | **Tissue Name** | **Rel. Exp.(%) Ag3217, Run 174416493** |
|---|---|---|---|
| Normal Colon | 11.9 | Kidney Margin (OD04348) | 9.9 |
| Colon cancer (OD06064) | 0.0 | Kidney malignant cancer (OD06204B) | 0.0 |
| Colon Margin (OD06064) | 0.0 | Kidney normal adjacent tissue (OD06204E) | 0.0 |
| Colon cancer (OD06159) | 0.0 | Kidney Cancer (OD04450-01) | 3.9 |
| Colon Margin (OD06159) | 6.3 | Kidney Margin (OD04450-03) | 0.0 |
| Colon cancer (OD06297-04) | 0.0 | Kidney Cancer 8120613 | 0.0 |
| Colon Margin (OD06297-05) | 0.0 | Kidney Margin 8120614 | 0.0 |
| CC Gr.2 ascend colon (OD03921) | 0.0 | Kidney Cancer 9010320 | 47.6 |
| CC Margin (ODO3921) | 0.0 | Kidney Margin 9010321 | 0.0 |
| Colon cancer metastasis (OD06104) | 0.0 | Kidney Cancer 8120607 | 3.7 |
| Lung Margin (OD06104) | 0.0 | Kidney Margin 8120608 | 0.0 |
| Colon mets to lung (OD04451-01) | 0.0 | Normal Uterus | 0.0 |
| Lung Margin (OD04451-02) | 0.0 | Uterine Cancer 064011 | 12.7 |
| Normal Prostate | 22.4 | Normal Thyroid | 13.6 |
| Prostate Cancer (OD04410) | 8.4 | Thyroid Cancer 064010 | 0.0 |
| Prostate Margin (OD04410) | 0.0 | Thyroid Cancer A302152 | 0.0 |
| Normal Ovary | 0.0 | Thyroid Margin A302153 | 7.6 |
| Ovarian cancer (OD06283-03) | 0.0 | Normal Breast | 0.0 |
| Ovarian Margin (OD06283-07) | 0.0 | Breast Cancer (OD04566) | 0.0 |
| Ovarian Cancer 064008 | 9.5 | Breast Cancer 1024 | 0.0 |
| Ovarian cancer (OD06145) | 0.0 | Breast Cancer (OD04590-01) | 19.3 |
| Ovarian Margin (OD06145) | **100.0** | Breast Cancer Mets (OD04590-03) | 35.8 |
| Ovarian cancer (OD06455-03) | 0.0 | Breast Cancer Metastasis (OD04655-05) | 24.5 |
| Ovarian Margin (OD06455-07) | 0.0 | Breast Cancer 064006 | 0.0 |
| Normal Lung | 0.0 | Breast Cancer 9100266 | 10.0 |
| Invasive poor diff. lung adeno (ODO4945-01 | 0.0 | Breast Margin 9100265 | 0.0 |
| Lung Margin (OD04945-03) | 0.0 | Breast Cancer A209073 | 0.0 |
| Lung Malignant Cancer (OD03126) | 17.4 | Breast Margin A2090734 | 9.1 |
| Lung Margin (OD03126) | 8.8 | Breast cancer (OD06083) | 0.0 |
| Lung Cancer (OD05014A) | 0.0 | Breast cancer node metastasis (OD06083) | 5.1 |
| Lung Margin (OD05014B) | 0.0 | Normal Liver | 6.7 |
| Lung cancer (OD06081) | 12.9 | Liver Cancer 1026 | 5.6 |
| Lung Margin (OD06081) | 0.0 | Liver Cancer 1025 | 8.5 |
| Lung Cancer (OD04237-01) | 0.0 | Liver Cancer 6004-T | 0.0 |
| Lung Margin (OD04237-02) | 0.0 | Liver Tissue 6004-N | 6.0 |
| Ocular Melanoma Metastasis | 0.0 | Liver Cancer 6005-T | 0.0 |
| Ocular Melanoma Margin (Liver) | 0.0 | Liver Tissue 6005-N | 0.0 |
| Melanoma Metastasis | 0.0 | Liver Cancer 064003 | 0.0 |
| Melanoma Margin (Lung) | 0.0 | Normal Bladder | 8.4 |
| Normal Kidney | 0.0 | Bladder Cancer 1023 | 0.0 |
| Kidney Ca, Nuclear grade 2 (OD04338) | 22.1 | Bladder Cancer A302173 | 8.7 |
| Kidney Margin (OD04338) | 0.0 | Normal Stomach | 0.0 |
| Kidney Ca Nuclear grade 1/2 (OD04339) | 40.9 | Gastric Cancer 9060397 | 0.0 |
| Kidney Margin (OD04339) | 0.0 | Stomach Margin 9060396 | 12.4 |
| Kidney Ca, Clear cell type (OD04340) | 0.0 | Gastric Cancer 9060395 | 6.4 |
| Kidney Margin (OD04340) | 0.0 | Stomach Margin 9060394 | 9.3 |
| Kidney Ca, Nuclear grade 3 (OD04348) | 0.0 | Gastric Cancer 064005 | 0.0 |

**Table EE. Panel 4D**

| **Tissue Name** | **Rel. Exp.(%) Ag3217, Run 164682517** | **Tissue Name** | **Rel. Exp.(%) Ag3217, Run 164682517** |
|---|---|---|---|
| Secondary Th1 act | 0.0 | HUVEC IL-1beta | 0.0 |
| Secondary Th2 act | 0.0 | HUVEC IFN ganuna | 0.0 |
| Secondary Tr1 act | 0.0 | HUVEC TNF alpha+IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 6.9 | Bronchial epithelium TNFalpha IL1beta | 4.1 |
| Primary Th2 rest | 0.0 | Small airway epithelium none | 8.2 |
| Primary Tr1 rest | 0.0 | Small airway epithelium TNFalpha + IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 3.2 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 6.9 |
| LAK cells IL-2+IL-12 | 0.0 | Lupus kidney | 0.0 |
| LAK cells IL-2+IFN gamma | 0.0 | NCI-H292 none | 5.3 |
| LAK cells IL-2+ IL-18 | 0.0 | NCI-H292 IL-4 | 0.0 |
| LAK cells | 22.7 | NCI-H292 IL-9 | 16.8 |
| NK Cells IL-2 rest | 0.0 | NCI-H292 IL-13 | 0.0 |
| Two Way MLR 3 day | 5.6 | NCI-H292 IFN gamma | 0.0 |
| Two Way MLR 5 day | 0.0 | HPAEC none | 8.5 |
| Two Way MLR 7 day | 0.0 | HPAEC TNF alpha + IL-1 beta | 0.0 |
| PBMC rest | 0.0 | Lung fibroblast none | 0.0 |
| PBMC PWM | 3.8 | Lung fibroblast TNF alpha + IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 7.2 | Lung fibroblast IFN gamma | 0.0 |
| B lymphocytes CD40L and IL-4 | 0.0 | Dermal fibroblast CCD1070 rest | 5.4 |
| EOL-1 dbcAMP | 5.3 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 8.3 |
| Monocytes rest | 0.0 | IBD Crohn's | 20.6 |
| Monocytes LPS | 0.0 | Colon | **100.0** |
| Macrophages rest | 0.0 | Lung | 47.6 |
| Macrophages LPS | 0.0 | Thymus | 19.3 |
| HUVEC none | 0.0 | Kidney | 0.0 |
| HUVEC starved | 0.0 | | |

**Table EF. Panel CNS_1**

| **Tissue Name** | **Rel. Exp.(%) Ag3217, Run 171694584** | **Tissue Name** | **Rel. Exp.(%) Ag3217, Run 171694584** |
|---|---|---|---|
| BA4 Control | 21.0 | BA17 PSP | 14.3 |
| BA4 Control2 | 23.3 | BA17 PSP2 | 27.2 |
| BA4 Alzheimer's2 | 9.9 | Sub Nigra Control | 16.6 |
| BA4 Parkinson's | 57.4 | Sub Nigra Control2 | 8.1 |
| BA4 Parkinson's2 | 52.5 | Sub Nigra Alzheimer's2 | 9.2 |
| BA4 Huntington's | 11.9 | Sub Nigra Parkinson's2 | 10.9 |
| BA4 Huntington's2 | 24.1 | Sub Nigra Huntington's | 5.3 |
| BA4 PSP Huntington's2 | 3.6 | Sub Nigra | 17.9 |
| BA4 PSP2 | 8.5 | Sub Nigra PSP2 | 1.9 |
| BA4 Depression | 20.0 | Sub Nigra Depression | 0.0 |
| BA4 Depression2 | 9.7 | Sub Nigra Depression2 | 4.1 |
| BA7 Control | 28.9 | Glob Palladus Control | 5.6 |
| BA7 Control2 | 31.0 | Glob Palladus Control2 | 4.6 |
| BA7 Alzheimer's2 | 15.7 | Glob Palladus Alzheimer's | 5.3 |
| BA7 Parkinson's | 31.0 | Glob Palladus Alzheimer's2 | 4.7 |
| BA7 Parkinson's2 | 26.2 | Glob Palladus Parkinson's | 29.5 |
| BA7 Huntington's | 19.9 | Glob Palladus Parkinson's2 | 8.5 |
| BA7 Huntington's2 | 55.9 | Glob Palladus PSP | 2.6 |
| BA7 PSP | 15.5 | Glob Palladus PSP2 | 4.0 |
| BA7PSP2 | 12.3 | Glob Palladus Depression | 4.0 |
| BA7 Depression | 13.5 | Temp Pole Control | 13.1 |
| BA9 Control | 15.3 | Temp Pole Control2 | 31.4 |
| BA9 Control2 | 31.6 | Temp Pole Alzheimer's | 8.0 |
| BA9 Alzheimer's | 9.4 | Temp Pole Alzheimer's2 | 8.0 |
| BA9 Alzheimer's2 | 10.4 | Temp Pole Parkinson's | 33.2 |
| BA9 Parkinson's | 42.6 | Temp Pole Parkinson's2 | 26.1 |
| BA9 Parkmson's2 | 25.9 | Temp Pole Huntington's | 22.2 |
| BA9 Huntington's | 11.6 | Temp Pole PSP | 6.0 |
| BA9 Huntington's2 | 29.9 | Temp Pole PSP2 | 2.3 |
| BA9 PSP | 11.0 | Temp Pole Depression2 | 15.1 |
| BA9 PSP2 | 9.5 | Cing Gyr Control | 34.2 |
| BA9 Depression | 16.4 | Cing Gyr Control2 | 13.2 |
| BA9 Depression2 | 12.8 | Cing Gyr Alzheimer's | 3.2 |
| BA17 Control | 68.3 | Cing Gyr Alzheimer's2 | 6.9 |
| BA17 Control2 | 28.3 | Cing Gyr Parkinson's | 17.9 |
| BA17 Alzheimer's2 | 18.7 | Cing Gyr Parldnson's2 | 14.8 |
| BA17 Parkinson's | 48.3 | Cing Gyr Huntington's | 6.6 |
| BA17 Parkinson's2 | **100.0** | Cing Gyr Huntington's2 | 9.7 |
| BA17 Huntington's | 29.5 | Cing Gyr PSP | 2.2 |
| BA17 Huntington's2 | 32.3 | Cing Gyr PSP2 | 6.1 |
| BA17 Depression | 21.0 | Cing Gyr Depression | 5.2 |
| BA17 Depression2 | 53.2 | Cing Gyr Depression2 | 8.8 |

**CNS_neurodegeneration_v1.0 Summary:** Ag3217 Two experiments with the same probe and primer set show that the CG57362-01 gene exhibits decreased expression in the temporal cortex of brains suffering from Alzheimer's disease (p

**Panel 1.3D Summary:** Ag3217 Highest expression of the C657362-01 gene is seen in a lung cancer cell line (CT=30.1). Significant expression is also seen in a cluster of lung cancer cell lines and samples derived from the brain. Thus, expression of this gene could be used to differentiate between brain-derived samples, the lung cancer cell lines and the other samples on this panel. Furthermore, therapeutic modulation of the expression or function of this gene may be effective in the treatment of lung cancer.

Among tissues with metabolic function, this gene is expressed in the adrenal and pituitary glands, and the pancreas. Thus, this gene product may be useful in the diagnosis and/or treatment of metabolic disease, including obesity and diabetes.

In addition, brain-preferential expression of thsi gene indicates that drugs that target this potential mediator of Alzheimer's disease, as seen in CNS_neurodegeneration_V1.0, are likely to have brain-specific action, a desireable feature of effective drugs.

**Panel 2.2 Summary:** Ag3217 Expression of the CG57362-01 gene is restricted to a sample derived from ovarian tissue (CT=34.9). Thus, expression of this gene could be used to differentiate between this sample and other samples on this panel.

**Panel 4D Summary**: Ag3217 Expression of the CG57362-01 gene is restricted to a sample derived from the colon (CT=34.8). Therefore, expression of this gene may be used to distinguish colon from the other tissues on this panel. Furthermore, expression of this ne is decreased in colon samples from patients with IBD colitis and Crohn's disease relative to normal colon. Therefore, therapeutic modulation of the activity of the GPCR encoded by this gene may be useful in the treatment of inflammatory bowel disease

**Panel CNS_1 Summary:** Ag3217 Expression in this panel confirms the presence of the CG57362-01 gene in the brain. Please see Panels 1.3D and CNS_neurodegeneration_v1.0 for discussion of utility of this gene in the central nervous system.

### F. NOV7a and NOV7b: Amino Acid Transporter

Expression of gene CG57387-01 and variant CG57387-02 was assessed using the primer-probe sets Ag3222 and Ag4965, described in Tables FA and FB.

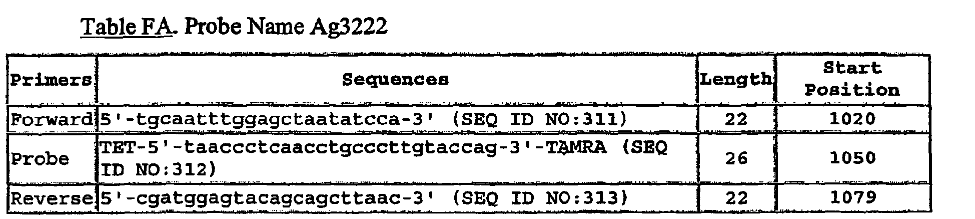

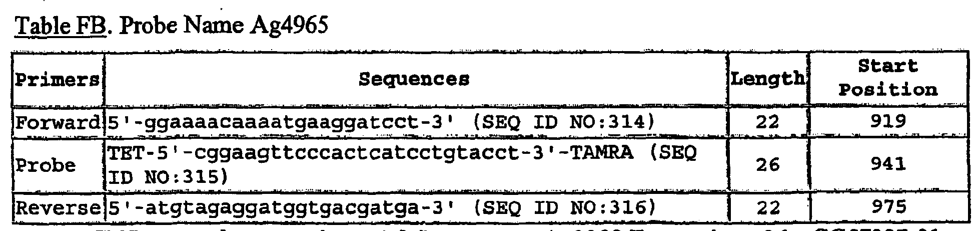

**CNS_neurodegeneration_v1.0 Summary:** Ag3222 Expression of the CG57387-01 gene is low/undetectable in all samples on this panel (CTs>35). (Data not shown.)

**General_screening_panel_v1.4 Summary:** Ag4965 Expression of the CG57387-01 gene is low/undetectable in all samples on this panel (CTs>35). (Data not shown.) The amp plot indicates that there is a high probability of a probe failure.

**Panel 1.3D Summary:** Ag3222 Expression of the CG57387-01 gene is low/undetectable in all samples on this panel (CTs>35). (Data not shown.)

**Panel 2.2 Summary:** Ag3222 Results from one experiment with the CG57387-01 gene are not included. The amp plot indicates that there were experimental difficulties with this run.

**Panel 4D Summary**: Ag3222 Expression of the CG57387-01 gene is low/undetectable in all samples on this panel (CTs>35). (Data not shown.)

### G. NOV7c: Amino acid transporter like

Expression of gene CG57387-03 was assessed using the primer-probe set Ag5127, described in Table GA. Results of the RTQ-PCR runs are shown in Tables GB, GC and GD. This probe/primer set recognizes a distinct portion of this gene and shows a distinctive expression pattern when compared to expression seen using probe and primer sets Ag3222 and Ag4965 that recognize variants CG57387-01 and CG57387-02. This observation may indicate that the probe/primer sets can distinguish splice variants of this gene. In contrast to the results obtained with Ag3222 and Ag4965 which show no expression of the CG57387-01 and CG57387-02 genes, the CG57387-03 gene produces a distinct expression profile. Please see below for presentation of the results and more complete discussion of the expression profile.

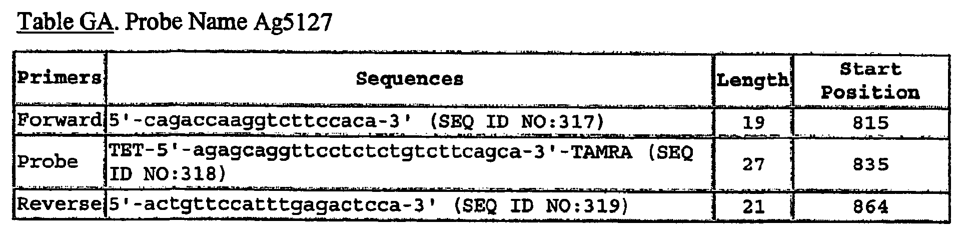

**Table GB. CNS_neurodegeneration_v1.0**

| **Tissue Name** | **Rel. Exp.(%) Ag5127, Run 226203927** | **Tissue Name** | **Rel. Exp.(%) Ag5127, Run 226203927** |
|---|---|---|---|
| AD 1 Hippo | 4.1 | Control (Path) 3 Temporal Ctx | 4.0 |
| AD 2 Hippo | 13.4 | Control (Path) 4 Temporal Ctx | 22.1 |
| AD 3 Hippo | 1.4 | AD 1 Occipital Ctx | 6.0 |
| AD 4 Hippo | 3.1 | AD 2 Occipital Ctx (Missing) | 0.0 |
| AD 5 Hippo | 69.3 | AD 3 Occipital Ctx | 6.0 |
| AD 6 Hippo | 33.0 | AD 4 Occipital Ctx | 11.9 |
| Control 2 Hippo | 22.4 | AD 5 Occipital Ctx | 25.7 |
| Control 4 Hippo | 3.5 | AD 6 Occipital Ctx | 9.5 |
| Control (Path) 3 Hippo | 1.8 | Control 1 Occipital Ctx | 1.8 |
| AD 1 Temporal Ctx | 4.7 | Control 2 Occipital Ctx | 69.3 |
| AD 2 Temporal Ctx | 14.5 | Control 3 Occipital Ctx | 12.1 |
| AD 3 Temporal Ctx | 1.6 | Control 4 Occipital Ctx | 2.2 |
| AD 4 Temporal Ctx | 11.6 | Control (Path) 1 Occipital Ctx | **100.0** |
| AD 5 Inf Temporal Ctx | 75.8 | Control (Path) 2 Occipital Ctx | 17.8 |
| AD 5 Sup Temporal Ctx | 21.5 | Control (Path) 3 Occipital Ctx | 1.0 |
| AD 6 Inf Temporal Ctx | 33.4 | Control (Path) 4 Occipital Ctx | 12.3 |
| AD 6 Sup Temporal | 38.2 | Control 1 Parietal Ctx | 1.6 |
| Control 1 Temporal Ctx | 0.5 | Control 2 Parietal Ctx | 22.2 |
| Control 2 Temporal Ctx | 31.0 | Control 3 Parietal Ctx | 6.5 |
| Control 3 Temporal Ctx | 2.4 | Control (Path) 1 Parietal Ctx | 64.6 |
| Control 3 Temporal Ctx | 3.8 | Control (Path) 2 Parietal Ctx | 48.6 |
| Control (Path) 1 Temporal Ctx | 55.9 | Control (Path) 3 Parietal Ctx | 1.5 |
| Control (Path) 2 Temporal Ctx | 46.7 | Control (Path) 4 Parietal Ctx | 37.9 |

**Table GC. General_screening_panel_v1.5**

| **Tissue Name** | **Rel. Exp.(%) Ag5127, Run 228783308** | **Tissue Name** | **Rel. Exp.(%) Ag5127, Run 228783308** |
|---|---|---|---|
| Adipose | 5.2 | Renal ca. TK-10 | 8.1 |
| Melanoma* Hs688(A).T | 2.5 | Bladder | 12.6 |
| Melanoma* Hs688(B).T | 2.0 | Gastric ca. (liver met.) NCI-N87 | 9.9 |
| Melanoma* M14 | 7.4 | Gastric ca. KATO III | 3.6 |
| Melanoma* LOXIMVI | 3.4 | Colon ca. SW-948 | 2.6 |
| Melanoma* SK-MML-5 | 6.1 | Colon ca. SW480 | 8.0 |
| Squamous cell carcinoma SCC-4 | 4.4 | Colon ca.* (SW480 met) SW620 | 3.6 |
| Testis Pool | 2.3 | Colon ca. HT29 | 1.7 |
| Prostate ca.* (bone met) PC-3 | 0.6 | Colon ca. HCT-116 | 7.3 |
| Prostate Pool | 6.8 | Colon ca. CaCo-2 | 42.0 |
| Placenta | 1.0 | Colon cancer tissue | 1.9 |
| Uterus Pool | 4.0 | Colon ca. SW1116 | 2.4 |
| Ovarian ca. OVCAR-3 | 6.2 | Colon ca. Colo-205 | 0.7 |
| Ovarian ca. SK-OV-3 | 17.2 | Colon ca. SW-48 | 0.9 |
| Ovarian ca. OVCAR-4 | 4.7 | Colon Pool | 6.1 |
| Ovarian ca. OVCAR-5 | 15.9 | Small Intestine Pool | 2.5 |
| Ovarian ca. IGROV-1 | 1.2 | Stomach Pool | 2.8 |
| Ovarian ca. OVCAR-8 | 3.0 | Bone Marrow Pool | 5.3 |
| Ovary | 1.6 | Fetal Heart | 1.6 |
| Breast ca. MCF-7 | 9.6 | Heart Pool | 0.6 |
| Breast ca.MDA-MB-231 | 10.6 | Lymph Node Pool | 9.9 |
| Breast ca. BT 549 | 9.8 | Fetal Skeletal Muscle | 2.5 |
| Breast ca. T47D | 2.6 | Skeletal Muscle Pool | 12.3 |
| Breast ca. MDA-N | 4.5 | Spleen Pool | 3.2 |
| Breast Pool | 5.6 | Thymus Pool | 9.5 |
| Trachea | 3.5 | CNS cancer (glio/astro) U87-MG | 13.7 |
| Lung | 1.4 | CNS cancer (glio/astro) U-118-MG | 9.9 |
| Fetal Lung | 4.9 | CNS cancer (neuro;met) SK-N-AS | 9.7 |
| Lung ca. NCI-N417 | 0.3 | CNS cancer (astro) SF-539 | 1.5 |
| Lung ca. LX-1 | 4.0 | CNS cancer (astro) SNB-75 | 28.7 |
| Lung ca. NCI-H146 | 0.2 | CNS cancer (glio) SNB-19 | 1.4 |
| Lung ca. SHP-77 | 3.8 | CNS cancer (glio) SF-295 | 11.3 |
| Lung ca. A549 | 8.4 | Brain (Amygdala) Pool | 3.9 |
| Lung ca. NCI-H526 | 1.0 | Brain (cerebellum) | **100.0** |
| Lung ca. NCI-H23 | 15.9 | Brain (fetal) | 17.9 |
| Lung ca. NCI-H460 | 10.3 | Brain (Hippocampus) Pool | 3.2 |
| Lung ca. HOP-62 | 3.9 | Cerebral Cortex Pool | 4.2 |
| Lung ca. NCI-H522 | 9.5 | Brain (Substantia nigra) Pool | 2.4 |
| Liver | 0.2 | Brain (Thalamus) Pool | 6.0 |
| Fetal Liver | 41.8 | Brain (whole) | 8.4 |
| Liver ca. HepG2 | 3.3 | Spinal Cord Pool | 3.3 |
| Kidney Pool | 6.3 | Adrenal Gland | 5.2 |
| Fetal Kidney | 1.0 | Pituitary gland Pool | 0.9 |
| Renal ca. 786-0 | 5.0 | Salivary Gland | 2.0 |
| Renal ca. A498 | 1.6 | Thyroid (female) | 0.2 |
| Renal ca. ACHN | 5.0 | Pancreatic ca. CAPAN2 | 2.8 |
| Renal ca. UO-31 | 7.0 | Pancreas Pool | 5.3 |

**Table GD. Panel 4.1D**

| **Tissue Name** | **Rel. Exp.(%) Ag5127, Run 225784356** | **Tissue Name** | **Rel. Exp.(%) Ag5127, Run 225784356** |
|---|---|---|---|
| Secondary Th1 act | 5.4 | HUVEC IL-1beta | 3.6 |
| Secondary Th2 act | 18.8 | HUVEC IFN gamma | 12.8 |
| Secondary Tr1 act | 19.1 | HUVEC TNF alpha + IFN gamma | 7.8 |
| Secondary Th1 rest | 5.4 | HUVEC TNF alpha + IL4 | 5.0 |
| Secondary Th2 rest | 4.2 | HUVEC IL-11 | 4.5 |
| Secondary Tr1 rest | 6.0 | Lung Microvascular EC none | 19.6 |
| Primary Th1 act | 8.2 | Lung Microvascular EC TNFalpha + IL-1beta | 12.8 |
| Primary Th2 act | 8.7 | Microvascular Dermal EC none | 10.8 |
| Primary Tr1 act | 13.4 | Microsvasular Dermal EC TNFalpha + IL-1beta | 4.2 |
| Primary Th1 rest | 9.5 | Bronchial epithelium TNFalpha + IL1beta | 5.7 |
| Primary Th2 rest | 9.0 | Small airway epithelium none | 7.3 |
| Primary Tr1 rest | 22.7 | Small airway epithelium TNFalpha + IL-1beta | 8.5 |
| CD45RA CD4 lymphocyte act | 10.4 | Coronery artery SMC rest | 5.1 |
| CD45RO CD4 lymphocyte act | 21.8 | Coronery artery SMC TNFalpha + IL-1beta | 1.5 |
| CD8 lymphocyte act | 17.2 | Astrocytes rest | 2.6 |
| Secondary CD8 lymphocyte rest | 16.8 | Astrocytes TNFalpha + IL-1beta | 1.2 |
| Secondary CD8 lymphocyte act | 5.5 | KU-812 (Basophil) rest | 16.7 |
| CD4 lymphocyte none | 5.2 | KU-812 (Basophil) PMA/ionomycin | 33.4 |
| 2ry Th1/Th2/Th1_anti-CD95 CH11 | 21.9 | CCD1106 (Keratinocytes) none | 14.9 |
| LAK cells rest | 42.0 | CCD1106 (Keratinocytes) TNFalpha + IL-1beta | 22.1 |
| LAK cells BL-2 | 14.5 | Liver cirrhosis | 2.8 |
| LAK cells IL-2+IL-12 | 10.4 | NCI-H292 none | 6.5 |
| LAK cells IL-2+IFN gamma | 10.0 | NCI-H292 IL-4 | 10.1 |
| LAK cells IL-2+IL-18 | 15.6 | NCI-H292 IL-9 | 13.7 |
| LAK cells PMA/ionomycin | 19.3 | NCI-H292 IL-13 | 11.2 |
| NK Cells IL-2 rest | 24.8 | NCI-H292 IFN gamma | 8.4 |
| Two Way MLR 3 day | 25.9 | BPAEC none | 3.6 |
| Two Way MLR 5 day | 13.0 | HPAEC TNF alpha + IL-1 beta | 12.8 |
| Two Way MLR 7 day | 10.7 | Lung fibroblast none | 19.1 |
| PBMC rest | 6.8 | Lung fibroblast TNF alpha + IL-1 beta | 11.3 |
| PBMCPWM | 3.6 | Lung fibroblast IL-4 | 27.0 |
| PBMC PHA-L | 10.3 | Lung fibroblast IL-9 | 48.3 |
| Ramos (B cell) none | 11.3 | Lung fibroblast IL-13 | 22.7 |
| Ramos (B cell) ionomycin | 8.7 | Lung fibroblast IFN gamma | 22.1 |
| B lymphocytes PWM | 8.9 | Dermal fibroblast CCD1070 rest | 17.3 |
| B lymphocytes CD40L and IL-4 | 18.9 | Dermal fibroblast CCD 1070 TNF alpha | 20.7 |
| EOL-1 dbcAMP | 84.1 | Dermal fibroblast CCD1070 IL-1 beta | 5.4 |
| EOL-1 dbcAMP PMA/ionomycin | 30.6 | Dermal fibroblast IFN gamma | 6.4 |
| Dendritic cells none | 36.6 | Dermal fibroblast IL-4 | 9.8 |
| Dendritic cells LPS | 21.3 | Dermal Fibroblasts rest | 15.1 |
| Dendritic cells anti-CD40 | 45.1 | Neutrophils TNFa+LPS | 14.7 |
| Monocytes rest | 84.1 | Neutrophils rest | 62.0 |
| Monocytes LPS | | 66.4 Colon | 11.7 |
| Macrophages rest | 73.7 | Lung | 4.9 |
| Macrophages LPS | 4.8 | Thymus | 51.4 |
| HUVEC none | 5.7 | Kidney | **100.0** |
| HUVEC starved | 7.0 | | |

**CNS_neurodegeneration_v1.0 Summary:** Ag5127 This panel does not show differential expression of the CG57387-03 gene in Alzheimer's disease. However, this expression profile confirms the presence of this gene in the brain. Please see Panel 1.3D for discussion of utility of this gene in the central nervous system.

**General_screening_panel_v1.5 Summary:** Ag5127 Highest expression of the CG57387-03 gene is seen in the cerebellum (CT=27.7). This high level of expression in the cerebellum among all tissues examined indicates a specific role for this gene product in this brain region. Given the role of amino acid transporters in lysosomal storage disorders and the role of lysosomal disorders in CNS disease, this gene product may play an important role in CNS disease. Therefore, pharmacologic modulators of the activity of this gene product may have utility in treatment of neurodegenerative disorders such as Niemann-Pick type C disease and other diseases such as Alzheimer's disease, Parkinson's disease, and huntington's disease. In particular, spinocerebellar ataxias may benefit from the modulation of this gene, since they typically involve cerebellar dysfunction.

Significant expression is also seen in a colon cancer cell line and two ovarian cancer cell lines. Thus, expression of this gene could be used to differentiate between these samples and other samples on this panel and as a marker of cerebellar tissue and ovarian and colon cancer. Furthermore, therapeutic modulation of the expression or function of this gene may be effective in the treatment of colon or ovarian cancer.

Among tissues with metabolic function, this gene is expressed at moderate to low levels in adipose, adrenal gland, pancreas, fetal heart, and adult and fetal skeletal muscle and liver. This widespread expression among these tissues suggests that this gene product may be useful for the diagnosis and/or treatment of metabolic disease, including obesity and diabetes.

In addition, this gene is expressed at much higher levels in fetal liver(CT=29) than adult liver (CT=37). Thus, expression of this gene could be used to differentiate between the two sources of this tissue (German et al., Neuroscience 2002 Feb 14;109(3):437-50; Sagne et al., Proc Natl Acad Sci U S A 2001 Jun 19;98(13):7206-11).

**Panel 4.1D Summary:** Ag5127 Highest expression of the CG57387-03 gene is seen in the kidney (CT=29.9). This gene is also expressed at moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1 .5 and also suggests a role for the gene product in cell survival and proliferation. Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### H. NOV8a and NOV8c: Lymphocyte Antigen Precursor

Expression of gene CG56417-01 and variant CG56417-03 was assessed using the primer-probe set Ag2917, described in Table HA. Results of the RTQ-PCR runs are shown in Tables HB, HC, HD, and HE.

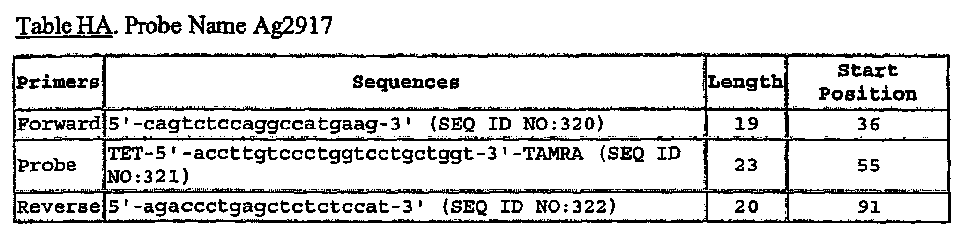

**Table HB. CNS_neurodegeneration_v1.0**

| **Tissue Name** | **Rel. Exp.(%) Ag2917, Run 209735957** | **Tissue Name** | **Rel. Exp.(%) Ag2917, Run 209735957** |
|---|---|---|---|
| AD 1 Hippo | 1.8 | Control (Path) 3 Temporal Ctx | 0.0 |
| AD 2 Hippo | 12.7 | Control (Path) 4 Temporal Ctx | |
| AD 3 Hippo | 0.0 | AD 1 Occipital Ctx | 6.6 |
| AD 4 Hippo | 0.0 | AD 2 Occipital Ctx (Missing) | 0.0 |
| AD 5 hippo | 51.8 | AD 3 Occipital Ctx | 5.3 |
| AD 6 Hippo | 3.7 | AD 4 Occipital Ctx | 3.6 |
| Control 2 Hippo | 0.0 | AD 5 Occipital Ctx | 36.3 |
| Control 4 Hippo | 6.9 | AD 6 Occipital Ctx | 29.9 |
| Control (Path) 3 Hippo | 5.4 | Control 1 Occipital Ctx | 0.0 |
| AD 1 Temporal Ctx | 1.1 | Control 2 Occipital Ctx | 12.7 |
| AD 2 Temporal Ctx | 7.3 | Control 3 Occipital Ctx | 4.5 |
| AD 3 Temporal Ctx | 0.0 | Control 4 Occipital Ctx | 0.0 |
| AD 4 Temporal Ctx | 6.7 | Control (Path) 1 Occipital Ctr | 15.5 |
| AD 5 Inf Temporal Ctx | **100.0** | Control (Path) 2 | 0.0 |
| AD 5 SupTempoml Ctx | 7.7 | Control (Path) 3 | 4.5 |
| AD 6 Inf Temporal Ctx | 9.4 | Control (Path) 4 Occipital Ctx | 16.6 |
| AD 6 Sup Temporal Ctx | 0.0 | Control 1 Parietal Ctx | 0.0 |
| Control 1 Temporal Ctx | 0.0 | Control 2 Parietal Ctx | 17.0 |
| Control 2 Temporal Ctx | 8.9 | Control 3 Parietal Ctx | 9.5 |
| Control 3 Temporal Ctx | 19.8 | Control (Path) 1 Parietal Ctx | 19.5 |
| Control 4 Temporal Ctx | 0.0 | Control (Path) 2 Parietal Ctx | 1.6 |
| Control (Path) 1 Temporal Ctx | 31.2 | Control (Path) 3 Parietal Ctx | 4.5 |
| Control (Path) 2 Temporal Ctx | 13.5 | Control (Path) 4 Parietal Ctx | 19.5 |

**Table HC. Panel 1.3D**

| **Tissue Name** | **Rel. Exp.(%) Ag2917, Run 161411861** | **Rel. Exp.(%) Ag2917, Run 165721698** | **Tissue Name** | **Rel. Exp.(%) Ag2917, Run 161411861** | **Rel. Exp.(%) Ag2917, Run 165721698** |
|---|---|---|---|---|---|
| Liver adenocarcinoma | 0.0 | 0.0 | Kidney (fetal) | 0.0 | 0.0 |
| Pancreas | 0.0 | 0.0 | Renal ca. 786-0 | 0.0 | 0.0 |
| Pancreatic ca. CAPAN 2 | 2.5 | 25.2 | Renal ca. A498 | 6.5 | 18.7 |
| Adrenal gland | 0.0 | 0.0 | Renal ca. RXF 393 | 0.0 | 0.0 |
| Thyroid | 0.0 | 0.0 | Renal ca. ACHN | 0.4 | 0.0 |
| Salivary gland | 0.0 | 0.0 | Renal ca. UO-31 | 0.0 | 0.0 |
| Pituitary gland | 0.0 | 0.0 | Renal ca. TK-10 | 0.0 | 0.0 |
| Brain (fetal) | 0.0 | 0.0 | Liver | 0.0 | 0.0 |
| Brain (whole) | 0.0 | **100.0** | Liver (fetal) | 0.0 | 0.0 |
| Brain (amygdala) | 5.6 | 10.9 | Liver ca. (hepatoblast) HepG2 | 0.0 | 0.0 |
| Brain (cerebellum) | 6.5 | 2.5 | Lung | 0.0 | 0.0 |
| Brain (hippocampus) | 0.6 | 41.2 | Lung (fetal) | 0.0 | 6.7 |
| Brain (substantia nigra) | 0.0 | 0.0 | Lung ca. (small cell) LX-1 | 0.0 | 0.0 |
| Brain (thalamus) | 6.6 | 0.0 | Lung ca. (small cell) NCI-H69 | 0.0 | 0.0 |
| Cerebral Cortex | **100.0** | 17.1 | Lung ca. (s.cell var.) SHP-77 | 0.0 | 0.0 |
| Spinal cord | 12.9 | 26.2 | Lung ca. (large cell)NCI-H460 | 0.0 | 0.0 |
| glio/astro U87-MG | 1.7 | 0.0 | Lung ca. (non-sm. cell) A549 | 0.0 | 0.0 |
| glio/astro U-118-MG | 0.0 | 0.0 | Lung ca. (non-s.cell) NCI-H23 | 0.0 | 0.0 |
| astrocytoma SW1783 | 6.4 | 0.0 | Lung ca. (non-s.cell) HOP-62 | 0.0 | 0.0 |
| neuro*; met SK-N-AS | 0.5 | 13.4 | Lung ca. (non-s.cl) NCI-H522 | 0.3 | 0.0 |
| astrocytoma SF-539 | 0.0 | 0.0 | Lung ca. (squam.) SW 900 | 0.0 | 0.0 |
| astrocytoma SNB-75 | 0.0 | 0.0 | Lung ca. (squam.) NCI-H596 | 0.0 | 0.0 |
| glioma SNB-19 | 0.0 | 0.0 | Mammary gland | 0.0 | 0.0 |
| glioma U251 | 0.0 | 0.0 | Breast ca.* (p1.ef) MCF-7 | 0.0 | 0.0 |
| glioma SF-295 | 0.0 | 0.0 | Breast ca.* (p1.ef) MDA-MB-231 | 0.0 | 0.0 |
| Heart (fetal) | 2.1 | 0.0 | Breast ca.* (pl.ef) T47D | 0.0 | 0.0 |
| Heart | 0.0 | 12.6 | Breast ca. BT-549 | 0.0 | 0.0 |
| Skeletal muscle (fetal) | 9.0 | 0.0 | Breast ca. MDA-N | 5.5 | 0.0 |
| Skeletal muscle | 0.4 | 38.4 | Ovary | 9.8 | 0.0 |
| Bone marrow | 0.0 | 0.0 | Ovarian ca. OVCAR-3 | 0.0 | 0.0 |
| Thymus | 7.4 | 0.0 | Ovarian ca. OVCAR-4 | 0.0 | 0.0 |
| Spleen | 13.4 | 37.6 | Ovarian ca. OVCAR-5 | 0.4 | 10.3 |
| Lymph node | 0.0 | 0.0 | Ovarian ca. OVCAR-8 | 0.8 | 17.0 |
| Colorectal | 0.0 | 0.0 | Ovarian ca. IGROV-1 | 6.3 | 15.0 |
| Stomach | 0.0 | 0.0 | Ovarian ca.* (ascites) SK-OV-3 | 0.0 | 0.0 |
| Small intestine | 0.0 | 0.0 | Uterus | 0.0 | 0.0 |
| Colon ca. SW480 | 0.0 | 7.7 | Placenta | 0.0 | 0.0 |
| Colon ca.* SW620(SW480 met) | 0.0 | 0.0 | Prostate | 0.0 | 0.0 |
| Colon ca. HT29 | 0.0 | 0.0 | Prostate ca.* (bone met)PC-3 | 0.0 | 0.0 |
| Colon ca. HCT-116 | 0.0 | 0.0 | Testis | 18.0 | 0.0 |
| Colon ca. CaCo-2 | 7.8 | 0.0 | Melanoma Hs688(A).T | 0.0 | 0.0 |
| Colon ca. tissue(ODO3866) | 0.0 | 0.0 | Melanoma* (met) Hs688(B).T | 1.2 | 0.0 |
| Colon ca. HCC-2998 | 0.0 | 0.0 | Melanoma UACC-62 UACC-62 | 0.0 | 0.0 |
| Gastric ca.* (liver met) NCI-N87 | 44.1 | 71.7 | Melanoma M14 | 0.0 | 0.0 |
| Bladder | 2.0 | 0.0 | Melanoma LOX IMVI | 0.0 | 0.0 |
| Trachea | 10.9 | 4.8 | Melanoma* (met) SK-MEL-5 | 0.0 | 0.0 |
| Kidney | 0.0 | 0.0 | Adipose | 9.2 | 0.0 |

**Table HD. Panel 2D**

| **Tissue Name** | **Rel. Exp.(%) Ag2917, Run 161463558** | **Tissue Name** | **Rel. Exp.(%) Ag2917, Run 161463558** |
|---|---|---|---|
| Normal Colon | 3.2 | Kidney Margin 8120608 | 0.0 |
| CC Well to Mod Diff (ODO3866) | 0.0 | Kidney Cancer 8120613 | 0.0 |
| CC Margin (ODO3866) | 0.0 | Kidney Margin 8120614 | 0.0 |
| CC Gr.2 rectosigmoid (ODO3868) | 0.0 | Kidney Cancer 9010320 | 0.0 |
| CC Margin (ODO3868) | 0.8 | Kidney Margin 9010321 | 0.9 |
| CC Mod Diff (ODO3920) | 0.0 | Normal Uterus | 0.0 |
| CC Margin (ODO3920) | 2.0 | Uterus Cancer 064011 | 1.7 |
| CC Gr.2 ascend colon (ODO3921) | 0.2 | Normal Thyroid | 3.8 |
| CC Margin (ODO3921) | 0.0 | Thyroid Cancer 064010 | 0.1 |
| CC from Partial Hepatectomy (ODO4309) Mets | 0.0 | Thyroid Cancer A302152 | 0.0 |
| Liver Margin (ODO4309) | 0.0 | Thyroid Margin A302153 | 6.0 |
| Colon mets to lung (OD04451-01) | 0.0 | Normal Breast | 0.0 |
| Lung Margin (OD04451-02) | 0.0 | Breast Cancer (OD04566) | 0.0 |
| Normal Prostate 6546-1 | 1.6 | Breast Cancer (OD04590-01) | 0.0 |
| Prostate Cancer (OD04410) | 0.2 | Breast Cancer Mets (OD04590-03) | 0.0 |
| Prostate Margin (OD04410) | 0.8 | Breast Cancer Metastasis (OD04655-05) | 0.0 |
| Prostate Cancer (OD04720-01) | 0.5 | Breast Cancer 064006 | 0.9 |
| Prostate Margin (OD04720-02) | 0.8 | Breast Cancer 1024 | 1.4 |
| Normal Lung 061010 | 0.0 | Breast Cancer 9100266 | 0.1 |
| Lung Met to Muscle (ODO4286) | 1.7 | Breast Margin 9100265 | 0.0 |
| Muscle Margin (OD04286) | 2.0 | Breast Cancer A209073 | 1.8 |
| Lung Malignant Cancer (OD03126) | 4.2 | Breast Margin A209073 | 1.2 |
| Lung Margin (OD03126) | 1.7 | Normal Liver | 0.0 |
| Lung Cancer (OD04404) | 0.0 | Liver Cancer 064003 | 0.0 |
| Lung Margin (OD04404) | 0.1 | Liver Cancer 1025 | 0.0 |
| Lung Cancer (OD04565) | 0.9 | Liver Cancer 1026 | 0.0 |
| Lung Margin (OD04565) | 0.0 | Liver Cancer 6004-T | 0.4 |
| Lung Cancer (OD04237-01) | 0.0 | Liver Tissue 6004-N | 1.4 |
| Lung Margin (OD04237-02) | 0.0 | Liver Cancer 6005-T | 0.0 |
| Ocular Mel Met to Liver (ODO4310) | 0.0 | Liver Tissue 6005-N | 0.0 |
| Liver Margin (ODO4310) | 0.0 | Normal Bladder | 0.0 |
| Melanoma Mets to Lung (OD04321) | 0.0 | Bladder Cancer 1023 Bladder Cancer 1023 | 0.0 |
| Lung Margin (OD04321) | 0.9 | Bladder Cancer A302173 | 0.0 |
| Normal Kidney | 0.5 | Bladder Cancer (OD04718-01) | **100.0** |
| Kidney Ca, Nuclear grade 2 (OD04338) | 0.0 | Bladder Normal Adjacent (OD04718-03) | 0.0 |
| Kidney Margin (OD04338) | 0.0 | Normal Ovary | 0.0 |
| Kidney Ca Nuclear grade 1/2 (OD04339) | 0.0 | Ovarian Cancer 064008 | 0.8 |
| Kidney Margin (OD04339) | 1.2 | Ovarian Cancer (OD04768-07) | 0.0 |
| Kidney Ca, Clear cell type (OD04340) | 0.0 | Ovary Margin (OD04768-08) | 1.6 |
| Kidney Margin (OD04340) | 0.1 | Normal Stomach | 0.0 |
| Nuclear grade 3 (OD04348) | 0.0 | Gastric Cancer 9060358 | 0.0 |
| Kidney Margin (OD04348) | 0.3 | Stomach Margin 9060359 | 0.0 |
| Kidney Cancer (OD04622-01) | 0.0 | Gastric Cancer 9060395 | 1.8 |
| Kidney Margin (OD04622-03) | 0.4 | Stomach Margin 9060394 | 14.5 |
| Kidney Cancer (OD04450-01) | 0.0 | Gastric Cancer 9060397 | 0.0 |
| Kidney Margin (OD04450-03) | 0.0 | Stomach Margin 9060396 | 0.0 |
| Kidney Cancer 8120607 | 0.0 | Gastric Cancer 064005 | 31.6 |

**Table HE. Panel 3D**

| **Tissue Name** | **Rel. Exp.(%) Ag2917, Run 164629842** | **Tissue Name** | **Rel. Exp.(%) Ag2917, Run 164629942** |
|---|---|---|---|
| Daoy-Medulloblastoma | 0.0 | Ca Ski- Cervical epidermoid carcinoma (metastasis) | 0.0 |
| TE671- Medulloblastoma | 0.0 | ES-2- Ovarian clear cell carcinoma | 0.0 |
| D283 Med- Medulloblastoma | 0.0 | Ramos- Stimulated with PMA/ionomycin 6h | 0.0 |
| PFSK-1- Primitive Neuroectodermal | 0.0 | Ramos- Stimulated with PMA/ionomycin 14h | 0.0 |
| XF-498- CNS | 0.0 | MEG-01-Chronic myelogenous leukemia (megokaryoblast) | 0.0 |
| SNB-78-Glioma | 0.0 | Raji- Burkitt's lymphoma | 0.0 |
| SF-268-Glioblastoma | 0.0 | Daudi- Burkitt's lymphoma | 0.0 |
| T98G-Glioblastoma | 0.0 | U266-B-cell plasmacytoma | 0.0 |
| SK-N-SH-NeuroMastoma (metastasis) | 0.0 | CA46- Burikitt's lymphoma | 0.0 |
| SF-295- Glioblastoma | 0.0 | RL-non-Hodgkin's B-cell lymphoma | 0.0 |
| Cerebellum | 7.5 | JM1- pre-B-cell lymphoma | 0.0 |
| Cerebellum | 0.0 | Jurkat- T cell leukemia | 0.0 |
| NCI-H292-Mucoepidermoid lung carcinoma | 0.0 | TF-1- Erythroleukemia | 0.0 |
| DMS-114- Small cell lung | 0.0 | HUT 78- T-cell lymphoma | 0.0 |
| DMS-79- Small cell lung cancer | 10.3 | U937- Histiocytic lymphoma | 4.3 |
| NCI-H146- Small cell lung cancer | 5.7 | KU-812- Myelogenous leukemia | 0.0 |
| NCI-H526- Small cell lung cancer | 0.0 | 769-P- Clear cell renal carcinoma | 0.0 |
| NCI-N417- Small cell lung cancer | 0.0 | Caki-2- Clear cell renal carcinoma | 2.5 |
| NCI-H82- Small cell lung cancer | 0.0 | SW 839-Clear cell renal carcinoma | 0.0 |
| NCI-H157- Squamous cell lung cancer (metastasis) | 0.0 | G401- Wilms' tumor | 0.0 |
| NCI-H1155- Large cell lung cancer | 0.0 | Hs766T-Pancreatic carcinoma (LN metastasis) | 42.9 |
| NCI-H1299-Large cell lung cancer | 0.0 | CAPAN-1-Pancreatic adenocarcinoma (liver metastasis) | 0.0 |
| NCI-H727- Lung carcinoid | 0.0 | SU86.86-Pancreatic carcinoma (liver metastasis) | 0.6 |
| NCI-UMC-11-Lung carcinoid | 0.0 | BxPC-3-Pancreatic adenocarcinoma | 18.7 |
| LX-1- Small cell lung cancer | 0.0 | HPAC-Pancreatic adenocarcinoma | 92.0 |
| Colo-205-Colon cancer | 0.0 | MIA PaCa-2- Pancreatic carcinoma | 0.0 |
| KM12-Colon cancer | 6.3 | CFPAC-1-Pancreatic ductal adenocarcinoma | 3.3 |
| KM20L2- Colon cancer | 0.0 | PANC-1- Pancreatic epithelioid ductal carcinoma | 0.0 |
| NCI-H716- Colon cancer | 0.0 | T24-Bladder carcinma (transitional cell) | 0.0 |
| SW-48- Colon adenocarcinoma | 0.0 | 5637- Bladder carcinoma | 0.0 |
| SW1116- Colon adenocarcinoma | 0.0 | HT-1197- Bladder carcinoma | 13.6 |
| LS 174T- Colon adenocarcinoma | 0.0 | UM-UC-3- Bladder carcinma (transitional cell) | 0.0 |
| SW-948-Colon adenocarcinoma | 0.0 | A204- Rhabdomyosarcoma | 0.0 |
| SW-480- Colon adenocarcinoma | 0.0 | HT-1080- Fibrosarcoma | 0.0 |
| NCI-SNU-5-Gastric carcinoma | 0.0 | MG-63- Osteosarcoma | 0.0 |
| KATO III- Gastric carcinoma | **100.0** | SK-LMS-1-Leiomyosarcoma (vulva) | 0.0 |
| NCI-SNU-16- Gastric carcinoma | 0.0 | SJRH30-Rhabdomyosarcoma (met to bone marrow) | 0.0 |
| NCI-SNU-1- Gastric carcinoma | 0.0 | A431- Epidermoid carcinoma | 0.0 |
| RF-1- Gastric adenocarcinoma | 0.0 | WM266-4-Melanoma | 0.0 |
| RF-48- Gastric adenocarcinoma | 0.0 | DU 145-Prostatecarcinoma(brain metastasis) | 0.0 |
| MKN-45- Gastric carcinoma | 0.0 | MDA-MB-468- Breast adenocarcinoma | 12.2 |
| NCI-N87- Gastric carcinoma | 4.0 | SCC-4- Squamous cell carcinoma of tongue | 0.0 |
| OVCAR-5- Ovarian carcinoma | 3.4 | SCC-9- Squamous cell carcinoma of tongue | 0.0 |
| RL95-2- Uterine carcinoma | 3.0 | SCC-15- Squamous cell carcinoma of tongue | 0.0 |
| HelaS3- Cervical adenocarcinoma | 0.0 | CAL 27- Squamous cell carcinoma of tongue | 0.0 |

**CNS_neurodegeneration_v1.0 Summary:** Ag2917 This panel does not show differential expression of the CG56417-01 gene in Alzheimer's disease. However, this expression profile confirms the presence of this gene in the brain. Please see Panel 1.3D for discussion of utility of this gene in the central nervous system.

**Panel 1.3D Summary:** Ag2917 Two experiments with the same probe and primer set both show highest expression of the CG56417-01 gene, a putative lymphocyte antigen, in regions of the brain (CTs=32-34). Preferential expression in the cerebral cortex indicates a role for this gene product in CNS processes. Lymphocytes are involved in the inflammatory response to brain pathologies such as trauma. Therefore, targeting this gene product with drugs or directing toxins to cells that specifically express this gene product may have utility in countering the inflammation that is thought to be part of the pathologic etiology of numerous CNS pathologies, such as stroke, trauma, and neurodegenerative disorders such as Alzheimer's, Parkinson's and Huntington's diseases.

Significant expression is also seen in a gastric cancer cell line. Thus, expression of this gene could be used to differentiate samples from the brain and the cancer cell line from other samples on this panel and as a marker for brain tissue and gastric cancer (Holmin et al., Neurosurgery 1998 Feb;42(2):291-8).

**Panel 2D Summary:** Ag2917 Highest expression of the CG56417-01 gene is seen in a bladder cancer (CT=29.4). Significant expression is also seen in a gastric cancer and normal thyroid. Thus, expression of this gene could be used to differentiate between these samples and other samples on this panel and as a marker for the presence of these cancers. Furthermore, therapeutic modulation of the expression or function of this gene may be effective in the treatment of bladder, gastric, or thyroid cancer.

**Panel 3D Summary:** Ag2917 Expression of the CG56417-O1 gene is limited to cell lines derived from gastric cancer, pancreatic cancer, and bladder cancer (CTs=32-35). This expression is consistent with expression in Panel 2D. Thus, expression of this gene could be used to differentiate between these samples and other samples on this panel.

**Panel 4.1D Summary:** Ag2917 Expression of the CG56417-01 gene is low/undetectable in all samples on this panel (CTs>35). (Data not shown.)

**Panel 4D Summary:** Ag2917 Results from one experiment with the CG56417-01 gene are not included. The amp plot indicates that there were experimental difficulties with this run.

### I. NOV8b and NOV8d: Lymphocyte Antigen Like

Expression of gene CG56417-02 and variant CG56417-04 was assessed using the primer-probe set Ag4251, described in Table IA.

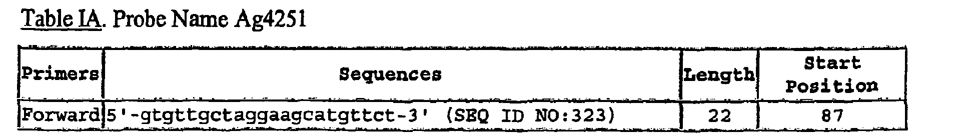

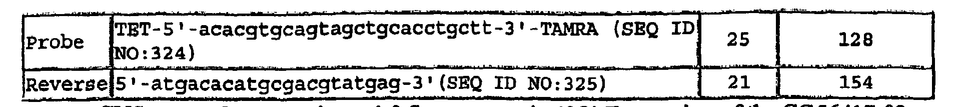

**CNS_neurodegeneration_v1.0 Summary:** Ag4251 Expression of the CG56417-02 gene is low/undetectable in all samples on this panel (CTs>35). (Data not shown.) The amp plot indicates that there is a high probability of a probe failure.

**General_screening_panel_v1.4 Summary:** Ag4251 Expression of the CG56417-02 gene is low/undetectable in all samples on this panel (CTs>35). (Data not shown.) The amp plot indicates that there is a high probability of a probe failure.

**Panel 4.1D Summary:** Ag4251 Expression of the CG56417-02 gene is low/undetectable in all samples on this panel (CTs>35). (Data not shown.) The amp plot indicates that there is a high probability of a probe failure.

### J. NOV9a and NOV9b: Early B-Cell Factor

Expression of gene CG57480-01 and variant CG57480-02 was assessed using the primer-probe set Ag3253, described in Table JA. Results of the RTQ-PCR runs are shown in Tables JB, JC and JD.

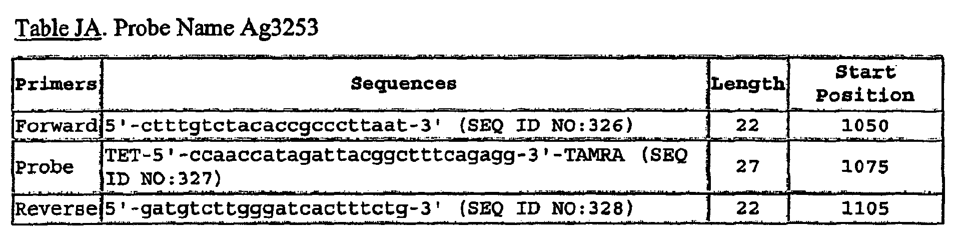

**Table JB. CNS_neurodegeneration_v1.0**

| **Tissue Name** | **Rel.Exp.(%)Ag3253, Run 206533590** | **Tissue Name** | **Rel.Exp.(%)Ag3253, Run 206533590** |
|---|---|---|---|
| AD1 Hippo | 0.0 | Control (Path) 3 Temporal Ctx | 0.0 |
| AD 2 Hippo | 7.9 | Control (Path) 4 Temporal Ctx | **100.0** |
| AD 3 Hippo | 0.0 | AD 1 Occipital Ctx | 0.0 |
| AD 4 Hippo | 0.0 | AD 2 Occipital Ctx (Missing) | 0.0 |
| AD 5 hippo | 45.7 | AD 3 Occipital Ctx | 0.0 |
| AD 6 Hippo | 94.6 | AD 4 Occipital Ctx | 23.0 |
| Control 2 Hippo | 23.5 | AD 5 Occipital Ctx | 15.6 |
| Control 4 Hippo | 0.0 | AD 6 Occipital Ctx | 0.0 |
| Control (Path) 3 Hippo | 0.0 | Control 1 Occipital Ctx | 37.9 |
| AD 1 Temporal Ctx | 51.4 | Control 2 Occipital Ctx | 0.0 |
| AD 2 Temporal Ctx | 0.0 | Control 3 Occipital Ctx | 0.0 |
| AD 3 Temporal Ctx | 39.8 | Control 4 Occipital Ctx | 55.1 |
| AD 4 Temporal Ctx | 21.2 | Control (Path) 1 Occipital Ctx | 42.0 |
| AD 5 Inf Temporal Ctx | 37.1 | Control (Path) 2 Occipital Ctx | 22.4 |
| AD 5 SupTemporal Ctx | 87.1 | Control (Path) 3 Occipital Ctx | 0.0 |
| AD 6 Inf Temporal Ctx | 76.8 | Control (Path) 4 Occipital Ctx | 70.7 |
| AD 6 Sup Temporal Ctx | 41.2 | Control 1 Parietal Ctx | 0.0 |
| Control 1 Temporal Ctx | 11.0 | Control 2 Parietal Ctx | 45.1 |
| Control 2 Temporal Ctx | 21.8 | Control 3 Parietal Ctx | 15.2 |
| Control 3 Temporal Ctx | 50.0 | Control (Path) 1 Parietal Ctx | 19.8 |
| Control 4 Temporal Ctx | 0.0 | Control (Path) 2 Parietal Ctx | 34.6 |
| Control (path) 1 Temporal Ctx | 54.0 | Control (Path) 3 Parietal Ctx | 17.7 |
| Control (Path) 2 Temporal Ctx | 40.6 | Control (Path) 4 Parietal Ctx | 0.0 |

**Table JC. Panel 1.3D**

| **Tissue Name** | **Rel. Exp.(%) Ag3253, Run 165524927** | **Tissue Name** | **Rel. Exp.(%) Ag3253, Run 165524927** |
|---|---|---|---|
| Liver adenocarcinoma | 0.0 | Kidney (fetal) | 0.7 |
| Pancreas | 3.3 | Renal ca. 786-0 | 0.0 |
| Pancreatic ca. CAPAN 2 | 0.0 | Renal ca. A498 | 0.8 |
| Adrenal gland | 1.2 | Renal ca. RXF 393 | 0.0 |
| Thyroid | 5.1 | Renal ca. ACHN | 20.9 |
| Salivary gland | 7.9 | Renal ca. UO-31 | 13.6 |
| Pituitary gland | 1.0 | Renal ca. TK-10 | 0.0 |
| Brain (fetal) | 0.0 | Liver | 0.7 |
| Brain (whole) | 3.8 | Liver (fetal) | 0.6 |
| Brain (ainygdala) | 0.0 | Liver ca. (hepatoblast) HepG2 | 0.0 |
| Brain (cerebellum) | 8.8 | Lung | 0.0 |
| Brain (hippocampus) | 1.8 | Lung (fetal) | 0.8 |
| Brain (substantia nigra) | 81.8 | Lung ca. (small cell) LX-1 | 0.0 |
| Brain (thalamus) | 0.0 | Lung ca. (small cell) NCI-H69 | 5.9 |
| Cerebral Cortex | 0.0 | Lung ca. (s.cell var.) | 0.0 |
| Spinal cord | 17.4 | Lung ca. (large cell)NCI-H460 | 0.0 |
| glio/astro U87-MG | 0.0 | Lung ca. (non-sm. cell) A549 | 2.1 |
| glio/astro U-118-MG | 0.0 | Lung ca. (non-s.cell) NCI-H23 | 0.0 |
| astrocytoma SW1783 | 0.0 | Lung ca. (non-s.cell) HOP-62 | 0.0 |
| neuro*; met SK-N-AS | 75.8 | Lung ca. (non-s.cl) NCI-H522 | 0.0 |
| astrocytoma SF-539 | 0.0 | Lung ca. (squam.) SW 900 | 2.4 |
| astrocytoma SNB-75 | 0.0 | Lung ca. (squam.) NCI-H596 | 67.4 |
| glioma SNB-19 | 0.0 | Mammary gland | 29.1 |
| glioma U251 | 0.0 | Breast ca.*(pl.ef) MCF- | 0.0 |
| glioma SF-295 | 0.0 | Breast ca.* (plef) MDA-MB-231 | 0.0 |
| Heart (fetal) | 22.5 | Breast ca.* (pl.ef) T47D | 0.0 |
| Heart | 23.3 | Breast ca. BT-549 | 0.0 |
| Skeletal muscle (fetal) | 57.0 | Breast ca. MDA-N | 0.0 |
| Skeletal muscle | **100.0** | Ovary | 3.3 |
| Bone marrow | 9.5 | Ovarian ca. OVCAR-3 | 0.0 |
| Thymus | 6.0 | Ovarian ca. OVCAR-4 | 0.0 |
| Spleen | 0.0 | Ovarian ca. OVCAR-5 | 1.0 |
| Lymph node | 98.6 | Ovarian ca. OVCAR-8 | 0.0 |
| Colorectal | 4.3 | Ovarian ca. IGROV-1 | 0.0 |
| Stomach | 4.7 | Ovarian ca.* (ascites) SK-OV-3 | 0.0 |
| Small intestine | 6.4 | Uterus | 25.0 |
| Colon ca. SW480 | 1.6 | Placenta | 11.0 |
| Colon ca.* SW620(SW480 met) | 0.0 | Prostate | 2.5 |
| Colon ca. HT29 | 0.0 | Prostate ca.* (bone met)PC-3 | 0.0 |
| Colon ca. HCT-116 | 0.7 | Testis | 11.0 |
| Colon ca. CaCo-2 | 2.8 | Melanoma Hs688(A).T | 0.7 |
| Colon ca. tissue(ODO3866) | 9.3 | Melanoma* (met) Hs688(B).T | 24.0 |
| Colon ca. HCC-2998 | 0.0 | Melanoma UACC-62 | 47.6 |
| Gastric ca.* (liver met) NCI-N87 | 0.0 | Melanoma M14 | 2.0 |
| Bladder | 2.3 | Melanoma LOX IMVI | 0.0 |
| Trachea | 14.8 | Melanoma* (met) SK-MEL-5 | 17.9 |
| Kidney | 0.0 | Adipose | 58.6 |

**Table JD. Panel 4D**

| **Tissue Name** | **Rel. Exp.(%) Ag3253, Run 164391363** | **Tissue Name** | **Rel. Exp.(%) Ag3253, Run 164391363** |
|---|---|---|---|
| Secondary Th1 act | 0.0 | HUVEC IL-1beta | 0.2 |
| Secondary Th2 act | 0.0 | HUVEC IFN gamma | 2.2 |
| Secondary Tr1 act | 0.0 | HUVEC TNF alpha + IFN gamma | 0.6 |
| Secondary Th1 rest | 0.0 | HUVEC TNF alpha + IL4 | 1.7 |
| Secondary Th2 rest | 0.0 | HUVEC IL-11 | 2.6 |
| Secondary Tr1 rest | 0.0 | Lung Microvascular EC none | 0.8 |
| Primary Th1 act | 0.0 | Lung Microvascular EC TNFalpha + IL-1beta | 0.1 |
| Primary Th2 act | 0.0 | Microvascular Dermal EC none | 0.9 |
| Primary Tr1 act | 0.0 | Microsvasular Dermal EC TNFalpha + IL-1beta | 0.5 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1beta | 0.0 |
| CD45RA CD4 lymphocyte act | 3.0 | Coronery artery SMC rest | 36.1 |
| CD45RO CD4 lymphocyte act | 0.0 | Coronery artery SMC TNFalpha + IL-1beta | 17.6 |
| CD8 lymphocyte act | 0.0 | Astrocytes rest | 0.0 |
| Secondary CD8 lymphocyte rest | 0.0 | Astrocytes TNFalpha + IL-1beta | 0.1 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 0.2 | CCD1106 (Keratinocytes) none | 0.0 |
| LAK cells rest | 0.0 | CCD1106 (Keratinocytes TNFalpha + IL-1beta | 0.0 |
| LAK cells IL-2 | 0.0 | Liver cirrhosis | 0.2 |
| LAK cells IL-2+IL-12 | 0.0 | Lupus kidney | 0.3 |
| LAK cells IL-2+IFN gamma | 0.0 | NCI-H292 none | 0.0 |
| LAK cells IL-2+IL-18 | 0.0 | NCI-H292 IL-4 | 0.0 |
| LAK cells PMA/ionomycin | 0.0 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 0.0 | HPAEC none | 0.0 |
| Two Way MLR 7 day | 0.0 | HPAEC TNF alpha + IL-1 beta | 0.0 |
| PBMC rest | 0.0 | Lung fibroblast none | 0.5 |
| PBMC PWM | 0.0 | Lung fibroblast TNF alpha + IL-1 beta | 0.5 |
| PBMC PHA-L | 0.0 | Lung fibroblast IL-4 | 1.0 |
| Ramos (B cell) none | 0.0 | Lung fibroblast IL-9 | 0.3 |
| Ramos (B cell) ionomycin | 0.0 | Lung fibroblast IL-13 | 0.8 |
| B lymphocytes PWM | 0.4 | Lung fibroblast IFN gamma | 2.2 |
| B lymphocytes CD40L and IL-4 | 0.0 | Dermal fibroblast CCD1070 rest | 15.3 |
| EOL-1 dbcAMP | 40.1 | Dermal fibroblast CCD1070 TNF alpha | 8.5 |
| EOL-1 dbcAMP PMA/ionomycin | 7.6 | Dermal fibroblast CCD1070IL-1 beta | 6.4 |
| Dendritic cells none | 0.0 | Dermal fibroblast IFN gamma | **100.0** |
| Dendritic cells LPS | 0.0 | Dermal fibroblast IL-4 | 36.1 |
| Dendritic cells anti-CD40 | 0.0 | IBD Colitis 2 | 0.2 |
| Monocytes rest | 0.0 | IBD Crohn's | 0.3 |
| Monocytes LPS | 0.1 | Colon | 2.2 |
| Macrophages rest | 0.0 | Lung | 3.5 |
| Macrophages LPS | 0.0 | Thymus | 0.9 |
| HUVEC none | 3.3 | Kidney | 3.3 |
| HUVEC starved | 8.8 | | |

**CNS_neurodegeneration_v1.0 Summary:** Ag3253 Expression of the CG57480-01 gene is low/undetectable in all samples on this panel (CTs>35). (Data not shown.)

**Panel 1.3D Summary:** Ag3253 Highest expression of the CG57480-01 gene is seen in skeletal muscle (CT=29). Significant expression is also seen in fetal skeletal muscle, adipose, fetal and adult heart, pancreas, and adrenal gland. This widespread expression among tissues with metabolic function suggests that this gene product may be important for the pathogenesis and/or treatment of disease involving these tissues, including obesity and diabetes.

Significant expression is also seen in cell lines derived from melanoma, lung cancer, renal cancer and brain cancer. Thus, expression of this gene could be used as a marker for the presence of these cancers.

Low but significant expression of this putative neuronal transcription factor is also seen in the substantia nigra, indicating a specific role for this gene product in this brain region. The substantia nigra is specifically vulnerable to neurodegeneration in Parkinson's disease. Neurodegeneration is known to involve gene regulation that is under the control of transcription factors. Therefore, drugs or treatments that modulate the activity of this gene product are potentially useful for the treatment of Parkinson's disease.

**Panel 4D Summary:** Ag3253 Highest expression of the CG57480-01 gene is seen in dermal fibroblasts treated with IFN-gamma (CT=28.6). Significant expression is also seen in a cluster of treated dermal fibroblast samples. This expression profile suggests that this gene product may be involved in skin disorders, including psoriasis.

The transcript is also expressed in differentiated EOL-1 cells, and is downregulated after activation suggesting that it may be important in eosinophil differentiation. Regulating the transcript with antisense strategies or the protein product with small molecule therapeutics may be usefue in the treatment of hematopoietic disorders involving eosinphils, parasitic infections and asthma.

### K. NOV10: High-Affinity CAMP-Specific and IBMX-Insensitive

Expression of gene CG57389-01 was assessed using the primer-probe set Ag3223, described in Table KA. Results of the RTQ-PCR runs are shown in Table KB.

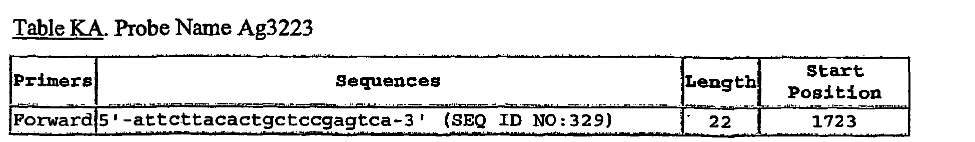

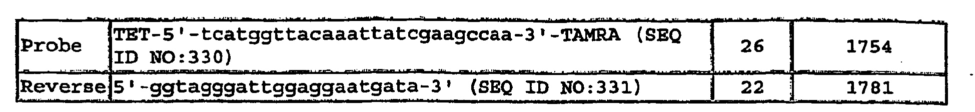

**Table KB. Panel 1.3D**

| **Tissue Name** | **Rel. Exp.(%) Ag3223, Run 165527086** | **Tissue Name** | **Rel. Exp.(%) Ag3223, Run 165527086** |
|---|---|---|---|
| Liver adenocarcinoma | 44.4 | Kidney (fetal) | 21.8 |
| Pancreas | 14.1 | Renal ca. 786-0 | 13.2 |
| Pancreatic ca. CAPAN2 | 33.7 | Renal ca. A498 | 23.7 |
| Adrenal gland | 42.9 | Renal ca. RXF 393 | 35.8 |
| Thyroid | 11.0 | Renal ca. ACHN | 6.6 |
| Salivary gland | 8.0 | Renal ca. UO-31 | 8.8 |
| Pituitary gland | 18.2 | Renal ca. TK-10 | 6.7 |
| Brain (fetal) | 7.9 | Liver | 14.0 |
| Brain (whole) | **100.0** | Liver (fetal) | 13.8 |
| Brain (amygdala) | 32.5 | Liver ca. (hepatoblast) HepG2 | 44.8 |
| Brain (cerebellum) | 21.2 | Lung | 13.8 |
| Brain (hippocampus) | 55.9 | Lung (fetal) | 9.8 |
| Brain (substantia nigra) | 80.1 | Lung ca. (small cell) LX-1 | 26.4 |
| Brain (thalamus) | 98.6 | Lung ca. (small cell) NCI-H69 | 4.0 |
| Cerebral Cortex | 67.4 | Lung ca. (s.cell var.) SHP-77 | 17.7 |
| Spinal cord | 58.6 | Lung ca. (large cell)NCI-H460 | 64.2 |
| glio/astro U87-MG | 32.5 | Lung ca. (non-sm. cell) A549 | 47.6 |
| glio/astro U-118-MG | 63.3 | Lung ca. (non-s.cell) NCI-H23 | 31.2 |
| astrocytoma SW1783 | 18.6 | Lung ca. (non-s.cell) HOP-62 | 15.5 |
| neuro*; met SK-N-AS | 13.1 | Lung ca.(non-s.cl) NCI-H522 | 6.7 |
| astrocytoma SF-539 | 34.4 | Lung ca. (squam.) SW | 11.1 |
| astrocytoma SNB-75 | 19.3 | Lung ca. (squam.) NCI-H596 | 7.7 |
| glioma SNB-19 | 32.3 | Mammary gland | 18.2 |
| glioma U251 | 64.2 | Breast ca.* (pl.ef) MCF-7 | 30.4 |
| glioma SF-295 | 9.3 | Breast ca.* (pl.ef) MDA-MB-231 | 37.1 |
| Heart (fetal) | 26.6 | Breast ca.* (pl.ef) T47D | 7.4 |
| Heart | 10.8 | Breast ca. BT-549 | 28.5 |
| Skeletal muscle (fetal) | 16.2 | Breast ca. MDA-N | 10.8 |
| Skeletal muscle | 18.3 | Ovary | 18.9 |
| Bone marrow | 4.2 | Ovarian ca. OVCAR-3 | 15.7 |
| Thymus | 7.3 | Ovarian ca. OVCAR-4 | 6.8 |
| Spleen | 14.9 | Ovarian ca. OVCAR-5 | 33.7 |
| Lymph node | 35.1 | Ovarian ca. OVCAR-8 | 3.6 |
| Colorectal | 43.5 | Ovarian ca. IGROV-1 | 7.7 |
| Stomach | 19.5 | Ovarian ca.* (ascites) SK-OV-3 | 18.2 |
| Small intestine | 49.0 | Uterus | 26.8 |
| Colon ca. SW480 | 29.7 | Placenta | 7.5 |
| Colon ca.* SW620(SW480 met) | 31.6 | Prostate | 6.5 |
| Colon ca. HT29 | 8.1 | Prostate ca.* (bone met)PC-3 | 19.2 |
| Colon ca. HCT-116 | 22.8 | Testis | 25.3 |
| Colon ca. CaCo-2 | 10.1 | Melanoma Hs688(A).T | 4.8 |
| Colon ca. tissue(ODO3866) | 20.2 | Melanoma* (met) Hs688(B).T | 9.0 |
| Colon ca. HCC-2998 | 13.1 | Melanoma UACC-62 | 12.4 |
| Gastric ca.* (liver met) NCI-N87 | 74.7 | Melanoma M14 | 74.2 |
| Bladder | 19.9 | Melanoma LOX IMVI | 3.3 |
| Trachea | 7.3 | Melanoma* (met) SK-MEL-5 | 12.3 |
| Kidney | 25.2 | Adipose | 17.1 |

**Panel 1.3D Summary:** Ag3223 The expression of the CG57389-01 gene, a nucleotide phosphodiesterase homolog, appears to be highest in a sample derived from a normal brain tissue. In addition, this gene is expressed in all CNS regions examined. Nucleotide phosphodiesterases are involved in a wide range of processes in the CNS. Therefore, this gene may be useful as a drug target in neurodegenerative diseases such as Alzheimer's, Parkinson's, Huntington's, stroke, head or spinal cord trauma, or neuropsychiatric disease.

In addition, there appears to be substantial expression in other samples derived from brain cancer cell lines, lung cancer cell lines and colon cancer cell lines. Thus, the expression of this gene could be used to distinguish normal brain tissue from other samples in the panel. Moreover, therapeutic modulation of this gene, through the use of small molecule drugs, protein therapeutics, or antibodies could be of benefit in the treatment of brain, lung or colon cancer.

Among metabolic tissues, this gene is expressed at low levels in pancreas, adrenal, thyroid, pituitary, heart, liver, adipose and skeletal muscle. Regulation of cyclic nucleotide levels is believed to be important for insulin secretion, which is dysregulated or impaired in diabetes. Thus, this gene product may be a small molecule target for the treatment of metabolic and endocrine disease, specifically Types 1 and 2 diabetes and obesity.

### L. NOV12a, NOV12b, NOV12c and NOV13: TWIK-3 and TASK-4

Expression of gene CG57220-01 and variants CG57220-02, CG57220-03 and CG57220-04 was assessed using the primer-probe sets Ag4293 and Ag937, described in Tables LA and LB. Results of the RTQ-PCR runs are shown in Tables LC, LD and LE. Please note that only variants CG57220-02 and CG57220-04 match the probe and primer set Ag937.

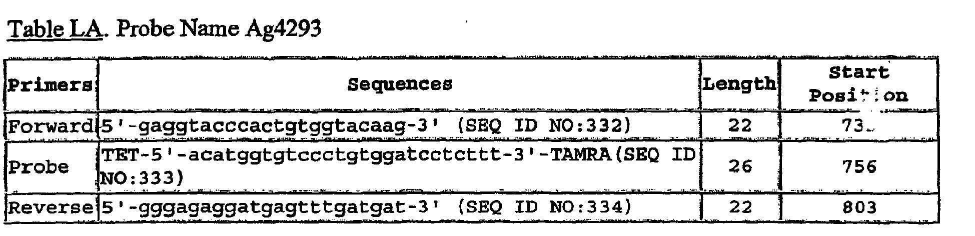

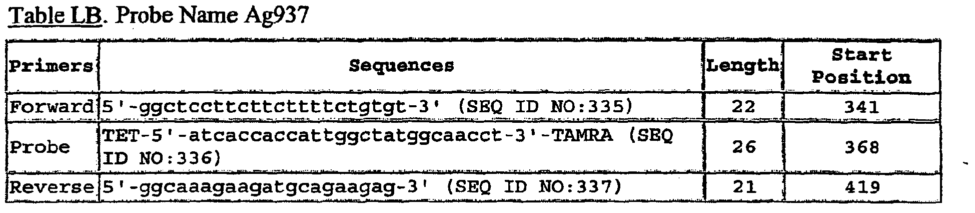

**Table LC. CNS_neurodegeneration_v1.0**

| **Tissue Name** | **Rel. Exp.(%) Ag4293, Run 224073647** | **Tissue Name** | **Rel. Exp.(%) Ag4293, Run 224073647** |
|---|---|---|---|
| AD 1 Hippo | 19.5 | Control (Path) 3 Temporal Ctx | 0.0 |
| AD 2 Hippo | 52.9 | Control (Path) 4 Temporal Ctx | 24.0 |
| AD 3 Hippo | 0.0 | AD 1 Occipital Ctx | 18.8 |
| AD 4 Hippo | 10.6 | AD 2 Occipital Ctx (Missing) | 0.0 |
| AD 5 Hippo | 26.4 | AD 3 Occipital Ctx | 0.0 |
| AD 6 Hippo | 82.4 | AD 4 Occipital Ctx | 11.4 |
| Control 2 Hippo | 50.7 | AD 5 Occipital Ctx | 33.4 |
| Control 4 Hippo | 49.0 | AD 6 Occipital Ctx | 23.3 |
| Control (Path) 3 Hippo | 11.7 | Control 1 Occipital Ctx | 8.5 |
| AD 1 Temporal Ctx | 20.9 | Control 2 Occipital Ctx | 52.5 |
| AD 2 Temporal Ctx | 47.6 | Control 3 Occipital Ctx | 0.0 |
| AD 3 Temporal Ctx | 0.0 | Control 4 Occipital Ctx | 0.0 |
| AD 4 Temporal Ctx | 3.2 | Control (Path) 1 Occipital Ctx | 27.9 |
| AD 5 Inf Temporal Ctx | 49.3 | Control (Path) 2 Occipital Ctx Occipital Ctx | 42.3 |
| AD 5 Sup Temporal Ctx | 27.5 | Control (Path) 3 Occipital Ctx | 17.9 |
| AD 6 Inf Temporal Ctx | 12.4 | Control (Path) 4 Occipital Ctx | 35.6 |
| AD 6 Sup Temporal Ctx | 13.6 | Control 1 Parietal Ctx | 10.0 |
| Control 1 Temporal Ctx | 12.7 | Control 2 Parietal Ctx | 0.0 |
| Control 2 Temporal Ctx | 3.1 | Control 3 Parietal Ctx | 0.0 |
| Control 3 Temporal Ctx | 0.0 | Control (Path) 1 Parietal Ctx | 24.7 |
| Control 3 Temporal Ctx | 0.0 | Control (Path) 2 Parietal Ctx | 53.2 |
| Control (Path) 1 Temporal Ctx | **100.0** | Control (Path) 3 Parietal Ctx | 10.8 |
| Control (Path) 2 Temporal Ctx | 52.5 | Control (Path) 4 Parietal Ctx | 56.6 |

**Table LD. General_screening_panel_v1.4**

| **Tissue Name** | **Rel. Exp.(%) Ag4293, Run 222182574** | **Tissue Name** | **Rel. Exp.(%) Ag4293, Run 222182574** |
|---|---|---|---|
| Adipose | 3.0 | Renal ca. TK-10 | 0.0 |
| Melanoma* Hs688(A).T | 0.0 | Bladder | 5.9 |
| Melanoma* Hs688(B).T | 0.0 | Gastric ca. (liver met) NCI-N87 | 0.0 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 3.3 | Colon ca. HT29 | 0.0 |
| Prostate ca.* (bone met) PC-3 | 0.0 | Colon ca. HCT-116 | 0.0 |
| Prostate Pool | 1.5 | Colon ca. CaCo-2 | 0.0 |
| Placenta | 7.5 | Colon cancer tissue | 2.1 |
| Uterus Pool | 2.5 | Colon ca. SW1116 | 0.0 |
| Ovarian ca. OVCAR-3 | 0.2 | Colon ca. Colo-205 | 0.0 |
| Ovarian ca. SK-OV-3 | 0.1 | Colon ca. SW-48 | 0.0 |
| Ovarian ca. OVCAR-4 | 0.2 | Colon Pool | 7.6 |
| Ovarian ca. OVCAR-5 | 0.5 | Small Intestine Pool | 3.1 |
| Ovarian ca. IGROV-1 | 0.0 | Stomach Pool | 4.2 |
| Ovarian ca. OVCAR-8 | 0.0 | Bone Marrow Pool | 1.2 |
| Ovary | 0.5 | Fetal Heart | 3.2 |
| Breast ca. MCF-7 | 0.2 | Heart Pool | 5.8 |
| Breast ca. MDA-MB-231 | 0.0 | Lymph Node Pool | 4.0 |
| Breast ca. BT 549 | 0.0 | Fetal Skeletal Muscle | 4.6 |
| Breast ca. T47D | 0.6 | Skeletal Muscle Pool | 0.0 |
| Breast ca. MDA-N | 0.4 | Spleen Pool | 0.7 |
| Breast Pool | 3.1 | Thymus Pool | 7.4 |
| Trachea | 1.2 | CNS cancer (glio/astro) U87-MG | 0.0 |
| Lung | 2.0 | CNS cancer (glio/astro) U-118-MG | 0.0 |
| Petal Lung | **100.0** | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 71.2 | CNS cancer (glio) SNB-19 | 0.2 |
| Lung ca. SHP-77 | 0.0 | CNS cancer (glio) SF-295 | 0.0 |
| Lung ca. A549 | 0.0 | Brain (Amygdala) Pool | 1.5 |
| Lung ca. NCI-H526 | 0.0 | Brain (cerebellum) | 1.7 |
| Lung ca. NCI-H23 | 0.2 | Brain (fetal) | 0.2 |
| Lung ca. NCI-H460 | 0.0 | Brain (Hippocampus) Pool | 1.6 |
| Lung ca. HOP-62 | 0.0 | Cerebral Cortex Pool | 1.1 |
| Lung ca. NCI-H522 | 0.0 | Brain (Substantia nigra) Pool | 0.8 |
| Liver | 1.0 | Brain (Thalanms) Pool | 0.5 |
| Fetal Liver | 1.3 | Brain (whole) | 1.4 |
| Liver ca. HepG2 | 0.0 | Spinal Cord Pool | 2.0 |
| Kidney Pool | 10.2 | Adrenal Gland | 0.6 |
| Fetal Kidney | 1.9 | Pituitary gland Pool | 1.2 |
| Renal ca. 786-0 | 0.0 | Salivary Gland | 0.4 |
| Renal ca. A498 | 0.0 | Thyroid(female) | 6.0 |
| Renal ca. ACHN | 0.0 | Pancreatic ca. CAPAN2 | 0.0 |
| Renal ca. UO-31 | 0.0 | Pancreas Pool | 3.1 |

**Table LE. Panel 4.1D**

| **Tissue Name** | **Rel. Exp.(%) Ag4293, Run 181981932** | **Tissue Name** | **Rel.Exp.(%) Ag4293, Run 181981932** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 2.8 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + ILr1beta | 0.0 |
| Primary Th2 act | 0.0 | Microvascular Dermal EC none. | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 1.8 | Small airway epithelium none | 0.0 |
| Primary Tr1 rest | 0.0 | Small airway epithelium TNFalpha + IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 1.0 |
| CD4 lymphocyte none | 2.0 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 0.9 |
| LAK ceus IL-2+IL-12 | 0.0 | NCI-H292 none | 6.7 |
| LAK cells IL-2+IFN | 0.0 | NCI-H292 IL-4 | 20.2 |
| LAK cells IL-2+IL-18 | 0.0 | NCI-H292 IL-9 | 10.7 |
| LAK cells PMA/ionomycin | 0.0 | NCI-H292 IL-13 | 29.1 |
| NK Cens IL-2 rest | 4.9 | NCI-H292 IFN gamma | 12.9 |
| Two Way MLR 3 day | 0.8 | HPAEC none | 0.0 |
| Two Way MLR 5 day | 0.0 | HPAEC TNF alpha + IL-1 beta | 0.0 |
| Two Way MLR 7 day | 0.0 | Lung fibroblast none | 0.0 |
| PBMC rest | 2.1 | Lung fibroblast TNF alpha + IL-1 beta | 0.0 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.0 | Dermal fibroblast CCD1070 rest | 0.0 |
| B lymphocytes CD40L and IL-4 | 0.0 | Dermal fibroblast CCD1070 TNF alpha | 0.7 |
| EOL-1 dbcAMP | 8.1 | Dermal fibroblast CCD1070 IL-1 beta | 1.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 0.0 |
| Dendritic cells anti-CD40 | 0.0 | Neutrophils TNFa+LPS | 0.0 |
| Monocytes rest | 0.0 | Neutrophils rest | 4.0 |
| Monocytes LPS | 0.0 | Colon | 4.8 |
| Macrophages rest | 0.0 | Lung | **100.0** |
| Macrophages LPS | 0.0 | Thymus | 24.0 |
| HUVEC none | 0.0 | Kidney | 77.9 |
| HLTVEC starved | 0.0 | | |

**CNS_neurodegeneration_v1.0 Summary:** Ag4293 No change is detected in the expression of the CG57220-01 gene in the postmortem Alzheimer's diseased brain when compared to controls. However this panel confirms the expression of this gene in the CNS in an independent group of patients. Thus, therapeutic modulation of the expression or function of this gene product may be useful in the treatment of neurologic disease. A second experiment with the probe primer set Ag937 showed low/undetectable levels of expression in all the samples on this panel (CTs>35). (Data not shown.)

**General_screening_panel_v1.4 Summary:** Ag4293 Highest expression of the CG57220-01 gene is seen in the fetal lung (CT=29)and a lung cancer cell line. In addition, this gene appears to be expressed at much higher levels in the fetal tissue than in the adult (CT=34.7). Higher levels of expression are also seen in fetal skeletal muscle C(T=33) than in adult skeletal muscle. Thus, expression of this gene could be used to differentiate between the two lung derived samples and other samples on this panel and as a marker to detect the presence of lung cancer. In addition, expression of this gene could be used to differentiate between the two sources of lung and skeletal muscle tissue. Furthermore, therapeutic modulation of the expression or function of this gene may be effective in the treatment of lung cancer.

Among tissues with metabolic function, this gene is expressed at low but significant levels in adipose, thyroid, pancreas, and adult and fetal heart. This widespread expression among these tissues suggests that this gene product may be useful for the diagnosis and/or treatment of metabolic disease, including obesity and diabetes.

**Panel 4.1D Summary:** Ag4293 Highest expression of the CG57220-01 gene is seen in the lung (CT=31). Significant levels of expression are also seen in a cluster of treated and untreated samples derived from the muco-epidermoid cell line NCI-H292, often used as a model for airway epithelium. The prominent expression in lung derived samples, both in this panel and General_screening_panel_v1.4 suggests that this gene may be involved in normal lung homeostasis as well as pathological and inflammatory lung conditions. Therefore, therapeutic modulation of this gene product may resuce symptoms associated with chronic obstructive pulmonary disease, asthma, allergy and emphysema.

**Panel CNS_1 Summary:** Ag937 Expression of the CG57220-02 gene is low/undetectable in all samples on this panel (CTs>35). (Data not shown.) M. NOV15: Cytokeratin

Expression of gene CG57454-01 was assessed using the primer-probe set Ag3244, described in Table MA. Results of the RTQ-PCR runs are shown in Tables MB, MC, MD and ME.

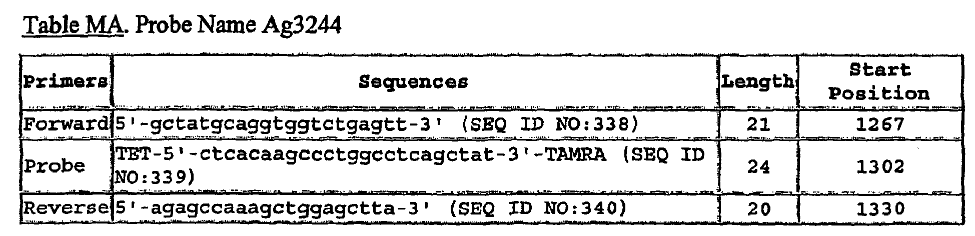

**Table MB. CNS_neurodegeneration_v1.0**

| **Tissue Name** | **Rel. Exp.(%) Ag3244, Run 206533582** | **Tissue Name** | **Rel. Exp.(%) Ag3244, Run 206533582** |
|---|---|---|---|
| AD 1 Hippo | 18.7 | Control (Path) 3 Temporal Ctx | 0.0 |
| AD 2 Hippo | 23.0 | Control (Path) 4 Temporal Ctx | 19.6 |
| AD 3 Hippo | 2.3 | AD 1 Occipital Ctx | 9.6 |
| AD 4 Hippo | 9.9 | AD 2 Occipital Ctx (Missing) | 0.0 |
| AD 5 hippo | 71.7 | AD 3 Occipital Ctx | 6.0 |
| AD 6 Hippo | 42.0 | AD 4 Occipital Ctx | 31.6 |
| Control 2 Hippo | 29.1 | AD 5 Occipital Ctx | 4.2 |
| Control 4 Hippo | 22.8 | AD 6 Occipital Ctx | 34.4 |
| Control (Path) 3 Hippo | 0.4 | Control 1 Occipital Ctx | 2.7 |
| AD 1 Temporal Ctx | 69.3 | Control 2 Occipital Ctx | 30.6 |
| AD 2 Temporal Ctx | 23.0 | Control 3 Occipital Ctx | 33.0 |
| AD 3 Temporal Ctx | 4.4 | Control 4 Occipital Ctx | 12.9 |
| AD 4 Temporal Ctx | 33.4 | Control (Path) 1 Occipital Ctx | 88.3 |
| AD 5 Inf Temporal Ctx | **100.0** | Control (Path) 2 Occipital Ctx | 9.0 |
| AD 5 SupTemporal Ctx | 23.8 | Control (Path) 3 Occipital Ctx | 0.0 |
| AD 6 Inf Temporal Ctx | 57.0 | Control (Path) 4 Occipital Ctx | 11.7 |
| AD 6 Sup Temporal Ctx | 42.0 | Control 1 Parietal Ctx | 24.5 |
| Control 1 Temporal Ctx | 4.4 | Control 2 Parietal Ctx | 59.5 |
| Control 2 Temporal Ctx | 28.7 | Control 3 Parietal Ctx | 4.5 |
| Control 3 Temporal Ctx | 15.5 | Control (Path) 1 Parietal Ctx | 59.0 |
| Control 4 Temporal Ctx | 5.3 | Control (Path) 2 Parietal Ctx | 23.2. |
| Control(Path) 1 Temporal Ctx | 28.7 | Control (Path) 3 Parietal Ctx | 0.0 |
| Control (Path) 2 Temporal Ctx | 32.5 | Control (Path) 4 Parietal Ctx | 37.6 |

**Table MC. Panel 1.3D**

| **Tissue Name** | **Rel. Exp.(%) Ag3244, Run 165524419** | **Tissue Name** | **Rel. Exp.(%) Ag3244, Run 165524419** |
|---|---|---|---|
| Liver adenocarcinonia | 91.4 | Kidney (fetal) | 10.6 |
| Pancreas | 3.4 | Renal ca. 786-0 | 8.3 |
| Pancreatic ca. CAPAN2 | 23.7 | Renal ca. A498 | 29.1 |
| Adrenal gland | 0.0 | Renal ca. RXF 393 | 1.7 |
| Thyroid | 13.4 | Renal ca. ACHN | 10.9 |
| Salivary gland | 4.1 | Renal ca. UO-31 | 42.3 |
| Pituitary gland | 57.0 | Renal ca. TK-10 | 9.4 |
| Brain (fetal) | 0.0 | Liver | 5.4 |
| Brain (whole) | 48.3 | Liver (fetal) | 12.5 |
| Brain (amygdala) | 82.9 | Liver ca. (hepatoblast) HepG2 | 18.3 |
| Brain (cerebellum) | 36.3 | Lung | 9.4 |
| Brain (hippocampus) | 88.3 | Lung (fetal) | 1.7 |
| Brain (substantia nigra) | 63.3 | Lung ca. (small cell) LX-1 | 52.5 |
| Brain (thalamus) | 41.8 | Lung ca. (small cell) NCI-H69 | 1.7 |
| Cerebral Cortex | 7.1 | Lung ca. (s.cell var.) SHP-77 | 19.3 |
| Spinal cord | 60.7 | Lung ca. (large cell)NCI-H460 | 9.5 |
| glio/astro U87-MG | 0.0 | Lung ca. (non-sm. cell) A549 | 20.6 |
| glio/astro U-118-MG | 0.0 | Lung ca. (non-s.cell) NCI-H23 | 0.0 |
| astrocytoma SW1783 | 0.0 | Lung ca. (non-s.cell) HOP-62 | 10.2 |
| neuro*; met SK-N-AS | 7.4 | Lung ca. (non-s.cl) NCI-H522 | 0.0 |
| astrocytoma SF-539 | 0.0 | Lung ca. (squam.) SW 900 | 7.4 |
| astrocytoma SNB-75 | 17.1 | H596 (squam.) NCI-H596 | 10.6 |
| glioma SNB-19 | 2.4 | Mammary gland | 7.0 |
| glioma U251 | 2.6 | Breast ca.* (pl.ef) MCF- 7 | 80.7 |
| glioma SF-295 | 0.0 | Breast ca.* (pl.ef) MDA-MB-231 | 34.9 |
| Heart (fetal) | 3.1 | Breast ca.* (pl.ef) T47D | 21.8 |
| Heart | 2.7 | Breast ca. BT-549 | 0.0 |
| Skeletal muscle (fetal) | 0.0 | Breast ca. MDA-N | 0.0 |
| Skeletal muscle | 0.0 | Ovary | 6.0 |
| Bone marrow | 0.0 | Ovarian ca. OVCAR-3 | 37.6 |
| Thymus | 0.0 | Ovarian ca. OVCAR-4 | 22.5 |
| Spleen | 0.0 | Ovarian ca. OVCAR-5 | 98.6 |
| Lymph node | 0.0 | Ovarian ca. OVCAR-8 | 5.2 |
| Colorectal | 9.3 | Ovarian ca. IGROV-1 | 6.0 |
| Stomach | 15.1 | Ovarian ca.* (ascites) SK-OV-3 | 5.2 |
| Small intestine | 39.8 | Uterus | 0.0 |
| Colon ca. SW480 | 70.7 | Placenta | 52.1 |
| Colon ca.* SW620(SW480 met) | 9.2 | Prostate | 19.1 |
| Colon ca. HT29 | 27.2 | Prostate ca.* (bone met)PC-3 | 0.0 |
| Colon ca. HCT-116 | 23.5 | Testis | 20.4 |
| Colon ca. CaCo-2 | 22.7 | Melanoma Hs688(A).T | 0.0 |
| Colon ca. tissue(ODO3866) | 85.9 | Melanoma* (met) Hs688(B).T | 0.0 |
| Colon ca. HCC-2998 | 17.8 | Melanoma UACC-62 | 0.0 |
| Gastric ca.* (liver met) NCI-N87 | **100.0** | Melanoma M14 | 0.0 |
| Bladder | 9.5 | Melanoma LOX IMVI | 0.0 |
| Trachea | 19.9 | Melanoma* (met) SK-MEL-5 | 0.0 |
| Kidney | 8.1 | Adipose | 3.8 |

**Table MD. Panel 2.2**

| **Tissue Name** | **Rel. Exp.(%) Ag3244, Run 174441100** | **Tissue Name** | **Rel. Exp.(%) Ag3244, Run 174441100** |
|---|---|---|---|
| Normal Colon | 31.0 | Kidney Margin (OD04348) | 12.8 |
| Colon cancer (OD06064) | 59.0 | Kidney malignant cancer (OD06204B) | 25.0 |
| Colon Margin (OD06064) | 8.0 | Kidney normal adjacent tissue (OD06204E) | 6.5 6.5 |
| Colon cancer (OD06159) | **100.0** | Kidney Cancer (OD04450-01) | 39.5 |
| Colon Margin (OD06159) | 43.2 | Kidney Margin (OD04450-03) | 2.2 |
| Colon cancer (OD06297-04) | 16.0 | Kidney Cancer 8120613 | 6.3 |
| Colon Margin (OD06297-05) | 38.7 | Kidney Margin 8120614 | 11.3 |
| CC Gr.2 ascend colon (OD03921) | 23.5 | Kidney Cancer 9010320 | 2.0 |
| CC Margin (ODO3921) | 20.7 | Kidney Margin 9010321 | 20.9 |
| Colon cancer metastasis (OD06104) | 12.9 | Kidney Cancer 8120607 | 17.1 |
| Lung Margin (OD06104) | 30.1 | Kidney Margin 8120608 | 4.4 |
| Colon mets to lung (OD04451-01) | 45.4 | Normal Uterus | 0.0 |
| Lung Margin (OD04451-02) | 3.3 | Uterine Cancer 064011 | 0.0 |
| Normal Prostate | 9.4 | Normal Thyroid | 8.1 |
| Prostate Cancer (OD04410) | 3.8 | Thyroid Cancer 064010 | 16.4 |
| Prostate Margin (OD04410) | 1.5 | Thyroid Cancer A302152 | 9.3 |
| Nomial Ovary | 2.6 | Thyroid Margin A302153 | 4.5 |
| Ovarian cancer (OD06283-03) | 7.5 | Normal Breast | 58.6 |
| Ovarian Margin (OD06283-07) | 1.3 | Breast Cancer (OD04566) | 16.0 |
| Ovarian Cancer 064008 | 3.0 | Breast Cancer 1024 | 17.4 |
| Ovarian cancer (OD06145) | 8.0 | Breast Cancer (OD04590-01) | 59.0 |
| Ovarian Margin (OD06145) | 3.2 | Breast Cancer Mets | 24.7 |
| Ovarian cancer (OD06455-03) | 10.4 | Breast Cancer Metastasis (OD04655-05) | 21.6 |
| Ovarian Margin (OD06455-07) | 0.0 | Breast Cancer 064006 | 12.8 |
| Normal Lung | 1.4 | Breast Cancer 9100266 | 10.0 |
| Invasive poor diff. lung adeno (ODO4945-01 | 14.2 | Breast Margin 9100265 | 1.4 |
| Lung Margin (ODO4945-03) | 2.5 | Breast Cancer A209073 | 7.6 |
| Lung Malignant Cancer (OD03126) | 25.3 | Breast Margin A2090734 | 24.7 |
| Lung Margin (OD03126) | 3.7 | Breast cancer (OD06083) | 46.0 |
| Lung Cancer (OD05014A) | 6.7 | Breast cancer node metastasis (OD06083) | 22.7 |
| Lung Margin (OD05014B) | 1.3 | Normal Liver | 5.5 |
| Lung cancer (OD06081) | 1.0 | Liver Cancer 1026 | 22.7 |
| Lung Margin (OD06081) | 1.1 | Liver Cancer 1025 | 68.3 |
| Lung Cancer (OD04237-01) | 18.6 | Liver Cancer 6004-T | 19.8 |
| LungMargm(OD04237-02) | 16.3 | Liver rime 6004-N | 11.6 |
| Ocular Melanoma Metastasis | 0.0 | Liver Cancer 6005-T | 62.4 |
| Ocular Melanoma Margin (Liver) | 10.0 | Liver Tissue 6005-N | 31.4 |
| Melanoma Metastasis | 11.3 | Liver Cancer 064003 | 7.4 |
| Melanoma Margin (Lung) | 4.1 | Nonml Bladder | 31.2 |
| Normal Kidney | 7.3 | Bladder Cancer 1023 | 20.2 |
| Kidney Ca, Nuclear grade 2 (OD04338) | 12.0 | Bladder Cancer A302173 | 20.6 |
| Kidney Margin (OD04338) | 16.2 | Normal Stomach | 51.1 |
| Kidney Ca Nuclear grade 1/2 (OD04339) | 22.5 | Gastric Cancer 9060397 | 49.0 |
| Kidney Margin (OD04339) | 3.6 | Stomach Margin 9060396 | 50.3 |
| Kidney Ca, Clear cell type (OD04340) | 11.7 | Gastric Cancer 9060395 | 52.1 |
| Kidney Margin (OD04340) | 3.7 | Stomach Margin 9060394 | 39.5 |
| Kidney Ca, Nuclear grade 3 (OD04348) | 1.1 | Gastric Cancer 064005 | 46.3 |

**Table ME. Panel 4D**

| **Tissue Name** | **Rel. Exp.(%) Ag3244, Run 164390753** | **Tissue Name** | **Rel. Exp.(%) Ag3244, Run 164390753** |
|---|---|---|---|
| Secondary Th1 act | 0.0 | HUVEC IL-1beta | 0.0 |
| Secondary Th2 act | 0.0 | HUVEC IFN gamma | 3.9 |
| Secondary Tr1 act | 0.0 | HUVEC TNF alpha + IFN gamma | 3.1 |
| Secondary Th1 rest | 0.0 | HUVEC TNF alpha + IL4 | 0.0 |
| Secondary Th2 rest | 0.0 | HUVEC IL-11 | 5.1 |
| Secondary Tr1 rest | 0.0 | Lung Microvascular EC none | 27.5 |
| Primary Th1 act | 0.0 | Lung Microvascular EC TNFalpha + IL-1beta | 39.5 |
| Primary Th2 act | 0.0 | Microvascular Dermal EC none | 0.0 |
| Primary Tr1 act | 0.0 | Microsvasular Dermal EC TNFalpha + IL-1beta | 6.3 |
| Primary Th1 rest | 0.0 | 0.0 Bronchial epithelium TNFalpha + IL1beta | 18.3 |
| Primary Th2 rest | 0.0 | Small airway epithelium none | 7.6 |
| Primary Tr1 rest | 0.0 | Small airway epithelium TNFalpha + IL-1beta | 23.7 |
| CD45RA CD4 lymphocyte act | 2.8 | Coronery artery SMC rest | 0.0 |
| CD45RO CD4 lymphocyte act | 0.0 | Coronery artery SMC TNFalpha + IL-1-beta | 0.0 |
| CD8 lymphocyte act | 3.1 | Astrocytes rest | 0.6 |
| Secondary CD8 lymphocyte rest | 0.0 | Astrocytes TNFalpha + IL-1beta | 1.3 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 0.0 | CCD1106 (Keratinocytes) none | 16.2 |
| LAK cells rest | 0.0 | CCD1106 (Keratinocytes) TNFalpha + IL-1beta | 25.0 |
| LAK cells IL-2 | 0.7 | Liver cirrhosis | 40.1 |
| LAK cells IL-2+IL-12 | 0.0 | Lupus kidney | 2.5 |
| LAK cells IL-2+IFN gamma | 0.0 | NCI-H292 none | 38.4 |
| LAK cells IL-2+IL-18 | 0.0 | NCI-H292 IL-4 | 100.0 |
| LAK cells PMA/ionomycin | 0.0 | NCI-H292 IL-9 | 58.2 |
| NK Cells IL-2 rest | 3.3 | NCI-H292 IL-13 | 59.0 |
| Two Way MLR 3 day | 0.0 | NCI-H292 IFN gamma | 87.7 |
| Two Way MLR 5 day | 0.0 | HPAEC none | 3.7 |
| Two Way MLR 7 day | 0.0 | HPAEC TNF alpha + IL-1 beta | 0.0 |
| PBMC rest | 0.0 | Lung fibroblast none | 2.4 |
| PBMC PWM | 0.0 | Lung fibroblast TNF alpha + IL-1 beta | 0.0 |
| PBMC PHA-L | 0.0 | Lung fibroblast IL-4 | 27.7 |
| Ramos (B cell) none | 0.0 | Lung fibroblast IL-9 | 13.8 |
| Ramos (B cell) ionomycin | 0.0 | Lung fibroblast IL-13 | 11.1 |
| B lymphocytes PWM | 0.0 | Lung fibroblast IFN gamma | 15.4 |
| B lymphocytes CD40L and IL-4 | 0.0 | Dermal fibroblast CCD1070 rest | 1.0 |
| EOL-1 dbcAMP | 0.0 | Dermal fibroblast CCD1070 TNF alpha | 7.8 |
| EOL-1 dbcAMP PMA/ionomycin | 0.0 | Dermal fibroblast CCD1070 IL-1 beta | 0.0 |
| Dendritic cells none | 3.2 | Dermal fibroblast IFN gamma | 80.1 |
| Dendritic cells LPS | 0.0 | Dermal fibroblast IL-4 | 98.6 |
| Dendritic cells anti-CD40 | 0.8 | IBD Colitis 2 | 3.5 |
| Monocytes rest | 0.0 | IBD Crohn's | 3.8 |
| Monocytes LPS | 0.0 | Colon | 51.4 |
| Macrophages rest | 0.0 | Lung | 17.9 |
| Macrophages LPS | 0.0 | Thymus | 9.3 |
| HUVEC none | 10.0 | Kidney | 1.5 |
| HUVEC starved | 4.7 | | |

**CNS_neurodegeneration_v1.0 Summary:** Ag3244 No change is detected in the expression of the CG57454-01 gene in the postmortem Alzheimer's diseased brain when compared to controls. See panel 1.3 for discussion of utility.

**Panel 1.3D Summary**: Ag3244 The expression of the CG57454-01 gene appears to be highest in a sample derived from a gastric cancer cell line (NCI-N87)(CT=33.9). In addition, there appears to be substantial expression in samples derived from ovarian cancer cell lines and colon cancer cell lines. Thus, the expression of this gene could be used to distinguish NCI-N87 cells from other samples in the panel. Moreover, therapeutic modulation of this gene, through the use of small molecule drugs, protein therapeutics or antibodies could be of benefit in the treatment of gastric cancer, colon cancer or ovarian cancer.

**Panel 2.2 Summary:** Ag3244 The expression of the CG57454-01 gene appears to be highest in a sample derived from a colon cancer sample (CT=33.1). In addition there appears to be substantial expression in other samples derived from colon cancers. Thus, the expression of this gene could be used to distinguish colon cancer from other samples in the panel. Moreover, therapeutic modulation of this gene, through the use of small molecule drugs, protein therapeutics or antibodies could be of benefit in the treatment of colon cancer.

**Panel 4D Summary:** Ag3244 The CG57454-01 transcript is expressed in dermal fibroblasts, lung fibroblasts, NCI-H292 cells and lung microvascular cells. The transcript is also expressed in normal colon but not in colitis samples. Thus, the transcript or the protein it encodes could be used to differentiate these cells and tissues from other samples on this panel that do not express this gene. Therapeutically, the protein encoded by this transcript could be used to design treatments for colitis, psoriasis, arthritis, asthma and emphysema.

### N. NOV16a: Protocadherin 10

Expression of gene CG57448-01 was assessed using the primer-probe sets Ag1096, Ag3242 and Ag704, described in Tables NA, NB and NC. Results of the RTQ-PCR runs are shown in Tables ND, NE, NF, NG and NH.

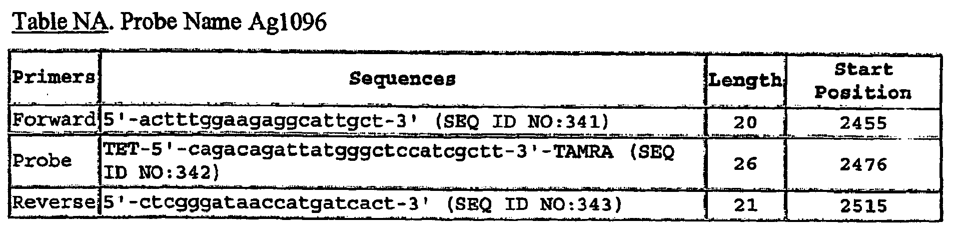

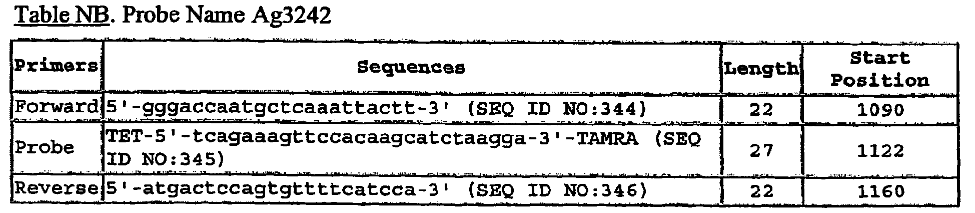

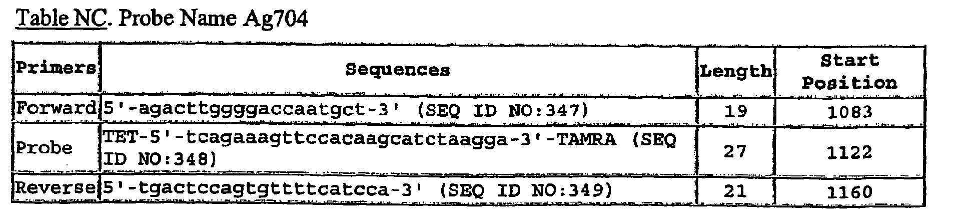

**Table ND. CNS_neurodegeneration_v1.0**

| **Tissue Name** | **Rel. Exp.(%) Ag1096, Run 206231465** | **Rel. Exp.(%) Ag3242, Run 206533581** | **Tissue Name** | **Rel. Exp.(%) Ag1096, Run 206231465** | **Rel. Exp.(%) Ag3242, Run 206533581** |
|---|---|---|---|---|---|
| AD 1 Hippo | 27.0 | 16.5 | Control (Path) 3 Temporal Ctx | 0.9 | 1.4 |
| AD 2 Hippo | 11.5 | 16.8 | Control (Path) 4 Temporal Ctx | 11.8 | 4.9 |
| AD 3 Hippo | 8.8 | 5.3 | AD 1 Occipital Ctx | 6.2 | 2.9 |
| AD 4 Hippo | 2.0 | 1.9 | AD 2 Occipital Ctx (Missing) | 0.0 | 0.0 |
| AD 5 Hippo | 46.7 | 36.6 | AD 3 Occipital Ctx | 5.6 | 2.8 |
| AD 6 Hippo | **100.0** | **100.0** | AD 4 Occipital Ctx | 6.3 | 4.1 |
| Control 2 Hippo | 51.1 | 35.1 | AD 5 Occipital Ctx | 25.0 | 16.8 |
| Control 4 Hippo | 5.3 | 4.0 | AD 6 Occipital Ctx | 5.3 | 1.0 |
| Control (Path) 3 Hippo | 4.6 | 1.0 | Control 1 Occipital Ctx | 0.8 | 0.3 |
| AD 1 Temporal Ctx | 3.6 | 2.7 | Control 2 Occipital Ctx | 17.1 | 19.5 |
| AD 2 Temporal Ctx | 12.5 | 7.5 | Control 3 Occipital Ctx | 3.1 | 3.5 |
| AD 3 Temporal Ctx | 2.5 | 1.0 | Control 4 Occipital Ctx | 1.4 | 0.8 |
| AD 4 Temporal Ctx | 6.3 | 3.3 | Control (Path) 1 Occipital Ctx | 34.6 | 30.1 |
| AD 5 Inf Temporal Ctx | 74.7 | 59.5 | Control (Path) 2 Occipital Ctx | 2.2 | 0.6 |
| AD 5 Sup Temporal Ctx | 66.0 | 61.6 | Control (Path) 3 Occipital Ctx | 0.7 | 0.4 |
| AD 6 Inf Temporal Ctx | 12.2 | 12.2 | Control (Path) 4 Occipital Ctx | 3.2 | 1.4 |
| AD 6 Sup Temporal Ctx | 14.7 | 12.3 | Control 1 Parietal Ctx | 1.3 | 1.4 |
| Control 1 Temporal Ctx | 1.9 | 2.2 | Control 2 Parietal Ctx | 21.8 | 19.6 |
| Control 2 Temporal Ctx | 21.8 | 22.2 | Control 3 Parietal Ctx | 5.9 | 5.7 |
| Control 3 Temporal Ctx | 5.9 | 4.6 | Control (Path) 1 Parietal Ctx | 38.7 | 38.7 |
| Control 3 Temporal Ctx | 1.7 | 0.9 | Control (Path) 2 Parietal Ctx | 9.5 | 4.3 |
| Control (Path) 1 Temporal Ctx | 33.9 | 28.7 | Control (Path) 3 Parietal Ctx | 1.4 | 0.5 |
| Control (Path) 2 Temporal Ctx | 17.3 | 12.3 | Control (Path) 4 Parietal Ctx | 16.8 | 15.5 |

**Table NE. Panel 1.2**

| **Tissue Name** | **Rel.Exp.(%) Ag1096, Run 125094875** | **Rel.Exp.(%)Ag1096, Run 134204525** | **Tissue Name** | **Rel. Eap.(%) Ag1096, Run 125094875** | **Rel.Exp.(%) Ag1096, Run 134204525** |
|---|---|---|---|---|---|
| Endothelial cells | 0.0 | 0.0 | Renal ca. 786-0 | 0.0 | 0.0 |
| Heart (Fetal) | 4.5 | 5.6 | Renal ca. A498 | 0.0 | 0.0 |
| Pancreas | 0.2 | 0.3 | Renal ca. RXF 393 | 0.1 | 0.1 |
| Pancreatic ca. CAPAN 2 | 0.0 | 0.0 | Renal ca. ACHN | 31.4 | 80.1 |
| Adrenal Gland | 1.6 | 2.8 | Renal ca. UO-31 | 0.0 | 0.0 |
| Thyroid | 0.1 | 0.4 | Renal ca. TK-10 | 1.0 | 1.3 |
| Salivary gland | 3.0 | 4.8 | Liver | 1.9 | 5.3 |
| Pituitary gland | 2.7 | 6.7 | Liver (fetal) | 0.8 | 1.7 |
| Brain (fetal) | 2.1 | 7.9 | Liver ca. (hepatobltst) HepG2 | 0.0 | 0.0 |
| Brain (whole) | 9.9 | 39.8 | Lung | 1.7 | 5.9 |
| Brain (amygdala) | 15.0 | 34.2 | Lung (fetal) | 1.8 | 9.9 |
| Brain (cerebellum) | 0.8 | 1.9 | Lung ca. (small cell) LX-1 | 0.4 | 1.2 |
| Brain (hippocampus) | 39.0 | 73.2 | Lung ca. (small cell) NCI-H69 | 0.2 | 0.5 |
| Brain (thalamus) | 2.5 | 9.2 | Lung ca. (s.cell var.) SHP-77 | 2.3 | 2.6 |
| Cerebral Cortex | 27.0 | 56.3 | Lung ca. (large cell)NCI-H460 | 50.7 | **100.0** |
| Spinal cord | 2.2 | 4.8 | Lung ca. (non-sm. cell) A549 | 0.1 | 0.0 |
| glio/astro U87-MG | 0.1 | 0.2 | Lung ca. (non-s.cell) NCI-H23 | 5.4 | 4.0 |
| glio/astro U-118-MG | 0.1 | 0.2 | Lung ca. (non-s.cell) HOP-62 | 15.3 | 33.2 |
| astrocytoma SW1783 | 0.0 | 0.0 | Lung ca. (non-s.cl) NCI-H522 | 0.0 | 0.0 |
| neuro*; met SK-NAS | 6.9 | 7.3 | Lung ca. (squam.) SW 900 | 5.4 | 12.5 |
| astrocytoma SF-539 | 0.0 | 0.0 | Lung ca. (squam.) NCI-H596 | 0.0 | 0.1 |
| astrocytoma SNB-75 | 7.0 | 6.8 | Mammary gland | 0.6 | 2.7 |
| glioma SNB-19 | 2.4 | 2.2 | Breast ca.* (pl.ef) MCF-7 | 0.0 | 0.0 |
| glioma U251 | 5.5 | 11.6 | Breast ca.* (pl.ef) MDA-MB-231 | 0.0 | 0.0 |
| glioma SF-295 | 0.0 | 0.0 | reast ca.* (pl. ef) T47D | 0.9 | 1.1 |
| Heart | 13.2 | 15.5 | Breast ca. BT-549 | 0.7 | 0.7 |
| Skeletal Muscle | 1.1 | 1.2 | Breast ca. MDA-N | 0.0 | 0.0 |
| Bone marrow | 0.0 | 0.1 | Ovary | 0.6 | 0.4 |
| Thymus | 0.5 | 1.7 | Ovarian ca. OVCAR-3 | 0.5 | 0.3 |
| Spleen | 0.4 | 0.8 | Ovarian ca. OVCAR-4 | 0.0 | 0.1 |
| Lymph node | 0.2 | 0.2 | Ovarian ca. OVCAR-5 | 15.4 | 15.2 |
| Colorectal Tissue | 4.3 | 8.1 | Ovarian ca. OVCAR-8 | 0.4 | 1.2 |
| Stomach | 3.9 | 8.8 | Ovarian ca. IGROV-1 | 0.1 | 0.0 |
| Small intestine | 1.9 | 3.7 | Ovarian ca. (ascites) SK-OV-3 | 1.9 | 1.3 |
| Colon ca. SW480 | 0.0 | 0.1 | Uterus | 2.2 | 10.6 |
| Colon ca.* SW620 (SW480 met) | 0.1 | 0.2 | Placenta | 8.2 | 10.1 |
| Colon ca. HT29 | 0.0 | 0.1 | Prostate | 3.4 | 3.5 |
| Colon ca. HCT-116 | 0.0 | 0.0 | Prostate ca.* (bone met) PC-3 | 2.6 | 3.5 |
| Colon ca. CaCo-2 | 0.0 | 0.0 | Testis | 1.3 | 2.1 |
| Colon ca. Tissue (ODO3866) | 0.3 | 1.1 | Melanoma Hs688(A).T | 0.0 | 0.0 |
| Colon ca. HCC- 2998 | 2.9 | 4.3 | Melanoma* (met) Hs688(B).T | 0.0 | 0.1 |
| Gastric ca.* (liver met) NCI-N87 | 0.0 | 0.0 | Melanoma UACC-62 | 0.0 | 0.0 |
| Bladder | 0.4 | 0.7 | Melanoma M14 | 0.5 | 0.1 |
| Trachea | 3.6 | 16.5 | Melanoma LOX IMVI | 0.1 | 0.0 |
| Kidney | 13.2 | 2.7 | Melanoma* (met) SK-MEL-5 | **100.0** | 86.5 |
| Kidney (fetal) | 7.2 | 10.3 | | | |

**Table NF. Panel 1.3D**

| **Tissue Name** | **Rel. Exp.(%) Ag3242, Run 165524415** | **Tissue Name** | **Rel. Exp.(%) Ag3242, Run 165524415** |
|---|---|---|---|
| Liver adenocaremoma | 0.0 | Kidney (fetal) | 2.7 |
| Pancreas | 1.0 | Renal ca. 786-0 | 0.0 |
| Pancreatic ca. CAPAN 2 | 0.0 | Renal ca. A498 | 0.0 |
| Adrenal gland | 0.3 | Renal ca. RXF 393 | 0.4 |
| Thyroid | 0.0 | Renal ca. ACHN | 100.0 |
| Salivary gland | 0.6 | Renal ca. UO-31 | 0.0 |
| Pituitary gland | 4.8 | Renal ca. TK-10 | 1.1 |
| Brain (fetal) | 11.8 | Liver | 1.0 |
| Brain (whole) | 40.6 | Liver (fetal) | 0.0 |
| Brain (amygdala) | 31.6 | Liver ca. (hepatoblast) HepG2 | 0.0 |
| Brain (cerebellum) | 3.0 | Lung | 4.8 |
| Brain (hippocampus) | 76.3 | Lung (fetal) | 2.2 |
| Brain (substantia nigra) | 2.1 | Lung ca. (small cell) LX- 1 | 3.7 |
| Brain (thalamus) | 6.3 | Lung ca. (small cell) NCI-H69 | 0.0 |
| Cerebral Cortex | 27.7 | Lung ca. (s.cell var.) SHP-77 | 16.0 |
| Spinal cord | 7.7 | Lung ca. (large cell)NCI-H460 | 46.0 |
| glio/astro U87-MG | 0.6 | Lung ca. (non-sm. cell) A549 | 0.0 |
| glio/astro U-118-MG | 0.5 | Lung ca. (non-s.cell) NCI-H23 | 8.0 |
| astrocytoma SW1783 | 0.0 | Lung ca. (non-s.cell) HOP-62 | 13.6 |
| neuro*; met SK-N-AS | 7.5 | Lung ca. (non-s.cl) NCI-H522 | 0.0 |
| astrocytoma SF-539 | 0.0 | Lung ca. (squam.) SW 900 | 18.8 |
| astrocytoma SNB-75 | 8.4 | Lung ca. (squam.) NCI-H596 | 0.0 |
| glioma SNB-19 | 3.0 | Mammary gland | 1.4 |
| glioma U251 | 66.4 | Breast ca.* (pl.ef) MCF-7 | 0.0 |
| glioma SF-295 | 0.6 | Breast ca.* (pl.ef) MDA-MB-231 | 0.0 |
| Heart (fetal) | 3.7 | Breast ca.* (pl.ef) T47D | 1.4 |
| Heart | 1.7 | Breast ca. BT-549 | 1.6 |
| Skeletal muscle (fetal) | 6.9 | Breast ca. MDA-N | 0.0 |
| Skeletal muscle | 1.9 | Ovary | 1.5 |
| Bone marrow | 0.0 | Ovarian ca. OVCAR-3 | 1.4 |
| Thymus | 0.4 | Ovarian ca. OVCAR-4 | 0.0 |
| Spleen | 0.0 | Ovarian ca. OVCAR-5 | 24.8 |
| Lymph node | 0.0 | Ovarian ca. OVCAR-8 | 0.6 |
| Colorectal | 3.5 | Ovarian ca. IGROV-1 | 0.0 |
| Stomach | 2.5 | Ovarian ca.* (ascites) SK-OV-3 | 5.4 |
| Small intestine | 7.2 | Uterus | 15.5 |
| Colon ca. SW480 | 0.0 | Placenta | 1.1 |
| Colon ca.* SW620(SW480 met) | 0.0 | Prostate | 1.4 |
| Colon ca. HT29 | 0.0 | Prostate ca.* (bone met)PC-3 | 0.4 |
| Colon ca. HCT-116 | 0.0 | Testis | 2.3 |
| Colon ca. CaCo-2 | 0.0 | Melanoma Hs688(A).T | 0.0 |
| Colon ca. tissue(ODO3866) | 0.0 | Melanoma* (met) Hs688(B).T | 0.0 |
| Colon ca. HCC-2998 | 2.1 | Melanoma UACC-62 | 0.0 |
| Gastric ca. * (liver met) NCI-N87 | 0.0 | Melanoma M14 | 1.6 |
| Bladder | 0.0 | Melanoma LOX IMVI | 0.0 |
| Trachea | 9.0 | Melanoma* (met) SK-MEL-5 | 58.6 |
| Kidney | 1.5 | Adipose | 1.5 |

**Table NG. Panel 2.2**

| **Tissue Name** | **Rel. Exp.(%) Ag3242, Run 174443348** | **Tissue Name** | **Rel. Exp.(%) Ag3242, Run 174443348** |
|---|---|---|---|
| Normal Colon | 4.4 | Kidney Margin (OD04348) | 1.0 |
| Colon cancer (OD06064) | 1.8 | Kidney malignant cancer (OD06204B) | 0.4 |
| Colon Margin (OD06064) | 8.9 | Kidney normal adjacent tissue (OD06204E) | 1.8 |
| Colon cancer (OD06159) | 2.0 | Kidney Cancer (OD04450-01) | 2.3 |
| Colon Margin (OD06159) | 7.1 | Kidney Margin (OD04450-03) | 0.9 |
| Colon cancer (OD06297-04) | 2.4 | Kidney Cancer 8120613 | 0.0 |
| Colon Margin (OD06297-05) | 2.2 | Kidney Margin 8120614 | 0.4 |
| CC Gr.2 ascend colon (ODO3921) | 0.9 | Kidney Cancer 9010320 | 1.4 |
| CC Margin (ODO3921) | 3.8 | Kidney Margin 9010321 | 1.3 |
| Colon cancer metastasis (OD06104) | 0.7 | Kidney Cancer 8120607 | 0.8 |
| Lung Margin (OD06104) | 0.0 | Kidney Margin 8120608 | 0.2 |
| Colon mets to lung (OD04451-01) | 1.7 | Normal Uterus | 5.3 |
| Lung Margin (OD04451-02) | 10.1 | Uterine Cancer 064011 | 0.5 |
| Normal Prostate | 0.4 | Normal Thyroid | 0.0 |
| Prostate Cancer (OD04410) | 0.3 | Thyroid Cancer 064010 | 0.7 |
| Prostate Margin (OD04410) | 0.0 | Thyroid Cancer A302152 | 0.9 |
| Normal Ovary | 0.0 | Thyroid Margin A302153 | 0.0 |
| Ovarian Cancer (OD06283-03) | 0.0 | Normal Breast | 0.6 |
| Ovarian Margin (OD06283-07) | 0.0 | Breast Cancer (OD04566) | 0.0 |
| Ovarian Cancer 064008 | 1.9 | Breast Cancer 1024 | 2.1 |
| Ovarian cancer (OD06145) | 0.0 | Breast Cancer (OD04590-01) | 0.0 |
| Ovarian Margin (OD06145) (OD04590-03) | 0.0 | Breast Cancer Mets | 0.0 |
| Ovarian cancer (OD06455-03) | 0.0 | Breast Cancer Metastasis (OD04655-05) | 0.4 |
| Ovarian Margin (OD06455-07) | 1.2 | Breast Cancer 064006 | 0.7 |
| Normal Lung | 2.3 | Breast Cancer 9100266 | 0.7 |
| Invasive poor diff. lung adeno (ODO4945-01 | 1.1 | Breast Margin 9100265 | 0.2 |
| Lung Margin (ODO4945-03) | 1.6 | Breast Cancer A209073 | 0.0 |
| Lung Malignant Cancer (OD03126) | 0.7 | Breast Margin A2090734 | 0.0 |
| Lung Margin (OD03126) | 1.2 | Breast cancer (OD06083) | 0.4 |
| Lung Cancer (OD05014A) | 1.2 | Breast cancer node metastasis (OD06083) | 0.9 |
| Lung Margin (OD05014B) | 4.4 | Normal Liver | 1.3 |
| Lung cancer (OD06081) | 0.8 | Liver Cancer 1026 | 0.3 |
| Lung Margin (OD06081) | 2.8 | Liver Cancer 1025 | 0.0 |
| Lung Cancer (OD04237-01) | 0.0 | Liver Cancer 6004-T | 0.0 |
| Lung Margin (OD04237-02) | 6.0 | Liver Tissue 6004-N | 0.0 |
| Ocular Melanoma Metastasis | 100.0 | Liver Cancer 6005-T | 2.7 |
| Ocular Melanoma Margin (Liver) | 0.7 | Liver Tissue 6005-N | 3.3 |
| Melanoma Metastasis | 1.2 | Liver Cancer 064003 | 7.1 |
| Melanoma Margin (Lung) | 4.9 | Normal Bladder | 0.0 |
| Normal Kidney | 0.0 | Bladder Cancer 1023 | 0.0 |
| Kidney Ca, Nuclear grade 2 (OD04338) | 1.3 | Bladder Cancer A302173 | 0.3 |
| Kidney Margin (OD04338) | 0.4 | Normal Stomach | 5.8 |
| Kidney Ca Nuclear grade 1/2 (OD04339) | 0.6 | Gastric Cancer 9060397 | 0.0 |
| Kidney Margin (OD04339) | 1.6 | Stomach Margin 9060396 | 2.6 |
| Kidney Ca, Clear cell type (OD04340) | 0.0 | Gastric Cancer 9060395 | 2.2 |
| Kidney Margin (OD04340) | 1.1 | Stomach Margin 9060394 | 3.4 |
| Kidney Ca, Nuclear grade 3 (OD04348) | 0.0 | Gastric Cancer 064005 | 0.3 |

**Table NH. Panel 4D**

| **Tissue Name** | **Rel. Exp.(%) Ag1096, Run 160353275** | **Rel. Exp.(%) Ag3242, Run 164390548** | **Tissue Name** | **Rel. Exp.(%) Ag1096, Run 160353275** | **Rel. Exp.(%) Ag3242, Run 164390548** |
|---|---|---|---|---|---|
| Secondary Th1 act | 0.0 | 0.0 | HUVEC IL-1beta | 0.0 | 0.0 |
| Secondary Th2 act | 0.0 | 0.0 | HUVBC IFN gamma | 0.4 | 0.0 |
| Secondary Tr1 act | 0.0 | 0.0 | HUVEC TNF alpha IFN gamma | 0.0 | 0.0 |
| Secondary Th1 rest | 0.0 | 0.0 | HUVEC TNF alpha + IL4 | 2.0 | 0.0 |
| Secondary Th2 rest | 0.0 | 0.0 | HUVEC IL-11 | 0.0 | 0.0 |
| Secondary Tr1 rest | 0.0 | 0.0 | Lung Microvascular EC none | 0.0 | 0.0 |
| Primary Th1 act | 0.0 | 0.0 | Lung Microvascular EC TNFalpha + IL- 1beta | 0.0 | 0.0 |
| Primary Th2 act | 0.0 | 0.0 | Microvascular Dermal EC none | 0.0 | 0.0 |
| Pnmary Tr1 act | 0.0 | 0.0 | Microsvasular Dermal EC TNFalpha + IL- 1beta | 0.0 | 0.0 |
| Primary Th1 rest | 0.0 | 0.0 | Bronchial epithelium TNFalpha + IL1beta | 0.0 | 0.0 |
| Primary Th2 rest | 0.0 | 0.0 | Small airway epithelium none | 0.0 | 0.0 |
| Primary Tr1 rest | 0.0 | 0.0 | Small airway epithelium TNFalpha + IL-1beta | 6.5 | 11.7 |
| CD45RA CD4 lymphocyte act | 0.0 | 0.0 | Coronery artery SMC rest | 0.7 | 0.0 |
| CD45RO CD4 lymphocyte act | 0.0 | 0.0 | Coronery artery SMC TNFalpha + IL-1beta | 0.0 | 0.0 |
| CD8 lymphocyte act, | 1.3 | 0.0 | Astrocytes rest | 13.9 | 16.8 |
| Secondary CD8 lymphocyterest | 0.7 | 0.0 | Astrocytes TNFalpha +IL-1beta | 3.6 | 5.3 |
| Secondary CD8 lymphocyte act | 0.0 | 0.0 | KU-812 (Basophil) rest | 0.9 | 1.0 |
| CD4 lymphocyte none | 0.0 | 0.0 | KU-812 (Basophil) PMA/ionomycin | **100.0** | **100.0** |
| 2ry Th1/Th2/Tr1_anti-CD95 CH11 | 0.0 | 0.0 | CCD1106 (Keratinocytes) none | 0.0 | 0.0 |
| LAK cells rest | 0.0 | 0.0 | CCD1106 (Keratinocytes) TNFalpha + IL-1beta | 0.0 | 0.0 |
| LAK cells IL-2 | 0.0 | 0.0 | Liver cirrhosis | 1.9 | 3.8 |
| LAK cells IL-2+IL-12 | 0.0 | 0.0 | Lupus kidney | 1.3 | 1.4 |
| LAK cells IL-2+IFN gamma | 0.8 | 0.0 | NCI-H292 none | 42.9 | 48.0 |
| LAK cells IL-2+IL-18 | 0.0 | 0.0 | NCI-H292 IL-4 | 14.7 | 14.8 |
| LAK cells PMA/ionomycin | 0.0 | 0.0 | NCI-H292 IL-9 | 40.6 | 36.6 |
| NK Cells IL-2 rest | 0.8 | 0.0 | NCI-H292 IL-13 | 12.2 | 7.4 |
| Two Way MLR 3 day | 0.0 | 0.0 | NCI-H292 IFN gamma | 16.8 | 10.9 |
| Two Way MLR 5 day | 0.0 | 0.0 | HPAEC none | 0.0 | 0.0 |
| Two Way MLR 7 day | 1.5 | 0.0 | HPAEC TNF alpha + IL-1 beta | 0.0 | 0.0 |
| PBMC rest | 0.0 | 0.0 | Lung fibroblast none | 0.0 | 1.7 |
| PBMC PWM | 0.0 | 0.0 | Lung fibroblast TNF alpha + IL-1 beta | 0.0 | 0.0 |
| PBMC PHA-L | 0.0 | 0.0 | Lung fibroblast IL-4 | 1.7 | 0.0 |
| Ramos (B cell) none | 0.0 | 0.0 | Lung fibroblast IL-9 | 0.0 | 0.0 |
| Ramos (B cell) ionomycin | 0.0 | 0.0 | Lung fibroblast IL-13 | 0.7 | 0.0 |
| B lymphocytes PWM | 0.0 | 0.0 | Lung fibroblast IFN gamma | 0.0 | 0.0 |
| B lymphocytes CD40L and IL-4 | 0.0 | 0.0 | Dermal fibroblast CCD1070 rest | 0.0 | 0.0 |
| EOL-1 dbcAMP | 0.0 | 0.0 | Dermal fibroblast CCD1070 TNF alpha | 0.0 | 0.0 |
| EOL-1 dbcAMP PMA/iomomycin | 0.0 | 0.0 | Dermal fibroblast CCD1070 IL-1 beta | 0.9 | 0.0 |
| Dendritic cells none | 0.0 | 0.0 | Dermal fibroblast IFN gamma | 0.0 | 0.0 |
| Dendritic cells LPS | 0.0 | 0.0 | Dermal fibroblast IL-4 | 0.0 | 0.0 |
| Dendritic cells anti-CD40 | 0.0 | 0.0 | IBD Colitis 2 | 0.8 | 2.6 |
| Monocytes rest | 0.0 | 0.0 | IBD Crohn's | 0.0 | 2.0 |
| Monocytes LPS | 1.7 | 0.0 | Colon | 28.1 | 23.2 |
| Macrophages rest | 0.0 | 0.0 | Lung | 34.9 | 51.4 |
| Macrophages LPS | 0.0 | 0.0 | Thymus | 35.1 | 28.3 |
| HUVEC none | 0.0 | 0.0 | Kidney | 8.2 | 7.4 |
| HUVEC starved | 0.0 | 0.0 | | | |

**CNS_neurodegeneration_v1.0 Summary:** Ag1096/Ag3242 Two experiments with two different probe and primer sets produce results that are in excellent agreement, with highest expression of the CG57448-01 gene in the hippocampus of a patient with Alzheimer's disease. No change is detected in the expression of this gene in the postmortem Alzheimer's diseased brain when compared to controls; however this panel confirms the expression of this gene in the CNS in an independent group of patients. See panel 1.2 for a discussion of utility. A third experiment with the probe and primer set Ag704 shows low/undetectable levels of expression (CTs>35). (Data not shown.) The data suggest that there is a probability of a probe failure.

**Panel 1.2 Summary:** Ag1096/Ag3242 Two experiments with two different probe and primer sets produce results that are in excellent agreement, with highest expression of the CG57448-01 gene in cancer cell lines derived from lung cancer and melanoma (CTs=24-25). Significant levels of expression are also seen in a renal cancer cell line, ovarian cancer cell lines and brain cancer cell lines. Thus, expression of this gene could be used to differentiate between these samples and other samples on this panel and as a diagnostic marker for the presence of these cancers. This gene encodes a protein that is homologous to cadherin, a cell-adhesion molecule. Therefore, therapeutic modulation of the expression or function of this gene may be effective in the treatment of lung, renal and melanoma cancers.

Expression of this gene is also high in many regions of the brain, including the amygdala, thalamus, cerebellum, and cerebral cortex, with highest expression in the hippocampus. Expression is also detected in the spinal cord. Cadherins can act as axon guidance and cell adhesion proteins, specifically during development and in the response to injury (ref 1). Therefore, manipulation of levels of this protein may be of use in inducing a compensatory synaptogenic response to neuronal death in Alzheimer's disease, Parkinson's disease, Huntington's disease, spinocerebellar ataxia, progressive supranuclear palsy, ALS, head trauma, stroke, or any other disease/condition associated with neuronal loss.

Among tissues with metabolic function, this gene is moderately expressed in pituitary gland, adrenal gland, thyroid, pancreas, skeletal muscle, and liver, reflecting the widespread role of cadherins in cell-cell adhesion. This expression suggests that this gene product may play a role in normal metabolic and neuroendocrine function and that dysregulated expression of this gene may contribute to metabolic diseases (such as obesity and diabetes) or neuroendocrine disorders (Ranscht, Int. J. Dev. Neurosci. 18: 643-651, 2000).

**Panel 1.3D Summary**: Ag3242 Highest expression of the CG57448-01 gene is seen in a renal cancer cell line (CT=31.1). Significant expression is also seen in cell lines derived from ovarian cancer, lung cancer, brain cancer and melanoma. This is in concordance with the results in the previous panel. Please see Panel 1.2 for discussion of utility of this gene in the treatment of cancer.

As in the previous panel, this gene is also highly expressed in the central nervous system. Please see Panel 1.2 for a fuller discussion of utility of this gene in the central nervous system.

Results from a second experiment with the probe primer set Ag704 are not included. The amp plot indicates that there is a high probability of a probe failure.

**Panel 2.2 Summary:** Ag3242 Highest expression of the CG57448-01 gene is seen in a sample derived from an ocular melanoma metastasis (CT=29). Thus, expression of this gene could be used to differentiate between this sample and other samples on this pane.

**Panel 2D Summary:** Ag704 Results from one experiment with the CG57448-01 gene are not included. The amp plot indicates that there were experimental difficulties with this run.

**Panel 4D Summary:** Ag1096/Ag3242 Two experiments with two different probe and primer sets produce results that are in excellent agreement, with highest expression of the CG57448-01 gene in the basophil cell line (KU-812) treated with PMA/ionomycin (CTs=30-32). Significant expression is also seen in a cluster of treated and untreated samples derived from the muco-epidermoid cell line NCI-H292. Thus, this gene, which encodes a cadherin homolog, is expressed in both a cell line that is often used as a model of airway epithelium (NCI-H292) and a cell line that is a reasonable model for the inflammatory cells that contribute to various inflammatory lung diseases. This suggests that suggest that therapeutic modulation of this gene product may reduce or eliminate the symptoms of patients suffering from pathological and inflammatory lung disorders, including chronic obstructive pulmonary disease, asthma, allergy and emphysema.

Low but significant levels of expression are also seen in the samples derived from normal colon, kidney, lung and thymus. This suggests that this gene product may play a role in the homeostasis of these tissues. Therefore, therapeutic modulation of the expression or function of this gene product may be important for maintaining or restoring normal function to these organs during inflammation.Results from a third experiment with the probe primer set Ag704 are not included. The amp plot indicates that there is a high probability of a probe failure.

### O. NOV16b: Protocadherin

Expression of gene CG57446-01 was assessed using the primer-probe set Ag3241, described in Table OA. Results of the RTQ-PCR runs are shown in Table OB.

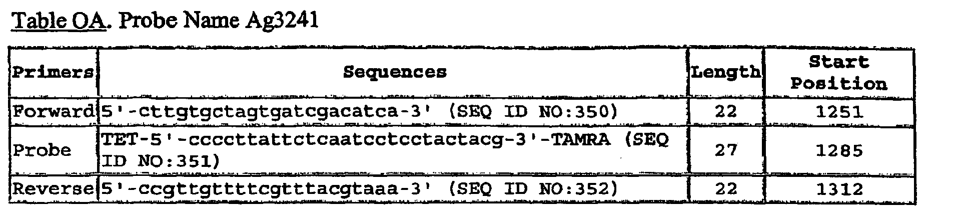

**Table OB. Panel 1.3D**

| **Tissue Name** | **Rel. Exp.(%) Ag3241, Run 165524414** | **Tissue Name** | **Rel. Exp.(%) Ag3241, Run 165524414** |
|---|---|---|---|
| Liver adenocarcinoma | 0.0 | Kidney (fetal) | 0.0 |
| Pancreas | 0.0 | Renal ca. 786-0 | 0.0 |
| Pancreatic ca. CAPAN 2 | 0.0 | Renal ca. A498 | 0.0 |
| Adrenal gland | 0.0 | Renal ca. RXF 393 | 0.0 |
| Thyroid | 0.0 | Renal ca. ACHN | 0.0 |
| Salivary gland | 0.0 | Renal ca. UO-31 | 0.0 |
| Pituitary gland | 0.0 | Renal ca. TK-10 | 0.0 |
| Brain (fetal) | 0.0 | Liver | 0.0 |
| Brain (whole) | 0.0 | Liver (fetal) | 0.0 |
| Brain (amygdala) | 0.0 | Liver ca. (hepatoblast) HepG2 | 0.0 |
| Brain (cerebellum) | 0.0 | Long | 0.0 |
| Brain (hippocampus) | 0.0 | Lung (fetal) | 0.0 |
| Brain (substantia nigra) | 0.0 | Lung ca. (small cell) LX-1 | 0.0 |
| Brain (thalamus) | 0.0 | Lung ca. (small cell) NCI-H69 | 0.0 |
| Cerebral Cortex | 0.0 | Lung ca. (s.cell var.) SHP-77 | 0.0 |
| Spinal cord | 0.0 | Lung ca. (large cell)NCI-H460 | 0.0 |
| glio/astro U87-MG | 0.0 | Lung ca. (non-sm. cell) A549 | 0.0 |
| glio/astro U-118-MG | 0.0 | Lung ca. (non-s.cell) NCI-H23 | 0.0 |
| astrocytoma SW1783 | 0.0 | Lung ca. (non-s.cell) HOP-62 | 0.0 |
| neuro*; met SK-N-AS | 0.0 | Lung ca. (non-s.cl) NCI-H522 | 0.0 |
| astrocytoma SF-539 | 0.0 | Lung ca. (squam.) SW 900 | 0.0 |
| astrocytoma SNB-75 | 0.0 | Lung ca. (squam.) NCI-H596 | 0.0 |
| glionia SNB-19 | 0.0 | Mammary gland | 0.0 |
| glioma U251 | 0.0 | Breast ca.* (pl.ef) MCF-7 | 0.0 |
| glioma SF-295 | 0.0 | Breast ca.* (pl.ef) MDA-MB-231 | 0.0 |
| Heart (fetal) | 0.0 | Breast ca.* (pl.ef) T47D | 0.0 |
| Heart | 0.0 | Breast ca. BT-549 | 0.0 |
| Skeletal muscle (fetal) | **100.0** | Breast ca. MDA-N | 0.0 |
| Skeletal muscle | 0.0 | Ovary | 0.0 |
| Bone marrow | 0.0 | Ovarian ca. OVCAR-3 | 0.0 |
| Thymus | 0.0 | Ovarian ca. OVCAR-4 | 0.0 |
| Spleen | 0.0 | Ovarian ca. OVCAR-5 | 0.0 |
| Lymph node | 0.0 | Ovarian ca. OVCAR-8 | 0.0 |
| Colorectal | 0.0 | Ovarian ca. IGROV-1 | 0.0 |
| Stomach | 0.0 | Ovarian ca.* (ascites) SK-OV-3 | 0.0 |
| Small intestine | 0.0 | Uterus | 0.0 |
| Colon ca. SW480 | 0.0 | Placenta | 0.0 |
| Colon ca.* SW620(SW480 met) | 0.0 | Prostate | 0.0 |
| Colon ca. HT29 | 0.0 | Prostate ca.* (bone met)PC-3 | 0.0 |
| Colon ca. HCT-116 | 0.0 | Testis | 0.0 |
| Colon ca. CaCo-2 | 0.0 | Melanoma Hs688(A).T | 0.0 |
| Colon ca. tissue(ODO3866) | 0.0 | Melanoma* (met) Hs688(B).T | 0.0 0.0 |
| Colon ca. HCC-2998 | 0.0 | Melanoma UACC-62 | 0.0 |
| Gastric ca.* (liver met) NCI-N87 | 0.0 | Melanoma M14 | 0.0 |
| Bladder | 0.0 | Melanoma LOXIMVI | 0.0 |
| Trachea | 0.0 | Melanoma* (met) SK-MEL-5 | 0.0 |
| Kidney | 0.0 | Adipose | 0.0 |

**Panel 1.3D Summary:** Ag3241 Expression of the CG57446-01 gene is restricted to fetal skeletal muscle (CT=33.8). Thus, expression of this gene could be used to differentiate between fetal and adult skeletal muscle (CT=40). This gene encodes a protein that is homologous to cadherin, a cell adhesion molecule that has been implicated in muscle differentiation. Thus, the relative overexpression of this gene in fetal skeletal muscle suggests that the protein product may enhance muscular growth or development in the fetus and may also act in a regenerative capacity in the adult. Therefore, therapeutic modulation of the protein encoded by this gene could be useful in treatment of muscle related diseases. More specifically, treatment of weak or dystrophic muscle with the protein encoded by this gene could restore muscle mass or function (Goichberg et al., J Cell Sci 2001 Apr;114(Pt 7):1309-19).

**Panel 2.2 Summary:** Ag3241 Expression of the CG57446-01 gene is low/undetectable in all samples on this panel (CTs>35). (Data not shown.) The amp plot indicates that there is a possibility of a probe failure.

**Panel 4D Summary:** Ag3241 Expression of the CG57446-01 gene is low/undetectable in all samples on this panel (CTs>35). (Data not shown.) The amp plot indicates that there is a possibility of a probe failure.

**Panel CNS_1 Summary:** Ag3241 Expression of the CG57446-01 gene is low/undetectable in all samples on this panel (CTs>35). (Data not shown.) The amp plot indicates that there is a possibility of a probe failure.

### P. NOV16c: Protocadherin

Expression of gene CG57444-01 was assessed using the primer-probe set Ag3240, described in Table PA. Results of the RTQ-PCR runs are shown in Tables PB, PC, PD, PE and PF.

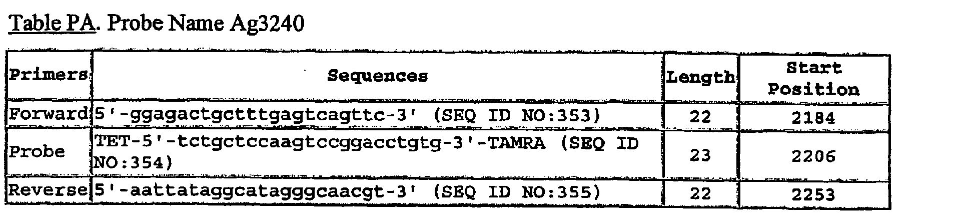

**Table PB. CNS_neurodegeneration_v1.0**

| **Tissue Name** | **Rel. Exp.(%) Ag3240, Run 210037849** | **Tissue Name** | **Rel. Exp.(%) Ag3240, Run 210037849** |
|---|---|---|---|
| AD 1 Hippo | 17.7 | Control (Path) 3 Temporal Ctx | 6.9 |
| AD 2 Hippo | 37.9 | Control (Path) 4 Temporal Ctx | 28.1 |
| AD 3 Hippo | 6.8 | AD 1 Occipital Ctx | 13.7 |
| AD 4 Hippo | 8.3 | AD 2 Occipital Ctx (Missing) | 0.0 |
| AD 5 Hippo | 55.1 | AD 3 Occipital Ctx | 8.7 |
| AD 6 Hippo | **100.0** | AD 4 Occipital Ctx | 21.8 |
| Control 2 Hippo | 23.5 | AD 5 Occipital Ctx | 8.4 |
| Control 4 Hippo | 23.5 | AD 6 Occipital Ctx | 17.6 |
| Control (Path) 3 Hippo | 8.4 | Control 1 Occipital Ctx | 4.3 |
| AD 1 Temporal Ctx | 18.3 | Control 2 Occipital Ctx | 24.0 |
| AD 2 Temporal Ctx | 32.5 | Control 3 Occipital Ctx | 14.2 |
| AD 3 Temporal Ctx | 6.3 | Control 4 Occipital Ctx | 13.0 |
| AD 4 Temporal Ctx | 24.7 | Control (Path) 1 Occipital Ctx | 51.4 |
| AD 5 Inf Temporal Ctx | 59.0 | Control (Path) 2 Occipital Ctx Occipital Ctx | 8.7 |
| AD 5 Sup Temporal Ctx | 53.6 | Control (Path) 3 Occipital Ctx | 4.9 |
| AD 6 Inf Temporal Ctx | 43.8 | Control (Path) 4 Occipital Ctx | 15.4 |
| AD 6 Sup Temporal Ctx | 56.6 | Control 1 Parietal Ctx | 11.6 |
| Control 1 Temporal Ctx | 9.6 | Control 2 Parietal Ctx | 37.1 |
| Control 2 Temporal Ctx | 14.6 | Control 3 Parietal Ctx | 15.5 |
| Control 3 Temporal Ctx | 8.9 | Control (Path) 1 Parietal Ctx | 36.3 |
| Control 3 Temporal Ctx | 9.0 | Control (Path) 2 Parietal Ctx | 22.7 |
| Control (Path) 1 Temporal Ctx | 34.9 | Control (Path) 3 Parietal Ctx | 5.8 |
| Control (Path) 2 Temporal Ctx | 19.3 | Control (path) 4 Parietal Ctx | 37.1 |

**Table PC. Panel 1.3D**

| **Tissue Name** | **Rel. Exp.(%) Ag3240, Run 165524271** | **Tissue Name** | **Rel. Exp.(%) Ag3240, Run 165524271** |
|---|---|---|---|
| Liver adenocarcinoma | 2.5 | Kidney (fetal) | 6.0 |
| Pancreas | 1.7 | Renal ca. 786-0 | 3.2 |
| Pancreatic ca. CAPAN 2 | 3.5 | Renal ca. A498 | 3.7 |
| Adrenal gland | 6.6 | Renal ca. RXF 393 | 6.7 |
| Thyroid | 3.9 | Renal ca. ACHN | 2.0 |
| Salivary gland | 3.7 | Renal ca. UO-31 | 0.4 |
| Pituitary gland | 14.0 | Renal ca. TK-10 | 0.2 |
| Brain (fetal) | 23.3 | Liver | 1.9 |
| Brain (whole) | 19.3 | Liver (fetal) | 2.8 |
| Brain (amygdala) | 21.9 | Liver ca. (hepatoblast) HepG2 | 0.4 |
| Brain (cerebelhxm) | 48.6 | Lung | 5.9 |
| Brain (hippocampus) | 23.3 | Lung (fetal) | 2.3 |
| Brain (substantia nigra) | 9.5 | Lung ca. (small cell) LX-1 | 1.0 |
| Brain (thalamus) | 17.8 | Lung ca. (small cell) NCI-H69 | 0.4 |
| Cerebral Cortex | 10.2 | Lung ca. (s.cell var.) SHP-77 | 0.6 |
| Spinal cord | 29.7 | Lung ca. (large cell)NCI-H460 | 3.5 |
| glio/astro U87-MG | 1.0 | Lung ca. (non-sm. cell) A549 | 0.1 |
| glio/astro U-118-MG | **100.0** | Lung ca. (non-s.cell) NCI-H23 | 11.5 |
| astrocytoma SW1783 | 4.2 | Lung ca. (non-s.cell) HOP-62 | 5.2 |
| neuro*; met SK-N-AS | 34.6 | Lung ca. (non-s.cl) NCI-H522 | 2.4 |
| astrocytoma SF-539 | 3.9 | Lung ca. (squam.) SW 900 | 5.8 |
| astrocytoma SNB-75 | 6.6 | Lung ca. (squam.) NCI-H596 | 0.9 |
| glioma SNB-19 | 8.1 | Mammary gland | 14.7 |
| glioma U251 | 9.4 | Breast ca.* (pl.ef) MCF-7 | 4.8 |
| glioma SF-295 | 3.2 | Breast ca.* (pl.ef) MDA-MB-231 | 4.9 |
| Heart (fetal) | 3.6 | Breast ca.* (pl.ef) T47D | 3.9 |
| Heart | 8.7 | Breast ca. BT-549 | 5.8 |
| Skeletal muscle (fetal) | 11.0 | Breast ca. MDA-N | 2.5 |
| Skeletal muscle | 8.1 | Ovary | 31.2 |
| Bone marrow | 1.4 | Ovarian ca. OVCAR-3 | 1.5 |
| Thymus | 1.5 | Ovarian ca. OVCAR-4 | 05 |
| Spleen | 5.8 | Ovarian ca. OVCAR-5 | 11.2 |
| Lymph node | 7.5 | Ovarian ca. OVCAR-8 | 0.5 |
| Colorectal | 6.2 | Ovarian ca. IGROV-1 | 5.7 |
| Stomach | 3.3 | Ovarian ca.* (ascites) SK-OV-3 | 6.9 |
| Small intestine | 12.4 | Uterus | 15.8 |
| Colon ca. SW480 | 0.1 | Placenta | 4.3 |
| Colon ca.* SW620(SW480 met) | 0.2 | Prostate | 5.3 |
| Colon ca. HT29 | 0.2 | Prostate ca.* (boae met)PC-3 | 4.1 |
| Colon ca. HCT-116 | 1.0 | Testis | 4.2 |
| Colon ca.CaCo-2 | 0.8 | Melanoma Hs688(A).T | 6.3 |
| Colon ca. tissue(ODO3866) | 3.2 | Melanoma* (met) Hs688(B).T | 8.0 |
| Colon ca. HCC-2998 | 5.1 | Melanoma UACC-62 | 2.8 |
| Gastric ca.* (liver met) NCI-N87 | 8.9 | Melanoma M14 | 5.1 |
| Bladder | 13.3 | Melanoma LOX IMVI | 0.4 |
| Trachea | 5.5 | Melanoma* (met) SK-MEL-5 | 3.0 |
| Kidney | 6.7 | Adipose | 3.8 |

**Table PD. Panel 2.2**

| **Tissue Name** | **Rel. Exp.(%) Ag3240, Run 174443262** | **Tissue Name** | **Rel. Exp.(%) Ag3240, Run 174443262** |
|---|---|---|---|
| Normal Colon | 13.7 | Kidney Margin (OD04348) | 40.9 |
| Colon cancer (OD06064) | 12.9 | Kidney malignant cancer (OD06204B) | 1.2 |
| Colon Margin (OD06064) | 20.3 | Kidney normal adjacent tissue (OD06204E) | 11.7 |
| Colon cancer (OD06159) | 0.4 | Kidney Cancer (OD04450- 01) | 9.0 |
| Colon Margin (OD06159) | 18.7 | Kidney Margin (OD04450-03) | 9.5 |
| Colon cancer (OD06297-04) | 5.6 | Kidney Cancer 8120613 | 1.4 |
| Colon Margin (OD06297-05) | 34.2 | Kidney Margin 8120614 | 14.1 |
| CC Gr.2 ascend colon (ODO3921) | 0.9 | Kidney Cancer 9010320 | 3.4 |
| CC Margin (ODO3921) | 4.6 | Kidney Margin 9010321 | 5.5 |
| Colon cancer metastasis | 0.8 | Kidney Cancer 8120607 | 7.0 |
| Lung Margin (OD06104) | 2.5 | Kidney Margin 8120608 | 9.5 |
| Colon mets to lung (OD04451-01) | 1.8 | Normal Uterus | 52.9 |
| Lung Margin (OD04451-02) | 10.9 | Uterine Cancer 064011 | 17.9 |
| Normal Prostate | 15.7 | Normal Thyroid | 2.7 |
| Prostate Cancer (OD04410) | 17.3 | Thyroid Cancer 064010 | 13.7 |
| Prostate Margin (OD04410) | 4.9 | Thyroid Cancer A302152 | 17.0 |
| Normal Ovary | 48.3 | Thyroid Margin A302153 | 8.4 |
| Ovarian cancer (OD06283-03) | 1.9 | Normal Breast | 42.6 |
| Ovarian Margin (OD06283-07) | 14.1 | Breast Cancer (OD04566) | 1.7 |
| Ovarian Cancer 064008 | 10.1 | Breast Cancer 1024 | 40.3 |
| Ovarian cancer (OD06145) | 41.2 | Breast Cancer (OD04590-01) | 8.4 |
| Ovarian Margin (OD06145) | 37.1 | Breast Cancer Mets (OD04590-03) | 9.4 |
| Ovarian cancer (OD06455-03) | 2.6 | Breast Cancer Metastasis (OD04655-05) | 8.9 |
| Ovarian Margin (OD06455-07) | 41.8 | Breast Cancer 064006 | 4.3 |
| Normal Lung | 23.8 | Breast Cancer 9100266 | 16.4 |
| Invasive poor diff. lung adeno (ODO4945-01 | 1.8 | Breast Margin 9100265 | 18.4 |
| Lung Margin (OD04945-03) | 21.3 | Breast Cancer A209073 | 4.1 |
| Lung Malignant Cancer (OD03126) | 10.4 | Breast Margin A2090734 | 16.4 |
| Lung Margin (OD03126) | 13.7 | Breast cancer (OD06083) | 34.2 |
| Lung Cancer (OD05014A) | 3.7 | Breast cancer node metastasis (OD06083) | 26.6 |
| Lung Margin (OD05014B) | 33.0 | Normal Liver | 9.7 |
| Lung cancer (OD06081) | 0.0 | Liver Cancer 1026 | 1.6 |
| Lung Margin (OD06081) | 15.3 | Liver Cancer 1025 | 9.2 |
| Lung Cancer (OD04237-01) | 1.1 | Liver Cancer 6004-T | 7.5 |
| Lung Margin (OD04237-02) | 31.6 | Liver Tissue 6004-N | 1.6 |
| Ocular Melanoma Metastasis | 0.6 | Liver Cancer 6005-T | 5.8 |
| Ocular Melanoma Margin (Liver) | 4.5 | Liver Tissue 6005-N | 17.1 |
| Melanoma Metastasis | 0.5 | Liver Cancer 064003 | 7.4 |
| Melanoma Margin (Lung) | 33.9 | Normal Bladder | 8.0 |
| Normal Kidney | 8.0 | Bladder Cancer 1023 | 4.6 |
| Kidney Ca, Nuclear grade 2 (OD04338) | 19.3 | Bladder Cancer A302173 | 3.3 |
| Kidney Margin (OD04338) | 54.7 | Normal Stomach | 32.5 |
| Kidney Ca Nuclear grade 1/2 (OD04339) | **100.0** | Gastric Cancer 9060397 | 0.0 |
| Kidney Margin (OD04339) | 18.3 | Stomach Margin 9060396 | 6.1 |
| Kidney Ca, Clear cell type (OD04340) | 6.7 | Gastric Cancer 9060395 | 6.9 |
| Kidney Margin (OD04340) | 9.5 | Stomach Margin 9060394 | 22.2 |
| Kidney Ca, Nuclear grade 3 (OD04348) | 8.7 | Gastric Cancer 064005 | 6.1 |

**Table PE. Panel 4D**

| **Tissue Name** | **Rel. Exp.(%) Ag3240, Run 164389761** | **Tissue Name** | **Rel. Exp.(%) Ag3240, Run 164389761** |
|---|---|---|---|
| Secondary Th1 act | 0.0 | HUVEC IL-1beta | 16.7 |
| Secondary Th2 act | 0.0 | HUVEC IFN gamma | 33.2 |
| Secondary Tr1 act | 0.0 | HUVEC TNF alpha + IFN gamma | 13.4 |
| Secondary Th1 rest | 0.0 | HUVEC TNF alpha + IL4 | 17.1 |
| Secondary Th2 rest | 0.0 | HUVEC IL-11 | 13.5 |
| Secondary Tr1 rest | 0.0 | Lung Microvascular EC none | 25.9 |
| Primary Th1 act | 0.0 | Lung Microvascular EC TNFalpha + IL-1beta | 23.5 |
| Primary Th2 act | 0.0 | Microvascular Dermal EC none | 35.6 |
| Primary Tr1 act | 0.0 | Microsvasular Dermal EC TNFalpha + IL-1beta | 21.6 |
| Primary Th1 rest | 0.0 | Bronchial epithelium TNFalpha + IL1beta | 27.7 |
| Primary Th2 rest | 0.4 | Small airway epithelium none | 9.4 |
| Primary Tr1 rest | 0.0 | Small airway epithelium TNFalpha + IL-1beta | 27.0 |
| CM5RA CD4 lymphocyte act | 8.5 | Coronery artery SMC rest | 31.6 |
| CD45RO CD4 lymphocyte act | 0.2 | Coronery artery SMC TNFalpha + IL-1beta | 11.0 |
| CD8 lymphocyte act | 0.0 | Astrocytes rest | 35.4 |
| Secondary CD8 lymphocyte rest | 0.2 | Astrocytes TNFalpha + IL-1beta. | 19.9 |
| Secondary CD8 lymphocyte act | 0.0 | KU-812 (Basophil) rest | 2.9 |
| CD4 lymphocyte none | 0.1 | KU-812 (Basophil) PMA/ionomycin | 8.2 |
| 2ry Th1/Th2/Tr1_anti-CD95 CH11 | 0.0 | CCD1106 (Keratinocytes) none | 3.5 |
| LAK cells rest | 1.5 | CCD1106 (Keratinocytes) | 0.9 |
| LAK cells IL-2 | 0.9 | Liver cirrhosis | 6.5 |
| LAK cells IL-2+IL-12 | 0.4 | Lupus kidney | 6.6 |
| LAK cells IL-2+IFN gamma | 1.9 | NCI-H292 none | 71.7 |
| LAK cells IL-2+IL-18 | 1.1 | NCI-H292 IL-4 | 55.9 |
| LAK cers PMA/ionomycin | 0.6 | NCI-H292 IL-9 | 87.7 |
| NK Cells IL-2 rest | 0.0 | NCI-H292 IL-13 | 28.7 |
| Two Way MLR 3 day | 1.6 | NCI-H292 IFN gamma | 43.2 |
| Two Way MLR 5 day | 0.4 | HPAEC none | 19.6 |
| Two Way MLR 7 day | 0.0 | HPAEC TNF alpha + IL-1 beta | 15.4 |
| PBMC rest | 0.4 | Lung fibroblast none | 49.7 |
| PBMCPWM | 1.4 | Lung fibroblast TNF alpha + IL-1 beta | 16.8 |
| PBMCPHA-L | 0.2 | Lung fibroblast IL-4 | 84.7 |
| Ramos (B cell) none | 7.6 | Lung fibroblast IL-9 | 59.0 |
| Ramos (B cell) ionomycin | 26.6 | Lung fibroblast IL-13 | 55.9 |
| B lymphocytes PWM | 3.7 | Lung fibroblast IFN gamma | **100.0** |
| B lymphocytes CD40L and IL-4 | 3.9 | Dermal fibroblast CCD1070 rest | 51.4 |
| EOL-1 dbcAMP | 5.1 | Dermal fibroblast CCD1070 TNF alpha | 33.2 |
| EOL-1 dbcAMP PMA/ionomycin | 5.7 | Dermal fibroblast CCD1070 IL-1 beta | 20.0 |
| Dendritic cells none | 0.7 | Dermal fibroblast IFN gamma | 36.6 |
| Dendritic cells LPS | 1.2 | Dermal fibroblast IL-4 | 52.5 |
| Dendritic cells anti-CD40 | 0.7 | IBD Colitis 2 | 2.8 |
| Monocytes rest | 0.0 | IBD Crohn's | 2.6 |
| Monocytes LPS | 0.2 | Colon | 24.8 |
| Macrophages rest | 0.2 | Lung | 59.5 |
| Macrophages LPS | 1.2 | Thymus | 42.9 |
| HUVEC none | 20.4 | Kidney | 17.3 |
| HUVEC starved | 51.4 | | |

**Table PF. Panel CNS_1**

| **Tissue Name** | **Rel. Exp.(%) Ag3240, Run 171694589** | **Tissue Name** | **Rel. Exp.(%) Ag3240, Run 171694589** |
|---|---|---|---|
| BA4 Control | 13.0 | BA17 PSP | 28.7 |
| BA4 Control2 | 21.6 | BA17 PSP2 | 6.7 |
| BA4 Alzheimer's2 | 4.8 | Sub Nigra Control | 41.8 |
| BA4 Parkinson's | 59.9 | Sub Nigra Control2 | 36.9 |
| BA4 Parkinson's2 | 34.6 | Sub Nigra Alzheimer's2 | 18.7 |
| BA4 Huntington's | 18.8 | Sub Nigra Parkinson's2 | 58.2 |
| BA4 Huntington's2 | 5.0 | Sub Nigra Huntington's | 85.3 |
| BA4 PSP | 2.7 | Sub Nigra Huntington's2 | 23.8 |
| BA4 PSP2 | 23.3 | Sub Nigra PSP2 | 11.6 |
| BA4 Depression | 19.2 | Sub Nigra Depression | 13.9 |
| BA4 Depression2 | 17.4 | Sub Nigra Depression2 | 11.4 |
| BA7 Control | 27.0 | Glob Palladus Control | 22.8 |
| BA7 Control2 | 15.9 | Glob Palladus Control2 | 17.9 |
| BA7 Alzheimer's2 | 13.5 | Glob Palladus Alzheimer's | 17.2 |
| BA7 Parkinson's | 34.2 | GlobPalladus Alzheimer's2 | 9.8 |
| BA7 Parkinson's2 | 26.8 | Glob Palladus Parkinson's | **100.0** |
| BA7 Huntington's | 43.5 | Glob Palladus Parkinson's2 | 10.5 |
| BA7 Huntington's2 | 40.9 | Glob Palladus PSP | 17.2 |
| BA7 PSP | 48.3 | Glob Palladus PSP2 | 7.4 |
| BA7 PSP2 | 17.1 | Glob Palladus Depression | 9.3 |
| BA7 Depression | 27.0 | Temp Pole Control | 11.8 |
| BA9 Control | 11.1 | Temp Pole Control2 | 33.4 |
| BA9 Control2 | 39.5 | Temp Pole Alzheimer's | 1.5 |
| BA9 Alzheimer's | 4.7 | Temp Pole Alzheimer's2 | 5.7 |
| BA9 Alzheimer's2 | 18.7 | Temp Pole Parkinson's | 39.8 |
| BA9 Parkinson's | 26.8 | Temp Pole Parkinson's2 | 12.9 |
| BA9 Parkinson's2 | 41.5 | Temp Pole Huntington's | 19.6 |
| BA9 Huntington's | 54.7 | Temp Pole PSP | 6.8 |
| BA9 Huntington's2 | 8.8 | Temp Pole PSP2 | 0.7 |
| BA9 PSP | 24.5 | Temp Pole Depression2 | 10.9 |
| BA9 PSP2 | 7.1 | Cing Gyr Control | 42.3 |
| BA9 Depression | 6.1 | Cing Gyr Control2 | 20.9 |
| BA9 Depression2 | 5.0 | Cing Gyr Alzheimer's | 23.3 |
| BA17 Control | 40.3 | Cing Gyr Alzheimer's2 | 7.9 |
| BA17 Control2 | 26.1 | Cing Gyr Parkinson's | 38.2 |
| BA17 Alzheimer's2 | 6.0 | Cing Gyr Parkinson's2 | 51.1 |
| BA17 Parkinson's | 52.1 | Cing Gyr Huntington's | 59.0 |
| BA17 Parkinson's2 | 32.1 | Cing Gyr Huntington's2 | 19.6 |
| BA17 Huntington's | 21.0 | Cing Gyr PSP | 51.1 |
| BA17 Huntington's2 | 15.4 | Cing Gyr PSP2 | 13.6 |
| BA17 Depression | 25.7 | Cing Gyr Depression | 18.3 |
| BA17 Depression2 | 10.2 | Cing Gyr Depression2 | 15.0 |

**CNS_neurodegeneration_v1.0 Summary:** Ag3240 Highest expression of the CG57444-01 gene is seen in the hippocampus of a patient with Alzheimer's disease (CT=27.6). This panel suggests a slight upregulation of this gene in the AD temporal cortex that falls short of significance (p = 0.051). Thus, this gene product may be a drug target for the treatment of Alzheimer's disease; specifically, it may be involved in compensatory synaptogenesis in response to neuronal loss.

**Panel 1.3D Summary:** Ag3240 The CG57444-01 gene, a cadherin homolog, is expressed in many samples in this panel, reflecting the widespread role of cadherins in cell-cell adhesion. Highest expression is seen in a brain cancer cell line (CT=28.6). Thus, expression of this gene could be used to differentiate between this sample and other samples on this panel. Overall, however, expression appears to be associated with normal tissues rather than cancer cell lines. This can be seen in the samples from normal ovarian and breast tissue, which show higher levels of expression than the cell lines derived from breast and ovarian cancers. Loss of function of the related E-cadherin protein has been described in many tumors, along with an increased invasiveness and a decreased prognosis of many carcinomas, including tumors of endocrine glands and their target systems (ref 1). Thus, this gene product might similarly be useful as a protein therapeutic to treat a variety of tumors.

In addition, this gene is moderately expressed in pituitary gland, adrenal gland, thyroid, pancreas, and fetal and adult skeletal muscle, heart and liver, again reflecting the widespread role of this cadherin homolog. This observation may suggest that this gene product plays a role in normal metabolic and neuroendocrine function and that disregulated expression of this gene may contribute to metabolic diseases (such as obesity and diabetes) or neuroendocrine disorders.

Expression of this gene is also high in many regions of the brain, including the amygdala, thalamus, cerebellum, cerebral cortex, hippocampus, and substantia nigra. Expression is also detected in the spinal cord. Cadherins can act as axon guidance and cell adhesion proteins, specifically during development and in the response to injury (ref 2). Manipulation of levels of this protein may be of use in inducing a compensatory synaptogenic response to neuronal death in Alzheimer's disease, Parkinson's disease, Huntington's disease, spinocerebellar ataxia, progressive supranuclear palsy, ALS, head trauma, stroke, or any other disease/condition associated with neuronal loss (Potter et al., Endocr. Rev. 20: 207-239, 1999; Ranscht, Int. J. Dev. Neurosci. 18: 643-651, 2000).

**Panel 2.2 Summary:** Ag3240 Highest expression of the CG57444-01 gene is seen in a kidney cancer (CT=29.7). Overall, however, expression appears to be associated with normal tissues, as seen in Panel 1.3D. Thus, higher levels of expression are seen in normal colon, ovary, lung and stomach, when compared to adjacent tumors. Thus, absence of expression of this gene could be used as a diagnostic marker of these cancers. Furthermore, therapeutic modulation of the expression or function of this gene could be effective in the treatment of colon, ovarian, lung and stomach cancers.

**Panel 4D Summary:** Ag3240 The CG57444-01 transcript is expressed in endothelium, fibroblasts and NCI-H292 cells. This gene is also expressed in normal colon, kidney, thymus, and lung. This transcript encodes a putative protocadherin family member. Protocadherins mediate cell:cell interactions and signalling. Since this gene product is expressed in endothelium, it may also be involved in normal blood cell recirculation through the tissues. Alternatively, the protein encoded by this gene may be important for regulating tissue or organ organization and normal function since it is expressed on fibroblasts and epithelium.

**Panel CNS_1 Summary:** Ag3240 Results from this experiment confirm expression of the CG57444-01 gene in the brain. Please see Panel 1.3D for discussion of utility of this gene in the central nervous system.

### Q. NOV17a and NOV17b: Cadherin 23

Expression of gene CG57429-01 and variant CG57429-02 was assessed using the primer-probe sets Ag3234, Ag3279 and Ag616, described in Tables QA, QB and QC. Results of the TQ-PCR runs are shown in Tables QD, QE, QF, QG, QH, QI, QJ and QK.

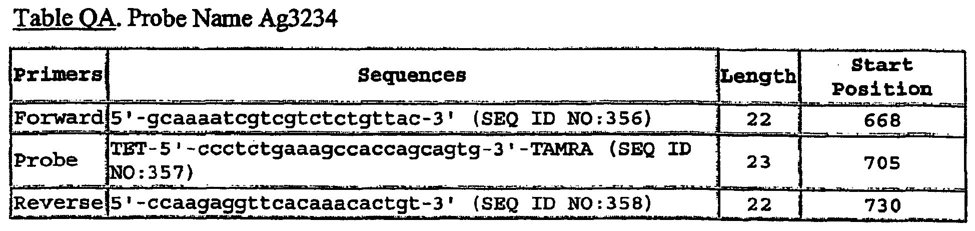

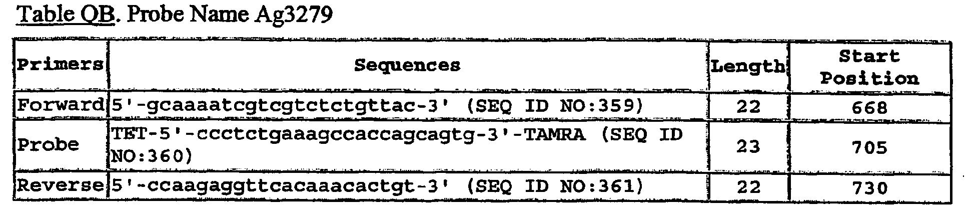

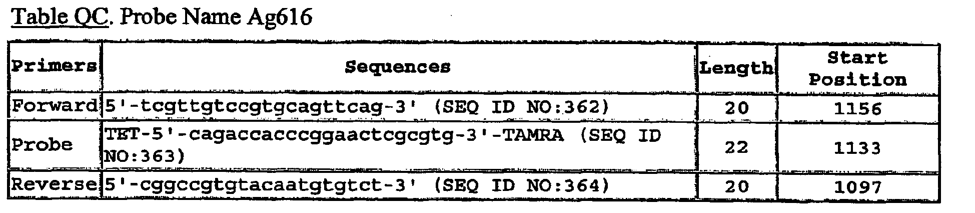

**Table OD. CNS_neurodegeneration_v1.0**

| **Tissue Name** | **Rel. Exp.(%) Ag3234, Run 209862304** | **Rel. Exp.(%) Ag3279, Run 210060481** | **Tissue Name** | **Rel. Exp.(%) Ag3234, Run 209862304** | **Rel. Exp.(%) Ag3279, Run 210060481** |
|---|---|---|---|---|---|
| AD 1 Hippo | 16.3 | 21.8 | Control (Path) 3 Temporal Ctx | 14.6 | 16.2 |
| AD 2 Hippo | 23.8 | 32.3 | Control (Path) 4 Temporal Ctx | 26.4 | 31.2 |
| AD 3 Hippo | 13.2 | 14.1 | AD 1 Occipital Ctx | 17.1 | 19.2 |
| AD 4 Hippo | 19.8 | 22.5 | AD 2 Occipital Ctx (Missing) | 0.0 | 0.0 |
| AD 5 hippo | 58.2 | 49.7 | AD 3 Occipital Ctx | 23.5 | 22.4 |
| AD 6 Hippo | 58.6 | **100.0** | AD 4 Occipital Ctx | 18.3 | 21.2 |
| Control 2 Hippo | 19.8 | 24.5 | AD 5 Occipital Ctx | 31.9 | 38.4 |
| Control 4 Hippo | 29.1 | 23.5 | AD 6 Occipital Ctx | 24.1 | 37.9 |
| Control (Path) 3 Hippo | 11.7 | 7.1 | Control 1 Occipital Ctx | 19.8 | 26.1 |
| AD 1 Temporal Ctx | 28.1 | 28.7 | Control 2 Occipital Ctx | 31.0 | 33.7 |
| AD 2 Temporal Ctx | 18.7 | 28.9 | Control 3 Occipital Ctx | 21.8 | 22.7 |
| AD 3 Temporal Ctx | 15.5 | 15.7 | Control 4 Occipital Ctx | 13.3 | 20.7 |
| AD 4 Temporal Ctx | 27.7 | 25.5 | Control (Path) 1 Occipital Ctx | 37.1 | 32.5 |
| AD 5 Inf Temporal Ctx | 38.2 | 52.1 | Control (Path) 2 Occipital Ctx | 14.7 | 12.6 |
| AD 5 SupTemporal Ctx | 45.7 | 49.7 | Control (Path) 3 Occipital Ctx | 20.3 | 19.2 |
| AD 6 Inf Temporal Ctx | 81.8 | 67.8 | Control (Path) 4 Occipital Ctx | 20.4 | 26.1 |
| AD 6 Sup Temporal Ctx | **100.0** | 94.6 | Control 1 Parietal Ctx | 14.2 | 22.4 |
| Control 1 Temporla Ctx | 10.1 | 14.2 | Control 2 Parietal Ctx | 35.8 | 44.4 |
| Control 2 Temporal Ctx | 15.0 | 11.9 | Control 3 Parietal Ctx | 5.5 | 12.8 |
| Control 3 Temporal Ctx | 8.6 | 14.7 | Control (Path) 1 Parietal Ctx | 17.7 | 21.9 |
| Control 4 Temporal Ctx | 8.9 | 12.1 | Control (Path) 2 Parietal Ctx | 19.2 | 25.3 |
| Control (Path) 1 Temporal Ctx | 23.7 | 31.0 | Control (Path) 3 Parietal Ctx | 13.0 | 17.9 |
| Control (Path) 2 Temporal Ctx | 14.3 | 16.6 | Control (Path) 4 Parietal Ctx | 32.3 | 33.0 |

**Table QE. General_screening_panel_v1.4**

| **Tissue Name** | **Rel. Exp.(%) Ag3279, Run 216512994** | **Tissue Name** | **Rel. Exp.(%) Ag3279, Run 216512994** |
|---|---|---|---|
| Adipose | 35.8 | Renal ca. TK-10 | 0.0 |
| Melanoma* Hs688(A).T | 0.0 | Bladder | 9.4 |
| Melanoma* Hs688(B).T | 0.0 | Gastric ca. (liver met.) NCI-N87 | 0.4 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.1 | Colon ca. SW480 | 2.1 |
| Squamous cell carcinoma SCC-4 | 0.0 | Colon ca.* (SW480 met) SW620 | 0.7 |
| Testis Pool | 11.0 | Colon ca. HT29 | 0.0 |
| Prostate ca.* (bone met) PC-3 ca.* | 0.1 | Colon ca. HCT-116 | 0.2 |
| Prostate Pool | 3.8 | Colon ca. CaCo-2 | 1.0 |
| Placenta | 0.6 | Colon cancer tissue | 0.8 |
| Uterus Pool | 3.1 | Colon ca. SW1116 | 0.0 |
| Ovarian ca. OVCAR-3 | 10.4 | Colon ca. Colo-205 | 0.0 |
| Ovarian ca. SK-OV-3 | 1.4 | Colon ca. SW-48 | 0.0 |
| Ovarian ca. OVCAR-4 | 0.0 | Colon Pool | 8.0 |
| Ovarian ca. OVCAR-5 | 4.0 | Small Intestine Pool | 12.1 |
| Ovarian ca. IGROV-1 | 2.4 | Stomach Pool | 6.1 |
| Ovarian ca. OVCAR-8 | 1.4 | Bone Marrow Pool | 8.4 |
| Ovary | 37.4 | Fetal Heart | 15.6 |
| Breast ca. MCF-7 | 0.1 | Heart Pool | 9.4 |
| Breast ca. MDA-MB-231 | 0.0 | Lymph Node Pool | 13.7 |
| Breast ca. BT 549 | 0.0 | Fetal Skeletal Muscle | 4.7 |
| Breast ca. T47D | 5.8 | Skeletal Muscle Pool | 4.9 |
| Breast ca. MDA-N | 0.0 | Spleen Pool | 5.8 |
| Breast Pool | 7.0 | Thymus Pool | 13.0 |
| Trachea | 4.3 | CNS cancer (glio/astro) U87-MG | 0.2 |
| Lung | 16.0 | CNS cancer (glio/astro) U-118-MG | 0.0 |
| Fetal Lung | 24.5 | CNS cancer (neuro;met) SK-N-AS | 0.0 |
| Lung ca. NCI-N417 | 2.2 | CNS cancer (astro) SF-539 | 0.0 |
| Lung ca. LX-1 | 0.3 | CNS cancer (astro) SNB-75 | 0.1 |
| Lung ca. NCI-H146 | 0.3 | CNS cancer (glio) SNB-19 | 1.8 |
| Lung ca. SHP-77 | 0.0 | CNS cancer (glio) SF-295 | 0.8 |
| Lung ca. A549 | 4.2 | Brain (Amygdala) Pool | 9.7 |
| Lung ca. NCI-H526 | 10.2 | Brain (cerebellum) | **100.0** |
| Lung ca. NCI-H23 | 12.0 | Brain (fetal) | 8.2 |
| Lung ca. NCI-H460 | 0.1 | Brain (Hippocampus) Pool | 10.2 |
| Lung ca. HOP-62 | 0.0 | Cerebral Cortex Pool | 7.9 |
| Lung ca. NCI-H522 | 6.0 | Brain (Substantia nigra) Pool | 6.0 |
| Liver | 9.1 | Brain (Thalamus) Pool | 10.9 |
| Fetal Liver | 3.5 | Brain (whole) | 16.0 |
| Liver ca. HepG2 | 0.0 | Spinal Cord Pool | 8.9 |
| Kidney Pool | 34.2 | Adrenal Gland | 3.9 |
| Fetal Kidney | 5.0 | Pituitary gland Pool | 0.6 |
| Renal ca. 786-0 | 0.0 | Salivary Gland | 2.5 |
| Renal ca. A498 | 1.1 | Thyroid (female) | 1.4 |
| Renal ca. ACHN | 0.0 | Pancreatic ca. CAPAN2 | 0.0 |
| Renal ca. UO-31 | 0.1 | Pancreas Pool | 9.5 |

**Table QF. Panel 1.1**

| **Tissue Name 11162134** | **Rel. Exp.(%) Ag616, Run** | **Tissue Name** | **Rel. Exp.(%) Ag616, Run 111162134** |
|---|---|---|---|
| Adrenal gland | 3.7 | Renal ca. UO-31 | 0.0 |
| Bladder | 11.7 | Renal ca. RXF 393 | 0.0 |
| Brain (amygdala) | 0.2 | Liver | 26.2 |
| Brain (cerebellum) | 76.8 | Liver (fetal) | 0.1 |
| Brain (hippocampus) | 7.4 | Liver ca. (hepatoblast) HepG2 | 0.0 |
| Brain (substantia nigra) | 76.3 | Lung | 4.2 |
| Brain (thalamus) | 16.2 | Lung (fetal) | 3.8 |
| Cerebral Cortex | 6.0 | Lung ca. (non-s.cell) HOP-62 | 0.0 |
| Brain (fetal) | 2.2 | Lung ca. (large cell)NCI- | 0.0 |
| | | H460 | |
| Brain (whole) | 31.0 | Lung ca. (non-s.cell) NCI-H23 | 6.0 |
| glio/astro U-118-MG | 0.0 | Lung ca. (non-s.cl) NCI-H522 | 18.6 |
| astrocytoma SF-539 | 0.0 | Lung ca. (non-sm. cell) A549 | 6.2 |
| astrocytoma SNB-75 | 0.0 | Lung ca. (s.cell var.) SHP-77 | 0.0 |
| astrocytoma SW1783 | 0.0 | Lung ca. (small cell) LX- 1 | 0.0 |
| glioma U251 | 0.0 | Lung ca. (small cell) NCI-H69 | 1.5 |
| glioma SF-295 | 0.0 | Lung ca. (squam.) SW 900 | 0.0 |
| glioma SNB-19 | 0.0 | Lung ca. (squam.) NCI-H596 | 18.4 |
| glio/astro U87-MG | 0.0 | Lymph node | 3.4 |
| neuro*; met SK-N-AS | 0.0 | Spleen | 2.9 |
| Mammary gland | 25.0 | Thymus | 5.6 |
| Breast ca. BT-549 | 0.0 | Ovary | 47.3 |
| Breast ca. MDA-N | 0.0 | Ovarian ca. IGROV-1 | 8.6 |
| Breast ca.* (pl.ef) T47D | 0.0 | Ovarian ca. OVCAR-3 | 13.5 |
| Breast ca.* (pl.ef) MCF-7 | 0.0 | Ovarian ca. OVCAR-4 | 0.0 |
| Breast ca.* (pl.ef) MDA-MB-231 | 0.0 | Ovarian ca. OVCAR-5 | 6.8 |
| Small intestine | 2.4 | Ovarian ca. OVCAR-8 | 0.2 |
| Colorectal | 0.0 | Ovarian ca.* (ascites) SK-OV-3 | 0.1 |
| Colon ca. HT29 | 0.0 | Pancreas | **100.0** |
| Colon ca. CaCo-2 | 0.0 | Pancreatic ca. CAPAN | 2 0.0 |
| Colon ca. HCT-15 | 0.0 | Pituitary gland | 0.0 |
| Colon ca. HCT-116 | 0.0 | Placenta | 0.5 |
| Colon ca.HCC-2998 | 0.0 | Prostate | 0.7 |
| Colon ca. SW480 | 0.0 | Prostate ca.* (bone met) PC-3 | 0.0 |
| Colon ca.* SW620 (SW480 met) | 0.0 | Salivary gland | 1.8 |
| Stomach 3.4 | | Trachea | 1.0 |
| Gastric ca. (liver met) NCI-N87 | 0.0 | Spinal cord | 10.4 |
| Heart | 46.3 | Testis | 10.2 |
| Skeletal muscle (Fetal) | 19.2 | Thyroid | 0.7 |
| Skeletal muscle | 19.2 | Uterus | 7.6 |
| Endothelial cells | 0.0 | Melanoma M14 | 0.0 |
| Heart (Fetal) | 4.1 | Melanoma LOX IMVI | 0.0 |
| Kidney | 0.6 | Melanoma UACC-62 | 0.0 |
| Kidney (fetal) | 0.2 | Melanoma SK-MEL-28 | 0.0 |
| Renal ca. 786-0 | 0.0 | Melanoma* (met) SK-MEL-5 | 0.0 |
| Renal ca. A498 | 0.0 | Melanoma Hs688(A).T | 0.0 |
| Renal ca. ACHN | 0.0 | Melanoma* (met) Hs688(B).T | 0.0 |
| Renal ca. TK-10 | 0.0 | | |

**Table QG. Panel 1.2**

| **Tissue Name** | **Rel.Exp.(%) Ag616, Run 118515000** | **Tissue Name** | **Rel. Exp.(%) Ag616, Run 118515000** |
|---|---|---|---|
| Endothelial cells | 0.0 | Renal ca. 786-0 | 0.0 |
| Heart (Fetal) | 8.5 | Renal ca. A498 | 0.1 |
| Pancreas | **100.0** | Renal ca. RXF 393 | 0.0 |
| Pancreatic ca. CAPAN 2 | 0.0 | Renal ca. ACHN | 0.0 |
| Adrenal Gland | 25.5 | Renal ca. UO-31 | 0.0 |
| Thyroid | 8.5 | Renal ca. TK-10 | 0.0 |
| Salivary gland | 8.1 | Liver | 69.7 |
| Pituitary gland | 6.2 | Liver (fetal) | 6.6 |
| Brain (fetal) | 17.8 | Liver ca. (hepatoblast) HepG2 | 0.0 |
| Brain (whole) | 52.9 | Lung | 21.5 |
| Brain (amygdala) | 23.5 | Lung (fetal) | 12.0 |
| Brain (cerebellum) | 66.4 | Lung ca. (small cell) LX-1 | 0.8 |
| Brain (hippocampus) | 21.2 | Lung ca. (small cell) NCI-H69 | 8.4 |
| Brain (thalamus) | 23.3 | Lung ca. (s.cell var.) SHP-77 | 0.0 |
| Cerebral Cortex | 0.0 | Lung ca. (large cell)NCI-H460 | 0.0 |
| Spinal cord | 19.3 | Lung ca. (non-sm. cell) A549 | 12.8 |
| glio/astro U87-MG | 0.0 | Lung ca. (non-s.cell) NCI-H23 | 11.2 |
| glio/astro U-118-MG | 0.0 | Lung ca. (non-s.cell) HOP-62 | 0.1 |
| astrocytoma SW1783 | 0.0 | Lung ca. (non-s.cl) NCI-H522 | 36.3 |
| neuro*; met SK-N-AS | 0.0 | Lung ca. (squam.) SW 900 | 1.6 |
| astrocytoma SF-539 | 0.0 | Lung ca. (squam.) NCI-H596 | 29.5 |
| astrocytoma SNB-75 | 0.0 | Mammary gland | 44.1 |
| gliomaSNB-19 | 0.1 | Breast ca.* (pl.ef) MCF-7 | 0.0 |
| glioma U251 | 0.0 | Breast ca.* (pl.ef) MDA- : MB-231 | 0.0 |
| glioma SF-295 | 0.0 | Breast ca.* (pl. ef) T47D | 0.1 |
| Heart | 69.7 | Breast ca. BT-549 | 0.0 |
| Skeletal Muscle | 32.1 | Breast ca. MDA-N | 0.0 |
| Bone marrow | 6.6 | Ovary | 53.2 |
| Thymus | 17.4 | Ovarian ca. OVCAR-3 | 18.9 |
| Spleen | 15.6 | Ovarian ca. OVCAR-4 | 0.4 |
| Lymph node | 15.9 | Ovarian ca. OVCAR-5 | 10.5 |
| Colorectal Tissue | 0.0 | Ovarian ca. OVCAR-8 | 3.6 |
| Stomach | 11.8 | Ovarian ca. IGROV-1 | 18.8 |
| Small intestine | 15.9 | Ovarian ca. (ascites) SK-OV-3 | 1.9 |
| Colon ca. SW480 | 0.4 | Uterus | 22.7 |
| Colon ca.* SW620 (SW480 met) | 0.2 | Placenta | 6.7 |
| Colon ca. HT29 | 1.0 | Prostate | 6.5 |
| Colon ca. HCT-116 | 0.0 | Prostate ca.* (bone met) PC-3 | 0.0 |
| Colon ca. CaCo-2 | 0.7 | Testis | 50.3 |
| Colon ca. Tissue (ODO3866) | 0.3 | Melanoma Hs688(A).T | 0.0 |
| Colon ca. HCC-2998 | 0.6 | Melanoma* (met) Hs688(B).T | 0.0 |
| Gastric ca.* (liver met) NCI-N87 | 0.2 | Melanoma UACC-62 | 0.0 |
| Bladder | 28.3 | Melanoma M14 | 0.0 |
| Trachea | 7.0 | Melanoma LOXIMVI | 0.0 |
| Kidney | 2.8 | Melanoma* (met) SK-MEL-5 | 0.0 |
| Kidney (fetal) | 5.3 | | |

**Table QH. Panel 1.3D**

| **Tissue Name** | **Rel. Exp.(%) Ag3234, Run 165524160** | **Tissue Name** | **Rel. Exp.(%) Ag3234, Run 165524160** |
|---|---|---|---|
| Liver adenocarcinoma | 5.8 | Kidney (fetal) | 2.4 |
| Pancreas | 43.2 | Renal ca. 786-0 | 0.0 |
| Pancreatic ca. CAPAN 2 | 0.0 | Renal ca. A498 | 0.0 |
| Adrenal gland | 4.5 | Renal ca. RXF 393 | 0.0 |
| Thyroid | 0.0 | Renal ca. ACHN | 0.0 |
| Salivary gland | 6.3 | Renal ca. UO-31 | 0.0 |
| Pituitary gland | 1.4 | Renal ca. TK-10 | 0.0 |
| Brain (fetal) | 5.8 | Liver | 6.3 |
| Brain (whole) | 45.4 | Liver (fetal) | 2.9 |
| Brain (amygdala) | 27.2 | Liver ca. (hepatoblast) HepG2 | 0.0 |
| Brain (cerebellum) | **100.0** | Lung | 12.2 |
| Brain (hippocampus) | 21.3 | Lung (fetal) | 6.5 |
| Brain (substantia nigra) | 21.0 | Lung ca. (small cell) LX-1 | 1.4 |
| Brain (thalamus) | 27.2 | Lung ca. (small cell) NCI-H69 | 1.2 |
| Cerebral Cortex | 14.3 | Lung ca. (s.cell var.) SHP-77 | 0.0 |
| Spinal cord | 35.4 | Lung ca. (large cell)NCI-H460 | 2.0 |
| glio/astro U87-MG | 0.0 | Lung ca. (non-sm.cell) A549 | 4.9 |
| glio/astro U-118-MG | 0.0 | Lung ca. (non-s.cell) NCI-H23 | 6.2 |
| astrocytoma SW1783 | 0.0 | Lung ca. (non-s.cell) HOP-62 | 0.0 |
| neuro*; met SK-N-AS | 1.6 | Lung ca. (non-s.cl) NCI-H522 | 0.6 |
| astrocytoma SF-539 | 0.0 | Lung ca. (squam.) SW 900 | 0.7 |
| astrocytoma SNB-75 | 0.0 | Lung ca. (squam.) NCI-H596 | 7.4 |
| glioma SNB-19 | 0.0 | Mammary gland | 28.9 |
| glioma U251 | 0.0 7 | Breast ca.* (pl.ef) MCF- | 0.0 |
| glioma SF-295 | 0.0 | Breast ca.* (pl.ef) MDA-MB-231 | 0.0 |
| Heart (fetal) | 15.2 | Breast ca.* (pl.ef) T47D | 0.0 |
| Heart | 13.3 | Breast ca. BT-549 | 0.0 |
| Skeletal muscle (fetal) | 9.9 | Breast ca. MDA-N | 0.0 |
| Skeletal muscle | 11.2 | Ovary | 50.7 |
| Bone marrow | 6.2 | Ovarian ca. OVCAR-3 | 7.1 |
| Thymus | 18.6 | Ovarian ca. OVCAR-4 | 1.3 |
| Spleen | 8.0 | Ovarian ca. OVCAR-5 | 6.0 |
| Lymph node | 14.4 | Ovarian ca. OVCAR-8 | 3.0 |
| Colorectal | 7.4 | Ovarian ca. IGROV-1 | 1.6 |
| Stomach | 4.5 | Ovarian ca.* (ascites) SK-OV-3 | 1.2 |
| Small intestine | 16.6 | Uterus | 42.6 |
| Colon ca. SW480 | 0.0 | Placenta | 1.7 |
| Colon ca.* SW620(SW480 met) | 0.0 | Prostate | 3.4 |
| Colon ca. HT29 Colon ca. HT29 | 0.0 | Prostate ca.* (bone met)PC-3 | 0.0 |
| Colon ca. HCT-116 | 0.0 | Testis | 15.6 |
| Colon ca. CaCo-2 | 0.0 | Melanoma Hs688(A).T | 0.0 |
| Colon ca. tissue(ODO3866) | 1.4 | Melanoma* (met) Hs688(B).T | 0.0 |
| Colon ca. HCC-2998 | 0.0 | Melanoma UACC-62 | 0.0 |
| Gastric ca.* (liver met) NCI-N87 | 0.0 | Melanoma M14 | 0.0 |
| Bladder | 5.2 | Melanoma LOX IMVI | 0.0 |
| Trachea | 4.1 | Melanoma* (met) SK-MEL-5 | 0.0 |
| Kidney | 0.0 | Adipose | 28.1 |

**Table QI. Panel 2.2**

| **Tissue Name Name** | **Rel. Exp.(%) Ag3234, Run 174442923** | **Tissue Name** | **Rel. Exp.(%) Ag3234, Run 174442923** |
|---|---|---|---|
| Normal Colon | 7.1 | Kidney Margin (OD04348) | 4.8 |
| Colon cancer (OD06064) | 6.7 | Kidney malignant cancer (OD06204B) | 22.2 |
| Colon Margin (OD06064) | 5.6 | Kidney normal adjacent tissue (OD06204E) | 3.4 |
| Colon cancer (OD06159) | 0.0 | Kidney Cancer (OD04450-01) | 0.0 |
| Colon Margin (OD06159) | 5.9 | Kidney Margin (OD04450-03) | 1.8 |
| Colon cancer (OD06297-04) | 0.0 | Kidney Cancer 8120613 | 0.0 |
| Colon Margin (OD06297-05) | 2.9 | Kidney Margin 8120614 | 1.4 |
| CC Gr.2 ascend colon (ODO3921) | 1.8 | Kidney Cancer 9010320 | 0.0 |
| CC Margin (ODO3921) | 0.0 | Kidney Margin 9010321 | 1.3 |
| Colon cancer metastasis (OD06104) | 1.2 | Kidney Cancer 8120607 | 1.9 |
| Lung Margin (OD06104) | 0.7 | Kidney Margin 8120608 | 1.5 |
| Colon mets to lung (OD04451-01) | 0.0 | Normal Uterus | 33.9 |
| Lung Margin (OD04451-02) | 57.4 | Uterine Cancer 064011 | 6.7 |
| Normal Prostate | 2.6 | Normal Thyroid | 1.3 |
| Prostate Cancer (OD04410) | 0.0 | Thyroid Cancer 064010 | 0.0 |
| Prostate Margin (OD04410) | 7.7 | Thyroid Cancer A302152 | 1.8 |
| Normal Ovary | **100.0** | Thyroid Margin A302153 | 0.8 |
| Ovarian cancer (OD06283-03) | 5.4 | Normal Breast | 22.5 |
| Ovarian Margin (OD06283-07) | 27.9 | Breast Cancer (OD04566) | 3.9 |
| Ovarian Cancer 064008 | 12.9 | Breast Cancer 1024 | 11.4 |
| Ovarian cancer (OD06145) | 12.7 | Breast Cancer (OD04590-01) | 20.0 |
| Ovarian Margin (OD06145) | 19.3 | Breast Cancer Mets (OD04590-03) | 17.2 |
| Ovarian cancer (OD06455-03) | 81 | Breast Cancer Metastasis (OD04655-05) | 31.0 |
| Ovarian Margin (OD06455-07) | 25.9 | Breast Cancer 064006 | 3.9 |
| Normal Lung | 14.8 | Breast Cancer 9100266 | 10.0 |
| Invasive poor diff. lung adeno (ODO4945-01 | 0.5 | Breast Margin 9100265 | 16.8 |
| Lung Margin (OD04945-03) | 28.7 | Breast Cancer A209073 | 10.4 |
| Lung Malignant Cancer (OD03126) | 7.0 | Breast Margin A2090734 | 28.1 |
| Lung Margin (OD03126) | 3.2 | Breast cancer (OD06083) | 12.6 |
| Lung Cancer (OD05014A) | 3.8 | Breast cancer node | 13.7 |
| Lung Margin (OD05014B) | 28.5 | Normal Liver | 41.5 |
| Lung cancer (OD06081) | 3.1 | Liver Cancer 1026 | 10.5 |
| Lung Margin (OD06081) | 18.2 | Liver Cancer 1025 | 40.1 |
| Lung Cancer (OD04237-01) | 2.7 | Liver Cancer 6004-T | 21.0 |
| Lung Margin (OD04237-02) | 12.2 | Liver Tissue 6004-N | 3.4 |
| Ocular Melanoma Metastasis | 0.0 | Liver Cancer 6005-T | 59.9 |
| Ocular Melanoma Margin (Liver) | 22.2 | Liver Tissue 6005-N | 59.5 |
| Melanoma Metastasis | 0.0 | Liver Cancer 064003 | 10.4 |
| Melanoma Margin (Lung) | 18.4 | Normal Bladder | 7.6 |
| Normal Kidney | 1.2 | Bladder Cancer 1023 | 2.5 |
| Kidney Ca, Nuclear grade 2 (OD04338) | 6.7 | Bladder Cancer A302173 | 1.4 |
| Kidney Margin (OD04338) | 1.7 | Normal Stomach | 12.3 |
| Kidney Ca Nuclear grade 1/2 (OD04339) | 22.1 | Gastric Cancer 9060397 | 1.0 |
| Kidney Margin(OD04339) | 5.0 | Stomach Margin 9060396 | 1.6 |
| Kidney Ca, Clear cell type (OD04340) | 3.5 | Gastric Cancer 9060395 | 1.3 |
| Kidney Margin (OD04340) | 3.7 | Stomach Margin 9060394 | 3.1 |
| Kidney Ca, Nuclear grade 3 (OD04348) | 0.8 | Gastric Cancer 064005 | 0.0 |

**Table QJ. Panel 4D**

| **Tissue Name** | **Rel. Exp.(%) Ag3234, Run 164328482** | **Rel. Exp.(%) Ag3279, Run 164634320** | **Tissue Name** | **Rel. Exp.(%) Ag3234, Run 164328482** | **Rel. Exp.(%) Ag3279, Run 164634320** |
|---|---|---|---|---|---|
| Secondary Th1 act | 0.0 | 0.0 | HUVEC IL-1beta | 0.0 | 0.0 |
| Secondary Th2 act | 0.2 | 0.0 | HUVEC IFN gamma | 0.0 | 0.0 |
| Secondary Tr1 act | 0.0 | 0.0 | HUVEC TNF alpha + IFN gamma | 0.0 | 0.0 |
| Secondary Th1 rest | 0.6 | 0.2 | HUVEC TNF alpha + IL4 | 0.0 | 0.0 |
| Secondary Th2 rest | 0.0 | 0.0 | HUVEC IL-11 | 0.0 | 0.0 |
| Secondary Tr1 rest | 0.3 | 0.1 | Lung Microvascular EC none | 0.0 | 0.0 |
| Primary Th1 act | 0.2 | 0.1 | Lung Microvascular BC TNFalpha + IL-1beta | 0.0 | 0.0 |
| Primary Th2 act | 0.0 | 0.0 | Microvascular Dermal EC none | 0.0 | 0.0 |
| Primary Tr1 act | 0.0 | 0.9 | Microsvasular Dermal EC TNFalpha + IL-1beta | 0.0 | 0.0 |
| Primary Th1 rest | 0.7 | 0.9 | Bronchial epithelium TNFalpha + IL1beta | 0.0 | 0.0 |
| Primary Th2 rest | 0.2 | 0.2 | Small airway epithelium none | 0.0 | 0.0 |
| Primary Tr1 rest | 0.8 | 0.2 | Small airway epithelium TNFalpha + IL-1beta | 0.0 | 0.0 |
| CD45RA CD4 lymphocyte act | 0.0 | 0.0 | Coronery artery SMC rest | 0.0 | 0.0 |
| CD45RO CD4 lymphocyte act | 0.0 | 0.0 | Coronery artery SMC TNFalpha + IL-1beta | 0.0 | 0.0 |
| CD8 lymphocyte act | 0.0 | 0.0 | Astrocytes rest | 0.0 | 0.0 |
| Secondary CD8 lymphocyte rest | 0.0 | 0.0 | Astrocytes TNFalpha + IL-1beta | 0.0 | 0.0 |
| Secondary CD8 lymphocyte act | 0.0 | 0.0 | KU-812 (Basophil) rest | 0.5 | 0.0 |
| CD4 lymphocyte none | 0.2 | 0.7 | KU-812 (Basophil) PMA/ionomycin | 0.2 | 0.3 |
| 2ry Th1/Th2/Tr1 anti-CD95 CH11 | 0.2 | 0.0 | CCD1106 (Keratinocytes) none | 0.0 | 0.0 |
| LAK cells rest | 6.2 | 7.6 | CCD1106 (Kemtinocytes) TNFalpha + IL-1beta | 0.0 | 0.0 |
| LAK cells IL-2 | 0.0 | 0.0 | Liver cirrhosis | 1.4 | 1.2 |
| LAK cells IL-2+IL-12 | 0.0 | 0.0 | Lupus kidney | 0.4 | 0.2 |
| LAK cells IL-2+IFN gamma | 0.1 | 0.4 | NCI-H292 none | 0.0 | 0.2 |
| LAK cells IL-2+ IL-18 | 0.4 | 0.0 | NCI-H292 IL-4 | 0.0 | 0.0 |
| LAK cells PMA/ionomycin | 1.3 | 2.7 | NCI-H292 IL-9 | 0.0 | 0.0 |
| NK Cells IL-2 rest | 0.0 | 0.0 | NCI-H292 IL-13 | 0.0 | 0.0 |
| Two Way MLR 3 day | 1.1 | 1.3 | NCI-H292 IFN gamma | 0.0 | 0.2 |
| Two Way MLR 5 day | 1.3 | 0.6 | HPAEC none | 0.0 | 0.0 |
| Two Way MLR 7 day | 0.8 | 0.4 | HPAECTNF alpha + IL-1 beta | 0.0 | 0.0 |
| PBMC rest | 3.3 | 3.1 | Lung fibroblast none | 0.0 | 0.0 |
| PBMCPWM | 0.0 | 0.1 | Lung fibroblast TNF alpha + IL-1 beta | 0.0 | 0.0 |
| PBMCPHA-L | 0.1 | 0.0 | Lung fibroblast IL-4 | 0.0 | 0.0 |
| Ramos (B cell) none | 0.0 | 0.0 | Lung fibroblast IL-9 | 0.0 | 0.0 |
| Ramos (B cell) ionomycin | 0.0 | 0.0 | Lung fibroblast IL-13 | 0.0 | 0.0 |
| B lymphocytes PWM | 0.0 | 0.0 | Lung fibroblast IFN gamma | 0.0 | 0.0 |
| B lymphocytes CD40L and IL-4 | 0.0 | 0.3 | Dermal fibroblast CCD1070 rest | 0.0 | 0.0 |
| EOL-1 dbcAMP | 0.0 | 0.2 | Dermal fibroblast CCD1070 TNF alpha | 0.2 | 0.6 |
| EOL-1 dbcAMP PMA/ionomycin | 0.1 | 0.0 | Dermal fibroblast CCD1070 IL-1 beta | 0 0 | 0.0 |
| Dendritic cells none | 25.9 | 49.0 | Dermal fibroblast IFN gamma | 0.0 | 0.2 |
| Dendritic cells LPS | 61.1 | 92.0 | Dermal fibroblast IL-4 | 0.2 | 0.0 |
| Dendritic ceus anti-CD40 | **100.0** | 94.6 | IBD Cohtis 2 | 0.0 | 0.7 |
| Monocytes rest | 12.2 | 23.0 | IBD Crohn's | 0.3 | 0.7 |
| Monocytes LPS | 2.6 | 2.5 | Colon | 3.0 | 2.8 |
| Macrophages rest | 92.0 | **100.0** | Lung | 7.4 | 9.7 |
| Macrophages LPS | 10.4 | 18.0 | Thymus | 1.2 | 3.7 |
| HUVEC none | 0.0 | 0.0 | Kidney | 35.4 | 33.4 |
| HUVEC starved | 0.0 | 0.0 | | | |

**Table QK. Panel CNS_1**

| **Tissue Name** | **Rel. Exp.(%) Ag3279, Run 171694591** | **Tissue Name** | **Rel. Exp.(%) Ag3279, Run 171694591** |
|---|---|---|---|
| BA4 Control | 3.2 | BA17 PSP | 13.4 |
| BA4 Control2 | 10.0 | BA17 PSP2 | 5.3 |
| BA4 Alzheimer's2 | 3.8 | Sub Nigra Control | 58.6 |
| BA4 Parhson's | 6.5 | Sub Nigra Control2 | 21.3 |
| BA4 Parkinson's2 | 11.7 | Sub Nigra Alzheimer's2 | 12.2 |
| BA4 Huntington's | 7.6 | Sub Nigra Parkinson's2 | 63.3 |
| BA4 Huntington's2 | 4.5 | Sub Nigra Huntington's | **100.0** |
| BA4 PSP | 5.1 | Sub Nigra Huntington's2 | 87.1 |
| BA4 PSP2 | 8.8 | Sub Nigra PSP2 | 16.8 |
| BA4 Depression | 6.7 | Sub Nigra Depression | 7.9 |
| BA4 Depression2 | 7.4 | Sub Nigra Depression2 | 24.3 |
| BA7 Control | 4.5 | Glob Palladus Control | 24.3 |
| BA7 Conbrol2 | 1.9 | Glob Palladus Control2 | 8.1 |
| BA7 Alzheimer's2 | 1.0 | Glob Palladus Alzheimer's | 14.9 |
| BA7 Parkinson's | 24.1 | Glob Palladus Alzheimer's2 | 1.2 |
| BA7 Parkinson's2 | 17.4 | Glob Palladus Parkinson's | 33.0 |
| BA7 Huntington's | 6.3 | Glob Palladus Parkinson's2 | 2.0 |
| BA7 Huntington's2 | 28.5 | Glob Palladus PSP | 3.0 |
| BA7 PSP | 18.9 | Glob Palladus PSP2 | 6.0 |
| BA7 PSP2 | 3.8 | Glob Palladus Depression | 8.8 |
| BA7 Depression | 1.7 | Temp Pole Control | 5.9 |
| BA9 Control | 5.3 | Temp Pole Control2 | 11.7 |
| BA9 Control2 | 4.1 | Temp Pole Alzheimer's | 7.4 |
| BA9 Alzheimer's | 3.6 | Temp Pole Alzheimer's2 | 3.0 |
| BA9 Alzheimer's2 | 8.8 | Temp Pole Parkinson's | 11.9 |
| BA9 Parkinson's | 18.6 | Temp Pole Parkinson's2 | 7.9 |
| BA9 Parkinson's2 | 20.4 | Temp Pole Huntington's | 8.8 |
| BA9 Huntington's | 15.0 | Temp Pole PSP | 4.7 |
| BA9 Huntington's2 | 7.4 | Temp Pole PSP2 | 0.0 |
| BA9 PSP | 5.3 | Temp Pole Depression2 | 12.6 |
| BA9 PSP2 | 1.6 | Cing Gyr Control | 18.0 |
| BA9 Depression | 4.7 | Cing Gyr Control2 | 20.2 |
| BA9 Depression2 | 4.5 | Cing Gyr Alzheimer's | 8.6 |
| BA17 Control | 20.2 | Cing Gyr Alzheimer's2 | 4.2 |
| BA17 Control2 | 7.9 | Cing Gyr Parldnson's | 12.2 |
| BA17 Alzheimer's2 | 3.2 | Cing Gyr Parldnson's2 | 15.3 |
| BA17 Parkioson's | 14.1 | Cing Gyr Huntington's | 28.1 |
| BA17 Parkinson's2 | 8.7 | Cing Gyr Huntmgton's2 | 4.7 |
| BA17 Huntington's | 22.2 | Cing Gyr PSP | 7.8 |
| BA17 Huntington's2 | 18.2 | Cing Gyr PSP2 | 11.2 |
| BA17 Depression | 4.9 | Cing Gyr Depression | 2.9 |
| BA17 Depression2 | 19.9 | Cing Gyr Depression2 | 8.7 |

**CNS_neurodegeneration_v1.0 Summary:** Ag3234/Ag3279 Two experiments with the same probe and primer set produce results that are in excellent agreement. Both experiments show a difference in expression of the CG57429-01 gene between Alzheimer's diseased postmortem brains and controls for this gene. Expression is increased in the temporal cortex of patients with AD (p = 0.016 for ag3234 and p = 0.024 for ag3279) and in the hippocampus. Both the temporal cortex and hippocampus are regions that show severe neurodegeneration in AD. In contrast, expression in the occipital cortex, a region that does not degenerate in Alzheimer's disease, is not disregulated. Together, these data suggest that the CG57429-01 protein product may be involved in the pathology or response to Alzheimer's disease. Therefore, this may be a useful drug target for the treatment of this disease.

**General_screening_panel_v1.4 Summary:** Ag3279 Highest expression of the CG57429-01 gene is in the cerebellum. Significant levels of expression are also seen in other regions of the brain including the amygdala, hippocampus, cerebral cortex, substantia nigra, and thalamus. Cadherins can act as axon guidance and cell adhesion proteins, specifically during development and in the response to injury (ref 1). Manipulation of levels of this protein may be of use in inducing a compensatory synaptogenic response to neuronal death in Alzheimer's disease, Parkinson's disease, Huntington's disease, spinocerebellar ataxia, progressive supranuclear palsy, ALS, head trauma, stroke, or any other disease/condition associated with neuronal loss.

In addition, this gene is highly expressed in pituitary gland, adrenal gland, thyroid, pancreas, adult and fetal skeletal muscle, heart and liver, reflecting the widespread role of cadherins in cell-cell adhesion. This observation may suggest that this gene plays a role in normal metabolic and neuroendocrine function and that disregulated expression of this gene may contribute to metabolic diseases (such as obesity and diabetes) or neuroendocrine disorders.

Overall, gene expression is associated with normal tissues rather than cancer cell lines. Loss of function of the related E-cadherin protein has been described in many tumors, along with an increased invasiveness and a decreased prognosis of many carcinomas, including tumors of endocrine glands and their target systems (ref 1). Thus, this gene product might similarly be useful as a protein therapeutic to treat a variety of tumors, since it is found in normal cells but missing from cancer cells (Potter et al., Endocr. Rev. 20: 207-239, 1999; Ranscht, Int. J. Dev. Neurosci. 18: 643-651, 2000).

**Panel 1.1 and 1.2 Summary: Ag616 Highest expression of the CG57429-01 gene, a** cadherin homolog, is seen in pancreas (CT=23.2). Significant expression is also seen in adrenal gland, fetal and adult skeletal muscle, liver and heart. This widespread expression among tissues with metabolic function is consistent with expression seen in General_screening_panel_v1.4. Please see that panel for further discussion of utility of this gene in metabolic disorders.

In addition, there is higher expression in adult liver (CT=27) when compared to experession in fetal liver (CT=34.8). Thus, expression of this gene could be used to differentiate between fetal and adult liver.

Overall, expression in this panel is in agreement with expression in the previous panel. Please see that panel for further discussion of utility of this gene.

**Panel 1.3D Summary:** Ag3234 Results from this experiment produce a similar profile to Panel 1.4. Please see that Panel for further discussion of expression and utility of this gene.

**Panel 2.2 Summary:** Ag3234 The expression of the CG57429-01 gene appears to be highest in a sample derived from a normal ovarian tissue (CT=32.3). In addition, there appears to be substantial expression in other samples derived from liver cancers. Furthermore, there appears to be expression specific to normal lung tissue when compared to malignant lung tissue. Thus, the expression of this gene could be used to distinguish normal ovarian tissue from other samples in the panel. Moreover, therapeutic modulation of this gene, through the use of small molecule drugs, protein therapeutics or antibodies could be of benefit in the treatment of liver cancer, ovarian cancer or lung cancer.

**Panel 4D Summary:** Ag3234/Ag3279 The CG57429-01 gene, a cadherin 23-like molecule, is expressed selectively at moderate levels (CTs=28.1-30.1) in resting and activated dendritic cells, and in resting and activated macrophages. Thus, small molecule antagonists or therapeutic antibodies that block the function of the CG57429-01 gene product may be useful in the reduction or elimination of the symptoms in patients with autoimmune and inflammatory diseases in which dendritic cells and macrophages play an important role in antigen presentation and other functions, such as, but not limited to, including Crohn's disease, ulcerative colitis, multiple sclerosis, chronic obstructive pulmonary disease, asthma, emphysema, rheumatoid arthritis, lupus erythematosus, or psoriasis.

**Panel CNS_1 Summary:** Ag3279 This panel confirms expression of the CG57429-01 gene in the brain. Please see Panel 1.3D for discussion of utility of this gene in the central nervous system. Results from a second experiment with Ag3234 are not included. The amp plot indicates that there were experimental difficulties with this run.

### R. NOV21: Platelet Glycoprotein V

Expression of gene CG57436-01 was assessed using the primer-probe set Ag3236, described in Table SA. Results of the RTQ-PCR runs are shown in Tables RB, RC, RD and RE.

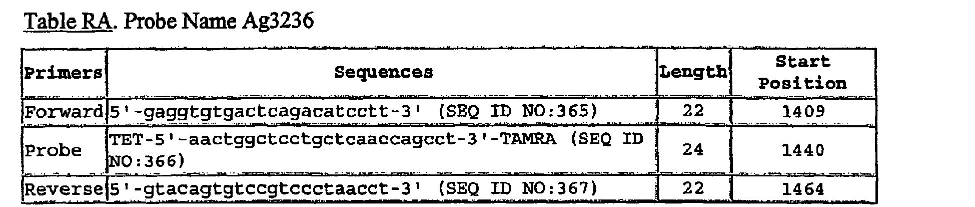

**Table RB. General_screening_panel_v1.4**

| **Tissue Name** | **Rel. Exp.(%) Ag3236, Run 214693630** | **Tissue Name** | **Rel. Exp.(%) Ag3236, Run 214693630** |
|---|---|---|---|
| Adipose | 0.5 | Renal ca. TK-10 | 0.0 |
| Melanoma* Hs688(A).T | 38.2 | Bladder | 0.6 |
| Melanoma* Hs688(B).T | 9.4 | Gastric ca. (liver met.) NCI-N87 | 0.0 |
| Melanoma* M14 | 0.0 | Gastric ca. KATO III | 0.0 |
| Melanoma* LOXIMVI | 0.7 | Colon ca. SW-948 | 0.0 |
| Melanoma* SK-MEL-5 | 0.0 | Colon ca. SW480 | 0.0 |
| Squamouscell carcinoma SCC-4 | 0.0 | Colon ca.* (SW480 met) SW620 0.0 | 0.0 |
| Testis Pool | 0.0 | Colon ca. HT29 | 0.0 |
| Prostate ca.* (bone met) PC-3 | 0.0 | Colon ca. HCT-116 | 0.0 |
| Prostate Pool | 0.1 | Colon ca. CaCo-2 | 0.0 |
| Placenta | 0.6 | Colon cancer tissue | 1.9 |
| Uterus Pool | 0.2 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.3 |
| Ovarian ca. OVCAR-5 | 0.1 | Small Intestine Pool | 0.0 |
| Ovarian ca. IGROV-1 | 0.0 | Stomach Pool | 0.2 |
| Ovarian ca. OVCAR-8 | 0.0 | Bone Marrow Pool | 0.3 |
| Ovary | 0.2 | Fetal Heart | 0.0 |
| Breast ca. MCF-7 | 0.0 | Heart Pool | 0.1 |
| Breast ca. MDA-MB-231 | 0.5 | Lymph Node Pool | 0.2 |
| Breast ca. BT 549 | 0.0 | Fetal Skeletal Muscle | 0.1 |
| Breast ca. T47D | 0.3 | Skeletal Muscle Pool | 0.1 |
| Breast ca. MDA-N | 0.0 | Spleen Pool | 0.1 |
| Breast Pool | 0.2 | Thymus Pool | 0.0 |
| Trachea | 0.2 | U87-MG U87-MG | 14.8 |
| Lung | 0.0 | CNS cancer (glio/astro) U-118-MG | **100.0** |
| Fetal Lung | 0.0 | CNS cancer (neuro;met) SK-N-AS | 0.0 |
| Lung ca. NCI-N417 | 0.0 | CNS cancer (astro) SF-539 | 13.2 |
| Lung ca. LX-1 | 0.0 | CNS cancer (astro) SNB-75 | 5.3 |
| Lung ca. NCI-H146 | 0.0 | CNS cancer (glio) SNB-19 | 0.0 |
| Lung ca. SHP-77 | 0.0 | CNS cancer (glio) SF-295 | 0.3 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 0.0 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.2 | Adrenal Gland | 0.1 |
| Fetal Kidney | 0.0 | Pituitary gland Pool | 0.0 |
| Renal ca. 786-0 | 0.1 | Salivary Gland | 0.0 |
| Renal ca. A498 | 0.1 | Thyroid (female) | 0.6 |
| Renal ca. ACHN | 0.0 | Pancreatic ca. CAPAN2 | 0.0 |
| Renal ca. UO-31 | 0.4 | Pancreas Pool | 0.1 |

**Table RC. Panel 2.2**

| **Tissue Name** | **Rel. Exp.(%) Ag3236, Run 174442924** | **Tissue Name** | **Rel. Exp.(%) Ag3236, Run 174442924** |
|---|---|---|---|
| Normal Colon | 0.2 | Kidney Margin (OD04348) | 0.5 |
| Colon cancer (OD06064) | 7.3 | Kidney malignant cancer (OD06204B) | 0.8 |
| Colon Margin (OD06064) | 0.2 | Kidney normal adjacent tissue (OD06204E) | 0.0 |
| Colon cancer (OD06159) | 0.3 | Kidney Cancer (OD04450-01) | 0.0 |
| Colon Margin (OD06159) | 0.6 | Kidney Margin (OD04450-03) | 0.2 |
| Colon cancer (OD06297-04) | 1.7 | Kidney Cancer 8120613 | 0.0 |
| Colon Margin (OD06297-05) | 2.4 | Kidney Margin 8120614 | 0.0 |
| CC Gr.2 ascend colon (OD03921) | 1.6 | Kidney Cancer 9010320 | 1.1 |
| CC Margin (OD03921) | 1.9 | Kidney Margin 9010321 | 1.1 |
| Colon cancer metastasis (OD06104) | 0.3 | Kidney Cancer 8120607 | 4.7 |
| Lung Margin (OD06104) | 0.6 | Kidney Margin 8120608 | 0.2 |
| Colon mets to lung (OD04451-01) | 0.6 | Normal Uterus | 1.4 |
| Lung Margin (OD04451-02) | 0.3 | Uterine Cancer 064011 | 0.0 |
| Normal Prostate | 0.0 | Normal Thyroid | 0.5 |
| Prostate Cancer (OD04410) | 0.3 | Thyroid Cancer 064010 | 1.0 |
| Prostate Margin (OD04410) | 0.0 | Thyroid Cancer A302152 | 11.9 |
| Normal Ovary | 2.7 | Thyroid Margin A302153 | 0.0 |
| Ovarian cancer (OD06283-03) | 13.9 | Normal Breast | 0.7 |
| Ovarian Margin (OD06283-07) | 1.0 | Breast Cancer (OD04566) | 3.7 |
| Ovarian Cancer 064008 | 27.0 | Breast Cancer 1024 | 3.4 |
| Ovarian cancer (OD06145) | 0.7 | Breast Cancer (OD04590-Breast Cancer (OD04590-01) | 8.4 |
| Ovarian Margin (OD06145) | 1.1 | Breast Cancer Mets (OD04590-03) | 9.9 |
| Ovarian cancer (OD06455-03) | 0.0 | Breast Cancer Metastasis (OD04655-05) | 0.5 |
| Ovarian Margin (OD06455-07) | 0.0 | Breast Cancer 064006 | 31.2 |
| Normal Lung | 0.0 | Breast Cancer 9100266 | 14.3 |
| Invasive poor diff. lung adeno (ODO4945-01 | 4.6 | Breast Margin 9100265 | 19.1 |
| Lung Margin (ODO4945-03) | 2.1 | Breast Cancer A209073 | 5.8 |
| Lung Malignant Cancer (OD03126) | 6.4 | Breast Margin A2090734 | 0.4 |
| Lung Margin (OD03126) | 0.5 | Breast cancer (OD06083) | 15.7 |
| Lung Cancer (OD05014A) | 0.5 | Breast cancer node metastasis (OD06083) | **100.0** |
| Lung Margin (OD05014B) | 0.0 | Normal Liver | 0.2 |
| Lung cancer (OD06081) | 0.8 | Liver Cancer 1026 | 0.3 |
| Lung Margin (OD06081) | 0.0 | Liver Cancer 1025 | 1.9 |
| Lung Cancer (OD04237-01) | 0.8 | Liver Cancer 6004-T | 0.0 |
| Lung Margin (OD04237-02) | 8.5 | Liver Tissue 6004-N | 0.0 |
| Ocular Melanoma Metastasis | 0.0 | Liver Cancer 6005-T | 0.8 |
| Ocular Melanoma Margin (Liver) | 0.0 | Liver Tissue 6005-N | 0.0 |
| Melanoma Metastasis | 0.0 | Liver Cancer 064003 | 0.0 |
| Melanoma Margin (Lung) | 1.9 | Normal Bladder | 1.8 |
| Normal Kidney | 0.0 | Bladder Cancer 1023 | 8.4 |
| Kidney Ca, Nuclear grade 2 (OD04338) | 0.2 | Bladder Cancer A302173 | 6.7 |
| Kidney Margin (OD04338) | 0.0 | Normal Stomach | 1.6 |
| Kidney Ca Nuclear grade 1/2 (OD04339) | 0.0 | Gastric Cancer 9060397 | 2.4 |
| Kidney Margm (OD04339) | 0.0 | Stomach Margin 9060396 | 0.8 |
| Kidney Ca, Clear cell type (OD04340) | 0.0 | Gastric Cancer 9060395 | 0.3 |
| Kidney Margin (OD04340) | 0.0 | Stomach Margin 9060394 | 0.9 |
| Kidney Ca, Nuclear grade 3 (OD04348) | 0.0 | Gastric Cancer 064005 | 0.0 |

**Table RD. Panel 3D**

| **Tissue Name** | **Rel. Exp.(%) Ag3236, Run 182114365** | **Tissue Name** | **Rel. Exp.(%) Ag3236, Run 182114365** |
|---|---|---|---|
| Daoy- Medulloblastoma | 0.6 | Ca Ski- Cervical epidermoid carcinoma (metastasis) | 0.0 |
| TE671- Medulloblastoma | 0.0 | ES-2- Ovarian clear cell carcinoma | 0.0 |
| D283 Med- Medulloblastoma | 0.0 | Ramos- Stimulated with PMA/ionomycin 6h | 0.0 |
| PFSK-1-Primitive Neuroectodermal | 0.2 | Ramos-Stimulated with PMA/ionomycin 14h | 0.0 |
| XF-498-CNS | **100.0** | MEG-01- Chronic myelogenous leukemia (megokaryoblast) | 0.0 |
| SNB-78-Glioma | 32.5 | Raji- Burkitt's lymphoma | 0.0 |
| SF-268- Glioblastoma | 0.3 | Daudi- Buridtes lymphoma | 0.0 |
| T98G- Glioblastoma | 0.2 | U266- B-cell plasmacytoma | 0.2 |
| SK-N-SH- Neuroblastoma (metastasis) | 0.0 | CA46- Burkitt's lymphoma | 0.0 |
| SF-295- Glioblastoma | 6.3 | RL-non-Hodgkin's B-cell lymphoma | 0.0 |
| Cerebellum | 0.0 | JM1-pre-B-cell lymphoma | 0.0 |
| Cerebellum | 0.0 | Jurkat- T cell leukemia | 0.0 |
| NCI-H292- Mucoepidermoid lung carcinoma | 0.0 | TF-1-Erythroleukemia | 0.2 |
| DMS-114-Small cell lung | 0.0 | HUT 78-T-cell lymphoma | 0.2 |
| DMS-79- Small cell lung cancer | 0.0 | U937- Histiocytic lymphoma | 0.0 |
| NCI-H146- Small cell lung cancer | 0.0 | KU-812- Myelogenous leukemia | 0.0 |
| NCT-HS26- Sman cell lung cancer | 0.0 | 769-P- Clear cell renal carcinoma | 0.0 |
| NCI-N417- Small cell lung cancer | 0.0 | Caki-2- Clear cell renal carcinoma | 0.0 |
| NCI-H82- Small cell lung cancer | 0.0 | SW 839- Clear cell renal carcinoma | 0.0 |
| NCI-H157-Squamous cell lung cancer (metastasis) | 0.0 | G401- Wilms' tumor | 0.5 |
| NCI-H1155-Large cell lung cancer | 0.0 | Hs766T-Pancreatic carcinoma (LN metastasis) | 0.7 |
| NCI-H1299-Large cell lung cancer | 0.0 | CAPAN-1- Pancreatic adenocarcinoma (liver metastasis) | 0.0 |
| NCI-H727-Lung carcinoid | 0.0 | SU86.86- Pancreatic carcinoma (liver metastasis) | 0.2 |
| NCI-UMC-11-Lung carcinoid | 0.0 | BxPC-3-Pancreatic adenocarcinoma | 0.0 |
| LX-1- Small cell lung cancer | 0.0 | HPAC-Pancreatic adenocarcinoma | 0.0 |
| Colo-205- Colon cancer | 0.0 | MIA PaCa-2- Pancreatic carcinoma | 0.0 |
| KM12-Colon cancer | 0.0 | CFPAC-1- Pancreatic ductal adenocarcinoma | 0.0 |
| KM20L2-Colon cancer | 0.0 | PANC-1-Pancreatic epithelioid ductal carcinoma | 1.6 |
| NCI-H716-Colon cancer | 0.0 | T24- Bladder carcinma (transitional cell) | 0.0 |
| SW-48-Colon adenocarcinoma | 0.0 | 5637- Bladder carcinoma | 0.0 |
| SW1116- Colon adenocarcinoma | 0.0 | HT-1197-Bladder carcmoma | 0.1 |
| LS 174T- Colon adenocarcinoma | 0.0 | UM-UC-3- Bladder carcinma (transitional cell) | 0.0 |
| SW-948- Colon adenocarcinoma | 0.0 | A204- Rhabdomyosarcoma | 0.9 |
| SW-480-Colon adenocarcinoma | 0.0 | HT-1080-Fibrosarcoma | 0.3 |
| NCI-SNU-5- Gastric carcinoma | 0.0 | MG-63- Osteosarcoma | 0.0 |
| KATO III- Gastric carcinoma | 0.0 | SK-LMS-1- Leiomyosarcoma (vulva) | 0.0 |
| NCI-SNU-16- Gastric carcinoma | 4.4 | SJRH30-Rhabdomyosarcoma (met to bone marrow) | 0.0 |
| NCI-SNU-1- Gastric carcinoma | 0.0 | A431-Epidermoid carcinoma | 0.0 |
| RF-1-Gastric adenocarcinoma | 0.0 | WM266-4- Melanoma | 0.7 |
| RF-48- Gastric adenocarcinoma | 0.0 | DU 145- Prostate carcinoma (brain metastasis) | 0.0 |
| MKN-45- Gastric carcinoma | 0.0 | MDA-MB-468- Breast adenocarcinoma | 0.0 |
| NCI-N87- Gastric carcinoma | 0.0 | SCC-4- Squamous cell carcinoma of tongue | 0.0 |
| OVCAR-5- Ovarian carcinoma | 0.0 | SCC-9- Squamous cell carcinoma of tongue | 0.0 |
| RL95-2- Uterine carcinoma | 0.0 | SCC-15-Squamous cell carcinoma of tongue | 0.0 |
| HelaS3- Cervical adenocarcinoma | 0.0 | CAL 27- Squamous cell carcinoma of tongue | 0.0 |

**Table RE. Panel 4D**

| **Tissue Name** | **Rel. Exp.(%) Ag3236, Run 164328488** | **Tissue Name** | **Rel. Exp.(%) Ag3236, Run 164328488** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.7 | Lung Microvascular EC none | 0.0 |
| Primary Th1 act | 0.0 | Lung Microvascular EC TNFalpha + IL-1beta | 0.6 0.6 |
| Primary Th2 act | 0.0 | Microvascular Dermal EC none | 0.6 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 1.1 |
| Primary Th2 rest | 0.0 | Small airway epithelium none | 3.2 |
| Primary Tr1 rest | 0.0 | Small airway epithelimn TNFalpha + IL-1beta | 0.0 |
| CD45RA CD4 lymphocyte act | 4.6 | Coronery artery SMC rest | 41.5 |
| CD45RO CD4 lymphocyte act | 1.2 | Coronery artery SMC TNFalpha + IL-1beta | 54.3 |
| CD8 lymphocyte act | 0.0 | Astrocytes rest | 17.1 |
| Secondary CD8 lymphocyte rest | 0.0 | Astrocytes TNFalpha + IL-1beta | 17.4 |
| Secondary CD9 | 0.0 | KU-812 (Basophil) rest | 0.5 |
| lymphocyte act | | | |
| CD4 lymphocyte none | 0.0 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.7 |
| LAK cells IL-2 | 0.0 | Liver cirrhosis | 5.2 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2+ IL-18 | 0.0 | NCI-H292 IL-4 | 0.7 |
| LAK cells PMA/ionomycin | 0.0 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 0.0 | HPAEC none | 0.0 |
| Two Way MLR 7 day | 0.0 | HPAEC TNF alpha + IL-1 beta | 1.7 |
| PBMC rest | 0.0 | Lung fibroblast none | 15.9 |
| PBMC PWM | 0.0 | Lung fibroblast TNF alpha + IL-1 beta | **100.0** |
| PBMC PHA-L | 0.0 | Lung fibroblast IL-4 | 24.1 |
| Ramos (B cell) none | 0.0 | Lung fibroblast IL-9 | 26.6 |
| Ramos (B cell) ionomycin | 0.0 | Lung fibroblast IL-13 | 6.6 |
| B lymphocytes PWM | 0.0 | Lung fibroblast IFN gamma | 28.3 |
| B lymphocytes CD40L and IL-4 | 0.7 | Dermal fibroblast CCD1070 rest | 46.3 |
| EOL-1 dbcAMP | 0.0 | Dermal fibroblast CCD1070 TNF alpha | 34.6 |
| EOL-1 dbcAMP PMA/ionomycin | 0.0 | Dermal fibroblast CCD1070 IL-1 beta | 74.2 |
| Dendritic cells none | 0.0 | Dermal fibroblast IFN gamma | 81.2 |
| Dendritic cells LPS | 0.0 | Dermal fibroblast IL-4 | 82.9 |
| Dendritic cells anti-CD40 | 0.0 | IBD Colitis 2 | 0.7 |
| Monocytes rest | 0.0 | IBD Crohn's | 1.3 |
| Monocytes LPS | 0.0 | Colon | 5.0 |
| Macrophages rest | 0.0 | Lung | 35.4 |
| Macrophages LPS | 0.0 | Thymus | 0.0 |
| HUVEC none | 0.0 | Kidney | 4.2 |
| HUVEC starved | 0.0 | | |

**CNS_neurodegeneration_v1.0 Summary:** Ag3236 Expression of the CG57436-01 is low/undetectable in all samples on this panel (CTs>35). (Data not shown.)

**General_screening_panel_v1.4 Summary:** Ag3236 Highest expression of the CG57436-01 gene is seen in a.brain cancer cell line (CT=23.6). Significant levels of expression are also seen in a cluster of cell lines derived from brain cancer and melanoma. Thus, expression of this gene could be used to differentiate between these samples and other samples on this panel and as a diagnostic marker for the presence of these cancers. Furthermore, therapeutic modulation of the expression or function of this gene or gene product may be effective in the treatment of brain cancer or melanoma.

Among tissues with metabolic function, this gene is expressed at moderate/low levels in pancreas, adrenal gland, adipose, adult and fetal skeletal muscle and adult heart. This widespread expression suggests that this gene may play a role in normal metabolic function and that dysregulated expression of this gene may contribute to metabolic diseases, such as obesity and diabetes.

**Panel 2.2 Summary**: Ag3236 Highest expression of the CG57436-01 gene is seen in a breast cancer metastasis (CT=29). Significant expression is also seen in a cluster fo breast cancer samples. This is in concordance with the expression in General_screening_panel_v1.4, where expression is predominantly in cancer cell lines. Thus, expression of this gene could be used to differentiate between these sample and other samples on this panel and as a marker to detect the presence of breast cancer. Furthermore, therapeutic modulation of the expression or function of this gene may be effective in the treatment of breast cancer.

**Panel 3D Summary:** Ag3236 Highest expression of the CG57436-01 gene is seen in brain cancer cell lines, consistent with General_screening_panel_v1.4. Thus, expression of this gene could be used to differentiate between this sample and other samples on this panel.

**Panel 4D Summary:** Ag3236 Highest expression of the CG57436-01 gene is seen in a cluster of treated and untreated samples derived from lung and dermal fibroblasts, with highest expression in TNF-alpha and IL-1 beta treated lung fibroblasts (CT=29.8). Therefore, therapeutic antibody antagonists that block the binding of von Willebrand factor or other proteins to the CG57436-01 gene product may be useful in the treatment of disorders that involve activation of the coagulation cascade or binding by other ligands, including clotting disorders, atherosclerosis, cardiovascular disease, and autoimmune and inflammatory diseases, such as Crohn's disease, ulcerative colitis, multiple sclerosis, chronic obstructive pulmonary disease, asthma, emphysema, rheumatoid arthritis, lupus erythematosus, or psoriasis.

### S. NOV22: GARPIN-like

Expression of gene CG57529-01 was assessed using the primer-probe set Ag3276, described in Table SA. Results of the RTQ-PCR runs are shown in Tables SB, SC and SD.

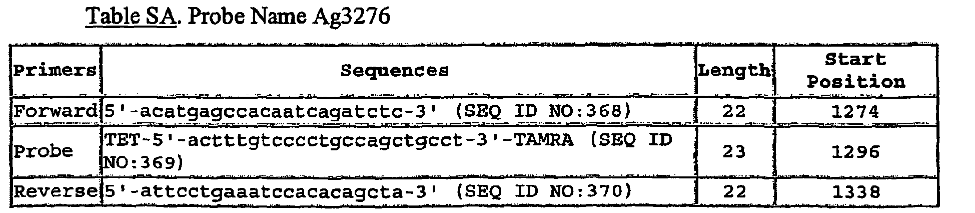

**Table SB. CNS_neurodegeneration_v1.0**

| **Tissue Name** | **Rel. Exp.(%) Ag3276, Run 206533680** | **Tissue Name** | **Rel. Exp.(%) Ag3276, Run 206533680** |
|---|---|---|---|
| AD 1 Hippo | 27.4 | Control (Path) 3 Temporal Ctx | 6.7 |
| AD 2 Hippo | 57.0 | Control (Path) 4 Temporal Ctx | 18.9 |
| AD 3 Hippo | 4.2 | AD 1 Occipital Ctx | 20.7 |
| AD 4 Hippo | 4.4 | AD 2 Occipital Ctx (Missing) | 0.0 |
| AD 5 hippo | 88.3 | AD 3 Occipital Ctx | 12.4 |
| AD 6 Hippo | 97.9 | AD 4 Occipital Ctx | 8.7 |
| Control 2 Hippo | 38.4 | AD 5 Occipital Ctx | 10.5 |
| Control 4 Hippo | 42.9 | AD 6 Occipital Ctx | 46.3 |
| Control (Path) 3 Hippo | 2.8 | Control 1 Occipital Ctx | 5.5 |
| AD 1 Temporal Ctx | 6.3 | Control 2 Occipital Ctx | 48.0 |
| AD 2 Temporal Ctx | 27.2 | Control 3 Occipital Ctx | 9.8 |
| AD 3 Temporal Ctx | 6.4 | Control 4 Occipital Ctx | 8.8 |
| AD 4 Temporal Ctx | 15.5 | Control (Path) 1 Occipital Ctx | 66.9 |
| AD 5 Inf Temporal Ctx | 66.0 | Control (Path) 2 Occipital Ctx | 6.0 |
| AD 5 SupTemporal Ctx | **100.0** | Control (Path) 3 Occipital Ctx | 2.6 |
| AD 6 Inf Temporal Ctx | 98.6 | Control (Path) 4 Occipital Ctx | 24.7 |
| AD 6 Sup Temporal Ctx | 99.3 | Control 1 Parietal Ctx | 6.4 |
| Control 1 Temporal Ctx | 6.7 | Control 2 Parietal Ctx | 33.9 |
| Control 2 Temporal Ctx | 29.3 | Control 3 Parietal Ctx | 14.3 |
| Control 3 Temporal Ctx | 5.3 | Control (Path) 1 Parietal Ctx | 60.7 |
| Control 4 Temporal Ctx | 14.9 | Control (Path) 2 Parietal Ctx | 21.6 |
| Control (Path) 1 Temporal Ctx | 53.2 | Control (Path) 3 Parietal Ctx | 2.4 |
| Control (Path) 2 Temporal Ctx | 24.0 | Control (Path) 4 Parietal Ctx | 32.1 |

**Table SC. Panel 1.3D**

| **Tissue Name** | **Rel. Exp.(%) Ag3276, Run 165524215** | **Tissue Name** | **Rel. Exp.(%) Ag3276, Run 165524215** |
|---|---|---|---|
| Liver adenocarcinoma | 0.5 | Kidney (fetal) | 2.6 |
| Pancreas | 1.6 | Renal ca. 786-0 | 0.0 |
| Pancreatic ca. CAPAN 2 | 0.4 | Renal ca. A498 | 0.3 |
| Adrenal gland | 9.0 | Renal ca. RXF 393 | 0.0 |
| Thyroid | 6.0 | Renal ca. ACHN | 0.4 |
| Salivary gland | 4.3 | Renal ca. UO-31 | 0.4 |
| Pituitary gland | 4.2 | Renal ca. TK-10 | 0.7 |
| Brain (fetal) | 1.7 | Liver | 4.0 |
| Brain (whole) | 8.5 | Liver (fetal) | 25.7 |
| Brain (amygdala) | 9.8 | Liver ca. (hepatoblast) HepG2 | 0.0 |
| Brain (cerebellum) | 7.3 | Lung | 15.2 |
| Brain (hippocampus) | 8.8 | Lung (fetal) | 7.3 |
| Brain (substantia nigra) | 7.3 | Lung ca. (small cell) LX-1 | 0.4 |
| Brain (thalamus) | 5.3 | Lung ca. (small cell) NCI-H69 | 0.0 |
| Cerebral Cortex | 4.0 | Lung ca. (s.cell var.) SHP-77 | 3.5 |
| Spinal cord | 16.6 | Lung ca. (large cell)NCI-H460 | 1.3 |
| glio/astro U87-MG | 9.0 | Lung ca. (non-sm. cell) A549 | 0.5 |
| glio/astro U-118-MG | 7.7 | Lung ca. (non-s.cell) NCI-H23 | 1.4 |
| astrocytoma SW1783 | 0.7 | Lung ca. (non-s.cell) HOP-62 | 0.2 |
| neuro*; met SK-N-AS | 0.7 | Lung ca. (non-s.cl) NCI-H522 | 0.0 |
| astrocytoma SF-539 | 3.1 | Lung ca. (squam.) SW 900 | 4.4 |
| astrocytoma SNB-75 | 1.8 | Lung ca. (squam.) NCI-H596 | 0.0 |
| glioma SNB-19 | 0.8 | Mammary gland | 17.4 |
| glioma U251 | 1.6 | Breast ca.* (pl.ef) MCF-7 | 0.0 |
| glioma SF-295 | 0.0 | Breast ca.* (pl.ef) MDA-MB-231 | 4.5 |
| Heart (fetal) | 3.8 | Breast ca.* (pl.ef) T47D | 0.0 |
| Heart | 5.2 | Breast ca. BT-549 | 15.7 |
| Skeletal muscle (fetal) | 7.7 | Breast ca. MDA-N | 21.6 |
| Skeletal muscle | 2.9 | Ovary | 5.7 |
| Bone marrow | 33.9 | Ovarian ca. OVCAR-3 | 2.2 |
| Thymus | 7.3 | Ovarian ca. OVCAR-4 | 0.0 |
| Spleen | 42.6 | Ovarian ca. OVCAR-5 | 0.8 |
| Lymph node | 37.4 | Ovarian ca. OVCAR-8 | 0.0 |
| Colorectal | 1.1 | Ovarian ca. IGROV-1 | 1.7 |
| Stomach | 7.5 | Ovarian ca.* (ascites) SK-OV-3 | 0.4 |
| Small intestine | 18.9 | Uterus | 14.3 |
| Colon ca. SW480 | 1.5 | Placenta | 8.6 |
| Colon ca.* SW620(SW480 met) | 1.5 | Prostate | 4.2 |
| Colon ca. H129 | 0.4 | Prostate ca.* (bone met)PC-3 | 0.4 |
| Colon ca. HCT-116 | 0.0 | Testis | 3.1 |
| Colon ca. CaCo-2 | 1.2 | Melanoma Hs688(A).T | 0.4 |
| Colon ca. tissue(ODO3866) | 4.7 | Melanoma* (met) Hs688(B).T | 0.0 |
| Colon ca. HCC-2998 | 0.6 | Melanoma UACC-62 | 25.0 |
| Gastric ca.* (liver met) NCI-N87 | 0.8 | Melanoma M14 | **100.0** |
| Bladder | 4.0 | Melanoma LOX IMVI | 0.0 |
| Trachea | 5.5 | Melanoma* (met) SK-MEL-5 | 4.4 |
| Kidney | 1.8 | Adipose | 5.0 |

**Table SD. Panel 4D**

| **Tissue Name** | **Rel. Exp.(%) Ag3276, Run 164635082** | **Tissue Name** | **Rel. Exp.(%) Ag3276, Run 164635082** |
|---|---|---|---|
| Secondary Th1 act | 34.6 | HUVEC IL-1beta | 1.9 |
| Secondary Th2 act | 26.2 | HUVEC IFN gamma | 5.3 |
| Secondary Tr1 act | 23.3 | HUVEC TNF alpha + IFN gamma | 0.7 |
| Secondary Th1 rest | 4.9 | HUVEC TNF alpha + IL4 | 0.9 |
| Secondary Th2 rest | 12.0 | HUVEC IL-11 | 3.8 |
| Secondary Tr1 rest | 15.3 | Lung Microvascular EC none | 2.3 |
| Primary Th1 act | 18.9 | Lung Microvascular EC INFalpha + IL-1beta | 0.2 |
| Primary Th2 act | 17.0 | Microvascular Dermal EC none | 1.4 |
| Primary Tr1 act | 27.0 | Microsvasular Dermal EC TNFapha + IL-1beta | 0.5 |
| Primary Th1 rest | 67.8 | Bronchial epithelium TNFalpha + IL1beta | 0.4 |
| Primary Th2 rest | 40.9 | Small airway epithelium none | 0.2 |
| Primary Tr1 rest | 18.8 | Small airway epithelium TNFalpha + IL-1beta | 0.0 |
| CD45RA CD4 lymphocyte act | 12.7 | Coronery artery SMC rest | 0.2 |
| CD45RO CD4 lymphocyte act | 26.1 | Coronery artery SMC TNFalpha + IL-1beta | 0.0 |
| CD8 lymphocyte act | 18.0 | Astrocytes rest | 0.0 |
| Secondary CD8 lymphocyte rest | 19.2 | Astrocytes TNFalpha + IL-1beta | 0.1 |
| Secondary CD8 lymphocyte act | 21.9 | KU-812 (Basophil) rest | 34.2 |
| CD4 lymphocyte none | 2.6 | KU-812 (Basophil) PMA/ionomycin | 66.0 |
| 2ry Th1/Th2/Tr1_anti-CD95 CH11 | 10.2 | CCD 1106 (Keratinocytes) none | 0.3 |
| LAK cells rest | 62.9 | CCD1106 (Keratinocytes) TNFalpha + IL-1beta | 0.2 |
| LAK cells IL-2 | 30.8 | Liver cirrhosis | 1.1 |
| LAK cells IL-2+IL-12 | 20.2 | Lupus kidney | 0.6 |
| LAK cells EL-2+IFN gamma | 30.1 | NCI-H292 none | 0.1 |
| LAK cells IL-2+IL-18 | 18.2 | NCI-H292 IL-4 | 0.0 |
| LAK cells PMA/ionomycin | 12.9 | NCI-H292 IL-9 | 0.1 |
| NK Cells IL-2 rest | 24.3 | NCI-H292 IL-13 | 0.2 |
| Two Way MLR 3 day | 40.3 | NCI-H292 IFN gamma | 0.0 |
| Two Way MLR 5 day | 21.9 | HPAEC none | 2.7 |
| Two Way MLR 7 day | 17.8 | HPAEC TNF alpha + IL-1 beta | 0.6 |
| PBMC rest | 13.7 | Lung fibroblast none | 0.3 |
| PBMC PWM | 46.7 | Lung fibroblast TNF alpha + IL-1 beta | 1.3 |
| PBMC PHA-L | 22.1 | Lung fibroblast IL-4 | 1.4 |
| Ramos (B cell) none | 0.5 | Lung fibroblast IL-9 | 1.5 |
| Ramos (B cell) ionomycin | 5.4 | Lung fibroblast IL-13 | 1.5 |
| B lymphocytes PWM | 50.3 | Lung fibroblast IFN gamma | 1.8 |
| B lymphocytes CD40L and IL-4 | 7.4 | Dermal fibroblast CCD1070 rest | 0.3 |
| EOL-1 dbcAMP EOL-1 dbcAMP | 46.0 | Dermal fibroblast CCD1070 TNF alpha | 45.7 |
| EOL-1 dbcAMP PMA/ionomycin | 34.4 | Dermal fibroblast CCD1070 IL-1 beta | 0.0 |
| Dendritic cells none | 76.3 | Dermal fibroblast IFN gamma | 0.8 |
| Dendritic cells LPS | 30.6 | Dermal fibroblast IL-4 | 0.8 |
| Dendritic cells anti-CD40 | 69.7 | IBD Colitis 2 | 0.3 |
| Monocytes rest | 60.7 | IBD Crohn's | 0.2 |
| Monocytes LPS | 20.6 | Colon | 2.9 |
| Macrophages rest | 100.0 | Lung | 7.6 |
| Macrophages LPS | 34.9 | Thymus | 1.0 |
| HUVEC none | 3.6 | Kidney | 10.8 |
| HUVEC starved | 7.8 | | |

**CNS_neurodegeneration_v1.0 Summary:** Ag3276 This panel does not show differential expression of the CG57529-01 gene in Alzheimer's disease. However, this expression profile confirms the presence of this gene in the brain. Please see Panel 1.3D for discussion of utility of this gene in the central nervous system.

**Panel 1.3D Summary:** Ag3276 Highest expression of the CG57529-01 gene is seen in a melanoma cell line (CT=28.5). Significant expression is also seen in a cluster of breast cancer and melanoma cell lines. Thus, expression of this gene could be used to differentiate between these samples and other samples on this panel and as a diagnostic marker for the presence of these cancers. Furthermore, therapeutic modulation of the expression or function of this gene may be effective in the treatment of breast cancer or melanoma.

This gene encodes a type 1 membrane protein with several leucine-rich-repeat domains, indicating that this gene product may be involved in extracellular signalling and/or interactions with the extracellular matrix. Among metabolically relevant tissues, this gene is expressed at low but significant levels in the adrenal gland, thyroid, pancreas, adipose, and fetal and adult heart, skeletal muscle and liver. As a potential extracellular signalling molecule, the this gene product may serve as an antibody target for diseases involving any or all of these tissues, including obesity and diabetes.

This gene is also detected in several regions of the CNS including amygdala, cerebellum, substantia nigra, hippocampus, thalamus, cerebral cortex and spinal cord. In Drosophilia, the LRR region of axon guidance proteins has been shown to be critical for function (especially in axon repulsion) (ref. 1). Since this gene encodes a leucine-rich-repeat protein that is expressed across all brain regions, it is an excellent candidate neuronal guidance protein for axons, dendrites and/or growth cones in general. Therefore, therapeutic modulation of the levels of this protein, or possible signaling via this protein, may be of utility in enhancing/directing compensatory synaptogenesis and fiber growth in the CNS in response to neuronal death (stroke, head trauma), axon lesion (spinal cord injury), or neurodegeneration (Alzheimer's, Parkinson's, Huntington's, vascular dementia or any neurodegenerative disease) (Battye et al., J. Neurosci. 21: 4290-4298, 2001).

**Panel 4D Summary:** Ag3276 Highest expression of the CG57529_01 transcript is found in resting macrophages and dendritic cells (CTs=25-27), cells that play a crucial role in antigen presentation that acts to activate or tolerize T cells. Significant expression of this transcript is also found in most of the cell types across this panel. This expression in cells that play a role in linking innate immunity to adaptive immunity suggests a role for this gene product in initiating inflammatory reactions. Therefore, modulation of the expression or activity of this gene may reduce or prevent early stages of inflammation and reduce the severity of inflammatory diseases such as psoriasis, asthma, inflammatory bowel disease, rheumatoid arthritis, osteoarthritis and other lung inflammatory diseases.

### T. NOV23: Centaurin Beta-2

Expression of gene CG57351-01 was assessed using the primer-probe set Ag3208, described in Table TA. Results of the RTQ-PCR runs are shown in Tables TB, TC, TD and TE.

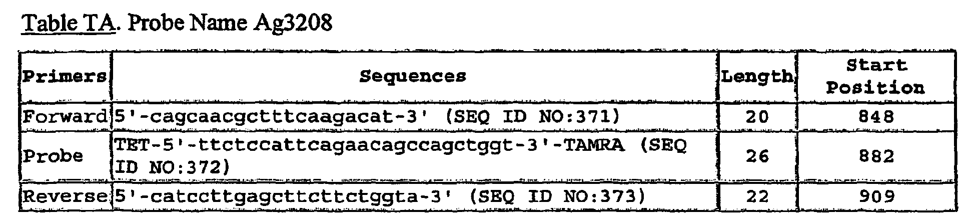

**Table TB. CNS_neurodegeneration_v1.0**

| **Tissue Name** | **Rel. Exp.(%) Ag3208, Run 209861777** | **Tissue Name** | **Rel. Exp.(%) Ag3208, Run 209861777** |
|---|---|---|---|
| AD 1 Hippo | 19.5 | Control (Path) 3 Temporal Ctx | 3.1 |
| AD 2 Hippo | 29.5 | Control (Path) 4 Temporal Ctx | 34.4 |
| AD 3 Hippo | 8.1 | AD 1 Occipital Ctx | 12.2 |
| AD 4 Hippo | 7.3 | AD 2 Occipital Ctx (Missing) | 0.0 |
| AD 5 hippo | 93.3 | AD 3 Occipital Ctx | 3.8 |
| AD 6 Hippo | 42.9 | AD 4 Occipital Ctx | 18.2 |
| Control 2 Hippo | 32.3 | AD 5 Occipital Ctx | 16.7 |
| Control 4 Hippo | 12.2 | AD 6 Occipital Ctx | 42.3 |
| Control (Path) 3 Hippo | 5.2 | Control 1 Occipital Ctx | 3.1 |
| AD 1 Temporal Ctx | 14.4 | Control 2 Occipital Ctx | 79.0 |
| AD 2 Temporal Ctx | 33.7 | Control 3 Occipital Ctx | 13.9 |
| AD 3 Temporal Ctx | 6.7 | Control 4 Occipital Ctx | 2.8 |
| AD 4 Temporal Ctx | 26.8 | Control (Path) 1 Occipital Ctx | 90.8 |
| AD 5 Inf Temporal Ctx | **100.0** | Control (Path) 2 Occipital Ctx | 14.3 |
| AD 5 SupTemporal Ctx | 58.6 | Control (Path) 3 Occipital Ctx | 1.5 |
| AD 6 Inf Temporal Ctx | 41.5 | Control (Path) 4 Occipital Ctx | 21.9 |
| AD 6 Sup Temporal Ctx | 44.1 | Control Parietal Ctx | 7.3 |
| Control 1 Temporal Ctx | 5.0 | Control 2 Parietal Ctx | 52.5 |
| Control 2 Temporal Ctx | 61.6 | Control 3 Parietal Ctx | 22.7 |
| Control 3 Temporal Ctx | 15.1 | Control (Path) 1 Parietal Ctx | 87.1 |
| Control 4 Temporal Ctx | 11.7 | Control (Path) 2 Parietal Ctx | 20.6 |
| Control (Path) 1 Temporal Ctx | 72.7 | Control (Path) 3 Parietal Ctx | 3.6 |
| Control (Path) 2 Temporal Ctx | 46.0 | Control (Path) 4 Parietal Ctx | 43.2 |

**Table TC. Panel 1.3D**

| **Tissue Name** | **Rel. Exp.(%) Ag3208, Run 168012842** | **Tissue Name** | **Rel. Exp.(%) Ag3208, Run 168012842** |
|---|---|---|---|
| Liver adenocarcinoma | 10.2 | Kidney (fetal) | 15.3 |
| Pancreas | 7.2 | Renal ca. 786-0 | 5.0 |
| Pancreatic ca. CAPAN 2 | 3.9 | Renal ca. A498 | 6.8 |
| Adrenal gland | 4.9 | Renal ca. RXF 393 | 10.4 |
| Thyroid | 4.7 | Renal ca. ACHN | 3.8 |
| Salivary gland | 11.0 | Renal ca. UO-31 | 5.9 |
| Pituitary gland | 20.3 | Renal ca. TK-10 | 2.0 |
| Brain (fetal) | 73.2 | Liver | 5.3 |
| Brain (whole) | 99.3 | Liver (fetal) | 5.2 |
| Brain (amygdala) | 35.8 | Liver ca. (hepatoblast) HepG2 | 10.2 |
| Brain (cerebellum) | **100.0** | Lung | 8.5 |
| Brain (hippocampus) | 21.8 | Lung (fetal) | 14.8 |
| Brain (substantia nigra) | 20.6 | Lung ca. (small cell) LX-1 | 18.0 |
| Brain (thalamus) | 29.1 | Lung ca. (small cell) NCI-H69 | 5.7 |
| Cerebral Cortex | 50.3 | Lung ca. (s.cell var.) SHP-77 | 42.3 |
| Spinal cord | 14.7 | Lung ca. (large cell)NCI-H460 | 0.6 |
| glio/astro U87-MG | 6.7 | Lung ca. (non-sm. cell) A549 | 7.1 |
| glio/astro U-118-MG | 9.7 | Lung ca. (non-s.cell) NCI-H23 | 3.7 |
| astrocytoma SW1783 | 6.7 | Lung ca. (non-s.cell) HOP-62 | 5.6 |
| neuro*; met SK-N-AS | 7.7 | Lung ca. (non-s.cl) NCI-H522 | 6.0 |
| astrocytoma SF-539 | 11.7 | Lung ca. (squam.) SW 900 | 7.2 |
| astrocytoma SNB-75 | 13.9 | Lung ca. (squam.) NCI-H596 | 11.7 |
| glioma SNB-19 | 3.1 | Mammary gland | 16.7 |
| glioma U251 | 19.6 | Breast ca.* (pl.ef) MCF-7 | 8.5 |
| glioma SF-295 | 9.8 | Breast ca.* (pl.ef) MDA- | 8.7 |
| Heart (fetal) | 37.1 | Breast ca.* (pl.ef) T47D | 9.7 |
| Heart | 9.0 | Breast ca. BT-549 | 5.2 |
| Skeletal muscle (fetal) | 12.8 | Breast ca. MDA-N | 5.5 |
| Skeletal muscle | 10.2 | Ovary | 4.9 |
| Bone marrow | 4.7 | Ovarian ca. OVCAR-3 | 4.3 |
| Thymus | 16.6 | Ovarian ca. OVCAR-4 | 5.0 |
| Spleen | 4.8 | Ovarian ca. OVCAR-5 | 16.5 |
| Lymph node | 6.5 | Ovarian ca. OVCAR-8 | 1.1 |
| Colorectal | 1.2 | Ovarian ca. IGROV-1 | 1.4 |
| Stomach | 6.7 | Ovarian ca.*(ascites) SK-OV-3 | 14.3 |
| Small intestine | 7.7 | Uterus | 10.5 |
| Colon ca. SW480 | 5.3 | Placenta | 2.6 |
| Colon ca.* SW620(SW480 met) | 19.9 | Prostate | 3.4 |
| Colon ca. HT29 | 4.1 | Prostate ca.* (bone met)PC-3 | 4.2 |
| Colon ca. HCT-116 | 3.9 | Testis | 3.3 |
| Colon ca. CaCo-2 | 8.5 | Melanoma Hs688(A).T | 3.9 |
| Colon ca. tissue(ODO3866) | 3.3 | Melanoma* (met) Hs688(B).T | 3.7 |
| Colon ca. HCC-2998 | 21.5 | Melanoma UACC-62 | 9.8 |
| Gastric ca.* (liver met) NCI-N87 | 4.6 | Melanoma M14 | 3.3 |
| Bladder | 2.0 | Melanoma LOX IMVI | 4.2 |
| Trachea | 4.7 | Melanoma* (met) SK-MEL-5 | 2.5 |
| Kidney | 9.9 | Adipose | 6.8 |

**Table TD. Panel 4D**

| **Tissue Name** | **Rel. Exp.(%) Ag3208, Run 164531754** | **Tissue Name** | **Rel. Exp.(%) Ag3208, Run 164531754** |
|---|---|---|---|
| Secondary Th1 act | 32.5 | HUVEC IL-1beta | 3.5 |
| Secondary Th2 act | 47.6 | HUVEC IFN gamma | 22.8 |
| Secondary Tr1 act | 53.6 | HUVEC TNF alpha + IFN gamma | 10.9 |
| Secondary Th1 rest | 14.3 | HUVEC TNF alpha + IL4 | 21.6 |
| Secondary Th2 rest | 25.2 | HUVEC IL-11 | 17.9 |
| Secondary Tr1 rest | 17.7 | Lung Microvascular EC none | 33.9 |
| Primary Th1 act | 28.5 | Lung Microvascular EC TNFalpha + IL-1beta | 37.1 |
| Primary Th2 act | 28.9 | Microvascular Dermal EC none | 48.6 |
| Primary Tr1 act | 33.0 | Microsvasular Dermal EC TNFalpha + IL-1beta | 18.8 |
| Primary Th1 rest | 59.0 | Bronchial epithelium TNFalpha + IL1beta | 43.8 |
| Primary Th2 rest | 33.7 | Small airway epithelium none | 21.5 |
| Primary Tr1 rest | 32.3 | Small airway epithelium TNFalpha + IL-1beta | 61.6 |
| CD45RA CD4 lymphocyte act | 33.0 | Coronery artery SMC rest | 21.6 |
| CD45RO CD4 lymphocyte act | 24.7 | Coronery artery SMC TNFalpha + IL-1beta | 13.7 |
| CD8 lymphocyte act | 30.1 | Astrocytes rest | 45.7 |
| Secondary CD8 lymphocyte rest | 29.7 | Astrocytes TNFalpha + IL-1beta | 32.8 |
| Secondary CD8 lymphocyte act | 30.6 | KU-812 (Basophil) rest | 24.5 |
| CD4 lymphocyte none | 13.5 | KU-812 (Basophil) PMA/ionomycin | 29.7 |
| 2ry Th1/Th2/Tr1_anti-CD95 CH11 | 36.3 | CCD1106 (Keratinocytes) none | 39.8 |
| LAK cells rest | 25.0 | CCD1106 (Keratinocytes) TNFalpha + IL-1beta | 25.0 |
| LAK cells IL-2 | 31.9 | Liver cirrhosis | 2.6 |
| LAK cells IL-2+IL-12 | 19.9 | Lupus kidney | 5.8 |
| LAK cells IL-2+IFN gamma | 26.2 | NCI-H292 none | 15.8 |
| LAK cells IL-2+IL-18 | 29.7 | NCI-H292 IL-4 | 16.0 |
| LAK cells PMA/ionomycin | 19.8 | NCI-H292 IL-9 | 17.6 |
| NK Cells IL-2 rest | 28.3 | NCI-H292 IL-13 | 18.6 |
| Two Way MLR 3 day | 32.5 | NCI-H292 IFN gamma | 12.9 |
| Two Way MLR 5 day | 29.1 | HPAEC none | 20.0 |
| Two Way MLR 7 day | 15.4 | HPAEC TNF alpha + IL-1 beta | 17.1 |
| PBMC rest | 23.8 | Lung fibroblast none | 59.0 |
| PBMC PWM | 44.4 | Lung fibroblast TNF alpha + IL-1 beta | 20.3 |
| PBMC PHA-L | 44.8 | Lung fibroblast IL-4 | 73.2 |
| Ramos (B cell) none | 29.5 | Lung fibroblast IL-9 | 62.4 |
| Ramos (B cell) ionomycin | 67.8 | Lung fibroblast IL-13 | 65.1 |
| B lymphocytes PWM | 42.6 | Lung fibroblast IFN gamma | 55.1 |
| B lymphocytes CD40L and IL-4 | 31.9 | Dermal fibroblast CCD 1070 rest | 89.5 |
| EOL-1 dbcAMP | 49.3 | Dermal fibroblast CCD1070 TNF alpha | 72.7 |
| EOL-1 dbcAMP PMA/ionomycin | 42.3 | Dermal fibroblast CCD1070 IL-1 beta | 43.2 |
| Dendritic cells none | 31.6 | Dermal fibroblast IFN gamma | 39.5 |
| Dendritic cells LPS | 21.2 | Dermal fibroblast IL-4 | 73.7 |
| Dendritic cells anti-CD40 | 33.0 | IBD Colitis 2 | 5.0 |
| Monocytes rest | 57.4 | IBD Crohn's | 3.4 |
| Monocytes LPS | 11.0 | Colon | 36.6 |
| Macrophages rest | 51.1 | Lung | 23.2 |
| Macrophages LPS | 5.8 | Thymus | 59.0 |
| HUVEC none | 28.3 | Kidney | **100.0** |
| HUVEC starved | 32.1 | | |

**Table TE. Panel CNS_1**

| **Tissue Name** | **Rel. Exp.(%) Ag3208, Run 171694583** | **Tissue Name** | **Rel. Exp.(%) Ag3208, Run 171694583** |
|---|---|---|---|
| BA4 Control | 17.9 | BA17 PSP | 9.7 |
| BA4 Control2 | 44.1 | BA17 PSP2 | 11.0 |
| BA4 Alzheimer's2 | 5.3 | Sub Nigra Control | 26.6 |
| BA4 Parkinson's | 37.6 | Sub Nigra Control2 | 23.5 |
| BA4 Parkinson's2 | 61.1 | Sub Nigra Alzheimer's2 | 10.7 |
| BA4 Huntington's | 32.8 | Sub Nigra Parkinson's2 | 31.2 |
| BA4 Huntington's2 | 14.5 | Sub Nigra Huntington's | 37.9 |
| BA4 PSP | 7.3 | Sub Nigra Huntington's2 | 24.0 |
| BA4 PSP2 | 20.4 | Sub Nigra PSP2 | 0.9 |
| BA4 Depression | 17.0 | Sub Nigra Depression | 3.4 |
| BA4 Depression2 | 3.8 | Sub Nigra Depression2 | 2.9 |
| BA7 Control | 38.2 | Glob Palladus Control | 23.5 |
| BA7 Control2 | 69.3 | Glob Palladus Control2 | 17.9 |
| BA7 Alzheimer's2 | 9.3 | Glob Palladus Alzheimer's | 4.9 |
| BA7 Parkinson's | 26.8 | Glob Palladus Alzheimer's2 | 6.9 |
| BA7 Parkinson's2 | 38.2 | Glob Palladus Parkinson's | **100.0** |
| BA7 Huntington's | 56.6 | Glob Palladus Parkinson's2 | 15.1 |
| BA7 Huntington's2 | 50.3 | Glob Palladus PSP | 2.2 |
| BA7 PSP | 22.4 | Glob Palladus PSP2 | 4.5 |
| BA7 PSP2 | 25.2 | Glob Palladus Depression | 7.5 |
| BA7 Depression | 7.7 | Temp Pole Control | 19.3 |
| BA9 Control | 27.7 | Temp Pole Contro12 | 66.4 |
| BA9 Control2 | 99.3 | Temp Pole Alzheimer's | 8.2 |
| BA9 Alzheimer's | 2.2 | Temp Pole Alzheimer's2 | 7.4 |
| BA9 Alzheimer's2 | 11.0 | Temp Pole Parkinson's | 36.1 |
| BA9 Parkinson's | 37.1 | Temp Pole Parkinson's2 | 42.3 |
| BA9 Parkinson's2 | 43.2 | Temp Pole Huntington's | 39.8 |
| BA9 Huntington's | 50.3 | Temp Pole PSP | 2.5 |
| BA9 Huntington's2 | 27.2 | Temp Pole PSP2 | 5.6 |
| BA9 PSP | 8.4 | Temp Pole Depression2 | 7.5 |
| BA9 PSP2 | 1.7 | Cing Gyr Control | 50.7 |
| BA9 Depression | 6.4 | Cing Gyr Control2 | 41.2 |
| BA9 Depression2 | 12.3 | Cing Gyr Alzheimer's | 14.5 |
| BA17 Control | 52.1 | Cing Gyr Alzheimer's2 | 10.5 |
| BA17 Control | 57.0 | Cing Gyr Parkinson's | 30.6 |
| BA17 Alzheimer's2 | 3.3 | Cing Gyr Parkinson's2 | 33.2 |
| BA17 Parkinson's | 37.4 | Cing Gyr Huntington's | 61.6 |
| BA17 Parkinson's2 | 45.1 | Cing Gyr Huntington's2 | 23.8 |
| BA17 Huntington's | 52.1 | Cing Gyr PSP | 9.4 |
| BA17 Huntington's2 | 22.1 | Cing Gyr PSP2 | 5.0 |
| BA17 Depression | 8.4 | Cing Gyr Depression | 3.6 |
| BA17 Depression2 | 27.5 | Cing Gyr Depression2 | 15.8 |

**CNS_neurodegeneration_v1.0 Summary:** Ag3208 This panel does not show differential expression of the CG57351-01 gene in Alzheimer's disease. However, this expression profile confirms the presence of this gene in the brain. Please see Panel 1.3D for discussion of utility of this gene in the central nervous system.

**Panel 1.3D Summary:** Ag3208 The CG57351-01 gene, a centaurin homolog, shows a brain-preferential expression pattern. Thus, the expression of this gene could be used to distinguish normal brain tissue from other samples in the panel. Because centaurins play a role in vesicular trafficing, they may be involved in synaptic vesicle movement/release. Therefore, this gene may be a drug target for the treatment of any disorder involving neurotransmission (epilepsy, bipolar disorder, schizophrenia, mania, depression, obsessive-compulsive disorder or any neuropsychiatric condition.

In addition, there appears to be substantial expression in samples derived from lung cancer cell lines and colon cancer cell lines. Moreover, therapeutic modulation of this gene, through the use of small molecule drugs, protein therapeutics or antibodies could be of benefit in the treatment of lung or colon cancer.

Among metabolic tissues, this gene is expressed at moderate levels in pancreas, adrenal, thyroid, pituitary, heart, liver, adipose and skeletal muscle. The role of centaurins in vesicular trafficking suggests that this gene may be involved in endocrine secretory processes. Therefore, this widespread expression in metabolic tissues suggests that this gene product may be important for the pathogenesis, diagnosis and/or treatment of metabolic and endocrine disease, including Types 1 and 2 diabetes and obesity.

**Panel 4D Summary:** Ag3208 The CG57351-01 transcript is expressed at high to moderate levels in a wide range of cell types of significance in the immune response and tissue response in health and disease. Therefore, targeting of this gene product with a small molecule drug or antibody therapeutic may modulate the functions of cells of the immune system as well as resident tissue cells and lead to improvement of the symptoms of patients suffering from autoimmune and inflammatory diseases such as COPD, emphysema, asthma, allergies, inflammatory bowel disease, lupus erythematosus, and arthritis, including osteoarthritis and rheumatoid arthritis

**Panel CNS_1 Summary:** Ag3208 This expression profile further confirms the presence of the CG57351-01 gene in the brain. Please see Panel 1.3D for discussion of utility of this gene in the central nervous system.

### U. NOV24: Sorting Nexin 9

Expression of gene CG57515-01 was assessed using the primer-probe set Ag3262, described in Table UA. Results of the RTQ-PCR runs are shown in Tables UB and UC.

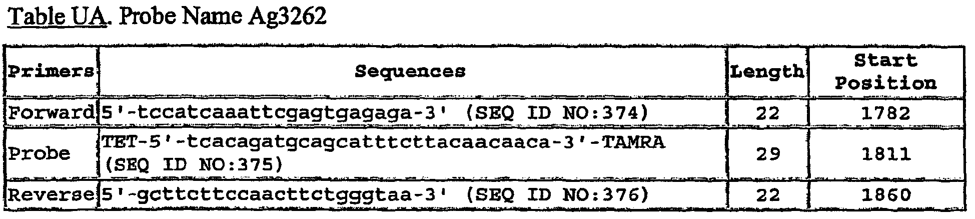

**Table UB. CNS_neurodegeneration_v1.0**

| **Tissue Name** | **Rel. Exp.(%) Ag3262, Run 210038237** | **Tissue Name** | **Rel. Exp.(%) Ag3262, Run 210038237** |
|---|---|---|---|
| AD 1 Hippo | 12.3 | Control (Path) 3 Temporal Ctx | 7.7 |
| AD 2 Hippo | 40.6 | Control (Path) 4 Temporal Ctx | 34.9 |
| AD 3 Hippo | 14.3 | AD 1 Occipital Ctx | 26.1 |
| AD 4 Hippo | 9.5 | AD 2 Occipital Ctx (Missing) | 0.0 |
| AD 5 hippo | 84.1 | AD 3 Occipital Ctx | 15.9 |
| AD 6 Hippo | 71.2 | AD 4 Occipital Ctx | 20.3 |
| Control 2 Hippo | 30.4 | AD 5 Occipital Ctx | 21.2 |
| Control 4 Hippo | 21.3 | AD 6 Occipital Ctx | 43.5 |
| Control (Path) 3 Hippo | 10.9 | Control 1 Occipital Ctx | 9.2 |
| AD 1 Temporal Ctx | 29.9 | Control 2 Occipital Ctx | 51.1 |
| AD 2 Temporal Ctx | 40.1 | Control 3 Occipital Ctx | 23.3 |
| AD 3 Temporal Ctx | 10.2 | Control 4 Occipital Ctx | 10.7 |
| AD 4 Temporal Ctx | 18.9 | Control (Path) 1 Occipital Ctx | 86.5 |
| AD 5 Inf Temporal Ctx | **100.0** | Control (Path) 2 Occipital Ctx | 10.7 |
| AD 5 SupTemporal Ctx | 74.7 | Control (Path) 3 Occipital Ctx | 5.1 |
| AD 6 Inf Temporal Ctx | 85.3 | Control (Path) 4 Occipital Ctx | 18.2 |
| AD 6 Sup Temporal Ctx | 76.8 | Control 1 Parietal Ctx | 12.2 |
| Control 1 Temporal Ctx | 14.6 | Control 2 Parietal Ctx | 69.3 |
| Control 2 Temporal Ctx | 32.5 | Control 3 Parietal Ctx | 16.3 |
| Control 3 Temporal Ctx | 17.8 | Control (Path) 1 Parietal Ctx | 72.2 |
| Control 4 Temporal Ctx | 7.8 | Control (Path) 2 Parietal Ctx | 42.3 |
| Control (Path) 1 Temporal Ctx | 62.4 | Control (Path) 3 Parietal Ctx | 12.1 |
| Control (Path) 2 Temporal Ctx | 33.9 | Control (Path) 4 Parietal Ctx | 41.8 |

**Table UC. Panel 4D**

| **Tissue Name** | **Rel. Exp.(%) Ag3262, Run 164634869** | **Tissue Name** | **Rel. Exp.(%) Ag3262, Run 164634869** |
|---|---|---|---|
| Secondary Th1 act | 15.2 | HUVEC IL-1beta | 7.1 |
| Secondary Th2 act | 11.7 | HUVEC IFN gamma | 7.6 |
| Secondary Tr1 act | 15.3 | HUVEC TNF alpha + IFN gamma | 3.6 |
| Secondary Th1 rest | 5.2 | HUVEC TNF alpha + IL4 | 6.6 |
| Secondary Th2 rest | 5.4 | HUVEC IL-11 | 3.4 |
| Secondary Tr1 rest | 5.4 | Lung Microvascular EC none | 5.1 |
| Primary Th1 act | 2.5 | Lung Microvascular EC TNFalpha +IL-1beta | 3.5 |
| Primary Th2 act | 2.2 | Microvascular Dermal EC none | 5.7 |
| Primary Tr1 act | 3.3 | Microsvasular Dermal EC TNFalpha + IL-1beta | 2.2 |
| Primary Th1 rest | 30.6 | Bronchial epithelium TNFalpha +IL1beta | 14.4 |
| Primary Tb2 rest | 18.2 | Small airway epithelium none | 5.7 |
| Primary Tr1 rest | 6.7 | Small airway epithelium TNFalpha + IL-1beta | 17.9 |
| CD45RA CD4 lymphocyte act | 15.1 | Coronery artery SMC rest | 9.8 |
| CD45RO CD4 lymphocyte act | 15.9 | Coronery artery SMC TNFalpha + IL-1beta | 7.3 |
| CD8 lymphocyte act | 11.5 | Astrocytes rest | 26.6 |
| Secondary CD8 lymphocyte rest | 5.2 | Astrocytes TNFalpha+IL-1beta | 11.0 |
| Secondary CD8 lymphocyte act | 20.0 | KU-812 (Basophil) rest | 1.4 |
| CD4 lymphocyte none | 4.3 | KU-812 (Basophil) PMA/ionomycin | 7.5 |
| 2ry Th1/Th2/Tr1_anti- | 13.2 | CCD1106 (Keratinocytes) none | 13.7 |
| CD95 CH11 | | | |
| LAX cells rest | 16.3 | CCD1106 (Keratinocytes) TNFalpha + IL-1beta | 6.0 |
| LAK cells IL-2 | 45.1 | Liver cirrhosis | 1.7 |
| LAK cells IL-2+IL-12 | 13.3 | Lupus kidney | 1.4 |
| LAK cells IL-2+IFN gamma | 18.9 | NCI-H292 none | 13.8 |
| LAK cells IL-2+ IL-18 | 14.1 | NCI-H292 IL-4 | 15.1 |
| LAK cells PMA/ionomycin | 3.5 | NCI-H292 IL-9 | 19.1 |
| NK Cells IL-2 rest | 16.8 | NCI-H292 IL-13 | 11.7 |
| Two Way MLR 3 day | 11.5 | NCI-H292 IFN gamma | 16.7 |
| Two Way MLR 5 day | 8.0 | HPAEC none | 7.3 |
| Two Way MLR 7 day | 12.0 | HPAEC TNF alpha + IL-1 beta | 3.7 |
| PBMC rest | 7.4 | Lung fibroblast none | 24.7 |
| PBMCPWM | 25.7 | Lung fibroblast TNF alpha + IL-1 beta | 14.1 |
| PBMCPHA-L | 6.6 | Lung fibroblast IL-4 | 100.0 |
| Ramos (B cell) none | 0.4 | Lung fibroblast IL-9 | 49.0 |
| Ramos (B cell) ionomycin | 2.2 | Lung fibroblast IL-13 | 51.8 |
| B lymphocytes PWM | 25.3 | Lung fibroblast IFN gamma | 93.3 |
| B lymphocytes CD40L and IL-4 | 17.2 | Dermal fibroblast CCD1070 rest | 30.6 |
| EOL-1 dbcAMP | 7.1 | Dermal fibroblast CCD1070 TNF alpha | 73.7 |
| EOL-1 dbcAMP PMA/ionomycin | 4.0 | Dermal fibroblast CCD1070 IL-1 beta | 19.3 |
| Dendritic cells none | 11.5 | Dermal fibroblast IFN gamma | 28.9 |
| Dendritic cells LPS | 5.6 | Dermal fibroblast IL-4 | 26.2 |
| Dendritic cells anti-CD40 | 7.9 | IBD Colitis 2 | 0.8 |
| Monocytes rest | 31.9 | IBD Crohn's | 1.0 |
| Monocytes LPS | 13.8 | Colon | 10.2 |
| Macrophages rest | 14.0 | Lung | 12.4 |
| Macrophages LPS | 10.9 | Thymus | 15.9 |
| HUVEC none | 12.7 | Kidney | 4.2 |
| HUVEC starved | 16.6 | | |

**CNS_neurodegeneration_v1.0 Summary:** Ag3262 This panel does not show differential expression of the CG57515-01 gene in Alzheimer's disease. However, this expression profile confirms the presence of this gene, a sorting nexin homolog, in the brain. Sorting nexins have been implicated in axonal guidance; Therefore, this gene may serve as a useful drug target in diseases/clinical conditions where neurons have degenerated/died and compensatory sprouting and/or synaptogenesis is desireable (spinal cord/head trauma, stroke, Huntington's, Parkinson's, or Alzheimer's diseases) (Worby et al., J Biol Chem 2001)

**General_screeniug_panel_v1.4 Summary:** Ag3262 Results from one experiment with the CG57515-01 gene are not included. The amp plot indicates that there were experimental difficulties with this run.

**Panel 4D Summary:** Ag3262 Highest expression of the CG57515-01 transcript is found in lung fibroblasts treated with either Il-4 or IFNg (CT 25.5). It is also observed at high levels in dermal fibroblasts. In addition, this transcript is expressed at high to moderate levels in a wide range of cell types of significance in the immune response and tissue response in health and disease. This transcript encodes a sorting nexin 9 like (SH3PX1) protein. This protein interacts with metalloprotease disintegrins, cysteine-rich proteins that may possess diverse biological functions such as cytokine and protein processing. This interaction is consistent with the expression profile of this gene product. Therefore, this gene may be a useful target for treatment of diseases such as osteoarthritis and rheumatoid arythritis, psoriasis, asthma and chronic obstructive pulmonary diseases (Howard et al., J Biol Chem 1999 Oct 29;274(44):31693-9).

### V. NOV26: Calpain

Expression of gene CG57509-01 was assessed using the primer-probe set Ag2073, described in Table VA. Results of the RTQ-PCR runs are shown in Tables VB and VC.

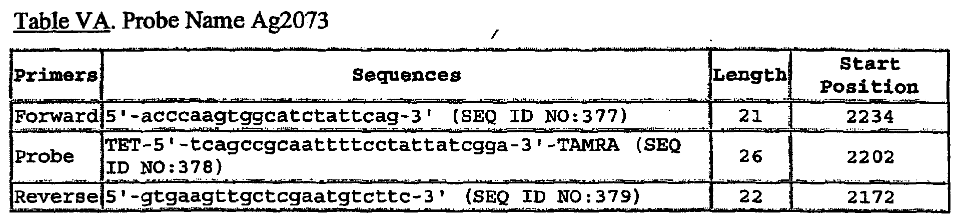

**Table VB. Panel 1.3D**

| **Tissue Name** | **Rel. Exp.(%) Ag2073, Run 165627447** | **Tissue Name** | **Rel. Exp.(%) Ag2073, Run 165627447** |
|---|---|---|---|
| Liver adenocarcinoma | 0.0 | Kidney (fetal) | 0.1 |
| Pancreas | 0.1 | Renal ca. 786-0 | 0.1 |
| Pancreatic ca. CAPAN 2 | 0.0 | Renal ca. A498 | 0.1 |
| Adrenal gland | 0.1 | Renal ca. RXF 393 | 0.0 |
| Thyroid | 0.5 | Renal ca. ACHN | 0.0 |
| Salivary gland | 0.3 | Renal ca. UO-31 | 0.0 |
| Pituitary gland | 0.3 | Renal ca. TK-10 | 0.1 |
| Brain (fetal) | 0.0 | Liver | 0.3 |
| Brain (whole) | 0.7 | Liver (fetal) | 0.3 |
| Brain (amygdala) | 0.4 | Liver ca. (hepatoblast) HepG2 | 0.0 |
| Brain (cerebellum) | 0.5 | Lung | 0.4 |
| Brain (hippocampus) | 0.8 | Lung (fetal) | 0.4 |
| Brain (substantia nigra) | 0.3 | Lung ca. (small cell) LX-1 | 0.2 |
| Brain (thalamus) | 0.5 | Lung ca. (small cell) NCI-H69 | 0.0 |
| Cerebral Cortex | 0.2 | Lung ca. (s.cell var.) SHP-77 | 0.0 |
| Spinal cord | 0.6 | Lung ca. (large cell)NCI- | 0.0 |
| glio/astro U87-MG | 0.0 | Lung ca. (non-sm. cell) A549 | 0.0 |
| glio/astro U-118-MG | 0.2 | Lung ca. (non-s.cell) HCI-H23 | 0.1 |
| astrocytoma SW1783 | 0.0 | Lung ca. (non-s.cell) HOP-62 | 0.0 |
| neuro*; met SK-N-AS | 0.0 | Lung ca. (non-s.cl) NCI-H522 | 0.0 |
| astrocytoma SF-539 | 0.0 | Lung ca. (squam.) SW 900 | 0.0 |
| astrocytoma SNB-75 | 0.0 | Lung ca. (squam) NCI-H596 | 0.0 |
| glioma SNB-19 | 0.1 | Mammary gland | 0.3 |
| glioma U251 | 0.4 | Breast ca.* (pl.ef) MCF-7 | 0.0 |
| glioma SF-295 | 0.0 | Breast ca.* (p1.ef) MDA-MB-231 | 0.1 |
| Heart (fetal) | 0.1 | Breast ca.* (pl.ef) T47D | 0.0 |
| Heart | 0.3 | Breast ca. BT-549 | 0.1 |
| Skeletal muscle (fetal) | 7.7 | Breast ca. MDA-N | 0.0 |
| Skeletal muscle | **100.0** | Ovary | 0.0 |
| Bone marrow | 2.0 | Ovarian ca. OVCAR-3 | 0.1 |
| Thymus | 0.2 | Ovarian ca. OVCAR-4 | 0.0 |
| Spleen | 0.4 | Ovarian ca. OVCAR-5 | 0.1 |
| Lymph node | 1.0 | Ovarian ca. OVCAR-8 | 0.0 |
| Colorectal | 0.2 | Ovarian ca. IGROV-1 | 0.0 |
| Stomach | 0.4 | Ovarian ca.* (ascites) SK-OV-3 | 0.2 |
| Small intestine | 0.9 | Uterus | 0.6 |
| Colon ca.SW480 | 0.0 | Placenta | 0.1 |
| Colon ca.* SW620(SW480 met) | 0.0 | Prostate | 0.3 |
| Colon ca. HT29 | 0.0 | Prostate ca.* (bone met)PC-3 | 0.1 |
| Colon ca. HCT-116 | 0.0 | Testis | 0.8 |
| Colon ca. CaCo-2 | 0.0 | Melanoma Hs688(A).T | 0.0 |
| Colon ca. tissue(ODO3866) | 0.0 | Melanoma* (met) Hs688(B).T | 0.0 |
| Colon ca.HCC-2998 | 0.0 | Melanoma UACC-62 | 0.1 |
| Gastric ca.* (liver met) NCI-N87 | 0.8 | Melanoma M14 | 0.1 |
| Bladder | 0.1 | Melanoma LOX IMVI | 0.0 |
| Trachea | 0.3 | Melanoma* (met) SK-MEL-5 | 0.0 |
| Kidney | 0.5 | Adipose | 0.5 |

**Table VC. Panel 5D**

| **Tissue Name** | **Rel. Exp.(%) Ag2073, Run 169269384** | **Tissue Name** | **Rel. Exp.(%) Ag2073, Run 169269384** |
|---|---|---|---|
| 97457_Patient-02go_adipose | 0.7 | 94709_Donor 2 AM - A_adipose | 0.2 |
| 97476_Patient-07sk_skeletal muscle | 7.9 | 94710_Donor 2 AM - B_adipose | 0.1 |
| 97477_Patient-07ut_uterus | 0.0 | 94711_Donor 2 AM - C_adipose | 0.0 |
| 97478_Patient-07pl_placenta | 0.2 | 94712_Donor 2 AD-A_adipose | 0.0 |
| 97481_Patient-08sk_skeletal muscle | 8.7 | 94713_Donor 2 AD - B_adipose | 0.7 |
| 97482_Patient-08ut_uterus | 0.1 | 94714_Donor 2 AD - C_adipose | 0.3 |
| 97483_Patient-08p1_placenta | 0.1 | 94742_Donor 3 U - A_Mesenchymal Stem Cells | 0.0 |
| 97486_Patient-09sk_skeletal muscle | 14.1 | 94743_Donor 3 U - B_Mesenchymal Stem Cells | 0.0 |
| 97487_Patient-09ut_uterus | 0.1 | 94730_Donor 3 AM - A_adipose | 0.1 |
| 97488_Patient-09p1_placenta | 0.1 | 94731 Donor 3 AM - B_adipose | 0.2 |
| 97492_Patient-10ut_uterus | 0.3 | 94732_Donor 3 AM - C_adipose | 0.2 |
| 97493_Patient-10p1_placenta | 0.1 | 94733_Donor 3 AD - A_adipose | 0.2 |
| 97495_Patient-11go_adipose | 0.2 | 94734_Donor 3 AD-B_adipose | 0.0 |
| 97496_Patient-11sk_skeletal muscle | 82.9 | 94735_Donor 3 AD - C_adipose | 0.2 |
| 97497_Patient-11ut_uterus | 0.1 | 77138_Liver_HepG2untreated | 0.0 |
| 97498_Patient-11p1_placenta | 0.3 | 73556_Heart_Cardiacstromal cells (primary) | 0.2 |
| 97500 Patient-12go_adipose | 0.5 | 81735_Small Intestine | 0.4 |
| 97501_Patient-12sk_skeletal muscle | **100.0** | 72409_Kidney_Proximal Convoluted Tubule | 0.2 |
| 97502_Patient-12ut_uterus | 0.3 | 82685_Small intestine_Duodenum | 0.4 |
| 97503_Patient-12pl_placenta | 0.1 | 90650_Adrenal_Adrenocortical adenoma | 0.1 |
| 94721_Donor 2 U - A_Mesenchymal Stem Cells | 0.2 | 72410_Kidney_HRCE | 0.3 |
| 94722_Donor 2 U - B_Mesenchymal Stem Cells | 0.0 | 72411 _Kidney_HRE | 0.1 |
| 94723_Donor 2 U - C_Mesenchymal Stem Cells | 0.4 | 73139_Uterus_Uterine smooth muscle cells | 0.1 |

**Panel 1.3D Summary:** Ag2073 The CG57909-01 gene, a calpain homolog, has low levels of expression in thyroid, pituitary, heart, adipose and liver. Calpain 10 was recently identified as a susceptibility gene for type 2 diabetes. Thus, this gene product may be a small molecule target for the treatment of endocrine and metabolic disease, including the thyroidopathies, Types 1 and 2 diabetes and obesity. In addition, this gene is highly expressed in skeletal muscle. Mutations in the calpain 3 gene have been proven to be responsible for limb-girdle muscular dystrophy (LGMD) type 2A. Thus, therapeutic modulation of this gene product may be a treatment for LGMD type 2A (Chae et al., Neuromuscul Disord. 2001 Sep;11(6-7):547-55; Huang and Wang, Trends Mol Med. 2001 Aug;7(8):355-62).

**Panel 4D Summary:** Ag2073 Results from one experiment with the CG56003-01 gene are not included. The amp plot indicates that there were experimental difficulties with this run.

**Panel 5D Summary:** Ag2073 Expression of the CG57509-01 gene is restricted to skeletal muscle, confirming the results from Panel 1.3D. Please see Panel 1.3D for discussion of utility of this gene in metabolic disease.

### W. NOV27: Kerratin 18

Expression of gene CG57484-01 was assessed using the primer-probe set Ag1597, described in Table WA. Results of the RTQ-PCR runs are shown in Table WB.

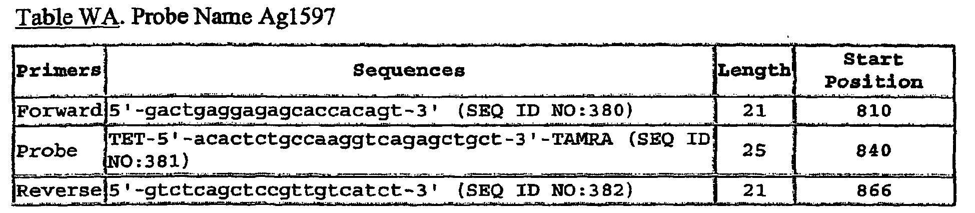

**Table WB. Panel 2D**

| **Tissue Name** | **Rel. Exp.(%) Ag1597, Run 164988404** | **Tissue Name** | **Rel. Exp.(%) Ag1597, Run 164988404** |
|---|---|---|---|
| Normal Colon | 2.3 | Kidney Margin 8120608 | 0.0 |
| CC Well to Mod Diff (ODO3866) | 0.0 | Kidney Cancer 8120613 | 0.0 |
| CC Margin (ODO3866) | 0.0 | Kidney Margin 8120614 | 0.0 |
| CC Gr.2 rectosigmoid | 0.0 | Kidney Cancer 9010320 | 0.0 |
| CC Margin (OD03868) | 0.0 | Kidney Margin 9010321 | 0.0 |
| CC Mod Diff (ODO3920) | 0.0 | Normal Uterus | 0.0 |
| CC Margin (ODO3920) | 0.0 | Uterus Cancer 064011 | 9.3 |
| CC Gr.2 ascend colon (ODO3921) | 0.0 | Normal Thyroid | 0.0 |
| CC Margin (OD03921) | 0.0 | Thyroid Cancer 064010 | 2.5 |
| CC from Partial Hepatectomy (ODO4309) Mets | 0.0 | Thyroid Cancer A302152 | 0.0 |
| Liver Margin (ODO4309) | 0.0 | Thyroid Margin A302153 | 0.0 |
| Colon mets to lung (OD04451-01) | 0.0 | Normal Breast | 2.4 |
| Lung Margin (OD04451-02) | 1.4 | Breast Cancer (OD04566) | 3.5 |
| Normal Prostate 6546-1 | 10.0 | Breast Cancer (OD04590- | 0.0 |
| Prostate Cancer (OD04410) | 7.7 | Breast Cancer Mets | 30.4 |
| Prostate Margin (OD04410) | 2.6 | Breast Cancer Metastasis | 74.7 |
| | | (OD04655-05) | |
| Prostate Cancer (OD04720-01) | 22.4 | Breast Cancer 064006 | 14.0 |
| Prostate Margin (OD04720-02) | 10.8 | Breast Cancer 1024 | 39.2 |
| Normal Lung 061010 | 9.3 | Breast Cancer 9100266 | 0.0 |
| Lung Met to Muscle (ODO4286) | 7.7 | Breast Margin 9100265 | 3.1 |
| Muscle Margin (ODO4286) | 0.0 | Breast Cancer A209073 | 23.2 |
| Lung Malignant Cancer (OD03126) | 21.0 | Breast Margin A209073 | 30.1 |
| Lung Margin (OD03126) | 6.1 | Normal Liver | 16.7 |
| Lung Cancer (OD04404) | 2.9 | Liver Cancer 064003 | 0.0 |
| Lung Margin (OD04404) | 0.0 | Liver Cancer 1025 | 27.0 |
| Lung Cancer (OD04565) | 0.0 | Liver Cancer 1026 | 0.0 |
| Lung Margin (OD04565) | 0.0 | Liver Cancer 6004-T | 0.0 |
| Lung Cancer (OD04237-01) | **100.0** | Liver Tissue 6004-N | 0.0 |
| Lung Margin (OD04237-02) | 10.9 | Liver Cancer 6005-T | 0.0 |
| Ocular Mel Met to Liver (ODO4310) | 2.9 | Liver Tissue 6005-N | 0.0 |
| Liver Margin (ODO4310) | 0.0 | Normal Bladder | 0.0 |
| Melanoma Mets to Lung (OD04321) | 2.0 | Bladder Cancer 1023 | 0.0 |
| Lung Margin (OD04321) | 0.0 | Bladder Cancer A302173 | 0.0 |
| Normal Kidney | 55.1 | Bladder Cancer (OD04718-01) | 0.0 |
| Kidney Ca, Nuclear grade 2 (OD04338) | 14.3 | Bladder Normal Adjacent (OD04718-03) | 0.0 |
| Kidney Margin (OD04338) | 0.0 | Normal Ovary | 0.0 |
| Kidney Ca Nuclear grade 1/2 (OD04339) | 0.0 | Ovarian Cancer 064008 | 0.0 |
| Kidney Margin (OD04339) | 0.0 | Ovarian Cancer (OD04768-07) | 0.0 |
| Kidney Ca, Clear cell type (OD04340) | 0.0 | Ovary Margin (OD04768-08) | 0.0 |
| Kidney Margin (OD04340) | 0.0 | Normal Stomach | 13.4 |
| Kidney Ca, Nuclear grade 3 (OD04348) | 0.0 | Gastric Cancer 9060358 | 0.0 |
| Kidney Margin (OD04348) | 36.9 | Stomach Margin 9060359 | 0.0 |
| Kidney Cancer (OD04622-01) | 0.0 | Gastric Cancer 9060395 | 0.0 |
| Kidney Margin (OD04622-03) | 4.7 | Stomach Margin 9060394 | 3.0 |
| Kidney Cancer (OD04450-01) | 0.0 | Gastric Cancer 9060397 | 0.0 |
| Kidney Margin (OD04450-03) | 0.0 | Stomach Margin 9060396 | 0.0 |
| Kidney Cancer 8120607 | 0.0 | Gastric Cancer 064005 | 0.0 |

**Panel 2D Summary:** Ag1597 Expression of the CG57484-01 gene is limited to samples derived from breast cancer and lung cancer (CTs=33). Thus, expression of this gene could be used to differentiate between these samples and other samples on this panel and as a marker to detect the presence of breast and lung cancers. This gene encodes a protein that is homologous to keratin 18. Expression of keratin 18 has been shown in both lung and breast cancers. Therefore, therapeutic modulation of the expression or function of this gene may be effective in the treatment ofbreast and lung cancers (Chu et al., Selection of invasive and metastatic subpopulations from a human lung adenocarcinoma cell line. Am J Respir Cell Mol Biol 1997 Sep;17(3):353-60).

### X. NOV29: Cholinephosphate Cytidylyltransferase

Expression of gene CG57589-01 was assessed using the primer-probe set Ag4146, described in Table XA. Results of the RTQ-PCR runs are shown in Tables XB and XC.

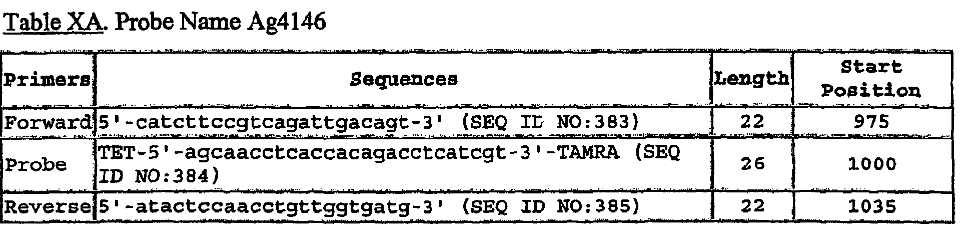

**Table XB. General_screeningyanel_v1.4**

| **Tissue Name** | **Rel. Exp.(%) Ag4146, Run 221290908** | **Tissue Name** | **Rel. Exp.(%) Ag4146, Run 221290908** |
|---|---|---|---|
| Adipose | 0.8 | Renal ca. TK-10 | 19.3 |
| Melanoma* Hs688(A).T | 4.7 | Bladder | 4.2 |
| Melanoma* Hs688(B).T | 4.8 | Gastric ca. (liver met.) NCI-N87 | 22.7 |
| Melanoma*M14 | 11.1 | Gastric ca. KATO III | 49.3 |
| Melanoma* LOXIMVI | 5.0 | Colon ca. SW-948 | 16.2 |
| Melanoma* SK-MEL-5 | 21.5 | Colon ca. SW480 | 24.1 |
| Squamous cell carcinoma SCC-4 | 4.7 | Colon ca.* (SW480 met) SW620 | 12.2 |
| Testis Pool | 27.9 | Colon ca. HT29 | 13.8 |
| Prostate ca.* (bone met) PC-3 | 3.3 | Colon ca. HCT-116 | 25.0 |
| Prostate Pool | 2.3 | Colon ca. CaCo-2 | 26.2 |
| Placenta | 2.7 | Colon cancer tissue | 2.2 |
| Uterus Pool | 0.4 | Colon ca. SW1 116 | 4.9 |
| Ovarian ca. OVCAR-3 | 15.4 | Colon ca. Colo-205 | 12.0 |
| Ovanan ca. SK-OV-3 | 11.4 | Colon ca. SW-48 | 6.5 |
| Ovarian ca. OVCAR-4 | 12.4 | Colon Pool | 1.8 |
| Ovarian ca. OVCAR-5 | 32.3 | Small Intestine Pool | 0.9 |
| Ovarian ca. IGROV-1 | 11.0 | Stomach Pool | 1.0 |
| Ovarian ca. OVCAR-8 | 5.1 | Bone Marrow Pool | 0.6 |
| Ovary | 2.3 | Fetal Heart | 2.9 |
| Breast ca. MCF-7 | 14.0 | Heart Pool | 1.7 |
| Breast ca. MDA-MB-231 | 21.9 | Lymph Node Pool | 2.1 |
| Breast ca. BT 549 | 11.5 | Fetal Skeletal Muscle | 0.8 |
| Breast ca. T47D | **100.0** | Skeletal Muscle Pool | 3.7 |
| Breast ca. MDA-N | 10.9 | Spleen Pool | 0.8 |
| Breast Pool | 1.6 | Thymus Pool | 1.6 |
| Trachea | 4.1 | CNS cancer (glio/astro) U87-MG | 26.8 |
| Lung | 0.4 | CNS cancer (glio/astro) U- 118-MG | 15.0 |
| Fetal Lung | 3.9 | CNS cancer (neuro;met) SK-N-AS | 12.5 |
| Lung ca. NCI-N417 | 2.6 | CNS cancer (astro) SF-539 | 22.4 |
| Lung ca. LX-1 | 13.7 | CNS cancer (astro) SNB-75 | 17.6 |
| Lung ca. NCI-H146 | 1.8 | CNS cancer (glio) SNB-19 | 12.2 |
| Lung ca. SHP-77 | 7.4 | CNS cancer (glio) SF-295 | 10.9 |
| Lung ca. A549 | 5.4 | Brain (Amygdala) Pool | 6.1 |
| Lungca.NCI-H526 | 5.5 | Brain (cerebellum) | 12.6 |
| Lung ca. NCI-H23 | 2.2 | Brain (fetal) | 7.1 |
| Lung ca. NCI-H460 | 1.2 | Brain (Hippocampus) Pool | 6.2 |
| Lung ca. HOP-62 | 5.8 | Cerebral Cortex Pool | 6.8 |
| Lung ca. NCI-H522 | 6.3 | Brain (Substantia nigra) Pool | 10.1 |
| Liver | 7.3 | Brain (Thalmus) Pool | 10.4 |
| Fetal Liver | 14.2 | Brain (whole) | 8.9 |
| Liver ca. HepG2 | 29.5 | Spinal Cord Pool | 5.3 |
| Kidney Pool | 2.8 | Adrenal Gland | 3.1 |
| Fetal Kidney | 3.3 | Pituitary gland Pool | 0.7 |
| Renal ca. 786-0 | 11.3 | Salivary Gland | 1.8 |
| Renal ca. A498 | 4.9 | Thyroid (female) | 2.0 |
| Renal ca. ACHN | 9.9 | Pancreatic ca. CAPAN2 | 12.5 |
| Renal ca. UO-31 | 4.2 | Pancreas Pool | 2.8 |

**Table XC. Panel 5 Islet**

| **Tissue Name** | **Rel. Exp.(%) Ag4146, Run 242413200** | **Tissue Name** | **Rel. Exp.(%) Ag4146, Run 242413200** |
|---|---|---|---|
| 97457_Patient-02go_adipose | 4.0 | 94709_Donor 2 AM - A_adipose | 18.0 |
| 97476 Patient-07sk_skeletal muscle | 1.2 | 94710_Donor 2 AM - B_adipose | 8.0 |
| 97477_Patient-07ut_uterus | 5.4 | 94711_Donor 2 AM - C_adipose | 4.7 |
| 97478 Patient-07pl_placenta | 6.0 | 94712_Donor 2 AD-A_adipose | 13.6 |
| 99167_Bayer Patient 1 | 10.9 | 94713_Donor 2 AD-B_adipose | 15.3 |
| 97482_Patient-08ut_uterus | 4.0 | 94714_Donor 2 AD-C_adipose | 14.4 |
| 97483_Patient-08pl_placenta | 1.3 | 94742_Donor 3 U - A_Mesenchymal Stem Cells | 8.2 |
| 97486_Patient-09sk_skeletal muscle | 0.7 | 94743_Donor 3 U-B_Mesenchymal Stem Cells | 6.0 |
| 97487_Patient-09ut_uterus | 1.1 | 94730_Donor 3 AM - A_adipose | 8.9 |
| 97488_Patient-09pl_placenta | 2.4 | 94731_Donor 3 AM - B_adipose | 8.6 |
| 97492_Patient-10ut_uterus | 4.4 | 94732_Donor 3 AM - C_adipose | 5.9 |
| 97493_Patient-10pl_placenta | 8.0 | 94733_Donor 3 AD - A_adipose | 17.6 |
| 97495_Patient-11go_adipose | 1.8 | 94734_Donor 3 AD - B_adipose | 4.8 |
| 97496_Patient-11sk_skeletal muscle | 3.7 | 94735_Donor 3 AD - C_adipose | 8.8 |
| 97497_Patient-11ut_uterus | 5.3 | 77138_Liver_HepG2tmtreated | **100.0** |
| 97498_Patient-1p1_placenta | 3.7 | 73556_Heart_Cardiac stromal cells (primary) | 3.7 |
| 97500_Pafient-12go_adipose | 9.3 | 81735_Small Intestine | 13.1 |
| 97501_Patient-12sk_skeletal muscle | 11.7 | 72409_Kidney_Proximal Convoluted Tubule | 4.1 |
| 97502_Patient-12ut_uterus | 7.0 | 82685_Small intestine_Duodenum | 3.9 |
| 97503_Patient-12pl_placenta | 6.5 | 90650_Adrenal_Adrenocortical adenoma | 2.6 |
| 94721_Donor 2 U - A_Mesenchymal Stem Cells | 5.8 | 72410_Kidney_HRCE | 37.1 |
| 94722_Donor 2 U - B_Mesenchymal Stem Cells | 7.5 | 7241_Kidney_HRE | 6.0 |
| 94723_Donor 2 U - C_Mesenchymal Stem Cells | 14.1 | 73139_Uterus_Uterine smooth muscle cells | 4.5 |

**General_screening_panel_v1.4 Summary:** Ag4146 The expression of the CG57589-01 gene appears to be highest in a sample derived from a breast cancer cell line (T47D)(CT=27.2). In addition, there appears to be substantial expression in other samples derived from breast cancer cell lines, ovarian cancer cell lines, brain cancer cell lines and colon cancer cell lines. Thus, the expression of this gene could be used to distinguish T47D cells from other samples in the panel. Moreover, therapeutic modulation of this gene, through the use of small molecule drugs, protein therapeutics or antibodies could be of benefit in the treatment of breast, ovarian, brain or colon cancer.

Among metabolic tissue, this gene has low levels of expression in adipose, heart, skeletal muscle, adrenal, pituitary, thyroid, and pancreas. Dysregulated levels of phosphatidylcholine may be involved in the pathogenesis of disease in these tissues. Therefore, this gene may be a small molecule target for the treatment of metabolic and endocrine disease, including Types 1 and 2 diabetes and obesity.

This gene, a cholinephosphate cytidylyltransferase homolog, is also expressed at moderate levels in all CNS regions examined. This protein has been shown to be upregulated in the brain during triethyltin-induced cerebral edema. Therefore this gene may be a drug target for the treatment of cerebral edema (Mages et al., Pharmacol Toxicol 1989 Oct;65(4):302-5).

**Panel 5 Islet Summary:** Ag4146 The CG57589-01 is expressed at a low level in the islets of Langerhans, with highest expression in a liver derived sample (CT=30.5). Thus, expression of this gene could be used to differentiate between this sample and other samples on this panel.

### Y. NOV30: mac25/IGFBP-7

Expression of gene CG57558-01 was assessed using the primer-probe set Ag3285, described in Table YA.

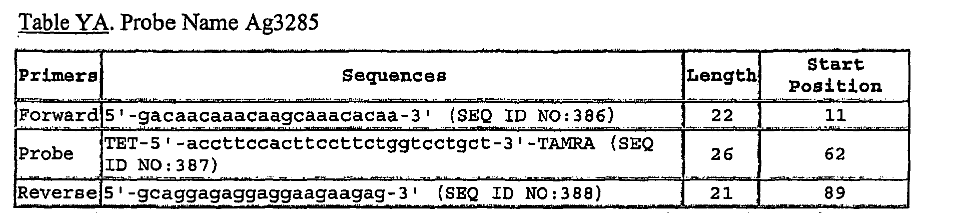

**CNS_neurodegeneration_v1.0 Summary:** Ag3285 Expression of this gene is low/undetectable (CTs > 35) across all of the samples on this panel.

**Panel 1.3D Summary:** Ag3285 Expression of this gene is low/undetectable (CTs > 35) across all of the samples on this panel.

**Panel 4D Summary:** Ag3285 Expression of this gene is low/undetectable (CTs > 35) across all of the samples on this panel.

### OTHER EMBODIMENTS

Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made to the invention without departing from the spirit and scope of the invention as defined by the claims. The choice of nucleic acid starting material, clone of interest, or library type is believed to be a matter of routine for a person of ordinary skill in the art with knowledge of the embodiments described herein. Other aspects, advantages, and modifications considered to be within the scope of the following claims.

In other definitions of the invention, polypeptides, nucleic acids, antibodies, and assorted products, methods and uses are provided as described in the following clauses:
Clause 1. An isolated polypeptide comprising an amino acid sequence selected from the group consisting of:
   (a) a mature form of an amino acid sequence selected from the group consisting of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94;
   (b) a variant of a mature form of an amino acid sequence selected from the group consisting of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, wherein one or more amino acid residues in said variant differs from the amino acid sequence of said mature form, provided that said variant differs in no more than 15% of the amino acid residues from the amino acid sequence of said mature form;
   (c) an amino acid sequence selected from the group consisting of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94; and
   (d) a variant of an amino acid sequence selected from the group consisting of SEQ ID NOS:2, 4, 6, 8, 10, 12,14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94 wherein one or more amino acid residues in said variant differs from the amino acid sequence of said mature form, provided that said variant differs in no more than 15% of amino acid residues from said amino acid sequence.
Clause 2. The polypeptide of clause 1, wherein said polypeptide comprises the amino acid sequence of a naturally-occurring allelic variant of an amino acid sequence selected from the group consisting of SEQ ID NOS:2, 4, 6, 8, 10, 12, 14,16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94.
Clause 3. The polypeptide of clause 2, wherein said allelic variant comprises an amino acid sequence that is the translation of a nucleic acid sequence differing by a single nucleotide from a nucleic acid sequence selected from the group consisting of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93.
Clause 4. The polypeptide of clause 1, wherein the amino acid sequence of said variant comprises a conservative amino acid substitution.
Clause 5. An isolated nucleic acid molecule comprising a nucleic acid sequence encoding a polypeptide comprising an amino acid sequence selected from the group consisting of:
   (a) a mature form of an amino acid sequence selected from the group consisting of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94;
   (b) a variant of a mature form of an amino acid sequence selected from the group consisting of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, wherein one or more amino acid residues in said variant differs from the amino acid sequence of said mature form, provided that said variant differs in no more than 15% of the amino acid residues from the amino acid sequence of said mature form;
   (c) an amino acid sequence selected from the group consisting of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94;
   (d) a variant of an amino acid sequence selected from the group consisting of SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, wherein one or more amino acid residues in said variant differs from the amino acid sequence of said mature form, provided that said variant differs in no more than 15% of amino acid residues from said amino acid sequence;
   (e) a nucleic acid fragment encoding at least a portion of a polypeptide comprising an amino acid sequence chosen from the group consisting of SEQ ID NOS:2, 4, 6, 8, 10, 12,14, 16,18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94, or a variant of said polypeptide, wherein one or more amino acid residues in said variant differs from the amino acid sequence of said mature form, provided that said variant differs in no more than 15% of amino acid residues from said amino acid sequence; and
   (f) a nucleic acid molecule comprising the complement of (a), (b), (c), (d) or (e).
Clause 6. The nucleic acid molecule of clause 5, wherein the nucleic acid molecule comprises the nucleotide sequence of a naturally-occurring allelic nucleic acid variant.
Clause 7. The nucleic acid molecule of clause 5, wherein the nucleic acid molecule encodes a polypeptide comprising the amino acid sequence of a naturally-occurring polypeptide variant.
Clause 8. The nucleic acid molecule of clause 5, wherein the nucleic acid molecule differs by a single nucleotide from a nucleic acid sequence selected from the group consisting of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93.
Clause 9. The nucleic acid molecule of clause 5, wherein said nucleic acid molecule comprises a nucleotide sequence selected from the group consisting of
   (a) a nucleotide sequence selected from the group consisting of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93;
   (b) a nucleotide sequence differing by one or more nucleotides from a nucleotide sequence selected from the group consisting of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, provided that no more than 20% of the nucleotides differ from said nucleotide sequence;
   (c) a nucleic acid fragment of (a); and
   (d) a nucleic acid fragment of (b).
Clause 10. The nucleic acid molecule of clause 5, wherein said nucleic acid molecule hybridizes under stringent conditions to a nucleotide sequence chosen from the group consisting of SEQ ID NOS:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93, or a complement of said nucleotide sequence.
Clause 11. The nucleic acid molecule of clause 5, wherein the nucleic acid molecule comprises a nucleotide sequence selected from the group consisting of
   (a) a first nucleotide sequence comprising a coding sequence differing by one or more nucleotide sequences from a coding sequence encoding said amino acid sequence, provided that no more than 20% of the nucleotides in the coding sequence in said first nucleotide sequence differ from said coding sequence;
   (b) an isolated second polynucleotide that is a complement of the first polynucleotide; and
   (c) a nucleic acid fragment of (a) or (b).
Clause 12. A vector comprising the nucleic acid molecule of clause 11.
Clause 13. The vector of clause 12, further comprising a promoter operably-linked to said nucleic acid molecule.
Clause 14. A cell comprising the vector of clause 12.
Clause 15. An antibody that immunospecifically-binds to the polypeptide of clause 1.
Clause 16. The antibody of clause 15, wherein said antibody is a monoclonal antibody.
Clause 17. The antibody of clause 15, wherein the antibody is a humanized antibody.
Clause 18. A method for determining the presence or amount of the polypeptide of clause 1 in a sample, the method comprising:
   (a) providing the sample;
   (b) contacting the sample with an antibody that binds immunospecifically to the polypeptide; and
   (c) determining the presence or amount of antibody bound to said polypeptide,
   thereby determining the presence or amount of polypeptide in said sample.
Clause 19. A method for determining the presence or amount of the nucleic acid molecule of clause 5 in a sample, the method comprising:
   (a) providing the sample;
   (b) contacting the sample with a probe that binds to said nucleic acid molecule; and
   (c) determining the presence or amount of the probe bound to said nucleic acid molecule,
   thereby determining the presence or amount of the nucleic acid molecule in said sample.
Clause 20. A method of identifying an agent that binds to a polypeptide of clause 1, the method comprising:
   (a) contacting said polypeptide with said agent; and
   (b) determining whether said agent binds to said polypeptide.
Clause 21. A method for identifying an agent that modulates the expression or activity of the polypeptide of clause 1, the method comprising:
   (a) providing a cell expressing said polypeptide;
   (b) contacting the cell with said agent; and
   (c) determining whether the agent modulates expression or activity of said polypeptide,
   whereby an alteration in expression or activity of said peptide indicates said agent modulates expression or activity of said polypeptide.
Clause 22. A method for modulating the activity of the polypeptide of clause 1, the method comprising contacting a cell sample expressing the polypeptide of said clause with a compound that binds to said polypeptide in an amount sufficient to modulate the activity of the polypeptide.
Clause 23. A method of treating or preventing a NOVX-associated disorder, said method comprising administering to a subject in which such treatment or prevention is desired the polypeptide of clause 1 in an amount sufficient to treat or prevent said NOVX-associated disorder in said subject.
Clause 24. The method of clause 23, wherein said subject is a human.
Clause 25. A method of treating or preventing a NOVX-associated disorder, said method comprising administering to a subject in which such treatment or prevention is desired the nucleic acid of clause 5 in an amount sufficient to treat or prevent said NOVX-associated disorder in said subject.
Clause 26. The method of clause 25, wherein said subject is a human.
Clause 27. A method of treating or preventing a NOVX-associated disorder, said method comprising administering to a subject in which such treatment or prevention is desired the antibody of clause 15 in an amount sufficient to treat or prevent said NOVX-associated disorder in said subject.
Clause 28. The method of clause 27, wherein the subject is a human.
Clause 29. A pharmaceutical composition comprising the polypeptide of clause 1 and a pharmaceutically-acceptable carrier.
Clause 30. A pharmaceutical composition comprising the nucleic acid molecule of clause 5 and a pharmaceutically-acceptable carrier.
Clause 31. A pharmaceutical composition comprising the antibody of clause 15 and a pharmaceutically-acceptable carrier.
Clause 32. A kit comprising in one or more containers, the pharmaceutical composition of clause 29.
Clause 33. A kit comprising in one or more containers, the pharmaceutical composition of clause 30.
Clause 34. A kit comprising in one or more containers, the pharmaceutical composition of clause 31.
Clause 35. The use of a therapeutic in the manufacture of a medicament for treating a syndrome associated with a human disease, the disease selected from a NOVX-associated disorder, wherein said therapeutic is selected from the group consisting of a NOVX polypeptide, a NOVX nucleic acid, and a NOVX antibody.
Clause 36. A method for screening for a modulator of activity or of latency or predisposition to a NOVX-associated disorder, said method comprising:
   (a) administering a test compound to a test animal at increased risk for a NOVX-associated disorder, wherein said test animal recombinantly expresses the polypeptide of clause 1;
   (b) measuring the activity of said polypeptide in said test animal after administering the compound of step (a);
   (c) comparing the activity of said protein in said test animal with the activity of said polypeptide in a control animal not administered said polypeptide, wherein a change in the activity of said polypeptide in said test animal relative to said control animal indicates the test compound is a modulator of latency of or predisposition to a NOVX-associated disorder.
Clause 37. The method of clause. 36, wherein said test animal is a recombinant test animal that expresses a test protein transgene or expresses said transgene under the control of a promoter at an increased level relative to a wild-type test animal, and wherein said promoter is not the native gene promoter of said transgene.
Clause 38. A method for determining the presence of or predisposition to a disease associated with altered levels of the polypeptide of clause 1 in a first mammalian subject, the method comprising:
   (a) measuring the level of expression of the polypeptide in a sample from the first mammalian subject; and
   (b) comparing the amount of said polypeptide in the sample of step (a) to the amount of the polypeptide present in a control sample from a second mammalian subject known not to have, or not to be predisposed to, said disease,
   wherein an alteration in the expression level of the polypeptide in the first subject as compared to the control sample indicates the presence of or predisposition to said disease.
Clause 39. A method for determining the presence of or predisposition to a disease associated with altered levels of the nucleic acid molecule of clause 5 in a first mammalian subject, the method comprising:
   (a) measuring the amount of the nucleic acid in a sample from the first mammalian subject; and
   (b) comparing the amount of said nucleic acid in the sample of step (a) to the amount of the nucleic acid present in a control sample from a second

## Claims

1. An isolated polypeptide comprising an amino acid sequence selected from the group consisting of:
(a) a mature form of an amino acid sequence selected from the group consisting of SEQ ID NOs: 26, 28, 30, 32, and 34;
(b) a variant of a mature form of an amino acid sequence selected from the group consisting of SEQ ID NOs: 26, 28, 30, 32, and 34, wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NOs: 26, 28, 30, 32, and 34; and
(d) a variant of an amino acid sequence selected from the group consisting of SEQ ID NOs: 26, 28, 30, 32, and 34, wherein one or more amino acid residues in said variant differs from the amino acid sequence of said mature form, provided that said variant differs in no more than 15% of amino acid residues from said amino acid sequence.

2. The polypeptide of claim 1, wherein said polypeptide comprises the amino acid sequence of a naturally-occurring allelic variant of an amino acid sequence selected from the group consisting of SEQ ID NOs: 26, 28, 30, 32, and 34.

3. The polypeptide of claim 2, wherein said allelic variant comprises an amino acid sequence that is the translation of a nucleic acid sequence differing by a single nucleotide from a nucleic acid sequence selected from the group consisting of SEQ ID NOs: 25, 27, 29, 31, 33, and 35.

4. The polypeptide of claim 1, wherein the amino acid sequence of said variant comprises a conservative amino acid substitution.

5. The polypeptide of claim 1 comprising a variant of amino acid sequence of SEQ ID NO: 26, wherein said variant has one or more amino acid changes selected from the group consisting of:
Lys to Arg at amino acid position 8;
Val to Ala at amino acid position 44;
Cys to Arg at amino acid position 46;
Ser to Tyr at amino acid position 55;
Leu to Pro at amino acid position 111;
Ser to Gly at amino acid position 114;
Ser to Asn at amino acid position 114; and
Ala to Val at amino acid position 134.

6. The polypeptide of claim 1 comprising a variant of amino acid sequence of SEQ ID NO: 32, wherein said variant has one or more amino acid changes selected from the group consisting of:
Ala to Val at amino acid position 40;
Val to Ala at amino acid position 80;
Cys to Arg at amino acid position 82;
Ser to Tyr at amino acid position 91;
Val to Ile at amino acid position 96;
Lys to Glu at amino acid position 111;
Arg to Gln at amino acid position 134;

7. An isolated nucleic acid molecule comprising a nucleic acid sequence encoding an amino acid sequence selected from the group consisting of:
(a) a mature form of an amino acid sequence selected from the group consisting of SEQ ID NOs: 26, 28, 30, 32, and 34;
(b) a variant of a mature form of an amino acid sequence selected from the group consisting of SEQ ID NOs: 26, 28, 30, 32, and 34, wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NOs: 26, 28, 30, 32, and 34;
(d) a variant of an amino acid sequence selected from the group consisting of SEQ ID NOs: 26, 28, 30, 32, and 34, wherein one or more amino acid residues in said variant differs from the amino acid sequence of said mature form, provided that said variant differs in no more than 15% of amino acid residues from said amino acid sequence;
(e) a nucleic acid fragment encoding at least a portion of a polypeptide comprising an amino acid sequence chosen from the group consisting of SEQ ID NOs: 26, 28, 30, 32, and 34, or a variant of said polypeptide, wherein one or more amino acid residues in said variant differs from the amino acid sequence of said mature form, provided that said variant differs in no more than 15% of amino acid residues from said amino acid sequence; and
(f) a nucleic acid molecule comprising the complement of (a), (b), (c), (d) or (e).

8. The nucleic acid molecule of claim 7, wherein the nucleic acid molecule comprises the nucleotide sequence of a naturally-occurring allelic nucleic acid variant.

9. The nucleic acid molecule of claim 7, wherein the nucleic acid molecule encodes a polypeptide comprising the amino acid sequence of a naturally-occurring polypeptide variant.

10. The nucleic acid molecule of claim 7, wherein the nucleic acid molecule differs by a single nucleotide from a nucleic acid sequence selected from the group consisting of SEQ ID NOs: 25, 27, 29, 31, and 33.

11. The nucleic acid molecule of claim 7, wherein said nucleic acid molecule comprises a nucleotide sequence selected from the group consisting of
(a) a nucleotide sequence selected from the group consisting of SEQ ID NOs: 25, 27, 29, 31, and 33;
(b) a nucleotide sequence differing by one or more nucleotides from a nucleotide sequence selected from the group consisting of SEQ ID NOs: 25, 27, 29, 31, and 33, provided that no more than 20% of the nucleotides differ from said nucleotide sequence;
(c) a nucleic acid fragment of (a); and
(d) a nucleic acid fragment of (b).

12. The nucleic acid molecule of claim 7, wherein said nucleic acid molecule hybridizes under stringent conditions to a nucleotide sequence chosen from the group consisting of SEQ ID NOs: 25, 27, 29, 31, and 33, or a complement of said nucleotide sequence.

13. The nucleic acid molecule of claim 7 comprising a variant of SEQ ID NO: 25, wherein said variant has one or more nucleotide changes selected from the group as set forth in Table 8K.

14. The nucleic acid molecule of claim 7 comprising a variant of SEQ ID NO: 31, wherein said variant has one or more nucleotide changes selected from the group as set forth in Table 8L.

15. A vector comprising the nucleic acid molecule of claim 7.

16. The vector of claim 15, further comprising a promoter operably-linked to said nucleic acid molecule.

17. A cell comprising the vector of claim 15.

18. An antibody that immunospecifically-binds to the polypeptide of claim 1.

19. The antibody of claim 18, wherein said antibody is a monoclonal antibody.

20. The antibody of claim 18, wherein the antibody is a humanized antibody.

21. A method for determining the presence or amount of the polypeptide of claim 1 in a sample, the method comprising:
(a) providing the sample;
(b) contacting the sample with an antibody that binds immunospecifically to the polypeptide; and
(c) determining the presence or amount of antibody bound to said polypeptide,
thereby determining the presence or amount of polypeptide in said sample.

22. A method for determining the presence or amount of the nucleic acid molecule of claim 7 in a sample, the method comprising:
(a) providing the sample;
(b) contacting the sample with a probe that binds to said nucleic acid molecule; and
(c) determining the presence or amount of the probe bound to said nucleic acid molecule,
thereby determining the presence or amount of the nucleic acid molecule in said sample.

23. A method of identifying an agent that binds to a polypeptide of claim 1, the method comprising:
(a) contacting said polypeptide with said agent; and
(b) determining whether said agent binds to said polypeptide.

24. A method for identifying an agent that modulates the expression or activity of the polypeptide of claim 1, the method comprising:
(a) providing a cell expressing said polypeptide;
(b) contacting the cell with said agent; and
(c) determining whether the agent modulates expression or activity of said polypeptide,
whereby an alteration in expression or activity of said peptide indicates said agent modulates expression or activity of said polypeptide.

25. A pharmaceutical composition comprising a pharmaceutically-acceptable carrier and
(a) the polypeptide of claim 1;
(b) the nucleic acid molecule of claim 7; or
(c) the antibody of claim 18.

26. A method for determining the presence of or predisposition to a disease associated with altered levels of the polypeptide of claim 1 in a first mammalian subject, the method comprising:
(a) measuring the level of expression of the polypeptide in a sample from the first mammalian subject; and
(b) comparing the amount of said polypeptide in the sample of step (a) to the amount of the polypeptide present in a control sample from a second mammalian subject known not to have, or not to be predisposed to, said disease,
wherein an alteration in the expression level of the polypeptide in the first subject as compared to the control sample indicates the presence of or predisposition to said disease.

27. A method for determining the presence of or predisposition to a disease associated with altered levels of the nucleic acid molecule of claim 5 in a first mammalian subject, the method comprising:
(a) measuring the amount of the nucleic acid in a sample from the first mammalian subject; and
(b) comparing the amount of said nucleic acid in the sample of step (a) to the amount of the nucleic acid present in a control sample from a second mammalian subject known not to have or not be predisposed to, the disease;
wherein an alteration in the level of the nucleic acid in the first subject as compared to the control sample indicates the presence of or predisposition to the disease.

28. Use of a NOV8 polypeptide, a NOV8 nucleic acid or a NOV8 antibody for the manufacture of a medicament for treatment of a disorder associated with T lymphocyte activation.
